# EUROPÄISCHE PATENTANMELDUNG

(11) **EP 1 344 834 A2**
(43) Veröffentlichungstag der Anmeldung: **17.09.2003**
(21) Anmeldenummer: 03004810.2
(22) Anmeldetag: 04.03.2003
(51) Int. Cl.: C12Q 1/68

(54) **Verfahren zur Voraussage der Toxizität einer Verbindung**

(30) Priorität: 14.03.2002 EP 02005336; 17.07.2002 EP 02015657
(71) Anmelder: F. HOFFMANN-LA ROCHE AG, 4070 Basel (CH)
(72) Erfinder: Boess, Franziska, 4125 Riehen (CH); Suter-Dick, Laura, 4103 Bottmingen (CH); Wolf, Detlef, 79639 Grenzach-Wyhlen (CH)

(57) **Abstract**

The present invention is based on the determination of the global changes in gene expression in tissues or cells exposed to known toxins, in particular hepatotoxins, as compared to unexposed tissues or cells as well as the identification of individual genes that are differentially expressed upon toxin exposure.

The invention includes methods of predicting at least one toxic effect of a compound, predicting the progression of a toxic effect of a compound, and predicting the hepatoxicity of a compound. Also provided are methods of predicting the mechanism of toxicity of a compound. In a further aspect, the invention provides probes comprising sequences that specifically hybridize to genes in Table 3 as well as solid supports comprising at least two of the said probes.

## Beschreibung

### Methods for the toxicity prediction of a compound

The present invention relates to toxicogenomic methods useful in the development of safe drugs. More specifically, the present invention relates to methods for the prediction of a toxic effect, especially hepatotoxicity, in animal models or cell cultures. The prediction of toxic effects comprises the steps of a) generating a database with the expression of marker genes elicited by known toxic compounds in animal models or cell culture systems, b) obtaining a biological sample from the model systems; c) obtaining a gene expression profile characteristic of a given toxicity mechanism and/or detecting and/or measuring the expression of (a) specific marker gene(s) d) comparing the expression profile and/or expression of specific marker gene(s) with the database of step a). Furthermore, expression profiles characteristic of different mechanisms of hepatoxicity as well as specific markers for hepatoxicity are provided.

The gene expression pattern governs cellular development and physiology, and is affected by pathological situations, including disease and the response to a toxic insult. Bearing this in mind, it becomes clear that the study of gene and protein expression in preclinical safety experiments will help toxicologists to better understand the effects of chemical exposure on mammalian physiology. On the one hand, the identification of a certain number of modulated genes and/or proteins after exposure to a toxicant will lead to the identification of novel predictive and more sensitive biomarkers which might replace the ones currently used. The knowledge regarding marker genes for particular mechanisms of toxicity, together with the rapidly growing understanding of the structure of the human genome will form the basis for the identification of new biomarkers. These markers may allow the prediction of toxic liabilities, the differentiation of species-specific responses and the identification of responder and non-responder populations. Gene expression analysis is an extremely powerful tool for the detection of new, specific and sensitive markers for given mechanisms of toxicity (Fielden, M. R., and Zacharewski, T. R. (2001). Challenges and limitations of gene expression profiling in mechanistic and predictive toxicology. *Toxicol Sci* **60**, 6-10). These markers should provide additional endpoints for inclusion into early animal studies, thus minimising the time, the cost and the number of animals needed to identify the toxic potential of a compound in development. Also, this will lead to the development of relevant screening assays *in vivo* and/or *in vitro.* The understanding of the molecular mechanisms underlying toxicity will also provide more insight into species-specific response to drugs and should immensely increase the predictability of potential risk accumulation for drug-combinations or drug-disease interactions. Moreover, chemically induced changes in gene expression are likely to occur at exposures to chemicals below those that induce an adverse toxicological outcome. As drug-induced liver toxicity is a major issue for health care and drug development, great interest lies in hepatotoxins. Currently, the predictivity of gene and protein expression for toxicity is a generally accepted assumption supported by some published results, but substantially more data are needed to prove the validity of this hypothesis (Waring, J. F., Ciurlionis, R., Jolly, R. A., Heindel, M., and Ulrich, R. G. (2001). Microarray analysis of hepatotoxins in vitro reveals a correlation between gene expression profiles and mechanisms of toxicity. *Toxicol Lett* **120**, 359-68; Bulera, S. J., Eddy, S. M., Ferguson, E., Jatkoe, T. A., Reindel, J. F., Bleavins, M. R., and De La Iglesia, F. A. (2001). RNA expression in the early characterization of hepatotoxicants in Wistar rats by high-density DNA microarrays. *Hepatology **33**,* 1239-58; Bartosiewicz M. J., Jenkins, D., Penn, S., Emery, J., and Buckpitt, A. (2001). Unique gene expression patterns in liver and kidney associated with exposure to chemical toxicants. *J Pharmacol Exp Ther* **297**, 895-905). A widespread approach to validate these new tools is the use of model compounds in animal models to produce expression profiles which are expected to be characteristic for the compound under examination. Model compounds that have been used for gene expression profiling in the liver include WY-14,643, phentobarbital, clofibrate, ethanol and acetaminophen. The majority of the published results confirm the regulation of genes previously identified and add a large number of genes modulated by the test compound. Thus microarrays showed the induction of cytochromes (CYP2B and CYP3A) as well as of genes related to apoptosis and DNA-repair by phentobarbital (Carver, M. P., and Clancy, B. (2000). Transcriptional profiling of phenobarbital (PB) hepatotoxicity in the mouse. *Toxicological Sciences* **54**, 383). Similarly, studies on the peroxisome proliferator WY-14,643 showed the induction of CYP4A, GST and acyl-CoA hydroxylase, as well as of genes associated with oxidative damage, with cell proliferation and with apoptosis (Carfagna, M. A., Baker, T. K., Wilding, L. A., Neeb, L. A., Torres, S., Ryan, T. P., and Gelbert, L. M. (2000). Effects of a peroxisome proliferator (WY-14,643) on hepatocyte transcription using microarray technology. *Toxicological Sciences* **54**, 383). Ruepp and co-workers investigated gene expression changes after treating mice with acetaminophen, and found that genes such as metallothioneins, c-fos, glutathione peroxidase and proteasome-related-genes were induced (Ruepp, S., Tonge, R. P., Wallis, N. T., Davison, M. D., Orton, T. C., and Pognan, F. (2000). Genomic and proteomic investigations of acetaminophen (APAP) toxicity in mouse liver in vivo. *Toxicological Sciences* **54**, 384). Similar results were also presented by Suter et al. (Suter, L., Boelsterli, U. A., Winter, M., Crameri, F., Gasser, R., Bedoucha, M., deVera, C., and Albertini, S. (2000). Toxicogenomics: Correlation of acetaminophen-induced hepatotoxicity with gene expression using DNA microarrays. *Toxicological Sciences* **54**, 383) and by Reilly et al. (Reilly, T. P., Bourdi, M., Brady, J. N., Pise-Masison, C. A., Radonovich, M. F., George, J. W., and Pohl, L. R. (2001). Expression profiling of acetaminophen liver toxicity in mice using microarray technology. *Biochem Biophys Res Commun* **282**, 321-8). So far, expression profiles and toxicity markers were only provided for specific model compounds in the prior art. Therefore, the technical problem underlying the present invention was to provide for gene expression profiles and toxicity markers, which are characteristic not only for a specific toxic compound, but for a specific mechanism of toxicity and which are reproducible.

The solution to this technical problem is achieved by providing methods for the prediction of toxic effects of a compound, for the prediction of the mechanism of toxicity of a compound, especially for the prediction of hepatotoxicity, by using reproducible gene expression profiles caused by known toxic compounds, gene expression profiles characteristic of a mechanism of hepatoxicity, and specific marker genes.

The present invention is based on the determination of the global changes in gene expression in tissues or cells exposed to known toxins, in particular hepatotoxins, as compared to unexposed tissues or cells as well as the identification of individual genes that are differentially expressed upon toxin exposure.

The invention includes methods of predicting at least one toxic effect of a compound, predicting the progression of a toxic effect of a compound, and predicting the hepatoxicity of a compound. Also provided are methods of predicting the mechanism of toxicity of a compound. In a further aspect, the invention provides probes comprising sequences that specifically hybridize to genes in Table 3 as well as solid supports comprising at least two of the said probes, and primers for specific amplification of the genes of Table 3.

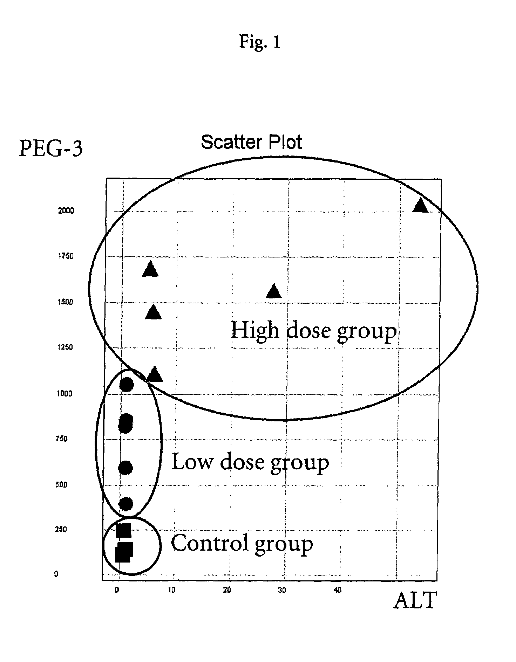

**Figure 1:** Effect of CC14 on PEG-3 (progression elevated gene-3) expression and circulating ALT (alanine aminotransferase) levels. Expression levels are expressed in arbitrary units. Circulating ALT-levels are expressed in µkat/ml. For each dose group (Control, Low dose= 0.25 ml/kg and High dose = 2 ml/kg) the values obtained for each of the 5 animals are represented.

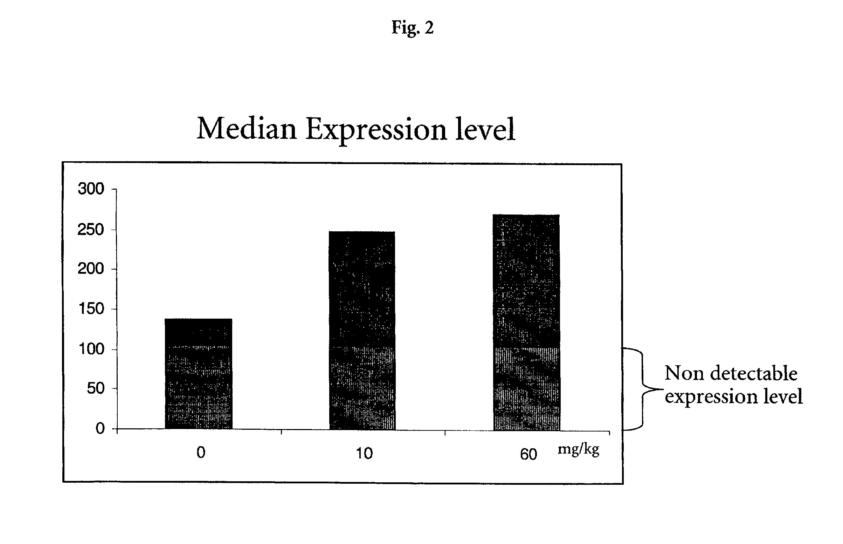

**Figure 2:** Effect of HDZ on PEG-3 expression. The median expression level of the gene PEG-3 for 10 control animals, 10 low-dose animals (10 mg/kg) and 10 high dose animals (60 mg/kg) are represented. Expression levels are expressed in arbitrary units. An expression of below 100 is considered as non-detectable.

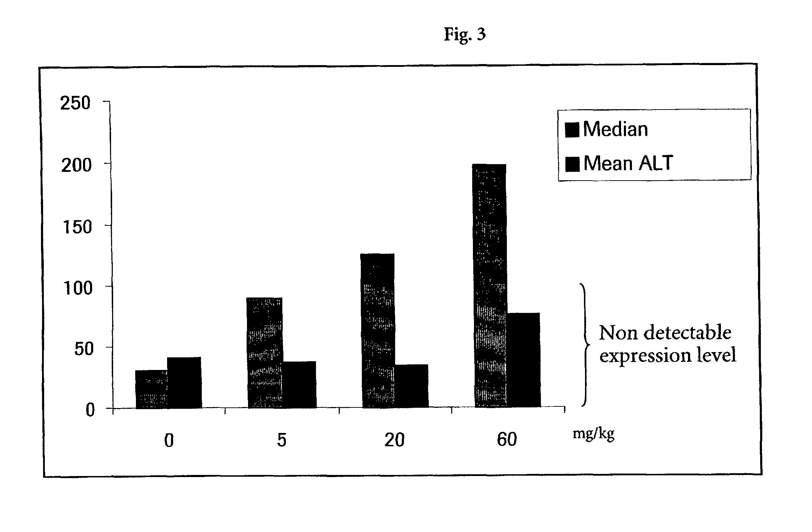

**Figure 3:** Effect of NFT on PEG-3 expression and on circulating ALT (alanine aminotransferase) levels. The median expression level of the gene PEG-3 for 10 control animals, 10 low-dose animals (5 mg/kg), 10 mid-dose animals (20 mg/kg) and 10 high dose animals (60 mg/kg) are represented. Expression levels are expressed in arbitrary units. An expression of below 100 is considered non-detectable

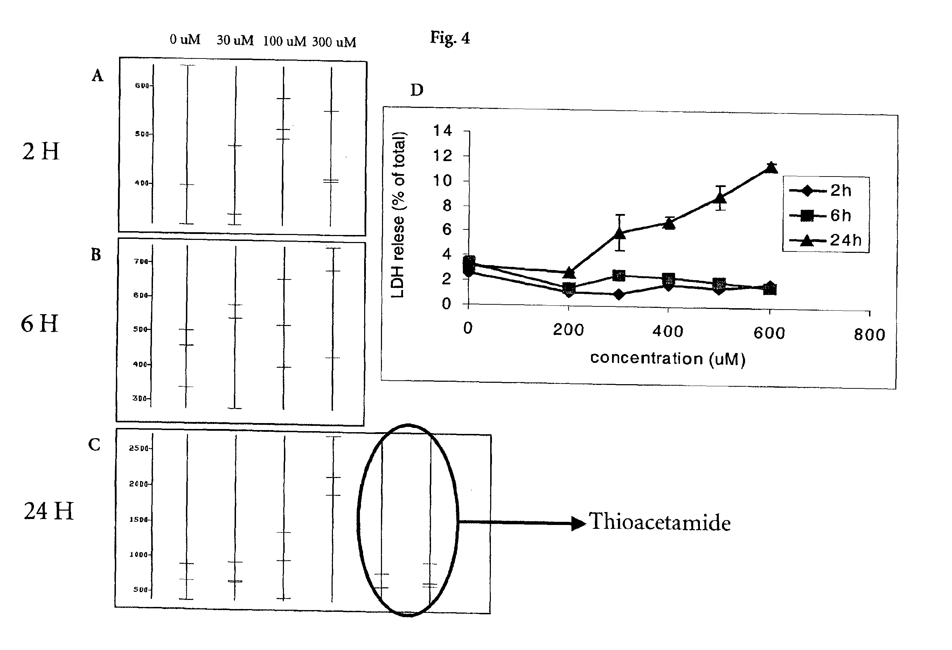

**Figure 4:** Effect of Thioacetamide and Thioacetamide-S-oxide on PEG-3 expression at 3 different time points by *in vitro* exposure of hepatocytes. Rat primary hepatocytes (in monolayer cultures) were exposed to 0, 30, 100 and 300 µM Thioacetamide-S-Oxide and to 3 and 10 mM Thioacetamide. Expression level of PEG-3 were at 2, 6 and 24 hs after exposure. For each dose and time point triplicate samples were analyzed. The expression level of PEG-3 in each experimental condition is displayed in Figure 4A through C, where each line represents the expression level for each replicate. Expression levels are expressed in arbitrary units. In addition, cytotoxicity (LDH-release) of Thioacetamide-S-oxide at several doses and time points is represented in Figure 4D.

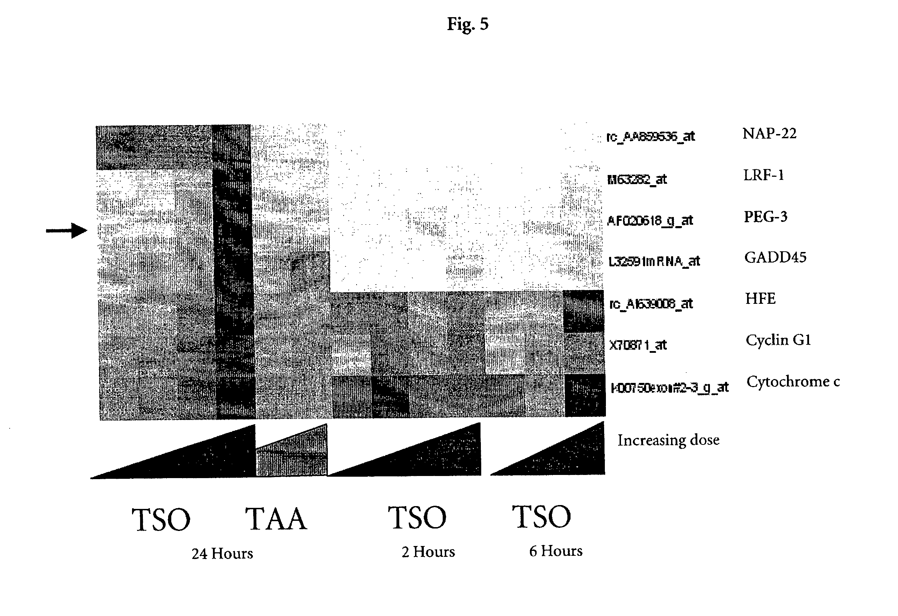

**Figure 5:** Effect of *in vitro* exposure of hepatocytes to Thioacetamide (TAA) and Thioacetamide-S-oxide (TSO) on the expression levels of PEG-3 and on some coregulated genes as determined by cluster analysis. Rat primary hepatocytes (in monolayer cultures) were exposed to 0, 30, 100 and 300 pM Thioacetamide-S-Oxide and to 3 and 10 mM Thioacetamide and analyzed at 3 different time points. Each line represents a single gene; the intensity of the grey colour is proportional to the expression level.

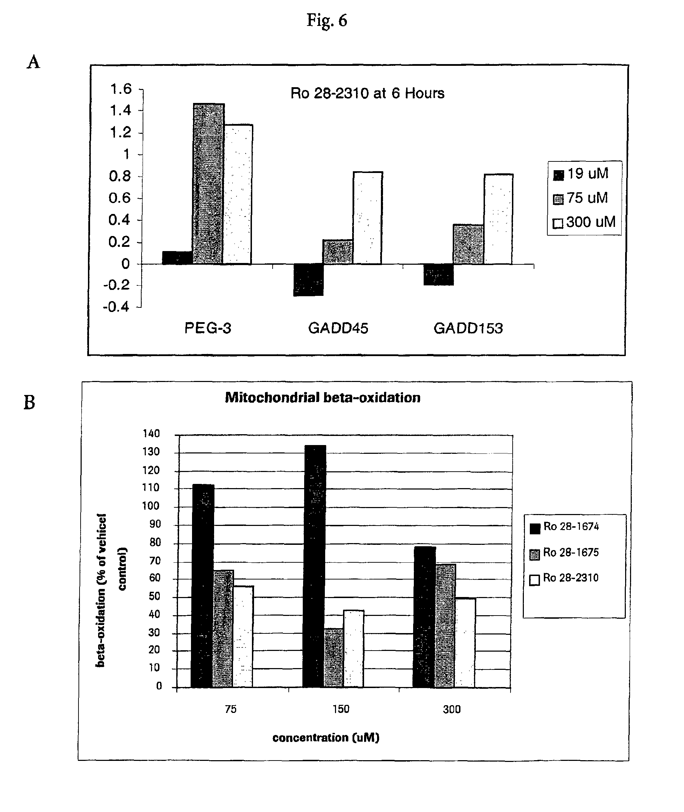

**Figure 6:** Effect of in *vitro* exposure to Glucokinase activators on the expression levels of PEG-3, GADD-45 and GADD-153 and on mitochondrial beta-oxidation. Figure 6A represents the induction of PEG-3, GADD-45 and GADD-153 with increasing doses of Ro 28-2310 at 6 hours. Figure 6B shows the inhibition of beta-oxidation by 3 glucokinase activators.

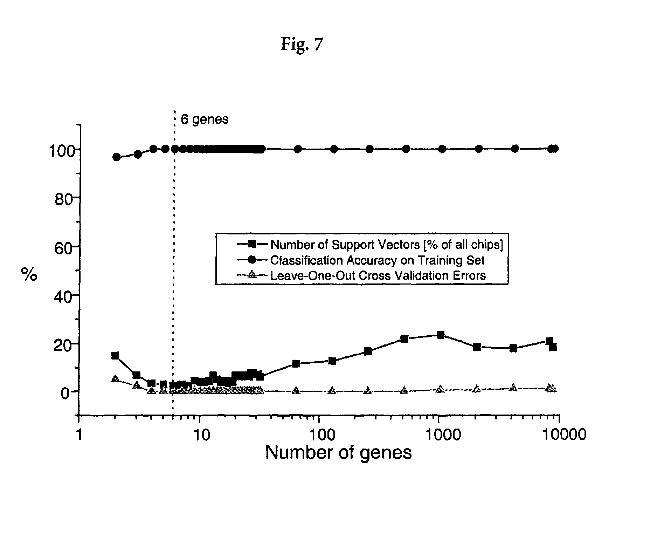

**Figure 7:** Recursive Feature Elimination (RFE) for generation of support vector machines (SVMs) for the direct acting group (see Example 9).

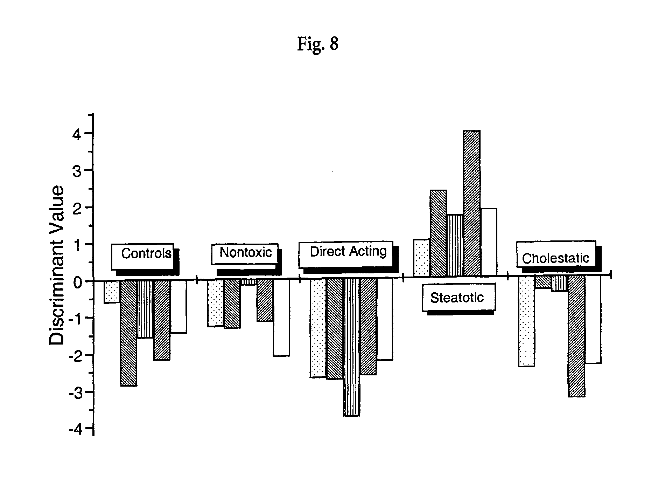

**Figure 8:** Classification of Amineptine as a steatotic compound. The bigger a positive discriminant value is, the better is the data fit into a specific class defined by the respective SVM. A negative discriminant value means that data do not fit into a compound class.

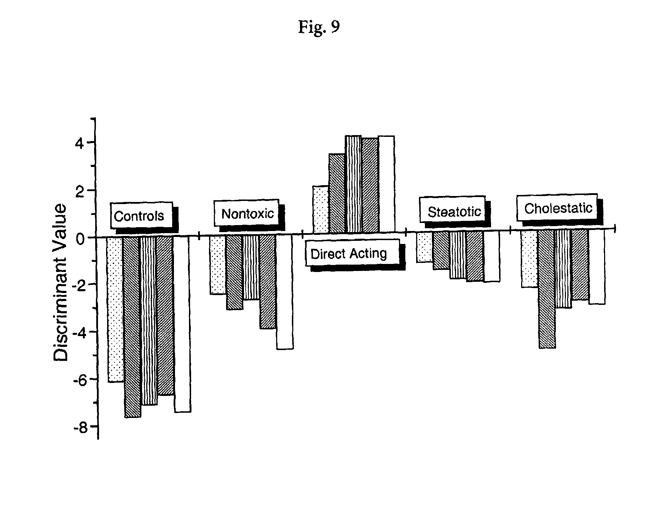

**Figure 9:** Classification of 1,2-Dichlorobenzene as a direct acting compound. The bigger a positive discriminant value is, the better is the data fit into a specific class defined by the respective SVM. A negative discriminant value means that data do not fit into a compound class.

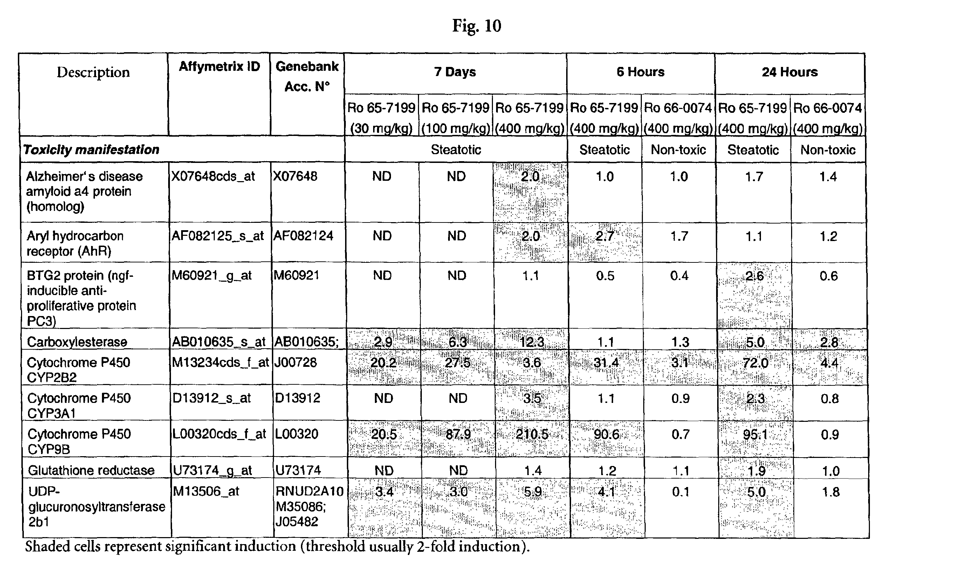

**Figure 10:** Differentiation of toxic and non-toxic compounds using RT-PCR

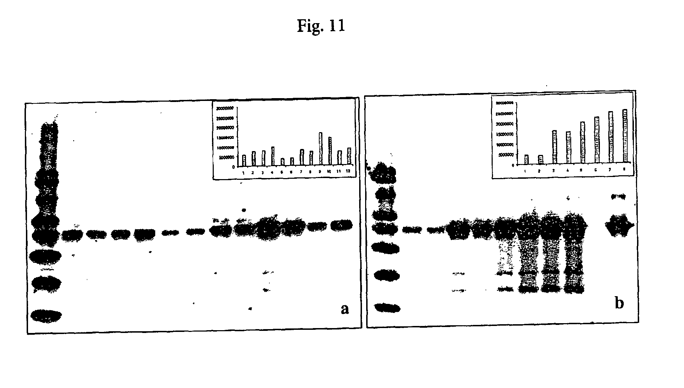

**Figure 11:** Western-Blots of liver extracts with antibody specific for CYP2B. Two representative animals from each treatment group were analyzed. **a**: lane 1: MW-markers; lanes 2 and 3: control (6 H); lanes 4 and 5: Ro 65-7199 (6 H); lanes 6 and 7: Ro 66-0074 (6 H); lanes 8 and 9: controls (24 H); lanes 10 and 11: Ro 65-7199 (24 H); lanes 12 and 13: Ro 66-0074 (24 H). **b**: lane 1: MW-markers; lanes 2 and 3: controls (7 Days); lanes 4 and 5 30 mg/kg/d Ro 65-7199 (7 Days); lanes 6 and 7: 100 mg/kg/d Ro 65-7199 (7 Days); lanes 8 and 9: 400 mg/kg/d Ro 65-7199 (7 Days); lane 11: Positive control (phenobarbital induced rat microsomal extract). Inlet bargraphs represent the densitometric quantification of each gel.

In the present invention it was found that marker genes are differentially expressed in tissues obtained after exposure of non-human animals, e. g. rats, to model toxic compounds at doses and/or time points in which these compounds did not elicit the conventionally measured response of elevation in plasma liver enzymes. It was also observed that this elevation of particular marker genes was evident not only after *in vivo* exposure of the test animals but also *in vitro* in primary hepatic cell cultures exposed to a similar group of hepatotoxins. Furthermore, the regulation of a group of genes by several compounds with a similar mechanism of toxicity provides a characteristic gene expression profile or "fingerprint" for said mechanism of toxicity.

In the present study, compounds with well characterized toxicity as Chlorpromazine, Cydosporine A, Erythromycin, Glibenclamide, Lithocholic acid, Ro 48-5695 (Endothelin receptor antagonist), Dexamethasone, 1,2-Dichlorobenzene, Aflatoxin B1, Bromobenzene, Carbon tetrachloride, Diclofenac, Hydrazine, Nitrofurantoin, Thioacetamide, Concanavaline A, Tacrine, Tempium (Lazabemide), Tolcapone (Tasmar), 1,4-Dichlorobenzene, Amineptin, Amiodarone, Doxycycline, Ro 28-1674 (glucokinase activator), Ro 28-1675 (glucokinase activator), Ro 65-7199 (5-HT6 receptor antagonist), Tetracycline, Dinitrophenol, Cyproterone Acetate, Phenobarbital, Clofibrate, Acetaminophen, Thioacetamide-S-Oxide, Perhexiline, Methapyrilene were selected as known hepatotoxins. These compounds are widely known to cause hepatic injury in animals and/or in man, as described in "Toxicology of the liver, 2^{nd}. Ed, Ed. By G.L. Plaa and W.R. Hewitt, Target organ toxicology series, 1997. A brief summary of the known effects of these compounds is listed below.

**Carbon tetrachloride** (CC14), **bromobenzene** and **1,2-dichlorobenzene** are halogenated, highly reactive compounds leading to toxicity in the liver in rodents and in man (Brondeau MT, Bonnet P, Guenier JP, De Ceaurriz J. (1983). Short-term inhalation test for evaluating industrial hepatotoxicants in rats. *Toxicol Lett.* **19**, 139-46; Rikans, L. E. (1989). Influence of aging on chemically induced hepatotoxicity: role of age- related changes in metabolism. *Drug Metab Rev* **20**, 87-110). For CC14, several studies have shown that the toxicity is mediated by its metabolic product, the highly reactive trichloromethyl free radical (Stoyanovsky, D. A., and Cederbaum, A. I. (1999). Metabolism of carbon tetrachloride to trichloromethyl radical: An ESR and HPLC-EC study. *Chem Res Toxicol* **12,** 730-6). This radical leads to lipid peroxidation and can react with cellular proteins and with DNA (Castro, G. D., Diaz Gomez, M. I., and Castro, J. A. (1997). DNA bases attack by reactive metabolites produced during carbon tetrachloride biotransformation and promotion of liver microsomal lipid peroxidation. *Res Commun Mol Pathol pharmacol* **95**, 253-8). Secondary liver injury following the administration of these halogenated compounds is believed to be caused by inflammatory processes originating from products of activated Kupffer cells (Edwards, M. J., Keller, B. J., Kauffman, F. C., and Thurman, R. G. (1993). The involvement of Kupffer cells in carbon tetrachloride toxicity. *Toxicol Appl Pharmacol* **119**, 275-9). Thus, the observed toxicity is due to direct action of the free radicals and to indirect action mediated by cytokines such as TNF alpha (DeCicco, L. A., Rikans, L. E., Tutor, C. G., and Hornbrook, K. R. (1998). Typical lesions produced by these compounds a few hours after a single administration are centrilobular cell degeneration and necrosis accompanied by lipid peroxidation, followed by hepatic regeneration starting 48 hours after administration. Elevation of serum enzyme activities is seen as a result of of the hepatocellular necrosis (e.g. AST, ALT, SDH).

**Hydrazine** and hydrazine derivatives are among the early drugs reported to cause damage to the liver. **Thioacetamide** and its metabolite **Thioacetamide-S-oxide** are also known to cause liver injury, histopathological examination showed necrotic hepatocytes around the central vein with infiltration of macrophages, neutrophils and eosinophils. Thus biochemical and histologic and clinical features indicate hepatocellular injury, with parenchimal degeneration and necrosis (Dashti, Jeppsson, Hagerstrand, Hultberg, Srinivas, Abdulla, Joelsson and Bengmark (1987). Early biochemical and histological changes in rats exposed to a single injection of thioacetamide. *Pharmacol Toxicol* 3,171-4.; Albano, Goria-Gatti, Clot, Jannone and Tomasi (1993). Possible role of free radical intermediates in hepatotoxicity of hydrazine derivatives. *Toxicol Ind Health* 3, 529-38.).

**Cyclosporine A** (CsA) is an immunosupressant that has been reported to induce cholestasis in transplanted patients. Several mechanisms have been proposed to explain this toxic manifestation: hepatotoxicity, competition for bilirary excretion, inhibition of bilirubin excretion, inhibition of the synthesis of bile acids, etc. (Le Thai, Dumont, Michel, Erlinger and Houssin (1988). Cholestatic effect of cyclosporine in the rat. An inhibition of bile acid secretion. *Transplantation* 4, 510-2.). In spite of the liver being one of the main target organs for CsA-induced toxicity, the kidney is also a target organ for toxicity. Nevertheless, nephrotoxicity seems to be the consequence of chronic exposure to the drug. Using animal studies (rats), it has been shown that the bile flow is significantly reduced after chronic (3 weeks) or acute (single dose) administration of CsA. This decrease in BA-flow is reflected by an increase in plasma bile acids and plasma bilirubin. No histopathological findings accompany this effect (Stone, Warty, Dindzans and Van Thiel (1988). The mechanism of cyclosporine-induced cholestasis in the rat. *Transplant Proc* 3 Suppl 3, 841-4, Roman, Monte, Esteller and Jimenez (1989). Cholestasis in the rat by means of intravenous administration of cyclosporine vehicle, Cremophor EL. *Transplantation 4,* 554-8).

**Tacrine** is a compound for the treatment of Alzheimer's disease in man. In treated patients, it shows hepatotoxicity with an incidence of 40-50%. In rodents, tacrine elicited hepatic toxicity manifested as pericentral necrosis and fatty changes, accompanied by an increase in circulating liver enzymes (Monteith and Theiss (1996). Comparison of tacrine-induced cytotoxicity in primary cultures of rat, mouse, monkey, dog, rabbit, and human hepatocytes. *Drug Chem Toxicol* 1-2, 59-70; Stachlewitz, Arteel, Raleigh, Connor, Mason and Thurman (1997). Development and characterization of a new model of tacrine-induced hepatotoxicity: role of the sympathetic nervous system and hypoxiareoxygenation. *J Pharmacol Exp Ther* 3,1591-9).

**Concanavaline A** is a model compound used for studying the role of liver-associated T cells in acute hepatitis produced in rats. Concanavalin A produces a severe hepatitis, which can be assessed by serum biochemistry showing increased interleukines (IL-6 and TNF-alpha), as well as alanine aminotransferase (ALT) (Mizuhara, O'Neill, Seki, Ogawa, Kusunoki, Otsuka, Satoh, Niwa, Senoh and Fujiwara (1994). T cell activation-associated hepatic injury: mediation by tumor necrosis factors and protection by interleukin *6. J Exp Med 5,* 1529-37).

**Chlorpromazine** has a dear and well-studied profile of producing liver injury in man. It is the most extensively studied neuroleptic and the hepytic injury that produces is hepatocanalicular cholestasis. Up to 1% of the treated patients develop jaundice. Some studies have shown that chlorpromazine inhibits Na+-K+-ATPase cation pumping in intact cells, therefore contributing to the chlorpromazine-induced cholestasis in animals and humans. (Van Dyke and Scharschmidt (1987). Effects of chlorpromazine on Na+-K+-ATPase pumping and solute transport in rat hepatocytes. *Am J Physiol* 5 Pt 1, G613-21.). **Lithocholic acid** is one of the bile acids transported into the bile canaliculi. An increase in the concentration of lithocholic acid causes intrahepatic cholestasis (Shefer, Zaki and Salen (1983). Early morphologic and enzymatic changes in livers of rats treated with chenodeoxycholic and ursodeoxycholic acids. *Hepatology* 2, 201-8).

**Erythromycine** have been incriminated as the cause of cholestatic liver injury. The pattern of injury is usually hepatocanalicular cholestasis. In rare casis, erythromycin can also lead to liver necrosis.(Gaeta, Utili, Adinolfi, Abernathy and Giusti (1985). Characterization of the effects of erythromycin estolate and erythromycin base on the excretory function of the isolated rat liver. *Toxicol Appl Pharmacol* 2, 185-92.). **Glibenclamide** has been associated with reversible cholestasis in clinical case studies (Del-Val, Garrigues, Ponce and Benages (1991). Glibenclamide-induced cholestasis. *J Hepatol* 3, 375).

**Dinitrophenol** is a widely used model compound for mitochondrial uncoupling. Dosing of animals with this compound leads to increased mitochondrial respiration, decreased ATP-levels and increase in body temperature (Okuda, Lee, Kumar and Chance (1992). Comparison of the effect of a mitochondrial uncoupler, 2,4- dinitrophenol and adrenaline on oxygen radical production in the isolated perfused rat liver. *Acta Physiol Scand* 2, 159-68).

**Dexamethasone** is a known glucocorticoid which is used in many experimental models to induce the activity of cytochromes P450 in the liver and in hepatocyte cultures (Kocarek and Reddy (1998). Negative regulation by dexamethasone of fluvastatin-inducible CYP2B expression in primary cultures of rat hepatocytes: role of CYP3A. *Biochem pharmacol* 9, 1435-43). Other drugs that are usually related with hepatomegaly and/or peroxisome proliferation and are known inducers of some cytochromes P450 in the liver and in hepatocyte cell cultures are **phenobarbital, cyproterone acetate** and fibrates such as **clofibrate** (Menegazzi, Carcereri-De Prati, Suzuki, Shinozuka, Pibiri, Piga, Columbano and Ledda-Columbano (1997). Liver cell proliferation induced by nafenopin and cyproterone acetate is not associated with increases in activation of transcription factors NF-kappaB and AP-1 or with expression of tumor necrosis factor alpha. *Hepatology* 3, 585-92.; Kietzmann, Hirsch-Ernst, Kahl and Jungermann (1999). Mimicry in primary rat hepatocyte cultures of the in vivo perivenous induction by phenobarbital of cytochrome P-450 2B1 mRNA: role of epidermal growth factor and perivenous oxygen tension. *Mol Pharmacol* 1, 46-53; Diez-Fernandez, Sanz, Alvarez, Wolf and Cascales (1998). The effect of non-genotoxic carcinogens, phenobarbital and clofibrate, on the relationship between reactive oxygen species, antioxidant enzyme expression and apoptosis. *Carcinogenesis* 10, 1715-22).

**Acetaminophen** is a widely used analgesic and antipyretic drug that causes acute liver damage upon overdosis. This drug is often missused for suicidal purposses. If overdosed, the hepatic glutathione pool becomes depleted and the metabolic activation of the compound leads to a highly reactive metabolite. This metabolite can bind to DNA and proteins in the cell, leading to hepatocellular necrosis (Tarloff, Khairallah, Cohen and Goldstein (1996). Sex- and age-dependent acetaminophen hepato- and nephrotoxicity in Sprague-Dawley rats: role of tissue accumulation, nonprotein sulfhydryl depletion, and covalent binding. *Fundam Appl Toxicol* 1, 13-22; Cohen and Khairallah (1997). Selective protein arylation and acetaminophen-induced hepatotoxicity. *Drug Metab Rev* 1-2, 59-77; Fountoulakis, Berndt, Boelsterli, Crameri, Winter, Albertini and Suter (2000). Two-dimensional database of mouse liver proteins: changes in hepatic protein levels following treatment with acetaminophen or its nontoxic regioisomer 3-acetamidophenol. *Electrophoresis* 11, 2148-61).

**Methapyrilene** is an antihistamin drug that causes acute periportal hepatotoxicity in rats, but also exerts a variety of toxic effects in the liver. Apparently, CYP2C11 is responsible for the suicide substrate bioactivation of methapyrilene and the acute toxicologic outcome largely relied upon an abundance of detoxifying enzymes present in the liver Another potentially very significant effect of MP is that it induces a large increase in hepatic cell proliferation coupled with mitochondrial proliferation. In addition, some results suggest that methapyrilene hydrochloride is a DNA damaging agent (Althaus, Lawrence, Sattler and Pitot (1982). DNA damage induced by the antihistaminic drug methapyrilene hydrochloride. *Mutat Res* 3-6, 213-8; Ratra, Cottrell and Powell (1998). Effects of induction and inhibition of cytochromes P450 on the hepatotoxicity of methapyrilene. *Toxicol Sci* 1, 185-96).

**Tetracyclines** and **Doxycyclines** lead to dose-dependent hepatic injury. The hepatotoxicity of tetracyclines is well known. The characteristic lesion is microvesicular steatosis which poor prognosis, resembling Reye's syndrome. The underlying mechanism of toxicity seems to be the inhibition of mitochondrial beta oxidation together with an inhibition of the transport of lipids from the liver (Hopf, Bocker and Estler (1985). Comparative effects of tetracycline and doxycycline on liver function of young adult and old mice. *Arch Int Pharmacodyn Ther 1,* 157-68; Lienart, Morissens, Jacobs and Ducobu (1992). Doxycycline and hepatotoxicity. *Acta Clin Belg* 3, 205-8).

**Diclofenac** is a widely used NSAID (non-steroid anti-inflammatory drug). Several cases related hepatic injury, sometimes with fatal outcome, with the administration of this compound. The The pattern of injury is usually hepatocellular with acute necrosis. The mechanism by which diclofenac elicits this effect is unknown, but some speculations have been made regarding metabolic idiosyncracy. Also, diclofenac can bind irreversibly to hepatic proteins via its acyl glucuronide metabolite; these protein adducts could be involved in the pathogenesis of didofenac-associated liver damage (Kretz-Rommel and Boelsterli (1994). Mechanism of covalent adduct formation of diclofenac to rat hepatic microsomal proteins. Retention of the glucuronic acid moiety in the adduct. *Drug Metab Dispos* 6, 956-61).

**Nitrofurantoin** is an antimicrobial widely used in the treatment of urinary tract infection which is known to cause acute and chronic liver injury. The injury can be either cholestatic or hepatocellular, and the underlying mechanism seems to be immunologic idiosyncrasy (Villa, Carugo and Guaitani (1992). No evidence of intracellular oxidative stress during ischemia- reperfusion damage in rat liver in vivo. *Toxicol Lett* 2-3, 283-90; Tacchini, Fusar-Poli and Bernelli-Zazzera (2002). Activation of transcription factors by drugs inducing oxidative stress in rat liver. *Biochem Pharmacol* 2, 139-148).

**Aflatoxin B1** is a contaminant in food, source: Aspergillus flavus and Aspergillus parasiticus. Aflatoxin induces also ROS production, lipid peroxidation and 8-OhdG formation in DNA. It reacts also with various liver and blood plasma proteins, particularly with serum albumin. Acutelly, it leads to liver necrosis, given chronically shows a carcinogenic effect (Liu, Yang, Lee, Shen, Ang and Ong (1999). Effect of Salvia miltiorrhiza on aflatoxin B1-induce oxidative stress in cultured rat hepatocytes. *Free Radic Res* 6, 559-68; Barton, Hill, Yee, Barton, Ganey and Roth (2000). Bacterial lipopolysaccharide exposure augments aflatoxin B(1)-induced liver injury. *Toxicol Sci* 2, 444-52).

**Amineptine, amiodarone** and **perhexiline** are drugs known to cause microvesicular steatosis through the inhibition of mitochondrial beta oxidation (Le Dinh, Freneaux, Labbe, Letteron, Degott, Geneve, Berson, Larrey and Pessayre (1988). Amineptine, a tricyclic antidepressant, inhibits the mitochondrial oxidation of fatty acids and produces microvesicular steatosis of the liver in mice. *J Pharmacol Exp Ther* 2, 745-50; Bach, Schultz, Cohen, Squire, Gordon, Thung and Schaffner (1989). Amiodarone hepatotoxicity: progression from steatosis to cirrhosis. *Mt Sinai J Med* 4, 293-6; Deschamps, DeBeco, Fisch, Fromenty, Guillouzo and Pessayre (1994). Inhibition by perhexiline of oxidative phosphorylation and the beta- oxidation of fatty acids: possible role in pseudoalcoholic liver lesions. *Hepatology* 4, 948-61; Fromenty and Pessayre (1997). Impaired mitochondrial function in microvesicular steatosis. Effects of drugs, ethanol, hormones and cytokines. *J Hepatol* Suppl 2, 43-53).

In the present invention it was found that the modulation of gene expression by several compounds that show a similar hepatotoxicity defines a characteristic profile which is expected to be similar for further compounds that elicit the same type of toxicity. Thus, these profiles can be used for the prediction of the toxic potential of unknown compounds. Said characteristic profiles (or "fingerprints) for classes of hepatotoxins are defined in Table 3.

Accordingly, the present invention relates to a method of predicting at least one toxic effect of a compound, comprising detecting the level of expression of one or more genes from Table 3 in a tissue or cell sample exposed to the compound, wherein differential expression of the genes in Table 3 is indicative of at least one toxic effect.

The present invention moreover provides a method of predicting at least one toxic effect of a compound, comprising:
(a) detecting the level of expression of one or more genes from Table 3 in a tissue or cell sample exposed to the compound;
(b) comparing the level of expression of the genes to their level of expression in a control tissue or cell sample, wherein differential expression of the genes in Table 3 is indicative of at least one toxic effect.

In a further embodiment, the present invention relates to a method of predicting the progression of a toxic effect of a compound, comprising detecting the level of expression in a tissue or cell sample exposed to the compound of one or more genes from Table 3, wherein differential expression of the genes in Table 3 is indicative of toxicity progression.

As defined in the present invention, a toxic effect includes any adverse effect on the physiological status of a cell or an organism. The effect includes changes at the molecular or cellular level. A preferred toxic effect is hepatotoxicity, which includes pathologies comprising among others liver necrosis, hepatitis, fatty liver and cholestasis.

The progression of a toxic effect is defined as the histological, functional or physiological manifestation with time of a toxic injury that can be detected by measuring the gene expression levels found after initial exposure of an animal or cell to a drug, drug candidate, toxin, pollutant etc.

In general, a method to predict a toxic effect of a compound or a composition of compounds comprises the steps of exposing a model animal or a cell culture to the compound or composition of compounds, detecting or measuring the differential expression (mRNA, protein-content, etc) of one or more genes from Table 3 in a biological sample of said model animal or said cell culture compared to a control, and comparing the determined differential expression to the differential expression disclosed in Table 3.

In the context of the present invention, the term "expression level" comprises, inter alia, the gene expression levels defined as RNA-levels, i.e. the amount or quality of RNA, mRNA, and the corresponding cDNA-levels; and the protein expression levels.

The term "differential gene expression" in accordance with this invention relates to the up- or down-regualtion of genes in tissues or cells derived from treated animals/cell cultures in comparison to control animals/cell cultures. These genes, which are differentially expressed, are also refered to as marker genes. Furthermore, it is envisaged that said comparison is carried out in a computer-assisted fashion. Said comparison may also comprise the analysis in high-throughput screens.

Most preferably, an increase or decrease of the expression level in (a) marker gene(s) as listed in Table 3 and as detected by the inventive method is indicative of hepatotoxic liability. It is also preferred that in addition to the said marker genes, the gene expression profile as depicted in Table 3 will also be analyzed in order to categorize hepatotoxic liability of the test compound(s).

It is also envisaged that the method of the invention comprises the comparison of differentially expressed marker genes, i.e. marker genes which are up or downregulated in tissues, cells, body fluids etc, from biological samples after exposure to model compounds (as exemplified in Tables 1 and 2), with markers which are not changed, i.e. which are not diagnostic for hepatotoxicity. Such unchanged marker genes comprise, inter alia, the ribosomal RNA control as employed in the appended examples, as well as house-keeping genes (N° 10 as depicted in Table 4).

The detection and/or measurement of the expression levels of the genes from Table 3 according to the methods of the present invention may comprise the detection of an increase, decrease and/or the absence of a specific nucleic acid molecule, for example mRNA or cDNA.

Methods for the detection/measurement of mRNA and or cDNA levels are well known in the art and comprise methods as described in the appended examples, but are not limited to microarray- and PCR-technology.

In addition, protein expression levels from marker genes as listed in Table 4 and of some genes in Table 3 can also be assessed. Methods for the detection/measurement of protein levels are well known in the art and include, but are not limited to Western-blot, two-dimensional electrophoresis, ELISA, RIA, immunohistochemistry, etc.

Additional assay formats may be used to monitor the induced change of the expression level of a gene identified in Table 3. For instance, mRNA expression may be monitored directly by hybridization of probes to the nucleic acids of the invention. Cell lines are exposed to the agent to be tested under appropriate conditions and time and total RNA or mRNA is isolated by standard procedures such as those disclosed in Sambrook *et al* (Molecular Cloning: A Laboratory 30 Manual, 2nd Ed. Cold Spring Harbor Laboratory Press, 1989).

Any assay format to detect gene expression may be used. For example, traditional Northern blotting, dot or slot blot, nuclease protection, primer directed amplification, RT-PCR, semi- or quantitative PCR, branched-chain DNA and differential display methods may be used for detecting gene expression levels. Those methods are useful for some embodiments of the invention. In cases where smaller numbers of genes are detected, amplification-based assays may be most efficient. Methods and assays of the invention, however, may be most efficiently designed with hybridization-based methods for detecting the expression of a large number of genes. Any hybridization assay format may be used, including solution-based and solid support-based assay formats.

In another assay format, cell lines that contain reporter gene fusions between the open reading frame and/or the transcriptional regulatory regions of a gene in Table 3 and any assayable fusion partner may be prepared. Numerous assayable fusion partners are known and readily available including the firefly luciferase gene and the gene encoding chloramphenicol acetyltransferase (Alam *et al.* (1990) *Anal. Biochem.* **188**, *245-254).* Cell lines containing the reporter gene fusions are then exposed to the compound to be tested under appropriate conditions and time. Differential expression of the reporter gene between samples exposed to the compound and control samples identifies compounds which modulate the expression of the nucleic acid.

Preferably in the method of the present invention, the expression of at least one gene as listed in Table 3 is detected/measured. Yet, it is also envisaged that the expression of at least two, at least three, at least five, at least ten, at least twenty, at least thirty, at least forty, at least fifty, at least one hundred genes as listed in Table 3 are detected/measured. Moreover, it is envisaged that the expression of nearly all genes from Table 3 or of all genes from Table 3 is detected. It is furthermore envisaged that specific patterns of differentially expressed marker genes as depicted in Table 3 are detected, measured and/or compared.

The above mentioned animal model to be employed in the methods of the present invention and comprising and/or expressing a maker gene as defined herein is a non-human animal, preferably a mammal, most preferably mice, rats, sheep, calves, dogs, monkeys or apes. Most preferred are rodent models such as rats and mice. The animal model also comprises non-human transgenic animals, which preferably express at least one toxicity marker gene as disclosed in Table 3.

Yet it is also envisaged that non-human transgenic animals be produced which do not express marker genes as disclosed in Table 3 or which over-express said marker genes.

Transgenic non-human animals comprising and/or expressing the up-regulated marker genes of the present invention or, in contrast which comprise silenced or less efficient versions of down-regulated marker genes for hepatotoxicity, as well as cells derived thereof, are useful models for studying hepatotoxicity mechanisms.

Accordingly, said transgenic animal model may be transfected or transformed with the vector comprising a nucleic acid molecule coding for a marker gene as disclosed in Table 3. Said animal model may therefore be genetically modified with a nucleic acid molecule encoding such a marker gene or with a vector comprising such a nucleic acid molecule. The term "genetically modified" means that the animal model comprises in addition to its natural genome a nucleic acid molecule or vector as defined herein and coding for a toxicity marker of Table 3 or at least a fragment thereof. Said additional genetic material may be introduced into the animal model or into one of its predecessors/parents. The nucleic acid molecule or vector may be present in the genetically modified animal model or cell either as an independent molecule outside the genome, preferably as a molecule which is capable of replication, or it may be stably integrated into the genome of the animal model or cell thereof.

As mentioned herein above, the method of the present invention may also employ a cell culture. Preferred are cultures of primary animal cells or cell lines. Suitable animal cells are, for instance, primary mammalian hepatocytes; insect cells, vertebrate cells, preferably mammalian cell lines, such as e.g. CHO, HeLa, NIH3T3 or MOLT-4. Further suitable cell lines known in the art are obtainable from cell line depositories, like the American Type Culture Collection (ATCC). Most preferred are primary hepatocyte cultures or hepatic cell lines comprising rodent or human primary hepatocyte cultures including monolayer, sandwich cultures and slices cultures; as well as rodent cell lines such as BRL3, NRL clone9, and human cell lines such as HepG2 cells.

Cells or cell lines used in the method of the present invention may be transfected or transformed with a vector comprising a nucleic acid molecule coding for a marker gene as disclosed in Table 3. Said cell or cell line may therefore be genetically modified with a nucleic acid molecule encoding such a marker gene or with a vector comprising such a nucleic acid molecule. The term "genetically modified" means that the cell comprises in addition to its natural genome a nucleic acid molecule or vector as defined herein and coding for a toxicity marker of Table 3 or at least a fragment thereof. The nucleic acid molecule or vector may be present in the genetically modified cell either as an independent molecule outside the genome, preferably as a molecule which is capable of replication, or it may be stably integrated into the genome of the cell.

In accordance with the present invention, the term "biological sample" or "sample" as employed herein means a sample which comprises material wherein said differential expression of marker genes may be measured and may be obtained. "Samples" may be tissue samples derived from tissues of non-human animals, as well as cell samples, derived from cells of non-human animals or from cell cultures. For animal experimentation, biological samples comprise target organ tissues obtained after necropsy or biopsy and body fluids, such as blood or urine. For possible clinical use of the markers, particular preferred samples comprise body fluids, like blood, sera, plasma, urine, synovial fluid, spinal fluid, cerebrospinal fluid, semen or lymph, as well as body tissues obtained by biopsy. Particularly documented in the appended examples are rat liver tissues and primary hepatocyte cultures. Peripheral blood samples were also obtained to analyze circulating liver enzymes.

The cell population that is exposed to the compound or composition may be exposed *in vitro* or *in vivo.* For instance, cultured or freshly isolated hepatocytes, in particular rat hepatocytes, may be exposed to the compound under standard laboratory and cell culture conditions. In another assay format, *in vivo* exposure may be accomplished by administration of the compound to a living animal, for instance a laboratory rat. Procedures for designing and conducting toxicity tests in *in vitro* and *in vivo* systems are well known, and are described in many texts on the subject, such as Loomis et al. (Loomis's Esstentials of Toxicology, 4th Ed. Academic Press, New York, 1996; Echobichon, The Basics of Toxicity Testing, CRC Press, Boca Raton, 1992; Frazier, editor, In *Vitro* Toxicity Testing, Marcel Dekker, New York, 1992) and the like. In *in vitro* toxicity testing, two groups of test organisms are usually employed: One group serves as a control and the other group receives the test compound in a single dose (for acute toxicity tests) or a regimen of doses (for prolonged or chronic toxicity tests). Since in some cases, the extraction of tissue as called for in the methods of the invention requires sacrificing the test animal, both the control group and the group receiving the compound must be large enough to permit removal of animals for sampling tissues, if it is desired to observe the dynamics of gene expression through the duration of an experiment. In setting up a toxicity study, extensive guidance is provided in the literature for selecting the appropriate test organism for the compound being tested, route of administration, dose ranges, and the like. Water or physiological saline (0.9% NaCl in water) is the solute of choice for the test compound since these solvents permit administration by a variety of routes. When this is not possible because of solubility limitations, vegetable oils such as corn oil or organic solvents such as propylene glycol may be used.

A method of predicting the mechanism of toxicity of a compound comprising detecting the level of expression in a tissue or cell sample exposed to the compound of one or more genes from Table 3 is also provided, wherein differential expression of the genes in Table 3 is associated with a specific mechanism of toxicity.

By "mechanism of toxicity" it is meant the measurable manifestation of the toxic event, regarding target organ, time of onset, underlying molecular mechanism (i.e. DNA-damage, formation of protein adduct, etc) histopathological and biochemical findings such as circulating liver enzymes. Gene expression profiles can also be characteristic of a toxicity mechanism.

Different mechanisms of toxicity are known for hepatotoxins. Direct acting compounds are those compounds that cause damage to macromolecules, in particular proteins and lipids by directly interacting with them. This interaction could occur through the test compound itself or, more commonly, through a highly reactive metabolite thereof. Histological manifestations of these class of hepatoxicity include hepatocellular necrosis, lipid peroxidation and elevation of circulating levels of enzymes of hepatic origin such as ALT (alanine aminotransferase). Inflammation can also be observed due to the activation of the hepatic Kupffer cells. **Steatotic** compounds are those that cause an accummulation of fat in the liver. There are two types of steatosis: macrovesicular steatosis and microvesicular steatosis. All the test compounds used in this invention belong to the latter type. Characteristic of microvesicular steatosis is the accumulation of small lipid vesicles in the hepatocytes (so-called fatty liver), which usually lead to accute liver failure. The underlying molecular mechanisms are thought to be an inhibition of mitochondrial beta oxidation (due to mitochondrial damage) and/or an inhibition of the export of fatty acids from the hepatocyte. Compounds leading to **cholestasis** impair the bile flow, causing the clinical manifestation of jaundice. Intrahepatic cholestasis involves usually the inhibition of the bile acid transporters in the hepatocytes, leading to an accummulation of bile acids. Increased bile acids are responsible for slight hepatocyte injury, little inflammation and the elevation of circulating alkaline phosphatase (G.L. Plaa and W.R. Hewitt Ed. "Toxicology of the liver, 2^{nd} Ed., Target organ toxicology series, 1997; Fromenty and Pessayre (1995). Inhibition of mitochondrial beta-oxidation as a mechanism of hepatotoxicity. *Pharmacol Ther* 1, 101-54; Jaeschke, Gores, Cederbaum, Hinson, Pessayre and Lemasters (2002). Mechanisms of hepatotoxicity. *Toxicol Sci* 2, 166-76).

Detection of toxic potential as identified and/or obtained by the methods of the present invention are particularly useful in the development of new drugs in terms of safety.

Moreover, a method of predicting at least one toxic effect of a compound, comprising detecting the level of expression of progression elevated gene 3 (PEG-3) or Translocon associated protein (TRAP) from Table 4 in a tissue or cell sample exposed to the compound is provided, wherein differential expression of PEG-3 and TRAP is indicative of at least one toxic effect. The preferred toxic effect of the compound in the present method is hepatotoxicity.

PEG-3 belongs to the family of GADD-45 and GADD-153, which are genes up-regulated upon DNA-damage. While GADD-genes are known stress-inducible markers that lead to a cell cycle arrest (Seth A, Giunta S, Franceschil C, Kola I, Venanzoni MC (1999). Regulation of the human stress response gene GADD153 expression: role of ETS1 and FLI-1 gene products. *Cell Death Differ* **6**(9), 902-7; Tchounwou PB, Wilson BA, Ishaque AB, Schneider J. Atrazine potentiation of arsenic trioxide-induced cytotoxicity and gene expression in human liver carcinoma cells (HepG2). *Mol Cell Biochem. 222,* 49-59; Tchounwou PB, Ishaque AB, Schneider J (2001). Cytotoxicity and transcriptional activation of stress genes in human liver carcinoma cells (HepG2) exposed to cadmium chloride. *Mol Cell Biochem.* **222,** 21-8; Tchounwou PB, Wilson BA, Ishaque AB, Schneider J (2001). Transcriptional activation of stress genes and cytotoxicity in human liver carcinoma cells (HepG2) exposed to 2,4,6-trinitrotoluene, 2,4-dinitrotoluene, and 2,6-dinitrotoluene. *Environ Toxicol.* **16**, 209-16.; Zhan Q, Fan S, Smith ML, Bae I, Yu K, Alamo I Jr, O'Connor PM, Fornace AJ Jr (1996). Abrogation of p53 function affects gadd gene responses to DNA base-damaging agents and starvation. *DNA Cell Biol* **15,** 805-15), PEG-3 is involved in progression (Park JS, Qiao L, Su ZZ, Hinman D, Willoughby K, McKinstry R, Yacoub A, Duigou GJ, Young CS, Grant S, Hagan MP, Ellis E, Fisher PB, Dent P (2001). Ionizing radiation modulates vascular endothelial growth factor (VEGF) expression through multiple mitogen activated protein kinase dependent pathways. *Oncogene* **20**, 3266-80.; Su ZZ, Goldstein NI, Jiang H, Wang MN, Duigou GJ, Young CS, Fisher PB (1999). PEG-3, a nontransforming cancer progression gene, is a positive regulator of cancer aggressiveness and angiogenesis. *Proc Natl Acad Sci U S A.* **96**, 15115-20; Su Z, Shi Y, Friedman R, Qiao L, McKinstry R, Hinman D, Dent P, Fisher PB (2001). PEA3 sites within the progression elevated gene-3 (PEG-3) promoter and mitogen-activated protein kinase contribute to differentialPEG-3 expression in Ha-ras and v-raf oncogene transformed rat embryo cells. *Nucleic Acids Res* **29,** 1661-71; Su, Z. Z., Shi, Y., and Fisher, P. B. (1997). Subtraction hybridization identifies a transformation progression- associated gene PEG-3 with sequence homology to a growth arrest and DNA damage-inducible gene. *Proc Natl Acad Sci U S A* **94**, 9125-30). The results of the present invention show that the up-regulation of PEG-3 seems to be triggered earlier than that of GADDs, so that it is a possible early marker for cell damage.

TRAP proteins are part of a complex whose function is to bind Ca²⁺ to the membrane of the endoplasmic reticulum (ER) and regulate thereby the retention of ER resident proteins (Hartmann E, Gorlich D, Kostka S, Otto A, Kraft R, Knespel S, Burger E, Rapoport TA, Prehn S (1993). A tetrameric complex of membrane proteins in the endoplasmic reticulum. *Eur J Biochem.* **214**, 375-81).

Compounds used in the method of the present invention may be unknown compounds or compounds which are known to elicit a toxic effect in an organism.

Compounds in accordance with the method of the present invention include, inter alia, peptides, proteins, nucleic acids including DNA, RNA, RNAi, PNA, ribozymes, antibodies, small organic compounds, small molecules, ligands, and the like.

The compounds whose toxic effect is to be predicted with the method(s) of the present invention do not only comprise single, isolated compounds. It is also envisaged that mixtures of compounds are screened with the method of the present invention. It is also possible to employ natural products and extracts, like, inter alia, cellular extracts from prokaryotic or eukaryotic cells or organisms.

In addition, the compound identified by the inventive method as having low toxic effect can be employed as a lead compound to achieve modified site of action, spectrum of activity and/or organ specificity, and/or improved potency, and/or decreased toxicity (improved therapeutic index), and/or decreased side effects, and/or modified onset of therapeutic action, duration of effect, and/or modified pharmakinetic parameters (resorption, distribution, metabolism and excretion), and/or modified physico-chemical parameters (solubility, hygroscopicity, color, taste, odor, stability, state), and/or improved general specificity, organ/tissue specificity, and/or optimized application form and route, and may be modified by esterification of carboxyl groups, or esterification of hydroxyl groups with carbon acids, or esterification of hydroxyl groups to, e.g. phosphates, pyrophosphates or sulfates or hemi succinates, or formation of pharmaceutically acceptable salts, or formation of pharmaceutically acceptable complexes, or synthesis of pharmacologically active polymers, or introduction of hydrophylic moieties, or introduction/exchange of substituents on aromates or side chains, change of substituent pattern, or modification by introduction of isosteric or bioisosteric moieties, or synthesis of homologous compounds, or introduction of branched side chains, or conversion of alkyl substituents to cyclic analogues, or derivatisation of hydroxyl group to ketales, acetales, or N-acetylation to amides, phenylcarbamates, or synthesis of Mannich bases, imines, or transformation of ketones or aldehydes to Schiffs bases, oximes, acetales, ketales, enolesters, oxazolidines, thiozolidines or combinations thereof.

In another embodiment, the present invention provides for a set of nucleic acid primers, wherein the primers specifically amplify at least two of the genes from Table 3. The set of nucleic acid primers may also specifically amplify at least 5, at least 10, at least 20, at least 30 of the genes from Table 3. The set of nucleic acid primers may also specifically amplify nearly all or all of the genes from Table 3.

Moreover, the present invention provides for a set of nucleic acid probes, wherein the probes comprise sequences which hybridize to at least two of the genes from Table 3. The set of nucleic acid probes may comprise sequences which hybridize to at least 5, at least 10, at least 20, at least 30 of the genes from Table 3. The set of nucleic acid probes may also comprise sequences which hybridize to nearly all or all of the genes from Table 3.

In a further embodiment, the set of probes maybe attached to a solid support.

A solid support comprising at least two probes, wherein each of the probes comprises a sequence that specifically hybridizes to a gene in Table 3 is also provided. The solid support may also comprise at least 5 probes, at least 10, at least 20, at least 30 probes. The solid support may also comprise all or nearly all probes, wherein each of the probes comprises a sequence that specifically hybridizes to a gene in Table 3.

Solid supports containing oligonucleotide or cDNA probes for differentially expressed genes of the invention can be filters, polyvinyl chloride dishes, particles, beads, microparticles or silicon or glass based chips, etc. Such chips, wafers and hybridization methods are widely available, for example, those disclosed in W095/11755. Any solid surface to which a nucleotide sequence can be bound, either directly or indirectly, either covalently or non-covalently, can be used. A preferred solid support is a DNA chip. These contain a particular probe in a predetermined location on the chip. Each predetermined location may contain more than one molecule of the probe, but each molecule within the predetermined location has an identical sequence. Such predetermined locations are termed features. There may be, for example, from 2, 10, 100, 1000 to 10000, 100000 or 400000 of such features on a single solid support. The solid support, or the area within which the probes are attached may be on the order of about a square centimeter.

Probes corresponding to the genes of Table 3 may be attached to single or multiple solid support structures, e.g., the probes may be attached to a single chip or to multiple chips to comprise a chip set. Probe arrays for expression monitoring can be made and used according to any techniques known in the art (see for example, Lockhart et al., *Nat. Biotechnol.* (1996) 14, 1675-1680; McGall et al., *Proc. Nat. Acad. Sci.* USA (1996) 93, 13555-60). Such probe arrays may contain at least two or more probes that are complementary to or hybridize to two or more of the genes described in Table 3. For instance, such arrays may contain probes that are complementary or hybridize to at least 2, 3, 4, 5, 6, 7, 8, 9, 10, 20, 30, 50, 70, 100 or more of the genes described herein. Preferred arrays contain probes for all or nearly all of the genes listed in Table 3. In a preferred embodiment, arrays are constructed that contain probes to detect all or nearly all of the genes of Table 3 on a single solid support substrate, such as a chip. The sequences of the expression marker genes of Table 3 are available in public databases and their GenBank Accession Number is provided *(see www.rzcbi.nlm.nih.gov*/*).* These sequences may be used in the methods of the invention or may be used to produce the probes and arrays of the invention. As described above, in addition to the sequences of the GenBank Accessions Numbers disclosed in Table 3, sequences such as naturally occurring variant or polymorphic sequences may be used in the methods and compositions of the invention. For instance, expression levels of various allelic or homologous forms of a gene disclosed in the Table 3 may be assayed. Any and all nucleotide variations that do not alter the functional activity of a gene listed in Table 3, including all naturally occurring allelic variants of the genes herein disclosed, may be used in the methods and to make the compositions (e.g., arrays) of the invention.

Probes based on the sequences of the genes described above may be prepared by any commonly available method. "Probe" refers to a hybridizable nucleotide sequence that can be attached to a solid support or used in a liquid form. As used herein a "probe" is defined as a nucleic acid sequence, capable of binding to a target nucleic acid of complementary sequence through one or more types of chemical bonds, usually through complementary base pairing, usually through hydrogen bond formation. As used herein, a probe may include natural (i.e., A, G, U, C, or T) or modified bases (7-deazaguanosine, inosine, etc.). In addition, the bases in probes may be joined by a linkage other than a phosphodiester bond, so long as it does not interfere with hybridization. Thus, probes may be peptide nucleic acids in which the constituent bases are joined by peptide bonds rather than phosphodiester linkages. Said probes are specific oligonucleotides or cDNA-fragments. Oligonucleotide probes or cDNAs for screening or assaying a tissue or cell sample are preferably of sufficient length to specifically hybridize only to appropriate, complementary genes or transcripts. Typically the oligonucleotide probes will be at least 10, 12, 14, 16, 18, 20 or 25 nucleotides in length. In some cases, longer probes of at least 30, 40, or 50 nucleotides will be desirable. Typically, the cDNA probes will be between 300 and 1000 nucleotides in length. As used herein, oligonucleotide sequences that are complementary to one or more of the genes described in Table 3 refer to probes that are capable of hybridizing under stringent conditions to at least part of the nucleotide sequences of said genes. Such hybridizable probes will typically exhibit at least about 75% sequence identity at the nucleotide level to said genes, preferably about 80% or 85% sequence identity or more preferably about 90% or 95% or more sequence identity to said genes. The phrase "hybridizing specifically to" refers to the binding, duplexing, or hybridizing of a molecule substantially to or only to a particular nucleotide sequence or sequences under stringent conditions when that sequence is present in a complex mixture (e.g., total cellular) DNA or RNA. Assays and methods of the invention may utilize available formats to simultaneously screen at least 2, preferably about tens to thousends different nucleic acid hybridizations. The terms "background" or "background signal intensity" refer to hybridization signals resulting from non-specific binding, or other interactions, between the labeled target nucleic acids and components of the oligonucleotide array (e.g., the probes, control probes, the array substrate, etc.). Background signals may also be produced by intrinsic fluorescence of the array components themselves. A single background signal can be calculated for the entire array, or a different background signal may be calculated for each target nucleic acid. One of skill in the art will appreciate that where the probes to a particular gene hybridize well and thus appear to be specifically binding to a target sequence, they should not be used in a background signal calculation. Background can also be calculated as the average signal intensity produced by regions of the array that lack any probes at all.

One of skill in the art will appreciate that an enormous number of array designs are suitable for the practice of this invention. The array will typically include a number of test probes, at least 2, preferably tens to thousends that specifically hybridize to the sequences of interest. Probes may be produced from any region of the identified genes. In instances where the gene reference in the Tables is an EST, probes may be designed from that sequence or from other regions of the corresponding full-length transcript that may be available in any of the sequence databases, such as those herein described. Any available software may be used to produce specific probe sequences, including, for instance, software available from Applied Biosystems (Primer Express). The said probes may be attached to the solid support by a variety of methods, including among others synthesis onto the glass and spotting of a specified amount of cDNA onto the support. In addition to test probes that bind the target nucleic acid(s) of interest, the arrays can contain a number of control probes. The control probes may fall into three categories referred to herein as 1) normalization controls; 2) expression level controls; and 3) unspecific binding controls. Normalization controls are probes that are complementary to labeled reference oligonucleotides or other nucleic acid sequences that are added to the nucleic acid sample to be screened. The signals obtained from the normalization controls after hybridization provide a control for variations in hybridization conditions, label intensity, 'reading" efficiency and other factors that may cause the signal of a perfect hybridization to vary between arrays. Signals read from all other probes in the array may be divided by the signal (e.g., fluorescence intensity) from the control probes thereby normalizing the measurements. Virtually any probe may serve as a normalization control. However, it is recognized that hybridization efficiency varies with base composition and probe length. Preferred normalization probes are selected to reflect the average length of the other probes present in the array. Expression level controls are probes that hybridize specifically with constitutively expressed genes in the biological sample. Virtually any constitutively expressed gene provides a suitable target for expression level controls. Typically expression level control probes have sequences complementary to subsequences of constitutively expressed "housekeeping genes" including, but not limited to the actin gene, the transferrin receptor gene, the GAPDH gene, and the like. Unspecific binding controls can be but are not limited to DNA from other species (i.e. Hering Sperm DNA) that should not hybridize with the target sequences or mismatched sequences. Mismatched sequences are oligonucleotide probes or other nucleic acid probes identical to their corresponding test or control probes except for the presence of one or more mismatched bases. A mismatched base is a base selected so that it is not complementary to the corresponding base in the target sequence to which the probe would otherwise specifically hybridize. One or more mismatches are selected such that under appropriate hybridization conditions (e.g. stringent conditions) the test or control probe would be expected to hybridize with its target sequence, but the mismatch probe would not hybridize (or would hybridize to a significantly lesser extent). Unspecific binding controls thus provide a control for non-specific binding or cross hybridization to a nucleic acid in the sample other than the target to which the probe is directed.

Cell or tissue samples may be exposed to the test compound *in vitro* or in *vivo.* When cultured cells or tissues are used, appropriate mammalian liver extracts may also be added with the test agent to evaluate compound s that may require biotransformation to exhibit toxicity. In a preferred format, primary isolates of animal or human hepatocytes which already express the appropriate complement of drug-metabolizing enzymes may be exposed to the test compound without the addition of mammalian liver extracts. The genes which are assayed according to the present invention are typically in the form of mRNA or reverse transcribed mRNA. The genes may be cloned or not. The genes may be amplified or not. The cloning and/or amplification do not appear to bias the representation of genes within a population. In some assays, it may be preferable, however, to use polyA+ RNA as a source, as it can be used with less processing steps. As is apparent to one of ordinary skill in the art, nucleic acid samples used in the methods and assays of the invention may be prepared by any available method or process. Methods of isolating total mRNA are well known to those of skill in the art. For example, methods of isolation and purification of nucleic acids are described in detail in Chapter 3 of Laboratory Techniques in Biochemistry and Molecular Biology: Hybridization With Nucleic Acid Probes, Part I Theory and Nucleic Acid Preparation, P. Tijssen, Ed., Elsevier, N.Y. (1993). Such samples include RNA samples, but also include cDNA synthesized from a mRNA sample isolated from a cell or tissue of interest. Such samples also include DNA amplified from the cDNA, and RNA transcribed from the amplified DNA. One of skill in the art would appreciate that it is desirable to inhibit or destroy RNase present in homogenates before homogenates are used. Biological samples may be of any biological tissue or fluid or cells from any organism as well as cells raised *in vitro,* such as cell lines and tissue culture cells. Frequently the sample will be a tissue or cell sample that has been exposed to a compound, agent, drug, pharmaceutical composition, potential environmental pollutant or other composition, In some formats, the sample will be a "clinical sample" which is a sample derived from a patient. Typical clinical samples include, but are not limited to, sputum, blood, blood-cells (e.g. white cells), tissue or fine needle biopsy samples, urine, peritoneal fluid, and pleural fluid, or cells therefrom. Biological samples may also include sections of tissues, such as frozen sections or formalin fixed sections taken for histological purposes.

Nucleic acid hybridization simply involves contacting a probe and target nucleic acid under conditions where the probe and its complementary target can form stable hybrid duplexes through complementary base pairing (See WO99/32660). The nucleic acids that do not form hybrid duplexes are then washed away leaving the hybridized nucleic acids to be detected, typically through detection of an attached detectable label. It is generally recognized that nucleic acids are denatured by increasing the temperature or decreasing the salt concentration of the buffer containing the nucleic acids. The term "stringent conditions" refers to conditions under which a probe will hybridize to its target sequence, but with only insubstantial hybridization to other sequences. Stringent conditions are sequence-dependent and will be different in different circumstances. Longer sequences hybridize specifically at higher temperatures. Generally, stringent conditions are selected to be about 5°C lower than the thermal melting point (Tm) for the specific sequence at a defined ionic strength and pH. Stringent conditions may also be achieved with the addition of destabilizing agents such as formamide. Under low stringency conditions (e.g. low temperature and/or high salt) hybrid duplexes (e.g. DNA:DNA, RNA:RNA, or RNA:DNA) will form even where the annealed sequences are not perfectly complementary. Thus, specificity of hybridization is reduced at lower stringency. Conversely, at higher stringency (e.g. higher temperature or lower salt) successful hybridization tolerates fewer mismatches. One of skill in the art will appreciate that hybridization conditions may be selected to provide any degree of stringency. In a preferred embodiment, hybridization is performed at low stringency, to ensure hybridization and then subsequent washes are performed at higher stringency to eliminate mismatched hybrid duplexes. Successive washes may be performed at increasingly higher stringency until a desired level of hybridization specificity is obtained. Stringency can also be increased by addition of agents such as formamide. Hybridization specificity may be evaluated by comparison of hybridization to the test probes with hybridization to the various controls that can be present (e.g., expression level control, normalization control, mismatch controls, etc.). In general, there is a tradeoff between hybridization specificity (stringency) and signal intensity. Thus, in a preferred embodiment, the wash is performed at the highest stringency that produces consistent results and that provides a signal intensity greater than approximately 10% of the background intensity. Thus, in a preferred embodiment, the hybridized array may be washed at successively higher stringency solutions and read between each wash. Analysis of the data sets thus produced will reveal a wash stringency above which the hybridization pattern is not appreciably altered and which provides adequate signal for the particular probes of interest.

The hybridized nucleic acids are typically detected by detecting one or more labels attached to the sample nucleic acids. The labels may be incorporated by any of a number of means well known to those of skill in the art (see WO99/32660).

The present invention includes databases containing DNA sequence information as well as gene expression information from tissue or cells exposed to various standard toxins, such as those herein described (see Tables 1-2). The Toxicogenomics database is supported by in-house developed software (RACE-R, F. Hoffmann-La Roche AG, Basle, Switzerland) which allows the storage, analysis and comparison of absolute (intensity) and relative (fold-induction) gene expression data obtained by a variety of methods such as the aforementioned Affymetrix high density arrays, low density spotted arrays, PCR, etc. This database allows also for the incorporation of additional data such as sample description, biochemical parameters, histological evaluation, etc. Additional databases may also contain information associated with a given DNA sequence or tissue sample such as descriptive information about the gene associated with the sequence information (see Table 3), or descriptive information concerning the clinical status of the tissue sample, or the animal from which the sample was derived. The database may allow the use of algorithms (i.e. Toxicology Model Matcher, F. Hoffmann-La Roche AG, Basle, Switzerland) for the extensive comparison of gene expression profiles between known or unknown test compounds and compounds which are already in the database as listed in Tables 1 and 2. Methods for the configuration and construction of such databases are widely available, for instance, see U.S. Patent 5,953,727. The databases of the invention may be linked to an outside or external database such as GenBank (www.ncbi.nlm.nih.gov/entrez.index.html); KEGG 25 (www.genome.adjp/kegg); SPAD (www.grt.kyushu-u.acjp/spad/index.html); HUGO (www.gene.ucl.ac.uk/hugo); Swiss-Prot (www.expasy.ch.sprot); Prosite (www. expasy. ch/tools/scnpsitl. h&l); OMIM (www. ncbi.nlm.nih.gov/omim); GDB (www.gdb.org); and GeneCard (bioinformatics.weizmann.ac.il/cards). In a preferred embodiment, as described in Tables 3, 7 and 8, the external database is GenBank and the associated databases maintained by the National Center for Biotechnology Information (NCBI) (www.ncbi.nlm.nih.gov). Any appropriate computer platform may be used to perform the necessary comparisons between sequence information, gene expression information and any other information in the database or information provided as an input. For example, a large number of computer workstations are available from a variety of manufacturers. Client/server environments, database servers and networks are also widely available and appropriate platforms for the databases of the invention. The databases of the invention may be used to determine the cell type or tissue in which a given gene is expressed and to allow determination of the abundance or expression level of a given gene in a particular tissue or cell. The databases of the invention may also be used to present information identifying the expression level in a tissue or cell of a set of genes comprising one or more of the genes in Table 3, comprising the step of comparing the expression level of at least one gene in Table 3 in a cell or tissue exposed to a test compound to the level of expression of the gene in the database. Such methods may be used to predict the toxic potential of a given compound by comparing the level of expression of a gene or genes in Table 3 from a tissue or cell sample exposed to the test compound to the expression levels found in a control tissue or cell samples exposed to a standard toxin or hepatotoxin such as those herein described.

The gene expression data generated by the methods of the present invention may be analysed by various methods known in the art, including but not limited to hierarchical clustering, self-organizing maps and support vector machines. Support Vector Machines (SVMs), a class of supervised learning algorithms originally introduced by Vapnik and co-workers, have already been shown to perform well in multiple areas of biological analysis (Boser, B.E., Guyon, I.M., Vapnik, V.N. (1992) A training algorithm for optimal margin classifiers. In *Proceedings of the 4*^{*th*} *Annual International Conference on Computational Learning Theory,* ACM Press, Pittsburgh, PA, 144-152; Vapnik, V.N. (1998) *Statistical Learning Theory.* Wiley, New York; Scholkopf, B., Guyon, I.M., Weston, J. (2002) Statistical Learning and Kernel Methods in Bioinformatics. In *Proceedings NATO Advanced Studies Inst. on Artificial Intelligence and Heuristics Methods for Bioinformatics,* San Miniato, Italy October 1-11 ).

Given a set of training examples, SVMs are able to recognize informative patterns in the input data and generalize on previously unseen data. Trivial solutions, which overfit the training data, are avoided by minimizing the bound on the expected generalization error. In contrast to unsupervised methods like hierarchical clustering and self-organizing maps, the SVM approach takes advantage of prior knowledge in the form of class labels attached to the training examples. The extraordinary robustness with respect to sparse and noisy data makes SVMs the tool of choice in a growing number of applications. They are particularly well suited to analyze microarray expression data because of their ability to handle situations where the number of features (genes) is very large compared to the number of training patterns (chip replicates). It has been demonstrated in several studies that SVMs typically tend to outperform other classification techniques in this field (Brown, M.P.S., Grundy, W.N., Lin, D., Cristianini, N., Sugnet, C. W., Furey, T.S., Ares, M., Haussler, D. (2000) Knowledge-based analysis of microarray gene expression data by using support vector machines. *PNAS* 97, 262-267; Furey, T. S., Cristianini, N., Duffy, N., Bednarski, D.W., Schummer, M., Haussler, D. (2000) Support Vector machine classification and validation of cancer tissue samples using microarray expression data. *Bioinformatics* 16, 906-914; Yeang, C., Ramaswamy, S., Tamayo, P., Mukherjee, S., Rifkin, R.M., Angelo, M., Reich, M., Lander, E., Mesirov, J., Golub, T. (2001) Molecular classification of multiple tumor types. *Bioinformatics* 17, 316-322). In addition, the method proved effective in discovering informative features such as genes which are especially relevant for the classification and therefore might be critically important for the biological processes under investigation (Guyon, I. M., Weston, J., Barnhill, S., Vapnik, V.N. (2002) Gene Selection for Cancer Classification using Support Vector Machines. *Machine Learning* 46, 389-442).

The SVM approach can be used to generate classifiers for discrimination of a specific toxicant class from all other classes, but also to generate discriminators to distinguish between a specific toxicant and controls can be defined. Alternatively classifiers for discrimination of toxic and non-toxic compounds can be constructed. These classifiers are useful to predict toxicity as well as for identification of a specific toxicity mechanism.

Recursive feature elimination (RFE) allows identifying genes that contribute to the greatest extent to classification. In each iteration, a certain fraction of genes is removed from the training procedure, selected by the corresponding weights in the decision function. The least important genes are omitted for the next iteration. During this process, quality parameters of the resulting SVM classifiers are monitored. The final choice of a best subset of genes is made on the basis of classification accuracy, model simplicity and gene count. This method makes no orthogonality assumptions about gene expression levels but implicitly takes into account correlation between the single gene expression measurements. It results in a minimized set of predictive genes by effectively removing noise and redundancy from the set of all genes on the chip. The support vector mechanism, where borderline (and not 'typical') training patterns play a crucial role in classifications, was shown to assist in the feature elimination process by preventing genes that are irrelevant for classification but nevertheless differentially expressed in the majority of chip samples from gaining predominant influence (Guyon, I.M., Weston, J., Barnhill, S., Vapnik, V.N. (2002) Gene Selection for Cancer Classification using Support Vector Machines. *Machine Learning* 46, 389-442). Using RFE a small subset of genes is selected. This subset can subsequently be used as a diagnostic biomarker set to predict toxicity and / or the mechanism of toxicity.

The present invention therefore also provides a computer system comprising a database containing DNA sequence information and expression information of at least two of the genes from Table 3 from tissue or cells exposed to a hepatotoxin, and a user interface.

The invention further includes kits combining, in different combinations, nucleic acid primers for the amplification of the genes of Table 3, solid supports with attached probes, reagents for use with the solid supports, protein reagents encoded by the genes of Table 3, signal detection and array-processing instruments, gene expression databases and analysis and database management software described above. The kits may be used, for example, to predict or model the toxic response of a test compound, to monitor the progression of hepatic disease states, to identify genes that show promise as new drug targets and to screen known and newly designed drugs as discussed above.

The databases packaged with the kits are a compilation of expression patterns from human or laboratory animal genes and gene fragments (corresponding to the genes of Table 3). In particular, the database software and packaged information include the expression results of Table 3 that can be used to predict toxicity of a test compound. In another format, database and software information may be provided in a remote electronic format, such as a website, the address of which may be packaged in the kit.

The invention is now described by reference to the following examples and figures which are merely illustrative and are not to be construed as a limitation of scope of the present invention.

### Examples

Commercially available reagents referred to in the examples were used according to manufacturer's instructions unless otherwise indicated.

### Example 1: Hepatotoxicity assay with non-human animals

All animals received human care as specified by Swiss law and in accordance with the "Guide for the care and use of laboratory animals" published by the NIH. Male Wistar rats (generally 5 animals/dose-group) were purchased from BRL (Fullingsdorf, Switzerland) and housed individually. Treated animals were dosed either orally, intraperitoneally, or intravenously with several doses of test compounds (Table 1). The test compounds were categorized according to their toxic manifestation in the rat liver. Control animals received the same volume of vehicle as placebo. Necropsy was performed 6 or 24 hours after a single administration and liver samples from the left medial lobe were placed immediately in RNALater (Ambion, TX, USA) for RNA extraction and gene expression analysis. Samples in RNALater were stored at -20°C until further processing. Additional liver samples were snap-frozen in liquid nitrogen for measurement of intrahepatic lipids and/or proteins.

### Example 2: Hepatocyte cell culture assay toxicity

Hepatocytes were isolated from adult male Wistar rats by two-step collagenase liver perfusion previously described (Goldlin C.R., Boelsterli U.A. (1991). Reactive oxygen species and non-peroxidative mechanisms of cocaine-induced cytotoxicity in rat hepatocyte cultures. *Toxicology* **69,** 79-91). Briefly, the rats were anaesthetized with sodium pentobarbital (120 mg/kg, *i.p.*). The perfusate tubing was inserted via the portal vein, then the v. *cava caudalis* was cut, and the perfusion was started. The liver was first perfused for 5 min with a preperfusing solution consisting of calcium-free, EGTA (0.5 mM)-supplemented, HEPES (20 mM)-buffered Hank's balanced salt solution (5.36 mM KC1, 0.44 mM KH₂PO₄, 137 mM NaCl, 4.2 mM NaHCO₃, 0.34 mM Na₂HPO₄, 5.55 mM D-glucose). This was followed by a 12-min perfusion with NaHCO₃ (25 mM)-supplemented Hank's solution containing bovine CaCl₂ (5 mM), and collagenase (0.2 U/ml). Flow rate was maintained at 28 ml/min and all solutions were kept at 37°C. After in situ perfusion the liver was excised and the liver capsule was mechanically disrupted. The cells were suspended in William's Medium E without phenol red (WME, Sigma Chemie, Buchs, Switzerland) and filtered through a set of tissue sieves (30-, 50-, and 80-mesh). Dead cells were removed by a sedimentation step (1xg for 15 min at 4°C) followed by a Percoll (Sigma) centrifugation step and an additional centrifugation in WME (50g, 3min). Hepatocyte viability was assessed by trypan blue exclusion and typically lied between 85% and 95%. The cells were seeded into collagen-coated 6-well Falcon Primaria® plates at a density of 9x10⁵ cells/well in 2 ml WME supplemented with 10% fetal calf serum (BioConcept, Allschwil, Switzerland), penicillin (100 U/ml, Sigma Chemie, Buchs, Switzerland), streptomycin (0.1 mg/ml, Sigma Chemie, Buchs, Switzerland), insulin (100 nM, Sigma Chemie, Buchs, Switzerland), and dexamethasone (100 nM). After an attachment period of 3 hrs, the medium was replaced by 1.5 ml/well serum-free WME, supplemented with antibiotics and hormones, and incubated overnight at 37°C in an atmosphere of 5% CO2/95% air. Cells were then incubated with the test compounds or vehicle (Table 2) and harvested for RNA extraction at 6 or 24 hours.

### Example 3: Measurement of circulating and hepatic enzymes

In the hepatotoxicity assay with non-human animals as described in Example 1, circulating enzymes of hepatic origin, as well as the hepatic lipid content were assessed. Blood samples for clinical chemistry were obtained shortly before sacrifice. The enzymatic activities of aspartate aminotransferase (AST), alanine aminotransferase (ALT), lactate dehydrogenase (LDH) and 5-nucleotidase (5-ND) were measured in serum samples. Liver lipids were extracted using liver homogenates as described by Freneaux et al (Freneaux, E., Labbe, G., Letteron, P., The Le, D., Degott, C., Geneve, J., Larrey, D., and Pessayre, D. (1988). Inhibition of the mitochondrial oxidation of fatty acids by tetracycline in mice and in man: possible role in microvesicular steatosis induced by this antibiotic. *Hepatology* **8**, 1056-62) and the contents of triglycerides, phospholipids and total lipids were measured. Automated analysis was performed using commercially available test kits (Roche Diagnostics, Mannheim, Germany) on a Cobas Fara autoanalyzer (Roche, Basel, Switzerland).

### Example 4: RNA sample preparation

RNA isolation from hepatocytes was typically performed by resuspending approximately 3 Mio. Cells/1.2 mL RNAzol (Tel-Test Inc., TX, USA). For RNA isolation from liver tissue, a portion of tissue of approximately 100 mg was transferred to a tube containing 1.2 ml RNAzol. Cells or tissue in RNAzol were disrupted in FastPrep tubes for 20 seconds in a Savant homogenizer (Bio101, Buena Vista, CA, U.S.A.). Total RNA was isolated according to the manufacturer's instruction and quantified by measuring the optical density at 280 nm. The quality of RNA was assessed with gel electrophoresis.

### Example 5: Synthesis and hybridization of cRNA

Double stranded cDNA was synthesized from 20 µg of total RNA using a cDNA Synthesis System (Roche Diagnostics, Mannheim, Germany) with the oligo(dT)24 T7prom)65 primer. The MEGAScript T7 kit (Ambion, Austin, TX, U.S.A.) was used to transcribe the cDNA into cRNA in the presence of Biotin-11-CTP and Biotin-16-UTP (Enzo, Farmingdale, NY, U.S.A.) according to the instructions supplied with the kit. After purification with the RNeasy kit (Qiagen, Hilden, Germany) integrity of the cRNA was checked using gel electrophoresis. 10-15 µg fragmented cRNA were used for hybridization to the RG-U34A array (Affymetrix GeneChip® array, Santa Clara, CA). The oligonucleotide array used in the present study contains probe sets for over 5000 rat genes. Hybridization and staining were performed basically as described previously (Lockhart DJ, Dong H, Byrne MC, Follettie MT, Gallo MV, Chee MS, Mittmann M, Wang C, Kobayashi M, Horton H, Brown EL (1996). Expression monitoring by hybridization to high-density oligonucleotide arrays. *Nat Biotechnol.* **14**, 1675-80.; de Saizieu A, Certa U, Warrington J, Gray C, Keck W, Mous J. (1998). Bacterial transcript imaging by hybridization of total RNA to oligonucleotide arrays. *Nat Biotechnol.* **16**, 45-8). Arrays were scanned with a confocal laser scanner (Hewlett-Packard, Palo Alto, CA, USA).

### Example 6: Expression analysis

After hybridization and scanning, the expression level of each gene was calculated by subtracting the fluorescence intensity of the mismatch probes from the match signal (=average difference) using the GENECHIP 3.1 software or its up-dated versions MAS 4.0 and MAS 5.0 (Affymetrix). Gene expression data were further analyzed with an in-house-developed data analysis tool (RACE-A, Hoffmann-La Roche, Basel, Switzerland). Data sets of treated versus time and vehicle matched control were generated for each treatment (treated vs. controls) and compared. Analyzed data were stored in a toxicogenomics database (RACE-R, Hoffmann-La Roche, Basel, Switzerland). For the gene expression analysis, difference of means, fold-induction, statistical significance (Students' t-test) were applied for querying the data base. Likewise, increase in circulating liver enzymes (ALT, AST, ALP, etc.) were analyzed. A linear regression was performed between gene expression levels for each gene and circulating enzyme levels. An example of correlation between gene expression and circulating ALT levels is given in Figure 1.

### Example 7: Detection of profiles and specific marker genes

Gene expression changes common to a number of compounds belonging to the same hepatotoxicity mechanism were considered profiles typical for this mechanism. The profiles were determined after the *in vivo* exposure of adult male Wistar rats to 18 compounds, from which 6 were steatotic, 6 were direct acting and 6 were cholestatic. For each tested compound, one or several independent experiments were performed. The results are depicted in Table 3, showing that 680 differentially expressed genes were identified *in vivo:* 30 of them were only regulated in livers after exposure to steatotic compounds; 18 were regulated after exposure to cholestatic compounds; and 559 after exposure to direct acting compounds. 11 genes showed regulation by all types of tested toxic compounds and are probably related to cellular stress. Others show regulation by two types of toxicity mechanisms.

In addition to the defined profiles, gene expression levels and their correlation (linear regression) with the circulating liver enzymes were used to select genes whose expression varied across the samples. These genes were chosen as possible toxicity markers and selected with less stringent filtering criteria. Candidate marker genes were categorized as follows:
a) differentially expressed genes (up-regulated in animals showing elevated enzymes in comparison to the matched controls, 2-fold change, p<0.05), b) differentially expressed genes (down-regulated in animals showing elevated enzymes in comparison to the matched controls, 2-fold change, p<0.05); c) genes that fulfilled the criteria in a) but that additionally showed an up-regulation at doses and/or time points at which no elevation of circulating enzymes could be detected; d) genes that fulfilled the criteria in b) but that additionally showed an down-regulation at doses and/or time points at which no elevation of circulating enzymes could be detected. Some of these marker genes are listed in Table 4. Among them, PEG-3 (progression elevated gene 3, # 1 in the said Table 4) and TRAP (translocon-associated protein, #9 in Table 4) showed very good characteristics as a possible early predictor *in vivo* (Michael Fountoulakis, Maria-Cristina de Vera, Flavio Crameri, Franziska Boess, Rodolfo Gasser, Silvio Albertini, Laura Suter: "Modulation of gene and protein expression by carbon tetrachloride in the rat liver", Toxicol and Appl. Pharmacol. 2002 Aug 15;183(1):71-80) as well as *in vitro.* This is represented in Figures 1 through 4.

### Example 8: Data validation for selected genes

The regulation of the mRNA levels of several candidate genes (Table 4) was verified with quantitative RT-PCR. Specific primers for these genes have been designed in order to evaluate the expression with RT-PCR using SybrGreen assay (Table 6). For each performed RT-PCR reaction, the specificity of the assay was evaluated with dissociation curve software (Applied Biosystems), as well as by assessment of the size of the product using either gel electrophoresis or Agilent Bioanalyzer. The obtained results are described in this example and generally confirmed the results obtained using GeneChips (analysis performed with microarray suite version MAS 4.0 or MAS 5.0).

Western-blot analysis was performed on 10 micrograms total liver protein following standard laboratory procedures. Proteins transferred onto a nitrocellulose membrane were incubated with the first antibody (Anti-cytochrome p450 2B1, raised in goat, purchased at GenTest, Massachusetts); followed by an incubation with the second antibody (donkey anti-sheep/Goat Immunoglobulin Horseradish Peroxidase Conjugated, from Chemikon). Chemoluminiscence was quantified by densitometry in a Multimage Light Cabinet (Alpha Inotech Corporation, San Leandro, CA, USA) using Lumilight (Roche Diagnostics AG, Rotkreuz, Switzerland) solution.

Real-time PCR is a truly quantitative method, while genechip-analysis is only semiquantitative for transcriptional expression studies. In a first step, the induction of the mRNA levels of 9 candidate genes (Table 5 and Fig. 10) was verified with quantitative PCR. The results obtained with both methods showed that the expression of these genes would allow the differentiation of a steatotic compound from a pharmacological analogue that does not show steatotic potential. These genes could be possible diagnostic and predictive molecular markers for different mechanisms of hepatic toxicity. As an internal control, the house-keeping gene glycernine-aldehyde-phosphate-dehydrogenase (GAPDH) was also analyzed.

### 5-HT6 receptor antagonists

RT-PCR results confirmed that the two 5-HT6 receptor antagonists could be distinguished using the expression levels of few marker genes (Fig. 10). Note that for some of the selected genes, a slight induction with the non-toxic compound Ro 66-0074 was also observed. However, this induction was minor when compared to the larger effect elicited by the toxic compound Ro 65-7199 so that differentiation of both compounds remains possible.

In addition, Western blots were performed using specific antibodies against the cytochrome P450 CYP2B family in order to evaluate if the clear induction of messenger RNA also led to an increase in the hepatic protein levels. The results of the protein levels of CYP2B closely paralleled the amounts of mRNA at 24 hours and at 7 days after Ro 65-7199 treatment. However, the induction of CYP2B could not be detected 6 hours after administration of Ro 65-7199, in spite of the increased levels of messenger RNA. This is due to the time lag between protein and mRNA induction (Fig. 11).

### Direct acting compounds regulate the GADD-family

Further experiments with RT-PCR confirmed results regarding the induction of genes from the GADD family, namely GADD-45, GADD-153 and PEG-3 by direct acting compounds (Hydrazine, Thioacetamide, 1,2-dichlorobenzene) (Table 9). The induction of these genes correlates with the histopathological findings in a time related fashion: While PEG-3 seems to be an early marker, GADD-45 and GADD-143 appear regulated at later time points, when the tissue damage is obvious by conventional endpoints. These results are in line with literature and confirm the assumption that PEG-3 is an early marker for hepatic damage.

### Induction of EGR1 by Tolcapone (Tasmar) and dinitrophenol

Tolcapone (Tasmar) is a human hepatotoxin with no known toxicity in the rat. In this experiment, a slight induction of EGR1 was detected after exposure of rats to a high dose of Tolcapone (300 mg/kg) and dinitrophenol (10 and 30 mg/kg). The induction was slight and showed high inter-individual variability but experiments with RT-PCR confirmed these results (Table 10).

EGR1 (early growth response gene 1, EMBL_ro:rnngfla) is a transcription factor that is also known by synonyms such as Zif268, NGF1-A, Krox24, TIS8. Its name derives of the kinetics of its induction, since it is a primary transcribed signal: the protein can be induced within minutes of a stimulus and then decays within hours (Khachigian, L.M. and T. Collins, Inducible expression of Egr-1-dependent genes. A paradigm of transcriptional activation in vascular endothelium. Circ Res, 1997. **81**(4): p. 457-61; Yan, S.F., et al., Egr-1: is it always immediate and early? J Clin Invest, 2000. **105**(5): p. 553-4). Nevertheless, maintained high expression of EGR1 has been described in atherosclerotic tissue and in connection to cell death in Alzheimer's disease, establishing a relationship between EGR1 overexpression and chronic conditions (McCaffrey, T.A., et al., High-level expression of Egr-1 and Egr-1-inducible genes in mouse and human atherosclerosis. J Clin Invest, 2000. **105**(5): p. 653-62). Its function under normal conditions is still unclear, since EGR1-nul mice display normal phenotype with exception of infertility in females (Lee, S.L., et al., Luteinizing hormone deficiency and female infertility in mice lacking the transcription factor NGFI-A (Egr-1). Science, 1996. **273**(5279): p. 1219-21). Thus, the physiological role of EGR1 might only become manifest upon environmental challenge. This gene has been found overexpressed in several pathologic conditions, including exposure to ionising radiation, prostate cancer and hypoxia (Weichselbaum, R.R., et al., Radiation-induced tumour necrosis factor-alpha expression: clinical application of transcriptional and physical targeting of gene therapy. Lancet Oncol, 2002. **3**(11): p. 665-71). The up-regulation of EGR1 after hypoxia leads to vascular and perivascular tissue damage. In particular in lung, EGR1 induction leads an increase of Tissue Factor (TF) and to deposition of fibrin in the lung vasculature (Yan, S.F., et al., Egr-1, a master switch coordinating upregulation of divergent gene families underlying ischemic stress. Nat Med, 2000. **6**(12): p. 1355-61). EGRl-deficient mice show significantly reduced prostate tumor formation and significantly less pulmonary vascular permeability and therefore better survival after ischemic injury (Abdulkadir, S.A., et al., Impaired prostate tumorigenesis in Egr1-deficient mice. Nat Med, 2001.7(1): p. 101-7; Ten, V.S. and D.J. Pinsky, Endothelial response to hypoxia: physiologic adaptation and pathologic dysfunction. Curr Opin Crit Care, 2002. **8**(3): p. 242-50).

The literature reports suggest a possible involvement of EGR1 as an early signal to injury that triggers subsequent tissue damage. In particular in the liver, a link between mitochondrial uncoupling (as caused by dinitrophenol) and induction of EGR1 was established. Also, tolcapone (Tasmar) has been described as having mitochondrial uncoupling properties *in vitro.* Thus, it is suggested that the induction of EGR1 in rats exposed to tolcapone (Tasmar) might lead to hepatic tissue injury if the physiological environment (i.e. existing disease or genetic background) is appropriate. This would explain the low incidence of human hepatotoxicity caused by tolcapone (Tasmar).

### Example 9: Expression data analysis with Support Vector Machines

Adult male Wistar rats were dosed *in vivo* and the resulting expression profile in liver was determined. SVMs were built for the discrimination between 3 different classes of hepatotoxicants, non-toxic substances and controls. The training set consisted of 180 gene expression profiles from individual animals treated with direct acting, cholestatic, steatotic, non-toxic compounds and corresponding vehicle-dosed controls. As a first step the chips were rescaled to a median value of 0 and a standard deviation of 1. Subsequently chips were presented to a linear kernel SVM (for classifier training the SVM software package from William Stafford Noble, Department of Computer Science, Columbia University, New York was used. Procedures for the Recursive Feature Elimination (RFE), automation of the whole training cycles and further data analysis were developed in house, using the PERL language.) Single binary classifiers for each category of chips were obtained by training one group against all others ('One-vs-AU' training method). Multi-class classification for a given test chip was then carried out by combining the outputs of all binary. classifiers. A leave-one-out cross validation procedure was applied to assess the quality of the trained machines with respect to the training data. This method consists of removing one sample from the training set, building a classifier on the basis of the remaining data and then testing on the withheld example. By removing all replicates of one compound from the training data, followed by classifying these chips with the resulting decision function, the individual contribution of the given compound for a successful classifier could be examined. RFE was used to investigate the relationship between the number of genes for generating the classifiers, the resulting prediction accuracy, cross-validation errors and the number of used support vectors. The first iteration reduced the gene count to a multiple of 2. In each subsequent iteration the gene count was halved until 32 genes remained. Afterwards only one gene per iteration was removed. An example of a RFE for the direct acting class is shown in Figure 7.

Based on classification accuracy, model simplicity and gene count a SVM was selected for each class. As can be seen the SVM for discrimination of direct acting compounds from all others was based on 6 genes. The corresponding gene numbers for the other SVMs were: Steatotic (6 genes), cholestatic (21 genes), non-toxic (19 genes) and controls (46 genes).

Compounds not present in the initial training set were subsequently classified based on their expression profiles. Expression profiles for individual animals were classified using the 5 previously generated support vector machines. The successful identification of amineptine as a steatotic compound is depicted in Figure 8 and the identification of 1,2-Dichlorobenzene as a direct acting compound is shown in Figure 9. The bigger a positive value is, the better is the data fit into a specific class defined by the respective SVM. A negative discriminant value means that data do not fit into a compound class.

The above-described method was used to find class-specific genes that allow discrimination of a class from all other classes (class-discriminating genes, Table 7).

The same approach was also used to find toxicant specific genes for each of the categories. Using RFE SVMs for the discrimination of direct acting compounds from controls were generated. Based on classification accuracy, model simplicity and gene count one SVM was selected for discrimination of the direct acting group from the control group. This classifier was based on 14 genes (specific genes for the direct acting group, Table 8).

The same procedure was repeated for the steatotic and the cholestatic class. The classifier for the cholestatic group contained 34 genes and the classifier for the steatotic group contained 3 genes (Table 8). The genes required to separate a class from all other classes or just from controls can therefore be different.

**Table 1**

| Compound | Dose levels | Target organ | Hepatotoxicity Mechanism |
|---|---|---|---|
| Chlorpromazine | 15 mg/kg | Liver | Cholestatic |
| Cyclosporine A | 5, 15 and 30 mg/kg | Liver | Cholestatic |
| Erythromycin | 734 mg/kg | Liver | Cholestatic |
| Glibenclamide | 2.5 and 25 mg/kg | Liver | Cholestatic |
| Lithocholic acid | 60 and 120 µmol/kg | Liver | Cholestatic |
| Ro 48-5695 (ETA) | 25 mg/kg | Liver | Cholestatic |
| Dexamethasone | 0.6 mg/kg | Liver | Cyp inducer/prolif |
| 1,2-Dichlorobenzene | 1.5 and 4.5 mmol/kg | Liver | Direct Acting |
| Aflatoxin B1 | 1 and 4 mg/kg | Liver | Direct Acting |
| Bromobenzene | 1 and 3 mmol/kg | Liver | Direct Acting |
| Carbon tetrachloride | 0.25 and 2 ml/kg | Liver | Direct Acting |
| Diclofenac | 10, 30, 100 mg/kg | Liver | Direct Acting |
| Hydrazine | 10, 60, 90 mg/kg | Liver | Direct Acting |
| Nitrofurantoin | 5, 20, 60 mg/kg | Liver | Direct Acting |
| Thioacetamide | 2, 10, 50 mg/kg | Liver | Direct Acting |
| Concanavaline A | 0.1, 20 mg/kg | Liver | Hepatitis/Infammation |
| Tacrine | 5, 15 and 35 mg/kg | Liver | Human Hepatotox |
| Tempium (Lazabemide) | 20 and 1000 mg/kg | Liver | Human hepatotox |
| Tolcapone (Tasmar) | 300 mg/kg | Liver | Human hepatotox |
| 1,4-Dichlorobenzene | 4.5 mmol/kg | Liver | Non toxic |
| Amineptin | 125, 250, 500 µmol/kg | Liver | Steatotic |
| Amiodarone | 50, 100, 600 mg/kg | Liver | Steatotic |
| Doxycycline | 5, 20, 40 mg/kg | Liver | Steatotic |
| Ro 28-1674 (GKA) | 250 mg/kg | Liver | Steatotic |
| Ro 28-1675 (GKA) | 100 mg/kg | Liver | Steatotic |
| Ro 65-7199 (5HT6) | 30, 100. 400 mg/kg | Liver | Steatotic |
| Tetracycline | 125, 200, 250 µmol/kg | Liver | Steatotic |
| Dinitrophenol | 10 and 30 mg/kg | Liver | Uncoupling |
| Ro 48-5695: Pyridin-2-ylcarbamic acid 2-[6-(5-isopropyl-pyridin-2-ylsulfonylamino)-5-(2-methoxy-phenoxy)-2-morpholin-4-yl-pyrimidin-4-yloxyl-ethyl ester; Ro 28-1674: 3-Cyclopentyl-2[S]-(4-methanesulfonyl-phenyl)-N-thiazol-2-yl-propionamide; Ro 28-1675: 3-Cyclopentyl-2 [R] -(4-methanesulfonyl-phenyl)-N-thiazol-2-yl-propionamide. | | | |

**Table 2**

| Compound | Test Concentrations | Hepatocyte culture System | Hepatotoxicity Mechanism |
|---|---|---|---|
| Chlorpromazine | 10, 30 100 µM | Monolayer | Cholestatic |
| Cyclosporine A | 0.5 and 5 µM | Monolayer | Cholestatic |
| Erythromycin | 100 and 300 µM | Monolayer | Cholestatic |
| Lithocholic acid | 10 and 30 µM | Monolayer | Cholestatic |
| Ro 48-5695 (ETA) | 20 and 60 µM | Monolayer | Cholestatic |
| Cyproterone Acetate | 5 and 25 µM | Monolayer | Cyp inducer/prolif |
| Phenobarbital | 200 and 2000 µM | Monolayer | Cyp inducer/prolif |
| Clofibrate | 100 and 1000 µM | Monolayer | Cyp inducer/prolif |
| Acetaminophen | 1000, 2500 and 5000 µM | Monolayer | Direct Acting |
| Acetaminophen | 1000, 2500 µM | Sandwich | Direct Acting |
| Bromobenzene | 1000 and 2000 µM | Monolayer | Direct Acting |
| Carbon tetrachloride | 3000 and 5000 µM | Monolayer | Direct Acting |
| Hydrazine | 8000 and 16000 µM | Monolayer | Direct Acting |
| Methapyrilene | 20 µM | Sandwich | Direct Acting |
| Methapyrilene | 100, 300 and 1000 µM | Monolayer | Direct Acting |
| Nitrofurantoin | 20, 100 and 200 µM | Monolayer | Direct Acting |
| Thioacetamide | 3000 and 10000 µM | Monolayer | Direct Acting |
| Thioacetamide-S-Oxide | 30, 100 and 300 µM | Monolayer | Direct Acting |
| Amineptin | 500, 1000 and 1500 µM | Monolayer | Steatotic |
| Amiodarone | 30, 70, 100 and 300 µM | Monolayer | Steatotic |
| Doxycycline | 100, 500 and 1000 µM | Monolayer | Steatotic |
| Perhexiline | 3, 10 and 30 µM | Monolayer | Steatotic |
| Ro 28-1674 (GKA) | 19 and 75 µM | Monolayer | Steatotic |
| Ro 28-1675 (GKA) | 19 and 75 µM | Monolayer | Steatotic |
| Ro 65-7199 (5HT) | 20 and 100 µM | Monolayer | Steatotic |
| Tetracycline | 100 and 500 µM | Monolayer | Steatotic |

| | | | | | | |
|---|---|---|---|---|---|---|
| Gene identifiers are given as the Affymetrix ID from the Affymetrix GeneChip® RG-U34A. The accession numbers refer to GenBank and for each type of hepatotoxicity, the direction of the gene regulation is indicated (1 for up-regulation, -1 for down-regulation). Blank cells indicate the lack of regulation under the used analysis criteria. | | | | | | |

| Affymetrix ID | Cholestatic | Direct Acting | Steatotic | Profile | Acc Number | SEQ ID NO |
|---|---|---|---|---|---|---|
| AF023087_{_}s_{_}at | 1 | 1 | 1 | Unspecific | AF023087 | 1 |
| D11445exon#1-4_5_at | 1 | 1 | 1 | Unspecific | D11445 | 2 |
| L25785_{_}at | -1 | -1 | -1 | Unspecific | L25785 | 3 |
| M18416_{_}at | 1 | 1 | 1 | Unspecific | M18416 | 4 |
| M58634_{_}at | 1 | 1 | 1 | Unspecific | M58634 | 5 |
| M60921_{_}g_{_}at | 1 | 1 | 1 | Unspecific | M60921 | 6 |
| rc_{_}AA891041_{_}at | 1 | 1 | 1 | Unspecific | AA891041 | 7 |
| rc_{_}AA893485_{_}at | -1 | -1 | -1 | Unspecific | AA893485 | 8 |
| rc_AI137856_s_at | 1 | 1 | 1 | Unspecific | AI137856 | 9 |
| rc_AI172293_at | -1 | -1 | -1 | Unspecific | AI172293 | 10 |
| U75397UTR#1_{_}s_{_}at | 1 | 1 | 1 | Unspecific | U75397 | 11 |
| AF003835_{_}at | | -1 | 1 | Steatotic/ Direct Acting | AF003835 | 12 |
| AF014503_at | | 1 | 1 | Steatotic/ Direct Acting | AF014503 | 13 |
| AF079864_{_}at | | -1 | -1 | Steatotic/ Direct Acting | AF079864 | 14 |
| D14989_{_}f_{_}at | | -1 | -1 | Steatotic/ Direct Acting | D14989 | 15 |
| D17370_{_}at | | -1 | -1 | Steatotic/ Direct Acting | D17370 | 16 |
| D17370_g_at | | -1 | -1 | Steatotic/ Direct Acting | D17370 | 17 |
| D44495_{_}s_{_}at | | 1 | 1 | Steatotic/ Direct Acting | D44495 | 18 |
| E01524cds_{_}s_{_}at | | 1 | 1 | Steatotic/ Direct Acting | E01524 | 19 |
| J02585_{_}at | | - 1 | -1 | Steatotic/ Direct Acting | J02585 | 20 |
| L16764_{_}s_{_}at | | 1 | 1 | Steatotic/ Direct Acting | L16764 | 21 |
| L16995_{_}at | | -1 | -1 | Steatotic/ Direct Acting | L16995 | 22 |
| M15481_{_}at | | -1 | -1 | Steatotic/ | M15481 | 23 |
| | | | | Direct Acting | | |
| M21208mRNA_s_at | | 1 | 1 | Steatotic/ Direct Acting | M21208 | 24 |
| M23572_at | | - 1 | -1 | Steatotic/ Direct Acting | M23572 | 25 |
| rc_AA799766_at | | 1 | 1 | Steatotic/ Direct Acting | AA799766 | 26 |
| rc_AA800224_at | | 1 | -1 | Steatotic/ Direct Acting | AA800224 | 27 |
| rc_AA891713_at | | 1 | 1 | Steatotic/ Direct Acting | AA891713 | 28 |
| rc_AA892775_at | | 1 | 1 | Steatotic/ Direct Acting | AA892775 | 29 |
| rc_AA946503_at | | 1 | 1 | Steatotic/ Direct Acting | AA946503 | 30 |
| rc_AI145931_at | | -1 | -1 | Steatotic/ Direct Acting | AI145931 | 31 |
| rc_AI169327_g_at | | 1 | 1 | Steatotic/ Direct Acting | AI169327 | 32 |
| rc_AI176546_at | | 1 | 1 | Steatotic/ Direct Acting | AI176546 | 33 |
| rc_AI 177004_s_at | | -1 | | Steatotic/ Direct Acting | AI177004 | 34 |
| rc_AI639391_at | | -1 | -1 | Steatotic/ Direct Acting | AI639391 | 35 |
| X05684_at | | -1 | -1 | Steatotic/ Direct Acting | X05684 | 36 |
| X52625_at | | -1 | -1 | Steatotic/ Direct Acting | X52625 | 37 |
| X91234_at | | -1 | -1 | Steatotic/ Direct Acting | X91234 | 38 |
| AA848218_at | | | 1 | Steatotic | AA848218 | 39 |
| AB010635_s_at | | | 1 | Steatotic | AB010635 | 40 |
| AF022136_at | | | -1 | Steatotic | AF022136 | 41 |
| AF087839mRNA#1_s_at | | | 1 | Steatotic | AF087839 | 42 |
| K02814_at | | | 1 | Steatotic | K02814 | 43 |
| L09647_at | | | - 1 | Steatotic | L09647 | 44 |
| L32132_at | | | 1 | Steatotic | L32132 | 45 |
| L36460mRNA_at | | | 1 | Steatotic | L36460 | 46 |
| M10068mRNA_s_at | | | 1 | Steatotic | M10068 | 47 |
| M14369exon#2_at | | | 1 | Steatotic | M14369 | 48 |
| M23566exon_s_at | | | 1 | Steatotic | M23566 | 49 |
| M35300_f_at | | | 1 | Steatotic | M35300 | 50 |
| rc_AA892522_at | | | -1 | Steatotic | AA892522 | 51 |
| rc_AA894316_at | | | -1 | Steatotic | AA894316 | 52 |
| rc_AA900582_at | | | 1 | Steatotic | AA900582 | 53 |
| rc_AI044985_at | | | -1 | Steatotic | AI044985 | 54 |
| rc_AI175764_s_at | | | -1 | Steatotic | AI175764 | 55 |
| rc_AI176351_s_at | | | 1 | Steatotic | AII76351 | 56 |
| rc_AI230256_at | | | -1 | Steatotic | AI230256 | 57 |
| rc_AI639108_at | | | -1 | Steatotic | AI639108 | 58 |
| rc_H31144_g_at | | | 1 | Steatotic | H31144 | 59 |
| S81478_s_at | | | -1 | Steatotic | S81478 | 60 |
| U02553cds_s_at | | | -1 | Steatotic | U02553 | 61 |
| U08214_s_at | | | 1 | Steatotic | U08214 | 62 |
| U35345_s_at | | | 1 | Steatotic | U35345 | 63 |
| U48220_at | | | -1 | Steatotic | U48220 | 64 |
| U88630_at | | | 1 | Steatotic | U88630 | 65 |
| X07648cds_g_at | | | 1 | Steatotic | X07648 | 66 |
| X62952_at | | | 1 | Steatotic | X62952 | 67 |
| X91810_at | | | 1 | Steatotic | X91810 | 68 |
| AA799276_at | | 1 | | Direct Acting | AA799276 | 69 |
| AB002086_g_at | | 1 | | Direct Acting | AB002086 | 70 |
| AB004096_at | | -1 | | Direct Acting | AB004096 | 71 |
| AB009636_at | | -1 | | Direct Acting | AB009636 | 72 |
| AB010466_s_at | | 1 | | Direct Acting | AB010466 | 73 |
| AB010963_s_at | | -1 | | Direct Acting | AB010963 | 74 |
| AB012230_g_at | | -1 | | Direct Acting | AB012230 | 75 |
| AB014722_g_at | | 1 | | Direct Acting | AB014722 | 76 |
| AB015433_s_at | | 1 | | Direct Acting | AB015433 | 77 |
| AB016536_s_at | | 1 | | Direct Acting | AB016536 | 78 |
| AB017188_at | | 1 | | Direct Acting | AB017188 | 79 |
| AB020504_at | | -1 | | Direct Acting | AB020504 | 80 |
| AF001417_s_at | | 1 | | Direct Acting | AF001417 | 81 |
| AF013144_at | | 1 | | Direct Acting | AF013144 | 82 |
| AF017637_at | | -1 | | Direct Acting | AF017637 | 83 |
| AF020618_at | | 1 | | Direct Acting | AF020618 | 84 |
| AF021935_at | | 1 | | Direct Acting | AF021935 | 85 |
| AF025308_f_at | | 1 | | Direct Acting | AF025308 | 86 |
| AF029240_g_at | | - 1 | | Direct Acting | AF029240 | 87 |
| AF029310_at | | 1 | | Direct Acting | AF029310 | 88 |
| AF030086UTR#1_at | | -1 | | Direct Acting | AF030086 | 89 |
| AF030087UTR#1_at | | 1 | | Direct Acting | AF030087 | 90 |
| AF030087UTR#1_g_at | | 1 | | Direct Acting | AF030087 | 91 |
| AF036335_at | | 1 | | Direct Acting | AF036335 | 92 |
| AF037072_at | | -1 | | Direct Acting | AF037072 | 93 |
| AF039890mRNA_s_at | | -1 | | Direct Acting | AF039890 | 94 |
| AF041066_at | | -1 | | Direct Acting | AF041066 | 95 |
| AF044574_at | | -1 | | Direct Acting | AF044574 | 96 |
| AF045464_s_at | | 1 | | Direct Acting | AF045464 | 97 |
| AF050661UTR#1_at | | 1 | | Direct Acting | AF050661 | 98 |
| AF054618_s_at | | 1 | | Direct Acting | AF054618 | 99 |
| AF058791_at | | 1 | | Direct Acting | AF058791 | 100 |
| AF061443_at | | -1 | | Direct Acting | AF061443 | 101 |
| AF062594_g_at | | 1 | | Direct Acting | AF062594 | 102 |
| AF062741_g_at | | -1 | | Direct Acting | AF062741 | 103 |
| AF063447_at | | 1 | | Direct Acting | AF063447 | 104 |
| AF067650_at | | 1 | | Direct Acting | AF067650 | 105 |
| AF069782_at | | 1 | | Direct Acting | AF069782 | 106 |
| AF080507_at | | - 1 | | Direct Acting | AF080507 | 107 |
| AF080507_g_at | | -1 | | Direct Acting | AF080507 | 108 |
| AF082124_s_at | | 1 | | Direct Acting | AF082124 | 109 |
| AF084186_s_at | | 1 | | Direct Acting | AF084186 | 110 |
| AF087037_at | | 1 | | Direct Acting | AF087037 | 111 |
| AJ011607_g_at | | -1 | | Direct Acting | AJ011607 | 112 |
| AJ012603UTR#1_at | | 1 | | Direct Acting | AJ012603 | 113 |
| AJ222724_at | | 1 | | Direct Acting | AJ222724 | 114 |
| AJ224120_at | | 1 | | Direct Acting | AJ224120 | 115 |
| D00636cds_s_at | | -1 | | Direct Acting | D00636 | 116 |
| D00636Poly_A_Site#1_s_at | | -1 | | Direct Acting | D00636 | 117 |
| D00698_s_at | | - 1 | | Direct Acting | D00698 | 118 |
| D10354_s_at | | 1 | | Direct Acting | D10354 | 119 |
| D10587_g_at | | 1 | | Direct Acting | D10587 | 120 |
| D10756_g_at | | 1 | | Direct Acting | D10756 | 121 |
| D12769_at | | 1 | | Direct Acting | D12769 | 122 |
| D 13122_f_at | | 1 | | Direct Acting | D13122 | 123 |
| D13623_at | | 1 | | Direct Acting | D13623 | 124 |
| D13623_g_at | | 1 | | Direct Acting | D13623 | 125 |
| D13667cds_s_at | | 1 | | Direct Acting | D13667 | 126 |
| D13907_at | | 1 | | Direct Acting | D13907 | 127 |
| D13978_s_at | | 1 | | Direct Acting | D13978 | 128 |
| D14014_at | | 1 | | Direct Acting | D14014 | 129 |
| D14425_s_at | | 1 | | Direct Acting | D14425 | 130 |
| D14564cds_s_at | | -1 | | Direct Acting | D14564 | 131 |
| D14987_f_at | | -1 | | Direct Acting | D14987 | 132 |
| D21800_g_at | | 1 | | Direct Acting | D21800 | 133 |
| D25224_at | | 1 | | Direct Acting | D25224 | 134 |
| D25224_g_at | | 1 | | Direct Acting | D25224 | 135 |
| D26564_at | | 1 | | Direct Acting | D26564 | 136 |
| D28557_s_at | | 1 | | Direct Acting | D28557 | 137 |
| D28560_at | | -1 | | Direct Acting | D28560 | 138 |
| D28560_g_at | | - 1 | | Direct Acting | D28560 | 139 |
| D30649mRNA_s_at | | -1 | | Direct Acting | D30649 | 140 |
| D30666_at | | -1 | | Direct Acting | D30666 | 141 |
| D30804_at | | 1 | | Direct Acting | D30804 | 142 |
| D30804_g_at | | 1 | | Direct Acting | D30804 | 143 |
| D31662exon#4_s_at | | -1 | | Direct Acting | D31662 | 144 |
| D31874_at | | 1 | | Direct Acting | D31874 | 145 |
| D38061exon_s_at | | 1 | | Direct Acting | D38061 | 146 |
| D38062exon_s_at | | 1 | | Direct Acting | D38062 | 147 |
| D38381_s_at | | -1 | | Direct Acting | D38381 | 148 |
| D38468_s_at | | 1 | | Direct Acting | D38468 | 149 |
| D43964_at | | -1 | | Direct Acting | D43964 | 150 |
| D45247_at | | 1 | | Direct Acting | D45247 | 151 |
| D50694_at | | 1 | | Direct Acting | D50694 | 152 |
| D63704_at | | -1 | | Direct Acting | D63704 | 153 |
| D63704_g_at | | -1 | | Direct Acting | D63704 | 154 |
| D82928_at | | 1 | | Direct Acting | D82928 | 155 |
| D85435_at | | 1 | | Direct Acting | D85435 | 156 |
| D85435_g_at | | 1 | | Direct Acting | D85435 | 157 |
| D87839_g_at | | -1 | | Direct Acting | D87839 | 158 |
| D87991_at | | 1 | | Direct Acting | D87991 | 159 |
| D88034_at | | 1 | | Direct Acting | D88034 | 160 |
| D88890_at | | 1 | | Direct Acting | D88890 | 161 |
| D89069_f_at | | 1 | | Direct Acting | D89069 | 162 |
| D89514_at | | 1 | | Direct Acting | D89514 | 163 |
| D89983_at | | 1 | | Direct Acting | D89983 | 164 |
| D90109_at | | -1 | | Direct Acting | D90109 | 165 |
| D90265_s_at | | 1 | | Direct Acting | D90265 | 166 |
| E12286cds_at | | -1 | | Direct Acting | E12286 | 167 |
| E12625cds_at | | -1 | | Direct Acting | E12625 | 168 |
| J02589mRNA#2_at | | -1 | | Direct Acting | J02589 | 169 |
| J02646_at | | 1 | | Direct Acting | J02646 | 170 |
| J02679_s_at | | 1 | | Direct Acting | J02679 | 171 |
| J02962_at | | 1 | | Direct Acting | J02962 | 172 |
| J03179_g_at | | 1 | | Direct Acting | J03179 | 173 |
| J03572_i_at | | 1 | | Direct Acting | J03572 | 174 |
| J03969_at | | 1 | | Direct Acting | J03969 | 175 |
| J04187_at | | -1 | | Direct Acting | J04187 | 176 |
| J04791_s_at | | 1 | | Direct Acting | J04791 | 177 |
| J04943_at | | 1 | | Direct Acting | J04943 | 178 |
| J05035_g_at | | -1 | | Direct Acting | J05035 | 179 |
| J05122_at | | 1 | | Direct Acting | J05122 | 180 |
| J05166_at | | 1 | | Direct Acting | J05166 | 181 |
| J05210_at | | -1 | | Direct Acting | J05210 | 182 |
| J05210_g_at | | -1 | | Direct Acting | J05210 | 183 |
| K01934mRNA#2_at | | -1 | | Direct Acting | K01934 | 184 |
| K03045cds_r_at | | 1 | | Direct Acting | K03045 | 185 |
| K03249_at | | -1 | | Direct Acting | K03249 | 186 |
| L01267_at | | 1 | | Direct Acting | L01267 | 187 |
| L03294_g_at | | 1 | | Direct Acting | L03294 | 188 |
| L07114_at | | -1 | | Direct Acting | L07114 | 189 |
| L07407_at | | 1 | | Direct Acting | L07407 | 190 |
| L08505_at | | 1 | | Direct Acting | L08505 | 191 |
| L12025_at | | 1 | | Direct Acting | L12025 | 192 |
| L12382_at | | -1 | | Direct Acting | L12382 | 193 |
| L12383_at | | 1 | | Direct Acting | L12383 | 194 |
| L13235UTR#1_f_at | | -1 | | Direct Acting | L13235 | 195 |
| L13600_at | | 1 | | Direct Acting | L13600 | 196 |
| L13635_s_at | | 1 | | Direct Acting | L13635 | 197 |
| L17127_g_at | | 1 | | Direct Acting | L17127 | 198 |
| L19031_at | | -1 | | Direct Acting | L19031 | 199 |
| L19931_at | | 1 | | Direct Acting | L19931 | 200 |
| L19998_at | | -1 | | Direct Acting | L19998 | 201 |
| L20900_at | | 1 | | Direct Acting | L20900 | 202 |
| L22294_at | | -1 | | Direct Acting | L22294 | 203 |
| L22339_at | | - 1 | | Direct Acting | L22339 | 204 |
| L22339_g_at | | -1 | | Direct Acting | L22339 | 205 |
| L23148_g_at | | 1 | | Direct Acting | L23148 | 206 |
| L24207_r_at | | - 1 | | Direct Acting | L24207 | 207 |
| L27075_g_at | | -1 | | Direct Acting | L27075 | 208 |
| L27843_s_at | | 1 | | Direct Acting | L27843 | 209 |
| L32591mRNA_at | | 1 | | Direct Acting | L32591 | 210 |
| L32591mRNA_g_at | | 1 | | Direct Acting | L32591 | 211 |
| L32601_s_at | | -1 | | Direct Acting | L32601 | 212 |
| L34049_g_at | | -1 | | Direct Acting | L34049 | 213 |
| L38482_g_at | | 1 | | Direct Acting | L38482 | 214 |
| L38615_g_at | | 1 | | Direct Acting | L38615 | 215 |
| L41275cds_s_at | | 1 | | Direct Acting | L41275 | 216 |
| L41685mRNA_at | | 1 | | Direct Acting | L41685 | 217 |
| M11266_at | | -1 | | Direct Acting | M11266 | 218 |
| M11942_s_at | | 1 | | Direct Acting | M11942 | 219 |
| M12156_at | | 1 | | Direct Acting | M12156 | 220 |
| M12919mRNA#2_at | | 1 | | Direct Acting | M12919 | 221 |
| M12919mRNA#2_g_ at | | 1 | | Direct Acting | M12919 | 222 |
| Ml3l00cds#3 f at | | -1 | | Direct Acting | M13100 | 223 |
| M13100cds#4_{_}f_{_}at | | -1 | | Direct Acting | M13100 | 224 |
| M13962mRNA#2_{_}at | | -1 | | Direct Acting | M13962 | 225 |
| M14972_{_}i_{_}at | | 1 | | Direct Acting | M14972 | 226 |
| M15883_{_}g_{_}at | | 1 | | Direct Acting | M15883 | 227 |
| M18363cds_{_}s_{_}at | | -1 | | Direct Acting | M18363 | 228 |
| M21842_{_}at | | -1 | | Direct Acting | M21842 | 229 |
| M22359mRNA_{_}s_{_}at | | -1 | | Direct Acting | M22359 | 230 |
| M22360_{_}s_{_}at | | - 1 | | Direct Acting | M22360 | 231 |
| M23601_{_}at | | -1 | | Direct Acting | M23601 | 232 |
| M24067_at | | 1 | | Direct Acting | M24067 | 233 |
| M24604_{_}at | | 1 | | Direct Acting | M24604 | 234 |
| M24604_g_at | | 1 | | Direct Acting | M24604 | 235 |
| M25157mRNA_{_}i_{_}at | | -1 | | Direct Acting | M25157 | 236 |
| M25490_{_}at | | - 1 | | Direct Acting | M25490 | 237 |
| M25804_{_}at | | 1 | | Direct Acting | M25804 | 238 |
| M25804_g_at | | 1 | | Direct Acting | M25804 | 239 |
| M27158cds_{_}at | | 1 | | Direct Acting | M27158 | 240 |
| M27207mRNA_{_}s_{_}at | | -1 | | Direct Acting | M27207 | 241 |
| M29249cds_{_}at | | 1 | | Direct Acting | M29249 | 242 |
| M31837_at | | -1 | | DirectActing | M31837 | 243 |
| M32062_{_}at | | 1 | | Direct Acting | M32062 | 244 |
| M32062_g_at | | 1 | | Direct Acting | M32062 | 245 |
| M33962_at | | 1 | | Direct Acting | M33962 | 246 |
| M36151cds_{_}s_{_}at | | 1 | | Direct Acting | M36151 | 247 |
| M37828_at | | -1 | | Direct Acting | M37828 | 248 |
| M55015cds_s_at | | 1 | | Direct Acting | M55015 | 249 |
| M57728_at | | 1 | | Direct Acting | M57728 | 250 |
| M58041_s_at | | -1 | | Direct Acting | M58041 | 251 |
| M59460mRNA#2_at | | -1 | | Direct Acting | M59460 | 252 |
| M60103_at | | -1 | | Direct Acting | M60103 | 253 |
| M61219_s_at | | 1 | | Direct Acting | M61219 | 254 |
| M63282_at | | 1 | | Direct Acting | M63282 | 255 |
| M64795_f_at | | 1 | | Direct Acting | M64795 | 256 |
| M64862_at | | -1 | | Direct Acting | M64862 | 257 |
| M69246_at | | -1 | | Direct Acting | M69246 | 258 |
| M73808mRNA_at | | 1 | | Direct Acting | M73808 | 259 |
| M75168_at | | 1 | | Direct Acting | M75168 | 260 |
| M76767_s_at | | -1 | | Direct Acting | M76767 | 261 |
| M77245_at | | 1 | | Direct Acting | M77245 | 262 |
| M77479_at | | -1 | | Direct Acting | M77479 | 263 |
| M81183Exon_UTR_g_at | | -1 | | Direct Acting | M81183 | 264 |
| M81855_at | | 1 | | Direct Acting | M81855 | 265 |
| M81920_at | | 1 | | Direct Acting | M81920 | 266 |
| M83675_at | | -1 | | Direct Acting | M83675 | 267 |
| M84719_at | | -1 | | Direct Acting | M84719 | 268 |
| M89945mRNA_at | | - 1 | | Direct Acting | M89945 | 269 |
| M89945mRNA_g_at | | - 1 | | Direct Acting | M89945 | 270 |
| M91466_at | | -1 | | Direct Acting | M91466 | 271 |
| M91652complete_seq_at | | -1 | | Direct Acting | M91652 | 272 |
| M91652complete_seq_g_at | | -1 | | Direct Acting | M91652 | 273 |
| M93297cds_at | | -1 | | Direct Acting | M93297 | 274 |
| M93401_at | | -1 | | Direct Acting | M93401 | 275 |
| M94043_at | | -1 | | Direct Acting | M94043 | 276 |
| M94555_at | | 1 | | Direct Acting | M94555 | 277 |
| M95591_at | | -1 | | Direct Acting | M95591 | 278 |
| M95591_g_at | | -1 | | Direct Acting | M95591 | 279 |
| M96674_at | | - 1 | | Direct Acting | M96674 | 280 |
| rc_AA686164_at | | 1 | | Direct Acting | AA686164 | 281 |
| rc_AA799418_at | | 1 | | Direct Acting | AA799418 | 282 |
| rc_AA799479_at | | 1 | | Direct Acting | AA799479 | 283 |
| rc_AA799481_at | | 1 | | Direct Acting | AA799481 | 284 |
| rc_AA799508_at | | 1 | | Direct Acting | AA799508 | 285 |
| rc_AA799531_at | | 1 | | Direct Acting | AA799531 | 286 |
| rc_AA799531_g_at | | 1 | | Direct Acting | AA799531 | 287 |
| rc_AA799560_at | | -1 | | Direct Acting | AA799560 | 288 |
| rc_AA799672_s_at | | 1 | | Direct Acting | AA799672 | 289 |
| rc_AA799735_at | | 1 | | Direct Acting | AA799735 | 290 |
| rc_AA799788_s_at | | 1 | | Direct Acting | AA799788 | 291 |
| rc_AA799814_at | | 1 | | Direct Acting | AA799814 | 292 |
| rc_AA799893_g_at | | 1 | | Direct Acting | AA799893 | 293 |
| rc_AA799997_at | | -1 | | Direct Acting | AA799997 | 294 |
| rc_AA800017_at | | 1 | | Direct Acting | AA800017 | 295 |
| rc_AA800169_at | | 1 | | Direct Acting | AA800169 | 296 |
| rc_AA800179_at | | 1 | | Direct Acting | AA800179 | 297 |
| rc_AA800218_at | | 1 | | Direct Acting | AA800218 | 298 |
| rc_AA800456_at | | -1 | | Direct Acting | AA800456 | 299 |
| rc_AA800738_at | | 1 | | Direct Acting | AA800738 | 300 |
| rc_AA800739_at | | 1 | | Direct Acting | AA800739 | 301 |
| rc_AA800750_f_at | | -1 | | Direct Acting | AA800750 | 302 |
| rc_AA800753_at | | 1 | | Direct Acting | AA800753 | 303 |
| rc_AA800797_at | | -1 | | Direct Acting | AA800797 | 304 |
| rc_AA800912_g_at | | 1 | | Direct Acting | AA800912 | 305 |
| rc_AA817846_at | | -1 | | Direct Acting | AA817846 | 306 |
| rc_AA817854_s_at | | -1 | | Direct Acting | AA817854 | 307 |
| rc_AA817987_f_at | | -1 | | Direct Acting | AA817987 | 308 |
| rc_AA818072_s_at | | 1 | | Direct Acting | AA818072 | 309 |
| rc_AA818122_f_at | | -1 | | Direct Acting | AA818122 | 310 |
| rc_AA818951_at | | 1 | | Direct Acting | AA818951 | 311 |
| rc_AA819776_f_at | | 1 | | Direct Acting | AA819776 | 312 |
| rc_AA849722_at | | 1 | | Direct Acting | AA849722 | 313 |
| rc_AA852004_s_at | | -1 | | Direct Acting | AA852004 | 314 |
| rc_AA858879_at | | 1 | | Direct Acting | AA858879 | 315 |
| rc_AA859648_at | | 1 | | Direct Acting | AA859648 | 316 |
| rc_AA859652_at | | 1 | | Direct Acting | AA859652 | 317 |
| rc_AA859663_at | | -1 | | Direct Acting | AA859663 | 318 |
| rc_AA859680_at | | 1 | | Direct Acting | AA859680 | 319 |
| rc_AA859680_g_at | | 1 | | Direct Acting | AA859680 | 320 |
| rc_AA859722_at | | 1 | | Direct Acting | AA859722 | 321 |
| rc_AA859980_at | | -1 | | Direct Acting | AA859980 | 322 |
| rc_AA859980_g_at | | -1 | | Direct Acting | AA859980 | 323 |
| rc_AA860030_s_at | | 1 | | Direct Acting | AA860030 | 324 |
| rc_AA866264_s_at | | - 1 | | Direct Acting | AA866264 | 325 |
| rc_AA866426_at | | -1 | | Direct Acting | AA866426 | 326 |
| rc_AA874791_at | | 1 | | Direct Acting | AA874791 | 327 |
| rc_AA874802_s_at | | -1 | | Direct Acting | AA874802 | 328 |
| rc_AA874889_g_at | | 1 | | Direct Acting | AA874889 | 329 |
| rc_AA875054_at | | 1 | | Direct Acting | AA875054 | 330 |
| rc_AA875126_g_at | | 1 | | Direct Acting | AA875126 | 331 |
| rc_AA875205_at | | 1 | | Direct Acting | AA875205 | 332 |
| rc_AA875205_g_at | | 1 | | Direct Acting | AA875205 | 333 |
| rc_AA875511_at | | -1 | | Direct Acting | AA875511 | 334 |
| rc_AA875531_s_at | | -1 | | Direct Acting | AA875531 | 335 |
| rc_AA875537_at | | 1 | | Direct Acting | AA875537 | 336 |
| rc_AA875563_at | | 1 | | Direct Acting | AA875563 | 337 |
| rc_AA875620_g_at | | 1 | | Direct Acting | AA875620 | 338 |
| rc_AA891226_s_at | | 1 | | Direct Acting | AA891226 | 339 |
| rc_AA891553_at | | 1 | | Direct Acting | AA891553 | 340 |
| rc_AA891689_at | | 1 | | Direct Acting | AA891689 | 341 |
| rc_AA891689_g_at | | 1 | | Direct Acting | AA891689 | 342 |
| rc_AA891739_at | | -1 | | Direct Acting | AA891739 | 343 |
| rc_AA891785_at | | 1 | | Direct Acting | AA891785 | 344 |
| rc_AA891790_at | | 1 | | Direct Acting | AA891790 | 345 |
| rc_AA891829_at | | 1 | | Direct Acting | AA891829 | 346 |
| rc_AA891838_at | | 1 | | Direct Acting | AA891838 | 347 |
| rc_AA891998_i_at | | 1 | | Direct Acting | AA891998 | 348 |
| rc_AA892006_at | | 1 | | Direct Acting | AA892006 | 349 |
| rc_AA892010_g_at | | 1 | | Direct Acting | AA892010 | 350 |
| rc_AA892014_r_at | | 1 | | Direct Acting | AA892014 | 351 |
| rc_AA892027_at | | -1 | | Direct Acting | AA892027 | 352 |
| rc_AA892053_at | | 1 | | Direct Acting | AA892053 | 353 |
| rc_AA892120_at | | 1 | | Direct Acting | AA892120 | 354 |
| rc_AA892154_g_at | | -1 | | Direct Acting | AA892154 | 355 |
| rc_AA892248_g_at | | -1 | | Direct Acting | AA892248 | 356 |
| rc_AA892251_at | | -1 | | Direct Acting | AA892251 | 357 |
| rc_AA892333_at | | 1 | | Direct Acting | AA892333 | 358 |
| rc_AA892367_i_at | | 1 | | Direct Acting | AA892367 | 359 |
| rc_AA892378_at | | 1 | | Direct Acting | AA892378 | 360 |
| rc_AA892500_at | | -1 | | Direct Acting | AA892500 | 361 |
| rc_AA892562_at | | 1 | | Direct Acting | AA892562 | 362 |
| rc_AA892562_g_at | | 1 | | Direct Acting | AA892562 | 363 |
| rc_AA892582_s_at | | 1 | | Direct Acting | AA892582 | 364 |
| rc_AA892598_at | | 1 | | Direct Acting | AA892598 | 365 |
| rc_AA892598_g_at | | 1 | | Direct Acting | AA892598 | 366 |
| rc_AA892602_at | | 1 | | Direct Acting | AA892602 | 367 |
| rc_AA892680_at | | 1 | | Direct Acting | AA892680 | 368 |
| rc_AA892799_i_at | | 1 | | Direct Acting | AA892799 | 369 |
| rc_AA892799_r_at | | -1 | | Direct Acting | AA892799 | 370 |
| rc_AA892828_at | | -1 | | Direct Acting | AA892828 | 371 |
| rc_AA892828_g_at | | -1 | | Direct Acting | AA892828 | 372 |
| rc_AA892832_at | | -1 | | Direct Acting | AA892832 | 373 |
| rc_AA892855_at | | -1 | | Direct Acting | AA892855 | 374 |
| rc_AA892861_at | | -1 | | Direct Acting | AA892861 | 375 |
| rc_AA892950_at | | 1 | | Direct Acting | AA892950 | 376 |
| rc_AA892986_at | | -1 | | Direct Acting | AA892986 | 377 |
| rc_AA893032_at | | -1 | | Direct Acting | AA893032 | 378 |
| rc_AA893199_at | | 1 | | Direct Acting | AA893199 | 379 |
| rc_AA893235_at | | 1 | | Direct Acting | AA893235 | 380 |
| rc_AA893239_at | | -1 | | Direct Acting | AA893239 | 381 |
| rc_AA893242_g_at | | -1 | | Direct Acting | AA893242 | 382 |
| rc_AA893280_at | | 1 | | Direct Acting | AA893280 | 383 |
| rc_AA893325_at | | -1 | | Direct Acting | AA893325 | 384 |
| rc_AA893366_at | | -1 | | Direct Acting | AA893366 | 385 |
| rc_AA893384_g_at | | -1 | | Direct Acting | AA893384 | 386 |
| rc_AA893471_s_at | | -1 | | Direct Acting | AA893471 | 387 |
| rc_AA893495_at | | -1 | | Direct Acting | AA893495 | 388 |
| rc_AA893517_at | | 1 | | Direct Acting | AA893517 | 389 |
| rc_AA893532_at | | 1 | | Direct Acting | AA893532 | 390 |
| rc_AA893562_at | | 1 | | Direct Acting | AA893562 | 391 |
| rc_AA893584_at | | 1 | | Direct Acting | AA893584 | 392 |
| rc_AA893690_at | | 1 | | Direct Acting | AA893690 | 393 |
| rc_AA893770_g_at | | 1 | | Direct Acting | AA893770 | 394 |
| rc_AA894027_at | | -1 | | Direct Acting | AA894027 | 395 |
| rc_AA894086_g_at | | 1 | | Direct Acting | AA894086 | 396 |
| rc_AA894258_at | | -1 | | Direct Acting | AA894258 | 397 |
| rc_AA894298_s_at | | 1 | | Direct Acting | AA894298 | 398 |
| rc_AA900476_at | | -1 | | Direct Acting | AA900476 | 399 |
| rc_AA924267_s_at | | 1 | | Direct Acting | AA924267 | 400 |
| rc_AA924289_s_at | | -1 | | Direct Acting | AA924289 | 401 |
| rc_AA924326_s_at | | 1 | | Direct Acting | AA924326 | 402 |
| rc_AA926193_at | | -1 | | Direct Acting | AA926193 | 403 |
| rc_AA944156_s_at | | 1 | | Direct Acting | AA944156 | 404 |
| rc_AA944397_at | | 1 | | Direct Acting | AA944397 | 405 |
| rc_AA945082_at | | 1 | | Direct Acting | AA945082 | 406 |
| rc_AA945867_at | | 1 | | Direct Acting | AA945867 | 407 |
| rc_AA946532_at | | -1 | | Direct Acting | AA946532 | 408 |
| rc_AA956958_at | | 1 | | Direct Acting | AA956958 | 409 |
| rc_AA963449_s_at | | -1 | | Direct Acting | AA963449 | 410 |
| rc_AA963839_s_at | | -1 | | Direct Acting | AA963839 | 411 |
| rc_AA965147_at | | 1 | | Direct Acting | AA965147 | 412 |
| rc_AA997614_s_at | | -1 | | Direct Acting | AA997614 | 413 |
| rc_AI008074_s_at | | 1 | | Direct Acting | AI008074 | 414 |
| rc_AI008131_s_at | | 1 | | Direct Acting | AI008131 | 415 |
| rc_AI009338_at | | -1 | | Direct Acting | AI009338 | 416 |
| rc_AI009806_at | | 1 | | Direct Acting | AI009806 | 417 |
| rc_AI011998_at | | 1 | | Direct Acting | AI011998 | 418 |
| rc_AI012595_at | | 1 | | Direct Acting | AI012595 | 419 |
| rc_AI012604_at | | 1 | | Direct Acting | AI012604 | 420 |
| rc_AI013513_at | | 1 | | Direct Acting | AI013513 | 421 |
| rc_AI014091_at | | -1 | | Direct Acting | AI014091 | 422 |
| rc_AI014163_at | | 1 | | Direct Acting | AI014163 | 423 |
| rc_AI031019_g_at | | 1 | | Direct Acting | AI031019 | 424 |
| rc_AI044900_s_at | | -1 | | Direct Acting | AI044900 | 425 |
| rc_AI044985_g_at | | - 1 | | Direct Acting | AI044985 | 426 |
| rc_AI045395_at | | -1 | | Direct Acting | AI045395 | 427 |
| rc_AI070295_at | | 1 | | Direct Acting | AI070295 | 428 |
| rc_AI070295_g_at | | 1 | | Direct Acting | AI070295 | 429 |
| rc_AI102103_g_at | | 1 | | Direct Acting | AI102103 | 430 |
| rc_AI105348_f_at | | 1 | | Direct Acting | AI105348 | 431 |
| rc_AI105348_i_at | | 1 | | Direct Acting | AI105348 | 432 |
| rc_AI111401_s_at | | 1 | | Direct Acting | AI111401 | 433 |
| rc_AI137790_at | | 1 | | Direct Acting | AI137790 | 434 |
| rc_AI169695_f_at | | -1 | | Direct Acting | AI169695 | 435 |
| rc_AI169735_g_at | | -1 | | Direct Acting | AI169735 | 436 |
| rc_AI170608_at | | 1 | | Direct Acting | AI170608 | 437 |
| rc_AI171966_at | | 1 | | Direct Acting | AI171966 | 438 |
| rc_AI172476_at | | 1 | | Direct Acting | AI172476 | 439 |
| rc_AI175486_at | | 1 | | Direct Acting | AI175486 | 440 |
| rc_AI175959_at | | 1 | | Direct Acting | AI175959 | 441 |
| rc_AI176488_at | | -1 | | Direct Acting | AI176488 | 442 |
| rc_{_}AI176595_{_}s_{_}at | | 1 | | Direct Acting | All 76595 | 443 |
| rc_{_}AI177161_{_}at | | -1 | | Direct Acting | AI177161 | 444 |
| rc_AI177161_g_at | | -1 | | Direct Acting | AI177161 | 445 |
| rc_AI177986_at | | 1 | | Direct Acting | AI177986 | 446 |
| rc_AI178135_at | | 1 | | Direct Acting | AI178135 | 447 |
| rc_AI178828_i_at | | 1 | | Direct Acting | AI178828 | 448 |
| rc_AI 179610_at | | 1 | | Direct Acting | AI179610 | 449 |
| rc_AI180442_at | | -1 | | Direct Acting | AI180442 | 450 |
| rc_AI228738_s_at | | 1 | | Direct Acting | AI228738 | 451 |
| rc_Al229637_at | | 1 | | Direct Acting | AI229637 | 452 |
| rc_AI230260_s_at | | 1 | | Direct Acting | AI230260 | 453 |
| rc_AI230294_at | | -1 | | Direct Acting | AI230294 | 454 |
| rc_AI230614_s_at | | 1 | | Direct Acting | AI230614 | 455 |
| rc_Al230712_at | | 1 | | Direct Acting | AI230712 | 456 |
| rc_AI231007_at | | 1 | | Direct Acting | AI231007 | 457 |
| rc_AI231807_g_at | | 1 | | Direct Acting | AI231807 | 458 |
| rc_AI232783_s_at | | -1 | | Direct Acting | AI232783 | 459 |
| rc_AI234604_s_at | | 1 | | Direct Acting | AI234604 | 460 |
| rc_AI235631_at | | 1 | | Direct Acting | AI235631 | 461 |
| rc_AI235890_s_at | | -1 | | Direct Acting | AI235890 | 462 |
| rc_AI236597_at | | 1 | | Direct Acting | AI236597 | 463 |
| rc_AI236601_at | | 1 | | Direct Acting | AI236601 | 464 |
| rc_AI237535_s_at | | 1 | | Direct Acting | AI237535 | 465 |
| rc_AI638948_at | | -1 | | Direct Acting | AI638948 | 466 |
| rc_AI638966_r_at | | -1 | | Direct Acting | AI638966 | 467 |
| rc_A1639008_at | | 1 | | Direct Acting | AI639008 | 468 |
| rc_AI639029_s_at | | 1 | | Direct Acting | AI639029 | 469 |
| rc_AI639067_at | | - 1 | | Direct Acting | AI639067 | 470 |
| rc_AI639167_at | | 1 | | Direct Acting | AI639167 | 471 |
| rc_AI639185_s_at | | -1 | | Direct Acting | AI639185 | 472 |
| rc_AI639393_at | | 1 | | Direct Acting | AI639393 | 473 |
| rc_AI639488_at | | 1 | | Direct Acting | AI639488 | 474 |
| rc_AI639518_g_at | | 1 | | Direct Acting | AI639518 | 475 |
| rc_H31287_g_at | | 1 | | Direct Acting | H31287 | 476 |
| rc_H31351_at | | 1 | | Direct Acting | H31351 | 477 |
| rc_H31722_at | | 1 | | Direct Acting | H31722 | 478 |
| rc_H31976_at | | 1 | | Direct Acting | H31976 | 479 |
| rc_H31982_at | | 1 | | Direct Acting | H31982 | 480 |
| rc_H33426_at | | -1 | | Direct Acting | H33426 | 481 |
| rc_H33426_g_at | | -1 | | Direct Acting | H33426 | 482 |
| rc_H33491_at | | -1 | | Direct Acting | H33491 | 483 |
| 546785_at | | -1 | | Direct Acting | S46785 | 484 |
| S46785_g_at | | -1 | | Direct Acting | S46785 | 485 |
| S55224_s_at | | 1 | | Direct Acting | S55224 | 486 |
| S61868_g_at | | 1 | | Direct Acting | S61868 | 487 |
| 566024_at | | 1 | | Direct Acting | 566024 | 488 |
| S69874_s_at | | 1 | | Direct Acting | S69874 | 489 |
| S71021_s_at | | 1 | | Direct Acting | S71021 | 490 |
| S72506_s_at | | 1 | | Direct Acting | S72506 | 491 |
| S76054_s_at | | 1 | | Direct Acting | S76054 | 492 |
| S76489_s_at | | -1 | | Direct Acting | S76489 | 493 |
| 579213_at | | 1 | | Direct Acting | S79213 | 494 |
| 579820_at | | 1 | | Direct Acting | S79820 | 495 |
| S80456_s_at | | 1 | | Direct Acting | S80456 | 496 |
| S82820mRNA_s_at | | 1 | | Direct Acting | S82820 | 497 |
| 585184_at | | 1 | | Direct Acting | S85184 | 498 |
| S85184_g_at | | 1 | | Direct Acting | 585184 | 499 |
| U01146_s_at | | 1 | | Direct Acting | U01146 | 500 |
| U01344_at | | -1 | | Direct Acting | U01344 | 501 |
| U03390_at | | 1 | | Direct Acting | U03390 | 502 |
| U05014_g at | | 1 | | Direct Acting | U05014 | 503 |
| U05784_s_at | | 1 | | Direct Acting | U05784 | 504 |
| U07201_at | | 1 | | Direct Acting | U07201 | 505 |
| U08141_at | | -1 | | Direct Acting | U08141 | 506 |
| U12268_at | | -1 | | Direct Acting | U12268 | 507 |
| U14746_at | | 1 | | Direct Acting | U14746 | 508 |
| U17035_s_at | | 1 | | Direct Acting | U17035 | 509 |
| U17697_s_at | | -1 | | Direct Acting | U17697 | 510 |
| U18729_at | | 1 | | Direct Acting | U18729 | 511 |
| U21101_at | | -1 | | Direct Acting | U21101 | 512 |
| U21719mRNA_s_at | | 1 | | Direct Acting | U21719 | 513 |
| U21871_at | | 1 | | Direct Acting | U21871 | 514 |
| U24174_at | | 1 | | Direct Acting | U24174 | 515 |
| U28504_at | | -1 | | Direct Acting | U28504 | 516 |
| U29873_at | | -1 | | Direct Acting | U29873 | 517 |
| U30186_at | | 1 | | Direct Acting | U30186 | 518 |
| U31777_g_at | | 1 | | Direct Acting | U31777 | 519 |
| U31866_at | | -1 | | Direct Acting | U31866 | 520 |
| U33500_g_at | | 1 | | Direct Acting | U33500 | 521 |
| U33541cds_at | | -1 | | Direct Acting | U33541 | 522 |
| U36482_g_at | | - 1 | | Direct Acting | U36482 | 523 |
| U38253_at | | 1 | | Direct Acting | U38253 | 524 |
| U38253_g_at | | 1 | | Direct Acting | U38253 | 525 |
| U40004_s_at | | -1 | | Direct Acting | U40004 | 526 |
| U44948_at | | 1 | | Direct Acting | U44948 | 527 |
| U50412_at | | -1 | | Direct Acting | U50412 | 528 |
| U52530_s_at | | -1 | | Direct Acting | U52530 | 529 |
| U53873cds_at | | -1 | | Direct Acting | U53873 | 530 |
| U55815_at | | 1 | | Direct Acting | U55815 | 531 |
| U60416_at | | 1 | | Direct Acting | U60416 | 532 |
| U60882_at | | 1 | | Direct Acting | U60882 | 533 |
| U63923_at | | 1 | | Direct Acting | U63923 | 534 |
| U64705cds_f_at | | 1 | | Direct Acting | U64705 | 535 |
| U66322_at | | - 1 | | Direct Acting | U66322 | 536 |
| U67915_at | | - 1 | | Direct Acting | U67915 | 537 |
| U68168_at | | -1 | | Direct Acting | U68168 | 538 |
| U72349_at | | 1 | | Direct Acting | U72349 | 539 |
| U73174_at | | 1 | | Direct Acting | U73174 | 540 |
| U75210_s_at | | -1 | | Direct Acting | U75210 | 541 |
| U75405UTR#1_f_at | | -1 | | Direct Acting | U75405 | 542 |
| U75917_at | | 1 | | Direct Acting | U75917 | 543 |
| U76714_at | | 1 | | Direct Acting | U76714 | 544 |
| U77918_at | | 1 | | Direct Acting | U77918 | 545 |
| U83896_at | | 1 | | Direct Acting | U83896 | 546 |
| U84410_s_at | | -1 | | Direct Acting | U84410 | 547 |
| U88036_at | | -1 | | Direct Acting | U88036 | 548 |
| U91561_g_at | | 1 | | Direct Acting | U91561 | 549 |
| U96490_at | | 1 | | Direct Acting | U96490 | 550 |
| V01225mRNA_s_at | | -1 | | Direct Acting | V01225 | 551 |
| V01274_at | | -1 | | Direct Acting | V01274 | 552 |
| X02610_at | | 1 | | Direct Acting | X02610 | 553 |
| X02741_s_at | | 1 | | Direct Acting | X02741 | 554 |
| X04069_at | | -1 | | Direct Acting | X04069 | 555 |
| X04267_at | | 1 | | Direct Acting | X04267 | 556 |
| X05137_at | | -1 | | Direct Acting | X05137 | 557 |
| X05472cds#1_s_at | | - 1 | | Direct Acting | X05472 | 558 |
| X06423_g_at | | 1 | | Direct Acting | X06423 | 559 |
| X06801cds_f_at | | 1 | | Direct Acting | X06801 | 560 |
| X07259cds_s_at | | 1 | | Direct Acting | X07259 | 561 |
| X07551cds_s_at | | 1 | | Direct Acting | X07551 | 562 |
| X07686cds_s_at | | - 1 | | Direct Acting | X07686 | 563 |
| X07944exon#1-12_s_at | | 1 | | Direct Acting | X07944 | 564 |
| X08056cds_s_at | | -1 | | Direct Acting | X08056 | 565 |
| X12367cds_s_at | | -1 | | Direct Acting | X12367 | 566 |
| X13044_at | | 1 | | Direct Acting | X13044 | 567 |
| X13058_at | | 1 | | Direct Acting | X13058 | 568 |
| X13527cds_s_at | | -1 | | Direct Acting | X13527 | 569 |
| X14181cds_s_at | | 1 | | Direct Acting | X14181 | 570 |
| X14254cds_g_at | | 1 | | Direct Acting | X14254 | 571 |
| X15580complete_seq_s_at | | -1 | | Direct Acting | X15580 | 572 |
| X16038exon_s_at | | 1 | | Direct Acting | X16038 | 573 |
| X16043cds_at | | 1 | | Direct Acting | X16043 | 574 |
| X16044cds_s_at | | 1 | | Direct Acting | X16044 | 575 |
| X16554_at | | 1 | | Direct Acting | X16554 | 576 |
| X17053mRNA_s_at | | 1 | | Direct Acting | X17053 | 577 |
| X52619_at | | 1 | | Direct Acting | X52619 | 578 |
| X52815cds_f_at | | 1 | | Direct Acting | X52815 | 579 |
| X53581cds#3_f_at | | -1 | | Direct Acting | X53581 | 580 |
| X53588_at | | -1 | | Direct Acting | X53588 | 581 |
| X55286_at | | 1 | | Direct Acting | X55286 | 582 |
| X57432cds_s_at | | 1 | | Direct Acting | X57432 | 583 |
| X57523_at | | 1 | | Direct Acting | X57523 | 584 |
| X57523_g_at | | 1 | | Direct Acting | X57523 | 585 |
| X58465mRNA_at | | 1 | | Direct Acting | X58465 | 586 |
| X58465mRNA_g_at | | 1 | | Direct Acting | X58465 | 587 |
| X59859_i_at | | 1 | | Direct Acting | X59859 | 588 |
| X60212_i_at | | 1 | | Direct Acting | X60212 | 589 |
| X60769mRNA_at | | 1 | | Direct Acting | X60769 | 590 |
| X61296cds#2_f_at | | -1 | | Direct Acting | X61296 | 591 |
| X62086mRNA_s_at | | - 1 | | Direct Acting | X62086 | 592 |
| X62145cds_at | | 1 | | Direct Acting | X62145 | 593 |
| X62295cds_s_at | | - 1 | | Direct Acting | X62295 | 594 |
| X62875mRNA_g_at | | 1 | | Direct Acting | X62875 | 595 |
| X64052cds_f_at | | -1 | | Direct Acting | X64052 | 596 |
| X66870_at | | 1 | | Direct Acting | X66870 | 597 |
| X67788_at | | 1 | | Direct Acting | X67788 | 598 |
| X69903_at | | - 1 | | Direct Acting | X69903 | 599 |
| X70369_s_at | | - 1 | | Direct Acting | X70369 | 600 |
| X70871_at | | 1 | | Direct Acting | X70871 | 601 |
| X74565cds_g_at | | 1 | | Direct Acting | X74565 | 602 |
| X76453_at | | -1 | | Direct Acting | X76453 | 603 |
| X77235_at | | 1 | | Direct Acting | X77235 | 604 |
| X77932_at | | -1 | | Direct Acting | X77932 | 605 |
| X77934cds_at | | -1 | | Direct Acting | X77934 | 606 |
| X78327_at | | 1 | | Direct Acting | X78327 | 607 |
| X78997_at | | 1 | | Direct Acting | X78997 | 608 |
| X79081mRNA_f_at | | -1 | | Direct Acting | X79081 | 609 |
| X81448cds_at | | 1 | | Direct Acting | X81448 | 610 |
| X84210complete_seq_s_at | | -1 | | Direct Acting | X84210 | 611 |
| X89225cds_s_at | | 1 | | Direct Acting | X89225 | 612 |
| X95189_at | | -1 | | Direct Acting | X95189 | 613 |
| X95986mRNA#1_f at | | 1 | | Direct Acting | X95986 | 614 |
| X97772_at | | 1 | | Direct Acting | X97772 | 615 |
| X97772_g_at | | 1 | | Direct Acting | X97772 | 616 |
| Y00396mRNA_at | | 1 | | Direct Acting | Y00396 | 617 |
| Y00396mRNA_g_at | | 1 | | Direct Acting | Y00396 | 618 |
| Y08355cds#2_at | | 1 | | Direct Acting | Y08355 | 619 |
| Y09333_at | | 1 | | Direct Acting | Y09333 | 620 |
| Y09365cds_s_at | | 1 | | Direct Acting | Y09365 | 621 |
| Y12635_at | | 1 | | Direct Acting | Y12635 | 622 |
| Y14933mRNA_s_at | | 1 | | Direct Acting | Y14933 | 623 |
| Y17295cds_s_at | | 1 | | Direct Acting | Y17295 | 624 |
| Z36944cds_at | | 1 | | Direct Acting | Z36944 | 625 |
| Z83757mRNA_at | | -1 | | Direct Acting | Z83757 | 626 |
| Z83757mRNA_g_at | | -1 | | Direct Acting | Z83757 | 627 |
| J03863_at | 1 | | 1 | Cholestatic/ Steatotic | J03863 | 628 |
| J05460_s_at | 1 | | -1 | Cholestatic/ Steatotic | J05460 | 629 |
| X13119cds_s_at | 1 | | 1 | Cholestatic/ Steatotic | X13119 | 630 |
| AF020618_g_at | 1 | 1 | | Cholestatic/ Direct Acting | AF020618 | 631 |
| AF039832_at | 1 | 1 | | Cholestatic/ Direct Acting | AF039832 | 632 |
| AF086624_s_at | 1 | 1 | | Cholestatic/ Direct Acting | AF086624 | 633 |
| AF089825_at | -1 | - 1 | | Cholestatic/ Direct Acting | AF089825 | 634 |
| D12769_g_at | 1 | 1 | | Cholestatic/ Direct Acting | D12769 | 635 |
| D37920_at | -1 | -1 | | Cholestatic/ Direct Acting | D37920 | 636 |
| D86580_at | 1 | -1 | | Cholestatic/ Direct Acting | D86580 | 637 |
| J02722cds_at | 1 | 1 | | Cholestatic/ Direct Acting | J02722 | 638 |
| J04171_at | 1 | 1 | | Cholestatic/ Direct Acting | J04171 | 639 |
| K03041mRNA_s_at | 1 | -1 | | Cholestatic/ Direct Acting | K03041 | 640 |
| L37333_s_at | 1 | -1 | | Cholestatic/ Direct Acting | L37333 | 641 |
| M57507_at | -1 | -1 | | Cholestatic/ Direct Acting | M57507 | 642 |
| M60921_{_}at | 1 | 1 | | Cholestatic/ Direct Acting | M60921 | 643 |
| M96548_{_}at | 1 | 1 | | Cholestatic/ Direct Acting | M96548 | 644 |
| rc_{_}AA799861_{_}g_{_}at | -1 | 1 | | Cholestatic/ Direct Acting | AA799861 | 645 |
| rc_{_}AA800678_{_}g_{_}at | -1 | -1 | | Cholestatic/ Direct Acting | AA800678 | 646 |
| rc_{_}AA891944_{_}at | -1 | -1 | | Cholestatic/ Direct Acting | AA891944 | 647 |
| rc_{_}AA900505_{_}at | 1 | 1 | | Cholestatic/ Direct Acting | AA900505 | 648 |
| rc_{_}AI009098_{_}at | - 1 | 1 | | Cholestatic/ Direct Acting | AI009098 | 649 |
| rc_{_}AI112173_{_}at | 1 | 1 | | Cholestatic/ Direct Acting | AI112173 | 650 |
| rc_{_}H31707_{_}at | -1 | 1 | | Cholestatic/ Direct Acting | H31707 | 651 |
| 561868_at | 1 | 1 | | Cholestatic/ Direct Acting | S61868 | 652 |
| U14005exon#1_{_}s_{_}at | -1 | -1 | | Cholestatic/ Direct Acting | U14005 | 653 |
| U42627_{_}at | 1 | -1 | | Cholestatic/ Direct Acting | U42627 | 654 |
| X07266cds_{_}s_{_}at | 1 | 1 | | Cholestatic/ Direct Acting | X07266 | 655 |
| X63594cds_{_}at | 1 | 1 | | Cholestatic/ Direct Acting | X63594 | 656 |
| X96437mRNA_{_}g_{_}at | 1 | 1 | | Cholestatic/ | X96437 | 657 |
| | | | | Direct Acting | | |
| AF000942_at | -1 | | | Cholestatic | AF000942 | 658 |
| AF075382_at | 1 | | | Cholestatic | AF075382 | 659 |
| D00403_g_at | 1 | | | Cholestatic | D00403 | 660 |
| J03865mRNA_f at | 1 | | | Cholestatic | J03865 | 661 |
| K03243mRNA_s_at | 1 | | | Cholestatic | K03243 | 662 |
| L13619_at | 1 | | | Cholestatic | L13619 | 663 |
| L13619_g_at | 1 | | | Cholestatic | L13619 | 664 |
| M11794cds#2_f_at | -1 | 1 | 1 | Cholestatic | M11794 | 665 |
| M33962_g_at | 1 | | | Cholestatic | M33962 | 666 |
| M63122_at | -1 | 1 | 1 | Cholestatic | M63122 | 667 |
| rc_AA685221_at | -1 | | | Cholestatic | AA685221 | 668 |
| rc_AA800613_at | 1 | | | Cholestatic | AA800613 | 669 |
| rc_AA866383_at | 1 | | | Cholestatic | AA866383 | 670 |
| rc_AA893192_at | 1 | | | Cholestatic | AA893192 | 671 |
| rc_AA893602_at | -1 | | | Cholestatic | AA893602 | 672 |
| rc_AA946108_at | 1 | -1 | -1 | Cholestatic | AA946108 | 673 |
| rc_AI102562_at | -1 | 1 | 1 | Cholestatic | AI102562 | 674 |
| rc_AI176456_at | -1 | 1 | 1 | Cholestatic | AI176456 | 675 |
| rc_AI176662_s_at | 1 | | | Cholestatic | AI176662 | 676 |
| rc_AI639141_at | 1 | | | Cholestatic | AI639141 | 677 |
| rc_H31118_at | 1 | | | Cholestatic | H31118 | 678 |
| U15211_g_at | -1 | | | Cholestatic | U15211 | 679 |
| X63594cds_g_at | 1 | | | Cholestatic | X63594 | 680 |

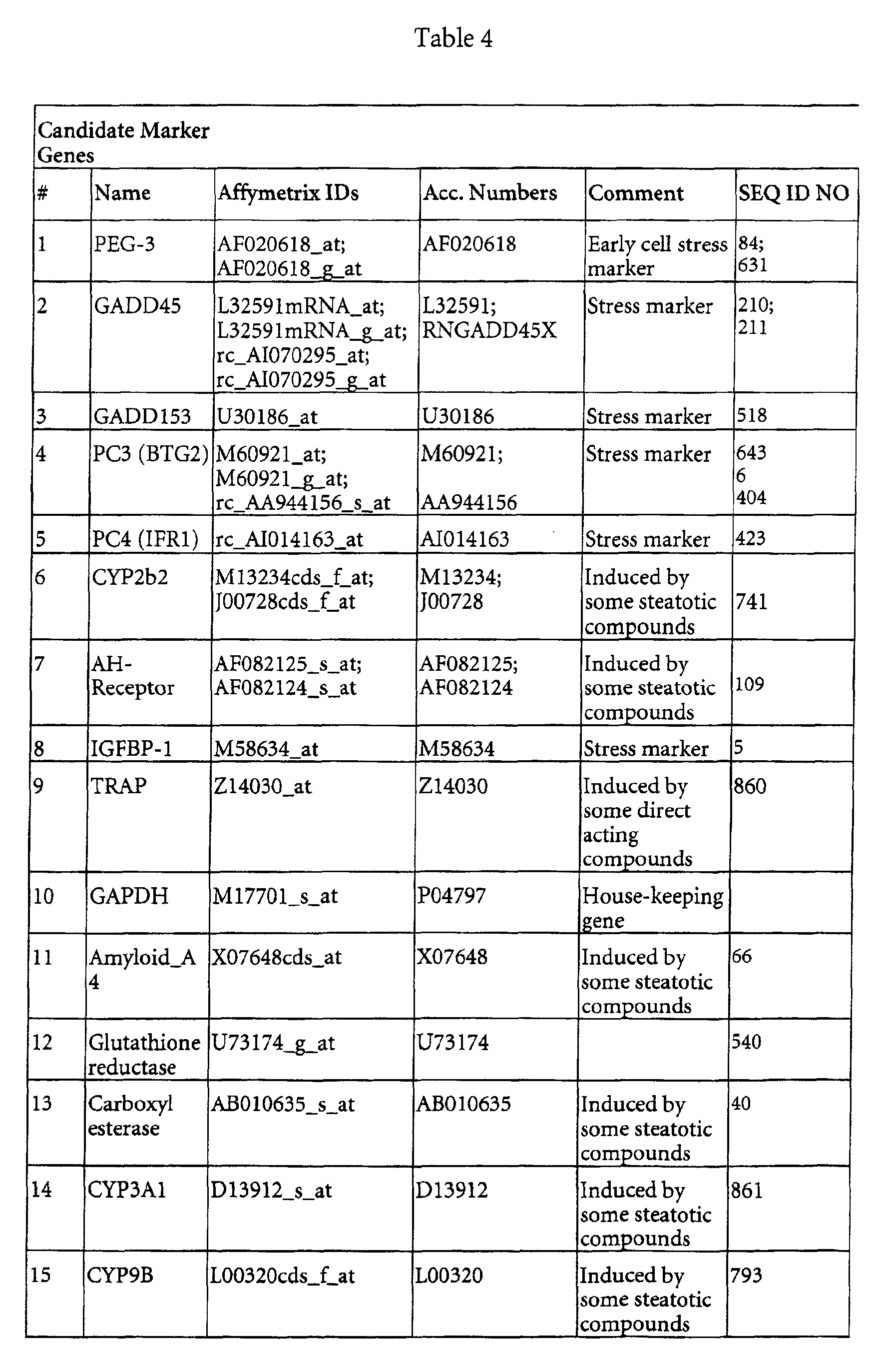

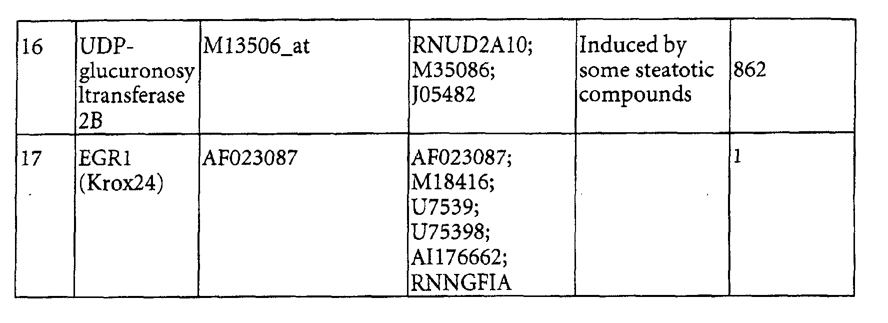

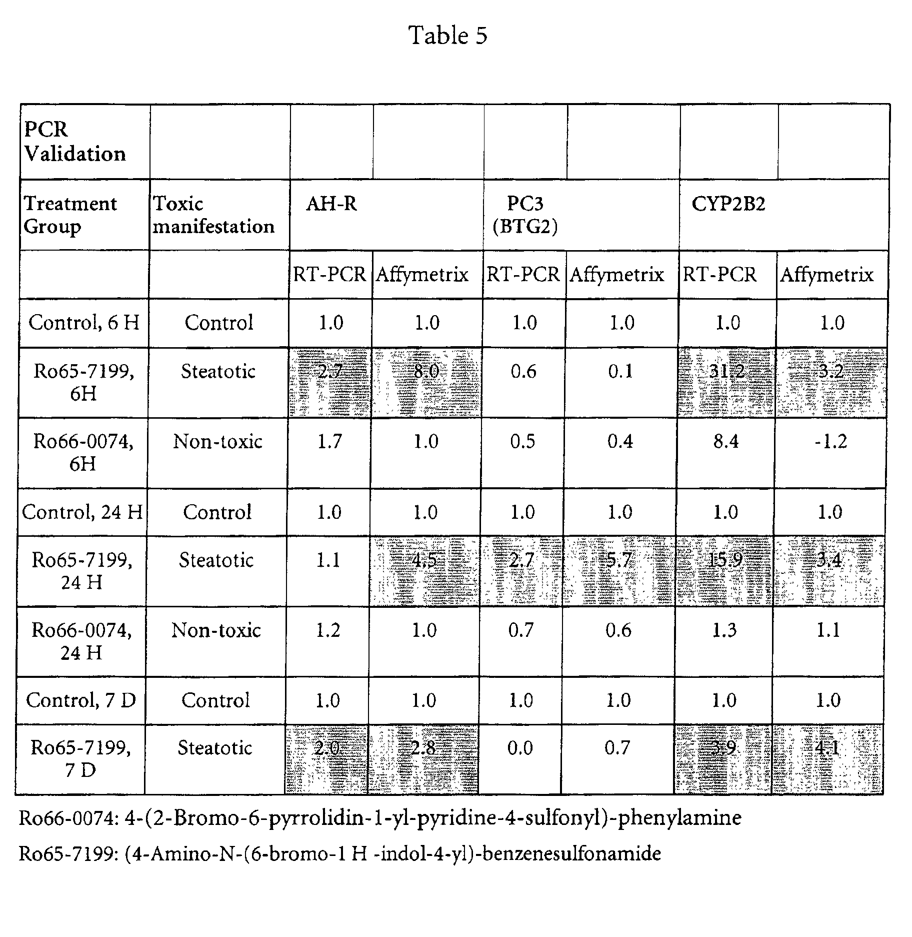

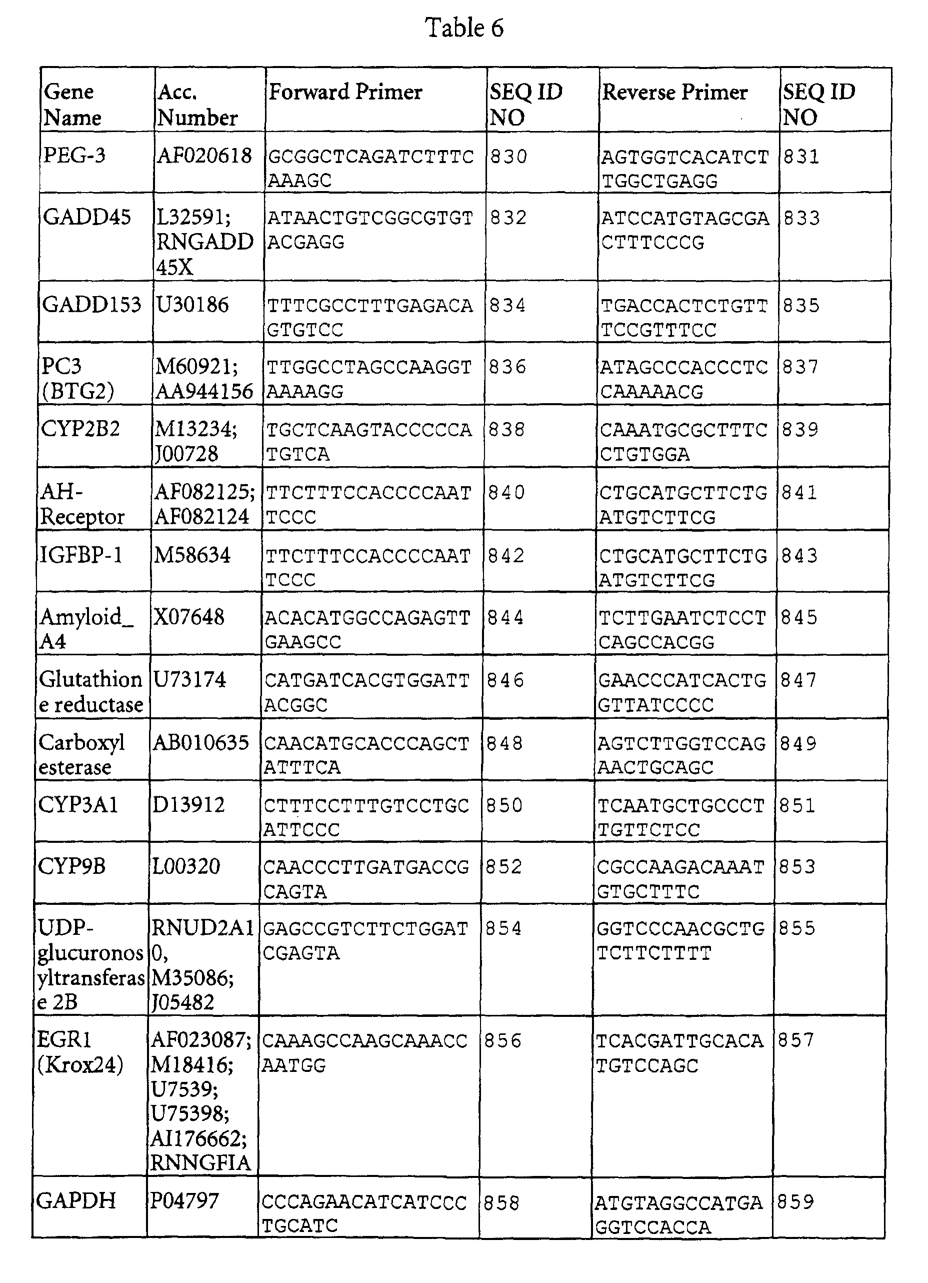

**Table 7**

| The accession numbers refer to GenBank. | | | |
|---|---|---|---|
| Affymetrix ID | Discrimination | Acc Number | SEQ ID NO |
| J03588_at | direct acting vs all other classes | J03588 | 681 |
| M13100cds#2_s_at | direct acting vs all other classes | M13100 | 682 |
| rc_AA800054_at | direct acting vs all other classes | AA800054 | 683 |
| rc_AI178750_at | direct acting vs all other classes | AI178750 | 684 |
| X53581cds#3_f_at | direct acting vs all other classes | X53581 | 685 |
| X58465mRNA_g_at | direct acting vs all other classes | X58465 | 686 |
| D78308_g_at | steatotic vs all other classes | D78308 | 687 |
| K00996mRNA_s_at | steatotic vs all other classes | K00996 | 688 |
| M94918mRNA_f_at | steatotic vs all other classes | M94918 | 689 |
| rc_AA892888_g_at | steatotic vs all other classes | AA892888 | 690 |
| rc_AA946503_at | steatotic vs all other classes | AA946503 | 691 |
| U88036_at | steatotic vs all other classes | U88036 | 692 |
| AF038870_at | cholestatic vs all other classes | AF038870 | 693 |
| AF076183_at | cholestatic vs all other classes | AF076183 | 694 |
| D00753_at | cholestatic vs all other classes | D00753 | 695 |
| J00738_s_at | cholestatic vs all other classes | J00738 | 696 |
| J03588_at | cholestatic vs all other classes | J03588 | 697 |
| K01932_f _at | cholestatic vs all other classes | K01932 | 698 |
| L27843_s_at | cholestatic vs all other classes | L27843 | 699 |
| M11670_at | cholestatic vs all other classes | M11670 | 700 |
| M15327_at | cholestatic vs all other classes | M15327 | 701 |
| rc_AA799899_i_at | cholestatic vs all other classes | AA799899 | 702 |
| rc_AA858673_at | cholestatic vs all other classes | AA858673 | 703 |
| rc_AA891220_at | cholestatic vs all other classes | AA891220 | 704 |
| rc_AA892333_at | cholestatic vs all other classes | AA892333 | 705 |
| rc_AA892775_at | cholestatic vs all other classes | AA892775 | 706 |
| rc_AA945143_at | cholestatic vs all other classes | AA945143 | 707 |
| rc_AA945321_at | cholestatic vs all other classes | AA945321 | 708 |
| rc_AI007820_s_at | cholestatic vs all other classes | AI007820 | 709 |
| rc_AI104524_s_at | cholestatic vs all other classes | AI104524 | 710 |
| rc_AI228674_s_at | cholestatic vs all other classes | AI228674 | 711 |
| rc_AI232087_at | cholestatic vs all other classes | AI232087 | 712 |
| X15734_at | cholestatic vs all other classes | X15734 | 713 |
| AB008424_s_at | non-toxic vs all other classes | AB008424 | 714 |
| AF045464_s_at | non-toxic vs all other classes | AF045464 | 715 |
| D78308_at | non-toxic vs all other classes | D78308 | 716 |
| J01435cds#8_5_at | non-toxic vs all other classes | J01435 | 717 |
| K01932_f_at | non-toxic vs all other classes | K01932 | 718 |
| M1 1794cds#2_f_at | non-toxic vs all other classes | M11794 | 719 |
| M13100cds#2_s_at | non-toxic vs all other classes | M13100 | 720 |
| M20131cds_s_at | non-toxic vs all other classes | M20131 | 721 |
| M64733mRNA_s_at | non-toxic vs all other classes | M64733 | 722 |
| rc_AA800054_at | non-toxic vs all other classes | AA800054 | 723 |
| rc_AA817964_s_at | non-toxic vs all other classes | AA817964 | 724 |
| rc_AA945054_s_at | non-toxic vs all other classes | AA945054 | 725 |
| rc_AA945169_at | non-toxic vs all other classes | AA945169 | 726 |
| rc_AI104679_s_at | non-toxic vs all other classes | AI104679 | 727 |
| rc_AI179012_s_at | non-toxic vs all other classes | AI179012 | 728 |
| rc_AI236795_s_at | non-toxic vs all other classes | AI236795 | 729 |
| S72505_f_at | non-toxic vs all other classes | S72505 | 730 |
| X03468_at | non-toxic vs all other classes | X03468 | 731 |
| X07467_at | non-toxic vs all other classes | X07467 | 732 |
| AB008807_g_at | controls vs all other classes | AB008807 | 733 |
| D00362_s_at | controls vs all other classes | D00362 | 734 |
| D00913_g_at | controls vs all other classes | D00913 | 735 |
| D25224_at | controls vs all other classes | D25224 | 736 |
| D25224_g_at | controls vs all other classes | D25224 | 737 |
| D43964_at | controls vs all other classes | D43964 | 738 |
| E01184cds_s_at | controls vs all other classes | E01184 | 739 |
| H32189_s_at | controls vs all other classes | H32189 | 740 |
| J00728cds_f_at | controls vs all other classes | J00728 | 741 |
| J02596cds_g_at | controls vs all other classes | J02596 | 742 |
| L37333_s_at | controls vs all other classes | L37333 | 743 |
| M11670_at | controls vs all other classes | M11670 | 744 |
| M15481_at | controls vs all other classes | M15481 | 745 |
| M20629_s_at | controls vs all other classes | M20629 | 746 |
| M28255_s_at | controls vs all other classes | M28255 | 747 |
| M31363mRNA_f_at | controls vs all other classes | M31363 | 748 |
| M58041_s_at | controls vs all other classes | M58041 | 749 |
| M64733mRNA_s_at | controls vs all other classes | M64733 | 750 |
| M76767_s_at | controls vs all other classes | M76767 | 751 |
| rc_AA800318_at | controls vs all other classes | AA800318 | 752 |
| rc_AA858673_at | controls vs all other classes | AA858673 | 753 |
| rc_AA860062_g_at | controls vs all other classes | AA860062 | 754 |
| rc_AA875107_at | controls vs all other classes | AA875107 | 755 |
| rc_AA891774_at | controls vs all other classes | AA891774 | 756 |
| rc_AA892775_at | controls vs all other classes | AA892775 | 757 |
| rc_AA892888_g_at | controls vs all other classes | AA892888 | 758 |
| rc_AA945143_at | controls vs all other classes | AA945143 | 759 |
| rc_AA946503_at | controls vs all other classes | AA946503 | 760 |
| rc_AI 008641_at | controls vs all other classes | AI008641 | 761 |
| rc_AI011998_at | controls vs all other classes | AI011998 | 762 |
| rc_AI 104524_s_at | controls vs all other classes | AI104524 | 763 |
| rc_AI136891_at | controls vs all other classes | AI136891 | 764 |
| rc_AI 169372_g_at | controls vs all other classes | AI169372 | 765 |
| rc_AI172017_at | controls vs all other classes | AI172017 | 766 |
| rc_AI228674_s_at | controls vs all other classes | AI228674 | 767 |
| rc_AI232087_at | controls vs all other classes | AI232087 | 768 |
| S61868_g_at | controls vs all other classes | S61868 | 769 |
| S72505_f_at | controls vs all other classes | S72505 | 770 |
| S76779_s_at | controls vs all other classes | S76779 | 771 |
| X15096cds_s_at | controls vs all other classes | X15096 | 772 |
| X15512_at | controls vs all other classes | X15512 | 773 |
| X56325mRNA_s_at | controls vs all other classes | X56325 | 774 |
| X57432cds_s_at | controls vs all other classes | X57432 | 775 |
| X74549_at | controls vs all other classes | X74549 | 776 |
| X76456cds_at | controls vs all other classes | X76456 | 777 |
| X79081mRNA_f_at | controls vs all other classes | X79081 | 778 |

**Table 8**

| The accession numbers refer to GenBank. | | | |
|---|---|---|---|
| Affymetrix ID | Discriminator | Acc Number | SEQ ID NO. |
| D25224_g_at | direct acting vs controls | D25224 | 779 |
| E01184cds_s_at | direct acting vs controls | E01184 | 780 |
| J02585_at | direct acting vs controls | J02585 | 781 |
| J02597cds_s_at | direct acting vs controls | J02597 | 782 |
| J03588_at | direct acting vs controls | J03588 | 783 |
| L19998_at | direct acting vs controls | L19998 | 784 |
| M13100cds#2_s_at | direct acting vs controls | M13100 | 785 |
| M94548_at | direct acting vs controls | M94548 | 786 |
| rc_AA800054_at | direct acting vs controls | AA800054 | 787 |
| rc_AI231807_g_at | direct acting vs controls | AI231807 | 788 |
| S76489_s_at | direct acting vs controls | S76489 | 789 |
| X53581cds#3_f_at | direct acting vs controls | X53581 | 790 |
| X57432cds_s_at | direct acting vs controls | X57432 | 791 |
| X58465mRNA_g_at | direct acting vs controls | X58465 | 792 |
| L00320cds_f_at | steatotic vs controls | L00320 | 793 |
| rc_AA946503_at | steatotic vs controls | AA946503 | 794 |
| X56325mRNA_s_at | steatotic vs controls | X56325 | 795 |
| AF038870_at | cholestatic vs controls | AF038870 | 796 |
| AF076183_at | cholestatic vs controls | AF076183 | 797 |
| D89375_s_at | cholestatic vs controls | D89375 | 798 |
| J00738_s_at | cholestatic vs controls | J00738 | 799 |
| J01435cds#1_s_at | cholestatic vs controls | J01435 | 800 |
| J03588_at | cholestatic vs controls | J03588 | 801 |
| J03863_at | cholestatic vs controls | J03863 | 802 |
| K01932_f_at | cholestatic vs controls | K01932 | 803 |
| K01934mRNA#2_at | cholestatic vs controls | K01934 | 804 |
| L27843_s_at | cholestatic vs controls | L27843 | 805 |
| M10068mRNA_s_at | cholestatic vs controls | M10068 | 806 |
| M11670_at | cholestatic vs controls | M11670 | 807 |
| M13100cds#3_f_at | cholestatic vs controls | M13100 | 808 |
| M14775_s_at | cholestatic vs controls | M14775 | 809 |
| M15327_at | cholestatic vs controls | M15327 | 810 |
| M20629_s_at | cholestatic vs controls | M20629 | 811 |
| M31018_f_at | cholestatic vs controls | M31018 | 812 |
| M34331_g_at | cholestatic vs controls | M34331 | 813 |
| M57718mRNA_s_at | cholestatic vs controls | M57718 | 814 |
| rc_AA800318_at | cholestatic vs controls | AA800318 | 815 |
| rc_AA858673_at | cholestatic vs controls | AA858673 | 816 |
| rc_AA859372_s_at | cholestatic vs controls | AA859372 | 817 |
| rc_AA945143_at | cholestatic vs controls | AA945143 | 818 |
| rc_AA945321_at | cholestatic vs controls | AA945321 | 819 |
| rc_AI072634_at | cholestatic vs controls | AI072634 | 820 |
| rc_AI102562_at | cholestatic vs controls | AI102562 | 821 |
| rc_AI104524_s_at | cholestatic vs controls | AI104524 | 822 |
| rc_AI105448_at | cholestatic vs controls | AI105448 | 823 |
| rc_AI228674_s_at | cholestatic vs controls | AI228674 | 824 |
| S76489_s_at | cholestatic vs controls | S76489 | 825 |
| X04979_at | cholestatic vs controls | X04979 | 826 |
| X15734_at | cholestatic vs controls | X15734 | 827 |
| X86561cds#2_at | cholestatic vs controls | X86561 | 828 |
| Y07704_at | cholestatic vs controls | Y07704 | 829 |

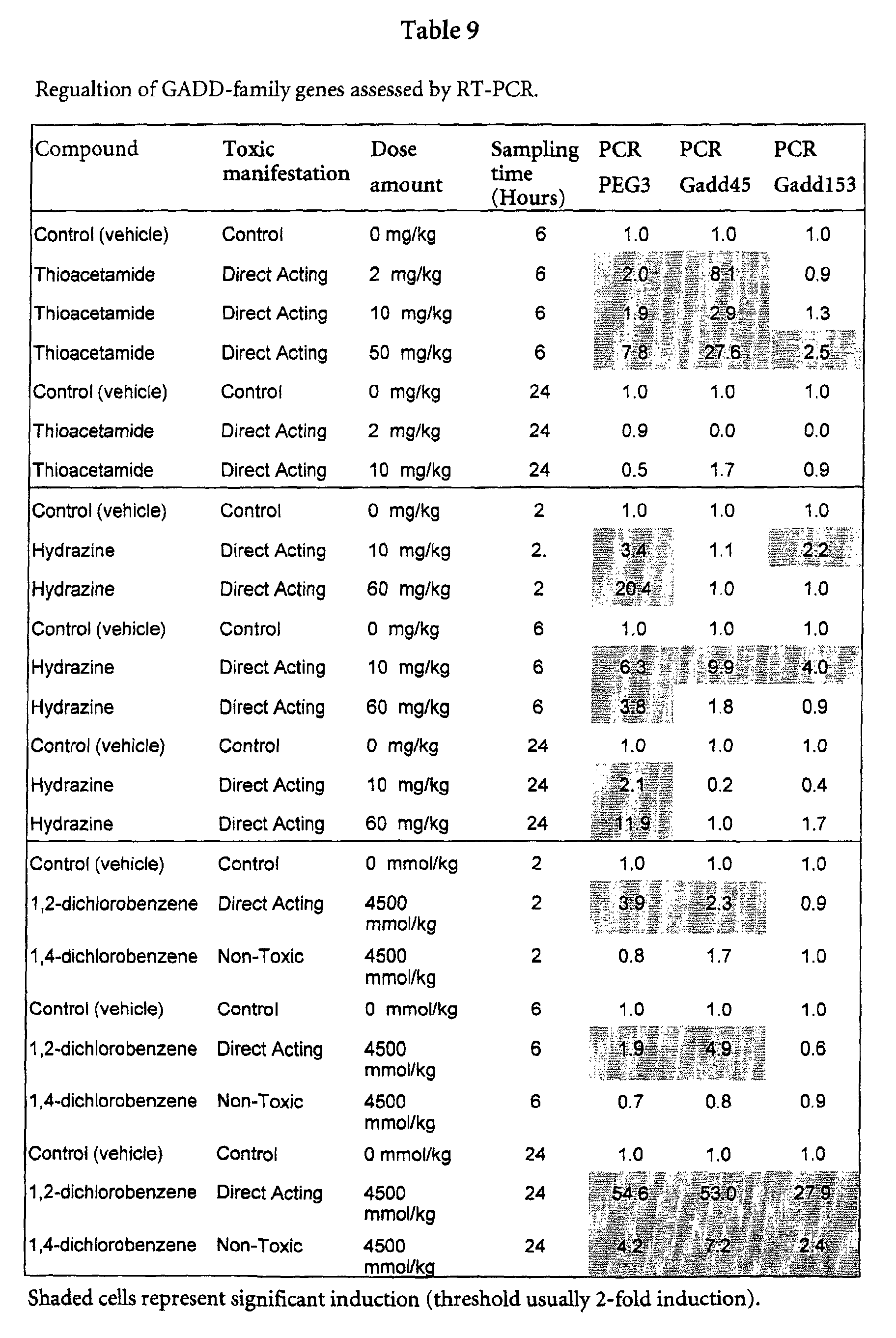

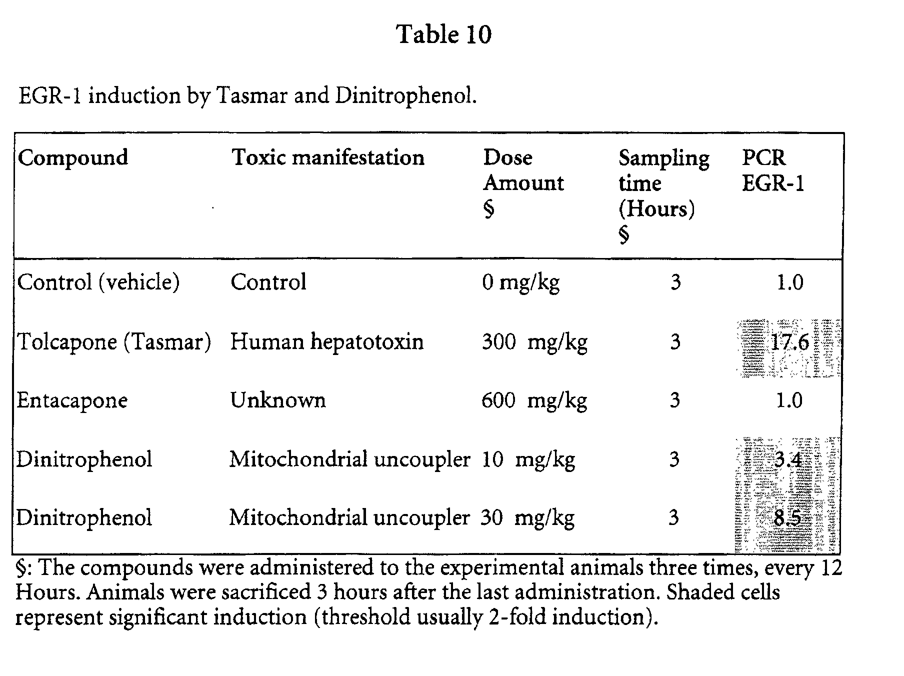

## Claims

1. A method of predicting at least one toxic effect of a compound, comprising detecting the level of expression of one or more genes from Table 3 in a tissue or cell sample exposed to the compound; wherein differential expression of the genes in Table 3 is indicative of at least one toxic effect.

2. A method of predicting at least one toxic effect of a compound, comprising:
(a) detecting the level of expression of one or more genes from Table 3 in a tissue or cell sample exposed to the compound;
(b) comparing the level of expression of the genes to their level of expression in a control tissue or cell sample, wherein differential expression of the genes in Table 3 is indicative of at least one toxic effect.

3. A method of predicting the progression of a toxic effect of a compound, comprising
detecting the level of expression in a tissue or cell sample exposed to the compound of one or more genes from Table 3, wherein differential expression of the genes in Table 3 is indicative of toxicity progression.

4. The method according to claims 1 to 3, wherein the toxic effect is hepatotoxicity.

5. The method according to claims 1 to 4, wherein the hepatotoxicity comprises at least one liver disease pathology selected from the group consisting of hepatitis, liver necrosis, protein adduct formation and fatty liver.

6. A method of predicting the mechanism of toxicity of a compound comprising detecting the level of expression in a tissue or cell sample exposed to the compound of one or more genes from Table 3, wherein differential expression of the genes in Table 3 is associated with a specific mechanism of toxicity.

7. The method according to any one of claims 1 to 6, wherein the expression levels of at least 2 genes from Table 3 are detected.

8. The method according to any one of claims 1 to 6, wherein the expression levels of at least 5 genes from Table 3 are detected.

9. The method according to any one of claims 1 to 6, wherein the expression levels of at least 10 genes from Table 3 are detected.

10. The method according to any one of claims 1 to 6, wherein the expression levels of nearly all genes from Table 3 are detected.

11. The method according to any one of claims 1 to 6, wherein the expression levels of all genes from Table 3 are detected.

12. A method of predicting at least one toxic effect of a compound, comprising detecting the level of expression of one of the genes selected from Table 4 in a tissue or cell sample exposed to the compound, wherein differential expression of the gene selected from Table 4 is indicative of at least one toxic effect.

13. The method according to claim 12, wherein the gene selected from Table 4 is progression elevated gene 3 or Translocon associated protein.

14. The method according to claims 12 and 13, wherein the toxic effect is hepatotoxicity.

15. The method according to any one of claims 1 to 14, wherein the level of expression is detected by an amplification, hybridization or reporter gene assay.

16. A set of nucleic acid primers, wherein the primers specifically amplify at least two of the genes from Table 3.

17. A set of nucleic acid probes, wherein the probes comprise sequences which hybridize to at least two of the genes from Table 3.

18. A set of nucleic acid probes, wherein the probes comprise sequences which hybridize to at least 5 of the genes from Table 3.

19. A set of nucleic acid probes, wherein the probes comprise sequences which hybridize to at least 10 of the genes from Table 3.

20. The set of probes according to claims 17 to 19, wherein the probes are attached to a solid support.

21. A solid support comprising at least two probes, wherein each of the probes comprises a sequence that specifically hybridizes to a gene in Table 3.

22. A computer system comprising a database containing DNA sequence information and expression information of at least two of the genes from Table 3 from tissue or cells exposed to a hepatotoxin, and a user interface.

23. A kit comprising at least one solid support according to claim 21 and gene expression information for the said genes.

24. Use of the method according to claims 1, 2, 4, 5, 7 to 11 and 15 for predicting at least one toxic effect of a compound.

25. Use of the method according to claims 12 to 14 for predicting at least one toxic effect of a compound.

26. Use of the method according to claims 3 to 5, 7 to 11 and 15 for predicting the progression of a toxic effect of a compound.

27. Use of the method according to claims 6 to 11 and 15 for predicting the mechanism of toxicity of a compound.

28. The methods, nucleic acid primers, nucleic acid probes, solid supports, computer systems and kits substantially as herein before described especially with reference to the foregoing Examples.


SEQUENCE LISTING

<110>  F. Hoffmann - La Roche AG

<120>  Methods for the toxicity prediction of a compound

<130>  21199

<150>  02005336.9
<151>  2002-03-14

<150>  02015657.6
<151>  2002-07-17

<160>  862

<170>  PatentIn version 3.1

<210>  1
<211>  353
<212>  DNA
<213>  Rattus norvegicus (Norway rat)

<400>  1
tagagcgcga tgaggaaaag ggctgagctg agctttggtt ctccagaatg taagaagaaa      60

aatttaaaac aaaaatctga actctcaaaa gtctattttt ttaactgaaa atgtagattt     120

atccatgttc gggagttgga atgctgtggt tacctactga gtaggcggtg actttgtatg     180

ctatgaacat gaagttcatt attttgtggt tttattttac ttcgtacttg tgtttgctta     240

aacaaagtga cttgtttggc ttataaacac attgaatgcg ctttactgcc catgggatat     300

gtggtgtgta tccttcagaa aaattaaaag gaaaataaag aaactaactg gtt            353

<210>  2
<211>  2496
<212>  DNA

<213>  Rattus norvegicus (Norway rat)

<400>  2
ctgcagtcag acagattctg aaatggttta aatagggagc tacaaacaag tcaataatta      60

tctaagcctg ctgtgttggt acctgagtct gagaagcact tgggaggtag aaggagaagg     120

catggaagtt caatagctcg ttctaggtca ataataataa tgggtagtaa taataataat     180

gataataata ataaaatact ttcaaggact cggttttaca atattcagat tgcacgtaaa     240

tagttgtgcc agcaggctaa tagttataga aaggcatagt cctttgcagt taaactggtg     300

cttgtgacac ctgtggcttt tatatcgggc gtccttcagc cagaaaaacc cacagctttc     360

cgtggacttc cttagtcaaa ccaaatatga ccttccgtag gtcaggttag gatgcttcag     420

gaccataccg gagttggagt tctggaagtt cccgaggttc aaaaagcaaa gaagagattg     480

ctacagcatt ctaaagtaaa cagggcttaa ccttggccgt gatctttctt ctcaccctcc     540

tcgtgcctcc cggttaaaaa ccaccagctg tgatttacca caaaaactgt aggcaacaaa     600

agcaaaggac ctcacgaggg gtaagagacg gtagatgtat tttttgcaaa tacattaatc     660

tgagacatga acggaatctg caaaactcaa aagacagaga agcctccatc ctcgcaaatc     720

actgtaatac taagtggagt cctaggtgcg tggcgcccac gtgcacataa cgcgtgtggc     780

ccacctgccc tgcgcactgg tactctgaag tctcaccact gccccctgag ccgtcacttg     840

tccagcgaag cgcgtcactc ccttcctctg gactttgggc aaaaagcaaa aatcccggag     900

tctaatcctt gggagtggag caagggggag gagcgatgtc ctttccggtt gtggggaaac     960

accctgtgct ccgggaattt ccctggcctg gagttctgga gtttcgagca taaaagggct    1020

cgccggagcc ctagagctgc agatcaggac tcagatccta aaccagctcc agcactccag    1080

actccagcca cactccaaca gagcaccatg gtctcagcca cccgctcgct tctctgtgca    1140

gcgctgcctg tgctggccac cagccgccaa gccacaggta ggtctcgcca ctgctgtgcg    1200

ggggaggagc gacctccggt gggcgcacgg cccacagtcc gctgacccgg tgtcttcccc    1260

cttaggggcg cccgtcgcca atgagctgcg ctgtcagtgc ctgcagacag tggcagggat    1320

tcacttcaag aacatccaga gtttgaaggt gatgccgcca ggaccccact gcacccaaac    1380

cgaagtcatg tgagtatctc tctgctcgcg cagcttctgc cactcccaga gtgacccaaa    1440

gcctccgcgc ccctacactc atcctagcgg aacttcctca cgtgggtcca tccttctctc    1500

ttcagagcca cactcaagaa tggtcgcgag gcttgccttg accctgaagc ccccatggtt     1560

cagaagattg tccaaaagat gctaaagtga gttgtgactt tgtgtttgta cttgggacta    1620

gagtcgagct tgggaatagt ggcatcagac gcctgaacgt taattatatc gaggatagtc    1680

tgtgcttatc tagagcctca ggaccggata agagagaagg ctttgatgac tctttgtaac    1740

aatgactctt ttttccgtct tcaggggtgt ccccaagtaa tggagaaaga agatagattg    1800

caccgatggc gtctgtctgg tgaacgctgg cttctgacaa cactagtttt acacatttta    1860

cgatttctat tgagggtcct atttatttta tgtatttatt tattccacca agtgtgtggt    1920

ttttatttta cattaatatt taacgatgtg gatgcgtttc atcgatggtc gttcaattcc 1980

aattgtgcag tttaaagatg gtaggcgtta aatatctcgt taaattaata tttattggga 2040

gaccattaag tgtcaaccac tgtgctagaa ggtgttgagc gggaagaagg gcggcagaga 2100

tgagagtctg ggatcgtgtt ttgtgttagg gtgaggaaat gtgtgagagg ctatgtttgt 2160

atgttttgaa aagaatgtta tttattgaaa gttgtctttc atattttatg gtcaacattg 2220

atgtgttgaa gcttcccttg gacattttat gtctagtttg tagggcacaa tgccctttta 2280

tattctttaa ccaatgctcc ttctcgtctc aggacagaga agttccaagg actgttacaa 2340

atgaaataaa aataaaagtt ttattaaaaa aataacatgg gtgctttttg ttttattctt 2400

cttgacatcg ttgtttatag ctaatcatgt gcctgtgctg gctgaaattt cttatgactt 2460

gcttacttgg ggaggaacat ttggtattcc tgaaaa 2496

<210> 3

<211> 1666

<212> DNA

<213> Rattus norvegicus (Norway rat)

<400> 3
cggcagccga gtcggattga gctgctgcag acgccaggcc actccagcca gcactgccgt 60

tttcacgccc cggctgcaga cagctaggag gctttatcta gtttgaacca ggctgctgga 120

gctcgctcct tccctctctt tttttccacg aggctgtttt tttatttggc tgcaattgca 180

tgaaatccca atggtgtaga ccagtggcga tggatctagg agtttaccaa ctgagacatt 240

tttcaatttc tttcttgtcg tctttgctgg gaactgaaaa cgcttccgtg agacttgaca 300

atagctctgg tgcaagtgtg gtagctatcg acaacaaaat agagcaagct atggatctgg 360

tgaaaagcca tttgatgtat gcagttagag aggaagtgga ggttctgaag gagcagatca 420

aagaactaat agagaaaaac tcccaactgg agcaggagaa caatctgttg aagacactgg 480

ccagtccgga gcagctcgcc cagtttcagg cccagctgca gactggctcc cctccggcta 540

ccacgcagcc acaggggacc acacagcccc ctgcacagcc agcgtcccag ggctcaggat 600

caaccgcata gcctgctatg ccccaacaga actggctgct gctgtctgaa ctgaacagac 660

cgaagagatg tgctagtgag aagccgcctc cagtcaccca tttcattgct gtctgcgaaa 720

gagacgtgag actcacacat gctgttctcg ctttctcccc agtattaagc actcatatgc 780

ttttggcttg aagaaatata ctagttgagt gaattaaagg ttaaacagag agtgagcatg 840

gatgtaccct gtgcaacgtg gcagatgtct gaggaatggt ttgattgacg ctgaggagga 900

gctctgtgcc ttttcaaccc tccccagccg cccactctac tcccaagctc tggggctcgc 960

ctgcatgggg ctcagaaggt gggctgctcc tggattttgt gttctcctct ccttcccttc 1020

aaagaatttg agaggccaga aacgagactg caaagggggg gatgcagtcc ttttacaaaa 1080

ccgacaactg tcaccaaagc ttataaaaca ggacagtact gtccctcttt tctgaaacat 1140

cagaagacac aaaactgtta gtgacacaac ggtgacaggt agctgggacc taggctatct 1200

tattatgaag gttgttttgc ttgttgtata tttgtgtatg tagtgtaacg aatttgtaca 1260

atagaggacc gtaactactg ttaggttgta cagattgaag tttagatgtt ccattggctg 1320

tctgaaaagg tgtggattgt ccttcctaga gagatctact taaaaactgc ttcgtgacaa 1380

aaaccacacc tgaagaaatt ttaagaattt ggcacagtta gtcactttgt gtcacccgga 1440

atctagctgc tgagtcttgc aaagtaaacc ccctgttgac tgatgtcagt tgagctagtg 1500

aatgaataga tggagaaacg tcagtcagtt gctgaggaag tggatttccc agtaggggtt 1560

tctgcagctc acctgtatag tcctgcgcat gttccccaca cagaacccac tgtatttacc 1620

tgttctactt gtcacctttc aataaagcat atcaaatgtt gatacc 1666

<210> 4

<211> 3112

<212> DNA

<213> Rattus norvegicus (Norway rat)

<400> 4
cgcagaactt ggggagccgc cgccgcgatt cgccgccgcc gccagcttcc gccgccgcaa 60

gatcggcccc tgccccagcc tccgcggcag ccctgcgtcc accacgggcc gcggccaccg 120

ccagcctggg ggcccaccta cactccccgc agtgtgcccc tgcaccccgc atgtaacccg 180

gccaacatcc ggcgagtgtg ccctcagtag cttcggcccc gggctgcgcc caccacccaa 240

catcagctct ccagctcgca cgtccgggat ggcagcggcc aaggccgaga tgcaattgat 300

gtctccgctg cagatctctg acccgttcgg ctcctttcct cactcaccca ccatggacaa 360

ctaccccaaa ctggaggaga tgatgctgct gagcaacggg gctccccagt tcctcggtgc 420

tgccggaacc ccagagggca gcggcggcaa taacagcagc agcagcagca gcagcagcag 480

cgggggcggt ggtgggggcg gcagcaacag cggcagcagc gctttcaatc ctcaagggga 540

gccgagcgaa caaccctacg agcacctgac cacagagtcc ttttctgaca tcgctctgaa 600

taacgagaag gcgctggtgg agacaagtta tcccagccaa actacccggt tgcctcccat 660

cacctatact ggccgcttct ccctggagcc tgcacccaac agtggcaaca ctttgtggcc 720

tgaacccctt ttcagcctag tcagtggcct tgtgagcatg accaaccctc caacctcttc 780

atcctcagcg ccttctccag ctgcttcatc gtcttcctct gcctcccaga gcccacccct 840

gagctgtgcc gtgccgtcca acgacagcag tcccatttac tcagctgcac ccacctttcc 900

tactcccaac actgacattt ttcctgagcc ccaaagccag gcctttcctg gctctgcagg 960

cacagccttg cagtacccgc ctcctgccta ccctgccacc aagggtggtt tccaggttcc 1020

catgatccct gactatctgt ttccacaaca acagggagac ctgagcctgg gcaccccaga 1080

ccagaagccc ttccagggtc tggagaaccg tacccagcag ccttcgctca ctccactatc 1140
cactatcaaa gccttcgcca ctcagtcggg ctcccaggac ttaaaggctc ttaataacac 1200
ctaccagtcc caactcatca aacccagccg catgcgcaag taccccaacc ggcccagcaa 1260
gacacccccc catgaacgcc cgtatgcttg ccctgttgag tcctgcgatc gccgcttttc 1320
tcgctcggat gagcttacac gccacatccg catccataca ggccagaagc ccttccagtg 1380
tcgaatctgc atgcgtaatt tcagtcgtag tgaccacctt accacccaca tccgcaccca 1440
cacaggcgag aagccttttg cctgtgacat ttgtgggaga aagtttgcca ggagtgatga 1500
acgcaagagg cataccaaaa tccacttaag acagaaggac aagaaagcag acaaaagtgt 1560
cgtggcctcc tcagctgcct cttccctctc ttcctaccca tccccagtgg ctacctccta 1620
cccatccccc gccaccacct catttccatc cccagtgccc acctcttact cctctccggg 1680
ctcctctacc tacccgtctc ctgcacacag tggcttccca tcgccctcgg tggccaccac 1740
ctatgcctcc gtcccacctg ctttccctgc ccaggtcagc accttccagt ctgcaggggt 1800
cagcaactcc ttcagcacct caacgggtct ttcagacatg acagcaacct tttctcctag 1860
gacaattgaa atttgctaaa gggaatgaaa gagagcaaag ggaggggagc gcgagagaca 1920
ataaaggaca ggagggaaga aatggcccgc aagaggggct gcctcttagg tcagatggaa 1980
gatctcagag ccaagtcctt ctagtcagta gaaggcccgt tggccaccag ccctttcact 2040
tagcgtccct gccctcccca gtcccggtcc ttttgacttc agctgcctga aacagccacg 2100
tccaagttct tcacctctat ccaaaggact tgatttgcat ggtattggat aaaccatttc 2160
agcatcatct ccaccacatg cctggccctt gctcccttca gcactagaac atcaagttgg 2220
ctgaaaaaaa aaatgggtct gggccctcag aaccctgccc tgtatctttg tacagcatct 2280
gtgccatgga ttttgttttc cttggggtat tcttgatgtg aagataattt gcatactcta 2340
ttgtactatt tggagttaaa ttctcacttt gggggagggg gagcaaagcc aagcaaacca 2400
atggtgatcc tctattttgt gatgatcctg ctgtgacatt aggtttgaaa cttttttttt 2460
tttttgaagc agcagtccta ggtattaact ggagcatgtg tcagagtgtt gttccgttaa 2520
ttttgtaaat actgctcgac tgtaactctc acatgtgaca aaatacggtt tgtttggttg 2580
ggttttttgt tgtttttgaa aaaaaaattt tttttttgcc cgtccctttg gtttcaaaag 2640
tttcacgtct tggtgccttt gtgtgacaca ccttgccgat ggctggacat gtgcaatcgt 2700
gaggggacac gctcacctct agccttaagg gggtaggagt gatgtttcag gggaggcttt 2760
agagcacgat gaggaagagg gctgagctga gctttggttc tccagaatgt aagaagaaaa 2820
atttaaaaca aaaatctgaa ctctcaaaag tctatttttt taactgaaaa tgtagattta 2880
tccatgttcg ggagttggaa tgctgcggtt acctactgag taggcggtga cttttgtatg 2940
ctatgaacat gaagttcatt attttgtggt tttattttac ttcgtacttg tgtttgctta 3000
aacaaagtga cttgtttggc ttataaacac attgaatgcg ctttactgcc catgggatat 3060
gtggtgtgta tccttcagaa aaattaaaag gaaaataaag aaactaactg gt 3112

<210> 5

<211> 1500

<212> DNA

<213> Rattus norvegicus (Norway rat)

<400> 5
cgccgagcac aaacccagcg agcattgaac actgcacacg gccatctgcc cagagagctg 60

tgaccaccac ttccgctact atctactcag aaagtcgtga ctactgagcc actgctgcct 120

gcccagattc tcatccaccg cctgctgcgt ctggttgcga tgccggagtt cctaactgtt 180

gtttcttggc cgttcctgat cctcctgtcc ttccaggttc gcgtagtcgc tggagccccc 240

cagccatggc actgtgctcc ctgcactgct gagaggctgg agctctgtcc acccgtgcct 300

gcttcgtgcc ccgagatttc tcggcctgcg ggctgtggct gctgcccgac atgtgccttg 360

ccactgggtg ctgcctgtgg tgtggccact gcgcgctgcg ctcagggact cagctgccgt 420

gcgctgccag gggagcctcg acctctgcat gccctcaccc gtggccaggg agcctgtgta 480

ctagaacctg ccgcacccgc cacgagcagc ttgtccggtt ctcagcatga agaggcaaag 540

gctgctgtgg cctctgagga tgagcttgcc gagagcccag agatgacaga ggaacagctg 600

ctggatagct tccacctcat ggccccatcc cgtgaggacc agcccatcct gtggaatgcc 660

attagcacct acagcagcat gcgggcccgg gagatcactg acctcaagaa atggaaggag 720

ccctgccaac gggaactcta taaagtgtta gagagattag ctgccgctca acagaaagca 780

ggagatgaga tctacaaatt ttatctgcca aactgcaaca agaatggatt ttatcacagc 840

aaacagtgcg agacatctct ggatggagaa gctgggctct gctggtgtgt ctacccatgg 900

agtgggaaga agatccctgg atctctggag accagagggg accccaactg ccaccagtat 960

tttaatgtgc aaaactgaaa gttgtttcct ccctccttct tcacacaaaa tatttaagta 1020

tatagtgtat ttatactccg gagcacacca ttttatatat gtgtatatgt atatatccag 1080

gaactagttt ttatactcca catgctgctt gatgtacaag tgggtttgta tttattcact 1140

ctaagtttat tttttctacc cctgtccttg tgctgtatta atttatataa ctgaagcttt 1200

tctcatctcc atacatgtaa atactaccat ctcagctctt ccagagttct gctttgaaag 1260

ggcagcgcgg tagtgcctag aacgagcaca agtcagtctg aggtaggggc ctttcagtgg 1320

gttcagggag gaaggttagc cctggctcgg ggagacttcc tcatcgaatc ccacaggtct 1380

gtgtctgatg cctattggct gggaaggttc cgatgttggt tgtgtaatca aagctaaacg 1440

tggaaagctg cgtcccatgc actgttaaac acacgtctgg aataaaacat tctacctgga 1500

<210> 6

<211> 584

<212>  DNA

<213>  Rattus norvegicus (rat)

<400>  6
gaaacactgg gaagaagtgg ccttccttca ggttcagtga cactgatgag ggcttctcag      60

aaggcctcga gtctctcaaa ccaaaggaca gagctagagc cagccagtca cccttagtga     120

ggatcccctt ccccatgtct ctccactgcc gtggcatccc atgtcctgga tttctcaatt     180

cctcagtttc tactcaaagg tgctacttac caaacactct gcccgtcccg ctctccccag     240

cttcgcacag ccgtcccagg tggcttcgtc tctcctgctt taaagttaac tttgggccca     300

cagacccgag agctgtgggt tgaagcaaag ctgtgaatcg ctccagatgg tccctgtgtt     360

ctgtccacac acaggtcccc gcctttttag aagcagcctc ctggtctcat gcttaaatct     420

gttcctcact gcccgtgttc actttagaaa tggcagaacc acagagctgg actgttgagc     480

aggcctgtct ctctcattaa atagaaataa gtaagtttgt aagctattcc gacagaagag     540

acaaaggtta ctgattgtac aatagcgctt ttatatggaa gact                      584

<210>  7

<211>  446

<212>  DNA

<213>  Rattus sp.

<400>  7
aaatagattc aataaaaagt caaacacaca cacaaacaca tcttaaaata gactttagac      60

acgaagtgcg tgtttcttct ccacagtact gtgcagaggg ggagggcagg gggcggaggt     120

tcctccctag tatccccaca ggctgagtac caggcggcgg ggccagctcc gccgcgacaa     180

cccccttctc ccctccctgt taaatacaca aatatattat attcaatatg aattcagtct     240

ctttccagaa aaaaaaaaca tacaaaatac gctggaaggg ggccatgtaa acctcgaggt     300

ggaaggactg ggcgcaggcg ggcaggccag agtccagtgt gtgagctgcg ccccagacct     360

ctgggcgagt gcccatcgcc tgcccccctc accccagtgg ggggcgggcg cccagccttc     420

aaggctgggg gtgtccgtat ggagca                                          446

<210>  8

<211>  434

<212>  DNA

<213>  Rattus sp.

<400>  8
aaaacataac tgagctaata tttttcaaag gattgtaaga agacaatgac ttaaaaagaa      60

aataaatttt ctgttatact aatacatagt gaacttatca agctactgta atactgtaaa 120

tagtcatgct tgtcaggatc tttctggaag gacatggcca agcatgagag ggtggggggc 180

atccatgcag tcattctagg ttagttgagg agtaggaaat tgagagtact tctcgttttg 240

atgcgaaggc ttctcaaatc atgaagatca ttacaaggac ggccgtaagt gagatgaatg 300

agcctataga ggagactgta tttcatgtgg tgtaagcatc tggataatca gagtaacgac 360

gaggtatccc cgctaatcct aggaagtgtt gagggaaaaa tgttatgttt acacctacaa 420

atataatggc aaag 434

<210> 9

<211> 384

<212> DNA

<213> Rattus norvegicus (Norway rat)

<220>

<221> misc_feature

<222> (153)..(153)

<223> n

<220>

<221> misc_feature

<222> (219)..(219)

<223> n

<400> 9
cggccgccat tctcgcctgc tggttccttg gcccgaagcc agctagtggc cacccccttg 60

ttcactcggc cagacttcgc ttcgtactcc acggccacgg cacagatgtg cacggagttg 120

gggtggacct tggaggatga ggcaatggag tantatcggg cctgcaggcg tggcagcagc 180

tcacacaggt ggtcgatggg tggccgcagt gatgggtant cttggaggat ggctaggatg 240

tgcctccggg cttccaccac ccagctcagg tacagctcct tgccctcgcc tgaggatgac 300

gccatcttgt gcaggtgctc ctgctccgag ggctctgagg cgtactgtgc cagttcgtag 360

agcacattgg tgcgtggcgg gtta 384

<210> 10

<211> 590

<212> DNA

<213>  Rattus sp.

<400>  10
cattgtaaaa cttactttaa tgatttctgt gcttttcctt ataagaataa aacatgacag     60

ctaatcttca atgctgaaaa ccagaggatc ggacaaaaat cacatttatt tcaccagctc    120

tgacaggtgc agatagagaa ttcacaaagc tccgagacca cctgacatac aaagacaagg    180

gggtggacat cagaagtggg gctcctcaat ttaaaaatgc cccaagtaac ttgaaagtcg    240

ttaagttata gtattctatg tacatcacgt aggaaatgag ccaatttaca agatgctgat    300

aaagatacta caatcaaaca atccttcaca gaaagaatag tcctgaactg gtcaaccgca    360

gaccaggggg gtttcagtca ctcgatcacc agctgatata ttcactttgt gatattttca    420

tttcaagtca attctaaaaa acaccatgac ttaaactcga aggcagacat tattgttgag    480

aattttctga aatggccagt gtatgtaaaa aggcttaaaa ggcaacgtca acttcagcct    540

cgggtggcgt gaagtggtga gttgtacatt aacacttcct cgggttcctg              590

<210>  11

<211>  393

<212>  DNA

<213>  Rattus norvegicus (Norway rat)

<400>  11
   gacacacctt gccgatggct gaacatgtgc aattcgtgag ggacacgctc acttctagcc     60
   ttaagggggt aggagtgatg tttcagggga ggctttagag cacgatgagg aagagggctg    120
   agctgagctt tggttctcca gaatgtaaga agaaaaaatt taaaacaaaa atctgaactc    180
   tcaaaagtct atttttttaa ctgaaaatgt agatttatcc atgttcggga gttggaatgc    240
   tgcggttacc tactgagtag gcggtgactt ttgtatgcta tgaacatgaa gttcattatt    300
   ttgtggtttt attttacttc gtacttgtgt ttgcttaaac aaagtgactt gtttggctta    360
   taaacacatt gaatgcgctt tactgcccat ggg                                 393

<210>  12

<211>  1182

<212>  DNA

<213>  Rattus norvegicus (Norway rat)

<400>  12
ggaagcttcc ccaccgcaaa gcagctagac gtgcgggcag tctcagctgt ccactgggag     60

tcgccaacac catctcttgg gctccagggt cgcaatcact accggggatc tccagcaagt    120

cttccggacc cacgcgtaga ggaggtggtg ccaccgcgcg caaacctggc gcggtcgtga    180

cgtcaggact acgctagatt ggcaattggc tggaggcatg tggcgcggac gggcactggc 240

aggagtgatt ggatcagctc ttaaggggcg gggtctggag gcggagcatg ctgccgagag 300

cgttgaagta cggcgctccg cacagcttct cgacactcct gtctggaatc gctgtgttct 360

aggtcagatc agacattctg tcacaatgcc tgaaatcaat gccagcaatc ttgatgaaaa 420

acaggttcag cttctagcag agatgtgtat tcttattgat gaaaatgaca ataaaattgg 480

ggctgacacc aagaaaaatt gtcacctgaa tgaaaacatt gacaaaggac taatacatcg 540

agctttcagt gtcttcttgt ttaatactga aaacaagctc ctgttacagc agagatcgga 600

tgctaaaatt acctttccag gttgtttcac caatagttgc tgtagtcatc cattaaataa 660

ccccggggag ctggaagaaa atgatgccat gggtgtcaaa cgagcagcac agaagcgctt 720

aaaggcggag ctggggatac ccttggaaga ggttgatcta aatgaaatga attatctaac 780

aagaatttac tacaaggccc aatctgatgg tatctggggt gaacatgaaa ttgattacat 840

tttgtttctg aggaagaatg taaccctgaa tccggatccc aacgagatta aaagctattg 900

ctatgtatca aaggaagaac tcaaagaaat tttgaagaag gaagccaggg gcgaaattaa 960

gtttactcca tggtttaaga ttattgcaga cgcttttctc tttaaatggt gggataactt 1020

aaaccatttg agtccttttg ttgaccatga gaaaatacat agaatgtgaa tgtgtaaatg 1080

atttgagatt tttttctacc tactaagaat ggtttgattt tgttttaaat tgggctccct 1140

tgtatgacac atagatattt tacaaccaga atttgttttg ag 1182

<210> 13

<211> 602

<212> DNA

<213> Rattus norvegicus (Norway rat)

<400> 13
gagggcagac atttggaaaa caggcaagac tttggagaaa gcagaccagg tatgatggcc 60

accttgccac caacagccca cacttcccag caacctgtaa acatagagga cgaagatggg 120

atcctggatg agtatgacca gtacagcctg gcccaatctt atgtcgtcgg tggaggtcgg 180

aaaggacgta ccaagagaga agctgctgcc aacaccaacc gccccagccc tggtgggcat 240

gagaggaagc tgctgaccaa gttccagaac tctgaaagga aaaaggcctg gcgctgagac 300

agagctggag atgaggccag accacggaca ccacacccag caatggatac aggaccttgg 360

agaaattagg agttgtaagc aggacctagg cctgcttgat tctttcccac ctaagtgagg 420

ctctggagag ggaacaccaa aaaaagccct tccggaccca gctaaagggc aagttaggag 480

cgagaagcag agggtagatg ggcagagcta agaaggcacc cccaaaaaga acagtgggaa 540

aaactggatg cccttgggtg gcagtaacaa taaactgtac aacagtgaca gcaaaaaaaa 600

aa 602

<210> 14

<211> 2910

<212> DNA

<213> Rattus norvegicus (Norway rat)

<220>

<221> misc_feature

<222> (1899)..(1899)

<223> n

<400> 14
ggcacgagct ttacttatgt cacaggcata ataaaacatg acttcattat ataaggggct 60

atgcttaaac tgaaagagaa gtaggagaga gttatggaga tgtgaatgga cctattttga 120

gctgtgaggc tacattttaa ggtacctact tagattataa gcctgatgtg aactccaaga 180

gttcaataat catgactatt catacacctt gcctgctgtg tctagcagat caaatgagga 240

aacactaacc ttgcggctga ctgtagaaat tagatcaatt tagaggagtg ggaacacatg 300

tttgttatca agagactacc atgtgtcatt gtgggaatag gagcagagac cttggtcctt 360

tgagattgct ttcttagaca taaaaggggg acgctggtga gagaagcaca caagtagaca 420

tgattatttc actcctctgt ctgccagatc cctagagcat cagctggtgt tccagcagct 480

tgttccccga gcctcctgag tagcattata gagaggtctg ttccagaaag cgaaagccag 540

atccagctgc cctcctggag cagaacaaca caatttccag gctgtccctg tggcctctcc 600

ctcttccagt ctgccccact tggtgtcagc gatgagctcc tgcaacttca ctcacgccac 660

cttcatgctt atcggtattc caggactgga ggaagctcac ttttggtttg gcttccccct 720

actttccatg tacgctgtag cattgtttgg aaattgcatt gtggtcttca tcgtgaggac 780

agagcggagc ctgcatgcgc ccatgtatct ttttctctgc atgctggcag ctattgatct 840

ggctttgtcc acctctacaa tgcccaagat tctcgccctc ttttggtttg actcccggga 900

gattactttt gatgcctgtc tcgcccagat gttcttcatt cacgctctct cagcaattga 960

atctaccatc ttgctggcca tggcctttga ccggtatgtg gctatctgcc acccactgcg 1020

tcacgctgct gtgctcaaca atacagtaac agtccaaatc ggcatggtgg ctctggtccg 1080

gggatcccta ttctttttcc cactcccact gctgatcaag cgactggcct tctgccactc 1140

caatgtgctc tcccactcct attgtgttca ccaggatgtg atgaagttgg cctatacaga 1200

cacattgccc aatgtagtct atggtctaac tgccattctg ctagtcatgg gtgtagatgt 1260

catgttcatc tccttgtcct atttcctgat tatacgagca gttctgcaat tgccttccaa 1320

gtctgagcga gctaaggcat ttggaacctg tgtgtcacac attggtgtag tcctggcttt 1380

ctatgtccca ctcattggcc tgtcagtagt gcaccgtttt ggaaacagcc tggatcccat 1440

tgtgcatgtt ctcatgggcg atgtctacct gctgctgcct cctgtgatca atcccatcat 1500

ctacggtgct aagaccaaac agatcagaac acgggtgctg gctatgttca agatcagttg 1560

tgacaaggac attgaagctg gaggaaacac gtgaccctta ccgttctatc acccttgtcc 1620

tacatggctt aatataatga ttaataattc atcacaccgg cctaactcca gtcacccaaa 1680

agcacattag tgctttaact gaatggacag gtgaactaaa gtatggttta tcttcatcat 1740

ggatacaatg tgaaatatct atcatgtgtt ttaaagcttg tgctataaaa tgagacccat 1800

tgataatact aaatgaaaca actaggattt gtgatttaaa ctaattgttt gtgatttgaa 1860

tgagatatgt tgaaattact agcaactttt tttattccna cttcctttgt tgtaaagcta 1920

tctgtaatgt ttaacttctg aattgtggaa catggagctg acagtatggt agccaagggt 1980

ttcctcactc atttcaatgc tattattcac tacccaggaa catttatgag ctctaaataa 2040

tatggctcat gaatgtatca tgtaacgtgt tccagtccct gcgttaagta cttttcagac 2100

tttctgtgat cttgctcttt tcccaaggaa gataccattg ttgcctgcca caacatgtga 2160

caaagacttt aatgagtaaa gaaactttct tgaatgttct gtgccaagat tttaaattaa 2220

attggataat tgaatacatt tgctttacca ccatgttata ttgcttcctg tttaacatct 2280

gccatctatt tcctctgcct attacaaatc ctgctttctc tctgccgtgt gctagcatgg 2340

acgtctttgt gtttgtggta agtgattaac cctgtcctgg tggagggcaa caaactgttc 2400

agcatgacag agtagctgct tcttctcata tgaacagagg agatttaaat tttttagtct 2460

cttacatatc cttgattgtg tcctctttgt attgtgtcct gtagctgtca aatctcttaa 2520

atgtataaca taagatattg cctgattctt gacttaggta tcaaacaaca taataattta 2580

tcttaaaatg ttcagtatta tttttactgt tttaatgttc ataagtaatg ttgggagaca 2640

tgagtgattt cccccagtcc tctttaccaa cagtgcaaag ggcctgccag gtatgggttg 2700

cacaactata aagatgttga atgaacttat gtagaatatt ccctttgtat tgagtggatg 2760

atgaaaatgt tcttagatta gggtattaat gacacctaca aaataatgtt aatatattcg 2820

atgtacgcat aatgcttaaa atgctgagat ttatgtagta tgtatctgcc acaataaaaa 2880

atcatttaaa ataaaaaaaa aaaaaaaaaa 2910

<210> 15

<211> 993

<212> DNA

<213> Rattus norvegicus (Norway rat)

<400> 15
ggcaagggct ggaatactaa aagttattca tgatgtcaga ctatacttgg tttgaaggaa 60

taccttttcc tgccttttgg ttttccaaag aaattctgga aaatagttgt aagaagtttg 120

tggtaaaaga agacgacttg atcatattga cttaccccaa gtcaggaacg aactggctga 180

tcgagattgt ctgcttgatt cagaccaagg gagatcccaa gtggatccaa tctatgccca 240

tctgggatcg ctcaccctgg atagagactg gttcaggata tgataaatta accaaaatgg 300

aaggaccacg actcatgacc tcccatcttc ccatgcatct tttctccaag tctctcttca 360

gttccaaggc caaggtgata tatctcatca gaaatcccag agatgttctt gtttctgctt 420

attttttctg gagtaagatc gccctggaga agaaaccaga ctcgctggga acttacgttg 480

aatggttcct caaaggaaat gttgcatatg gatcatggtt tgagcacatc cgtggctggc 540

tgtctatgag agaatgggac aacttcttgg tactgtacta tgaagacatg aaaaaggata 600

caatgggatc cataaagaag atatgtgact tcctggggaa aaaattagag ccagatgagc 660

tgaatttggt cctcaagtat agttccttcc aagtcgtgaa agaaaacaac atgtccaatt 720

atagcctcat ggagaaggaa ctgattctta ctggttttac tttcatgaga aaaggcacaa 780

ctaatgactg gaagaatcac ttcacagtag cccaagctga agcctttgat aaagtgttcc 840

aggagaaaat ggccggtttc cctccaggga tgttcccatg ggaataaatt ttcaaaagtt 900

ttaaatattt tatgaacact gatgtttatg tttatgttgt tctatgatgt ctgaataact 960

gaatgtgatc attgaataaa tcctgttgtg gat 993

<210> 16

<211> 1743

<212> DNA

<213> Rattus norvegicus (Norway rat)

<400> 16
   ccgtcccagc atgcagaagg acgcctcctc cagcggcttc ctgcccagct tccagcactt 60
   tgccactcag gccatccacg tgggaccaga gccggagcaa tggagttcgc gtgctgtggt 120
   gctgcccatt tcgctggcca ccacgttcaa acaggactct ccaggccagt cctcgggttt 180
   tgtatacagc cgctctggaa atccgacgag gaattgcttg gaaaaagcag tggctgcact 240
   ggatggggca aagcactgtt tgaccttcgc tcggggcctt gccgccacca caacgattac 300
   ccatctttta aaagcaggag atgaagtcat ttgcatggat gaagtgtatg gaggcaccaa 360
   caggtacttc aggagggtgg catccgagtt tggactgaag atttcttttg tggattgttc 420
   caaaaccaaa ttgctggagg cagcgatcac accacagacc aagcttgttt ggattgaaac 480
   acccacaaac ccaaccttga agttggccga catcaaagcc tgcgcacaaa ttgtccacaa 540
   acacaaagac atcattctgg ttgtagataa cactttcatg tctgcatatt tccagagacc 600
   tttggctctg ggtgctgata tttgtatgtg ttctgccaca aaatacatga acggccacag 660
   tgatgttgtc atgggcttag tgtctgttac ttccgatgac ctcaacgaac ggcttcgttt 720
   cctgcagaat tctctcgggg cagttccttc tcctttcgat tgttacctct gctgccgagg 780
   cctgaagaca ctgcagatcc ggatggagaa acacttcagg aatgggatgg cagtggcccg 840
   tttcctggag tctaatcccc gggtagaaaa ggttatttat cctgggctac cgtctcaccc 900
   tcagcatgag ctcgccaaac gtcagtgcac gggctgcccc gggatggtca gtttctatat 960
   caagggtact ctgcagcatg ctcaggtctt cctcaaaaat ataaagctgt ttgctctggc 1020
   tgagagcctg ggaggatatg agagtctggc tgagcttcca gcaatcatga cccatgcctc 1080
   cgtgcctgag aaggacagag ctaccctcgg gatcagtgac acactgatcc gactttctgt 1140
   gggcctagag gatgaaaagg accttctcga agacctgggt caagctttaa aggcagcgca 1200
   cccttaaagt tcgagtcaaa gccggcattc cagtgctgcc atcagcagca gcagccaagg 1260
   ggccagacct tctgaataac tggacagacc attaaggagc atctgcagaa cttcgcagtg 1320
   aacattttaa gaccctagtg attttacagc tgtaacctta cagggatctt cccttaagga 1380
   ctgtcttctg ctaacaggtt gttctgttag tatcattctg atagttttgc tgtatttgtg 1440
   ttcaaggaag agagttgtat tattttgggg atcatgttgc ttctttttcc ctttttcttt 1500
   cttcggtagc ctaagatatg ttttaatcat gtttacaaaa tttagtattg atgttttatg 1560
   aagttaaatt attcaatgaa cggtcttaaa tcaactgtag ggtttttttt tttgaaaaat 1620
   tattgaaagt gggggtcttt tatttaatta ccataagcca aaaaaatcaa atatttggaa 1680
   tatctactgt gaaattctag tgattaaagg ttgtacttga tacttgttgt ttttcttaaa 1740
   tgg 1743

<210> 17

<211> 343

<212> DNA

<213> Rattus norvegicus (rat)

<400> 17
ccaaggggcc agaccttctg aataactgga cagaccatta aggagcatct gcagaacttc 60

gcagtgaaca ttttaagacc ctagtgattt tacagctgta accttacagg gatcttccct 120

taaggactgt cttctgctaa caggttgttc tgttagtatc attctgatag ttttgctgta 180

tttgtgttca aggaagagag ttgtattatt ttggggatca tgttgcttct ttttcccttt 240

ttctttcttc ggtagcctaa gatatgtttt aatcatgttt acaaaattta gtattgatgt 300

tttatgaagt taaattattc aatgaacggt cttaaatcaa ctg 343

<210> 18

<211> 1213

<212> DNA

<213> Rattus norvegicus (Norway rat)

<400> 18
agacagactc cattctttgt gcagtgaggg gctccctgcc tcgttgggag gcagcgtagt 60

aaacactgct tcggtgctcc agacgcctaa gggctttcgt tacagcgatg ccgaagcggg 120

ggaagagagc ggcagcggaa gacggggaag aacccaagtc cgagccagag accaagaaga 180

gtaagggggc agcaaagaaa actgagaagg aggccgcagg agagggccct gtcctgtatg 240

aggaccctcc agatcagaaa acgtcagcca gtggcaaatc tgccacactc aagatatgct 300

cctggaatgt ggatgggctt cgagcctgga ttaaaaagaa aggcttggat tgggtaaagg 360

aagaagcacc agacatcttg tgcctccaag agaccaaatg ctcagagaac aaactcccgg 420

ctgaactgca agagctgcct ggactcaccc atcagtactg gtcagctcca tcagacaaag 480

aaggatatag tggtgtgggc ctactttccc gccaatgccc gctcaaagtc tcttatggca 540

ttggtgagga agaacatgat caagaaggcc gggtgattgt ggctgaattt gagtccttta 600

tcttggtaac agcctatgtt ccgaacgcag gaaggggtct ggtaagactg gagtaccgac 660

agcgatggga tgaagccttc agaaagtttc taaaggactt ggcttcccgg aaacctcttg 720

tgctgtgtgg ggatctcaat gtggctcatg aagaaataga ccttcgtaac cccaaaggaa 780

acaaaaagaa tgctggtttt actccccagg agcgccaagg ctttggggaa atgctacagg 840

ctgtaccact ggctgacagc ttccggcatc tctaccccaa cactgcctac gcttatactt 900

tctggactta catgatgaat gcccgctcta agaatgttgg ttggcgcctt gattactttt 960

tgctgtctca ctctctttta cctgctttgt gcgacagcaa gatccggtcc aaggctcttg 1020

gcagtgacca ctgtcccatc accctttacc tagcactgtg acactcccct caagtagctt 1080

catgctggga aatagcctcc tctcctccag gagaccagtg cgttatctct tcttcaggtg 1140

tttactcccc tctaaaccaa acttctggtt tcctttaaac aatccaagtg aaataaaagt 1200

cctacttttc aac 1213

<210> 19

<211> 1872

<212> DNA

<213> Rattus norvegicus (Norway rat)


<400> 19
atgatccaaa caacggcccc acccgtcaaa gagagcagct tcgtggaaaa gatgaagaaa 60

acgggaagga acattatcgt attctatggc tcccagacgg gaaccgctga ggagtttgcc 120

aaccggctgt ccaaggatgc ccaccgctac gggatgcggg gcatgtccgc agaccctgaa 180

gagtatgact tggccgacct gagcagcctg cctgagatcg acaagtccct ggtagtcttc 240

tgcatggcca catacggaga gggcgacccc acggacaatg cgcaggactt ctatgactgg 300

ctgcaggaga ctgacgtgga cctcactggg gtcaagtttg ctgtatttgg tcttgggaac 360

aagacctatg agcacttcaa tgccatgggc aagtatgtgg accagcggct ggagcagctt 420

ggcgcccagc gcatctttga gttgggcctt ggtgatgatg acgggaactt ggaagaggat 480

ttcatcacgt ggagggagca gttctggcca gctgtgtgcg agttctttgg ggtagaagcc 540

actggggagg agtcgagcat tcgccagtat gagctcgtgg tccacgaaga catggacgta 600

gccaaggtgt acacgggtga gatgggccgt ctgaagagct acgagaacca gaaacccccc 660

ttcgatgcta agaatccatt cctggctgct gtcaccgcca accggaagct gaaccaaggc 720

actgagcggc atctaatgca cctggagttg gacatctcag actccaagat caggtatgaa 780

tctggagatc acgtggctgt gtacccagcc aatgactcag ccctggtcaa ccagattggg 840

gagatcctgg gagctgacct ggatgtcatc atgtctctaa acaatctcga tgaggagtca 900

aacaagaagc atccgttccc ctgccccacc acctaccgca cggccctcac ctactacctg 960

gacatcacta acccgccacg caccaatgtg ctctacgaac tggcacagta cgcctcagag 1020

ccctcggagc aggagcacct gcacaagatg gcgtcatcct caggcgaggg caaggagctg 1080

tacctgagct gggtggtgga agcccggagg cacatcctag ccatcctcca agactaccca 1140

tcactgcggc cacccatcga ccacctgtgt gagctgctgc cacgcctgca ggcccgatac 1200

tactccattg cctcatcctc caaggtccac cccaactccg tgcacatctg tgccgtggcc 1260

gtggagtacg aagcgaagtc tggccgagtg aacaaggggg tggccactag ctggcttcgg 1320

gccaaggaac cagcaggcga gaatggcggc cgcgccctgg tacccatgtt cgtgcgcaaa 1380

tctcagttcc gcttgccttt caagtccacc acacctgtca tcatggtggg ccccggcact 1440

gggattgccc ctttcatggg cttcatccag gaacgagctt ggcttcgaga gcaaggcaag 1500

gaggtgggag agacgctgct atactatggc tgccggcgct cggatgagga ctatctgtac 1560

cgtgaagagc tagcccgctt ccacaaggac ggtgccctca cgcagcttaa tgtggccttt 1620

tcccgggagc aggcccacaa ggtctatgtc cagcaccttc tgaagagaga cagggaacac 1680

ctgtggaagc tgatccacga gggcggtgcc cacatctatg tgtgcgggga tgctcgaaat 1740

atggccaaag atgtgcaaaa cacattctat gacattgtgg ctgagttcgg gcccatggag 1800

cacacccagg ctgtggacta tgttaagaag ctgatgacca agggccgcta ctcactagat 1860

gtgtggagct ag 1872

<210> 20

<211> 4689

<212> DNA

<213> Rattus norvegicus (Norway rat)

<400> 20
ctgaacaccc atccggtgag acaggagggc aggtttccaa gcgccgctcc gccacacttg 60

atctcagcgc tgggaaagtg aagcgagcaa ccgacagcca cgatgccggc ccacatgctc 120

caagagatct ccagttccta cacgaccacc actaccatca cagagcctcc ttccggaaac 180

ctgcagaatg gacgagagaa gatgaagaag gtgcccctct atctggaaga agacatccgt 240

cctgaaatga gagaagatat ccacgacccc agctaccagg atgaggaggg gcccccgccc 300

aagctggagt acgtctggag gaacatcatc ctcatggccc tgctgcacgt gggagccctg 360

tacgggatca cactgatccc ctcctccaag gtctacacct tgctctgggg gatattttac 420

taccttatca gtgccctggg catcacagcc ggggctcatc gcttgtggag ccacaggact 480

tacaaggctc ggctgcccct gaggatcttc ctcatcattg ccaacaccat ggcgttccag 540

aacgatgtat atgaatgggc ccgggatcac cgcgcccacc acaagttctc tgagacacac 600

gccgaccctc acaactcccg ccgtggcttt ttcttctctc acgtgggttg gctgcttgtg 660

cgcaaacacc cggctgtcaa agagaagggc ggaaagctgg acatgtctga cctgaaagct 720

gagaagctgg tgatgttcca gaggaggtac tacaagcctg gcctcctgct catgtgcttc 780

atcctgccca cactggtgcc ctggtactgc tggggcgaaa ctttcctgca cagcctgttc 840

gtcagcacct tcttgagata cactctggtg ctcaacgcca cctggctggt gaacagcgct 900

gcccacctct atgggtatcg cccctacgac aagaacattc aatctcggga gaacatcctg 960

gtttccctgg ggtccgtggg cgagggcttc cacaactacc atcacgcctt cccctacgac 1020

tactctgcca gtgagtaccg ctggcacatc aacttcacca cgttcttcat cgactgcatg 1080

gctgccctgg gcctggctta cgaccggaaa aaagtttcta aggccgctgt cttagccagg 1140

attaagagaa ctggagatgg gagccacaag agtagctgag ttctgggctt ctgagttcct 1200

gttccaaaag ttttctgggc agagatttaa tattctgttg attaactact ggataatgct 1260

atcggaatgt taatgatgca tttaaactat tccggtacag tattatgaga aagctttgat 1320

catgttttga ggtaatgcat attttaatta gccaggatta accatgccac aatacatata 1380

tatttctaag catacatgat aaatgcatat acaattgtgc acatcagctt taaataataa 1440

cgatgaattt gaacattcta tacagagagt attaaagcca aggaacatgc tgttttgatg 1500

ctagggcaag catggtgctc tttccctgtt tgcactgcat ccagcttcgt tccttcctct 1560

gtcatcacca tcttcaggca aatagctgac cagccactgg cctgtgtcca ccttccaaag 1620

cccaggcaac ctttctgctt tctgaaatac tgatccttcc tcctggatat gtctttcctt 1680

gttctgaact ttgagattgc tggctcagac ttaagataga acaaaaacct tcctggaagt 1740

tcccactggt gaacttcact ataggttttg ccagagggaa tagggaaatt atcttgattg 1800

tagcaagtag atcaccttga gagaagaatt agcacgcacg gtgtggtttg taagtaagga 1860

tgggagagag ctaggaaaag gtggccatat ctaatggcct acttaccaag cccccaggcc 1920

tctctgcata ggcatggagt ccttccagtc catattctga acaacagttt ttagtgagat 1980

ttgaataatt aaattatttt cagagtgaag ggaatgggaa atgcatggtc tgtgccaggg 2040

gcggagagtg gggaagggtt agcttttggt aactgaagat ttcttcaatg gaaagactct 2100

taatgttaaa tgtgcctaga actggtaact aaacagctga catttggagg gaatgtggtg 2160

aaaactatgt aaatataagc aaaatccgca catgaaaata tttcacttag aaaaagtgtt 2220

gctaaagaaa aaaattcgaa ggacaagagg tgttgcctgg gttgccagtt tctttcgtgg 2280

cagggcagga aatagtgagg ttgagaggca gcatctgtaa gtagatgcta agaggtacat 2340

ttctaaatga agcccttggg gaacatctgc cagggatcta gagagtgctg gtttcatcca 2400

ttgttttaat ttcctccttg gattgagtag aaacgtggct tcttgtggtg ttgaaccttc 2460

cccgacttct ttgctttgtt gccatctagc ctgcccagca ctgcctaggg agcgcctacc 2520

caccctgatt tcttctgcca gctcctaccc accctgattt cttctgccac ttctttcttt 2580

ggcattcatt tttctttccc tggacaggaa gctagttacc cgagtgtatc aggagcacct 2640

ggcttgcctt tgtccaggcc cattcctcct ggtatccttg ggtgtggaac tgctggacag 2700

cttcaggaag cagagccagg tgccactttt ctgtggctcc agatcctccc tacctccacc 2760

cctatgtgcc tctgccacag taatttcaga aaagcttggt acaccctcta gcaacatctt 2820

ggttcagaga gtctctctgg cttgtgagtt aacagctcag ccaaatgctg tgttgtctca 2880

gtgaggcaac cactgaacga gagggttggt tgttgggctg tctctgttgg agtcagagta 2940

gacaacatgt tcacttcttc ctgtctgctt tgcaaggtgt ctgtgactga tcaaggcagg 3000

cagggagtgg tatagtactt ctgaaaggaa gaagttccct atttatctgt taaaagacca 3060

gaggcatggg caagtgctca ggacctcact tcgggaagag ggtctgcttc ctgaaagcca 3120

gtgtataagg aaaagcttcg actgaacctg gtatctaagg taccatgaac taacactcta 3180

tcacacagcc cagggcaaac agtggtatag aaaagtctag aagaaaacat cacatcagta 3240

ttttagaacc atccaccttt actgtccctg tagcccaatc cagagcctgg cttttagatc 3300

tggctgcgta cagtgccaaa tgctcagttt acttgtaaga tcagagcctt tctttctgtg 3360

ttaattgaac acatgctctg agctgttttg ttgaagtaga aaatctagta gaaaatcact 3420

gtagatctga tgacctggaa acctctggaa atgccttcgg gtggttttat ttttccaggt 3480

cacagatgcc tgatttataa gatgaacaat gaaatcagct ttctttctct tctgatctta 3540

ttccctggat ttgatgcagc ccatgttcca aagcaaagct acatagagac ttcaggctga 3600

ggtcctggtg tctttaagca aggacactct ttcagatcct ctcagaggcg tatttgtaac 3660

agtttccaca tgaatgtact aatcatgttg ggtgccttat cgctttccta atctacagtg 3720

cctgttagcc cagcctcact ccttccctta accctgagat cccgtagatg gagcgggtgt 3780

agacaagagg ctggctggag gatcatcagt acagtgattt gcagaaatct tttatgggct 3840

tcattttgga aaaatttgtt tttagtaaat gctcaaattt ggtgggaagt aatttgaatg 3900

tatttgattg tattgtcctc attacaattg ttaattagac acgttctaca tgacagtagt 3960

actcagggga gttttctccc tgggctgcta atatctgggt gtaatctctg aaactgtagg 4020

tccagtgagg cggcgtgaaa agttagcaaa gccaccatct gctggtggtt taagccaagg 4080

cctctcttct tcgccactga cttgctatgt tatcctttct cttgtaacaa acccatccca 4140

gagatgaggc ctttaatcat cccaagaacc tcctgggcta agtatctgac agtctcacat 4200

ctcaacatcg tgtattaagt gtccagaaca tcagctcagg aggacacgct gaaactctca 4260

ctctgggcga gtcctgataa aacaggctcc taatactgac ctacctcaag ggcagttctg 4320

aggtgattag agccttttct ttttttaagc aagtgtttgg agatgcatgg cagaggtttt 4380

cctgcagact tgtaaagagc tagatgtgtt ctaagaccat ctttagtcta cattgctctt 4440

cccagagatc tacaactatg acccagccaa agtgcaagcc tacctactca ctgccataaa 4500

aactattcag gaaacctttt cctggcctga atgagatttt gttttttttt tttttatgtg 4560

gggcggttat ttgtgaccca agtgtaattt ggatgatttt taattaatat caactcttga 4620

agcctaatat gtactggatt tgagattgca tttgttccta ataaaagtgg atctagctgt 4680

gctgcctgg 4689

<210> 21

<211> 2455

<212> DNA

<213> Rattus norvegicus (Norway rat)

<400> 21
agagaagcag agaagcagag caagcggcgc gttcccgaac ctcgggcaag accagcctct 60

cccagagcat ccccaccgcg aacgcaccct tctccagagc ataccccagc ggaggccacc 120

cttccccaga gcatccccgc cgccaagcgc aaccttccag aagcagaccg cagcgacatg 180

gccaagaaaa cagcgatcgg catcgacctg ggcaccacct actcgtgcgt gggcgtgttc 240

cagcacggca aggtggagat catcgccaac gaccagggca accgcacgac ccccagctac 300

gtggccttca ccgacaccga gcggctcatc ggggacgccg ccaagaacca ggtggcgctg 360

aacccgcaga acaccgtgtt cgacgcgaag cggctgatcg gccgcaagtt cggcgacccg 420

gtggtgcagt cggacatgaa gcactggccc ttccaggtgg tgaacgacgg cgacaagccc 480

aaggtgcagg tgaactacaa gggcgagaac cggtcgttct acccggagga gatctcgtcc 540

atggtgctga ccaagatgaa ggagatcgcc gaggcgtacc tgggccaccc ggtgaccaac 600

gcggtgatca ccgtgcccgc ctacttcaac gactcgcagc ggcaggccac caaggacgcg 660

ggcgtgatcg cgggtctgaa cgtgctgcgg atcatcaacg agcccacggc ggccgccatc 720

gcctatgggc tggaccggac cggcaagggc gagcgcaacg tgctcatctt cgacctgggg 780

ggcggcacgt tcgacgtgtc catcctgacg atcgacgacg gcatcttcga ggtgaaggcc 840

acggcgggcg acaccgacct gggcggggag gacttcgaca accggctggt gagccacttc 900

gtggaggagt tcaagaggaa gcacaagaag gacatcagcc agaacaagcg cgcggtgcgg 960

cgcctgcgca cggcgtgcga gagggccaag aggacgctgt cgtccagcac ccaggccagc 1020

ctggagatcg actctctgtt cgagggcatc gacttctaca cgtccatcac gcgggcgcgg 1080

ttcgaggagc tgtgctcgga cctgttccgc ggcacgctgg agcccgtgga gaaggccctg 1140

cgcgacgcca agctggacaa ggcgcagatc cacgacctgg tgctggtggg cggctcgacg 1200

cgcatcccca aggtgcagaa gctgctgcag gacttcttca acgggcgcga cctgaacaag 1260

agcatcaatc cggacgaggc ggtggcctac ggggcggcgg tgcaggcggc catcctgatg 1320

ggggacaagt cggagaacgt gcaggacctg ctgctgctgg acgtggcgcc gctgtcgctg 1380

ggtctggaga ccgcgggcgg cgtgatgacg gcgctcatca agcgcaactc caccatcccc 1440

accaagcaga cgcagacctt caccacctac tcggacaacc agcccggggt gctgatccag 1500

gtgtacgagg gcgagagggc catgacgcgc gacaacaacc tgctggggcg cttcgagttg 1560

agcggcatcc cgccggctcc caggggcgtg ccccagatcg aggtgacctt cgacatcgac 1620

gccaacggca tcctgaacgt cacggccacg gacaagagca ccggcaaggc caacaagatc 1680

accatcacca acgacaaggg ccgcctgagc aaggaggaga tcgagcgcat ggtgcaggag 1740

gccgagcgct acaaggcgga ggacgaggtg cagcgcgaga gggtggctgc caagaatgcg 1800

ctcgagtcct acgccttcaa tatgaagagc gccgtggagg acgagggtct caagggcaag 1860

atcagcgagg ctgacaagaa gaaggtgctg gacaagtgcc aggaggtcat ctcctggctg 1920

gactctaaca cgctggctga gaaagaggag ttcgtgcaca agcgggagga gctggagcgg 1980

gtgtgcaacc cgatcatcag cgggctgtat caggtgcggg gtgctcccgg ggctgggggc 2040

ttcggggccc aggcgcccaa gggaggctct gggtcggggc ccaccatcga ggaggtggat 2100

tagaggcttt tctggctctc agggtgttgg ctagagacag actcttgatg gctgctggtg 2160

cacgattctt atcaagttac tccttctctc cggagttcag tttaaagtta cagcctttta 2220

tacggtaatt gatttgagtt tgttacattt tgtatgctcg tgggtttttt atatattcaa 2280

attaaggttg catgttcttt gcgtttaatc taagtagctg tgtaaaaatg gtgtttcctt 2340

cctgcgaaca cctcagcact gccaccctgt gtacagtttt ttccttgcat ccctacaaac 2400

tgagaaaaaa agttatcttt tgtaacttaa acattcaaaa taaaatgtta caagt 2455

<210> 22

<211> 3115

<212> DNA

<213> Rattus sp.

<400> 22
gaattccggt ccgcagccta ggggcggggc gcggacgacg gagccatgga ttgcacattt 60

gaagacatgc ttcagctcat caacaaccaa gacagtgact tccctggcct atttgatgcc 120

ccctatgctg ggggtgagac aggagacaca ggccccagca gccctggtgc cagctctcct 180

gagagcttct cttctcctgc ttctctgggc tcctctctgg aagccttcct gggaggaccc 240

aaggtgacac ctgcaccctt gtcccctcca ccatcggcac ccactgctgt aaagatgtac 300

ccgtccgtgc cccccttctc ccctgggcct ggaatcaaag aggagccagt gccactcacc 360

atcctgcagc ccccagcacc acagccatcg ccagggaccc tgttgcctcc gagcttccct 420
cctccacctg tgcagctcag ccctgctcct gtgctggggt actcaagcct gccttccggc 480
ttctcaggaa cccttcctgg gaacacccag cagacgccat ctagcctgcc actgggctcc 540
acgccaggaa tctcgcccac ccccttacac acccaggtcc agagctcggc cgcccagcag 600
ccgccgccag cctcagcagc ccctagaatg agcactgtgg cctcacagat ccagcaggtc 660
cccgttgtac tgcagccaca cttcatcaag gcagactcgc tgctgctgac agctgtaaag 720
acagacacag gagccacaat gaagaccgca ggcatcaaca ccctggctcc tgcgacagcc 780
gtgcaggcag gccccttgca gaccctggtg agtggaggga ccatcctggc cacagtccca 840
ctggtggtgg acacagacaa actgcccatc caccgactag cagctggtgg caaggccctg 900
ggctcagctc agagccgtgg tgagaagcgc acagcccaca atgccattga gaagcgctac 960
cgttcctcta tcaatgacaa gattgtggag ctcaaggacc tggtggtggg cactgaggca 1020
aagctgaata aatctgctgt cttgcgcaag gccatcgact acatccgctt cttacagcac 1080
agcaaccaga aactcaagca ggagaacctg accctgcgaa gtgctcacaa aagcaaatca 1140
ctgaaagacc tggtgtcagc ttgtggcagt ggaggaggca cagatgtgtc tatggagggc 1200
atgaaacctg aagtggtaga aacgctgacc cctccaccct cagacgccgg ctcaccctcc 1260
cagagtagcc ccttgtcctt gggcagcaga ggcagcagca gtggtggcag tgactctgag 1320
cccgacagcc cagcctttga ggataaccag gtgaaagccc agcggctgcc ttcacatagc 1380
cgaggcatgc tggacccgtc ccgcctggcc ctgtgtgtac tggtcttcct gtgtctgacc 1440
tgcaacccat tggcctcact gtttggctgg ggcatcctca ctccctctga tgcttcgggt 1500
gtgcaccgta gttctgggcg cagcatgctg gaggccgaga gcagagatgg ctctaattgg 1560
acccagtggt tgctgccacc cctagtctgg ctggccaatg gactactagt gttggcctgc 1620
ttggctcttc tctttgtcta cggggaacct gtgaccaggc cacactccgg cccggctgta 1680
cacttctgga gacatcgcaa acaagctgac ctggatttgg cccggggaga ttttgcccag 1740
gccgctcaac agctgtggct ggccttgcaa gccctgggcc ggcccctgcc cacctcaaac 1800
ctggatctgg cctgcagcct gctttggaac ctcgtccgcc acctgctgca gcgtctttgg 1860
gtgggccgct ggctggcagg ccaggctggg ggcctgcaga gggactacag gctgagaaag 1920
gatgctcgtg ccagtgcccg agatgcggct gtcgtctacc ataagctgca ccagctgcat 1980
gccatgggca agtacacagg aggccatctt gttgcttcta acctggcact gagtgccctt 2040
aacctggctg agtgtgcagg agatgctata tccatggcaa cactggcaga gatctacgtg 2100
gcagctgccc taagggtcaa aaccagcctc cccagagcct tgcacttctt gacacgtttc 2160
ttcctaagta gtgcccgcca ggcctgcctg gcacagagtg gtgcagtgcc tcttgccatg 2220
cagtggctct gccaccctgt aggtcaccgt ttcttcgtgg atggggactg ggctgtacac 2280
ggtgcccccc aggagagtct gtacagcgtg gctgggaacc cagtggatcc actggcccag 2340
gtgacccgac tattctgtga acatctcctg gagcgagcat tgaactgtat cgctcagccc 2400

agcccagggg cagctgatgg acacagggag ttctcagatg cccttggata tctacagttg 2460

ctaaatagct gttctgacgc tgtcggagct cctgcgtgca gcttctctgt cagttccagc 2520

atggctacca ccactggcac agacccagtg gccaagtggt gggcctcact gacagccgtg 2580

gtgatccact ggctgaggcg ggatgaggag gcagctgaac gcttataccc actggtagag 2640

cacattcccc aagtgctgca ggaaactgag agacccttcc cagggcagct ctgtactcct 2700

tcaaggctgc ccgggctctg ctggaccaca gaaaggtgga atccagccca gccagcctgg 2760

ccatctgtga gaaggccagt gggtactgcg ggacagctta gcctctacat caactgccag 2820

ttccattgac aaggccgatg cagctgctcc tgtgtgatct acttcttgtg gcccgcacca 2880

gcctatgcgg cgccaacagt cagcagcttc agcccaggga gctcacggta ccagcaatgg 2940

accccaggcc tctgctctgg agctgcgtgg tttccaacat gacctgagca gcctgaggcg 3000

cttggcacag agcttcggcc tgctatgagg agggtcttcc tacatgaggc cacagctcgg 3060

ctgatggcag gagcaagtcc tgcccggaca caccagctcc tggaccgcgg aattc 3115

<210> 23

<211> 1346

<212> DNA

<213> Rattus norvegicus (Norway rat)

<400> 23
gaattccggg agacaacctt gtcaagcacc taattgtgcc atccgtgcga tgccagccag 60

ctccatccac cttacaggag tgaagtcagc gttcatatac gctgtgtggt ctggcagctg 120

agatagtgtt tcccaaaggg actgtggaat gttacctcag caggcattca tttcgcgttt 180

ggaaaatcgt ctccaaatga actttctttc cgtgctgggt ctgcagaaca tttcagaagc 240

ggagctagca atctgcttca actttttttc ccccggtgct tgcagcagga gatgcctgaa 300

gggagctgct gcgtggtggt ctgaaacata gttcaaggaa tagaggtcaa ctgcctagag 360

cggtgcaggc agggcacagg ctggctttgt actctgagtc tcaaggtatt tcccagtgcc 420

tgagccaggg ggaaggtggg gggaggaagg gaaggagctg ccacttggat cgctattctg 480

ggtagccaag acagatagcc atacaatgga aattagtggc ttcaacttgg gggaaaggat 540

ggactctaac ttcgagctgt gcagttcgcc cattgtttga atggacaaaa ggcagtttac 600

ccaggctcct agcatacctg cctgggtgtc caaatgtaac tagatgcttt cacaaacccc 660

acccacaaaa caacacatgt tcttaagtcc tgggctttgt tttcacttcg gcctcataat 720

acccactctg acctgctgtg taaacgaccc gggacgtacc aaaatgagcg cacctccaat 780

aaagatacac atcatgtcgt cttcacatct cttctacctg gcactctgct tgctcacctt 840

taccagctcg gccacagccg gaccagagac cctttgcggg gctgagctgg tggacgctct 900

tcagttcgtg tgtggaccaa ggggctttta cttcaacaag cccacaggct atggctccag 960

cattcggagg gcaccacaga cgggcattgt ggatgagtgt tgcttccgga gctgtgatct 1020

gaggaggctg gagatgtact gtgtccgctg caagcctaca aagtcagctc gttccatccg 1080

ggcccagcgc cacactgaca tgcccaagac tcagaaggaa gtacacttga agaacacaag 1140

tagaggaagt gcaggaaaca agacctacag aatgtaggag gagcctcccg aggaacagaa 1200

aatgccacgt caccgcaaga tcctttgctg cttgagcaac ctgcaaaaca tcggaacacc 1260

tgccaaatat caataatgag ttcaatacca tttcagagat gggcatttcc ctcaatgaaa 1320

tacacaagta aacattcccg gaattc 1346

<210> 24

<211> 860

<212> DNA

<213> Rattus norvegicus (Norway rat)

<400> 24
gaattcagac aacaacaaca gctgtgaagg ccgggaccca gatgtgtttt cagataggca 60

catccttgcc acggtgggag acatctttgg ggcgggcata gagacaacta ccactgtgct 120

caagtggatc ctggctttcc tggtgcacaa tcctgaggtg aagaagaaga tccaaaagga 180

gattgaccag tacgtaggct tcagccgaac accaactttc aatgaccggt ctcacctcct 240

catgctggag gccactatcc gagaagtgct gcgtatcagg ccggtggctc ccatgctcat 300

cccccacaag gctaacgtcg actccagcat tggagagttt actgtcccca aggacacaca 360

tgtggtcgtc aatctctggg cactgcatca cgatgagaat gaatgggacc agccagatca 420

gttcatgcct gaacgcttct tagatccaac gggaagccat ctcattacac ccacgcagag 480

ttacttgccc ttcggagctg gtccccgatc ctgcatcgga gaggctctgg cccgtcagga 540

gctctttgtc ttcacggcct tgctactgca gaggtttgac ttggatgtgt cagatgataa 600

acaactgccc cgcctggagg gtgatcccaa ggtagtcttt ctgatcgacc ctttcaaagt 660

aaagatcacg gtgcgccagg catggatgga tgcacaggct gagcttacca cctagaggcc 720

acaactaaca tccccgatcc atacctcaac acccacagta caatcttaga ggtgctagtc 780

ccagtgcctc ctacggtcct cctccctcct acccatttct agttggcagc aatggggggt 840

gatacacata aattaaagtt 860

<210> 25

<211> 3157

<212> DNA

<213> Rattus norvegicus (Norway rat)

<400> 25
gttgtttcag gatgtgcttc tccgtccgct ccagggagcg gtcacttctt tgcagagcgt 60
ggtccatctc caaagtcaag cttgccccct cttgttatcc caccaagtga aagctcggga 120
cagcgtgaag aggatcaagt tctgtgtggt tttaagaaac tctcagtgaa tggggtgtgc 180
gcttccacac ctccacttac acccattcaa agctgctctt ccccgtttcc ctgtgcagct 240
ccctgtgatc gcagttcccg accgctccca ccattgccca tctctgagga cccatctctg 300
gatgaggccg actgtgaggt ggaatttcta accagtgcag atacagactt ccttttagaa 360
gactgcgtgc cttccgattt caaatacgat gttcctggca ggcgaagctt ccgtgggtgc 420
ggacagatca attatgcgta ttttgacagc ccaactgttt ctgtggcaga tcttagctgt 480
gcatctgacc agaacagagt tgttccagat ccaaaccctc ccccgcctca aagccatcgc 540
agattaagga ggtctcactc aggaccagcc gggtcgttta acaagccagc catccggata 600
tctagctgca cacacagagc ctctcctagc tcggatgaag acaagcctga gatcccgccc 660
cgggttccta tacctcccag gccagccaag ccagactaca gaaggtggtc agcagaagtg 720
acctctaaca catacagtga tgaagatagg cctcccaaag tccccccgag agaacctttg 780
tctcggagta actcccgtac cccaagtcct aaaagccttc cgtcttacct caatggggtc 840
atgcctccaa cacagagctt tgcccccgac cccaagtatg tcagcagcaa agccctgcag 900
agacagagca gcgaagggtc tgccaaggcc ccctgcatcc tgcccatcat tgagaatggg 960
aagaaggtca gctccacgca ttattaccta ctacctgaga gaccgccata ccttgacaaa 1020
tatgagaagt attttaggga agcagaagaa gcaaacccaa gcacccagat tcagccatta 1080
cctgctgcct gtggtatggt ctctgccaca gacaagctgg cctccagaat gaaaatggat 1140
gtgggcggcc acgggaagcg caaacactta tcctacgtgg tttctccgta gatatggggt 1200
catggttcaa cagaagttac atggaacgga tggctgccaa gtttccaatt tgaggttcat 1260
agaacagtgt caagtggcaa catgaagtgg tggactctgc cttggtgagg aaggcataga 1320
gccgttatga ggtgctgttg tgctgggagt ccctgaccta tcagcatagg aagaaaaaag 1380
tatgatttaa agatgtgcta gagggacact tttacatttg actacattat atacctatgt 1440
ataaacgtgc ggtgtaacca tagaccatag ctgcaggata accaattagt cactctagag 1500
taatctatat tcagaacaat tcaaacgagc tggaggcaca gctccagaca gtgtgaaaat 1560
tgagcaaacg ggagagagca gtactgttga tcagttataa atgtagagat tcctggcatt 1620
caggcctgct atctagtttg ttttcttccc cttccctgcc agcagtcttc tccatacacg 1680
acagggcgtg ctcttcgcca ggcctgtaac gtcttgttga aatcgttcta tggcctaata 1740
cttgccgctc tgggcgtttg tcttgagagg agaggacagc cgtttctgga ccatgttatc 1800
tatcacctgt gtatgtctct cttggaaatg gacagaattg gtgacttttc catgctattc 1860
ctgcttttcc cgtccactga agaggctttc aagagtgcag ttgagtggtg ctggccgtca 1920
gtgttgggta taagttttat agaccagccc agtgattagc catgattgag agttatcgtg 1980
gggtgtatgg gatgttgttt tgtttttgag actttaaagt acaacacaag ctggtcttgt 2040

gttttggttc ctattcagta tttcctgggg attgtttgct ttttaagtga aacacttctg 2100

accaatagca cagaacgtct taatgccaga ggtcacttca gcatcttcct gcttagaaaa 2160

ctcacagctg gctgcctcac tgccctgaga gtcagtgaga cgtgtagctt gtgttcaatt 2220

tttacatcct ctgattgttt atcttgtata gataagcaca aagagaaggt gcttgctaac 2280

agagggacac cgctgccatg tcccaacaag ctgttcagtt taaactgctg aatgacatta 2340

tttgagctat ttaaagctta tttttagtat gaactaaatg aaggttaaaa catgctttag 2400

aaaatgcact gatctccgca ctgtgtgtac agtattggac aaaggatttg ttcattttgt 2460

tgcattattt tgaatattgt cttttcattt taataaagtt atattactta tttatgacac 2520

cgttaatgtc ttgtctaaac tcttatgtct caagcaattg gtgttatttt ttcaagcatg 2580

acagtgtttg gggttcaata catgttgaaa ctattgtaga tttctaaatg acaatctgag 2640

caactttact tgggtgttat aaaaatgtat gggctgaaga cttggtcagt tgttgaagtg 2700

cttactctgg acttggtccc gagtatcacg taaacagggc atgcccataa gtccagcact 2760

tgagatggtg gctggagaat cagcagttca caacgtcatc tttggttccc ctaggccaca 2820

tgaatccctg tctcaaaaaa gaaaaagaaa aaaagtatgg caaaaatcaa gccttttttg 2880

ctctcacctg gaacaataaa cagtaaactt ggttctggag cttctgtgga aacagtaaca 2940

cgaggaaggc caggctgtgc agtccgtagg acactctgtg tgcagacttc ttgagcttta 3000

ggactcagtc tgggtcactc tgggcccttt gtgaccttca ctgcttgctt gtctgaggcc 3060

ttaatccccg tggccaggca ctgcagagag ccttgcagac tgctgggctg ctgtatccaa 3120

gctggtggcc ctgtgtgtca ctctagcttg ggaattc 3157

<210> 26

<211> 567

<212> DNA

<213> Rattus sp.

<400> 26
gatgcctgac aattggacaa gtccctttct gacaacagac cattatgttg aatcctgcct 60

gcaagacaag ctgctcgaat tcacttaagg agctggaggg cagtgctgaa gggggccagg 120

ttctcacagg acttaagcca ccgctgcaca ttggtgggcg ctgccccact gcttccccca 180

gtctgctgga gcacggacca cagcaccaca tctgccacag tgagctcatt cccaaccaac 240

cacgggcttt tccccaaagc ggagttcata gagcggaaaa cagccgcttt ttccttactg 300

ctcccttctc tcagctgaaa catggcgata tccacccagc tgtcgatgag ggttaggtgg 360

acagcgttat gcttctgacc aaatagagag aacaggaagc gtgcgatgtt cccttctcct 420

tcaatggggc acatcgtttg tacactgaac ttcatctgtg tcttgggcac gttcttccaa 480

atcagagtga agcccagctg atactcgtgg cgggactgtt ttctagcctg ctccccgaag 540

cacttgagaa gattctcagg tacattc                                          567

<210>  27

<211>  583

<212>  DNA

<213>  Rattus sp.


<400>  27
accaaaaaat attccaggta cctcttcaga tagatcactt tcaaaatata tttatagacc      60

ctttgccagt gctgtcatcc caaaagctgc cagaactctg gaagtcccgt ggtccagccc     120

tcagcccagg aatataggta gagtctccat gatgggtggt aagagagcag tacctgggct     180

gtgctggctc cttctttcct cataggtaag gtatgacatt aaaggacaat aaatacgtgt     240

tgaatattag caaaaaaaga ttacaagtcc tatgctttgt tgccgtggct ctttgaagtt     300

catctacctc actggcagct ggaagacagt tctgctgctt cgttctcagc tcttggcctt     360

ctgggctgtg tccacactaa accctatggc tatactgtct tgatttttca agaattcact     420

taagaggaag aaagcagaaa ccttagctgc aagtacattc cccaatttaa attgtggtag     480

attagtgata acatgaaaaa ctgagggatg atggtggtac agtcacatct ccaggggcca     540

agacatcttg cttgtgaact cagtgagtca caggaggaaa agc                       583

<210>  28

<211>  450

<212>  DNA

<213>  Rattus sp.


<400>  28
aagtcaaata atttattagc acatagaacc ctccagaaaa atattgcctt gtggtctgtt      60

gggcaggagc aaaggcagca cctgaatgtg gccatcatct tcagtgtgaa cttctaagtc     120

ctagcccagg tgggtagccc tcacttctca tagcttcaga atatggtatt ggggcaggtg     180

ggtggggcag ccctgctact cattttgaac actgaagatc aggtcattcc atggttatca     240

ctttaataat gtaaaactca agcaggtgat gagctctcac tcagctgtct aaatgcaccc     300

tctacctaca ggagcagtga ctaaggaccc tttggtcccc acatccctgg tcggcggtgg     360

aggaagggca ggccaggcag gcatgaggca caaacagtct ttactgggtt cacaccaaga     420

gtccattggt cttgaggacc tctgtgaact                                      450

<210>  29

<211>  711

<212>  DNA

<213>  Rattus sp.

<400>  29
caaacttttt ttttattttt tttttttttt ttgcgttgaa aatcatttaa tgagaattat      60

actttttctc tatcagatta gatctgttct agtgattatt gtctggtgtt acaagctcat     120

ctgtctcaga gttggttagt gtgcatgaag ctacttaaga aagatatgga caagcacagc     180

tcactggtcc ccctgagcac ctcacaagac tgggaacatc ctctcaaggg ttaaaacaga     240

cgggcatacg ggctgagaat ttattttgaa ggcaggataa acaggccagc tctaagaaga     300

tactgttttg aaagctcaca cgtttcataa gtttctgagt tcaggcagtc tcagttctca     360

tccactgagt cctgggccct caaaagccct ccaggtccac cacagaggct gttctaactg     420

ggtgctgagc ctatacaaag ccctggcact cacacaactg ccattcccca agatcaactc     480

gtctcctgta aatatgtgct gtttgtatcc ggcacagggc tgatgaagat ggatcccgat     540

ggagcctact gtgcattact tcctgatgtg cagagatgtg tgtggtgaga ctgtgcggga     600

tgctgggagc agtgtgcagt gcgttgggag actgagaagt gaaagccctg ttggtttgag     660

gggaaagcaa ggaaatgtga cctttcccat agctactccc acagtgagaa a             711

<210>  30

<211>  654

<212>  DNA

<213>  Rattus sp.

<400>  30
aactggggac atgtgtttat tcagcagaaa gggggagccc gttggtgggg cagcgttaac      60

acaggtggat ggggagagct gatcaaataa gagggatcag atgcttggtg gaatcatggc     120

tggtggggca gccaggtgat tctctggcaa caggaaagat ggagcggcag acagacaggt     180

gggacctgaa ccatcaggcc actgcacatc ccagtcagcc acgctcaccg tctgttcagt     240

tgtcaatgca ttggtcggtg ggaacagaga aaacgatgtt gttatccttg aggcccagag     300

acttggcaaa gctgacgaat cgctccttca gttcatcgga cagccccttg gttcttccgt     360

acagggtgac tttgaagtac tgtttgtttt cagaggtctt ctggaaaaat accatggcaa     420

actggtcgta gtcagtgtcg gccacttgca catcgtagct ctgtatctga gggtagctgt     480

gaatattccc cagggtgaac tggccaggcc tggagcttgg aacgaatgtt ctgatccagt     540

agcgacagcc ctggcccctg acgaggatgg aagtgacgtt gtagctattg tcttcctgta     600

actcatagat ggtgctgtac atggtaaagc ggctttgtct ttctttctgg accg          654

<210>  31

<211>  421

<212>  DNA

<213>  Rattus norvegicus (Norway rat)


<400>  31
tttttttttt tttttttgcc ttttaaaaaa taagatttat tttaatttac gtgtattctg      60

gagaggacta tgtacatttg agtgcagatg cctgaggcag ctgaggcact ggatcccctg     120

gagcttggct ttcaggcagt tgagtgcctg acatgggtgc tgggaactga acttgggtct     180

ttggcaagag cagtttaggc tcttgaccac tgagctggct ccgcagcctc ccacactggc     240

ctttgaagaa atactgatct aagagagcgt ggttccactc agtagctctt gggtctcagt     300

ccaggtctat tcccaggagg cctagtggat cctgcgggtc gtgtagtcca gaaccatgct     360

ggccgcacca agcagggccg ggtcaaccaa gtctgaaacc actacatcca catcctgcac     420

g                                                                     421

<210>  32

<211>  644

<212>  DNA

<213>  Rattus sp.


<400>  32
gctgtgtgat agttctttat ttcaccattt aagagaaaga aagatggagg aaaggtaaac      60

agtgttcagg cttcagcttt tgccagggga aggcttcggg tcatcgagac cccaaggtat     120

tgccaggtgc acaaatctgg attccgtggc aggcaggcaa agtgatcgct ctggtagccc     180

ttctcagagc ccatgaggat ctgatctgtc cacaagcaat gactgtcact ctccagtttg     240

caagggatgg ctgaacaggg aaacactgtg cacaccccac agccagcact ataggtcttt     300

acgaaggcct tttgctgagc agggctcaga ttatgccagg gaaccaggaa gctgcaggca     360

gtgatgtgca aatttccgtt ccttaaacgg cccgcgatga gaaactcctc gctgcggttc     420

tgggacttgt ggacatatcc acagaggctc tccatggctg gggtgtaggc gaaccggaaa     480

cctgtggcat ttcccacagc gtcgaatcct ttgagcatct tagtcatctt gatctcataa     540

cgctggtata aggtggtctc gatgatttct ggggaaccca tgaatttagc ccttataacc     600

aggtccgagt tgcagaaagc tgtctgtggg tgggttgggg caca                      644

<210>  33

<211>  678

<212>  DNA

<213>  Rattus sp.

<400> 33
atctcatctg tatttacctt tttaaagcag aatgtgattg ggcactgtta ttttcacatt 60

cacaagcctt gctgagttac aagacctagg ggaacttagg gttttgttct cagtactttg 120

gaaaacaagc cacttgggga attcctgtca agttgtttta gcttgtgttt acttctaaga 180

ctagtacatg cagaattaac tacagggaat gaaaaaaatt taagatgaaa cttaagtcat 240

cttaatttgg tctactaaag gaatccagct caacagctaa acacttcaga ccacatagtt 300

aacagtaaca gtaggttaca ttacgtctta caacaaacgt tctatcaacc tcttgagtca 360

aacctatagt atcacagtat cacatgtaga aatttttacc ttcccctagt tttcatgcca 420

cacagatgtt ttaaatgtta acaaaaataa acaaaaatca tggaaaatat attatcagaa 480

ggaatgaagg taagcatcaa acacatagtt ctggtgaagc ctagtctact tcttccatgc 540

gtgatgtgtc atcatctcct tccaggggtg gcatttcttc agttacagca gcactggtat 600

catccacagt aggatcatcc tcatcaatac ctagaccaag cttgatcatc ctgtagatcc 660

tgttagcatc ctcgtgcc 678

<210> 34

<211> 332

<212> DNA

<213> Rattus sp.


<400> 34
atgtgtaggg gggaattcct tttggctaat caaactttgt gaaaacctag gttaaatttt 60

ctaattttta attgaaccat ttaaaaaaaa caaacaaaac ccaaaaaatt cccaaacccc 120

aaagggaaaa aggggtctgg tgagtttgag ctgtcaagtc tataacctgg gtctgtaaca 180

ctgtaaccta atagtgccct gcccatccct cctactttac actttatccc ccaggtaagg 240

ggctcagcac cacagcaagc ttccgtgcag cagctccact ggatttcaat gcaactgagc 300

agcagtcacg ggcccctggc caaaaagttc cc 332

<210> 35

<211> 375

<212> DNA

<213> Rattus norvegicus (Norway rat)


<400> 35
ttgagctgtg aggaatcttt attccctcag gtgaagcatt cagagaaaca gctctgagta 60

cagaattctg ggctcctctc tccaacacca ttctgcttca cacacaccct ccaccaccgg 120

ttttgcttgt gtgtgaacct atcccccagt cttcccatcc tcttcttaag tcccatcatt 180

ctggggcctc ttctccttag cattaactgc aggctcagag acagggagtg caatggctga 240

cagggaacca actaccatcc tccagggcca ttaccttcac cggggagcaa aaggtacatt 300

gcaagtgccc aggagagccc acaggaggaa gatggcatgg tattctaggc tctgtcacag 360

ggagagctct ttgaa 375

<210> 36

<211> 13011

<212> DNA

<213> Rattus norvegicus (Norway rat)

<400> 36
gaattccagg gcatcagcca aggataaccc cagtgagctc attaccccaa aaagttgggc 60

atcagggtag ttccaagaat ctggatagag agtgtcctct ggtaggagca tgtgctcagc 120

ttactcctca cccagtctcc cacgggtgct attccccact gaccaaactc tgtggaagcc 180

ctgggacaaa gcctttggct ccttgcccca aatcagagaa gaaggggcca gagacagggt 240

ctggagtgac cccagatgca tggcacactc tcctcaactc cctggggcct cctgtcagga 300

caggaaagca aaagcatgcc cagctgtcag tcctggattt taatgattaa ctggcacttc 360

aaacacagcc tgtgccaaac aggagtcttg gattccacca tctttttctt ttctttcttt 420

tctttctttc tttttttttt ttgatatttt tatttacatt tcaaatgtta ttcccttccc 480

cggtttccct ttctatgagg atgttccccc tccccaacca ccccccattc ctgcctctct 540

ctctctctct ctctctctct cacacacaca cacacacaca cacacacaca cacacacaca 600

ctcctctgta ctgggggtgg ggtgtgggtg tccagccttg gcaggaccaa gggcttccca 660

ttggtgccca acaaagctgt cctctgctac atatgcagct gaagccatgg gtctgtccat 720

gtgtactctt tgggtagtgg tttagtccct ggagctctgg ttggttggca ttgttgttca 780

tatggggtct caagcccctt cagctccttc aatcctttct ctaaagggtt ccaccatctt 840

aaccctactt ccacacttac ctcagtgaac aacagacact tctcccctcc cgcctgcttg 900

ggttatatca gggccccttg aaaagccctc tgtgcctttt ctccctacag ctcctcccta 960

attctttggc tcaagagaag gcccaccttg acctggttgg cctttgtgac tattgtggga 1020

ggtcagaatc cactggagac ctcacccagt ccgaagcctt atgtctcacc tccccaggaa 1080

agccctttgc tcccctatcc caggaataca gggtgttagc tatcacatct tcaaaggttg 1140

gcttcagaga aagatgactt cctcctgctt gcagtacctc agcactggca gatggatggg 1200

acatttgacc ccagagcaaa accagacttc tccatgttgt actccaagca ctgaaagcct 1260

cccttaccag tacagctcct cattccactc tgccttccct tttccaaaac acatctcaca 1320

gacttctccc tccccacagt gccacccagg ctagcctgtc acctcccacc ctgagcaact 1380

cccaagactc acctgtgtct ccctaatcca ccactgactc caccactgac tccagccaga 1440

gcaaccgtaa actttacttg aattatgtcc ctggcagcaa tgccagttgc cgctcagtgc 1500
ccagctttac catggccacg ttctccctcc cagcttcttc tacacggaaa ggtttttctt 1560
cctcagagtt gtgggatata tttaacctgc agaggaatgt gtgtgtgtgt gtgtgtgtgt 1620
gtgtgtgtgt gtgtgtgtgt gtgtgtttcc tctgcccctg ctggtcccct ctcatcttta 1680
atagtgtagg cagaatcaca tcaaagagag ccgcatcttc attaggaggt acctaagaca 1740
catcactgca acgcccggcc cctctaccca gtaccgagat ccatccctga cagcccctgc 1800
cctgtttata gcatttattt atttgctagt agtctgtccc ccacccctgc tatactataa 1860
tccacagacg ggaaagtttc catctgcctg actcccactg tatcctcaag gtctgtcact 1920
ggccggtgtc atagtgagaa tgccacggaa ggcctcccag agaacccccc tgttttatca 1980
actttgcgga acatgcaaaa ccacctaaga ctggagtcag gaggatcccc agagtagggc 2040
cttctgggat tcaagagagc agtaagtgcc aggtggtccc cttggtagtg ataggggatt 2100
ctgcctgcag gactgcagtt taccatcatg gtggtcaaca aggcaaggtc attctccctg 2160
gtgctatggt atgatctccc tggtgagatc gatgcccttg tgatggctga ggagatcccc 2220
tgtactcagg tttccatgtg tttatccata cggctggtca acaacaatac aagtttatga 2280
gttagtgcct aggaatgttc atggtttcat ctctgggttc tcagaggaac atgtgcgaga 2340
aactgagaga ccctctaacc tttaactacc aaagggtatc cctgttttcc acagcctagt 2400
ccccaccttc aagctcttgt tctgagaata cacctaagat tctcttcaat aaaatgcaaa 2460
gagaaaggca ttgtactgtt ggcaatcaac caatctcttc tctaatgatg agccaacaat 2520
gtgaatggac taatgattga tcaggaaaaa atagatgacc taggggaagg ggagagatgg 2580
gggccccaca acaggcagtc cttgagaagg gactctgtgg ggatggggct gcctgctggg 2640
tgtgcccctt ttctattctc tggcttcctc agataagacc agcagttagg gtacactctc 2700
ctcccagtct gccgttcttg cagacacagt tccacgcttt ggaagcatgt ctgtccagga 2760
gaacactcta ccccagcagc tctggccgtg gatctttagg tcccaaaaag acttggcaaa 2820
gtctgcctta agtggggctc ccggaggtaa gaagaggaag ggaagccact gaagagagag 2880
gagaattagg gtagaatcag cgttgagaga tggaggcctt gtggggtagg atgcccaata 2940
tagcctcacc tcggctaaat acagacctga tctgagcctt tgatccaggc tctgcagaca 3000
ggccaaaggg gatccagcag catgggcgca cggggcactc ccgtggttcc tggactctgg 3060
cccccagtgt acaaggcttc cgttggcaag agagatgcta gctggttata ctttaaccag 3120
gactcatctc atctgagcca ggccccatcc cactgacaaa ggcgcagtat aaagcagacc 3180
cacagacaca gcaggtaagc aacgtagcag catggaaggt gcttctaaac cgggtgctct 3240
actgagcagg gctgggtcaa gctacggatc cagcgcagcc ttgaatgggt ggagggaagg 3300
tggaggggag gggagtggag aaggccagag aattgcctgg agctggaaga agtaaacaag 3360
aaaatgaggg aaagtaagct gagtgtttgc aggcctagat aatgaggtgc aggcttgaag 3420
gaggcctctg ggagggctga agctgagtgg tctgcagcgg tgtgacgctt ggggaaggtc 3480

tctgtagctt ggggatagac ctggatttga atcatctcac ttggctgatg catttttttt 3540
ttcttagaca atgtattttt ttttcccctt ggtctttttc ttacttgtaa agtggggata 3600
gagcaggacc ctggagacta aacaaagtag cgggtgtgtg atagtcaaat gggaggaaga 3660
cctcgcacca tctggagatc tgaatagaca cagggtgcac tggcaggaag cacatggggg 3720
acaggcaggt gtctgtgcaa gagcctgagt ttccatctac ggcagggcca gcgggatacc 3780
ttcgacgtgc gagtgtggct caactgaccc aggagctggg cactgccttc ttccagcagc 3840
agcaactgcc ggcagctatg gcggacacct tcctggaaca cctctgcctt ctggatatcg 3900
actcacagcc tgtggctgct cgtagcacca gcatcattgc caccattggt aagaccaccc 3960
aagccctaga agccacacag ggccacccac atggctcagg tgtctcctta ctttgctatc 4020
cttgttgtgg tcattcattc aacaaatatt tgtgagattt gtgcaggggt agggggtggg 4080
tttgtttttg tttttgtttt tgtttttttg agatcgggtc tttttatgtt gtagctctga 4140
ctgatggata ggcaggtctc aaatttagaa atctccctac ctttgcctcc tgagagttgg 4200
actgggacca ccttgctcca catgaggctg ggtttttgag acagggtctc aggtagcctt 4260
ggatatttct agcttatttc atagacccta ttatatcgtc ttagcctggt gaaagcatag 4320
gtatgtgata ccacacccaa cttaacaaat gcttgttgag tacctggaaa acaaggtttg 4380
gaattggggg gtagactcta tctcaaaaac aaataagaac gaggaaaggt gtcatctgca 4440
cacagatggc cttccaagga taaggacggc tgggatcaca gagaagtgaa aggtatatat 4500
aatgaactga ggaccagaca gacacacacc taatacacac atgtcacaca acatacacac 4560
acaccacaca catacacatg caccacacac atacacacac acaccacaca tatactacat 4620
acccacacac acaccactca catatcacac acatgtacca cacatacaca catcttgcac 4680
acacacacac accacacaca tatcactcac acatcacata catataccac atatacacac 4740
agcacacaca tacaccgtat atgcaccata tcacatacac acacaccatt cacacgtcac 4800
acacacacat gcactataca cacacaccac acataacact actcacacat gacacacata 4860
tactacacat acacaaatca ctcacacata ccattcatac acatacatca tacacacata 4920
ccacacacca ctcacatata caccacacac acaaacatgt acaggccaca cacacacaca 4980
cacacacaca cacacacaca cacacacaca caccagtcct cacacattat ggctcagcat 5040
gctcagcaga taaagtcttg tcatggagct gggtgacctg agttcaatcc ctgaaaccac 5100
atggaggatt cggccacaca caccaaaaac aaaaacagaa ctcagtccag ccaggctatg 5160
aggccctgtt tcaaggagag agggtgggca gtagctctgt tgttagaaca cttgcctcct 5220
gcatgaagcc ctgggtccgt cctagcgcta cataaattgg ctgtcgtaga gtacaccctt 5280
aacccagcac aggggaggac aggagaacca agcccaaggt tatccccaga agccagagca 5340
aacccaaggc tagcctgggt tatgggagtc cttgtctcag aaggaaggac tgttattcct 5400
aaagaatggg agataaggat gggtgtagct cagtactata gcatttattt gcctcgcatg 5460
tagaaggccc tggcctccat ctccagagca gaaaaaaaaa atggtggcca gggaagaaga 5520

gggaggggga agtgcataca gcgagctgct gctgctgctg caggctgacg tggatgaaga 5580

cagggacaga tctcagttct gtcagtgaga ttgctcagct ttcaaagtgc ctttttgacc 5640

tgcctggcgt tctgaccctt cacagcctac agtaacctcc acagcataca cttatgtcag 5700

gtcacttgct aacaaaggtc ctgaggcctg actgagattt agacattcca caagctgcct 5760

tcccaagggc tgtgtactgg ttggagtggg ggaaaaaaag agtttgtgag tagaggaact 5820

tcgaaggact cctgggtagg ggagtctctc cgatgttgca gatgggtttg actgtcaggc 5880

ttgggaggac cttcttagtg ggttctctgg ttccagggcc agcatcccgc tctgtggacc 5940

gcctcaagga gatgatcaaa gcagggatga acattgcacg actcaacttc tcccatggct 6000

cccatgaggt gtggggaaga agtcatggag ggtggagctg agaatgcccc caaggtcctg 6060

ttcactttgc cttaagatcc aggttttgtt ctctctctct ctctctctct ctctctctct 6120

ctctctctct ctctcgcttt ctctctggta gtaccatgca gaatccatcg ccaacatccg 6180

ggaggcaact gagagttttg caacctcccc actcagctac agacctgtgg ccatcgccct 6240

ggacaccaag ggacctgaga tacgaaccgg agtcttgcag ggggtgagaa gcctttgggt 6300

ccgagctgtc tggggcctgg aggagcatga gagccctgga gcaagagaga gggtccctac 6360

cctctcaggg tccacttgca aaactcaggc ttctctaccc accccactcc cctccagggt 6420

ccggagtcgg aggtggaaat tgtgaagggc tcacaggtgc tggtgacggt ggacccgaag 6480

ttccagacaa ggggtgatgc aaagacagtg tgggtggact accacaatat cacccgggtc 6540

gttgcagtgg ggggccgcat ctacattgac gacgggctca tctccttagt ggtacagaaa 6600

atcggtacag aaatgcatcc tgatcttatg aggcgctccc aaaaccctga aacagggcat 6660

tttctcttcc ctttggtttc tatgttctgt cagctcctta cccatacctt cccctagtgg 6720

tgcttgcttc tccacctgtc ctagtggtcc ctgctgtcat catgggttgg agctgagcat 6780

ttgatgcctc ttcttgtgtg tggagtgagt gaagggttca gaagattcca atttctggag 6840

gatccaagct taagagttcc ctctgcagtg cctatatcgc agctcagcct tacagacact 6900

ggttctctct atacccgcag gcccagaggg actggtgaca gaagtggagc acggtggtat 6960

cttgggcagc aggaagggtg tgaacttgcc aaacactgag gtggacctgc ccgggctgtc 7020

tgagcaagac cttttggatc tgcgcttcgg ggtgcagcat aatgtggaca tcatctttgc 7080

ctcctttgtg cggaaagcca gtgacgtgtt agcagtccgg gatgccctgg ggccagaagg 7140

acagaacatc aaaattatca gcaaaatcga gaaccatgaa ggcgtgaaga agtgaggctt 7200

agtctttggt ccgtcagccc tctgtttctc tttccctctc ttctgcatga tatttcctgc 7260

catctcctcc tctgcctagc ctggattcct ccaaccacca ggtttgatga aattctagaa 7320

gtgagcgatg gcatcatggt ggcacggggt gacctgggca ttgagatccc tgcggagaag 7380

gttttcttgg ctcagaagat gatgattgga cgctgcaacc tggccggcaa gcctgtcgtt 7440

tgtgccacac aggtctggag caaacccttg ggctttaaag gtgctgagaa acctacatgt 7500

tgctgtctgg tgcctggtca caaaagacga tgacatgaac ttccagggtc ttactgtgac 7560

cctggcactc taagcgtgca ggtgccagag aaatagcgtg ggcgggttcg ggtccccagt 7620

cagaatgtga cttctacagc tttgacatcc atggtgtcac tcagatgctg gagagcatga 7680

tcactaaggc tcgaccaact cgggcggaga caagcgatgt ggccaatgcc gtgctggatg 7740

gggctgactg tatcatgctg tccggagaga ccgccaaggg cagttttcct gtggaagctg 7800

taatgatgca acatgcggta ggtactccgt tctgaaaagc tgctgggcca gcgacacaga 7860

cccctctgga accccgttga ctactcaacc tgacctactg gtccctgcag attgcgcggg 7920

aggcagaggc cgctgtgtac caccgccagt tgtttgagga gctacgccgg gcagcgccgc 7980

tgagccgtga cccaactgag gtcactgcga ttggagccgt ggaggcttcc ttcaagtgct 8040

gtgcagcagc catcatcgtg ctgacgaaga ctggccggtg agaaacatat tggaaacagg 8100

gctttgggca ggggcggctg acacagaggc taataaaaaa agcactgctt tagggcaagc 8160

aaagaaacag ccatggatgc tagaggccag gagaggctag aggcttggac aggtggcttt 8220

ttgatgatct agaatcagag ccagtgatgg gggggggggg gcgcacgggg acggggacgc 8280

agcaggtgat aacccaagca cgttggcctt agggtatttg aagaccaggc tctgacagat 8340

cttcactgtg ctgaggtgtt gctgaaaaac ccagtaggcc caagagctga ggatttttct 8400

caaacttagt agtgagatcc taagtaattg ttagggctta caagccttga ctccatggat 8460

ctgtgtgagt ttttcatttg tccccacccc accccacccc catggggttg tgaggattaa 8520

caaaaaatgg ttaagagtca tatggaggtt gggaatgatg taaacatatc ttctagacag 8580

ttttaccctt tttttgttgt ttgtttggta ggatttttct gacataggga cttattagtc 8640

ctggctggcc tgaaacttcc ttagtacctg aagtagcctt gaacttaacc ctttaccccg 8700

agggctgagg ttgtgggcgt gtctcaccac gcctggctgg ctttacattt ctaatttttt 8760

ggaggattta ttacttttat gtgtgcatgt acagttctaa ggcgagaaga ggttggaccc 8820

tctggagctg gagttacagg cagttgtaag ggatttgatg tgggtgctca gaaccaactc 8880

tggtcctcag aaagagcagt gcgagccttt aagaactgag ccatctctct agcccaggtt 8940

ttacatttat ctttcaatat ggattgacat tttgcctgcc cttacgtctg agcaccattt 9000

gcatgtctgg tgccagtgaa ggccagaaga ggatgttaga gcccctgaaa ctggacttaa 9060

aaaccattgt tagccaccat gctggtgttg aaaacgtaat cccagcgtgt gctcttaatg 9120

gtgagccgtc tctacatcag ggtaggtctt cttactactt aatgagaaag acaaagatca 9180

gaggagtata ccttacccaa ggacacaggg tttgttagtg acagtgacac ctggaaccat 9240

aattcaaact cgtttcttgg gtcaacactc agagctaacc ttttcttcct catgcagttc 9300

agcccagctt ctatctcaat accgacctcg ggcggctgtc attgctgtga ctcgatctgc 9360

ccaggctgcc cgacaggtcc acctgtcccg aggagtcttc cccttgctct accgtgagcc 9420

tccagaggcc atctgggcag atgatgtgga tcgaagggtc caatttggca ttgaaagtgg 9480

tgagccttgc cctctgttcc acagcctgcc ttcttacccc gactccaaag ctctgtctgt 9540

cctcactggc ccaagttttc taggaatcgc taagatggac atgtcttggg ctttattttt 9600

gtttttttgt ttgtttgttt tttgtttttt ttttaaagat ttattcattt attatatata 9660
agtacactgt agctgtcttc agacacacca gaagagggca tcagattctg ttacagatgg 9720
ttgtgagcca ccatgtggtt gctgggattt gaactcagga cctctggaag aacagtcagt 9780
gctcttatcc gctgagccat ctctccagcc catgtcttga gctttaaagc aggaagggta 9840
ggctgagagg aaaggagtga gagcaggacc cctgagaagt ccttgctgct gtctgccaca 9900
gcctagtagg acacatcagt aagaggaggt tccagaagag ctggaactaa attttaattt 9960
atagttgtcc ttgtcccatt agtgcagcca catgggtgaa aaggctcaat gctgaaaaac 10020
agtatacagc atccctccac acacacatcc tctcaaacca ctgcctttgg ttcctttccc 10080
ctacaggata ggtagccttg aggtggtcga tgctaatggc tgacccaggt tcttcaatga 10140
ctgtcatatt tttctccacc aggaaagctc cgtggtttcc tccgtgtggg tgatctggtg 10200
attgtggtga caggttggcg gcctggctct ggctatacca acatcatgcg ggtgctgagc 10260
gtatcctgaa atgcctctcc ccattctgac ccagttacac cctatttctt tcaacccaca 10320
cccctcccat agtcctacat ctgccatcta gccccatccc tgtgctttac acagccctga 10380
atgtctgtgt ccaattatac agtggccacc ggcagcatcg gttgtatatc cctgtctcaa 10440
tccgctcagc tggactctaa gataccctga gcctttaatc ccagcccagc tggtgattcg 10500
attccttccg ggtcccaatc attggaatgg gggagtggaa acagggtgat cttgtccaat 10560
tttcatacaa tcatgatttt aaaacactgt ctgatataac cctcatgatc agttcctagc 10620
aaagtgtcat ctcctaatgg cctcaagtca gggcagaata ctccttcaag gagcacagct 10680
ccacacttta gggaaggctg gggcagctgg gtactggaga gaactaagac aggcggcttt 10740
tctctctctc tctctctctt tttttttttc ttttcttttt ctttttttcg gagctgggga 10800
ccgaacccag ggccttgtgc ttgctaggca agcgctctac cactgagcta aatcccaacc 10860
ccagcttttc tctttttaat acaagctctc actggcctca aactcctaag tcctcctgcc 10920
tggccctcct aagggtaggg actacaggca tgagtgacca gctggacttc gggtgcctta 10980
ttttcttact gactccacaa accatggttg ttctcctgcc cactgctctg ctgggtcaga 11040
tgatccagaa attcttccac aaccacttgg ctcccacata caaattagaa gcaaactgaa 11100
tcttttcttt taaacccaac tgtttaggtg caattataaa aacaactccc acaggcaaag 11160
aatcccagaa tctcctaccc taggagatgt atagtcctgg ccccacccat caatctgtag 11220
tatactcctg aagcgggaca gaactggtgg acaggggact cctcttgtcc ctaagaaagt 11280
ggaggcactg ttggcccacc cctcctaggt ttgaatactc caggccctcc tcttagcacc 11340
aacagcaaat ccagatgagg aaaaaaaaat aagtgcagtt ctcctgctgc cctcctcttt 11400
tcactacctc aatacagcaa gtttgagtat tgctgctgat ggcagtgtgc aaggccacaa 11460
agatgtcccc cctcagcccc ctaccagaag gtggagagga cagaggaatg aataataaag 11520
tgaatgcgtc aaattagcaa atgaataaat aaaattaata agtgactgaa taagacacag 11580
ggaaagataa aaggtcacat gaggattcag ccccttaccc cctacctggg ggagctaggc 11640

tagcacagta aggaggcgcc aagtatccct tagctttagg gcaacaccat tgagtcaggg 11700

agcacagtga agagcagctt ctagtttaca aaggtagtgc tttccacaca ggagctgcgg 11760

agggcagact acagtgggca gagatacccg ttctcacgtg gtgtagacaa ggacggtccg 11820

tccacacgtg gcagtgagag ggaagatggt cacacctagg gccctagaca tctgttggct 11880

gtactagata attgacagta cccagctacg acaatgggga tgggaagaaa ggacacactg 11940

ggcacaacct gcagggtcac tgacagggag acagccatct agtcactcgg cagtgaagga 12000

agcagcaagt acttgtgctt aataaaggca gggctgtggg cccaagcctg cacctggcag 12060

gactgtggcc tggggacacc ttgttaaagg gctttgagtc acttctactt ggcagtggct 12120

acacattgta cttagcacaa tgccttgacg gggtaggcac tggtacctat gacaaatggg 12180

tatgtgatta ggattcgcag gctgcagctg ctatgagctg aggacacaga ggacaacttt 12240

gtggtagtaa agggcagtgt gttagatccc cgcagccttc cccaggggcg tctaccctca 12300

ggcacacctt attgcgtgcc aatagctaag gccagtgcaa tctaggattt cacatgttgg 12360

cagaaatttg gggacctctg ggggtaactg gctcaggcac tgatgacctg gatggctgga 12420

cagtccctgg aggcttggcc aggagcccca agggcagcac gctcacttcc agagcctttg 12480

cgcctaggag agccatcccc cgtggctcgc tgaggcagag agggttgaga ggcagagagt 12540

gggcggcccc taggtcctag cctccgacca cctcctcctg gggagggccc aacagagaca 12600

cctgggaacg ccctgtccgg gataggctgg catggagggt tcgggcaggt ggagggggag 12660

ggcccaacag agacaccttg ggaacccctg tccgggatag gctggcatgg agggttcggg 12720

caggtggagc aggcaggcga gaagtggacg cccagggtcc ccgggccagc agaggacctg 12780

ctcggacagg caccagtggg gaggctgggg agcctgctga gcgtctagca gatcgagcca 12840

ggagggtggc tggagaatca ggggagaggg gcagagggcc tcgctgatga gtcaaggcta 12900

tggccacgtt ggaggtcaca gcagctgcct gcagaggtgc gtgtaccagt ggttcccaca 12960

gcactggctt tccactgagg cgagctcctg tgcccggagc cagaccccga a 13011

<210> 37

<211> 3275

<212> DNA

<213> Rattus norvegicus (Norway rat)

<400> 37
   cccagggtcc gatcgcgttt ggtgcctgaa ggaggaaccg gtgacagacc tggagactac 60
   agttctctgt ccttcacaca gctctttcac catgcctggg tcacttcctt tgaatgcaga 120
   ggcttgctgg ccaaaagatg tgggaatcgt tgcccttgaa atctactttc cttctcagta 180
   tgttgatcaa gctgagttgg aaaaatacga tggtgtagat gctggaaagt ataccattgg 240
   cctgggccag gccaggatgg gcttctgcac ggatcgcgaa gacatcaact ctctctgcct 300
   gactgtggtt cagaaactga tggagagaaa tagcctttcc tatgactgca ttgggcggct 360
   ggaagtcggg acagagacaa tcatcgacaa atcaaaatcc gtgaagtcta atttgatgca 420
   gctgtttgag gagtctggga atacagatat agaaggaata gatacaacta atgcatgcta 480
   tgggggcaca gccgcagtct tcaatgctgt gaactggatc gaatccagct cttgggatgg 540
   acgatacgct ttggtagttg caggagacat cgctatatat gcctcaggaa acgccaggcc 600
   tacaggtgga gttggagctg tggctctgct aattgggcca aatgctcctg taatttttga 660
   ccgagggctt cgtgggacac acatgcagca tgcctacgac ttttacaagc ctgacatgct 720
   ctctgaatac cctgtggtag atggaaaact ctccatacag tgctacctca gcgcattgga 780
   ccgctgctat tctgtctacc gcaaaaagat ccgggcccag tggcagaaag agggaaagga 840
   taaagatttt accctgaatg attttggctt catgatcttt cactcgccat actgtaaact 900
   ggtgcagaaa tctctagcta ggatgttcct gaatgacttt cttaacgatc aaaacagaga 960
   caaaaacagt atttacagtg ggctggaagc ctttggggat gtgaaattag aagatactta 1020
   cttcgacaga gatgtggaaa aggcatttat gaaggctagt gctgagctat tcaaccagaa 1080
   aacaaaggca tctttgcttg tatcgaatca aaatggaaac atgtacacat cctctgtata 1140
   cggttccctt gcttctgttc tggcacagta ctcacctcaa cagttggccg ggaagaggat 1200
   tggagtgttc tcttacggtt ctggcttggc tgccacactc tactccctta aagtcacaca 1260
   agatgccaca ccaggatctg ctcttgacaa aataacagca agtttatgtg accttaagtc 1320
   aaggcttgac tcaagaacgt gtgtggcacc agacgtcttt gctgaaaaca tgaagctcag 1380
   agaggacaca catcacttag ccaactatat tccccagtgt tcaatagatt cactcttcga 1440
   aggaacatgg tatctagtca gagtggatga aaagcacaga agaacttacg cccggcgtcc 1500
   ctccacaaat gaccacagtt tggatgaagg agtgggactt gtgcattcaa acacagctac 1560
   agagcatatt ccaagcccgg ctaagaaagt gccaagactt cctgcgacct cgggcgaacc 1620
   tgagtcggct gtcatcagta acggggagca ctgagagccg tggccttcac agaggctcgg 1680
   ggctggatgg ggtatgggaa acgttagagg aatggatgtc ttgggacaat tttacagatt 1740
   acgtgttgct taaaaatgta atgtaactga cacagagccc agaaaactgt tgtgtttttg 1800
   gagaagtctc gctgagctcc taacacactt cctgctgtgg gctggccaat ggtgaatgta 1860
   ctgcgatggt gttaagggct ctgcagaacg tcatacctcg ctgcatgttt acacgcatgc 1920
   gggttaggct tcaaactcgg tctgaactga gtgcttctga ctgcaaaggc agaggtactg 1980
   ctgtccagtt taaaaaattg tttttttttt ttttaatgtg taagaatttt tatacttaaa 2040
   taaaaaaaag tacctgtagc ttttggggga aaaaaaaaaa cctttttcta ggttggggat 2100
   tgtggaattt aaatgttaca cataaactct gcttaatggc aaggcaaaca tttatctttt 2160
   tcgaagattt ataaatcctg aagagaaaaa aagagggtat ggttctagga tctggatgaa 2220
   ccatcagtga gaaaggttag tcataatcaa gtgagcagaa ggatgctggc gttgagcagg 2280
   cctctgtcac agcaaccagg gctctgtggg cacagctgag ggaactttct ggccaggtgc 2340
   ccgtgactgc tgctcagctg cactgagatg cagtggagct gctgcacgga agcttgctgt 2400
   ggtgctgaac gccttacctg cggataaagt gtaaagtagg agggatgggc agggcactat 2460
   taggttacag tgttacagac ccaggttata gacttgacag ctcaaactca ccagacacct 2520
   ttttcccttt gtggtttgtg tattttttgt gttttgtttg ttttttttta tattgttcaa 2580
   tttaaaaaat ttagaaaatt ttaaccttac gttttcacat agtgtgatta gccaaaagga 2640
   atttcacttc aagatctaga aatagaattc ataacatttt ttcctaaact ttgactttta 2700
   aaacaacgaa aattaccaca tgagatgaac aagaaaattc attagaaagt tctctgggtg 2760
   atttttggtg ctgaactgac atgagcctca tagactgtaa aacagaggta gttgaaacta 2820
   atgtacagaa ctacattttt taattttatt tgcatttaat tctgtgaagt ttcagttatc 2880
   taaaataaac acataaacgt gtaatgtttc agattgcaag gtgagatgta atgtagcatt 2940
   tgtaagatat tcttgtcaat attaactggt aggattttga tttgtacagt tttaattggt 3000
   taaaatgatc tcattttaac atccactgct atagatgaat gatgtaagct cagatttaat 3060
   gaatggtggg gaaatggtgc atgtaatttt ttcgcaagta tcgagagttc tgtatgtttt 3120
   gaaaagaata atttaacgtt tgggttgcca ggaagggggc tttcccagag tccattgcca 3180
   ggcgttgggc aagcctcgca atgttggcac ggagcgttaa ccacacctta ctaatagcaa 3240
   ggggaataac tttgaaataa agttttagac aaata 3275

<210> 38

<211> 1289

<212> DNA

<213> Rattus norvegicus (Norway rat)

<400> 38
   cgcggccgct tctcactggc ccagagcaag tgaagaccag tggagaatga acccgttttc 60
   ctctgagtca gcatggctct gcttgactgc cactgcggtg ctggggggca tgcttctctg 120
   caaagcctgg agttcagggc agctaaggag ccaggtggtg tgtctggctg gcctctgggg 180
   aggtgcctgc ctgctcagcc tgtccctcct ctgcagcttg ttcctcttat cggtgtcctg 240
   cttcttcctt ctgtatgtga gttcatctga ccaagacttg ttacctgtgg atcaaaaggc 300
   tgtgctggtg acaggtgccg attccggttt cggtcatgct ttggccaagc acctggacaa 360
   gctgggtttc acagtgtttg ctggagtgct ggataaagag ggcccaggag cggaggaatt 420
   gaggaaaaac tgctctgagc gcctttcggt gctccagatg gatgtcacca agccagagca 480
   gataaaagat gttcacagcg aagtcgcaga gaagatacaa gacaaaggac tgtgggctgt 540
   ggtcaacaat gctggggtct tgcactttcc gatcgacggg gaactcattc ccatgaccgt 600
   gtacagaaaa tgcatggcag tgaacttctt cggagccgtc gaggtcacca aggtgtttct 660
   gcctctactt agaaagtcca aggggaggct ggttaacgtc agcagcatgg gagccatgat 720
   tccatttcag atggtggccg cctacgcctc cacgaaggca gctataagca tgttctcagc    780
   cgtcatcagg caagagcttg ccaaatgggg agtcaaagtc gtgaccatcc atcctggagg    840
   cttccaaaca aacatcgtag gctcacagga cagttgggat aaaatggaga aggagatcct    900
   ggaccacttt tccaaagaga ttcaggagaa ctatggacag gaatatgtcc atacgcagaa    960
   gctcgccctc ccggtcatga gagagatgtc caacccggat atcacccctg tgctgcggga   1020
   catccagcac gccatttgtg ccaagaaccc ctcttccttt tattgctcag ggagaatgac   1080
   ttacctgtgg atctgcttcg cggcctactc cccgatcagc ttattggact atattttgaa   1140
   aaactatttt actccaaaac tcatgccaag agctttaagg acagcaagtt agaagaacaa   1200
   gggcccgggt agggtgggaa atgccacatg cttaggatga aagggaaacc tcttattaaa   1260
   ctaactttct acctcaaaaa aaaaaaaaa                                     1289

<210>  39

<211>  447

<212>  DNA

<213>  Rattus sp.

<220>

<221>  misc_feature

<222>  (404)..(404)

<223>  n

<400>  39
tctcagctac tgtatgcgaa gtctgattat attggtgcgt ttttacagtt agggttttgc     60

aataacttct atattttaat agaaacgaat tcctaaaatc cctcctctcc ccaatttcag    120

gaatttaaaa ttaagtagaa caaaacaaaa acccagcgca cctattacag ctgtcatcca    180

ccgtcatggg aatcagtttt cattaaactt gaagcagttg tggcatcatc ggcagtgttt    240

tggttcagac acctattcac agaaaagcat gatgggaaaa tatttcctga cttgagtgtt    300

cctttttaaa tgtgaatttt tatttcgttt taattatttt aaaatattta agccttcttc    360

ttgatcttaa agatcgtgta gcttggggtt ggggacggat gaanggcgag tgagtctaag    420

gataatgaaa taatcagtga ctgaaac                                        447

<210>  40

<211>  1902

<212>  DNA

<213>  Rattus norvegicus (Norway rat)

<400> 40
tagcccgacg aactgagaac tggccatggc acggaaacaa ccacatagct ggctgaatgc 60
tgtgctcttt gggctcctgc ttattcttat ccatgtgtgg ggtcaggact caccagagtc 120
cagctccatc aggaccacac acacgggcca ggtccgagga aagcttgacc acgtgaggga 180
cactaaagct ggtgtccaca ccttcctggg aattcccttt gccaaggctc ctgtaggacc 240
gctgcgcttt gcacctcctg aggaccctga gccatggagt ggtgtgagag atgggacctc 300
acatccggcc atgtgtctgc aaaatattga tatgctggat gaagtaggcc tgacagatat 360
gaaaatgata ctgtcttcca ttcctatgtc tgaggactgc ctgtatctca acatctatac 420
accagcccat gcccatgagg gctctaacct gcctgtgatg gtgtgcatcc acggaggtgc 480
actggttata ggaatggctt ccatgtgtga tggatctcta ttggcagtca atgaggactt 540
ggtggttgtc gctatccagt atcgtctggg tgtcctgggc tttttcagca ctggagatga 600
gcatgccaga ggcaactggg gatacctgga ccaagtggct gccctgcgat gggtccagca 660
gaatatcgcc cattttggag gcaaccctaa ccgggtcact atttttggcg tgtctgcagg 720
tggcacaagt gtgtcttcac atgttatatc ccccatgtct caagggctct tccatggtgc 780
catcatggag agtggagtgg ccctgctgcc tgaccttatc tctgaaacct ctgagacggt 840
ctccactaca gtggccaagc tctctggatg tgaggccacg gactcagaga ccctggtgcg 900
ctgcctgaga gccaagagtg gagcagagat tctggtcatt aacaaggtct tcaagatgat 960
tcccgctgtg gtggatggag agttcctacc caggcatccc aaagagctgt tggcatctga 1020
ggattttcgc cctgtcccca gcatcattgg tgttaacact gatgagtact gttgcaccat 1080
tcctatggtc atgggcactg ctcaaataat aaaggagcta tccagagaga acctgcaggc 1140
tgttctaaag gatacagcag cacaaatgat gcttcctcct gagtgtggtg acctgctaat 1200
ggaagagtac atggggaata ctgatgatcc ccagacccta caaatacagt acgctgagat 1260
gatgggagac ttcctgtttg tgatccctgc actccaagtt gcacactttc aacgttccca 1320
tgcccctgtc tacttctatg agttccaaca tgcacccagc tatttcaaga atgtcaggcc 1380
accccacgtg aaggctgacc atgctgatga ggttcctttt gtctttgggt ccttcttctg 1440
gggcataaaa gttgacttca ctgaggagga gaagctgctg agtaggcgga tgatgaagta 1500
ctgggccaat tttgcaagac acgggaaccc caacagcgag ggtctaccct actggcctgt 1560
gttggaccac gacgagcagt acctgcagtt ggacacccag cctgctgtgg accgagccct 1620
gaaggccaga aggctgcagt tctggaccaa gactctgccc cagaagatcc aggagctaaa 1680
tggagctcag aaaaaccatg cagagctgta gtgtctggtg aaaggaacag agtgtgggag 1740
tgagggcagg tgggatcatt ctgagtttca aagtctaatt ttctgttcca acacgcagaa 1800
tcctttccaa ccccaatatt ttccctttct gacatgaatg agaagccctc cgtgtgttac 1860
tctttattct tctgggcaaa atttaattgg actcaataaa ga 1902

<210> 41

<211> 2833

<212> DNA

<213> Rattus norvegicus (Norway rat)

<400> 41
ctcgaggccc acagagaaga acgtcttcat tgtctttatg atggctgtgg ctggactgtc 60

tctgtttctc agcctggctg aactctacca cctgggctgg aagaagatcc gacagcgcct 120

ggccaagtca cggcagggtg acaagcacca gcttcttggc ccctccacca gcctggtcca 180

gggcctcact cctcctcctg acttcaacca gtgcctaaag aacagcccag atgagaaatt 240

cttcagtgac ttcagtaata acatgggctc ccggaagaat ccagaccctc tggccactga 300

ggaagtgcca aaccaggagc agatcccgga ggaaggtttc atccacacac agtatggcca 360

gaagcccgag cagcccagtg gcgcctctgc aggccaccgc tttcctcagg gctaccacag 420

tgacaagcgc cgccttagta aggccagcag caaagcaagg tcagatgacc tgtcagtgtg 480

accctcctct tagggaggac cagtcccaga aggaagccaa ggagggtgag gcaggacaga 540

agcggtctct ctgactgtat accttcctca gcaccttctc tcccgggtct ggatggtgtt 600

gctcattaat cctccaaatt tcagttctga gccgtattct caaagattgg gctactcctc 660

aaatccccaa ccccacatcc tctaccagta ttcgcagtga agactttcaa gtgatttagt 720

gatatggtgg gagcaggaat gggaaccttg gcaaaatctg gtctgggtag gacagacatg 780

aagattcgac attagcaaca gataaaccct caccccaaat cttgtcttct tcggtactag 840

gcccatcctt tttatttttt aaaggaagaa tccaaggttg agagtattca tcaaataatc 900

tgcattgtat tgttcctttg aatctgtcgt ttccatgtgc catacctcag agtgctggtg 960

aggtaactgt gggttgccct tggctgcatc tccctcctcc cagtcttaaa ggggtgtcct 1020

tggtcctcgg ccagcatcag ctttacaagt gccttggtat gtgcctttgg aaaacactgc 1080

atgttgatga tgctgcagcc ttactgccag gctagacacc cagctcagaa gggtgccaag 1140

acctctagaa gtcactgtgc acataagcat tgctggaatt gccaccagct gtcatcctgc 1200

agtcctttac agttcaacct agtgatagaa accgtcctgg acagctcacc cagtgttcct 1260

ctaaaatctt ggttaacagg aatgtccaaa gtcaccatcc tctcttggac cctggccaga 1320

ttaccagcca ccaagttcag tggaatttcc ccaggattta atgaaacaaa gagagcattg 1380

tgcttctcag gttccttggg gggtgttggg ggggattctt ctgtggagat gaaaagagag 1440

gagggggagg acactggaaa actattttcc cttctgcata tgccttccac tgcctcccag 1500

cttggactgc tgagaggtgt ggtgatagat agcaacggag gcccaagatc tctctcttag 1560

aggcatcaag gggacctgga agccgagatg gagctccggt tggttttgac acggccctta 1620

tttagtgcag atgctcctgt gtttccttgg gacccttagg tacatcaggg agggactgta 1680

gaaaattcct gccttcctca gggtctatcc tgggctatct actgagagga tccacagtcg 1740

gtgtctctcc ttatgcagat ggaaagatgc tcagcgtcag atatcaagac ttacaaacac 1800

aatgctctgt gtgtgtgtgt gtgtgtgcat gtgtgtgtat gagtgtgtgc atactcaacc 1860

atctatgtgt gtatatgtgt gtgtgtgcat gctcaaccat ctgtgtgtgt gtgtgtgtgt 1920

gtgtgtgtgt gtgtgtgtgt gtgtataccc gtgtatgcat gcagatagaa tggtaaagaa 1980

gagccctgaa gaactgtaag gaaacatgta gaaaacttgg gtttttctgc ctccttttct 2040

gttgcccgac aggacagctg agcttgcttc ctaatcagcc taaaggtcaa acctgggaag 2100

tcagtacagg agggtgaagg ttcagaggtc aacccctgcc catgtgacca tctctgggtc 2160

cctctgctgg tatttggatg gctgaagata cttgggaaag gctgactggc tgtcccagcc 2220

ccagtgtatc tcattcattc ctgacaccga cgctatgatt ctctgctttt ctagttatgg 2280

gatgtgtgtt tgtcttctga aatggggcct tttgagttag aaaatctgag accagtcttc 2340

gatggtgagg tttttcagaa gagaaagaga aagtaatatg tgtgactggt tttaaataaa 2400

atacgtctct acatgttaac tctggtttgt gtctcctttg gactgtggct ttaagagggt 2460

ggcagacttg atggagaagg gagagcatgg agtcagacgc acaagggtta aaatcccaac 2520

tgctggtctc tacctggcca tcagtgagct ctttcacttc tgaagtctgt tagtcacctg 2580

taagtcaatc cagtgatgtt cactccatta ggctgtgaag tgagaggctg tgtaggaaga 2640

gaaagacaga tcctctcagg cacaagagtt tacaattgag gagccattgt cattattttc 2700

aatacagagt ggtccagaac ccgaggtact gagggataat tctagacctt cctcttttgg 2760

aactcacagc attatctacc tggggggaac aaatgaactc acagaaagga cacaagtagg 2820

gtgcaggaca ggg 2833

<210> 42

<211> 1147

<212> DNA

<213> Rattus norvegicus (Norway rat)

<400> 42
gcagaaagta gtcatgacag cctttgcgga tcgcacggtt gtaaccatag ctgtaagtga 60

catgcatggg gtggggtggg gtcggggtcg ggtgtggggt cactcattct tctgtagtga 120

gcttcaagtc tgagaaggta aaacatactt gtgttgattg tgtatttctt tcttgtttca 180

cattatatat attttcaatg acatcatctg aaatataagg acaccagctg atcacatgct 240

aagtgtctgg gctaatgttt ggaaggaaca tttcagaaag aacgctccta agttccatta 300

ccaatttagc taatattcat gtacattttc cctatttcta tcatttaaag tagaagacag 360

tgggagacct gacagccggg gaaggtgata tccgtacatc caatagcaat caggtccttg 420

agtcaattag ccccttgtcg aatttcttgc ttgtaagggc attagttttc tgtcccttcc 480

tcttgatctt tgttatcatt gccacccacc gaagactcta tttcgtgata cacttgtcca 540

ttcttcctgc tcattctctt ctctctccac ctctctctcc tccagcaccg tgtctcctct 600

attatggatg cgggccttgt tttagtcttt tctgagggta ttttagtgga gtgcgatact 660

ggtccaaacc tgctccagca caagaatggc ctcttttcta ctttggtgat gaccaacaag 720

tagaccagca agatctgctc ctccaagtgt ctcgttctct ggtagttgtc atagcagaat 780

ctgctgagta aggcagagag gaggcttcga ttagagggag ggctggggga ggaactaacc 840

ccgggtctaa ctgcacaagt cagctcaccc aagtgcttgc attgtgtggc ctcttatgtt 900

ttcaaagaca gcatgtagga agcaaaacca ggctcacctg tggcctaagg acaagttaat 960

ctgaggctgt tttctagtca cccttaaaat ttctgtgtgt tacctgccca aagatggatt 1020

tcagggatta ctgagtgtaa ctgcggagag ccctgtgctt tgagggacac ttgctactct 1080

tggaagaaag accactcttt gaagtgcatc tttctcaagc tccagagagc gatttcccat 1140

gggcccc 1147

<210> 43

<211> 1389

<212> DNA

<213> Rattus norvegicus (Norway rat)

<400> 43
tgctcctctg ctccaggctc ctgccaagtt tagcgcagga agaaggcgcc caggaattga 60

actgcaatga tgagactgta tttcaggctg tggatactgc tctgaagaaa tataacgctg 120

agttagaaag cggcaaccag tttgtgttgt accgagtgac tgagggcact aagaaggatg 180

gcgctgaaac attgtattcc ttcaagtatc aaatcaagga gggcaactgc tctgttcaga 240

gtggcctcac ctggcaggac tgtgacttca aggacgctga ggaagccgct actggcgaat 300

gcacaacaac tttggggaag aaagaaaata aattctccgt agccacccag atctgcaata 360

ttactccagg taagggtcct aagaagacag aggaggacct ctgtgtcggg tgtttccaac 420

ccataccgat ggatagctca gacctgaagc ctgttctgaa acacgctgtg gagcatttca 480

acaacaacac gaagcacacc cacctctttg ctctcagaga agtaaagagt gcccactcac 540

aggtggtggc tggcatgaat tataaaatta tctactccat tgtgcaaaca aattgttcaa 600

aggaggattt tccttccctc catgaagact gtgtacccct tccctatggc gatcatggtg 660

agtgtacggg tcatacccac gtggatattc ataacacaat tgccggcttc tcacagagct 720

gtgaccttta tccaggagat gatttgtttg aactacttcc caagaattgc cgtggctgcc 780

ccagggagat acctgtagac agcccggagc tgaaggaggc acttggtcat tccattgcga 840

gacttaatgc acagcataac catattttct atttcaagat tgacaccgtg aaaaaggcaa 900

catcacaggt ggttgctgga gtaatatatg tgattgagtt catagccaga gaaactaact 960

gttccaagca aagtaaaaca gaactgacag cggattgtga gaccaaacac ctcggtcaaa 1020

gcctcaactg caatgctaac gtgtacatga gaccttggga gaacaaagtc gtcccgactg    1080

tcagatgcca agcactagat atgatgattt ctaggcctcc aggattttca cctttccggc    1140

tggtgcgagt acaagaaact aaagaaggaa cgactaggct cctaaactca tgtgagtaca    1200

agggcagact ctcaaaggca ggggcaggcc cagcacctga gcgtcaggca gaagcttcaa    1260

ccgtgacacc atagcccggc aaagacccgg agtggaagga ccagaagact cctgggatgt    1320

gtgcagcatg gaagcatgtt tcttcatcac ctgatcctgg gtgaaataaa gttcagactc    1380

gacgagttc                                                            1389

<210>  44

<211>  2218

<212>  DNA

<213>  Rattus norvegicus (Norway rat)

<400>  44
tctcccgggt attggctgca gctaagcggg gcttcctggg ccgactgagg tgggtagcca      60

gaaaaaggcc tgaggtggct gacaaccagg gcggccagac aacgcgagtc ctgcgcgcct     120

tctgaggcct ccctgggact taactgtaac ggggaggggc ctctggagca gcggccagcg     180

agttaaagta tgctgggagc cgtgaagatg gaagggcacg agccatccga ctggagcagc     240

tactacgcgg agcctgaggg ctactcttcc gtgagcaaca tgaacgccag cctggggatg     300

aatggcatga acacttacat gagcatgtcc gcggctgcaa tgggcagtgg ttccggcaac     360

atgagcgcag gctccatgaa catgtcatcc tatgtgggcg ctggaatgag cccgtcgctg     420

gctggcatgt ccccgggcgc gggcgccatg gcgggcatga gcggctcagc tggggcggcc     480

ggcgtggcgg gcatgggacc gcacctgagt ccgagtctga gcccactcgg gggacaggcg     540

gccggggcta tgggtggcct tgctccctac gccaatatga actccatgag tcctatgtac     600

gggcaggcgg gcctgagccg cgctcgggac cccaagacgt accggcgcag ctacactcac     660

gccaagcctc cctactcgta catctcgctc atcaccatgg ccatccagca gagccccaac     720

aagatgctga cgctgagcga gatctatcag tggatcatgg acctcttccc tttctaccgg     780

cagaaccagc agcgctggca gaactccatc cgtcattctc tctccttcaa cgactttctc     840

aaggtgcccc gcgcgccaga caagcctggc aagggctcct tctggaccct gcaccctgac     900

tctggcaaca tgttcgagaa cggttgctac ctgcgccgcc agaagcgctt caagtgtgag     960

aacgaactgg cgttgaagga agcagcgggt gcgggcagtg gcggaggcaa gaagaccgct    1020

cctgggacac aggcttctca ggttcagctc ggggaggccg caggctcggc ctctgagact    1080

ccggcgggca ccgagtcccc ccattccagc gcttctccgt gtcaggagca caagcgaggt    1140

ggcctgagcg agctgaaggg aacacctgcc tctgcgctga gtcctccgga gccggcgccc    1200

tcgcctgggc agcagcagca ggctgcagcc cacctgctgg gcccacctca ccatcctggc    1260

ctgccaccag aggcccacct gaagcccgag caccattacg ccttcaacca ccccttctct 1320

atcaacaacc tcatgtcctc cgagcagcaa catcatcaca gccaccacca ccatcagccc 1380

cacaaaatgg acctcaagac ctacgaacag gtcatgcact accctggggg ctacggttcc 1440

cccatgccag gcagcttggc catgggccca gtcacgaaca aagccggcct ggatgcctcg 1500

cccctggctg cagacacttc ctactaccag ggagtgtact ccaggcctat tatgaactcg 1560

tcctaagaag atggctttca ggccctgcta gctctggcca ctggggagaa ggacaaggga 1620

aatgacaggc tgagtggaaa cattggggga actttgagga aaaagtagcc accacactta 1680

aggccccaaa ggagcagttt cacctgtctg tgtccctaaa tagctgggcc acactgatct 1740

gtcattctaa atagggaagg gaatggaaat atatatgtat acacataaac ttgttttaat 1800

ggaggacctt tggttcccac tatgtagact actgcttctc aaggcacctg cagattttga 1860

tttttgttct tgattctctc tctattgctg tcgttgacag agaagtctga cttaaaaaaa 1920

aaacaaacta aacaaaaaaa aaaacttttg atgagtgact tgagtgtaaa accatgtagt 1980

tttaacagaa aaccagaggg ttgtattgat gtttaaaaga gaaaaaaaaa taatgatgta 2040

agagtctggt ataaatgacc aggagaaagg acaaaaatgc atccattctg gacatggtga 2100

aatccaggtc tcgggtctga tttaatttat ggtttctgcg tgctttattt atggcttata 2160

aatgtgtgtt ctggctagaa tgccagagtt ccacaaatct atattaaagt attattgc 2218

<210> 45

<211> 2622

<212> DNA

<213> Rattus norvegicus (Norway rat)

<400> 45
cacctctgcg tattcacgga atggggaaat gccaagagcc ttccagtacg atttggtgtc 60

cagttctacc ctgtacaccc cttccgtgaa cttctcatct gtggtgagcc cgtgcagctc 120

tccagactcg gcggtcttcc cagaggcaaa cggctcccag cttccgtctg cagtcttttt 180

gaacactttc acggtcacag tcctcactca cccacggagc atccaggatg aagtctgcga 240

ctgggcctct gcttcctaca ttgctggggc tactgctcct gtctatacca aggactcagg 300

gtgtcaaccc cgccatggtg gtcaggatca ccgacaaggg cctggagtac gcggccaagg 360

aggggctgtt gagtctgcag agagagctgt acaagatcac actgcctgac ttcagcgggg 420

acttcaagat caaggctgtg ggccgtggac agtacgagtt tcatagcctg gagatccaga 480

gctgtcagct gcgtggctcg tccctgaagc cgctcccagg ccgaggcctg agtctctcca 540

tctctgactc ttcgatcagc gtccggggca aatggaaagt gcgcagatcc ttcgtgaaac 600

ttcacggctc ctttgacctg gatgtcaaaa gtgtcactat ttcagtggac ctcctcctgg 660

gcgtggatcc ctcagaacgg cccacagtca ccgcctctgg atgcagcaac cgcattcgtg 720

atttggaatt gcacgtatca ggaaatgtgg ggtggctgct gaatcttttc cacaaccaga 780

tcgagtccaa gctccagaaa gtattggaaa gtaagatttg tgagatgatc cagaagtctg 840

tgacctctga tctgcagcct tatctccaaa ctctgccagt cacagcggat atcgacacta 900

tcctgggcat tgactacagt ttggtggcgg ctccccaagc aaaggcccag acgctggatg 960

tgatgtttaa gggtgaaatt tttaatcgga atcaccgctc cccagtcact acccccaccc 1020

cgaccatgag cctacctgag gacagtaaac aaatggtcta ctttgccatc tcagatcagg 1080

ccttcaacat agccacccgg gtttaccacc aggccgggta cctgaacttt accatcacag 1140

atgacatgtt accgcctgac tccaacatcc ggctgaacac caaggccttc cgccccttca 1200

ctcctctgat aaccagaaag taccccgaca tgaacttgga gcttcttgga acagtggtct 1260

ctgccccact tctgaatgtc agtcctggga atctgtcctt ggccccacag atggagattg 1320

aaggctttgt gatcctgccc agctccgccc gcgaatctgt cttccggctt ggcgtggtca 1380

cgaatgtatt cgtctcatta acttttgaca acagcaaggt caccgggatg ctgcatccag 1440

agaaggcgca agtgagactg atcgaatcca aagtcggcat gttcaatgtg aacctgttcc 1500

aggcattcct caactactac cttctcaaca gcctctaccc tgatgtcaac gatgagctgg 1560

ccaagggctt ccccctccct ctaccaaggc gtattaagct ccacgacctc gacttccaga 1620

tccacaagaa cttcctatac ttgggtgcca atgtccagta catgagagtc tgaggacaag 1680

aagaaagatg ggcctcagag gccacagcgg gacctgccat ttgtaattcc agatgcgtag 1740

cacatctcca gagagtctca aaatacaaag aagtttctgt tcctggctct ggtggatcct 1800

gtccccacag tcctcttcgc caggtgcacc ctcagcctgg acttgactct gacctctcca 1860

gggagaagcc ctcccctcac caacctctcc agggagagcc cctcccccca ccaacctctc 1920

cagggagacc cctctcccac cactgacctg gaatcactta aagagcaggc actgtggttt 1980

tgagtgcacc ttctcacctt catgtctgac ggagtgctgg cacttagtag gtcctcaata 2040

aatatttata gaatgacatg acagcccagc tgaacctctt tattgctaga ccatctggtc 2100

tgagccagcc ttagatgctc tgtcagagct gttatctcca aggctagacc accttttcac 2160

tcttgttggc ctctgctatg agggcctcaa caagggagtg aatgactaca cacacacaca 2220

cacacacacg cgcacacaca cacacacgca cgcacgcacg cacgcacacg catgcacatg 2280

catacactca cgcacgtgca cgcacacacc actcccttca ccagcacgtg tctaggcttc 2340

tagccttatt cccacagata cctcctcctt gcctcctgct tgctgcagac aacagaccca 2400

gaaggaaagc aaaattgtag ccccccgagg ctgtccccat ggaggtctgt gcaagtgaga 2460

aagagatgga gccaaggaag gttttggttg gacccaaatc aaacgctcat cggactgttg 2520

ttcacgagcc acatgcctgc gaggagagac catgatttct aactaccgaa caataagcct 2580

ttgatcagac ttaataaaga gtcatttccg tgttatgtaa aa 2622

<210> 46

<211>  577

<212>  DNA

<213>  Rattus norvegicus (Norway rat)


<400>  46
ggaattcggc acgaggcagg ctgagctaca gaccctgtca acatgtttgt gacatacgtc      60

cttgcctctg ctttgctctt tgggtctgtc ctgggccaga gatgcagcac ctcctggggc     120

atccaacaca cctcttacct tattgaaaac ctgaaggacg acccatcatc aaaatgcagc     180

tgcagtgcca acgtgaccag ctgcttgtgc ctccccatcc catctgatga ttgtaccaca     240

ccgtgcttcc aggagggaat gtcacaggtg accaatgcca cccagcaatc aaaattctca     300

cctttttctt ttcgggtgaa aaggatagtt gaaaccctaa agagcaacaa gtgtcagttt     360

ttctcctgtg aaaagccgtg caaccagacc acagcaggca acaccgtgtc atttctgaag     420

agtctcctga agaccttcca gaagacagag gtgcaagtgc agagaagcag ggcgtgaaga     480

cagatactat ttattctatt tattgaattt acaaaacctt ttctccctaa ttgttttaat     540

tgttacaatg aagaaataaa ctaagctatt ctagatt                              577

<210>  47

<211>  2401

<212>  DNA

<213>  Rattus norvegicus (Norway rat)


<400>  47
caacatgggg gactctcacg aagacaccag tgccaccatg cctgaggccg tggctgaaga      60

agtgtctcta ttcagcacga cggacatggt tctgttttct ctcatcgtgg gggtcctgac     120

ctactggttc atctttagaa agaagaaaga agagataccg gagttcagca agatccaaac     180

aacggcccca cccgtcaaag agagcagctt cgtggaaaag atgaagaaaa cgggaaggaa     240

cattatcgta ttctatggct cccagacggg aaccgctgag gagtttgcca accggctgtc     300

caaggatgcc caccgctacg ggatgcgggg catgtccgca gaccctgaag agtatgactt     360

ggccgacctg agcagcctgc ctgagatcga caagtccctg gtagtcttct gcatggccac     420

atacggagag ggcgacccca cggacaatgc gcaggacttc tatgactggc tgcaggagac     480

tgacgtggac ctcactgggg tcaagtttgc tgtatttggt cttgggaaca agacctatga     540

gcacttcaat gccatgggca agtatgtgga ccagaggctg gagcagcttg gcgcccagcg     600

catctttgag ttgggccttg gtgatgatga cgggaacttg gaagaggatt tcatcacgtg     660

gagggagcag ttctggccag ctgtgtgcga gttctttggg gtagaagcca ctggggagga     720

gtcgagcatt cgccagtatg agctcgtggt ccacgaagac atggacgtag ccaaggtgta     780

cacgggtgag atgggccgtc tgaagagcta cgagaaccag aaaccccctt tcgatgctaa     840

gaatccattc ctggctgctg tcaccgccaa ccggaagctg aaccaaggca ctgagcggca 900

tctaatgcac ctggagttgg acatctcaga ctccaagatc aggtatgaat ctggagatca 960

cgtggctgtg tacccagcca atgactcagc cctggtcaac cagattgggg agatcctggg 1020

agctgacctg gatgtcatca tgtctctaaa caatctcgat gaggagtcaa acaagaagca 1080

tccgttcccc tgccccacca cctaccgcac ggccctcacc tactacctgg acatcactaa 1140

cccgccacgc accaatgtgc tctacgaact ggcacagtac gcctcagagc cctcggagca 1200

ggagcacctg cacaagatgg cgtcatcctc aggcgagggc aaggagctgt acctgagctg 1260

ggtggtggaa gcccggaggc acatcctagc catcctccaa gactacccat cactgcggcc 1320

acccatcgac cacctgtgtg agctgctgcc acgcctgcag gcccgatact actccattgc 1380

ctcatcctcc aaggtccacc ccaactccgt gcacatctgt gccgtggccg tggagtacga 1440

agcgaagtct ggccgagtga acaagggggt ggccactagc tggcttcggg ccaaggaacc 1500

agcaggcgag aatggcggcc gcgccctggt acccatgttc gtgcgcaaat ctcagttccg 1560

cttgcctttc aagtccacca cacctgtcat catggtgggc cccggcactg ggattgcccc 1620

tttcatgggc ttcatccagg aacgagcttg gcttcgagag caaggcaagg aggtgggaga 1680

gacgctgcta tactatggct gccggcgctc ggatgaggac tatctgtacc gtgaagagct 1740

agcccgcttc cacaaggacg gtgccctcac gcagcttaat gtggcctttt cccgggagca 1800

ggcccacaag gtctatgtcc agcaccttct gaagagagac agggaacacc tgtggaagct 1860

gatccacgag ggcggtgccc acatctatgt gtgcggggat gctcgaaata tggccaaaga 1920

tgtgcaaaac acattctatg acattgtggc tgagttcggg cccatggagc acacccaggc 1980

tgtggactat gttaagaagc tgatgaccaa gggccgctac tcactagatg tgtggagcta 2040

ggagctacca ccctcccacc cctcgctccc tgtaatcacc taacttctgc cgacctccac 2100

ctctggtggt tcctgcctgg cctggacaca gggaggccca gggactgact cctcctggcc 2160

tgagtggtgc cctcctgggc ccctaggcag agcccggtcc attgtatcag gcagcccagc 2220

cccagggcac atggcaagag ggactggacc cacctttggg tgatgggtgc cttaggtcct 2280

ctgcagctgt acagaagggg ctcttctctc cacagagctg gggtgcagcc cccacacgtg 2340

attttgaatg agtgtaaata attttaaata acctggccct tggaataaag ttgttttcag 2400

t 2401

<210> 48

<211> 2639

<212> DNA

<213> Rattus norvegicus (Norway rat)

<400> 48
aaatatagta tttaatattt ttttgaaaga ctcagcccat tacaatacag aatggaatca 60

ccatattcct agtctcttct tccttcacca acagcctggt gctaacacaa tgcattcatc 120

ttaatatttc tgtatagaca tcagtgataa gaaggcctcc aggattttca cctttccggg 180

cacctcgagt gaaaaagcct aaagaaagta caactgtaag tccatcctac attgccaggg 240

tgcaagaaga gagggatcca ggaaatgaac aaggacccat ccatgggcat ggctggttgc 300

atgcaaagca aataaagaat aagaatcacc aaggtcataa gcatgggcat ggtattggcc 360

atggacacca gaaaccacat ggccttggtc atggacatca acttaaactt gatgatctta 420

aacagcaaag ggaagacggc tatgaccata gacatccagt gggacatggt catggtcaga 480

ggcatggtca tggtcatggt catggtcacg gtcgtgataa acacacaaat aaagacaaaa 540

acaatgtcaa gcacactgac cagagagcag agcctttgac aagctcttct gaagacaata 600

ctacatctac acagatacag gggaggacag agggcttcac cttgaaccct cccctagctc 660

agccagctgt tatctctcgt ggttttcagg actcaggttt cactgaaggt gtgatagcta 720

ccacatcacc atatgacacg gagacccatg atgatttgat ccctgatatc catgtacaac 780

cagatagcct ttcatttaag ctgatatctg actttccaga agcaacttcc cacaagtgtc 840

ctgggcgccc atggaagcca gttagtagga aggatccaac catagaaaca acagaatttt 900

ctgattttga tctcctcgat gctctttctt aacttataca gcgtaggaat ctttacaaat 960

gctttcccag cctctttttc tactgcccaa acacaaatat tgtgacataa gtcatcaagc 1020

catgaggctc agaacagcct gtcagtagga ctttataaat ccctgtggac tgataataaa 1080

actgccatcc ttctgaattc cttctgagcc tgcctcacac gctctctgaa ccaatacagg 1140

aagaagccta ccagaatcca ctgctcagat aatgagtggg tatctcaaga tacacatcgc 1200

atttccatac agaattatgg tctctgtgtt tagaaaacag aaaatcaaga gactgaaggt 1260

tgagtttatg gatgggggaa aataacagca aaacttccag atgtcagaga aagataagaa 1320

aacagaaaca ggctgatcaa agggagaaag tgggcagtaa tgacttgact ttatgtttct 1380

caagcaggtt aagtatatca aacgagactc ccccttgagc aggttagcct tggatttcct 1440

tttgtgggtg atggtgttcc tcactagtct acccctggct agtctttgtc atagctttca 1500

agcaagagct ttttggtagt gttgctgagg tcagatcaag caatccttac ttctcagaag 1560

ttctgcatct aacaccaagg gcagaaagta gaaggaagaa tattgaagta ggtttgctgt 1620

ggcaaccttt tagttcttgt gataaggcat gtcgtgggag ttgatggaaa cttcattcct 1680

accttttaga agccagtcct tagcttcacc ttaaattgct ctatcttttt gctatgacgc 1740

tgaagactat tgactttgga gaaagagaaa gaggttatca ctcatggcct tagaatgtga 1800

ggaaggtgtg tggtttacaa cccatgctgt gcttttgctg gaaaaagaag ataggacttt 1860

ctgaaggcag agataagtcc tccaggctcc agcagacagt tccataccca tgatctctga 1920

gagactctag gtaaattccc tggccactaa acaaagacct gaaccccaga agtgaccctg 1980

tagggaggag ggacccctga caggtatgga aggagaacac agagtggggg atcagaacag 2040

tcagaatgtg tgtgattgtg ggactcactg cagagtcccc acaaactccg attacagaaa 2100

gcaaggtgcc tgcacaatgc actggagaga tgtgtgggaa taggaaataa ggatgctgct 2160

tcacacggct acacagcctg tgtgtgtgtg tgtatggctg ttgtgggaaa ctgcatttga 2220

gctgagtgtt ctttcatttt aatcattgtt ttcctttaaa atggagaccc aatgtcactg 2280

ggaaacattc tcaccctgta tctggctgcg ctgctttgcc taagttgagc agaagcacga 2340

gattaacagc gttttactat attacagagg ctcctaaact catgtgagta caagggcaga 2400

ctcttaaagg caggggcagg cccagcgcct gagcgtcagg cagaagcttc aaccgtgaca 2460

ccatagcccg gcaaagaccc ggagtggaag gaccagaaga ctcctgggat gtgtgcagta 2520

tggaagcatg tttcttcatc acctgatcct gggtgaaata aagttcagac tcgacgagtt 2580

cacagtgtct ccttcagcca ttcctatctt gtagtgaatt gaagctgtct ccaaagctt 2639

<210> 49

<211> 250

<212> DNA

<213> Rattus norvegicus (Norway rat)

<400> 49
cttaattttc tcttctttcc actaccacag attatggaaa tgcctcagga cgcagtgaat 60

aagaagtgtt tcgccagagc cctgacctca ggacttccca agaaaaacag tgtatttgta 120

tttccagaga tttgatcaat aaaccatttt tttcatatct acccatcacc attccctcat 180

taattcagtc atcatttttt tacattttca tgcttccaca tctagtactc aataaaaact 240

gatgaaaatc 250

<210> 50

<211> 300

<212> DNA

<213> Rattus norvegicus (Norway rat)

<400> 50
gtcccaggat ttatgaccct gtgtgtggga ctaacggaat tacttacccc agtgaatgca 60

gtctgtgctt tgaaaacagg aaattcggaa catctatcca cattcagagg agatagagcg 120

tctgcaaaaa cagatcgaac ggcataagaa gaagattaat acctaaagaa tagtgaggca 180

ttgagtgcac acagtcagtc tctcacatag tggcagtatc attcccactc ttatagagat 240

tgttttgaat gattgatgtt tgaccatgtg tgctactaac agataataaa ttatcaccag 300

<210> 51

<211> 560

<212>  DNA

<213>  Rattus sp.

<400>  51
aaatgaaaca aggtttatgc cacacagtcc aacaatgtat aaagagcttg aagtagaaaa      60

gcttgtggaa aatgtatact ctttacatgg tacatacagt tttatagctc ataaataaac     120

acagagaaca ggagccattg ttcacattac cgaccagcaa cacagagcag cagtaacaac     180

acaaacacaa tttccttcga ctcagccaca aaaacttgaa atggtctcac catacttgag     240

gaaaatgaca ggtggtgaag tgttacagcc acacccacat caccaagtgc tttaagttgt     300

ctctgggtta gaaatatcaa caaccgtatt aactccagag agaagaaggc agggtaagac     360

cctgtcctat aaggcactta ataaattgac actacccttg tcttaaaata ctgcaaattt     420

gccttgaaat atgctgatgt tttacggcga aatttaggtt tcttttatcc aggtttagac     480

atagacataa tttgaataaa ttgataatac tgtctgatgg ccactcttta caccagcttc     540

aaaatggacc catttaaact                                                 560

<210>  52

<211>  479

<212>  DNA

<213>  Rattus sp.

<400>  52
gaatctaggg catctcaggg atccccggga acaatcttgc ctcaggcccc tcccccgcaa      60

ctccctgcat ctgcatcccc cttgcctgaa agcccttcta agctcggaaa ctgtgagcta     120

aggagacata gcacaaaatc ataaatgagt tgcggggaga ggctggaaac agtgtgcaag     180

aaatacaggc caggggttgg ggatttagct cagcggtaga gcgcttgcct agcaaacaca     240

aggccctggg ttcggtcccc agctccaaaa aaaagaaaaa aaagaaaaaa gaaaaaaaga     300

aaaacaggcc aatgctacat gtgaggcttt gtggctgggc tgtgacccag cgatatgggc     360

agtggagggg atcttttgtc tttggggact ctctggagcc caacgatcca ggctgtggtg     420

caggggatag taaagttaaa atgctaccca ccttttggcc ggttcctttc ccctcaacc      479

<210>  53

<211>  494

<212>  DNA

<213>  Rattus norvegicus (Norway rat)

<400>  53
cggccgctcc aacatcagcc gaggtagcct ggggttggta ccagaggccc actgacacgc      60

tgggtttctg aggtctggtc cagtggacag aatcttcttc ttttacagcc tcctcatcga    120

gtgatttcag gatctccttc ttcgtgtcct ggttaccagc aagggcaaat gcataggcca    180

acagcgcctt agtgtagaca tggctgccac cagctcctcc ccttgctgac ttccaggctg    240

tgtccaggca aaagagggca ttgcggacaa caggatgagt gacaggaaga gacatctcca    300

ggagagctat ggtgatgtag gcagacaagg tgacttcatc ttctactcct cccttcatgg    360

cattgttgag cagtgacccg gagctcctga aacaaccatt gtccttctgc tgctgagaga    420

gccataagaa ggcttgggtg atgtgtactt catcgatgaa gatatatttt cgagcctgag    480

caaaactctt cagt                                                      494

<210>  54

<211>  454

<212>  DNA

<213>  Rattus norvegicus (Norway rat)

<400>  54
tttttttttt tttttttctg tgaacacatt tatctttatt gagttctcac cctgggtcac     60

acaggaaggc gtttgctcat ctgtgctctc atccaccccc agccctgtta agtggcactc    120

agtgtgttta tttaacagaa aaggaaactg aggttcagag tagagggttt cagcgaagac    180

cagtcagcca ggagggacag ttggatccca agctctggga gtcctccctt ttgtctgcct    240

ccaactccga ctcagctctt ttcctcacca gctacattca tgagagattc tggaaccttc    300

cagttcatgc ccaccccgcc agtccagtct agggaccctt cacagaatgg ttttcttcac    360

gtgggctcga gggcgatcag gtaacagagg tgggtgagtg tccgagccca gaaagatgag    420

gtcgcttgag aggaagtcct tctctttgct ggaa                                454

<210>  55

<211>  441

<212>  DNA

<213>  Rattus sp.

<400>  55
atgcttaaaa atatatatgt attggggcat ggttaatcct ggctaattaa aatatgcatt     60

acctcaaaac atgatcaaag ctttctcata atactgtacc ggaatagttt aaatgcatca    120

ttaacattcc gattgcatta tccagtagtt aatcaacaga atattaaatc tttgcccaaa    180

aaacttttgg aacaggaact caaaagccca aaactcagct actcttgggg ctcccatctc    240

cagttctctt aatcctggct aaaacagcgg ccttaaaaac ttttttccgg tcgtaagcca    300

ggcccagggc agccatgcag tcgatgaaaa acgtggtgaa attgatgtgc cagcggtact    360

cactggcaaa gtagtcctag gggaaggcct gatggtagtt gtggaaaccc ttgcccacgg 420

tacccaggga aaccaggatg t 441

<210> 56

<211> 540

<212> DNA

<213> Rattus sp.

<400> 56
ttgagttctc tgataaatac atagagtttt aatgatcagt attcgatagt ggatgaagac 60

atgcattagt tagactagaa atgccacaca tttgttcata tatttgttca ctataaaatt 120

ttctgtttcg aaatttaacg ttttggttcc cattttagaa tacacaataa tcaggaggat 180

acatgatggg gagccagttt tcagtaaaag acgcacaatg ggtccatccg agtaatttca 240

tcagttgaat acaatttttc cagttttctt ttgttggttt ttcttctacg agtttggttg 300

caaacttaag gcctctcccg tacatcttat ttactaatgc atgcttgtat gcaaatgtca 360

aaaccttagt gtcaaaaaga tctgtccact ttgtagtttc ccaataggtt tctgatagag 420

attccaaggt actttctcct tcctcttctt ttgcttctgc atcttcagct gctgccccat 480

cgtggggctg tgcatgaaga aagtggtctg ccagagcaca ttctttcctg cagagggcat 540

<210> 57

<211> 499

<212> DNA

<213> Rattus sp.

<220>

<221> misc_feature

<222> (461)..(461)

<223> n

<400> 57
tttttccttt tttttttttt tttttatttt tactttctct ttttcttttg cacaacaaga 60

agtctacagg atcttttaag actgttgttt tgttctgctt tgttatcaac catttcacca 120

ggagaacaag ttgaatggac ctttttaaaa agaaaacgga aggaaaacta agagtgatca 180

tctcccccag gtgttctccg gcctggactg tgataaccgt tatttatgag agactttcaa 240

tgccctttct acagtcggaa ggttttcttt atatacttat tcccaccatg gggagcgaaa 300

atgttaaaaa aaaaaaaaat cacaaggaat tgcccaatct aagcagactt tgcctttttt 360

caaaggtgga gcgtgaatac cagaaggacc cagtattcgg ttacttaaat gaagtctttg 420

gtcagaaatg gcctttttga cacgagccta ctgaatgctg ngtatatatt tatatataaa 480

tatatatata tatatattg 499

<210> 58

<211> 413

<212> DNA

<213> Rattus norvegicus (Norway rat)

<400> 58
ttattaaaaa aaaaagtttt attttggttt acgtttccag agggatgaat ccatcaaggc 60

agggaggcgg gacagcaggg ggcaggcaca gaagcaacag gaagttgaaa attcacatct 120

tcaaacacaa gaaggaagca gaaagggggg gtgaggagaa agcagtgttt gatatttcct 180

acacacacat gtcaacattc accgttctta gaccactgag tcaggctctg acatccttct 240

gagcctcaca agggaatggt tttgccattc ccatgaggcc atgcactgag gtactaaaca 300

tggctgtggc catgtcaaca acatagcccc actctggacc tcactctaga cactgtaaag 360

aggacaggag gaccccatgc atgtaactat ggggaaagct atcattcgcg ctg 413

<210> 59

<211> 355

<212> DNA

<213> Rattus sp.

<220>

<221> misc_feature

<222> (61)..(61)

<223> n

<220>

<221> misc_feature

<222> (75)..(75)

<223> n

<220>

<221> misc_feature

<222>  (221)..(221)

<223>  n

<220>

<221>  misc_feature

<222>  (243)..(243)

<223>  n

<400>  59
gacgtaaaat agaaacagac tttatttctc tggaagaagc agatatccat ggctgggaca      60

nagctttggc aacanaggcg atgggaacac atcaaatgga cacaggggag gaacaggcat     120

caaacaggac aagtactggt gccgctgggg tctccctcca cacccggggc ctggggccct     180

ggtccctgcc agagaagatc ctggcgcctc ttctgtttct nagccacttc aggctgttta     240

canttacaag atctaagacc agccaagccc gagttcacag tgaagccaca ggtcacattc     300

tgtccaacac tccacattcc tacaggggtt ccctgggaaa agggggcctg gtcct          355

<210>  60

<211>  1912

<212>  DNA

<213>  Rattus sp.

<400>  60
cagattagga tcagcgagca cttgaggact tagggccaca aaaaaccgca caagatcgac      60

agactatttc tggagagctg cagaacgggc acgctggggt cgctggtgct ggccatggtg     120

atggaggtgg gcatcctgga cgccgggggg ctgcgcgcgc tgctgcgaga gcgcgccgct     180

cagtgcctgc ttctggattg tcgctccttc ttcgccttca acgccggcca catcgtgggc     240

tcagtgaacg tgcgcttcag caccatcgtg cggcgccgcg ccaagggcgc catgggcctg     300

gagcatatcg tgccgaacac cgaactgcgc ggccgcctgc tggccggagc ctatcacgcc     360

gtagtgctgt tggacgaacg cagcgccgcc ctggacggcg ccaagcgcga cggaaccctg     420

gccctggccg cgggcgcgct ctgccgagaa cgccgctcca ctcaagtctt ctccctccaa     480

ggaggatatg aagcgttttc ggcttcctgc cctgagctgt gcagcaaaca gtccaccccc     540

atggggctca gcctcccgct gagtactagt gtgcctgaca gtgcagaatc cggatgcagc     600

tcctgtagta cccctctcta cgaccagggg ggcccagtgg agatcctgtc cttcctgtac     660

ctgggcagtg cttaccatgc ttcccggaaa gatatgctcg acgccttggg tatcactgct     720

ttgatcaacg tctcggccaa ttgtcctaac cactttgagg gtcactacca gtacaagagc     780

atccctgtgg aggacaacca caaggcagac attagctcct ggttcaacga ggcgattgac     840

tttatagact ccatcaagga tgctggagga agggtgtttg tgcactgcca ggccggcatc 900

tccaggtcag ccaccatctg ccttgcttac ctcatgagga ctaaccgagt gaagctggac 960

gaggcctttg agttcgtgaa gcagaggcgg agtattatct cccccaactt cagcttcatg 1020

ggccagctgc tgcaatttga gtcccaagta ctggcccctc actgttctgc agaagctggg 1080

agcccggcca tggctgtcct tgaccggggc acctctacta caacggtctt caacttccct 1140

atctccatcc ctgttcaccc cacgaacagt gccctgaact accttcaaag ccccatcaca 1200

acctctccga gctgctgaag ggccagggga ggtgtggagt ttcacgtgcc accgggacga 1260

cactcctccc atgggaggag caatgcaata actctgggag aggctcatgt gagctggtcc 1320

ttatttattt aacacccccc ccccaaacac ctcccgagtt ccactgagtt cccaagcagt 1380

cataacaatg acttgaccgc aagacatttg ctgaactcag cccgttcggg accaatatat 1440

tgtgggtaca tcgagcccct ctgacaaaac agggcagaag ggaaaggact ctgtttgagc 1500

cagtttcttc ccttgcctgt tttttctaga aacttcgtgc ttgacatacc taccagtatt 1560

aaccattccc gatgacatac acgtttgaga gttttacctt atttatttgt gtgggtgggt 1620

ggtctgccct cacaaatgtg attgtctact catagaacaa cgaaatacct cactttgtgt 1680

gtttgcgtac tgtactatct tgtaaataga cccagagcag gctttcagca ctgatggacg 1740

aagccagtgt tggtttgttt gtagctttta gctatcaaca gttgtatgtt tgtttattta 1800

tgatctgaag taatatattt cttcttctga gaagacattt tgttactagg atgacttttt 1860

ttttatacag cagaataaat tatgacattt ctattgaaaa aaaaaaaaaa aa 1912

<210> 61

<211> 1908

<212> DNA

<213> Rattus norvegicus (Norway rat)

<400> 61
gcgcggtgaa gccagattag gatcagcgag cacttgagga tttagggcca caaaaaaccg 60

cacaagatcg acagactatt tctggagagc tgcagaacgg gcacgctggg gtcgctggtg 120

ctggccatgg tgatggaggt gggcatcctg gacgccgggg ggctgcgcgc gctgctgcga 180

gagcgcgccg ctcagtgcct gcttctggat tgtcgctcct tcttcgcctt caacgccggc 240

cacatcgtgg gctcagtgaa cgtgcgcttc agcaccatcg tgcggcgccg cgccaagggc 300

gccatgggcc tggagcatat cgtgccgaac accgaactgc gcggccgcct gctggccgga 360

gcctatacgc ccgtagtgct gctggacgaa cgcagcgccg ccctggacgg ccgcaagcgc 420

gacggcaccc tggccctggc cgcgggcgcg ctctgccgag aagcgcgctc cactcaagtc 480

ttcttcctcc aaggaggata tgaagcgttt tcggcttcct gccctgagct gtgcagcaaa 540

cagtccaccc ccatggggct cagcctcccg ctgagtacta gtgtgcctga cagtgcagaa 600

tccggatgca gctcctgtag cacccctctc tacgaccagg ggggcccagt ggagatcctg 660

tccttcctgt acctgggcag tgcttaccat gcttcccgga aagatatgct cgacgccttg 720

ggtatcactg ctttgatcaa cgtctcggcc aattgtccta accactttga gggtcactac 780

cagtacaaga gcatccctgt ggaggacaac cacaaggcag acattagctc ctggttcaac 840

gaggcgattg actttataga ctccatcaag gatgctggag gaagggtgtt tgtgcactgc 900

caggccggca tctccaggtc agccaccatc tgccttgctt acctcatgag gactaaccga 960

gtgaagctgg acgaggcctt tgagttcgtg aagcagaggc ggagtattat ctcccccaac 1020

ttcagcttca tgggccagct gctgcaattt gagtcccaag tactggcccc tcactgttct 1080

gcagaagctg ggagcccggc catggctgtc cttgaccggg gcacctctac tacaacggtc 1140

ttcaacttcc ctgtctccat ccctgttcac cccacgaaca gtgccctgaa ctaccttcaa 1200

agccccatca caacctctcc gagctgctga agggccaggg gaggtgtgga gtttcacgtg 1260

ccaccgggac gacactcctc ccatgggagg agcaatgcaa taactctggg agaggctcat 1320

gtgagctggt ccttatttat ttaacacccc cccccccaaa cacctcccga gttccactga 1380

gttcccaagc agtcataaca atgacttgac cgcaagacat ttgctgaact cagcccgttc 1440

gggaccaata tattgtgggt acatcgagcc cctctgacaa aacagggcag aagggaaagg 1500

actctgtttg agccagtttc ttcccttgcc tgttttttct agaaacttcg tgcttgacat 1560

acctaccagt attaaccatt cccgatgaca tacacgtttg agagttttac cttatttatt 1620

tttgtgtggg tgggtggtct gccctcacaa atgtcattgt ctactcatag aagaacgaaa 1680

tacctcactt tttgtgtttg cgtactgtac tatcttgtaa atagacccag agcaggcttt 1740

cagcactgat ggacgaagcc agtgttggtt tgtttgtagc ttttagctat caacagttgt 1800

agtttgttta tttatgatct gaagtaatat atttcttctt ctgtgaagac attttgttac 1860

tgggatgact ttttttatac aacagaataa attatgacgt ttctattg 1908

<210> 62

<211> 2370

<212> DNA

<213> Rattus sp.

<400> 62
gtggaattct tagagccaaa tgtgaatgat agcaataatc tatacattct agtagccagg 60

gaggacaagt tttgccatga aagatccagg ttacaaaact ctaatgcagt tcaaactcag 120

aatgatgaca cattccaaca ttcaatcagg aagggctaca aagaccctat ctaaaaaaac 180

aagaaacaaa aaccaaatca agtcaataaa ccaaagtacc actataggaa gatttgtaaa 240

tgttaaatta ttttaccaac gatctttatt tttaaaataa gcccaacttc aatcaaagtg 300

aagtttttaa tgttaattct ttatacaaat caatctttag agtgacagtt ctgagtttct 360

ctgtgtcacc ttggctgtcc tccaacagag gttgacctgc ctctgcctgc ccgagtgcta 420

cgattgaaga ccgtgccacc aacgctcgag gccttaaaga tgataaccta caaaattctg 480

ctttaaagaa tattctattg tgacgtaagc ctgtaaatct taccacttga gaggtctctg 540

atataattag atcacaagtt taaggtcagt ttgtgtcaca cagttcaagg ctagactgaa 600

cttcttcaca gcaagacctt atctcaaaaa caagcaaaaa cataaacaaa aaaggaaaaa 660

aagaaaaaag aaaaaaaccc acccaactac aataagatca caactcaaga attttaagtt 720

taacaaacat acttaagact tctcccaaaa aaaaaaaagt atattcccag aaatatagaa 780

actctctatt tcaatgattt tgtatatatc cttagagaag aaacattttc ttggcacaga 840

ggcagagctg aaacaatgtc ttactatgta agacattctc tcgtgccgaa ttcggcacga 900

gagaagaagg gcaggatgct gggggtctac tgcgagagtg gtacatgatc atctctcgag 960

agatgttcaa ccctatgtat gccttgttcc gtacctcacc tggtgaccgg gtcactacac 1020

gatcaatcca tcttcccact gtaaccccaa ccacctcagc tacttcaagt ttagctggac 1080

gatatgtagc caaagctgta atgacaaccg ccctcctgga gtgctacttt actaggtctt 1140

tctacaaaca catcttgggc aaatctgtca ggtatacaga tatggagagt gaggattacc 1200

acttctacca gggtctagtt tatctgcttg aaaatgatgt ctctacatta ggttatgacc 1260

tcaccttcag cactgaggtt caagaatttg gagtatgtga ggttcgtgac ctcaaaccca 1320

atggagccaa catcttagta acagaggaga acaagaagga atatgtacac ctggtttgcc 1380

agatgagaat gacaggagcc atccgcaaac agctggcagc cttcttagaa ggcttctatg 1440

aaatcattcc aaagcgcctc atatccatct ttactgagca ggagttagag ctgctctata 1500

cagggctgcc taccatcgac atagatgacc taaaatctaa cactgagtac cacaagtacc 1560

agtccaactc cattcagatc caatggttct ggagagcatt gcgttccttt gaccaagcag 1620

accgtgccaa gttcctccag tttgtcacag gtacttccaa ggtgcccctg caaggatttg 1680

ctgcccttga aggcatgaat ggcattcaga agtttcagat tcatcgagac gacagatcca 1740

cagatcgcct gccttcagct cacacatgtt ttaatcaact ggacctgcct gcctatgaga 1800

gctttgagaa gctccgccac atgctactat tggccatcca ggagtgctct gaaggctttg 1860

ggctggctaa taaataaggc cctgcctact tctgtggggt tttctaccca tttttggacc 1920

tggggagggg ggggggttta aaagatccag aaagaaaatg tcaaaatcca ataaatgaaa 1980

ttcaccaact caccatgtgt gtgtccagct gcccatcttc ctagtgcata cctgttcgct 2040

tctcattctc tttcctctac cctttccttg cctttccctt ttccatgccg tccatgatcc 2100

ccaccccatg tgttaagaag gcagttgctt tgcagggacc tgtttgtcca actgaacagt 2160

gtgctcctca gattctgtgt tcagaaggat ttgctgcatt gagacttgaa acctttggta 2220

aggggaaaat tatatatata tatatttttt tgttctgttt gcatttctta atttgtgctt 2280

ggaatgtgtt gatgtgcaca gctaatgatt caatgcgaga caagattggc atctgtgttg 2340

tggaggttca aataaagagc actcttcata 2370

<210> 63

<211> 1756

<212> DNA

<213> Rattus norvegicus (Norway rat)

<400> 63
ctgggtcggg gagacctttc ctctctggcc gtttcctaat tctgaatcat gtctgataac 60

ggggagctag aggacaagcc cccagcacct ccagtgcgga tgagcagcac catttttagc 120

actggaggaa aggatccttt atcagccaat cacagtttga agcctttgcc ttctgttcca 180

gaggaaaaaa aaccgaggaa caaaatcatc tccatattct caagcacaga aaaaggaagt 240

aaaaagaaag aaaaagaacg gccagagatt tctccgccgt ctgattttga gcataccatc 300

catgttggct ttgatgctgt tacgggagag ttcactggca tgccagagca gtgggcacgg 360

ctgttgcaga cctccaacat taccaaactg gagcagaaga agaacccgca ggctgtgctg 420

gatgtcttga agttctacga ctccaacact gtgaagcaga agtacctgag cttcactcct 480

cctgagaaag atggctttcc ttctggaaca ccagcactga acaccaaggg gtcagagaca 540

tcagctgtgg tgacagagga agacgatgac gatgaagatg ctgctcctcc cgtcattgcc 600

cctcggccag atcatacaaa atcaatctac acaaggtctg tcatcgaccc tattcctgct 660

ccagttggtg actctaatgt cgacagtggt gccaagtctt cagacaaaca gaaaaagaaa 720

gccaagatga ccgatgaaga gattatggag aaattaagaa ctattgtgag cataggtgac 780

cctaagaaaa aatacacaag atatgaaaaa atcgggcaag gggcttctgg tacagttttt 840

actgcaactg atgtggccct ggggcaagag gttgctatca agcagattaa tttacagaaa 900

caaccaaaga aggaattgat cattaatgaa attctggtga tgaaggagtt aaagaatccc 960

aacatagtta acttcttgga cagttacctg gtaggagacg agttgtttgt ggtaatggag 1020

taccttgctg gtgggtccct cactgatgtc gtgacagaaa cctgcatgga tgaagcgcag 1080

atcgcagctg tgtgcagaga gtgtttacag gcgttggagt ttttacatgc taatcaagtg 1140

atccacagag acatcaaaag tgacaatgtg cttttgggaa tggaaggctc agttaaactc 1200

actgattttg gcttctgtgc ccagatcacc cctgaacaga gcaaacgcag tactatggtt 1260

ggaacgccat actggatggc accggaggta gtcacgcgga aagcctatgg ccccaaagtt 1320

gacatatggt ctctgggcat catggctatc gaaatggtgg aaggagagcc tccatacctc 1380

aatgaaaatc ctttacgggc attatacctg atagcaacga atggaacacc tgagctccag 1440

aatccagaaa aactttcccc catatttcgg gatttcttaa atcggtgttt ggaaatggat 1500

gtggagaaga ggggttcagc caaagaacta ttacagcatc ctttcctgaa actggccaaa 1560

ccattatcca gcttgacscc tctgatcctg gcagctaaag aagcaatgaa gagtaaccgc 1620

taacgtcgtc agcgaggcct catgttcctt tatccatttt ctaaagaagt cttttaatac 1680

atgaaattgt tactctgagg ggtgtaaaga gacagtttgc atcacatgga gaaaagcaag 1740

caaacttatt attttc 1756

<210> 64

<211> 2746

<212> DNA

<213> Rattus norvegicus (Norway rat)

<400> 64
gctcctcaca gctccccctc cacctctgag tggatcctcc tctgagtttc tcttcttcct 60

cagagctcct cctcctcccg gtcctgcaag gctccagact tctcgacttg gtttcagaaa 120

gcaccggtgg ctgtagtcgg gattgagagg tgtttccaaa gaaacccaaa gagcagcagg 180

gcagccatga ggatgccgac ggggtctgaa ctgtggccca tagccatatt cacgatcatc 240

ttcctgcttc tggtggacct gatgcacagg cgccagcgct ggacttctcg ctaccctccg 300

ggccctgtgc cctggcctgt gctgggcaac ctgctgcaga tagacttcca gaatatgcca 360

gcgggctttc aaaagctgag atgtcgcttt ggggacctgt tcagcttaca gctggccttt 420

gagtcggtgg ttgtactgaa tgggctgcca gccctgcgag aggcactggt gaaatacagc 480

gaggacaccg ctgaccggcc accgctgcat ttcaatgacc agtcgggctt tggaccacgc 540

tctcaaggtg tggtcctcgc gaggtatgga cctgcctggc gtcagcagcg gcgcttctct 600

gtgtccacct tccgtcactt tggcctgggc aagaagtcac tggagcagtg ggtgacagag 660

gaggccagat gcctctgtgc tgccttcgct gaccatagtg gattcccttt cagccctaac 720

actctactgg acaaagcagt gtgtaacgtg atcgcgtccc tcctctttgc ctgccgcttt 780

gaatacaatg acccacgctt catcaggctc ctggacttgc tgaaggacac tcttgaggag 840

gaatctggat tcctgcccat gctcctgaat gtgttcccga tgctcctaca catcccaggg 900

cttcttggca aggtattctc tggaaagaag gccttcgttg ccatgctgga cgagctgcta 960

actgaacaca aggtgacctg ggaccctgcg cagccacccc gagatctgac tgatgccttc 1020

ctggctgagg tggagaaggc caaggggaat cctgagagca gcttcaatga tgagaacctg 1080

cgtgtggtgg tggctgacct gttcatggcg gggatggtga ccacctccac cacactgacc 1140

tgggccctgc tgttcatgat cctgcgtcca gatgtgcagt gccgagtaca acaggaaatc 1200

gatgaggtca tagggcaggt gcggcgtcca gagatggcag accaggcacg aatgccgttc 1260

accaatgctg tcatccatga ggtgcagcgc tttgcagaca ttctccctct tggtgtgcct 1320

cacaagactt ctcgtgacat tgaagtgcag ggcttcctta tccctaaggg gacgaccctc 1380

atcatcaacc tgtcctcagt gctgaaggat gagactgtct gggagaagcc cctccgcttc 1440

caccctgaac acttcctgga tgcccagggc aactttgtga agcatgaggc cttcatgcca 1500

ttctcagcag gccgcagagc atgcctgggg gagcccctgg cccgcatgga gctcttcctc 1560

ttcttcacct gcctcctgca gcgcttcagc ttctccgtgc ccgctgggca gccccggccc 1620

agcaactatg gcgtctttgg tgctctgacc accccgcgcc cctaccagct ctgtgcttca 1680

ccccgctaag gggaggcaca gcatctcact cactgtgctt gctggggtcc tagtgtgcaa 1740

taaatggttt tactctgaac cgaatcatcc ctgtgagctc tccaggctgt aaggggcctg 1800

agcagccttc ccgtggacat ccgcacccct acttaatctt ccttgaccat gtgccccaat 1860

ggaagggctg ctctactgac ctccgaaatg gcagccattc ttgctttcac ccctgccccc 1920

tcttttcacc caaattgatg atgtttattc atagatgcca acatctggaa ggagggccag 1980

aaaggactgc tgtgaagggt cagtgtaagt cacacagatg agggaagggg cggtggaggt 2040

aatggtgggc agaattgtcc cctttccact tgagatgttt ctcccagacg cccccatttc 2100

agacccacta cacaaccaag gctaactcct cagccagcat catcacaact tcttatatga 2160

cgtcgcagag atgtagagaa gtcggggagg ctggaaatga catgcaggtt aagtgcccaa 2220

ggttacctgt tgggtaccac atgcttccct aaacggtttt gtgggggtcc agaagcaggt 2280

tgcctcctaa gcttctttgt caccattaat tccatgaccc agcagggata ctggtgtcca 2340

ggcccatgca cagtaagaaa gtgactctaa ccagggatgg aaggacccgc aagcttagtg 2400

ttgacacaga ctcccagacc ttagcacaac tgactccatg gtagaagtac catttgggcc 2460

ataaaactta gcacgtagac agcagctcct ctcataatga aaacaaagac ctaacccatc 2520

aaattctatc ctgggaaggt ctcttgaagc actcctcttg gcttcttggc ttctgtagtt 2580

ctcctagcta actgctcttg ctaactgaag tatgtcaacc caggatatgg ttgttggtaa 2640

aagctcgccc tgagaacagc tcaggacgac attgaggtga cccagtgtag tcaccagcca 2700

gctaataaag acctcctttt ggtttaaaaa aaaaaaaaaa aaaaaa 2746

<210> 65

<211> 1395

<212> DNA

<213> Rattus norvegicus (Norway rat)

<400> 65
atggaactaa tttctgaaaa gtccaaagag ggagcacatc cctgggacaa agctgaacag 60

agtgactttg aagctgtgga agcgctcatg tccatgagct gcgactggaa gtctcacttc 120

aagaaatacc ttgaaaacag gcctgtcaca ccagtgtctg atacctcaga ggaagacagt 180

ttgcttccag ggacgcctga ccttcagacc gtcccagcat tctgtttgac tccaccttac 240

agcccctctg acttcgaacc ctcgcagggg tcaaatctga cggcaccagc gccacctact 300

ggtcacttca gatcgctctc cgatgctgcc aagcctccca gcatcgctcc ttttaaagag 360

gaggaaaaga gcccattagc tgcccctccc ctccctaagg ctcaagccac cagtgtcatc 420

cgtcacacag ctgatgccca actctgtaac caccagtcct gccctgtgaa agcagctagc 480

atcctcaact accaggacaa ttctttccgg agaagaaccc acataaatgt cgaggctact 540

cgaaagaaca taccctgtgc tgctgtgtca ccaaacagac ccaagtctga gcccagcaca 600

gcggcgaatg gcgctgagaa ggccggcact gcgccgtatg actttgctgt gccttcctca 660

gagacagtca tctgtaggtc ttctcagcca gctcccacgt ccccagtaca gaactcagta 720

ctgatgtctt cacctacagt gtccactggg ggagtgccac ccttgcctgt catctgccag 780

atggttcccc ttccagcaaa caactctctc gttaccacag ttgtccccag cagtccgccc 840

agccagccac ccgctgtctg ctcacccgtg ttgttcatgc actcagtgcc caagggcacg 900

gtcatgtttg tggtacccca gcccgttgta cagagcccaa agcctccagt ggtgagcccc 960

aatggcacca gactgtctcc cattgcccct gcgcctggat tctctccttc agcagcaagg 1020

gttactcccc agattgattc gtccagagta agttgtcaca tctgtagcca cccaggatgt 1080

ggcaagactt actttaaaag ttcccatctg aaggcccacg tgagaacaca cacaggggaa 1140

aaacctttca gctgcagctg gaaaggctgt gaaaggaggt ttgctcgttc cgatgaactg 1200

tccagacacc ggcggacaca cacaggtgag aagaaatttg cctgtcccat gtgtgaccgt 1260

cggttcatga gaagtgacca tttaaccaaa catgcccggc gacacctatc agccaagaag 1320

ctgccaaact ggcaaatgga agtgagcaag ttaaatgaca tcgctctgcc tccggccaca 1380

gcgtcggcac agtga 1395

<210> 66

<211> 2170

<212> DNA

<213> Rattus rattus (black rat)

<400> 66
ccacgcagga tcacgatgct gcccagcctg gcactgctcc tgctggccgc ctggacggtt 60

cgggctctgg aggtgcccac tgatggcaat gccggtctgc tggcagaacc ccagatcgcc 120

atgttctgtg gtaaactcaa catgcacatg aatgtgcaga atggaaaatg ggagtcagac 180

ccatcaggga ccaaaacctg cattggcacc aaggagggaa tcctgcagta ctgccaagag 240

gtctaccctg aactgcagat cacaaacgtg gtggaagcca accagccagt gaccatccag 300

aactggtgca agcggggccg caagcagtgc aagacgcaca cccacatcgt gattccttac 360

cggtgcctag ttggtgagtt tgtaagcgat gcccttctcg tgcccgacaa gtgcaagttt 420

ctacaccagg agcggatgga cgtttgtgag acccatcttc actggcatac tgttgccaaa 480

gagacatgca gtgagaagag cactaacttg cacgactatg gcatgctgct gccctgtggc 540

atcgacaagt tccgaggggt cgagttcgtg tgctgcccgt tggcggagga gagcgacagc 600

atcgattctg cggacgcaga ggaggacgac tccgatgtct ggtggggtgg agcggacaca 660

gactatgctg atggcggtga agacaaagtc gtagaagtag ccgaagagga ggaagtggcc 720

gatgttgagg aagaagaagc tgaggatgac gaggatgtgg aggatgggga tgaggtggag 780

gaggaggccg aggagcccta cgaagaggcc acagagagaa caaccagcat tgccaccact 840

accacaacta ccactgagtc tgtggaggag gtagtccgag ttcccacgac ggcagccagc 900

acccctgacg cagtcgacaa gtacctggag acccccggag atgagaacga gcacgcccat 960

ttccagaaag ccaaagagag gttggaagcc aagcaccgag agagaatgtc ccaggtcatg 1020

agagaatggg aggaggcaga acgtcaagcc aagaatttgc ccaaagctga caagaaggcc 1080

gttatccagc atttccagga gaaagtggaa tctctggaac aggaagcagc caacgagagg 1140

cagcagcttg tagagacaca catggccaga gttgaagcca tgctcaatga tcgccgtcgc 1200

ctggccctcg agaattacat caccgcactg caggcggtgc ctccaaggcc tcatcatgtg 1260

ttcaacatgc tgaagaagta cgtccgtgca gagcagaagg acagacagca caccctaaag 1320

cattttgaac atgtgcgcat ggtggacccc aagaaagctg ctcagatccg gtcccaggtt 1380

atgacacacc tccgtgtgat ctacgagcgc atgaaccagt ctctgtccct gctctacaac 1440

gtccctgccg tggctgagga gattcaagat gaagttgacg agctgcttca gaaggagcag 1500

aactactccg acgacgtctt agccaacatg atcagtgaac ccagaatcag ttacggcaac 1560

gatgctctca tgccttcttt gactgaaacg aagaccactg tggagctcct tcccgtgaat 1620

ggcgaattca gcctggatga tctccaaccg tggcatcctt ttggggtgga ctctgtgcca 1680

gccaatacag aaaatgaagt tgagcctgtc gacgcccgcc ccgctgctga ccgaggactg 1740

accactcgac cagggtctgg gttgacaaac atcaagacag aagagatctc agaagtgaag 1800

atggatgcgg agttcggaca tgattcaggc ttcgaagtcc gccatcaaaa actggtgttc 1860

tttgcagaag atgtgggttc aaacaaaggt gccatcattg gactcatggt gggtggcgtt 1920

gtcatagcaa cagtgattgt catcaccttg gtgatgctga agaagaaaca gtacacatcc 1980

atccatcatg gcgtggtgga ggttgacgct gctgtgaccc cggaggagcg ccacctctcc 2040

aagatgcagc agaatggata tgagaatcca acatacaagt tctttgagca gatgcagaac 2100

taaaccccgc cccccgccac agcagcggcc tctgaacttg acagcaaaac cattgcttca 2160

ctacccatcg 2170

<210> 67

<211> 1796

<212> DNA

<213> Rattus norvegicus (Norway rat)

<400> 67
gttcacagcc actgcgccct cgctctcttc ttgcagatct tgcagccgca gcaagccagg 60

ccacctcgtc cttcgaagcc atgtccacca ggtccgtgtc ctcgtcctcc taccgcagga 120

tgttcggtgg ctccggcaca tcgagccggc ccagctccaa ccggagctat gtgaccacat 180

ccacccgcac ctacagccta ggcagcgcgc tgcgccccag cactagccgc agcctctatt 240

cctcgtcccc gggtggcgcc tatgtgaccc ggtcctccgc cgtgcgcctg cggagcagca 300

tgcccggcgt gcggctgctg caggactccg tggacttctc gctggccgac gccatcaaca 360

ccgagttcaa gaacacccgc accaacgaga aggtggaatt gcaggagctg aatgaccgct 420

tcgccaacta catcgacaag gtgcgcttcc tcgagcagca gaacaaaatc ctgctggccg 480

agctcgagca gcttaagggc cagggcaagt cgcgcctggg cgacctctac gaggaggaga 540

tgagggagtt gcgccggcag gtggatcagc tcaccaatga caaggcccgt gtcgaggtgg 600

agagggacaa cctggccgag gacatcatgc ggctgcgaga aaaattgcag gaggagatgc 660

tccagaggga ggaagccgag agcaccctgc agtcattcag acaggatgtt gacaatgctt 720

ctctggcacg tcttgacctt gaacgtaaag tggaatcctt gcaggaagaa attgcctttt 780

tgaagaagct gcacgatgaa gagatccagg agctgcaggc ccagattcag gaacagcatg 840

tccagatcga tgtggacgtt tccaagcctg acctcaccgc tgccctgcgt gatgtccgcc 900

agcagtatga aagtgtggct gccaagaacc tccaggaggc cgaggaatgg tacaagtcca 960

agtttgctga cctctctgag gctgccaacc ggaacaacga tgccctgcgc caggcaaagc 1020

aggagtcaaa cgaataccgg agacaggtgc agtcactcac ctgcgaagtg gatgccctta 1080

aaggcactaa tgagtccctg gagcgccaga tgcgtgaaat ggaagagaat tttgcccttg 1140

aagctgctaa ctaccaggac actattggcc gcctgcagga tgagatccag aacatgaagg 1200

aagagatggc tcgccacctt cgtgaatacc aggacctgct caatgtaaag atggctcttg 1260

acattgagat cgccacctac aggaagctgc tggaagggga ggagagcagg atttctctgc 1320

ctcttccaaa cttttcttcc ctgaacctga gagaaactaa cctggagtca cttcctctgg 1380

ttgacaccca ctccaaaaga acactcctga ttaagacggt tgaaaccaga gacggacagg 1440

tgatcaatga gacttctcag caccacgatg accttgaata aaaactgcac aggctcagtg 1500

caacggcgca gtaccagcaa gaaggaaaaa aaaatcgtat cttagaaaaa agagctttca 1560

agtgccttta ctgcagtttt caggagcgca agatagatct gggatagaaa cgagctcagc 1620

acataacaac tgacaccccc aaaaggcgta gaaaaggttt acaaaataat ctagttttac 1680

gaagaaatct tgtgctagaa tactttttaa agtatttttg aataccatta aaactgcttt 1740

ctttttccag aaaatatctg accaacttgt tactgcttca ataaaacttc agaaat 1796

<210> 68

<211> 2924

<212> DNA

<213> Rattus norvegicus (Norway rat)

<400> 68
atggctcagt ggaaccagct ccagcagctg gacacgcgct acctggagca gcttcatcag 60

ctgtacagcg atagcttccc catggagctg cggcagttcc tggcgccttg gattgagagc 120
caagattggg catatgcagc cagcaaagag tcacacgcca ctctggtgtt tcataacctc 180
ttgggcgaga tcgaccagca gtatagccga ttcctgcagg agtccaatgt cctctatcag 240
cacaacctgc gaagaatcaa gcagttcctg cagagcaggt atcttgagaa gccaatggaa 300
attgcccgga ttgtggcccg atgcctgtgg gaagagtctc gcctcctcca gacggcagcc 360
acggcagccc agcaaggggg ccaggccaac caccccacag ctgccgtagt gacggagaag 420
cagcagatgc tggaacagca tcttcaggat gtccggaagc gtgtgcagga tctagaacag 480
aaaatgaaag tggtggagaa tctccaggat gactttgatt tcaactataa aaccctcaag 540
agtcaaggag acatgcagga tctgaatgga aacaaccagt ctgtgaccag acagaagatg 600
cagcagctgg agcagatgct cacggccctg gaccagatgc ggaggagcat cgtgagcgag 660
ctggcagggc tcttgtcagc aatggagtac gtgcagaaga cactgaccga tgaagagctg 720
gctgactgga agaggcggca gcagatagcg tgcatcggag gccctcccaa catctgcctg 780
gaccgtctgg aaaactggat aacttcatta gcagaatctc aacttcagac ccgccaacaa 840
attaagaaac tggaggagct gcagcagaaa gtgtcctaca agggggaccc tattgtgcag 900
caccggccaa tgctggagga gaggatcgtg gatctgttca gaaacttaat gaagagtgcc 960
ttcgtggtgg agcggcagcc ctgtatgccc atgcacccgg accggccctt agtcatcaag 1020
actggtgtcc agtttaccac aaaagtcagg ttgctggtca aatttcctga gttgaattat 1080
cagcttaaaa ttaaagtgtg cattgataag gactctgggg atgtcgctgc cctcagaggg 1140
tctcggaaat ttaacattct gggcacgaac acaaaggtga tgaacatgga ggagtccaac 1200
aacggtagcc tgtctgcaga gttcaagcac ctgaccctga gggagcagag atgtgggaat 1260
gggggccgtg ccaattgtga tgcctccttg attgtcactg aggagctgca cctgatcacc 1320
tttgagacag aggtgtacca ccaaggtctc aagatcgacc tagagaccca ctccttgcca 1380
gtcgtggtga tctccaacat ctgtcagatg cctaatgctt gggcatcaat cctgtggtat 1440
aacatgctga ccaataaccc caagaacgtg aacttcttca ctaagcctcc gattggaacc 1500
tgggaccaag tggccgaggt gctgagctgg cagttctcgt ccaccaccaa gcgagggctg 1560
agcatcgagc agctgaccac cctggccgag aagctcttag ggcctggtgt gaactactca 1620
gggtgtcaga tcacatgggc taagttttgc aaagaaaaca tggccggcaa gggcttctcg 1680
ttctgggtct ggctagacaa tatcatcgac cttgtgaaaa agtatatctt ggccctttgg 1740
aatgaagggt acatcatggg tttcatcagc aaggagcggg agagggccat cctaagcaca 1800
aagcccccgg gcaccttcct gctgcggttc agtgagagca gcaaggaagg aggggtcact 1860
ttcacttggg tggaaaagga catcagtggc aagacccaga tccagtctgt agaaccatat 1920
accaagcagc agctgaacaa catgtcattt gctgaaatca tcatgggcta taagatcatg 1980
gacgctacca acatcctggt atccccactg gtctacctct accctgacat tcccaaggag 2040
gaggcattcg gaaagtattg tcgccccgag agccaggagc accctgaagc tgacccaggt 2100

agtgctgccc cttacctgaa gaccaagttc atctgtgtga caccaacgac ctgcagcaat 2160

accattgacc tgccgatgtc cccccgcact ttagattcat tgatgcagtt tggaaataac 2220

ggtgaaggtg ctgagccctc agcaggaggg cagtttgagt cgctcacgtt tgacatggat 2280

ctgacctcgg agtgtgctac ctccccgatg tgaggagctg aaaggcaaag ctacagagta 2340

tgactgagac actgcccatg tcaccttagc agcaactcag atcactgaac tcactgtgtc 2400

cagatttttt ctttaacctc ctacttctgc tgtctttggg cagtctgggt actttttaaa 2460

atagagaact gagtgagcgt gggtgatgcg ctcttatgtg aggaggaggg cacctcttga 2520

gtccgtgatg gctgtgaaag cagagggctg ggggggagcg aaaggaaaca cctatgttct 2580

gtccgcctgt cctcctggtt tgttgttgac aagcgcctcc tggtgccatg gcatcctgct 2640

gcccctttct gtgagctgat accccaggct gggactcccg gctctgcact ttcaacctgg 2700

ttaatgtcca catagaagct aggactaagc ctataggctt ctctttaagt taaaaaaaaa 2760

aaaaatacta agaattaaag ggcaaaacac actgaaaaca gcatagcctt tccatatcaa 2820

agaaaactcg ggtaacagcc ttttcggtgc tttaagctgt cttaggctga tcatttatat 2880

aaactctgca atggttcaat caatctgtaa agacagctga gaac 2924

<210> 69

<211> 608

<212> DNA

<213> Rattus sp.

<400> 69
taataacaag tgtacaattt agcataagga atcggagagc ctctccagag aagtcggttt 60

ctttgctgca agaagaatga ggttctgaac ccttatccaa gaacagaagc catcagccaa 120

gtctccacat ttctctgcaa aatgttgtag cctctataac tgtatgatag tgtaatgcat 180

gccttcagtt gtaagtggcc agatcgcgct acagtgacat tgaaacctgc tctctaattg 240

gccctgtaca gtttgcttat ttataaattc atttaaaaca ctacagctgt tgaatggtta 300

caacctacgc ctccggtcct aacttcagtt gttctcctcg gtgtgcagcc agctgttcca 360

cactgtatta ttgtaactta tttagtgaag tcagaagcag tagacagatg ttggtgcaat 420

acaagtattg tgtgcattta tcgtaataaa gtgctccgcg tcggttcagt tcctcacagc 480

ttctcacagt gcatgtctga ctgtagtctg taaatagagg tcagtgtccg tgctgctaac 540

aggtatcgat cgcacagaca tgatttcagg taaataaatc gattctacga taaatgggta 600

aaagaaaa 608

<210> 70

<211> 1451

<212>  DNA

<213>  Rattus norvegicus (Norway rat)

<400>  70
gccaggcgcc ctgccgctgg acactgtaga acggcgggcg gaacgcagac ctgggttcgg      60

gctgttgcag tgtccgccgg agcggtttgg ggagtgcacg gggtgaagat ggcggaggag     120

cggcaggacg cgttgaggga gttcgtggcg gtgacgggcg ctgaggagga ccgggcccgt     180

ttcttcttgg agtcagctgg ctgggacctg cagattgcac ttgcaagctt ttatgaggat     240

ggaggggatg aagacattgt gaccatttca caggcaaccc caagttcagt gtccagaggc     300

acagctccta gtgataatag agtaacatcc ttcagagacc tcattcatga ccaagatgaa     360

gaggaagagg aagaagaggg acagaggttt tatgctgggg gctcagagag aagtggacag     420

cagattgttg gtcctccccg gaagaaaagt cccaacgagc tggtggatga tctgtttaag     480

ggtgccaaag agcatggggc tgtagctgtg gaacgagtga ccaagagccc tggagagacc     540

agtaaaccga gaccatttgc aggagggggc taccgccttg gagcagcacc agaagaggag     600

tctgcctatg tggcaggaga aaggaggcgg cattcaggcc aggatgttca tgtagtgcta     660

aagctctgga aaactggatt cagcctagac aatggtgacc ttagaagcta ccaagaccca     720

tccaatgccc agtttctgga gtctatccgt agaggggagg ttcctgcaga gcttcggagg     780

ttagctcacg gtgggcaggt gaacttggat atggaggatc atcgggacga ggacttcgtg     840

aagcccaagg gagctttcaa agccttcact ggcgagggcc agaaactagg cagcacggcc     900

ccgcaggttt taaacaccag ctctccagcc cagcaagcag aaaatgaagc caaagctagc     960

tcgtccatct taatcaatga ggcagaacct accacgaaca tccaaatccg actggcagat    1020

ggcgggagac tggtgcagaa attcaaccac agccacagga tcagcgacat ccggctcttc    1080

atcgtggatg cacggccggc tatggctgcc accagttttg tccttatgac taccttccct    1140

aacaaagagc tggctgatga gaaccaaacc ctgaaggaag ccaatcttct caacgctgtc    1200

atcgtgcagc ggttaacata accgcccagc tgctgcctca cctccctatg tgtcccccat    1260

gactgtggcc atgcccgtgg ggattacctc ctgccccctg tacacctagc tcttccagtg    1320

ccacgtctcg tccgtagctc tggattctta gacttcagtt ggacatttgt tttctcttta    1380

gttgtatttc ctgggttttt gtgatgatca atggacttta aagaaaaaaa ataaaaaaca    1440

caaaaaattg g                                                          1451

<210>  71

<211>  2015

<212>  DNA

<213>  Rattus norvegicus (Norway rat)

<400> 71
gtgagagttc ctttatacgt tacacattcc tcctctaaga cgagaccact ccaggctgaa 60

agtagtgaag attttaaaac ttactctgat gaaaactttc tttttaaaac agggcgccat 120

cgttgtattg gagaaaattt tgcctatgtt caaattaaga caatttggtc cactatgctt 180

cgtttatatg aatttgacct catcaatgga tattttccca gtgtgaatta tacaacaatg 240

attcataccc cagaaaaccc agtaatccgt tacaaacgaa gatcaaaatg aagaaaggaa 300

caaggagcca gtgtggagac gggactgcaa gctgcagctt ggcagagaat gaagctttga 360

cacagctttc atactgtact gttttttaag tgtgtggttc tgaaagccag tttgatttta 420

atgttttatt aactcggtga tttttgtcag acctaatggc atttgaaaca gttataatag 480

ttctgatagg atttcaggga agccaagttt atgttagaaa tcgtttaggg gagcctcggt 540

attcagagat gatacagaat atagcatcca ggtaactaac ttcagaagca cacgttgccg 600

tagggagatt ccggcttgga actagtttgg gaagttttaa gcctggtcag atgctacaga 660

ggcaatgggt cattggtgtg gttgggccac ttctgtgcgt aaagatgtga gagggtgaag 720

gataagtttt ctgcgaagct ctagatggtg tgagtgccct ttgtagtgtt aactgagagc 780

accactccag cgagatggca gcaatcttgg accttatctt gataacctta tttcctaaaa 840

ataataaata ctaaagagta cttatgttat tggttccaga aaaatccaaa atcaaatcct 900

tgtggaattt ttaattttaa ttaaaaaaaa aaaaaaacaa gtaccatgat tttaaaagtg 960

tatgattctg agcttagtga attctggcct tgagattgag gaatggggac atggtatcat 1020

tgcccgtgtt ctttggaggc tgtgctcagg agccaaccta acagattgtt accatgggcc 1080

taattctgac ctgcccataa tctgtattag gaatcaagag atctgttgct gggtgtggtg 1140

ctgcacacct gtaatactag tgctcgggct gaggcagaag gattgagagt ttgaggccaa 1200

cctagagcta catagcaaga cttaacaccc tccccaaaat aaaacctttt ttctctaaag 1260

tatgtgtact ggctggtctt aggtgacaac ctgacacacg ctagggtcat cagagaaaag 1320

ggaacctcag ttgaggaaat gctgtaagga tgcttagtgg tcaatgagag agggcccagc 1380

ccactgtggg tggttccacc cctaggctgg tcatcctggg tcctaagaaa gcaggctgac 1440

taagacacca ggagcaagac agtaagcagc atccttcatg gcctctgcat cagctcctgc 1500

cttaggttcc tgacccgctt gagttcccgt cctgactttc tttgataatg aacagtagta 1560

tggaagtgta agccaaataa cccaccccca ccctcccaac ttgctttttg ctcatggtgt 1620

tttgtagcaa tagaaaccct aactgttaca gctgtaagag gcttttgaag actcttcaaa 1680

tgaaggccca aatctctgct gttaaaggtt tcagattaaa attctctatg agaaaagttt 1740

tgctggtcta tattcatgga tttgaagctg tgcttcagta agtacagttc aagaggtctg 1800

ggaatggggt tggggattta gctcagtggt agagcgcttg cctaggaagc gcaaggccct 1860

gggttcggtc cccagctccg aaaaaaagaa caaaaaaaaa agaggtctgg gaattcagaa 1920

acttagatcc tatttgcctg aaatcggctc ccttcagtat tacctttagt tatttagata 1980

agtcactctc gtgatccgtt gacctgcagg tcgac 2015

<210> 72

<211> 5990

<212> DNA

<213> Rattus norvegicus (Norway rat)

<400> 72
gaggaagcaa agaccgggca aaacacatga aggaaaattt ggaacttctt ttataatatc 60

aacaagacat ttggggccaa ataaatcctc tccgttacac aaaaccaaaa atggcataca 120

attggcaaac agagccaaac cgtgctgaac cacaggaagg tggacatgat caccagcagt 180

gtcaccatgc agaccagcac ctttcttcca ggcaagtcag gttgggtttt gatcagcttg 240

tggaagagct cagtaacaaa actccactgc ctgaggatga aaaagaaggc acgtgttttg 300

taccagatac accaaacttg gattcaaaat ggcaatccat atatggaccc cacccaaggc 360

acttcaatga attcacttct cagagtcccc acttctccca gcttcctttt ggaaaagcat 420

cagccattgg ttttaatcct gctgtattac ctgcacatca gttcattcat gagggagcct 480

cctggagaaa tcccacaaga aaatatcatg gtggtgagga tcccaggttc agtgctctaa 540

ctccgtcatc cactggcttg gataaatgtc atcaacaagg acaatcaggg accgaacatt 600

gtaactatta tgtggaacct gaaaacaatg ttccccatca ttattcaccc tactcaatgg 660

actccatacc cgatagtgag gaaaaaggaa gtggagatgc ggatcttgta gaaccttctc 720

tggtgttctc taaagactcc tttctaccca gggcatcgga gaacatgtca gtggaaagca 780

cagagcccat tggttgcccc cttgaaatag ttgaagcacc ccaagggagt aacaagagcc 840

tcgcctcctt ttgcaacaat gtaacaaaaa taagaggact atatcatgca agtgacacta 900

attccaattc cggaaagatc tgggccatca ccacagccta tccatctcgg ctcttcgctg 960

acacccagtt cagagttaaa atttccactg ataactcggc acaacttctt cttcttaagc 1020

cacccgctaa ttatcttgtc aaagacctaa ttgccgaaat tctactttta tgtgcaaatg 1080

agcagctttc ccccaaagag tatcttctaa gtatatgcgg ttctgaggaa tttttacaga 1140

cggatcactg tctagggagc cacaaaatat ttcagaaaag taaatctgtc attcaactcc 1200

atctccagag aagcagggac actccaggaa aattatcccg gaagagggat gatgaccgca 1260

gtcgggtcca tctgaaccaa cttctagaat ttacacatat ttggaaaata tccagacaat 1320

gcctctccac agtaatgaaa agctacaacc tccatgtcga gcacctgttg aaaacccagg 1380

aagatgtgga ggagaaacct ctgtcatcca tgttttcctg tggccgacac cctcctcagc 1440

cacatgggaa tgacattatt gaagatgtta gaaacatatg cagtgttctg gggtgtattg 1500

aaaccaaaca agtttcagat gcagtaaaag aactaactct aattctgcag agaccatcac 1560

agaattttca tcagaattca gagacttcaa aaaaaggctt catagagaac gtgacatcgg 1620

aactgtcgag gtccctccat cagctggttg acgtgtactg cagtagcttt tgtacagatt 1680

tccggcctgc gcgcgcacct ggaggcgtct cccgcgacca cgctgggctc cactcccacc 1740
tgagcttcac ggtgtgttcc ctgcacaatg ttccagaaac ttgggcacac agctacaaag 1800
cattttcatt ttcctgctgg ctcacatatg ctgggaagaa gctgtgccaa gtgaaaagct 1860
gcagatccct gccagtcaca aagtcattct ctttttcggt gaactggaat gaaataatca 1920
attttcctct tgagataaag tcacttccaa gagaatccat gctcgttata aagctgtttg 1980
ggattgacag tgccacccac agcgcaaatc tgctggcctg gacctgcctt ccactatttc 2040
caaaagaaaa gtctccgctg gggtctaggc ttctcagcat gacactacag agtgagcctc 2100
ctatagaaat gatggctcca ggagtatggg atgggagcca gcctacccca ctgaccctgc 2160
agatagattt tccagctgcc acgtgggagt acgtgaaacc tgagactgaa gagaacagaa 2220
ctgaccacca agagcctcca agagagtgtt taaaacacat cgccagactc tcccaaaagc 2280
agcctccctt gctactttct gtggaaaaga ggagatattt gtggttttat cgtttctact 2340
gcaacaatga gaactcctct ctccctctgg tcttgggcag cgcccctggt tgggatgaag 2400
ggacagtttc ggaaatgcat gccgtcttga gaaggtggac attttcccat ccgttggaag 2460
ctcttggcct tttgacttcc aggtttccag accaagacat tcgtgaagtt gccgttcaac 2520
agttagacaa cttcttgacc gatgagctgc tggactgcct cccacagcta gttcaggctg 2580
tcaagtttga gtggagtctc gaaagtccct tggtggaact cctgcttcat cgatccttgc 2640
aaagcatccg agtggctcac cgcctgttct ggctgctgcg ggatgcacaa ggtgaagact 2700
actttaaaag ctggtaccag gagcttttgg ccgctctcca gttctgtgca ggagaagccc 2760
tgatcgaaga gctttccaaa gagcagaaac ttgtcaaact cctgggtgat attggagaaa 2820
aagtgaagtc ggctggcgat gctcagagaa aggatgtgct aaagaaggag attggcagtc 2880
tagaagaatt ctttaaagat ataaagactt gccatcttcc tctgaacccg gccctgtgcg 2940
taaaaggaat tgatcgggat gcatgttcat atttcacatc taatgccttg ccattgaaga 3000
tcactttcat caatgctaat ccaatgggca aaaatatcag tgttattttt aaggccggcg 3060
acgatcttcg gcaggatatg cttgttctgc agattattca agtgatggac aacgtttggc 3120
ttcaggaggg cctcgatatg caaatgatca tttatggatg tctagccaca ggaaaggctc 3180
aaggattcat agagatggtg cctgatgctg taacgcttgc caagatccat ctgcactctg 3240
ggctgatagg acccctgaaa gaaaacacca tcaagaagtg gttcagtcag cacaaccact 3300
taaaggaaga ttatgaaaag gccttgagga acttttttta ctcttgtgct ggctggtgtg 3360
tggtgacatt catcttggga gtctgtgacc gacataatga caatatcatg ctgacaaagt 3420
caggccacat gtttcatatt gactttggaa aattcttggg tcacgcacaa acatttggcg 3480
gtataaaaag ggaccgagcg cctttcattt ttacttcaga gatggagtac tttattacgg 3540
agggtgggaa aaacacacag cattttcaag acttcgtgga actctgctgc agagcctaca 3600
acattgtgag gaagcacagc caactgctcc tgagccttct agaaatgatg ctgcatgccg 3660
ggcttcctga gctgaggggg attgaagacc tgaaatacgt acacgacaat ctccggccac 3720

aagacacaga cctggaagcc acaagtcatt ttaccacgaa gataaagcag agtctggagt 3780
gcttcccagt taaactgaat aacctgatcc acacgcttgc acagatgcca gccttcagcc 3840
ttgccagacc tgcccctcag actcctcccc aggagtgctg cgtcctgaat aaaaccagga 3900
caattcagag agtcacaatt ttagggttca gcaagacaca cagcaacctg tacctgatcg 3960
aggtgacacg cagcgacaac aggaaaaacc tggccaaaaa gtccttcgag cagttttaca 4020
gacttcacag ccagattcag aagcagttcc ccttgttgac tctcccagag tttcctcact 4080
ggtggcatct acctttcaca gactcgcacc atgagagaat ccgagatctg agtcactacg 4140
tggaacaggt gctgcacgga tcttacgaag tcgcaaacag tgattgtgta ctcagttttt 4200
ttctctctga acatatacaa cagacccttg aagactctcc atttgtggac ccaggtgacc 4260
attctccaga caagagcccc caggtgcagt tgctgatgac ctatgaggac acaaagctca 4320
ccatcctagt gaaacacttg aaaaacatcc atctcccaga tggctcagcg cccagcgcac 4380
atgttgaaat ttatcttctg ccacatccca gtgaagttcg caggaagaaa acaaagtgcg 4440
ttccaaaatg cactgaccca acttacaatg aaattgtggt atatgatgac gtctcaggac 4500
ttcagggaca tgttttaatg ctcattgtga agagcaaaac tgtatttgtg ggagcggtta 4560
acattcagct ctgcagtgtt cccctcaatg aagaaaagtg gtacccatta gggaacagta 4620
tcatctgacc aatgccatga atgtatgcat tattgattaa gtacttgtgt gttttcagct 4680
tccatttccc ctatagcata cacaaggcat ctttcttgcg gaagatggct tggagcagtg 4740
gttctcactc agcgtcccta acactgcgac cctttaatac aattcctggt gattgtagtg 4800
acccaaacca caaaattatt ttagttgcta tttcacaact gtaattttga cacggttatg 4860
aattgcaatg tatatatctg atctacagga tacctactat tcgacccctg tgataaaagg 4920
gtcattggac aatcccaaag ggtcatgact catgggttga gaaccacagg cttagagtgg 4980
tcacagaaga agcagatcaa aatcagtctt ttgtagctct ttcttctcta ccttctcctt 5040
attttcttat catattttct ccttggaata ttcatcatgg aaaatcccat atgcaaagtc 5100
atgaaagaat gattcattta atatgcattt ttgaatcaaa ctaagttcat gtcttgcctt 5160
aattgcttgt tgaggtcaaa attatacttt tagggtgttt tctaaagcta ggagaagctc 5220
atgtaagggt taagaatatt tgcaatatat ttcaaaagtt aaatatgtgt acaagccaca 5280
tatctagtca tgattgaatt tattgagaga attggtgatc tccaaccatg tgctataatt 5340
tttctatcaa aaaaaaatcc ctaagatttt tctattgcat agattttttt tctttaagaa 5400
tttcatgcat gtatatagtg ctttcgttat tgtagcttct ctctttttta gttgtcccca 5460
cacccatcaa caactgttct tctctaaaac tcctgtattc ctgtggggag ttttattttt 5520
aagatgggca tcaaactata tatcccagct gacctagagc ttgctatgtt gaccaggtgg 5580
gccccaaatc acagtggtcc tcctgcctct gtttcccgac cgctaaggtt ccaggtcatt 5640
ggatcttgtc tgtaattttt aagttcagtt cttagaattt gatattgatc aatccagtgt 5700
cattgtgtct tccagcctcg gtcatgttca caccttaaat cttattaatc tccaaaccca 5760

aaatatccaa cttttaagtt caccatttaa aacgcctctt tgcgtgttaa atactctcac 5820

tgcacttgaa ccaacacctt gtgttcgcac ggaccagata gatgatctca cagtttgtca 5880

cctgtgtaac aggcaaaccc agaggacgcc tccaagataa tcaaactgga ggtttcaaaa 5940

ataaaacatc tgccataaaa aaaaaaaaaa aaaaaaaaaa aaaaaaaaaa 5990

<210> 73

<211> 5728

<212> DNA

<213> Rattus norvegicus (Norway rat)

<400> 73
gctctgggac agagtctcat actgatgaac ggagagcact caatggccac gcctggagag 60

tcctgcgcag gcctgagggt ctggaaccag acagaacagg agcctgtggc ctatcacttg 120

ctcaacctgt gcttcctgcg agccgccggg agctgggtgc cccccatgta cctctgggtc 180

cttggcccca tctacctcct ctacatccat cgccatggct gctgctacct ccggatgtcc 240

cgcctcttca aaatcaaaat ggtgctcggc tttgccctca tccttctcta caccttcaac 300

gcggccgtgc ctctctggag gatccaccgg ggcatgcccc aggccccaga gcttctcatt 360

caccctaccg tgtggctcac caccatgagc ttcgccacct tcctgatcca catggagaga 420

aagaaggggg tccgtgcatc tgggttgttg ttcgggtact ggctgctctg ctgcctcgtg 480

ccagccatcg acactgtcca gcaggcctcc gcagggagct tccgccagga gcccctccac 540

cacctggcca cctacctgtg cttgtccctg gtggtggcag agctggtgct gtcttgtctg 600

gtagaccagc cacccttctt ctcggaagac tccaagccat tgaatccatg tccagaggcc 660

gaggcctctt ttccctccaa ggccatgttc tggtgggcct ctggactgct atggaagggc 720

tacaggaaac tgctggggcc aaaagacctc tggtcacttg agagagaaaa ctcttcagaa 780

gaacttgttt cccagctgga aagagaatgg aggaggaact tcagtgagct gccggggcac 840

aaagggcaca gtggtatggg gacccccgag acagaggcct tcctgcagcc agagaggagc 900

cagcggggcc cgctgctcag ggctatctgg cgtgtgttcc ggtccacttt cctgctgggg 960

accctcagcc tggtcattag cgatgccttc aggtttgctg ttcccaagct cctcagtctg 1020

tttctggagt tcatgggcga cctcgagtcc tcggcttgga cgggctggct cctggctgtg 1080

ctgatgttct tgtcggcctg cctacagaca ctgtttgaac agcagtacat gtacagagtc 1140

aaggtcctgc agatgaggct gcgaacagcc atcactggcc tggtgtacag aaaggtcctg 1200

gtcctgtcca gtggttccag aaagtccagt gcagcagggg acgtggtcaa cctggtgtca 1260

gtggacgtac agcggctggt cgagagcatc ctccacctca acgggctgtg gctgctcttc 1320

ctgtggatca ttgtgtgctt tgtctacctg tggcagctcc ttgggccctc tgccctcaca 1380

gccgttgctg tcttcctgag ccttctcccc ctgaacttct tcattaccaa gaagaggagc 1440

ttccatcagg aagaacagat gaggcagaag gcctcccgag cacggctcac cagctccatg 1500
ctcagaactg tgagaaccat caagtcccac ggctgggagt gtgccttcct ggagcgactc 1560
ctgcatatcc ggggccagga gctaggtgcc ctgaagacct ccgccttcct cttctctgtg 1620
tctctcgtgt ccttccaagt gtctacattt ctggtggcgc tggttgtgtt tgctgtccac 1680
accctggtgg cagaggacaa cgccatggat gcggagaagg cgtttgtgac gctcacggtg 1740
ctcagcatcc ttaacaaagc ccaggccttc ctccccttct ctgtgcactg cctcgttcag 1800
gctcgggtgt cctttgaccg cctagctgct ttcctgtgcc tggaagaagt agaccccaat 1860
ggcatggtct tgagtccctc cagatgctcc tcgaaggatc gaatttctat acacaatggc 1920
accttcgctt ggtcccagga gagcccgccc tgcctgcacg ggatcaacct caccgtgccc 1980
cagggctgtc tgctggctgt tgtgggtcca gtgggggctg gaaagtcctc cctgctgtct 2040
gccctgcttg gggagctgtt gaaggtagaa gggtctgtga gcattgaggg ttccgtggcc 2100
tacgtgcctc aggaggcttg ggtccagaat acctctgtgg tggagaatgt gtgcttcagg 2160
caggagctgg atctgccatg gttgcaggaa gttctagaag cctgtgcctt ggggtctgat 2220
gtggccagct tccctgcagg agttcacacc ccagtagggg agcagggcat gaatctttct 2280
gggggccaga agcagcggct gagcttggct cgggctgtgt acagaagggc tgctgtgtac 2340
ctgatggatg accccctagc agccctggat gcgcatgtca gccaggaagt cttcaaacag 2400
gtcattggcc ccagtggact tctccaaggt acgactcgga tccttgtaac acacacgctg 2460
catgtcctgc cccaagctga ccagatcctg gtgctggcca atgggaccat cgcagagatg 2520
ggctcctacc aagaccttct gcataggaac ggagccctgg tgggtcttct ggatggagcc 2580
agacagcctg caggcgaagg agaaggagaa gcacatgctg cagccaccag tgatgacctt 2640
ggaggctttt ctggaggtgg gacgcccacg cgcagaccag agaggcccag acccagtgac 2700
gcagcccctg tgaagggcag tacttcagag gcacagatgg agccttctct ggatgacgtt 2760
gaggtcactg gactgacagc aggagaggac agtgtgcagt atggccgggt gaagagcgcc 2820
acatacctga gctacctgcg ggcggtgggc acaccgctct gcacctacac cctgttcctc 2880
ttcctctgcc agcaagtggc gtccttctgc caaggctact ggctgagcct ctgggccgac 2940
gacccggtcg tggatgggaa gcagatgcat tcagccctgc gtggctccat ctttggactc 3000
cttggctgtc tgcaagccat cggactgttt gcctccatgg ctgcggtgtt cctgggtgga 3060
gcccgagctt catgcctgct tttccggagc ctcctctggg acgtggctcg ctctcccatt 3120
ggcttctttg agcgcacacc agtcgggaac ctgctgaacc gtttttccaa ggagacggac 3180
atagtggatg tggacatccc agacaagatg aggaccctgc tgacctatgc ctttggactc 3240
ctggaggttg gcctggcagt gtcgatggcc acaccactgg ctattgtggc catcctacct 3300
cttatgctcc tttatgctgg gtttcagagc ctctacgtgg ccacatgttg ccagctgaga 3360
cgcctggagt cggccagtta ctcctcagtg tgttcccatc tggctgagac cttccagggc 3420
agtcaggtgg tcagggcctt ccaggcccag gggcccttca cagctcagca cgatgccctc 3480

atggatgaga accagaggat cagtttcccg aggctggtgg ctgacaggtg gctggctgcc 3540

aacctggagc tcctggggaa tggcctggtg tttgtggccg ctacatgtgc tgtgctgagc 3600

aaggctcacc tgagtgctgg cctcgcgggc ttctcggttt ctgctgccct ccaggtaaca 3660

cagactctgc agtgggtggt ccgcagctgg acagatctgg agaacagcat ggtggccgtg 3720

gagcgagtac aggactacgt tcacaccccc aaggaggctc cctggaggct gccctcctct 3780

gcagcccagc ctctctggcc ctgtggggga cagattgagt tccgagactt tgggctcaga 3840

caccgaccag agctgcccat ggctgtgcag ggtgtgtccc tgaagatcca tgcaggggag 3900

aaggtgggca tcgtgggcag gacaggggcc gggaagtcct ccctgacttg gggcctgctg 3960

cggcttcagg aggccactga gggtggtatt tggatcgatg gggtccccat caccgacatg 4020

gggctgcaca cactgcggtc cagaatcacc atcatccctc aggaccctgt cctgttcccg 4080

ggctcgctgc ggatgaacct ggacctgctt caggagaaca cagatgaggg catctgggca 4140

gcgctggaga cggtgcagct caaggccttc gtgaccagcc tgcctggcca gctgcagtat 4200

gagtgctcag gccagggaga tgacctgagt gtgggtcaga agcagctcct gtgtctggca 4260

cgtgcccttc tccggaaaac ccagatcctc atcctggatg aagccactgc ctccgtggac 4320

ccagggacgg agatacagat gcaggcggcc ctcgagcgct ggtttgcaca gtgtacagtg 4380

ctgctcattg ctcaccgcct gcgctccgtg atgaactgcg ccagggttct agtcatggat 4440

gaggggcagg tggcagagag tggcagtcca gcacagctgc tggcccagaa aggcctgttt 4500

tacaggctag cccaggagtc gggcctagcc tgagtcagga ctcttcccaa acctcctgga 4560

gccagccaca gagcctgcag tagctggaga tgccagagac tcaggggcca catgatgccc 4620

aatctaaact cctttttggg aggaagatag cagagagagt gacagagtat tggaatacca 4680

gacccagaag aacccagcat gcccaggttg gcttgagcaa ggccacaccc accccaggcc 4740

aaaaagaaca gtgactctca gcccaagctg tctacttcaa ggccataccc accccaggcc 4800

attcaggttg gatgccctgg accggggtga tggcgtgcac atatccccta actccttatt 4860

ttgaggtcat tgtagagttc actcacagtt ttaagaagcc acatggagag aagccgcaaa 4920

ccctctgccc tgtttattcc gggggtgaca ccttgtccaa ccctaggaca agatgaagca 4980

tcacactgac tccgactgac ttgtctttac ctctgctgcg tgtgcatcag tgtttggact 5040

ccgtgctttg tgctctcatt ggtttttgag acaggatttc acatagccca ggctggccct 5100

gaactcactt tgttgctgag gatggccttg aacatctgat gctcctgcct tccctcccaa 5160

gtgctgggat tatggcctgt gtcaccacgc cctgtgtggg ggtctcaaac aaggctttgt 5220

gtgtgcttga caggcactca ctctaaaaac tgtgttacag ccccggctct ggattcggtt 5280

ctactcctgt ttaaaattgt agtggtgaag ggtctcttgc tcaaactggc ctcaaactcg 5340

agatgctcct gtctcggtct ccagagtgct ggaatgacag acgtgtgcca ctacacctgc 5400

cttgactcac cacagctaag tagtgacatc cccatgggcc agggctggtg agtcccgtgc 5460

gtgacagtgt gctgagcagt acccttcgct tctgctcaga gatgcccttc taaagctgtg 5520

gcaaagagat ttccacacac tgccgtgccc ccccaggact gcatcatgaa ttgatccgcc 5580

ctaatagcac ccatgactcc ctgagcagtg atatgttggg ttcaggagag gattcctgct 5640

tgcttcttgg acagggcttg ctcttccctc gaccctgagg cttctctgat tggctaccct 5700

taataaagga tttacgggat ttcctttc 5728

<210> 74

<211> 555

<212> DNA

<213> Rattus norvegicus (Norway rat)

<400> 74
atgactgttg cctcctgtgg ccatggggaa gaagctggtg atggcccaga agcgcggaga 60

gactcgagcc ctctgcctgg gggtggcgat ggtcgtgtgt gccgccatca cttactacat 120

cctgggtacc actgtgctgc ccctctacca gaaaagtgtg tggacccagg aatccacctg 180

tcacttggtt gaaaccaaca tcaaggacca ggaagagctg gagggcagga aggtgcccca 240

gtacccatgc ctttgggtca atgtatcagc tgtgggcaga tgggccatgc tgtatcacac 300

agaagacact cgggatcaaa accaacagtg ctcctatatc cccaggaacc tggacaacta 360

ccagacagcc ttggttgatg tgaagaagct cagaagtaga ccaatctctt ctgcacagca 420

gcatggagtc accaggaacg gcaggggtcc cgggcaggca taattctctt cctcttgtcc 480

tccatttaat ttgttcaacc tctttggaca atcatttctt gctcaagaat gtttaacagc 540

tctctccggt tgact 555

<210> 75

<211> 814

<212> DNA

<213> Rattus norvegicus (Norway rat)

<400> 75
atgaactctg gcgtgaacct gcagaggtcg ctgtcttctc caccgagcag caaaagaccc 60

aaaactatat ccatagatga aaatatggag ccaagtccta caggagactt ttacccctct 120

ccaaattcac cagctgctgg aagtcgaaca tggcatgagc gagatcaaga tatgtcttct 180

ccaactacaa tgaagaagcc tgagaagcca ctgtttagct ctacatctcc acaggattct 240

tccccaagat tgagcacttt tccccagcac caccatcccg gaatacctgg agtcgcgcac 300

agtgtcatct caactcgaac tccacctcca ccctcaccgt tgccatttcc aacgcaagct 360

atccttcctc cagcaccatc aagttacttt tctcatccaa caatcagata tcctcctcac 420

ctgaatcctc aggatactct gaagaactat gtaccttctt atgacccgtc cagtcctcaa 480

acgagccagc ctaacagcag tggtcaagta gtagggaaag tgcctggcca tttcacgcct 540

gtcttggcac cctctcccca tcccagtgca gtgcgacctg tgaccctgac catgacagat 600

actaaaccca tcactacatc cactgaagcc tacacagcct caggcacatc tcaagccaat 660

cgatatgtgg gactaagccc aagagaccca tccttcctgc atcagcaaca gctgaggatt 720

tgtgactgga ccatgaatca aaacggcagg catttatacc ccagtaccag tgaggataca 780

ttgggaatta cttggcaaag tcctggtacc tggg 814

<210> 76

<211> 2532

<212> DNA

<213> Rattus norvegicus (Norway rat)

<400> 76
cccattttgc gtcgtctggg ttcgggtggc ggcccggctc caggcggacg cactaatcat 60

ggggtcgtca aagaagcacc gtggagagaa ggaggcagcc gggactacgg ctgcggccgg 120

taccgggggc actaccgagc agccgccacg gcatcgagag cacaagaaac acaagcatcg 180

gagcagcggc ggcggcagca gcggcggcga acgacggaag cggagccgcg agcgtggggc 240

cgagcgcggg agcgggaggc gcggggccga agccgaggcc cgcagcggcg cgcatggtcg 300

tgaacgaagc caagcggaac cctcggagcg gcgcgtgaag cgggagaaac gcgatgaagg 360

ctatgaggcc gctgccagct ccaaagccag ctctggagat gcctcatcac tcagcatcga 420

ggagaccaat aaactccgag caaagttggg gttgaagccc ttggaagtca atgctgttaa 480

gaaagaggcg ggcaccaagg aggagcccgt ggcagccgat gttatcaacc ccatggcctt 540

gcgacagcgt gaagaactac gggagaagct ggcagctgcc aaggaaaagc gcttgctgaa 600

ccaaaaattg gggaaaataa agacactggg ggaggatgac ccctggcttg atgacactgc 660

agcctggata gagaggagcc ggcagctaca gaaagagaag gacttggcgg agaagcgggc 720

caagctgcta gaagagatgg accaagagtt tggtgtgagc actttagtgg aggaggagtt 780

tgagcagagg cgacaggacc tgtacagcgc ccgggacttg caaggcctca ccgtggaaca 840

tgcaatcgat tcctttcgag aaggggagac tgtggtcctc actctcaagg acaaaggggt 900

tctgcaggag ggggaggatg tgctggtgaa tgtgaacatg gtggacaagg agcgagcaga 960

caagaacgtg gaacttcgga agaagaagcc tgactacctg ccctatgtgg aggacgagag 1020

tgtggatgac ttggcacagc aaaaacctcg ctctatcctg gccaaatatg atgaggaact 1080

ggagggcgag cggccacatt ccttccgttt agagcagggt ggcatggccg atggcctgag 1140

agaacgagag ctcgaagaaa tccggaccaa gctacggctg caggctcagt ctctgaacac 1200

tgtagggccc cggcttgcct ctgagtacct cagtccggag gagatggtga ctttcaaaaa 1260

gaccaaacgg agagtgaaga aaatccgaaa gaaggagaag gaggtaataa tgcgggcaga 1320

tgacttgctg ccccttggcg aagaccagac tcaggacggg gactttggat ccaggcttcg 1380

gggccggggt cggcgccgag ttcctgaagt ggaggaggag gcccttgagg atgaagagaa 1440

ggaccctgtg gcccagccgc caccatcaga tgacactcgc gtagagaaca tggacatcag 1500

tgatgaggag gatggggggg ctcttccctc ggggccccca gagctggagg aggacgaagc 1560

ggagctggag ctgcagaagc agctggagaa ggggcgccgg ctgcggcagc tgcagcaact 1620

gcagcagctg cgggacagcg gtgagaaggt gctggagatt gtgaagaaac tggagtctcg 1680

ccaacgtggc tgggaagagg aagaggatcc tgagcggaag gggaccatcg tgttcaatgc 1740

cacttctgag ttctgtcgca ctctggggga aatccccact tacggattag ctggcaaccg 1800

agaggagcag gaggagctca tggactttga aagggatgaa gagcgctcgg ccaatggtgg 1860

ctctgaatca gatggcgaag agaacattgg ctggagcacc gtcaacctgg atgaggagaa 1920

gcaacatcag gatttctccg cttcctctac caccatcctg gatgaagagc ccattgtgaa 1980

cagagggctg gcggctgccc tgctcctgtg tcagaacaaa ggactgttgg agaccacggt 2040

acaaaaggtg gcccgagtaa aggctcccaa taagtcactg ccttcagcag tgtactgcat 2100

cgaggataag atggccattg atgacaagta cagccgacgt gaggagtaca ggggcttcac 2160

ccaggacttc aaggagaagg atggctacaa gcccgatgtt aagattgaat atgtggatga 2220

gacgggcagg aaactgactc ccaaggaggc attccggcag ctgtcccacc gcttccatgg 2280

gaagggctca gggaagatga agactgagcg gcggatgaag aagctggatg aggaggcgct 2340

cctaaagaaa atgagttcca gtgacacgcc cctgggaacc gtggcactgc tccaggagaa 2400

gcagaaggct caaaagacac cgtacatcgt gctcagtggc agtggcaaga gcatgaatgc 2460

gaacaccatc accaaatgaa gcacctccct gtcccagatg taatgttaaa taaagttccc 2520

gccttatttt ta 2532

<210> 77

<211> 1940

<212> DNA

<213> Rattus norvegicus (Norway rat)

<400> 77
cacaaccacc aaatatatcc acacgttgac gtgatttctt gcccttactc acactaagcc 60

cgcgtgtcga tccatctcta tggatcccga acctactgaa cactccaccg gcggcggctc 120

ggttccccgc cagccgccca gcgcgcagac ggggcttgat gtccaggttg tcagcgcagc 180

tggcgactca ggtaccatga gccaggacac cgaagtggac atgaaagatg tggagctgaa 240

cgagctggaa ccggagaagc agcctatgaa tgcagcggac ggggcggcag ccggggagaa 300

gaacggtctg gtgaagatta aggtggccga agacgaggcg gaagccgggg tcaagttcac 360

aggcttatcc aaggaggagc tattgaaggt agctggcagc ccgggctggg tgcgcacccg 420

ctgggcgctg ctgctgctct tctggctcgg ttggctgggt atgctggcgg gcgccgtggt 480

tatcatcgtt cgggcgccac gctgccgtga gctgccggta cagagatggt ggcacaaggg 540

cgccctctac cgcatcggcg accttcaggc cttcgtaggc ccggaagcga gaggcatagc 600

tggtctgaag aaccatctgg agtacttgag caccctgaag gtgaagggcc tagttttggg 660

cccaattcac aagaaccaga aggatgaagt caatgaaacc gacttgaaac agattgatcc 720

cgatttaggc tcccaggaag attttaaaga ccttctacaa agtgccaaga aaaagagcat 780

tcacatcatt ttggacctca ctcccaacta taagggccag aatgcatggt tcctccctcc 840

tcaggctgac attgtagcca ccaaaatgaa ggaggctctg agttcttggt tgcaggacgg 900

tgtggatggg ttccaagttc gggatgtggg aaagctggcg aatgcatcct tgtacttggc 960

tgagtggcag aatatcacca agaacttcag tgaggacagg cttttgattg cagggaccgc 1020

gtcctctgac ctgcaacaaa ttgtcaacat acttgaatcc accagcgatc tgctgctgac 1080

cagctcatac ctgtcacagc ccgttttcac tggggagcat gcagaactcc tagtgattaa 1140

gtatttgaat gccactggca gccgctggtg cagctggagt gtgtcgcagg caggactcct 1200

gacatccttt ataccggctc agtttctccg actctaccag ctgctgctct tcactctgcc 1260

aggaactcct gttttcagct atggggatga gcttggcctt caggcagttg cccttcctgg 1320

acagcctatg gaggctccat tcatgctgtg gaatgagtct agcaactccc aaacctcaag 1380

tcctgtaagc ctcaacatga cagtgaaggg ccaaaatgaa gaccccggct ccctcctcac 1440

ccagttccgg cgactgagtg acctccgtgg taaggagcgc tctctgttac acggtgactt 1500

tgatgcactg tcttcctcat ctggcctctt ctcctacgtc cgccactggg accagaatga 1560

gcgttacctg gtggtgctca acttccagga tgtgggcctg tcagccaggg taggagcctc 1620

caacctccct gctggcataa gcctgccagc cagtgctaac cttttgctta gtactgacag 1680

cacccggcta agccgtgagg agggcacctc cctgagcctg gaaaacctga gcctgaatcc 1740

ttatgagggc ttgttgttac agttcccttt tgtggcctga tccctctaca cagaacctgc 1800

cacccttctt tcctctctca ggcctttgga attctggtct ttctctcctt attttgtttt 1860

tgtttttaaa cttttgcaga ttacatatga attcttacac tgggtgtttt tgtcttcaaa 1920

ataaaaaaaa tcacccctgc 1940

<210> 78

<211> 3061

<212> DNA

<213> Rattus norvegicus (Norway rat)

<400> 78
tgtgaccgcg gccaagcccg ctcgccttct gcggcgagta gggcctcgcg gcgtcatcgg 60

cggcgaggag ccgctgcggc ctggccgacc atgtcggacg cggctgagga gcagcccatg 120

gagacgacgg gcgccaccga gaacggacac gaggccgccc ccgaaggcga ggccccggtc 180

gagcctagcc ctggcagcgg ccccgcgact ccggcggggt ctgtgggcgg gaccacggcc 240

gccccgagcg ggaaccagaa cggcgccgaa ggcgaccaga tcaacgccag caagaacgag 300

gaggacgcgg gaaaaatgtt cgttggtggt ctgagctggg ataccagcaa aaaagatcta 360

aaggattatt ttactaagtt tggagaggtc gttgactgta caataaaaat ggatccgaac 420

actggtcgat caagagggtt tgggtttatt ctcttcaaag attcttctag tgtggagaag 480

gtcttagatc agaaggagca cagactggat ggtcgtgtca ttgaccctaa aaaggctatg 540

gctatgaaga aggaccctgt gaagaaaatc tttgtgggag gtctaaaccc tgaagccaca 600

gaggaaaaga tcagagaata ctttggccag tttggggaga ttgaggccat tgaacttcca 660

atagatccca agttgaacaa aagacggggt tttgttttta ttacatttaa agaggaagac 720

cctgtgaaga aagttctgga gaaaaaattc catactgtca gtggtagcaa gtgtgaaatc 780

aaggttgccc agcccaaaga ggtgtaccag caacagcagt atggctctgg gggccgagga 840

aatcgcaatc gagggaaccg aggcagtggt ggaggtcaga gtcagagttg gaatcagggc 900

tacggcaact actggaacca gggctacggc taccagcagg gctacgggcc gggctatggc 960

ggctacgact actcgcccta tggctattac ggctacggcc ccggctacga ctacagtcag 1020

ggtagtacaa attacggaaa gagccagcga cgtggtggtc atcagaataa ctacaagcca 1080

tactgagagg cagcaggagc ggccgagtga cgacgcacac gctttgtttg gacgcggagt 1140

gaacacaatt atgtaccaaa tttaacttgg caaactttta tggcctgtcc catgtgcatc 1200

ttatttaaaa tttcccccgg aaatcactct cctgtttact attccagagc tctagttgtt 1260

gtaggcagcg tgtggcgtct cagaggtcag agcggcatta gggggctgat tttattacca 1320

ggttaccctg ttctagattg gagggtctgc ttcctgctgc cgctctgcag cctggacctg 1380

tggaccctgg ttgtaaagag taaactgtat cttaggaaac cagtgtcacc tttttcacct 1440

tttaatttta tattatttgt gtcatacatt tccagtaatg gaagtgttaa ttttactgta 1500

ctttttggta ccttttggga atctaatgta ttgtaaggta ttttacacgt gtcctgattt 1560

tgccacgacc tggatattga agctatccaa gcttttgaaa taaaaatccc tgagagtctg 1620

gtccactaac tccagggact gtgggacaga ggaacaaaca agaaagaact cgaggcctcg 1680

ctgagtggca cgcttgctga gtgctgccac atcccgcaag ctgagcaggt atcccaactc 1740

tgttcctgcc cggtagacca cccgaggtgt gagtgtggtc ttgtcttcca gattcgtagg 1800

acagaagctc caggaggagg acccgcccaa catggcatcg gagagcggga agctatgggg 1860

tggccgattt gcaggctcgg tcgaccccac catggacaag ttcaactcat ctatcgccta 1920

tgaccggcat ctgtggaatg tggacctgca gggaagcaag gcctacagca ggggcctgga 1980

gaaggcaggg cttcttcacc aaagctgaga tgcagcagat actgcaaggc ctggacaagg 2040

tggctgaaga gtgggcccaa ggcatcttca aattgtaccc taatgatgaa gacatccacc 2100

ttcctcaagg tgctcattga agccatggta gaccgggcag aggcggagtg tgaagtcctc 2160

ttccctgggt acacacactt gcagagagct cagcccatcc gctggagcca ctggatcctg 2220

agtcacgccg ttgcgctgac acgagattta gagagactga aggaggtgca gaagcggatc 2280

aatgtcctgc cactgggcag tggggccatt gcaggcaacc ctctaggtgt ggaccgggag 2340

ttcctctgtg cagaactgaa ctttggagcc attacgctca acagtatgga tgccaccagc 2400

gagagagact tcgtggctga gttcctgttt tgggcttctc tgtgcatgac ccatctcagc 2460

aggatggcag aagacctgat tctctacggt accaaggaat tcaactttgt gcagctctcc 2520

gatgcctaca gcaccggaag cagcttgatg ccccagaaga aaaacccaga cagcttggag 2580

ctgatccgga gcaaggcggg ccgagtgttt gaagtgtctg ataccatgac agctgtcctc 2640

caagtagcca ctggagtcat ctctacactg cagattcatc gtgagaacat ggcacaggca 2700

ctcagccctg acatgctggc taccgacctt gcctactacc tggtccgcaa agggatgcca 2760

ttccgccagc ccacgaggcc tcagggaaag ctgtggtcgt ggcagagatg aaaggggtgg 2820

ctctcaacca gctgtcactt caggagatgc agaccgtcag tcccctgttc tcgagtgacg 2880

tgaatctcgt gtgggactac agccacagcg tggagcagta cacagccttg ggtggcacag 2940

cacagtccag tgttgagtgg cagatcagcc aggtgcgggc cctgctgcag atgtagcagc 3000

cctagattcc acccagtcaa agctgcgccc caataaagtg gagccctgga gaggccgcca 3060

t 3061

<210> 79

<211> 1334

<212> DNA

<213> Rattus norvegicus (Norway rat)

<400> 79
gagcgccagg ccaatcggag gtgctgttgt ttgtcagtcc cggagccggt ccgcaaagga 60

aaggtggcaa gatggtgttg gagagcacta tggtttgtgt ggacaacagt gagtacatgc 120

ggaatggaga cttccttccc acccggctgc aggcccagca ggatgctgtc aacattgtat 180

gtcactcaaa gacccgaagc aaccctgaga ataacgtggg cttgatcacg ctggccaatg 240

actgtgaggt gctgaccaca ctcacccctg acactggccg aatcctctcc aagctccaca 300

ctgtccagcc caaaggcaag atcaccttct gcactggcat ccgcgtggcc catctggctc 360

tgaagcaccg gcagggcaag aatcacaaga tgcgcatcat cgccttcgtg ggtagccctg 420

tggaggacaa cgagaaggat ctggtgaaac tagctaaacg ccttaagaaa gaaaaagtga 480

atgttgacat catcaatttt ggggaagagg aggtgaacac agagaagctg acagcctttg 540

tgaacacatt aaacggcaag gacggaactg gatcacatct agtgacagtg cctcctgggc 600

ccagcctggc cgatgctctc atcagttctc ctattctagc tggggaaggc ggcgccatgc 660

tgggtcttgg tgccagtgac tttgagtttg gagtagatcc cagtgctgat cctgaattgg 720

ccctggccct tcgtgtctct atggaagagc agcggcagcg gcaggaggag gaggcacgac 780

gggctgctgc agcctctgca gctgaggctg gaattgccac acctgggact gaaggtgaac 840

gagactcaga tgatgcccta ctgaagatga ccatcagcca gcaggagttt ggccgtgcgg 900

ggcttcctga cctaagcagc atgacagagg aagagcagat tgcctatgcc atgcagatgt 960

ccctgcaggg agcagagttt ggccaagcag aatcagggga catcgatgcc agctcagcca 1020

tggacacatc tgagccagtc aaggaggagg atgactatga cgtgatgcag gacccagagt 1080

tccttcagag cgtcctagag aaccttcccg gtgtggatcc caacaatgaa gccattcgaa 1140

atgcaatggg ctctctggcc tcccaggcca ccaaggatgg caagaaggac aagaaagagg 1200

aagagaagaa gtgaggccgg agggaggggt agccgagtct gccctgggga ctgtaaggat 1260

ggggttggat aaagacttag atgtgctctg cggccttgac aacggaataa agcttggagt 1320

tttccctttg cttc 1334

<210> 80

<211> 853

<212> DNA

<213> Rattus norvegicus (Norway rat)

<400> 80
ggccacgcgt cgactagtac gggggggggg ggggaatgag gcggaccatc tgaccccgct 60

cgctcgctcc acgcgctggc acgggagcga cagccaactg cagagcgaat tcctctcacg 120

ccagtcactc agagcaatga aactgtaaca gttgttttcc caaatactgt acgatggtac 180

gactgaccag caacttcctt ttgttttgta ttatatatat ctttaaaata tcttgtcagt 240

tttactatgt atgttcatcc attcaccaaa gccctcaaaa agaagtttct ccacagaaca 300

ggcacctcag gctctgtccc aaggaatgtg ttttaatttt tgcccggata aaagaaaata 360

agctttgcac acactctcga ttctttattt gcaggcaaac ttcactcttt attatctgaa 420

acttttccat tctcagcctc ttagaggcac agatggctca agtttcagtg gcaaaaaagt 480

cttttttttt ttttctttct gtaaccaaac tagagcaaat ttggaattca gtctgggact 540

aaaacgtcac agcagaaaaa aaatgaaaaa aaaataattt gctttttctt tctttcattt 600

agcagcataa ataagtttgg ccactgggag tacagtacag gggttgggac aacgatcccg 660

tatttgaaga cctacttcta gcaccagcat caagaactaa atccacctca ggactcacag 720

aacccaggac aacttgccat ctttgagcaa catatgcatt gaagagtgta tatagaagca 780

acagtaaata gattaacaga ggctaatact gtgattgatt gacattggca atggttggca 840

aaaaaaaaaa aaa 853

<210> 81

<211>  1356

<212>  DNA

<213>  Rattus norvegicus (Norway rat)

<400>  81
ggagactgtc ttttccaacc cgacatggat gtgctcccaa tgtgtagcat cttccaggaa      60

ctacagattg tgcacgaaac gggctacttc tcggctctgc cgtccctgga ggaatattgg     120

caacagacct gcctggagtt ggaacgctat cttcagagtg agccctgcta cgtgtcagcc     180

tctgagataa aatttgacaa ccaggaagac ctgtggacca aaatcattct agcacgggag     240

aggaaggagg aatcagaact gaagatttct tctagtcccc cagaggactc tctgatcagc     300

tccggcttta attataactt agagaccaat agcctgaact ctgatgtcag cagcgaatct     360

tcagacagtt cggaggaact ttcgcccacg accaagttta cctctgaccc cattggtgaa     420

gtcttagtca attcaggaaa tctgagttcc tcggtcattt ccacacctcc ttcttctccc     480

gaagtaaata gggaatcttc tcaactatgg ggctgtgggc caggagacct gccctcacct     540

gggaaggttc gaagtgggac ctcggggaag tctggcgaca agggtagtgg cgacgcctcc     600

ccagatggca gaagaagggt acatcggtgc cattttaacg gctgcaggaa agtttacact     660

aaaagctccc acttgaaagc acatcagcgc actcacacag gagaaaagcc ttacagatgc     720

tcttgggaag gttgtgagtg gcgttttgca agaagtgatg agttgaccag acacttccga     780

aagcatactg gtgccaagcc ctttaaatgc tctcactgtg acaggtgttt ctccaggtct     840

gaccacctgg ccctgcacat gaagaggcat ctctgaggga gcagaggatg aatcctgtag     900

gctaaaagag gcttccaggc taagaggcgg ccatggaagg agggatacct gtaccagcca     960

aagcatgcca ttgcttccta cccagttacc tccagaggcc tctctttgga aggtcttttg    1020

agggctacaa aagtcatgtc agaagcggca tagcacccac ggtgcatggt gtttgggtga    1080

ccccggactc accactggtt tctaaccttc tgagaggctc taagcttttc gccgtgagca    1140

tgcgcactga gaatgttaat gggtgggaat gactgactgt atgttgagga tctattactg    1200

actgtatggc gaggcagact ttttttttcc ccccttgtgg tagcaaatac ctgcaagaga    1260

cagaaaaaaa aagcagtttg aatgttttgt gtgtgaggag tattccaagg gatgagttga    1320

ccaccaatca tttcctgaag ggtgtctgca ccttag                              1356

<210>  82

<211>  2436

<212>  DNA

<213>  Rattus norvegicus (Norway rat)

<400>  82
agctttccgg ggcagcgagt ggcggggccg ggtgctgagc gagcggggcg tggagagcgt      60

cgcgcggccc ctcgcgccgg gctccgtttg caggccacag cccccgcgag gtggcccgcc 120

ggccctgggc cgcccgtcct ctggcagctg tggtgagcgc agcgtcgggc cggcatgaag 180

gtcacgtcgc tcgacgggcg ccggctgcgc aagatgctcc gcaaggaggc ggaggcgcgc 240

tgcgtggtgc tcgattgccg gccctacctg gccttcgccg cgtcgagcgt gcgcggctcg 300

ctcaacgtca acctcaactc cgtggtgctg cggcgggccc ggggcggcgc ggtgtctgcg 360

cgctacgtgc tggccgacga ggcagcccgc gctcggctgc tgcaggaggg cggcggcggc 420

gtggcggcgg tggtcgtgct ggaccagggc agccgccact ggcagaagct gcgggaggag 480

agtgccgcgc gcgtcgtcct cacctcgctg ctggcctgtc tgtccgccgg accgcgggtc 540

tacttcctta aaggtgggta cgagaccttc tactcacagt atcctgagtg ctgtgtggat 600

gcgaagccca tttcacaaga gaagctcgaa ggtgaaagag gcctcctcag ccagtgcgga 660

aagcccattc tcagcgtcgc ctacagacca gcttatgacc agggtggccc agttgaaatc 720

cttcccttcc tctaccttgg aagtgcctac catgcatcca agtgcgagtt cctcgccaac 780

ctgcacatca cagccctgct gaatgtttcc cgccggacct ctgaggcctg cacaacccac 840

ctacactaca agtggatccc tgtggaggac agccacaccg ccgacattag ctcccacttt 900

caagaagcaa tagattttat tgactgtgtc agggaagagg gaggcaaggt cctggttcac 960

tgtgaagccg gggtctcccg gtcgcccacc atctgcatgg cttacctcat gaagaccaag 1020

cagttccgcc tgaaggaggc cttcgagtat atcaagcaga ggaggagcgt ggtctctccc 1080

aactttggct tcatgggaca gctcctgcag tatgagtctg agatcctgcc ctccacaccc 1140

accccccaac ctccctcctg ccaaggggag gcagccagct ccacctttat aggccactta 1200

cagacactga gccctgatat gcagggtgcc tactgcacat tccctacctc agtgctggca 1260

ccggtgccca cccacgccac cgtcgcagag ctccacagga gccccgtggc cacagccaca 1320

tcctgctgag accggtcggc taccagcgca tccccaagag caactgtgac ctttggattt 1380

tttaaacttg tggacatttc atacccgtgc aatactgaag acctctctct gtcccgctgc 1440

cccggtgaga tggtgagggg tcagcaggct tgcagatgca cttcaggcta acccggagga 1500

tggtttctcg cgattgtagg aaggccaagc catgcccccc tagcacagcg gcgtgctaac 1560

tactgtactt ccagaagccc cgcccactca ggaccgcctc atccttgcac ctcagaagtc 1620

ccggcttctc atttcaagtg taaggcaata cacagtcgca gcaaagtagg agcaagctgt 1680

gctggaccag gaggggagga gtccgcccgt ctgggaggaa gcacaagttt cactgttaat 1740

ttgaatttcg gccaactttg tctgtctctg tcctctgtca cttcagggaa gagagctggt 1800

caccgctcag tcagaaaagt taaccccgct ggatttgtca agacaaaagg acctgcccgt 1860

ctgaacccag tgtttctgag gttctgtcta ggatcccatg gaagctgttg gtgtaaggag 1920

aagctcctga ctcattggag tttcttgctc accgagggct ccttggtgac cttggacttt 1980

ggcatggttt ttacaaatac ttgaacctgt cccattgtat ctctccctaa agcacctctg 2040

gtgtcattca gaaagttgtc agaccctaga ccaaaaacca cccctttgag ggggtagcag 2100

gaactgcctg cgttctgggt cagtggtggt gactgacata ctttttcagt ttagtgtcct 2160

gtgtgctttt tttgtcatcc attgtgacaa tgtttccctc cctaccctgg ggagtcgttt 2220

tcaaactact gattctgggg tctgcatcgt ttgcaatgtg gtactactat gtccttcgta 2280

gattgttttt ccaagggggg aaaggcaata agtcaccccc aaacccatgt gaatgtgaag 2340

aaaagcagtg ttgatgtttt ttttatatat atatatatac atgtagtaca aaattaaaaa 2400

aatgtcaaaa aataaaaata aaaagtgcta agtgaa 2436

<210> 83

<211> 2150

<212> DNA

<213> Rattus norvegicus (Norway rat)

<400> 83
actcgcgctc cccgagtgcc acatcactgc gccctcccgc gcatccgcgc atctgtgccc 60

accatgccta caacgccgct gctgctggct gcgctagctg ctttggccgc gctggccgtt 120

gctgcgtacc ccagctgctc gcctggacct gacccctccg gtaaatgtca gagactggcc 180

tccacacaca gtgccacctg tgtggacctg catctcagga cctgtgcgga tgcggcctat 240

aaccatacat cgttccccac acccctggag caccgttcgt gggaggcagt ggaggccagc 300

cctgaataca cactgttggg tgtgctgcac tttctcctgg agggtcagtg caaccctgat 360

ctgcggctgc tagggtgctc tgtgctggcc cccagatgcc aaggaggcca cacacaaagg 420

ccctgccgcc gtgtctgtga gggcctgcgg gaggcctgcc agccagcctt tgacgccatc 480

gacatggcct ggccctactt cctagactgt acccaatact ttgcaccaga ggaggaaggc 540

tgctatgacc ctctcgaaca gctccgggga gagctggatg tcgaggaggc tctgccctct 600

ggcctgccac ctaccttcat tcgctttgcc caccactcct atgctcagat ggtccgggta 660

ctgaagagga cagcggctcg ctgctcccag gtggccaaga cgtacagcat cgggcgcagc 720

tttgaaggca aggacctggt agtcatcgag ttctcaagcc gtccaggcca gcatgaactg 780

atggagcctg aggtgaagct cattgggaac atccatggca acgaggtggc aggacgggaa 840

atactcatct acctggccca gtacctgtgc tccgagtacc tgctgggcaa cccccgtatc 900

cagcgcctgc tgaacaccac ccgcatccac ctgctgccat ccatgaaccc tgatggctac 960

gaggtggcgg ctgcagaggg tgctggctac aacggctgga ccagtgggag gcagaatgca 1020

cagaacctgg atctgaaccg caacttccct gacctcacgt cagagtacta ccggctggcc 1080

tccactcgcg gtgtgcgcac ggaccacatc cccatctcgc agtactactg gtggggtaag 1140

gtggcccccg agaccaaggc catcatgaag tggatacaga ccattccctt cgtcctctca 1200

gccagcctgc atgggggtga cctggtggta tcctacccct tcgacttctc caagcacccc 1260

catgaggaga agatgttttc tcccactccg gatgagaaga tgtttaagct gctggccaga 1320

gcctatgctg atgtccaccc catgatgatg gacaggtcgg agaacaggtg tgggggcaac 1380

ttcctgaagc gggggagcat catcaacggg gcggactggt acagcttcac tggaggtatg 1440

tctgacttca actacctaca caccaactgc tttgagatca cagtggagct gggatgtgtg 1500

aagttcccac cagaggaggc gctctacggc ctgtggcaac acaacaagga gcccctactg 1560

aacttcctgg agatggtaca ccgaggaatc aagggtgtgg tgacagataa atacggaaag 1620

cccgtcaaaa acgcacggat tctagtgaaa ggcatccgcc atgacgtcac cacagcccct 1680

gatggtgact attggagact ccttcccccg ggttcgcaca tcgttattgc tcaggcccct 1740

ggctactcca aggtaatgaa gagagtcacc attcccttgc gcatgaagag ggccggccgt 1800

gtggacttca tcctccagcc tctggggaca ggacctaaga acttccttcc tggaccttca 1860

agagccttac ctcggtccct ggatccacag ggagcgcctg cacagctgga ttttgagccc 1920

cccagggcgc gtaggcagcc agctagtggg agcaagccat ggtggtgggc ttacttcaca 1980

tccctgagtc cacacaagcc acgctggctt cttaagtact agcacccctc tcaggcccag 2040

ccctgatgtc cacctccacg gcccgggctt ctgtctggag tctgccagcc acagacatca 2100

cacacagcct ctgcggctta ttaaagtgtg tttacccact gtgaaaaaaa 2150

<210> 84

<211> 2225

<212> DNA

<213> Rattus norvegicus (Norway rat)

<400> 84
ctgcagtact tgtacattgc taaataaaga gagggactcc aggaggagca gcctgggtct 60

aagaggtagg cagaaggagg ttttaggggc ctgagcacaa gcttgaggag agaaaggtta 120

ttaaaaagcc agacgcttac aggtctcaga agggctagcc agaaactgtg gctggggtta 180

aggaaagggt ttaagagtgt gggcttttgg ttctgaggat gtagaacgtg aatgttgaga 240

gaagaaccaa gtggcggagt tgggtgtgag caatgctatt aggaatttga ggcagggatt 300

cacgcgctgc tgtgactatt ttttaacaat gactcagtgc tgtgacctga tactgtttcc 360

agagcgactt ctaaacaaat tccccctttc taggccagac acatggcccc aagcccaaga 420

ccccagcatg tcctgcactg gaaggaagcc cactctttct acctcctgtc tccactgatg 480

ggcttcctca gccgggcctg gagccgcctg aggggcccgg aggtctcaga ggcctggttg 540

gcagaaacag tagcaggagc aaaccagata gaggctgatg ctctgttgac gcctcccccg 600

gtctctgaaa atcacctacc tctccgagag actgaaggaa atggaactcc tgaatggagt 660

aaagcagccc agaggctctg ccttgatgtg gaagcccaaa gttcccctcc taaaacttgg 720

ggactttcag atattgatga acataatggg aagccaggac aagatggcct tagagagcaa 780

gaagtggagc acacagctgg cctgcctaca ctacagcccc ttcacctgca aggggcagat 840

aagaaagttg gggaggtggt ggctagagaa gagggtgtgt ccgagctggc ttaccccaca 900

tcacactggg agggtggtcc agctgaggat gaagaggata cagaaaccgt gaagaaggct 960

caccaggcct ctgctgcttc catagctcca ggatataaac ccagcacttc tgtgtattgc 1020

ccaggggagg cagaacatcg agccacggag gaaaaaggaa cagacaataa ggctgaaccc 1080

tcaggctccc actccagagt ctgggagtac cacactagag agaggcctaa gcaggaggga 1140

gaaactaagc cagagcaaca cagggcaggg cagagtcacc cttgtcagaa tgcagaggct 1200

gaggaaggag gacctgagac ttctgtctgt tctggcagtg ccttcctgaa ggcctgggtg 1260

tatcgcccag gagaggacac agaggaggaa gaagacagtg atttggattc agctgaggaa 1320

gacacagctc atacctgtac caccccccat acaagtgcct tcctgaaggc ctgggtctat 1380

cgcccaggag aggacacaga agaggaagat gacggtgatt gggattcagc tgaggaagac 1440

gcgtctcaga gctgtaccac cccccataca agtgccttcc tgaaggcctg ggtctatcgc 1500

ccaggagagg acacagaaga ggaagacgac agtgagaatg tggccccagt tgactcagaa 1560

acagttgact cttgccagag tacccagcat tgtctaccag tagagaagac caagggatgt 1620

ggagaagcag agccccctcc cttccagtgg ccttctattt acctggacag aagccagcac 1680

caccttgggc tgcccctaag ctgccccttc gactgcagaa gcggctcaga tctttcaaag 1740

cccccgcccg gaatcagggc cctgagattc ctctgaaggg tagaaaggtg cacttctctg 1800

agaaagttac agtccatttc cttgctgtct gggcaggacc agcccaggct gctcgtcgag 1860

gcccctggga gcagtttgca cgagatcgaa gccgctttgc tcgacgcatt gccaggcaga 1920

ggagcagctg ggtccttacc ttacccctgc tttcagggcc agagcatgga cacgccttag 1980

aaacctaccc cttcctctgt cgtcctcgtc tcttccactg cctgagcctt gctcttccac 2040

tgaggccaca cccctcagcc aagatgtgac cactccctct ccccttccca gtgaaatccc 2100

tcctcccagc ctggacttgg gaggaaggcg ggctaagcct gagtagtttt ttgtgtattc 2160

tatgagtgtt agtctcttaa tacgaatatg taacgccttt tgcatttgta aaaaaaaaaa 2220

aaaaa 2225

<210> 85

<211> 6335

<212> DNA

<213> Rattus norvegicus (Norway rat)

<400> 85
   catttgcagc acattttggg ctttccctcc ccctccatac attccccccc tcctttttca 60
   atttttcttc ttcccgggaa gtgaattgct gatgccaagg ggactttaat tccattaatg 120
   atgcaactgg attcgtttca ggattatgtt gcacgaattg aattttgaat gaaggaggtt 180
   ttttttttct ttttgaaaga agtgttgatt ctttagttcg ttgtactttt aattattatt 240
   ttatttaaat atacaattta attgtattat tctcttaaaa atattaaatc tatattttat 300
   ggaaacttac cctcaaaata tatatgttta tgggtgaaat tgaagacgct tccgttaagt 360
   gaggtgactg gtgtattgga tgtttaattg agcaccggca ttgcatgaca gttgttgaat 420
   aacaagtggt ttatttttaa aaaccacatc tttaaaattt taggttcaga tagtaacagt 480
   agacatcgta agggcattta aaagaaaacc agcagaaatc gaagcaaaca tgtctggaga 540
   agtgcgtttg aggcagttgg aacagtttat tttagatggg ccagctcaga ccaatgggca 600
   gtgcttcagt gtggagacgt tactggacat actcatctgt ctttatgatg aatgcaataa 660
   ttctccattg agaagagaga agaacattct tgaatatcta gaatgggcta aaccattcac 720
   ttctaaagtg aagcaaatgc gcttacatag agaagacttc gaaatattaa aggtgattgg 780
   tcgaggagct tttggtgagg ttgctgtagt taaactgaaa aatgcagata aagtatttgc 840
   catgaaaatt ctgaacaagt gggaaatgct gaaaagagct gagacagcgt gctttcgtga 900
   agagagggat gtgttggtaa atggagacag taagtggata accactctgc actatgcttt 960
   tcaggatgac aataacttat acctggttat ggattattat gttggtgggg atttacttac 1020
   tctgctcagc aaatttgaag atcgattgcc agaagagatg gctcggtttt acttggctga 1080
   gatggtgata gccattgact cagttcacca gctccactat gttcacagag acatcaaacc 1140
   tgacaatata ttgatggata tgaatggaca tattcgttta gcagattttg gttcttgtct 1200
   gaagctgatg gaagatggaa cggtccagtc ctcagtggca gttggaactc cagactacat 1260
   ttccccggaa atccttcagg ctatggagga cgggaaaggc agatatggac cagagtgtga 1320
   ctggtggtcc ttgggagtct gcatgtatga gatgctttac ggagaaacac cattctatgc 1380
   ggaatctctg gtggagacat atggaaaaat tatgaatcat aaagagaggt ttcagtttcc 1440
   aacccaagtg actgatgtgt ctgaaaatgc taaagatctt attcgaagac tcatttgtag 1500
   cagagaacat cgacttggcc agaatggaat agaagacttt aagaaacatc cgtttttcag 1560
   tggaattgat tgggataaca tccgaaactg tgaagcacct tacattccag aagttagtag 1620
   tccaacagac acgtcaaatt tcgatgtgga tgacgactgt ttaaaaaact ctgaaacaat 1680
   gcccccacca acacatactg cattttctgg ccatcatcta ccatttgttg ggtttacata 1740
   tactagtagc tgtgttcttt ctgatcggag ctgtttaaga gtcacagcag gtcccacctc 1800
   actggatctt gacgtcaatg tccagaggac tctagataat aacttagcaa ctgaagctta 1860
   tgaaagaaga attaaacggc tagaacagga aaagcttgaa cttactagaa aacttcaaga 1920
   gtcaacacag actgttcaag ccctgcagta ttcaactgtt gatggtccac taacggcaag 1980
   caaagattta gaaattaaaa gtctgaaaga agagattgaa aaactaagaa aacaagtagc 2040
   agaagtgaat catttggaac aacaacttga agaagctaat tctgtgagac gagaactaga 2100
   tgatgctttt aggcaaatca aagcttttga aaaacaaatc aaaactttgc agcaggaaag 2160
   agaagagcta aataaggaac tagtccaggc tagtgagcga ttaaaaaacc aatccaaaga 2220
   gctgaaagac gcacactgtc agaggaaact ggccatgcag gagttcatgg agatcaatga 2280
   gcgactaaca gaattgcaca cccaaaagca gaaacttgct cgccatgtcc gagataagga 2340
   agaagaggtg gacctggtga tgcaaaaagc tgagagctta aggcaggagc ttcgcagagc 2400
   agaaagagcc aagaaagagc tggaggttca cactgaagct ctaattgctg aagcctcaaa 2460
   ggaccggaag ctccgagagc agagcagaca ttattctaaa cagctggaaa atgaattgga 2520
   gggactgaag caaaaacaaa ttagttactc accaggaata tgcagcatag aacaccagca 2580
   agagataact aaactgaaaa ctgacttgga aaagaaaagt atcttttatg aagaagaaat 2640
   ctctaaaaga gaaggaattc atgcaagtga gattaaaaat ctgaagaagg agctgcatga 2700
   ctcagaaggc cagcaacttg ctctcaacaa agaaattatg gttttaaaag acaaattgga 2760
   aaaaaccaga agggaaagtc aaagtgaaag ggaagaattt gaaaatgagt tcaagcaaca 2820
   atatgaacgg gaaaaagtat tattaactga agagaataaa aagttaacga gtgaactcga 2880
   taagctaact tcgttgtatg agagcttaag tttgcgcaac cagcacttag aagaagaggt 2940
   caaagatcta gcagataaga aggaatcagt tgcccactgg gaagcccaaa tcacagagat 3000
   aatccaatgg gtgagtgatg agaaggacgc acggggctat cttcaggcct tagcttctaa 3060
   aatgactgaa gaattagaag cgttaaggaa ttccagcctg gggacacgag cgacagatat 3120
   gccctggaaa atgcgtcgtt ttgcaaaact ggacatgtca gctagactag agttgcaatc 3180
   tgccctggat gcagaaatca gagctaagca ggccatccag gaagagctga ataaagtcaa 3240
   agcatccaac atcataacag aatgtaaact gaaagattca gagaagaaga acttggaact 3300
   gttgtccgaa atcgagcagc tgataaagga cactgaagag cttagatctg aaaagggtgt 3360
   agagcaccga gactcacagc actctttctt ggcatttttg aatacgccta ccgatgctct 3420
   ggatcaattt gaacgctctc cgtcctgtac tccagctggc aaaggcagac gtattgcaga 3480
   ctctgctccg cttccagttc acacaccaac tttaaggaaa aagggatgcc cagcttcagc 3540
   tggttttcca cctaagcgca agactcatca gtttttcgtg aagtctttca ctgcccctac 3600
   caagtgccat cagtgtacct ccttgatggt cggtttgata agacagggct gttcctgcga 3660
   agtctgtggg ttctcatgtc atataacttg tgtgaacaaa gccccgacca cttgcccagt 3720
   tcctcctgag caaactaaag gcccactcgg tatagacccc cagaagggag tagggacagc 3780
   gtatgagggc catgtccgga tccctaagcc ggcgggagtg aagaaagggt ggcagagagc 3840
   actggctgtg gtctgtgact tcaaactgtt tctttatgat atcgcggagg gaaaagcatc 3900
   gcagcccagc tccgtcatta gtcaagtgat cgacatgaga gatgaagaat tttccgtgag 3960
   ttcagtcttg gcttctgatg ttattcatgc aagtcgaaaa gatattccct gtatatttag 4020
   agtcacagcc tcccagctct cagcacctag tgacaaatgt tccatcctga tgctagccga 4080
   cagcgagact gagaggagta agtgggtggg agtgctgagt gaactccaca aggttctgaa 4140
   gaaaaacaag ttcagagacc gctcagtgta tgtccccaaa gaggcttacg acagcactct 4200
   acccctcatt aaaacaacgc aggccgcggc aatcatagat catgagcggg tagctttggg 4260
   aaatgaagaa ggattatttg ttgtgcatgt cactaaagat gagattatta gagttggtga 4320
   taataagaaa attcatcaga ttgaactcat tccaagtgac cagcttgttg ctgtgatctc 4380
   aggccgaaat cgccacgtcc gacttttccc tatgtcagct ttggatgggc gagagacgga 4440
   tttttataaa ctggcagaga ctaaagggtg tcaaaccata gctgctggga aggtgcgcca 4500
   tggagctctg tcctgcctgt gtgtggccat gaaaagacag gtcctctgct atgaactgtt 4560
   ccaaagcaag accaggcaca ggaagtttaa ggagattcaa gtcccatgta atgtccagtg 4620
   gatggccatc ttcagtgaac atctctgtgt gggattccag tcaggatttc tcagataccc 4680
   cctgaatggg gaaggaagtc catgcaatat gctccattca aatgaccaca cactggcatt 4740
   cattacacac caaccaatgg atgctatctg tgccgttgag atctccaaca aagagtattt 4800
   gctgtgtttt agcagcattg ggatatacac ggactgccag ggccgaagat ctagacagca 4860
   ggaactgatg tggccagcaa atccttcctc ctgttgttac aatgcaccat atctctccat 4920
   ttacagtgaa aatgcagttg atatctttga tgtaaactcc atggaatgga tacagacgct 4980
   tcctctcaag aaggttcgac ctttaaacac tgaaggatca ttaaatcttt tagggttgga 5040
   gaccattaga ttaatatatt tcaaaaataa gatggcagaa ggggatgaac ttgtagttcc 5100
   tgaaacatca gataatagcc gaaaacagat ggttagaaac atcaacaaca aacggcgtta 5160
   ctccttccgg gtcccagaag aagagaggat gcagcagaga agggagatgc tacgagatcc 5220
   agaaatgaga aataaattaa tttctaaccc aactaatttt aaccacatag cacacatggg 5280
   tcctggagat ggaatacaga tcctaaaaga cctgcccatg aacccacggc ctcaggaaag 5340
   ccggacagta ttcagtggct ccgtcagtat tccctccatc accaagtccc gccctgagcc 5400
   aggccgctcc atgagtgcca gcagtggcct gtctgcacgg tcatcagccc agaatggcag 5460
   tgcgctaaag agggaattct ctggaggaag ctacaacacg aagcgacagc ccatgccctc 5520
   tccctcagaa ggctctttgt catcaggagg cgtggaccaa ggaagtgacg cccccgtgag 5580
   ggactatgat ggagaggact ctgattctcc aaggcattcc acagcttcca acagctctaa 5640
   cctgagcagc cccccaagcc ccgtttcacc ccgaaagacc aagagcctct ccttggagag 5700
   cacagaccgt gggagctggg atccatgagc aggaccagtg actccaggca gggaccctgc 5760
   cctcccactc tgacaacgcg cctggcagca gcatcacctt ccacactgcc tgccaccgag 5820
   gattccttca cagccaggcc cgcttcaccc ttcccccaac ccccaggtag cttctgtaga 5880
   cagactgcgc agacgcagcg ctaagagtag accggttgtg ttctcaccgt agccctcacg 5940
   tagccctcac gtagcgccca gcagcctcca tgagcgtcag ctgactgttg ccaggaaata 6000
   aaccctcatg aattaaaaca atgcatattt ttactacaat aaagagttta aataaataca 6060
   tagatgtaga cgttaaatac tgaagtataa atagtcaaag aagcatctct tgtgttcgat 6120
   gaaagatgga tgcgatataa gaaagatcat ttaatttaaa aaaaaaaagt gttgtttctt 6180
   atctgtttcc cagctaagtc ataatacagg gtagaaaggc ctcaagtgac atttataaag 6240
   acacagaagt caggtcccgg tggtgtctgt cctcaagcct aggcagctgt ggtcacaggg 6300
   aagtctaatg tctggagatt actcgtgccg aattc 6335

<210> 86

<211> 4076

<212> DNA

<213> Rattus norvegicus (Norway rat)

<400> 86
   gggcttgcag ccagcgcctc ctcttaagag aagcgtctct ccaccgcatc cacaggcttg 60
   tgttgtgatg tattcgcgaa gtggctttct tctgaagact ccagggtctg acttctgaag 120
   agaagaagga agaggagggt ggaggagcac acagaaaggc ctggaggcgt cccctggtgt 180
   tcaggcagct tctgggtcag aacttggaga caccatgaca aacagcgtct gtcccacagc 240
   ccaaggttca ggcaaagtca tagccaggcg gtgtggtcag ggcccgggct ggggattccc 300
   catctcccca gtttcacttc tgcttctaac ctgtgtcagg tccttctgcc cggatactga 360
   tgacgcgcta gcaattctct cattcctatt gggtgccgtg attccggtga accaatcagc 420
   gttgcccaga cactagatgt aaactccacg cagccgcggg actcagccgc gtccgggacc 480
   tcggataggg gcgatggcac cgcgcacgct gctcctgctg ctggcggccg ccctggcccc 540
   gacccaggtt cgcgcgggtg agtgcggggt cgggagggaa acggcctctg cggggaggcg 600
   cgggagcggc acggggaagc ggtccccggt cgcacccaag accccggccc cttctccacc 660
   gcgtccggag ccccggcccc tgctccccgc gcgccggcag ccgcccggga tcctgggagg 720
   aggtcgggat ctcaccgcgc gccgccccca ggctcacact cgctgcggta tttccacacc 780
   gccgtgtccc ggcccggcct cggggagccc cggttcatta tcgtcggcta cgtggacgac 840
   acggagttcg tgcgctacga cagcgacgcg gagaatccga gatacgagcc gcgggttggg 900
   tggatggagc gggagggtcc ggagtattgg gagcgggaga cactgatggt caaggaaaac 960
   gagcaggttt accgagtgag cctgaggacc ctgctccgct actacaacca gagcgagggc 1020
   ggtgagtgac cccgggtcgg aggtcaggac ccctccccgt ccccacacgg ccggagactt 1080
   ccgggtccga agtccaggtt cgggaacaga accgacccgg accggtttcc ctttcacttt 1140
   ggaggagtcc gcgggtgggc ggaacgggtg ggcggggccc ggcctgaccg cggggtcccg 1200
   caggctctca caccatccag tggatgtccg gctgtgacgt ggggtcggac gggagcctcc 1260
   tccgcgggta tgatcagttc gcctacgacg gccgcgatta cctcgccctg aacgaagacc 1320
   tgaaaacgtg gatggcggcg gacacggcgg cacagatgac ccgaaacaag tgggatcagg 1380
   caggtgaagc agagagacac aaggcctacc tgcagggcac gtgtgtggag tggctccgca 1440
   gatacctgga gctcgggaag gagacgctgc tgcgctcagg tgcgggggcc gcgggcttct 1500
   cctcctctgc cctcgggctg ggctcagtcc ctggggaaga agaaaccctc agctgggtga 1560
   tgcccctgtc tcagagggga gacagtgtcc ctgggtctcc ctgatccctc atcacggtga 1620
   ctgcactgcc tctcccacag ggcccagcct tctccctgga gagttctgag tccctctcag 1680
   gagaaggagg gaatttcccc cttaacagca ggggctccct gctgtcagcc atggcctctc 1740
   ccaggcatgt tctctgccca cacccactct ctgaggacac tgactcctgt cctgctgagt 1800
   gtgtcagccc ttacacctca ggaacagaag tctcctttac ctcacaggac tccactactc 1860
   taggctggtt cccacagttt ctaaaagatt ccccgggttt ctcacagatc cctccctgat 1920
   gtggggttgg catcccttca ataccccatt tctctctctt cctacagtgg agaatggtac 1980
   catgagccct tatggggtac cctggaggaa tataaattgt gggatttctt tttctttttt 2040
   ctttttttct ctctttttct tatcttttac ttcctttttt ctttcctttt tttttttttt 2100
   ttttttttgg aaggcattat tttacttcca gtcaggtttt ctctgcaaac acatattaaa 2160
   tcaattattt tctattccct cttctgttct gtgaccaccc atctcatgct ataaaacacc 2220
   cgtaaaggac ggccatgttg acccactcgc tcatgtggat tccctcttag cttctgagtc 2280
   cctcaggaaa gtgtgcagtt ctgtgctgag ggaaccagct ctccctgcag ctcactaggg 2340
   agatgacagt tgaagtgtca gacacacaca gttcagtgtc atccttgatt taactgtgtc 2400
   ctgtgtagat ttcagtttgt cttgttaact gtgtgatttc ttaaatcttc cacacagatc 2460
   ccccaaaggc acatgtgacc cttcacccca gacctgaagg tgatgtcacc ctgaggtgct 2520
   gggccctggg cttctaccct gctggcatat tcctgacctg gctgttgaat ggggaggatc 2580
   tgacccagga catggagctt gtggagacca ggcctgcagg ggatggaacc ttccagaagt 2640
   gggcatctgt ggtggtgcct cttgggaagg agcagaatta cacatgcctt gtggagcatg 2700
   aggggctgcc tgatcctctc accctgagat ggggtaagga gggtgtgggt atagagctgg 2760
   ggtcaggaaa agctggagcc ttttacagac cctgagctgg tcaaggctga gagctggtgt 2820
   tcctgacccc caccttcatt tcctgtacct gtccttccca gaggctcctc catccactga 2880
   ccccaacatg gaaaccactg tcatttatgt tgtcgttgga gctgtggtca tcactggagc 2940
   tgtgaccatg attggagctc tgctgactgt tgtgaggggg aggaagagaa acacaggtag 3000
   gaaagggcag ggtcttgttt cccctcagct tcctttagaa gagcgctctg atcattaatg 3060
   ggaagcacag ccacacccca cattgctgct gtctccaact gggtcctctg tcagttctgg 3120
   aaacttcctc ctgtcaagac cttccttgaa ctctcacagc ttttcttctc acaggtggga 3180
   aagtaggagt ctacacccct gctccaggtt agtgttgggg caggagtgtc cctgagggta 3240
   ttggagtgaa gctggagatg tggggaactc ggggaatcca tagtagctcc tccatagaaa 3300
   tcttctgggg gcctcagttg taccgtgatg tgaatacata catatacaca tatacatata 3360
   cagaacacaa tacgtgtgtg tgtgtgtgtg tgtgtgtgtg tgtgtgtgtg tgtgtgtgta 3420
   tgttttcttt taccctaggc aaggacagct ccgagagctc tgatgtgtct ctcccagatt 3480
   gtaaaggtaa ctctgggtct gactggggag gggcaatgtg gacatgattg cgtttcaggg 3540
   actcccagaa tcccctgtga gtggagggtt gttgggctgt tgtcttcaca gtgatggttt 3600
   gtgactctcg ttctctagca tgaagacagc tgcctggagt ggactgagtg acagacgatg 3660
   tgttcaggtc tctcctgtga catccagagt cctcaggtct ctttagacac cagtgtctga 3720
   tgttccctgt gattctatgg cttcagtgtg aagaactgtg gagcccaggc tgccctgcac 3780
   acctggaccc tgtccctgca ctgcctgcat tcccttccac attcaacctt gctggtccag 3840
   cctggggttg ggggggtggg gacatatgca tcctgtcact cagtgctgct ttgagctgca 3900
   actcctcact tctacactga gaataagaat ctgagtgtga acttgactgt tcacatcctt 3960
   gacacaagtg ttgattgctt tttaaattac tggattgaat acttagaggt tctttgtttt 4020
   ttatttttgt tttgttttgt tttctttgtt ttgttttaaa ataaatggca ggtgga 4076

<210> 87

<211> 4969

<212> DNA

<213> Rattus norvegicus (Norway rat)

<400> 87
aagcttacac agaccctaca acaagacttg gtgaaaccgt gtgcccaaga gcacaagtgc 60

ggttccgtgc ggtttagtgc ggtttacacg acaggaggtc gtgtaaacag ctcctgctgc 120

tttctctttc ataaagacac aagtgataca gtctcgcctc tcgttcctgt tggacgcagg 180

cgtctgtaga agccaatcag cagcacagaa gttccaaagt acgagtcaac agaaacacaa 240

cccacattca tgtttttcac agtccctggg cgtgaggatg ttacctcctg cctatttgct 300

ccaactactg gcaggcacca tggtcctgac ccagactagt aagtgcgggg aggggtcggg 360

aaagaaaaga ctctgagagg tgaaaggggg tggggtgtgg ggccaggggt ggtggcgggg 420

gggggcagca ccctagggtc tgtcacccta tcccagtccc tcctctgcca gcacctgaga 480

tcctgagcct agaggagctc agggtctcac tgcgcgccgc ccccaggctc acactcgctg 540

cggtatttct acaccgccct gtcccggccc ggcctcgggg agcccaggtt cattgtcgtt 600

ggctacgtgg acgacacgca gttcgtgcgc tatgacagcg atgtggagaa tccgaggatg 660

gagccacggg cgcggtggat ggagcaggag gagccggcat attgggagcg ggagactcgg 720

aaagccaggg acaccgggag gaacttcaaa gtgaatctga ggaccctgct ccgctactac 780

aatcagagtg acgacggttg agttgactgc cggagggatc acaaacagga tccctccatg 840

ttccctgaga ctggcctggg tcatcttgaa cccggccggg tcaaagtatc atccaaacgc 900

ctacctagaa atcccagaca agaaagcccc ggcagagttc tgcttaggtt tcggttgcac 960

ttttgtttct aaaaattctg gcatatctac tgacagcggg caagatggcc acgagggggc 1020

gctgaaccgg cgtttcccct ttcagaatct cacacgctgc agtggatgta cggctgcgac 1080

gtggggcccg atgggcacct gctccgcggt tattgtcagg aggcttatga cggccgggat 1140

tatatctcct tgaacgagga cctgcgctcc tggaccgcga ctgacatggc ctcacaggcc 1200

tctaagatca agtcagagga agttggtgag gcccatcacc agagggccta cctgcaaggt 1260

ccttgcgtgg agtggctcca cacataccta catctgggaa aggagacgct gctgcgctca 1320

ggtgccctgg agacccctcc ttagttttcc tctgggggtt tgggggggaa aggcttgggg 1380
tatagctcag gggcagaacg ctgtccagca gccacagtgc tgaagagaag ggagaaagac 1440
tcccaaggct gcttttcctc tgtcaggaag actaagcctt aatcaagcag atcagcttga 1500
caatgtccct ggacccatta ggggcatttt cctcaggcct ccctctatgc cctactcagt 1560
gtctctgaga gtcagactcc agcgttgttc tcagtctcta ggccctcacc caggctcagg 1620
acgagaaggc ccatgcccag gagtcctggc cagagaccct ggagtctcct gccccatagt 1680
tgtcagactc acatcctaaa gcagacaaag gaacagcgcc gggaggtgca tgtgctcctg 1740
ctggtgtctg gattgtggac accctaatcc catcccaggt ctcctgacca tcccaggatg 1800
gtcacatgga caatactgtg tccctggaag aaagctagat gcctcagtct tacaagtctc 1860
tccctcagac cccccaaagg cacatgtgac ccttcacccc agacctgaag gtgatgtcac 1920
cctgaggtgc tgggccctgg gcttctaccc tgctgacatc accttgacct ggcaattgaa 1980
tggggaggac ctgacccagg acatggagct tgtggagacc aggcctgcag gggatggaac 2040
cttccagaag tgggcagctg tggtagtgcc ttctggggag gagctaaagt acacatgcca 2100
tgtggagcat gagggactgc ctgaacctct cgccctgaga tggggtaagg agggtgtggg 2160
tgcagatctg gggtcaggga atgtggagcc ttctgcagac cctgagctgg tcagggatga 2220
gaatcatgac tcttccttcc cagagccttc tccattcacc gactccagca tgccagtgat 2280
tgttgtcctt ggagctgtgg ccatcattgg agctgtggcc atcattggag ctgtggccat 2340
cattggagct gttgtgagga ggaggaagag aaacacaggt aggaaagggc agggtctgac 2400
tcttctctca gcctctttga agtgtgctgt gctcattaag gggaaacaca gccacaccct 2460
gcattgccta gtctctaacc tggactgctg tcagtgctgg aaacttccta gggctggtgt 2520
ttccctgact tctcatgact ttccttctcg acaggtgaaa aaggcagcta tgctcatgtt 2580
ctaggtaggt gtgggtgggg ttggccgtgg agtggggtgc acccttgtac ccagaggccc 2640
ttgggatggg agctggcgct tatggaggag ttctgccagg cccataattc cctcttgcca 2700
tattggtcac ctgaatatac ctgcatttat aattcctcta ctcaggcagc aaggctttcc 2760
agatctctga ctggcctcag aaggcatgaa aaggtatgaa aacccatggg ggcctgggat 2820
cggacctgag gcaaagggaa gaacagggcc ttgtgggatg atgtcacagg tgaggcaggt 2880
acaagataga aggaggcctg ttattggatg agaaagaagg atgggtggga gacaagtttg 2940
aaggaagaag aggatactgg aacagagaga gaggaggaga ggagggagag ggagagaagc 3000
cttggcaggc aatatatcag gtgacgttaa gattctcctc tgtgtactta gaggttgtta 3060
ttaatgttcc taagggatgg atggtaccgg gctttgtatg tttaactcgg caattatatc 3120
ttatcaattg gctctaagat tattgttggt gtgttctttt atgtgatggt ttgagtgtag 3180
gaaagtgtgc agtgacagga gacgatgggc cagcacagag ttgggatgtg tttccaccca 3240
agatatctaa cagatatctt ggggcactgc agtgcagacc tagcggggta aaagacaact 3300
atactttttt tactttttat atttttacaa caacagggtc tctcagattt agacatttga 3360

atgactgagc atctttacaa tgacttcagg actcttgttc tattgcgttc agacagttgc 3420

ctcgtggggc tgagcgacaa gttctgacta aacatctgag ggagctgctt cccctgagga 3480

caatgtgaag aactggcaat cccagacctt cttgcctccc tggacacctg acttcacaac 3540

agaaaataat ctgactctgg gctgggtgtt tcctattgct tttatctgca ctgagtggaa 3600

atgcgatttc caacagtgta acgcagcacc gttgtgtggt ggtagtgaca ttgcacacag 3660

gtttgtgagc tgggggtgtg atgtgctttt cttactgtag gcagaacaag ctagaagtcc 3720

accattcctc ttatggtcat tgttttaaaa caaaacaaat gctggagaga tggctcagtg 3780

ttaagagcac tgacttctct tacagaggtc ctggttcaaa tcccagcaac cacatggtgg 3840

ctcacaacca tatgtaatga gatctgatgc tctcttctga tgtgtctgaa cacagctata 3900

tatgataaat aaatctaaaa aatatttttt aaatggccag acagatggca tgagtagggc 3960

acaccatggt caactctgct gtctgtgtat ctctagacat ccagtgctct ttagagtcca 4020

cacaaggtca ttaaaaggac tggggtggat ggtgactgga gagatagctc agtggttaag 4080

agtgtttgct gcttcatggg tggtggtggt ggcacaagcc tttaatccaa acactcagga 4140

ggcagaggca ggtggatctc tgagttcaag gccagcctgg cctataaggc aagttccaag 4200

atagccaagg atacacagag aaaccctgtt tgaaaaaacc caagcctaga cctggagagt 4260

tggctcaatg gttaagagca ctgactgctt ttccagaggt cctgagttca aggcccagca 4320

aaggcacatg gtggctcaca accatctgta atgggatctg atgcactttt ctggtgtgtc 4380

taaagcgaca tggtaactcg ctgtctgtaa ctagtaggca aacagttcat atctcacaca 4440

gacatgaatg cagatagagc accaacacat ataaaattaa atctttaaaa aatgaaaggt 4500

ctgggggctg gatgaatggt gtttgtcttt gaaatgtctg acagtgctgt gactcacagc 4560

ctgcatagca gctcctattg cctggtgttc accatctgtc ttggttataa aaaggtttcg 4620

taatgtgaaa cccttcaaat gagctggtgc ccttctcacc agtttgcatc ttgacaggat 4680

tcctgaaggg cttatattct gggccttccc attgtggagg attaggtttt acacaacgta 4740

tcctctctag cattgcaatt tccctgagcc ttaaaatccc ttcatcgacg ctgagccaag 4800

ggatatctgg catctgcaac tccttttccg taggccgtca tttgatgaac gcttcagcca 4860

accattcaaa caaactttct gacacttttt tttaactgtg tggccttcca tattaaaccc 4920

agaatctcca ctcaaagggc ccatgtcaat aaactcagcc tgatctatt 4969

<210> 88

<211> 2847

<212> DNA

<213> Rattus norvegicus (Norway rat)

<400> 88
cagctccaag gcacttgctc catttggggt gtgcctgcac ctagctggtt gcaaattggg 60

ccacagagga tctggaaagg atggaacaac gggctagctt agactcagag gagtctgagt 120
ccccacccca agagaactcc tgcctggacc ctccagacag agaccctaac tgcaagccac 180
ctccagtcaa gccccacatc ttcactacca ggagtcgtac ccggcttttt gggaagggtg 240
actcggagga ggcctctccc ctggactgcc cttatgagga aggcgggctg gcttcctgcc 300
ctatcatcac tgtcagctct gttctaacta tccagaggcc tggggatgga cctgccagtg 360
tcaggccgtc atcccaggac tccgtctccg ctggtgagaa gcccccgagg ctctatgatc 420
gcaggagcat cttcgatgct gtggctcaga gtaactgcca ggagctggag agcctgctgc 480
ccttcctgca gaggagcaag aagcgcctga ctgacagcga gttcaaagac ccagagacag 540
gaaagacctg tctgctaaaa gccatgctca atctgcacaa tgggcagaat gacaccatcg 600
ctctgctcct ggacgttgcc cggaagacag acagcctgaa gcagtttgtc aatgccagct 660
acacagacag ctactacaag ggccagacag cactgcacat tgccattgaa cggcggaaca 720
tgacgctggt gaccctcttg gtggagaatg gagcagatgt ccaggctgcg gctaacgggg 780
acttcttcaa gaaaaccaaa gggaggcctg gcttctactt tggtgagctg cccctgtccc 840
tggctgcgtg caccaaccag ctggccattg tgaagttcct gctgcagaac tcctggcagc 900
ctgcagacat cagcgcccgg gactcagtgg gcaacacggt gcttcatgcc ctggtggagg 960
tggcagataa cacagttgac aacaccaagt tcgtgacaag catgtacaac gagatcttga 1020
tcctgggggc caaactccac cccacgctga agctggaaga gatcaccaac aggaaggggc 1080
tcacgccact ggctctggct gctagcagtg ggaagatcgg ggtcttggcc tacattctcc 1140
agagggagat ccatgaaccc gagtgccgac acctatccag gaagttcacc gaatgggcct 1200
atgggccagt gcactcctcc ctttatgacc tgtcctgcat tgacacctgt gaaaagaact 1260
cggttctgga ggtgatcgct tacagcagca gtgagacccc taaccgtcat gacatgcttc 1320
tcgtggaacc cttgaaccga ctcctacagg acaagtggga cagatttgtc aagcgcatct 1380
tctacttcaa cttcttcgtc tactgcttgt atatgatcat cttcaccgcg gctgcctact 1440
atcggcctgt ggaaggcttg cccccctata agctgaaaaa caccgttggg gactatttcc 1500
gagtcaccgg agagatcttg tctgtgtcag gaggagtcta cttcttcttc cgagggattc 1560
aatatttcct gcagaggcga ccatccctca agagtttgtt tgtggacagc tacagtgaga 1620
tacttttctt tgtacagtcg ctgttcatgc tggtgtctgt ggtactgtac ttcagccaac 1680
gcaaggagta tgtggcttcc atggtgttct ccctggccat gggctggacc aacatgctct 1740
actatacccg aggattccag cagatgggca tctatgctgt catgattgag aagatgatcc 1800
tcagagacct gtgccggttt atgttcgtct acctcgtgtt cttgtttgga ttttccacag 1860
ctgtggtgac actgattgag gatgggaaga ataactctct gcctatggag tccacaccac 1920
acaagtgccg ggggtctgcc tgcaagccag gtaactctta caacagcctg tattccacat 1980
gtctggagct gttcaagttc accatcggca tgggcgacct ggagttcact gagaactacg 2040
acttcaaggc tgtcttcatc atcctgttac tggcctatgt gattctcacc tacatccttc 2100

tgctcaacat gctcattgct ctcatgggtg agaccgtcaa caagattgca caagagagca 2160

agaacatctg gaagctgcag agagccatca ccatcctgga tacagagaag agcttcctga 2220

agtgcatgag gaaggccttc cgctctggca agctgctgca ggtggggttc actcctgacg 2280

gcaaggatga ctaccggtgg tgtttcaggg tggacgaggt aaactggact acctggaaca 2340

ccaatgtggg tatcatcaac gaggacccag gcaactgtga gggcgtcaag cgcaccctga 2400

gcttctccct gaggtcaggc cgagtttcag ggagaaactg gaagaacttt gccctggttc 2460

cccttctgag ggatgcaagc actcgagata gacatgccac ccagcaggaa gaagttcaac 2520

tgaagcatta tacgggatcc cttaagccag aggatgctga ggttttcaag gattccatgg 2580

tcccagggga gaaataatgg acactatgca gggatcaatg cggggtcttt gggtggtctg 2640

cttagggaac cagcagggtt gacgttatct gggtccactc tgtgcctgcc taggcacatt 2700

cctaggactt cggcgggcct gctgtgggaa ctgggaggtg tgtgggaatt gagatgtgta 2760

tccaaccatg atctccaaac atttggcttt caactcttta tggactttat taaacagagt 2820

gaatggcaaa tctctacttg gacacat 2847

<210> 89

<211> 453

<212> DNA

<213> Rattus norvegicus (Norway rat)

<400> 89
ttcttccctc actttgactt aaatgagact ttagaaaagg aagatgaatg tgtctcatta 60

tttgccatgt ttaatcagaa tcgctgattc ttcagttctt gaatttttgt aaggcgtctt 120

tagaatggga tgatggtatt gagtaattgg gtggagtttg gtcggtgaca aaaatacttt 180

ctagaaattt gtttgtaact aatagcggaa aactcaaaaa taatgtaatt ggggagctgg 240

agtgatggct cagtggctaa gagtagttgt tgatctttca gaagacctgg gtttgattcc 300

cagcaacgcc ctcttctggc ctccttgggc accagacaca tacatggttc acaggcatac 360

ttgaagccaa aacactacta cacataaact aaaataaaaa atacttgggg ggggggcagg 420

caaaacaaaa caaaacaaca acaaaaaaaa aaa 453

<210> 90

<211> 816

<212> DNA

<213> Rattus norvegicus (Norway rat)

<400> 90
gttgtgctag attaaagttt cagagctcag tagattgaga ttgtaatgtc tgtcatgtat 60

gcatgcattt ctgtaacaga catgcatcaa cttttacata agtacgcttg tgtgactcgt 120

tgagaaaaca caggaaagaa agattgtaag tccaaatcat gtcttccact ttgctcttct 180

atatttcttg accacttaga aaaaaagctc acaagccttt gaagaaacag cagcaggctg 240

ttattttttt caaggtttac aaattaagtg cttgattttt aatgtattct cgctactgtt 300

ttgattttca gatcttgatt caatatttga agatgtgcct tttattgggt aatgttaatt 360

ctcgaatgta gtaatgttgt accacaattg taatgttaca tgatcatggg tgtggcattc 420

tctgacaaat tatgtgacaa gaaagtcatt ttcagagaac tcatggctcc gattgcacag 480

tccccttgct aaagcaaagg gcactctctt aaggctactt cagaactggt ctttgtattt 540

tctctaagtg atggatgcgt cgtgtccatt tagaggcagg gtaccacgtg tcccaggcag 600

ccctagatat gtgccatgag aaagttcaaa gactaacaaa gtctacagga actcaaaatt 660

tcatttttgt aatggtaacc ccccaagatt ttctccaaga aataaattat tttgtacttt 720

ataacttaaa agcagctagt agtacatttc tgagatatag caggagacca aaacattttt 780

ggaaaaagaa taaatatata aagagaaaaa aaaaaa 816

<210> 91

<211> 217

<212> DNA

<213> Rattus norvegicus (rat)

<400> 91
tggatgcgtc gtgtccattt agaggcaggg taccacgtgt cccaggcagc cctagatatg 60

tgccatgaga aagttcaaag actaacaaag tctacaggaa ctcaaaattt catttttgta 120

atggtaaccc cccaagattt tctccaagaa ataaattatt ttgtacttta taacttaaaa 180

gcagctagta gtacatttct gagatatagc aggagac 217

<210> 92

<211> 1039

<212> DNA

<213> Rattus norvegicus (Norway rat)

<400> 92
gaaacgcctc ctcgttttgc tcagcatggc acatttgagt atgaatattc tcaacgatgg 60

aagtctttgg atgaaatgga aaaacagcaa agggaacaag ttgaaaaaaa catgaaggat 120

gcaaaagaca agttggaaag tgaaatggaa gatgcctacc atgaacacca agcaaatctt 180

ttgcgccaag atctgatgag acgccaggaa gaattaaggc gcatggagga acttcacagt 240

caagaaatgc agaaacgtaa agaaatgcag ttgaggcaag aggaggaacg gcgtacaaga 300

gaggaagaga tgatgattcg ccaacgtgag atggaagaac aaatgagacg ccaacgagaa 360

gaaagctaca gcaggatggg ctacatggat cccaagagaa agagacatga gaatgggtgg 420

tggtggaaca atgaacatgg gagatcccta tggttcagga ggccagaaat ttccaccgct 480

aggtggtggt ggtggcatag gttatgaagc taatcctgga gttccaccag caaccatgag 540

tggttccatg atgggaagcg acatgcgtac tgagcgcttt gggcagggag gtgcgggtcc 600

tgtgggtgga cagggtccta gaggaatggg gcctggaacg ccagcaggat atggtagagg 660

gagagaagag tatgaagggc caaataaaaa accccgattt tagatactta ggctttcatt 720

ccagtttggt tttgtcttct tgtttagata ccacttaaat tcttggcatt ttagtaagaa 780

agctaccttt ttatggatgt tagcagttta ttgacctgat atttgtaaat ggcctgtttg 840

ggcaggtaaa attatgtaac gcagtgttca aaacaggaga aaaatttttt catttacttt 900

ttttttaact gtataatgtt tctcaagtta atggcagtgt accttgtgcc actgaatttc 960

caaagtgtac catttttttt ttactgtgct tcaaataaat agaaaaaaat agttatgaac 1020

aaaaaaaaaa aaaaaaaaa 1039

<210> 93

<211> 1053

<212> DNA

<213> Rattus norvegicus (Norway rat)

<400> 93
ggagctgtcc agcgctgaga gacaggaaag gccatggcta aggagtgggg ttacgccagc 60

cacaatggtc ctgagcactg gcatgaactt tatccaattg ccaaagggga caaccagtca 120

cccattgaac tgcatactaa agacatcagg catgatcctt ctctgcagcc ttggtcagta 180

tcttatgatc ctggctctgc taagaccatc ctgaacaatg ggaagacctg cagagttgtg 240

tttgatgata ccttcgacag gtccatgctg agaggtggcc ctctctctgg accctaccga 300

cttcgccaat tccatcttca ctggggctcc tcggatgacc atggctctga gcacacagtg 360

gatggagtga agtatgctgc tgagcttcac ctggttcact ggaacccgaa gtataacacc 420

ttcggagagg ctctgaagca gcccgatggg attgctgtgg ttggcatttt tctgaagata 480

ggacgggaga aaggcgagtt ccaaattctc cttgatgccc tggacaaaat taagactaag 540

ggcaaggagg ctccttttaa tcacttcgac ccatcgtgcc tgttccctgc ttgccgggac 600

tattggacct accatggctc cttcaccacg ccaccctgcg aggagtgcat tgtgtggctg 660

ctactgaaag agcccatgac agtgagctca gaccagatgg ccaagctgcg cagcctgttc 720

gccagtgcag agaatgagcc cccggtgcct ctggtgggga attggcgccc tcctcagccg 780

atcaagggca gggtggtgag ggcctccttc aagtaaggct ctggacgtgc cctcttcagg 840

aaaggaattc tgtccctgaa gagccagctt gcctcctcct ggtgctccct gctccaagct 900

atcctacaac actgagagaa agaagagacg cagtcagatg aaaccgcagt gccttttgac 960

atgatctaac ccagaagcat gaatttcaca cctaaccttt ttaataacta ccttttctaa 1020

aaaaattagt atttctagta taaattcagt gaa 1053

<210> 94

<211> 1305

<212> DNA

<213> Rattus norvegicus (Norway rat)

<400> 94
ggagaggaga gaagagagag agattcattt gccttccttg gattgctgag ggaagagaag 60

ttggtcagga gggaaagggg aggaatctga gctatgttca aagaagcctg acatctgtcg 120

aggatcccag ccccaaggtg gagccagtgt gcttatgatg acctgctggg aactggcttc 180

attctgctct gcctgcccgt cctggagcct ggtgaatcct tccctgagtc taacctccgt 240

cctcatgaga ctgttctctc catttctgct tgcaggaaga ttagtgtcac cgcctttccc 300

cctgcctctg ggtgccaagc ctgcagcctg cccgtcagcc ccagctccag ctgatccccc 360

accatccagg tcgcctgcag cctgtaacta cccactgtgt tggttacagc agtcatctat 420

cccgtgcgcc ctgaagccag ctctgtacag tttcgtttct gatctctcca gagcccaagc 480

agagtagacc cctgtccagc ctagtgacct tcgcctgagc gctggttaat atttgaccaa 540

aggcggtggg gctcctcccc ctgggaagat ataagctggt ctggggctac tctgctttct 600

tcttggcctg agctgttccg agctccctgc ccaccagcat catggccaag ggtttctaca 660

tttccaagac cctgggcatc ttgggcatcc tgttaggtgt ggcagccgta tgcaccatca 720

tagctctgtc ggtggtctac gctcaggaga agaacaggaa tgcggagaac tctgccatag 780

cccccacgct cccaggcagc acctcagcca ccacctcaac taccaaccct gctatagatg 840

aaagcaaacc ttggaaccag tatcgcttgc ctaagactct tatacccgac tcctaccagg 900

tgaccttgag gccttacctc acccccaacg agcagggcct gtacatcttc aaaggttcca 960

gtactgtccg ctttacctgc aacgagacca caaatgtcat cattatccac agcaagaagc 1020

tcaactacac caacaaaggg aaccacaggg tggcgttgcg agccctgggt gacactccgg 1080

cacctaacat cgacacaacg gaactggtag agcgcacgga gtacctggtg gtgcacctgc 1140

agggctccct ggtaaagggc catcagtacg agatggacag tgagttccag ggggagctgg 1200

ctgatgatct ggctggcttc taccgcagcg agtacatgga aggtggcaac aagaagtagg 1260

ttgcacgggg ctgcagctgg ggttatgggg agggaggggc tggaa 1305

<210> 95

<211> 546

<212>  DNA

<213>  Rattus norvegicus (Norway rat)

<220>

<221>  misc_feature

<222>  (27)..(27)

<223>  n

<400>  95
ggaattccgc taatagctag actggtncca gtcagacgga ggaaacctgg ccagcttttg      60

cactttctag gtgacgatgg acatacagag gacccaatcc ttgcttctgc tcttgttgct     120

gaccctgctg gggttagggc ttgtacagcc ctcctatggc caagatagaa tgtaccaacg     180

gttccttaga cagcatgtgg accctgaggg tacaggcggc agcgacaact actgcaacgt     240

gatgatgcag agacggagga tgacttctac ccagtgcaaa cgcttcaaca ccttcatcca     300

cgaagacatc tggaacattc gcagcatctg tgatactgcc aatatcccat gcaagaatgg     360

caatatgaac tgtcacgaag gcatagtgag ggtcactgac tgcagagaga cagggagctc     420

tgtgccccac aactgtaggt acagggcgag agccagcact aggcgagttg tcattgcctg     480

tgagggtacc ccagaggtcc cagtgcactt tgacagatag atgacatctg tagctgctac     540

tgctgg                                                                546

<210>  96

<211>  1076

<212>  DNA

<213>  Rattus norvegicus (Norway rat)

<400>  96
cacacagcaa acatgaccca gcagccgcct gacgttgagg aggatgactg tctttctgaa      60

taccaccacc tcttctgtcc ggaccttctc caggacaaag tggcttttat cactggtggt     120

ggttctggga ttggcttccg gatcgccgag attttcatga ggcatggctg ccacactgtc     180

atcgtcagca ggagtctgcc gagagtgtcc gaggctgcta agaagttggt tgctgccact     240

ggaaagcggt gtctccctct gtctatggat gtccgagttc ccccagctgt catggctgct     300

gtggaccaag cgctgaaaga atttggcaaa atcgacatcc tcattaactg tgctgcaggg     360

aactttttat gccctgccag tgcattgtct ttcaatgcct ttaagactgt ggtggacatt     420

gacacccttg gcaccttcaa tgtgtctcgt gtgctttatg agaagttctt ccgggaccat     480

ggaggagtga tcgtgaacat taccgccacc ctcagtatgc gggggcaggt gctgcagctc     540

catgcaggcg ctgccaaggc ggctgtggat gctatgacgc gacacttggc tgtggagtgg     600

ggtccccaga atatccgtgt caacagcctg gctcctggtg ccatcagcgg cactgagggt 660

ctgcggagat taggaggccc caaggccagt tcgaaattta agtatctttc aagtcctatt 720

ccaagactcg gaaccaagac agaaatcgcc cacagcgtgc tgtacctagc cagccctctg 780

gcttcctatg tctcagggat tgtgttggtg gttgatggtg gtagctggat gacgctccca 840

aatgacattg ggcgactgct agagtttgaa tcctcctctg ctaagctgta gtgtttgaag 900

agcacaccca aggcttcaag catgttaaag caacagaatc aactgaacta cgtcctctac 960

cccaagatac cttttttgac acataaacat tgattgcctt aagaaagttg tactgaggag 1020

gccgtgttct tccatgggga ggcttccctg tctcacatag tctatagtca cacgaa 1076

<210> 97

<211> 1272

<212> DNA

<213> Rattus norvegicus (Norway rat)

<400> 97
gaattcgact gctggaacca acgtcctctc ttaccctcca ccttcttctg ccacctctac 60

cacggtcacc atgtcgcaag cccggcctgc cactgtgctg ggtgccatgg agatgggtcg 120

ccgcatggat gtgacctcca gctccgcgtc ggtgcgcgcc ttcctgcagc gcggccacac 180

ggagatagac accgccttcg tgtatgcgaa cggtcagtct gagaccatcc taggagacct 240

ggggctcgga ctgggccgca gcggctgcaa agtaaaaatt gccaccaagg ctgccccaat 300

gtttgggaag acactgaagc cagccgatgt tcggttccag ctggagacgt cactgaagag 360

gctgcagtgt ccccgggtgg acctcttcta tttacacttt ccagaccacg gcactcctat 420

agaggagacc ctgcaggcct gccaccagct gcatcaggag ggcaagtttg tggagcttgg 480

tctgtccaac tatgtctcct gggaagtggc tgagatttgt accctctgca agaaaaatgg 540

ctggatcatg ccaactgtgt accagggcat gtacaacgcc atcaccaggc aggtggagac 600

tgagctcttc ccctgcctca gacacttcgg actaaggttc tacgccttca accctttggc 660

tgggggcctg ctgactggca gatataaata ccaggataag gatgggaaga atcctgagag 720

ccgcttcttt gggaatccat tttctcaact gtacatggac cgctactgga aggaggaaca 780

cttcaatggc atcgccttgg tggagaaggc tctgaagact acctatggcc ccactgcccc 840

cagtatgatc tcagctgccg tacggtggat gtaccatcac tcacagctca agggcaccca 900

aggggatgca gtcattctgg gcatgtccag tctggaacaa ctggagcaga acttggcctt 960

ggtcgaggaa gggcctctgg agccagctgt tgtggatgcc tttgaccaag cctggaacct 1020

agttgcccac gagtgtccca actatttccg ctaagataca tctgccttgg ggatggcgca 1080

gcttactgcc tgccccgcct tgtcctgggc tcgatctgat ctggttcttt cctttttaga 1140

caggtcactg tctttttctt ccctgctttc tatacagcca gttgctttca aagtgagagc 1200

tggctgagcc ccaatacctc ctgctgaata aaactgttcc ctgtcacagc ctgggctaca 1260

actggcggcc ga 1272

<210> 98

<211> 459

<212> DNA

<213> Rattus norvegicus (Norway rat)

<400> 98
ttcattccca gcatgatttc caaccttcct aacagaccac gttctttccc agaaaggata 60

gggttttagg catagatgag tacacagcac acatgtgagt tacataagca ggctgaactc 120

tagcctttct gttcgtttga tttcattctt ctaaaagtgc tgcttacagc aggaataggt 180

tacctcatgg cttcaagaac tcaacatcag accacagagt agcttcacag ggacccaaag 240

gagcagaact tgtagtgaga aaaaaccaaa acaacaacaa caaaatccct aagaactggg 300

tatctgccat ccaacacaca tggttttaaa acaattttta aatttaaacg tatttttata 360

tttctttctt tccccatccc tacccaccca tctttaagtt ctttctcaaa aaacaaataa 420

tctaatacaa caaaaccccc caaaaaccaa aaaaaaaaa 459

<210> 99

<211> 1416

<212> DNA

<213> Rattus norvegicus (Norway rat)

<400> 99
atgtggaaag cctctgcagg ccatgctgtg tccatcaccc aggatgatgg ggaagccgat 60

gactgggaga ctgaccctga ttttgtgaac gatgtgagtg aaaaggaaca gagatggggc 120

gccaaaaccg tgcagggatc ggggcaccag gaacacatca acatacacaa gcttcgagag 180

aatgtcttcc aagaacacca gacactcaag gagaatgagc tggaaacggg acccaaggct 240

tcccatggct atggcgggaa gtttggtgtg gagcaagata ggatggacaa atcagctgtg 300

ggccatgagt accagtcaaa gctctccaag cactgctcac aagtggactc agtccggggc 360

ttcggaggca agttcggtgt ccagatggac agggtggatc agtctgctgt aggctttgaa 420

taccagggga agacggagaa gcacgcctcc cagaaagact actccagtgg tttcggtggc 480

aaatatggag tgcaagctga ccgtgtagac aagagtgctg tggggtttga ctaccagggc 540

aagacggaga agcacgagtc acagaaagat tactccaaag gttttggtgg caaatacgga 600

attgacaagg acaaggtgga taaaagcgct gtgggctttg agtatcaagg caaaacagag 660

aagcatgagt cccagaaaga ctatgtaaaa ggctttggag gaaagtttgg tgtgcagaag 720

gatcggatgg acaagaacgc atccaccttt gaagaagtgg tccaggtgcc gtctgcctat 780

cagaagactg tccccattga ggctgtgacc agcaaaacca gtaatattcg tgctaacttt 840

gaaagcctgg caaaggagag agagcaggag gacagacgga aggcagaagc cgagagagct 900

cagcggatgg cccaagaaag gcaggagcag gaggctcgaa ggaagctgga agagcaagcc 960

agagccaaga agcagacacc ccctgcatcc cctagtcctc agccagctga agacagacca 1020

ccatccagcc ccatctatga ggatacagct ccgttgaagg ccgagcccag ctacggaagt 1080

agtgagcctg agcctgagta cagcactgag gctgcaggcc ttcctgaggc cagcaaccag 1140

caaggtctgg cctacacatc agagcctgtt tacgagacca cagaggttcc tggccactac 1200

caagcagagg atgacaccta cgatgggtat gagagtgacc tgggcatcac agccatcgcc 1260

ctgtatgact accaggctgc tggcgatgat gaaatctcct ttgaccctga tgatgtcatc 1320

accaacatag aaatgattga cgatggctgg tggcgtgggg tgtgcaaagg cagatatggg 1380

ctcttcccgg ccaactatgt ggagctgcgg cagtag 1416

<210> 100

<211> 816

<212> DNA

<213> Rattus norvegicus (Norway rat)

<400> 100
cggcacgagc ttgagtttcc gatgagacac aagactgaag aggaaaactg atctccccca 60

aagacataga ataattggta aatctggacg agaacacagg ctcataactg cgaagagcaa 120

ggatttttat attactctta taggaaagag accgaaagat ctttacaagc tatcttgcta 180

ggagatgccc aaagtcaaaa gaagccggaa agcacctcca gatggctggg agctgatcga 240

gccaacgttg gatgaactag atcaaaagat gagagaagct gaaactgaac cccatgaggg 300

gaagaggaag gtggagtctc tgtggcccat cttcaggatc catcaccaga aaacccgcta 360

catcttcgac ctcttttaca agaggaaagc cataagcaga gaactctatg aatactgcat 420

taaagaaggc tatgcagaca aaaacctgat tgcgaagtgg aagaagcagg gctatgagaa 480

tctatgctgt ctacgctgca tccagactcg agacaccaac ttcggcacca actgcatttg 540

ccgggttccc aagagcaagc tggaagtggg tcgcatcatc gattgcacgc actgtggctg 600

ccggggttgg tctggttgac tgacgcccct gactcctctg catcttggac tttggacctt 660

gggctcctgc ttattgggcc actgcctcct gaagcaactc ttctcagatt ggcactcaga 720

gaaaagaccc aggggaagca tgagctctac aggtgacagc tttagtgttc accagctgag 780

atctgtaaaa ataaaacctt tagacctctt aaaaaa 816

<210> 101

<211>  3559

<212>  DNA

<213>  Rattus norvegicus (Norway rat)

<220>

<221>  misc_feature

<222>  (11)..(11)

<223>  n

<220>

<221>  misc_feature

<222>  (16)..(16)

<223>  n

<220>

<221>  misc_feature

<222>  (3298)..(3298)

<223>  n

<220>

<221>  misc_feature

<222>  (3362)..(3362)

<223>  n

<220>

<221>  misc_feature

<222>  (3412)..(3412)

<223>  n

<220>

<221>  misc_feature

<222>  (3480)..(3480)

<223>  n

<400> 101
ccatctaagc ntaaanactg caaagcactc acatcgctca gcaatgccgg gcccgctagg 60
gctgctctgc ttcctcgccc tggggctgct cggctcggcc gggcccagcg gcgcggcgcc 120
gcctctctgc gcggcgccct gcagctgcga cggcgaccgt cgggtggact gctccggaaa 180
ggggttgacg gccgtaccgg agggtctcag cgccttcacc caagcactgg atatcagtat 240
gaacaatatc acccagttac cagaagatgc atttaagagt ttcccatttc tagaggagct 300
acaactggct ggtaacgacc tttctcttat ccatccaaaa gccttgtctg ggctgaaaga 360
actcaaagtc ctaacactcc agaataatca gttgagaaca gtgcccagtg aagccattca 420
cggactgagt gctttgcagt ctttacgctt agatgccaac catattacct cagtcccgga 480
ggacagtttt gaagggcttg tccagttacg ccatctgtgg ctggatgaca acagcttgac 540
ggaagtgccc gtgcgtcccc tcagcaacct gccaaccctg caggcgctga ccttggctct 600
caacaacatc tcaagcatcc ctgacttcgc tttcaccaac ctttcaagct tggtggttct 660
gcatctgcat aacaataaaa ttaaaagcct cagtcaacac tgttttgatg gactagataa 720
cctggaaacc ttggacttga attacaatta cttggatgag tttcctcagg ctattaaagc 780
ccttcccagc cttaaagagc tgggatttca cagtaattct atttctgtta ttcctgatgg 840
agcatttggt ggtaatccac tgctaagaac tattcatttg tatgataatc ctctgtcttt 900
tgtggggaac tcagcatttc acaacctgtc tgatctgcat tgcttagtca ttcgtggtgc 960
aagcctggtg cagtggttcc ccaatctgac cggaactgtc catttggaga gtctaacctt 1020
gacagggaca aaaataagca gcatacctga tgatctgtgc caaaaccaaa agatgctgag 1080
gactctggac ttatcttata acaatataag agaccttcca agttttaatg gttgtcgtgc 1140
attggaagaa atttcattgc agcgtaatca aatctcccta ataaaggaaa atacttttca 1200
aggcctaaca tctctaagga ttctagatct gagtagaaac ctgatccgtg aaattcacag 1260
tggagctttt gcgaagcttg ggacaattac taacctggat gtaagtttca atgaattaac 1320
ttcatttcct acggaaggcc taaatgggct caatcaacta aagcttgtgg gtaacttcaa 1380
gctgaaagac gccttggcag ccagagactt tgctaatctc aggtctctat cagtaccata 1440
tgcttatcag tgttgtgcat tttgggggtg tgactcttat gcaaatttaa acacagaaga 1500
taacagcccc caagaacaca gtgtgacaaa agagaaaggt gctacagatg cagcaaatgt 1560
caccagcact gctgagaacg aagaacatag ccaaataatt atccactgta caccttcaac 1620
aggtgctttc aagccctgtg aatatttact gggaagctgg atgattcgcc ttacagtgtg 1680
gttcattttc ctggtcgcct tgcttttcaa cctgcttgtc attttaacag tgtttgcgtc 1740
ttgttcatca ctgcctgcct ccaaactctt cataggcttg atttctgtgt ctaacttact 1800
catgggcatc tatactggca tccttacttt tcttgatgct gtgtcctggg gccgatttgc 1860
cgaatttggc atttggtggg aaactggcag cggctgcaag gtagccgggt ctctggcagt 1920
cttctcctca gagagcgctg tattcctatt aacactggca gctgtggaaa gaagcgtatt 1980
tgcaaaggat ttgatgaaac acgggaagag cagtcacctc agacagttcc aggtggccgc 2040

cctcttagct ttgctgggtg ccgcagtggc aggctgcttc ccccttttcc acggagggca 2100

atattctgca tcgcccttgt gtttgccgtt tcctacagga gaaaccccat cgttaggatt 2160

cactgtgacc ttagtgctat taaactcact ggcattttta ctaatggcca ttatctacac 2220

taaactatac tgcaacttag agaaggagga cctgtcggaa aactcccagt ctagcgtgat 2280

taagcacgtt gcctggctca tcttcacaaa ctgcatcttc ttctgccctg ttgcattttt 2340

ctcatttgca ccattgatca cggcaatctc catcagcccc gagataatga agtctgttac 2400

actgatattc ttcccgttgc ctgcttgcct gaatccggtc ctgtatgttt tcttcaaccc 2460

aaagtttaaa gaagactgga agctactgaa gcggcgtgtt accaggaaac acggatctgt 2520

ttcagtttcc atcagcagcc aaggcggttg tggggaacag gatttctact atgactgtgg 2580

catgtattcc cacttgcagg gtaacctgac tgtctgtgac tgctgtgagt catttctttt 2640

gacaaaacca gtatcatgca aacacttaat aaaatcgcac agttgtcctg tattgacagc 2700

ggcctcttgc cagaggccag aggcctactg gtctgattgt ggtacacagt cagcccattc 2760

tgactatgca gatgaagaag attcctttgt ctcagacagc tctgaccagg tgcaggcctg 2820

tggacgagcc tgcttctacc agagtcgtgg attccctctg gtgcgctatg cttacaatct 2880

acagagagtc agagactgaa ctccagtgtc tgcctgcgac tgtttttccc catcaaccaa 2940

atcacagtgc tggcatggga gctatctgtc tttagacctt tcatctggaa agcacttgta 3000

gaatcacttc ctggtgtcat gcagaagagg aggaaggtgg cagtttattt ctcagatcag 3060

acatctgtga agaatgggtg cctaaactat aaattgttga aaaaaaattg tgatgcccca 3120

gtaatgtgtg ctctattcta aataaatgca acttgaaaat ggtttgatgt atggcatagc 3180

aatataaaat tagggtttta ttttttttct ttctgatcca tcaggtgcaa agttcaaact 3240

atttctgaat taagcacaaa ataaaggaca gctgttaata tattttaaat gaaaatgnga 3300

aattgtaaaa ctaagaaaat gccttgctaa tttcacctaa tgattcatca ttaatctcag 3360

gntgactgac ttcagggcca caaaagagac tgtctcccag ctagagttat gnagagttca 3420

aacaggcatc cccttcatgt gttttctgtg tccataaatt aaaggttctg ttcaaatgan 3480

ttgtaaatct aaaacctgaa gatgttttaa ggactattga cagctgttag attttgggta 3540

ccgagctcga attcgtaat 3559

<210> 102

<211> 1607

<212> DNA

<213> Rattus norvegicus (Norway rat)

<220>

<221> misc_feature

<222>  (1094)..(1094)

<223>  n


<400>  102
catgctgaaa tatcgcggtt taccatatgg ggatggtggc gacaatctcc tggagcccgt      60

cagatcgcgg aaattaccga tgcctgcgca gctccttccg ctcagctccg cgtccgagcc     120

tcctggaacg atatttggag ttcttaaaag atggcagaca ttgacaacaa agaacagtct     180

gaacttgatc aagatttgga agatgttgaa gaagtagaag aagaagaaac gggtgaagaa     240

acaaaaatca aagcacgtca gctaactgtt cagatgatgc aaaatcctca gattcttgca     300

gctcttcagg aaagacttga tggtctggta gacacaccaa caggatacat tgaaagcttg     360

cctaaggtag tcaaaagacg ggtgaatgct ctcaagaatc ttcaagttaa atgtgcacag     420

atagaagcca aattctatga ggaagttcat gaccttgaga gaaagtatgc tgttctctat     480

cagcctctgt ttgataagcg atttgagatc attaatgcaa tttatgaacc tacagaagaa     540

gaatgtgaat ggaaaccaga tgaggaagat gaagtttcgg aggagctgaa agaaaaggcc     600

aagattgaag atgagaaaaa ggatgaagaa aaagaagacc ctaaaggaat tcctgagttt     660

tggttgacag tttttaagaa tgatttgctc agtgatatgg ttcaggaaca tgacgaacct     720

attctgaagc acttgaaaga tattaaagtg aagttttcgg acgctggcca gcctatgagt     780

tttatcttag aatttcactt tgaacccaac gaatatttca caaatgaagt gttaacaaag     840

acttacagga tgaggtcaga accagatgat tctgatccct tttcttttga tggaccagaa     900

attatgggtt gtacagggtg ccagatagat tggaaaaaag gaaagaatgt tactttgaaa     960

accattaaga agaagcagaa acacaagggc cgtgggacag ttcgtactgt gactaaaaca    1020

gtttccaaga cttctttctt taactttttt gctcctcctg aagttcctga gaatggagat    1080

ctggatgacg atgntgaggc aatactggct gcagactttg aaattggtca ctttttacgt    1140

gagcgtataa tcccaagatc agtgttatac ttcactggag aagctattga ggacgatgac    1200

gatgactatg atgaagaagg tgaagaagct gatgaggaag gggaagaaga aggagatgag    1260

gaaaacgatc cagactatga cccaaagaag gatcaaaacc cagccgagtg caagcagcag    1320

tgagcagtga ctggccttga ggacggcctc cctgtaatag cctaaacatg actcacttac    1380

ttacagcctt atggttttgt attttcttga tagaatcagt aagtttctaa gggaaaggaa    1440

attgatattt tgcagaccaa tttgttctaa ccagcatccc aactctagct ctgtagccac    1500

gttaccgagt ccagcccttt actgcatgct caggtcgctg cagtctggtt ctcctgagag    1560

atttcatcat gtagctattg gtacattatg aaaccactgt gaacaat                  1607

<210>  103

<211>  1750

<212>  DNA

<213>  Rattus norvegicus (Norway rat)

<400>  103
tggtgaggag aggacgagga tactagaatg tcaagtactg cgtcctaccg gatcttcaat     60

tttgcaagga atcgtattgc tgtattacga gggggtagac gcttgtactc cagggcagcc    120

acgagtagga atcaagtgaa atggaggctc ttttctcccg catcattggc cgtgaacgac    180

agttctccac atggtggctt tgctctgcgg aaagcttaca gacacacatc aaccgaggaa    240

gaggatttcc acttgcagct cagccccgaa caggtcagtg acctgctccg agctggcgag    300

tcatcccaca aggttctgga tttcaacagc ggagttccaa attcagtgtt aaggtttgag    360

agcaaccagt tggcggccaa ttccccagtg gaagaccgac aaggtgtggc ttcctgcgtg    420

cagacgaggg ggacggtgtt tggtattttt gatgggcatg gtggccatgc gtgtgcacag    480

gcggtgagcg agaggctttt ctattacatg gcggtgtccc tcatgtccca taagaccttg    540

gagcagatgg aggaagccat ggagaacatg aagcctttgc tgcccatcct gcagtggctc    600

aagcaccccg gagacagtat ttataaagat atcacatcgg ttcatctgga ccacctccga    660

gtctattggc aggaactcct tgacctacat atggaaacgg gactaagcac tgaagaggca    720

ttgatgtact ccttccagag actggactcc gacatctcgc tagaaattca ggctcctctg    780

gaagatgagg taacgaagaa cttatccctc caggttgctt tttctggggc gacagcctgc    840

atggcccacg tcgacggagt tcacctgcac atagcaaatg ctggcgactg ccgagctatc    900

ctgggtgtcc agggagacaa tggagcgtgg tcatgtttac ctcttacctg tgaccacaat    960

gcctggaatg aggcagagct gtcccggctt aagagagaac accccgagtc tgaggacagg   1020

acactcatca tagatgacag gctgctgggt gtcctcctac cctgcagggc cttcggggac   1080

gtccagctaa aatggagtaa agagttgcag cggaacgtcc tagagagggg atttgatacc   1140

gaggccctca acatctacca gtttaccccg ccccactacc acactccacc ctacctgact   1200

gctaagcctg aggtcacata ccacaggctt aggccccaag ataagttcct cgtgctggcc   1260

tcagatggcc tctgggacat gctggacaat gaggatgtgg taaggctggt ggtggggcac   1320

ctgtccaagg tcggtcacca gaagccagcc ctggatcaaa gaccagccaa cctgggccac   1380

atgcagagtc tgctgctgca gaggaaagcc agcggactcc atgcagctga ccagaatgca   1440

gccacacatc tgatcagaca tgccatcggg agcaacgagt atggggaaat ggagccagag   1500

cgtctggctg ccatgttgac gctgccagag gatgtggcca ggatgtaccg ggatgacatc   1560

actgtcatgg tggtgttttt taactcagaa tcaattgata cctattttaa ggagggttaa   1620

gagtctcacc ctgttgccaa ggctaactcg aatgcccttt atttagaaca tttccactca   1680

ttccttagtg agcaatccaa accttagtaa agaacaggca gacataggtt gctgagtaat   1740

tccggaattc                                                          1750

<210>  104

<211>  1511

<212>  DNA

<213>  Rattus norvegicus (Norway rat)

<400>  104
tgggctcaag gaaggaagcg ttgtagctcg cgtccagggg cgcggcgtgt acgggtggct     60

ctcttcgcag ctcgcggagg cgaaccgggc aacagtgaca tggcagaaca ggatgtggaa    120

aatgagcttt tggattatga tgaagatgaa gagccccagg taccccagga gagcactcca    180

gctcccccga agaaagatgt caaaggatct tatgtctcca tccacagttc tggcttccgg    240

gactttctgc tgaagccgga gctcctgaga gctatagttg actgtggctt tgaacatcct    300

tcagaggtcc agcatgaatg tattccccag gccattctgg gtatggatgt cctgtgccaa    360

gccaagtctg ggatgggcaa gacagctgtg tttgtgctgg ccaccctgca gcagattgaa    420

cccatcaatg gccaggtatc agtactggtc atgtgccaca caagggagct ggccttccag    480

atcagcacgg agtatgagcg cttctcgaag tacatgccca gtgtcaaggt atctgtgttc    540

tttggaggcc tctccattaa gaaagatgaa gatgtgttaa agaagaactg tccccatgtt    600

gtggtgggga caccaggccg gatcctggcc ctcgtgcgga gcaggagcct caacctgagg    660

aatgtgaagc actttgtgct agatgaatgt gacaagatgc tggaacagct ggacatgcgc    720

cgggatgtac aggagatctt tcgtctgaca ccccatgaga agcaatgtat gatgttcagc    780

gccaccctga gcaaggagat ccggccagtc tgcaggaagt tcatgcagga tcctatggag    840

gtgtttgtgg acgacgagac caagctcaca ctgcatgggc tgcagcagta ttacgtcaaa    900

ctcaaggaca gtgagaagaa tcgtaaactc ttcgacctcc ttgacgtgct agagtttaac    960

caggtggtga tctttgtcaa gtctgtgcag cgctgcatgg ccctggccca gctcctagtg   1020

gaacagaatt ttccggctat cgctattcac agaggcatgg cccaggagga gcgcctgtcc   1080

cgataccagc agttcaagga cttccagcgt cgcatcctag tggctactaa tctgtttggc   1140

agaggcatgg acattgagag agtcaacatc gtcttcaact atgacatgcc agaggactcg   1200

gatacctacc tgcaccgagt ggctcgtgct ggtcgctttg gtaccaaggg tctggcagtc   1260

acttttgtgt cagatgagaa tgatgccaaa atccttaatg acgttcagga ccggtttgaa   1320

gtgaatgtgg ctgagcttcc agaagaaata gatatctcca catacattga gcagagccgg   1380

taaccatgtg tgtagccagg cacatggctt tctctcctgc tgcttcagat cctcctccta   1440

ggtggcaatc ggcggcctct ctttttattg ttccaaagct ttagctatgt taagaataaa   1500

cttttattgt g                                                        1511

<210>  105

<211>  3106

<212>  DNA

<213>  Rattus norvegicus (Norway rat)

<400>  105
ctcactatag ggctcgagcg gccgcccggg caggtctgag gtagaggcac tgcctggact     60

tgcagaacct cgattgggtc catatcatgc tgacttttcc ttgctaggaa tctcccagga    120

gcttctggaa aatctaggac gaagactaag gtacaggagc cctaagtccc catggcttca    180

ctgagccggg tcctccgtgt agctgccacc tgtcctcgag ggagatctgc tcgcaacctg    240

gggctacagc ccctagccag ggaggccagg cccaccactg agaagagtgt tccataccag    300

tgcaccctga aggaggaggc ccaaggtgcc tctgtggtgc cccaaggacc aagccagccc    360

ctgcctagca cagccaatgt tgtggtcatt ggtgggggca gtttgggctg ccagactctg    420

taccacttgg ccaagctggg tgtaggtgga gttgtgctgc tggagagaga gcgactgacc    480

tctgggacca cctggcacac agcaggccta ctgtggcagc tgcggcccag cgatgtggaa    540

gtagagcttc tggctcacac acggcaagtg gtgagccatg atctggaaga ggagactggg    600

cttcacacag gctggatcca gaatggtggc ctcttcatcg cctccaacca gcagcgcctg    660

gatgaataca agaggctcat gtcgttgggc aaggcctacg gtatagaatc ccatgtgctg    720

agccctgcag agaccaagga tctgtatccg ctgatgaatg tggacgacct ctatgggacc    780

ctgtatgtgc ctcgggatgg taccatggac ccggctggca cctgcactac cctcaccaga    840

gcagcagtgg ctaggggagc tcaggtcatt gaaaactgtg cagtgactgg catccgtgtg    900

cggacagacg actttggagt gcggcgggtg acagctgtgg agactcaaca tggctccatc    960

cagacaccct gcgtggtcaa ctgtgcaggg gtgtgggcga gctccgtggg ccggatggct   1020

ggagtgaagg tcccgctggt ggccatgcac catgcctacg ttgtcactga gcgcattgag   1080

gggatccaga acatgcccaa tgtccgggat catgatgctt ctgtctacct ccgcctgcag   1140

ggggatgcct tgtctgtggg tggctatgag gccaacccca tcttttggga tgaggtgtca   1200

gacaaatttg cctttggcct ctttgacctg gactgggatg tgttcactca gcacattgaa   1260

ggtgccatta accgtgtccc cgtgttggag aaaacgggga tcaagtccac tgtctgtggc   1320

cctgaatcct tcacacccga ccataagccc ctgatggggg aggctcctga gctccgaggc   1380

ttcttccttg gctgtggctt caacagtgca ggcatgatgt tgggtggtgg ctgtggacag   1440

gagctggccc attggattgt gcatgggcgc ccagagaagg acatgtacag ctatgacatc   1500

aggcgtttcc accactcgct cacggaccac ccacgctgga tccgagagcg cagccacgag   1560

tcctatgcca agaactactc ggttgtcttt cctcacgatg agcccctggc gggccgcaac   1620

atgaggaggg accctctgca cgaggagctg cttggacaag gctgtgtgtt tcaagaacga   1680

cagggctggg aacggccagg atggttcaac ccccaagaaa cagctcaggt cctagactac   1740

gactactatg gggcctacgg acaccaggca cacaaggact acgcctacag caggctgctg   1800

ggcgatgaat ataccttcga cttcccaccc caccactgcg tgatccagaa ggaatgtctg   1860

gcctgcagaa cggctgccgc cgtgttcaac atgtcttact tcgggaagtt ctaccttctg   1920

ggggcagacg ccaggaaggc tccagactgg ctcttctcag cagatgtcaa ccgaccccca 1980

ggctccacgg tgtacacatg catgctgaac caacggggag gcaccgagag cgacctgact 2040

gttagctgcc tggcccctgg tgcccaggcc agtcccctgg cccctgcctt tgaaggggat 2100

ggctactacc tagccgtagg tggggctgtg gcccaacata actggtccca cataaacacg 2160

gtgctgcagg atcaggagtt ccggtgccag cttatggatt gttctgaaga tctgggcatg 2220

ctcagtatcc agggcccagc cagccgtgac attctccagg atgtgcttga tgctgacctg 2280

agcaatgaag ccttcccctt ctccacccac cagctggtga gagctgctgg acacctggtc 2340

cgagccatcc ggctatcctt tgtcggggag ctgggatggg agctgcatgt gccccaagcg 2400

tcctgcttgc ctgtgtaccg ggctgtgatg gctgcagggg cgaagcacgg cctagtcaat 2460

gcaggctacc gtgccatcga ctccctgagc attgagaaag gctatcggca ctggcatgca 2520

gacctgaggt ccgatgacag ccccctggag gcagggttgg ccttcacctg caagctcaag 2580

acctcagtgc ccttcctagg ccgagaggcc ctggagaagc agcgtgccac aggcctccga 2640

cgacgcctgg tgtgcctcac tgtggaagag gaggttccta tgttcggcct agaggccatc 2700

tggagaaatg gccaagtggt gggccatgtc cggagagctg actttggatt caccgtgaac 2760

aagaccatcg cctatggcta catccgagac cccagtggag ggccagtctc tctggacttt 2820

gtgaagaatg ggactatgcc cttggagaga atgggggtca cctatgctgc ccaggttcac 2880

ctgaagtctc cctttgatcc tgacaacaag cgggtgaagg gaatctattg atgggctgca 2940

gggtggaggc tacagctgct caggcccctg gaaactgcca cacttcccat acggcctgtg 3000

gactcctata ctgtacaaac agccctgtgt cctgagaccc aagataaggg caaaacacag 3060

acttcccacc ctgagcctcc atctggtcct gctaactgcc acagtc 3106

<210> 106

<211> 1552

<212> DNA

<213> Rattus sp.

<400> 106
ggcacgaggg aacgtctagg caacgtggtc tccccgcccg cgggtaggca aaggcgtttg 60

cgcttcccag cgtctgaggc ctagggagcc ttcagtagcc gaaagttagt cttttgcagt 120

ggagtaaggg ctcgcggtta gccgcgtagc gcccggatct ggcctcacca tgttggtcct 180

atttgaaacg tccgttggct acgccatctt taaggttctg aatgagaaga aacttcaaga 240

ggttgatagt ttgtggaaag aatttgaaac tccagagaaa gcaaataaaa tagtaaagct 300

aaaacatttt gagaaatttc aggatacagc agaagcatta gcagcgttca cagctctgat 360

ggaaggcaag atcaataagc agctgaaaaa agttttgaag aaaatagtca aagaagccca 420

tgaacctctg gctgtagctg atgctaagct aggaggggtc ataaaggaaa aattgaatct 480

cagctgtatc catagtcctg ttgttaatga acttatgaga ggaatacgat cacaaatgga 540

tggcttgatt cctggggtag aaccacggga gatggcagcc atgtgtcttg gactagccca 600

cagcctatct cgatacagat tgaaattcag tgctgataaa gtagacacaa tgatcgttca 660

ggcaatttcc ttgttagatg acttggataa agaactaaac aactacatta tgcggtgtag 720

ggaatggtat ggctggcatt ttcctgagtt agggaaaatt atttcagata atttgacata 780

ctgcaagtgt ttacagaaag ttggagacag gaagaactat gcatctgcca ctctttctga 840

attcctgtca gaggaagtag aagctgaagt gaaagcagct gcagagatct ctatgggaac 900

agaggtttct gaagaagata tttgcaacat tctacatctg tgtactcagg tcattgaaat 960

ttctgaatat agaactcagc tgtatgaata tctgcaaaac cgaatgatgg ccattgcacc 1020

caatgttaca gtcatggttg gggagttggt tggagcgcgg cttattgctc atgcaggttc 1080

tcttttgaat ttggccaagc atgcagcttc tacagttcag attctgggag cagaaaaggc 1140

acttttcagg gccctcaaat ctagacgaga cacacctaaa tatggcttta tttatcatgc 1200

ttctcttgta ggccagacga gtcccaaaca caaaggaaag atttcacgaa tgctggcggc 1260

caaaactgtg ttggctatca gatacgacgc ctttggtgaa gattccagct ctgcaatggg 1320

agctgagaac agagccaaat tagaggccag attgaagatt ttggaggaca gagggataag 1380

aaaaataagt ggaacgggaa aggcattagc aaaagcagaa aagtatgaac acaaaagtga 1440

agtgaagact tacgatccct ctggtgactc cacacttcca acttgttcta aaaaacgcaa 1500

aatagaagag gttgataaag aggatgaaat tactgaaaaa aaaaaaaaaa aa 1552

<210> 107

<211> 1068

<212> DNA

<213> Rattus sp.

<400> 107
   gtgtcctcat cagggtcaca aacctgtgag gaaaccctga agacttgctc tgtgatagcc 60
   tgcggcagag acgggagaga tgggcccaaa ggggagaagg gagaaccagg tcaagggctc 120
   aggggcttgc agggccctcc agggaaactg gggcctccag gaagtgtagg agcccctgga 180
   agtcaaggac caaaaggcca aaaaggggat cgtggagaca gcagagccat tgaggtgaag 240
   ctggcaaata tggaggcaga gataaacacc ctgaagtcaa aactggagct aaccaacaag 300
   ttgcatgcct tctccatggg taaaaagtct gggaagaagt tctttgtgac caaccatgaa 360
   aggatgccct tttccaaagt caaggccctg tgctcagagc tccgaggcac tgtggctatc 420
   cccaggaatg ctgaggagaa caaggccatc caagaagtgg ctaaaacctc tgccttccta 480
   ggcatcacgg acgaggtgac tgaaggccaa ttcatgtatg tgacaggggg gaggctcacc 540
   tacagcaact ggaaaaagga tgagcccaat gaccatggct ctggggaaga ctgtgtcact 600
   atagtagaca acggtctgtg gaatgacatc tcctgccaag cttcccacac ggctgtctgc 660
   gagttcccag cctgaggaaa ccagtgcctc catcgtctcc ttggctctca gtcgcttcca 720
   aagaaaattc agttactggt ttctcaagtt tagtgttaag tgattctttt gatgggagag 780
   aatgtatttg cttgtggcat gaggacacga atagaagctg accgggaggc accaggatct 840
   ggttgagcac ggagcaaagg tcacatccat tttgctagga acacagcaag aagtcaacta 900
   tggaaaacct acaataaata tcccctgccc ttttcaccag aggaccaaag gtggtcctta 960
   tctgtgccag agtggcagct gatctcagcc ataaaagcac ccaattccct ttctccatga 1020
   attgtcacta agtggtgtca aacgtgcctg tttgaagtca tcctcagt 1068

<210> 108

<211> 542

<212> DNA

<213> Rattus norvegicus (rat)

<400> 108
tggctaaaac ctctgccttc ctaggcatca cggacgaggt gactgaaggc caattcatgt 60

atgtgacagg ggggaggctc acctacagca actggaaaaa ggatgagccc aatgaccatg 120

gctctgggga agactgtgtc actatagtag acaacggtct gtggaatgac atctcctgcc 180

aagcttccca cacggctgtc tgcgagttcc cagcctgagg aaaccagtgc ctccatcgtc 240

tccttggctc tcagtcgctt ccaaagaaaa ttcagttact ggtttctcaa gtttagtgtt 300

aagtgattct tttgatggga gagaatgtat ttgcttgtgg catgaggaca cgaatagaag 360

ctgaccggga ggcaccagga tctggttgag cacggagcaa aggtcacatc cattttgcta 420

ggaacacagc aagaagtcaa ctatggaaaa cctacaataa atatcccctg cccttttcac 480

cagaggacca aaggtggtcc ttatctgtgc cagagtggca gctgatctca gccataaaag 540

ca 542

<210> 109

<211> 2479

<212> DNA

<213> Rattus norvegicus (Norway rat)

<400> 109
caggtggagc gggcagggcg cggggcacca tgagcagcgg cgccaacatc acctatgcca 60

gccgcaagcg gcgcaagccg gtgcagaaaa cagtaaagcc cgtccctgct gaaggaatta 120

agtcaaaccc ttctaaacga cacagagacc ggctgaacac agagttagac cgcctggcta 180

gcctgctgcc cttcccacaa gatgttatta ataagctgga caaactctcc gttctaaggc 240

tcagcgtcag ctacctgagg gccaagagct tctttgatgt tgcattaaaa tccaccccgg 300
ctgacagaag tagaggccag gaccagtgta gagcacaagt cagagactgg caggacttgc 360
aagaaggaga gttcttgtta caggcgctga atggctttgt tctggttgtc acggcagatg 420
ccttggtctt ctatgcgtct tccactatcc aagattacct gggctttcag caatctgatg 480
tcatacatca gagcgtgtat gagcttatcc atacagaaga ccgagctgag ttccagcgcc 540
agcttcactg ggctctaaac ccctcacagt gcacagactc tgcacaagga gtagacgaga 600
ctcatggcct cccacagcca gcggtctact acacgccaga ccagcttcct ccagagaata 660
ccgctttcat ggagaggtgc ttcagatgcc ggctgaggtg cctgctggat aattcatctg 720
gtttcctggc aatgaatttc caagggaggt taaagtatct tcatggacag aacaagaaag 780
ggaaagacgg agcgctactc cctccacagt tggctttgtt tgcaatagct actccacttc 840
agccaccgtc catcctggaa attcgaacca aaaacttcat cttcaggacc aaacacaaac 900
tggacttcac acctattggc tgtgatgcca aagggcagct tattctgggc tacacagaag 960
tagagctgtg caacaaagga tcgggatatc agtttatcca cgccgctgac atgcttcact 1020
gcgcagaatc ccacatccgc atgattaaga ctggagaaag tggcatgaca gttttccggc 1080
ttcttgcaaa acacagtcga tggaggtggg tccagtccaa tgcacgcttg atttacagaa 1140
atggaagacc agattacatc atcgcaactc agagaccgct aacggatgaa gaaggacgcg 1200
aacatttaca gaagagaagt atgacactgc cattcatgtt tgccactgga gaggctgtac 1260
tgtacgagat ctccagccct ttctctccca taatggatcc cttgccaata cgcaccaaaa 1320
gcaacactag taggaaagac tgggctcccc agtcaacccc gagtaaggat tctttccacc 1380
ccaattccct tatgagtgcc ttgatccaac aggacgagtc catctatctc tgtcctcctt 1440
cgagccccgc accattagac agccattttc tcatggactc catgagtgag tgcggcagtt 1500
ggcaaggcag ctttgcagcc gcaagcaatg aagctctgct gaaacacgag gaaatcagac 1560
acactcagga cgtgaacctt acactctctg gaggcccctc ggagctcttc ccagataata 1620
aaaataatga cttgtatagc atcatgagaa acctagggat cgatttcgaa gacatcagaa 1680
gcatgcagaa tgaggagttc ttccgaaccg actcctccgg tgaggttgac ttcaaagaca 1740
tcgacataac agacgaaatc ctgacgtacg tgcaggattc tctgaacaat tcaactctgc 1800
tgaattcagc ttgccagcaa cagcctgtga gccagcacct aagctgcatg ctgcaggagc 1860
gcctgcagct ggagcaacaa cagcagcttc agcagcagca ccccactcag acactggagc 1920
cccagcgcca gttgtgtcag gtggaggtcc cccagcacga gctgggtcag aaaacgaagc 1980
acatgcaagt caatggcatg ttcgccagtt ggaaccctgc ccctcccgtg tctttcagct 2040
gtcctcagca ggaacgaaag cactatagcc tcttctccgg cttacagggg actgcacagg 2100
agtttcccta caagtcagag gtggacagta tgccttacac acagaacttt gctccctgca 2160
accagtcact gctaccagaa cattccaagg gtacacagtt ggacttccct ggaagggatt 2220
ttgaacgatc cctgcaccct aacgcttcta atttagaaga ctttgtcagt tgtttacaag 2280

ttcctgaaaa ccaaagacac gggataaact cacagtcagc catgtttcag agtccgagca 2340

ttttaaatga agcctactcg gcagacttga gcagcattgg ccaccttcag actgctgctc 2400

acctccctcg cctggcagaa gcccagcctc ttcctgatat cacacccagc ggattcctgt 2460

agctcgaggc caggatgaa 2479

<210> 110

<211> 7420

<212> DNA

<213> Rattus norvegicus (Norway rat)

<400> 110
atggatccaa gtggggtcaa agtgctggaa acagccgagg acatccagga gagacgacag 60

caggttctgg atcggtacca ccgcttcaag gagctctcta ccttgcggcg gcagaaactg 120

gaggattcct atcgcttcca gttttttcag agagatgctg aggagctgga gaagtggatt 180

caggagaagc ttcaagttgc ctctgatgag aactacaaag accccaccaa cttgcaggga 240

aagctccaga aacaccaagc ctttgaagct gaagtacagg ccaactcagg agccattgtg 300

aagctggatg agacaggaaa cttgatgatt tctgaagggc actttgcatc tgagaccatc 360

cggacacgtt taatggagct gcaccgccag tgggagttgc ttttggagaa gatgcgggag 420

aaaggaatca aactgctgca ggcacagaag ctggtgcagt atttgcggga gtgtgaggat 480

gtaatggact ggatcaatga caaggaagca attgtgacgt ctgaggagct gggccaggat 540

ttggagcatg tagaggtact acagaagaag tttgaagagt ttcaaactga tctggctgct 600

catgaagaaa gagttaatga agtaaaccag tttgctgcca aacttatcca ggagcagcac 660

ccggaagagg agctgatcaa gaccaagcag gaggaggtga atgcagcttg gcagcgactg 720

aaaggcctgg ctcttcaaag gcaggggaag ctctttggtg ctgccgaggt tcagcgcttt 780

aacagggatg tagatgagac cattggttgg attaaggaga aagagcagtt aatggcctct 840

gatgactttg gcagagactt agcaagtgtt caagctctgc ttcggaagca tgagggtctg 900

gagagagatc ttgctgctct agaggacaag gtgaaagccc tgtgtgccga ggctgaccgc 960

ctgcaacagt cacaccctct gagtgccaac cagatccagg tgaagcgaga ggaactaatt 1020

accaactggg agcagatccg aactctggcc gcagagagac atgcacggct tgatgactca 1080

tacaggcttc agcgctttct tgctgacttc cgtgacctca cgagctgggt gactgaaatg 1140

aaagccctca tcaatgcaga cgagcttgcc aatgacgtgg ctggtgctga ggccctgctg 1200

gacaggcatc aagagcacaa gggtgaaatc gatgctcatg aagatagctt taagtctgca 1260

gatgagtctg ggcaggccct actcgctgct ggtcactatg cctcagatga agtgagggag 1320

aagctgagca tcctctctga ggagagagct gccctgctgg agctgtggga gcttcggagg 1380

cagcagtatg agcagtgcat ggacttgcag ctcttctacc gagacactga gcaggtggac 1440

aactggatga gcaaacagga ggcattcctg ctaaatgaag atttgggtga ctccttagac 1500

agtgtggaag ctcttttgaa gaagcatgag gactttgaga aatctctcag tgcccaggaa 1560

gaaaaaatca cagcacttga tgagtttgca accaagctta ttcagaacaa ccactacgca 1620

atggaagatg tagccactcg acgagatgct ctcctgagcc gccgcaatgc cctccatgag 1680

cgagccatgc atcgccgggc acagctggcc gattccttcc acctgcagca gttcttccgc 1740

gattccgatg agctcaaaag ttgggtcaat gagaagatga aaacggccac tgatgaagct 1800

tacaaagatc cgtccaacct gcaagggaaa gtccaaaagc accaggcttt tgaggctgag 1860

ctctcagcca accagagccg tattgatgcc ctagagaaag ctgggcaaaa actaatagat 1920

gtgaaccact atgccaagga agaagtagca gctcggatga atgaggtcat cagtttgtgg 1980

aagaaacttc tagaggccac agaactgaaa ggagtcaagc tccgagaagc caaccagcag 2040

caacaattta atcgcaatgt tgaggacatt gagttgtggc tgtatgaagt tgaaggtcac 2100

ttggcttcag atgattatgg taaagacctc actaatgtcc agaacctcca gaagaagcat 2160

gctctgctag aggcagatgt tgctgctcac caggatcgaa ttgacggcat cacaattcag 2220

gcccgccagt tccaagatgc tggccatttc gatgccgaaa acattaaaaa gaagcaagag 2280

gcccttgtag ctcgctatga ggctctcaag gaacccatgg tggcccggaa gcagaagctg 2340

gcagattctc ttcgtctgca gcagctcttc cgagatgtgg aggatgagga aacctggatt 2400

cgagaaaagg agcctattgc tgcgtccact aacagaggca aagatcttat tggagtccag 2460

aatctgctaa agaagcacca agctttacag gcagaaattg ctggccatga acctcgcatc 2520

aaagcagtga cacaaaaggg caatgccatg gtggaggaag gccattttgc tgctgaggat 2580

gtgaaggcca aactgagtga gctcaaccag aagtgggagg cactgaaagc caaagcctcc 2640

cagcggaggc aggatctgga ggactcacta caggcccagc agtactttgc cgacgccaat 2700

gaagctgagt cctggatgcg ggagaaggag cccattgtgg gcagtaccga ctatgggaag 2760

gatgaagact ctgctgaggc tctgctcaag aagcatgaag ctttgatgtc cgatctcagt 2820

gcctacggca gcagcattca agctttgcga gagcaggctc agtcatgccg gcaacaagtg 2880

gcccccatgg atgatgagac tggcaaggag ctggtcttgg ctctctatga ctatcaagag 2940

aagagccctc gtgaggtcac catgaagaaa ggggatatcc tcaccttgct caacagcaca 3000

aacaaggact ggtggaaagt ggaagtgaat gaccgtcagg gttttgtgcc agctgcgtat 3060

gtgaagaagc tggaccccgc ccagtcagcc tcaagggaga acctcctgga agaacagggc 3120

agcattgctc tgcggcaggg gcagatcgac aaccagacac gcataactaa ggaggccggc 3180

agtgtatctc tgcgtatgaa acaggtggaa gaactgtatc agtctctgct ggagctgggt 3240

gagaagagaa aaggcatgtt ggagaagagt tgcaagaagt tcatgttgtt ccgggaagcg 3300

aacgagctac agcagtggat caacgagaag gaagctgctc taacgagtga agaggttggc 3360

gctgacttgg agcaggtcga ggtgctgcag aagaagttcg atgacttcca gaaggatctg 3420

aaagccaatg agtcccggct gaaggacatt aacaaagtgg ccgaggacct ggagtctgaa 3480

ggtctcatgg cggaagaagt gcaggccgtg cagcagcagg aggtgtatgg tatgatgccc 3540
agggatgaag cagattccaa gaccgcctcc ccatggaagt ctgctcgact gatggtccac 3600
acagtggcca ccttcaactc catcaaggag ctgaatgagc gctggcggtc cctgcaacag 3660
ctggctgagg aacgtagcca gctcctgggc agtgcacacg aagtacagag gttccacagg 3720
gatgcggatg aaaccaaaga atggattgag gagaagaacc aggctctgaa cacagacaac 3780
tatggccatg atctagctag cgtccaggcc ctgcagcgca aacacgaagg cttcgagagg 3840
gaccttgcag ctcttggtga caaggtgaat tcccttgggg aaacagccca gaggctgatc 3900
cagtcccacc ctgaatctgc agaggactta aaggaaaagt gcacagagtt aaaccaggcc 3960
tggaccagcc tagggaagcg tgcagaccag cgcaaggcca aactgggtga ctcccatgac 4020
ctgcagcgct tccttagcga tttccgggac ctcatgtctt ggatcaatgg aatacgaggg 4080
ttggtatctt cagatgaact ggccaaggat gtcactggag ctgaggcttt gctggagcga 4140
caccaggaac accggacaga aattgatgcc agggctggca ctttccaggc atttgagcag 4200
tttgggcagc agctgttggc tcatgggcac tatgccagcc cagagatcaa ggagaaactt 4260
gatattcttg accaggagcg cacagacctg gagaaggcct gggttcagcg cagaatgatg 4320
ctggaccact gcctggagtt gcagctgttc catcgagact gtgagcaagc agagaactgg 4380
atggctgccc gggaagcctt cctaaacaca gaagacaaag gagactcgct ggacagtgtg 4440
gaggctctga tcaaaaaaca tgaagacttc gacaaagcta tcaatgtcca ggaggagaag 4500
atagctgccc tgcaggcctt tgccgaccag ctcattgctg tggaccacta tgccaaggga 4560
gacattgcaa accgacgcaa tgaggtcctg gacaggtggc gccgcctaaa agcccagatg 4620
attgagaaaa ggtcaaagct cggagaatct caaacacttc agcagttcag ccgggatgta 4680
gatgagattg aagcctggat cagtgagaag ttacaaacag ccagcgatga gtcatacaag 4740
gaccccacca acatccagag caagcaccag aagcaccaag cctttgaggc agaactgcac 4800
gccaatgctg accgaatccg tggagttatt gacatgggca actccctcat tgagcgtggg 4860
gcctgtgctg gcagtgagga tgctgtcaag gcccgcctgg ctgcccttgc agaccagtgg 4920
cagttcctgg tgcagaagtc agctgagaag agccagaagc tgaaagaggc caataagcag 4980
cagaacttca acaccgggat caaagacttt gacttctggc tttctgaggt ggaggctctc 5040
ctggcatctg aagactacgg caaagacctg gcttccgtga acaacctgct caaaaagcat 5100
cagctgctgg aggcagacat atcggcccac gaggatcgtc tgaaggacct gaacagccag 5160
gctgacagcc tgatgactag cagtgccttc gacacctccc aagtgaaaga gaagcgggac 5220
accatcaatg gacgctttca gaagatcaag agcatggcaa cctcccgaag agcaaaactg 5280
agcgagtccc atcgcctgca ccagtttttc cgagacatgg atgacgagga gtcctggatc 5340
aaggagaaga agttgttagt gagctctgag gactatggca gagacctcac tggtgttcaa 5400
aatctgagga agaaacacaa gcggctagaa gccgaactgg ccgcacacga accagccatt 5460
cagggtgtcc tggacacggg gaagaagctg tctgatgaca acaccatcgg gcaggaggag 5520

atccagcagc gtctcgcaca gtttgtggag cactggaagg aactgaaaca gctagcagct 5580

gcacggggcc agcggctgga ggagtccttg gagtatcagc agtttgtggc caacgtggag 5640

gaggaggagg cttggatcaa tgagaagatg accctggtgg ccagcgaaga ctacggggac 5700

actcttgctg ccatccaggg cttactgaag aaacatgaag cttttgagac agacttcact 5760

gtccacaagg atcgagtgaa tgatgtctgt actaatggac aagacctcat taagaagaac 5820

aatcaccatg aggagaacat ctcttcaaag atgaagggtc tgaatggtaa agtgtctgac 5880

ctggagaaag cagcagctca gaggaaagcg aagctggatg agaactcggc cttccttcag 5940

ttcaattgga aggctgacgt ggtggagtcc tggattggtg aaaaggagaa cagcttgaaa 6000

acagatgatt atggccgaga tctgtcttct gtccaaactc tgctcaccaa gcaggagaca 6060

tttgatgctg gcctgcaggc cttccagcag gagggcattg ccaatatcac tgccctcaaa 6120

gaccagctgc tagctgccaa gcacattcag tcgaaggcca tcgaggcccg acatgcctcc 6180

ctcatgaaga ggtggaccca gctgttggcc aattcagcta cccgcaagaa gaagttgcta 6240

gaggcccaga gtcatttccg aaaggtagaa gacctcttcc tgacctttgc caaaaaggca 6300

tcggctttca acagctggtt tgagaatgca gaagaggacc tcacagaccc agtgcgctgc 6360

aactctctgg aagaaatcaa agccctccga gaggctcatg atgccttccg ctcatcgctc 6420

agctctgcgc aggccgactt caaccagcta gccgagctgg accgtcagat caagagtttc 6480

cgagtggcct ccaatcccta cacctggttc accatggagg ccctggaaga gacgtggagg 6540

aacctacaga agatcattaa ggagcgagaa ctggagctgc agaaggaaca gcggcggcag 6600

gaggagaatg acaagctacg ccaagagttt gcccagcatg ccaacgcgtt ccaccagtgg 6660

atccaggaaa caagaacgta tctcctcgac gggtcctgca tggtcgaaga gtcgggaact 6720

ctggaatctc agcttgaagc taccaaacgc aagcaccagg agattcgggc catgagaagt 6780

cagctgaaga agattgagga cctgggggct gccatggagg aagccctcat cctggacaac 6840

aagtacactg agcacagcac tgtgggcctg gcccagcagt gggaccagtt agaccagctg 6900

ggcatgcgca tgcagcacaa cctggagcag cagatccagg ccaggaacac aacaggagtc 6960

actgaggagg ccctcaagga gttcagcatg atgttcaaac acttcgacaa ggacaagtct 7020

ggccggctga atcatcaaga gttcaaatcc tgccttcgtt ctctgggtta cgacctgcca 7080

atggttgagg aaggagagcc tgatcctgag tttgaggcca tactggacac tgttgatccc 7140

aacagggacg gccacgtctc cctgcaagag tacatggctt tcatgatcag ccgtgaaacc 7200

gagaatgtca agtccagtga agagatcgag agtgctttcc gggccctcag ctccgagggc 7260

aagccttatg tgaccaagga ggagctctac cagaacctga cccgggaaca agctgactac 7320

tgtgtctccc acatgaagcc ctatgtggat ggcaagggcc gcgaacttcc aactgccttc 7380

gactacgtgg agttcacccg ctctctcttt gtgaattgat 7420

<210> 111

<211>  1259

<212>  DNA

<213>  Rattus norvegicus (Norway rat)

<400>  111
ggcacgaggg cgagggtgca gccccggagc ggcggcgggg caaaatgaaa aacgaaattg      60

ctgctgttgt cttctttttc acaaggctgg ttcgaaagca tgataagttg aaaaaagaag     120

cagttgagag gtttgctgag aaattgactc aaatacttca agagaaatac aaaaatcact     180

ggtatccaga aaaaccctca aaaggacaag cctacagatg cattcgtgtc aataagtttc     240

agagagttga tcccgacgtc ctgaaagcct gtgaggacag ctgcatcctg tacagtgacc     300

tgggcttgcc aaaggagctt acactctggg tggatccgtg tgaggtgtgc tgccggtatg     360

gaaagaaaaa caatgcattc attgttgcca gctttgaaaa tgaggatgag aacaaggatg     420

agatctccaa gaaagttagc agggctcttg ataaggtgac ctctgattat cattcagggt     480

cctcctcctc agatgaagac acaagcaagg aagtagaagt gaaaccgagt gcagtggcta     540

caacgccaag ccccgtgtac cagatttcag aactgatatt cccacctctt ccaatgtggc     600

accctttgcc cagaaaaaag ccaggaatgt accgaggggg tggccatcag agtcactacc     660

ctcctcctgt tccatttgct tatccaagtc caggaaggaa gaataaagcg ttccgcccaa     720

ttccagtgac atgggtacct cctcctggaa tgcattgtga tcggaatcac tggattaatc     780

ctcacatgtt agcacctcac tagttcattt ggattgggcg gatgtcattt tgatagaaag     840

gaagaaatac cttcttagat acttaagagt ttcacaactt gtagtgaagt cagatggaca     900

aaaccatcag gcttattttt atagaaaagc tattgagata atctttctta aagtatatat     960

atatatgcac tttagatata ttgatatagt ttgagaaact ttattaaagt tagtcaagtg    1020

cctgagtttt taatattgga cttgagtatt tatatattgt gcattgactc tgttggatac    1080

aaaacactgt aggagggcga tatgttttag cacctttgag catttacttt atggagaata    1140

tgtaagttat ttatacagaa ggacatttat tttatgtcac atagaagaat tgtgtgaaat    1200

catgtagttg caaataaaaa gtagtttgag gcgtgaaaaa aaaaaaaaaa aaaaaaaaa     1259

<210>  112

<211>  1563

<212>  DNA

<213>  Rattus norvegicus (Norway rat)

<400>  112
gtcaagatgc agttctcagg aaggacccgg aagaagctga gattggcagg tgaccagaga      60

aacgcttgtt accctcacag ccttcagttc tatctgcagc cacctactga aaacatatca     120

ttgacagagt ttgaaagctt ggcttttgat agagtaaaat tgcttaaagc aattgagaat     180

cttggtgtga gctatgtgaa aggaaccgaa cagtaccaga gtaaactgga ggctgagatt 240

cgaaagctca agttttcgta cagggagaac ctggaggatg agtacgagcc tcggaggagg 300

gaccacatct cccacttcat cctgcgcctc gcttactgcc agtcggaaga tcttagacgg 360

tggtttattc aacaggagat ggatctgctt cggttccgat tcagtatttt acccaaggat 420

aaagtccaga gtttcttgaa ggatactcac ttgcattttg aggctatcag tgatgaggag 480

aagacccttc gggaacagga tatcatggcg tcctctccca gcctaagtgg ggtcaggtgg 540

gaatcggagt cagtgtataa ggtccccttt gctgacgctc tggacctgtt cagaggaagg 600

aaagtctact tggaagacgg ctttgcttat gtgccactta aggacattgt ggccattatc 660

ctgaacgagt ttagagccac gctgtctaag gccttggcac taacagccag gtccctgcct 720

gctgtgcagt ccgatgaacg acttcagcct ctgctcagcc acctcagtca ttcttacacc 780

ggccaagatt atagtaccca gaagagcacc gggaagattt ccttagatca gattgattcg 840

ctttcaacaa aatccttccc accttgcatg cgtcagctgc acaaggcgct gagggaaaac 900

caccatcttc gtcatggagg ccggatgcag tatggcctgt tcctcaaggg cattgggcta 960

acgttggagc aagcattgca gttctggaag caagagttta tcaaaggaaa gatggaccca 1020

gacaagtttg ataaaggtta ctcttacaat atccgacata gctttggaaa ggaaggcaag 1080

aggacagact atacgccatt cagttgcatg aagattatcc tgaccaaccc accaagccag 1140

ggggatttcc atgggtgccc attccgtcac agtgatgcag agctgctgaa gcagaagatg 1200

cagacctaca agatccctgc ctcggggatc agccagattt tggatttggt aaaggggaat 1260

cattaccagg tggcctgtca gaagtacttc gagatgacgc acaatgtgga cgattgtggc 1320

ttttctttga atcatccaaa tcagttcttt tttgagagcc agcgaatcct aactggtggc 1380

aaagatatca agaaggaagc aagccaccca gaaacgcctc agcacaaacc cagcacccag 1440

aagaccaagg atgccacgtc tgctctggcc tctctagatt cctccctgga aatggatctg 1500

gaggggctag aagactactt tagtaaatga cgtggcccct ggagcaactg gagcaaatac 1560

att 1563

<210> 113

<211> 4128

<212> DNA

<213> Rattus norvegicus (Norway rat)

<400> 113
ggttccggct gtgggcagca gggtggtccg cgactgcggc cggaagcagg ctgagcggct 60

ctcggcgagc gccgcctgta cttccgggga cttcagccta gctcttgagt tttccctgta 120

ggcggcgcgg gagggaaaag tttgcagggc cgtgctggga agatcacctc cgcttcccaa 180

tgtgagcagt ttctcgaacg ctctctctag aaggttgccc agagaggtgg tggacgggaa 240

catgaggcag cgtctcctct tcctgaccac tttggtgcct ttcgtcctgg caccccgacc 300
tccggaggaa ccaggctctg gctcccacct gcgacttgag aagcttgatt ctttgctctc 360
agactacgat atcctctctt tatctaatat ccagcagcac tccataagga aaagggatct 420
acagtctgcg acacacttag aaacactact aactttttca gctttgaaaa ggcattttaa 480
attatacttg acatcaagta cagaacgctt ttcacagaac ttgcgagtcg tggtggtgga 540
tgggaaagag gaaagcgagt acagtgtgaa gtggcaggac ttcttcagcg gacatgtggt 600
tggtgagcct gactctaggg ttctagcgca cataggagat gatgatgtca cagtaagaat 660
caacacagat ggggcagaat ataacataga gccactttgg aggtttgtta atgatactaa 720
agataaaagg atgctggtgt ataagtctga agatatcaag gatttttcac gtttgcagtc 780
tccaaaagta tgtggttatt taaatgcaga tagtgaagag ttgcttccaa aagggctcat 840
agacagagag ccatctgaag agtttgtccg tcgagtgaag aggcgagctg aacctaaccc 900
attgaagaat acttgtaaat tactggtggt ggcagatcat cgattttata agtacatggg 960
ccgaggagaa gagagcacta ctacaaatta cttaatagag ttaattgacc gagttgatga 1020
catataccgg aacacctcgt gggacaatgc aggatttaaa ggttatggag tacagataga 1080
acagattcga attctcaagt ctccacaaga ggtaaaacct ggtgaaagac acttcaatat 1140
ggcaaaaagt tttccaaatg aagaaaagga tgcttgggat gtgaagatgc tgctggagca 1200
atttagcctt gatatagctg aagaggcctc taaagtctgc ctggctcatc ttttcaccta 1260
ccaagatttt gatatgggaa ctcttggatt agcttacgtt ggttctccca gagcaaacag 1320
tcatggaggg gtttgtccaa aagcttatta caacccaggt gtgaagaaga acatctattt 1380
gaatagtggt ctgacaagta caaaaaatta tggtaaaacc atccttacaa aggaggctga 1440
cctggttaca actcatgaat tgggacacaa ttttggagca gaacatgatc ctgatgggct 1500
ggcagagtgt gccccaaatg aggaccaagg aggaaagtat gttatgtacc ccatagctgt 1560
gagtggtgac catgagaata ataagatgtt ttcaaactgc agtaaacagt ccatctacaa 1620
gaccatagaa agcaaggctc aagagtgctt ccaggagcgc agcaacaagg tgtgcggcaa 1680
ctccagggtg gacgaaggag aagagtgtga cccgggcatc atgtacctga acaacgacac 1740
ctgctgcaat agtgactgca cactgaagcc aggtgtgcag tgcagtgata ggaatagtcc 1800
ttgctgtaaa aattgtcagt ttgagacggc ccagaagaag tgccaggagg ctatcaatgc 1860
tacttgcaaa ggagtgtctt actgcacagg gaatagcagt gagtgccccc caccaggaga 1920
tgctgaagat gacactgtgt gcttggacct gggcaagtgc aaggctggga aatgcatccc 1980
tttctgcaag agggagcagg agctggagtc ctgcgcatgt gctgacaccg acaactcgtg 2040
caaggtatgc tgcaggaacc tttctggccc atgtgtgcct tacgtcgatg cagagcaaaa 2100
gaacttattt ttgaggaaag ggaagccctg tacagtaggg ttttgtgaca tgaatggcaa 2160
atgtgagaaa cgggtacagg acgtaattga gcggttttgg gatttcattg accagctgag 2220
catcaacact tttgggaagt ttctggcaga caacatcgtt gggtctgttc tggttttctc 2280

cttgatattt tggattcctt tcagcattct tgtccactgt gtggataaga aactggacaa 2340

gcagtatgag tccctgtctc tgtttcatca cagtaacatt gagatgttga gcagcatgga 2400

ctcagcatct gttcgcatta tcaagccgtt ccctgcaccc cagactccag gtcgtctgca 2460

ggccctgcag ccggctgcaa tgatgccgcc agtgtctgcg gctccaaaac tggatcacca 2520

gaggatggac accatccagg aagaccccag cacagattca catgtggatg atgatggctt 2580

tgagaaggac cccttcccca acagcagtgc agccgccaag tcctttgagg acctcacaga 2640

ccacccagtc accaggagtg agaaggccgc ctccttcaag ctgcagcgtc agagccgagt 2700

tgatagcaaa gagacagagt gttagtggga gcctgggcct gctctggggg acacagacct 2760

acagatgttc cacagagctg acctgaatca aaatagactg taatgatctg agaaacggga 2820

gcagcttagc agatgctggt catgtgacag gaccttcaca tgacatcctg tgtatgtagg 2880

ccctttgaag aggtgaggta aatctggctt atttaaggct ttcaggtttt ggggttttct 2940

tttgtaatct aaaaatctcc tttgacctgt ggtgcagaag cagaaaataa ggctggatca 3000

agttcctggt gacagcactg attaagcctt cagtctgttt ttctgtatgc tgtaaggtcc 3060

tctcactacg gtgaagcact tagcagtgag gataactgga acacagactt tttgggggtt 3120

ttggccttca catgtgcgtt gtctccaaat gccgtctgtc ttggcactga actttgtgga 3180

tacagtcctt tccaagatga agactagctc tggaacagtc tatacaaaca gccacatgaa 3240

cttcaagatg gcctattaac tgcttagccc caggttccct ggacagtttt tccgttttgc 3300

aaaaagtgtc ttcacgggtc tctaaacatt gtttgtctta aaaaatactg ggctggagag 3360

ttggccccac agttaagagc acttggtttc attcccagta cccacacggt ggctcttaac 3420

tccaatccca ggggagatga catcttctga actctagaac acaaggcatg cattggtgca 3480

gtcaaacatg caggcaacac acacaaattt agaaagccaa gtgctgtaag aagttgcaca 3540

tcccccaacc tacagtgctt cgcggtctga gctggagtgg agctctctcc tgaacactct 3600

ggaaggctgg caggtgctga gctggctagg gagacgggtg gggcccagcc ctggaagaac 3660

cgtcttcagc ctcagcaagc agacactcgc gttacctcag gctttggggc ttcaggaccc 3720

aggatgtttt gtacttattg tgtgaggata aataccatta gcctgaattc tcatttctgt 3780

aagttttagt tataaaagct ttttttcttc taagaattgc aatgtgctca ccagcaaaca 3840

cgtcttaacc agaataactt gtaactctga ggacagttaa ccaaacctgt gccttgtctc 3900

tcctgagggt gtctgttaca ctaatacttg aacatgtacc ttgtggtatt ggctcctttt 3960

actagtcatg acagccttat atgttagtta cactttgagg atttgctcta aggtgagtgg 4020

ggtgtgtggg tgtatgtatg aatgaaacag ttggcagaat ataagaaaac catttttata 4080

aaattgtgac tttttaaaac aaaaaaaaaa aaaaaaaaaa aaaaaaaa 4128

<210> 114

<211> 1200

<212>  DNA

<213>  Rattus norvegicus (Norway rat)


<400>  114
ttcggtcgac tgagagatcc tgagcagtgc cacctcacca gcctcgagat ggacaacagg      60

aagcgcctgg cctatgccat catccagttc ctgcatggcc agctgcggca cggtgggctc     120

tcgtccgacg cccaggagag cctagaggtt gccatccagt gtctggaaac agcctttggg     180

gtgacgctgg aggacagtga ccttgctctc ccgcagaccc tgccagagat atttgaagca     240

gccactgcca gcaaggagat gccacaggac ccgaggggcc ctgacaggac accaccctct     300

gaggaggact cagctgaggc agagcgcctt aaaacagaag gtaacgagca gatgaagttg     360

gaaaactttg aggcagcagt gcacctctat ggcaaagcca ttgagctcaa ccccgccaac     420

gccgtttact tctgtaacag agctgcggcc tacagcaagc tgggaaacta cgtgggggcg     480

gtacaggact gtgagcgcgc cattggcatt gatcccgggt acagcaaggc ctatggccgc     540

atgggccttg cgctgtccag cctgaacaaa catgcggagg ctgtggcgta ctacaagaag     600

gccctggagc tggaccctga caacgacacg tacaagtcca acctcaagat cgcagagctg     660

aagttacggg aggcgccgag ccctacgggc ggcgtgggca gcttagacat cgctggcctg     720

ctgaacaacc cgcacttcat cactatggct tcgagcttaa tgaacagccc ccagctccag     780

cagctcatgt cgggaatgat ctctggaggc cataaccctc tgggcacccc aggcagcagt     840

ccacagcaca gtgacctggc cagtctcatc caggcgggcc agcagtttgc acagcagatg     900

cagcagcaga acccagagtt cgtggagcaa atccggagcc aggtggtgcg cagccggaca     960

cccagtgcca gccacgagga gcagcaagag tgacaacgct gacccgagtg gcccctctgc    1020

tccgcgcgcg gcctcctggt gttttagcaa tttctccatc gggctgctca agagagaaac    1080

tggacctgca tgttaagacg gactcatccc cccgccctgt gttctcttca tccctccccc    1140

tttgtttttt gtactcgtaa ctggctccac accttcctgg aagagtctac atttgactac    1200

<210>  115

<211>  1194

<212>  DNA

<213>  Rattus norvegicus (Norway rat)


<400>  115
agagagagag agagagagag agagagagag aacccaccca cccggcgact aatctgatcc      60

cggctgtccc ccgggaccag cgaggtccca gaagacccac gagggagcgg gcgtaacgcg     120

tggctgcggg tgggagccat ggacgccttc atccgagtcg ccaaccaaag ccaaggtcgg     180

gaccgacttt tcagagccac tcaacacgca tgcatgttgc ttagatattt gttagagtct     240

aaggctggca aagaggcggt ggtaacgaag ctcaagaatc tggagactag tgtgagcact     300

ggccgtaaat ggttcagact aggcaacgtg ctccatgcca tccaggccac tgagcagagc 360

atccaagcca ctgaccttgt gccccgccta tgcctaacat tagccaacct gaaccgcgtg 420

gtttattaca tctgtgacac tgtcctctgg gcgaagagtg tgggtctgac atctggaatc 480

aacagagaga agtggcaaat gcgggcggcc cgccactact actatttcct cttgctgagc 540

ctggtccggg atctgtatga ggtcttgctg catatgggac aagttgcacg cgacagagca 600

aagagagaga agtcctccgg ggaccctcct aagtacagcg tcgctaatga agaaagtgaa 660

tggctccagt ccttcctcct cctcctcttc cagtctctaa agcgaaatcc gcccttattc 720

ctggacaccg tgaagaactt ctgtgacatc ctgatccctt tgaaccagct cgggatctac 780

aagtccaacc ttggcgtggt aggatttgga ggtctcgtgt cctctgtggc tggcctcatc 840

actgtggtgt atcctcagtt gaaactgaag gcccgctagg gtgtttggaa aatttaagac 900

tgacgttcag tggagcaaac atttgctttt gtcatgatgt ctactgtact taattttttt 960

taatcatgtg agcatcttac caaccggtga tgtgagcaga ggtaggaccc acaacggagc 1020

ctgaagactg atgacgtttt tgtaaacacg gcagtaactt ctgcacattt ccccttcagt 1080

gacttctgac tactgcaaaa acatttgtgc cgtcattgaa gacgtgtaaa ggggaagtca 1140

gaacattgct gagcatcttt tctgtacata gtaagagctc atatatctaa caaa 1194

<210> 116

<211> 1893

<212> DNA

<213> Rattus sp.

<400> 116
gggtgtagaa cggtgccacc actgtcttct tcgccaccat gggggcccag ctgagcacgt 60

tgagccgagt ggtactctcc ccggtctggt tcgtctacag cctcttcatg aagctgtttc 120

agcgctcctc accggccatc accctcgaga accccgacat caagtaccct ctgcggctca 180

tcgacaagga gattatcagc cacgacactc ggcgcttccg atttgcactc ccttcgcccc 240

agcacatcct gggccttcct atcggccagc acatctacct ctccaccagg atcgatggca 300

acttggtcat tcgtccctac acccctgtgt ctagtgatga tgacaagggc tttgtggact 360

tggtggtcaa ggtttacttc aaggacacgc atcccaagtt tccagctgga gggaaaatgt 420

ctcagtacct ggaaaacatg aatattggag acaccattga attccggggc cccaatgggc 480

tactggtcta ccagggcaaa gggaagttcg ccatccgtgc agacaagaag tccaaccctg 540

ttgtcaggac ggtgaagtct gtaggcatga ttgcaggagg gacaggcatc accccaatgc 600

tgcaggtgat ccgagccgtc ttgaaggacc cgaacgacca cactgtgtgc tatctgctct 660

tcgccaacca gtccgagaaa gacatcctgc tgcggcctga gctggaggaa ctgaggaacg 720

aacattcttc tcgcttcaag ctctggtaca cagtggacaa agcccccgat gcctgggact 780

atagccaagg cttcgtgaat gaggagatga tcagggacca tcttccacct cctggggagg 840

agacactgat actgatgtgt ggacccccac cgatgatcca gtttgcctgt ttgccaaacc 900

tggagcgtgt gggccatccc aaggagcgat gcttcacctt ctgatggctg gatgctggcc 960

actcccatgc ctgctgctca cgcactcacc acaaccacct tccacccctt ccttccccac 1020

tactgtcctt taccctgaca tatgcccaca tccatgctgg ggcctgggtt cagcctggcc 1080

tgcccagccc tggtcatcca gctgtactgg cccctgaggg gcccctttgg gagcaggcct 1140

gtgtatcagg tggcttctgt tgaccacttt ctgaataggc ttctgtctgg tactaactgg 1200

ccattaccag agatggtcca tgaccacccc tttatacaca cacacataca catacagaga 1260

cagagagaca gagagacaga gatagacaga gatagacaga gagacagata cagacataga 1320

gacagacaga gacagagaga gaggagtcag agagctgtta gtaccatgtc tacccgttac 1380

catggaccgc aatgtgtagt aaggaaatga aaggtaaaca gttatctaca gtcctaggaa 1440

ccatgcctgt cacctaacca cctactgcct taggctatgg cctggtctgt gcccctaaac 1500

actacattag acagtgacac ccagaggttc tcttaggagg gtgtctggca gtgacaggcc 1560

agcccccact tgctggccag gagttccttg ggtcagcacg tggacccttc caaaacccca 1620

cacactgccc ctcccctcca tgatgccttg agcaccctaa gttgtcagcc caacagagtt 1680

tggtagacct tggggtacct ggcttgtttc tccatcccat acctccctcc catctggttc 1740

ccagcctggg gggttctgag cagagcctct tttctcggag acagacccgg tgcctggcgc 1800

tgccttcagc agacagcaga cagcctcctg cacactggct ttttttagtc atttatgggc 1860

aaaatgagtt aaaataaaac tttgcaaatc ctg 1893

<210> 117

<211> 1893

<212> DNA

<213> Rattus sp.

<400> 117
gggtgtagaa cggtgccacc actgtcttct tcgccaccat gggggcccag ctgagcacgt 60

tgagccgagt ggtactctcc ccggtctggt tcgtctacag cctcttcatg aagctgtttc 120

agcgctcctc accggccatc accctcgaga accccgacat caagtaccct ctgcggctca 180

tcgacaagga gattatcagc cacgacactc ggcgcttccg atttgcactc ccttcgcccc 240

agcacatcct gggccttcct atcggccagc acatctacct ctccaccagg atcgatggca 300

acttggtcat tcgtccctac acccctgtgt ctagtgatga tgacaagggc tttgtggact 360

tggtggtcaa ggtttacttc aaggacacgc atcccaagtt tccagctgga gggaaaatgt 420

ctcagtacct ggaaaacatg aatattggag acaccattga attccggggc cccaatgggc 480

tactggtcta ccagggcaaa gggaagttcg ccatccgtgc agacaagaag tccaaccctg 540

ttgtcaggac ggtgaagtct gtaggcatga ttgcaggagg gacaggcatc accccaatgc 600

tgcaggtgat ccgagccgtc ttgaaggacc cgaacgacca cactgtgtgc tatctgctct 660

tcgccaacca gtccgagaaa gacatcctgc tgcggcctga gctggaggaa ctgaggaacg 720

aacattcttc tcgcttcaag ctctggtaca cagtggacaa agcccccgat gcctgggact 780

atagccaagg cttcgtgaat gaggagatga tcagggacca tcttccacct cctggggagg 840

agacactgat actgatgtgt ggacccccac cgatgatcca gtttgcctgt ttgccaaacc 900

tggagcgtgt gggccatccc aaggagcgat gcttcacctt ctgatggctg gatgctggcc 960

actcccatgc ctgctgctca cgcactcacc acaaccacct tccacccctt ccttccccac 1020

tactgtcctt taccctgaca tatgcccaca tccatgctgg ggcctgggtt cagcctggcc 1080

tgcccagccc tggtcatcca gctgtactgg cccctgaggg gcccctttgg gagcaggcct 1140

gtgtatcagg tggcttctgt tgaccacttt ctgaataggc ttctgtctgg tactaactgg 1200

ccattaccag agatggtcca tgaccacccc tttatacaca cacacataca catacagaga 1260

cagagagaca gagagacaga gatagacaga gatagacaga gagacagata cagacataga 1320

gacagacaga gacagagaga gaggagtcag agagctgtta gtaccatgtc tacccgttac 1380

catggaccgc aatgtgtagt aaggaaatga aaggtaaaca gttatctaca gtcctaggaa 1440

ccatgcctgt cacctaacca cctactgcct taggctatgg cctggtctgt gcccctaaac 1500

actacattag acagtgacac ccagaggttc tcttaggagg gtgtctggca gtgacaggcc 1560

agcccccact tgctggccag gagttccttg ggtcagcacg tggacccttc caaaacccca 1620

cacactgccc ctcccctcca tgatgccttg agcaccctaa gttgtcagcc caacagagtt 1680

tggtagacct tggggtacct ggcttgtttc tccatcccat acctccctcc catctggttc 1740

ccagcctggg gggttctgag cagagcctct tttctcggag acagacccgg tgcctggcgc 1800

tgccttcagc agacagcaga cagcctcctg cacactggct ttttttagtc atttatgggc 1860

aaaatgagtt aaaataaaac tttgcaaatc ctg 1893

<210> 118

<211> 1052

<212> DNA

<213> Rattus sp.

<400> 118
ggacgtacca aaatgagcgc acctccaata aagatacaca tcatgtcgtc ttcacatctc 60

ttctacctgg cactctgctt gctcaccttt accagctcgg ccacagccgg accagagacc 120

ctttgcgggg ctgagctggt ggacgctctt cagttcgtgt gtggaccaag gggcttttac 180

ttcaacaagc ccacaggcta tggctccagc attcggaggg caccacagac gggcattgtg 240

gatgagtgtt gcttccggag ctgtgatctg aggaggctgg agatgtactg tgctccgctg 300

aagcctacaa agtcagctcg ttccatccgg gcccagcgcc acactgacat gcccaagact 360

cagaaggaag tacacttgaa gaacacaagt agaggaagtg caggaaacaa gacctacaga 420

atgtaggagg agcctcccga ggaacagaaa atgccacgtc accgcaagat cctttgctgc 480

ttgagcaacc tgcaaaacat cggaacacct gccaaatatc aataatgagt tcaataccat 540

ttcagagatg ggcatttccc tcaatgaaat acacaagtaa acattccgac attgtcttta 600

ggagtggttt gttaaaaaaa aaaacaaaaa acaaaaacaa aaacaaaaaa aaagcttgca 660

ccttgcaaaa gtggtcctgg cgtgggtaga ttgctgttaa tcctttatca ataacgttct 720

atagagaata tataaatata tatataatta tatctcctag tccctgcctc ttaagagccg 780

aaaatgcatg ggtgttgtag acattcggtt gcactaaatt cctctctgaa ttttggctgc 840

tgaagccgtt catttagcaa ctgtttatag gtggttgatg aatggttcct tatctccatt 900

tcttcctatg tagcttaagc cgcttccttc acagaatcta ataatctcgt ctaggccatt 960

agccctgccc tttcttaaca ttcttgtatt tgttgaattt ggcctcctcg aaagcaatag 1020

caactgggtg gcccacccaa gttttaacgc cc 1052

<210> 119

<211> 1744

<212> DNA

<213> Rattus norvegicus (Norway rat)

<400> 119
ctgcagtgtg aagggtgttg tcttacctct cgcagacttt cccattccca gccctgattt 60

ccccactgga cccttctcct tctgaaccag catcctgcct ggtttgagca gtcatggcct 120

cacgggtgaa tgatcaaagc caggcttcaa ggaatgggct gaagggaaag gtgctaactc 180

tggacactat gaacccatgt gtgcggaggg tggagtatgc agttcgagga cccattgtgc 240

agcgtgcctt ggagctggag caggagctgc gtcagggtgt gaagaagccg tttactgagg 300

tcatccgtgc caacattggg gatgcacaag ccatggggca gagacccatc accttcttcc 360

gccaggtcct ggccctctgt gtctacccca atcttctgag cagtcctgac ttcccagagg 420

atgccaagag aagggcagaa cgcatcttgc aggcctgcgg gggccacagc ctgggtgcct 480

atagcattag ctctggaatc cagccgatcc gggaggatgt ggcgcaatac attgagagaa 540

gagacggagg catccccgca gacccgaaca acatatttct atccacaggg gccagcgatg 600

ccatcgtgac aatgctcaag ctgctggtat ctggcgaggg ccgtgcacga acaggtgtac 660

tcattcccat tcctcagtac ccactgtact cagccgcgct ggctgaactg gacgccgtgc 720

aagtggacta ctacctggac gaagagcgcg cctgggctct ggacatcgca gagctgcggc 780

gcgctctgtg ccaggcacgt gaccgttgct gccctcgagt actgtgcgtc atcaaccccg 840

gcaaccccac tgggcaggtg cagacccgtg agtgcatcga ggccgtaatc cgctttgctt 900

tcaaagaagg actcttcttg atggctgatg aggtatacca ggacaacgtg tatgccgagg 960

gctctcagtt ccattcattc aagaaggtgc tcatggagat ggggccaccg tattccacgc 1020

agcaggagct tgcttctttc cactcagtct ctaagggcta catgggcgag tgcgggtttc 1080

gtggtggcta tgtggaggtg gtaaacatgg atgctgaggt gcagaaacag atggggaagc 1140

tgatgagtgt gcggctgtgt ccaccagtgc caggccaggc cttgatggac atggtggtca 1200

gtccgccaac accctccgag ccgtccttca agcagtttca agcagagaga caggaggtgc 1260

tggctgaact ggcagccaag gctaagctca cggagcaggt cttcaatgag gctcccggga 1320

tccgctgcaa cccagtgcag ggcgccatgt attccttccc tcaagtgcag ctgcccttga 1380

aagcggtgca gcgtgctcag gaactgggcc tggcccctga catgttcttc tgcctgtgcc 1440

tcctggaaga gactggcatc tgcgttgtgc ccgggagtgg ctttgggcag caggagggca 1500

cctatcattt ccggatgacc attctgcccc ccatggagaa actgcggctg ctgctggaaa 1560

aactcagtca cttccatgcc aagttcaccc atgagtactc ctgaagccac tgctagggcc 1620

acactggaca gtctctgacg caacaaaccg agggtcctta ggaaccctca gtatttctga 1680

ttttgtctag ggtctcggta actgtcctgc gggtccctaa taaatctgat gtcagcctga 1740

aaaa 1744

<210> 120

<211> 2064

<212> DNA

<213> Rattus sp.

<400> 120
cccagcaccc gcgatcgcgc gggctctgtg ctccgagggc cgtgctcgcc tggtgcctac 60

tccagggctg tgtacaccgc gcgggcggcg gctgagcccg caccaggagg gcgcgggagc 120

cgcagggccc tggagctttg ggcgactgct acgcggcctt ggccgaggct cttccttgct 180

cgccgttctg tcgcgggcct ggcccgtgaa ccgctcacag tatggcccga tgctgcttct 240

acacggcggg gacactgtct ctgctgctgc tggtgaccag tgtcacgctg ctagtggctc 300

gagtctttca gaaggcagtg gaccagacga tcgagaagaa tatggtatta caaaatggta 360

ccaaggtctt tgattcctgg gagaagcccc ctctacctgt gtacatccag ttttatttct 420

tcaatgtcac caatccagag gagatcctcc aaggagaaat ccccctgcta gaagaagtgg 480

ggccgtacac ctacagggag ctcaggaaca aggcaaacgt tcagtttgga gaaaatggaa 540

caaccatatc tgccgtcacc aataaggcat atatttttga acgaaaccag tctgttggag 600

accctaccgt tgacttgatt agaacaataa atattcctct gttgactgtt gtggaaatgg 660

cccagcagcc cttcctcagg gagatcatcg aggccatgct gaaagcttat cagcagacgc 720

tgtttgtcac tcacactgta catgaactgc tctggggcta caaagatgag gtcttgtcgc 780

tcgtccatat tttcagacct gacgtctccc ctaactttgg cctgttctat gagagaaatg 840

gaactaatga tggggagtat gtttttctga ctggagagga caattacctg aactttacaa 900

aaattgtgga gtggaatgga aaaacgtcgc tggactggtg gacgacggac acgtgcaata 960

tgatcaacgg gacagacgga gattcttttc acccattaat aagcaaggat gagaccctgt 1020

acatcttccc atctgacttc tgcaggtccg tctatataac tttcagtagc tttgagaacg 1080

tagaaggact gcctgctttt cggtataagg tgcctgcaga aatactagcc aattcctccg 1140

aaaacgctgg cttctgtata cccgagggaa actgcatgga cgcgggagtg ctgaacgtca 1200

gcatttgcaa gaatggtgcg cccattatca tgtctttccc acacttttac caagccgacg 1260

agaagttcgt ttcggccata aaaggcatgc gtccaaacaa ggaagaacat gagtcatttg 1320

tggacattaa tcctttgaca ggaattattt taagaggggc caagagattc caaatcaaca 1380

cgtacgttaa gaagctggat gactttgtgg aaacgggaaa cattaggact atggttttcc 1440

cagtgatgta tctcaatgag agtgttctca ttgacaaaga gactgcaagt caactgaagt 1500

ctgtgattaa cacaactttg attgtcacca acatacccta catcatcatg gcactgggcg 1560

tgttctttgg cttgattttc acgtggctgg cgtgtcgagg acaggggtct acggatgagg 1620

gaactgcaga tgaaagggca cccctcatac ggacctaatg ggacttacct gttgcctgag 1680

cttggtgaga gaatgtgaga actgacctga cctggaccag gacagggaaa agcctgcatc 1740

ctcacgggct cctggcctgt caagaaggga gtgaagccgc agcactggca cgcgagaaga 1800

ccctcctgga cagaggggac cgagcaggtg acatggctgg caattacgct ttataaaatc 1860

atgtctctga aactgtgtca atgtgtctta gggagtattt aataaattgt gtagaaacct 1920

tttgtgattg ggctctggga gctgtggggt tttgtaaccc ctgttgtaga tacagtgtct 1980

gagtcacttg ttaatgttaa cttggcctga cccgattcag tatgcctcac tctccaaact 2040

ttgtgtttaa aatatgaaaa aaaa 2064

<210> 121

<211> 970

<212> DNA

<213> Rattus sp.

<400> 121
   gcggtgtggt tgagtagggt gctgctttca gtcgtgtggc ctttggaact ccgcgtagca 60
   ctgccgcctc ctcctgtcct cgccatgttc ctcactcggt ccgagtacga caggggtgtg 120
   aacacttttt ctcctgaagg aagattattt caagtggaat atgccattga gggccataag 180
   cttggttcta cggccattgg catccagaca tcagagggtg tatgtctagc tgtggagaag 240
   agaattacct cgccactaat ggagcctagc agcattgaga aaatcgtaga gattgatgct 300
   catataggtt gtgccatgag tgggctaatt gctgatgcta aaactttaat tgataaagcc 360
   agagtggaga cacagaacca ctggttcacc tataatgaga caatgacagt tgagagtgtt 420
   acccaggctg tgtccaatct ggctttgcag ttcggagaag aagatgcaga tccaggtgct 480
   atgtctcgtc cctttggagt agcattgttg tttggaggag ttgatgagaa agggccccaa 540
   ctgtttcaca tggacccatc tgggaccttc gtacagtgtg atgctcgagc aattggttct 600
   gcgtcagagg gtgcccagag ctccttgcag gaagtttacc acaagtctac gactctgaag 660
   gaggccatca agtcttcact catcatcctc aagcaagtca tggaggagaa gctgaacgca 720
   actaacatcg agctggccac agtgcagcct ggtcagaatt tccacatgtt cacaaaggaa 780
   gaactggagg aggtgatcaa ggacatttaa ggagggccca tcctcgaact tctgtgggac 840
   agtttcagtt ctaatggctc ttagacttta tttccaactc cacgtcgtga aaatatccag 900
   tatatgtatg tgtgtttttt tatgatgtct gtacataaca gcaattctga aataaaaaaa 960
   atttacaaat 970

<210> 122

<211> 2721

<212> DNA

<213> Rattus norvegicus (Norway rat)

<400> 122
gcggccgtcg gcggacgcgg gaagaagtgt cttgtaggcg cggcgcccca ctcagccccg 60

gcccccctca tccgcgtact cggctgatgc ctcagggcgt cagctcggcg gagaggcggt 120

cgaggcgtgc gcctggctag cagttggctt tccccgtgac tcggacacgt ttgcagtcga 180

ataaacttgc gaccgccacg tgtggcatct ctccaaggaa ccggctcagg aggagggcgc 240

gcccatcgtg ggatcgcgcg gggcgcccgg cagggcggcg gcgacagcgg cagagccggc 300

cgcggctgct gcgtgcggag gcgctgccgg ttacgtaaag atgaggggct gaggtcgcct 360

cggcgctccc gcgagtcgca agctcccgcg cccccgcccc ctcggccgcg ccgcaccgcg 420

ctcgtcgtcc gagaccaggg cagggctagc cgaagctccg cagccaccct caagtttggc 480

cgcaccgccc gggctgccgt cgcccgcacc atgtccgcgg ccgcctacat ggacttcgtg 540

gctgcccagt gtctggtttc catctccaac cgcgccgctg tgccggagca cgggggcgct 600

ccggacgccg agcgactgcg actacctgag cgcgaggtga ccaaggaaca cggtgacccg 660

ggggacacct ggaaggatta ttgcacactg gtcaccatcg ccaagagctt gttggacctg 720

aacaaatacc gacccatcca gaccccctcg gtgtgcagcg acagtctgga gagtcccgat 780

gaggatatag gatccgacag cgacgtgacc accgaatctg ggtcgagtcc ttcccacagc 840

ccggaggaga gacaggattc tggcagcgcg cccagcccgc tctccctcct ccactctgga 900

gtggcttcga aggggaaaca cgcctccgaa aagaggcaca agtgccccta cagtggctgt 960

gggaaagtct atggaaaatc ctcccatctt aaagcccatt acagagtgca tacaggtgaa 1020

cggcccttcc cctgcacgtg gccagactgc cttaaaaagt tctcgcgctc ggacgagctg 1080

acccgccact accggaccca cactggcgag aagcagttcc gctgtccgct gtgtgagaag 1140

agattcatga ggagtgacca cctcaccaag catgcccggc gccacaccga cttccacccc 1200

agcatgatca agagatcaaa aaaggctctt gccagcccct tgtgaggtgc tccccatatg 1260

gcaaccaggc agagatggtc ccggaaggac agagctccca ggaaacagac ggacacatgg 1320

aaatctgcca ccgcagagtt gtgctggcca caggaggtca ctgctctttg gtcaatattc 1380

tgacactctc cctgcgctgt ttccaaaaag cacacggtag cctaagatca aagtcaacat 1440

ttggtcccct tgcagaggcc actctgaacc gtctctgact gttggagggc aggcaaataa 1500

atgcttttgg tattttctcc tttgttttgg ggggtggggg gagggggcaa ggccaagact 1560

tggggtagga tttttgaaga ttcaggctgg tgtacatacg tctgactggg tgagttgacc 1620

cctggcctcc cacagtgact ctggatcctt gatgcttgct gactctcaga attgtttttc 1680

ttctctctca atgtcacgac cagagtgctt cagcttgttt agcagagcca ctctcgaatc 1740

acgttaactt ttgagattta aaaaacaaaa caaacaacaa aaaaaaaaaa aaaacccctc 1800

catagcacag ctgtctttta tgcaagcaag agcacaccta ctccagcatg atctgtcatc 1860

taaggacttc aaaacaaaac aaaacaaaaa aaaaagttac ttatagtcag tggataagca 1920

gagtccgaat ttacactaaa caagacagac cttcgagggg tcataagtcc ggaactttca 1980

aaccttgctt cgtatgaatc gtactatctg aacataaact gcacttttat tttctaatac 2040

cgagggtgaa tacggtaaat ccatgccttg agggtagaag ccgacattct gtttggcaca 2100

cgttattatc tgcttatttt ccaggataca catttccctg agaacccacg gcagagatgt 2160

gagggcagaa tgtatggaaa cacatgctga ggaggggaag gaggagggga gtgctttaaa 2220

gatacaaaaa aaaaaaaaaa aaaaaaaaga gcaaacaatt ttaacttcat gaatttgttt 2280

gaattttccc aagcttcaga taacaacgcc actcacggga ctaggacgct gcaagagcat 2340

tgggctctgt gtagcattag ctgataaatg ggtgtatagc catcaccaga cctggaaatg 2400

cccccttttg gtgggggtgg gggtgtttct tggtctctct ttcgttccta tgaaatttcc 2460

actaagaggt gcaagagtta tatcaaagga aagggaactg aaatatggaa aaaaaaaagt 2520

ttgcccctgt aagtagtctc tagggaagga agatgccaca atggtttcag aggcgactct 2580

gcagaacagt cccttctgct gaacataggt cagagaaaca ctcgtgaatg gcatcacatc 2640

gccaactgtc tgcatcatgg cccaccacta cttagcttgt atctcggtgt ctacacaatg 2700

gcctgaacca ctggtcacca t 2721

<210> 123

<211> 481

<212> DNA

<213> Rattus norvegicus (Norway rat)

<220>

<221>  misc_feature

<222>  (2005)..(2005)

<223>  n


<220>

<221>  misc_feature

<222>  (3503)..(3503)

<223>  n


<400>  123
caaccacgcc cgcaacggag ctgagcaacg ccgaagataa tggcaggctc ggcgttggcg      60

gttcgggctc ggctcggtgt ctggggtatg agggtcctgc aaacccgagg cttcggctcg     120

gactcgtcgg agagcatgga ttcgggcgct ggctccatcc gagaagctgg tggggccttc     180

gggaaacgag agaaggctga agaggatcgg tacttccgag agaagactag agagcagctg     240

gctgccttga agaagcacca tgaagatgag attgaccacc attcgaagga gatagagcgt     300

ctgcaaaaac agatcgaacg gcataagaag aagattaaat acctaaagaa tagtgagcat     360

tgagtgcaca cagtcagtct ctcacagagt ggccagtatc attccccact tctatagaga     420

ttgttttgaa tgattgatgt ttgacctgtg tgctactaac agataataaa ttatcaccag     480

g                                                                     481

<210>  124

<211>  2842

<212>  DNA

<213>  Rattus sp.


<400>  124
tcgcggaagg tgacgtggac acggaagtgg tcgtcgtcgc ggcggcaccg gtgggagcgg      60

ggccctgcac ttggagtgcg gcgggcaagc ggacgggtgg cggaggcctc tcagcggcgg     120

cggcggcgac ttaaggcgca ggcgtggtcc gttgggtgcg aatccgctga gcccacgagc     180

ggcctcttag ccctctctcc tgcccgtcgg aaaccgggag cagggacccg cttagccggc     240

gtcatcatga ccaagaccgg tagcaagggc gggaacctcc gcgacaagct ggacggcaat     300

gagctggacc tgagtctcag cgacctgaat gaggtccccg tcaaggagct ggctgcactt     360

ccaaaggcca ccgtgttgga tctgtcctgc aataaactga gcactcttcc gtcggatttc     420

tgtggcctca cgcacctggt aaagctggac ctcagcaaga acaagctgca gcagctgccc     480

gcagactttg gtcgcctggt taaccttcag catttggatc tcctcaacaa caggctggtc 540
accctgcctg tcagctttgc tcagctcaag aatctgaagt ggctggatct gaaggacaat 600
cccttggatc ctgtcctggc caaggtggca ggtgattgct tggatgagaa gcaatgtaag 660
cagtgtgcaa acaaggtgtt acagcacatg aaggccgtgc aggcagatca ggaacgagag 720
cggcagcgcc ggctggaagt ggagcgagag gcagagaaga agcgtgaggc caagcagcaa 780
gctaaggaag caaaggagcg cgagctgagg aagcgggaga aggcggagga gaaggagcgt 840
cggcgaaagg agtatgatgc tcagaaagct tccaagcggg agcaagagaa gaagcctaag 900
aaggaaacaa atcaggcccc aaaatcgaag tctggctctc gccctcgcaa gccaccaccc 960
cgaaaacaca atcgctcctg ggctgtgctg aaggggttgt tgctgctgct gctgctatgt 1020
gtagcaggag ggctggttgt atgccgggtg acagggctgc aacagcagcc cctctgcacc 1080
agcgtgaacg ccatctacga caatgccgtc cagggcctgc gccatcatga gatcctccag 1140
tgggtcctcc agaccgactc ccagcagtga gctcatcctc agcaccgctg cctcccagcc 1200
tcggagcttg gattcctatg gaattgggtt ctgctggaca caacttcttt ttagcgtcag 1260
acctacctgc catcatcaaa tggctgctga gtggtacttg agatctcccc tttgtaggac 1320
ttctctgttc cttagtcagg gttccctggt ggaatgagga gaaatggaga ggggggagga 1380
agagttacct gcatgcctaa aggaataggc ttaggggtgg ggagagagaa ggcataggct 1440
tttctagtta tgcaaagctg tgtaaggcaa ggttcctttc tactaaatgg tcagctgtca 1500
ctacatttat acttttgtat gtcacaaacc ctttctttca ttcctccctg ggtaaccagg 1560
acggattgga gggcagtgtg ttactgggac taggggacta ggaatacttg ggtaaattca 1620
gcctaagctg ggagggtaaa gtaatacatt tccttaaaga tctcagacag tcaagcattt 1680
tagcaatgtc caaaatgtct ggctatgaac acatgttcac tgccattggt ccagtgtaac 1740
actttgaggc aggaggtgcc gtccatgact tacttgccta cagtgttcaa gctagtccaa 1800
ggcacaaccc agctttcact ccagttttct tcctttcctt tatgtcattt ggcctccttt 1860
ataatactca aggggatgaa ctcacaccag agttgtctta gctaaagtga atctttcata 1920
atagacggtc ttaccaccca caaatagatc tcatcagggt cctgggaaac taatcctgtg 1980
gaattttgcc tcagcttaaa tggcttccac aaaatggcag caggctgggc tccttgcctc 2040
ccttttagag cattaaactc cctgatggcc tggaagcaca ggggcagatc tctgcagcgg 2100
cactgtgact gccctactag cacttggtat gatgaaatac ctcaaaggca acctagaaac 2160
ttgatctcac agaagcaggt gcagagttgc ttctggacct gtaacagaag ggaaggaata 2220
gaacagtggg agccaaaggg aaacaaagtc acacggtggc gctgcaagtg atacataagt 2280
aaacattagc acaaaccagg gcagcagcac ccacctccct gctgctacca gaaagcattc 2340
tccccgcttc cctgtctctt cacaacagct gcaggaaggg atcggaaacc tgtctcggtg 2400
cttatttgct aaaactccca actgcaagct ctccctagag gagcaggacc tgtcggagtt 2460
cagacagtgt agccccagtg gcccatgtgc ttaggtcagc cactcaagac tgtcctgaca 2520

cgggaagaaa ggcctttgtt tttccctccc ccagatagtt ctgccgtgta ggtccacacc 2580

ttactcagaa tcactacaca ttcctttagt cttcctccaa gctccagagc catcggtaca 2640

aatgctttat tgagacaaaa tacatactac atatggtgac atcatgaaaa cagaagtcag 2700

cctcatagat ccctggctgg ttgaggcagc tcagtggctg ggcgtagtca agccaacccg 2760

caggcaagag ttcactctga cttcgagatt tgatgcttat tctttggatt tctacaatta 2820

ttaaatccgt gtctgagtgg tc 2842

<210> 125

<211> 175

<212> DNA

<213> Rattus norvegicus (rat)

<400> 125
agccatcggt acaaatgctt tattgagaca aaatacatac tacatatggt gacatcatga 60

aaacagaagt cagcctcata gatccctggc tggttgaggc agctcagtgg ctgggcgtag 120

tcaagccaac ccgcaggcaa gagttcactc tgacttcgag atttgatgct tattc 175

<210> 126

<211> 1835

<212> DNA

<213> Rattus norvegicus (Norway rat)

<400> 126
aagcttgagt gtgagagtca gggtgtgtga gttggcacat gtgtaagagt gtgagagttt 60

atgagttctg taagatgtgt gtgtgaatgt gagttctgtg agtgtgacct gtgtgtgagc 120

gtgagcgtga gagggaggat gtggcggagt ctccacacac acatatccat ttgctgaaat 180

gaccctgttc atcagccaag ccagggacag caaatgcagt ctctgggcca tagtggaccc 240

catactcttt ttttttaatg taaataaaat gcacacacac aagtatgcat gcacatatgc 300

gtgaacacat acacacacaa gcatgcatgt atatgggtgt gtgtgtgtgc acgcaacata 360

catattcttg ataaagatat acctttactg gaacaaaaga ttgcagttag agtgttaaaa 420

tattctccaa gtagctgctt tcttttctct tcctctccaa ctgctgaaat tgaatggaca 480

gacttgtcca ccagtataaa ggcatatctg caattaacca acatataaaa aatgtcacat 540

tttgtctttg aaatcttttt agaggattta tgtactgatt ttctgacttc aggcatgact 600

ttttttttta ttaccaggga atttcatata tgcatgcaat gtgctttgct caaacccact 660

tctctctcac ctctcctttt caatttcttc attatcccct ctccactttt ccattcccaa 720

cttcatgtgc tcttttttgt ttttcttcct agccagccag atatccagca tgaatggggc 780

aggagctcga gaatcccacc catgtgagga gctactggtg actggggatg atagggagag    840

acggtcagtt ctcttcaggc ccctgagagg ctagcccacg aggcagaggg tagtcctaca    900

ccttgtatgt acaggcagca ccaaataaat gaagctgtgt tggaacacag ccacacccat    960

cctgcctgct atggaacccc ggggctcagg tgggtgattg cagtacatca gtcctacaac   1020

cctcaggcac ttgctctctg actcttgaaa ctttcagacc cccagctggg ctgggagggg   1080

agccaggaag aggggtgtga tgctcttcat actgctgttc tttgacagga caccagaggc   1140

tcccctcccc ctgtggaagg ttcacacctc caactgtcct gtccaccctc acagaaggac   1200

aaacatcgat cagggtcaaa ttgacaataa aagggctgga gcaagcaaca gggactcacc   1260

aaccaggcct cgcctctgag ttcagcccag agctagctgg gaaatgttcc ggatgttggc   1320

caaggccagt gtgacgctgg gctccagggc agcaagttgg gtacggaaca tgggctcgca   1380

ccagctgctg gtgccacccc cagaggccct gagcaagccc ctgtcaattc ctaagaggct   1440

cctgttgggt ccgggaccct ccaacctggc tcctcgtgtg ctagcagctg gaagtctgag   1500

gatgattggc cacatgcaaa aagagatgtt tcaggtaggc cttggtggca ggaggggtgg   1560

tgatcactgg gtagcaggca gaggagagga tctgacctgt gtgctcactg tctagatcat   1620

ggatgagatc aagcagggca tccagtatgt gttccagacc aggaaccccc tcacactggt   1680

tgtcagcggc tcaggacatt gtgccatgga gactgccctg ttcaacctcc tggagcctgg   1740

ggactccttt cttgtgggaa ccaatggcat ctgggggata cgggctgcag agatcgctga   1800

gcggattggt aagggagagt ggtctgtgac ctcgg                              1835

<210>  127

<211>  1570

<212>  DNA

<213>  Rattus norvegicus (Norway rat)

<400>  127
   gcggcggctg tatcccggac gttgttgccg gtggccggac ggcgcctgtg gggatttacg     60
   cgaaggcttc cgctccgcgc cgccgctgct cagccattgt actttggagg ggaccgactc    120
   agaagtacac aggctgctcc acaagttgtt ctgaatgttc ctgagacaca agtcacttgt    180
   ttggaaaatg gactcagggt agcttctgaa aactctggga tctcaacatg caccgttggg    240
   ctgtggatcg atgctggcag tcggtatgag aatgaaaaga acaatggcac cgctcacttt    300
   ctggagcaca tggctttcaa gggcactaaa aagaggtccc agttagacct tgaacttgag    360
   attgagaaca tgggtgccca tctcaatgcc tatacctcca gagagcagac tgtctactac    420
   gccaaagcct tctccaaaga tttgccaaga gctgtagaaa ttcttgctga cataattcag    480
   aacagtacat tgagagaggc agagattgaa cgtgagcgtg gagtcatcct cagagaaatg    540
   caggaagttg agaccaactt gcaagaagtt gtctttgatt atctgcacgc cacagcctat    600
   caaaacaccg cgctcggacg gacaattctg ggaccaaccg agaacatcaa atctataagc 660
   cgtaaggact tagtggacta cataaccaca cattataagg gaccaagaat cgtgctggct 720
   gctgctggag gtgtttgcca taacgaactg ctggagttag cgaagtttca ttttggtgac 780
   tctttgtgcg cacacaaagg agatgtacca gctctgcctc cctgtaaatt cactggaagc 840
   gagattcggg tgagggatga caagatgcca ctggcgcacc tcgcagtggc tattgaagca 900
   gttggttgga cgcacccaga cacgatctgt ctcatggtcg caaacacact gataggcaac 960
   tgggaccgct ctttcggagg aggaatgaat ttatcgagca agctggccca gctcacgtgc 1020
   catggcaatc tctgccacag cttccagtcc ttcaacacct cctacacaga cacaggattg 1080
   tgggggctct atatggtttg tgaacaagcc acagttgctg acatgctgca cgctgtacaa 1140
   aaggaatgga tgcgtctgtg cacagctgtt agtgagagtg aggtcgcacg cgccaaaaac 1200
   cttctcaaga caaacatgct gctgcagctt gatgggtcaa ctccgatctg tgaagacatc 1260
   ggtaggcaga tgctatgcta caacaggaga atccccatcc ctgagcttga ggccaggatc 1320
   gatgctgtgg acgcggagat ggttcgagaa gtgtgcacca agtacattta tggcaaaagc 1380
   ccggccattg ctgctctcgg tcctattgag cgcttaccag attttaacca gatttgtagt 1440
   aacatgcgct ggactcgtga ctgatttttg tgatgaatta tcctacacat acatatttat 1500
   aaaactgaga cctagaagtt ttgaaagact gctcatcttc aagtgaaaaa ataaaaactt 1560
   tatttgaaac 1570

<210> 128

<211> 2424

<212> DNA

<213> Rattus sp.

<400> 128
ggctgactga cagccttttt tttttttttt ttaatattta attggagata gggtctataa 60

cccaggctac cttgagctac tttatgcagc ttgggatgac ctagaacttc tgatcttcct 120

acttcaatct cccgaaagct gaggttactg gcatacacac catgcttcgt tagtagtctg 180

tgttggccta gaacctgcga caagcctttt gcctcagctc ccagagtact ggaatgatca 240

gcatacaaaa ccatacctga tttagcacgt gacctacctt ctgcccacta ttttgagaca 300

gggtctcact atctagtact ggttggccca taactcacta tgcaaactaa gctgtcttag 360

aactggcaga tccgtctgtc tctgcctccc aagggttgaa attgttttga aacaggttca 420

tctgcaacct agacctggaa ctcccagcaa tcttcctgcc tcggcctccc aaatgctgct 480

gggatgacag gcctctcatg tcttcacccc agtacagacc ttatgtgctt gcccctcagt 540

acagctagtg ctgattccct gaaaaatcac ctcttcctgc tactagtagc tcaaccagag 600

atagtcccgc ccataggcgt gattgatgag tcacacccac ctctcccaat tggttctact 660

cttccaggag gcggggacaa ggcaggggca gcagacgcca tccggaccag aaaaagccag 720

gagagaccag aaggtgcctg gtccctgaga gtctggtcca ctaactccag ggactgtggg 780

acagaggaac aaacaagaaa gaactcgagg cctcgctgag tggcgcgctt gctgagtgct 840

gccacatccc gcaagctgag caggtatccc aactctgttc ctgcccggta gaccacccga 900

ggtggtgagt gtggtcttgt cttccagatt cgtaggacag aagctccagg aggaggaccc 960

gcccaacatg gcatcggaga gcgggaagct atggggtggc cgatttgcag gctcggtcga 1020

ccccaccatg gacaagttca actcatctat cgcctatgac cggcatctgt ggaatgtgga 1080

cctgcaggga agcaaggcct acagcagggg cctggagaag gcagggcttc tcaccaaagc 1140

tgagatgcag cagatactgc aaggcctgga caaggtggct gaagagtggg cccaaggcat 1200

cttcaaattg taccctaatg atgaagacat ccacacggcc aacgagcggc gcctgaagga 1260

actcattggt gaagctgcag ggaagttaca cacaggcaga agtcgcaatg accaggtggt 1320

cacggacctc aggctgtgga tgaggcaaac ctgctcaaaa ctctccacct tcctcaaggt 1380

gctcattgaa gccatggtag accgggcaga ggcggagtgt gaagtcctct tccctgggta 1440

cacacacttg cagagagctc agcccatccg ctggagccac tggatcctga gtcacgccgt 1500

tgcgctgaca cgagatttag agagactgaa ggaggtgcag aagcggatca atgtcctgcc 1560

actgggcagt ggggccattg caggcaaccc tctgggtgtg gaccgggagt tcctctgtgc 1620

agaactgaac tttggagcca ttacgctcaa cagtatggat gccaccagcg agagagactt 1680

cgtggctgag ttcctgtttt gggcttctct gtgcatgacc catctcagca ggatggcaga 1740

agacctgatt ctctacggta ccaaggaatt caactttgtg cagctctccg atgcctacag 1800

caccggaagc agcttgatgc cccagaagaa aaacccagac agcttggagc tgatccggag 1860

caaggcgggc cgagtgtttg gacggtgcgc aggactcctg atgaccctca agggacttcc 1920

aagcacctac aacaaggact tacaggaaga caaggaggct gtgtttgaag tgtctgacac 1980

catgacagct gtcctccaag tagccactgg agtcatctct acactgcaga ttcatcgtga 2040

gaacatggca caggcactca gccctgacat gctggctacc gaccttgcct actacctggt 2100

ccgcaaaggg atgccattcc gccaggccca cgaggcctca gggaaagctg tggtcgtggc 2160

agagatgaaa ggggtggctc tcaaccagct gtcacttcag gagctgcaga ccgtcagtcc 2220

cctgttctcg agtgacgtga atctcgtgtg ggactacagc cacagcgtgg agcagtacac 2280

agccttgggt ggcacagcac agtccagtgt tgagtggcag atcagccagg tgcgggccct 2340

gctgcagatg tagcagccct agattccacc cagtcaaacg tgcgccccaa taaagtggag 2400

ccctggagag gccgccataa accc 2424

<210> 129

<211> 3751

<212> DNA

<213>  Rattus rattus (black rat)

<400>  129
agaaagagag ctacagactc cgcgcgctcc ggaagaccgg gtcgtagagc tcgagacaac      60

tcccgccagc agcaagacac gggagaccca accaggatga caaccctccc caggccgccc     120

gctccatgga acaccagctc ctgtgctgcg aagtggagac catccgccgc gcgtaccctg     180

acaccaatct cctcaaccga ccgggtctgc gagccatgct taagactgag gagacctgcg     240

cgccctccgt ttcttacttc aagtgcgtgc agagggagat tgtgccatcc atgcggaaaa     300

tcgtggccac ctggatgcta gaggtctgcg aggagcagaa gtgcgaagag gaggtcttcc     360

cgctggccat gaactacctg gaccgtttct tgtctctgga gcccctgaag aagagccgcc     420

tgcagcttct gggggccacc tgcatgttcg tggcctctaa gatgaaggag accattcccc     480

tgactgccga gaagttgtgc atctacactg acaactctat ccgccccgag gagttgctgc     540

aaatggaact gcttctggtg aacaagctca aatggaacct ggccgccatg actccccacg     600

atttcatcga acacttcctc tccaaaatgc cagaggcgga tgagaacaag cagatcatcc     660

gcaaacatgc acagaccttt gtggccctct gtgccacaga tgtgaagttc atttccaacc     720

caccctccat ggtggctgct gggagtgtgg tggccgcgat gcagggcctg aacctgggca     780

gccccaacaa cttcctctcc tgctaccgca caacgcactt tctttccaga gtcatcaagt     840

gtgacccgga ctgcctccgt gcctgccagg aacagattga agcccttctg gagtcaagcc     900

tgcgccaggc ccagcagaac atcgatccca aggccaccga ggaggaaggg gaagtggagg     960

aagaagctgg tctggcttgc acacccaccg acgtccgaga tgtggacatc tgagggccca    1020

ccgggcaggc gggagtcacc aagtagtggc atccgcgaag agaaaggagc cagcccgggt    1080

gctcctgacg aggtccccct tggggatgtg ttgttaccag aaggggaagt tttgttctct    1140

ttgttggttg tttttcctta atctttctcc tttctatctg atttaagcaa aagagaaaaa    1200

aatacctgaa agctgtctta aagagagaga gagagataga attcgcatca ccctgagtat    1260

agggagacgg gggggtgcct acaaaaatag aattctgtac cccagtaatc aactagtttt    1320

ctattaatgt gcttgtctgt tctaagaata ggattaacac acaggaagtc ttgagaagga    1380

tttttgattc ttttatgtgt ttaagaaaaa aaagcttaag aaacattgct ttaaaaaaag    1440

aaggaaaaaa aatacagcaa accattgtta aagtagaagg gttttaggta gagaaatgta    1500

ctctgctttg gtgaaaaggc acagatttgg gaaggtcctc aacctcactc ccctgcttgt    1560

tcagtgccta cagacctgtt acctgatgat acctgtgctt tatcccagga gtggggcaga    1620

cctcttaacc ttactgatgg tcgattcgac ctctagcggg ctcgtgggtg tggcaacctc    1680

cagggctcat gttatgggcc ctctgccccc cacaacctgc aggttcacag agcgccagcc    1740

agcacgcagg ggtagggatg aaatagtgac ataatatatt ctatttttgg ggaaccttcc    1800

tgttttgggg gttcctgttt agagagatgc tggtttttgg ctggtggccc tgcagcccac    1860

tcaacatcag gttgaaccca cagcttttct tttgtgtgtg gtttgttttg ttatgttttt    1920

ccttctccat gttcccaaaa ccattccatt tcaaagcact tttggtcagc tagctggagg 1980

cagtgttgct gggtgtgtgt gtgtgtgtgt gtgtgtgtgt gtgtgtgtgt gtgtgtcggt 2040

gggggagggg gtctagtgga atggttgggg atgtccacac actcaattct gatggctgca 2100

cagcaggtgt gtagggctgg tagcatgagg tgcttgggaa gttgtgttgg gtcgagaaga 2160

gaaagctctg ttctcgtacc accgggatct tcctgcaaag tcggagggat cttttggtgc 2220

cagctggcgt ttggaagtag ggaccgtgat ggcattacct ggacaagggg attcaggacg 2280

actcttaagt ctttcacaca ggaggctttt aaacactaaa atctctaatt tatacgtaag 2340

gctacagaag agtatttatt gggaaggttg cccatgaccc gtgtgactca aagcaatgtg 2400

atcttccctt gattcaaacg cacaccttct tccctgctgg cgaaggtttt aggccattgt 2460

ctgagagact ggtcttttat tggccaagtg gggtgtcctc caaaaaaacc aaaccacaaa 2520

gacggagatg tgggtctcct tgactttccc aacccaattg cccccattgg agagccatcc 2580

aaacctgagg aaaattaggg gaccccaaaa gagtcttgag tctggcacat tcttgccgcc 2640

gctcccaagt tagcaacagt aggtaatttg cacacctctg gctctgtgcc tttctgttca 2700

ggatgtgttg gtgggaggtg gagagcgggt ggctggagag gggatgtgag agaagaagta 2760

tggagggtag ggaccacacg ggacagaccg cggctccttt cacagcgctg ctaccaatga 2820

ctcccaggat ccccggcgtt cggaaccaga ttcacgttgc tttgtatctt tcatgttgtt 2880

ttcgctgcta ttggagggtc agttttattt tgttgttgtt ggtttgtttg ttttttttta 2940

caatgtcata aactgccatg ttcaagtttt aatttcctcg tagaagagtg tatttacaga 3000

tgccctctcc gggacttttt ctatttattg tgattcaatt ttggattaat gtgattaccg 3060

ctgtattccc aacaaaaggt gttttccaca cacacacaca cacacacaca cacacacaca 3120

cacacacaca ggttcctgta cacaatacct catgtatcac ctaacaatac ataggcctgc 3180

caggcgggtg gggcggtctg cctccaggga ccctgggacc ctgctgggga tcatcctgtc 3240

atgctgggcc ttcatttgat ctgggacata gcatcacagc agtcaggcca actgtgttct 3300

gttagttatc aatattgtta cttgtagcgg cctgttgtgc atgccaccat gctgctggac 3360

ccggagagat ttgttctgag tctctggtgc atcatataat ctgttaggtt ctagtgttct 3420

gtcttgtttt gtgttactca cagcattgtg ctaatgtaaa gccagccgca atgctgtagg 3480

ccccaggttc cctagcaagc tgccaaacca aaagggtcac caccagctta gctgaggcgt 3540

cccaaccagg caggaccctt gagggctgct gtgtccatgg tgatggggtg aagttttggc 3600

caaagggcca aaggctggtg gatccacaca gtctgccctg tgacatgaat ggctttgagg 3660

ggctctggct ggtggtcagg ttggctttgt gtattctggt tgacacacca tggcgcttcc 3720

cagcacagac atgtgaccag catggtccag g 3751

<210> 130

<211> 840

<212>  DNA

<213>  Rattus sp.

<400>  130
gccacttctg gcactgggaa acgcttcctc agccacacca tggagcaagg aacggacttg      60

cagtcacaga ttttcttccc tacagaaaag aatttttgga aaaaaggcaa agaccacttc     120

aggcaaaata agtacccatt ctcgagggaa ctttataacc tgatttttgc tgacagaaag     180

ggaaatgagg cgagttaccc tttggaaatg tgctcacact tcgatgctga tgagattaaa     240

aggctaggaa agagattcaa gaagcttgac ttggacaact ctggttcttt gagcgtggag     300

gagttcatgt ctctgcctga gttacaacag aaccctttag tacagcgggt catagatata     360

ttcgacacag acgggaatgg agaagtggac ttcaaagaat tcattgaagg agtctctcag     420

ttcagtgtca aaggcgataa ggaacagaag ttgaggttcg ctttccgtat ctacgacatg     480

gataaagacg gctatatttc caatggagag ctcttccagg tgttgaagat gatggtgggc     540

aacaacctga aagatacgca gttacagcag attgtagaca aaaccataat aaacgcagat     600

aaggacgggg acgggagaat atcctttgag gagttctgtg ctgttgtagg tggcctagat     660

atccacaaaa agatggtggt ggatgtgtga ctctttgaag actctaccac ccagcacttt     720

tgctttcttc tccatctctg aagatctgct caagacgtcc agcagtgctc tctgtgtgtg     780

taaatggaag tatttttctc tgtgaagcca cattttccaa catgagcctc ataaaaaaaa     840

<210>  131

<211>  128

<212>  DNA

<213>  Rattus norvegicus (Norway rat)

<400>  131
cctcttgtac caggtccttg acaacctaga cagccacctg aagaggtctg agtacttccg      60

cttcctctgg tttcctcaca ctgagaacgt cagcatcatc taccaagacc acaccaacaa     120

ggtaactg                                                              128

<210>  132

<211>  1000

<212>  DNA

<213>  Rattus norvegicus (Norway rat)

<400>  132
ctggaatcct aacagcatct acacaaagct atttataatg ccagactata cttggtttga      60

aggaatacct tttcatgcct ttgggatttc aaaagaaact ttgcaaaatg tttgtaataa     120

gtttgtggtg aaagaagaag atttgatttt attggcttat cccaagtcag gaacgaactg 180

gctgattgaa attgtctgct tgattcagac caagggagat cccaagtgga tccaatctgt 240

gactatctgg gatcgctcac cctggataga gactgatgta ggatatgata tattaatcaa 300

aaagaaagga ccacgactca tgacctccca tcttcccatg catcttttct ccaagtctct 360

cttcagttcc aaggccaagg tgatctatct catcagaaat cccagagatg ttcttgtttc 420

tggttattat ttctggggta attcaactct tgtgaagaag ccagactcac tgggaactta 480

tgttgaatgg ttcctcaaag gaaatgttct atatggatca tggtttgagc acatccgtgc 540

ctggctgtcc atgcgagaat gggacaactt cttgttactg tactatgaag acatgaaaaa 600

ggatacaatg ggaaccataa agaagatatg tgacttcctg ggaaaaaaat tagagccaga 660

tgagctggat ttggtcctca agtatagttc cttccaagtc atgaaagaaa atgacatgtc 720

caattatagc ctcctgatga aaaaatcaat tttactggga attggcctca tgaggaaagg 780

cactattggg gactggaaga atcacttcac tgtatcccaa gctgaagcct ttgataaagt 840

gtttcaggag aaaatggccg gtttccctcc agggatgttc ccatgggaat aaaatttcaa 900

aagttttaaa tattttatga acattgatat ttatgtttct gttgttctat gtctgaataa 960

ctgaatgtgg tcatcgaata aatcctgttc tggtattgtg 1000

<210> 133

<211> 828

<212> DNA

<213> Rattus sp.

<400> 133
aattccgttg ctgtcggaaa gccagaaccg gtttgtaggc agctcagtgg agatcgggtt 60

cctattacag tgcaatcatg tctgttatgt cctataatgg aggggccgtc atggccatga 120

agggaaagaa ctgtgtggcc atcgctgcag acaggcgttt cggaatccag gcccaaatgg 180

tgaccacgga cttccagaag atctttccca tgggtgacag gctctacata ggcctggccg 240

gcctggccac tgacgtccag acagttgccc agcgtctcaa gttccgactg aacctgtacg 300

agctgaagga aggacggcag atcaagcctt acaccctgat gagcatggtg gccaacctct 360

tgtatgagaa acggtttggt ccctactaca cagaacctgt cattgctggc ttggacccga 420

agacctttaa gcccttcatt tgctctctgg acctcattgg ctgtcccatg gtgacagatg 480

acttcgtagt cagtggcacc tgctctgaac aaatgtatgg gatgtgtgag tctctctggg 540

agcccaacat ggatccagag cacctgtttg agaccatttc tcaggccatg ctgaatgctg 600

tggacaggga tgccgtgtcg ggcatgggcg tcattgtcca catcattgaa aaagacaaga 660

tcaccaccag gacactgaag gcgcggatgg actaacctgt gctctggccc cgtctccctt 720

ttgttgtttt tctttttaat aaaatctcct ttctttcatg cctgcccgcc cttgtttctg 780

cgactttgtc cccatcaggg agagcccaat acagagcgac tttatctc 828

<210> 134

<211> 1018

<212> DNA

<213> Rattus norvegicus (Norway rat)

<400> 134
gctggaacgg gtccgtacgg cgttgttctt ggttcccatc gtaacttaaa agggaaactt 60

acacaatgtc cggaggcctt gacgtcctgc agatgaagga ggaggatgtc ctcaaattcc 120

ttgctgcagg aacccactta ggtggcacca accttgactt tcagatggag cagtacatct 180

acaaaaggaa aagtgacggt atctacatca tcaacctgaa gaggacttgg gagaagctgt 240

tgttagccgc tcgagctatt gttgccattg agaaccctgc tgatgtcagc gtcatctcct 300

ccaggaacac tggccagcga gctgtgctga agtttgccgc tgccacagga gccactccaa 360

ttgctggccg cttcacacct gggaccttca ctaaccagat ccaagcagcc ttcagggagc 420

cccggcttct agtggtgact gatccccggg ctgaccacca gcccctcaca gaggcctctt 480

acgtcaacct gcccaccatt gctctttgta acacagattc tcccctgcgc tatgtggaca 540

ttgccatccc atgcaacaac aagggagctc actcagtggg tctcatgtgg tggatgctgg 600

ccagggaagt actccgcatg cgaggaacca tctcccggga gcacccatgg gaggttatgc 660

ctgatcttta cttctacagg gacccagagg agattgagaa ggaggagcag gctgccgctg 720

agaaggctgt gaccaaggag gaattccagg gtgaatggac ggcaccagcg cctgagttca 780

ctgctgctca gcctgaggtg gccgactggt ctgagggtgt gcaggtgccc tctgtgccca 840

ttcagcagtt ccccacagaa gactggagtg cacagccggc cactgaggac tggtcagcag 900

ctcccacagc acaggctact gagtgggttg gagccaccac tgagtggtcc tgagctcctc 960

tgcaggtgcc tgagcgaagg gaaaaaaggt ggaaggaaaa taaagttcct gaaagctg 1018

<210> 135

<211> 514

<212> DNA

<213> Rattus norvegicus (rat)

<400> 135
agaggcctct tacgtcaacc tgcccaccat tgctctttgt aacacagatt ctcccctgcg 60

ctatgtggac attgccatcc catgcaacaa caagggagct cactcagtgg gtctcatgtg 120

gtggatgctg gccagggaag tactccgcat gcgaggaacc atctcccggg agcacccatg 180

ggaggttatg cctgatcttt acttctacag ggacccagag gagattgaga aggaggagca 240

ggctgccgct gagaaggctg tgaccaagga ggaattccag ggtgaatgga cggcaccagc 300

gcctgagttc actgctgctc agcctgaggt ggccgactgg tctgagggtg tgcaggtgcc 360

ctctgtgccc attcagcagt tccccacaga agactggagt gcacagccgg ccactgagga 420

ctggtcagca gctcccacag cacaggctac tgagtgggtt ggagccacca ctgagtggtc 480

ctgagctcct ctgcaggtgc ctgagcgaag ggaa 514

<210> 136

<211> 1604

<212> DNA

<213> Rattus norvegicus (Norway rat)

<400> 136
cgagcggact ctctagccct tgtgggtccc tgttcagcgc ccggccgtgt ggagacccgg 60

gagccagtcc gggcctccga ggcgaagatg gtggactaca gcgtttggga tcacatcgag 120

gtgtcggacg atgaggacga gacgcacccc aacatagaca cggccagcct gttccgctgg 180

cggcaccagg cccgggtgga acgcatggaa cagtttcaga aggagaaaga agaactggac 240

cggggctgcc gggaatgcaa gcgcaaggta gccgagttcc agcgcaagct gaaggaactg 300

gaagtggctg agggtggtgg ccaggtggag cttgagcggc ttcgagcgga ggcacagcag 360

ctgcgcaagg aggagcgaac tgggagcaga agctggagga catgcggcaa aaaggagaag 420

aacatgccct ggaatgtgga cacactcagc aaagatggct tcagcaagag catggtcaat 480

accaagcctg agaaggcaga ggaagactca gaggaggcaa gggagcagaa acacaagacc 540

ttcgttgaaa aatacgagaa acagatcaaa catttcggca tgctccaccg ctgggatgac 600

agccagaagt acctgtcaga caatgtccac ctggtgtgcg aggagacagc caactacctg 660

gttatctggt gcattgacct ggaagtcgag gagaagtgtg ccctgatgga gcaggtggct 720

caccagacca tggtcatgca gttcatcctg gagttggcca agagtctgaa ggtcgacccc 780

cgggcctgct tccggcagtt tttcaccaag atcaagactg ctgaccagca gtacatggag 840

ggcttcaagt atgaactgga ggcctttaag gagcgtgtgc ggggccgcgc caagctgcga 900

atagagaagg ccatgaagga gtacgaggag gaggagcgca agaagaggct aggccctggt 960

ggcctggacc ctgtggaggt ctacgaatct ctgcctgagg agctgcagaa gtgctttgat 1020

gtgaaggatg tacagatgct gcaagacgcc atcagcaaga tggatcctac tgatgccaag 1080

tatcacatgc agcgttgcat cgattctggc ctctgggtcc ccaactccaa gtctggtgag 1140

gccaaggagg gggaggaggc gggccccggg gacccattgc tggaagccgt ccccaaagcg 1200

ggcttcgaga aagacatcag tgcgtgaccc ttctccagaa aaggaaaccc agccaccatg 1260

tccctagctc ccgacttctt cacttctgct gctcctcacg tcctggccca tggcctgccc 1320

catgtcccct ctcctccggc ctgtccccag cactcatcag attctctgct tcgggttgag 1380

gggctctgct gtctgcagcc cccccacccc catcagcgtt atgccaaagt cctgggggtc 1440

tggggagggg cagcagctgc caggttggtc caccgggttg gggatacaca tgtccccatc 1500

tgggcagggt ctgtccctat gggtctatgg tcactgtttc tcttctgtct agctttctgt 1560

cctgcaactg gcaacaatgt ccaataaaca gttccaggtg ctgg 1604

<210> 137

<211> 1500

<212> DNA

<213> Rattus norvegicus (Norway rat)

<400> 137
taaccgcgcc aaccgccacc gaggtgcccg gagagaggcg gagaggcggc atgagcgagg 60

cgggcgaggc caccaccggc ggcaccacgc acccgcaggc cgcggccgac gcgcccgccg 120

cggcgccccc ggaccccgcg cctaagagcc cggcggccag cggcgcgccc caggccccgg 180

cgcccgccgc gctgctcgcg ggagccccgg cgagagccag cccccgggcc cgccccggcc 240

tcatcagccc ccgcgggaag cgaggacgcg agaagaaagt tctcgccacc aaagtccttg 300

gcactgtcaa atggttcaac gtcagaaatg gatatggatt tataaaccga aacgacacca 360

aagaagatgt gtttgtacac cagactgcca tcaagaagaa taaccacgtc aagtatctgc 420

gcagtgtggg ggatggagaa actgtagagt ttgatgtggt tgaaggagaa aagggtgctg 480

aagcagcaaa tgtgactggc ccagatggag ttcctgtaga agggagtcgc tatgctgctg 540

atcggcgccg gtacagacgc ggctactatg gcaggcgccg aggacctccc cgtaatgctg 600

gtgagattgg agagatgaag gatggagtcc ccgagggagc gcagctccag gttcatcgga 660

atcccactta ccgcccaagg ttccgcaggg gacctgctcg cccacgacct gcccctgcta 720

ttggagaggc tgaagataaa gaaaatcagc aagcggccaa tggtccaaac cagccgtctg 780

cccgccgtgg attccgacgc ccctacaact acaggcgccg cccccgtccc ctcaacgctg 840

tttcacaaga tggcaaagag accaaggcag gtgaagcacc aactgagaac cccgctccag 900

ccaccgaaca gagcagtgcc gagtgaccct ggctcccagg caccttcacc accagcaggg 960

tgaccttaag aattaatgac cattcaaaaa caaggcaaaa agcacaccca cgaccttacc 1020

aacaccaaag aaacatctaa gcaataaaac ggaagactaa caagatttgg acattagaat 1080

gtttactgct attctctacg aaactaacaa ctgcaaaggg aaggagcccg cactgtccat 1140

caagctgcgt cccgggaacc tgcacaggca gagagcagcc tccccatttc agcaacctag 1200

tgctttatat ttttttcctg gtttttactg ttttggtaat atgaattaaa agaagaaata 1260

ttaataccac atggggattg ccccaaccaa agaaatctga aatatatagt aaatgctctt 1320

tttcctttgt tgttcatttt ggatgctggt gctaaacttc caagtgtcat gatttaagaa 1380

gaaattttat gcccttattt attcctagga tgaggggaga acatttttgc tttcttacat 1440

agctctctct gaaatgtgca gtaacaagtt cctcaaaaat aaaattttta ccttcaaaga 1500

<210> 138

<211> 3216

<212> DNA

<213> Rattus norvegicus (Norway rat)

<400> 138
ggtacccaac agcctgaact cagagccccg agagcagagc attcagggca agcagaaaca 60

ccctgcagag gctttccaag aatccctcgg catggcaaga caaggctgtc tcgggtcatt 120

ccaggtaata tccttgttca cttttgccat cagtgtcaat atctgcttag gattcacagc 180

aagtcgaatt aagagggcag aatgggatga aggacctccc acagtgctgt ctgactctcc 240

atggaccaac acctctggat cctgcaaagg tagatgcttt gagcttcaag aggttggccc 300

tccagactgt cggtgtgaca acctgtgtaa gagctacagc agctgctgcc acgatttcga 360

tgagctctgt ttgaaaacag tccgaggctg ggagtgcacc aaagacagaa gtggggaagt 420

acgaaacgag gaaaatgcct gtcactgccc agaagactgc ttgtccaggg gagactgctg 480

taccaactac caagtggtct gcaaaggaga atcacactgg gtagatgatg ctgcgagaaa 540

tcaaagttcc gaatgcctgc aggtttgtcc gcctccgtta atcatcttct ctgtggatgg 600

tttccgtgca tcatacatga agaaaggcag caaggttatg cccaacattg agaaactgcg 660

gtcctgtggc acccatgtcc cctacacgag gcctgtgtac cccacaaaaa ccttccctaa 720

tctatatacg ctggccactg gtttatatcc ggaatcccat ggaattgtcg gtaattcaat 780

gtatgatcct gtctttgatg cttcgttcca tctacgaggg cgagagaagt ttaatcatag 840

gtggtgggga ggccaaccgc tatggattac agccaccaag caaggggtga gagctggaac 900

attcttttgg tctgtgagca tccctcatga acggaggatc ctaaccattc ttcagtggct 960

ttctctgcca gacaacgaga ggccttcagt ttatgccttc tactcagagc agcctgattt 1020

ttctggacac aagtacggcc cttttggccc tgagatgaca aatcctctga gggagattga 1080

caagaccgtg gggcagttaa tggatggact gaaacaactc aggctgcatc gctgtgtgaa 1140

cgttatcttt gttggagacc atggaatgga agatgtgaca tgtgacagaa ctgagttctt 1200

gagcaactat ctgactaatg tggatgacat tactttagtg cctggaactc tgggaagaat 1260

tcgagccaaa tctatcaata attctaaata tgaccctaaa accattattg ctaacctcac 1320

gtgcaaaaaa ccggatcagc actttaagcc ttacatgaaa cagcaccttc ccaaacggtt 1380

gcactatgcc aacaacagaa gaattgaaga catccattta ttggtcgatc gaagatggca 1440

tgttgcaagg aaacctttgg acgtttataa gaaaccatca ggaaaatgtt ttttccaggg 1500

tgaccacggc tttgataaca aggtcaatag catgcagact gttttcgtag gttatggccc 1560

aacttttaag tacaggacta aagtgcctcc atttgaaaac attgaacttt acaatgttat 1620

gtgcgatctc ctaggcttga agcccgctcc caataatgga actcatggaa gcttgaatca 1680

cctactgcgt acaaatacct ttaggccaac catgccagac gaagtcagcc gacctaacta 1740

cccagggatt atgtaccttc agtccgagtt tgacctgggc tgcacctgtg acgataaggt 1800

agagccaaag aacaaattgg aagaactcaa taaacgtctt cataccaaag gatcaacaga 1860

agctgaaacc gggaaattca gaggcagcaa acatgaaaac aagaaaaacc ttaatggaag 1920

tgttgaacct agaaaagaga gacatctcct gtatggacgg cctgcagtgc tctatcggac 1980

tagctatgat atcttatacc atacggactt tgaaagtggt tatagtgaaa tattcttaat 2040

gcctctctgg acatcgtata ccatttctaa gcaggctgag gtctccagca tcccagaaca 2100

cctgaccaac tgtgttcgtc ctgatgtccg tgtgtctcca ggattcagtc agaactgttt 2160

agcttataaa aatgataaac agatgtcata tggattcctt tttcctccct acctgagctc 2220

ctccccagaa gctaagtatg atgcattcct cgtaaccaac atggttccaa tgtaccccgc 2280

cttcaaacgt gtttgggctt atttccaaag ggttttggtg aagaaatatg cttcagaaag 2340

gaatggagtc aacgtaataa gtggaccgat ttttgactac aattacgatg gcctacgtga 2400

cactgaagat gaaattaaac agtatgtgga aggcagctct atacctgtcc ccacccacta 2460

ctacagcatc atcaccagct gcctggactt cactcagcct gcagacaagt gtgacggtcc 2520

cctctctgtg tcttccttca tccttcctca ccgacccgac aatgatgaga gctgtaatag 2580

ctccgaggat gagtcgaagt gggtagagga actcatgaag atgcacacag ctcgggtgcg 2640

ggacattgag cacctcactg gtctggattt ctaccggaag actagccgta gctattcgga 2700

aattctgacc ctcaagacat acctgcatac atatgagagc gagatttaac tttctgggcc 2760

tgggcagtgt agtcttagca actggtgtat atttttatat tgtgtttgta tttattaatt 2820

tgaaccagga cacaaacaaa caaagaaaca aacaaataaa aaaaaaaacc acttagtatt 2880

ttaatcctgt accaaatctg acatattaag ctgaatgact gtgctatttt ttttccttaa 2940

ttcttgattt agacagagtt gtgttctgag cagagtttat agtgaacact gaggctcaca 3000

atccaagtag aagctacgtg gatctacaag gtgctgcagg ttgaaaattt gcattgagga 3060

aatattagtt ttccagggca cagtcaccac gtgtagttct gttctgtttt gaaagactga 3120

ttttgtaaag gtgcattcat ctgctgttaa ctttgacaga catatttatg ccttatagac 3180

caagcttaaa tataataaat cacacattca gatttt 3216

<210> 139

<211> 419

<212> DNA

<213> Rattus norvegicus (rat)

<400> 139
gggcctgggc agtgtagtct tagcaactgg tgtatatttt tatattgtgt ttgtatttat 60

taatttgaac caggacacaa acaaacaaag aaacaaacaa ataaaaaaaa aaaccactta 120

gtattttaat cctgtaccaa atctgacata ttaagctgaa tgactgtgct attttttttc 180

cttaattctt gatttagaca gagttgtgtt ctgagcagag tttatagtga acactgaggc 240

tcacaatcca agtagaagct acgtggatct acaaggtgct gcaggttgaa aatttgcatt 300

gaggaaatat tagttttcca gggcacagtc accacgtgta gttctgttct gttttgaaag 360

actgattttg taaaggtgca ttcatctgct gttaactttg acagacatat ttatgcctt 419

<210> 140

<211> 2744

<212> DNA

<213> Rattus norvegicus (Norway rat)

<400> 140
tgaggacagt tcctttttcc ctccctagag gaaagaaaga gccttatttt ctggctaaac 60

aggtttacac agctacagga acaatggatt ccaggctagc tttagccacg gaggagccca 120

ttaagaaaga cagcctgaag agatataaga tcctttgtgc ggttcttctt gctttgctgg 180

tgatcgtgtc gcttggattg ggcctgggcc tgggactcag gaaacctgaa gagcatatag 240

gcagctgcag gaaaaaatgc tttgattcgt cacacagagg actggagggt tgccgctgtg 300

actccggatg cacagaccgg ggagactgct gctgggactt tgaagatacc tgtgtgaaat 360

caactcaaat ctggacgtgt aattcgttcc gctgtgggga gaccagacta gagacagccc 420

tctgttcctg tgcggatgac tgtctgcaga ggaaggactg ctgcactgac tacaaggctg 480

tgtgccaagg agaagtccca tgggtgacag aagcctgtgc ctcatcccag gagccccagt 540

gtccagaagg gtttgaccag ccgccggtta tcttgttctc catggacggc tttagagctg 600

agtacttaca aacatggagc accctgctgc caaacattaa caaactgaaa acatgtggac 660

tccattccaa atatatgaga gcagtgtatc ccaccaaaac ctttccaaat cattatacca 720

ttgtcacggg tttgtatcca gagtcgcacg gcatcattga caataacatg tatgatgtgt 780

acctcaacaa gaatttttca ctttcttcag tggagaaaag taaccctgcc tggtggagtg 840

ggcagccgat ttggttaaca gcaatgtatc aaggcttaaa agctgcttcc tactactggc 900

caggatcaga tgtggctgtc aatggctcct tccctaacat ctacaggaat tacagcaatt 960

ccgtcccata tgaaagtagg attgctacac tgttgcaatg gctggacctg cccaaagctg 1020

aacgacccag tttttacacc atatatgtgg aagagcccga ttctgcgggg cacaagagtg 1080

gaccagtcag tgctggagtc attaaagcct tacagttagt tgatgatgcc tttgggatgt 1140

tgatggaggg tttgaaacag aggaatttac acaactgtgt caacataatc gttctggctg 1200

atcatggaat ggaccagacc tcttgtgaca gagtggaata catgacagat tattttcctg 1260

aaataaactt ctacatgtac caaggacctg cccctcgaat caggactcgt aatatacctc 1320

aggacttctt tacctttaat tccgaagaga ttgtcagaga cctcagctgt cgaaaatccg 1380

atcaacactt caaaccctac ttgactccgg atttgccgaa acgactgcac tatgccaaga 1440

acgtcaggat tgacaaagtt catctaatgg tggatcggca atggctggct tacaggaaca 1500

aaggagttcc caactgcgaa ggcgggacac acggttacaa caatgaattt aaaagtatgg 1560

aggcaatctt tttggcacat ggacccagct ttaaagagaa gactgttatc gaaccatttg 1620

aaaacatcga agtctataat ctactgtgtg atcttctgca catccaaccg gcaccaaaca 1680

acggttctca cggtagcctg aatcatcttc tgaaggcacc attttaccag ccgtcgcatg 1740

cagaagaatt gtcaaagtct gctggctgtg gttttactac tccattgcct aaggactcac 1800

taaactgctc ctgcctcgca ctgcagactt ctggtcaaga agagcaagtc aaccaaagac 1860

taaatctcaa tagaggagag gtctcggcaa cagagaagac aaatttgcca tttgggaggc 1920

ccagggtgat acagaagaac aaagaccact gtctgcttta ccacagggaa tacgtcagtg 1980

ggtttgggaa agctatgaag atgcccatgt ggagttcata cactgttccc aagccgggag 2040

acacatcatc tcttcctccc acagttccag actgtctgcg ggctgatgtc agggttgatc 2100

cttcggagag ccaaaaatgt tccttctatt tagcagacca gaatatcgac catggcttcc 2160

tctatcctcc cgcaatcaaa ggaaataatg agagtcaata tgatgcttta attacaagta 2220

atttggtccc aatgtataaa gaattcaaaa aaatgtggga ctacttccat aaagttctcc 2280

ttataaaata cgctatagaa agaaatggag tgaatgtggt tagtggaccc atatttgatt 2340

acaattatga tggccatttt gatgcccccg atgaaatcac aaactatgta gctggcaccg 2400

atgttcctgt cccgacacac tactttgtgg tactgacaag ctgtaaaaac aaaacccaca 2460

cacccgacag ctgccctggg tggctggatg tcctgccctt tgtcgtccct caccggccca 2520

ccaaacccgt ggaaagctgt cctgaaaaca aagcagaaga cctttgggtt gaagagagat 2580

ttaaagcaca catcgcccgg gtccgtgatg tggagctcct cactggcctt gacttttatc 2640

aggaaaaaac acagcccgtc tctgaaattt tgcagctgaa gacatatttg cccacattcg 2700

aaaccattat ttgaatagat gtgtaaagcc atttgacaaa atga 2744

<210> 141

<211> 2779

<212> DNA

<213> Rattus norvegicus (Norway rat)

<400> 141
tgtaaaactt gattcccgtt gagatctgtt gattgtattt ttgagcacat gaataaccac 60

gtatcttcaa caccgtctac catgaagcta aaacaaacca tccaccccat acttttatat 120

ttcatacatt ttataatatc actctatact attttaacat acatcccatt ttattttttg 180

tgtgagtcaa aacaagagaa accaaaccac attaaagcaa agcctgtcag ttcaaaaccg 240

gactctgcat acaggtctgt caacagtatg gatggcttag cttcagtatt gtatcctggc 300
tgcgacacac ttgataaagt ttttatgtat gcaaaaaaca aatttaagga caaaagacta 360
ttgggaacac gtgagatttt gaatgaggaa gatgaaatac aaccaaatgg aaaggttttt 420
aaaaaggtta ttctggggca ctataattgg ctttcctatg aagatgtctt cattcgagcc 480
ctcgattttg gaaatgggtt acaaatgttg ggccagaagc cgaaggccaa catcgccatc 540
ttttgtgaga ccagggctga gtggatgatt gctgcgcagg cgtgtttcat gtacaacttc 600
cagcttgtta cactgtatgc gactctggga ggtccagcca ttgtccatgg actgaatgag 660
acagaggtga ccaacatcat tactagtaaa gaactcctgc aaacaaagct gaaggatatc 720
gtctctttgg tcccacgtct gcggcatatc attactgttg atgggaagcc tccaacctgg 780
tctgagttcc ccaagggcgt cattgtacac accatggctg cagtgcaggc tctaggagta 840
aaggctgacg tggacaagaa agctcacagc aaaccactgc cctcagatat tgcagtaatc 900
atgtacacaa gtggatccac aggaattcca aagggagtca tgatctcaca cagcaacatc 960
attgcctcta taacggggat ggcgagaagg attccaagac tgggagagga agatgtatac 1020
attggatatt tgcccctggc acatgttcta gaattaagcg ctgagcttgt gtgtctttct 1080
catggatgcc ggattggcta ctcttcacca cagacattag cagatcagtc ttcaaaaata 1140
aagaaaggaa gcaaaggaga cacatccgtt ctgaagccca cgctgatggc agctgtgccg 1200
gaaatcatgg atcggatcta caaaaatgtc atgaataaag tgaatgaaat gagtgctttt 1260
caacgaaact tgtttatttt ggcatataat tataagatgg agcagatttc aaaagggtgt 1320
agtaccccgc tgtgtgaccg ctttgttttc cggaatgtcc gaaggctgct gggtggaaat 1380
attcgcgttt tattgtgcgg tggtgctcca ctttctgcaa cgacacagcg attcatgaat 1440
atctgcttct gttgtcccgt tggccagggg tatggactca cagaatctac tggggctgga 1500
acaattacag aagtgtggga ctacaatacc ggcagagtgg gagcaccatt agtttgctgt 1560
gaaatcaaat taaagaactg ggaggaaggt ggctatttta atactgacaa accacatccc 1620
agaggtgaaa ttctgattgg tggccaaaat gtgacaatgg ggtactacaa aaatgaagca 1680
aaaacaaagg ccgatttctt tgaagatgaa aacggacaga ggtggctgtg cactggcgat 1740
attggagagt ttgaccctga tggctgcctc aagatcattg atcgtaaaaa ggaccttgtg 1800
aaactacagg caggagagta tgtttctcta ggcaaagttg aggcagcttt gaagaacctc 1860
ccactgatag ataacatttg tgcatatgca aacagttacc attcttacgt aattggattt 1920
gttgtgccaa atcaaaagga acttacggag ctagctagaa cgaaaggatt taacggaact 1980
tgggaagagc tgtgtaacag cagtgaaatg gaaaacgagg tccttaaagt gctttctgag 2040
gctgctattt cagcaagtct ggaaaagttt gaaatcccac tgaaaattcg tttgagccct 2100
gacccatgga ctcccgaaac tggtctggtg actgatgcct tcaagttgaa acgtaaagaa 2160
cttaaaacac actaccaggc agacattgag cggatgtacg gaagaaaata attagtttgg 2220
gcattggttt gctacagtga gctcagatca aatagggaaa tacttgaaat gtatgtctca 2280

ggccaaggca aactccattc ctcatattaa accctggctg ttacttctca ctacgtcacc 2340

atttttaact gacaggatta gtaaactatt aagacagcaa acatgtgtct gtctctgttt 2400

tttcccctcc tccagtttgc tttggcatct atgactgtgt ttgtcaatag gagacttttt 2460

caaaatcata ctggggaagc agtgatttta aaacctcaag tttttaaaca tgatttatat 2520

gttctgtaca attgttcagt ttgtaacttt ttaaagtttg gatgtataga aggataaata 2580

ggaaatataa aaattggtta tttgggggct tttttactta ttgtatttaa aaataaaagg 2640

gtatcaatgt gaaattatgt aaattttaaa tgcttatgaa tcaaatcatt gttgaacaaa 2700

agatttgttg ctgtgtaatt attgtcttgt acgcatttaa gagaaataaa tatactcaga 2760

cttatgtttt aagaaatgg 2779

<210> 142

<211> 900

<212> DNA

<213> Rattus rattus (black rat)

<400> 142
tggagcggcg gcggcttgag ctcgcgcgtc cgcagtgatg agctacgacc gcgccatcac 60

cgtcttctcg cccgacggcc acctcttcca agtggagtac gcgcaggagg cggtcaagaa 120

gggctccacc gcggttggtg ttcgaggaag ggacattgtt gttcttggtg tggaaaagaa 180

gtcggtggcc aagctacaag atgaaagaac ggtccggaaa atctgtgctc tggacgataa 240

tgtctgcatg gcctttgcag ttgtagcctc tgtctcaggt ctcactgccg atgcaaggat 300

agtcatcaac agagccaggg tagagtgcca gagccaccgg ctgaccgtgg gggacccagt 360

gactgtggag tacatcaccc gctacattgc cagtctgaag cagcgttaca cacagagcaa 420

tgggcgcagg ccatttggta tctctgccct aattgtgggt ttcgacttcg atggcacccc 480

cagactctat cagactgacc cctcaggcac ataccatgct tggaaggcca atgccatagg 540

ccggggtgcc aagtcggtgc gtgaatttct ggagaagaac tacacagatg atgccattga 600

aacagacgat ctgaccatca aactggtgat caaggcgctg ctggaagtgg tccagtcagg 660

tggcaaaaac attgaacttg ctgtcatgag gcgggatcag cccctcaaga ttctaagtcc 720

tgaagaaatt gagaagtatg ttgctgaaat cgagaaggag aaagaagaaa atgaaaagaa 780

gaaacaaaag aaagcatcat gagggcagcg gtcagagcag ctttgagcct ccatccgagc 840

aaacgggtgc tgtgggcctt gtggccattt gtcggagtcc aataaactac tacgtttttt 900

<210> 143

<211> 144

<212> DNA

<213>  Rattus norvegicus (rat)

<400>  143
atgttgctga aatcgagaag gagaaagaag aaaatgaaaa gaagaaacaa aagaaagcat      60

catgagggca gcggtcagag cagctttgag cctccatccg agcaaacggg tgctgtgggc     120

cttgtggcca tttgtcggag tcca                                            144

<210>  144

<211>  6125

<212>  DNA

<213>  Rattus norvegicus (Norway rat)

<400>  144
   aattcccagg gactaaacca ccacccaaag agtacacatg gacagaccca tggctccacc      60
   tgcatatgta gcagaggatg gccttgttga gcatcagtgg gagggaaaac ccttggtcct     120
   gctaaggctt gatcccccag tgtcagggaa tatcagggcg gggaggcggg aagaggtggg     180
   ggtaacacct catagaaaaa ggggcaagca gatgagatag ggggtttatt gatgggaacc     240
   aggaaaggag ataacatttg aatgtaatga ataaaaaata tccaataaaa aaaggaaaaa     300
   aagaatgtat ggtagaaaca gtatcaacta atcagacccc tcagaactcc cagagactaa     360
   gccacccacc aaagagcata tgatgggtgg tccaagaccc ttggaacata tgaatcagag     420
   gactgccttg ctgtgcctca gttggagagg gtgcacctaa tctgatgtga cttgatgcct     480
   caggggaggg gatgcagtag gggaagaggg gaataccctc tcagaggcaa agaggagggg     540
   gaatgagatg aagaactgtg gagagaggaa accaagaggg gagtaacatc tggaatgtaa     600
   ataaataaaa ttacttacta aataaataaa ttatttaatt ataatgaact atattttatt     660
   aattacacta attgatgaag ttagttataa taataattat taaataactt aaataaagta     720
   gaagtaaata taaaaataaa tgtaaggaaa gaacaacaac aaaagaatgt gcaatagagc     780
   aaactgcaca ccttacaacc cagcaagcaa aggataacag cactggccag tgttccacaa     840
   tccctttaaa gatcattcct ccaatgacct gaaaacttca aacaggctct tacccctaaa     900
   gagtcacaac atcccacggt accaagcctt taacacatgg actttttaca atattgaaaa     960
   tcaaattata ataccaaata atcctaaata taatattatg ttatgagacc ctgaacattg    1020
   tttaaataat ttagaacatg ttatagttat ttcaggaagc agcatacatg tttggagtaa    1080
   ggccagtcat tctaacctgc cttgtattag gtacaaggag gatctgtccc atatatatgc    1140
   ctgttagcta atttgacagg aaagttctac ccattagctc cattccttaa atctgattac    1200
   aatttgatat atttgataca ggctgagggt aagcctagga ggttcataag tgtcttttaa    1260
   gggggacatt tttacatata gaaggctgtc ctcaatctcc tgtctttctc cttcagcctt    1320
   cagagtgcta ggagtacaga tggccaccac acttggctta tattgtgcat tttattcagg    1380
   ctttatactt gtgctagtga aatgagttag tacatgttcc tcctattgaa ccatatctct 1440
   aaattgctaa agtgtgtgtg tgtgtgtgtg tgtgtgtgtg tgtgtgtgtg tgtgtgtgtg 1500
   tatgcttgag atggggttgt taatgtagcc taactggccc tcaaagtcac cgagctattt 1560
   caaattcacc accctgtctc acaatctgaa atattggtat cataaatatg tgtcaccaca 1620
   ccaagccttt gggcagattt tcatttgcat taatagatga cagtgccatc ttaaagacta 1680
   caagagactg ctaattgaga tgtataaatt cccttgactt aaatactgaa atgatccttc 1740
   ataagaagga tgggctgcat gcatggtgag aatcttcagg gactaagctt atggattttt 1800
   ttgtttcccg aaggtaccat ggctgaggaa accgccccag ctgttctgga gcggcaccaa 1860
   gggtccttgt actccctttt tcctgatcac agtgtgaaga aatactttaa ccaagtggat 1920
   atctccaatg gtttggattg gtccctggac cataaaatct tctactacat tgacagcctg 1980
   tcctacactg tggatgcctt tgactatgac ctgccaacag gacagatttg tatgaacatc 2040
   tttattattt ttcataataa tatttttctt atttttgtat ctccttaagc catgtgacaa 2100
   tgtcattatt tgtagagtag aggacttcct gatataatga tgatggcaca gacttcacta 2160
   agctgccagt gaaactgtgg tcctcaatac atctccagtg tcttatttga gactagggct 2220
   gtatctgcac tagacagtgc agataggcct tgacttttcc tctccatcac taaaaagggc 2280
   ctccctagag aatgaatcct gccttatcag acagtgccaa aagcaaatat gcaacagaat 2340
   gtgataaaag agggagactg aaactttggt tcctgagtct tatttgatct tagttactag 2400
   aggcagtatc agggcaaatg aacaaccttt gaaatggaaa aataacagaa aagtgcttcc 2460
   aggttggttc agttccatga atgttctgta aaatgagcca tcactggaaa cttcgaggaa 2520
   agaaattatt tagaagaatt tagaaaggag accttgaaac tgtttgatga aaagtgtcag 2580
   acaggaagag caaaaccaat cataaaaatg acttgaaaaa atcacaggat ttatgtagat 2640
   ttaatcaagg gcaaactttt ccagtccaga tgctttgctt tgaactatgt agtgctcctt 2700
   tttatgacta ttcttaccca atttccttac acatggcttt aacctttttc cattatactt 2760
   caatctgatt gctggaatgc ccctgccttt tgtagctggc tgctcctctg cactgtttcc 2820
   tgaggagctg aggatcaaac aatttctttc tttttttttt tttttggttt ctttttttca 2880
   gagctgggga ccgaacccag ggccttgctc ttcctaggca agtgctctac cactactgag 2940
   ctaaatcccc aaccaaacaa tttctttata tgttcactga gatgcaattc cagcactccc 3000
   tccctttgga tggtcattgt ttggatacta tgaatcatct tcaaacaccc tgtggatttt 3060
   agaatgccct tcctttaaga agcaagctct ttcaatactt tttcttgatt tttgtagaaa 3120
   gtaagacaaa gaatatgtct cccaaggttt tttgcatgca ttgttcatat agtaccattt 3180
   agctgcctta tagatgttag acttggtcaa gtatcacttg ggaacaagtg atctaatccc 3240
   agactgacaa atgtctgaga tgggttgcca agcctactga ggtgcactct atctatagac 3300
   tctatggtgg ctgatcctca agactaatcc taacactgaa gcagagatta ctttaagagt 3360
   ctgaagacag gggttgggga tttagctcag tggtagagtg cttgcctagc aagtgcaagg 3420
   ccctgggttc ggttctcagc tccgaaaaaa agaaaagagt ctgaagacag cttgggctac 3480
   acaatgaata ctacaacctt ggatgtagta tcagctttat ttggcttagt agtagaggag 3540
   ttgtctagca tacataaggt tctgaattct atcctcagaa ctgcaaaaaa gaaagaaaga 3600
   aagaaaggaa ggaaggaagg aaggaaggaa gaagaaagaa ggaagaagaa agaagaaaag 3660
   ggaaaagaaa gcatttatat ctttataaag acaaaaggga gtgaatgtat cccaaatcat 3720
   atatttaaaa tcctttgcta agtgatcagt atttataacc agcttgagaa cacactgttt 3780
   gtcagcattg tgcgacccac tgtttggtca tgttttggtt ttgtttttca accatagcca 3840
   accgcaggac tgtttacaag atggaaaaag atgaacaaat cccagatgga atgtgcattg 3900
   atgttgaggg gaagctttgg gtggcctgtt acaatggagg aagagtaatt cgcctagatc 3960
   ctgagacagg tatgctggta gcaaaataga atgtcgcacc cactccagaa aagaccagtg 4020
   ctagacacca ttactaatac tgttagagcc atattaatgg tcttaactga atgattttgg 4080
   aaataaaagc aaaagctaca actccaaatt tatagtcaaa acgtcttttc ctgtggaaac 4140
   tcaattctca gaaaaggtac attaaaagtt aaatactgga taggagccag ataatatggt 4200
   agcttataaa cctaagtgta aggaaggtaa aactaaaggg tggtgagttt cgggcaacct 4260
   gggcgacata gtgatactaa gccatgttgg gttcagccta tattgactcc cttcaaggaa 4320
   cctccgcagt ctcttacacc atccctgtca tccatgaatg gtccagatgc atagtatatg 4380
   ttggaaattt ctagaactaa atatttaata atgtatttgg tgacctaggg aaaagactgc 4440
   aaactgtgaa gttgcctgtt gataaaacaa cttcatgctg ctttggaggg aaggattact 4500
   ctgaaatgta cgtgacatgt gccagggatg ggatgagcgc cgaaggtctt ttgaggcagc 4560
   ctgatgctgg taacattttc aaggtgagat ggtctttttt cctgtgtgtg tggaaggggt 4620
   gcattatggc gggtattctg ttaaaaataa ttgattggtg cttttgcaaa catcccaaac 4680
   ctatttccat ttatcatgat aagctagtct ctctagactt aattttcagt tttttcaaat 4740
   atacattata taaagttcaa gatggtgtgt cttacctatt tccggtattt taaaatattt 4800
   tcatgtttca atattatttg taacatcatc tgattgtatt gatgtcgtaa agagatgtgt 4860
   aggtttttaa caatttttgt gatagaacaa acactgattt aaaaaaaaca tacacattac 4920
   aagtttgaaa acacgtgtgg aaatactggc tttggtgatg gacctctgta tctcagccct 4980
   tgacaatctg aagcataaag atggtgagct caagctatct tgagacatat aacacaggta 5040
   cttttctttt caaaaaaaag aggtataggg ctggagagat ggctcagtgt gttaagagca 5100
   ctgactgctc ttccagaggt cctgagttca attcccagca accacatggt ggctcacaac 5160
   catctgtaac gggatccaat gccctcttct ggtttatctg aagacagtaa taacagtgta 5220
   ctcataccaa taaaatgaat aaataaatcc tttttgaaaa atagcaatac cttcttggca 5280
   gatgagaaat gatctaataa gtgtctctat taatgtgcat ccttgctcat tggaccagaa 5340
   gtatgtgaat gactgcttga accattctgc tccaataagc aagcatgcac actgatttac 5400
   ttagttattt tataatttat cagtttcact cctttttttt caacagataa caggtcttgg 5460
   ggtcaaagga attgctccat attcctatgc agggtaaact gcagctcttc cttgctgtca 5520
   gaagaaaaag ctttgaagac aactggagaa ttaagggaga gaaatcaatg aactttcaat 5580
   cttgtttttt taatgaggca gtgatattat agcatggtta aactgcttta atttacactt 5640
   ttgattgggt gctggggaat aaacctaaag catggcatat taatgaagaa catatggtaa 5700
   ccatgaactc catctctgga ttcctttatt tacaattttt taaaggttga atattcgcac 5760
   cagagaatga caagtggttt tgacaacata ctctaggcct tctattaaaa acaatctgta 5820
   gaaaaggagc tcaactatca atcatcctga tcaattgctc tggggcttga aatgagacaa 5880
   tttccattct ttttttttgc attccatact tgctgtacat aaggcttcca aaaaatcaac 5940
   gcatagaaac ttagttgtaa tttataatga ttgtgtgact tttcatgtgt tattttacaa 6000
   actaaaatat taccagatgt tattcaaaca ctggagcaca aactgtggtt ttaactgcca 6060
   cctagtggcc tatacaaata aatgcatcac attacaaaga attttctttc catcctttta 6120
   ctttt 6125

<210> 145

<211> 3455

<212> DNA

<213> Rattus norvegicus (Norway rat)

<400> 145
cgggcgcgga cctgaggtgc tgctgccggg tggtccttcc tgtgttgtcc gcgccctgga 60
ccatggcggc gctggcgggt gaagaggcct ggaggtgtcg gggctgtggc aactatgttc 120
ccctgagcca gaggctgtac aggacagcca acgaggcctg gcatagctcc tgcttccggt 180
gttccgagtg ccaggagtcc ctcaccaact ggtactatga gaaggacggg aagctctact 240
gccacaagga ctactgggcc aagtttggag aattctgcca cgggtgctcc ctgctgatga 300
cagggccagc catggtggct ggggagttta agtaccaccc agagtgcttt gcctgcatga 360
gttgcaaggt gatcatcgag gatggggatg cgtacgccct ggtgcagcat gctacactct 420
actgcgggaa gtgccacaat gaggtggtgc tggcacccat gtttgaaaga ctgtctacgg 480
agtctgtcca ggaccagctg ccctactctg tcacgctcat ctccatgcct gccaccaccg 540
agtgcagacg tggcttctct gtgtctgtgg aaagcgcctc ctctaactat gccaccaccg 600
tgcaagtgaa agaggtcaac cggatgcaca tcagccccaa caaccgaaat gccatccacc 660
ccggggaccg catcttggag atcaatggga cccctgtccg cactctccga gtagaagagg 720
tggaggatgc gattaaccag acgagccaga cacttcaact gctgattgaa catgaccctg 780
tcccccagcg cctggaccag ttacggctag acacccggct ttctccccac atgcagagta 840
gtggacatac tctcatgctc agcaccttgg acgccaagga aaatcaggag ggaacactga 900
ggagacgttc tctgaggcgc agtaacagca tctccaagtc tcctggcccc agctccccca 960

aggagccgct gctcctgagc cgagacatca gtcgctcaga gtcccttcgc tgctccagca 1020

gctactcaca gcagatcttc cggccctgtg acctgatcca cggggaggtc ctggggaagg 1080

gcttcttcgg acaggccatc aaggtgactc acaaagccac aggcaaagtg atggtcatga 1140

aggagttaat tcgctgtgat gaggaaaccc agaagacttt cctgactgag gtgaaagtga 1200

tgcgaagcct ggaccacccc aacgtgctta agttcatcgg ggtcctgtac aaggacaaga 1260

agctgaatct gctgacagag tacattgagg ggggcaccct gaaggacttt ctgcgcaatg 1320

tggacccgtt tccctggcaa cagaaggtca ggtttgccaa aggcatcgct tccggaatgg 1380

cttatttaca ctccatgtgc atcatccacc gagatctgaa ctcgcacaac tgtctcatca 1440

aattggacaa gacggtggtg gtagcagact tcgggctgtc acgtctcata gtggaagaga 1500

ggaaaaggcc tccagtagag aaggccgcca ccaagaaacg caccttacgc aagagtgatc 1560

gcaagaagcg ctacactgtg gtgggaaacc cctactggat ggcccctgag atgctgaatg 1620

gcaagagcta cgatgagact gtggatgtct tctcttttgg gattgttctc tgtgagatca 1680

ttgggcaggt atatgctgat cccgactgcc ttccccgcac actggacttc ggcctcaacg 1740

taaagctttt ctgggagaag tttgtcccaa cagactgtcc cccagccttc ttccccctgg 1800

ctgctatctg ctgcaaactg gagcctgaga gcagaccggc attctcaaag ctggaggact 1860

cattcgaggc gctctccctg ttcttgggag agttggccat cccactgcca gcagagctgg 1920

aagagctgga ccacactgtg agcatggagt atggcctaac ccgggactcg ccaccctagc 1980

cctggtccat ccccctgcag ggggacattc cacagccagc attgccccct ctgcgccctg 2040

tcctcgctgt gagcgagcaa gcgggactgc ctaggcttcc tgtggattga cggcttgctt 2100

agaagcagaa caagccatcc ctacctcccc aggaggcact tgggtgcagg gaagcaattc 2160

tccacaggtt tgggcctagt ttctgtctgt aaatccaaca ctcacctgaa agctgtgaag 2220

aggataaaag cctggattct agaccaagag tctgttactg gggaccgccc gggacctctg 2280

gactgagatg ccgggagcct gtagctttac actaacctac acggcctggg cccgccgggc 2340

cggatgagtg gactggcctg agggcctctg gttcagcaag gtgcaggact tgaagcgata 2400

gtatgagttt cagaaacggg gccgtcaaaa cgtgtgaaat gacagagcgg tttctccttc 2460

catcctagcc atgccctttg gagcaggttt tgaagagtct tagtagtggt aaaacctaca 2520

ggatccaaaa aaaaaggact ggtttctgct cagcagccca gggaccacat cagtgtgtgg 2580

ggaatttcca cttcagcacc ctgtctcaac aagcagtctc aaaaacacca aagaaggtgg 2640

ggctggaaag aaagcggccc tgccgtctaa aagccccaca cccaggctgg tgggctcagt 2700

cagaaactta aggtgttgcc taacaataga tgggttctgg atgccagtgt ggtttgcctc 2760

agactttggg cctaagcagg gagaaggggg tcccattaat ttctttgcac ccttgtggca 2820

gctactgcat accctctagg atgtctggtc caggcagcaa cttgggacag gcattggtgg 2880

ctgctgagtc ccaaactgag atatgaagag agctccctga gctggacctt gtagagtcta 2940

cctctgttgt gctggtccag cttacttcct gagacacact cgagggccgt gggcatggga 3000

aactctaggg gcactttcca cccctggcta ccaggtgtcc agggactccg tagctcacca 3060

aggttcgcca tctcctaagt gggagctcac atcatattta acacattggg aatgggtttg 3120

gttataaata cgtgtgaggg tttgtgctgg tggggggatc cggggagagt ggctgctctt 3180

attagctttt atctgtgggg tgatatgtga aatattttgt acatagacaa gatgaattat 3240

gggatgggaa gtctttggcc actttacttg ctgtgtagac tgtacttgtg tgtggagttt 3300

gcaaacgtgc cgcagaggga atcctgaact cccgacatca gtgtggttca gcgtcctgcg 3360

gcgaattatg cacgcgctgt ccccagcgag tgtgggagtg gtgggcctgc actgggccat 3420

gaggcagact aaataaatta agcagttaac agagc 3455

<210> 146

<211> 855

<212> DNA

<213> Rattus norvegicus (Norway rat)

<400> 146
atggcttgcc ttcttcctgc tgctcgactt cctgcaggct ttctcttctt agtgctctgg 60

ggctcagttc taggtgacaa gctgctggtg gtcccccagg atggcagcca ctggcttagc 120

atgaaggaga tagtggagca cctcagtgaa cgcggacacg acattgtggt gctagtgcca 180

gaagtcaatt tgcttttggg agaatccaaa tactacagga ggaaaagctt cccggtcccc 240

tacaacctag aagagttgcg gacccgctat cgctcctttg ggaacaacca ctttgctgcc 300

agttcccccc tgatggctcc tctaagagag tacaggaaca acatgattgt cattgacatg 360

tgctttttca gctgccagag cctcctgaag gactcggcca ccctcagctt cctcagggag 420

aaccagtttg atgctctgtt cacagacccg gccatgccct gtggtgtgat cctggctgag 480

tatctcaagc tgccttccgt ctacctcttc agaggtttcc catgctctct ggagcacatg 540

cttggtcaaa gcccaagccc cgtatcctat gttcccagat tctacaccaa attctcagac 600

cacatgacat ttccccaacg gctggccaac ttcattgcta acatcttgga gaactacctt 660

tatcattgtc tgtactcaaa gtatgagatc cttgcctacg acctcctcaa gagagatgtg 720

tccctacctg ccttacacca gaactctctg tggctgttac ggtatgattt tgtgttcgaa 780

tacccccggc cagtcatgcc caacatgatc ttcattggag ggaccaactg caagaagaag 840

gggaacctgt ctcag 855

<210> 147

<211> 858

<212> DNA

<213> Rattus norvegicus (Norway rat)

<400> 147
atggctcctg cagacgttcc agcctctctt cctctcggtc tgtgcctgct gctggcctct 60

ggctttgggc atgcaggcaa gctgctggtg gtgcccatgg atggcagcca ctggttcacc 120

atgcagatgg ttgtggagaa gctccttccc aaaggccatg aggtggtggt ggttgttcca 180

gaggtcagtt ggcagctggg aaaaccactg aattttacgg tgaaaacgta ttcagtttct 240

cacactcagg aggatttaaa tcgggagttc aagtttttta ttgactctca gtggaaaact 300

caacaagaga gcggagttct tcctctactg actagccctg cccagggttt cttcgaatta 360

ctgttttcac actgtaggag tttgtttaag gacaagaagt tagtggagta cttgaagcag 420

agttcgtttg atgctgtgtt tctggatcct tttgatgtgt gtggcttaac tgttgccaag 480

tacttttctc tcccgtcagt ggtcttcagc agggggatat tttgtcacta tcttgaagaa 540

ggctcccagt gccccagtcc tccttcatat gtccccagac ctatcttgaa actcacagat 600

accatgactt tcaaggaaag agtgtggaac cttctttcct acatggggga gcatgcattc 660

tgtcccagtt ttttcaaaac tgctaccgac attgcctctg aagttctcca gaccccggtg 720

actatgacag acctcttcag cccagtgtcc gtttggttgt tacgcacaga cttcacgttg 780

gaattaccca gacctgtgat gcccaatgtg atccacattg gagggatcaa ctgccaccaa 840

aggaagccag tttccaag 858

<210> 148

<211> 1987

<212> DNA

<213> Rattus norvegicus (Norway rat)


<400> 148
tgcaagactg tcagctggga aggaaacttg gaggcctgaa ctgctgaagg agagctaaga 60

tggagatcat tcccaacctt tctatagaga cctgggtgct tctagctact agcttgatgc 120

tcttctacat atatgggacc tattctcatg gcctgtttaa gaaactagga attcctggac 180

ccaaacctgt gcctttattt ggcaccattt tcaactacgg tgatggcatg tggaaatttg 240

atgatgactg ctataaaaag tatggaaaaa tatgggggtt ttatgagggc ccacagcctt 300

ttttggctat catggatcca gagatcatca aaatggtgct ggtgaaagaa tgttactcag 360

tcttcacaaa ccgtcggtgt tttgggccaa tgggatttat gaaaaaggcc attaccatgt 420

ctgaggatga agaatggaag agacttcgaa caatcctgtc tccaaccttc accagtggca 480

aactcaagga gatgttcccc ctcatgagac agtatggaga tacattgttg aagaacttga 540

ggcgagaaga agcaaaaggg gagcccatca acatgaaaga catctttgga gcttatagca 600

tggacgtgat cactggcaca tcatttggag tgaacgtcga ttccctcaac aatccacagg 660

atcccttcgt gcagaaagcc aagaagatct taaaatttca aatttttgat ccatttcttc 720

tctctgtagt tctgtttcca tttcttactc caatatatga gatgttaaat ttttcaattt 780

ttccaagaca gtcaatgaac tttttcaaaa aattcgtaaa aacaatgaag aaaaatcgcc 840

ttgattcaaa ccagaagaac cgagtggatt ttcttcaact gatgatgaat actcagaact 900

ccaaaggcca agagtcccag aaagctcttt ctgatctaga aatggcagca caagctatta 960

ttttcatttt tgggggttat gatgccacaa gcacctccat ttccttcata atgtatgaac 1020

tggccactcg ccccaatgtg caaaagaaac tccagaatga gattgataga gctctgccca 1080

ataaggcacc tgtcacctat gatgctctga tggaaatgga gtacctggac atggtggtga 1140

atgaaagtct aagattgtac ccaattgcta ccaggctaga cagagtctca aaaaaggatg 1200

tggaaatcaa tggagttttt attcccaaag ggactgtagt tacgatacca atctatcctc 1260

ttcatcggaa ccctgagtac tggctagagc ctgaggaatt caaccctgaa aggttcagca 1320

aggagaacaa gggcagcatt gatccttatg tatatctgcc ctttggaaat ggacccagga 1380

actgcattgg catgaggttt gctctcatca gcatgaaact tgctgtcata ggagtcctgc 1440

agaacttcaa tatccagcct tgtgagaaga cacagatccc tctgaagatc agtaggcaac 1500

caattttcca accagaagga cccatcatcc taaagcttgt gtcaagagat taaacccaga 1560

tttggacagt gaatttccct caggaaccat gttataatct tcaaggagac tgtttcacag 1620

aacaccagag aatttaatta acattagaat aagagcaata taatataggc ttcatcaatt 1680

ttcctcgatt actgagtatt cagaaattca ctgaacaggc tcagtggctc tgcggtgtat 1740

catctatttt atgattcaaa gaaaattatt aactcaatgg tagatgtgga ggttcattat 1800

atgattcttg tggaccatct atacagattc cagttagttc catcagttct gtattctaac 1860

tgcagtagct gtttcttaga gttctcatca atagaaactg ttgtattgac agttagtaaa 1920

tgtgtagcaa attttctctt tgtaaaaata tatgatatta agaatataaa taaatatatc 1980

tttcaag 1987

<210> 149

<211> 2355

<212> DNA

<213> Rattus norvegicus (Norway rat)

<400> 149
   gctcttttct tctccctcgc tatccgctcc cccgcccccg tgcctctggc tctgcgcctg 60
   gctccctcgg gtccgctccc ctttcccgcc ggcctggccc ggcgtcacgc tcccggagtc 120
   tccccgctcg gcggcgtctc attgtgggag ggggtcagat caccccgccg ggcggtggcg 180
   ctgggggcca gcggaggggg aggggcctta gtcgttcgcc cgcgccgccc gcccgcctgc 240
   cgagcgcgct caccgccgat ctccctcctt gctctgcagc cgcggcccat ggagcccgcc 300
   ggcccggccc ctggccgcct agggccgctg ctgttctgcc tgctgctctc cgcgtcctgt 360
   ttctgtgcag gagccagcgg gaaagaactg aaggtgactc aggctgacaa atcagtgtct 420
   gttgctgctg gagattcggc cactctgaac tgcactgtgt cctccctgac gcctgtggga 480
   cccattaagt ggttcaaagg agaagggcaa aatcggagcc cgatctacag tttcatagga 540
   ggagaacact ttcctcgaat tacaaatgtt tcagatgcta ctaagagaaa caatatggac 600
   tttagcatct gtatcagtaa tgtcacccca gaagatgctg gcacctacta ctgtgtgaag 660
   ttccagaaag gaatagtaga gcctgacaca gaaattaaat ctggaggggg aacaacgctc 720
   tatgtactcg ccaaaccttc ttcaccggaa gtatcgggcc cagactccag ggcctctcct 780
   ggacagacag tgaacttcac ctgcaagtct tacggcttct ctccccggaa tatcaccctg 840
   aagtggctca aagatgggaa agaactctcc catttggaga ccaccatctc cagtaaaagc 900
   aatgtctcct acaacatctc cagcacagtc agcgtgaaac taagccccga ggacattcat 960
   tctcgggtca tctgcgaggt agcccacgtc accttggaag gacgcccgct taatgggacc 1020
   gctaactttt ctaacatcat ccgagtttca cccaccttga agatcaccca acagcccctg 1080
   acgcccgcga gccaggtgaa cctcacctgc caggtgcaga agttctaccc caaggctctc 1140
   cagctgaact ggctggagaa tggaaactta tcacggacgg acaagcccga gcatttcaca 1200
   gacaacaggg atgggaccta taattacaca agcctgttcc tggtgaactc atctgctcac 1260
   agagaggatg tggtattcac gtgccaggtg gagcatgaca gtcagccagc gatcaccgaa 1320
   aaccataccg tgcgggcatt tgcccactcg agtagtggag gcagcatgga aaccatccct 1380
   gataataatg cttactacaa ctggaacgtc ttcatcggtg tgggtgtggc gtgtgctttg 1440
   ctagtagtcc tgctgatggc tgccctctac ctcctccgaa tcaaacagaa gaaagccaag 1500
   ggctcaactt cttccacacg gttgcacgag cccgagaaga atgccaggga aataacccag 1560
   atccaggaca caaatgacat caacgacatc acatacgcag acctgaatct gcccaaagag 1620
   aagaagcccg cccccccggt ccccgagccc aacaaccaca cagaatatgc aagcattgag 1680
   acaggcaaac tgcctaggcc agaggatacc ctcacctatg ctgacctgga catggtccac 1740
   ctcaaccggg cacagccaac ccccaagcct gagccatcct tctcagagta tgccagtgtc 1800
   caagtccaga ggaagtgaat ggggctgtgg ttggctctag gccccatccc cacaagtttt 1860
   cttgtcctac atggagtggc catgatgagg acaaccagcc agccagccct gtctccagaa 1920
   ggccaggtgg cacaggtcct aggaccaggg gtaagggtgg cttctgtctt ccctccgtgg 1980
   ctctccaaca cctcttgaca cccatgtccc cttcttctgg agctgggtgt tgcagaacca 2040
   gagggggaac tggagaaagc tgcctagaat ccaagaagcg ttgtgcctca gcccatcaca 2100
   ctgggtctgg atcctggtct tggcaacccc caggttgctt ccttgatgct ccagcgcctg 2160
   gtcttctgtg tggagaagag ttcaccatct ccatccaact tgagcttcgg ggccagactc 2220
   cctttagatc agaccgcccc atgtgtggaa gaactacacc aggagtcaac aagttttcag 2280
   ccaacagtgc tagcctcccc acctcccagg ctgacgagcc ctgaggagaa ggaaccctct 2340
   tcccctagac cagct 2355

<210>  150

<211>  1879

<212>  DNA

<213>  Rattus norvegicus (Norway rat)

<400>  150
aagacttttt ccccagcctt aactggatag tctgaagttt tcaaaactct tatccacaaa      60

gttgtcagaa ccttgattgg gaagtcctgt gcatctgtgc taacctacag ggcctcctta     120

tccagagcac tctgcatttc agagggtcgc tgtcgaacta cggttttggc gaagacattc     180

ctgaagaatt gtctgaggtt tcctctgcaa aaatggccaa gctgacagct gttcctctca     240

gtgcacttgt tgatgagcct gtgcatatcc gggtcacagg cctgaccccc ttccaggtgg     300

tgtgccttca ggcatcactg aaagatgata agggaaacct gtttaattct caggccttct     360

acagggccag tgaagtgggt gaggtagatc tggaacgtga ttcctctctt ggaggagact     420

acatgggggt ccaccccatg ggtcttttct ggtccatgaa acctgaaaag ctattgacta     480

gattggtaaa aagagatgtg atgaataggc cccacaaagt ccacataaaa ctttgccatc     540

catactttcc agtagaaggc aaagttatca gttcctcctt ggatagtctg attctggaaa     600

ggtggtatat ggcacctggt gtcactagga tccatgtaaa ggaaggccga atccggggag     660

ccctgtttct gcctccagga gaaggtcctt tcccaggggt cattgacttg tttggaggag     720

ctggtggact gtttgagttc cgggccagcc tcctggccag tcatggcttt gccactttag     780

ctctggctta ctggggctat gatgacctgc cctctcgact ggagaaggta gatctagagt     840

attttgaaga aggtgtagag tttctcctga gacatcctaa ggtcctgggc ccaggggttg     900

gcatcctttc tgtgtgcatt ggagcagaga ttggactttc tatggctatt aacctaaaac     960

agataacagc cactgtactt ataaatgggc ctaattttgt ttctagcaat ccacatgtat    1020

atcgtggtaa ggtcttccag cctacaccct gcagtgaaga atttgtaacc accaatgctt    1080

tgggacttgt agagttctat cgaacctttg aggaaactgc agataaggat agcaaatact    1140

gttttcccat tgaaaaagct cacggacatt ttcttttcgt ggtgggagaa gatgataaga    1200

acctcaacag caaagtgcat gctaagcaag ccatagccca gctgatgaaa agtggaaaga    1260

agaactggac tctgctgtct taccctgggg caggtcacct gattgagcct ccctactccc    1320

cactgtgctc agcctcaagg atgccctttg taatcccaag catcaactgg ggaggagagg    1380

ttatcccaca cgcagctgcg caggaacatt cttggaagga gatacagaaa tttctcaagc    1440

agcatcttaa tccaggtttc aacagtcagc tgtgagtgga cttgattata ttactggaaa    1500

gaggagctgg gcatctcctg gccagctcca ctcctcactt ccatagagga atgtctttaa    1560

tctcttatca catgaggaag aagagtacca ccagaaaatg ccgaaggaca gagagtgata    1620

acctcatgac tttggaaggg gagacatgtt ttccatggaa taaaatgtcc ctcagtgaga    1680

gtcctatatc tgtataaata aaatcttagg gttttcctaa aatgttcaac accacagcaa 1740

ctttctgtga tgataattat caaggaaatt atcactgata atccacagga tactttagtt 1800

tataaaagag acatgaaaag aattatatat tgttacttat taatttctta aaactcacat 1860

taaatatgct tagatcatc 1879

<210> 151

<211> 846

<212> DNA

<213> Rattus rattus (black rat)

<400> 151
cgttgctgtc gggcgctggc cagcgtgttg cagcggccga tgccggtgaa tcagcacggg 60

ttttttggac tcggaggtcg tgcagatctg ctggacttgg gtccggggag tcctggtgat 120

gggctgagcc tagccgcgcc gagctggggt gtcccggagg agccacgaat cgaaatgctt 180

catggaacca ccaccctggc cttcaagttt cagcatggag tcattgttgc agcggattcc 240

cgggccaccg caggtcccta cattgcttcc cagacagtga agaaagtgat agagatcaac 300

ccttaccttc tgggcaccat ggctgggggt gcagcggatt gcagcttctg ggagcggttg 360

ttggctcgac agtgtcgaat ctatgagctt cgcaacaagg aacgcatctc ggtcgcggcg 420

gcctccaaac tgcttgccaa catggtgtat cagtacaaag gcatggggct gtccatgggc 480

accatgatct gtggctggga taagagaggc ccgggcctct actatgtaga cagtgagggg 540

aacaggatct ctggaaccgc cttctcagtg ggatctggct ccgtgtatgc gtttggagtc 600

atggatcgag gctactccta tgacctacaa gtggaggagg cctatgacct ggcccgccga 660

gccatctacc aagccaccta cagagatgcc tactccggag gagcagtcaa cctctaccat 720

gtgcgggagg atggctggat ccgtgtctcc agtgacaatg tagctgactt acatgacaag 780

tatacttcat ctatcccctg agggaaggtg gagtgccctg gttataataa ataatagtac 840

ccgttc 846

<210> 152

<211> 1403

<212> DNA

<213> Rattus norvegicus (Norway rat)

<400> 152
   gggcttgcgg cttctaaaat gccggattac ctaggtgcag accagcggaa aaccaaagag 60
   gatgagaagg acgacaagcc catccgagct ctggatgaag gggatatcgc cttgttgaaa 120
   acttatggcc agagtactta ttcgaggcag atcaagcagg ttgaagatga cattcagcaa 180
   cttcttaaaa aaattaatga gctcactggt atcaaagagt ccgacactgg gctggcccct 240
   ccagccctct gggatttggc tgcagacaaa cagacacttc agagtgaaca gccgctacag 300
   gttgcaagat gtaccaagat aatcaacgcc gattcagagg acccgaaata catcatcaac 360
   gtgaagcagt ttgccaagtt cgtggtggat ctcagtgatc aggtggcacc tactgacatt 420
   gaggaaggga tgagagtcgg cgtggacaga aataaatacc aaattcacat tccactgcct 480
   cctaagattg acccaacagt taccatgatg caggtggagg aaaaacctga tgtcacatac 540
   agtgacgttg gtggctgtaa ggaacagatt gagaaattgc gggaagtggt tgaaacccct 600
   ctactccatc cagagaggtt tgttaacctt ggaatcgagc ctccgaaggg tgtgttgtta 660
   tttgggccac caggcacagg caagaccctc tgtgccaggg cagtggccaa tcggactgat 720
   gcttgcttca tcccagttat cgggtctgag cttgtacaga agtacgtcgg tgagggggct 780
   cgaatggttc gggagctttt tgaaatggcc aggacaaaaa aagcctgcct tattttcttt 840
   gatgaaattg atgccattgg aggggctcgg tttgacgatg gcgctggggg tgacaatgag 900
   gtgcagagga ccatgttgga gctcatcaat cagctggatg gctttgatcc tcggggcaac 960
   atcaaagtgc tcatggccac taaccgacct gatactctgg acccagcgct gatgaggcca 1020
   gggaggctgg acagaaagat tgagttcagc ttacctgact tagagggtcg aacgcacatc 1080
   tttaagattc atgctcgctc aatgagtgtg gaaagagaca tccgatttga gctactagcc 1140
   cgcctgtgtc caaacagtac tggagcggag attagaagcg tttgcacaga ggctggtatg 1200
   tttgcaatcc gagcccggag aaagattgct acagagaagg acttcttaga agctgtaaat 1260
   aaggtcatca agtcgtatgc caaatttagt gctactcccc gctacatgac atacaactga 1320
   gccacagagg ctttgactga aggaacttcc tgtgcgttca taacttgtta ccgcctcaca 1380
   aaaataaaag acttaaaaac tgt 1403

<210> 153

<211> 2091

<212> DNA

<213> Rattus norvegicus (Norway rat)

<400> 153
   attttcaagg gccagcgaga gagggagttt ggcacagttt gtggagaact caaagaaaaa 60
   ccaactctgt tcgcagtccc cagctcctcc agccatggca ccacaagaac gacttctcat 120
   ccgcgggggt cgcgtggtca atgatgactt ctcacaggtg gccgacgtgc tagtggagga 180
   cggcgtggtg cgggcgctgg gacggacttt gctgcctccc ggggacacat cccgggggct 240
   gcggatccta gatgcagcgg gcaagctcgt cctgccggga ggcatcgaca cacacacgca 300
   catgcagttc ccgttcatgg gctcgcagtc agtcgacgac ttccaccagg gcaccaaggc 360
   tgctttggca ggaggcacca ccatgatcat tgattttgcg attcctcaga aaggcagctc 420
   cctcattgaa gcttttgaga cctggcgcaa ctgggcagac cccaaagtct gctgtgacta 480
   tagcctgcac gtggcagtga catggtggag tgacaaggta aaagaagaaa tgaaaaccct 540
   tgcccaagat aaaggcgtta actctttcaa gatgtttatg gcctacaaag acctgtacat 600
   ggtgcaagac cagcaaatgt acgctgcctt ttctcagtgc aaggagatag gggccattgc 660
   tcaggtgcat gccgagaatg gagatttgat tgcagagggg gccaagaaga tgctggcact 720
   ggggataacg ggccccgagg ggcacgagct gtgccgcccg gaagcagtgg aggcagaggc 780
   caccttgaga gccatcacca ttgctagcgc tgtgaactgc cctctataca tcgtgcacgt 840
   gatgagcaaa tccgcagcga aggtgatagc tgatgcgaag agagaaggaa aggtggtcta 900
   tggagaacca attgcagcag gtctgggcac ggatggcact cagtactgga ataaagaatg 960
   gcgccatgca gcccaccatg tcatgggtcc cccactgaga cctgatccat caacgcctgg 1020
   ctttctcatg aatctgttgg ctaatggcga tctgaccaca acagggagtg acaactgcac 1080
   tttcaacacc tgccaaaaag ctctagggaa ggatgacttc actaagattc ccaatggggt 1140
   gaatggtgtc gaggacagga tgtcggtgat atgggaaaag ggcgtgcaca gtggcaaaat 1200
   ggatgaaaat agatttgtgg cagttaccag cacaaatgca gccaaaatct ttaatcttta 1260
   tccgaaaaaa ggaagaatag ctgtaggctc agatgctgac atggtgatct gggacccaga 1320
   agccaccagg acgatctcag ccaaaacaca tcatcaggcc gttaacttca acattttcga 1380
   gggcatggtt tgccatgggg tgcccctggt gactatttca agaggcagag tggtgtatga 1440
   agcaggcgtt ttcgacgtca cagcaggaca cgggaagttt attccgcgac aacctttcgc 1500
   tgagttcatt tacaaacgag tcaagcagcg agaccagacc tgcacaccta taccggtgaa 1560
   gcgtgcgccc tacaagggag aagtcatcac attgaaaccc agagagacaa aagaagacga 1620
   cacagctggg accaggatgc agggccattc ctgatttgga tgctggggtt aagcaagtgc 1680
   aaacagagtg aaggctccca ggctcccctc cccaccatgc tgccaagccc tggagaagca 1740
   cctgccattt caactctcca agatccttta ggaaaaattc atcgctctag gcctctttga 1800
   tttttctctc agaagcaata gcagcctgcc tcaccctgcc tttgttgctg tggaagattg 1860
   aggatataaa tgaacattat tgggatgaaa cgtctgcatg aagattcatt gaagattcct 1920
   ttttcaaatg ccatctctcc ttaccctaga tttctttctt tgggttttta aagatttcct 1980
   tctggtgtaa aggttttgtt tgtttgtttt gttttaatgt ttatgtttgt tttaaaaatc 2040
   agtgatttta catttcattg caactaataa acatctggag cttcattctg c 2091

<210> 154
<211> 179
<212> DNA
<213> Rattus norvegicus (rat)

<400> 154
ctgagttcat ttacaaacga gtcaagcagc gagaccagac ctgcacacct ataccggtga 60

agcgtgcgcc ctacaaggga gaagtcatca cattgaaacc cagagagaca aaagaagacg 120

acacagctgg gaccaggatg cagggccatt cctgatttgg atgctggggt taagcaagt 179

<210> 155

<211> 1621

<212> DNA

<213> Rattus norvegicus (Norway rat)

<400> 155
gtggatcgcc cgggagaact gggcccaggg ctcaggtgga gctgccgatg ccccgggctg 60

aaaggtgaga ccacgctggt gaggctggga caagaccttg cacctccaga cctgggccga 120

gaccacaagg ggagccgcca agatgccaga ggaaaatatc ttcctgttcg tgcctaatct 180

tattggttat gcccggattg ttttcgccat catttccttc tactttatgc cctgctgccc 240

cttcacggcc tcctccttct atctactcag cggacttcta gacgctttcg atggacacgc 300

agctcgagcc cttaatcaag gaacccgatt tggggccatg ctcgacatgc tgacggaccg 360

ttgcgccacc atgtgtctct tggtcaacct ggccctgctc taccctcggg ccactcttct 420

gttccagctc agcatgagcc tggacgtggc cagtcactgg ctgcatctgc acagttctgt 480

ggtgcgaggc agtgagagcc acaagatgat tgacctgtct gggaaccctg tgcttcgaat 540

ctactatact tccaggcctg ctctgttcac cctgtgtgct ggaaatgaac tcttctactg 600

cctcctgtac ctgttcaatt tctccgaggg accactagtc ggctctgtgg ggcttttccg 660

aatgggcctc tggatcacag ctcccattgc cctgctcaag tccatcatta gtgtcatcca 720

cctcgtcacg gctgcaagga acatggctgc cctcgatgca gcagaccgcg ccaagaagaa 780

atgatcctct cccatcccca gctgcccacc tgccctgggt atctactgtg ccacatggct 840

cctctcctcc taagaggtcc cagtgtcatg tctttctcat gtgttctcta acctgctggg 900

attgggggtc accctctgtt tggtgttgtc actgtctttt tactcctgat aactagaccc 960

aacccgtgac cccaaggacc tggcactgca aagcaggaag ggtgctcagg aggcccactc 1020

atccagccgg tgctccagga ggcctgtctg aagactgggt gttgccacat atcctgctgg 1080

atcagcctgg ggtctggcct gagactttgg gaacttatac gggcaaagga gtcaggctga 1140

tgggccaaga aggctggggt accaggccag ggagggaagg ggccaagggc caactagatc 1200

tttcccttag gcaagctcct ctatcctgtc ctgctctgga aacctccata ataaaggggg 1260

gcaccttcac tctttttgga tttgtttact ttgggacact cttagatgat ggcctagggt 1320

tcaaaggagc tgggtaaaaa aaaaagaaat ccacctggat gcttttccca caaaggtggt 1380

tggctcctcc ctgtcattcc agtgacctca gagaggcctc gaggttcaca agcgttgtcc 1440

tgcccacctg ctctatgtcc tggctggcca ctttcactgt aaccttgatg tttttgaccc 1500

tcatgagaat gtaagcttca caagagcaga tttagatctt tctcgtctct gcctggagtg 1560

aaaagtagcc tggcatgtgg taactgctta ataaatattc atggaaagat gaaaaaaata 1620

c 1621

<210> 156

<211> 1049

<212> DNA

<213> Rattus norvegicus (Norway rat)

<400> 156
gccttcggtt ttagggagag caggccgggc ggtcagagat catgggggag agcgcactgg 60

agcccgggcc tgtgcccggg gcgccggctg ggggtccggt gcacgccgtc accgtggtga 120

ctttgctgga gaagctggcc accatgctag aggcgctgcg ggagaggcag gggggcctgg 180

ctgagaggca gggcggcctg gcgggctcgg tgcgccgcat ccagagtggc ctgggcgcgc 240

tgagtcgcag ccacgacacc accagcaaca cactggcgca gctgctggcc aaggcggagc 300

gcgtgggctc ccacgccgac gcagcccagg agcgggcagt gcaccgcgcc gctcaggtgc 360

agcgactgga ggccaaccac gggttgctgg tggcgcgcgg gaagctgcac gtcctgctct 420

tcaaggagga gactgaaatt ccagcccgcg ccttccagaa agcaccagag ctcttgggcc 480

cggaggacca gttggtgcta ggcccagagc agccagagga tgaagttgga gagagttctg 540

atgaggaacc cgtggagtcc cgggctcagc ggctgcgacg cactggctta cagaaggttc 600

aaagcctgaa aagggctttt tccagtcgta aaggctctga agcagcacag cccacgccag 660

tcaagccgcc acgcctaggt cctgtccgga actccgaagg cccggcagaa ggccagcctg 720

cagctcagcc tgcaatggag cctgtgctcc cgtctgccct ggagccagaa cctcctcagc 780

ctaccaagga agatcctgag agacctgtgc ttcaaataga gagcgcagcc tgatccctgg 840

ggctgcctgc cccattcagc ccttatgcct tgtcccaaaa ataaatacta atcgagtgca 900

gcacttacat ccaaataagg agagaatcct gcatccactg cccggctcca atccttcctt 960

cctggttttc cagtctggta ccctgtgtcc tctgaaagag gaacattcgg ccttgtttag 1020

gttcaccacc aataaaagta attttctct 1049

<210> 157

<211> 319

<212> DNA

<213> Rattus norvegicus (rat)

<400> 157
ctccgaaggc ccggcagaag gccagcctgc agctcagcct gcaatggagc ctgtgctccc 60

gtctgccctg gagccagaac ctcctcagcc taccaaggaa gatcctgaga gacctgtgct    120

tcaaatagag agcgcagcct gatccctggg gctgcctgcc ccattcagcc cttatgcctt    180

gtcccaaaaa taaatactaa tcgagtgcag cacttacatc caaataagga gagaatcctg    240

catccactgc ccggctccaa tccttccttc ctggttttcc agtctggtac cctgtgtcct    300

ctgaaagagg aacattcgg                                                 319

<210>  158

<211>  1726

<212>  DNA

<213>  Rattus norvegicus (Norway rat)

<400>  158
cgatcgcgca agtcggaccc gtggatcaca gcctgtagat cgcggcccgg gcctggagga     60

caacagcaag tgaagggggt tcctcttcct gaaggagggg tcatggcctt cttgttgact    120

acccgacggc tggtctgcag ttcccagaaa aacctccacc tcttcacacc tggatccaga    180

tacatcagcc aagctgctgc caaagttgac tttgagtttg attatgatgg accactcatg    240

aagacagaag tcccgggccc tagatctcag gagctaatga aacagctgaa cacaatccag    300

aatgcagagg ccgtgcactt tttctgcaac tacgaagaga gccgaggcaa ctacctcgtg    360

gacgtggatg gcaaccgcat gttggacctg tattctcaga tctcctctgt acccatcggt    420

tacaaccatc cggctctggc gaaactcgtt caacagcctc aaaacgcgag cactttcatc    480

aacagacctg ccctgggcat cctgcctcca gagaactttg tggacaagct ccgggagtcc    540

ttgatgtcgg tggcgcccaa aggcatgtgt cagctcatca cgatggcctg cgggtcctgc    600

tccaatgaga atgcattcaa gaccatcttc atgtggtacc ggagtaaaga acgaggtcag    660

agaggtttct ccaaagagga gctggagact tgcatggtta accagagtcc tggatgccca    720

gactacagca tcctctcctt catgggtgct ttccacggga ggaccatggg ttgcttagcg    780

accacacact ccaaagcaat tcacaagatt gacatccctt cctttgactg gcccattgct    840

ccattcccac ggctgaaata tcccctggag gagtttgtga cggacaatca gcaagaggag    900

gcccgctgtc tagaagaggt ggaggatcta attgtgaaat atcggaaaaa gaagagaaca    960

gtggctggga tcatcgtgga gcccatccag tccgaaggtg gagacaacca cgcatcagat   1020

gacttcttcc ggaagctgag agacatagcc aggaagcatg gctgtgcctt cttggtggac   1080

gaggttcaga ctggaggagg ctgtacaggc aagttctggg cccatgaaca ctggggcttg   1140

gatgacccag ccgacgtgat gtcgttcagc aagaagatga tgactggggg cttcttccac   1200

aaggaggagt ttcgaccaag tgctccttac cggatcttca acacctggct gggggaccca   1260

tccaagaact tgctgctggc tgaggtcatc aacatcatca agcgggaaga cctgctcaac   1320

aacgtggccc atgccgggaa gaccctactg accgggctgc tggacctcca ggcccagtac   1380

ccccagttcg tcagccgggt gaggggacga ggcaccttct gttccttcga cactcccgac 1440

aaagccatac ggaataaact catcctaatt gccaggaaca aaggtgtggt actggggggc 1500

tgcggtgaca aatccatacg tttccgtccc acgctggtct tcagggatca ccatgcccac 1560

ttgttcctca acattttcag tggtatctta gcagacttca agtaaagaag ccatctccac 1620

gacattcaga gaaagctctg tcccagcggt gtcaacttga ttagtttgcc taattcatat 1680

tttcacttca aagtttatca gaggcgaatg cataaactaa agggtc 1726

<210> 159

<211> 1156

<212> DNA

<213> Rattus rattus (black rat)

<400> 159
cctggagctt tcgccttcgc ctccggtacc gctacctgtt ctgaacggat ctccggccga 60

ctcgtccctg cgtctcatgg ccgctagcag atccctggtg cccgaccggc tgcgcctacc 120

actctgcttc ttgggtgtct ttgtctgcta cttctactat gggatcctgc aggagaagat 180

aacaagagga aagtatggag aaggacccaa acaggagaca ttcacctttg ccttaacttt 240

ggttttcatc cagtgtgtga tcaatgctat gtttgccaag atcttgatcc agttttttga 300

cactgccagg gtggatcgca ctcggacctg gctctatgct gcctgctctg tctcctatgt 360

gggcgccatg gtctccagca actcagcact acagtttgtc aactatccaa ctcaggtcct 420

tggtaaatcc tgcaagccaa tcccagttat gctcctcgga gtgaccctct tgaagaagaa 480

gtacccattg gccaagtacc tgtgtgtgtt gctaattgtg gctggcgtgg ctcttttcat 540

gtataagccc aagaaggtgg ttgggataga agagcacacg gtcggctttg gagagctcct 600

tctgctcttg tctctgaccc tggatggact gacaggtgtt tcccaggacc atatgcgggc 660

tcattaccaa acaggttcca atcacatgat gttgaacatc aacctttggt ccacggtctt 720

gctcggtgct gggatcctgt ttactgggga gctctgggag ttcttgagtt tcgccgagag 780

gtacccgacc atcatctata acatcctgct ctttggcttg accagtgcct tgggtcagag 840

ctttatcttc atgacagtcg tgtacttcgg ccccctgacc tgctccatca tcaccacgac 900

tcggaagttc ttcaccatct tggcttctgt gatcctcttt gccaatccca tcagctccat 960

gcagtgggtg ggcaccgtgc tggttttcct gggtctgggt cttgatgcca agtttgggaa 1020

aggaacaaag aagacctccc actaggaaaa gagaggcttc ctccactcca gaaacactta 1080

aattattatc tccaacagtg acatcttggg aaaatggact cagtcacgat aagggactgg 1140

gttccaatct ttttat 1156

<210> 160

<211>  3100

<212>  DNA

<213>  Rattus norvegicus (Norway rat)

<400>  160
gagtgctggg tttggtggcc aaagccccca tcctaggccg gcatgtctct gcagaggact      60

gtacgtgtat ccctggaaca cccaaccagc gctgtgtgtg tggcaggggt tgagaccatc     120

gtggacattt atgggtcggt tcctgagggc acagacatgt ttgaggtcta tgggacaccg     180

ggagtggaca tctacgtgtc tcccagcatg gagaggaacc gagagcgagc tgacaccagg     240

cggtggtgct tcaacaaggg gctggagatc atcgtggtca tgaactctcc tagcaatgac     300

ctcaacgaca gtcacgttca aatagcttac cactccagtc acgagcattt gcccctggcc     360

tatgcagtac tctacctcac ctgtgtggac atcaccctgg actgtgacat gaactgtgct     420

gaccggcagg acaggagctt cgtggataag aggcagtgga tgtggggacc tgatgggtac     480

ggagccatct tgctggtgaa ctgtgatcgg gacgaggtca gctctgatgc ccaagataac     540

tgtgaccaat gtgtgcgctg cctgcaagat ctggaagaca tgtccgtcat ggtcctacgg     600

acccagggcc ccgaatctct ctttgacgac cacaggctta tcctccacac ctccagctgt     660

gatgcggagc gggcacgggt cttccacgtc tgtggccccg aggattcctg cgaagcctat     720

aggtgtgttc tgggcccgga ccggatgtcc tatgaggtac cccgcctgaa gggctatgag     780

gagcgcttct acgtggaggg cctctccttc cctgatgcag gcttcccagg gatcctctcc     840

ttccatatca ccctgctgga tgactccaat gaggactact ccgagacccc gatcttcacg     900

gacacagtgg tgttccgagt ggcaccctgg atcatgacgc ccagcaccct gccacccctg     960

gaggtgtacg tgtgccaggt gaggaataac acctgctttg tggaagctgt ggaggagctg    1020

gccaggaagg caggctgcaa actcaccatc tgtcctcagg cagagaaccg caatgaccga    1080

tggatccagg atgagatgga gctggggtac acgcaggcgc cacacaagac tttgcctgtg    1140

gtctttgatt ctccgaggaa tggggagctg cagggtttcc cttacaaaag gatcctgggt    1200

ctagattttg gctacgtgac tcgagaacct ccagacagtt ctgtgagcgg tctggactcc    1260

tttggcaacc tggaggtcag cccgcccgtg gtggccaatg ggaaggagta tccactgggg    1320

aggattctca tcgggggcaa cctgcctggg tcgagaggcc gcagagttac ccaggtggta    1380

cgagacttcc tccatgccca gaaggtgcaa cctctggtgg agttgtttgt ggactggctg    1440

gctgtgggcc acgtggatga atttctgagc ttcgtccctg caccagatgg aaagggcttc    1500

cggctcctcc tggccagccc tggtgcctgc ttcaggctat tccaggaaaa gcagaagtgg    1560

ggccatggcc ggtccctcct tttcgagggg gtcattggtg acaggcgggt ccagaccatc    1620

tccatcaatc aggttctttc caaccagagc cttatcaact ttaataagtt tgcacagagc    1680

tgtattgact ggaaccgtga ggtgctgaag cgggagctag gcctggggga gagcgacatc    1740

attgacatcc cacagctctt caagtcggag aagaggaagg cggtggcgtt cttcccggac    1800

ctggtgaaca tgctggtact gggcaagcac ctgggcatcc ctaagccctt cgggcccatc 1860

atcaatggcc gctgctgcct ggaggagaag gtgcgctccc tgctggagcc gctgggtctg 1920

cactgcacct tcattgatga cttcacaccg taccacatgc tgcacgggga ggttcactgt 1980

ggcaccaacg tgcgccggga gccctttgcc ttcaagtggt ggcacatggt gccctgagcc 2040

gcccgccacc catcccccat ccgtccccca ggatagggca tccgcccggc aggtggagac 2100

tgcgacagac cccagagcac ccagagactc ccagtttcta gactgaatga cccaagcttc 2160

tcagaagcct tttctcttga aatgtccgca tccacctgct gcccattcag ttcccagaca 2220

tgaatcctct tggtgccccc aaagaagggt cccatgtcaa ccaaactctc ctgcgggtcg 2280

agatgatcca tggggataac tttaaatacc attcaccccc cccccacccc cgcaaactcc 2340

aaaactcctc tttgtgggga aaaatccgct tgggtatcta gaagcattga ccttggcttg 2400

tgcttcctat ggtggaaggc ctggggcggt gggggttggt ggggttgttt tggccattgc 2460

ctcctgccct gcaggggatg gttgtaaagt cagactattt cacaccctca ctactcgact 2520

agagggagtc taccctccca ggaagcaccc cgggtccatt caggtcaaag tgtgtccatc 2580

atgtccactg caagctcccg gcaggaccgg cctttactgc aagccagcct gggctatcac 2640

acggtgggga cctgtcacac aatggggacc tctagatgct tttgcaatga cagatgtccc 2700

ttagctttcc aggcttccca tcatccgatg tctgggaatc tggttgtcca aatccttttg 2760

gagtttctct gaagtgaatg attggggggc tggattccca ggggagaacg cctttcctaa 2820

gacaggattg accctcgggg tcccaccatc tgaaggggtt cctccatcag cttgcctggg 2880

ggctctgtgc agaggagacc ccaggctgac ttagcccaga gccttctgcc tatacagcca 2940

tcccgtagta gaggcccggg aagtgccacc ctgggtgcca tcaaggcagg acttagaggg 3000

acagttaagc ctctccacat actgggagaa gggagggatg tggctgtctc agtatgggtc 3060

ttctgtgagt tcagctggcc aaaaaataaa acagaggctc 3100

<210> 161

<211> 1523

<212> DNA

<213> Rattus norvegicus (Norway rat)

<400> 161
acttctacat caagatccgt cctggggagt atgagcagtt cgagagcacc atcggcttca 60

agctccctaa ccttcacctt cattgcgcca cggcggcttt cggacgagcc tctgactcgc 120

gcacgcgtta gactccttgg tccgtgttac aagacgggtc gggtgggtag ccgacatcgc 180

cgccgacccc gtgccgtcgc agccaagatg tccggtccca ccaccgacac gccggccgcc 240

atccagatct gccggatcat gcgtccggat gatgccaacg tggccggcaa tgttcacgga 300

gggaccattc taaagatgat cgaggaggct ggggtcatca tcagcacccg gcactgtaac 360

agccagaatg gggagcgctg tgtggctgcc ctggcccggg tggagcgcac tgacttcctg 420

tcgcccatgt gcatcggtga ggtggctcac gtcagcgcag agatcaccta tacttccaag 480

cactctgtgg aggtccaggt ccacgtgttg tcggagaaca tcctcacagg taccaaaaag 540

ctgaccaata aggccacctt gtggtatgtg cccctgtcat tgaagaatgt ggacaaggtc 600

cttgaggtgc ctcctattgt gtatttacgg caggaacagg aggaggaggg tcggaaacgc 660

tatgaagccc agaagctaga acgcatggag accaagtgga ggaacggaga cattgtccag 720

cccatcctga acccagagcc gaacacagtg agctacagcc agtccagcct gatccacctg 780

gtggggccct cagactgcac tcttcatggc ttcgtgcacg gaggtgtcac catgaagctc 840

atggatgagg tggccgggat tgtggctgcg cgccactgca agaccaatat agtgactgcc 900

tctgtggatg ctattaattt ccatgacaag atccggaaag gctgtgtcat caccatctct 960

ggacgcatga ccttcacaag caataagtct atggaaattg aggtcctggt ggacgctgac 1020

cctgtggtgg acaactcaca gaagcgctac cgggctgcca gtgccttctt cacctacgtg 1080

tccctgaatc aggagggcaa gccgctgcct gtgcctcagc ttgtgccgga gacggaggac 1140

gagaagaagc gttttgaaga aggcaaaggc cgctatctgc agatgaaggc gaagcgacag 1200

ggccatacag agcctcagcc ctagatgtct tcctccctcc catcctgtcc cgtcctgggt 1260

cagcacagtt gtggcagtag tcctgtgtgc agtcacttag aagtcgcccc cttggccaaa 1320

ccccgatttc ctttgagagc tggtgttgtg aagtaccgtg tgacagtgtt acctgtggcc 1380

tgttcccaaa acctgtgcac caaagcttta tttatatccc tccagtccct gtcccatgtt 1440

gtcccaaagg ccatcgtgga caccagagca cactgactgg cctggagaag ccagcaccac 1500

taataaagct gctgtctggc tgg 1523

<210> 162

<211> 981

<212> DNA

<213> Rattus norvegicus (Norway rat)

<400> 162
tcctttggtc tccgacggcc tttctaccca cacgcagcca tgtcttccga cagacccgtg 60

gcactggtga ctggtgctaa caaaggaatc ggattcgcga tcgtacgtga tctctgtcgt 120

aaattcttgg gggacgtggt cctcacggcg cgggacgagt cacggggcca cgaggcggtg 180

aagcagctgc agaccgaggg cctgagccca cgcttccacc agctggacat cgacaacccg 240

cagagcatcc gcgcgctgcg tgactttctg cttcaggagt acggaggact gaacgtgctg 300

gtcaacaatg cgggcatcgc cttcaaagtt gttgacccca cccccttcca cattcaagca 360

gaggtgacaa tgaaaaccaa cttttttggt acccaagatg tctgcaagga gctactccct 420

ataataaaac cccaaggcag agtggtgaat gtatcaagcg gcatgagtcg cagggccctg 480

aaaagctgca gcccggagct gcagcagaag tttcgaagtg agaccatcac tgaggaagag 540

ctggtggggc tcatgaacaa gtttatagag gatgcaaaga aaggagtcca tgcgaaagaa 600

ggctggccca atagtgcata tggggtcacc aagatagggg tgacagtcct gtccagaatc 660

tatgccagga aactcaatga ggagaggaga gaggacaaga tcctcctgaa tgcctgctgc 720

cctgggtggg tcagaaccga catgacagga ccagaagcca ccaaaagccc agaagaagga 780

gcagagaccc ccgtgtactt ggcccttttg cctccaggtg cagaggggcc tcacgggcag 840

tttgttcaag ataaaaaagt tgaaccatgg tgaatccaac tctcaccccc accccttcta 900

tcctgacttg gtgaaagcca agggacattt ataatatacc atcacttctg gaaaaataaa 960

cataactaag tctttaagca c 981

<210> 163

<211> 1928

<212> DNA

<213> Rattus norvegicus (Norway rat)

<400> 163
gagcctacgc acgtgtcctg tctgtctcca gactccgcga cccggcgcag cagccatggc 60

ttcctcccag cttgccctgt tcagtgtgtc tgacaaaact ggcctcgtgg aatttgccag 120

aaaccacgcg tctcttggtt tgagtttggt cgcttctgga ggcacggcaa aagcgatcag 180

ggatgctggc ctggcagtga gagatgtgtc tgagctaact gggttccctg aaatgttagg 240

ggggcgtgtg aaaaccttgc atcctgcagt ccatgctgga atcttagcac gcaatattcc 300

agaagatgct gctgacatgg ccaggcttga tttcaacctc ataagagttg ttgtctgtaa 360

cctgtacccg ttcgtgaaga ctgtggcttc tccagatgta accgttgagg cagctgttga 420

gcaaattgac attggtggcg tgaccttact gagagctgca gccaaaaacc atgctcgagt 480

gacagttgtg tgtgagccag aggactatgg tgcggtggct gcagagatgc agggctctgg 540

taataaggac acctccctgg agaccaggcg ccacttggca ttgaaggcat tcacgcatac 600

cgctcaatat gatgaagcga tttcggatta cttcaggaga cagtacagta aaggaatttc 660

tcagatgccc ttgaggtatg ggatgaaccc ccatcagact cctgcccagc tgtatacact 720

gaagcccaag cttcccatca cagttctcaa cggagcccca ggattcataa acctctgcga 780

tgctttgaat gcctggcagc tggtgacaga gctgagagga gcagtagaca ttccagctgc 840

ggcgtctttc aagcacgtca gcccagcagg tgctgctgtt ggggttccac tcagtgagga 900

cgaagccaga gtctgcatgg tctatgacct ctatccgacc ctcacgcccc tggccattgc 960

atatgcaaga gcaagaggag ctgataggat gtcttcattt ggtgattttg ttgcattatc 1020

tgatgtttgt gatgtcccaa ctgcaaaaat tatctccaga gaagtgtcag atggtattgt 1080

tgccccagga tatgaggaag aggccttgaa aatactttct aaaaagaaaa atggaagcta 1140

ctgtgttctt cagatggacc agtcctacaa accggatgaa aatgaagttc gaacactctt 1200

tggtcttcgt ttaagccaga agagaaataa tggtgttgtt gacaagtcgt tgtttagcaa 1260

tattgtcacc aagaataaag atctgccgga atctgctctc cgagacctca ttgtcgccac 1320

catcgctgtc aagtatactc agtctaattc ggtgtgctat gccaaggacg ggcaggttat 1380

tggcatcgga gcaggacagc agtctcggat acactgtaca cgccttgcag gtgataaggc 1440

aaactcttgg tggcttcggc accatccacg agtgctttcc atgaagttta aagctggggt 1500

gaagagagca gaagtctcca atgccatcga tcagtatgtg accggcacca ttggtgaggg 1560

tgaagacctg gtgaaatgga aagcactgtt tgaagaggtt cctgagttac tcactgaggc 1620

ggagaagaag gaatgggtgg acaagctgag tggcgtgtct gtcagttccg atgctttctt 1680

tcctttcagg gataatgtag accgagcgaa aaggagtggt gtggcctaca ttgttgctcc 1740

atccggatct actgccgaca aggttgtgat cgaggcgtgt gacgagctgg ggatcgtgtt 1800

ggctcataca gatctgcgtc tcttccacca ctgaccttgg ctgacattgt tacattgttt 1860

cctagtggga gatactcatg tgtgggaccc tgaaagtatc tctttttaaa cagagtcatt 1920

cttatctt 1928

<210> 164

<211> 4269

<212> DNA

<213> Rattus norvegicus (Norway rat)

<400> 164
tctcggccgc ggtgtttccg tttctctccc tggttgtcgc ctgctctgct cttctcatcc 60

ttctcgctct gctgctctgc ggagtgacga ggctgaatct tctccccacc cagttcgcgc 120

ttttcttctt tttctcgcgc cgctccattt ctccagtccg gctgccgccg ccactgctgc 180

tcagctggtg tcggtgccgc gcgtttcccc cacgtcgtcc ccgcagccta tggcccaggc 240

cgccttgggt atttctgctc aaggtaacca catccctctt taaaaattcc gccgaaaaag 300

agaagacgct ttacccgact ctttgggccg ttatctcacg gcgaactttc tgaccgagta 360

tacaactacc cagaaggcct aggagaagtg ctgtatagag agcagttcga ttttaacgct 420

gagccacctt gggaacctag ctgatgatag gggggttcca tctcctaact tgtccatgga 480

ggtcttcact tcagaaaccc aagactcaca ttcatccagc ttggtgtcaa gtgggctgtt 540

gctgccagga ttatcttgtg attatatgaa agatgtatca gtttcttctg aagtgcagtc 600

aactgtagaa gcctttgtag caggtgtgta acaagtttgt tgcatattct aaggacccag 660

acataggctt ggtggcccgt ctcttgtttt tcctggttta tgactttcgg ctttgtggaa 720

tacggctgag atgaaaggat ttattgacga tgcaaactac tccgttggcc tgttggatga 780

aggaacaaac cttggaaatg ttattgataa ctatgtttat gaacataccc tgacaggaaa 840

aaatgcattt tttgtggggg atcttgggaa gatcgtgaag aagcacagtc agtggcagaa 900

cgtggtggct cagataaagc cattttacat ggtgaagtgc aactccactc cagccgtact 960

ggagatcttg gcagctcttg gaactggatt tgcttgttcc accaaaaatg aaatggcttt 1020

agtgcaagaa ttaggcgtat ctccagaaaa catcatttat acaagtcctt gtaagcaagc 1080

atctcagata aagtacgcag caaaagttgg agtaaatatt atgacatgtg acaatgaagt 1140

tgaattaaag aaaattgcga ggaatcaccc aaatgccaag gtcttactac atattgcaac 1200

agaagataat attggaggtg aagatggtaa tatgaagttt ggcactacac tgaagaactg 1260

taggcatctt ttggaatgtg ccaaggaact tgatgtccaa ataattgggg ttaaatttca 1320

tatttcaagt gcttgcaaag aatatcaagt ctatgtccat gccctgtctg atgctcgatg 1380

tgtgtttgac atggctgggg agtttggctt tacaatgaac atgttagaca tcggtggagg 1440

cttcacagga actgaaattc agttggaaga ggttaatcat gttatcagtc ctctgttgga 1500

tatttacttc cctgaaggat ctggcattca gatcatttcc gaacctggaa gctactatgt 1560

atcttctgca tttacgcttg cagtcaatat tattgctaag aaagttgttg aaaatgataa 1620

attatcctct ggagtagaaa aaaatgggag tgacgagcca gccttcgtgt actacatgaa 1680

tgacggtgtt tatggttctt ttgcaagtaa gctttctgag gacttaaata ctgttccaga 1740

ggttcacaag aaatacaagg aagatgagcc tctgtttaca agcagccttt ggggtccatc 1800

ctgtgacgag cttgatcaaa ttgtggaaag ttgtcttctt cctgagctga gcgtgggaga 1860

ttggcttatc tttgataaca tgggagcaga ttctttgcac ggaccgtctg cttttagtga 1920

cactcagagg ccagctattt atttcatgat gtcactcagt gattggtatg agatgcaaga 1980

tgctggaatt acttcagatg caatgatgaa aaacttcttc tttgcaccct cttgcattca 2040

gctgagccaa gaagacaact tctccactga agcttaaaca ggcattaacg cttctttaga 2100

tctgaagtcg caggttaagc ttgtctggtc tacattccag tgtggggaaa aattcaatca 2160

atctttattc tgttaatttt cttggaaaca aattcatagt aatagctgtt tgggaccaga 2220

caaaatcagc tttcatctat aattcattgg gggtaatagg ggatttattt agataatgta 2280

tccagattta aacataccag tttgacccac cccttaagcg tttaaaataa aatatgcaac 2340

aaaatggatg acttagtgga gatggaagcc cattaattgg gttccccatc aaatcgttta 2400

catacaagta cacagttttt ataataagga ttcctgttaa aagtcttgta aagttgatgt 2460

ctttcaccca gatctatcac atgtgagtgg aagaccagtg tgacttcatt agatacgttt 2520

agtgtattta gagtgtgtaa atttgtgctt tggactgtag tttaataaat gtaaaattgc 2580

atcatagtat ttgttgtatt tgacctaatg taaccttgta tgattgcaat aaaactttgt 2640

gtagatttta ccattttttc aggctaaaaa tttgggaatg gggctagcta gcaaaggtag 2700

ttttgaacta gttgtgtgta tggtctgttt tgagagtttt ctttatagtc catttgtttg 2760

gtttggctca atatactttt tagttattta attagtaatt taagtaaaaa gtgtttggta 2820

atcattgtga agttcagatt cattatggag agtcgatgtg cagtaagcat gatgtttaac 2880

aattttaaca ccagaaaatg ttaatcttgc ataaaccaat tgtaataata ataggtgttt 2940

ctgtatagat aggatgcata gagtacctta gtaaatcttt gaatcacaaa tcttttggct 3000

gatagaaaag attttattaa acactgaaaa cttggtcggg gatggggaga gagactgcag 3060

aagactgcag aaccctaacc ttaaagaagg gctatgtcta tttccagcat cctgatgccc 3120

cttcgcacag acatttaaat cgagagggct ttggggtggt gaagcacact cttcaatttc 3180

tcacttgtaa tggtatcaaa ttgcaatttt tttgttgttg ctgatcttgg tttgaaccta 3240

gtcccttagt ttcttgttaa ttcccatttg ttcaagctta gtaattcttc cttccgtact 3300

aaagcttggt gggggtgggg gggaggagtt agcttcacta acctgacact gttgccagga 3360

attttggctt tgttcactgc tagtgtgtta gtcctaagtc tcagaatcac agaagtaatg 3420

agacaactta caggggtcac ttttcctacc ttactctatg ttcacaagtg gaattcctga 3480

cctccaagag gaaatgtgac ttcactttgg tgccaatgga caggaaattc tacctgtgct 3540

acataggaga agtgtgggat acacttgaat agctggattt tacaccttga tttcaaggtg 3600

gaaagaaatt gatcatgaat ctctaatttc aaatcaaacc agtaggtgct taatattttt 3660

gatttgaata ttgcccactt gaatatcatg ggttctatta attataagaa caactaagga 3720

ccccagtcca ctgtcatcta gttgcccttg gacttttttc caaggtatac tatggggttt 3780

atgcaaaatt tgaagctacc atatgcattg ttgtggtcca ggaatgccaa aacttaaagc 3840

gtggtacaac ctgagtcctt ggcctgactg tacaggcgtg gtctctgtgg catcagtttt 3900

gccttctgca taggagaaag gttgtatgta acacagcccg gaattgctat ctcacatgtg 3960

aatgataggt aaacataaga atgtctgtat tgtttttgaa gactgtttgc cataaatcta 4020

aagtcaaacc tatgtatttc aatttggtat gcttaagaat aattaatgta aaatcttttt 4080

atatgatatt gtaatctcag ttcaaattta attgcaaata taaaacccaa atgatttcta 4140

tatatagtaa attgaactgt caaggtaact tgtgtgtgat tctgaataca cagatacaat 4200

gtttttattc ctcatgcttt actttgatgg cttctatcat gaaatagatg tgaaatttta 4260

tgaaaaaaa 4269

<210> 165

<211> 3657

<212> DNA

<213> Rattus norvegicus (Norway rat)

<400> 165
ccaacacaga actatggaag tccacgaatt gttccggtat tttcgaatgc cagagctgat 60

tgacattcgg cagtacgtgc gtacccttcc aaccaacaca ctcatgggct tcggggcttt 120

tgcagcgctc accaccttct ggtatgccac ccggccgaag gccctgaagc caccatgtga 180

tctgtccatg cagtctgtgg aagtaacggg tactactgag ggtgtccgaa gatcagcagt 240

ccttgaggac gacaagctct tgctgtacta ctacgacgat gtcagaacga tgtacgatgg 300
cttccagagg gggattcagg tgtcaaatga tggcccttgt ttaggttcta gaaagccaaa 360
ccagccatat gagtggattt cttacaaaca ggttgcagaa atggctgagt gcataggctc 420
ggcgctgatc cagaagggtt tcaaaccttg ctcagagcag ttcatcggca tcttttctca 480
gaacagacct gagtgggtga ccatcgagca ggggtgcttc acttactcca tggtggttgt 540
tccgctctat gacacgcttg gaaccgacgc catcacctac atagtgaaca aagctgaact 600
ctctgtgatt tttgctgaca agccagaaaa agccaaactc ttattagaag gtgtagaaaa 660
taagttaaca ccatgcctta aaatcatagt catcatggac tcctacgaca atgatctggt 720
ggaacgcggc cagaagtgtg gggtggaaat catcggccta aaagctctgg aggatcttgg 780
aagagtgaac agaacgaaac ccaagcctcc agaacctgaa gatcttgcga taatctgttt 840
cacaagtgga actacaggca accccaaagg agcaatggtc acccaccaaa acattatgaa 900
cgattgctcc ggttttataa aagcgacgga gagtgcattc atcgcttccc cagaggatgt 960
tctgatatct ttcttgcctc tcgcccatat gtttgagacc gttgtagagt gtgtaatgct 1020
atgtcatgga gctaagatag gatttttcca aggagacatc aggctgctta tggatgacct 1080
caaggtgctt cagcctacca tcttccctgt ggttccgaga ctgctaaacc ggatgtttga 1140
cagaattttt ggacaagcaa acacgtcagt gaagcgatgg ctgttggatt ttgcctccaa 1200
aaggaaagag gcggagcttc gcagtggcat cgtcagaaac aacagcctgt gggataaact 1260
catctttcac aagatacagt cgagcctggg cgggaaagtc cggctgatga tcacaggagc 1320
agccccagtg tctgccacag tgctgacgtt tctgagggcg gctctcggct gtcagttcta 1380
tgaaggctac ggacagaccg agtgcaccgc tggttgctgc ctgagcttgc ctggagactg 1440
gacagcaggc catgttggtg cccccatgcc ttgcaattat atcaagcttg tagatgtgga 1500
agatatgaat taccaggctg ccaagggcga gggtgaggtg tgtgtgaaag gggcaaatgt 1560
gttcaaaggc tacttgaaag acccagcaag aacagctgaa gccctagata aagatggttg 1620
gttacacacg ggggacattg gaaaatggct gccaaatggc accttgaaga ttatcgacag 1680
gaaaaagcac atatttaaac tagcccaagg agagtacata gctccagaaa agatcgaaaa 1740
tatctacctg cggagcgaag ccgtggccca ggtgtttgtc catggagaaa gcttgcaggc 1800
ctttctcata gccattgtag tacctgatgt ggagatccta ccttcctggg cacagaagag 1860
aggatttcaa gggtcctttg aagaactgtg caggaacaag gatatcaata aagctatcct 1920
ggaagacatg gtgaaacttg ggaagaatgc tggtttgaag ccatttgaac aggtcaaagg 1980
cattgctgtg cacccggaat tattttctat tgacaatggc ctcctgactc caacactgaa 2040
ggcaaaaagg ccagagctgc ggaactattt caggtcacag atagatgaac tgtactccac 2100
catcaagatt taacatgagg aagaatcctt agaggaaatg gcgcacctcc acaatcctcc 2160
tcgtaccaat ggcctttgag ttggtaactc tgcctgcagc gagtgtggga aaggaaatgc 2220
catgctggac ttgtgcacag ggtcttacca tagggatagc agagggcacg gaacactgcc 2280

ttacagtcac ctgtgttgta gcccatgatc ctggggatac acagtttcca aaacgagcct 2340

taaacattgt aaaggggaac ccataaaagt gctaagttat ttaagacttc aaccaataag 2400

gtgggtgtta aaacttctgt ctcctgtttt tctaaccaag gggttaggac ttactctttc 2460

tgatacgtct gctgctggct gcacaccctg cagccgtctg ctgctctgaa gagtacagta 2520

cactggaaga aagctacccc tttaagagca actgtccagg ctgaagaaag tcacagtgga 2580

ccagagattt ctcttcttaa ccccttcccc ttgtcccttt ccctcaccac tcacatgcag 2640

tacatttatg tgacccttct ggtttgtaag ggttccaaag ggtccctgtt gtgcctgctg 2700

gtaacctcgt gtttccagta cactaaagaa cagatcctcc tcagaacaat gcgggtaccc 2760

agtctcagaa aaggaaaaaa aaaaaaaggt ggctcctgcg ttgagccagc aacttgcagg 2820

ctcacacaga tgtcctcaga ctccgctcca tctctgctcc ctgcccctcc cattcttccc 2880

aggggttatg agaaaccctg ccttagactc tgcagtgaag atggtgttcc gaagaatgac 2940

ctggatgggc catgctcaac tgtgattcct cccccgctgt ccactcgctc gctcgtcgtg 3000

gaaccagccc gtatctgcag cggcctctgt gctccaaatg tggttttcta gcaaatgcta 3060

atgtggtgat caacttgctt tgttttcttt ccctcccagt taaactcagg cacagcatgt 3120

cttcagaagt atctaggcag ttggtatacg tgggtactta ttaaagtgga cagtaataag 3180

taaatgtcct tattataaaa ctacctgatt tatgtgaaat gcatttgata agccaaaaat 3240

ctggggtgta gaaatctggg ggaaaaaaaa tcatatcctg ggatcaactt cccaaggtat 3300

attttttaag ttaatttggg aaatgaacag catttcactt tgccgcgagt cttggccaca 3360

tttgacctat ttgagctgtt tatacatttg aagccactgt tgttggctgt gtgacagtgt 3420

gtgtatcttg aagcacaact gggtttgcac taaagaatca tcattgtaat aacactattt 3480

ggtagcctaa cttcattttc gtattcttaa ctgcacagga agtcgataat ttgtcacaac 3540

ggggttttga atgtttgctt tcagtgttgg ctatttctat gttttataaa ccaaaacaga 3600

atttccaaaa acaatgaagg aaaccaaaat aaatatttct gcatttggag tgagtgg 3657

<210> 166

<211> 1174

<212> DNA

<213> Rattus norvegicus (Norway rat)

<400> 166
ccgcagcctt ctttgacgat caccgaaccg tagttgaggc cgtgttcctg tgtccgctgc 60

tgtgtctggt tgtaacctcg ccggagctat gtttcgcaac cagtatgaca acgatgtcac 120

tgtttggagc cctcagggca ggattcatca aatcgaatat gcaatggaag ctgtaaagca 180

aggctcagcg acagtcggcc tgaagtcgaa gacacatgca gtgctggtcg cgctgaagag 240

agcacagtca gagcttgccg ctcaccagaa gaaaattctc cacgttgaca accatattgg 300

tatctcaatt gcgggtctaa ctgctgatgc cagactgtta tgtaacttta tgcgccagga 360

gtgtttggat tccagatttg tatttgacag accacttccc gtatctcgcc ttgtgtctct 420

aattggaagc aagacccaga taccaacaca gcgatatggc cggagaccgt atggtgttgg 480

gctgctcatt gctggttatg atgacatggg ccctcacgtt ttccaaacct gcccatctgc 540

taactatttt gactgcagag ctatgtccat cggagcccgt tcccagtcag ctcgcactta 600

cctggagaga cacatgtctg aatttatgca gtgcaatttg gatgaactgg ttaagcatgg 660

tcttcgcgcc ttaagagaaa cactccctgc agaacaggac ctgaccacaa agaatgtttc 720

cattgggatt gttggtaaag acttggaatt tactatctat gatgatgatg atgtgtctcc 780

attcctggat ggtcttgaag aaagaccaca gagaaaagca cagccttcac aggctgctga 840

tgaacctgca gaaaaagctg atgaaccaat ggaacattaa gtgataaagg ttatgaggac 900

atgaggatgc aggggcatac actggtgaca ataatctgta ttttaaacca acagctgtaa 960

tgtattgggt ggtatgtttt agaaatcagt ccaactgtga gttttctcta agcagcttca 1020

cagaaaccat ataatggggt gcattttctt tgaaagggtc tacataatca ttttctagga 1080

cgtataggta tctatatcaa tgtttttata tgaagaaaat aagtgtcttt gcagttttaa 1140

agacaactgt gaaataaaat tgtttcacca cctg 1174

<210> 167

<211> 1983

<212> DNA

<213> Rattus sp.

<400> 167
ggaccgtcgc catgcgtcgt gtaccgctgc tgctcgtgct gggcttgctg ttcgtgctgg 60

gcttgctgtt cgctggccct gtcgcccctt cgcgcctcat ctcgaagcgc ccttcccaac 120

ttggtggctt ctcctgggat aactgtgatg aaggaaagga ccctgcagtg atcaaaagcc 180

tcacgctcca acctgacccc attgtcgttc ctggagatgt gatcgtcagt gctgagggca 240

agaccagcat tcccctcact tctcctcaga aggtggagct caccgtggag aaggaagtgg 300

ctggcttctg ggtcaagatc ccttgcgtag aacagctagg aagctgtacc tatgagaatg 360

tctgtgacct gatagaccaa tacatccccc ctggagagac ctgcccagag ccgctgcaca 420

cctacgggct gccctgccat tgtcccttca aggaaggcac ctactcactg ccttcgagca 480

acttcacagt gcctgatctg gagcttccaa gctggctaag cacgggcaac taccgcatcc 540

agagcatctt gagcagcggt ggaaagcgcc tggcctgcat caagattgcc gcctctctca 600

agggcaaata accaggcagc agccaccata gaatgaaagg gcaccaggaa ggcatgcttc 660

ttcttctgtg ttccggggcc cagagctgcc tgcctctaac tccgttctat ggttctctac 720

cctttctgca aatcacgaca acctgacctg agggaggatg gaccaggcgg gtctcgacat 780

ctgttgggct gtacgccatg ctccttcgca cccctaccaa ctttctaaga gtttctctca 840

tgtcccaaac agtcaaggaa tgggaaccaa catgttatgg aatcttaagt tcaagctggc 900

ccaacatggg cctcctgaag ctatgctcac cccacctctt cgttttagcc accaacttaa 960

aaaccaagat gacagtatta atctctcatt ccaccctaaa cctctcatcc agcaaagagg 1020

tctaggtagc cagtcttagg tctgcccccc tattaacttc catgattaac tacattccct 1080

ttgcaacttg cctagccaac actggggctg acagatcctg aaagggcagg atggcgtgtg 1140

acagaaaccc tgggagagtt attgttgggc atgccccatt aacctcagta aagcacaggt 1200

gcccagggcc aagcagatcg agggagccaa agcagtcact cccaggagcg atgcccactt 1260

ttcttccttt ggtatgacgc atacagaatg ggatgacatt ccttatacct gacaagaaac 1320

aaaactgaaa ccccaacagc acatgtgctc cagtgccagg aggccagggt ctggaatggc 1380

atgtctagtc ctggaggtac agtccagtga cctgtaagtg ccatgtaagg aatgttaccg 1440

gatatttgca acagttttct tccttaggcc ccaaacccca gacttttcta tgaagtctta 1500

gattttttaa ctattagaaa caaattgttt tttatatact gttcaggcca aaggggccac 1560

gtggacttgt ctggggggtg gggggggaca tagctggctc acaggtggct gctttttttc 1620

tcacacctgt tcgtcttctc agtctcaagt tatctgactt catttctctc cccctcaagg 1680

tcaagtcctt ggtaccagac ctcagagagg gctcagcctg cactggagca ggtctgcatg 1740

gcctctgaga gctgggccct gtgccagcct cagagcccca tcaccatcag ggtatagggg 1800

ttcacactgg agtggatctc cccaccccca ccccccagca gccaccttgt ggaacagggg 1860

tcccatgtag agagtggatc ccacaccggg ttgtaccatg acaacttcct gagcctccgt 1920

cagcccggtt ctggactgca agactagtct gtcaataaat cttcttaaaa aaaaaaaaaa 1980

aaa 1983

<210> 168

<211> 1733

<212> DNA

<213> Rattus sp.

<400> 168
ggccgctgca cctgcccggt cttgcgccga gaccgctgtc aatcgcgggg caccaggaat 60

aaaaggcccc ttgcagagat ttgaaaatgg ccatgaacaa aagtgttggc ctcttcagct 120

ccgcatccct ggctgtggac tacgtagatt ccctgctgcc tgagaatcct ctgcaggaac 180

cctttaagaa tgcttgggtc tacatgttgg acaactatac aaagttccag atcgcaacct 240

ggggctccct gatcgttcat gagacgatct atttcctgtt ctctttacct ggctttctat 300

tccagtttat acctttcatg agaaagtaca agattcaaaa ggataaacca gaaacctttg 360

aaggccagtg gaagtgtttg aaaggaattc tgtttaatca tttttttatc cagttgcctc 420

tgatttgtgg aacatactat tttacagagt ttttcaatat tccttatgat tgggaaagaa 480

tgccaagatg gtatttcact ttggcaaggt gtttgggctg tgcagtcatc gaggatacct 540

ggcactattt cctgcacaga ctccttcacc acaagaggat ttataaatac attcataaag 600

tccatcacga gttccaggct ccatttggaa tcgaagcaga atatgcacat cccttggaaa 660

ccctaattct tgggactgga tttttcattg gaattgtgct tttgtgtgat catgtaattc 720

ttctgtgggc atgggtgacc atgcgtttgc tagaaaccat cgatgtgcac agtggttatg 780

atatccctct caacccgttg aactacatcc ctttttatac cggggctcgg catcacgatt 840

tccaccacat gaacttcatt gggaattacg cctccacctt cacgtggtgg gacagaatct 900

ttgggacgga tgtccagtac catgcctaca ctgaaaagat gaagaagctt gggaaaaagt 960

ctgagtgagc acctgctctc cgccctcctg actgcagctg gcaactaaca ttgcttctgg 1020

ggagcagaac taagcacgtg actcggctgc tggtgatagg aacattaaca acctttaagt 1080

actctgcggg atgagaactc ttctgctcga cacccaagat ctgtaaatgt gggaacaaag 1140

aaatagatac ataagtacag ttttcaggag gaggttttaa aggccatatt gccaacctca 1200

ccgaaaggtt tcaggaaccc gaggaagtgt taatgtacaa ctcaccactt cacgccaccc 1260

gaggctgaag ttgacgttgc cttttaagcc tttttacata cactggccat ttcagaaaat 1320

tctcaacaat aatgtctgcc ttcgagttta agtcatggtg ttttttagaa ttgacttgaa 1380

atgaaaatat cacaaagtga atatatcagc tggtgatcga gtgactgaaa cccccctggt 1440

ctgcggttga ccagttcagg actattcttt ctgtgaagga ttgtttgatt gtagtatctt 1500

tatcagcatc ttgtaaattg gctcatttcc tacgtgatgt acatagaata ctataactta 1560

acgactttca agttacttgg ggcattttta aattgaggag ccccacttct gatgtccacc 1620

cccttgtctt tgtgtgtcag gtggtctcgg agctttgtga attctctatc tgcacctgtc 1680

agagctggtg aaataaatgt gatttttgtc cgaaaaaaaa aaaaaaaaaa aaa 1733

<210> 169

<211> 2634

<212> DNA

<213> Rattus norvegicus (Norway rat)

<400> 169
acactgtgac cagaaggact ttgatgttca aaatgcccag aaagtggatt tctgctctgt 60

tcctgctaca gataagttac tgtttcaaat ctgggcactg tgggaaggtg ttggtgtggc 120

cgatggactt tagtcactgg atgaatataa aaataatcct tgatgaactt gtacagaggg 180

gccatgaggt cactgttctg aaaccttcgg cttacttttt tcttgatccg aaaaaatcgt 240

ctgaccttaa gtttgaaatt ttttctacat ctatcagtaa agatgagctg caaaatcatt 300

tcataaaact tttggatgtg tggacttatg agttgccaag agatacatgt ttgtcatatt 360

ctcctatcct tcaaaatcta gtttatgaat tttcttattt ttatctaagt atttgtaaag 420

acgctgtttc aaacaagcag ctcatgacaa aactacagga atccaagttt gatgttcttt 480

tcgcagatcc tgtggcttcc tgtggggatc tgatagctga actgctccac attccttttc 540

tgtacagtct tagcttctct ccaggccaca aacttgaaaa gtccattgga aaatttatac 600

tccctccatc ttatgtgcct gtaattttgt cgggactggc tggcaaaatg acattcatag 660

acagggtaaa aaatatgata tgtatgcttt atttcgactt ttggttcgag agacttagac 720

acaaggaatg ggacacgttt tacagtgaga ttttgggaag gcccaccacc gtagatgaga 780

caatgagcaa agtagaaata tggcttatta gatcctattg ggatttgaaa tttccccacc 840

caacattacc aaatgttgac tatattggag gactccattg caaacctgct aaacccttgc 900

ctaaggatat ggaagaattt gtccagagct ctggagagca cggtgtggtg gtgttttctc 960

tggggtcaat ggtcagcaac atgacagaag aaaaggccaa cgcaattgca tgggcccttg 1020

cccagattcc acaaaaggtt ctttggaaat ttgatggcaa aaccccagca acattaggac 1080

ccaataccag agtctacaag tggctcccgc agaatgacct cctgggtcac ccaaaaacca 1140

aagcctttgt aactcatggt ggagccaatg gcctctatga ggcaatctat catggaatcc 1200

ctatgattgg cattcctctg tttggagatc aacctgataa tattgcccac atggtggcca 1260

aaggagcagc tgtttcattg aatatcagga caatgtcaaa gttagatttt ctcagtgcac 1320

tggaggaagt catagacaat ccgttctata aaaaaaatgt tatgttgttg tcaaccattc 1380

accatgacca gcctatgaag cccctggaca gagctgtctt ctggattgag tttatcatgc 1440

gccacaaagg ggccaagcac ctgagaccac ttggacataa ccttccctgg taccagtacc 1500

actctctgga tgtgattgga ttcctgctca cctgttttgc agtcattgca gctcttactg 1560

taaaatgtct cttgttcatg taccgattct ttgtaaagaa ggaaaagaaa atgaagaatg 1620

agtagagctc attgacaatg cactacagaa atgaaatttc agcgtcattc taatttatga 1680

accaccttct aaaaattact aatttttatt caaggtagat aacctttgta ggaagacata 1740

taactccgtg aatactgata tgtactcaaa aatccatcat ttttaaattt taaaaccact 1800

taatgtaaaa gttacattgt agaaaaatgt gcagaataaa attattcttg atagagtcca 1860

aataatcaaa gtattaacct taaaatattt gaatagtgtc cattagcttc tttgtctaat 1920

actgaatctg tagctttcat acaataaaat gtagataact tgtacacgat aaatactggc 1980

atataatttt ttttctgtaa tagacctaat tacttgttgt ctgggataaa gtgtggttag 2040

tttggatatc agttttaaag gaatctgttg gctcttttgt tcctctaaca taatatgcta 2100

acatagagca tctcaaggga aaagcagaac agtttatttg gagttgtgac cacagttcct 2160

taaaatgtag agtagcggag aaggcagggc agcagtcatc cagaggagga agctcacttg 2220

tcacatctca accatatatg gaaagcagag agtgagcaag aagtagggct aagctatgaa 2280

ctttcaaagc ttagtcgagt gacgtacttc atccaaaaag ctttaaccac tttaattaag 2340

attccataac ctcaatctgc actacaaatt ttggaaaatg tgctcaagtt cataaaccta 2400

tctgaaacat tttacattca aaccaataac aggaaatcat tctgtttctt aactttttta 2460

gcacacctta tgctgttaac attctagtcc tccaagttct aacacaccac catagaacac 2520

ctgtacttct tcagtatact ttaactatga tcatgaggaa taatgttcat tggaaatgaa 2580

acttatgaac agggaacaaa ttgaagccct aaataaagta aacttagtat tgag 2634

<210> 170

<211> 1377

<212> DNA

<213> Rattus norvegicus (Norway rat)

<400> 170
gttcgggatt cacacataca cttcagaatg ccgggtctaa gttgtagatt ttatcaacac 60

aaatttcctg aggtcgaaga tgtagtgatg gtgaatgtaa gatccattgc tgaaatgggg 120

gcctatgtca gcttgttgga atataataac attgaaggca tgattcttct tagtgaatta 180

tccagacgac gtatccgttc tataaacaaa ctgatccgaa ttggcagaaa tgaatgtgta 240

gttgtcatta gagtggataa agaaaaagga tatatagatt tgtcaaaaag aagagtttct 300

ccagaggaag caatcaaatg tgaagacaaa ttcacaaaat ccaaaactgt ttatagcatt 360

cttcgccatg ttgctgaggt attagagtat accaaggatg agcagctgga aagcctattc 420

cagaggactg cctgggtctt tgatgacaag tacaagagac ctggatatgg tgcctatgat 480

gcctttaagc atgcagtctc agacccatct atcttggata gtttagattt gaatgaagat 540

gaaagagaag tactcattaa caatatcaat aggcgtttga ccccacaagc tgtcaagatt 600

cgagcagata ttgaggtagc ttgctatggt tacgaaggca ttgatgctgt aaaagaagcc 660

ctgagagcag gtttgaattg ttctacagaa accatgccca tcaagattaa tctaatagct 720

ccacccaggt atgtgatgac aacaacgacc ctagagagga cagaaggact ctctgttctc 780

aatcaggcta tggcagtcat caaagaaaag attgaggaga agaggggagt gttcaatgtt 840

cagatggagc ccaaagtggt tacagataca gatgagactg aacttgcaag gcagctggaa 900

cggcttgaga gagaaaatgc agaagtggat ggagatgatg atgcagaaga aatggaagcc 960

aaagctgaag attaaccttt tggaaaacag tccaatttaa ggagtacgaa gcagcccttt 1020

ctggctgtaa accctagact tgaaagtttt ccagtattga aaacttcaaa gctgaatatt 1080

tttatttcca agtatttaag tattcgacaa gccagaatct aaatgccctc cttcatgtca 1140

gctgttttca catagtggct ctaacacctc aagcgttttt aagggagtgg cttgatttga 1200

ccagagacaa atgttaaacc gcagtcctaa aattgggctt gcggttttca tttctgatgt 1260

ctctggattg gcacccttat ggtttagaga attaccaggg gctccagaca ccaacaatcc 1320

caacctttct atataaaatg tactcaagca aacatcaaat aaatttctgg gatattt 1377

<210> 171

<211> 1501

<212> DNA

<213> Rattus norvegicus (Norway rat)

<400> 171
cagggtcgtc ctggcaacca gctgctcagc caatcagcgc ttgacactac gatccgcccc 60

caacttctgg agccatggcg gtgagaagag ccctgattgt attggcccac gcagagagga 120

catcattcaa ctatgccatg aaggaggctg ctgtggaggc tctgaagaag aaaggatggg 180

aggtggtcga atctgacctc tatgctatga actttaaccc cctcatttcc agaaacgaca 240

tcacagggga gccgaaggac tcggagaact ttcagtaccc tgttgagtca tctctggcgt 300

ataaggaagg ccgcctgagc ccggatattg tagctgaaca gaaaaagctg gaagctgcag 360

acctggtgat atttcagttc ccattgtatt ggtttggggt gcccgccatt ctgaaaggct 420

ggtttgagag agtgcttgta gcaggattcg cctacacgta tgccaccatg tatgacaagg 480

gtcctttcca gaataagaag accttgcttt ccatcaccac cgggggcagc ggctccatgt 540

actctctgca gggtgtccac ggggacatga acgtcattct ctggccaatt cagagtggca 600

ttctgcgctt ctgtggcttc caggtcttag aacctcaact ggtgtacagc attggccaca 660

ccccacccga tgcccgcgtg caggtcctgg aagggtggaa gaagcgtctg gagactgtct 720

gggaggagtc accactctac tttgctccaa gcagcttgtt tgacctaaac ttccaggcag 780

gattcttact gaaaaaagag gttcaagagg agcagaaaaa gaacaagttt ggcctttctg 840

tgggccatca tttgggcaag tccattccag ccgacaacca gatcaaagct agaaaataag 900

gttttccata ccatgtagtt agacccaggt ttctttttcc ccagcttgcc tggcttgctt 960

tcagttttcg cctttgttcc acaaggatag gaaaaggagg aggctcgcct catgcgtttt 1020

tggatagttc tgccacggcg tgtgacagca aaatgaacga ggtcagatta ggggcctcag 1080

gtggcctggg atatgaatca gggagaggtg tagccgcgag cggggaaaat actcttctag 1140

gtcttttgta cactataagc ttttttcttc gggctagcct ggctaaatgg catccaatcc 1200

tccacccact tgttgctatt agttacctct ctgtggttta gggcaggagg gaattgctca 1260

aacaatggct gagggactaa cttgtttagc agttagctaa agcctgttta tgatccatcc 1320

tggtttcaat tactgtgcag tgactgacaa gcctcggggg attgctctcc agctcttctc 1380

tgccttgtac atagcacacc caggtcctgg gaaatgaata caaaaacagg tctccgcctc 1440

attcttgcta ttctttcttt gtgtgtgtgt gtgtggaaat aaatggatat ttcacacgtc 1500

a 1501

<210> 172

<211> 948

<212>  DNA

<213>  Rattus norvegicus (Norway rat)

<400>  172
aacagctagc ggagcggcag gaggagcact aaccaggaaa atggcagacg gcttctcact     60

taatgatgcc ttagctggct ctggaaaccc aaaccctcga ggatggcctg gtgcatgggg    120

gaaccagcct ggggcaggag gctacccagg ggcctcctat cctggggcct acccaggaca    180

ggctcctcca gggggttatc ctggacaggc tcctcctagt gcctatccgg gcccaactgg    240

ccctagtgct tatcctggcc caactgcccc tggagcttat cctggcccaa ctgcccccgg    300

agccttccca gggcaacctg ggggacctgg agcctacccc agtgctcctg gggcctaccc    360

cagtgctcct ggggcctatc ctgctactgg cccctttggt gccccgactg gaccactgac    420

agtgccctac gatatgccct tgcctggagg agtcatgcct cgcatgctga tcacaatcat    480

aggcacagtg aagcccaacg caaacagtat cactctgaat ttcaagaaag ggaacgacat    540

cgccttccac tttaaccccc gcttcaatga gaacaacaga agagtcatcg tgtgcaacac    600

gaagcaggac aataactggg gaagggaaga aagacagtca gctttcccct ttgagagcgg    660

caaaccattc aaaatacagg tcctggttga agccgaccac ttcaaggttg cggtcaatga    720

tgttcatctg ttgcagtata accatcggat gaagaacctc agggaaatca gccaactggg    780

gatcattggt gacataaccc tcaccagcgc ttcccacgcc atgatctaag ccagaagggg    840

tgggccggca ccagaactgc cctgtgtgtt atgagcggga aactttgcat ttctctctcc    900

ttatacttct tgtaagacat ccatttaata aagtctcgtg ctgagaga                 948

<210>  173

<211>  1671

<212>  DNA

<213>  Rattus norvegicus (Norway rat)

<400>  173
cctttctttg cgagaagtgc aaaattggct agagccagag tctcgcccac ctggtacaga     60

aggagcgcgc gcctgtgtcc cttggggacc cacagttgca aagagacagc tgcttgattt    120

ggtcacccac tcgcccagac tataggagcc tcccgggaca ctcttgagtt gcacctttct    180

gcagagcaga ctgtttgagc ccccgcagtg ctcgtaggat ttgaagcgtt ctgatactgg    240

aggcttgctc gccgcgctcg cccgactctg ggacacattt gtccgagcca caggagtgtg    300

cgggtgactg acatatcatc tcttcgactc tccatgagct catgaacctg ggagcaggtg    360

cccggaaatg gcgcggcctc tgagcgacag gaccccgggc cccctgctgc tgggtggccc    420

ggctggggcc ccccctggcg ggggagcgct gcttgggctg aggagccttc tgcagggaaa    480

cagcaagccc aaagaaccgg ccagctgtct cctgaaggaa aaggagcgca aggcaactct    540

gccctcagcc cccgtcccgg gacccgtcct ggaaacggcg ggcccagcgg atgccccaac 600

tggggcggtt agtggcggtg ggtcccctcg ggggcgctca gggcctgtgg ctggcccgag 660

tctttttgcg ccgctgctgt gggaacgcac tttgcctttc ggggacgtgg aatacgtgga 720

cctggacgcc ttcttgctgg agcacgggct accgccgagc ccgccgcccc ccgggggcct 780

gtcgccggca ccctctccag cgcgcactcc cgcgccctcc cccgggcccg gctcttgcag 840

ctcctcttcc ccccgctcct cgcccgggca cgcccccgcg cgggccactc tgggagccgc 900

cggcggccac cgcgcaggct tgacctctag ggacacaccc agtcctgtgg acccagacac 960

cgtggaggtg ctaatgacct ttgaacctga tccggctgat cttgccctgt caagcattcc 1020

aggccatgag acttttgacc ctcggaggca ccgcttctca gaggaggaat tgaagcctca 1080

gccaatcatg aagaaggcaa ggaaagtcca ggtgcccgag gaacagaagg atgagaagta 1140

ctggagccgg aggtacaaga acaatgaagc agccaagagg tcgagagacg caagaagact 1200

caaggagaac cagatatctg tgcgggcagc cttcctggag aaggaaaacg ccctattgcg 1260

gcaggaggtg gtggctgtgc ggcaggagct gtcccactac cgtgctgtgc tttcacgcta 1320

ccaggcccaa cacgggacac tgtgaggcca cttccaccct gccagggcag agtcctgttc 1380

cttgttcaga cttacacctg acacccccct tcctccttgt cccatggcca atggtctggc 1440

cagctaggtg cccaggaacg tcatgatgca gacaaataca tttatatttt taagaaaaag 1500

ctagccttcc cccacctccc ttgtgggggt ggggagggtc ctgtgtgcac ttttagcatg 1560

tcgaggaacc catccatcca accgcctcca tcaacacaat cctgaataaa tcttgagaac 1620

ccaaaaaaaa aaaaaaaaaa aaaaaaaaaa aaaaaaaaaa aaaaagaatt c 1671

<210> 174

<211> 2415

<212> DNA

<213> Rattus norvegicus (Norway rat)

<400> 174
   cacggcgctc cttagggcca ccgctcggcg cgccgggaca gaccctcccc actcctgcct 60
   gcaggatcgg aacgtcaatt aacggctgac actgcccccc acctcttccc acccatctgg 120
   gctccagcga ggaacggatc tcggggtaca ccatgatctt gccattttta gtactggcca 180
   tcggaccctg ccttaccaac tcatttgtgc cagagaaaga gaaagacccc agttactggc 240
   gacagcaagc ccaagagacc ttgaaaaatg ccctgaaact ccaaaaactc aacaccaacg 300
   tggccaagaa catcatcatg ttcctgggag atggtatggg cgtctccaca gtgacagctg 360
   cccgcatcct taagggccag ctacaccaca acacgggcga ggagacacgg ctggagatgg 420
   acaagttccc ctttgtggct ctctccaaga cgtacaacac caacgctcag gtccccgaca 480
   gcgcgggcac tgccactgcc tacttgtgtg gcgtgaaggc caacgagggc accgtgggag 540
   tgagcgcggc cactgagcgc acgcgatgca acaccactca ggggaacgag gtcacgtcca 600
   tcctgcgctg ggccaaggat gctgggaagt ccgtgggcat cgtgaccacc actcgggtga 660
   accacgccac tcccagtgca gcctatgcgc actcggccga tcgggactgg tactcggaca 720
   atgagatgcg cccagaggct ctgagccagg gctgcaagga catcgcctat cagctaatgc 780
   acaacatcaa ggacatcgat gtgatcatgg gtggtggccg gaagtacatg taccccaaga 840
   acagaactga tgtggaatat gaactggatg agaaggccag gggcaccaga ctggatggcc 900
   tggacctcat cagcatttgg aagagcttca aacctagaca caagcactcc cactatgtct 960
   ggaaccgcac tgaactgctg gcccttgacc cctccagggt ggactacctc ttaggtctct 1020
   ttgagcccgg ggacatgcag tatgagttga atcggaacaa cctgactgac ccttccctct 1080
   cggagatggt ggaggtggcc ctccggatcc tgacaaagaa tcccaaaggc ttcttcttgc 1140
   tagtggaagg aggcaggatt gaccacgggc accatgaagg caaggccaag caggcgctgc 1200
   atgaggccgt ggagatggat gaggccatcg gaaaggcggg caccatgact tcccagaaag 1260
   acacgttgac tgtggttact gctgatcact cccacgtttt cacgtttggt ggctacaccc 1320
   ccaggggcaa ctccattttt ggtctggctc ccatggtgag cgacacggac aagaagccct 1380
   tcacagccat cctgtatggc aacgggcctg gttacaaggt ggtggacggt gaacgggaga 1440
   acgtctccat ggtggattat gctcacaaca actaccaggc ccagtccgct gtccccctgc 1500
   ggcacgagac ccacggtggg gaagatgtgg cggtctttgc caagggccct atggctcacc 1560
   tgcttcacgg cgtccatgag cagaactaca tcccccacgt catggcgtat gcctcctgca 1620
   ttggagccaa ccttgaccac tgtgcctggg ccagctctgc gagcagcccc tccccagggg 1680
   ccctgctgct tccactggct ctgttccccc tacgcaccct gttctgaggg cccaggtccc 1740
   acaagagccc acaatggaca gccggctccc ctccctttgt ggcctgccac ctggccgccc 1800
   acactcaacg ggaggcccca ggcaacctcg agcaggaaca gaagtttgct acctgcctca 1860
   cttccgcccg gaaccctccg tgggtcggat tcctggctct gccgttgttt ctctattcac 1920
   tgccttttgg ccagcagggt gggtttctct cttgggccgg caggacacag actgcgcaga 1980
   ttcccaaagc accttatttt tctaccaaat atactctcca gaccctgcaa ccatcatgga 2040
   acattccaga tctgaccttc tctcccctac cccttctctc tggaacactg ggtcccatag 2100
   tcacagccag tccctcaacc caaccctcct tggagggaag accaggtctg ctcagggtga 2160
   gactcccagg aagccacctc cggggttggc tgtctaccca gggtggccag gctgggaaga 2220
   acaacccagc cggacaggac gcacacactc cccacccagc tccagagact cgccaaccct 2280
   tcactgaagc gactcccctg tttggaatag caaaaaaaaa aagaaagaaa aaaaagaaaa 2340
   aaattttaat ttctcttttt ggtgttggtt aaaagggaac acaagacatt taaataaaat 2400
   gttccaaata aaaaa 2415

<210> 175

<211>  1232

<212>  DNA

<213>  Rattus norvegicus (Norway rat)

<400>  175
gttttccgtc cggcttctct cacactcaag tgcgcgcctc cacctcatgg aagactcgat      60

ggacatggac atgagccctc ttaggcctca gaactacctt ttcggttgtg aactaaaggc     120

tgacaaagat tatcacttta aagtggataa tgatgaaaat gagcaccagt tatcattaag     180

aacggtcagt ttaggagcag gggcaaaaga tgagttgcac atcgtagagg cagaagcaat     240

gaactatgaa ggcagcccaa ttaaagtaac actggcaact ttgaaaatgt ctgtacaacc     300

aacagtttcc cttgggggct tcgaaattac accacctgtg gtcttgaggt tgaagtgtgg     360

ttctgggcct gtgcacataa gtggacagca cctagtagct gtagaggaag atgcagagtc     420

agaagatgaa gatgaggaag atgtaaaact cttaggcatg tctggaaaga gatctgctcc     480

cggaggtggt aacaaagtcc cacagaaaaa agtaaaactt gatgaagatg atgatgagga     540

tgatgaagat gatgaggatg atgaagatga tgatgatgat gattttgatg aagaggaaac     600

tgaagaaaag gttccagtga agaaatctgt acgagatacc ccagccaaaa atgcacaaaa     660

atcaaaccaa aatgggaaag atttaaaacc atcaacacca aggtcaaagg gtcaagagtc     720

cttcaaaaaa caggaaaaaa ctcccaaaac acccaaagga cctagctctg tagaagacat     780

taaggcaaaa atgcaagcaa gtatagaaaa aggtggttct cttcccaaag tggaagccaa     840

gttcattaat tatgtgaaga attgtttccg gatgactgac caggaggcta ttcaagatct     900

ctggcagtgg aggaagtctc tttaagaaaa tggtttaaac agtttgaaat aattctgtct     960

tcatttctgt aatagttgct atctggctgt cctttttata atgcaaagtg agaactttcc    1020

ctactgtgtt tgataaatgt tgtccaggtt caattgccaa gaatgtgttg tctaaaatgc    1080

ctgtttagtt ttcaaggatg gaactccgcc ctttacttgg ttttaagtat gtatggaatg    1140

ttatgatagg acatagtagt agtggtggtc agatgtggaa atggtaggga gacaaatata    1200

catgtgaaat aaactcagta ttttaataaa gt                                  1232

<210>  176

<211>  2259

<212>  DNA

<213>  Rattus norvegicus (Norway rat)

<400>  176
ctggctacta tgctggacac aggactgctc ctggtggtca tactggcctc cctaagtgtc      60

atgttcttgg tgtccctctg gcagcagaaa atcagggaga gattgcctcc aggacccact     120

cctttgcctt tcattggaaa ttatctgcag ctgaatatga aagacgtata cagttccatc     180

acacagctca gtgagcgcta tggtcctgtg ttcaccattc accttgggcc tcgacggatt 240
gttgtgcttt atggatacga tgcagtcaaa gaggctttgg tggaccaagc tgaggagttc 300
agtggacgtg gcgaactgcc tacctttaat atactcttca aaggctatgg tttttcattg 360
agcaatgtgg aacaggccaa gcgtatcagg cgcttcacca tagccacatt gagagatttt 420
ggtgtgggca agcgtgatgt acaggagtgt atcctggagg aggcaggcta tttgatcaag 480
acgttgcagg gcacttgtgg agcccccatt gacccttcca tctacctgag caaaacagtc 540
tccaatgtca ttaactccat tgtcttcggg aaccgcttcg actatgagga caaagagttc 600
ttgtcactgt tggagatgat cgatgaaatg aatatatttg cagcctcagc cacagggcag 660
ctctatgaca tgttccattc agtgatgaag tacctgcctg gaccacagca acagatcatc 720
aaggttactc agaaactgga agacttcatg atagagaaag tgaggcagaa ccatagtacc 780
ctggacccca attccccaag gaacttcatt gactcctttc tcatccgcat gcaagaggag 840
aaatatgtta attcagaatt ccacatgaac aacctagtga tgtcatcatt aggcctcctc 900
tttgctggga ctgggtcagt cagctccacg ctataccatg gtttcctgct actcatgaag 960
catccagatg tggaagccaa ggtccatgag gaaattgagc gagtgatcgg caggaaccga 1020
cagcctcagt atgaggacca catgaagatg ccctacaccc aggctgtgat caatgagatc 1080
caaagatttt ctaacttggc tcccttgggc attcctcgaa ggattatcaa gaacacaacc 1140
ttccgtggct tcttcctccc caagggcacc gatgtattcc ctataatagg ttctctgatg 1200
acagaaccaa agttcttccc taaccacaaa gacttcaacc cccagcactt cctggatgac 1260
aagggacagt tgaagaagaa tgctgcattt ctcccttttt ccattggaaa gcgattctgc 1320
ttgggagata gcctggctaa aatggagctc ttcctgctgc tcaccaccat cttgcagaac 1380
ttccgtttta agttcccaat gaatctagaa gacatcaacg agtaccccag tcccataggg 1440
tttaccagga tcataccaaa ttacaccatg agcttcatgc ccatctgatt ctgagttgaa 1500
tcaaggtggg gcaagaggga gggagagcct gaagtggggc cagggtgcag gtggagagaa 1560
cagagaagat gaagatgagg gttaagaagg gaccacaccc atggaagaaa cacaaaagac 1620
ttctcagttt ggtaaaattg taacagtcct aataaaaaga aagaaacacc cagtaggcag 1680
cagtaacaac aactgagact catggggcaa aggtggctca cctctgcaga agctgtcctg 1740
cccttctctc actcagtcct ctacacaaga gcagcatgtc cccaagccca acgtacaggt 1800
tcaaaagata gaacttaaaa aatttgaacc taaactgagg tggaaaagac acagttagct 1860
aggattgaca cattggactc tatcaccagc attcaggagg gagggaacat ggctccctag 1920
gaggcctgcc agaattacaa agtgaaactc atctcaaaaa aggaacaaca gaaaataaaa 1980
tttcaaattg atttctctta gaccataaga gtccagatct gtatccaaag ctatttggtt 2040
atattttttg ttattgttgt tttgtttaca cattgtgttt ttctttcggt ttgtaagtct 2100
gtttgggata tttaatttac atttactgat tagtgtgggt ggtagggcat accatggctc 2160
aaatgtggaa accaaagaaa agcttttgga agtgtcatct cccttacaat acgtgtgtcc 2220

aagaactcaa attcagacaa taaagcttga tagcaagca 2259

<210> 177

<211> 2153

<212> DNA

<213> Rattus norvegicus (Norway rat)

<400> 177
cggccgggtg cgccttgggg tttagtggcg gcttctccat gggtccagcc agccgcttcc 60

ctgtgctgtg aggagacagc attcagagtt gaccttgtga gagctggcca taatttaatt 120

ccatctctag gttttctgta ggcacatcga gaaccaacca tgggcagctt tactaaggaa 180

gagtttgact gccatatcct cgatgaaggt ttcactgcta aggacattct ggaccaaaaa 240

atcaatgaag tttcttcctc tgatgataag gatgctttct atgttgcgga cctcggagac 300

gttctaaaga agcatctgag gtggctgaaa gctcttcccc gtgttactcc cttctatgct 360

gtcaagtgta atgacagcag agccatagtg agcaccctgg ctgccattgg gacaggattt 420

gattgtgcaa gcaagactga aatacagttg gtgcaggggc ttggggtgcc tccagagagg 480

attatctatg caaatccttg taagcaagtg tctcagatca agtatgctgc cagtaatgga 540

gtccagatga tgacttttga cagtgaaatt gagttgatga aagttgccag agcacatcca 600

aaggcaaagt tggttttgcg gattgccact gatgattcca aagcagtttg tcggctcagt 660

gttaagtttg gtgccacact gaaaaccagc aggcttctct tggaacgggc aaaagagcta 720

aatattgatg tcattggtgt cagcttccat gtgggcagtg ggtgtactga ccctgagacc 780

ttcgtgcagg cagtgtcaga tgcccgctgt gtctttgaca tgggaacaga agttggtttc 840

agcatgtatc tgcttgacat tggtggtggc tttcctgggt ctgaagacac gaagcttaaa 900

tttgaggaga tcaccagtgt aatcaaccca gctctggaca agtacttccc atcggactct 960

ggagtgagaa tcatagctga gccaggcaga tactacgtcg catcagcttt cacacttgca 1020

gtgaatatca ttgccaaaaa aaccgtgtgg aaggagcaga ccggctcgga cgatgaagat 1080

gagtcaaacg agcaaacttt gatgtattac gtgaatgatg gagtgtatgg gtcatttaac 1140

tgcattcttt atgaccatgc acatgtgaag gccctgctgc agaagagacc caagccagat 1200

gagaagtatt actcatccag catctgggga ccaacatgtg atggccttga tcggatcgtc 1260

gagcgctgta gcctgcctga aatgcatgtg ggtgattgga tgctgtttga gaacatgggt 1320

gcatacactg ttgctgctgc ttctactttc aatgggttcc agaggccaaa catctactac 1380

gtaatgtcac ggtcaatgtg gcaactcatg aagcaaatcc agagccatgg cttcccgcca 1440

gaagtggagg agcaggatgt tggcactctg cccatgtctt gtgcccagga gagcgggatg 1500

gaccgtcacc ctgcagcctg tgcttctgct agtatcaatg tatagatgcc attcttgtag 1560

ctcttacctg caagtttagc ttgagttcac ggcatttggg gggaccattt aacttaatta 1620

ctgctagttt ggaatgtctt tgtaagagta gggttggcac caatgcagta tggaaagact 1680

aggagatggg ggtcacactt actgtgttcc tatggaaact ttgaatattt tatatggatt 1740

tttattcact tttcagacct gatactaatg agtgcccctc ggctgctgag caagcatttg 1800

tagcttgtac attggcagaa tgggctaaaa gcttatgttg tgacccattt tgaaaataaa 1860

gtatcttgaa atgattggac attggagaat gtgtgcaagt atcccttaca gaaggcacaa 1920

acttctgcac aggctgtgtg ttacagcagt gagtctagcc cagcagagat gtggatgata 1980

caaagctgtg ccccctctgt acagcatcaa tgtgcttagc ccatctcaag tgtttactgt 2040

gaacttggtg cccaaagtct cttaagagtg tcatctgcct agtggcctct tgacttggcc 2100

acttcctaag gagagggcat ctgaggctct ttgaaccttg cctgcagaaa ccc 2153

<210> 178

<211> 1164

<212> DNA

<213> Rattus norvegicus (Norway rat)

<400> 178
   gtgtctgttc tgcggaacag taggcagttg ttttccgtcc ggcttctctc acactcaagt 60
   gcgcgcctcc acctcatgga agactcgatg gacatggaca tgagccctct taggcctcag 120
   aactaccttt tcggttgtga actaaaggct gacaaagatt atcactttaa agtggataat 180
   gatgaaaatg agcaccagtt atcattaaga acggtcagtt taggagcagg ggcaaaagat 240
   gagttgcaca tcgtagaggc agaagcaatg aactatgaag gcagcccaat taaagtaaca 300
   ctggcaactt tgaaaatgtc tgtacaacca acagtttccc ttgggggctt cgaaattaca 360
   ccacctgtgg tcttgaggtt gaagtgtggt tctgggcctg tgcacataag tggacagcac 420
   ctagtagctg tagaggaaga tgcagagtca gaagatgaag atgaggaaga tgtaaaactc 480
   ttaggcatgt ctggaaagag atctgctccc ggaggtggta acaaagtccc acagaaaaaa 540
   gtaaaacttg atgaagatga tgatgaggat gatgaagatg atgaggatga tgaagatgat 600
   gatgatgatg attttgatga agaggaaact gaagaaaagg ttccagtgaa gaaatctgta 660
   cgagataccc cagccaaaaa tgcacaaaaa tcaaaccaaa atgggaaaga tttaaaacca 720
   tcaacaccaa ggtcaaaggg tcaagagtcc ttcaaaaaac aggaaaaaac tcccaaaaca 780
   cccaaaggac ctagctctgt agaagacatt aaggcaaaaa tgcaagcaag tatagaaaaa 840
   gcgcattgaa cattcctggg cactactggt aaattaagcc caaagatggg gaaagaggaa 900
   aaggagaaac aaatatagta ccatcaacaa tccagactga agtcttctat tttaatctca 960
   atcccctttc ctgattggcc atccattccc ccttgcaggc tggaagcaat cgaaaaccta 1020
   aagcattttt ctttttcact cgggtgatgc agaaaacttg actgcttttc tataccactt 1080
   gtgcatatgc cttaactctg accatgtttt aattttaacc tttgtatcct tagctgctcg 1140
   aaataaattt ttgaatgaac caat                                          1164

<210>  179

<211>  2470

<212>  DNA

<213>  Rattus norvegicus (Norway rat)

<400>  179
acctcagcta tggagttgga tgagctgtgc ctgctcgaca tgctggtcta cttggaaggt      60

ttcatggcct tcgtgtccat tgtggggctc cggtcggttg gctctccgta cggccgctac     120

tcgccgcagt ggcccggcat ccgagtgccc gcgcgacctg cctggttcat acaggagctg     180

ccctcgatgg cctggccgct gtacgagtac attcgtcctg cagccgcgcg actgggcaac     240

ctgcctaacc gcgtcctgct ggctatgttt ctgatccact acgtgcaaag gacgctggtt     300

ttcccggttc tgatcagggg agggaagccc accctcctgg tcacctttgt cttggccttc     360

ctgttctgca ccttcaacgg ctatgtacag agcagatact tgagccagtt tgcggtttat     420

gctgaagact gggtgaccca tccctgtttc ctgacaggct ttgccctgtg gttagtgggc     480

atggtgataa atatccactc agaccacatc ctgaggaatc tgagaaaacc aggggaaact     540

ggatacaaga tacccagggg aggcctgttt gaatacgtat ctgcagccaa ctattttggg     600

gagctcgtgg agtggtgtgg ctttgcactg gccagctggt ccctccaggg tgtagtgttt     660

gcactgttca cactcagcac actgctcacc agagcgaagc agcaccatca gtggtaccat     720

gagaagtttg aagattaccc caagtcaaga aaaatactga ttccatttgt gctttagtgc     780

tctgttagcg ctgttgcctc ccatgagctg agtctgtctg tctccctggt gactttgctc     840

tgagcactta cgaatgaatt gttttcctta attctcctgc agcccctttc tcaggaaagg     900

ctgggggtgg gggggtgtcg tcccctggta aaggacaaag ccaatgataa actaatccac     960

cacatgcagt taggggctac actgcctgct ggatccgaag caggtagccc tgagtcatta    1020

tggcgctctc tgacttcagc aatcagcagc ccttacaatc ctgcaagatt ccacccaagt    1080

cagcagcagt cacgggcctc cttcactgat gtgtgttctg cctgctcagc ccctgccaca    1140

gaggcctgga ggtgtgggag tgtggcctaa gcacagtctg ccatccttga ccgcagacct    1200

cttggaccca cccccactcc ctccagacac tggtaagaga agccttcctg caacatgtcc    1260

tgtcctcagg aggtgagaca gcagagtgct tccattcact cgatgacccc atttttgctc    1320

ttcctttggg ctagaattca ttaaggtcct taaaaacaaa acaaaacttt ttcttaatag    1380

tacaaaacaa aatatcaaaa caaaatttgt tattttgaat gcacccaagg accaatcatg    1440

tcacacaaag aaagctcagg gctagcctgg gctgtgtagg gagaccctgt ttgggaaaaa    1500

aaaaatgaag ataacaacca gctaactgtc caaagaagtg accgcagtaa taaaagacgc    1560

cgcccacaca ggcaccagct tgggagaaag atgtgcgcct gggattgtaa acccactgtt    1620

gctctgggca ggctgaggcc cactggtgaa gagccattcc caccggaccc atgcacactg 1680

cctcggggca gcgtctgcac tcacctctca cccgctgcca ccacagctaa agaacagaac 1740

ggatagaact gtgtgctctg aacccagtga agatgctgca tgaagacctg caggcacacc 1800

tgatggcgtg cacggtgccc accactcgcc tgactcggac catctctgtg tgcccgctgc 1860

cacctctgtg tgccctttcc agctggcttt cccatcaggg cttcctcagc tcttctgctc 1920

tcagacagaa actattctct gttcctctgg ttcgcagaat gtctagattt gacccagaaa 1980

acttcatgac acagctactt cattttaaca aagagcagtg tttaatgggg aactaccctt 2040

caatccttta ccctcccctt ttctggccaa gtaactgctt gaaaacctaa agcactaaac 2100

attgtaggtc tcctctcaaa acctcaggcc tgtctggtgt gttctgaaac gtttgtgtgg 2160

aaggagatat tcagctgaga ccctgggaat gtttgctgtg aacttgacct cccttggagg 2220

gcatggtgct agataaactt ggaacctagg actccaggtt gctaggcgga tgccctgaca 2280

ctaagccaca tcacacatta gctctgtgat gcctctttct ttatgaagga ccaagctgcc 2340

cacatactaa gtgagattaa tttaagagga atcctgtcct aacactgtat acttcattcc 2400

ctacaactca acttacttgt atgaaccatg attgttaagg aaattaataa actacattta 2460

taagtaaaaa 2470

<210> 180

<211> 781

<212> DNA

<213> Rattus norvegicus (Norway rat)

<400> 180
tgtggatctt tccagaacag cagttgcaat cactatgtct caatcctggg tacccgccgt 60

gggcctcact ctggtgccca gcctgggggg cttcatggga gcctactttg tgcgtggtga 120

gggcctccgc tggtatgcta gcttgcagaa accctcctgg catccgcctc gctggacact 180

cgctcccatc tggggcacac tgtattcggc catggggtat ggctcctaca taatctggaa 240

agagctggga ggtttcacag aggaggctat ggttcccttg ggtctctaca ctggtcagct 300

ggctctgaac tgggcatggc cccccatctt ctttggtgcc cggcagatgg gctgggcttt 360

ggtggacctc atgcttgtca gtggggtggc aaccgccact accctggctt ggcaccgagt 420

gagcccaccg gctgcccgct tgctgtatcc ttacctggcc tggctggcct ttgccaccat 480

gctcaactac tatgtatggc gtgataactc tggtcggcga gggggctccc ggctcacaga 540

gtgaggacac ctagccatca ggaatgcagc cctgccagcc aggcatcatg ggttgaggtc 600

atcctgcttt catgaccatt gggcctgctg gtctacctgg tcttagtcca ggaagccacc 660

aggtaggtca aggtggtcag tgctaagtcc catgcgggga cagttgtacc tgcttttctg 720

cactgctgca ggcgtgccct aggagcatgg ggcctttaaa gctaaataaa gtctttaact 780

t                                                                    781

<210>  181

<211>  4057

<212>  DNA

<213>  Rattus norvegicus (Norway rat)

<400>  181
   aaaagagttg ggagaagttg ggagcggcgg gggcgcgccc cggggtgggc accgggaaat      60
   cgtcgggagc gcgcgagggt tccggaggat caggggagac ccggagaccc ccgggacacg     120
   aaatctagag ctagcgggtt atgcctccac cttgttgccc tgaaagccga agcgacagtg     180
   agaagggcta agatttggcc atgagcagcg cccccaggcg ccccgcctcg ggcgcagatt     240
   ctttgcacac gccagagcca gagagcctga gccccggaac acctgggttc cctgagcaag     300
   aggaggaaga tgaacttcgt accttaggtg tggagcggtt cgaggagatc ctccaggaag     360
   ctggatccag aggaggagaa gagccaggcc gcagctatgg ggaggaagac tttgaatacc     420
   accgccagtc ctcccaccat atccatcatc cactctccac ccacctgcct cctgacgccc     480
   gccgtcgcaa gactccccag ggcccaggac ggaaacctcg gaggcgccct ggagcctctc     540
   ccactggaga gacccccact attgaggaag gggaggaaga tgaggatgaa gtcggtgaag     600
   ctgaagggtt cagagctccc ccgcaacaac catctcctgc ctcttcaccc tctgcagttc     660
   agtttttctc ccaagaggat gaaggtacag ataggaaggc agagagaacc agcccatctc     720
   cccctacaca gacgccccac caggaagcag ctccccgggc cagcaaaggg gcacagacag     780
   gaactctggt ggaggagatg gtggccgtgg ccagcggcac ggctggaggt gacgacggag     840
   gtgctgcagg gcgccccctg accaaagccc agcctggaca tcgaagttac aaccttcagg     900
   agagaaggcg aattggcagt atgacagggg tggagcaggc cctgctgccc agggtcccta     960
   ctgatgagag tgaggctcag acgctggcca cagctgacct cgacctcatg aaaagtcacc    1020
   ggtttgagga cgttccgggg gtacggcgac atttggtgag gaagaatgcc aaagggtcta    1080
   cacaggctgc ccgggaaggc cgagagcctg gccccacacc tcgggcacga ccacgggccc    1140
   cccataagcc tcatgaggtg ttcgtagagc tgaatgaatt gcagttggac aaaaaccagg    1200
   agcctcagtg gcgggagaca gcccggtgga taaaatttga ggaggacgtg gaagaggaga    1260
   ctgagcgctg gggcaagcct cacgtggcat cactgtcctt ccgcagcctc ctggagctcc    1320
   gcaggacact ggcccatgga gctgtgctct tggacctcga tcagcagacc ctgcctgggg    1380
   tggcccatca ggtggtcgag cagatggtta tctctgacca gatcaaagca gaggacagag    1440
   ccaatgtgct acgagccctt ctgctgaaac acagccaccc aagtgatgag aaagaattct    1500
   ccttcccccg gaacatctca gcgggctctc tgggctctct cctggggcat caccacgccc    1560
   aggggactga gagtgatcct cacgtcactg agcctctcat cggtggtgtt cctgagaccc    1620
   ggctggaggt ggatagagag cgtgagctgc cgcccccagc cccacctgca ggtattaccc 1680
   gctccaagtc caagcatgag ctgaagctgc tggagaagat ccctgagaat gcagaggcca 1740
   cagtggtcct cgtgggctgt gtggagttcc tctcccgccc caccatggcc tttgtgcgcc 1800
   tgcgggaggc tgtggaactg gatgcagtac tggaggtgcc tgtgcctgtg cgcttcctct 1860
   tcctgctgct ggggcccagc agcgccaaca tggactacca tgagattggc cgatccatct 1920
   ccaccctcat gtctgacaag caattccacg aggcagccta cctggcagat gaacgggatg 1980
   acttgctgac tgctatcaat gccttcctgg actgcagtgt tgtgctaccg ccttctgaag 2040
   tgcagggcga ggagctgctg cgttctgttg cccatttcca gcgccagatg ctaaagaagc 2100
   gagaggagca gggccgcctg ctgccccctg gtgctgggct agagcccaag tctgcccaag 2160
   ataaggcact cctgcagatg gtagaggtgg caggtgcagc tgaagatgat ccccttcgga 2220
   ggacaggccg gccctttggg gggctgatcc gtgacgtgcg gcggcgctac ccgcactacc 2280
   tgagtgactt ccgagatgca ctagaccccc agtgcctggc tgctgtcatt ttcatctact 2340
   ttgccgccct gtcgcctgcc atcacttttg gggggctact gggggagaag acacaggacc 2400
   tgataggagt gtcagagctg atcatgtcca cagcgctcca gggagtgatc ttctgcctgc 2460
   tgggcgccca gccactgctg gtgatcggct tctcggggcc tctgctggtc ttcgaggagg 2520
   ccttcttctc gttctgcaag agcaaccagt tggagtactt ggtgggccga gtttggattg 2580
   gcttctggct ggtgctcctg gccctgctca tggtggctct ggaggggagc ttcctggtcc 2640
   gctttgtctc ccgattcacc caggagatct ttgccttcct catatcactc atcttcatct 2700
   atgagacctt ctataagctg atcaagatct ttcaggagca ccccctccat ggctgctcag 2760
   tctccaacga ctcagaggca gacagcagca gtaacaatat gacttgggca gcaaccacac 2820
   tggcaccaga caacagcagt gcgtctgggc aggagaggcc ccggggccag cctaacactg 2880
   ccttgctatc actggtgctg atggccggca ctttcttcat cgccttcttc ctgcgcaaat 2940
   tcaagaacag ccggttcttc cctggccgga tccggcgggt aattggggac tttggggtgc 3000
   ccatcgcgat cctcatcatg gtgcttgtgg attacagtat tgaggatacc tacacccaga 3060
   agctaagtgt gcccagtggg ttctcagtga cagccccaga caagcggggc tgggtcatca 3120
   acccccttgg agagaagact cctttccctg tgtggatgat ggtggccagc ctgttgcctg 3180
   ccgttctggt gttcatcctc atcttcatgg agacacagat caccacgttg atcatctcca 3240
   agaaagagcg gatgcttcag aagggctctg gcttccacct tgatctgttg ctcattgtag 3300
   ccatgggtgg catctgtgcc ctctttggcc tgccttggtt ggctgctgcc actgtccgct 3360
   ctgtcactca tgccaatgca ctcactgtca tgagcaaggc tgtggcacct ggtgacaaac 3420
   ccaagattca ggaagtcaag gagcagcggg tgaccgggct gctggtggcc ctgcttgtgg 3480
   gactctccat ggtcattggg gacctactgc ggcagatccc cctggctgtg ctctttggca 3540
   ttttcttata catgggagtt acttccctta atgggatcca attctacgag cggttgcacc 3600
   tgctactcat gccgcccaaa caccacccag atgttaccta tgtcaagaag gttcggacca 3660
   tgcggatgca cctgttcact gccttgcagc tgctctgtct ggccctgctt tgggcggtca 3720
   tgtccacagc tgcttccctg gccttcccct tcatcctcat cctcacagtg cctttgcgca 3780
   tggtggtact tacccggatc ttcactgagc gagaaatgaa atgtctggat gctaatgagg 3840
   cagagccagt gtttgatgag tgtgaaggtg tggacgagta caacgagatg cccatgcctg 3900
   tgtagctgct gtccagaggc atagcagagg gactaagggg caggggctgg ggggctgggg 3960
   agctggggct cccctcctcc tgtccctcct cattttattt taagtgaata atttaaagcc 4020
   cccttgtccc caccctgcag taaagtgctt caacccc 4057

<210> 182

<211> 4269

<212> DNA

<213> Rattus norvegicus (Norway rat)

<400> 182
taagctggtg cttacggaca gagagccaca ctcgggcttt ctcgaagagg taaaccaggt 60

ccctctgcag ccatgtcagc caaggcaatt tcagagcaga ccggcaaaga actcctttac 120

aagtacatct gtaccacctc agccatccag aaccggttca agtatgcccg ggttactccc 180

gacacagact gggcccatct cctgcaggac cacccctggc tgcttagcca gagcttggta 240

gtcaagccgg accagctgat caaacgtcga ggaaagcttg gtctagtcgg ggtcaacctc 300

tctctggatg gagtcaaatc ctggctgaaa cctcgactgg gacatgaggc caccgtcggc 360

aaggccaaag gcttcctcaa gaactttctg attgagccct tcgtccccca cagtcaggcg 420

gaggagttct acgtgtgcat ctatgctacc cgggaaggag actacgtcct gttccaccat 480

gaagggggtg tggatgtggg cgatgtggac accaaagccc agaagctgct tgtgggtgtg 540

gacgagaaac tgaacgctga agacattaag agacacctgt tggtccacgc ccccgaagac 600

aagaaagaaa tcctggccag cttcatctcc ggcctattca atttctacga agatctttac 660

ttcacctacc ttgagatcaa cccccttgtg gtgaccaaag atggtgtcta catccttgac 720

ctggcggcca aggtggacgc cactgctgac tacatctgca aagtcaagtg gggtgatata 780

gagttccctc ccccctttgg gcgtgaggca tacccagagg aagcctacat tgcagacctg 840

gatgccaaaa gtggggcgag cttgaagctg accttgctga accccaaggg gcggatctgg 900

accatggttg ccgggggtgg cgcctctgtc gtgtacagtg ataccatctg tgatcttgga 960

ggtgtcaacg aactggcgaa ttacggggag tactctggtg cccccagtga acaacagacc 1020

tatgactacg ccaagaccat cctctcactt atgactcgag agaagcaccc ggatggcaag 1080

atcctcatca ttggaggcag cattgcaaac ttcaccaacg tggccgccac cttcaagggc 1140

attgtgagag caattcgaga ttaccagggt tccctgaagg agcacgaggt caccatcttt 1200

gttcgaagag gtggcccgaa ctatcaagag ggattacgag tgatgggaga agttgggaag 1260

accactggaa tccccatcca tgtctttggc acagaaactc acatgacggc cattgtgggc 1320
atggcctggg caccggccat tcccaaccag ccacccacag cggctcacac tgccaacttc 1380
ctccttaatg ccagtgggag cacatcgaca ccagcaccca gcaggacagc gtctttttcc 1440
gagtccagag ctgacgaggt ggcccctgca aagaaagcca agccagccat gccccaagat 1500
tcagtcccaa gtccaagatc cctgcaagga aagagtgcca ccctcttcag ccgacatacc 1560
aaggctatcg tatggggcat gcagacccgg gctgtgcaag gcatgctgga ctttgactac 1620
gtgtgctccc gagatgagcc ttcagtggct gctatggtct acccgttcac gggggatcat 1680
aagcagaagt tttactgggg acacaaggaa atcctgatcc ctgtcttcaa gaacatggct 1740
gacgccatga aaaagcatcc ggaggtagac gtgctgatca actttgcatc tctgcgatcg 1800
gcttatgaca gcaccatgga gaccatgaac tatgcacaga tccggaccat agccatcata 1860
gcagaaggca tccctgaggc tctcacacgg aagctcatca agaaggcaga ccagaagggc 1920
gtgaccatca ttgggccagc cacggttggg ggcatcaagc ctggatgctt taagattggg 1980
aatactggtg ggatgctgga caacatcctg gcctccaaac tgtatcgccc aggcagtgtg 2040
gcctacgtct cgcgttcagg aggcatgtct aacgaactca ataatatcat ctctcggacc 2100
acagatggtg tctacgaggg tgttgccatc ggcggggaca ggtaccctgg gtccacattc 2160
atggatcacg tgctgcgtta ccaagacact ccaggagtca agatgattgt agttcttggg 2220
gagatagggg gtacagaaga atataagatc tgccggggca tcaaggaggg ccgcctcacc 2280
aagccagtgg tctgctggtg catcgggacc tgtgccacca tgttctcttc tgaggtccag 2340
tttggccacg ctggggcttg tgccaaccag gcttctgaaa cggcagtagc caagaaccag 2400
gccttgaagg aagcgggagt gtttgtgccc cgaagctttg atgagctcgg agaaatcatt 2460
cagtccgtgt atgaagatct tgtggccaaa ggcgccattg tacctgctca ggaagtgcca 2520
cctccaacag tacccatgga ctactcttgg gccagggagc tgggtttaat ccgaaaacct 2580
gcctcattca tgaccagcat ctgtgacgag cgggggcagg aactcattta tgcgggcatg 2640
cccatcaccg aggtcttcaa ggaagagatg ggcattggtg gtgtcctggg cctcctctgg 2700
ttccagagaa ggttgcccaa gtattcctgc cagttcattg agatgtgtct catggtcacc 2760
gctgatcacg ggccagctgt ctccggggcc cataacacta tcatctgtgc tcgggctggg 2820
aaggacctgg tctccagcct cacctcaggg ctgctcacca ttggggaccg gtttgggggt 2880
gccttggacg cagcagcgaa gatgttcagt aaagcctttg acagcggcat tattcccatg 2940
gagtttgtga acaagatgaa gaaggagggg aaactgatca tgggcatcgg ccatcgagtc 3000
aaatcgataa acaacccaga catgcgagtg cagatcctca aagactttgt caaacagcac 3060
ttccccgcca ccccgctgct cgactatgca ctggaagtgg agaaaatcac cacctcaaag 3120
aagccaaatc ttatcctgaa cgtggatggt ttcatcggcg ttgcgtttgt ggacatgctt 3180
aggaactgtg gctccttcac ccgggaggaa gctgacgagt atgttgacat tggagccctc 3240
aatggcgtct ttgtgctggg aaggagtatg ggcttcatcg ggcactatct tgaccagaag 3300

aggctgaagc aagggctgta tcgtcacccc tgggacgaca tttcctatgt tctcccggaa 3360

cacatgagca tgtaaccgag ccagcagccc taccgtagaa aaaggaagac aaaaactccc 3420

tcctcgacaa tatagcggac agacagctgg aaacagagcc cgttatgggc tgggcctgga 3480

atggaaatag ccattgatgt gcaggcatgg aaagccaaca ccacaggccc attcagtcca 3540

cacagagaag cttagtattt ttttttatat atatatctat atatatataa gcatagaaat 3600

ttaaaaccaa gccaatactt gtgacgtttg cgctgctacc tgctgtatct attacatgga 3660

agactgtaag caagcgctgt cagaataatg ttcttctagg gccttatgat gttgctttct 3720

ttttttaatt agttgaaaat ttatttttcc tctagaacta gtggatccga cttttaagac 3780

ttcaggatac tatctgtttg taggaccact gtctggtatc ccacctccca ctcatcttca 3840

caccacatga agaacactgt attaatctga ttttttagga tctttttttt tttttttgtg 3900

ttatgtgtta agggtttatt tagtatccca ctgaaacgtt ctgtgtttcg gaccaatgtc 3960

tacttatgtc aaggggagga gggttggggc cattgtaccc ttagccatcg tcacacatgt 4020

ggagtagtaa cttaaatgta aagttgtaac atacaagtgt ttaaaatgga aaccgcaaag 4080

caaaaagctg tgaaacgtct cgtgtcttgt gttctctgtg ttcatgcagc tgacttgtct 4140

gttactgaag tgtgggtcca aagactcaca tctgttccgc atctgtaacc cacagagatt 4200

ctggcagctg ccacctcagt ctcttctctg tattatcatg tttggtttaa ataaactaga 4260

tagtaaaaa 4269

<210> 183

<211> 133

<212> DNA

<213> Rattus norvegicus (rat)

<400> 183
cttgtgttct ctgtgttcat gcagctgact tgtctgttac tgaagtgtgg gtccaaagac 60

tcacatctgt tccgcatctg taacccacag agattctggc agctgccacc tcagtctctt 120

ctctgtatta tca 133

<210> 184

<211> 1606

<212> DNA

<213> Rattus norvegicus (Norway rat)

<400> 184
ctgcagtcaa gtgtactggg tgccaagggg acacagcttc cttctaactg gttgagcaag 60

ctgctaaaga aagagttggc cgcccactga ggcagtcatg cagacctgaa gtgacaagca 120

gaagcctggc aggtttgtcc ctgggtagat ggatcgcctg atacggacac tggcgaccaa    180

acgctgggat tggctcaaaa caaggccgtg ttgatccagt gactgggttt tggcgtcctg    240

tcaatctgct gtctgctcaa aagcctagaa atagtgcggg ggcagtttgc tggtctctga    300

gaaaggaagc agccatgcaa gtgctaacga aacgctaccc caagaattgc ctactgaagg    360

tcatggatcg gtactcggct gtggtgcgga acatggagca agtggtgatg atccccagcc    420

ttctgaggga tgtggagctg agtgggtccg gaggctcggt ccaggacgga gcccccgatc    480

tctataccta cttcaccatg ctcaagagca tctgtgtaga agtagaccac gggctgctgc    540

cgcgggagga atggcaggcc aaggtggcag gcaatgaagg cagtgaggct gagaatgagg    600

ctgctgaaac agaggaggct gaggaagaca ggctttcgga ggagctggac ctagaagccc    660

agttccacct gcacttctcc agcctccatc acatccttac ccacctgacc cagaaagcac    720

aggaggtgac gcagaaatac caggaaatga cggggcaggt cctgtaggtc ttcgactcta    780

tggaagatca ctttacacga gagggaagac gatgaggacc cagcggtgca tcagccccga    840

gaccaggaag gaagacctgg acccaggtgg atgcgaaggc ggctcctgtc cctggtgtcc    900

cccacctccc tctgagcgca tctgtggact tgctgcaggc tcacctttaa gatgtctgtt    960

gtgattccca acttctcttc catgtcttag cagtttgagg tttcacaggc agcaagtatc   1020

agtagaaggc agaggtaata gcaagcggcc agagttcaca tcccatccct gggatgtcct   1080

ttagtgcctg gggtcacagt tactctttca tgtataaagc agggacgctg tatttacctc   1140

acaaggttac taaacggaca cggaatcaaa ggcacctgtc acgtgcggat gtctgaacag   1200

ctggtgtttc tcttaccttc ctcagagagc atgctctgtg tagctcttgc ttttgaccct   1260

cacccaggaa aaggttgagg tagttgagtg tgtgggatga gcttctcatc ctgttggatc   1320

tgatgaggct gagcacaagt gaactatgca actattatag tagcaattaa aaaatacatg   1380

gcatgctgat cttttttttt cttcttctta ttgattcttc atgaatttca catcatgacc   1440

cccagtccca ttcatcttct gcctccatct tcttcgtccc tccatatctg ccctccaccc   1500

ttgcaacttc ctccacaaaa gaaaacaaac aaataaacaa aacttcgctg tggaagctac   1560

ggtgtgtcac acagtctacc cttttcccaa acagcttttc ctgcag                  1606

<210>  185

<211>  593

<212>  DNA

<213>  Rattus norvegicus (Norway rat)

<400>  185
ggctgcagcc tcctctcgct aaggaaaggc tgcctgcccg ttctagggag tcagtggtta     60

gcaagttggt cagggccccg gggctactgt gtctctctga cttacagacg atgaccactg    120

gatcatcgat acggactacg acaccttcgc tctgcagtat tcctgccgcc tgcagaatct    180

ggatggcacc tgtgcagaca gctactcctt tgtgttttct cgtgacccca atggcctgac 240

cccggagaca cggaggctgg tgaggcagcg acaggaggag ctgtgcctag agaggcagta 300

cagatggatc gagcacaatg gtggagcaac tcttcctgag ggacttagca gggctgggag 360

gataagaggt gcagcatctg tgcaagtggg ttacccgagg cctcctttgg accaagtctg 420

attaaatttt tccaggactg ccatcagcca cacagacaag gaccaaaggt ttctcttccc 480

ctaaacgcta ggcacttttc tataaccaaa gttgtgaaac cactctcttc ttatgttttt 540

gctccccaca tttgctgcta gtgcacaagc cgagaccatc gcaagataag gtt 593

<210> 186

<211> 3097

<212> DNA

<213> Rattus norvegicus (Norway rat)

<400> 186
ggtgcaggat accttgagaa aatggctgag tatctgaggc tgccccactc cctggcgatg 60

atccgcctct gcaacccgcc agtcaacgcc gtcagtccaa ctgtaatcag ggaagtaaga 120

aatggactcc agaaagctgg atcagaccac acggttaaag ccattgtgat ctgtggcgca 180

aacggaaact tctgtgcagg tgctgatatc catggcttta gtgcttttac ccctggcctt 240

gcattaggaa gcttggtaga tgaaatacag agataccaga agccggtgtt ggccgctatc 300

caaggtgtgg ctctcggagg aggactggag ctggccttgg gctgtcacta tcggattgcc 360

aatgcaaagg ctcgtgtcgg cttgccagaa gtcacactgg ggattcttcc tggtgcaaga 420

ggaacccagc ttctccccag ggtcgttgga gttcctgttg ctcttgacct aattacctca 480

ggaaaatatc tttcagcaga tgaagcactc aggcttggaa ttctggatgc agtcgtgaag 540

tcagacccag ttgaagaagc catcaaattt gctcagaaga ttatagataa acccatagaa 600

ccccgcagga tctttaacaa gccagttcca agcttgccca acatggacag tgtttttgca 660

gaagccatcg ccaaggtacg aaaacagtac cctggtgtcc tggctccgga gacgtgtgtc 720

cgctcaatcc aggcctctgt gaagcatccc tacgaagtag gcatcaagga agaggaaaag 780

ctgtttatgt acctccgggc atccgggcag gctaaagccc tacagtatgc cttctttgct 840

gaaaagtctg caaataagtg gtcaactccc tcaggagcgt cttggaaaac agcctctgct 900

caacccgtct cctcagttgg cgttcttggc ttgggaacga tgggccgagg catcgccatt 960

tcttttgcga gagtggggat ctctgtggtt gctgtggagt cagacccaaa gcagctagat 1020

gctgcaaaga agataatcac tttcaccttg gagaaggaag catccagagc gcatcagaac 1080

ggccaagctt cggcaaaacc aaaactcagg ttctcctcat ccacaaagga actttcaact 1140

gtggatttgg tggttgaagc agtgttcgaa gacatgaacc tgaagaaaaa ggtctttgct 1200

gagctgtcag ccctgtgcaa gccaggagcc tttctgtgca ccaatacctc ggcgctgaac 1260

gtggacgaca ttgcttcttc cacagatcgc cctcagctgg tgattggcac ccacttcttc 1320

tcaccagccc atgtcatgag gttgctagag gtcattccta gccgatactc ttcccctact 1380

accatcgcca cggttatgag cttgtccaaa aagatcggaa agattggagt agtggttggc 1440

aactgctatg gatttgttgg gaatcggatg ttggctccct attacaacca ggggtttttc 1500

ttgttagagg aaggtagcaa gccagaggat gtagacgggg tcttggaaga gtttggtttt 1560

aaaatgggac ccttcagagt gtcagacctc gcagggctag atgtgggttg gaaaattcgc 1620

aaggggcaag gccttactgg accatcattg ccaccaggga cccccgtccg aaagaggggc 1680

aacagcaggt actccccact tggcgatatg ctctgtgaag ctgggcggtt tggtcagaag 1740

acaggtaaag gctggtatca gtatgacaag ccactgggtc gcatccacaa acctgatccc 1800

tggctttcta cgttcctgtc acaatataga gaggttcacc acatcgagca gcgcaccatc 1860

agcaaggagg agatcctgga gcgttgctta tattccctca tcaatgaggc gttccgcatc 1920

ttggaggagg ggatggctgc tcgcccagag cacattgatg tcatctactt gcacgggtac 1980

gggtggccaa ggcacaaggg cgggcccatg ttctatgctg cctcagttgg gttgcccaca 2040

gttctagaga aactgcagaa atattacagg cagaaccctg acatccccca gctggagccc 2100

agtgactacc tcagaaggct ggtagcccag ggaagccctc ctctgaagga atggcaaagc 2160

ttggcagggc cccacggcag caaactgtga ttcagccttc tgcgttttgc cacgtatgtt 2220

ggcacctaat ttcagtatga ttccttccta aaatccaaac agatcgctct gaagtacaaa 2280

tcatgattgc tgctgctgct gctgctgata gctggtcagt aaaagctcct ctccgtatct 2340

ttctacctca taattccaat ggtcagactt caggaatgtc tacagttagt ctatacctct 2400

gactgcactt ctatcggaaa aattcaacga atgtatgttt actactacaa gcattcttct 2460

cttttgactg tttgcccact ctaacaaaaa cgtatacaaa atgttgctaa tagaataatt 2520

cacacctcca gttgacaacc caaatggcct tagcagatca acacaggtgg catatacacg 2580

cacttgacac attccagctg tgtgaagtta ctgccacctg ttgtaacatg gtgctgggtt 2640

agagcactgt tctgagtgag ggaggcaggg cagagaaggc aggcagataa ggccacacat 2700

catatggatg tggatataaa attccaggat atgcaggtct gtggacaaca tagatgagta 2760

gcccaaggct gtaactgttc atacatgcat atatttttgg gtcctttttt ttttcggagc 2820

tggggaccga acccagggcc ttgcgcttcc taggtaagcg ctctaaccac tgagctaaat 2880

ccccagcccc gaggctgtaa ctgttagagg ttaaaaatca ctactgatta gtacagagta 2940

tctttgggag ctgataaaat tttctaaaat tactctagtg gtggatctgt aacacattga 3000

gttcaatggt tcaaagtgaa ttatatttta tatatatata tatatatata tatatataca 3060

tatatatata gtctatctca ataaagttaa taaaatt 3097

<210> 187

<211> 966

<212>  DNA

<213>  Rattus norvegicus (Norway rat)

<400>  187
cagcgtccgg ctctggactg tctatcgctc tctgacgttc agtcccgggt cgctgcttgt      60

gcgcgcacgc cgcctcacac gacaaactta tggcggagcg cggggaactc gatctgacgg     120

gcgccaaaca gaacaccggc gtgtggctgg tgaaggttcc taaatatttg tcccagcaat     180

gggcaaaagc ccctggaaga ggtgaagttg ggaaactgag gattgccaag aatcaaggaa     240

ggactgaggt atcatttact ttgaatgagg atcttgcgaa tattcatgat atcggtggga     300

aaccagcttc agtcagtgct cctagagaac atccatttgt cttgcaaagt gttggaggac     360

agacattaac agtgttcacc gagagctcct cagataaact gtcattggaa ggaatagtgg     420

ttcagagggc ggaatgccga cctgctgcca gtgaaaacta catgaaacta aagagattgc     480

aaatagaaga atcttccaaa cctgtaaggc tctcacaaca ggctgacaaa gttgtaacca     540

ccaattacaa gcctgtcgct aaccatcagt acaatattga atatgaaagg aagaagaaag     600

aagatggaaa gcgagctcgg gcagacaaac aacacgtcct agacatgctg ttctcagcct     660

ttgagaaaca tcagtactat aatcttaagg acttggtgga catcacaaag caacctgtgg     720

gatacctgaa agaaatcttg aaagaaatcg gcattcagaa tgtaaaagga attcacaaga     780

atacgtggga gctgaagcca gagtacaggc attaccaaac agaggagaag agcgactgag     840

agaagcgtgt gcaacagtta caggcagatc tctgccacca cacgctgact gcctgcccac     900

cctgacagga caaagacaac tgagtctgtc tgccattcct cagtaaactg ttaaagctgg     960

gactcc                                                                966

<210>  188

<211>  3617

<212>  DNA

<213>  Rattus norvegicus (Norway rat)

<400>  188
ttctgcccct tgtagctgtt ctgccctccc ctttaaaggt tgacttgccc cgcggcgctc      60

caccgcgctc tagtcctctg acgcctccgg ctcaaccctt tgcaacgcgg atccccgccc     120

gcctgactgc ccgcgcagcg cagttccagc agcaaagcag aagggcgcgc cgagatggag     180

agcaaagccc tgctcctggt ggccctggga gtttggctcc agagtttgac cgccttccgc     240

ggaggggtgg ccgcagcaga cgggggaaga gatttctcag acatcgaaag taaatttgcc     300

ctaaggaccc ctgaagacac agctgaggac acttgtcatc tgattcctgg attagcagac     360

tctgtgtcta actgccactt caaccacagc agcaaaacct ttgtggtgat ccatggatgg     420

acggtgacag gaatgtatga gagttgggtg cccaaacttg tggctgccct atacaaaaga     480

gaacctgact ccaatgtcat tgtagtagac tggttgtatc gggcccagca acattatcca 540
gtgtctgccg gctataccaa gctggtggga aatgatgtgg ccaggttcat caactggctg 600
gaggaagaat ttaactaccc cctagacaat gtccacctct tagggtacag tcttggagcc 660
catgctgctg gcgtggcagg aagtctgacc aacaagaagg tcaatagaat tactggcttg 720
gatccagctg ggcctaactt tgagtatgca gaagccccta gtcgcctttc tcctgatgat 780
gcggatttcg tagatgtctt acacacattt accagggggt cgcctggtcg aagtattggg 840
atccagaaac cagtagggca tgttgatatt tatcccaatg gaggcacttt ccagccagga 900
tgcaacattg gagaagccat tcgtgtaatt gcagagaagg ggcttggaga tgtggaccag 960
ctggtgaagt gctcgcacga gcgctccatc catctcttca ttgactccct gctgaatgaa 1020
gaaaacccca gcaaggcata caggtgcaat tccaaggagg cctttgagaa agggctctgc 1080
ctgagttgca gaaagaatcg ctgtaacaac gtgggctatg agatcaacaa ggtcagagcc 1140
aagagaagca gtaagatgta cctgaagact cgctctcaga tgccctacaa agtattccat 1200
taccaagtca agattcactt ttctggaact gagaatgaca agcaaaacaa ccaggccttc 1260
gagatttctc tgtatggcac agtggctgaa agtgagaaca ttcccttcac cctgccggag 1320
gtcgccacaa ataaaaccta ctccttcttg atttacacgg aggtggacat cggggaattg 1380
ctgatgatga agcttaagtg gaagaacgac tcctacttcc gctggtcaga ctggtggagc 1440
agtcccagct ttgtcatcga gaagatccga gtgaaagccg gagagactca gaaaaaggtc 1500
atcttctgtg ccagggagaa agtttctcat ctgcagaaag gaaaggacgc tgcagtgttt 1560
gtgaaatgcc atgacaagtc tctgaagaag tcgggctgac actggacaaa ccaacaagag 1620
aagaaagcat ctgagttctt tgaagaccga agaaaatgaa gtaaatttta tttaaaaaaa 1680
atacccttgt ttgggtgttt gaaagtggat tttcctgagt attaatccca gctatatctt 1740
gttagttaaa tagaagacag tgtcaaatat taaaaggtgg ctaacacaac gtgaggaacc 1800
taatggccga tagcatgtcc tccagcatca gaagacagca gagaggagaa gcatgccatc 1860
ttatatccct taagaaggaa tcatttgttc ccaaccatac aagactcctt catgtgaccc 1920
atttggtcat ggtctaaaat tagtaagggc ctcttatttt cattagatct ctgaggtttt 1980
aaattgagac cttctcaaag ttctcttgaa gtctaatata gacaacattt ttttgtgctg 2040
tgagtcagat ccatttcttt agcagttgaa acagctggcc attgtaacta gttcttttac 2100
catcaggata tagcacccct accaaataaa ataaataaat aaagtgacca gggacatgtg 2160
actttgcaaa agcaatggac gacgtggctc gtggattcct gacccttagt cccaccacaa 2220
cgaagtacaa gtcagtagag gtacaaaacc tagactgagt aattcttagt agacttcaag 2280
ttttatggct taattcctct gtcttttaaa aacgtgtcac atattataac attattctct 2340
agacagatgt tgaaatgagc ttgtgattca ggtgacatat gaattgagct gagagaaaat 2400
aatgccctgg ctgattttat ttctctgttt tgctttcttg agaaaaggaa tacttgtccc 2460
actccgtatc tgagcctgac caagaactaa actatgtact tcaggcttac cttgaactct 2520

caaccatcct gccttggctt cctgagtgct gggagcttga taaccataat tttattatca 2580

gatttttctt agtcattttc accaatagaa cacattcaat gcccaatcgt tagcatttcg 2640

tttgagactc atcttgaccg tacctctgtc acacgtctaa cacatcacat taatttctag 2700

tttagaagtg atcaagttca aattctgcac tgcgcaaagt acaagtttta gagcaggacc 2760

attttttttt taccacgtaa aagtcgaaat tactaggaaa tgtgtatatc gatgcttgta 2820

cactgttgcg tgcaaagtga ggagccttct attgtgatag ccatagacag taccaggctc 2880

gttgccgctc ttttgtttta ctataaaaaa ataatgaaga attatttatg aacaagatct 2940

catatgttca gattgctttt actattcatc aatataaaat gttaaaaaaa aaaaataaaa 3000

caagttctat ctcagaggct gttgctggga acagaaactg tgaaatgtgt gggtatctga 3060

acacctacac acaagcaaag ccccacaaga gtctttgtca ttcaatgtca ttcagaaagg 3120

aaagagtcaa gagatatacc ataatatgtc agagaagtag ttccagatat gctggaatgc 3180

tagcccttgc taggagaaag ttggttgtgc ctatgtaata taggacaaaa gtgactggtt 3240

tcattaggct cagtgtcatt ctaacaataa aatgatgtac catatgtcac cagcatcccc 3300

attattgcta attcatggca gtatatacgt acatatctca atgatgcttt gactttaaat 3360

tttatttatt agctgtaaat aatgtgtggg tgtgtaagga agcttgtaaa cactggaaac 3420

gctgttgtgg ctatctgggg tgtagatttg tggtgctaac tctgtgtcca cctccatcag 3480

tgattgtctc actgagccaa ctcactctga tgaacagcac aatggaatag cttttgaagg 3540

aagaaaataa actcacctgt gtgaagaaat gggatctgct ttcaataaaa tcgagaacgt 3600

tttatccgga atccgcg 3617

<210> 189

<211> 821

<212> DNA

<213> Rattus norvegicus (Norway rat)

<400> 189
aggaaggagt ccagagacac agagagcaag atgagttccg agacaggccc tgtagctgtt 60

gatcccactc tgaggagaag aattgagccc cacgagtttg aagtcttctt tgacccccgg 120

gaacttcgga aagagacctg tctgctgtat gagatcaact ggggaggaag gcacagcatc 180

tggcgacaca cgagccaaaa caccaacaaa cacgttgaag tcaatttcat agaaaaattt 240

actacagaaa gatacttttg tccaaacacc agatgctcca ttacctggtt cctgtcctgg 300

agtccctgtg gggagtgctc cagggccatt acagaatttt tgagccgata cccccatgta 360

actctgttta tttatatagc acggctttat caccacgcag atcctcgaaa tcggcaagga 420

ctcagggacc ttattagcag cggtgttact atccagatca tgacggagca agagtctggc 480

tactgctgga ggaattttgt caactactcc ccttcgaatg aagctcattg gccaaggtac 540

ccccatctgt gggtgaggct gtacgtactg gaactctact gcatcatttt aggacttcca 600

ccctgtttaa atattttaag aagaaaacaa cctcaactca cgtttttcac gattgctctt 660

caaagctgcc attaccaaag gctaccaccc cacatcctgt gggccacagg gttgaaatga 720

cttctgggag ttggggatgg atgaaatgac tccttgtatg tcttgacagc agcaattgat 780

tacccactaa agagcgactg ccacaaggaa tctagaagtc g 821

<210> 190

<211> 405

<212> DNA

<213> Rattus norvegicus (Norway rat)

<400> 190
attctctctt cgagctttag agagatggag acagacacac tcctgctatg ggtgctgctc 60

ctctgggttc caggctccac tggtgacatt gtgctgaccc agtctcctgc tttggctgtg 120

tctctagggc agagggccac catctcctgt agggccagtg aaagtgtccg ttcatctatg 180

cactggtacc aacagaaatc gggacagcaa cccaaactcc tcatctacgg tgcatctaac 240

ctagcatctg gggtccctgc caggttcagt ggcagtgggt ctgggacaga ctttaccttc 300

accatagatc ctgtagaggc tgatgatatt gcaacctatt actgtcagca gagtaggaat 360

gatccgtaca cgtttggagc tgggaccaag ctggaactga aacgg 405

<210> 191

<211> 14279

<212> DNA

<213> Rattus norvegicus (Norway rat)

<400> 191
gcggacacag cgtcttctgt cgccggtttc tttcgctcac tgctccctcg ttccggaggt 60

agctcttctc ggctctctct ctctctcttc tctatctctc tcccttccgc ggatccgcca 120

tgtcggagac cggcggcggt gaggacggct cggccggcct ggaggtgtcc gcggtgcaga 180

atgtggcgga cgtgtcggtg ctgcagaagc accttcgtaa gctggttccg ctgctgctgg 240

aggacggcgg cgacgcgccg gctgcgctgg aggcggcgct ggaggagaag agcgccctgg 300

agcagatgcg caagttcctg tcagacccgc aggtccacac ggtcctggtg gagcgctcca 360

ccctcaaaga ggacgttggt gatgaaggag aagaggagaa agaattcatt tcctataaca 420

tcaacataga cattcattac ggggtgaagt ccaatagctt ggcgttcatc aagcgagccc 480

ctgtgattga tgcagacaag ccggtgtctt cccagctccg agtcctcacg ctgagcgaag 540

actcgcccta cgagaccctg cactccttca tcagcaatgc agtggctcct ttctttaagt 600

cctacatcag agagtctggc aaagcagaca gggatggaga taagatggct ccttcggttg 660

agaaaaagat tgcagagctt gagatggggc tccttcacct gcagcagaac attgagatcc 720

cggagatcag cttgccaatc caccccatta tcaccaacgt cgctaagcag tgctacgagc 780

gtggagaaaa gcccaaagtt acagatttcg gtgataaggt tgaagaccca acctttctta 840

accagttaca gtctggagtt aatcggtgga tccgagaaat tcaaaaggtg accaagcttg 900

accgagatcc agcatcagga actgccttgc aggaaatcag cttttggcta aacttggagc 960

gtgcattgta ccgcatccag gagaagcggg agagcccaga ggtgctcctg actctggaca 1020

tcttgaagca tggcaaacgt ttccatgcca ctgtcagttt tgacacagac acaggtctta 1080

agcaggcgtt ggaaacggtg aatgactaca atcctctgat gaaagacttc cccctgaatg 1140

acttgctgtc tgccactgag ctggacaaga taaggcaggc acttgttgca attttcacac 1200

atctgagaaa gatccgaaac acaaaatacc ccattcagcg ggccctgcga cttgtggagg 1260

caatttccag agacttgagt tctcagttgc tcaaagtctt gggcacgagg aagttgatgc 1320

atgtagcgta tgaagagttt gaaaaggtca tggtggcttg cttcgaagtc ttccagacgt 1380

gggatgatga gtatgagaaa ctccaagtgc tgctgaggga catcgtcaag aggaagaggg 1440

aggagaacct gaagatggtg tggcgcatca accctgctca caggaagctg caggcccgcc 1500

tggaccagat gaggaagttc cgccgccagc acgagcagct gagagctgtc attgtcagag 1560

tcctgcgccc acaggtcaca gcagttgcac agcagaacca aggagaggca cctgaacccc 1620

aagacatgaa agtggccgag gtcctctttg atgctgcaga tgccaacgcc atcgaggagg 1680

tgaacctggc ctatgagaac gtcaaggaag tagatgggct ggatgtttcc aaagaaggga 1740

cggaggcttg ggaggctgca atgaagagat acgatgagag gatcgaccgc gtggagaccc 1800

gcatcactgc ccgcctccga gatcagctcg gcacagccaa gaatgccaat gagatgttca 1860

ggattttctc caggttcaac gcactgttcg tccgcccaca catccgaggg gccattcgtg 1920

aataccagac ccagctgatc cagcgtgtaa aagatgacat tgagtctctg cacgacaagt 1980

ttaaggtcca gtacccacaa agccaagctt gtaaaatgag tcatgtccgc gacttgcccc 2040

ctgtgtcagg gtctatcatc tgggctaaac agattgaccg acagctcact gcctacatga 2100

agcgagtgga agacgtcctg ggcaagggtt gggagaacca cgtggaaggg cagaagctga 2160

agcaggatgg tgacagcttt cgaatgaagc tcaacaccca ggaaatcttc gatgactggg 2220

cgaggaaggt tcagcaacgc aaccttggtg tttccgggcg catcttcacc attgagagcg 2280

ctcgcgtgcg gggccgctct ggaaacgtcc tcaagctaaa ggtgaacttc cttccagaga 2340

tcataacact ctccaaagaa gtccggaacc tcaagtggct tggcttccgc gtgccacttg 2400

caattgtgaa caaggctcac caagccaacc agctctaccc atttgccatc tcgctgattg 2460

aaagcgtccg cacttacgaa cgtacctgtg agaaggtgga ggagcgcaat accatttcac 2520

tgctagtggc cggcctgaag aaggaggtgc aagccttgat cgcagaaggc atcgcgttgg 2580

tgtgggagtc ctacaaactc gacccctatg tgcagcgcct ggcagagacg gttttcaact 2640

tccaagagaa ggtggacgac ctcctgatta tcgaggagaa gatagacctg gaagtccgtt 2700

ccttggaaac ctgcatgtat gatcacaaga cattctctga aatcttgaac agagtgcaga 2760

aagcagtgga cgacctcaac ctgcactcct actccaacct gcccatctgg gtcaacaagc 2820

tggacatgga gattgaaagg atactgggtg tccgcctgca agctggcctt cgagcttgga 2880

cccaggttct cctcggacaa gctgaagata aagcagaggt agacatggac accgatgctc 2940

cacaagttag tcacaagcct ggtggagagc ccaagatcaa aaacgttgtt catgagctaa 3000

gaataacaaa tcaggtaatc tatttgaatc cacccatcga agagtgcagg tacaagctat 3060

accaggagat gtttgcttgg aagatgattg tgctgtccct gcccaggatc cagagtcaga 3120

ggtaccaggt gggagtgcat tatgagttga ccgaggaaga gaagttctat cggaatgcct 3180

tgacacgcat gcctgacggc cctgtcgcac tggaagagtc ctactcggcg gtcatgggca 3240

ttgtgactga agtcgagcag tatgttaagg tttggcttca gtaccagtgt ctgtgggaca 3300

tgcaggcaga gaacatttac aacaggctgg gggaggacct cagcaagtgg caagcgctcc 3360

tggtgcagat caggagagcc cgagggacct tcgacaatgc cgagaccaag aaggagtttg 3420

gaccagtggt gatagattac ggcaaggtcc agtccaaggt gaacttgaag tatgactcct 3480

ggcataagga ggttctcagc aaatttgggc agatgctggg ctcaaacatg acagaattcc 3540

attcccagat ctcaaagtct cgccaagagc tggagcagca ctccgttgac acagccagca 3600

cctctgacgc ggtgaccttc atcacctatg tgcagtctct gaaacggaag atcaagcagt 3660

tcgagaagca agttgaactc taccgcaatg gtcagcgtct gctggaaaaa caaaggttcc 3720

agttcccacc ttcttggctt tacattgaca acatcgaggg tgagtggggg gccttcaatg 3780

acatcatgcg tcggaaggac tctgccattc agcaacaggt tgcaaacctg caaatgaaga 3840

tcgtgcagga ggaccgagct gtggaaagcc ggactaccga tctgttgaca gactgggaga 3900

agaccaaacc tgtcacaggc aacctacgtc ctgaggaggc tcttcaggcc ctcaccatct 3960

atgaagggaa gttcggccga ctgaaggatg acagggagaa gtgtgccaag gccaaggagg 4020

cactggagct cacagacacg gggctcctca gcggcagtga agaacgagtc caggtggcct 4080

tggaggagtt acaagacctc aagggggtgt ggtcagagct ttctaaggtc tgggaacaaa 4140

ttgatcaaat gaaggagcag ccatgggtct cagttcagcc tcgtaagctt cgacaaaacc 4200

tagacggcct cctgaaccag ctgaagaact tccctgcaag gcttcggcag tacgcctcct 4260

atgagtttgt tcagaggctg ctgaaaggct acatgaagat aaacatgttg gtaattgaat 4320

tgaaatcgga agcacttaaa gaccggcact ggaagcagct gatgaagagg cttcacgtta 4380

attgggtggt ttctgagctg accctgggcc agatctggga tgttgacctt cagaagaacg 4440

aagccattgt caaggatgtg ctgctagtgg cacaaggaga gatggctttg gaagaatttt 4500

tgaagcagat acgggaagtg tggaacacat acgaactaga cttggtcaac tatcagaaca 4560

agtgccgtct gatccgaggc tgggatgacc tcttcaacaa ggtcaaggag cacatcaaca 4620

gcgtctctgc catgaagctc tctccgtatt acaaggtgtt tgaagaagat gccctgagct 4680

gggaggacaa gctgaaccga atcatggctc tctttgacgt gtggattgat gtgcagaggc 4740
gctgggtcta cttggaaggc atcttcacag gcagtgcaga catcaaacat ctgctgcctg 4800
tggaaaccca gcgctttcag agtatcagca cggagttttt ggctctgatg aaaaaggtct 4860
ccaagtctcc acttgtgatg gatgttctga acatacaggg agtccagagg tctctggaaa 4920
ggttggccga cctgcttgga aagatccaga aagcattagg ggagtacctg gaacgagagc 4980
gctcatcttt ccccaggttc tactttgtgg gtgatgagga tttgcttgaa atcattggaa 5040
atagcaagaa tgtggctaag ttgcagaagc acttcaagaa aatgttcgct ggcgtttcca 5100
gcatcatctt aaatgaggac agctctgtgg tcctcggcat ttcctcccga gaaggggagg 5160
aggttatgtt taaaactcca gtgtccatca ctgaacaccc caaaatcaac gagtggctca 5220
ctctggtgga aaaggagatg agagtcacct tggccaaact tctggcagaa tctgtaacag 5280
aagtggagat ttttggaaaa gcaacttcta ttgacccaaa tacctacatt acttggattg 5340
ataaatacca ggcccagcta gtggttttgt cggcccagat agcctggtct gagaacgtgg 5400
agaatgcact aagcaacgtc ggaggaggcg gagacgtggg gcccttgcag tctgtgctca 5460
gcaacgtgga ggtcacactc aacgtcttag cagattctgt cctcatggag cagccccctc 5520
tccggagaag gaaactggag cacttgatca cagagttggt tcaccaaaga gatgttacca 5580
ggtccttgat caaaagcaag attgacaacg ccaaatcttt cgaatggctc agccagatgc 5640
gattttactt tgaccctaag caaacggacg tgttacagca gctgtccatc cagatggcaa 5700
atgccaagtt taactacggc tttgagtacc tgggcgttca agacaagctg gttcagacgc 5760
ccctcacgga ccgctgctac ttgacaatga cacaggctct ggaggccagg ctcgggggct 5820
ccccgttcgg accggctgga acaggaaaga cggagtctgt caaagccctc ggccatcagc 5880
tggggcgatt cgtcttagtt ttcaactgtg acgaaacctt tgatttccag gcgatgggac 5940
ggatcttcgt ggggctttgc caagtgggcg cgtggggctg cttcgatgag tttaaccggc 6000
tggaggagcg gatgctgtcc gctgtgtccc agcaggtgca gtgcatacaa gaggcactgc 6060
gggagcactc caaccccaac tacgacaaga cctcagcccc cattacttgt gagctgctga 6120
acaaacaagt caaggttagc ccagatatgg ctatcttcat caccatgaat ccaggctacg 6180
caggccgctc aaacctccct gacaacctca agaagctctt ccggagcctg gcgatgacca 6240
agcctgaccg gcagctgatc gcccaggtca tgctgtactc acagggcttc cggactgctg 6300
aggtccttgc caacaaaatc gtcccgttct ttaaactgtg tgacgagcag ctgtcttctc 6360
aaagccatta tgactttggt ctgcgggctt tgaagagcgt gctcgtgagt gcaggcaacg 6420
tgaagaggga gaggatccag aagataaaga gggagaaaga agagcgaggg gaagccgtcg 6480
atgaaggaga gatcgctgag aacctgcctg agcaggagat tctgatccag agtgtctgcg 6540
agacgatggt gcccaagctc gtggcggaag acatcccgct gctcttcagt ctcctgtcag 6600
acgtgttccc tggggtgcag taccaccgtg gggagatgac agcccttcga gaagagctga 6660
agaaagtgtg tcaggagatg tacttgacgt atggtgatgg agaagaagtt ggcgggatgt 6720

gggttgaaaa ggttctccag ctctatcaga tcacccagat taaccatggc ctgatgatgg 6780

ttgggccttc aggaagcggg aagagcatgg cctggcgtgt gctgctgaaa gctcttgaga 6840

ggctcgaggg tgtggagggt gtggcccaca taattgaccc caaggccatc agcaaagacc 6900

acctctacgg aaccttggat cccaacacca gggagtggac agacggactc ttcacacatg 6960

tgctgagaaa gatcatagac aatgtgagag gcgagctgca gaagcgccag tggattgtct 7020

tcgatggtga tgtggacccc gagtgggtgg agaacctcaa ctccgtgctc gacgacaaca 7080

agctcttgac attgcccaat ggagagcgtc ttagcctgcc acccaatgtg cgaataatgt 7140

ttgaggtgca ggacttgaag tatgcaaccc tggccaccgt gtctcgctgc ggcatggtgt 7200

ggttcagtga ggacgtgctg agcacggaca tgatcttcaa caacttcctg gccaggctgc 7260

gcagcattcc cctggatgag ggggaggatg aggcacagcg aaggcgcaaa ggcaaagaag 7320

acgagggtga agaggccgcc tctccaatgc tgcagattca aagagatgca gcaacgatca 7380

tgcagccata cttcacgtcc aatggcctgg tcaccaaggc gctggagcat gccttcaagc 7440

tggagcacat catggacctg acccgcctgc gctgcctggg ctcactcttc tccatgctgc 7500

accaggcctg ccgcaatgtg gcccagtaca atgccaacca tcctgatttc cccatgcaga 7560

ttgagcagct ggagcgctac attcagcgct atctggtgta tgccatcctc tggtccttat 7620

ctggagacag ccggctgaaa atgagagcag agctgggcga gtacatcaga aggatcacca 7680

ctgtgcctct gcccaccgcg cccaacatcc ccatcattga ctatgaggtg tccatcagtg 7740

gagaatggtc accgtggcag gccaaggtgc ctcagattga agtggagaca cacaaggtag 7800

cagccccaga tgtcgtcgtg ccaaccctag atacagtccg ccatgaagct ctcttgtaca 7860

cctggctggc tgagcacaaa cccctggtct tatgcggccc tcctgggtct ggcaagacca 7920

tgacactctt cagcgccctc cgagccttgc ctgacatgga ggttgtgggc ctgaacttct 7980

caagtgccac tacaccagag ctgttgctga agacgtttga ccactactgc gaataccggc 8040

gcacccctaa cggtgttgtc ttggctcctg ttcagcttgg aaagtggcta gtcctgtttt 8100

gtgatgaaat caacttgcca gatatggata agtacggtac ccagagagtc atctccttca 8160

tcagacagat ggtggagcat ggaggcttct atcgtacctc agaccaaaca tgggtgaagc 8220

tagagagaat ccagtttgtt ggagcttgca atccgcccac tgaccctggg agaaagcccc 8280

tctcacatag gttcttgcgc cacgtgcctg ttgtgtatgt ggattaccca ggaccagcct 8340

ccctgacgca gatctatggc accttcaacc gcgccatgct gagactcatt ccctcgctcc 8400

gcacctatgc agaacccctc accgcagcca tggtggagtt ctataccatg tcccaggaga 8460

gatttactca ggacacacag ccacactata tctattcacc ccgtgagatg actcggtggg 8520

taagaggaat ctttgaagca ctgagacccc tggagacatt gcctgttgaa ggtctcattc 8580

gcatttgggc acatgaagcc ctccgtctct tccaagacag gcttgtggag gatgaagagc 8640

ggcgctggac tgatgagaac atcgacatgg ttgccttgaa gcatttccct aacatagaca 8700

aagagaaagc catgagccgg cctatcctgt acagcaactg gctgtcaaag gactatatcc 8760

cagttgacca agaggagcta agggattatg tcaaggctag gctgaaggtc ttctatgaag 8820
aagagcttga cgttcccctg gtcttgttca atgaagtgct ggaccatgtg ctcaggattg 8880
acaggatatt ccgacaacct caaggccact tgcttctcat tggtgttagt ggagctggaa 8940
agaccaccct gtctcggttt gtcgcctgga tgaatggctt gagtgtctat cagattaagg 9000
tccacaggaa atacactggg gaagactttg atgaagacct caggacagtg ctgcgacgct 9060
cgggctgcaa aaatgagaag atagcattca taatggatga atccaatgtc ttagactctg 9120
ggttcctgga acgaatgaat actctgctgg ccaacggcga ggttcctggg ctctttgaag 9180
gggacgagta tgccacgttg atgactcagt gcaaagaggg agcacagaag gagggcctca 9240
tgctggactc acatgaggag ctctacaagt ggttcaccag ccaggtgatc cgcaacctgc 9300
acgtggtgtt caccatgaac ccgtcctcag aggggctcaa ggacagggcg gccacgtcac 9360
cagccctttt caacaggtgt gtgctgaact ggtttggtga ctggtccaca gaagcactct 9420
atcaagttgg caaagaattc accagtaaaa tggacctgga gaagccaaac tatatagtgc 9480
ctgactacat gccagttgtg tacgataaac ttccacagcc cccaacacac cgggaagcca 9540
ttgtgaacag ctgtgtgttt gttcatcaga ctcttcacca ggctaacgca cgcctggcaa 9600
agcgaggcgg cagaacaatg gccatcacac ctcggcacta cctggacttc atcaaccact 9660
acgccaacct cttccatgag aagcgcagcg agttggagga gcagcagatg cacctgaacg 9720
ttgggctccg aaaaatcaaa gagactgtgg accaggtgga agaactgcgc cgtgacctga 9780
ggatcaagag ccaagagctg gaggtcaaga acgcagcagc caatgacaag ctgaagaaaa 9840
tggtgaaaga ccagcaagag gctgagaaga aaaaggtcat gagccaagaa attcaggagc 9900
agctgcacaa gcagcaggaa gtgattgcag ataagcagat gagtgtcaag gaggatctgg 9960
ataaagtaga gcctgctgtc atcgaggccc agaatgctgt gaagtccatc aagaagcagc 10020
acctggtgga ggtaaggtcc atggccaacc ctccagctgc cgtgaagctg gctctggagt 10080
ccatctgcct gctgcttggt gagagcacca cggactggaa gcagatccgc tccatcatca 10140
tgagagagaa cttcatcccc accatcgtca acttctcagc cgaggagatc agtgacgcca 10200
taagagagaa gatgaagaaa aactacatgt ccaaccccag ttacaactac gaaatcgtca 10260
accgggcttc tctggcttgc ggccctatgg tgaagtgggc aattgcacag ctcaattatg 10320
cagacatgtt aaagcgagtg gagcccctgc ggaatgagct gcagaagctg gaagatgatg 10380
ccaaggacaa ccagcagaaa gccaatgagg tggagcagat gatccgggat ctggaagcca 10440
gcatcgcccg ctacaaggag gagtatgctg tcctcatctc ggaggcccag gccatcaagg 10500
cagacctggc agctgtcgaa gcgaaagtaa accgaagcac tgcacttcta aagagtttat 10560
ctgctgaaag ggaacgttgg gaaaagacaa gtgaaacgtt caaaaaccag atgtctacca 10620
ttgctgggga ctgcctcctg tcggcggcct tcattgctta tgcaggctac tttgaccagc 10680
agatgcgcca gaacttgttc accacctggt ctcatcactt acagcaagcc aacatccagt 10740
tccgcacaga cattgccagg acggagtacc tctccaatgc tgacgagcgc ctccgctggc 10800

aggccagctc cctgcctgct gacgacctgt gcacagaaaa cgccatcatg ctaaagcggt 10860

tcaataggta cccgttgatc attgacccct ccggacaggc cacagaattc atcatgaatg 10920

agtataagga ccggaagatc acaaggacca gcttcttaga cgatgccttc cggaagaatt 10980

tggagagtgc tctgagattt ggcaatcccc ttctcgtcca ggatgtggag agctacgatc 11040

cagttttgaa tccagtactg aaccgtgaag tccggcgaac aggagggaga gtgctgatta 11100

ccctcggtga ccaagacatt gacctgtcac catcctttgt catctttctg tctacccgag 11160

acccaactgt ggagttccca ccagatctct gttcccgggt gaccttcgtg aacttcacgg 11220

tcacccgcag cagtttacag agccagtgtc tcaatgaagt tcttaaagca gaaaggcccg 11280

atgtggatga gaagcgttct gacctcttga agctgcaagg ggagttccag ctccgtttac 11340

ggcaactgga aaagtctctg ctccaagctc tgaacgaggt aaaaggccgc attctggatg 11400

acgacaccat cattacaacc ctggaaaatc tgaagcggga agccgccgag gtcaccagaa 11460

aggtggagga gacggacatt gtcatgcagg aggtagagac cgtgtcccag cagtacctgc 11520

cgctgtccac tgcatgcagc agcatctact tcactatgga gtccctgaag caggttcact 11580

tcctgtatca gtactcactg cagttcttcc tggacatcta ccacaatgtg ctctatgaga 11640

accccaacct gaagggcgcc accgatcaca cacagcgcct ctctgtcatc accaaggacc 11700

tcttccaggt ggcctttaac cgggtggccc gaggcatgct gcaccaggac cacatcacct 11760

tcgccatgct gctggcgagg atcaagctga aaggcaccat gggggagccc acctatgacg 11820

cagagttcca gcacttcctc cgagggaagg agatcgtgct gagtgctggc tccaccccca 11880

aagtccaggg cctgactgtg gagcaggcgg aagctgtggc aaggttgagc tgtctgcctg 11940

cctttaaaga tctcattgcc aaggttcagg cagatgagca gtttggcatt tggctggaga 12000

gcagttcacc agagcagaca gtgccatacc tctggactga ggagacgcct gcaactccca 12060

tcgggcaggc catccaccgc ctactcctga ttcaagcttt ccgacccgac cgcctgctgg 12120

ccatggccca catgtttgtt tccacaaacc tcggagagtc cttcatgtcc atcatggagc 12180

agccacttga cctcacccac attgtgggca cagaggtgaa gccaaacaca cctgtcctga 12240

tgtgctctgt gcctgggtat gatgccagcg gacatgtcga ggacctcgcc gcagagcaga 12300

acacacagat cacttctatt gccattggct cagcagaagg ctttaaccaa gcagataagg 12360

cgatcaacac cgcggtgaag tcaggcaggt gggtgatgct gaagaatgtg cacctggccc 12420

ccgggtggct gatgcagctg gaaaagaagc ttcactccct gcagccacac gcctgcttcc 12480

gactcttcct caccatggag atcaacccca aggtgcccgt gaacctgctc cgtgcaggtc 12540

ggatctttgt gtttgagccg ccacctggcg tgaaggccaa catgctgagg accttcagca 12600

gcatccccgt ctccaggatg tgcaagtctc caaatgagcg agcccgcttg tacttcctgc 12660

tggcctggtt ccatgctgtc atccaagagc gcctgcgcta tgctccactc gggtggtcca 12720

agaaatacga atttggagaa tctgaccttc gatcggcttg tgacacggtg gacacatggt 12780

tggatgacac agccaagggc cgacagaaca tctcaccaga taagatccca tggtctgccc 12840

tgaagacatt gatggcccag tccatctatg gtgggcgggt ggacaatgag tttgaccagc 12900

gtctgctcaa taccttcctg gagcgcctgt ttaccacccg gagtttcgat agtgaattca 12960

agctggcgtg caaggtcgat gggcacaagg acattcagat gcctgacggt atcaggcgag 13020

aggagtttgt gcagtgggtg gagctgctgc ctgacgccca gacaccctca tggctgggcc 13080

tccccaacaa tgctgaaagg gtcctgctta ccacccaagg cgtggacatg atcagcaaga 13140

tgctgaagat gcagatgctg gaggacgagg acgacctggc ctacgcagag acggagaaga 13200

agacaaggac ggactccacc tcagacggcc gtccagcctg gatgagaaca ctgcacacga 13260

cggcctcaaa ctggctgcac ctcatcccac agacactgag cccactgaag cgcacggtgg 13320

agaacatcaa ggaccctttg tttaggttct tcgagagaga ggtgaagatg ggggccaagt 13380

tactgcaaga tgtgcgccaa gaccttgctg acgtcgtcca agtgtgcgag gggaaaaaga 13440

agcagacaaa ctacctgcgc acgctcatca atgagctggt gaaagggatc ttgccccgga 13500

gctggtccca ttacacagta cctgcaggca tgacggtgat ccagtgggtg tcagatttca 13560

gcgagaggat caaacagctg cagaatatct cacaggctgc tgcagccggg ggtgccaagg 13620

aactgaagaa catccacgtg tgcctgggtg gcctgtttgt gcctgaggcc tacatcactg 13680

ccaccagaca gtatgtggca caagccaaca gctggtccct ggaggagctc tgcctcgaag 13740

tcaatgtcac cgcctcccag agtaccaccc ttgatgcgtg cagctttggg gtcacaggtc 13800

tgaagctcca aggggccacg tgcagcaaca acaaactctc gctgtccaat gcgatctcca 13860

cagtcctccc cctcacacag ctacgctggg ggaagcaaac tagtgctgag aagaaggcca 13920

gtgtggtgac tttacctgtc tatctgaact tcacgcgggc agacctcatc ttcactgtag 13980

actttgaaat tgctacaaag gaagatcctc gaagcttcta tgagcgtgga gttgcagttc 14040

tgtgcactga gtaaagctaa gcttttcccg aatcctcttt ctggaatagt agaacccaat 14100

atttaactct cattcatcct tacacatttg gagggtttga tggacccgtg aaaagcaagt 14160

ggtcggttag cgttggtggt gaaaggtgct gcagttggaa atggctgggg aagggtgaag 14220

tgtgcggttt ccaagggctg accaaatggc tggttttatt aaataaaaca ctaagcatg 14279

<210> 192

<211> 2171

<212> DNA

<213> Rattus norvegicus (Norway rat)

<400> 192
ccttgccgct cgctgctagc ttggatccgc gtggactaca gggactgaat cggacccgga 60

accacatggc cccactcgcc ggtgcctctc gctcccgggt gtggtcagcg gggctactga 120

ggctgctgct gctgtcctgc tttacgctcc agaaagcggg tggggagata gctgtgcagg 180

tgctctccaa ttcgaccggc ttcttgggag ggtctacagt cttgcactgt agtctggctt 240

ccaaagacaa tgtgacaatc actcagctaa catggatgaa gagggatcca gatggatccc 300

acccttccgt gcctgtcttc caccccaaga aggggcccag catctctgat ccagagaggg 360

tgaagttctt ggttgccaag gtgtacgagg atctgaggaa cgcatctctg gccatctcga 420

acttgcgtgt agaagacgaa ggcatctatg agtgtcagat tgccacgttc cccacaggca 480

gtaagagcgc caatgtctgg ctgaaggtgt tcgcccgacc taaaaacaca gcagaggccc 540

tggagccctc tcccaccttg atgccgcagg acgtggccaa atgcatctct gctgatggtc 600

accctcctgg acgaatcacg tggtcctcga atgtgaatgg aagctaccgt gaaatgaagg 660

aaacagggtc ccagccgggc accaccacag ttatcagcta cctctccatg gtgccttcta 720

gccaggcaga tggcacgaac atcacctgca cagtggaaca tgaaagcttc caggagccgg 780

accagcagcc attgatcctt tccctacctt atccacccga agtgtccatc tctggctatg 840

aaggcaactg gtacattggc ctcactaacg tgaacctgac ctgtgaagct cgcagcaaac 900

caccgcccac caactatagc tggagcacgg ccacgggtcc ccttcccaac tccactcatt 960

tccaggaaaa cggcagtcac ctgctaatct ccaccgtgga tgacctcaat aacacgatct 1020

ttgtgtgcaa agccatcaat gccctagggt ctggcagggg ccaagtgacc atcctagtta 1080

aagaggcatc tgagattctg ccgccaaaga caagcttagg cactggctac atcattgcca 1140

tcgtcttttg tgtcctgatc atcggagtag tagcaggcat tgtattctgg aaatacaggc 1200

gtggttgtgg tcggcagtcc aggaccttag acagggagaa cgtccgctat tcagcagcga 1260

atggcgtctc tgtcccaaac gtggagacga acaacttgag gtgatggtgc tggggtagac 1320

agaactaagg aacttgaaga cataacaact ggaaccctac ttccacaaaa gaaaaagcct 1380

ccagagagac ttgactgtcc agtgtggcga acatagcaag gttgggggtc tccttggccg 1440

ctgccgaatt ccgcattgtc gaaaggactc atggaacccg gtgtgctgac tcacacttga 1500

catctcagca agcgagggcc acataaagca aggttgagtc tagcacggct gtagagagaa 1560

gccctgtcta tacacaggca agctaagggg ctttgagaca gtcagaaact gaagtctttc 1620

tttgggtaag gtaaatcctc tacctcgtgt atgtgacaaa cttgaaagac ttctacctct 1680

gagactcaag tgcggactct ctttatagct gactcagctg gggctaaccc ctctctcctc 1740

tctggacaag gtctcagagt gtagccaaag ctagaccgaa actcacagag gtccgtctgt 1800

ctctacctcc caagtgctgc agttaaaggt ttgtgtgtgc cacactcctt tgctaggtct 1860

ttttaataaa gtaaatattt aataaagtaa tatatttata aaaaaactag ttataatata 1920

tattttttga gacagtgttt cctgtagccc aggctgacct caaacttact atgtagccaa 1980

gaatgatagt aaactaattt attttaattt gtcttcaagc ttaaacatag cccaacccct 2040

gctcctttcc ctctcttctc tcaatccatt ttcgtcttct ttttcttccc agacactatt 2100

ctgatgtatg tcttcattgc aaacatttta ttgaccttcg taaaaatgtg tgaaccacag 2160

ataaaaaaaa g 2171

<210> 193

<211> 826

<212> DNA

<213> Rattus norvegicus (Norway rat)

<400> 193
gcggcttgcg gagcgggagg cagcggccgg agagccaggc ggaacctgcg ggggcggagg 60

cggcggtggc agccggggcg cagatagcag gaacaggatc aagccctcca taccagggac 120

caggctaaaa ccagattacc actgccccta cctgggccct gttgacttaa agcagccact 180

gtaatcatgg gcaatatctt tgggaacctt ctgaagagcc tgatcgggaa gaaggagatg 240

cgcatcctga tggtgggcct ggatgctgca ggaaagacca ccatcctcta taagctgaag 300

cttggcgaga ttgttaccac cattcccacc atcgggttta atgtggagac agtggaatat 360

aagaatatca gcttcacagt atgggacgta ggtggccagg acaagattcg gcccctctgg 420

agacactact tccagaatac ccaaggcttg atatttgtgg ttgacagcaa tgaccgggag 480

cgagtgaacg aggcccggga agagctgatg aggatgctgg cggaggatga gctccgggat 540

gctgtgctgc ttgtgtttgc aaacaaacag gatttgccga atgctatgaa tgctgctgag 600

atcacagaca agctgggcct gcactctctg cgccaccgca attggtacat tcaggccacc 660

tgtgccacca gcggggacgg gctctatgaa ggcttggact ggctggccaa tcagctcaaa 720

aacaagaagt gagagccagg cagccctctc tgactacccc acccactcct gaaacatacc 780

tgttgtctcc ttaccccaat tccccctttt cttcctcatg aaatgt 826

<210> 194

<211> 1608

<212> DNA

<213> Rattus norvegicus (Norway rat)

<400> 194
gacagccgaa cacttaacgc cgcgccggag ccaccgcacc agtcgccgag caggtcccgg 60

tcccgctacg tcgtgtcccg cggctctgcc gacactccgg cctgagagac gggtcttcag 120

ccttttctct tccgcccact cagttgtgag ccggaaccgt gaaggcccga gggcggacca 180

gaccctctct ttttttgacc atcaagtgcc accaccgcta tgggcctcac catctcctct 240

ctcttctcgc gcctcttcgg caagaagcag atgcgcattt tgatggttgg attggatgct 300

gcgggcaaga cgacgattct gtataaactg aaactagggg agatagtcac caccattcct 360

accattggtt ttaatgtgga aacagtagaa tataagaaca tttgtttcac agtttgggat 420

gttggtggtc aagataaaat taggcctctc tggaggcatt acttccagaa tacccagggt 480

ctcattttcg tggtagatag caatgatcgt gaaagaattc aggaaggagc agccgtgctg 540

cagaaaatgc ttctggaaga tgagctgcag gatgctgtgc tgctgctttt tgccaacaaa 600

caggatttgc caaatgctat ggccatcagt gagatgacag acaagttagg tcttcagtct 660

ctccggaaca gaacatggta tgtccaagcc acttgtgcta cacaaggaac tggtctgtac 720

gaggggctgg attggctgtc aaatgaactt tcaaaacgct aaatgaaact ggatatctaa 780

ccaaggacat gtttgataga attggtctag gcttgttaca acaaaattag tttgcatctt 840

ggttattaaa ggatatctga gacggtttgg gcagaaatta cagcatttaa aacttgtttt 900

gttgccaatt attgtttacc aagaacaatg ttgctgttta gcaatatgct tgggtttaag 960

agaaattctc cttgggggaa aaaagtgtcc attattatac ttcccctgaa cctgaatgcc 1020

tggatagata gcgattgtga cacccttaaa taaatctgtt ttgagtgttt tttgagccca 1080

caccaataat gttttaaagt tacccccttg gaactcactg atacctatca ttcctcgggc 1140

agtctgctga gttaaaaatg tagcattcca tttgtattta tttccccctt gccaaaaaga 1200

tttttctaat aatgcttgta ctggccaggg atgagctcca caacactatt cagttttctt 1260

gtactgagga ttcccccacc tccctcacat tattgactcc tggcttctat cagccaaact 1320

ttgatgtagt ttggatttga tagctaattc tgggctggtt tcaaagagtt catattgaga 1380

taacttttaa tactcagcaa attgtcttcc tttatattat cttttttatg ttgcatgttg 1440

ccttttggaa tcagcctgac tctttgccca gcatatgata gttctgctgg tattttactg 1500

tttactgatg aacatatctt cattaagaat tttttgaaaa ctcatcaaat tcaatgaata 1560

agttttcttc ataacccatt tggaattatt cctaataaaa tgattttt 1608

<210> 195

<211> 123

<212> DNA

<213> Rattus norvegicus (Norway rat)

<400> 195
cagaagttta aagtaatcct tggctaccta gtgagtgtaa ggccagcctg gaatcaataa 60

gagttggttt caaagaagca ggaggagaag gaggaacagg aacaggagga ggagcagggg 120

gag 123

<210> 196

<211> 2558

<212> DNA

<213> Rattus norvegicus (Norway rat)

<400> 196
ctcttgaggc ctcttgtctg agaggcactg aacgcaggag tctgcaggtg tggtccagat 60

ctccagatcc cccaacccac tgccaccatg gtaggaaaag gtgccaaagg gatgctgaat 120
ggtgctgtgc ccagcgaggc caccaagaag gaccagaacc tcacacgggg caactggggc 180
aaccagatcg agtttgtact gacgagcgtg ggctatgccg tgggcctggg caatgtgtgg 240
cgtttcccat acctctgcta tcgcaacggg ggaggcgcct tcatgtttcc ctacttcatc 300
atgctggtct tctgcggcat tcctctcttc ttcatggagc tctccttcgg ccagtttgca 360
agccagggct gcctgggggt ctggaggatc agccccatgt tcaaaggcgt gggctatggt 420
atgatggtgg tgtccacgta catcggtatc tactacaacg tggtcatctg catcgccttc 480
tactacttct tctcgtccat gacgcatgtg ctgccctggg cttactgcaa taatccctgg 540
aacacacctg actgtgccgg tgtgctggat gcttccaatc tcaccaatgg ctcccggccc 600
actgccctgt ctggcaacct gtctcacctg ttcaactaca ccttgcaaag gaccagcccc 660
agtgaggagt actggaggct gtatgtgctg aagctgtcgg atgacattgg agattttgga 720
gaagtgcggc ttcctctcct aggctgcctt ggcgtctcct gggtggttgt cttcctctgc 780
ctcattcgag gagtcaagtc ttcagggaaa gtggtgtact tcacggccac atttccctat 840
gtggtgctga ccattctgtt tgttcgtgga gtgaccctgg aaggagcctt cacgggtatc 900
atgtactacc tgaccccaaa gtgggacaag atcctggagg ccaaggtgtg gggggatgca 960
gcctctcaga tcttctattc cctgggctgt gcatggggtg gcctcatcac catggcatcc 1020
tacaacaaat tccacaacaa ctgctaccgg gacagcgtca tcatcagcat caccaattgt 1080
gctaccagtg tctatgctgg cttcgtcatc ttctctatcc taggcttcat ggccaatcac 1140
ctgggtgtgg atgtgtctcg ggtggcagac cacgggcccg ggctagcttt cgtggcttac 1200
cccgaggctc tcacactgct tcccatctcc ccgctctggt ccttgctgtt tttcttcatg 1260
ctcatcctgc tgggactcgg tactcagttc tgcctcctgg agaccctagt cactgccatt 1320
gtggatgagg tggggaatga gtggattctg cagaagaaga cctacgtgac cttgggtgtg 1380
gctgtggctg gcttcttgct gggtatccct cttaccagcc aggcgggcat ctactggctg 1440
ctgttgatgg acaactacgc agccagcttc tccttggttg tcatctcctg catcatgtgc 1500
gtgtccatca tgtatatcta tgggcaccgg aactacttcc aggacattca gatgatgctg 1560
gggttcccac cgcctctctt cttccagatc tgttggcgtt ttgtctctcc cactatcatc 1620
tttttcattc tcatcttcac ggtgatccag taccggccaa tcacttacaa ccactaccag 1680
tacccaggct gggctgtggc catcggcttc ctcatggctt tgtcgtctgt catctgcatc 1740
ccattgtacg cattgttcca gctctgccgc acagatgggg acacacttct tcagcgtttg 1800
aaaaatgcca caaagccaag cagagactgg ggccctgccc tcctggagca ccggactggg 1860
cgctatgccc ccactacaac cccctctcct gaagatgggt ttgaggttca gccactgcac 1920
ccggacaagg cccagatccc catcgtgggc agtaacggct ccagccgcct ccaggactcc 1980
cggatatgag cacagttgtt gcaaggggag aagccccacc caacccttgc tcctaccaca 2040
gagactgagg aggtggtgga ccggtgtgac tgcctgcccc atcatgccct ggccagggtg 2100

gctgctgtca ccttggccac cactgctcat gtccccagtg tttacacatc ctttggatgc 2160

caagacagca gctgggggtg gaaaagtgat gggggtgggg gggaggtgcg tgggggtggg 2220

gcatggggcg gcagtgtcca aagcactttg gaggaggtct caggccatgt ccccagaggc 2280

ttccaggctt cattatccct catgctctgc cctgagctgt ttcactacgg gataagaggt 2340

tttgtttcca caccccacct gttcccctcc agcctcatga ccagtgttcc tgaccccaga 2400

acagacccta gcctcttgcc caggcaaatc tgtatcttcc tgctccggac aaggtgaggc 2460

gcccgggggc tgtacagtgt tacttgttgg actgcaccac tcagccctgt ttatttgtgt 2520

aattattttt ttaaaaaatt gtattacctg tggttgcc 2558

<210> 197

<211> 1579

<212> DNA

<213> Rattus norvegicus (Norway rat)

<400> 197
tagaggagtt gagttcgtgt gtgtagggag gcagacagct ccgacgcaga ctacggacct 60

ctccaggtct cagccgtcaa agaccctgtc cggtagggag tggctcgctt tgagggcaag 120

gcttctcgga tcgaggcttc ttcatggccg ctcgggtcgc gagcggccgg gcgttcgctc 180

tttgcggagg acggcgtcta acagagcgca gttgattgag gaaacactag ccggggatca 240

tgagtggctg tcgagtgttc attgggagac taaatcccgc agcgagggag aaagatgtgg 300

aaagattctt caagggttac ggacggatca gagatattga tttgaaaaga ggttttggtt 360

ttgtggaatt tgaggaccca agggatgcag atgacgctgt ttatgaactt gatgggaagg 420

aactttgcag tgaaagggtt actattgaac atgctcgggc tcggtctcga ggtggaagag 480

gtagaggacg atactccgac cgttttagca gtcgcagacc tcgaaatgat agacgaaatg 540

ctccacctgt aagaacagaa aatcgactta tagttgagaa tttatcctca agagtcagct 600

ggcaggatct caaagatttc atgagacaag ctggggaagt aacctttgcg gatgcacatc 660

gacctaaact aaatgaaggg gtagttgagt ttgcctctta tggtgactta aagaatgcta 720

ttgagaaact ttctggaaag gaaattaatg ggagaaaaat caaattaatt gaaggcagca 780

aaagacacag gtcaagaagc aggtcacgat ctcggaccag gagttcctct aggtcccgta 840

gccgatcccg ttcacgcagg agcaagtctt acagccgatc aaggagcagg agccggagcc 900

ggagcaagtc ccgttctggt agtaggtctc ccgtgcctga gaagagccag aaacgtggtt 960

cttcgagtag atctaaatct ccagcatctg tggatcgcca gaggtcccga tcccggtcca 1020

ggtccagatc agttgacagt ggcaattaat taaactgtaa ataacttgcc ctgggccttt 1080

ttttaaacaa aacaaaacaa attcccaaac catacttgct aaaaattctg gtaagtatgt 1140

gcttttctgt ggggtgggat ttggatgggg ttgggctgga tttctttgta gatgtggacc 1200

accaaggggt tgttgaaaac taattgtatt aaatgtcttt tgataagcct tctgctcaca 1260

cttttgtgac tgtctgaagt atatagtttg tgtatattga tagagctctt ttataactaa 1320

agcatttaat tttttgtact agaaaaactt ttgaatattt tagttcattg ttcagatatg 1380

ttaacataag attagtttaa ttgctcaatt aaactaatga atagctttgg acactttaaa 1440

agagctctaa atttgcttgt atataaaagc ttagtttgag ttttccttgt tagggtcaag 1500

ggtgtcttct tgcccattaa cagctgtaga tagacttaaa gcagctgttt gttactgata 1560

ataaaatact aagtattat 1579

<210> 198

<211> 831

<212> DNA

<213> Rattus norvegicus (Norway rat)

<400> 198
ggcacgagtg actaagatgg aagcgttttg ggagtcacgg actggccact gggcggaggt 60

ccggggcagt tttaccggcg tcccgtcacc cccagctgcc tcatggatcc gatgtctgcg 120

cctgcgaggt ccatcactcg gacccagaat ccgatgctga ctgggacatc cgtactaggg 180

gtgaagttcg actgcggagt ggtgattgct gcagacatgc tgggctccta cggctccctg 240

gctcgtttcc gcattatctc tcgtattatg cgagtcaacg acagcactat gctaggtgct 300

tcaggagact atgctgattt ccagtatttg aagcaagttc tcggccagat ggtgattgat 360

gaggagctgt tcggagatgg acatagctat agtcctagag ctatccattc atggttgaca 420

agggccatgt acagtcgccg ctccaagatg aaccctctgt ggaacaccaa ggtcattgga 480

ggctatgctg gtggagaaag ctttcttggt tatgtggaca tgcttggtgt agcttatgaa 540

gccccttcac tagccactgg ttatggtgca tacttggctc agcctctgct tcgagaagtt 600

ctagagaagc aaccggtgct gagtcagaca gaggcccgag agcttgtgga gcgctgcatg 660

cgagtgctgt actaccgaga cgctcgttcg tataaccggt ttcaagttgc tactgtaact 720

gaaaaaggtg tggagataga aggaccactg tcagcacaga ccaactggga cattgctcac 780

atgatcagtg gctttgaatg aaatccagat gaagtgtcca gagttaagct t 831

<210> 199

<211> 2758

<212> DNA

<213> Rattus norvegicus (Norway rat)

<400> 199
catgagtgta cttctctctt ggctaagaaa gacagcaaag caaagacttt taaagcgaag 60

aagctggaaa caatcagaag aacaccatgg aagaaacaga gaaaaagatt gcaacacaag 120

agggtagatt gttttccaag atgaaggtgt ttctgctgtc attaacatgt gcatgtttaa 180

ccaaatcact atcaggagta tatatgaatt ccatgctcac acaaatagag agacaatttg 240

atatctccac atctgtagct ggattaatca atgggagctt tgagattgga aacctttttt 300

tcattgtatt tgtgagttat tttggaacaa aactgcacag acctgtggtg attggtattg 360

gatgtgtgat tatgggcctg gggtgtctct taatgtcatt acctcatttc ttcatgggca 420

gatacgaata tgaaacaacg atctcaccta caggcaactt gtcctcaaac agcttcttgt 480

gtatggagaa ccgaacacag accttaaagc caacgcaaga cccagcagaa tgtgtgaaag 540

aaatgaaatc attaatgtgg atatgtgtaa tggtaggaaa tattatacgt ggaattggtg 600

aaactcctat tgtgcccttg ggtatctctt acatcgaaga ctttgccaaa tcagaaaact 660

ctcctttata cattgggatt ttagaaatgg ggaaggttgc tggcccaatt tttggacttt 720

tgttgggatc ttactgtgca caaatttatg tagacattgg gtctgtgaat acagatgacc 780

tgaccataac acccagtgat acacgctggg ttggtgcttg gtggattggc tttttggtct 840

gtgcaggagt gaatatcctg accagcatcc cctttttctt tttgccgaaa gcacttccaa 900

agaaaggaca acaggagaat gttgctgtga ctaaagatgg caaagttgag aaatatggag 960

gacaagccag agaggaaaat cttggaatca ctaaagattt cttgacgttc atgaagaggc 1020

tcttctgcaa tccgatttac atgcttttca tccttacaag tgtgctccag gtgaatggct 1080

ttattaataa atttacgttc ttgcccaaat acctggaaca gcagtatgga aaatctactg 1140

cagaggcaat attcctcata ggtgtttata gcttacctcc catatgcctc gggtatctaa 1200

ttggtggctt tattatgaag aagtttaaga ttactgtcaa gaaagctgca tacctagcat 1260

tttgcctatc ggtgtttgaa tatcttcttt ttctttgtca ctttatgttg acctgtgaca 1320

atgcagcagt tgctggatta accacctctt acaaaggagt tcagcaccaa ctacatgtgg 1380

agagcaaggt ccttgctgac tgcaacacaa ggtgtagctg ctcaacgaac acatgggatc 1440

cagtgtgtgg agacaatggt gtagcataca tgtcagcttg ccttgcgggc tgtaagaagt 1500

ttgttggaac tggaaccaac atggtgtttc aggattgcag ctgcattcag tcattaggaa 1560

actcgtctgc agtcctgggt ctgtgtaaaa aaggtcctga gtgtgccaac aggctgcagt 1620

actttttaat cttaacaata attatcagtt tcatctactc acttacagcc atacctgggt 1680

acatggtttt tttgaggtgt gtcaagtctg aagagaagtc acttggagtt ggattacata 1740

cattttgcat aagagtattt gctggtattc ccgcacctgt ttactttggc gctttgatag 1800

acagaacctg tttacattgg ggaaccctga aatgtggtca gcgaggggca tgcaggatgt 1860

atgacataaa cagtttcagg cacatttacc tggggttgcc tatagcacta agaggatcaa 1920

gctatctgcc tgccttcttc attctgatac ttatgaggaa attccagttc cccggggaca 1980

ttgactcttc agcaactgat catacagaga tgatgctcgg agagaaggaa agcgagcaca 2040

cagatgtgca tggaagtcct caggtcgaga atgacggaga actgaaaacg aagctgtaat 2100

gagttttcta ctgccctgtt caaggccatg aacagaatgc acacttccct tcctcggaat 2160

cctgagagat acaataggaa ccttctcttt aaggacctca acaattgttt ttctcactat 2220

aaaaataatt gctgatatca tttttatagt gagaaaaaca ataaaaataa ttgctgatat 2280

cattttcaga atttcaggtg atatttaaga tttccctggg gaagactttt atggtgaccc 2340

ccacactaca ctttaaagct tccttcattt tcacataaca ttttctctta actcaatcaa 2400

gggaaatatg tgtttcccac acatctttaa atgagtataa aactttccta ttcctgaaac 2460

agtatttaat ttcattgaat ttatggttca cagttggaag tatttagagc tgaaaagata 2520

cattctcatt aagccacatt gaaaagttaa cttttctatg ttcaggatgt ctccatatgt 2580

ctatatgtag gagttctcaa atattattat tattgtagac atgatgctcc tagattagac 2640

ttctcactcc tgtgtcattt cctgtttcta gtcccttctt tcattactcc tgtcacacag 2700

ttgaatgttt tgtttagagc actgacaaac ttaatcaggt tattaaaaat cacattag 2758

<210> 200

<211> 2287

<212> DNA

<213> Rattus norvegicus (Norway rat)

<400> 200
tctaaaggaa agatcacagc tttcccagac tgggagaaaa aatatggaat gtggtgtacc 60

actgactgac cataaccaaa tgaactgtag tgacagtagt ctaaaagcca gcggttaaca 120

atttcttcag acttcaacac tatccatcgc tttccagagc agactcgctg tttacaagaa 180

ctcttggcct cccgaagata gcaacctcaa attttgttta tttaaaatat ttctgcaaaa 240

ggaaaagtac cccagagccc attttccaaa atggccatgg atgggtatct gtggatggtc 300

attttgggtt tcattattgc tttcatcctg gcattttcag ttggtgccaa tgatgttgcc 360

aactcctttg gaaccgcggt gggctctggc gtggtgaccc tgagacaggc atgcattctg 420

gcttcgatat ttgaaaccac cggctctgtg cttttgggag cgaaagtggg agagactatc 480

cggaagggca tcattgacgt gaacctctac aatgagactg tggaaacgct catggctggg 540

gaagtgagtg caatggtcgg ctcagcggtc tggcagttga tcgcgtcctt cctgagactc 600

ccaatctcag ggacacactg catcgtgggc tctaccattg gcttctcgct cgtggccatc 660

ggcccaaagg gagtgcagtg gatggaactc gtcaagatcg ttgcctcctg gttcatatcc 720

ccacttctgt ctggcttcat gtctggtgtg ctgttcatcc tgatcagaat gttcatccta 780

accaaggagg accctgttcc aaacggtctc caggcacttc ctctgtttta tgctgccact 840

atagcaataa acgtcttctc catcatgtac acaggagcac cagtgctggg cctgtcgctc 900

cccatctggg caatagcgct catctccttc ggcgttgccc tgctgttcgc cttcttcgtg 960

tggctgttcg tgtgtccgtg gatgaagagg aaaatagcgg gcaggttaga gaaggagagt 1020

gctctgtccc gcgcgtctga cgaaagcctc aggaaggtac aggaagctga gtcccctgtc 1080

tttaaggagc ttcctggtgc caaagccagt gatgacagcg ctgtccccct caccagtctg 1140

gctggggagg ctgcgggggc ctcagaaggc acgtcagcag ggaaccaccc ccgggcttcc 1200

tacggccgag cactttccat gactcacggc tctgcaaagt cacccatctc caatggaacc 1260

tttggcttcg aaggccacat gaggaatgac ggtcacgttt atcatactgt acacaaagac 1320

tcgggactct acaaagactt gctgcacaaa atccatgtag acaagggccc cgaggagaaa 1380

ccagctcagg aaaacaacta caggctgctc cggaggaaca acagctacac gtgctacacg 1440

gcagccatct gcggcatgcc ggtgcacgcc accttcaggg cctcggacac ctcctccgcc 1500

cccgaggata gcgagaagct ggtgggtgac accgtgtcct attccaagaa gaggctccgc 1560

tacgacagct actccagcta ctgcaacgcg gtggccgagg cggagatcga ggccgaagag 1620

ggcggcgtgg aaatgaggct ggcctctgag ctgaccgatc ctgaccagcc tcacgaggat 1680

cctgcggaag acgagaagga ggagaaggac tccgcagagg tccacctgct cttccacttc 1740

ctgcaagtcc tcactgcctg ctttgggtcc tttgctcacg gtggcaacga tgtgagcaat 1800

gctatcggtc cccttgtggc cctgtggctg atatataaac aaggcggagt tacacaagaa 1860

gctgctactc cagtctggct gctgttttat gggggcgttg gcatctgcac aggtctctgg 1920

gtttggggca gaagagtgat ccagaccatg gggaaggatc tcacacccat cacaccgtcc 1980

agtggcttca ctattgagct ggcctctgcc ttcaccgtgg tgatcgcctc caacattggg 2040

cttcccgtca gcaccacaca ctgcaaggtg ggctcagtgg tagccgtggg ctggatccgc 2100

tcccggaagg ctgtggactg gcgcctcttc cggaacatat ttattgcctg gttcgtgacc 2160

gtgccggtgg ccggactttt cagtgctgct atcatggctc tcctcatgta tatatgtggt 2220

tttgtttctt ccagtcggta aatggccaca gggatgctct gacacggata tggtcctcac 2280

ctttaga 2287

<210> 201

<211> 1227

<212> DNA

<213> Rattus norvegicus (Norway rat)

<400> 201
ggaattcgca acgcctacac aaagatccct atcactgagc acccggaggc aaggctcaga 60

accccaggat cagcaacatg gagttctccc gtccaccgct agtgcatgtg aagggtatcc 120

cactcatcaa atactttgca gagacaattg ggccattgca gaacttcaca gcctggcctg 180

atgacttgct gatcagcaca tacccaaagt ctggtactac ctggatgagt gagatcctgg 240

atatgatcta tcagggtggc aagctagaga agtgtggccg cgcccccatc tatgcccggg 300

tacccttcct tgagttcaaa tgtccagggg ttccctcagg tcttgaaact ttggaagaga 360

caccagcccc acggctcctt aagacacatc tgcccctgtc cttgctccct cagagtctgc 420

tggatcagaa ggtcaaggtg atctacattg cccgaaatgc aaaggatgtg gttgtctcct 480

attataactt ctacaacatg gccaagctgc accctgatcc aggcacctgg gacagcttct 540

tggagaactt catggatggg gaagtgtcct atgggtcgtg gtaccagcac gtgaaggagt 600

ggtgggagct gagacacact caccctgttc tctatctctt ctatgaagac ataaaggaga 660

accccaaaag ggagatcaag aagattctag agtttttggg gcgctctcta cccgaggaga 720

ctgtggattc cattgttcac cacacatctt tcaagaaaat gaaagagaac tgcatgacta 780

actacacaac catccccact gagattatgg accacaatgt ttctcccttc atgaggaaag 840

gtactactgg ggactggaaa aataccttca ctgtagccca gaatgagcgc tttgatgccc 900

actatgctaa gacaatgaca gattgtgact tcaagtttcg ttgtgaacta tgagtggatt 960

atggctatac tgggaaccaa ggcaaactga cacagcccat catgatctca agtaaaatgt 1020

gatgtgttca atctacttgt tgtatgccta gaggaaatct gagctaagag aataggattg 1080

gggatgtggc tgaggcagag ggttttatca acgcatgtca ggaaagcaat cagtcccaac 1140

acctaaaaag aacctaaagt acaaacatgc aaaaaatagt aagataaact atattttacc 1200

tgaaagaata aatgccactg ggaaatg 1227

<210> 202

<211> 2094

<212> DNA

<213> Rattus norvegicus (Norway rat)

<400> 202
ggcacgagct ccgatctccg ctgagaggct cctgggggcc ggggctccga ggaaaatggt 60

tcgatgttat taaaaatgaa tcttaagaag aaaaatgaat cacagcagtt aaactatgga 120

gttctgctac ttgtaagaag tggagaagcc tgagattaca tcactgaccc ttgatcctcc 180

actgagctaa aacaccagct ggtaattgcc tatgatttta tagacttccc tccatctgct 240

gggtccaagt gtccgtctga ctgctctggt accggagcat ctttatttct gcatctaaac 300

ttgtaaaaag cacatcgaat cttgttcccc aggagaaaat cttcaatgta accattttca 360

atgtatccga tgatacaagc gcattgtaat ctccaggtag aagcagcttt atcagtggaa 420

agggtttaat agaacatatc ctatcatgct ttttctctgc cccttctcaa atcatcagca 480

gtagaaaaga gaagaaaaca tgtcaggaca caaatgttat tcctgggagt tgcaggatcg 540

gtttgctcaa gataagtcag ttgtcaataa gatgcaacag aaatattggg aaacgaatga 600

ggcctttatc aaagccacag ggaagaagga agatgaacat gtcgttgctt ctgatgcaga 660

cctggatgcc aagctagagc tgtttcattc gattcagaga acctgtttgg acttgtctaa 720

agcaattgtg ctctatcaaa agagaatatg tttcttgtct caagaagaaa atgaactggg 780

aaaatttctc cgatcccaag gcttccagga caaaacccga gcaggaaaaa tgatgcaagc 840

cacaggaaag gccctctgct tttcctccca gcaaaggttg gccttgagaa accctttgtg 900

tcgatttcac caagaagtag agacttttag acatcgggcc atctccgata cctggctgac 960

agtgaaccgc atggagcagt gccggacaga atatagaggg gcgttattgt ggatgaagga 1020

cgtgtctcag gaactggatc cagacctcta caagcaaatg gagaagttca ggaaggtaca 1080

gacacaagtc cgcctcgcga agaagaactt tgacaaattg aagatggatg tgtgtcaaaa 1140

ggtggatctt cttggagcaa gcagatgtaa cctcttatct cacatgctag caacatatca 1200

gaccactctg ctccactttt gggagaaaac ttctcacacc atggcagcca tccatgagag 1260

cttcaaaggc tatcaaccat atgaattcac aacgttaaag agcttacaag accctatgaa 1320

aaagctagtc gagaaagaaa agaagaagag ctcccggagg gaaaaccggg aggctgtggc 1380

acaggagccg aggcagttaa tttcattgga ggaagagaac cagcacaaag aatcctctac 1440

ttgccagaag gaggagggaa aaagcgttcc gtcgtctgta gacaagagtt ctgcagatga 1500

tgcatgctca ggacccatag atgaactatt agacgtgaaa cctgaggaag cttgcctggg 1560

tcccatggca gggaccccag aacctgaaag tggggacaag gacgacctcc tgctgttgaa 1620

cgagatcttc agcacttcca gcctggatga aggggagttc agcagggagt gggctgcggt 1680

gttcggagac gaccggctga aggaaccagc ccccatgggg gcccagggag agccagaccc 1740

caagccccag ataggctctg cgttccttcc ttcacagctt ttagaccaaa acatgaaaga 1800

tctccaggcc tctctccaag agcctgccaa ggctgcctcg gacctgactg cctggttcag 1860

cctctttgct gacctcgacc ccctatcaaa tcctgatgct attgggaaaa ccgataaaga 1920

acacgaattg ctcaatgcat gagtctgcaa ccttcaacag gggagccctc cggccactcc 1980

gcaacacctc atccagggct tgcagaagtc taacgtgctc agtacgctgt tttaatattt 2040

acatgccatt ttaataaaac gagagggtca aggccctgtt tctatcgcta taaa 2094

<210> 203

<211> 1507

<212> DNA

<213> Rattus norvegicus (Norway rat)

<400> 203
attccggagc tccggctcct ccggcggggc tcggtatgag gctggcaagg ctgctgcggg 60

gcggcacgag cgtcaggccg ctctgtgccg tcccctgcgc cagccgtagt ctggcctcgg 120

actcggcctc gggctccggg cccgcgtcgg agagcggcgt tccgggccag gtggacttct 180

atgcgcgctt ctcgccgtcg ccactctcca tgaagcagtt cctggacttc gggtcagtaa 240

atgcttgtga aaagacctcg ttcatgtttc ttcgacaaga gttgcctgtt agattggcaa 300

atataatgaa agaaataagt ctccttcccg ataatcttct caggacccca tccgtacagc 360

tggtacaaag ttggtatatc caaagccttc aggagctgct tgactttaag gacaagagtg 420

ctgaggatgc taaaactatt tatgaattca cagacacagt gataaggatc agaaaccggc 480

acaatgatgt catccccacc atggcccagg gtgtgactga atacaaggag agcttcgggg 540

tggatcctgt caccagccaa aatgttcagt actttttgga tcggttctac atgagtcgca 600

tctcaattag aatgctactc aaccagcact ctttattgtt tggtggaaaa ggaagtccat 660

ctcatcgaaa acacattgga agcataaatc caaactgtga tgtagtcgag gttattaaag 720

atggctatga gaatgcgaga cggctttgtg atttgtatta cgttaactct cctgaactag 780

aacttgaaga actaaatgcg aaatcaccag gacagccaat acaagtggtt tatgttccgt 840

cccatctcta tcacatggtg tttgaactgt tcaagaacgc aatgagggcc accatggagc 900

accatgcaga caaaggcgtt tatcccccga ttcaagttca cgtcacactg ggggaggagg 960

atttgactgt gaagatgagt gaccggggag gtggtgttcc gctgaggaag atcgacagac 1020

tcttcaacta catgtactca actgcacccc ggcctcgtgt tgagacgtcc cgtgcggtgc 1080

ccctggctgg atttggttac ggattgccca tatcacgcct ctatgcacag tacttccagg 1140

gagacctaaa gctgtattcc ttagagggct acgggacgga tgctgtcatc tatattaagg 1200

ctctgtcaac ggagtccatc gaaagactcc ctgtgtataa taaagctgcc tggaagcatt 1260

acagaaccaa ccacgaggct gacgactggt gtgtgcccag cagagagccg aaagacatga 1320

cgacgttccg aagctcgtaa ctgcacatgg gacgcctggc aaagccaaat gtggtttttg 1380

ccaaacttgg aagagagatg tatttggaac cacattgtac caaatatgtc atgtgtgatt 1440

tttcacaatc agtgtttgct aaagctccta aaggatcaat tatagcaact ttattcaaaa 1500

aaaaaaa 1507

<210> 204

<211> 1363

<212> DNA

<213> Rattus norvegicus (Norway rat)

<400> 204
aacctgtcaa gtccccattc taagatgtcc ttggaaaaaa tgaaagacct tcaccttggt 60

gaacaggacc tacagccaga aaccagagaa gtgaatggga ttctcatgtc caagttgatg 120

agtgataact gggacaaaat ctggaacttc caagcaaagc ctgatgatct ccttattgca 180

acctatgcaa aagcaggtac cacctggacg caggaaattg tggacatgat ccaaaatgat 240

ggggatgttc aaaaatgcca acgggccaac acctatgacc gacatccttt cattgagtgg 300

actttgcctt cacccctcaa ctcaggtctg gatctggcta acaaaatgcc atcacctaga 360

accctgaaga ctcatctgcc tgttcatatg ctgccacctt ccttctggaa agaaaactca 420

aaaattatct atgtggccag aaatgccaag gactgcctgg tatcttacta ttacttctca 480

agaatgaata aaatgctgcc tgaccctggt accctgggag aatacattga acagttcaaa    540

gctggaaaag tgctgtgggg ctcctggtat gaccatgtaa agggatggtg ggatgtgaaa    600

gaccaacacc gtattctgta tctcttctat gaagacatga aagaggaccc taaaagagaa    660

attaagaaga tagcaaaatt cctggaaaaa gacatatcag aggaagttct taataaaatc    720

atctaccaca cctcctttga tgtaatgaag gaaaacccaa tggccaacta taccactcta    780

ccctccagta tcatggacca ctctatatct cctttcatga ggaaagggat gcctggagac    840

tggaagaact actttactgt ggcacaaagt gaggattttg atgaagacta ccggaggaag    900

atggcaggga gcaatattac cttccgcaca gagatctgag agcagtgagg aagagagaag    960

ccctagattt cctgactata tgctttagct atttgagctt cattcctgag ttttgtatgt   1020

cctgtgatac tatttcatca aaatgtaatc agaccttcca cactaggtga ttatccttat   1080

tgatacctac tatacaacca tgcactttta ctgcacttac gcaaataaca gataccttca   1140

ctagcctgta attgtcttgt ttcacggcaa atctcatgaa tagagagaca cacaaaacag   1200

gttagacata agaaagtaaa taagaaaagc caaacgaatg agaagtgagc actgtgcatt   1260

aaccaaaggc tatttaattt tcttaacaat tgtcttcatc tgttctcttt aacgaaatac   1320

ctaatttgtt tataaagaat aaaaatgatt tcttatgcaa aac                     1363

<210>  205

<211>  238

<212>  DNA

<213>  Rattus norvegicus (rat)


<400>  205
aagactaccg gaggaagatg gcagggagca atattacctt ccgcacagag atctgagagc     60

agtgaggaag agagaagccc tagatttcct gactatatgc tttagctatt tgagcttcat    120

tcctgagttt tgtatgtcct gtgatactat ttcatcaaaa tgtaatcaga ccttccacac    180

taggtgatta tccttattga tacctactat acaaccatgc acttttactg cacttacg      238

<210>  206

<211>  1124

<212>  DNA

<213>  Rattus norvegicus (Norway rat)


<400>  206
cggcacgagc taacaccctg ttctcagact cctccgcgcc tctccgcctg tcctcaggat     60

catgaaggtc gccagtagca gtgccgcggc caccgcaggc cccagctgtt cgctgaaggc    120

aggcaggacg gcgggcgaag tggtgcttgg tctgtcggag caaagcgttg ccatctcgcg    180

ctgcgctggg acgcgcctgc ccgccttgct ggacgaacag caggtgaacg ttctgctcta 240

cgacatgaac ggctgctact cacgcctcaa ggagctggtg cctaccctgc ctcagaaccg 300

caaagtgagc aaggtggaga tactgcagca tgttatcgac tacatcaggg acctgcagct 360

ggagctgaac tctgagtctg aagtcgcgac cgccggaggc cgggggctgc ccgtccgggc 420

cccgctcagc accctgaacg gcgagatcag tgccttggcg gccgaggtga ggtccgagtc 480

agagtattac attattctcc tgtgggaaac taaggccacg ggaggggggt gtccccctta 540

cttctcagga gcatagttat ttaggggcga ccaataggaa aaagctcgcg ctttcatcgt 600

gcctcctgga gtagagaagt gggaatgcct ctcccctcca gttctttcca gtgggtctca 660

tgccttatct cgctctggtg ttcacaggcg gcatgtgttc cagccgacga ccgcatcttg 720

tgtcgctgag gcggcgcact gaggaaccag atggactcca gcccttcagg aggcaagagg 780

aaaaaaagtg ctctcggttc cccagagcaa cccggggaaa gacactaccg cggccacggg 840

actcttgacg gatctgtcca gggggtagag ggttgatcaa cggagtctcg ccctctccac 900

ctttcagcct ccagagactt tgaggagggg gttattcaac cccgtgtgtt tctgtttttt 960

tgaaaaagca gacatttttt ttaaatggtc acatttcgtg cttctcagat ttctgagaaa 1020

atgttttgta ttgtatatta caatgatcac tggctgagaa tattgtttta caatagttct 1080

tatgggggtg ggttttttgt tgttattaaa caaacacttt agat 1124

<210> 207

<211> 2015

<212> DNA

<213> Rattus norvegicus (Norway rat)

<400> 207
gcagagcatc agaggcccag ctagagggac aacacagagg agtaatttgc tgacagacct 60

gcagggatgg acctgctttc agctctcaca ctggaaacct gggtcctcct ggcagtcgtc 120

ctggtgctcc tctacggatt tgggacccgc acacatggac ttttcaagaa acaggggatt 180

cctgggccca aacctctgcc tttttttggc actgtgctga attactatat gggtttatgg 240

aaattcgatg tggagtgcca taaaaagtat ggaaaaatat gggggttgtt tgatggtcaa 300

atgcctctgt ttgccatcac ggacacagaa atgatcaaga atgtgctagt gaaggaatgc 360

ttttctgtct tcacaaaccg gcgggatttt ggcccagtgg ggattatggg gaaagccatc 420

tctgtatcta aggatgagga gtggaagaga tatagagcct tactgtcacc cacgttcacc 480

agtggaagac tcaaggagat gttccctgtc atcgaacagt atggagacat tttggtaaaa 540

tacttgaggc aagagaaagg caaacctgtc cctgtgaaag aagtgtttgg tgcctacagc 600

atggatgtga tcaccagcac atcatttgga gtgaatgttg attccctcaa caacccgaag 660

gatccttttg tggagaaagc caagaagctc ttaagaattg atttttttga tccgttgttc 720

ttgtcagtag tactctttcc attcctcacg ccagtatatg agatgttaaa catctgcatg 780

ttcccaaaag attcaataga atttttcaaa aaatttgtgt acagaatgaa ggaaacccgc 840

ctggattctg tgcagaagca tcgagtggat tttcttcagc tgatgatgaa tgctcataat 900

gattctaaag acaaagaatc tcatacagcc ctatccgata tggagatcac agcccagtca 960

atcattttta tttttgctgg atatgaaccc accagcagca cactttcctt tgtcctgcat 1020

tccctggcca ctcacccaga tacacagaag aaactgcagg aggagatcga cagggctctg 1080

cccaataagg cacctcccac ctatgatact gtgatggaaa tggaatacct ggatatggtg 1140

ttgaatgaaa ccctcagatt gtatccaatt ggtaatagac ttgagagagt ctgtaaaaaa 1200

gatgttgaaa tcaatggtgt gtttatgccc aaagggtcag tggtcatgat tccatcttat 1260

gctcttcacc gtgatccaca gcactggcca gagcctgagg aatttcgccc agaaaggttc 1320

agcaaggaga acaagggcag cattgatcct tatgtatatc tgccctttgg aaatggaccc 1380

aggaactgca ttggcatgag gtttgctctc atgaatatga aactcgctct cactaaagtt 1440

ctgcaaaact tctccttcca gccttgtaag gaaacacaga tacctctgaa attaagcaga 1500

caaggacttc ttcaaccaac aaaacccatt attctaaagg ttgtgccacg ggatgaaatc 1560

ataactggat catgatttcc cctcaaggag ttctgctgaa ttcgtcagaa atgtggtgtc 1620

taagaacacc agacccttta atttatgtca tgaataaaat tcagatgaaa ttagggctta 1680

atcgactttg ttttgattcg gtacatcttt gatctttctc agtgtctaca atgtacccat 1740

ctaatataaa ggaaatgaca agtcagtgac agaacaggac ttaacctttg gtgattctca 1800

tgggactacc tccatttgtt tctggttgtc tctgttaatt tcttttgata gtaaccttgt 1860

ctctgtaatt tgatcaagaa ttttcatgaa aatgtgaact attgtgacac ctttaattgt 1920

agatttggta tcagatgttt tagatgcatt attctacact aaatgttaca tggaaaaaat 1980

gtgaataaac acttctttaa aaatccccag gggca 2015

<210> 208

<211> 13553

<212> DNA

<213> Rattus norvegicus (Norway rat)

<400> 208
gatcatcctt cctgaggctc ctgaatgctg aatacctgaa tcccttccca cagctgttct 60

ccatccgtga gtggggaccc tttagggggg ctttgaatga ccctttcata ggagttgcat 120

gtacagatat tctgcatata agatatttac attatattta taacagtata acagtagcaa 180

aattatagtt ataaagtagc aacaaaataa ttttatggtt gaaggttgcc acaacttgat 240

tattaagggt cacagtatta ggtaggttga gaacactgct ttactacatt ctgctgaagg 300

ttctttttct ttctattgtt tttgtttgtt cgtttgtttg ttttttctgt gtgtagccct 360

ggtggtcctg gaacttggtt tgtagaccag gctggcctta aactcacaga gatccacctc 420
gctctggctc gcaagtgctg gaactaaaga tatgtgccac catgctggac taaaggttat 480
tcttttctaa ccaaagggat aaccaaattc atagagctct acctgcctat aaaaaagtta 540
ttagtgtgta catgtagggg ggcacagtac tacagtgtat gtgtgtggag gtctgaggac 600
aattttgtgg agccgttttt ctttaattgc ctatatgtgg gtttagccat ggaactctca 660
tctttttgca tagtaagtca tttacccact gagccatttg tctgggacac tccactggtg 720
attcacatta actagttttc taactagcct gtgaagacca gctagtccca agggttcatg 780
ttgttattcc agcatttggg aagctgaggc aggagaatag acatttgaag ttgttctcaa 840
tttatacaac gagtttacaa acaacctggg ctacatatta ccctgtctca aacagaaaaa 900
taaatgaccg taaaagctct gatttctgtc ctccagggcc tgaatttgag caacttcata 960
gaagacattt acttagggag aagtgatccc tcacagagat ttgggtaggt tctaggcctt 1020
tctctcaact tcaggagcaa ggggcactat aaactgttat ttctaaagaa taagtatgag 1080
ggaagggacc aaaaaaatga catcatcaag gagcacacgc tggtcaagca tgggtgtaac 1140
aagttcagcc agcctttgga ggagggcttg ggagtggggg aggggggctt gggaaaagtg 1200
tttgcccagg agagtgttgc aacattgttg cagggagctt ttgacccagg gtttttgcca 1260
gggcatagcc atgccacgct gattctctgc actctgggct gatgcatcac ctgagccgat 1320
tagaggatga ttctggcctc cttcctagag ctggaaaatt gtttgtttct gggaccgtct 1380
gggaagacat actgggacta aggtacaagc aaaagaggct ttcactccag ggaagcagta 1440
aatcggagtt ctgatttcca ggacgttctg attttatata ttctgctcca caagtcgggc 1500
acctgttaga ctgggtgctc cacttttgga tttggaaaat atgttctacc tctcatatgt 1560
aacagaaaag aagaggaagc tgttcattgc tttcagtgtc ttagtttgag ttgtgggagg 1620
gagcaagagg ttggacaggg atggccccag tgctaggttt taatctcaga taaatgatca 1680
gctccgccca gatccgcgct caggtgagcc cgtaagagga ctcgcgttgg aactggcgtc 1740
cttctggcct cactagttta cctcttcctc ctatctatgt tctacgtcgg gcagtctgat 1800
tgtgtagcgt ggtcatgtag tctgattcta gtaaattcta tggggtgggt ttcaggagcg 1860
gagaggcgcc acgtgtgaaa ggaaagcaga cgtagctttc caaagcaggt ctaccgcact 1920
ggtttggtgt ggcgacttac aaacgccaga gataagaacg tcagttgctc ccaggggagg 1980
gagggagggg acgccaggtc ccgggagaag tgctactgcc ttttgggcca acaaccccct 2040
cccacagcta cccaatccag cagctggccg gcccagcccc tgaagcgatc aggccacagc 2100
ccccagcctc gtaggttctc ctcctagagc tgccccgccc atctccccat cctaggccgc 2160
ccacggcccc tcccacttcc aagactacaa gtcccgtcag ccccgggtca ggcatgaggg 2220
cggggccacg cagcagcgaa tggggaggag cctagagctc aggctaggga acgcgtgtgc 2280
ccaatcgcca ggctgcatgg cctgtgagct gatggggggc ggggaggagc ccgcttgggc 2340
cgggacaaaa gccggatccc cggcagctgc aggcggctgg agcgatccgg ggagttgggg 2400

taagctggtg cttacggaca gagagccaca ctcgggcttt ctcgaagagg tgagtactgc 2460
aatcgtaggc gcgggtgcga ccccctagtc cccactaagc ttcctgcttc cctcccccaa 2520
cccggaacca cctccgggct cgctggctgg aagatcccgt gcgctccctg cacctgctcg 2580
gaagttttgt gcgggtctca gctttatagc agtcttcgcc ccagccgcgg gccgcatgtc 2640
tacactgccc ccccaccctc agtctttttc tagttcctgg gagtttgcac attgcttgcc 2700
accgaccttg cttctcctcc ctcctccgga ttgcctttgc gtgtcctgct tcttctcagc 2760
tccacccagg accagctctt gagtctcttc gggttttttg ggacactttt ttgggcccca 2820
ttcgggttca ctaccggatg ccggggcggg tagtagagat ggcggtggct actctcccca 2880
cgccggccgc tcacaaggcg gactcaagca ggcccgtcct cttagttctc tggtgaatcc 2940
cttaagctct atgtcgaagt ggagcgagcc ggccctgacc acgcggggtc gtgctgctta 3000
agagtgcctg aggccttgct gcgaggactt gactccggag cggcggcgct ggttgggctc 3060
tccctgctaa ggagctccct accggtgtag cccccaaaca acagatggtc agagtgctct 3120
ggagtctgga tgagacaagc tcctagggtg tgtgtatatg tgttcgtgcg cgcgcgcgtg 3180
tgtgcaaaag atgctacctc tccctcctgt caggcctgac agaggtcgga ttcccaaagc 3240
ctggtgcttt ctccagtctt gaagttctgc catgagggga tccctcctga tataattttg 3300
gaaaggacag cataagggac tttagtctgc cagcccaccc tgtctgtccc cgctttaaca 3360
ttgctcagta ttttgggttt ctttaatatc gagagataac ttcccgggaa ggatcatttc 3420
cacctcttag aaagctgctt aaacatccct ccggaggttg gggggcgaag gaggccttga 3480
agacagagta gctgctcttt ttaaatctcc atttaggcac tgtctgctgc agcagagaat 3540
tctcaagaga attttgccct gttcctttgc tatagggatt gcttgcttgt cgtgcccttc 3600
ctccccccct tcccagccct taaagtgaga gtgttttgtc caccctaaga aacatcctca 3660
gtgggtccca gaggagcttt cttttttttt tttttggttc tttttttcgg agctggggac 3720
cgaacccagg gccttgtgct tcctaggtaa gcgctctacc actgagctaa atcccagccc 3780
ctgaggagct ttcttaaaaa gcagtcttca ttcattctcc tttcctctgg gacttgctcc 3840
tggggctctg tgagtgaatg cagtcctggg tcactcactg tagaaaatag ccctttcaac 3900
tcaaaagcct ccctttcttg gagagagaca gagagagaga gaatgtgagt gtgtgtgttc 3960
atatatgtac gtgcatagat gcatgcccat gttggattct taggcatggt ttcccatgaa 4020
gagtttgcag gttgttacca ggtagcattt tgatcagcag ctgagaaagg ctttgggggt 4080
gtgtgcaggg ggtctccgat cccagattcg gatgtccttt gtctcactgt ccctaacgac 4140
agaagtttcc atgagtttga gtgtgtgcac acttccctcg ggttttccag gctttttaga 4200
ttctgaaagg aattatgaga ccacaaggat ccattgctct agagagcagc gaatcgatga 4260
ctgaagtttt gattgatctc cactgaggcc tccctggatg gatgagctat actgctgaga 4320
agccaggccc ttcctccagt tagtctatga gccctttgtc tggcctaagc cacagtactc 4380
tggtggcaca tcttgttttg gtaaactggc aaattgttcc tgtatcgtct ccctgttatg 4440

ttgtctcctg acagaagaca tccttctgca taaccccgca ctaagatccg agacctaaga 4500
aaattgtgat ttccttcatt tgctaaggtt gtcctttttc cttcatcttt cacttagttg 4560
ggtcttgggt ctcttttggg aggtactacg tcctggggac tgttgtcagt ttcctgtcaa 4620
ctgtctttat gagtaggctt ggcctagggg cttcctttct cctgcagtgt gtgatcctgt 4680
ttgttgaagg tgtacgagtg gctggaagtg gtcagtgtcg cccccctccc ctttctgatt 4740
aaggtgccct ggcttcattc tgtttgggag gtgggtaggg gtgggtgtgc ccattcgatt 4800
tcaggaccag tttttctggt ctgagtcgag aaggttggga aatgtaagtg gagtgggtgg 4860
gggtggagga ctctggggca gtggggctgg ttgggatatc tcagtcagaa acaaatgaag 4920
gaggaattac aagccatccc aggagaagcc cctttccagc tacccacttt gaaccaggtc 4980
ccaagctgag tgtgcccatt gtggtgaatt agagacagaa ggtggcggcc cagccaggac 5040
tagcatctga agcctctcca gcgtggcctg ttgcagggag ttcagtgtgg ccctgtgaca 5100
cttggctttc ctgggctagg agagaacact agccttcctg gatgtgtcag cctgccagct 5160
ccttaactcc ccctccttac gtgtacttga ctttctctct tgaacaaatt caacagcttc 5220
cactttgtct ctttgggaga ggcaggggca acttgtctag atctaggcat gcacttggca 5280
taataatggc tttcgttctc catctcatct cagtgttctc cacaagcagc tctggggaga 5340
gagggtgtgt ctgcaaggta gtgggcagga aggatgaaag aatatgcctt agagattctc 5400
tggagtcgag gggggccagt cttgggagaa atggcctttc tgttgggttg cagtagctgt 5460
ttcctagaga ctcatttgga agagtgttag gacccaagtc ctaactggtg agtcaggctt 5520
cttgggcaag ttcattccct cccgctgaat ttttgattct tggtagaaga aatgggattg 5580
tgaatcttag tggacttgaa gcgctcacgt gagatttagt taatctcttc cagggctggg 5640
gagcaaaccc agggccttat gtgtgttggg ctagcactct accaatgagc ttcatttgca 5700
gctcctaaga ggagtttctc tacacgttac caaagacagc ttgtgccttc tccccagttc 5760
tggcttagtc gaggaatgac tattacagag tggtccccag agagacctct gttttgctta 5820
gagggttttg ctccacacta acagctaaga tgactcaatg ggtggatgag atgatccgta 5880
ggtcatagtt tcctgccctg ggactgctat gtacttggag gtgggagtct atcctagtag 5940
gaacgagctt tgaggtaact tcctttcctc cctcccatcc caccagggcc tgtactctat 6000
tagggcacag gggatatatg tgagtcagcc tgggagtggg aagaaacctg gggaggaaac 6060
aaggccagca gtgttctaac aggtcatagg acataaagag tagttcagtg tgtctgggcc 6120
caccttcgag aacagtattc acatttggat ctggctcttg gagttcctag cttccccttt 6180
gatctgaatt tttgctctgg actgaaggtg gttgtggcca gctactttgc ccagagggaa 6240
tagagacaag gttaagggga ataagagaca aggttaaggt taagccaaga ggaattgcac 6300
tttttttttg ttgttgttgt ttgttctccc ttccccttcc cctccctccc cctccttggc 6360
tcccttcttc ctttccttcc tctctttctt tctctttttg agaccagatc tcatatggcc 6420
taggctggcc ttgactctgt gatccttgcc cctctacctc ccaggtgagg ggaattatgg 6480

gaagacacta ctgaatctgg caccaccttc atcgttttta aaatttcttc acctccaggt 6540

ctaaatctgt gctgctgaca gtgaagctgc ttgggccaca ccttccatcc cctcctgtca 6600

gggctgcctg ccctttttct tcgttttgag aaaagtcaag atctagtttt attgtctggg 6660

ggcagctggg aatagatcca acctgttggg gggaagattg tgccttggcc tttcttccca 6720

aactgaccaa ccgttcccca tcgtcgtccc cttgcaggta aaccaggtcc ctctgcagcc 6780

atgtcagcca aggcaatttc agagcagacc ggcaaagaac tcctttacaa gtacatctgt 6840

accacctcag ccatccagaa ccggttcaag tatgcccggg ttactcccga cacagactgg 6900

gcccatctcc tgcaggacca cccctggctg cttagccagg taagccctta ttttcagctc 6960

aggctctgag gaggagaaag cctttgattt gtcggaggtg tcccaaggag ttccatgatt 7020

gcagattcct ctgtgtctgc agctggctga gcagggtctt gccttgactc tgggcccttc 7080

ctttggaacc agacagactg gtacccagat cacacctcca tttatgaacc gcgtggcctg 7140

gggcgaatcc atttcacttc ccttgagtta ggttccccac ctgtcaaata gggttgacac 7200

tttatgagct ataaggatta gcaggaacac ccaggaagca tttactgtgc tgtccagcag 7260

ttggtagaag ctcagttaaa aaaaaaaata tgcctggtgt ggtggtgcct ttaatcacaa 7320

cacttgggag gcagaggcag gaggatctct gtgagtttga gaccagcctg gtgtacaaag 7380

tgtccaggaa tattacacag agaaaccctg tcttggaaag cccccctcca ggctcctccc 7440

ccacccaaaa aaggcttata cctacctccc ttccccaaag tggaacatct caggacattt 7500

aagaagtggg accttcaggc tagagagatg gctcagtggt taagatcgag taatcttctt 7560

gtagaggacc tgggtttggc tctagtttcc acgttgcagc ttacaaccat gggtaactct 7620

ggttccaggc gagctgatgt cctctcctgc atacatgtag gcaaagaaca cctacacata 7680

agaatgaatg aatatgggtt cggtccccag ctccgaaaaa aaaagaacca aaaaaaaaaa 7740

aaaaaaaaag aatgaatgaa taaagaaata ggaccttgta ctttgtgaaa atggctgatg 7800

tctgagcatg gaggagatca gcctggagtt gacgatccca ccatagcatt tttggggtgg 7860

cttcgggaag ccctagactt ctttggcggg cctagacaag gccttatggt gtttcgtttt 7920

ttcctttctg aggcagctgg gatggtagtg aatttcctgt ctgcctgtcc cctgatggtc 7980

aagagatttg gctggggtag tcaaggaaag tttccggggc aggaaagtgg ccctagagga 8040

taaatgtcta gctagatctg gaagcggggt gatcaaagct atacaatttc actgctacat 8100

ccccgtttcc ttccccaact ccttccattc cagagcttgg tagtcaagcc ggaccagctg 8160

atcaaacgtc gaggaaagct tggtctagtc ggggtcaacc tctctctgga tggagtcaaa 8220

tcctggctga aacctcgact gggacatgag gccaccgtga gtcctggtgt ggggtgggga 8280

ggggtcaggg tggtaacaag cagcatggtg acctctagat caaccaacct atggctcata 8340

acctaaccag tcacatagca acagagaaaa agatggcgtg cacactgccc tggctgcagc 8400

ccagtcttgc cggtgagaag atcagtccac tgatagtgtt ttagataagg tgcactgcgg 8460

ttgtaagccc agaacagtta ggaagctaga agctgtgacc accagcatgg agatagggct 8520

cgtacttact ctgagaactg cctgctctcc agtttccaaa ttcagttgtg tgagtttggg 8580
atgacgtgcg taccaggagg atgggagtaa accgagatgg aaaggggagc cacatcatta 8640
ttaggcaaaa aagatacgct ggtgggagag ccatgggtgg agacagttac taatgctgaa 8700
gacttaattc agttctccca cgtccgcgat tcctctcagc tatgaggatg agagctgaga 8760
tgaaatgtgg cttaggaggg ttggggattt agctcagtgg tagagcgctt gcttagcaac 8820
tgcaaggccc tgggttcagt ccccagctcc gaaaaaaaaa aaaaagaaat gtggcttagg 8880
taggctaagc tgtgctctga actgtttctt ctctttcaca gtcttcaagg ggggtgggtc 8940
tgaatcttgg cctagcctag gaaggatgcg tttctatctg cttggcacag aatcccgcag 9000
atactggtgc ttgtctggga taggcatggt cagagaagag ctgttttttt ttttcctatg 9060
gctgccctgg gaggtggcag tacccatttt cctccatgtt gggcttcctc aaagcagctc 9120
ctactgtttg tttccggtcc ttgtgggtaa agacagatct ttgaagttgt gtccaggaat 9180
ggagagatct gacctctggg ttctcaggcc cctggggaga gagtggcagg gctgggtact 9240
gaacagtgcc cccagggagc aagcacatca tgctctcctc cctgcaaact gatgggaggg 9300
agatgtcacg gacatctgag cttggcagca gtgtcagacc tggctggagc ggctcattac 9360
ctctgatgga tcactgtctg gtccctgact cagcagtgat gcacggcagg cggggggcag 9420
gtggggggac tggggatgag gcctgtctcg cctttcctgc cgccagggac ccactcaagc 9480
tgattagctc tgcaggccgg ctgtcttcag ccaggaaaca caggaggaaa aagtttacct 9540
aatcagcctt gggctctctt ctctcccacc cctcaaggcc tagatgcatg tcctcaggct 9600
gggtcactat gtcttcctca gggcaaagta gtgtgccgtc accatcatac ccaagttccc 9660
atctcccttc ttgactgcct gggctaggga gattgatcag tgggcaaggt gcttgtcacc 9720
aggcctggtg acttgtgttt gatccttggg atccataagg tggagggaga gaaccatctt 9780
gagttaccct cacatcccca ctccttgaca tgacattcgt atgttcctgt cctcctcccc 9840
aaacataagt aaaaatattc acgcaaagag gggatcttct aagaactcct taagttcatc 9900
cgggacccca ttgtcctgac ttgtggacaa gtcactttgc cagatgtggt ggtagtgctt 9960
gggccttgcc ttcgtgaaga atgtaccaca catctaagcg gcatctccct caatttctcc 10020
ttaggtcggc aaggccaaag gcttcctcaa gaactttctg attgagccct tcgtccccca 10080
cagtcaggta tgtgaggcag tgccaaattg gatcttcttc ctccttcagc tttctgattg 10140
tcctccaaaa atgttgaaag gagaggttac ggtgccaagg ggagaagcca agggcacaag 10200
ccaagggcac cagggtctct aaagctccag ctgaggcctg gaggaaatta tgagcaagga 10260
gtccagagat gaaggaaggc ttggtttgca gcagagcagc tagagcaggt aacagggact 10320
tcctgttgtt ggggtcagga cttgagggca ggaccccttc ccaagatggg acccagttga 10380
ggaagttgag gcctgtcttt tgcttgtcgc ctaggagaaa gtagcacgca gatcgtgggg 10440
tgaatacttt gtgttaggat ttagaaatga gaccacaggg cttgggttcc actcctgggt 10500
gaagccctct gaccccttgc cagttgagca cccttaccat gctctctgca tgctctgtgt 10560

ccttgaaagg tggaggagtt ctacgtgtgc atctatgcta cccgggaagg agactacgtc 10620

ctgttccacc atgaaggggg tgtggatgtg ggcgatgtgg acaccaaagc ccagaagctg 10680

cttgtgggtg tggacgagaa actgaacgct gaagacatta agagacacct gttggtccac 10740

gcccccgaag acaagaaaga gttgagttct gagagggaca gtgtgggcac agccgggggg 10800

gggacaagaa gtccagtgtg acttcagggg acataggaga gagaaagcag agtggaacag 10860

ctggcgggca tagctctgtc ttgaggaagg tagccacttg tgtggggagg ctgctgcctt 10920

gcaatcagtc tgcagcatgc cagtctcatc cctcacctct tgcccctgtc tgtctgtctg 10980

gtgctccctc tgtctctgag cttgacagct tctttccacc tctgtgtgag gagtgacagc 11040

cagggagcag ggctggcaga ggacagtggg ggccaaaggg tcaggcgtga agtggggctg 11100

aactgtcaga cagtgagcct gctgtgtgta ggtgacgggg tgggaatggg agctgggtgc 11160

ctcgtcctgc actgactgat taactggcct tctttccctc tcccttctag aatcctggcc 11220

agcttcatct ccggcctatt caatttctac gaagatcttt acttcaccta ccttgagatc 11280

aacccccttg gtaacatgct ttgtttgctg ggacgggatg gggatggggt gctgccccca 11340

tgcctacagt aactctggcc ctggtgccag aaggcagtac aaaacttctg tgggtaccca 11400

gggtgtcccc ataagggtgg tctccagtgt cacctccggt ttctaagcac ccgggactgt 11460

aacttattca taccataacc aactgcagct tctttctctg gccctaattt gtcctgctct 11520

ggggaatcct ggctttggac tgtctatggt gttgtctctg cctgccagtt aggctcccca 11580

ggctctggtc tgatcctcct ggtacctgga ggcctctctc ttgggtattg gtcaatcatt 11640

tactacccct aacaccaaga agagctggct gtaggctctc aagctgtgaa ggaggcagat 11700

ggcctggcag aatctggcta ggcctctcag ggcaaagtcc atgaagaagg gaattaccaa 11760

atttcagctg agaatgcagc tcctctaatg ggccagatgg ctgtgtgcta acttaattag 11820

ctaaatggcg ggggacaaat atggatgctc aggtggagga attgcagatg aatgtgttgc 11880

cgggggccca gagcactctg gggtaccttg cacagacact gagacttcct cagctagacc 11940

acagaagccc gggatgtgca caccttctgg tagtagaggt ttatgaatgg cgtgtgatgc 12000

tgtgctgcta cttgggtttg aacccaaaac ctagaccaac agggctcttc gtcgttggtt 12060

tgggttttgc tgttgttttg agttagggcc aagtttagtc cacactgtcc tcaaacccac 12120

tcactatagc cgaggatgag cttgaacttc tgtcctgatt cccctgtgct tccgtacccg 12180

gcttccacag ttgttattta agttaatttt aaatggttaa agattatttt attgatgtct 12240

ttgtgaggat gtgtgcctat gttggccaga aaagtatgtc tgacggctgg agttacagtt 12300

ttgagctgcc tcacgtgggt tctggcactt gaactcatgt tctgtagagg agcagcaagc 12360

tctctttaac tatctctcat cccctagtta gtattttctt atatatttgt ttatttcccc 12420

ccctgtgtcg cgtgtgcgcg cgtgaagcag agaggacagc ctatgagagt ccggtctctg 12480

ctcctaccat atggcctgga ggcaaatacc tttctgcact gatcaatctc acagtctttt 12540

atttattctt taatatatta tttggtataa gggattgaac ctatggcctc tcccatgtac 12600

tttttttttt tttttttttt tggttctttt tttcggagct ggggaccgaa cccagggcct 12660

tgcgcttcct aggtaagcgc tctaccactg agctaaatcc ccagcccctc ccatgtacta 12720

ttaaccccag aaaacagggt gtttttgttt gttcgtttgt ttgtttgttt ttcatgtcag 12780

ggctatgggt aggccagtgc ttgagtattt aaagcaggga gaatggcatt cttttgaaga 12840

tgtcccaaaa cttgctatga taactaacag gtctcgtttg acctgttttc aggtcagtga 12900

gtggggactg ttctgacagg attttccttg tgctggcgac gtatcacaat acgtggttat 12960

tcggtgtgtg tgagagggca ctggcgggag tctggagggg tggttggaga gttggtgtct 13020

gacactgttt tacttttctc ctagtggtga ccaaagatgg tgtctacatc cttgacctgg 13080

cggccaaggt ggacgccact gctgactaca tctgcaaagt caagtggggt gatatagagt 13140

tccctccccc ctttgggcgt gaggcatacc cagaggtaag gcagctttgg gaaggagata 13200

gagggtgatg gggcctagga gacagtgact aatcctagat gatcatttgg tccccttttt 13260

ggctctgtat ttggcaggaa aggaaagaca gagagccatt taattttctt aaaaccccag 13320

tgaggttggg ggtcggggtg gaaatcagcc aaggtcctga atcctggcca gaaacccttt 13380

tagacagcgc tctcagtcag cctccccggg cttctgactt gatttgctgc tccttgatta 13440

ttccaagtgg ctcttcccaa ccctgatggg aaggaaggtt cttctcctgg ccactggctg 13500

agaattttaa ttgctgcttc ccatcccatc ctctgagagg tcctcccagc agg 13553

<210> 209

<211> 2638

<212> DNA

<213> Rattus norvegicus (Norway rat)

<400> 209
cacaatcttc aatgagtaga catattcctc agttctgtgg tgttctcggt cacacattta 60

tggagtttct gaagggcagt ggagactact gccaggcaca gcacgacctc tatgcagaca 120

agtgaactgt agaaattcat tactactcca ccaagaagcc cccataagag tggatagcct 180

ggacacagtc gtgttgaatt gaaatctgca gagcattttc caagagctca gacctggatg 240

gggtaaacct cagtgcactt cctctgtatc gcctcagtat tcctggattg aagagtcact 300

gcttcttgtg aggaggttca tttcattgcc cgtttctccc gactcatact caaagcactg 360

agaatttcaa gtggagtata ttgaatattg aagtagactt caggttgttt tttggttttg 420

ttttggtttt ttgttttgtt ttgttttgtt ttgttttggt tttcagtttt tgtttggaat 480

catttctgta ttcaattttt taattctttc ataaccctat tgggtgtttt tttaaactaa 540

attaacatgg ctcgaatgaa ccgccctgct cctgtggaag tcacatacaa gaacatgaga 600

tttcttatta cacacaatcc aaccaatgcg accttaaaca aatttataga ggaacttaag 660

aagtatggag ttaccacaat agtaagagta tgcgaagcaa cttacgacac tactcttgtg 720

gagaaagaag gcattcatgt tcttgactgg ccttttgatg atggtgcacc accatccaac 780
cagattgttg atgactggtt aagtcttgtg aagattaagt ttcgtgaaga acctggttgc 840
tgtattgctg tccattgtgt cgcaggcctt ggcagagctc cggtgcttgt tgccctagca 900
ttaattgaag gtggaatgaa atatgaagat gcagtacaat tcataagaca aaagcggcgc 960
ggagctttta acagcaagca acttctgtac ctggagaagt accgtcctaa aatgcggctc 1020
cgcttcaagg attccaacgg tcatagaaac aactgttgta ttcaataaaa ctggggtgcc 1080
tgatgccatt gccttggaag aggaacttca gatgggacct gatttgttat ttacccaatg 1140
tgtccactta cctgtggaag ctccagggga atattgaaaa agttttacca ggccacaagc 1200
ttgacagaat tgcaacctct ataattgggc tatgatcaac acgtttggac acttagcaaa 1260
agatttttgc tggtcagcat ttaaaatgtg cttattattt gtaccaattg acctttccta 1320
aaataaggta ttgagtaatg tcattaaatg tactcctgtg ccagaatatt attagtctat 1380
aaggaattta gaaggattag gtgccaaaat acccagcaca atacttgtat atttttagca 1440
tcatacagaa ccaaaattcc aagaactaag aactctccag accttccatg gtgtattcct 1500
tcagtcattt caaacaccgc agggcttctc ttgttatctg cctgctcact ctatgtttac 1560
atctcccaca cttacaccag aacacatcag gtttgcttag ctatctttta agtcttgcaa 1620
tgattattta atgtctctgt cttattttgt gctgttttgg gaaacctcca tttgaaaatc 1680
aactttgtta cagaagcaca tatcttcaat aatgtctcca gacaaaaagc cttatagtta 1740
atttaatgtt tgcactcggg tgcaacctga cagggagggc ctgaacaaga aaggagagga 1800
ggctattaaa tatttttagt aatatgttgc ctttgtcttg tgcagaacat gtagagtatg 1860
ctctttaatt tagtaaatat ttttaagacg tagagataca ttgttgtagc taaccactta 1920
atcaaaattt ctgaaattct tgtgttttcc atacctatct gaggttttcc aacttgtttg 1980
aattatggtt ttccccttct cttcccaatc tcttgcaaaa aagtaaaagt gggatctgct 2040
agtgaactga gcagaaatat tttatacgcc ttttgagcta tgtaacttaa taattggata 2100
cttgatcatt tgttttatta tgtaatcgat aaaatggtga tgtgtattaa tgttagttca 2160
accatatatt tatactgtct gggaatgtgt ggttatagtt ctgtgggaga aatagtttgt 2220
cagtgttcac cagcttgtaa aaacttagtg cgagagcttc aacatctaaa taaatgatga 2280
aacgcattcg tcactgaggt cactttgctt aaaattaact taatttgtag aaaacagtgg 2340
attcaattat tatcatttca gtttatggac aaatttgtta gggttaccaa gtgcgtttaa 2400
aaattgctct ttaaaggtct agataattgt gaatcaattg aatgttgggt accaagggaa 2460
aacggtttgt aatagttgat gaccttgatt tttaattcaa ttccaccagt cacttgtagc 2520
tttatgcagt ttccaatcca cttttctcat ttttaagttt attacttacc tgtatatatt 2580
ttgaaattaa tttgaacctg cgtatttggc acatgatggc ttataaattt taactttc 2638

<210> 210

<211>  711

<212>  DNA

<213>  Rattus norvegicus (Norway rat)

<400>  210
gggactcgca cttgcaatat gactttggag gaattctcgg ccgcagagca gaagatcgaa      60

aggatggaca cggtgggcga tgccctggag gaagtgctca gcaaggctcg gagtcagcgc     120

accataactg tcggcgtgta cgaggcagcc aagctgctca acgtagaccc ggacaacgtg     180

gtcctgtgcc tgctggctgc ggatgaagat gacgaccggg acgtggctct gcagatccat     240

ttcaccctca ttcgtgcttt ctgttgcgag aacgacatca acatcctgcg ggtcagcaac     300

ccgggtcggc tggcagagct gttgctactg gagaacgaca agagccccgc tgagagcggg     360

ggcctggcgc agaccccgga cttacactgt gtgctggtga cgaacccaca ttcatcacaa     420

tggaaggatc ctgccttaag tcaacttatt tgtttttgcc gggaaagtcg ctacatggat     480

cagtgggtgc cagtgattaa tctccccgaa cggtgattcc ccgaacggtg atggcatctg     540

aatggaaata actgaaccaa attgcactga agttttgaaa tacctttgta gttactcaag     600

cagtcactcc ccacgctgat gcaaggatta cagaaactga tgtcaagggg ctgagttcaa     660

ctacaggagg gctaggagat gactttgcag atggacagag aggtgaaaat a              711

<210>  211

<211>  421

<212>  DNA

<213>  Rattus norvegicus (rat)

<400>  211
ggcgatgccc tggaggaagt gctcagcaag gctcggagtc agcgcaccat aactgtcggc      60

gtgtacgagg cagccaagct gctcaacgta gacccggaca acgtggtcct gtgcctgctg     120

gctgcggatg aagatgacga ccgggacgtg gctctgcaga tccatttcac cctcattcgt     180

gctttctgtt gcgagaacga catcaacatc ctgcgggtca gcaacccggg tcggctggca     240

gagctgttgc tactggagaa cgacaagagc cccgctgaga gcgggggcct ggcgcagacc     300

ccggacttac actgtgtgct ggtgacgaac ccacattcat cacaatggaa ggatcctgcc     360

ttaagtcaac ttatttgttt ttgccgggaa agtcgctaca tggatcagtg ggtgccagtg     420

a                                                                     421

<210>  212

<211>  1215

<212>  DNA

<213>  Rattus norvegicus (Norway rat)

<400>  212
ggcacgagag aaagcgactc ttctagggaa gagcagcatc tgagaatgaa ttccaaaatt      60

cagaagatgg agttaaacga tggtcactcc atccctgtac tgggctttgg cacctatgca     120

accgaagaga atctcagaaa aaagtctatg gagtccacga aaatagctat agatgttggg     180

ttccgccata ttgattgttc tcacttgtac cagaatgaag aagagatagg tcaggccatt     240

gtaagcaaga ttgaagatgg cactgtgaaa agggaagata tattctatac ttcaaagctt     300

tggtcaactt cccatcgtcc agagttggtc agacccagct tggaaaattc actgaggaaa     360

cttaatttgg actatgtaga cctctatctc attcatttcc cggtatctct gaagccaggg     420

gatgagcttt tacctcaaga tgagcatgga aacttaatac ttgacacagt ggatctctgc     480

gacacctggg aggccatgga gaagtgtaag gatgcaggat tggccaagtc catcggggtg     540

tccaacttta accgcaggca gctggagaag atcctgaata agccggggct caagcacagg     600

cctgtgtgca accaggtaga atgccatctt tatctcaacc agagcaagct gctcgcttac     660

tgcaagatga atgacatcgt tctggttgcc tatggtgccc tgggaactca gagatacaaa     720

tactgtatta atgaagatac cccagttctc ttggatgatc ccattctttg taccatggca     780

aagaagtaca agcggactcc agccctgatt gcccttcgct accagctgga gcgtgggatt     840

gtgaccctag tcaagagttt caatgaggag agaatcagag agaacctgca ggtctttgat     900

ttccagttgg cttcagatga catggaaatt ttagataacc tggacagaaa tcttcggtac     960

tttcctgcta atatgtttaa ggctcaccct aactttccat tctctgatga atactaagat    1020

ggcagcccta gccatgagtt ctgctcgaag ctcctttgtg tgatgctcga ctctcagagg    1080

ccaataatac aacacactga ctccaatcca tactgcttag caactcaccc ccagtttgag    1140

ctgtgtctgt acatcgggga gcaaattcac taaattttcc tgcttttctg taaataaata    1200

aaaatatttt gcttc                                                     1215

<210>  213

<211>  15438

<212>  DNA

<213>  Rattus norvegicus (Norway rat)

<400>  213
aggagccagc gggtagtgct gaacaagact cctgttccgg agcggagtaa cctgcctgtg      60

cccgctgcga cgcgcgccca cctcccgggg aaggaacggc gaggcccggg gccgtctccg     120

cgatggagcg cggggccgca gcggcggcgt ggatgctgtt gctggcgatc gctgcctgcc     180

tggagccagt cagtagccaa gaatgcggca gtgggaattt tcgctgtgac aatggatact     240

gtatccctgc gtcctggagg tgtgatggga ccagagactg tttggatgac acagatgaaa     300

ttggctgtcc tcccaggtcc tgcgaatcag gccttttcct gtgtcctgct gaggggacct 360
gcatccctag ctcctgggtg tgtgacgagg ataaggattg ttcggacggt gcagacgaac 420
agcaaaattg tgctgggacc acgtgctcgg ctcagcagat gacgtgctcc aacggtcagt 480
gtattccgag cgaatacagg tgcgaccatg tcagcgactg ccccgacggc tctgacgaga 540
gaaactgcca ttatccaaca tgtgatcaac ttacttgtgc caatggagcc tgctataaca 600
cgagtcagag gtgtgaccaa aaagttgact gcagggattc ctcggacgaa gctaactgca 660
ctaccctgtg ttcgcaaaaa gaatttgaat gtggcagtgg agagtgtatc cttcgcgcct 720
acgtctgtga ccatgacaat gactgcgaag ataacagcga tgagcgcaat tgcaactatg 780
acacctgcgg aggtcaccag ttcacttgct ccaatggcca gtgcatcaac cagaactggg 840
tttgtgatgg agatgacgac tgccaagatt ctggtgatga agatggatgt gaaagtaacc 900
agagtcatca taggtgttac ccaagagaat gggcgtgtcc tgggtctgga cgatgcatct 960
caattgataa agtttgcgat ggagttcctg actgtccaga aggagacgat gagaacaacg 1020
tcacaagcgg aagaacctgt ggtatgggtg tctgctctgt cttaaactgt gagtaccagt 1080
gccatcagac gccatttgga ggagaatgct tctgcccccc aggtcatatt atcaacagca 1140
atgacagccg tacttgcatt gatttcgacg actgccagat atggggaatt tgtgaccaga 1200
agtgtgaaaa ccgacagggc cgtcatcagt gcctctgtga agaagggtat atcctggagc 1260
gagggcagca ttgcaaatcc agtgactcct tcagtgcagc ctctgttatc ttctccaatg 1320
gtcgggattt gctagttgga gatcttcatg gaagaaactt ccggattcta gcagagtcta 1380
agaaccgagg aatggttatg ggagtggatt ttcactatca gaaacacaga gtttttggga 1440
cagaccccat gcaggaaaag gtgttttcca ctgacatcaa tggcttaaat acccaagaaa 1500
ttcttaatgt ttctgttgac actccagaga atctggctgt ggattggatt aataataaac 1560
tttatctggt agagacaaag gtcaaccgca ttgacgtggt caacttggag ggaaatcagc 1620
gtgttactct cataactgaa aacttagggc atcctagagg aattgccctt gatccaactg 1680
tgggctattt gtttttctca gattggggaa gcctctctgg gcaacccaag gtggagagag 1740
ccttcatgga tggtagcaat cggaaagacc tggtaacaac caaagtgggg tggcctgctg 1800
ggatcactct ggatctggtg tccaagcgag tttactgggt ggattctcgt tatgactaca 1860
ttgaaactgt aacatatgac ggaattcaaa ggaagacggt agctcgtgga ggctcccttg 1920
ttccgcatcc ctttggaata agcttatttg aggaacacgt gttctttact gattggacaa 1980
agatggctgt gatgaaagcc agcaagttca cagaaaccaa ccctcaagtg taccaccagt 2040
cttccctgag gcctcacgga gtgacggttt accatgccct caggcaaccc aatgccacca 2100
atccgtgcgg gagcaacaat gggggctgtg cgcaggtctg tgtcctcagt cacaggacgg 2160
ataatggtgg cctgggctac cggtgcaagt gtgaattcgg ctttgaactg gatgatgatg 2220
agcaccgctg tgttgccgtt aagaacttcc tgctcttctc atcgaaaacg gctgtgcgtg 2280
gaatcccatt caccctctcc acccaggaag atgtcatggt tcctgtcaca gggagtccat 2340

ccttcttcgt tggaattgat tttgatgccc aacacagtac tgtcttctat tcagatttgt 2400
caaaagacat aatttataaa caaaaaattg atggcacagg aaaagaggtt atcacagcaa 2460
accggttgga aagtgttgag tgtttgactt ttgactggat ctcaaggaat ctttactgga 2520
cagatggagg actaaagagt gtcactgtcc tgaggctggc tgataagtcc cgacgacaga 2580
taatcagcaa cttaaacaac cctcgttcca ttgtggtgca tccgaccgct gggtatatgt 2640
tcctgagtga ttggttccgt cctgcaaaaa ttatgagagc atggagcgat ggttctcacc 2700
taatgcccat agtcaatact agtctggggt ggcccaatgg cttggccatc gattggagtg 2760
cttcacgatt gtactgggta gatgcctttt ttgataagat tgaacacagc acccttgatg 2820
gtttagatag aaaaaggctg ggccatgtag accagatgac tcatccattt ggactcaccg 2880
tttttaaaga taatgtgttc ataacagact ggagactggg tgctattatt cgagtgagga 2940
aatcagatgg tggtgatatg acagttattc gaagaggcat cagcagcgta atgcacgtga 3000
aagcctacga tgctgacctc cagactgggt ctaactactg cagtcagacc acccacgcca 3060
acggtgactg cagccacttc tgcttcccgg tcccgaactt ccagcgggtg tgcggctgtc 3120
cctatggaat gaagcttcag agggatcaaa tgacttgtga gggagaccca gcccgagagc 3180
cacccacaca gcagtgtggc tccctctcct ttccctgcaa caatggcaag tgtgtgccca 3240
gtttcttccg ctgtgatgga gtggacgatt gccatgacaa cagtgatgag catcagtgcg 3300
gggtgtttaa taatacttgc tcaccttcgg ctttcgcctg cgtccgcggt ggacagtgca 3360
tccctggcca gtggcactgt gacagacaga atgactgttt agacggcagt gatgagcaaa 3420
actgccccac acatgccacg tcgtccactt gcccgtccac ctccttcacc tgcgacaatc 3480
acgtgtgcat cccaaaagac tgggtctgtg acacagacaa tgattgctcg gatggctcgg 3540
atgaaaagaa ctgccaagct tcagggacct gccagcctac acagtttcgg tgccctgacc 3600
accgatgcat cagcccgctg tatgtctgtg atggggacaa ggactgcgcg gatgggtctg 3660
atgaggcggg ctgtgtgtta aactgtacga gtgcccagtt caaatgtgcc gatgggagtt 3720
cttgcattaa cagcaggtac cgctgcgatg gggtttacga ctgcagggac aactctgacg 3780
aggcaggctg ccccaccagg cctcccggca tgtgccaccc ggatgagttc cagtgccaag 3840
gagacggtac atgcatccct aacacctggg agtgtgacgg gcatccagac tgtatccacg 3900
ggtccgacga gcacactggc tgtgttccta agacctgctc gccgactcat ttcctctgtg 3960
acaatggaaa ctgcatctac aaagcgtgga tctgtgatgg ggacaatgat tgtagggata 4020
tgagtgatga gaaggactgt cctacccagc cttttcactg tcctagcacg cagtggcagt 4080
gcccgggcta cagcacctgt atcaatctga gtgccctgtg tgacggcgtc tttgactgtc 4140
cgaatgggac tgacgagtcc ccactttgca atcaagacag ctgctcccat tttaatggtg 4200
gctgtactca tcagtgcatg caagggccct tcggagccac atgcctatgc ccattaggat 4260
accaacttgc caatgatacc aagacctgtg aagatatcaa tgagtgcgat attccaggct 4320
tctgcagcca gcactgcgtc aacatgagag gttccttccg gtgcgcttgt gatccagaat 4380

atacgctgga aagtgatggg cggacttgca aagtcacagg atctgaaaat ccgttgttag 4440
ttgtagcaag tcgtgacaaa atcattgtgg acaacatcac tgcccacacg cacaatctct 4500
actcgttggt ccaggatgtt tcttttgtgg ttgctcttga ttttgattca gtcactggtc 4560
gtgtcttctg gagtgactta ctgcagggta aaacctggag tgtctttcaa aacggaacag 4620
acaagagagt ggtccatgac agtggcctct ctgtgacaga aatgattgca gtagattgga 4680
ttggtcgcaa cctttactgg acggactatg ctcttgaaac aatcgaagtt tctaaaattg 4740
atggaagtca cagaacagta ctgatcagca aaaatgtcac aaaaccgagg ggactcgcgt 4800
tagatcccag aatgggtgac aatgtaatgt tttggtctga ctggggccat caccctcgca 4860
ttgagcgagc cagcatggat ggcaccatgc gcacagtcat tgtccaggaa aagatctact 4920
ggccctgtgg cttaagcatc gactacccca accgcctcat ctacttcatg gatgcctatc 4980
ttgactacat tgaattctgt gattacgacg gacataaccg aaggcaggtc attgctagcg 5040
atttggttct ccaccatccg cacgccctca ctctgttcga agactttgtg tactggactg 5100
accgtggtac tcggcaggtt atgcaggcca acaagtggca tggggggaac cagtcagttg 5160
taatgtacag cgttcaccaa ccgcttggaa taactgccat tcatccttca aggcaaccac 5220
cttcccggaa tccatgtgcc tctgcctctt gcagccatct ttgcctgctt tctgcacagg 5280
cgcctagaca ttattcttgt gcttgccctt caggatggaa cctctccgat gattctgtca 5340
actgtgtgag aggtgaccaa cctttcttga tgtccgtaag agacaatata atttttggaa 5400
tctcccttga tcctgaggtg aagagcaatg atgccatggt ccccatatca gggatacagc 5460
atggttatga tgttgaattt gatgactctg agcaattcat ctactgggtt gagaatccgg 5520
gtgaaattca cagagtgaag actgatggca gcaacaggac agtgtttgct cctctgtccc 5580
tgctggggtc ttctttaggc ctggccttgg actgggtatc aaggaacatt tattatacca 5640
ctcctgcaag tcggtccatt gaggtgttga cgctcaaggg agatacgagg tatggaaaaa 5700
ccctgattgc caatgacggc acccccctcg gagttggttt ccccgttggc atagctgttg 5760
atcctgcacg tgggaaacta tattggtcag accacgggac ggacagtggg gttcctgcca 5820
agatcgcaag tgctaatatg gatggaacat ctttaaaaat tctcttcact gggaacctgc 5880
agcatctgga ggttgtcacc ctggacattc aggagcagaa gctgtactgg gcagtcacca 5940
gcagaggcgt gattgagaga ggaaatgtgg acggtacaga gaggatgatc ctggtgcacc 6000
atctggccca cccatggggc cttgtggtct atggctcctt cctttactac tctgatgaac 6060
agtatgaggt cattgagaga gttgacaagt cctctgggaa taacaaagtc gtcctcagag 6120
ataacgttcc gtacctgagg ggtcttcggg tttaccaccg ccgcaatgcc gctgactctt 6180
ccaatggctg tagcaacaac cccaacgctt gccagcagat ctgcctgccc gtgccaggtg 6240
gaatgttctc ctgcgcctgt gccagtggct ttaagctcag ccctgacggt cgctcctgtt 6300
ctccatataa ctcgttcatg gtcgtttcca tgctgcccgc agttagaggc tttagcttgg 6360
agttgtctga tcactcggag gccatggtgc ccgtagctgg ccaaggacga aatgttctgc 6420

atgcggacgt ggatgttgcc aacggcttta tttactggtg tgattttagc agctctgtaa 6480

gatcctctaa tggaattcgt agaatcaaac ccgacgggtc taattttacc aacgtcgtta 6540

cctatggcat tggggcaaat ggaatccggg gtgttgcact ggactgggca gcaggaaatc 6600

tttattttac caatgccttt gtgtacgaaa ccctgataga agtgttacgg atcaatacca 6660

cgtaccgccg tgtactcctc aaagtctcag tggatatgcc ccggcatatt attgttgacc 6720

ccaagcatag atatctcttc tgggccgact atgggcagaa gccaaagatc gaacgctctt 6780

ttcttgactg taccaatcga actgtccttg tttccgaagg cattgtcacc cctcggggct 6840

tggccatgga ccatgacact ggctatattt attgggttga tgactcctta gatttaattg 6900

ccaggatcca tcttgatgga ggagaatctc aggtggttcg ctatggcagc cgttacccaa 6960

ctccctatgg catcactgtg tttggagagt ctatcatttg ggtggataga aacttgaaaa 7020

aagtcttcca agccagcaag caaccaggga atacagatcc acccgtagtg ataagagata 7080

agatcaacct gctccgtgac gtgaccatct ttgacgagca cgcccagccc ctctcaccag 7140

cagagcttaa caataaccct tgtctgcaaa gcaatggtgg gtgttcccac ttttgctttg 7200

ccttacctga gttgcctact cccagatgtg gatgtgcttt tgggactctg ggaaatgatg 7260

gcaagagctg tgctacctcc caagaagatt tcctcatcta ttccttaaat aattccctga 7320

gaagtttaca ctttgaccct agagaccata gcctaccttt ccaagtgata agtgtggcgg 7380

gaactgccat tgcactggat tatgaccgta gaaacaatag aatcttcttc acacaaaagc 7440

taaactctct ccgtggccag atttcctatg tcagcctgta ttcagggagc agctctccaa 7500

ctgtcctgct gtcaaatata ggggtgactg atggcattgc ctttgattgg ataaatagaa 7560

gaatttatta cagtgacttt tcaaatcaga caataaattc catggctgaa gatggatcca 7620

atcgtgctgt gattgcccgt gtttccaaac ccagagctat tgtattagat ccttgccgag 7680

ggtacatgta ttggactgac tggggtacca acgccaaaat cgagagagcc acactgggag 7740

gaaacttccg ggtccccatt gtgaacacca gcttggtttg gcccaatgga ctcgctttgg 7800

accttgagac tgaccttctg tactgggcag atgctagctt gcagaaaata gaacgcagta 7860

ctctcacggg cacgaaccga gaagtcgttg tcagcactgc ctttcattcc tttggcttga 7920

ctgtctatgg ccagtacatt tactggacag acttgtacac cagaaaaatc taccgggcta 7980

acaagtacga cgggtcagac ctggtagcaa tgaccacacg tttgcccacc cagcctagtg 8040

gtatttccac agtggtcaaa acccagcggc agcagtgtag caatccttgt gaccagttca 8100

acggaggatg cagccatatc tgtgctccag gtccaaatgg agctgagtgt cagtgtcctc 8160

acgaaggcaa ctggtatctg gccaacgata acaagtactg cgttgtggac acggggaccc 8220

gctgtaatca actccagttc acctgcctca atgggcattg tattaatcag gattggaaat 8280

gtgacaatga caacgactgt ggtgatggca gtgatgagct tcctacagtc tgtgcctttc 8340

acacctgccg atcaacagcc ttcacttgtg gcaacgggcg atgtgtgcca taccactatc 8400

gctgtgatta ctacaatgac tgcggggaca acagcgacga ggcaggttgc ctgttcagaa 8460

actgcaacag caccacagag ttcacgtgca gcaatgggcg gtgcataccg ctcagttatg 8520
tctgcaatgg cataaacaat tgccatgaca atgacacctc ggatgagaaa aactgccctc 8580
cccacacttg cccgccggat ttcacaaaat gccagaccac gaatatttgt gttccccgag 8640
ctttcttgtg tgatggagac aatgactgtg gagacgggag tgacgagaac cccatttact 8700
gcgcctcaca cacatgccgc agcaacgagt tccagtgcct ctcccctcag cggtgtattc 8760
caagctattg gttctgtgac ggtgaagccg actgtgcaga tggctcggat gaacctgaca 8820
cttgtgggca ttctgtgaac acctgcaggg ccagtcagtt ccagtgtgat aatggcaggt 8880
gcatctcagg caattgggtc tgtgatggtg ataacgactg tggggacatg agtgacgagg 8940
accagcggca tcactgtgag cttcagaact gttccagcac tcagtttacc tgtgtcaaca 9000
gcagacctcc caacaggagg tgcatcccac agtactgggt ctgtgacggt gatgcggact 9060
gctcggacgc cctggacgag ctgcagaatt gcaccatgag aacctgctct gcaggggagt 9120
tcagctgtgc aaatggacga tgtgtcaggc agtcattcag gtgtgatcgg cgcaatgact 9180
gtggagacta cagtgatgag aggggctgct cgtacccacc ctgccatgca aatcaattta 9240
cgtgtcagaa cgggcggtgt atccctaggt tctttgtttg tgatgaggac aatgactgtg 9300
gagatggttc tgatgagcag gagcacctgt gtcacacccc agaacccaca tgcccccttc 9360
atcaattccg gtgtgacaac gggcactgca tcgagatggg gcgagtctgt aaccatgtgg 9420
acgactgctc agacaacagc gatgagaaag gctgtggcat taacgagtgc ctagattctt 9480
caatcagtcg ctgtgaccac aactgtacag ataccataac tagtttctac tgttcctgtc 9540
tccctggcta caagctcatg tctgacaaac gctcttgcgt ggatattgac gaatgcaagg 9600
agtcacccca gctctgtagc cagaagtgtg agaatgtggt aggctcctat atttgcaagt 9660
gtgcccctgg ctacatccgt gagccagatg ggaagagctg ccggcagaat agtaacatcg 9720
agccctatct catctttagc aaccgttact atataagaaa cttaactaca gatggctcat 9780
cttactccct gattttgcaa ggattgggca atgttgtggc cctggacttt gatagagtag 9840
aaaagaggct gtattggatt gatgcagaga agcaaatcat tgagaggatg tttttgaata 9900
agacaaaccg ggagaccatc ataaaccaca ggttaagacg tgctgaaagt ctggctgtag 9960
actgggtcag cagaaaactc tattggttgg atgccatctt ggactgcctg tttgtctctg 10020
accttgaagg tcgacaccgc aaaatgatag cccagcactg tgtggatgct aacaatacct 10080
tctgctttga acatcccagg ggaattgtcc ttcatcctca aagagggcat gtctactggg 10140
cagattgggg tgttcacgca tacattggaa ggattggcat ggatggaacc aataagtctg 10200
tgatcatctc taccaagata gaatggccca atgccatcac cattgactac accaacgatc 10260
tcttgtactg ggcagatgct cacctgggtt atatcgagtt ctctgatttg gaaggtcacc 10320
accgacacac ggtgtacgat ggatcactgc ctcacccctt tgctcttacg atttttgaag 10380
acactgtttt ttggacagat tggaatacac ggacggttga aaagggaaac aaatatgatg 10440
ggtcaggtcg agtggtgctg gtgaacacaa cgcacaaacc cttcgacatc cacgtgtacc 10500

atccataccg gcagccgatt atgagcaatc cctgtggtac caacaacggt ggctgttctc 10560
atctgtgcct catcaaagcg ggggggagag gattcacctg cgcgtgtccc gatgacttcc 10620
agaccgttca gcttcgagac agaacactct gcatgcccat gtgctccagc acgcagttcc 10680
tgtgtggcaa caatgaaaag tgtatcccca tatggtggaa atgtgatggc cagaaagact 10740
gctctgatgg ttctgatgaa ccagacctgt gcccacaccg cttctgccgt ctggggcagt 10800
tccagtgccg ggatggcaac tgcacgagcc cccaggctct gtgcaatgct cgccaggatt 10860
gtgctgatgg ctctgatgaa gaccgtgttc tctgtgagca tcaccggtgt gagtccaacg 10920
agtggcagtg cgccaacaag cgttgcatcc cacagtcctg gcagtgtgac tcagtgaacg 10980
actgcctgga taactcagat gaggacactt cgcactgcgc cagcaggacc tgcaggcccg 11040
gccagttcaa gtgtaacaat ggtcgctgta tcccgcagag ctggaagtgt gacgtagaca 11100
atgattgtgg agactactcc gatgagccca tcgacgagtg cacgaccgct gcttacaact 11160
gtgacaacca cacagaattc agctgcaaga ctaactaccg ttgcatcccg cagtgggcgg 11220
tgtgcaatgg ttttgatgac tgcagggaca acagtgatga acaaggctgt gaatcggtgc 11280
catgccatcc ttctggggat ttccggtgtg cgaatcacca ctgcatccct ctccgctgga 11340
agtgtgacgg cactgacgac tgtggagata actcggatga ggaaaactgc gttcctcggg 11400
agtgctcaga gagcgagttt cgatgtgctg atcagcaatg catcccctct cgatgggtct 11460
gtgaccaaga aaatgactgt ggagacaact cggatgaacg ggactgtgag atgaagacct 11520
gccatcctga acattttcag tgtacgagtg gacactgtgt gcccaaggcg ttggcatgcg 11580
atgggagggc agactgtctg gatgcgtctg atgaatctgc ttgtcctact cgctttccca 11640
acggcaccta ctgcccagcc gccatgttcg aatgtaaaaa ccacgtgtgc atccagtcgt 11700
tttggatatg tgatggggaa aatgactgtg tcgacggttc cgatgaggag atccacctgt 11760
gcttcaacat tccgtgtgaa tcaccacagc gattccggtg tgacaacagc cgctgtgttt 11820
atggccatca gctgtgcaat ggtgtggatg actgcggaga tggaagtgac gagaaagaag 11880
agcactgtag aaaaccaacc cacaaaccgt gcacagacac tgaatataag tgtagcaatg 11940
ggaactgcat ttcgcagcac tacgtgtgtg ataatgtgaa tgactgcgga gacctttctg 12000
atgaaactgg ttgcaatcta ggagataaca gaacgtgtgc tgaaaacata tgtgaacaga 12060
actgtaccca gctgagcagc ggaggcttta tctgctcctg cagacccggg ttcaaaccta 12120
gtacttcgga caaaaattcc tgtcaagaca tcaatgaatg tgaggagttt ggcatctgcc 12180
cccagagctg tcgaaacagc aaaggaagtt atgaatgttt ctgtgtcgat ggcttcaagt 12240
ctatgagtac ccattatgga gaacggtgtg cagctgatgg aagccctcct ctcttgctcc 12300
tgcctgagaa tgtccgaatc cggaagtaca atacctcctc tgagaagttc tcagagtatc 12360
tggaagagga ggagcatatc caaactattg actatgactg ggatcccgag cacataggcc 12420
tcagtgttgt ctattacact gtgctggcac agggctctca attcggtgct atcaaacgtg 12480
cctacattcc caactttgaa tctggtagta acaatcccat acgtgaagtt gacctgggct 12540

tgaaatattt aatgcagcca gatggattag ccgtggactg ggtcggaagg catatttact 12600
ggtcagatgc taagagccaa cggattgagg tggctaccct cgatggaagg taccggaagt 12660
ggctgatcac cacccaactg gatcagccag ctgccattgc tgtgaatcct aagctagggc 12720
ttatgttctg gactgaccag ggaaaacagc ccaaaatcga gtctgcctgg atgaacgggg 12780
aacatcgcag cgttctggtt tctgagaacc ttggctggcc aaacggtctt tccatagatt 12840
acctgaatga tgaccgggtc tactggagcg actccaaaga agatgtcatt gaagccataa 12900
aatatgatgg gactgacagg agacttatca taaatgaagc gatgaagccc ttcagtctgg 12960
acatcttcga agacaagcta tactgggtag ctaaggaaaa gggagaagtg tggagacaaa 13020
ataaattcgg gaaagagaac aaagagaaag tgctggtggt gaacccgtgg ctcactcaag 13080
ttcgaatctt ccatcaactg agatacaatc agtcagtgtc caacccttgc aagcaggtct 13140
gtagtcacct ctgcctgctg agaccaggag gctacagctg tgcctgtccc caaggctccg 13200
acttcgtaac tggcagcact gtccagtgtg atgcagccag tgaacttccc gtcaccatgc 13260
ctcccccatg caggtgcatg cacggaggaa attgctattt tgatgagaat gaactcccca 13320
aatgcaagtg ttccagtggc tatagcggag aatactgtga ggttgggctc tcgagaggca 13380
tccctccagg gacgacaatg gctgttctgt tgaccttcgt gatagtcatc attgttggag 13440
ctctggtgct tgttggactc tttcactaca ggaaaactgg ctccctctta cccactctgc 13500
ccaagctgcc aagcctaagc agccttgcca aaccctctga aaatggaaat ggggtgacgt 13560
tcaggtcggg ggctgatgtg aacatggaca tcggtgtgtc tcctttcggc cctgagacaa 13620
ttattgacag atccatggcg atgaatgaac actttgtcat ggaggtgggc aagcagcctg 13680
tgatatttga aaacccaatg tatgcagcca aggacaacac ttccaaagtg gccctggcag 13740
ttcaggggcc atcaacaggc gcacaggtga ctgtaccaga aaatgtggaa aaccagaatt 13800
atggaaggcc catagatcct tctgagatag ttccagagcc aaagccagcc tcccctggag 13860
ctgatgaaat tcagggcaaa aaatggaaca tcttcaaacg aaaacccaaa cagacaacga 13920
actttgagaa cccaatctac gcagagatgg acagtgaggt aaaggatgct gtggctgtgg 13980
ccccccctcc gtctccttct ctccctgcca aagcttcaaa aagaaatttg actccaggct 14040
acactgccac agaagacacc tttaaagaca ctgcaaatct cgttaaagaa gattccgacg 14100
tatagcctcg ccagctctct agggaacatt tagacactca cttttgcaca tatatttttt 14160
acagacaaat gaaaaagaag ttaacattca atactttata aaaaaaatat atttttctct 14220
gtttgcctat aattgaaggt gtgtcgtgtg tcttttttac tgatgccact tcatattttt 14280
acaattatca cgggcactgt gtatatcttg gactgatgtt gtctggaggt gatgctgtga 14340
acattactgg acttgtgtaa actttggaag gattatcaca cctctgcccc tgctaacacg 14400
aaggtgcccg ttgagtgggt cgctgagctg aagaatacac gatctggtga ggaaaggaat 14460
tggcctaggg ctttggatct gtctagagag gagataacac tcatatttcc tttgaaacaa 14520
tacaggaatt ctttatgctc atttgagggc ccatttttac acattggcac ttaattaata 14580

gtcaacgttc aacatcacgt gtcaacctga ttagtgagtg tgcagatagg ggccagtgtt 14640

ggcttcatgc gctgtagatg gctcagacaa cacagacggt acacagggtg actcagctgg 14700

acccttctgc gctcctccgc cttctgaggc cagctatgtt ttgattgtgc ttcgtcccta 14760

gttgccaagt caacatttgc acctgccgtg tcacgtccca tactgagttt ctgtctcatg 14820

gaacgagctc ttgcttgaca gaccttaaat caacttaagc ccttgcctgt catctcaggt 14880

gtcccctacc ttcgtgactg gaattccgta gttgactgta tgtactaaag ccagacccgt 14940

gtctccacag agcagggcgc cgaacagaca ccagtcgatc gaagcctcag ctgggtgtct 15000

gactctgggt gcacaatgtt attgtactga ctatttattt cctcagttga cgacgaatag 15060

aaagggaaaa cattcttcct gagcctttct caggcctttg cctaaatccg tgcatttgtg 15120

tccgaacgta ctgtacgtcc cagctcttct tttgagtggt tggtgtatcc tctgtcacag 15180

gaagtatggc acgagcatgc gttttccaca gcgagtagca ttcctggttc tctgtgttca 15240

ccttgcacat gtgttaatga atgaatggga gggtgggaga atggatgaat gaaatatact 15300

actgattatt ttattctcga gtcctcgaaa tattttgttg ctcgtatttt gagtacccat 15360

tgtaattact ttgattagaa attaaaaagc tactggaaac gctgctgctg gaaatttcta 15420

ataaatgtgt attttatc 15438

<210> 214

<211> 3974

<212> DNA

<213> Rattus norvegicus (Norway rat)

<400> 214
tcgactaccc atattcccca ccagccaacc ggttcctcac caagatgtgg cacccaaaca 60

tctatgaggt aagacactag aggcctgctt caggcacact ccaccacaca cttctctgcc 120

tcccttgcac atgctccacc cctcctctaa ccctggtcac tcttgcagac aggggacgta 180

tgcatctcca tccaccatcc cccagttgat gacccacaga gtgggagcta ccctcagagc 240

ggtggaaccc cacgcagaat gtcaggtgag aattgccatg gaactgcacc gtgggcaccc 300

gcaaacaccc tgtaccctaa gatccaccct tccctgctcc agtcaggggt tcccagagtc 360

tggggagctg agcttgcacc tacggcccta caggaccatc ctcctgagtg tgatctccct 420

gctgaatgag cccaacacct tctcgcctgc caacgtggac gcctcggtga tgtacagaaa 480

atggaaggag agcaagggga aggaccgcga gtacacggac atcatccggt gagtgcttcc 540

tactgtgaag agtgatctgg ggtgtgggtg gggtggggac ttgttgtatg tttccatttt 600

ttgcatcccg ggctggatgt gtggaggact ctggggcccc cagagcacgg agttgaccac 660

ccagggtggc caagatcccc tctgccttgt tgttgactta actccatgag caactgggct 720

ctctgggcca caccctcact gtctgacaga gagcatcaat cacttgcatt tcgtgcttct 780

gaaacttaga gctttttttt ttttttttgc aaagtgggaa gggggctgag cccactgtga 840
gtctgtcctc agttagggac cagggctgcg acctgttgct gtctatgagt ggcagtgtcc 900
acacctatca tcgcttctta gcaggagctc ctggcctgtc tggggccagg cagtctcccc 960
tacacccctc acgttgggta tgaagttgca cttgaggggt tcagtctttg tcgcttagct 1020
tctgtgtttt ctgagacagg ctttcatata actccgtatc tccttgtggt ccctctgatg 1080
tcaggatgac aacacacatt cctcggaagt gaccccagtg cttgtctaca cagtgaccca 1140
aacacagggc ctgtcctgtc cccaagcctg ccctctcaga gttggtacag gccgcagctc 1200
ggtaggcagt gctggtctag catgtgcaca cacacagggt tagtctctgt cttaggcaag 1260
gtggatgtag ccagcatggt ccttcatgga ggtataagtt actcaggaag gtctgagcgc 1320
tgcactacgg aggtgtcccc ttcaccatgg tcaggtcagc aacggtcccg gtcactggaa 1380
gtccctgcag actgtcccct ttgttttctg ttcagactgg ctgaacagaa ctttgttcct 1440
cctgctcttc ctcagctgag aggaaggctg gctgccctag ctgttctata tagttctggc 1500
tgtcctggaa gaggagaccc tgggtctggc cctgtcactg cactctccac cctcctggca 1560
gcctttaatc cacttctggc tgcttcctcc ctggatccct ggcccagagc aggagcccag 1620
gctattttta ccattctgac tccaggagcc acttgctctg accaggaggc ctgtaccact 1680
cctggaactc aacctgagcc agtatctagt catgtccctg cctgagcaca aggacaggat 1740
agctaggagc ctgtgagaag gaccttgaga tggatctagg ctattgggtg tgctgctgtc 1800
ccacgtgccc ctccaccata gccttgtccc cattccccta ttccttctgg gcagctgaaa 1860
acagtgtctg ctctgcttga ctgcactggg acctgtgccc caagactctg gcctgacggt 1920
ggcctggcct ctcaggcacc tagttaattg ctgcctgccc tgtgcaagga agcccttaca 1980
tgttgcttca cccatggcct gtgggacctg gaacctcagt tgttccggaa ctgcccttca 2040
tcatggtgcc ctcaggtcag gctgcaattt gttgttggtg tgaggaggtg catgttacct 2100
agcccacaac tagggtagtg ccccccacag tctgggatta cagctgtttc tgtggtgctg 2160
ggagctggac atgggtcctt atgcttacag aaggagcact ctggctgccc ctgttgtcgg 2220
ttatagccga ggtcagcctg aatctcatag tagttctcct gcctccgcct ccaaggactg 2280
ggtgacagac cgtctcccac agctggctgt ggttacattt ctgctggagt tggtcactga 2340
gccactcctt gtcgcctggc cctctcctcc cacctgctcc ctgcccccat tcatctcatc 2400
gtcctgggcc gtggcctcac ccgccctgtc tccccaggaa gcaggtcctg gggaccaagg 2460
tggacgcaga gcgcgatggc gtgaagtgcc caccacgctg gccgagtact gcgtgaagac 2520
caagcgccgg cgcccgacga gggctcagac ctcttctacg acgactacta tgaggacggt 2580
gaagtggagg aggccgacag ctgctttggg gatgaagagg atgactctgg caccgaggag 2640
tcctgaccca cgtccctgaa ataaacttac aaattttacc tcagcaggac ccgtgtgggc 2700
ctcaggcgac agactacctc accgaggttc aatgtgggct cccatcccat gatccttagg 2760
tcctcttacc ctgtattccc catgggtttt cctcggcttt ggagaagagc tgtggtgcag 2820

ggcagacacc ccactcagcc ttcagaggat gacaagcctg ggaggctggg aaccgactgc 2880

ccaggggagc cagagactcc agcccaaaga gggccagccc cacagtctcc tctctgcttt 2940

tggtgtgtgt gaaatccaaa taaaatagct ttctgagaag ctgagagaag cagccaggac 3000

gccggtggca ctgggtactg agggctacct gggtacccag gcagggctgt gggatgggca 3060

ggaagaggta gcgatggtgg cctgtttgtt ggggctcagt agcatacagg agccccaggc 3120

ccagaagaaa cttgctgcct gcaaatccca cccgcctgca tcgggcactg tggttgcgtc 3180

caccccaagc ctgtgttcag gaggcctggg tcctgaggcc tacaggggat tggtattccc 3240

aacagtcctt gtgttctggg cacttcctca gccctggaaa ctagtgctgc ctgaggagtc 3300

cctctgatac ctgagctgca gcctggtgac tccacagcag gggcaggctg agtgaattct 3360

ctgaccacac catgctgtgc agggcacctg cagtgacccc gtgctgagat agcagatctg 3420

ctctgccacc tgcatgttta cgggtgctgt ggaaagaaag tgtcacttcc tcttatgcgg 3480

gcctgttaat gtccaaagtt ccagtcaacc catgccagtc agaactgatc agagaaggac 3540

acatcggaaa aaggtgttca ggcgatcctc tgcccataca atttgctgcc agcctgatct 3600

acagttgcaa aaccagcggg aaggctcagt gggcaaaggc ctgcaccaag cctagcagag 3660

gacccacttg gctccaggtg gtccctgtgg gggttgaggt cagttgagac aaatcccccc 3720

cccccccccg gagttcaccc cagtgctaaa aggagcagtg tgccaggaga gggagcatcg 3780

cttggctgta gttgctgccc ccagacacag ggcaactttg acagcagagt tgctttctaa 3840

aaccacacag cagggtgtga aggaggagga gctgacacgt gggtaaggtg ggtggtgctt 3900

ggaaggccac ttgtctccac cggaagagaa agtgtggggc cccttccccc agggaccacc 3960

agtcctgtgc cagg 3974

<210> 215

<211> 1882

<212> DNA

<213> Rattus norvegicus (Norway rat)

<400> 215
tggagtttga gcttggcgag cagctggaca acgagcgagt tgggatggcc accagctggg 60

gaagcatctt gcaggatgag aagcagctgg aagagttggc acagcaggcc atagaccggg 120

ccctggctga gggggtgttg ctgaggtccg caaagaaccc cagctcctct gacgtggtga 180

cgtatgcccc attcacgctc ttcccctcac cagtgcccag cactctgctg gagcaggcct 240

atgctgtgca gatggacttc aacatcctgg tagatgctgt cagccagaac tccgccttcc 300

tggagcaaac actgtctagc accatcaaaa aggatgagta tactgcccgt ctctttgata 360

tctacaagca agtcctgaaa gagggcatag cccagactgt gttcctgggc ctcaatcgtt 420

cagattacat gttccagtgc agcgcagacg gctccaaagc cctgaaacag attgagatca 480

acactatctc tgccagcttt gggggcctgg cctcccggac tccggctgtg caccgacatg 540

ttctcaatgt cctgaataag accaacgaag cttccaagat cctgtccaac aaccccagca 600

agggactggc cctggggatc gccaaagcct gggagctcta tggctcagcc aatgccgtgg 660

tgctactgat tgctcaggag aaggaaagga acatatttga ccagcgtgcc atagagaacg 720

agctgctaga caggaagatc catgtaatcc gccgaagatt tgaagatgtc tctgaaaggg 780

gttctctaga ccaaaaccga aggctgttta tggaggacca ggaagttgct gtggtttact 840

tccgagatgg ctacatgccc agtcagtata acgcacagaa ctgggaagct cgcctgctgc 900

tagagagatc atgtgctgcc aagtgtcccg acattgccac acagctggct ggcactaaga 960

aggtgcagca ggaactgagc agggtgggcc tgctggaagc gctgctcccg ggccagcccg 1020

aggctgtggc ccgcctccgt gccacctttg ctggcctcta ttcactggac atgggtgaag 1080

aaggggacca ggctgtcgct gaggcccttg ctgcccctag ccactttgtg ctgaagcccc 1140

aaagagaggg cggaggtaat aacttctatg gggaggaaat ggtacacgct ctggagcagc 1200

tgaaagacag cgaggagaga gcctcctaca tcctcatgga gaagattgaa cctgagcctt 1260

ttaggaattg cttactacgg cctggcagcc ctgcccaagt ggtccagtgc atctcggagc 1320

tgggtatttt tggagtctat gtcagacagg gaacaacact tgtgatgaac aagcatgtgg 1380

ggcatctgct tcgaaccaaa gccattgaac atgcagatgg aggtgtggca gcaggagtgg 1440

cagtcctgga caacccctac cctgtgtgaa gacatgttct gggcttcact caagagacct 1500

tctatcctct gtacttggca ctcctctcct gaggggctac ccctgtacct gtgttagggg 1560

agggagcttg tctctttcat agacctccag gggctttagg gaagggaaaa tcccgggtcc 1620

cttctctcag ccttccatcc aaggaccaga aaagctatga ttccattgga agagttctgg 1680

agctccccag atttggagtg ggaatggaag ctcctttgag gcaaaggccc acaaacccca 1740

cacatcttca ttgccctctc gccagccttt ccagcaggtt ctagtgcctt gacctggggt 1800

aggaccaagt gacaggagga agagggtaga tgggcataga cttccccagc tctgccctaa 1860

ataaaacaat gctgattcaa tg 1882

<210> 216

<211> 810

<212> DNA

<213> Rattus norvegicus (Norway rat)

<400> 216
agccgagagg tgtgagctgc tacagtgccc gagttaaggg gaattggagg caggcgccgt 60

ccacagcgat atcgagacac tcagagccac aggcaccatg tccgatcctg gtgatgtccg 120

acctgttccg cacaggagca aagtatgccg tcgtctgttc ggtcccgtgg acagtgagca 180

gttgagccgc gattgcgatg cgctcatggc gagctgtctc caggaggccc gagaacggtg 240

gaactttgac ttcgccactg agacgccact ggagggcaac tacgtctggg agcgtgttcg 300

gagcccaggg ctgcccaaga tctacctgag ccctgggtcc cgccgccgtg atgacctggg 360

aggggacaag aggcccagta cctcctcggc cctgctgcag gggccagggc cagctccgga 420

ggaccacgtg gccttgtcgc tgtcttgcac tctggtgtct cacgcccctg agaggcctga 480

agactccccg ggcgggaccg ggacatctca gggccgaaaa cggaggcaga ccagcctaac 540

agatttctat cactccaagc gccgattggt cttctgcaag agaaagccct gaagtgccca 600

cgggaggctc gccttcttct gctgtgggtc aggaggcctc ttccccatct tctgccttag 660

ccttcattca gtgtgtctta attattattt gtgttttaat ttaaacttct cctgtatata 720

ctctgcctgc cccctcccag cctccaaact tagttattta aaaaacaaaa caaaacaatg 780

aaattagtag gacggtaaaa aaaaaaaaaa 810

<210> 217

<211> 402

<212> DNA

<213> Rattus norvegicus (Norway rat)

<400> 217
cggggtgtgg tgaaggatgc cacggagtcc acagcccgag tagaattgca ctctacctgc 60

cagaccatct ctgtggaccg ccagcggctc accacagttg actcccagcg tccaggtggc 120

atgacttcca cctatggaag gactcccatg tatggctctc agactcccat gtatggctct 180

ggctcccgaa cacccatgta tggctcccag acacctctgc aggatggtag ccgcactcca 240

cattatggct cacagacacc cctgcatgat ggtagccgca ctcctgctca gagtgggggc 300

tgggatccca acaaccctaa cacaccttcc agggctgagg aggaatacga atatgccttt 360

gacgacgaac ccaccccatc cccacaagca tatggggggc cg 402

<210> 218

<211> 1519

<212> DNA

<213> Rattus norvegicus (Norway rat)

<400> 218
tgcaactgaa agcattctta gcttgccagt ggcccccact gcctgcctgc ctgcggaact 60

ctctagacca tagattcctc ctccactcta gcaagagaag atgctgtcta atttgaggat 120

cctgctcaac aaggcagctc ttagaaaggc tcacacttcc atggttcgaa attttcggta 180

tgggaagcca gtccagagtc aagtacagct gaaaggccgt gacctcctca ccctgaagaa 240

cttcacagga gaggagattc agtacatgct atggctctct gcagatctga aattcaggat 300

caaacagaaa ggagaatact tgcctttatt gcaagggaaa tccttaggga tgatttttga 360

gaaaagaagt actcgaacaa gactgtccac agaaacaggc ttcgctcttc tgggaggaca 420

tccttctttt cttaccacac aagacattca cttgggcgtg aatgaaagtc tcacagacac 480

agctcgtgtg ttatctagca tgacagatgc agtgttagct cgagtgtata aacaatcaga 540

tctggacatc ctggctaagg aagcaaccat cccaattgtc aacggactgt cagacctgta 600

tcatcctatc cagatcctgg ctgattacct tacactccag gaacactatg gctctctcaa 660

aggtctcacc ctcagctgga taggagatgg gaacaatatc ctgcactcca tcatgatgag 720

tgctgcaaaa ttcgggatgc accttcaagc agctactcca aagggttatg agccagatcc 780

taatatagtc aagctagcag agcagtatgc caaggagaat ggtaccaggt tgtcaatgac 840

aaatgatcca ctggaagcag cacgtggagg caatgtatta attacagata cttggataag 900

catgggacaa gaggatgaga agaaaaagcg tcttcaagct ttccaaggtt accaggttac 960

aatgaagact gctaaagtgg ctgcgtctga ctggacgttt ttacactgct tgcctagaaa 1020

gccagaagaa gtagatgatg aagtgtttta ttctccgcgg tcattagtgt tcccagaggc 1080

agaaaataga aagtggacaa tcatggctgt catggtatcc ctgctgacag actactcacc 1140

tgtgctccag aagccaaagt tctgatgcct gcaagaggac gaaaaaccca aaagacaaaa 1200

aaatctgttc tttagcagca gaataagtca gtttatgtag aaaagagaag aattgaaatt 1260

gtaaacacat ccctagtgcg tgatataatt atgtaattgc tttgctattg tgagaattgc 1320

ttaaagcttt tagtttaagt gctgggcatt ttattatcct gcttgacttg acttaagcac 1380

tctcttcaat tcacaacttc tgaatgatat ttgggtttca tattaattat catacacatt 1440

tccttccact aagcattaaa cactatgctt acaatgcata ccatctaagt cattaaatgt 1500

aatccatgct tattacctt 1519

<210> 219

<211> 2073

<212> DNA

<213> Rattus norvegicus (Norway rat)

<400> 219
gtctctgtgt ggtctcgtca tcagcacagc tgggcctaca cgcaagcaac catgtctaag 60

ggacctgcgg ttggcattga tcttggcacc acctactcct gtgtgggtgt cttccagcat 120

ggaaaggtgg aaataattgc caatgaccag ggtaaccgca ccacgccgag ctatgttgct 180

ttcaccgaca cagaacgatt aattggggat gcggccaaga atcaggttgc aatgaacccc 240

accaacacag tttttgatgc caaacgtctg atcggacgta ggttcgatga tgctgttgtt 300

cagtctgaca tgaagcactg gcccttcatg gtggtgaacg atgcaggcag gcccaaggtc 360

caagtcgaat acaaagggga gacaaaaagt ttctatcctg aggaagtgtc ttcaatggtt 420

ctgacaaaaa tgaaggaaat tgcagaagct taccttggaa agactgttac caatgccgtg 480

gtcaccgtgc cagcttactt caatgactct cagcgacagg caacaaaaga tgctggaact 540

attgctggcc tcaacgtact tcgaattatc aatgagccaa ctgctgctgc tattgcctat 600

ggcttagata agaaggtcgg ggctgaaagg aatgtgctca tttttgactt gggaggtggc 660

acttttgatg tgtcaatcct cactatcgag gatggaattt ttgaagtcaa atcaacagct 720

ggagacaccc acttgggcgg agaagacttt gacaaccgaa tggtcaacca tttcattgct 780

gagtttaagc gaaagcacaa gaaggacatc agtgagaaca agagagctgt caggcgtctc 840

cgcactgcct gtgagcgggc caagcgcacc ctctcctcca gcacccaagc cagtattgag 900

attgattctc tctatgaggg aattgacttc tacacctcca ttacccgtgc tcgatttgag 960

gagttgaatg ctgacctgtt ccgtggcaca ctggaccctg tagagaaggc ccttcgagat 1020

gccaaactag acaagtcaca gatccatgat attgtcctgg tgggtggttc taccagaatc 1080

cccaagatcc agaaacttct gcaagacttc ttcaatggaa aagagctgaa taagagcatt 1140

aaccccgatg aagctgttgc ctatggtgca gctgtccagg cagccattct atctggagac 1200

aagtctgaga atgttcagga tttgctgctc ttggatgtca ctcctctttc ccttgggatt 1260

gaaactgctg gtggagtcat gactgtcctc atcaagcgca ataccaccat tcccaccaag 1320

cagacccaga ctttcaccac ctactctgac aaccagccag gtgtactcat ccaggtgtat 1380

gaaggtgaaa gggccatgac caaggacaac aacctgcttg ggaagtttga gctcacaggc 1440

atacctccag caccccgtgg ggttcctcag attgaggtta cttttgacat tgatgccaat 1500

ggcatcctca atgtttctgc tgtagataag agcacaggaa aggagaacaa gatcaccatc 1560

accaatgaca agggccgctt gagtaaggag gatattgagc gcatggtcca agaagctgag 1620

aagtacaaag ctgaggatga gaagcagaga gataaggttt cctctaagaa ctcgctggag 1680

tcttatgctt tcaacatgaa agcaactgtt gaggatgaga aacttcaagg caagatcaat 1740

gatgaagaca aacagaagat tcttgacaag tgcaacgaaa tcatcagctg gctggataag 1800

aaccagactg cggagaagga agaatttgag catcagcaga aagaactgga gaaggtctgc 1860

aaccctatca tcaccaagct gtaccagagt gctggtggca tgcctggagg aatgcctggt 1920

ggcttccctg gtggaggagc tcctccatct ggtggtgctt cttcaggccc caccattgaa 1980

gaggtcgatt aagtcaaagt agagggtata gcattgttcc acagggaccc aaaacaagta 2040

acatggaata ataaaactat ttaaattggc acc 2073

<210> 220

<211> 1696

<212> DNA

<213> Rattus norvegicus (Norway rat)

<400> 220
cgctgaacgc tctcatcatc ctaccgtcat gtctaagtca gagtccccca aggaaccgga 60

acagctgcgg aagctcttca ttggagggct gagcttcgaa acaaccgacg agagtctgag 120

gagccatttt gagcaatggg gaacactcac cgactgtgtg gtaatgagag atccaaacac 180

caaaagatcc agaggctttg ggtttgtcac atatgccact gtggaggaag tggatgctgc 240

catgaatgca agaccacaca aagtggatgg aagagttgtg gaacctaaga gagctgtgtc 300

aagagaagat tctcagagac caggtgccca cttaactgtg aagaagatct ttgttggcgg 360

tattaaagaa gacactgaag aacatcacct acgagattat tttgagcagt atgggaaaat 420

tgaagtgatt gaaattatga ctgacagagg cagtggaaaa aagaggggat ttgcgtttgt 480

cacctttgat gaccatgact ctgtggataa gattgttatt cagaaatacc atactgtgaa 540

tggccacaac tgtgaagtaa gaaaggctct gtgcaaacaa gagatggcta gtgcttcatc 600

cagccagaga ggtcgaagtg gttccggaaa ctttggtggt ggtcgtggag gtggtttcgg 660

tggcaatgac aattttggtc gaggagggaa cttcagtggt cgtggtggct ttggtggcag 720

ccgtggtggt ggtggatatg gtggcagtgg ggatggctat aatggatttg gcaatgatgg 780

aagcaatttt ggaggtggtg gaagctacaa tgattttggc aattacaaca accagtcatc 840

aaattttgga ccgatgaaag gaggaaactt tggaggcagg agctctggcc cttatggtgg 900

tggaggccag tactttgcta aaccacgaaa ccaaggtggc tatggaggtt ccagcagcag 960

cagtagctat ggcagtggca ggaggttcta attacagcca ggaaacaaag cttagcagga 1020

gagccagaga agtgacaggg aagctacagg ttacaacaga tttgtgaact cagccaagca 1080

cagtggtggc agggcctagc tgctacaaag aagacatgtt ttagacaata ctcatgtgtg 1140

tgggcaaaaa ctccaggact gtatctgtga ctaattgtat aacaggttat tttagtttct 1200

gttctgtgga aagtgtaaag cattccaacg aagggtttta ctgtagacct tttcacccca 1260

tgctgttgat tgctaaatgt aaaagtctga tcatgacgct gaataaatgt gtcttttttt 1320

ttttttttta atgtgctgtg taaagttagt ctactctgaa gccatcttgg taaacttccc 1380

caacagtgtg aagttagaat tccttcaggg tggtgccaaa ttccatttgg aatttattta 1440

tggttgcttg ggtggagaag ccattgtctt caaaaacctt ggtgttgcta aactgccagt 1500

tactgttgta acttaatgag tttcaccatt taaagggtca tccaagcaag atcacaattt 1560

ggttataaaa tggttgttgg acacctatga agagaaaatt gaataacggt ctcagataaa 1620

ataagagatg ggaatgaagc ttgtgtatca tccattatca tgtgtactca ataaacgatt 1680

taattctctt gaatgg 1696

<210> 221

<211> 1442

<212> DNA

<213> Rattus norvegicus (Norway rat)

<400> 221
gtccccccca ccccagctga ataggctgcg ttctcttgga acgcgccgca gaacgaggtt 60

ctgtgaccct agccgcgttc cctccttagt tcctttcgcc tacccacccg cgtacccgac 120

agacccaccc cgtcctgtgc caggaaagcg ctgccaccgg caccatgccc cacccatacc 180

cagcactgac cccggagcag aagaaggagc tggctgacat cgctcaccga attgtagctc 240

cgggcaaggg catcctggct gcagacgagt ccactggaag cattgccaag cgcctgcagt 300

ccattggcac cgagaacacc gaggagaaca ggcgcttcta ccgccaactg ctgctgactg 360

ccgatgaccg tgtgaatccc tgcattggag gggtgatcct tttccacgag acactgtacc 420

agaaggcaga tgatggccgt cccttccccc aagttatcaa gtccaagggt ggtgttgtgg 480

gcattaaggt agataagggt gtagtgcccc tggctggaac caatggcgag accactactc 540

aagggctgga cgggctgtct gagcgctgtg cccagtataa gaaggatgga gccgactttg 600

ccaagtggcg ctgtgtgcta aagattgggg agcatactcc ctcgtccctc gccatcatgg 660

aaaatgccaa tgttctggcc cgttacgcta gcatctgcca gcagaatggc attgtaccca 720

ttgtggagcc tgaaattctc cctgatgggg accatgactt gaagcgctgc cagtatgtaa 780

ctgagaaggt actggcagct gtctacaagg ctctgagtga ccaccatgtc tatctggaag 840

gcacactgct gaagcccaac atggtcaccc ctggccatgc ttgcacccag aaattttcca 900

atgaggaaat tgccatggca accgtcacag cacttcgtcg aacagtgccc cctgccgtcc 960

ctggggtcac tttcctgtct ggagggcaga gtgaggaaga ggcatccatc aacctcaatg 1020

ctatcaacaa gtgtcccctg ctgaagccat gggccttgac tttctcctat ggccgagccc 1080

tgcaggcctc tgctctaaag gcttggggtg ggaagaagga gaacctgaag gcagcccagg 1140

aggagtacat caagcgagcc ctggccaaca gcctcgcttg tcaaggaaag tacactccaa 1200

gtggccagtc tggagccgca gccagtgaat ctctcttcat ctctaaccat gcctactaac 1260

cagagctgat ctaaggctgc tccatcgaca ctccaggccc ctgcctaccc acttgctatt 1320

gaagaggggc cttcaggctc tttcccatca ctcttgctgc cctcgtgtgt gcagtgttgt 1380

ctgtgaatgc taaatctgcc atcccttcca gcccactgcc aataaacagc tatttaaggg 1440

gg 1442

<210> 222

<211> 386

<212> DNA

<213> Rattus norvegicus (rat)

<400> 222
cctcaatgct atcaacaagt gtcccctgct gaagccatgg gccttgactt tctcctatgg 60

ccgagccctg caggcctctg ctctaaaggc ttggggtggg aagaaggaga acctgaaggc 120

agcccaggag gagtacatca agcgagccct ggccaacagc ctcgcttgtc aaggaaagta 180

cactccaagt ggccagtctg gagccgcagc cagtgaatct ctcttcatct ctaaccatgc 240

ctactaacca gagctgatct aaggctgctc catcgacact ccaggcccct gcctacccac 300

ttgctattga agaggggcct tcaggctctt tcccatcact cttgctgccc tcgtgtgtgc 360

agtgttgtct gtgaatgcta aatctg 386

<210> 223

<211> 8048

<212> DNA

<213> Rattus norvegicus (Norway rat)

<220>

<221> misc_feature

<222> (2005)..(2005)

<223> n

<220>

<221> misc_feature

<222> (3503)..(3503)

<223> n

<400> 223
gaattcggcc caggataaac agatatatag gtaaatgact ggatagatac atggatttga 60

tctctaactc aacttaggca ccatagaaaa caatccttga attattttaa taattttaaa 120

tcctagttta ctgagaaaca cgttgacgag gcagggcttc agcctctctg aagcagcact 180

gcccctctgt ggccacacag gaagtcttca ctcagcagtt tcctgactta aggcctaggc 240

ctgaggaaat gcattgaata tttctctggg ggacaatagt tctgagaagg aaagagagtt 300

tgtctatgtt gaggtcagtt aaggatggtt ttctttcctt ccctcccagc cttttctcca 360

tgcaacatca tgagtcccca tctttctatc ctgcctaaaa ccacttcctt ctgggaaatg 420

agaaaatcca ctcatacaaa atggaaacat cccgaggatc ccggcccgca gcagctctct 480

gcccccagac cctgtgagag agagacccaa ccgcctggtc aggtgggcac tcctgaggct 540

gcagagggaa gagaccacca acactgctca cccctgccca catccctggc ccaagaggaa 600

actgtataag gcctctgggt cctgtggggg agggcccagg agcggcagga cccctgtgcc 660

tgagagacca ccaccagaac cagaaggaaa cagaccggat aaacagttct ctgcaccaaa 720

tcccgtggag ggagagctga accttcagag agaaagacaa gcctgggaaa ccagaagaga 780

ctgctctctg cacacacatc tcggacgcca gaggaaaaag ccaaagacca tctggaaccc 840
tggtgcactg aagctcccgg aaatggtgca caggtcttcc tggttgctgc cgctgcagag 900
agcccgtggt agcaccccac gagcgaactt gagcctcagg accacaggta agaccaactt 960
ttctgctgca agaaagctgc ctggtgaact caagacacag gcccacagga acagctgaag 1020
acctgtagag aggaaaaacc acacgccgga aagcagaaca ctctgtcccc ataactgact 1080
gaaagagagg aaaacaggtc tacagcactc ctgacacaca ggcttatagg acagtctagc 1140
cactgtcaga aatagcagaa caaagtaaca ctagagataa tctgatggcg aaaggcaagc 1200
gcaggaaccc aagcaacaga aaccaagact acatggcacc atcggagccc aattctccca 1260
tcaaaacaaa catggaatat ccaaacacac cagaaaagca agatctagtt ccaaaatcat 1320
ttttgatcat gatgctggag gacttcaaga aagacgtgaa gaactcctta gagaacaagt 1380
agaagcctac agagaggaat cgcaaaaatg cctgaaagaa tcgcaaaaat ccctgaaaga 1440
attccaagaa aacataaata aacaagtaga agcccataga gaggagacac aaaaatccct 1500
gaaagaattc caggaaaaca taaataaaca agtagaagcc catagagagg agacacaaaa 1560
atccctgaaa gaattccagg aaaacataaa taaacaagta gaagcccata gagaggagac 1620
acaaaaatcc ctgaaaggaa ttccaggaaa acacaatcaa acagttgaag gaattaaaaa 1680
tggaaataga agcaatcaaa aaagaacaca tggaaacaac cctggatata gaaaaccaaa 1740
agaagagaca aggagctgta gataaaagct tcaccaacag aatacaagag atggaagaga 1800
gaatctcagg agcagaagat tccatagaaa tcattgactc aactgtcaaa gataatgtaa 1860
agcggaaaaa gctactggtc caaaacatac aggaaatcca ggactcaatg agaagatcaa 1920
acctaaggat aataggtata gaagagagtg aagactccca gctcaaagga ccagtaaata 1980
tcttcaacaa aaccatagaa gaaancttcc ctaacctaaa aaaagagata cccatagaca 2040
cacaagaagc ctacagaact ccaaatagat tggaccagaa aagaaacacc tcccgtcaca 2100
taattgtcaa aacaccaaac gcacaaaata aagaaagaat attaaaaaca gtaagggaaa 2160
aaggtcaagt aacatataaa gggagaccta tcagaatcac accagacttc tcgccagaaa 2220
ctatgaaggc cagaagatcc tggactgatg ttatacagac cctaagagaa cacaaatgcc 2280
agcccaggtt actgtatcca gcaaaactct caattaacat tgatggagaa accaagacat 2340
tccatgacaa aaccaaattt acacaatatc tttccacaaa tccagcacta caaaggataa 2400
taaatggtaa agcccaacat aaggaggcaa gctataccct agaagaagca agaaactaat 2460
cgtcttggca acaaaacaaa gagaatgaaa gcacacaaac ataacctcac atccaaatat 2520
gaatataacg ggaagcaata atcactattc cttaatatct ctcaacatca atggcctcaa 2580
ctccccaata aaaagtcata gattaacaaa ctggatacac aacgaggacc ctgcattctg 2640
ctgcctacag gaaacacacc tcagagacaa agacagacac tacctcagag tgaaaggctg 2700
gaaaacaaat ttccaagcaa atggtcagaa gaagcaagct ggagtagcca ttctaatatc 2760
aaataaaatc aatttccaac taaaagtcat caaaaaagat aaggaaggac acttcatatt 2820

catcaaagga aaaatccacc aagatgaact ctcaatccta aatatctatg ccccaaatac 2880

aagggcacct acatacgtaa aagaaacctt actaaagctc aaagcacaca ttgcacctca 2940

cacaataata gtgggagatt tcaacacacc actctcatca atggacagat catggaaaca 3000

gaaattaaac agtgatgtcg acagcataag agaagtcatg agccaaatgg acttaacgga 3060

tatttttaga acattctatc ctaaagcaaa aggatatacc ttcttctcag ctcctcatgg 3120

cactttctcc aaaattgacc atataattgg tcaaaaaacg ggcctcaaca ggtacagaaa 3180

gatagaaata atcccatgcg tgctatcgga ccaccacggc ctaaaactgg tcttcaataa 3240

caataaggga agaatgccca catatacctg gaaattgaac aatgctctac tcaatgataa 3300

cctggtcaag gaagaaataa agaaagaaat taaaaacttt ttagaattta atgaaaatga 3360

aggtacaaca tacccaaact tatgggacac aatgaaagct gtgctaagag gaaaactcat 3420

agcgctgagt gcctgcaaaa agaaacagga aagagcatat gtcagcagct tgacagcaca 3480

cctaaaagct ctagaacaaa aangaagcaa atacactgag gaggagtaga aggcaggaaa 3540

taatcaaact cagagctgaa atcaaccaag tagaaacaaa aaggaccata gaaagaatca 3600

acagaaccaa aagctggttc tttgagaaaa tcaacaagat agataaaccc ttagccagac 3660

taacgagagg acacagagag tgtgtccaaa ttaacaaaat cagaaatgaa aagggagaca 3720

taactacaga ttcagaggaa attcaaaaaa tcatcagatc ttactataaa aacctatatt 3780

caataaaact tgaaaatctt caggaaatgg acaatttcct agacagatac cacgtatcga 3840

agttaaatca ggaacagata aaccagttaa acaaccccat aactcctaag gaaatagaag 3900

cagtcattaa aggtctccca accaaaaaga gtccaggtcc agacgggttt agtgcagaat 3960

tctatcaaac cttcatagaa gacctcatac caatattatc caaactattc cacaaaattg 4020

aaacagatgg agcactaccg aattccttct acgaagccac aattactctt atacctaaac 4080

cacacaaaga cacaacaaag aaagagaact tcagaccaat ttcccttatg aatatcgatg 4140

caaaaatact caataaaatt ctggcaaacg aattcaagag cacatcaaaa caatcatcca 4200

ccatgatcaa gtaggcttca tcccaggcat gcagggatgg tttaatatac ggaaaaccat 4260

caacgtgatc cattatataa acaaactgaa agaacagaac cacatgatca tttcattaga 4320

tgctgagaaa gcatttgaca aaattcaaca cccttcatga taagagtccc ggaaagaata 4380

ggaattcaag gcccatacct aaacatagta aaagccatat acagcaaacc agttgctaac 4440

attaaactaa atggagagaa acttgaagca atcccactaa aatcagggac tagacaaggc 4500

tgcccactct ctccctactt attcaatata gttcttgaag ttctagccag accaatcaga 4560

caacaaaagg agatcaaggg gatacagatc ggaaaagaag aagtcaaaat atcactattt 4620

gcagatgaca tgatagtata tttaagtgat cccaaaagtt ccaccagaga acgactaaag 4680

ctgataaaca acttcagcaa agtggctggg tataaaatta actcaaataa atcagttgcc 4740

ttcctctata caaaagagaa acaagccgag aaagaaatta gggaaacgac acccttcata 4800

atagacccaa ataatataaa gtacctcggt gtgactttaa ccaagcaagt aaaagatctg 4860

tacaataaga acttcaagac actgaggaaa gaaattgaag aagacctcag aagatggaaa 4920
gatctcccat gctcatggat tggcaggatt aatatagtaa aaatggccat tttacccaaa 4980
gcaatctaca gattcaatgc aatccccatc aaaataccaa tccaattctt caaagagtta 5040
gacagaacaa tttgcaaatt catctggaat aacaaaaaac ccaggatagc taaagctatc 5100
ctcaacaata aaaggacttc agggggaatc actatccctg aactcaagca gtattacaga 5160
gcaatagtga taaaaactgc atggtgattg gtacagagac agacagatag accaatggaa 5220
tagaactgaa gacccagaaa tgaacccaca cacctatggt cacttgattt ttgacaaagg 5280
agccaaaacc atccaatgga aaaaagatag cattttcagc aaatggtgct ggttcaactg 5340
gagggcaaca tgtagaagaa tgcagatcga tccatcctta tcaccctgta caaagcttaa 5400
gtccaagtgg atcaaggacc tccacatcaa accagacaca ctcaaactaa tagaagaaaa 5460
actagggaag catctggaac acatgagcac tggaaaaaat ttcctgaaca aaacaccaat 5520
ggcttatgct ctaagatcaa gaatcgacaa atgggatctc ataaaactgc aaagcttctg 5580
taaggcaaag gacactgtgg ttaggacaaa acggcaacca acagattggg aaaagatctt 5640
taccaatcct acaacagata gaggccttat atccaaaata tacaaagaac tcaagaagtt 5700
agaccgcagg aaacaaataa ccctattaaa aatggggttc agagctaaac aaaaattcac 5760
agctgaggaa tgccgaatgg ctgagaaaca cctaaagaaa tgttcaacat ctttagtcat 5820
aagggaaatg caaatcaaaa caaccctgag atttcacctc acagtagtga gaatggctaa 5880
gatcaaaaac tcaggtgaca gcagatgctg gcgaggatgt ggagaaagag gaacactcct 5940
ccattgttgg tgggattgca gactggtaaa accattctgg aaatcagtct ggaggttcct 6000
cagaaaattg gacattgaac tgcctgagga tccagctata cctctcttgg gcatataccc 6060
aaaagatgcc tcaacatata aaagagacac gtgctccact atgttcattg ccgccatatt 6120
tataatagcc agaaactgga aagaacccag atgcccttca acagaggaat ggatacagaa 6180
aatgtggtac atctacacaa tggaatatta ctcagctatc aaaaacaacg agtttatgaa 6240
attcgtaggc aaatggttgg aactggaaaa tatcatcctg agtgagctaa cccactcaca 6300
gaaagacata catggtatgc actcattgat aagtggctat tagcccaaat gcttgaatta 6360
ccctagatcc ctagaacaaa cgaaactcaa gacggatgat caaaatgtga atgcttcact 6420
ccttctttaa atgaggaaaa agaataccct tggctgggaa gggagaggca aagattaaaa 6480
cagagactga aggaacaccc attcagagcc tgccccacag gtggcccata catatacagc 6540
cacccaattg gacaagatgg atgaagcaaa gaagtgcaga ctgacaggag ccggatgtag 6600
atcgctcctg agagacacag ccagaataca gcaaatacag aggcgaatgc cagcagcaaa 6660
ccactgaact gagaataggt cccccgttga aggaatcaga gaaagaactg gaagagcttg 6720
aaggggctca agaccccaaa agtacaacaa tgtcaagcaa ccagagcttc cagggactaa 6780
gccactacct aaatactata catggactga ccctggactc tgaccccata ggtagcaatg 6840
aatatcctag taagagcacc agtggaaggg gaagccctgg gtcctgctaa gactggaccc 6900

ccagtgaact agactatggg gggagggcgg caatgggggg agggttggga gggggacacc 6960

cataaggaag gggagggggg agggggatgt ttacccggaa accgaaaggg aataacactt 7020

aaattatata agaaatactc aagttaatta aaaataataa taataataat aaaaagaatt 7080

actcataaaa taaagacaag atgatggata aaattaaaaa aacaaaaaaa acaaaaaaaa 7140

accaaaatgg aaacatgaaa atgctcacta cttacaatag tgctttatgt aataagcaaa 7200

tgactgcatt tgacacgagt tataatttat taacccctta gttagactac ttgtacacat 7260

ggtgattatt aatccacaat tattcttttt cactaaaatc cttatatttt attctttgag 7320

attataatat aatcacacaa attccctctt ccctgtcatc tcttcaaaac cttccatata 7380

gtcctccatg ctgtcttttg aacgttatgg cttctttttt atattcttgt tttatattat 7440

atatatataa tatatattat aatatatatc atactatata tattcctcag gactgaccaa 7500

tccatattgg ataaccaatt ggtgtgctcg tctcagggaa agaccatttc cccaccatca 7560

gcattctcgg tttccagtag ttcattgtct agaatggaga ctgtgtgtgc tttccccgtc 7620

cactttagct gctgtgtttc agctcctctt gaggcagtta tgttggtgag acctcatctg 7680

tgcggcttcc aacattcctc agagacacag tgagttacac agtgtcacct gatcctctaa 7740

ctcccaccat ctttcctgct gtcttctcca gggtttcctg aacctcagca ggagcagggg 7800

ttgtactgta gacacgccca ccttcactag gatccacagc tcctcacttt gatttgctgt 7860

ggttttctgc aatggcttcc atgtcctagt aaagtcatgg agaccccata accacgctgt 7920

actaaaccag acaatcccta gctgcattct aagttcttgt ccttatccca tacatgtgca 7980

tttagagagt ctgcttattt tgctcattat agaacgagtc aatcattctc tttgagttgt 8040

aaaagctt 8048

<210> 224

<211> 8048

<212> DNA

<213> Rattus norvegicus (Norway rat)

<220>

<221> misc_feature

<222> (2005)..(2005)

<223> n

<220>

<221> misc_feature

<222> (3503)..(3503)

<223>  n

<400>  224
gaattcggcc caggataaac agatatatag gtaaatgact ggatagatac atggatttga      60
tctctaactc aacttaggca ccatagaaaa caatccttga attattttaa taattttaaa     120
tcctagttta ctgagaaaca cgttgacgag gcagggcttc agcctctctg aagcagcact     180
gcccctctgt ggccacacag gaagtcttca ctcagcagtt tcctgactta aggcctaggc     240
ctgaggaaat gcattgaata tttctctggg ggacaatagt tctgagaagg aaagagagtt     300
tgtctatgtt gaggtcagtt aaggatggtt ttctttcctt ccctcccagc cttttctcca     360
tgcaacatca tgagtcccca tctttctatc ctgcctaaaa ccacttcctt ctgggaaatg     420
agaaaatcca ctcatacaaa atggaaacat cccgaggatc ccggcccgca gcagctctct     480
gcccccagac cctgtgagag agagacccaa ccgcctggtc aggtgggcac tcctgaggct     540
gcagagggaa gagaccacca acactgctca cccctgccca catccctggc ccaagaggaa     600
actgtataag gcctctgggt cctgtggggg agggcccagg agcggcagga cccctgtgcc     660
tgagagacca ccaccagaac cagaaggaaa cagaccggat aaacagttct ctgcaccaaa     720
tcccgtggag ggagagctga accttcagag agaaagacaa gcctgggaaa ccagaagaga     780
ctgctctctg cacacacatc tcggacgcca gaggaaaaag ccaaagacca tctggaaccc     840
tggtgcactg aagctcccgg aaatggtgca caggtcttcc tggttgctgc cgctgcagag     900
agcccgtggt agcaccccac gagcgaactt gagcctcagg accacaggta agaccaactt     960
ttctgctgca agaaagctgc ctggtgaact caagacacag gcccacagga acagctgaag    1020
acctgtagag aggaaaaacc acacgccgga aagcagaaca ctctgtcccc ataactgact    1080
gaaagagagg aaaacaggtc tacagcactc ctgacacaca ggcttatagg acagtctagc    1140
cactgtcaga aatagcagaa caaagtaaca ctagagataa tctgatggcg aaaggcaagc    1200
gcaggaaccc aagcaacaga aaccaagact acatggcacc atcggagccc aattctccca    1260
tcaaaacaaa catggaatat ccaaacacac cagaaaagca agatctagtt ccaaaatcat    1320
ttttgatcat gatgctggag gacttcaaga aagacgtgaa gaactcctta gagaacaagt    1380
agaagcctac agagaggaat cgcaaaaatg cctgaaagaa tcgcaaaaat ccctgaaaga    1440
attccaagaa aacataaata aacaagtaga agcccataga gaggagacac aaaaatccct    1500
gaaagaattc caggaaaaca taaataaaca agtagaagcc catagagagg agacacaaaa    1560
atccctgaaa gaattccagg aaaacataaa taaacaagta gaagcccata gagaggagac    1620
acaaaaatcc ctgaaaggaa ttccaggaaa acacaatcaa acagttgaag gaattaaaaa    1680
tggaaataga agcaatcaaa aaagaacaca tggaaacaac cctggatata gaaaaccaaa    1740
agaagagaca aggagctgta gataaaagct tcaccaacag aatacaagag atggaagaga    1800
gaatctcagg agcagaagat tccatagaaa tcattgactc aactgtcaaa gataatgtaa    1860
agcggaaaaa gctactggtc caaaacatac aggaaatcca ggactcaatg agaagatcaa    1920

acctaaggat aataggtata gaagagagtg aagactccca gctcaaagga ccagtaaata 1980

tcttcaacaa aaccatagaa gaaancttcc ctaacctaaa aaaagagata cccatagaca 2040

cacaagaagc ctacagaact ccaaatagat tggaccagaa aagaaacacc tcccgtcaca 2100

taattgtcaa aacaccaaac gcacaaaata aagaaagaat attaaaaaca gtaagggaaa 2160

aaggtcaagt aacatataaa gggagaccta tcagaatcac accagacttc tcgccagaaa 2220

ctatgaaggc cagaagatcc tggactgatg ttatacagac cctaagagaa cacaaatgcc 2280

agcccaggtt actgtatcca gcaaaactct caattaacat tgatggagaa accaagacat 2340

tccatgacaa aaccaaattt acacaatatc tttccacaaa tccagcacta caaaggataa 2400

taaatggtaa agcccaacat aaggaggcaa gctataccct agaagaagca agaaactaat 2460

cgtcttggca acaaaacaaa gagaatgaaa gcacacaaac ataacctcac atccaaatat 2520

gaatataacg ggaagcaata atcactattc cttaatatct ctcaacatca atggcctcaa 2580

ctccccaata aaaagtcata gattaacaaa ctggatacac aacgaggacc ctgcattctg 2640

ctgcctacag gaaacacacc tcagagacaa agacagacac tacctcagag tgaaaggctg 2700

gaaaacaaat ttccaagcaa atggtcagaa gaagcaagct ggagtagcca ttctaatatc 2760

aaataaaatc aatttccaac taaaagtcat caaaaaagat aaggaaggac acttcatatt 2820

catcaaagga aaaatccacc aagatgaact ctcaatccta aatatctatg ccccaaatac 2880

aagggcacct acatacgtaa aagaaacctt actaaagctc aaagcacaca ttgcacctca 2940

cacaataata gtgggagatt tcaacacacc actctcatca atggacagat catggaaaca 3000

gaaattaaac agtgatgtcg acagcataag agaagtcatg agccaaatgg acttaacgga 3060

tatttttaga acattctatc ctaaagcaaa aggatatacc ttcttctcag ctcctcatgg 3120

cactttctcc aaaattgacc atataattgg tcaaaaaacg ggcctcaaca ggtacagaaa 3180

gatagaaata atcccatgcg tgctatcgga ccaccacggc ctaaaactgg tcttcaataa 3240

caataaggga agaatgccca catatacctg gaaattgaac aatgctctac tcaatgataa 3300

cctggtcaag gaagaaataa agaaagaaat taaaaacttt ttagaattta atgaaaatga 3360

aggtacaaca tacccaaact tatgggacac aatgaaagct gtgctaagag gaaaactcat 3420

agcgctgagt gcctgcaaaa agaaacagga aagagcatat gtcagcagct tgacagcaca 3480

cctaaaagct ctagaacaaa aangaagcaa atacactgag gaggagtaga aggcaggaaa 3540

taatcaaact cagagctgaa atcaaccaag tagaaacaaa aaggaccata gaaagaatca 3600

acagaaccaa aagctggttc tttgagaaaa tcaacaagat agataaaccc ttagccagac 3660

taacgagagg acacagagag tgtgtccaaa ttaacaaaat cagaaatgaa aagggagaca 3720

taactacaga ttcagaggaa attcaaaaaa tcatcagatc ttactataaa aacctatatt 3780

caataaaact tgaaaatctt caggaaatgg acaatttcct agacagatac cacgtatcga 3840

agttaaatca ggaacagata aaccagttaa acaaccccat aactcctaag gaaatagaag 3900

cagtcattaa aggtctccca accaaaaaga gtccaggtcc agacgggttt agtgcagaat 3960

tctatcaaac cttcatagaa gacctcatac caatattatc caaactattc cacaaaattg 4020

aaacagatgg agcactaccg aattccttct acgaagccac aattactctt atacctaaac 4080

cacacaaaga cacaacaaag aaagagaact tcagaccaat ttcccttatg aatatcgatg 4140

caaaaatact caataaaatt ctggcaaacg aattcaagag cacatcaaaa caatcatcca 4200

ccatgatcaa gtaggcttca tcccaggcat gcagggatgg tttaatatac ggaaaaccat 4260

caacgtgatc cattatataa acaaactgaa agaacagaac cacatgatca tttcattaga 4320

tgctgagaaa gcatttgaca aaattcaaca cccttcatga taagagtccc ggaaagaata 4380

ggaattcaag gcccatacct aaacatagta aaagccatat acagcaaacc agttgctaac 4440

attaaactaa atggagagaa acttgaagca atcccactaa aatcagggac tagacaaggc 4500

tgcccactct ctccctactt attcaatata gttcttgaag ttctagccag accaatcaga 4560

caacaaaagg agatcaaggg gatacagatc ggaaaagaag aagtcaaaat atcactattt 4620

gcagatgaca tgatagtata tttaagtgat cccaaaagtt ccaccagaga acgactaaag 4680

ctgataaaca acttcagcaa agtggctggg tataaaatta actcaaataa atcagttgcc 4740

ttcctctata caaaagagaa acaagccgag aaagaaatta gggaaacgac acccttcata 4800

atagacccaa ataatataaa gtacctcggt gtgactttaa ccaagcaagt aaaagatctg 4860

tacaataaga acttcaagac actgaggaaa gaaattgaag aagacctcag aagatggaaa 4920

gatctcccat gctcatggat tggcaggatt aatatagtaa aaatggccat tttacccaaa 4980

gcaatctaca gattcaatgc aatccccatc aaaataccaa tccaattctt caaagagtta 5040

gacagaacaa tttgcaaatt catctggaat aacaaaaaac ccaggatagc taaagctatc 5100

ctcaacaata aaaggacttc agggggaatc actatccctg aactcaagca gtattacaga 5160

gcaatagtga taaaaactgc atggtgattg gtacagagac agacagatag accaatggaa 5220

tagaactgaa gacccagaaa tgaacccaca cacctatggt cacttgattt ttgacaaagg 5280

agccaaaacc atccaatgga aaaaagatag cattttcagc aaatggtgct ggttcaactg 5340

gagggcaaca tgtagaagaa tgcagatcga tccatcctta tcaccctgta caaagcttaa 5400

gtccaagtgg atcaaggacc tccacatcaa accagacaca ctcaaactaa tagaagaaaa 5460

actagggaag catctggaac acatgagcac tggaaaaaat ttcctgaaca aaacaccaat 5520

ggcttatgct ctaagatcaa gaatcgacaa atgggatctc ataaaactgc aaagcttctg 5580

taaggcaaag gacactgtgg ttaggacaaa acggcaacca acagattggg aaaagatctt 5640

taccaatcct acaacagata gaggccttat atccaaaata tacaaagaac tcaagaagtt 5700

agaccgcagg aaacaaataa ccctattaaa aatggggttc agagctaaac aaaaattcac 5760

agctgaggaa tgccgaatgg ctgagaaaca cctaaagaaa tgttcaacat ctttagtcat 5820

aagggaaatg caaatcaaaa caaccctgag atttcacctc acagtagtga gaatggctaa 5880

gatcaaaaac tcaggtgaca gcagatgctg gcgaggatgt ggagaaagag gaacactcct 5940

ccattgttgg tgggattgca gactggtaaa accattctgg aaatcagtct ggaggttcct 6000

cagaaaattg gacattgaac tgcctgagga tccagctata cctctcttgg gcatataccc 6060
aaaagatgcc tcaacatata aaagagacac gtgctccact atgttcattg ccgccatatt 6120
tataatagcc agaaactgga aagaacccag atgcccttca acagaggaat ggatacagaa 6180
aatgtggtac atctacacaa tggaatatta ctcagctatc aaaaacaacg agtttatgaa 6240
attcgtaggc aaatggttgg aactggaaaa tatcatcctg agtgagctaa cccactcaca 6300
gaaagacata catggtatgc actcattgat aagtggctat tagcccaaat gcttgaatta 6360
ccctagatcc ctagaacaaa cgaaactcaa gacggatgat caaaatgtga atgcttcact 6420
ccttctttaa atgaggaaaa agaataccct tggctgggaa gggagaggca aagattaaaa 6480
cagagactga aggaacaccc attcagagcc tgccccacag gtggcccata catatacagc 6540
cacccaattg gacaagatgg atgaagcaaa gaagtgcaga ctgacaggag ccggatgtag 6600
atcgctcctg agagacacag ccagaataca gcaaatacag aggcgaatgc cagcagcaaa 6660
ccactgaact gagaataggt cccccgttga aggaatcaga gaaagaactg gaagagcttg 6720
aaggggctca agaccccaaa agtacaacaa tgtcaagcaa ccagagcttc cagggactaa 6780
gccactacct aaatactata catggactga ccctggactc tgaccccata ggtagcaatg 6840
aatatcctag taagagcacc agtggaaggg gaagccctgg gtcctgctaa gactggaccc 6900
ccagtgaact agactatggg gggagggcgg caatgggggg aggttgggga gggggacacc 6960
cataaggaag ggggaggggg agggggatgt ttacccggaa accgaaaggg aataacactt 7020
aaattatata agaaatactc aagttaatta aaaataataa taataataat aaaaagaatt 7080
actcataaaa taaagacaag atgatggata aaattaaaaa aacaaaaaaa acaaaaaaaa 7140
accaaaatgg aaacatgaaa atgctcacta cttacaatag tgctttatgt aataagcaaa 7200
tgactgcatt tgacacgagt tataatttat taacccctta gttagactac ttgtacacat 7260
ggtgattatt aatccacaat tattcttttt cactaaaatc cttatatttt attctttgag 7320
attataatat aatcacacaa attccctctt ccctgtcatc tcttcaaaac cttccatata 7380
gtcctccatg ctgtcttttg aacgttatgg cttctttttt atattcttgt tttatattat 7440
atatatataa tatatattat aatatatatc atactatata tattcctcag gactgaccaa 7500
tccatattgg ataaccaatt ggtgtgctcg tctcagggaa agaccatttc cccaccatca 7560
gcattctcgg tttccagtag ttcattgtct agaatggaga ctgtgtgtgc tttccccgtc 7620
cactttagct gctgtgtttc agctcctctt gaggcagtta tgttggtgag acctcatctg 7680
tgcggcttcc aacattcctc agagacacag tgagttacac agtgtcacct gatcctctaa 7740
ctcccaccat ctttcctgct gtcttctcca gggtttcctg aacctcagca ggagcagggg 7800
ttgtactgta gacacgccca ccttcactag gatccacagc tcctcacttt gatttgctgt 7860
ggttttctgc aatggcttcc atgtcctagt aaagtcatgg agaccccata accacgctgt 7920
actaaaccag acaatcccta gctgcattct aagttcttgt ccttatccca tacatgtgca 7980
tttagagagt ctgcttattt tgctcattat agaacgagtc aatcattctc tttgagttgt 8040

aaaagctt                                                              8048

<210>  225

<211>  2472

<212>  DNA

<213>  Rattus norvegicus (Norway rat)

<400>  225
ccccgcatgt ccccaagacg gagtgtgtgt tggttcgtac tgggccagct gctgtgcagc      60

tgcgccgtgg cgctgcaggg cgggatgctg ttcccgaagg agaccccttc gcgggaactc     120

aaggtgctgg acggcctgtg gagcttccgt gccgattact cgaacaatcg gttgcagggc     180

ttcgagaagc agtggtaccg gcagccgcta cgggagtcgg gcccaacctt ggacatgccg     240

gtcccttcca gcttcaatga catcacccaa gaagcagagc ttcggaactt cattggctgg     300

gtgtggtatg aacgggaagc agtgcttcca cagcgatgga cccaggacac cgacaggaga     360

gtggtgttga gaatcaacag cgcccattac tatgcagttg tgtgggtgaa tgggattcat     420

gtggtggaac atgagggagg tcacctcccc tttgaggctg acatcaccaa gctggtccag     480

agtgggcccc tgaccacctt ccgggtcacc atcgccatca acaacacact gaccccttat     540

acccttccac cggggaccat tgtctacaag actgatcctt ccatgtatcc caagggttac     600

ttcgtccagg acataagctt cgacttcttc aactatgcgg ggctgcaccg gtctgtggtc     660

ctgtacacca cccctaccac ctatatcgat gatatcactg tgaccactga cgtggaccgg     720

gacgttgggc tggtgaacta ctggatttct gtccagggca gtgaccattt ccagctagaa     780

gtgcgtcttc tggatgagga tggcaaaatt gtggcccgtg gaacagggaa tgagggtcaa     840

cttaaggtgc cgcgtgccca cctctggtgg ccttacctga tgcatgagca tccagcctac     900

ctgtactcct tggaggtgac gatgacaaca cctgagtctg tgtctgactt ctataccctc     960

cctgtcggga ttcgaacagt ggctgtcaca aagagcaagt tcctcataaa tgggaagcct    1020

ttctacttcc aaggcgtcaa caagcatgag gattcagata tccgagggag aggcttcgac    1080

tggcctctgc tgataaagga tttcaacctc ctccgttggc tcggggcaaa ttcctttcgt    1140

accagccact atccctactc ggaggaggta cttcagctct gtgaccgata tggaattgtg    1200

gtcatcgatg agtgtcccgg tgtgggcatc gtgctgcccc agagttttgg caacgtgtct    1260

cttcggcacc acctagaggt gatggacgag ctggtgcgca gggacaaaaa tcaccctgcg    1320

gtcgtgatgt ggtctgtggc caatgagcct gtctcttctc tgaaacctgc cggatattac    1380

ttcaagacgc tgatcgccca caccaaagcc ctggacccca cccgtcctgt gacctttgtg    1440

agcaatacca gatatgacgc agacatgggg gccccgtacg tggacgtgat ttgtgtgaac    1500

agttacttat cctggtatca tgactacggg catctggagg tgattcagct gcagctgact    1560

agccagtttg agaactggta taagatgtac cagaagccaa ttatccagag cgagtatgga    1620

gcagacgccg tctcggggct tcatgaggat ccacctcgca tgttcagtga ggagtaccag 1680

acagctctcc tggagaatta tcatttgatc ctggatgaga aacgaaaaga atatgtcatc 1740

ggagagctca tctggaattt tgctgacttc atgacgaacc agtcaccact gagagtaaca 1800

ggaaacaaga aggggatctt cactcgacag agaaacccca agatggcagc cttcattttg 1860

cgagagagat actggaggat tgccaacgaa accagaggtt acggttcagt gccgaggacc 1920

cagtgtatgg gaagcagacc gttcacattc taaagctacg actacctcac cgcagacagg 1980

gcctgatgaa gtgaaaggta ttctctcagc agaagagttt ttcctctgga gtttgtgaac 2040

caacacaagg aggactttag aagtgacatg gaagttttcc ttgtgtgggg ataatgactt 2100

gcagtgcctt aggctcagta atgtgtcctg ctgagaagtg tcccaggtca tttctgtcag 2160

gttttttttg gccttggctt tgtgtactct tgaaaaccta ctgtgtgaat actcacacta 2220

agagctcctg gttaccggct aagggacggc tcacaattta agaatactga ctgttctacc 2280

aggggttcca gattcgattc ctagcaccca cgtggtcgtt aactatgagc tgttgcccca 2340

tttctcaggt gatgtacgaa catacatgtg gggggagggg aatagtcata cgtgtaaaat 2400

gaaaataaat ctaaataaaa ctgttttctt tcattataaa aaaaaaaaaa aaaaaccaac 2460

taatagaaac gt 2472

<210> 226

<211> 2462

<212> DNA

<213> Rattus norvegicus (Norway rat)

<400> 226
gatggctgca ccatgagcgt ctctgcactg agctccaccc gcttcacggg cagcatctct 60

ggcttcctcc aagtggcctc cgtgcttggt ctgcttctgc tgctggtcaa agcagtccag 120

ttctacctgc aaaggcaatg gctactcaag gctttccagc agttcccatc acctcccttc 180

cactggttct ttgggcacaa gcagtttcaa ggtgacaaag aactacagca aattatgaca 240

tgtgtggaga atttcccaag tgcctttcct cgatggttct ggggaagcaa agcctactta 300

attgtctatg accctgacta catgaaggtg attctcgggc gatcagatcc aaaggccaat 360

ggcgtctaca gattgctagc tccttggatc ggatatggtt tgctcttgct gaatggacaa 420

ccgtggttcc agcaccggcg aatgctaacc ccagccttcc actatgacat tctgaaaccc 480

tatgtaaaaa acatggctga ctccattcga ctgatgctag acaaatggga acagctggca 540

ggtcaagact cctctataga aatctttcaa catatctcct taatgaccct agacactgtc 600

atgaagtgtg ccttcagcca caatggcagt gttcaggtgg atggaaatta caagagctat 660

atccaggcca ttgggaactt gaatgacctc tttcactccc gtgtgaggaa catctttcat 720

cagaatgata ccatctataa tttttcttcc aatggccact tgttcaaccg tgcttgtcaa 780

cttgcccatg atcacacaga tggtgtgatc aagctaagga aggatcagct gcagaatgcg 840
ggagagctgg aaaaggtcaa gaagaaaaga cgtttggatt ttctggacat cctcttactt 900
gccagaatgg agaatgggga cagcttgtct gacaaggacc tacgtgctga ggtggacaca 960
tttatgttcg agggtcatga caccacagcc agtggagtct cctggatctt ctatgctctg 1020
gccacacacc ctaagcacca acaaagatgc agagaggaag ttcagagtgt cctgggggat 1080
gggtcctcca ttacctggga tcacctggac cagattccct acaccaccat gtgtatcaag 1140
gaggccctga ggctttaccc acctgttcca ggcattgtca gagaactcag cacatctgtc 1200
accttccctg atgggcgctc tttacccaag ggtatccaag tcacactctc catttatggt 1260
ctccaccaca acccgaaggt gtggccaaac ccagaggtgt ttgacccttc caggtttgca 1320
ccagactctc cccgacacag ccactcattc ctgcccttct caggaggagc gaggaactgc 1380
attgggaaac aatttgctat gagtgagatg aaggtgattg tggccctgac cctgctccgc 1440
tttgagctac tgccagatcc caccaaggtc cccatcccct taccacgact tgtgctgaag 1500
tccaaaaatg ggatctacct gtatctcaag aagctccact aattccgttg tggagctccg 1560
aaatctgaaa tgagtttcac tggcagaaag ctgagttggt ggtgtgacta gccttcttca 1620
gaagagtgct tcagagagtc ctctcctcct ctcttcagta cagatcaccc ttctcagcac 1680
tggaatattc ctctgcttta aagccagcac ccttcccata ccccctcttc taaaagcctt 1740
cccttttaca aatgttctta tgacatcatc aagaccactg aaaaactcca agataatttc 1800
ccatctcaat attccttact ccatctaacc tactaagtcc cttttgaatt atgaggaata 1860
attcaatttg ttccatgggc tccaaaactc aaggcctgag cattattgtg aaacctttat 1920
tcagcctaat atcatcttca caagactgtt acctggtacg ttcatctaaa tctcccctgc 1980
atagtctctc tacctgacta ttcctcacac aagtttcttt accttccctc ctttctccaa 2040
taaagtgtcc agtgtcctgc acaaaaagct caaggagaac tgattatcac cttctgattc 2100
gttcattgat gcattcaaat taaacctcca catagtagag actttttcaa ctattataaa 2160
aaccatcctg agccagacct gcagtcaaca gcaagagcag gaagcgcata ggaactacac 2220
ctgcaaccaa gctggcacaa agaccaagaa ttctgaagca gcccaaactc aagatgacat 2280
atttttacaa gttagagaaa aatcaagatc tgagttatct tgacaaactc gggatggaaa 2340
gtaggaggga ggggaaagca aataaatact tccttattgt gtagcataaa aaaaccgaat 2400
tcgtaggagg gaggggaaag caaataaata cttccttatt gtgtagcata aaaaaaccga 2460
at 2462

<210> 227

<211> 982

<212> DNA

<213> Rattus norvegicus (Norway rat)

<400>  227
gggagctgac agcagccacg cggggaagat ggctgaggac ttcggcttct tctcgtcgtc      60

ggagagcggg gcccccgagg ccgccgagga ggacccggcg gccgccttcc tggcccagca     120

ggagagcgag attgctggca tcgagaatga ctcgggtttc ggggcacctg ccgccagcca     180

ggtggcctct gcgcagcccg gactcgcgag cgggggtggt tcggaggaca tggggactac     240

agtcaatgga gatgtgtttc aggaggctaa cgggcctgcc gatggctacg ctgcgattgc     300

ccaggcggac aggttgactc aggagcctga gagcatccgc aagtggagag aggagcagaa     360

gaaaaggctg caggagttgg atgctgcctc gaaggtgacc gaacaggagt ggcgggagaa     420

ggccaaaaaa gacctggagg agtggaacca gcgccaaagt gaacaggttg agaagaacaa     480

gatcaacaac aggatcgctg acaaagcgtt ctaccagcag ccagatgctg ataccattgg     540

ctatgtggca tcggaagagg cttttgtgaa agaatccaag gaggagaccc caggcacaga     600

gtgggagaag gtggcccagc tgtgtgactt caaccctaag agcagcaagc aatgtaaaga     660

cgtgtcccgc ctgcgctcgg tgctcatgtc cctgaagcag acgccactgt cccgctagtg     720

cctgtcacca cgggccttgg tggggcagag cagcagctgc ttcagccagg gtggaacttc     780

tctggcagct gccacacacg cctgttctgt tcctctgagt ctctgggagc tgggaagcgg     840

gacccttacc cctttcaccc accctgtcct tcctggtccc ctgttccagc ccctcatgac     900

tcctgtcagt ccacttgatt gtgactgtcc ctcctgatgt atttttcttg gcttaaaggg     960

tgtgttaact ctttttacac tt                                              982

<210>  228

<211>  662

<212>  DNA

<213>  Rattus norvegicus (Norway rat)

<400>  228
   tttgttatta caggaaagag gatatgtgca ggagaagccc tggctcgcac ggagctgttt      60
   ttgttcttca ccaccatttt acagaatttt aacctgaagt ctctggttga tgtaaaagac     120
   attgacacaa caccagctat cagtggattt ggccatttgc ccccttttta cgaggcttgt     180
   tttattcctg tgcaaagggc agactctcta agctctcatc tgtaatgtct cttctgaggg     240
   tcctgtctac ttcattcttg gtactatagt agctttaact cacatatccc catttccttc     300
   ggatccagtg aacatcaaac ctcattgagt tgagttccct gagtcaatat atagttctat     360
   tcctgttccc tatatcttgt gacgttccct atatcttgtg acattcccat gcagtactta     420
   catagttagt gctaatactt gtatgacttc attactgtta atactgtttt cactatataa     480
   aagcaaaata ttttagaata tgagaattca gagtcatctg ttcccttcat gtgctaaata     540
   aatactaatt tttggaccag ttcatagact ctttttttta atgggtataa tgtaaggaag     600
   aagtaaagaa aacaaactct caggagacca tatttgtctt catttataca aaaagggacc 660
   ga 662

<210> 229

<211> 405

<212> DNA

<213> Rattus norvegicus (Norway rat)

<400> 229
aaatatagag tggccctgag caggcttcct cagcagattc atgattatct gaatgcatct 60

gactgggaga gacaagtagc tggtgccaag gaaaaattaa cttctttcat ggaaaactat 120

agaattacag ataatgatgt actaattgcc ttagatagtg ccaaaatcaa cttgaatgaa 180

aaactctctc aacttgagac atacgcgata taatttgatc agtatattag agataattat 240

gatgcacagg acttaaaaag aactattgct cagattattg atagaatcat tgaaaagcta 300

aaaatgcttg acgaacagta tcatatccgt gtaaatctag caaaatcaat ccataatctc 360

tatttatttg ttgaaaatgt tgatcttaac caaatcagta gtagt 405

<210> 230

<211> 1195

<212> DNA

<213> Rattus norvegicus (Norway rat)

<400> 230
gatcccattc tttatgaaag tccgcctagt ggatatcaag ggagatccta tcccaaatga 60

gcaggtcttc atcaaagcac aagaagctgg ctacaccaat gctactacca ctcatcagca 120

tggcttggca aagttctcca tagataccag cagcatctca ggctattccc tcaatatcaa 180

agtctaccac aaggaggaaa gttcatgtat ccattcctct tgcacagcag aaagacacgc 240

agaggaacat catacggcct atgctgttta ctccctcagc aagagctaca tctaccttga 300

cacagaagct ggcgtcttgc cctgcaacca gatccacaca gttcaggcgc attttattct 360

gaaggggcag gtcttgggag tgctgccaca gattgttttc cactacctgg tcatggccca 420

gggcagcatc ctccagactg gaaaccacac tcatcaagtg gagccaggag tatctcaagt 480

acaaggcaac ttcgccttgg agatccctgt ggagttcagc atggttcccg tggctaaaat 540

gctcatctac accatcttgc ctgatggaga agtgattgca gattctgtaa cgtttcaagt 600

tgaaaaatgt cttcgcaaca aagtgcacct gagcttcagc ccatcccaaa gtcttccagc 660

ctcacaaacc catatgcggg ttacagcttc tcctcagtcc ctctgtggcc tgagagctgt 720

ggaccaaagc gtgctgctgc tgaagcctga ggctgagctc tccccttcct tgatatatga 780

tctaccaggt atgcaagaca gcaacttcat tccaagttct tatcatccat ttgaagacga 840

atatgattgt ttgatgtacc aaccaagaga cactgaagag ctcacatact cagtaccata 900

tggacgagag aaggatgtct acagatatgt gagggacatg ggtttaacag cattcaccaa 960

cttgaagatc aaacatccta catattgtta tgaaatgaac atggttgtac tatcagcacc 1020

tgctgtggaa tctgaacttt caccaagagg gggagagttt gaaatgatgc ccttgggagt 1080

aaataaaagt ccactaccta aagactcctc cacacgtgaa gaaccaagac agcaagataa 1140

atggaaagga attaaaaacc cataaaattt atatgttgaa taaagtacga cattt 1195

<210> 231

<211> 831

<212> DNA

<213> Rattus norvegicus (Norway rat)

<400> 231
ttgacaagga cttgttccac tgtctttcct tcactatccc tagattgccc tcttctgaag 60

aggaggagtc actcgacatc aacattgaag gggcaaagca taaattcagc gaaaggcgtg 120

tggtgcttgt gaagaacaaa gaaagtgttg tctttgtcca gactgataag cccgtgtaca 180

agccaggaca gtcagttaaa tttcgggttg tctctatgga taaaaatcta caccccctga 240

atgagttgtt tcctctggct tacattgagg atccgaaaat gaaccgaatt atgcagtggc 300

aggacattaa gacagagaat gggcttaagc aattgtcctt cagcctgtca gcagagccca 360

ttcagggccc ctacaagata gtgattctca aacagtcagg agtgaaggaa gagcattcct 420

tcaccgtcat ggaattcgtg cttcccagat ttggtgtcga tgtgaaggtc ccaaacgcca 480

tctctgtgta cgatgaaata atcaatgtga ctgcctgtgc gatatatacc tatgggaagc 540

ctgttccagg atgccatgga aatccttcct ttagctctga gacaaagtct gcatgcaaag 600

aagaggactc cgagctagac aacaacggct gcagcacaca agaagtgaac atcactgagt 660

tccaattaaa ggaaaattac ctcaaaatgc accaggcttt ccatgtgaat gcaactgtta 720

cagaagaagg gacagggtca gagttcagtg gatctggaag aattgaagtc gaaagaacca 780

gaaacaaatt cctatttctg aaagcagact cacacttcag acacgggatc c 831

<210> 232

<211> 2389

<212> DNA

<213> Rattus norvegicus (Norway rat)

<400> 232
gtctcaggca gaggtccaga ctcagtggaa gcagaggaga gagcctgaaa cctggcgagc 60

accatgagca acaaatgcga tgtgatcgtg gtgggggcgg gcatctcagg tatggcagca 120

gccaaacttt tgcatgactg tggcctcagt gtggtggttc tggaagcacg agactgtgtg 180

ggaggcagga cttacacaat taggaataaa aatgttaaat atgtggacct tggaggatct 240

tatgttgggc cgacccagaa tcgtatctta cgattggcca aagagctagg attggagacc 300

tataaagtga atgaagttga gcggctgatc cactttgtaa agggaaaatc atatgccttc 360

aggggcccat tcccaccagt gtggaatcca atcacttacc tagattataa caacctctgg 420

agaacaatgg atgagatggg ccaagagatt cccagtgatg ctccatggaa ggcacccctt 480

gctgaagagt gggactacat gacaatgaaa gagttgctag ataagatctg ctggaccaac 540

tctacaaagc agattgccac actctttgtg aacctatgtg taactgcgga gacccatgag 600

gtttctgcac tgtggttcct gtggtatgtg aagcagtgtg ggggtacaac cagaatcata 660

tcaacaacca atggaggaca ggagaggaaa tttattggtg gatctggtca agtgagtgag 720

cggataaagg atatccttgg ggacagagtg aagctggaga ggccggtgat ccacattgac 780

cagacaggag aaaatgttgt tgtgaaaacc ctaaaccatg aaatatatga ggctaaatat 840

gtgattagtg ccatcccacc tgttttgggc atgaagattc accatagtcc tcctctgccc 900

attctaagaa accagctgat tactcgtgtg cctttgggtt cagttattaa gtgcatggtt 960

tattataaag aacccttctg gaggaaaaag gatttctgtg gaaccatggt tattgaagga 1020

gaggaagctc caattgcgta cacattggat gataccaagc cagatgcagg ctgtgctgct 1080

ataatgggat ttatccttgc tcacaaagct agaaaactgg tacgccttac taaagaagaa 1140

agactgagga agctctgtga gctatacgcg aaagttctga actctcaaga agctctgcag 1200

ccagtccatt atgaagagaa gaactggtgt gaggagcagt actccggggg ctgctacaca 1260

gcctacttcc ctcctggcat cttgacccag tatggaaggg ttctacgcca gccagtgggc 1320

aagattttct ttgcaggcac cgagacagct tcacattgga gtggctacat ggagggggct 1380

gtagaggctg gagagagagc tgccagagag attcttcatg ccattgggaa gattccagag 1440

gatgaaattt ggcagccaga accagaatct gtggatgtcc cagcaagacc cattaccaac 1500

accttcctgg agagacactt gccttctgta ccaggtctac taaagctgct tggattgacc 1560

accatcttgt cagcaacagc tcttggtttc ctggcccaca aaaagggtct gtttgtacgt 1620

ttctaaagat gggctttagg accatatcca caggtttctc attcagtgtg tcacaaaagc 1680

ttttggaagg agttgggata aaaatctgac aaaggtgcag agattatgga gtgagaaagc 1740

acagtaactt ggtctccatt ttggctatct tttagcatcg ctgtggtcca ctcattttca 1800

actttcctgc actctgaata ttgagaacag atacacaggc tctctcacaa cctacctgcc 1860

ctatgcacat agttgttttt caaaacccta tgcctttgtg cttgtctttc ttctggtgtg 1920

ttaggtcctc acctatatca agttcttcat cattgtacct agaatcctgt cttgttagaa 1980

ccagaaggca ttagacactg tagcttattg tctactttag agttaaataa accaaatgca 2040

acagaagtga aatctaacca cacaaggcct acacaaagct actggtattt gggtgactgg 2100

aacacaagct gatgcttttc tcacctccca aggttcattc ccctgtgatc ctcctccacc 2160

ttatgtcata gtcattcacg gatcattgtt cttgtggatt tactctgtat taactggtat 2220

tgtgttactc agtagattct tctaggcttg ctattttgtg tagtgttgcc agctgattct 2280

aatttttctt gagaatggga gtcttgtctt tgtcatttct tttttgcatc ttccagtatg 2340

cttccactca tagatttaag acatgcttaa ataattaaaa ataaagctg 2389

<210> 233

<211> 3053

<212> DNA

<213> Rattus norvegicus (Norway rat)

<400> 233
cccccgagag ctttgtgaag gaggaacgct gcacacccgc ctcccgcagc acacagccaa 60

ccacagctga gcgacacgca acaagagcca atcacaaggc acttccgaaa gctccaggat 120

gcagatgtct tcagccctca cttgcctcac cctgggcctg gttctggtct ttgggaaagg 180

gttcgcttca ccccttccag agtcccatac agcccagcag gccaccaact tcggagtaaa 240

agtgtttcag catgtggtcc aggcctccaa agaccgaaat gtggtcttct ctccctacgg 300

cgtgtcctcg gtgctggcta tgctgcagct gaccacagca gggaaaaccc ggcagcagat 360

ccaagatgct atgggattca atatcagtga gaggggcaca gctcctgccc tccgaaagct 420

ctccaaggag ctcatggggt catggaacaa gaatgagatc agtactgcgg acgccatctt 480

tgtccagcgg gacctagagc tggtccaggg cttcatgccc cacttcttca agctcttccg 540

gaccacggtg aagcaggtgg acttctcaga ggtggaaaga gccagattca tcatcaacga 600

ctgggtggag aggcacacca aaggtatgat cagtgactta ctggccaagg gggctgtaaa 660

tgagctgaca cgcctggtgc tggtgaacgc cctctatttc aacggccaat ggaagacccc 720

cttcttagag gccagcaccc accagcgcct gttccacaag tctgatggta gcaccatctc 780

cgtgcccatg atggctcaga acaacaagtt caactacact gagttcacca ctccggatgg 840

gcacgagtac gacatcctgg aactgcccta ccacggcgaa accctcagca tgttcattgc 900

agcacccttt gaaaaagatg tgcccctctc cgccatcacc aacattttgg acgctgagct 960

catcagacaa tggaagagca acatgaccag gctgccccgc ctcctcatcc tgcctaagtt 1020

ctctctggag actgaagtgg acctcagagg gcccctggag aagctgggca tgactgacat 1080

cttcagctca acccaggccg acttcacaag tctttccgac caagagcagc tctctgtagc 1140

acaagcacta caaaaggtca agatcgaggt gaacgagagc ggcacagtgg cgtcttcctc 1200

cacagccatt ctagtctcag cccgcatggc ccccacggag atggttttag accgatcctt 1260

tctctttgtg gttcggcaca atccaacaga gacaatcctc ttcatgggcc agctgatgga 1320

gccttgagag tgggatgaga agcctttcct ttgggacaaa actggacgtg ttataagcag 1380

agactctgaa gaaaagaatt gttttaagga ctctttgggg agaaagagaa ggcctttctt 1440

tcttaccccg gcactggtaa atctttccaa ccagcctccc agacctcaga ctctcgaaga 1500

ggaaagagtc taactccctc actagggacc tatcttacta aggtctcatc caaccataga 1560

actcacagaa tctggatctg cccagcattc agcctttgga cccagttccc accaaggccc 1620

cagcagggcc aacccactac gccttcactc agcaaagtct tttgtgttcc agtcacactc 1680

tgggtacctc ttgtatcgtc ctccattgct atgaaggatg acccaggcca aaggaagaag 1740

cactgtccta tctcaaggtc cactgtggaa atgaacacct tgcccatccc caaggggcag 1800

cagatagaca gatcgaatga tcgcccgata tcaagccttc tcccagctcc cgtcctgccc 1860

tcccttccct gacagccgcc ttgtgttatt tcagagtgta gatgacttgt ttacagcttt 1920

tttcgaccca caaacttttc tcattttgaa agcgtgaaag aaaggtcaga tgtgcacgtg 1980

ccttgctctt tatcctgggt ctccctgtga ggggagaggg gtcctgggga gattccaggg 2040

gtgtgattga atatttatct tgtttatctt atacgtttgt tggggagaag aagcactatt 2100

aaggagaaag ccttttattt aaaccatggc atatggtgtc ccatttgggg tctgtatccc 2160

tgtatgtcag ggaggcatca ctccacaaac ccgcccctcg ggtggcccgg cgtcggggct 2220

cacactgccg cctagtggca gccgaacacg cccttgcccc atccctcccc cgcatcctcc 2280

cccgtggctc ttttccttag ggatcttgcc aaggtgatgc ttggcagccc acggtaaagg 2340

aagggggaaa aagattaggt gggagagaga gagagagaga gagagagaga gagagagaga 2400

gagagagaga gagagagaga gagagagaga aagagagaga gatgtttgag agagggcaaa 2460

gtggtttcaa atttttccaa tacattcaga agccgagtgg gaaagggggc tgtgtgacct 2520

aacaggacag aactttctcc aattactggg tgactcagct gcactggtga ctcacttcaa 2580

tgtgtcattt ccggctgctg taagtgagca gtggacacgt gggggggggg gggtgaggat 2640

gaaagaaaca gccagctcct ggtcaaccac cttagttaga taatcttttt tgaaagcttc 2700

ctagctgggg gtatgatcag aaaaccaatt tactgaaaaa ctgcacagga aggtaacgtg 2760

aatctaattt catagcgggc cgctctgcat ccgttacatc tccactggaa aaaaataatc 2820

attttctttt tgtgtgtgtg tgtgtgtttt agcttttctc cctctccctc tttctctctc 2880

atttcattat gcactggata accatacacc gtgtaccaca ggggcccaaa tgtggggtca 2940

catggtcttg aattttgtgg ggtacatatg cctttgtttg tttgttttca cttttgatat 3000

ataaacaggt aaatgtgttt ttaaaaaata ataaaaatag agaatatgca gac 3053

<210> 234

<211> 1160

<212> DNA

<213> Rattus norvegicus (Norway rat)

<400>  234
ttgtcagcaa gacctcgctc cccttacagt aactctcatc tagacgtcgc aactccgcca      60

ccatgtttga ggcacgcctg atccagggct ccatcctgaa gaaggtgctg gaggccctca     120

aagacctcat caatgaggcc tgctgggaca tcagttcggg cggcgtgaac ctacagagca     180

tggattcgtc tcacgtctcc ttagtgcagc ttactctgcg ctccgaaggc ttcgacacat     240

accgctgcga tcgcaacctg gccatgggcg tgaacctcac cagcatgtcc aaaattctaa     300

aatgtgctgg taatgaagac atcattacat taagggctga agataatgct gataccttag     360

cactagtatt tgaagcacca aatcaagaga aagtttcaga ctatgagatg aagttaatgg     420

acttagacgt tgagcaactt ggaatcccag aacaggagta cagctgcgta gtaaagatgc     480

catctggtga atttgcacgt atatgccggg accttagcca tattggagat gctgtggtga     540

tctcctgtgc aaaggacggg gtgaagtttt ctgcgagtgg ggagcttggc aatgggaaca     600

ttaagttgtc ccagacaagc aatgttgata aagaagagga agctgtgtcc atagagatga     660

atgagccagt tcagctaact tttgctctga ggtacctgaa ctttttcaca aaagccactc     720

cactgtctcc tacagtaaca ctcagtatgt ctgcagatgt accccttgtt gtagagtata     780

aaattgctga catgggacac ttaaagtatt atttggctcc caagatcgaa gatgaagaag     840

gatcttaggc gttgctagaa attgagaaac taaacctttg acgatcgctt ctgagatgcc     900

agcgtgttct gaggtctttt ctgtcaccaa gtttgtacct gagtattctt aaatattaaa     960

ataaaatgta gatatcttct gtaaataacc tttttttccc tctccataat ttgcttacag    1020

aataagctcc aaagtaaaaa ctggttttgt taacataaat gtctctgcct tacaaatact    1080

ggtgatttcc ataaatgatc ttgacgctaa atgcagtttt aagaaatatt tttcaattta    1140

aataaagtta actgaatttg                                                1160

<210>  235

<211>  318

<212>  DNA

<213>  Rattus norvegicus (rat)

<400>  235
ccatattgga gatgctgtgg tgatctcctg tgcaaaggac ggggtgaagt tttctgcgag      60

tggggagctt ggcaatggga acattaagtt gtcccagaca agcaatgttg ataaagaaga     120

ggaagctgtg tccatagaga tgaatgagcc agttcagcta acttttgctc tgaggtacct     180

gaactttttc acaaaagcca ctccactgtc tcctacagta acactcagta tgtctgcaga     240

tgtacccctt gttgtagagt ataaaattgc tgacatggga cacttaaagt attatttggc     300

tcccaagatc gaagatga                                                  318

<210>  236

<211>  601

<212>  DNA

<213>  Rattus norvegicus (Norway rat)


<400>  236
ggcggcttct ctcgtctcct tgctttttgc gccgcgcgtc tcccggggaa gcatggcgat      60

gaaggccgtg tgcgtgctga agggcgacgg tccggtgcag ggcgtcattc acttcgagca     120

gaaggcaagt ggtgaaccag tggtggtgtc aggacagatt acaggattaa ctgaaggcga     180

gcatgggttc catgtccatc aatatggaga caatacacaa ggctgtacca ctgcaggacc     240

tcattttaat cctcactcta agaaacatgg cggtccagcg gatgaagaga ggcatgttgg     300

agacctgggc aatgtggctg ctggaaagga cggtgtggcc aatgtgtcca ttgaagatcg     360

tgtgatctca ctctcaggag agcattccat cattggccgt actatggtgg tccacgagaa     420

acaagatgac ttgggcaaag gtggaaatga agaaagtaca aagactggaa atgctggaag     480

ccgcttggct tgtggtgtga ttgggattgc ccaataaaca ttccctatgt ggtctgagtc     540

tcagactcat ctgctggcct gctaactgta gaaaaaaacc aaaccattaa actgtaatct     600

t                                                                     601

<210>  237

<211>  1904

<212>  DNA

<213>  Rattus norvegicus (Norway rat)


<400>  237
tcccccatca aaaccactaa ataagaaatg ccctacaacc ggatcttatg gaggcatttt      60

ctcaattgag gctttcttct ttcaagttga cagaaaacta agcaggacac ttccccaccc     120

ttctccccac tggatgagcg gacgtgccct gcactgggtg aatgaggaca ttactgaccg     180

ctccttcctg gggtttggct cctgctctca cggcagacac tgaaaatcac aggctatgcg     240

agttggagcc agtttgtccc aaaccgattt tagatctctg taccctctct aggctatgct     300

ctaaggagct cgtctctagg gccagccagt gctccagctg aggctgagag agatggcaca     360

cagtaggggt gctggagcag cccctccggg aagaggtctg gggccatgtc aggacccggc     420

agcctctgat tgtgtcctac cctccccttc caggccttcg ccccggcagc tgcagtcacc     480

aaccacagca tcctttgggt ttgacctatt gcgcacatga cccccaatta gtcctggcag     540

catctcctgc ccctcctgct tacattaggg gctcaggcag tggatataaa aggtattgca     600

gaacagacaa gtcccacaca gcaactcggt gcagacctag cagacaccat gaggaccctc     660

tctctgctca ctctgctggc cctgactgca ttctgcctct ctgacctggc aggtatgtgc     720

cctcctggtt catttctttg gatagccacc ctcctgaagg tctcgggatc tgctttggga     780

tggcagaagg gaagggacaa actaccagag agacagacag cagggaggga acagaactaa 840

ctgcactgtt ttctttacag gtgcaaagcc cagcgactct gagtctgaca aaggtactga 900

cagggagcct ggcagaacct cagccttgcc cctcaccctg tcccctaagc ccccagaccc 960

ctctgccttc tgtctgggtg tccccacttt ccctcccaaa cccagaatta cctgatcctg 1020

tgcctctgtt ccacagcctt catgtccaag caggagggca gtaaggtggt gaatagactc 1080

cggcgctacc tcaacaatgg acttgggtga gtggggagga ggcgctgggc cagggccctt 1140

tgctttccat attggtaggg aggacttgtg ctgggtggtc tctatgaact gccgagggtc 1200

ccatgcctag gaccctggag cctcttgtgc agtgggaaga gtgtgtgtat accccgtgtt 1260

ggttaatgcc actgcgtatt ggttgacgct actatatgct tctcagagcc ccagccccct 1320

acccagatcc cctggagcct cacagggagg tgtgtgagct caaccccaat tgtgacgagc 1380

tagcggacca cattggcttc caggacgcct acaagcgcat ctatggcacc accgtttagg 1440

gcatgtgttg ccctggagcc caacggcagc ttcagctttg gctactctcc aggactcgac 1500

cctccctgtt ccctctctct gcctcgaaag tatggacggc acagctgctc caaaataaag 1560

tccagatgag gaacggttgg gctcgagtct gtccattgtg gtggtggtag gggttggaga 1620

aggcttcatg ttcaatccca gacagcccca gtccggttca tttgcgtctt tattatactg 1680

ctctgtgatg gatcgcacca gaggtccaca gccgcctccc cacagtgatt cccaccacat 1740

ccattatctc accgccaaca cagacacacc cagacatgca gatcagaaaa ggcctggtca 1800

cagtggtaag caggtcaggc agctcacgtg tagacagagt tgctgacacg tggcagtctg 1860

gtcctggggc agggtgggta tgtggccctc atggaacaaa gctt 1904

<210> 238

<211> 2297

<212> DNA

<213> Rattus norvegicus (Norway rat)

<400> 238
agacctgaaa ggggaatttc ctaaatctcc aggctggaca gtttgtttct ccctctcacc 60

tcctataact catctgagtc ttatctccat atcacatttc taagtctctt gtactcagag 120

tcattgtgac tcttgggagg ccagagccag gctgaacaga gtccttccct gcagtactct 180

atgagaatat gtctttgggt ttgaaagaaa ggtggtgtta tcacctacat tggctccagc 240

ggatcctccc ccagccggac cagcccagag tccctctata gtgacagctc caatggcagc 300

ttccagtccc tgactcaagg ttgtcccaca tacttcccac catcacctac tggctccctc 360

acccaggacc ctgcccgctc atttggcact gtgccaccca gcctcagtga tgatagctcc 420

ccttcttctg cttcatcatc atcatcatct tcctcctcct ccttctataa tgggagcccc 480

cccggaagtc tacaagtggc catggaagac agcagccgag tgtcccccag caagggcacc 540

agcaacatta ccaagcttaa cggcatggtg ctactgtgta aagtgtgtgg ggacgtggcc 600

tcaggcttcc actatggcgt gcatgcctgc gagggctgca agggcttttt ccgccggagc 660

atccagcaga acatccagta caagcggtgt ttgaaaaacg agaactgctc cattgtccgt 720

atcaatcgca accgctgcca gcagtgtcgc ttcaagaagt gtctctccgt tggcatgtct 780

agagacgctg tgcgttttgg acgtatcccc aagagagaga agcaacgaat gcttgctgag 840

atgcagaacg ccatgaactt ggccaacaac caactgagca gcctgtgccc tctagagacc 900

tcacctgccc cgcaccccac ctcaggctcc gtgggcccct caccacctcc tgcaccagcc 960

ccgacacctt tggtgggctt ctctcagttc ccacaacagc tgacaccacc cagatccccg 1020

agtcctgagc ccacggtgga ggatgtgata tcccaggtgg ccagggccca tcgagaaatc 1080

ttcacctatg cccatgacaa attaggcacc tcacctggca acttcaatgc caatcatgca 1140

tcaggtagcc ctccggctac cactccacag tgctgggaga gtcagggatg cccgtctacc 1200

cccaacgaca acaacctttt ggcggctcag cgtcataatg aagcactgaa tggtctacgc 1260

cagggcccct cctcctaccc tcctacctgg ccttctggcc ctgcccacca cagctgccac 1320

cagcctaaca gcaatggcca tcgcctgtgc cccacccacg tatactcggc cccagaaggc 1380

aaggcacctg ccaacggtct acggcaaggc aacaccaaga atgttctgct ggcatgtccc 1440

atgaacatgt atccccatgg acgtagtggc cggactgtgc aggagatctg ggaagacttc 1500

tctatgagct tcacacccgc tgtgcgggag gtggtagaat ttgccaaaca gatccccggc 1560

ttccgtgacc tttctcagca cgaccaggtg accctgctta aggctggcac ctttgaggtg 1620

ctgatggtgc gctttgcgtc attgttcaac gtgaaggacc agacagtcat gttcctgagc 1680

cgcacaacct acagcctgca ggagctcggt gccatgggca tgggtgacct gctcaatgcc 1740

atgtttgact tcagcgagaa gctcaactct ctggcgctta ctgaggagga gctgggcctt 1800

ttcacggcag tggtacttgt ctctgcagag ggctcgggaa tggagaattc cgcttcggtg 1860

gagcagctcc aggagacgct gctgggggct cttcgggctc tcgtgctgaa gaaccggccc 1920

tcggagactt cccgcttcac caaactgctg ctcaagctgc cggacctgcg gaccctgaac 1980

aacatgcatt ccgagaagct gctgtccttc cgggtggacg cccagtgacc cgcccggccg 2040

gccttctgcc gctgccccct tgtacagaat cgaactctgg acttctctct cctttacgag 2100

acgaaaagga aaaccaaacc agaatcttat ttatattgtt ataaaatatt ccaagatgag 2160

cctctggccc cctgagcctt cttgtaaata actcttcccc cccaccccca tgctcccttc 2220

ctcccgtatt taaaccactc ttgctctccc accctcctct ggcccctcga tttgttctgt 2280

tcctgtctca aatccaa 2297

<210> 239

<211> 516

<212> DNA

<213>  Rattus norvegicus (rat)

<400>  239
aggaccagac agtcatgttc ctgagccgca caacctacag cctgcaggag ctcggtgcca     60

tgggcatggg tgacctgctc aatgccatgt ttgacttcag cgagaagctc aactctctgg    120

cgcttactga ggaggagctg ggccttttca cggcagtggt acttgtctct gcagagggct    180

cgggaatgga gaattccgct tcggtggagc agctccagga gacgctgctg ggggctcttc    240

gggctctcgt gctgaagaac cggccctcgg agacttcccg cttcaccaaa ctgctgctca    300

agctgccgga cctgcggacc ctgaacaaca tgcattccga gaagctgctg tccttccggg    360

tggacgccca gtgacccgcc cggccggcct tctgccgctg cccccttgta cagaatcgaa    420

ctctggactt ctctctcctt tacgagacga aaaggaaaac caaaccagaa tcttatttat    480

attgttataa aatattccaa gatgagcctc tggccc                              516

<210>  240

<211>  3291

<212>  DNA

<213>  Rattus norvegicus (Norway rat)

<400>  240
   acaatacgat agcaacatga cagttatgaa gtagcaacaa aaaataaatt tagggtgtgt     60
   gtgtgggggg atggtcaaca caacatgagg aactgtatta aaggtcgaga agcactgctc    120
   taaaagaaaa acataggaag gactctcttc ttcaatctct tacactggaa ctcagctaaa    180
   agcctttccc ttccatctaa agcagttccc accctctggc gtcccctggc tttcagtgca    240
   ttcccaggtg atctgagatc ctgaagctag ctattctcat cctgttgccc tgggagtttc    300
   agcactcctg gtacaacctg ttcctcgctc ctcctgctct tttcctcctc cccttcctgt    360
   cttgcggcag gggccccagc tgaaccctcc agagagacac tctccactcc gtccccaaag    420
   gagaaacgcc cgggggagga gcagaggagg gagccacaga gggcggaggc cggggagctc    480
   agccagagag gggctgctgg aggttgcatc tgctggaagg aggtttttcc cgctgcctgg    540
   taaccagctg ccagaacact gagagggaga gagagaggca gggagcaggg gcagcagctt    600
   cttgacgtca ggaccagcca aggatcgggc cagctacccc ggccagccta gagtcagcgg    660
   ggccctcggc tggagaggag acctacgcga ggcttggagc gtgagttggg ggtgtgggaa    720
   ccggttgtct ggggcgcagc atgccccctg cccccggacc atggagatct ccctggtgcc    780
   cctggagaat ggcagtgcca tgaccctcag aggaggaggg gaggcagggg caagctgtgt    840
   gcagaccccc aggggagagt gtgggtgccc tccgacgtct ggactcaata atcagtccaa    900
   agaaacactg ttgagggggc gcacaacgct cgaggatgcg aaccagggtg gacggccttt    960
   gccccctatg gctcaggagc tgccacaacc tagaaggcta tctgctgagg atgaggaggg   1020
   agaaggcgac cctggcctgg gcacagtgga ggaggaccag gctcctcagg atgcagggtc 1080
   actccatcac cagcgagtcc tcataaacat ctccgggttg cgtttcgaga cgcagctggg 1140
   caccctggca cagtttccca acaccctcct gggggaccca gccaagcgcc tgcactactt 1200
   cgaccccttg agaaatgaat acttcttcga ccgcaaccgg cccagcttcg atggcatttt 1260
   gtactactac cagtctgggg gccgcctgcg caggcccgtc aatgtctccc tggatgtgtt 1320
   tgcagatgag atccgctttt accagctggg ggacgaggcc atggagcgct tccgggagga 1380
   tgagggcttc atcaaggaag aggagaagcc cctgccccgc aacgagttcc agcgccaggt 1440
   atggcttatc ttcgaatacc cagaaagctc tgggtccgca agagccatcg ccatagtgtc 1500
   ggtcctggtc attctcatct ctatcatcac cttctgcctg gagactctgc ctgagttcag 1560
   ggatgagcgg gagctgctac gccatccccc agtgccgccc cagcccccag cccctgcccc 1620
   tgggatcaat ggcagcgtct ctggagcact ttcctctggc cctacggtgg ctccactctt 1680
   gcctaggaca ctggccgatc cattcttcat cgtggagacc acatgtgtga tctggttcac 1740
   ttttgagttg ctcgtgcgct tctttgcctg ccccagcaag gcagaattct ctcggaatat 1800
   catgaacatc atcgatgtcg tggccatctt cccctacttt atcaccctgg gcaccgagct 1860
   ggcagagcaa caaccagggg gagggggtca gaatggcgag caggccatgt ccctagccat 1920
   cctcagggtg atccgcctgg tgcgggtgtt tcggatcttc aagctctccc gccactccaa 1980
   gggactgcag atcctgggta agaccttgca agcatccatg cgggaactcg ggctactcat 2040
   cttcttcctc ttcattggag tcatcctctt ctccagcgct gtctacttcg cagaggcaga 2100
   caatcacggg tcccatttct ctagtatccc agatgccttc tggtgggcag tagtcactat 2160
   gaccactgta ggctatggag acatgagacc catcactgta gggggcaaga tcgtgggttc 2220
   actgtgcgcc atagctgggg tcctcaccat tgccctgcct gtccccgtca tcgtctccaa 2280
   ctttaattac ttctatcatc gggagacaga ccacgaggag caggctgccc tgaaggaaga 2340
   acaaggcaac cagaggcggg agtctgggct ggacacaggg ggtcaacgga aggtcagctg 2400
   cagcaaggcc tccttttgca agactggggg ttccctggag agttctgaca gtatcagaag 2460
   gggtagctgt cctctagaaa agtgtcacct caaggccaag agcaacgtgg acttgcggag 2520
   gtccctgtat gccctctgtc tggacactag ccgtgaaaca gatttgtaaa gagagatcca 2580
   ggcagactgg caccagtgaa gctggccaca ggggtgcccc ttgagcctgg gcatctgctt 2640
   tacaccaccg agtatttaag cccacctggt cacccaccga tgtctgcctt tctttcaccc 2700
   ttctcacctt cctgccctct aacttcccca ttttaactcc tctttccata acccccaggg 2760
   tcgcctattt ttaacaagta tgacattcca tgacgcagga gccgttgaag tgctgagccc 2820
   tcactggaag atggatgcat tcatagccag ttttctacac ccagcagagg gataatcaaa 2880
   caaatttgac tttaaaaagc ttagatccca agagaatcca tagcattccc tacccgtgtt 2940
   ccaaatttac tttacatggg attacatttg tgtatgggca aatattattt ttatggccga 3000
   tgactgcatt ttgtacagca gtgcacatga agaatatttg ggtgtgctct taagatatac 3060
   tttgaattat gaaagtgttg ggggggtcac catttcctgg gacactcagg gacaaagacc 3120
   ctaaaagagt tgttcaggtt tctgtgttct caagcttctt attattctgg gatgtactat 3180
   tggtgcttct ggtctaggag agagaatgtt ctagaagacc agcaaatcta atcttgtgac 3240
   tgtcttcaca gaagcatttt aaggatgttg gaagaaggat ttggagaatt c 3291

<210> 241

<211> 147

<212> DNA

<213> Rattus norvegicus (Norway rat)

<400> 241
tcaatttccc caaaagccaa aaattgggag acaattttac atggactttg gaaaacattt 60

ttttcctttg cattcatctc tcaaacttag tttttatctt tgaccaactg aacgtgacca 120

aaaaccaaaa gtgcattcaa ccttacc 147

<210> 242

<211> 4346

<212> DNA

<213> Rattus norvegicus (Norway rat)

<220>

<221> misc_feature

<222> (31)..(31)

<223> n

<400> 242
ataagcgcag ttccttccgc cctggtctcc nttggctgga gacggcggaa gggcggcttg 60

gtggcctcca ttgagatccg gaggatccaa ggactgtgta gctacaatgt tgtcaagact 120

tttccgtatg catggcctct ttgtggcctc ccatccctgg gaggtaattg tgggaacggt 180

gacacttact atctgtatga tgtccatgaa catgttcacc ggcaacaaca agatctgtgg 240

ttggaattat gagtgcccaa aatttgaaga ggacgtgctg agcagcgaca tcatcatcct 300

cacgataacc cggtgcatcg ccatcctgta catctacttc cagttccaga acctgcgtca 360

gcttgggtca aagtacattt tgggtattgc cggcctcttc acaattttct caagtttcgt 420

cttcagcact gtcgtcattc atttcctcga caaagaattg acaggcttaa atgaagcttt 480

gccctttttc ctgctcttga ttgacctttc tagagcgagt gcattggcca agtttgccct 540

gagttcaaac tcacaggatg aagtaaggga gaatatagcg cgtggatggc cgatcctggg 600

ccccacgttc acccttgacg ctctggtgga atgtcttgtg attggagttg gcaccatgtc 660
aggggtgcgg cagcttgaga tcatgtgctg ctttggctgt atgtccgtgc ttgccaacta 720
ctttgtcttc atgacattct tcccagcctg cgtgtccctg gtcctagagc tttctcggga 780
aagccgtgag ggtcgtccaa tttggcagct cagccatttt gccagagttt tagaagaaga 840
agagaataaa ccaaacccag taacccaaag ggtcaagatg atcatgtctt taggcctggt 900
tcttgttcac gctcacagtc gctggatagc tgatccttct cctcagaaca gcacagcaga 960
acagtctaag gtttccttgg gtctggctga agatgtgtcc aagagaattg agccgagtgt 1020
ttctctctgg cagttttacc tctccaagat gatcagcatg gacatcgagc aagtgattac 1080
cctgagctta gcgttgcttt tggctgtcaa gtatattttc tttgaacaag cagagacaga 1140
atcaacactc tcattaaaaa atcctatcac atctcctgtc gtgaccccaa agaaagctca 1200
agacaactgt tgtagacgtg agcctctgct tgtgaggagg aaccagaagc tttcgtcagt 1260
ggaggaggat ccaggagtga accaagacag aaaagttgag gttataaaac ctttagtggc 1320
agaagccgag acttcgggca gagctacgtt tgtgcttggc gcctctgcag ccagccctcc 1380
attggccctg ggggcacagg agcctgggat cgaactcccc agcgagcctc gacctaatga 1440
agagtgtcta cagatactgg agagtgcaga gaaaggtgcg aagttcctta gtgatgcaga 1500
gatcatccag ttggtcaatg ctaagcacat cccagcctac aaactggaaa ccctcatgga 1560
gacgcacgag cgtggtgtgt ctattcgccg gcagctcctc tccgccaagc ttgcagagcc 1620
atcttctctg cagtacctgc cttacagaga ctataattac tccttggtga tgggagcttg 1680
ctgtgagaac gtgatcggat atatgcccat ccctgttgga gtggcaggac ctctgtgcct 1740
ggatggaaaa gagtaccagg tgccaatggc aacaacagaa ggttgtcttg tggccagcac 1800
gaacagaggc tgcagagcga tcagtcttgg tggaggtgcc agcagccggg tccttgcaga 1860
tgggatgagc cgaggcccag tggtgcgtct tcctcgtgct tgtgactcag cagaggtgaa 1920
gagctggctt gaaacacctg aagggtttgc agtggtaaag gaggccttcg acagcacgag 1980
cagatttgca cgtctacaga aacttcacgt gacgctggca ggacgcaacc tctacatccg 2040
tctccagtcc aaaacggggg acgccatggg gatgaacatg atttccaagg gtacggagaa 2100
agcacttctg aagctgcaag agggcgtgcc ggagctgcag atactggcgg tcagtggtaa 2160
ctattgcacc gacaagaaac ctgctgccat aaactggatc gaagggagag gaaagactgt 2220
ggtttgtgaa gctgtcattc cagccaaggt ggtgagagaa gtattaaaga cgactacgga 2280
agctatggtt gacgtaaaca ttaacaagaa tcttgtgggc tctgccatgg ctggtagcat 2340
aggaggctac aacctccatg ctgccaacat cgtcactgcc atctacattg catgtggcca 2400
ggatgcagca cagaatgtgg ggagttcaaa ctgtattacg ttaatggaag caagtggtcc 2460
cacaaatgaa gacttataca tcagctgtac catgccgtct atagagatcg gaaccgtggg 2520
tggtgggacc aaccttctac ctcagcaagc ctgcctgcag atgctaggtg ttcaaggggc 2580
gtgcaaagac aatcctggag aaaatgcacg gcagcttgca cgaattgtgt gtggcactgt 2640

gatggctggt gagttgtcct tgatggcagc attggcagca ggacatcttg tcagaagtca 2700

catggttcac aacagatcaa agataaattt acaagatctg caaggaacat gcaccaagaa 2760

ggcagcttga gcatcctgac atactggact gaaacacggg cattgggttc tccaggacta 2820

acatgaaatc tgtgaattaa aaatgtcagt gcagtgtctt gtggaagatg aacgtgatca 2880

gtgaggcagg cacctgcttg gtttctggcc tctttagaga cgtctgcggt cctttgcaca 2940

gactcctcag acgtgggaac tatggttctt tccgtgccgt gttctagaaa atctcatgtg 3000

gatgtcatgt gctctgagca ccacagatgt gactgcagct cgtttctgaa agctgccaca 3060

agctggaagc tggtgttttg acgaaatgat ggatcctggt gatcagtgtg gggctcacct 3120

ccaatgggtt aaaatggagt tttaaatgac actgtagctg acagaacttc tgatttttat 3180

ttattcagtc tgggttgtag aactttgcaa tctaagttta ttttttgtaa actaataatt 3240

catttggtgc tggtctattg atttttgggg acctacttct tcatggaaga attactttta 3300

ttctcaaact acagaataat gtgctaagta gtgctaaata gttctcgacg aagaaaagag 3360

tcgctgtgtt catctcagtc tttgttcaga gaggccttta gctgtgcaca gtctgcacta 3420

aagacacaga gctcttagtg ttagagcaga gtacaagcag gtcggacctc tcaggactta 3480

gagtgtggga gcagttttta aagaaaacaa ccattgctct aagccaactt tcttgggaga 3540

cattttaact tatttagctg aattctagat ttcttggtaa actatcaaat ctgtatatgt 3600

tgtaataaag tgtctaatgc taggagttta ttggaagtgt ttaagaaata aaagtactca 3660

tcagctttta cacggataaa atgctctagc ttgggccaga gaagacctcg ctcaggagca 3720

ttggtccggg aaagcctggc ttgcttgccg agcctaatga agggaagtca gcctacagag 3780

ccagtgatgg agccacatga atggccctgg agctgtgtgc cttgttcctg tggccaggag 3840

ttaggtgact gaatcattta tgggctcctt tggtggaccc cataaaagct cttagcttcc 3900

ctcagggggg gtcagcagag ttgttgaatc ttaatttttt ttttaatgta ccagtttgtg 3960

ataaataata ataaagagct ccttattttg tattctatct aatgcttcgt ttggtcttgg 4020

aagcggacgt ctgtgtagaa gacggactct gaaagtgagc atcgatgacg cgcgcgcgcg 4080

cgactgacgc tagtgatggc tagatatgtg gaaggtagct tcgtgtgtat gtaaagtttc 4140

cagttgcgcc tcttgtgact tttcagccag taccatttta tttacctgag cttgtcgtgg 4200

gaagtggtag taaaccgcat tgagtgttga tgctggtgac ttgtgatcag tgtcatcgtg 4260

ctaagtccat gttttgtaca attactaaat tgtatacatt ttgttataga atactttttc 4320

cagttgagta attatgaaag gaagtt 4346

<210> 243

<211> 2352

<212> DNA

<213> Rattus norvegicus (Norway rat)

<400> 243
gggagcagcg agcaagcagg tcctcagcgt ccagtcaccg ctctaagcca ggcgccatgc 60
atcccgcgcg ccccgcgctc tgggcggctg cgctcaccgc cctcactctg ctccgcggac 120
cgccagtggc gcgggccggc gcgggcgcgg tgggcgcggg ccccgtggtg cgctgcgaac 180
cgtgcgacgc gcgtgcgctg gcccagtgcg cgcctccgcc caccgcgccc gcgtgcacgg 240
agctggtgcg agaacccggc tgcggctgct gcctgacttg cgcgctgcgc gaaggcgacg 300
cgtgcggcgt ctacacggag cgctgtggca ccggcctccg ctgccagccg cgaccggccg 360
agcagtatcc cctgaaggcg ctgctgaatg gccgcgggtt ctgcgccaac gccagcgccg 420
ccagcaacct gagtgcctac ctcccctccc agccgtctcc tggaaacacc actgagtctg 480
aggaggacca caatgctggg agtgtggaaa gccaggttgt ccccagcaca catcgcgtga 540
ctgattccaa gttccatcca ctccattcaa agatggaggt catcataaaa ggccaggcta 600
gggacagcca gcgctacaaa gttgactatg agtcccagag cacagacacc cagaacttct 660
cctccgagtc taagcgggag acagaatatg gtccctgccg cagagaaatg gaggacacac 720
tgaatcatct gaagttcctc aatgtgctga gtcccagggg cgtccacatc ccaaactgtg 780
acaagaaggg gttctataag aagaaacagt gtcgcccttc caaaggcaga aagcggggct 840
tctgctggtg cgtggacaag tacgggcagc cattgccagg ctatgacacc aaggggaaag 900
acgacgtgca ttgcctcagc gtgcagagcc agtagatacc gctgtgccac ttaacgtgga 960
gctcaaatac gccttatttt gcacaaaaga ctgccaacaa cgtgatcagc agctggctat 1020
accttgattt atatttctct ctctctctct ctctctctct ctctctctct ctctctcttt 1080
tgtggtgaac tgaataaaaa caaacaaaac acatacaaaa acaaaaacaa aaaaaaaagc 1140
caagtttaga cagatttctg aaatgcctct ggttgtttaa atagtgaact tggtcatctt 1200
tgtatctcgc agtagtcaac caaaagcagt ttgaattttc ttgttgcttc ctatgaaaac 1260
cacacgtgta ctccaggcca cggatgccgt cgccccctaa ctcacccacc cactgtgggc 1320
ttcagtgctg ctggccctct gccttcttga tttcagaggc tctgttgctg atagagaaaa 1380
accctctttc catcccctgt aagtaagtgc aggcactgtg gagaatgggg aagcctggaa 1440
cccagtgacc cggacgtctg gaagcatcct cctgaggcct ctggtcctta ttgtgccatc 1500
tctgaatcaa gggcctggcc ctgtatctgc aagtggcctg acctacttgg gaactgtggg 1560
agagaaaaat gtgttgtctc tcttactaaa aatgactaag aatgttctag ggcgctccga 1620
gagcccataa agacaaggac aaggaccttc ctttgtcagg cagcttcctg atgacttggc 1680
ccagcagaaa tatcaaactc catgtgcaga gatgtcgcaa ataacggtgc gcttagttct 1740
ccggatgact tcaagaaaac agtgttttct ggcccagcct ctcaaaataa aatttgttgt 1800
ggggtggggc tgaggggagg cagctttcaa aagagagaag gttttcatct tccttgttgg 1860
agaccctggt aagaacatgg agagaatcac ctgtttgttg atcttggggt ccttctcaaa 1920
ctttctttat aattcatgcg tatatgcaga caaaatatgt tcttaattgt taacattgta 1980

tacaacatag cccaaatata ttagaatctg tactagataa tcctagataa aaggttagag 2040

atgctaggtg atgtaaccac agacacgccc gaggaaagga gcctgtgtct ggaggctggg 2100

ccgctttccc cgaggccaag gccatggtgg tctggcaata cagggtgtga ggagactgta 2160

ctgcatccca cggggtggac atgcgctgta cagagctttc cttgagagca caaaggaatc 2220

ttgagacatt ctgcctgcct gtcagctttt ctttattttt ttaattaagt ttttggggga 2280

aaaatgtatt tttgaaaagt ttgtcttgca atgtatttat aaatagtaaa taaagttttt 2340

ttactattta ag 2352

<210> 244

<211> 1341

<212> DNA

<213> Rattus norvegicus (Norway rat)

<400> 244
ccagcaattt catctcctag acctcatcag actctgaccc agttcttgaa tgactttgga 60

gacccagatg tttcagaatg cacattctgg aagccaatgg ctactcccac cactgacaat 120

gttgctgctg tttgcttttg cagacaggca gacgggagat cttctgaagg ctgtggtgaa 180

acgtgatccc ccatggatcc aggtgctcaa ggacgacact gtgacgctga cgtgcgaagg 240

gacccacaat cctggaaact cttctaccca gtggttccac aaccagagct ccacctgggg 300

ccaggtccaa gccagctaca cgtttaaggc cacagtcaat gacagtggag aataccggtg 360

ccgaatggcg cacaccagcc tcagcgaccc catacatctg gaagtgattt ctgactggct 420

gctgctccag acccctcaac tggtgtttga ggaaggggaa accatcacat taaggtgcca 480

tagctggaag aacaaacagc tgactaaggt cttgttgttc cagaatggaa aacctgtgag 540

gtattattat caaagcagca atttttcaat ccccaaagcc aaccacagtc acagtgggaa 600

ctactactgc aaagcatatc taggaaggac aatgcatgtg tccaagcctg tcaccatcac 660

tgtccaaggt tcagcaaccg cgtccaccag ctctctagtg tggttccatg ccgctttctg 720

cctagtgatg tgcctcctgt ttgcagtgga caccggcctg tatttctgtg tacggagaaa 780

tcttcaaacc tcgggggagg actggaggaa atccctgtca gtcggaaagt acaaggctcc 840

acaggacaaa tgacatccca tcgtatggct aaacagcggc agcttctttt cagccacacc 900

gccgcttcat ctacagcctt cctttgaaag caacttacca ccaggctgga tatttggtcc 960

tgcaatcaca actgctagga ggctgaggca ggaggatcac caaaggctgc ccgggtttta 1020

gagaagagag agtgcaagtc tatcctggat aacccaataa gaccctgggt ttagatggct 1080

tatcaggaaa gagcacccat tgctaagctc acagacaaga ccacagattg atccctaagg 1140

acacagggca aaggggagaa acaaattcct gaaagttgtc ctctgacctc cacacgtcca 1200

ccatggcaga tgcacacaat aaacaaatta aaatgtaata tatatatttt aaacaagagg 1260

tggggaaagg ctgggggttg gtggcacagt ttttatccag gacacatgat gctctggatt 1320

ttgacctcct ataataaaac g 1341

<210> 245

<211> 574

<212> DNA

<213> Rattus norvegicus (rat)

<400> 245
tgtttgcagt ggacaccggc ctgtatttct gtgtacggag aaatcttcaa acctcggggg 60

aggactggag gaaatccctg tcagtcggaa agtacaaggc tccacaggac aaatgacatc 120

ccatcgtatg gctaaacagc ggcagcttct tttcagccac accgccgctt catctacagc 180

cttcctttga aagcaactta ccaccaggct ggatatttgg tcctgcaatc acaactgcta 240

ggaggctgag gcaggaggat caccaaaggc tgcccgggtt ttagagaaga gagagtgcaa 300

gtctatcctg gataacccaa taagaccctg ggtttagatg gcttatcagg aaagagcacc 360

cattgctaag ctcacagaca agaccacaga ttgatcccta aggacacagg gcaaaggggg 420

agaacaaatt cctgaaagtt gtcctctgac ctccacacgt ccaccatggc agatgcacac 480

aataaacaaa ttaaaatgta atatatatat tttaaacaag aggtggggaa aggctggggg 540

ttggtggcac agtttttatc caggacacat gatg 574

<210> 246

<211> 4127

<212> DNA

<213> Rattus norvegicus (Norway rat)

<400> 246
agccgctgct ggggaggttg gggctgaggt ggtggcgggc gacgggcctc gagacgcgga 60

gcgacgcggc ctagcgcggc ggacggccga gggaactcgg gcagtcgtcc cgtcccgcca 120

tggaaatgga gaaggaattc gagcagatcg ataaggctgg gaactgggcg gctatttacc 180

aggatattcg acatgaagcc agtgacttcc catgcagaat agcgaaactt cctaagaaca 240

aaaaccggaa caggtaccga gatgtcagcc cttttgacca cagtcggatt aaattgcatc 300

aggaagataa tgactatatc aatgccagct tgataaaaat ggaggaagcc cagaggagct 360

atatcctcac ccagggccct ttaccaaaca cgtgcgggca cttctgggag atggtgtggg 420

agcagaagag caggggcgtg gtcatgctca accgcatcat ggagaaaggc tcgttaaaat 480

gtgcccagta ttggccacag aaagaagaaa aagagatggt cttcgatgac accaatttga 540

agctgacact gatctctgaa gatgtcaagt catattacac agtacggcag ttggagttgg 600

agaacctggc tacccaggag gctcgagaga tcctgcattt ccactacacc acctggcctg 660

actttggagt ccctgagtca cctgcctctt tcctcaattt cctattcaaa gtccgagagt 720

caggctcact cagcccagag cacggcccca ttgtggtcca ctgcagtgct ggcattggca 780

ggtcagggac cttctgcctg gctgacacct gcctcttact gatggacaag aggaaagacc 840

cgtcctctgt ggacatcaag aaagtgctgt tggagatgcg caggttccgc atggggctca 900

tccagacggc cgaccaactg cgcttctcct acctggctgt gatcgagggt gcaaagttca 960

tcatgggcga ctcgtcagtg caggatcagt ggaaggagct ttcccatgaa gacctggagc 1020

ctccccctga gcacgtgccc ccacctcccc ggccacccaa acgcacattg gagcctcaca 1080

atggcaagtg caaggagctc ttctccaacc accagtgggt gagcgaggag agctgtgagg 1140

atgaggacat cctggccaga gaggaaagca gagccccctc aattgctgtg cacagcatga 1200

gcagtatgag tcaagacact gaagttagga aacggatggt gggtggaggt cttcaaagtg 1260

ctcaggcatc tgtccccact gaggaagagc tgtccccaac cgaggaggaa caaaaggcac 1320

acaggccagt tcactggaag cccttcctgg tcaacgtgtg catggccacg gccctggcga 1380

ctggcgcgta cctctgttac cgggtatgtt ttcactgaca gactgctgtg aggcatgagc 1440

gtggtgggcg ctgccactgc ccaggttagg atttggtctg cggcgtctaa cctggtgtag 1500

aagaaacaac agcttacaag cctgtggtgg aactggaagg gccagcccca ggaggggcat 1560

ctgtgcactg ggctttgaag gagcccctgg tcccaagaac agagtctaat ctcagggcct 1620

taacctgttc aggagaagta gaggaaatgc caaatactct tcttgctctc acctcactcc 1680

tcccctttct ctggttcgtt tgtttttgga aaaaaaaaaa aaagaattac aacacattgt 1740

tgtttttaac atttataaag gcaggttttt gttattttta gagaaaacaa aagatgctag 1800

gcactggtga gattctcttg tgccctttgg catgtgatca gattcacgat ttacgtttat 1860

ttccggggga gggtcccacc tgtcaggact gtaaagttcc tgctggcttg gtcagccccc 1920

ccaccccccc accccgagct tgcaggtgcc ctgctgtgag gagagcagca gcagaggctg 1980

cccctggaca gaagcccagc tctgcttccc tcaggtgtcc ctgcgtttcc atcctccttc 2040

tttgtgaccg ccatcttgca gatgacccag tcctcagcac cccacccctg cagatgggtt 2100

tctccgaggg cctgcctcag ggtcatcaga ggttggctgc cagcttagag ctggggcttc 2160

catttgattg gaaagtcatt actattctat gtagaagcca ctccactgag gtgtaaagca 2220

agactcataa aggaggagcc ttggtgtcat ggaagtcact ccgcgcgcag gacctgtaac 2280

aacctctgaa acactcagtc ctgctgcagt gacgtccttg aaggcatcag acagatgatt 2340

tgcagactgc caagacttgt cctgagccgt gatttttaga gtctggactc atgaaacacc 2400

gccgagcgct tactgtgcag cctctgatgc tggttggctg aggctgcggg gaggtggaca 2460

ctgtgggtgc atccagtgca gttgcttttg tgcagttggg tccagcagca cagcccgcac 2520

tccagcctca gctgcaggcc acagtggcca tggaggccgc cagagcgagc tggggtggat 2580

gcttgttcac ttggagcagc cttcccagga cgtgcagctc ccttcctgct ttgtccttct 2640

gcttccttcc ctggagtagc aagcccacga gcaatcgtga ggggtgtgag ggagctgcag 2700

aggcatcaga gtggcctgca gcggcgtgag gccccttccc ctccgacacc cccctccaga 2760

ggagccgctc cactgttatt tattcacttt gcccacagac acccctgagt gagcacaccc 2820

tgaaactgac cgtgtaaggt gtcagcctgc acccaggacc gtcaggtgca gcaccgggtc 2880

agtcctaggg ttgaggtagg actgacacag ccactgtgtg gctggtgctg gggcaggggc 2940

aggagctgag ggtcttagaa gcaatcttca ggaacagaca acagtggtga catgtaaagt 3000

ccctgtggct actgatgaca tgtgtaggat gaaggctggc ctttctccca tgactttcta 3060

gatcccgttc cccgtctgct ttccctgtga gttagaaaac acacaggctc ctgtcctggt 3120

ggtgccgtgt gcttgacatg ggaaacttag atgcctgctc actggcgggc acctcggcat 3180

cgccaccact cagagtgaga gcagtgctgt ccagtgccga ggccgcctga ctcccggcag 3240

gactcttcag gctctggcct gccccagcac accccgctgg atctcagaca ttccacaccc 3300

acacctcatt ccctggacac ttgggcaagc aggcccgccc ttccacctct ggggtcagcc 3360

cctccattcc gagttcacac tgctctggag caggccagga ccggaagcaa ggcagctggt 3420

gaggagcacc ctcctgggaa cagtgtaggt gacagtcctg agagtcagct tgctagcgct 3480

gctggcacca gtcaccttgc tcagaagtgt gtggctcttg aggctgaaga gactgatgat 3540

ggtgctcatg actcttctgt gaggggaact tgaccttcac attgggtggc tttttttaaa 3600

ataagcgaag gcagctggaa ctccagtctg cctcttgcca gcacttcaca ttttgccttt 3660

cacccagaga agccagcaca gagccactgg ggaaggcgat ggccttgcct gcacaggctg 3720

aggagatggc tcagccggcg tccaggctgt gtctggagca gggggtgcac agcagcctca 3780

caggtggggg cctcagagca ggcgctgccc tgtcccctgc cccgctggag gcagcaaagc 3840

tgctgcatgc cttaagtcaa tacttactca gcagggcgct ctcgttctct ctctctctct 3900

ctctctctct ctctctctct ctctctctct ctctaaatgg ccatagaata aaccatttta 3960

caaaaataaa agccaacaac aaagtgctct ggaatagcac ctttgcagga gcggggggtg 4020

tctcagggtc ttctgtgacc tcaccgaact gtccgactgc accgtttcca acttgtgtct 4080

cactaatggg tctgcattag ttgcaacaat aaatgttttt aaagaac 4127

<210> 247

<211> 562

<212> DNA

<213> Rattus norvegicus (Norway rat)

<400> 247
agagactccc caagggattt cgtgtaccag ttcgagggcc agtgctacta caccaccggg 60

acgcagcgca tgcggctcgt gaccagacac atctacaacc gggaggagta cgtgcgcttc 120

gacagcgacc tgggcgagta ccgcgcgctg accgagctgg ggcggccctc agccgagtac 180

tggaataagc agtacctcga gcagacgcgg gccgagctgg acagggtctg cagatacaac 240

tacgaggggc cgggggctct cacctccctg agacggcttg agcagcccaa tgtggccatc 300

tccctgtcca ggacagaggc ccttaaccac cacaacctgc tggtctgctc agtgacagat 360

ttctacccag cccagatcaa agtgcgctgg ttccggaatg gccaggagga gacgacgggg 420

gtcgtgtcca cacagcttat taggaatggg gactggacct tccagatcct ggtcatgctg 480

gagatcacgc ctcagcgggg agatgtgtac acctgccatg ttgaccaccc cagccttcag 540

agccctgtca cagtggagtg gc 562

<210> 248

<211> 1957

<212> DNA

<213> Rattus norvegicus (Norway rat)

<400> 248
gggcatgagt ggctcggctc tgagcttcac catattccca ggaagtatcc ttgggttcct 60

ccaaatagcc actgtgctca ctgtgctcct gctgctcctc aagacagccc agttctacct 120

gcacaggcga tggttactca gagctaccca gcagttccca tccccacctt ctcactggtt 180

ctttgggcat aagatcccaa aggaccagga ttttcaagat attctaacac gggtaaagaa 240

tttcccaagt gcctgtccac agtggctctg ggggagcaat gtgcgaatcc aagtgtatga 300

ccctgaatac atgaagctga ttctggggag atcagaccca aaagctcatg gttcctacag 360

atttctagct ccctggattg ggtatggttt gcttctgctg aatggacaga catggttcca 420

gcaccgacga atgttgactc cagctttcca ctatgacact ctgaaacctt atgtgggcat 480

catggcagac tctgttcgca taatgctgga taaatgggaa cagattgttg gacaggattc 540

caccctggag atcttccaac acatcacctt gatgaccttg gacaccatca tgaagtgtgc 600

cttcagccaa gagggcagtg tccagttgga cagaaaatac aagtcctaca tcaaggcagt 660

tgaggacctg aacaatctct tttttttccg tgtacagaac atgtttcatc agaatgactt 720

catctatagt ctgtcctcta atggccgcaa ggcccacaat gcctggcaac ttgcccatga 780

ttacacagac caagtgatca aatcaaggaa ggctcaactt caggacgagg aagagttgca 840

aaaggttaag cagaaaagac gattggattt cctggacatc cttctgtttg ccagaataga 900

aaatggaagc agcttatctg ataaggacct gcgtgctgaa gtggatacct tcatgttcga 960

gggccatgac accacagcta gcggtatctc ttggatcttc tatgctttgg ccacaaatcc 1020

tgaacaccag cagggatgca ggaaggagat ccagagtctc ctaggagatg gagcttctat 1080

cacctgggac gacctggaca agatgcccta caccaccatg tgcatcaagg aagctctgag 1140

gatctaccca cctgtaacag ctgtgagcag aatgctcagc acacctgtca ccttcccgga 1200

tggacgctct ttacccaaag gtatcacagt catgctctcc ttctatggcc ttcatcacaa 1260

cccaactgtg tggccaaatc cagaggtgtt tgacccttat cgatttgcac cagagtcttc 1320

ccgacacagc cactcattcc tgcccttctc aggaggagca aggaactgca ttgggaaaca 1380

gtttgccatg aatgaactga aggtggccgt ggccctgacc ctgctccgct ttgagctgct 1440

gccagatccc accaggatcc caatccccat accaagactc gtgttgaagt ccaagaatgg 1500

gatctacctg cgtctcaaaa agctccaata atcttgacag cacaagacag ctccaacagc 1560

atgctgtctg ccatttgtct ttctgtcagt cactcttgtc cccactccat ctgctcacat 1620

ctcattcttt cttctcacct tgttcacctc cacccacctt ctgctgtgct tctggtctcc 1680

ttgtctgtca gtctttctat ctcgacttct ggaatttcta cctgcttttc tctctacctg 1740

gcccctacct gacttcatgt ttgaccttga ccttaatgat ctccctaaat tacaccctgc 1800

cttctcttct gcatatttcc ttctattcta ctgtttgtct gtgcttaaat tgagctttat 1860

accaatgtca taattgatta caatagaagt attgccctat gattcgatat accaggtgtt 1920

tttcatgatt tcattagaat aaagctcagg tcattgc 1957

<210> 249

<211> 3641

<212> DNA

<213> Rattus norvegicus (Norway rat)

<400> 249
ggatccaatt tgatatggtt atatccaatt ataatttgta taagcaccac atatgtatta 60

ggtgtttgtt gactccactg caacacaaat tgtactagct gtccctgctg ctgctgcact 120

caaacttgtt cctaagttag ggccttgtgt gcacctacca ttgagccaca ttcctcactc 180

cagttagcaa aatttccaaa ctattatatg ccaggcaatg gcttaggtac caagaataaa 240

caatccgtgt tctgtctggg taaggggcaa attaacaata aacgcccaag tactaggcaa 300

ctgataatac ctaacagtgt aacctagggt cacaagagta agtctcttag tggcacttta 360

caccagaggg ctgaggaaag cccaacagat tgaattgaca aagagaagtc atgtcatttc 420

acactcagag ctcatcttct ctcctcccga atgcctttag cttgctctgc ttggaaatat 480

ccaggtcttc ctcacaccat ccttcctatc cctctaccca agagtctccc ttggctccat 540

actatacagt tgaaggtgtg accggtctca ctttaaagac aatgccccca cacaacactg 600

gtccgcatca cctgccctga ctagagagca cttgtgtgta gctgtggctg ccctccacca 660

tgttgggaag gccttaggaa gtaagcatca cgcagtgact atttctactt atctccaaag 720

gcccgtgttc agatagaaaa cccggggtct caacacatcc cacttgagtt aaaatctccc 780

gaggatggca aaggaggggg caggggtatc tccgagttcg aaggcagcct ggtctagaat 840

gagtcccggg gcagccaggg ctacaaagag aaaacctatc tcaaaaactc caatcaaggg 900

gttggggatt tagctcagcg gtagagcgct tgcctagcaa gcgcaaggcc ctgggttcga 960

tccccagctc cgaaaaaaga accaaaaaac aaaaaactcc aatcaaatct tctagattgg 1020

gccgggcgtg acgacgcggt cgaggctagc ctggtctaca cagtgtgctc taccacggcc 1080

agagcaaata gtgaggccct atctcaaccc ccaccagcac ctccacaggt aatttttaat 1140

cgatattgtc agctgatgag ggcacccgtt tgctacgctt ggaaccagtt agtggcaatc 1200

cgtttaaagc aatccaagac aactctctcg tcctctgcca cggagggaac tcgtgagcgg 1260

tcccaggcag gccgagtact tctcccacca cacaccagga agtcacctct ctgaacctgg 1320

agttatacct accgcgtgag gtcagagatt aaatggactg tttgtgcact gctcacacac 1380

acacacacgc gcgcgcgcgc gcgaaattgc ttttattagg agctctcagg aaagtggtgg 1440

ctcgcatcag gctaccacag ccaagaggac cgccacctct accgagggca ggccaaggcc 1500

cactaaacgg cccgaatgaa ctctaggcct tccggcgctc tccagctctt cagcgcggcg 1560

aacgttctag gcccctgaga agtccaccgg gaggcgcagg tttccgctac gcgaggggga 1620

aatccccgag aaatgcccag accacctaag cacagacgtc catgcggcgt acggatacct 1680

gagggaatcc gggccatacc gccacctact cgcgcctcac tcaagccgcg ccaaactcgc 1740

gcgtttcact gcgcaggcgt gtacgcagcg ggcgctcagg aacactcaaa ggaccagcac 1800

actgaccaat caacgtccgc cttggccaaa actagtcccg ccctcaaaag cagacgtcag 1860

gccccgcccc taagagcccc gccctccccc acgctttagg cccgacccac gccgtcctgg 1920

gttaaggctc gcggttggtt cgggggacgg acgcgagctc tggttggtgg aggtgaggtc 1980

acgagaacac cttcgtcgcc tttccagagg cgattactgg gcaagctcag tcttttgcct 2040

cagacgctag ctgtagctgg caggcggttg tacgtgctcc agagtcttca ttacccgccg 2100

atccagtcgt cttcgcgtgg gttccgctaa gctcttctga gctgctcgct ctctacacac 2160

gccgtcgccg taatccgcca tcatggtgaa actcgcaaag gtaataggcc tgacgcgcga 2220

acacggacga ctaggcctgc tttctgagag acgcgcgcgc gcccgtcgcg gaggggagga 2280

gggcctgcgc acagtcccgg gcgcgttcga gggcgcatgc tgagggaagt ctcgcgcgac 2340

tagcggaagg gtctcgcggt gtttggcctc tgacttgagg gatgagagga gccgaggcag 2400

gttctgggag ggcgatgtag agtgcaccgg ggccgggggt cggtgtgagg ggaagtcgga 2460

ggagttgagc gcggagaggg ggttggaggc tcggcgagtg aagtgggcca accttaagcg 2520

gctaccgctc tcgggggccc ccgcctcccg gcgcacgtgc tccggtgcgc ggcccgccac 2580

gtggttacgg cgctccgcac gtgtcgcgcg tccgaggcag tgaggggagt gggagtacac 2640

gggaaagggg gacagtagag ttgggccatg ttacctagcg atcgcatgtc cctgaccgtg 2700

acggtagatg gacacgtggt gggctcctga ggtagcgcgt ggtgacccgg cctaggtgac 2760

tccacgtggt ttggctgtaa gcgtgagaaa gtaggaagta gctccgatac agagatgctt 2820

ttaagaggtg ccttcgagat aacccccttc cccggcaaat agttaaaatg gaacctttaa 2880

ctgggttttt ttcttttttg tggaaggtgc cagaaaataa agtgccacga ctaagtagac 2940

tttaaaaacc tgtggctgtt tcctggtgaa ggtttctgtc tgtaagacaa attatgcgca 3000

gttctcagaa aaatttgcca ggaagagtgt gctgcctggc cacgagaagt ctttatgctt 3060

gtgttcttta gtaagaaagg aggtggtggc cgaggacaaa gtggcttaca atgcgggtga 3120

tttttttgtg tgcatttgta ggccggcaaa acccacggag agtccaagaa aatggctcct 3180

cctccaaagg aggtggaaga agatagtgag gatgaagaaa tgtcagaaga tgaagatgac 3240

agcagtggag aagaggaggt aggatgctat ttgaaaaatt gtgtgatttg cagtggtaga 3300

ttgactggaa gtgtcagaaa taacagtcag ttcggggggt gtctgggcag agttttgtat 3360

gttgtattga gtagagttta ctgtagattt gtggggtggt tttccctccg tttttgggaa 3420

aattgcttat tctagcctaa tttttattca gtccatggtc acttggcttt gtgaattttg 3480

gcaggacatt atgacaggaa gctgtagacc agcctaacct cagataggtt ctcctgaatt 3540

aggtgttagc cacagtatcc acgacaaatg acaagtttag tgagaaaccc agactttgat 3600

ctaggtagag atggaatcag ggtctcagct taggtggtac c 3641

<210> 250

<211> 1712

<212> DNA

<213> Rattus norvegicus (Norway rat)

<400> 250
atggcgacgg cagtgtgggc ggctgcgcgg ctacttcggg gttctgcggc gctgtgcgcg 60

cggcccaagt tcggttctcc tgcacacaga cggttcagca gtggcgccac ctaccccaac 120

atccctctct cttctccctt gcccggagtg cccaagccta tttttgctac agttgatgga 180

caggaaaagt ttgaaaccaa agtcaccact ctggacaatg ggcttcgtgt ggcatctcaa 240

aataagtttg gacagttctg taccttagga attcttatta attcaggatc acgatatgaa 300

gcgaaatatc ttagtggaat tgctcatttt ctggaaaaat tggcattttc gtctaccgct 360

cgatttgaca gcaaagatga aattctgctt accctggaaa aacatggtgg tatctgtgac 420

tgccagacct cgagagacac caccatgtat gctgtgtctg ctgacagcaa aggcttggac 480

actgtggtgg gcctgctggc tgatgtggtt ctgcaccccc gcctgacaga tgaggaaatt 540

gagatgacga ggatggctgt tcagtttgaa cttgaggacc tgaacatgag acctgaccca 600

gagccactgc tcaccgagat gattcatgaa gctgctttta gggagaacac ggtgggcctg 660

caccggtttt gtcctgtgga gaatatcggg aagattgacc gagaagtgct gcattcctac 720

ctgaagaact actacacccc cgaccgcatg gtgcttgctg gagtgggcgt ggagcatgag 780

cacctggtcg aatgtgccag aaagtacctc ctgggagtgc agcctgcctg gggagctccg 840

ggagctgtgt ggatgttgac agctcagtgg cacagtacac gggggggatc atcaaggtgg 900

agagagacat gtcaaatgtc agccttgcgc ccaccccgat tccagagctc acacatatat 960

ggtggggcta gagagctgct ccttctggag gaggatttca tcccctttgc tgtgttgaat 1020

atgatgatgg gaggcggggg ctccttctcg gccggagggc ctggcaaagg catgttctcc 1080

aggctctacc tcaacgtgct caatcggcac cactggatgt ataatgcaac ctcctaccac 1140

cacagctatg aagacacagg cctcctgtgc atccatgcca gcgcagaccc tcgacaggtt 1200

cgagaaatgg tagaaatcat cacaaaggag tttattttga tgggccgaac ggtggatttg 1260

gtggagctgg agcgggccaa gacacagctg atgtccatgc tcatgatgaa cttggagtcc 1320

aggcctgtga tctttgagga tgtggggagg caggtgttgg ctacacattc aagaaagctg 1380

ccccatgagc tgtgcacact cattcgcaat gtgaagccgg aagatatcaa gagggttgct 1440

tccaagatgc tccgaggcaa gcctgctgtg gcggctctgg gtgatttgac tgacctgccc 1500

acgtatgaac acatccaggc agcgctgtcc agcagagatg ggcgcctgcc cagaacatac 1560

cggctgttcc ggtagagccc tccagcggag gctgcagagt gcttctgtgc atgttactgt 1620

tggtgtggac actgcataag aatagctagc tctgtgaaca gtgccaacag ggaagctctg 1680

gaaacttgaa tgaaagttac tgttgccact gc 1712

<210> 251

<211> 1715

<212> DNA

<213> Rattus norvegicus (Norway rat)

<400> 251
gaaaaagaaa aaccaacaag aaagccaatg gctctcttca tttttctggg gatttggctt 60

tcttgtttgg tttttctttt tctatggaat cagcaccatg tcagaaggaa gctcccacct 120

ggtcccactc ctctaccaat ttttggcaat attttgcaag tgggtgttaa aaatatgagc 180

aaatctatgt gcatgctagc aaaagagtac gggcctgtgt tcaccatgta tctgggcatg 240

aagcccactg tggtgctgta tggatatgaa gtattgaaag aagctctgat tgaccgggga 300

gaagagtttt ctgataaaat gcattcgtca atgctcagta aagtcagcca agggttaggc 360

attgtcttca gcaatggaga aatatggaaa caaacacggc gtttctccct catggtctta 420

aggtctatgg ggatgggaaa gagaactatt gaaaaccgaa ttcaggagga agttgtttat 480

ctgctggaag cactgagaaa aaccaatggg tccccgtgtg accccagctt ccttctggcc 540

tgtgttccct gcaatctgat ctccagtgtc attttccagc accgttttga ctacagtgat 600

gagaaattcc aaaaattcat agagaatttc catacaaaaa ttgaaatttt agcatctcct 660

tgggcccagt tgtgcagtgc ctatcctgtt ctatactatc tcccgggaat ccataacaaa 720

ttcttaaaag atgtgactga acagaaaaag ttcatcttga tggaaataaa caggcatcga 780

gcatctctga acctcagtaa ccctcaggac tttattgact atttcctgat taaaatggaa 840

aaggaaaaac acaatgaaaa gtctgaattt accatggaca acctgatagt caccataggt 900

gatctttttg gagcaggaac agagacaacg agttccacca taaaatatgg gcttttgctc 960

ttgctgaagt acccagaggt cacagctaaa attcaggaag aaattactcg cgtgattggt 1020

agacaccgga gaccctgcat gcaggacagg aaccacatgc cctatacgga tgctgtgctg 1080

catgagatcc agagatacat tgattttgtt cccattccct tgcctcgtaa gacaacgcag 1140

gatgtggagt tcagaggata ccacattccc aagggcacaa gcgtaatggc atgtctgact 1200

tctgccctgc acgatgacaa agaatttccc aacccagaga agttcgaccc tggccacttt 1260

ctcgatgaga aaggcaactt caagaagagt gactacttca tggctttctc agcaggaaga 1320

agagcctgta ttggagaagg tttagcccgc atggagatgt tcttaatcct gaccagcatt 1380

ttacagcatt ttattttaaa acctcttgtc aatccagagg acatcgacac aaccccagtt 1440

caacctggtt tattgtctct gcccccaccc tttcagctct gtttcattcc agtctgaagc 1500

ggcatagtct ttcacacaga tgactgagac cctgcttcta cttgtgagca aacaggtcct 1560

cctgtcctcg tgtacctgta actggcagtc cttcctgaat ggcagggatg ctacctctga 1620

actgtcctac atttttttca attctttgtg aaccagttcc aatttttcat atgtgaaaaa 1680

acttcactgt tttagcacta ataaagtttt ttttc 1715

<210> 252

<211> 735

<212> DNA

<213> Rattus norvegicus (Norway rat)

<400> 252
agatctccct atctaaggag tccagcaatg gggtcaacgc caatgggaag taaatgctaa 60

aatatattct tattcaataa cttcttactg gacttgagta ctcttagagc ttccctgagt 120

ctgttttgtt attgaatggt tagtaaatgt atttctgtat tagagctaaa aataaaatgt 180

caacttcgag ttgtcaatca gtagcctgct aggattcctt tccttaaaag taatcagacc 240

caccagagct cccagggact aaactgccaa ccaaagagta cacatggagg gacccacagc 300

tccagcgtag caaaggatgg ccttgtctgg catcagtggg aggggaggcc ctgggtcctg 360

tggagccttg atgccccagc ataaggggaa tgctagagca gtgaggtagg aatgggtggg 420

taggtcgggg agcaccctct tagaggcaaa ggggaaaggg atgggatggg aggcttgcag 480

aggggagacc aggaaggggg acaacatttg aaatgcaaat aaataaaata tccaataaaa 540

aaatgttaag gcatgaggag atggctcagt ggttaagagc actgactgtt cttacagaag 600

gtcctgagtt caatccccag caactacatg gtagcttaca gccatctgta atgggatctg 660

atgccctctt ctggtgtgtc tgaagacagc tgcagtgtac tcacagacaa ttaataaata 720

aaatctttaa aaaat 735

<210> 253

<211>  4190

<212>  DNA

<213>  Rattus norvegicus (Norway rat)

<400>  253
ttcaagatcc tgtacaatgg gcagagcgtg gaggtggatg ggcactccat gcggaagctg      60

attgcagacc tgcaacccaa cacggagtac tccttcgtcc tgatgaaccg cggcagcagc     120

gctgggggcc tacagcacct ggtgtccatc cgcactgccc cagacctcct gcctcagaag     180

ccactgcctg cctctgcctt tatagaggat ggccgcttct ccctctccat gcctcaagtg     240

caggacccct cgctagtcag gtggttctac attgtggtgg tgcccattga ccgtgtgggc     300

gggaacctgc tggcaccaag atggagcaca ccagaggagc tggagctgga cgagcttctg     360

gaggccatcg agcagggtga ggagaaacag cggaggcgtc ggcgccaagc agagcggctg     420

aagccttacg tggcagctca agtggacgag ctccctgaga ccttcaccct gggggacaag     480

aagaactacc gtggcttcta caaccggccc ctgtctccgg atctgagtta ccagtgcttt     540

gtgctcgcct ccctcaagga acccatggac cagaagcgct atgcctccag cccctactcg     600

gacgagatcg tagtccaggt gacaccagcc cagcagcagg aggagcccga gatgctgtgg     660

gtgacaggcc ctgtcctggc agtcattctc atcatactca ttgtcatcgc tatcctccta     720

ttcaagagga agagaacaca ttccccatcg tcaaaggatg agcagtcaat cgggctgaag     780

gactccctgt tggcccactc ttctgaccct gtggaaatgc gaaggcttaa ctaccagacc     840

ccaggtatgc gagaccaccc gcccatcccc atcactgacc tggcagacaa tattgaacgt     900

ctcaaagcca atgatgggct caagttctcc caggagtatg agtccattga ccctggacag     960

cagttcacgt gggagaattc caactcagag gtgaacaagc ccaagaaccg ctatgcaaac    1020

gtcattgcct atgaccactc tcgagtcctc ctcacctcca ttgatggtgt tcctgggagt    1080

gactacatca atgccaacta cattgatggc taccgaaagc agaatgccta catcgccaca    1140

cagggtccgc tgcctgagac catgggtgat ttctggagga tggtgtggga acagcgtaca    1200

gccacagtgg tcatgatgac caggctagag gagaaatccc gggtgaagtg tgatcagtat    1260

tggccagccc gtggcactga gacctatggc ctcattcagg tgaccctggt ggacactgtg    1320

gagttggcca cgtacaccat gcgcaccttc gcactccata agagcggctc cagtgagaag    1380

cgtgagctgc gtcagttcca gttcatggcc tggccagacc atggggttcc tgagtacccc    1440

actcccatct tggccttcct gagacgggtc aaggcctgta acccgctaga tgcaggaccc    1500

atggtggtgc attgcagtgc tggtgtgggc cgcacgggct gcttcattgt catcgacgca    1560

atgctggagc gtatgaagca cgagaagaca gttgacatct atggccacgt gacgtgcatg    1620

cgctcgcaaa ggaactacat ggtgcagaca gaggaccagt atgtgttcat ccacgaggca    1680

ctgttagagg ctgccatgtg cggacacact gaggtgcttg ctcgaaacct ctatgcccac    1740

atccagaagc taggccaagt gcctcccggg gagagtgtca ctgccatgga gcttgagttc    1800

aagttgctgg ccaactccaa ggcccacacg tctcgcttca ttagtgccaa cctgccctgc 1860
aacaagttca agaaccgcct ggtgaacatc atgccctatg agctgactcg agtgtgcctg 1920
caacccatcc gtggtgtgga gggctctgac tacatcaatg ccagctttct agatggctac 1980
agacagcaga aggcctacat agctacacag gggcctctgg ccgagagcac cgaggacttc 2040
tggcgcatgt tatgggagca caattccacc atcatcgtca tgctgacgaa gcttcgggag 2100
atgggcaggg agaaatgtca ccagtactgg ccagcagagc gctctgctcg ctatcaatac 2160
tttgtcgttg acccgatggc tgagtacaac atgccccagt atatcctgcg tgaattcaaa 2220
gtcacagatg cccgggatgg gcagtcaagg acaatccgac agttccagtt tacagactgg 2280
ccagagcaag gagtacccaa gacgggggaa ggcttcatcg acttcatcgg gcaggtgcac 2340
aagacaaagg agcagtttgg acaggatggg cccatcacag tacactgcag tgctggtgtg 2400
ggccgcacag gggtgtttat caccctgagc attgtcctgg agcgcatgcg gtacgagggc 2460
gtggttgaca tgttccagac cgtgaagacc ctccgcacac agcgccctgc aatggtgcag 2520
acagaggacc aataccagct gtgctaccgt gcggccctgg aatacctcgg cagctttgat 2580
cactatgcaa cgtaactact gctcccctct cctccgccgc tcccctgagg ggctccggag 2640
gggacccagc tcctctgagc cataccgacc atcgtccagc cctcctgcac ggatgctgtt 2700
gccggcagag cacagcccac tgggatcaca gcatttcggg aacattgcca caccagtcag 2760
agagcccaga acacctgggc aagtaggcgg actggcagcc tggctctggg ccctcgtcca 2820
ccgggcccaa gtggagccct gcttcaagct ctctgttcag ctccgcgatt ctcatgcttc 2880
tcatggggtg ggaaaggggg cagagccccc cacgttttat acatttaggc ggggtagact 2940
gcggggtcct agcctcttcc tccgactttc cttttgcagg tctttcacta cagattgggc 3000
tgctgtggga gttgggactt acttgttttc ctttttgagt tcacgttgga tccttgtaca 3060
acttctgcgg aaggacacag tacacgcttt ccttcctcga gcagagctag ggccctacct 3120
gagcaagtcg gctgtggccc gggaggcagc tgagactcct gctgtcctcc agcctttcag 3180
aggagatcct attccagcca aatgcaggga aacactttat tttgttttag gttttgtttt 3240
tccttgagag ccttttttta ggccccacag acagtggtgg gcggggaggc gataggaaac 3300
acatccccag tgtgcattta acattcatag cctacaacca cagacgtgtc tggggggagc 3360
ctggcaaggc gttcctcgtc accatcgtgt ttgcaaaggt tcaaaaaagc aaaaattaaa 3420
aaaaaaaaaa cataaaaata ttttttttta aaaaaaagaa ataaagattc atcccctggc 3480
ctctccttct gctccgaagt gggaggcaac tcaatgtgcc tggggctcgt tgtgggccca 3540
cagagtcttg ctgtcatccc cagcggtgca gtgtggcagg tgtttgtaag ctgtgggttc 3600
tggccacctc ggtaactgaa tggcacaggt gagggcctgt gccacgcccc acacacctca 3660
gggccaagcg ggggcgtggc cggcccttca ggtctagcca gtgggcctgg tagcacatgt 3720
ctgtcctcag cgggacagat gctttctctc ctggtttgca gctgtcttca aatcccccat 3780
gactcgccct tcccactcct tcaagtcgcc ctcacaaacc aatgtggcaa gactactgga 3840

cttgtatcca tggtactcta ctcagtcctc ttatctcagc ttgctgaggg gcagggagag 3900

ggtctcttcc tctgggcagc actatctagg tagtaagtgg gggcgggaaa gggtgcatag 3960

ctgttttagc cgaaggactc gatgtggacg tcctcagata tagctaggct aagcggagat 4020

cgacggtccg gggttggtgg tgttggatga gacattcatt tttaccttgt ggacgctagt 4080

gctgtagagt tcactgttgt acacagtctg ttttctattt gttaaggaaa actacagcat 4140

cattgcatac ttcttgatgg taataaattt gaataatcag atttcttaca 4190

<210> 254

<211> 1688

<212> DNA

<213> Rattus norvegicus (Norway rat)

<400> 254
cagaaggagt catggctgcc aaagtgtttg agtccatcgg aaagttcggc ctggccttag 60

cagttgcagg aggcgtggtg aactctgctc tatataacgt ggatgccgga cacagagctg 120

tcatcttcga ccgattccgt ggcgtgcagg acatcgtggt aggggaaggg actcacttcc 180

tcatcccctg ggtacagaag ccaatcatct ttgactgccg ctctcgacca cgtaatgtgc 240

cggtcatcac cggcagcaaa gacttgcaga atgtcaacat cacactacgt atcctcttcc 300

ggccggtggc cagccagctt cctcgtatct acaccagcat tggcgaggac tatgatgagc 360

gggtgctgcc atctatcacc acagagatcc tcaagtcggt ggtggctcga ttcgatgctg 420

gagaattgat tacccagcga gagctggtct ccaggcaggt gagtgatgac ctcacagagc 480

gagcagcaac attcgggctc atcctggatg acgtgtccct gacacatctg accttcggga 540

aggagttcac agaggcggtg gaagccaaac aggtggctca gcaggaagca gagagagcca 600

gatttgtggt ggaaaaggct gagcagcaga agaaggcggc catcatctct gctgagggtg 660

actccaaagc ggctgagctg atcgccaact cactggccac cgccggggat ggcctgatcg 720

agctgcgaaa gctggaagct gctgaggaca ttgcttatca gctctcccgc tctcggaaca 780

tcacctacct gccagcaggg cagtccgtgc tcctccagct cccccagtaa ggccagccag 840

ccagggcctc catcgctctg aatgacgcct tccttctgcc ccaccccaga aatcactgtg 900

aaatttaatg attggcttaa catgaaggaa ataaaggtaa aatcacttca tatctctaat 960

tatcaaatga agcttttatt gttacacttt ttgcccactt tcataacaaa attgccaagt 1020

gcctatgcag actggccttc caccctgggt gctggcagtc ggcggaagaa aggcagggca 1080

gtgtgtgtgg tggacgggga gccagctggc agcctgagta gaccttgagc ctccattctg 1140

ccatatattg aagatttaca gacagtggtg cacacacgtg aaccaaaagc aagccctcaa 1200

tttttccagc catacgaacc cggacagatg cagctgagga gggcctgagg aagtggtctg 1260

tcttaactgt aaggccattc cctcttaacc gtgaccagcg gaagcaggtg tgtgcgtgcg 1320

actagggcat ggagtgaaga atctgcccat cacggtgggt gggcctaatt ttgctgcccc 1380

caccagagac ctaaactttg gatagacttg gatagaataa gaggcctgga ctgagatgtg 1440

agtcctgtgg aagacttcct gtccaccccc cacattggtc ctctcaaata ccaatgggat 1500

tccagcttga aggattgcat ctgccggggc tgagcacacc tgccaaggac acgtgcgcct 1560

gccttcccgc tccctctctt cgagattgcc cttccttccc aagggctgtg ggccagagct 1620

ccgaaggaag caatcaagga aagaaaacac aatgtaagct gctgtcaata aatgacaccc 1680

agaccctc 1688

<210> 255

<211> 1893

<212> DNA

<213> Rattus norvegicus (Norway rat)

<400> 255
tgtctcactc agcgagacgc gcggtgcacg gtgcttcccc ggcggacgac cgaccaaccc 60

gcgctccggc agagtccttg gcgctcgcct gccggcggga cagacagccc gcctctagcc 120

gctctctgga ccctggccgc cccgagcgaa gactggagca aaatgatgct tcaacatcca 180

ggccaggtct ctgcctcaga agtcagcgcg accgccatcg tcccctgcct ctcacctcct 240

gggtcactgg tgtttgagga ttttgctaac ctgacacctt ttgtcaagga agagctgaga 300

ttcgccatcc agaacaagca cctttgccat cggatgtcct ctgcgctgga gtcagtcacc 360

atcaacaaca gacctctgga gatgtcagtc accaagtctg aggtggcccc tgaagaagat 420

gagagaaaaa ggaggcggcg ggaaagaaac aaaattgctg ctgccaagtg tcgaaacaag 480

aaaaaagaga agacagagtg cctgcagaag gagtcagaga aactggagag tgtgaatgcc 540

gaactgaagg cccagatcga ggagctgaag aatgagaagc agcatctgat ttacatgctc 600

aacctgcacc ggcccacgtg tatcgtccgg gctcagaacg ggcggacgcc ggaagacgag 660

aggaaccttt ttatccaaca gataaaagaa ggaacattgc agagctaagc agaggtggca 720

tgggggcaat tggggagtcc ttactgaatc ctccttttcc accccaaacc ctgaagccat 780

tggaaaactg gcttcctgtg cacttctaga atctcagcag ccacgagctg ttgggtcagg 840

agggcctgcg gtgactactg cgttgtccca ctctgtcccc gagtgaaccg tggagcaggc 900

aggagcatcc tttgtctcac cggctccagg atttaggcct taccatcccg gccattctca 960

gatgacctag ctggccccag gctggggtcc catgcaaagc aggatcgcac taatgggatg 1020

caggcagaag tgtctacctt gacaggtggg gtggaccacg tcctccactg cggctgacaa 1080

catccctcct agggaagatg gagtgagaac attcatcatt gaagttgtcc aatggccagg 1140

gtatgctttc tagaaactat gctgttctgt cctagactga ctgtgcatag ggcattcatt 1200

tctgagcctg gtgttgtgct atttagatgt ttgtcttgca caacattggc gtgatttttt 1260

tccgggagtt tcatcagacc tgatttccga gagtttgggg gtctgccact gtggacaata 1320

tcccccaaaa gtgtttgggt ggccatgtaa actggctgat gaccagctgt gctactctgt 1380

gctgaccgag gactgatgcc tccttcccct gtacccactg ctgaggaaga acccgggcac 1440

agcagctgtc cttggctaca aactgttaca atgtcacaga acgaaggcac aaagtcccgc 1500

tttcaaaggg cgtaggactc cacactcagt gacagggcag gaagagccaa ggattctccg 1560

ttttcccttc cttcccacca aaaaccacag cccgtggaga ctggtatttg aagccaggag 1620

tggggcaagg aaggtgtctg cactgtggga tgttaactgc gcttttgtct tgaagctatt 1680

ttgagatgcg gtccagagta tttcagctgg gaggtccctc ccactggcca ccagggctct 1740

ggctactgtt aaaattctga tgtttctgtg aaatcctcag tgttcaatcc agactcagta 1800

gtatattaca gttttctgta agagagaacg ttacttattt atcccagtat tcctagcctg 1860

tcaacgtaat aaaatatcag aatgagacct ggt 1893

<210> 256

<211> 4795

<212> DNA

<213> Rattus norvegicus (Norway rat)

<400> 256
   ctgcagcaca gccctgccct tctcttgccc tggagccaca accctcttta cagctcctag 60
   ggatctgatt cgctttgacc tcccactggg tagcccgatc ttgggctggt tataaagtcc 120
   atgagcctga ctcagacctg tccggaatct ccaatggggg ccatggcgcc gcccttgctg 180
   ctcccgctgc tggtggctgc cctggccccg actcagaccc aagccggtga gtgcggggtc 240
   ctgagggaaa tggcctctgg ggtaggggcg ggggcggcac cggggaagac cggtccccgc 300
   gtcgaacacc agaccctccg ccccttctcc acccgcgacc cgagccccgc gccctgctcc 360
   cggcccggcc cgcgcacccg cccggggtcc cggtaggagg tccgggtctc accgcgagcc 420
   gcccccaggc tcacactcgc tgcggtattt caccaccgcg gtgtcccggc ccggtctggg 480
   ggagccccgg taacggaagt gggctatgtg gacgacacgg agttcgtgcg cttcgacagc 540
   gacgcggagt atccgaggat ggagccgcgg gtccggtgga tggagcggga gggcccggag 600
   tattgggagc gggagatgca gagagccaag ggcaaccagc agtggttccg agtggacctg 660
   aggaccctgc tccgctacta caaccagagc gagggcggtg agtgaccctg ggacggaggt 720
   caggatccct ccgcgtcccc acacagcagg aaacgtcccg ggtcccaagt ccgagtttca 780
   ggagctgaac ccacccgagc ccgccctttc agtttggagg agtccccggg tgggcggggc 840
   gggggcgggg cgggggcggg ggcggggcgg gtgggcgggg actgaccgct ggtcccgcag 900
   gctcgcacac cttccaggtg atgtctggct gtgacgtggg gtcggaaggg agcctcctcc 960
   gcaggtatga gcagttcgcc tatgatggcc aagattacat cgccctgaac gaagacctga 1020
   aaacctggac agcggcggac acagcggcac ggatcacccg gagcaagtgg gatcaggctg 1080
   gtgatgcaga ttattacaag gcctacctgg agggcacgtg cgtggagtgg ctccgcagat 1140
   acctggagca cgggaaggag gcgctgctgc gtctaggtgc ggagatgcgg gcttctcctc 1200
   cctctgccct cgggctgggt ctcactcctg ggaaagaaga aaccctcagc tgggtgatgc 1260
   ccctgtctca gaggggagag aatgtccctg ggtctcctga tccctcacga cagtgactgc 1320
   actgcctctc ccagggccca gccttctccc tggagagttc tgagtccctc tcaggtggga 1380
   aggagagaac ttccccactg aacagcaagg gctccctgct gtcagccatg gcctctccca 1440
   ggccaggttc tctgcccaca cccactcact gaggacactg actcctgtcc tgctgagtgt 1500
   gtcagccctt cacacctcag gaccagaagt ctcctttatc tcagaggaga catggactcc 1560
   actacactag gctggttctc ccagtctata gaactttcca aagaatacat tctcacagat 1620
   ccctccctga tgtggggttt gcctctcttc cacaccccat ttctctctat tcctacagtg 1680
   gagaatggtc ccatgagccc ttatggggta ccctggagga tataaactgt gggatttctt 1740
   tttctttttt tctctctttt ttcttatttt ctttttcttt ttattttttt cctttttttt 1800
   tttttttgga aggcattatt tttcttctag tcaggttttc tctgcaaaca catatttaat 1860
   aaattatttt ccattccctc ttctgttcta tgaccaccca tctcatgcta tagaacatca 1920
   cataaggatg gccatgttga cccactggct catgtggatt ccctcttaga ttctgagtcc 1980
   ctcaggaaag tgtgcagttc tgtgctgagg gcaccagctc tctagtcgat gacagttgaa 2040
   gtgtcagaca cacacagttc agtgtcatca ttgatttaac tgtgtcttgt gtagatttca 2100
   gtttgtcttg ttaactgtgt gatttcttaa atcttccaca cagatccccc aaaggcacat 2160
   gtgacccttc accccagacc tgaaggtgat gtcaccctga ggtgctgggc cctgggcttc 2220
   taccctgctg acatcaccct gacctggcag ttgaatgggg aggacctgac ccaggacatg 2280
   gaacttgtgg agaccaggcc tgcaggggat ggaaccttcc agaagtgggc atctgtggtg 2340
   gtgcctcttg cgaaggagca gaattacaca tgccgtgtgg agcatgaggg gctgcctaag 2400
   ccgctttccc agagatgggg taaggaggga gccttctata gaccctgagc tggtcagggc 2460
   tgagagctgg ggtcatgacc cccaccttca tttcctgttc ctgtccttcc cagagtctcc 2520
   tccatccact gactccaacg tggaaaccat tgttgtttct gttgttcttg gagctggggc 2580
   catcattgga gctctggtgg ctgttgtgag gaagagatgg ataaaaacag gtaggaaagg 2640
   gcagggtctg tttcccctca gcttccttta gaagagcgct ctgctcatta ataggaaaca 2700
   tggccacacc ctccatgcta ctgtctccaa ctgggtcctc tgtcagttct ggaaacttcc 2760
   tcctgtcaaa acattcagtg aaatcttaca tatttccttc tcacaggtgg aaaaggagga 2820
   gtctacaccc ctgctctagg ttagtgtggg ggcaggagtg tccctgaggg tattggagtg 2880
   aagctggaga tgttgggagc tctgggaatc catagtagct cctccagaga aatcttctgg 2940
   gggtgtcagt tgtaccgtga tatgaataca tacatacaca tatacacata catatctttt 3000
   gttttaccct agtataccct gtgcagtgac agctcccatc atctcttatg tgtctttctc 3060
   agattgtaaa ggtaactctg ggtctgactg gggaggggca atgtggacat gattgggttt 3120
   cagggactcc cagaatcccc tgtgagtgga gggttgttgg gctgttgtct tctcagtgat 3180
   ggtttatgac tctcgttctc tagcatgaag acagctgcct ggagtggact gagtgacaga 3240
   cgatgtgttc aggtctctcc tgtgacatcc agagtcctca gttctcttta gacaccagtg 3300
   tctgatgttc cctgtgatct atggcttctg tgtgaagaac tgtggagccc agcctgccct 3360
   gcacacctgg acactgtccc tgcactgcct gtgttccctt ccacagccaa acttcctggt 3420
   ccagcctggg ggagagagag agagagagag agagagagag agagagagag agagagagag 3480
   agagagagag agagagagag agagagagag agagagagag agagcagcag agagagagag 3540
   agagagagtg tgtgtgtgtg tgtgtgtgtg tgtgtgtgtg tgtgtgtgtg tgtgatatct 3600
   gtatcttggg agctccatgg agccctgagc tgcagctcct cacttccaca ctgagaataa 3660
   tgaatctgag tgtgaacttg attgttcaca tacttgaccc aatgtagata ttttttttaa 3720
   ttattggatt gaatacttag aggttttttg ttctggggtc ttgttttgtt ttgttgataa 3780
   aaataattgg cagatggaga agtttccaga atctgtgtca caagctgtgt gtatctggtg 3840
   ggacagtgtg aggctgtggg agctgagtgt ggacaggctg tgcccaggtg tgctcagtca 3900
   gttcttgtca ttgatggact caggtctcag ctctgtctcc tccaggctgt gccctctcca 3960
   tcactgtgaa gacatgggga gagtctgtga tcacaaggac acagggaagg ccagagcctt 4020
   gtccttgtcc tgtccccagg attatgagcc cccagggctg aagatcagag actcaagtgg 4080
   ggtcctctaa agtgcagggt gcacaagatg agtcactaca tctcctccca ctcacttgat 4140
   ttagctgcta atgatggggt cgggaatctg ccctgggctc tccctgtggc catgctgctg 4200
   tggtgatttc tgcttctcct ttctggtgtc tttcccaggg ttctcagggt ctggtgtgat 4260
   ctgtctgctc atctctcagg accacctcca cagctgagat aagacctaac agagaggctt 4320
   ctcccctgta acttgggtac cgattcctga accaggactg tcttaccttt tccgtcttgt 4380
   gttctggagt taactccctg aagtctctta atctccaccc ccagctctgt gcacctgggt 4440
   cttgcagctg atgcacctgt ggttgctctg cctttggatg gctctgtatg gcaggagagc 4500
   aaagtccccc tcagaggcct gggaccattt aaatcatctc atattcatag cactaaagac 4560
   acggaggcag aagctgaggg aaaaacctga cagctcagca aggaaacttt attttcttaa 4620
   ctacatacgg gatatctatt taccagcctg atgctacaac taaactccag cccttcagat 4680
   atctgtctgt gtcactctat cagtccagtc tgcctttctc ttgagctcct gcaaacccaa 4740
   gagggctcct gagctctctt tcccggattt atattttatc tgtgtcctgc cagct 4795

<210> 257

<211> 2748

<212> DNA

<213> Rattus norvegicus (Norway rat)

<400> 257
ccggaagccc gaagcaccgg agtcccgcag aacctgactc cggcctgtca ccaccaccaa 60
aggctagggg acgtcgcctc ggtcactatg gggctcctgc tcaagcctgg agcgcgccag 120
ggcagcggca cctcctcggt cccagacaga cgttgtcccc gctccgtctt cagcaacatt 180
aaggtatttg ttctttgcca tggcctgcta cagctctgcc agctgctcta cagcgcctac 240
ttcaagagca gtctcaccac aatcgagaag cgctttgggc tctccagctc ttcctctggt 300
ctcatctcca gtttgaatga gatcagcaac gctaccctca tcatcttcat tagctacttc 360
ggcagccggg tcaaccgccc acggatgatt ggcatagggg gtctcctcct ggctgcaggg 420
gcctttgtcc tcaccctccc acacttcctg tcagagccct atcaatacac ctcgaccacg 480
gatggaaaca ggagcagctt tcagactgac ctctgtcaga agcatttcgg agccctgccc 540
cccagtaagt gccatagcac cgtgccagat acccacaagg agaccagcag cctgtggggc 600
ctgatggtgg ttgctcaact actggccggc attgggacag tgcccatcca gccctttggg 660
atctcctacg tggatgactt tgccgagcct accaactcac ctctgtatat ctccatccta 720
ttcgccatcg ctgtgttcgg accggctttc gggtacctgc tgggctcagt catgctgaga 780
atcttcgtgg actacggcag agtggacact gctaccgtaa acctgagccc aggtgaccct 840
cggtggattg gagcctggtg gctgggcctg ctcatctcct caggcttctt gattgtcacc 900
tctttgccct tctttttctt tccccgagca atgtccagag gagcagagag gtctgttacc 960
gcagaggaaa caatgcagac ggaggaggac aagtcaagag gctccctgat ggatttcatt 1020
aaacggttcc cccgcatctt cctgaggctg ctgatgaacc cgctcttcat gctggtggtc 1080
ctgagccagt gtaccttctc ctcagtcatc gctggcctct ccacgttcct caacaagttc 1140
ctggagaagc agtatggagc cacggcagcc tatgccaact tcctcatcgg tgctgtaaat 1200
cttccggctg cagccttggg gatgctgttt ggaggaatcc tcatgaagcg ttttgttttc 1260
cctctgcaaa ctatcccccg agtggctgcc accatcatca ccatctccat gatcctctgt 1320
gtacctctct tctttatggg atgctccaca tcagccgtgg ctgaggtcta ccctcccagc 1380
acatcaagtt ctatacatcc gcagcagcct cctgcctgcc gcagggactg ctcgtgccca 1440
gattccttct tccacccagt ctgtggagac aatggagtcg agtacgtttc cccttgccac 1500
gccggctgca gcagcaccaa cacaagctca gaagcttcta aggaaccgat ctacttgaac 1560
tgcagctgtg tgagtggagg atcggcgtca caagacaggc tcatgcccca cgtcctgcgc 1620
gcactactgc tcccgtccat cttcctcatt tcctttgcgg cgctcattgc ctgcatctcc 1680
cacaacccgc tctacatgat ggtccttcgc gtggtgaacc aggatgaaaa gtcgtttgcc 1740
attggggtac agttcttgtt gatgcgcttg ctggcctggc tgccggctcc atccctttat 1800
ggcctcctca tcgactcctc ctgtgtccgg tggaactacc tatgctcagg gagacgaggg 1860
gcctgtgcgt attatgacaa cgatgctctc cgaaacaggt acctgggcct acagatggtc 1920
tacaaggcct tgggcacact gctgctcttc ttcatcagct ggaggatgaa gaagaacagg 1980

gaatacagcc tgcaggagaa cacctcaggc ctcatctgac cctcagctgg gactactgcc 2040

ccaccccaga gactggatcc tatcccttcc acacctacct gtattaacta atgtcaacat 2100

gccttcctcc tcctcttcct cctcctcctc ctccttcttc ttcctctctc tctctctctc 2160

tctctctctc tctctctctc acacacacac acacacacac acatgagaga gagttcactc 2220

accctttgag atcacctgcc ttttctcttc tgcctaaagt cttaaggcct gaagtacact 2280

gagctgaatg agcaccgggc ctgagagttt agtttctcca agtccttgga aggtatcccc 2340

agcgtaggcc ctacgtcctc cagacaagat gcccataatg aggcggcctc tgttttcacc 2400

agtgtctcag gaatacttaa tggagtgaaa agagggagtc ttgccttctt gggccaggca 2460

gcccggatct cctctgcctc tgcccacacc caggagagcc agaggagaag caggtagttg 2520

gtttcttatc tgctccagcg gggctaaggg agctgggtgt gtccactttt catctggatt 2580

ccgtctagca tgaaagccgt gccctcgagg ctgttttgga aaccaccatt ttgggaagta 2640

tccctctcta taaactatgc cccggtatct gaggaggaat gaagggagga acaaggctgg 2700

atcatggaaa actgttcaca ggaaccagag gcctatcctc ccgtcggg 2748

<210> 258

<211> 2063

<212> DNA

<213> Rattus norvegicus (Norway rat)

<400> 258
agcggacgct gtctgaggag tagagcctgc ctgagagcga gtgtcccgtc ggtcccggag 60

cagcccagcc cggcccagcc ctcacaggtc ctctgtggtg cacacagccc acctcccagc 120

catgcgctct ctccttctgg gcaccttatg cctcctggct gtggccctgg cagccgaggt 180

gaagaaacct gtagaggccg cagcccctgg tactgcggag aagctgagtt ccaaggcgac 240

cacactggca gagcgcagca caggcctggc cttcagcctg tatcaggcaa tggccaaaga 300

tcaggcggtg gagaatatcc tcctgtcacc cttggtggtg gcctcatccc tgggccttgt 360

gtcactgggt ggtaaagcca ccacagcttc gcaggcgaag gcagtgttga gcgctgagaa 420

gctgcgggat gaggaggtgc acacggggct gggcgagctc gtgcgctccc tcagtaactc 480

cactgcgcgc aatgtgacct ggaaactggg cagccgcctg tacgggccca gctccgtgag 540

cttcgccgat gacttcgtgc gcagcagtaa gcaacactac aactgtgaac actccaagat 600

caacttccga gacaagcgca gcgcgctgca gtccatcaac gagtgggcct cgcagaccac 660

cgacggcaag ctgccagagg tcaccaagga tgtggagcgc acggatgggg cactgctcgt 720

taatgccatg ttctttaagc cgcactggga tgagaagttc caccataaga tggtagacaa 780

ccgtggcttc atggtgaccc gctcctacac tgtgggtgtt acgatgatgc accggacagg 840

cctctacaac tactatgacg acgagaagga gaagctgcag ctggtggaga tgcccttggc 900

ccataagctc tccagcctca tcatcctcat gccccaccac gtagaaccgc tcgagcgcct 960

ggagaagttg ctgaccaagg agcaactaaa gacctggatg gggaagatgc agaagaaggc 1020

tgttgccatc tccctgccca agggagtggt ggaggtgacc catgacctgc agaaacatct 1080

ggccggactg ggcctgactg aggccatcga caagaacaag gcagacttat cgcgcatgtc 1140

tggcaagaag gatctgtacc tggccagtgt gttccacgcc actgccttcg agtgggacac 1200

agagggcaac ccctttgacc aggacatcta cgggcgtgag gagctgcgca gccccaagct 1260

gttctatgcc gaccacccct tcatcttcct ggtgcgagat aatcagagcg gctccttgct 1320

gttcattggc cgcctggtcc ggcccaaggg agacaagatg cgagacgagt tgtagagtcc 1380

aagagtggtc gtggcgtggc atggcgggaa gtagccagag gttcctgaga cacatgggtg 1440

ctattgtggg gggaggggga ggcaccaacc ctggatagtc cacgggtggg ggggggttgg 1500

cagtgcagat cggaattccc ataggtctga gtaggtattt ttagacaaga gtccactccg 1560

ttagacatgg agcccagaca ctatgagacc aaattcaggg gtgtcacagc cattctagcc 1620

ctgccccaca agttttatat ccaatctagc ctcaacagtc aatcagtgtt catattcgtg 1680

gccaagcatt ctgtctgtga gacaatcgag ttgggtgggg gtagggcggc ctagcctctc 1740

tgtcaccatg ccctgagccc tcggttttcc tgttcacctc tgattccctt taaccacaaa 1800

actaggagcc tgtggtccca gaactgccca cccttaaaat cgattctttg ccgcagcagt 1860

gggagttggg gagaccatgc ataggagggc tccttggcat atccggccct agagccatta 1920

cattacattg acaactcaac tttgcttctg ttttctccct tttgagtttt ttcaaggaat 1980

gggggtttgg ggaaggggaa gcctttattg ctttcaaaac aagaacagtt tgtcaatttt 2040

ttttgaataa accttttcca atg 2063

<210> 259

<211> 1836

<212> DNA

<213> Rattus norvegicus (Norway rat)

<400> 259
ctcgagaccc cgtccccctc ccagtggacg ccaagtgcgc ttgcgcggta acagccgccc 60

gcacttgtgc catttccccc ttcttccgag ccacctttcc cccctccctc ccttccctat 120

ccccttccct gccctgcttc cctcccttcc ccctagactc ttcgttgtgg tcttcccgtt 180

cctgatcctt cccctacccc acccccaatc cccaggaccc tggcgcccca gccgcaggtg 240

ctgcgcccac gggagccacg atgtgcgtcc tctcagaacc tgtgggcctc cttgccctca 300

ctgccctccg tttcctcctc gcaggccccc acatccttca gaatgacgtt ggacgtgggg 360

ccagaggatg aactcccaga ctgggctgcg gccaaagagt tttaccagaa gtatgacccc 420

aaggacatca ttggcagagg agtgagctct gtggtccgcc gctgtgtcca tcgagctact 480

ggtgatgagt ttgcagtgaa gatcatggag gtgtcagctg aacggctgag tcttgagcag 540

ctggaggagg tgcgggatgc cacacggcga gagatgcaca tccttcgcca ggtcgctggc 600

cacccccaca tcatcactct catcgattcc tacgagtctt ctagcttcat gttcctggtg 660

tttgacctga tgcggaaagg agagctgttt gactatctca cagagaaagt ggccctctca 720

gaaaaggaaa ccaggtccat tatgaggtct ctgctggagg cggtgaactt tcttcatgtc 780

aacaacattg tacatcgaga cctgaagcct gagaatatcc tcctagatga caatatgcag 840

atccgccttt cagattttgg gttctcctgc catttggaac ctggcgagaa gcttcgagag 900

ctgtgtggga ctccgggata tctagcacca gagatcctta aatgctccat ggatgaaacc 960

cacccaggct acggcaagga agtcgacctc tgggcttgtg gggtaatcct gttcacactt 1020

ctggctggct cgccaccatt ctggcatcga cgccaaatcc tgatgctacg catgatcatg 1080

gaaggccaat atcagtttag ttcacccgag tgggatgatc gttcaaacac cgtcaaagac 1140

ctgatcgcaa agctgctgca ggtggatcct aacgcacgct tgaccgctga gcaagcccta 1200

cagcacccct tctttgagcg ctgtaaaggc agccagccct ggaacctcac acctcgccag 1260

cggttccggg tggcagtgtg gacaatactg gctgctggac gagtagcctt aagcagtcac 1320

cgtttacggc cactaactaa gaatgcactc ttgagagacc cctatgcact gcggccagta 1380

cggcgcctca ttgacaactg tgccttccgg ctctatggac actgggtgaa gaagggggag 1440

cagcagaacc gggcggccct cttccagcac cagcctccta ggcctttccc catcattgcc 1500

actgacctgg aaggagactc cagtgctatc acagaggatg aagttacctt ggtacggagc 1560

taggaccttc tccctggcca agccacgaag taggactccc caggaggaga ttttcttttt 1620

ctatcattcc aaccacttgg gactcagatc ttagccctgc cttgccaagt ctacaactga 1680

gaatgctgct tgtcacagaa accagccctt tacccctacc tacctactct ggacttggag 1740

atcggggcgg agatggaagg gaacctcttc ccagcattgt gcctggcctt gccatcaccg 1800

cctgtgctgc atctgaaata aaacctgcta caagcc 1836

<210> 260

<211> 1643

<212> DNA

<213> Rattus norvegicus (Norway rat)

<400> 260
ccttgctgtt ctccaacccc tctctttccc cttgcttcct ctcacctgct ctgggactcc 60

taactcagag acctcccttc tccccccgcc ggctggatta tggcagagaa cgatgtggac 120

aatgagctct tggactatga agacgatgag gtggagacgg ccgctggggc agatggaacc 180

gaagctcccg ccaagaaaga cgtcaagggc tcctatgtct ccatccacag ctccggcttt 240

cgagatttcc tacttaagcc agagttgctc cgggccattg ttgactgtgg ctttgagcat 300

ccatcagagg tccagcatga atgcatcccc caggccattc tggggatgga tgtcctgtgc 360

caggccaagt caggcatggg aaaaacagca gtgtttgtcc tggccacact gcagcagctg 420

gagccagtta ctgggcaggt gtcagtgctg gtgatgtgtc acaccaggga gctggctttc 480

cagatcagca aggaatatga gcgcttctca aagtacatgc cgaatgtcaa ggtggcagtg 540

ttttttggcg gactgtctat caagaaggat gaagaggtgc tgaagaagaa ctgcccacac 600

attgttgtgg ggactcctgg ccgaattcta gccctggccc gaaataagag cctgaacctc 660

aaacacatta aacactttat cttggacgaa tgtgacaaga tgcttgaaca gctcgacatg 720

cgtcgggatg tccaggaaat ttttcgcatg accccccatg agaagcaggt catgatgttc 780

agtgctacct tgagcaaaga gatccgccca gtgtgccgca agttcatgca agatcctatg 840

gagatcttcg tggatgacga gaccaagttg acactgcacg ggttgcagca atactacgtg 900

aaactgaagg acaacgagaa gaaccggaag ctctttgacc ttctcgatgt cctcgagttc 960

aaccaggttg tgatctttgt gaagtccgtg cagcgctgca tcgccttggc ccagcttcta 1020

gtggagcaga acttgccagc cattgctatc caccgcggga tgccccagga ggagaggctc 1080

tctcggtatc agcagttcaa ggattttcag cggaggatac ttgtggctac caacctgttt 1140

ggccgtggca tggacattgt gcgcgtgaac atcgctttta actatgacat gccagaggac 1200

tccgacacct acctgcacag ggtggccaga gcgggccggt ttggcaccaa gggcttggca 1260

tcacatttgt gtcagacgag aacgatgcca agatcctgaa tgacgtgcag gaccgtttcg 1320

aggtcaacat cagtgagctg cctgatgaga tcgacatttc ctcctacatt gaacagacac 1380

ggtagaggaa tcacccattc tggactgtgg cagtctgtgt agaagaggac gaaggggtca 1440

gggaggaaac actactgccc cacccctgac acctcagccc accctctcca tgcctctctc 1500

tgcgtcacca ccactcctaa acctagtcct gatttatcag agttgtttgt ttgtttgttt 1560

gtttttgttt tttaacaaaa ctaagaatga aaaaaaaaaa aaaaaaaaaa aaaaaaagta 1620

ccttctgagg cgaaaagaac cag 1643

<210> 261

<211> 9136

<212> DNA

<213> Rattus norvegicus (Norway rat)

<400> 261
agagacggca gcagcgtccc gtccagttcg cctgccgcgc tcgcttgtcg tctgcctcca 60

gatcccagac agagaagagc catggaggag gtggtgatag ccggtatgtc cgggaaattg 120

cccgagtcag agaacctgca ggagttctgg gccaacctca ttggcggtgt ggacatggtc 180

acagacgatg acaggaggtg gaaggctggg ctctatgggt tgcctaagcg gtctggaaag 240

ctgaaggatc tgtccaagtt cgacgcctcc ttttttgggg tccaccccaa gcaggcacac 300

acaatggacc cgcagctccg gctgctgctg gaagtcagct atgaagctat tgtggacgga 360

ggtatcaacc cggcctcact ccgaggaaca aacactggtg tctgggtggg tgtgagtggt 420

tccgaggcgt cggaggccct gagcagagat cctgagactc ttctgggcta cagcatggtg 480

ggctgccaga gagcaatgat ggccaaccgg ctctctttct tcttcgactt caaaggaccc 540

agcattgccc tggacacagc ctgctcctct agcctactgg cactacagaa tgcctatcag 600

gctatccgca gtggggagtg ccctgctgcc attgtgggcg ggatcaacct gctgctaaag 660

cctaacacct ctgtgcagtt catgaagcta ggcatgctca gccccgatgg cacctgcaga 720

tcctttgatg attcagggaa cgggtattgc cgtgctgagg ctgtcgtggc agttctgctg 780

actaagaagt ccttggctcg gcgagtctat gccactattc tgaatgccgg gacgaacaca 840

gatggctgca aggagcaagg cgtgacattc ccctctggag aagcccagga acaactcatc 900

cgttctctgt atcagccggg cggtgtggcc cccgagtctc ttgaatatat tgaagcccat 960

ggcacgggca ccaaggtggg ggacccccag gaactgaacg gcattactcg gtccctgtgt 1020

gctttccgcc agagcccttt gttaattggc tccaccaaat ccaacatggg acaccctgag 1080

cctgcctcgg ggcttgcagc cctgaccaag gtgctgttat ccctagaaaa tggggtttgg 1140

gcccccaacc tgcatttcca caaccccaac cctgaaatcc cagcacttct tgatgggcgg 1200

ctgcaggtgg tcgataggcc cctgcctgtt cgtggtggca tcgtgggcat caactcgttt 1260

ggcttcggag gtgccaatgt tcacgtcatc ctccagccca acacacagca ggccccagca 1320

cctgccccac atgctgccct accgcatttg ctgcatgcca gtggacggac catggaggca 1380

gtgcagggcc tgctggaaca gggccgccag cacagtcagg acttggcctt tgtgagcatg 1440

ctcaatgaca ttgcagcaac ccctacagca gccatgccct tcagaggtta cactgtgtta 1500

ggtgttgagg gccatgtcca ggaagtgcag caagtgcctg ccagccagcg cccactctgg 1560

ttcatctgct cagggatggg cacacagtgg cgtggaatgg ggctgagcct tatgcgcctg 1620

gacagtttcc gtgagtccat cctgcgctct gatgaggctc tgaagccctt gggagtcaaa 1680

gtgtcagacc tgctgctgag cactgatgag cacacctttg atgacatcgt gcattccttt 1740

gtgagcctca ccgccatcca gattgccctc atcgacctgc tgacgtctat ggggctgaaa 1800

cctgatggca tcattgggca ctccttggga gaggttgcct gtggctatgc agatggctgt 1860

ctctcccaga gagaggctgt gcttgcagcc tactggagag gccagtgcat taaggatgcc 1920

aaccttccgg ctggatccat ggcagctgtt ggtttgtcct gggaagaatg taaacaacgc 1980

tgccctcctg gtgtggtgcc tgcctgccac aactctgagg acactgtgac catctctgga 2040

cctcaggctg cagtgaatga atttgtggag cagctaaagc aagagggcgt gtttgccaag 2100

gaggtgcgaa caggtggcct ggccttccac tcctacttca tggaaggaat tgcccccacg 2160

ctgctgcagg ctctcaagaa ggtgatccgg gagccacggc cacgctcagc acgctggctc 2220

agcacctcta tccctgaggc ccagtggcag agcagcctgg cccgcacatc ttctgctgag 2280

tacaacgtca acaacctggt gagccctgtg ctcttccagg aagcactgtg gcacgtcccc 2340

gagcacgccg tggtgctgga gattgcaccc catgcactgt tgcaggctgt cctgaagcga 2400

ggcgtgaagc ctagctgcac catcatcccc ttgatgaaga gggaccataa agataacttg 2460

gagttcttcc tcaccaacct cggcaaggtg cacctcacag gcatcgacat caaccctaat 2520

gccttgttcc cacctgtgga attcccggtt ccccgaggga ctcctctcat ctcccctcac 2580

atcaagtggg accacagtca gacttgggat atcccagttg ctgaagactt ccccaacggt 2640

tccagctcct cctcagctac agtctacaac attgacgcca gttccgagtc atctgaccac 2700

tacctggtcg accactgcat tgacggccgt gtcctcttcc ctggcactgg ctacctgtac 2760

ctggtgtgga agacactggc tcgaagcctg agcttgtccc tagaagagac ccctgtggtg 2820

tttgagaacg tgacatttca tcaggccacc atcctgccca ggacaggaac cgtgcctctg 2880

gaggtgcggc tgctagaggc ctcacatgca tttgaggtgt ctgacagtgg caacctgata 2940

gtgagcggga aagtgtacca gtgggaagac cctgactcca agttattcga ccacccagaa 3000

gtcccgatcc ccgccgagtc cgagtctgtc tcccgcttga cgcagggaga agtatacaag 3060

gagctgcggc tacgtggcta tgactatggc cctcatttcc agggcgtcta tgaggccacc 3120

ctcgaaggtg agcaaggcaa gctgctctgg aaagacaact gggtgacctt catggacaca 3180

atgctgcaga tatccatcct gggcttcagc aagcagagtc tgcagctacc cacccgtgtg 3240

actgccatct atattgaccc tgcaacccac ctgcagaagg tgtacatgct ggagggagac 3300

actcaagtgg ctgacgtgac cacgagccgc tgtctgggcg tgaccgtctc tggtggtgtc 3360

tacatttcga gactacagac aacagcaacc tcacggcggc agcaggaaca gctggtcccc 3420

accctggaga agtttgtctt cacaccccat gtggagcctg agtgcctgtc tgagagtgct 3480

atcctgcaga aagagctgca gctgtgcaag ggtctggcaa aggctctgca gaccaaggcc 3540

acccagcaag ggctgaagat gacagtgcct gggctagagg accttcccca gcatggactg 3600

cctcgactct tggctgctgc ctgccagctg cagctcaacg ggaacctgca actggagtta 3660

ggtgaggtac tggctcgaga gaggctcctg ctgccagaag accctctgat cagtggcctc 3720

cttaactccc aggccctcaa ggcctgcata gacacagccc tggagaacct gtctactctc 3780

aagatgaagg tggtggaggt gctggctgga gaaggccact tgtattccca catctcagca 3840

ctgctcaaca cccagcctat gctgcaactg gagtatacag ccaccgaccg gcacccccag 3900

gccctgaagg atgttcagac caagctgcag cagcatgatg tagcacaggg ccagtgggac 3960

ccttctggtc ctgctcctac caacctgggt gctcttgacc ttgtggtgtg caactgtgcg 4020

ttagccaccc tgggggatcc agccctggcc ctggacaaca tggtagctgc cctcaaggat 4080

ggtggtttcc tgctaatgca cacagtgctc aaaggacatg cccttgggga aaccctggcc 4140

tgcctccctt ctgaggtgca gcctgggccc agcttcttaa gccaggaaga gtgggagagc 4200

ctgttctcaa ggaaggcact gcacctggtg ggccttaaaa agtcattcta cggtactgcg 4260

ctgttcctgt gccgccgtct cagcccacag gacaagccca tcttcctgcc tgtggaggat 4320

actagtttcc agtgggtgga ctctctgaag agcattctgg ccacatcctc ctcccagcct 4380

gtgtggctaa cagccatgaa ctgccccacc tcaggtgtgg taggcttggt gaactgtctc 4440
cgaaaagagc cgggtggaca ccggattcgg tgtatcctgc tgtccaacct cagcagcaca 4500
tctcacgtcc ccaagctgga ccctggctct tcagagctac agaaggtgct agagagtgat 4560
ctggtgatga acgtgtacag ggacggtgcc tggggtgcct tccgtcactt ccagttagag 4620
caggacaagc ccgaggagca gacagcacat gcctttgtaa acgtccttac ccgaggggac 4680
cttgcctcca tccgctgggt ctcttctccc ctgaaacaca tgcagccgcc ctcgagctca 4740
ggagcacagc tctgcactgt ctactatgcc tcactgaact tccgagatat catgctggcc 4800
acgggcaagc tgtcccctga tgccattcca ggtaaatggg ccagccggga ctgcatgctt 4860
ggcatggagt tctcaggccg tgataagtgc ggccggcgtg tgatggggct ggtacccgca 4920
gaaggcctgg ccacctcagt cctgttatca cccgacttcc tctgggatgt accctctagc 4980
tggaccctgg aggaggcggc ttctgtgcct gttgtctaca ccaccgccta ctactcctta 5040
gtagtgcgtg gtcgtattca gcacggggaa actgtgctca ttcactcggg ctccggtggt 5100
gtgggccaag cggccatttc cattgccctt agcctgggct gccgagtctt caccactgtg 5160
ggctccgctg agaagcgagc ttacctccag gccagattcc ctcagctgga tgacaccagc 5220
tttgctaact ctcgagacac atcgtttgag cagcatgtgt tactgcacac aggtggcaaa 5280
ggggtggacc tggtcctcaa ctccctggca gaagagaagc tgcaggccag tgtgcggtgc 5340
ttggctcagc atggccgctt cctagagatc ggcaaatttg atctttctaa caaccaccct 5400
ctgggcatgg ccatcttctt gaagaacgtc actttccatg ggatcctgct ggatgcactt 5460
tttgaggggg ccaacgacag ctggcgggag gtggcagagc tgctgaaggc cggcatccgt 5520
gatggggttg tgaagcctct caagtgtaca gtgtttccca aggcccaggt ggaggacgcc 5580
ttccgataca tggctcaagg aaaacatatt ggcaaagtcc ttgtccaggt acgggaggag 5640
gagcccgagg ctatgctgcc aggggctcag cccaccctga tttccgccat ctccaagacc 5700
ttctgcccag agcataagag ttacatcatc actggtggcc taggtggctt tggcctggaa 5760
ctggcccggt ggcttgtgct tcgtggggcc caaaggcttg tactaacttc ccgatctgga 5820
atccgcacag gctaccaagc caagcacgtt cgggagtgga ggcgccaggg catccatgtg 5880
ctagtgtcga caagcaatgt cagttcactg gagggggccc gtgctctcat cgctgaagcc 5940
acaaagcttg ggcccgttgg aggtgtcttc aacctggcca tggttttaag ggatgccatg 6000
ctggagaacc agactccaga actcttccag gatgtcaaca agcccaagta caatggcacc 6060
ctgaaccttg acagggcgac ccgggaagcc tgtcctgagc tggactactt tgtggccttc 6120
tcctctgtaa gctgcgggcg tggtaatgct ggccaatcca actatggctt cgccaactct 6180
accatggagc gtatttgcga acagcgccgg cacgatggcc tcccaggtct tgccgtgcaa 6240
tggggtgcca ttggtgacgt gggcattatc ttggaagcga tgggtaccaa tgacacagtc 6300
gttggcggca cactgccaca gcgcatctcc tcctgcatgg aggtgctgga cctcttcctg 6360
aatcagcccc acgcagtcct gagcagtttt gtgctggttg agaagaaagc tgtggcccat 6420

ggtgatggtg aagcccagag ggatctggtg aaagcagtgg cacacatcct aggcatccgc 6480
gacctcgcag ggattaacct ggacagctcg ctggcagacc tcggcctgga ctcgctcatg 6540
ggtgtggaag tgcgccagat cctggaacgt gaacatgatc tggtgctacc cattcgtgaa 6600
gtacggcaac tcacactgcg gaagcttcag gaaatgtcct ccaaggctgg ctcagacact 6660
gagttggcag cccccaagtc caagaatgat acatccctga agcaggccca gctgaatctg 6720
agtatcctgc tggtgaaccc tgagggccct accttaacac gactcaactc agtgcagagc 6780
tctgagcggc ctctgttcct ggtgcacccc attgaaggtt ccatcactgt gttccacagc 6840
ctggctgcca agctcagtgt gcccacctac ggtctgcagt gcacccaagc ggcccccctg 6900
gacagcattc caaacctggc tgcctactac attgattgca tcaagcaggt gcagcctgag 6960
gggccccacc gagtggctgg gtattctttt ggagcttgtg tagccttcga gatgtgctcc 7020
cagctgcagg cccagcaggg cccagccccc gcccacaaca acctcttctt gtttgatggc 7080
tcacacacct acgtattggc gtacacccag agctaccggg caaagctgac cccaggctgt 7140
gaggctgagg ctgaagctga agccatatgc ttcttcatta agcagtttgt tgatgcagag 7200
catagcaagg tgctagaggc cctgctacca ctgaagagcc tggaggaccg ggttgctgct 7260
gctgtggacc tcatcactag aagccaccag agcctggacc gccgtgacct gagctttgct 7320
gccgtgtcct tctactacaa gcttcgagcc gccgaccagt ataaacccaa ggccaagtac 7380
cacggcaatg tgatcctgct gcgggccaag acaggtggca cctacggcga ggacttgggt 7440
gccgattaca acctgtccca ggtgtgtgat gggaaggtgt ctgtgcacat cattgagggt 7500
gaccatcgta cgctgctgga gggcaggggc ctggagtcta tcatcaacat catccacagc 7560
tccctggctg agcctcgagt gagtgtacgg gagggctaga cctgcctacc atgaagccac 7620
gacccacacc ggccaccaga gatgctccga tccccatcac accctgagtg cagggactgg 7680
ggagggtcct gctggtggga ccccctcacc ccagtggccc agcaccaccc cctcccctgg 7740
tggctgctac aaacaggacc atcacatgtg tcccagccac ttagtggggt tcccagagcc 7800
actgacttgg aggcaccctg gtctgtgaag agtcagtgga ggccagcaag agccaaactg 7860
agccttttct gccaagtgac atttgtcaca ctggttgttt ctccattaaa ttctcatatt 7920
tattgcattg ctgggaaaga ccgcccaccc cagggttaac tcattccaga acccctaaag 7980
tgggaaaagc catgtgggga aggctgctgg ctggagcccc tttttgtctt agccctgtac 8040
ccgctcactg cagggcaggg tatggagagg gctggttccc ggggaacgag gaccccagca 8100
gacactgtag cccatggccc ttggtcccca gcactcccgg ctgcacccat gatgcagggc 8160
ctaccagact ctgcggaccg caccgggcac tcactgtatt tgttttccaa gattcaaatt 8220
gctgcttggg ttttgaattt actgcagctg tcagtgtaaa gaaacatgtc tgaactgtgt 8280
cctttttaca ccaacctggt aaaaatgctc ttgatgctgt cccgttgcca caattaaact 8340
gcacgtgagc tctggcttcc gttcagtctc tttccagtcc cagacctgag tccccagagc 8400
ctccacagct cttacagtga gaatcaaatt ggcccactcc ttggaaggcg tggcattctg 8460

tcagagtaaa aggaaagtag agtgtgctga ttcacgttca gcgtgtgggg ctggctagag 8520

accttggcac tgtagtgaac agaatgtgtc cacctttaag tcaccctgaa ggcatcacca 8580

tagctacagc ctcacccagg ggtagagaat agtactgtct acttgttgac tacctggcag 8640

ttggtgccag cccctataga ggaaaacagc agtgtgtggc cactgtgaga agcatatccc 8700

tggaaacagg tgaccagagc agagggctaa tgcctacctg agtcacacaa aactgaccag 8760

gcttgagtgt ccagaagagt ctatcagaag gccacagcat tcagtcctat ccacagagag 8820

cagcagacta agttgtctcc ttgccagctt agaaaactgc agtgctgggg tacaggtagg 8880

gtgttcagga ggtccgggcc ccagtgatta gtctaagact gaagcatctg gttggctgtg 8940

gtcccaccta gaaaattctt aaagctcttg tcatgtactt cctgggaagg acctaccctg 9000

tctcaataat gtctctagct cgttggagtc tactgactca aacatttata aagtgtccta 9060

gaaaggcctg actcccctac aaggctgtgt gatccttcaa actcacatat gtgagccaat 9120

aaaaccttga gactct 9136

<210> 262

<211> 3679

<212> DNA

<213> Rattus norvegicus (Norway rat)

<400> 262
tgacatggta aagagtctgg ccacagataa aggaccgaaa tgactgactc aaaatacttc 60

acaacaacca agaaagggga gatcttcgag ttgaaggcag agcttaacag tgacaagaag 120

gagaagaaga aggaggcagt aaagaaagtg attgcatcaa tgaccgtggg caaagatgtc 180

agtgctctct tccctgatgt ggtgaactgc atgcagacag acaacctgga gctaaagaag 240

ctggtgtacc tgtacttgat gaactatgcc aagagtcagc ctgacatggc cattatggct 300

gtcaacacct tcgtgaagga ctgtgaggac cccaaccccc ttatccgggc cctggctgtg 360

aggactatgg gctgcatccg agttgacaag atcacagagt acttgtgtga accactccgg 420

aaatgcctga aggatgaaga cccatatgtg cgcaagacag cagctgtgtg tgtagccaag 480

ctccacgaca tcaacgccca gctggtagag gaccagggtt tcctggacac actcaaagac 540

ctcatctctg actctaaccc catggtggta gcaaatcgag tggctgccct gtcagagatc 600

gctgagtctc accccagtag caacctgctt gacttgaagg cacagtctat caacaagctg 660

ctgacggccc ttaatgagtg caccgagtgg gcccagatct tcattctgga ctgtctgggc 720

aactacatgc ccaaggatga ccgtgaggcc cagagcatct gcgagcgggt cactcctagg 780

ctttcccatg ccaactctgc tgtggtgctg tccgcagtga aggtcctgat gaagttcatg 840

gagatgctgt ctaaggacct ggactactat gccacactac tcaagaagct ggcccctccc 900

ctggtcaccc tgctgtcagc agagccagag ccgcagtatg tgcctctgcg caatattaac 960

ctcattgtac agaaaaggcc agagattttg aagcatgaaa tgaaggtttt ctttgtgaag 1020
tacaatgacc ccatctatgt gaagctggag aaactggaca ttatgatccg cctggcctcc 1080
caggccaaca ttgcccaggt tttggcagaa ctcaaagagt acgcgacaga agtagatgtg 1140
gattttgtac ggaaggcagt gcgtgccatt ggccgctgtg ccatcaaggt ggagcaatct 1200
gcagagcgct gtgtgagcac attgcttgat ctcatccaga ccaaggtcaa ctatgtggtc 1260
caggaagcca ttgtggtcat taaagacatc ttccgcaagt accccaacaa gtacgagagt 1320
gtgatagcca cactgtgtga gaatctggac tcgctggatg agcctgaggc tcgcgctgct 1380
atgatctgga tagtgggcga gtatgctgag cggattgaca atgcggacga gctgcttgag 1440
agcttcctcg acggcttcca tgacgagagc acccaggtcc agctacagct gctgacagcc 1500
attgtgaagc tcttcctgaa gaagccaaca gagacgcagg agctggtgca gcaggtcctc 1560
agtttggcca ctcaggactc ggacaatcca gaccttcggg accgtggcta catctactgg 1620
cgcctgctct ccacggaccc tgtagctgcc aaagaggtgg tactggctga gaagcccctc 1680
atctcagaag agacagacct tattgagccc acactgctgg atgaactcat ctgctacatt 1740
ggcacactgg cctctgtcta ccacaagcca cccaatgcct ttgtggaggg cggccgggga 1800
gtggtgcaca agagtctgcc acctcgcact gcctcgagcg aaagcacaga gagccctgag 1860
gcagcccctg ccggagcacc tgctagtgac cagcctgatg tcattcctgc ccagggtgac 1920
ctgttaggtg acctcctcaa cctggacctt gggcccccag tgagtggccc acccttggct 1980
gcctcctcag tgcagatggg agctgtggat cttcttggtg gtggcctcga cagcctgatg 2040
ggggatgagc ctgaaggatt tggagacagc aactttgggg cacctccagc atcagtggcg 2100
gcggcagcac cagccagact tggagcaccc gtcagcagtg gcttaagtga cctctttgac 2160
ctgaccagtg gagtgggcac cctctcaggg tcctatgtcg ctcccaaagc agtttggctc 2220
cctgccatga aggccaaggg gctggaaatc tctggcacct tcacccggca ggttggctcc 2280
atctccatgg atctgcagct gactaacaag gccctgcagg tcatgacaga tttcgctatc 2340
cagtttaatc ggaacagctt tggcttggcc cccgctgcgc cactccaagt gcacgcccca 2400
ctgagcccca accagacggt ggagatctcc ctgcctctta acacggtggg ctcagtcatg 2460
aagatggagc ctctgaacaa tcttcaggtg gctgtgaaga acaacattga cgtcttctac 2520
ttcagcactt tgtacccact gcatgtcctc ttcgtggagg acgggaagat ggaccggcag 2580
atgttcctgg ccacatggaa ggacattccc aacgagaatg aagcccagtt ccagatcaga 2640
gactgcccgc tgaacacaga ggctgcaagc agcaagctgc aaagcagtaa cattttcact 2700
gtcgccaaga ggactgtgga gggccaggac atgctctacc aatctctgaa gttgaccaat 2760
ggcatctggg tgctggcaga gctgcggatc cagccaggca accccagttt cacgctgtcc 2820
ctgaagtgtc gagccccaga ggtgtcccag catgatatcc aggcctacga gaccatcctc 2880
aagaactgaa gtccctctcg ctccacccca gccttctcct tctgccagcc cagccatgga 2940
accctgtggt ggcagcacct cctcctttct ttggggagaa gccagtgggc tcctgaccca 3000

cccaggaggc ctcccctgat cctaactgag ggcctggagc agggccaggt ctcctagagt 3060

gaaatctccg tgcatctcca gtggctgctt ttttctattg ctgttgatgt gggatcaaag 3120

gaagtggatc cagcacagca tcagaagctg gacgggggcc acagggtgct cttccttcct 3180

tctgctctcc tggggtgaac atggaaccct cctggggtcc cagcccataa tccctgccct 3240

gtctcctcag tgagcctgtc tgggtagagt cctctgggtg ccaatattac tgtttgggac 3300

tctgagagct ggaggtggtg atggacagac atgagcccag gggagcaacc tgggctggca 3360

ctgtctgccc catataacac cagttggtgc tctgtgacca cctgcatggc ccagagcccg 3420

atctaggatt ctgttccctt aggccctgga ggatggtcac tgcctaccaa acattcctcg 3480

gtgtcctctc tgtgagtcca gggctctcag accagaagtg gggtgaaagg tggggcaagg 3540

ggcagtggga ccgcttctag tgtgagctcc tgacccactg gctgccctgg ctccccagtc 3600

tgtgtgtgac agtgacaggt tgatcgtcgc tactgtttga taataaagag ttctgctttt 3660

ggcaaaaaaa aaaaaaaaa 3679

<210> 263

<211> 1663

<212> DNA

<213> Rattus norvegicus (Norway rat)

<400> 263
cagccacatt ttgtccacaa actctgtcct gaaaggggac tgactgaaga aaacatccag 60

caagctctgg gcaaggaagg acagcagcag agagcgaggg ccgtgttcgc tgtgccagag 120

gatggaggtg cacaacgtat cagccccttt caatttctcc ctgccgcctg gctttggcca 180

ccgggccaca gacaaggcgc ttagcatcat cctggtgtta atgttgctgc ttatcatgct 240

ctcactgggc tgcaccatgg aattcagcaa gatcaaggct cacttgtgga agcccaaagg 300

ggtgatcgtt gccttggtgg cccagtttgg catcatgccc ctcgctgctt ttcttctcgg 360

caagatcttt cacctgagca acattgaagc tctggccatc ctcatctgtg gctgctctcc 420

cggggggaac ttgtccaacc tcttcaccct ggccatgaag ggggacatga acctcagcat 480

cgtgatgacc acctgctcca gcttcagtgc cttgggcatg atgccactcc tcttatacgt 540

ctacagcaaa ggcatctacg atggagacct taaggacaag gtgccctaca aaggcattat 600

gatatcacta gtcatagttc tcattccttg caccataggg atcgtcctca agtccaaaag 660

gccacactat gtaccctaca tcctcaaggg aggcatgatc atcaccttcc tcctctctgt 720

ggctgtcaca gccctctctg tcatcaatgt gggcaacagc atcatgttcg tcatgacacc 780

acacttactg gctacctcct ccctgatgcc cttctctggc tttctgatgg gttacattct 840

ctctgctctc ttccaactca atccaagctg cagacgcacc atcagcatgg aaacaggatt 900

ccaaaacatt caactctgtt ctaccatcct caatgtgacc ttcccccctg aagtcattgg 960

gccacttttc ttctttcctc tcctctacat gattttccag cttgcagaag gacttctcat 1020

catcattatc ttccggtgct atgagaaaat caagcctcca aaggaccaaa caaaaattac 1080

ctacaaagct gctgcaactg aggatgctac tccagcagct ctggaaaaag gtacccacaa 1140

tgggaatatt cctcctctcc aacctggtcc ttcccctaat ggcctgaatt ctggtcagat 1200

ggcaaattag aatgtgaaac ttcgaagcag caagaaaagg aacgaacgtc gacgttgccg 1260

gaatgtttgt ctagcacttc gggcaaacca tcagaaccat ggagccatga actgagacag 1320

aagggcatct atctatccag taactgtaac ccataccaat ttgcttttgt ttaaattttc 1380

tatttaaaag ataaacaaga attaggcaaa aatgttcctg cctataatcc cgatgctcag 1440

aaactcaaga tcaaccttaa gtatacaaaa caagactgtc tcaagaaacc aaaaacactt 1500

ttcagtggct atgaactcta tgaaagctga accaaacagc ttcatctgat aaacattaac 1560

ttcactattt ccaaactttc cagtaagcag gtgttttgtt cattaaacat ccacaacctg 1620

cttcatgtta ctcaaaatga aataaagtgc aactcctagt tct 1663

<210> 264

<211> 485

<212> DNA

<213> Rattus norvegicus (Norway rat)

<400> 264
gaattcattt ctctactgaa accttaaagt agtatttggt cattcattgt atgtgtctta 60

tatacccacc cacacacaga cacaaaggaa atctatcaaa ttaatcctga tgtattcgaa 120

gtggttattc aattcattta tggcagagca atatcagtcc taatgactcg tgaaagtgtt 180

actaactgaa tcattatctt acatttactg tttagtaagc atattttgaa aaatgtatgg 240

ctagagtgtc ataataaaat ggtatatctt tctttagtaa ttacattaaa attaatcatg 300

tttgattaac tggttcctct gatgtagtgt gtcatttctt aacatataga gaactgtaga 360

gcattttttt atttaatggc agtgtttgca actcacaggc acagaaacac tcagactaat 420

catttatcat agagatatag aacttacagg ttaaagtatt cacaatgtat gtaagagagt 480

tatga 485

<210> 265

<211> 4254

<212> DNA

<213> Rattus norvegicus (Norway rat)

<400> 265
gctcccatct tcgaggctca gctcaactca gagctacttc ttccaaattc tacatcttgg 60

cggacttcgc gaaggaaacc cggagtgtta cgtgaggtcc tgatggagtt tgaagagggc 120
cttaacggaa gagcagacaa gaacttctca aagatgggca aaaagagtaa aaaggagaag 180
gagaagaaac ctgctgttgg catattcggg atgtttcgct atgcagattg gcttgacaag 240
ctgtgcatgg ctctgggaac tctcgctgct atcatccacg gaaccctgct tcccctcctg 300
atgctggtgt tcggatacat gacagatagt tttaccccaa gcagagaccc gcattctgac 360
cgagcgatta ctaatcaaag tgaaatcaac agtacacata ccgtcagcga cacgagtctg 420
gaggaggaca tggccatgta tgcctactat tacacgggca ttggtgccgg tgtgctcatc 480
gttgcctaca tccaggtttc actttggtgc ctggcagctg ggagacaaat acacaagatt 540
aggcagaagt ttttccatgc catcatgaat caggagatag gctggtttga cgtgaatgac 600
gctggggagc tcaacacccg tctcacagat gacgtctcca aaattaatga cggaattggt 660
gacaaacttg gaatgttctt tcagtccata acgacatttt cagccggttt tataatagga 720
tttataagtg gttggaagct aacccttgta attttggccg tcagccctct tattgggttg 780
tcatctgcca tgtgggcaaa ggtactgact tcatttacta ataaggaact ccaggcttat 840
gcgaaagctg gagcagttgc cgaagaagtc ttagcagcca tcagaactgt gattgcgttt 900
ggaggacaaa agaaggaact tgaaaggtac aataaaaatt tagaagaagc taaaagagtt 960
ggcataaaga aagccatcac ggccaacatt tccataggta ttgcctacct gttggtctat 1020
gcgtcttatg cactggcatt ctggtatggg acctccttgg tcctctcaaa tgaatattct 1080
attggacaag tgcttaccgt cttcttctct attttattgg ggactttcag tattggacat 1140
ttagccccaa acatagaagc ctttgcaaat gcaagagggg cagcctatga aatcttcaag 1200
ataattgata atgagccaag catcgacagc ttctcaacca agggacacaa accagacagt 1260
ataatgggaa atttggaatt taaaaatgtt tacttcaact acccatcacg aagtgaagtt 1320
aagatcttga agggcctcaa cctgaaggtg aagagcgggc agacggtagc cctggttggc 1380
aacagtggct gtgggaaaag cacaactgtc cagctgctgc agaggctcta cgaccccata 1440
gagggcgagg tcagtattga cggacaggac atcaggacca tcaatgtgag gtatctgcgg 1500
gaaatcattg gggtggtgag tcaggaaccc gtgctgtttg ccaccacgat tgccgaaaac 1560
attcgctatg gccgagaaaa cgtcaccatg gatgagatag agaaagctgt caaggaagcc 1620
aatgcctatg acttcatcat gaaactgccc cacaaatttg acaccctggt tggtgagaga 1680
ggggcgcagc tgagtggggg acagaaacag aggatcgcca ttgcccgggc cctggtccgc 1740
aaccccaaga tccttttgtt ggatgaggcc acgtcagcct tggacacaga aagcgaagcc 1800
gtggttcagg ccgctctgga taaggctaga gaaggccgga ccaccattgt gatagctcac 1860
cgcttgtcta cagtgcgcaa tgctgacgtc attgctggtt ttgatggtgg tgtcattgtg 1920
gagcaaggaa atcatgaaga gctcatgaaa gagaagggca tttacttcaa acttgtcatg 1980
acacagacta gaggaaatga aattgaacca ggaaataatg cttatgaatc ccaaagtgac 2040
actggtgcct ctgagttgac ttcagaagaa tcaaaatctc ctttaataag gagatcaatt 2100

cgcagaagta tccacagaag acaagaccag gagagaagac ttagttcgaa agaggatgtg 2160
gatgaagatg tgcctatggt ttccttttgg cagatcctaa agctaaatat tagtgaatgg 2220
ccctatttag ttgtgggtgt actttgtgct gttataaatg ggtgcataca accagtgttt 2280
gccatagtgt tttcaaagat tgtaggggtt ttttcaagag acgacgacca tgaaaccaaa 2340
caacggaatt gtaacttgtt ttcccttctc tttctggtca tgggaatgat ttcttttgtt 2400
acgtacttct ttcaaggctt cacatttggc aaagctggag agatcctcac caagcgactc 2460
cgatacatgg tcttcaaatc catgctgcga caggatataa gctggtttga tgaccataaa 2520
aacaccactg gctcgctgac taccaggctc gctagtgacg cttctaatgt taaaggggct 2580
atgggctcca ggcttgctgt agttacccag aatgtagcaa accttggcac aggaattatc 2640
ttatccttag tcttagtcta tggctggcag cttacacttt tacttgtagt aattatacca 2700
ctcattgtct tgggtggaat tattgaaatg aaactgttgt ctggtcaagc cttgaaggac 2760
aagaaagagc tagagatctc tgggaagatc gctacagaag caattgaaaa cttccgcact 2820
gttgtctctt tgactcggga gcagaagttt gaaactatgt atgcccagag cttgcagata 2880
ccatacagaa atgctttgaa gaaagcacac gtctttggga tcaccttcgc cttcacccag 2940
gccatgattt atttttccta tgctgcttgt ttccggttcg gtgcctactt ggtggcacga 3000
gaactcatga cgtttgaaaa tgttatgttg gtattttctg ctgttgtctt tggtgccatg 3060
gcagcaggga ataccagttc attcgctcct gactacgcga aggccaaagt ctcggcatcc 3120
cacatcattg ggatcattga gaaaatcccc gagattgaca gctacagcac ggagggcttg 3180
aagcctaatt ggttagaagg aaatgtgaaa tttaatggag tcaagttcaa ctatcccacc 3240
cgacccaaca tcccagtgct tcagggactg agcttcgagg tgaagaaggg gcagacgctc 3300
cgcctggtgg gcagcagtgg ctgcgggaag agcacggtgg tccagctgct cgagcgcttc 3360
tacaacccca tggctggaac agtgtttcta gatggcaaag aaataaagca actcaatgtc 3420
cagtgcgtcc gcgcactggg cattgtgtcc caggagccca tcctgtttga ctgcagcatc 3480
gccgagaaca tcgcctacgg agacaacagc cgtgtcgtgt ctcatgagga gatcgtgagg 3540
gccgccaggg aggccaacat ccaccagttc atcgactcac tgcctgagaa atacaacacc 3600
agagtgggag acaaaggaac tcagctgtcg ggcgggcaga agcagcgcat cgccatcgcg 3660
cgcgccctcg tcagacagcc tcacatctta cttctggatg aagcgacatc agctctggat 3720
acggagagtg aaaaggtcgt ccaggaagcg ctggacaaag ccagggaagg ccgcacctgc 3780
gttgtgatcg cgcaccgcct gtccaccatc cagaacgcag acttgatcgt ggtgattcag 3840
aacggccagg tcaaggagca cggcacccac cagcagctgc tggcccagaa aggcatctat 3900
ttctcgatgg ttcaggctgg agcaaagcgc tcatgagctg ggagtatttg aggtgctaag 3960
tatttctaat attggtgttc aaacatggca cgtaaccaaa gttaaaaggt taaaagcact 4020
gttaaaggta atttcatcaa gacgagaagc cttcagagac ttcataatta aatgaaccga 4080
aattgaaaaa aaaatcatta aacagggcca cattttttaa ttgtattatg tgattcaaga 4140

gaacatatag tttttttaaa aagaaatgtg tagttttgtt tcagtttttt taatttctac 4200

cctattccct taaatgatca taaaggctgt aaaaagcact atttttttgc ggcc 4254

<210> 266

<211> 2667

<212> DNA

<213> Rattus sp.

<400> 266
gtaaactgct ctgtggactg ccaactccag gtcttcaatt ggtcctgtcc atctactccc 60

tcaactcccc caccctcaac accaacaaca ccaacatcct cccagacaac cacaccctcc 120

acaccatcta ccacatcttc gaagtctaca ccctctacgc cccaatccac atcatccaag 180

tcaacgccca gcacaccacc caaaaccacc cttcctgggt gcctcgattt tgatcctccc 240

agacaggtca atgagacctg gtggctgtgt aactgtacca tggccatctg caaatatgac 300

aacgtagtgg agattgtgga gctggagtgc aaccccccac ccatgcccac ctgttccaat 360

ggcctcaaac ccgtgcgtgt ccctgaccct gatggctgct gctggcactg ggaatgtgac 420

tgctactgca ctggctgggg ggacccacac tttgtcacct tcgatggact ttactacagt 480

taccagggta actgtaccta tgtgctggta gaggagatta cccccacagt ggacaacttt 540

ggggtctaca ttgacaacta ccactgcgat gccaacgaca aggtgtcctg cccccgcaca 600

cttattgtgc accatgagac ccaggaagtg ctgatcaaga ctgtgcacat gatgcccatt 660

gaggtggagg tacaggtgaa caagcagtta gtggctttgc cctacaagaa gtatggactg 720

gaggtatacc aatctggcat caactttgtg gtggacatcc ccaggctggg tgcccaggtc 780

tcctacaatg gcctgtcttt ctccatccgg ctgccttacc acctgtttgg caacaacaca 840

aagggccaat gtggcacctg caccaacaat actgctgatg actgtatcct acccagtggg 900

gagatcattt ctaactgtga agttgctgct gatgagtggt tggtgaatga cccgtccaag 960

ccacactgtc cccacaaagg cctcaccact aagcgcccag ccatcacgac accaggacct 1020

ttccctgaaa actgcactgt gtctcctgtc tgtcagctca tcatggacag cttattctcc 1080

cagtgccacc ccttcgtgcc tcccaagcac tactacgaag cttgcctgtt tgatagctgc 1140

tttgtggctg gctctggtat ggagtgtgcc agtgtacagg cctatgctgc cctctgtgct 1200

caggaaggtg tctgcattga ctggcggaat cacacccagg gggcctgcgc tgtgacgtgt 1260

ccagctcaca ggcagtatca ggcctgtggc ccttcagagg aacccacctg ccagtccagc 1320

tcccctaaga attccacgct cttggtggaa ggctgcttct gtcctgaagg gaccacaaag 1380

tttgcccctg gctatgacgt ctgtgtgaag atatgtggat gtgtgggacc ggacaatgtg 1440

cccagagagt ttggagagca ctttgaattt gactgcaagg actgtgtttg cctggagggt 1500

ggaagtggta ttgtctgcca accaaagaag tgtgctaggg gcaacctgac cacatgtgag 1560

gaggatggca cctaccttgt tgtggaggcc gacccagatg acaagtgctg caacaccacc 1620

tcatgtaagt gtgaccccaa acggtgcaaa gctgaacgcc cctcatgtct gctgggattc 1680

gaagtgaaga gtgagcacgt gcctgggaaa tgctgtcctg tctactcctg tgtgcccaag 1740

ggtgtgtgcg tgcacgagaa tgctgaatac cagcctggat ccccagtgta ctctaacaag 1800

tgccaggact gtgtatgcac cgacagcatg gacaacagca cccagctcaa tgtcatctcc 1860

tgtacccatg tgccctgcaa catctcctgc agctctggct ttgagcttgt ggaggttcct 1920

ggggaatgtt gcaagaagtg ccagcagacc cattgcatca tcaagaggcc cgagcagcag 1980

tacatcattc tgaagcctgg ggagatacag aaaaacccca acgacaggtg caccttcttc 2040

agctgcatga aaatcaacaa tcagctcatc tcttcagtct ccaatatcac ctgccctgac 2100

tttgacccaa gtgattgtgt tccgggctcc atcacataca tgccgaatgg ctgctgtaag 2160

acatgcatcc ataaccctaa caacacagtc ccttgctctg ccatacccgt catgaaggag 2220

atttcctaca atggctgcgc caagaacatc tccatgaact tctgcgctgg gtcctgtggg 2280

acttttgcca tgtactctgc ccaggcccag gatctggatc atgggtgttc ttgctgcagg 2340

gaggagagga ccagtgtacg aatggtttcc ctggactgcc cagatggcag taagctgagc 2400

catagctaca cacacatcga gagctgtctg tgccagggca ccgtctgtga gctgccacag 2460

gcccagcaaa gccgcaccag gcgttctagt ccccggcttc taggaaggaa gtgaacgggt 2520

tacaccgtgc acatctcttc ccatcttgag ccatcctgct gattctctga gcttccccac 2580

ttcctgcttg acttcttctc tgcacatatt tattttcgga gtttctgttc cattctttgc 2640

tttctgataa taaactcagg catagcc 2667

<210> 267

<211> 840

<212> DNA

<213> Rattus norvegicus (Norway rat)

<400> 267
atcacgacag cctactacag gggtgccatg ggtatcatgc tggtctacga cattaccaat 60

gagaagtcct tcgacaacat ccggaattcc attcggaaca ttgaggagca tgcctctgca 120

gatgtggaga agatgatact ggggaataag tgtgatgtga acgacaagag gcaggtgtcc 180

aaggaacggg gagaaaagct ggcactcgac tatgggatca agttcatgga gaccagcgcg 240

aaggccaaca tcaatgtgga gaacgcattt ttcactctcg ccagggatat caaagcaaaa 300

atggacaaaa attggaaccc aacaaccctc aggagcagcc atggagtcaa gatcacagtg 360

gagcagcaga agaggaggag cttcttccgg tgcagtctcc tgtgaggaca aggtctctgc 420

ccatttgagc ctgcgctcag cccagctgac tgtgcctgcc ccgggcgagc ctccacctcc 480

gatgctcgga cacagctgcc cggactcaat tgagaaattg attattttag taaccgtctg 540

atcttttcca ccttcagaga tggaccaagt tagaattttg ctgtttttcc tgtgtcatct 600

gccagtcagg cctatccgtc ccctgccgca ttgccataga tggagagagg tgggttgggg 660

caaggcagcc tccatctcca gccttctgcc cctccaccac agaagggagg tcagtggcag 720

tggcagtgcc agcctgcctg gccccatact ccaagcacca ccaccagggc cactgagggc 780

aagctggcac cctgccttca cccgttctca gttgtggaca agtgacaact tgtccactgt 840

<210> 268

<211> 2042

<212> DNA

<213> Rattus norvegicus (Norway rat)

<400> 268
gaacataaag tcagattgct aaacttctgt gtcgactgaa aaacatggtg aagcgagttg 60

caattgtggg agctggggtc agtggcctgg cctccatcaa gtgctgcctg gaagaaggac 120

tagaacccac ctgcttcgag agaagctgtg acttgggagg actttggaga ttcacggaac 180

atgttgaaga aggaagagcc agcctttaca actcagtggt ttctaacagc agcaaggaga 240

tgtcttgtta ctccgatttc ccttttccag aagactaccc aaactttgtg ccaaattctc 300

tgttcctgga atatctccag ctgtatgcaa cccagttcaa ccttctgaga tgcatctatt 360

tcaacaccaa agtgtgcagt ataacaaaac gcccagattt cgctgtctct ggacaatggg 420

aagtggtcac tgtctgtcaa gggaagcaaa gctcagacac ctttgctgct gtcatggtct 480

gcactgggtt tctaactaac ccacatctgc ccctggattc ctttccaggc atacaaactt 540

ttaaggggca gtacttccac agccggcagt ataaacatcc agacgtattt aaggacaagc 600

gagtccttgt ggttggaatg ggaaattctg gtacagacat tgccgtggag gccagtcact 660

tagcgaaaaa ggtgtttctc agcaccaccg gaggggcatg ggtgatcagc cgagtctttg 720

attcagggta cccctgggac atgatattca tgacgcgatt tcagaacatg ctcagaaatc 780

ttctcccaac tccagttgtg agttggttga tatcaaagaa gatgaacagc tggttcaacc 840

acgtgaatta cggtgtggct ccagaagaca ggactcagct gagagagcct gtgctgaatg 900

atgagctccc aggccgcatc atcactggga aagtgttgat caagcccagc atcaaggagg 960

tgaaagaaaa ctctgtcgtc tttaacaata caccgaagga ggagcctatt gacgtcatcg 1020

tctttgccac tggatactcc tttgcgttcc ccttcctcga tgaatcaata gtgaaagttg 1080

aggatggcca ggcatcactg tacaagtaca tcttcccggc acatctgcca aaaccaactc 1140

tggccgtgat tggcctcatc aaacccctgg gttccatgat acccacagga gagacacaag 1200

ctcgatgggt tgttcaggtc ctgaaaggtg cgactacatt accacccccg agtgtcatga 1260

tgaaagaagt caatgaacgg aagaagaaca agcatagcgg atttggcttg tgctactgca 1320

aggctttgca atccgattac ataacgtaca tagatgacct cctgacctcg atcaacgcaa 1380

aaccggacct gcgggccatg ctcctgactg acccacgcct ggctctgagc atcttcttcg 1440

gcccatgcac accttaccat ttccgcctga ctggtccagg aaagtgggaa ggagccagaa 1500

aggccatctt gacccagtgg gaccgaacag tgaacgtcac caaaactcga accgtacaag 1560

aaaccccatc tacctttgaa actttgctta aactctttag ttttctggct ttgcttgtgg 1620

ctgttttctt tattttcctg taagtgaaag atctaactgg ctttccaaat gtgtggagta 1680

taaccttcca acttctctaa tgtaacaatt tcaccttcgt aattgtaaac cacgtccaga 1740

gacacccaac ccctacctct ccccaactca cctcattggc accttcattg ctgggtctct 1800

tgctagtcca tcaggtttag tgcaagaaaa taatgtccag caattctgtt cacttaaaat 1860

gttggaagga tccaggcccc ctttcaggaa gaatctgccc ccagagagga ctctgagcat 1920

tctttcaatc taaaaaactg ctttccctag atcttaatga aaagcccaac ttcgcggaat 1980

attggtctgc actaaaatag ttctctgtgt attagttgac tacaaataaa atggaagaaa 2040

ct 2042

<210> 269

<211> 1898

<212> DNA

<213> Rattus norvegicus (Norway rat)

<400> 269
taagtacctg agggtaaggt gttgttctct ctcatatggt catatagtct tctgagtagt 60

ctaaacctca ttatatgacc caaactggct ttgaactctt tttttttttt tttttttttt 120

tttggttctt tttttcggag ctggggaccg aacccagggc cttgcgcttc ctaggcaagc 180

gctcactgag ctaaatcccc aaccccggct ttgaactctt gatcttgttg cctcaccact 240

tcgaagggct ggaaaaataa gtgacctcac ctagctagaa tcagagatgt aagaagtgct 300

ttgccagagt tggaaatagg aagctgcgag gctggttatt ccaggatctt aataacaaca 360

tcagccagaa gtacttgaag tgctacaaac aggaagtaga tagccgaatt cccagacttt 420

tggtctggat gtactgacac cgcccaccgc tcggtgggct gatggccctc gcgccaccct 480

ttcattggcc agtcgtcaca aggcccatac tcacacgagg tcattcagcc aatcagcgaa 540

ctcggcctcg cccatcatga ggcgcaagtg tcttacttgc ggcttggtcc gcggcaggct 600

gggagtcgta gtattatatt tgggttctgc ctactgagcc gggagtctgg gaactacaac 660

tcccagagtg ctgagcggat gcacgctctg cttttaggtg taagccgcaa acatcttgga 720

ccccgggaga atccgcgttg aagcacagag catttagctc ctctgtcaga atgaatgggg 780

accagaaact ggatgttcat aaccaagaaa agcagaattt catccagcac ttctcccaga 840

ttgtcaaggt gctgactgag gatgaactgg gacacccaga gaagggagat gctattaccc 900

ggatcaaaga ggtcctggag tacaacactg taggaggcaa gtacaatcgg ggtctgacgg 960

tggtacagac cttccaggaa ctggtggaac caaggaaaca ggatgctgag agcctacagc 1020

gggccctgac ggtgggctgg tgtgtagaac tgctccaggc tttcttcctc gtgttagatg 1080

acatcatgga ctcttcctac actcgccggg ggcagatctg ctggtatcag aagccgggca 1140

taggcttgga tgccatcaac gatgctctgc ttctggaagc cgctatctac cgcctgctta 1200

agttctactg cagggagcag ccctactacc tcaacctgct ggagctcttt ctacagagtt 1260

cctatcagac tgagatcggg cagactctcg acctcatcac agcaccccag ggccaagtgg 1320

atcttggtag atacactgaa aagaggtaca aatctatcgt caagtacaag acagctttct 1380

actctttcta cctgcctatc gcggctgcca tgtacatggc tggaattgat ggggagaagg 1440

aacacgctaa tgccctgaag atcctgctgg agatgggcga gttcttccag aaccaggacg 1500

actaccttga tctctttgga gaccccagtg tgaccggaaa ggtcggcact gacatccagg 1560

acaacaaatg cagctggctg gtggttcagt gtctgctacg agccactcct cagcagcgcc 1620

agatcttaga ggagaattat gggcagaagg acccagaaaa agtggcgcgg gtgaaagcac 1680

tgtacgagga gctggatctg cggagtgtgt tcttcaagta cgaggaagac agttacaacc 1740

gcctcaagag tctcatagag cagtgctccg cgcccctgcc cccatccatc ttcctggaac 1800

tagcaaacaa gatctacaag cggagaaagt aacctcgaat tgtagaggct gcgagggagg 1860

ggtctcaata aattattgtt caacaaaaaa aaaaaaaa 1898

<210> 270

<211> 522

<212> DNA

<213> Rattus norvegicus (rat)

<400> 270
ctggagctct ttctacagag ttcctatcag actgagatcg ggcagactct cgacctcatc 60

acagcacccc agggccaagt ggatcttggt agatacactg aaaagaggta caaatctatc 120

gtcaagtaca agacagcttt ctactctttc tacctgccta tcgcggctgc catgtacatg 180

gctggaattg atggggagaa ggaacacgct aatgccctga agatcctgct ggagatgggc 240

gagttcttcc agaaccagga cgactacctt gatctctttg gagaccccag tgtgaccgga 300

aaggtcggca ctgacatcca ggacaacaaa tgcagctggc tggtggttca gtgtctgcta 360

cgagccactc ctcagcagcg ccagatctta gaggagaatt atgggcagaa ggacccagaa 420

aaagtggcgc gggtgaaagc actgtacgag gagctggatc tgcggagtgt gttcttcaag 480

tacgaggaag acagttacaa ccgcctcaag agtctcatag ag 522

<210> 271

<211> 1859

<212>  DNA

<213>  Rattus norvegicus (Norway rat)

<400>  271
ggcacgagcg gtctcggcgc tgtggccatg cctggcggca ccttagcggc tgtcctgagc      60

ccgacacaac cccggtagag gactccccgg gcccggctgg cccggccatg cagctagaga     120

cgcaggacgc gctgtacgtg gcgctggagc tggttatcgc cgcgctggca gtggcgggca     180

acgtgctggt gtgcgctgcg gtgggagcct cgagtgcttt acagaccccc accaactact     240

ttctggtgtc cctggcgacg gcggacgtgg ctgtgggact cttcgccatc ccctttgcca     300

tcaccatcag cctgggcttc tgcacggact ttcacagctg cctcttcctc gcctgcttcg     360

tgctggtgct cacacagagc tccatcttta gcctcttggc ggtggctgtc gaccggtatc     420

tggccattcg cgtcccgctc aggtataaag gtttggtcac tggaacacga gcaagaggga     480

tcatcgctgt cctctgggtc cttgcctttg gcattggact gactcctttc ctgggttgga     540

acagtaaaga ccgtgccacc agcaactgca cagaacctgg ggatggcatc acgaataaga     600

gctgctgccc tgtgaagtgt ctctttgaga acgtagttcc catgagctac atggtttatt     660

tcaacttctt tgggtgtgtc cttcctccac tgctcatcat gatggtgatc tacatcaaaa     720

tcttcatggt ggcctgcaag cagcttcagc acatggaact gatggagcac tccaggacca     780

cgctgcagcg ggagatccac gcggccaagt cactggctat gattgtgggc atctttgctc     840

tgtgttggct ccccgtgcat gccatcaact gcatcaccct cttccatcca gccctggcca     900

aggacaagcc caaatgggtg atgaatgtgg ccatcctcct gtcacacgcc aattcagttg     960

tcaatcccat tgtctatgcc tacaggaacc gggacttccg ctacagtttc cacaggatca    1020

tctccagata cgttctctgc cagacggaca ccaagggtgg gagcgggcag gccgggggac    1080

agtcaacttt cagtctgagc ttgtgaccta ggctctggcc tttgggagaa gaaggcttaa    1140

aataaacaat ggactggaca cagctggtga cctcactgtg gaggacaact accctctcaa    1200

gcatgtggcc cacctgccct gaacgcttgc caggagtcac acaagtctgg ctcacacgta    1260

catgcaacta ggaggccctg aggctaacag atacacttag gaatctattc agctgctctt    1320

acttactgtg tggatggcgg gcggctagaa ctgattccaa aaactgtttt atttttaaga    1380

atctgcctca ttcgtggtag aaaaatgact gaaacttacc ttactgtgaa acactgtgaa    1440

ctatataatg taagtatttt tcacttacag caatgggaaa ataaaagttg gctctattaa    1500

catatactca ttccggggac agcaactcag aaaactgaag tataattctt cagtcaagat    1560

actgtagtgt taatttaggg aatggtacta ccctaagttt gaaagtagaa ttatgtaaca    1620

aaacaatttg agttcagtat cttccttgcc cacaaaacac tagaggtgga aacttggaga    1680

gctgggtcct tcctccactg cacccctgct ttagagctag gtactagagt ttgcttctct    1740

ctcacattac agtttattgg atcccaaagt atgaaaattt tactgagcaa aatcccctag    1800

ctgtgtatta tatccagtga aataaaactt tgaaaggtga aaaaaaaaaa aaaaaaaaa     1859

<210>  272

<211>  2793

<212>  DNA

<213>  Rattus norvegicus (Norway rat)

<400>  272
acagccgaga atgggagtag ggcggagtgt ttgagcagca cacccatttc ctctccgctc      60

ttcgtctcgt tctcgtggcc tgtccaccca tccatcatct gccggccacc gctctgaaca     120

ccttccacca tggccacctc agcaagttcc cacttgaaca aaggcatcaa gcagatgtac     180

atgaacctgc cccagggcga gaagatccaa ctcatgtata tctgggttga tggtaccggg     240

gaagggctac gctgcaagac ccgtactctg gactgtgacc ccaagtgtgt agaagagtta     300

cccgagtgga actttgatgg ttctagtacg tttcagtctg aaggctccaa cagcgacatg     360

tacctccatc ctgtggccat gtttcgagac cccttccgca gagaccccaa caagctggtg     420

ttctgcgaag tattcaagta taaccggaag cccgcagaga ccaacctgag gcacagctgt     480

aagcgtataa tggacatggt gagcagccag cgcccctggt ttggaatgga acaggagtat     540

actctcatgg gaacagacgg ccaccctttc ggctggcctt ctaatggctt ccctggaccc     600

caaggaccct attactgcgg tgtgggagct gacaaggctt atggccgaga tatcgtggag     660

gctcactacc gggcctgctt gtatgctgga atcaagatca cagggacaaa tgccgaggtt     720

atgcctgccc agtgggaatt ccagatagga ccctgcgaag ggatccgcat gggagatcat     780

ctctgggtag cccgttttat cttgcatcgg gtatgcgaag actttggggt gatagcaacc     840

tttgacccca agcccattcc agggaactgg aatggggcag gctgccacac caactttagc     900

accaaggcca tgcgggagga gaatggtctg aggtgcattg aggaggccat tgataaactg     960

agcaagaggc accagtacca catccgtgcc tacgacccca aggggggcct ggacaacgcc    1020

cgccgtctga ctggattcca cgaaacctcc aacatcaacg acttttccgc tggcgttgcc    1080

aaccgcagcg ccagtatccg cattccccgg attgtcggcc aggagaagaa gggttacttt    1140

gaagaccgtc ggccttctgc caattgcgac ccctatgcgg tgacggaagc catcgtccgc    1200

acgtgtctcc tcaacgaaac tggcgacgag cccttccaat acaagaacta agcggactcg    1260

acttccagtg atcttgagcc cttcctagtt caccccactc ccaactgttc cctctcccac    1320

tggtccccac tgtaactcaa aaggatggaa taccaaggtc tttttattcc ttgcgcccag    1380

ttaatttttg cctttattgg tcagaataga ggggtcaggt tcttaatctc tacacaccca    1440

accccttctt tcctagctag ctttccagtg ggggaacggg agggggtggg gaagggtaac    1500

ccaccgcttc atctcagcgg gaatgcatgt cctgtaggca tagctgtcac aaatcgggtg    1560

tacttgtggt gagggaggac tggttttttt tttccttcag gataattgaa agggcaggcc    1620

caacagctta gattaacatt ttctctgtca gtagagagct gttatttctt ccggtgaaac    1680

cagctttcta ttgaagtctg gtgaggagtt ggaggttggt ctcttggctt ccttagctta 1740

gggaagggga gttcaccctc ccttcatgaa acacagttca cctgacaaat ggccctactg 1800

taaaggaaga aaaaagtttc ttggtcctcc atttataact caaagcagag tagtattttt 1860

atatttaaat gttaaaaaca aaaaagttat atatatgggt gtgtggatat atatgtcttt 1920

tctaattgag aaaaccatcc tattccctgg gtgccaagtt tgagtgagga gctcggtgta 1980

gaagtgaggc actcttgagg taggggtggg gatgcagtac tgggaaagtt ggttatcttg 2040

ggggttcagc ttcattacta cttagggttt ccctgcccac tctgcaggag cagatgttgg 2100

acaggtagcc agtgggatgc cactgcttgc cgccactgtc cctgggctta gtttaagggg 2160

acgtgtatac ctaatccaca cacgagttag aagtatgagt tggctggtca acttgaacat 2220

tgttacaggc gggtgggtgt tagtgggggg ttattttttg gtgggactag catgtcacta 2280

aagcgggcct tttgatatat taaatttttt aaagcaaaac aagtttagat tttaatcaag 2340

ttcgtagggt ttctaacttt acagaattgc ctgtttgttt caatgactcc ttccacttgg 2400

ctcttagggg aactgaggac aggcctggag ttaatacact tgtcattctg tgtcctagtg 2460

tcctcttcct ccggcagact gtccccttcc ttctgaaaaa gccgatagag tcttgtttta 2520

tttttctttt ataataaaca caccccacct ccatcccagc ttgttgcctt gcagttttct 2580

ggatgtttgt gtcggcagca ggcagctgtg gtttttttct cttgccacga tgactctaat 2640

taccatgtat agtatgttca gttagataac tcactgtaaa cagactgtaa ctgagagcag 2700

agcttgtaaa tcaacctaac gtttataaga tttcctctga cttgtttctt tgtggttcca 2760

aaaaaaaaaa aaaaaaaaaa aacctcaaaa act 2793

<210> 273

<211> 160

<212> DNA

<213> Rattus norvegicus (rat)

<400> 273
tctaacttta cagaattgcc tgtttgtttc aatgactcct tccacttggc tcttagggga 60

actgaggaca ggcctggagt taatacactt gtcattctgt gtcctagtgt cctcttcctc 120

cggcagactg tccccttcct tctgaaaaag ccgatagagt 160

<210> 274

<211> 507

<212> DNA

<213> Rattus norvegicus (Norway rat)

<400> 274
tttagattta tttgtttact ttgagtgttt tgcctacctg tatgtatgca ccatgtgggt 60

gctgggagtc aaacgcagag ctctgggaaa gcagcaagta ttccagactg ctaagccgtc 120

tctcccaccc ctcataatta attctcaaca ggcagttgcc aaacaaatag aaagaagcta 180

ttaaaactcc tgtttctttt gtagcgtgct cttcaggatc ccaatgttgc tgctttcatg 240

gtggagccca tccagggtga agcgggtgtt atcgttccag atccaggata cctgacggga 300

gttcgagaac tttgcacccg gcaccaggtt gtcccactca ctttgcagtg acgccaggct 360

cgcctgcact tgtttggact ctgctgacag gctagatgat ggattccaag gcttgaggaa 420

catgctaatg cttgcacctt ctgaggaaca cgctaatgct tgcaccctct gaggaacatg 480

ctaaatgctt gcaccttctg aggaaca 507

<210> 275

<211> 2059

<212> DNA

<213> Rattus norvegicus (Norway rat)


<400> 275
ggcaggagaa gtgatctgtt atctcacctg gcacatgaag cccattcctg gaactatgca 60

aagggcagag tctggtgagc catggcggcg gcagtggcgg cagcagcagc agtgcggtcc 120

cggatcctgc aggtttcttc taaagtaaac tccacatggt atccagcatc ttctttctct 180

tcatcatcag tgccaactgt aaagctcttc attgatggga aattcgttga atccaaaagt 240

gacaaatgga ttgacatcca caacccagcc accaatgagg ttgttggtcg ggttcctcag 300

tccaccaaag ctgaaatgga ggcagccgta gccgcctgca aacgcgcttt ccctgcgtgg 360

gcagacacat ctattttaag ccggcagcag gtcctgcttc gttatcagca actcattaaa 420

gaaaacctga aagaaatcgc caggttaatc acattggaac aagggaagac tctggctgat 480

gctgaaggag atgtgttccg aggccttcag gtggttgagc atgcctgcag tgtgacgtcc 540

ctcatgctgg gagagaccat gccatctatt accaaagaca tggaccttta ttcctaccgc 600

ctgcctctgg gagtgtgtgc aggcatcgca ccatttaatt ttcctgccat gatccccctt 660

tggatgtttc ccatggctat ggtctgtgga aatactttcc taatgaaacc atcagagcga 720

gtacctggag caaccatgct tcttgctaag ttgcttcagg attctggtgc ccccgacgga 780

acactgaaca tcatccatgg acaacatgaa gctgtaaatt ttatttgcga tcatccagac 840

atcaaggcaa tcagctttgt gggatccaac caggcgggag agtacatctt cgaaagaggg 900

tcaagaaatg gcaagagggt tcaagccaat atgggagcca agaaccatgg ggtagtcatg 960

ccagatgcca ataaggaaaa taccctcaac cagctggttg gggcagcatt tggagctgct 1020

gggcagcgct gcatggctct ttcaacagcc gtccttgtgg gagaagctaa gaagtggctg 1080

ccagaactgg tagagcgtgc caagaatctc cgcgtcaatg caggagatca gcctggagct 1140

gatcttggtc ccctgatcac cccccaggcc aaagagagag tctgcaatct gattgatagt 1200

ggagcaaaag agggggcttc catccttctg gatgggcgga aaattaaagt taagggctac 1260

gaaaacggta actttgttgg accaaccatc atctccaatg ttaagccaag tatgacttgt 1320

tacaaagagg agatttttgg tccagttctt gtggtcttag agacagaaac cctggatgaa 1380

gccatcaaga ttgttaatga caacccatat ggaaacggga ctgctatctt taccaccaac 1440

ggagccatag ctcggaaata cgctcacatg gtggatgtgg gacaggtggg cgtgaatgtc 1500

cccattccag tgcctttgcc aatgttttcc ttcaccggct ctcgatcttc cttcagggga 1560

gacaccaatt tctatggcaa acagggcatc cagttttaca ctcagttaaa gactatcaca 1620

tctcagtgga aggaagaaga tgctactctt tcctcacctg cagtggtcat gcctaccatg 1680

gggcgttaga acgttcaaga ctccatccag cctgagtaac ttcccactgt ccttggcaat 1740

cttcgtctgc catctttgct caaaacagat ccatgaggat ggagtatatt gcaatgaaat 1800

tctttctcag aactcgcaat cccttcaaag ttatagggag tacaggataa caccaacgcc 1860

aggccttcac ttgttctcca tacttctcta cggtttttga ggttaactgt tgttaagcgt 1920

gaagtgtttg cctagaataa gagcttagaa ctgtcttcta acaattcttt tctgatgact 1980

tggagggaca acgtaggggc ccggtccttg gcaatcttcg tctgccatct ttgctcaaaa 2040

acagtccatg aggatggag 2059

<210> 276

<211> 1405

<212> DNA

<213> Rattus norvegicus (Norway rat)

<400> 276
gaattcccag ggcaagctcc aggctccgca agacccgcgg gcctccagga tgcagacacc 60

gcacaaggag cacctgtata agctgctggt aatcggcgac ctaggtgtag gcaagaccag 120

catcatcaag cgctacgtgc accaaaactt ctcctcccac taccgggcca ccattggtgt 180

ggacttcgcg ctgaaggtgc tccactggga cccagagacg gtggtgcgcc tgcagctctg 240

ggacatcgca ggtcaagaaa gatttggtaa catgacaaga gtctattacc gagaagctat 300

gggagcattt attgtttttg atgtcaccag accagccaca tttgaagcag tggcaaaatg 360

gaaaaatgat ttggactcaa agttaactct ccctaatggt aagccagtat cagtggtttt 420

gttggccaac aaatgtgacc aagggaagga tgtgcttgtg aacaatggac tcaagatgga 480

ccagttctgc aaggagcatg gtttcgtagg atggtttgaa acatcagcca aggaaaatat 540

aaacattgat gaagcgtcaa gatgcctggt caagcacata cttgcaaatg agtgcgactt 600

catagagtct atagaaccgg acattgtgaa gccccacctc acatcaccca aggttgtcag 660

ctgctctggc tgtgccaaat cctagaaggc acctctgctg gcatatgaca gaaagaaccc 720

gtggccctca tgaatcgtgc ttcagttttt ccttatccat tttgggtaag catcaggata 780

gggaagcaca cgtgacaagc caaagataat atgactctat gtttcctgtc aaagaggaac 840

agcaaatgtt ctttatgtgt ttttccccac cgtcagcaca gtgtttacaa gcttttaaaa 900

tattagtctg ttgcaatatg ctgttttatc actgagcaaa gtcactcagg gacacagaca 960

gccttggtat tcattccttt aaatcaacaa aagcttctgg tcttcttgag aaggggacta 1020

agagagcaag gcagaggtca agctaagtgt ggggatttat cttgccctgg tgcgtctttg 1080

ttcaggtatc aatttgttcc tgggtggtct gataggtcta ttaaatagga acactatttt 1140

ttccattcat ggcagaccta agggttgcct gtgatgtttc tcttcagagt cgtgtgcaca 1200

ggcagcctgg gcttttgttg ttacttgctg tgccctgaat gctggtttaa ctggaaactg 1260

tatggaaaga tctgctccct gtatgtgcct ttctttcagc tttctctgac tcaaactgcg 1320

ggactgctct gtacatgtaa gatatattat atatattttt cacaagtgaa aaataaaaca 1380

ttaaaagtgt tctttcccgg aattc 1405

<210> 277

<211> 707

<212> DNA

<213> Rattus norvegicus (Norway rat)

<400> 277
ctttggtgtc tgagctcagc aagaggcgct cgggggacgc tgagggacag ctttaacacc 60

cgcacaacac agggtacctg gttcccagtc cagagctgct tggagggcag agatgtcgcg 120

agcagctaat cgccgcccag ggctctccgc aggacaattg gccgctgcaa ctgcatctcc 180

tctcctgtca ctgctgctgc tgctcgcctg ctgtgcggac gcttgcagag gtacgccaat 240

atcgcctcaa agattaccac cagaacaaga actacaattg tggaatgaga ttcctgaagc 300

ttgtgcgtcc tttctgtctg ttgattccca gcctcaggca tctgttgcat tgaggaagct 360

ttgccgtgtc ctgatggaga ttttccagaa gcctcaggag caaactgaga aagataatgc 420

taagaggttc ttatttcatt attcaaagac acagaagttg ggaaattcaa atgttgtgtc 480

gtctgtcgtg catccgttgc tgcagctcgt tcctcaactg catgagagaa gaatgaagag 540

atacaaagtg aatgaatacc agggtcctgt agctccaagt gggggattct ttttatttag 600

gccacgcaat gggaagaggt caacaagttt catttaaaat ggatgccaaa tgatcttttc 660

attgaaaact ctgctatggg atataaaatg tactcacatt tgttctc 707

<210> 278

<211> 1662

<212> DNA

<213>  Rattus norvegicus (Norway rat)

<400>  278
ggccccgctc ctcgccctgc ctaccgccgg catctaaaca caggtgggag tgggagatcc      60

cgacaggtga gccccgcgcc ccgcagccac aaggatggag ttcgtgaagt gtctaggcca     120

cccggaggag ttctacaacc tgctgcgatt ccgcatggga ggccggcgga atttcatacc     180

caagatggac cggaactcgc tcagcaacag cttgaagact tgctataagt atcttgatca     240

gaccagtcgc agcttcgccg cggttatcca ggcgctggat ggggacatac gtcatgcggt     300

gtgtgtgttt tacctgatcc tccgagccat ggacacagtg gaggatgaca tggccatcag     360

tgtggagaag aagatcccac tgctgcgaaa ctttcacact ttcctctatg agccggagtg     420

gcggttcacc gagagcaagg agaagcaccg agtagtgctg gaggacttcc ccacgatctc     480

cctggagttt agaaatttgg ctgagaaata tcaaacagtg atcgctgaca tctgtcacag     540

gatgggatgt gggatggcag aatttctaaa caaggatgta acctccaaac aggactggga     600

caagtactgt cactatgttg ctggactggt gggaatcggc ctttctcgcc tattctctgc     660

ctcagagttt gaagatccca tagttggtga agacacagag tgtgccaatt ctatgggtct     720

gtttctgcag aaaacaaata tcattcgtga ttatctggaa gaccaacaag aaggaagaca     780

gttttggcct caagaggtat ggggcaaata tgttaagaag ctggaagact ttgttaagcc     840

agagaacgta gatgtggccg tgaagtgctt gaatgaactc ataaccaacg ccctacaaca     900

catccctgac gtcatcacct acctgtcaag gctccggaac caaagtgtgt ttaacttctg     960

tgccattcca caggtaatgg ccattgctac gctggctgcc tgttacaata accatcaggt    1020

attcaaggga gtagtgaaga ttcggaaggg gcaagcagtt accctcatga tggatgccac    1080

caacatgcca gctgtcaaag ctatcatata ccagtacata gaagagattt atcaccgggt    1140

ccccaactca gacccgtcag ctagcaaggc caagcagctc atctccaaca tcaggacgca    1200

gagccttccc aattgccagc tcatctcccg aagccactac tcccccattt acctgtcctt    1260

catcatgctc ttggctgccc tgagctggca gtacttgagc actctgtccc aggtcacaga    1320

agactatgtc cagagagaac actgactttg tttagctgga agcggaagtc cacgtgaagt    1380

gggtttttct tcttccccca gctggatttt gacttccctt ggtttttcct tctactctaa    1440

tctttcggag aactgagtgt gggaccttta ggaactctga agaggaaagg acgccttgcc    1500

ctcagcagcc tggtgcttcc tggatgtggt ccctgcctct tgtagccact ggcatcatgt    1560

tgaccgaagc actggaaagg ccacatgtga tcctagtgaa cctggctaga atgctgattg    1620

aatctattta atttgaaaca gcctttgaat acctatcaca gt                        1662

<210>  279

<211>  311

<212>  DNA

<213>  Rattus norvegicus (rat)

<400>  279
ccccatttac ctgtccttca tcatgctctt ggctgccctg agctggcagt acttgagcac      60

tctgtcccag gtcacagaag actatgtcca gagagaacac tgactttgtt tagctggaag     120

cggaagtcca cgtgaagtgg gtttttcttc ttcccccagc tggattttga cttcccttgg     180

tttttccttc tactctaatc tttcggagaa ctgagtgtgg gacctttagg aactctgaag     240

aggaaaggac gccttgccct cagcagcctg gtgcttcctg gatgtggtcc ctgcctcttg     300

tagccactgg c                                                          311

<210>  280

<211>  1875

<212>  DNA

<213>  Rattus norvegicus (Norway rat)

<400>  280
gaattcgcgg ccgccgccgg gccccagatc ccagtgcgcg aggagcccag tcctagaccc      60

agcaacctga ggagaggtgc acacaccccc aaggacccag gcacccaacc tctgccagat     120

gtgggggggt ggctacccag aggcatgctc ctcacccagc tccactgtcc ctacctgctg     180

ctgctgctgg tggtgctgtc atgtctgcca aaggcaccct ctgcccaggt aatggacttt     240

ttgtttgaga agtggaagct ctatagtgac cagtgccacc acaacctaag cctgctgccc     300

ccacctactg agctggtctg caacagaact ttcgacaagt actcctgctg gcctgacacc     360

cctcccaaca ccactgccaa catttcctgc ccctggtacc taccttggta ccacaaagtg     420

cagcaccgcc tagtgttcaa gaggtgtggg cctgatgggc agtgggttcg agggccacgg     480

gggcagtcat ggcgcgacgc ctcccaatgt cagatggatg atgacgagat cgaggtccag     540

aagggggtag ccaagatgta tagcagctac caggtgatgt acactgtggg ctacagtctg     600

tccctggggg ccttgctcct ggcgctggtc atcctgctgg gcctcaggaa gctgcactgc     660

acccggaact acatccacgg gaacctgttc gcgtccttcg tgctcaaggc tggctctgtg     720

ctggtcattg attggctgct caagacacgc tatagccaga agattggaga tgacctcagt     780

gtgagcgtct ggctcagtga tggggcggtg gctggctgca gagtggccac agtgatcatg     840

cagtacggca tcatagccaa ctactgctgg ttgctggtgg agggtgtgta cctgtacagc     900

ctgctgagca tcaccacctt ctcggagaag agcttcttct ccctctatct gtgcatcggc     960

tggggatctc ccctgctgtt tgtcatcccc tgggtggtgg tcaagtgtct gtttgagaat    1020

gtccagtgct ggaccagcaa tgacaatatg ggattctggt ggatcctgcg tatccctgta    1080

ctcctggcca tactgatcaa ttttttcatc tttgtccgca tcattcatct tcttgtggcc    1140

aagctgcgtg cccatcagat gcactatgct gattacaagt tccggctagc caggtccacg    1200

ctgaccctca ttcctctgct gggagtccac gaagtggtct ttgcctttgt gactgatgag 1260

catgcccagg gcaccctgcg ctccaccaag ctcttttttg acctgttctt cagctccttt 1320

cagggtctgc tggtggctgt tctctactgt ttcctcaaca aggaggtgca ggcagagcta 1380

ctgcggcgtt ggaggcgatg gcaagaaggc aaagctcttc aggaggaaag gatggccagc 1440

agccatggca gccacatggc cccagcaggg acttgtcatg gtgatccctg tgagaaactt 1500

cagcttatga gtgcaggcag cagcagtggg actggctgtg agccctctgc gaagacctca 1560

ttggccagta gtctcccaag gctggctgac agccccacct gaatctccac tggactccag 1620

ccaagttgga ttcagaaagg gcctcacaag acaacccaga aacagatgcc tggccaaggc 1680

tgaagaggca aagcagcaag acagcagctt gtactatcca cactccccta acctgtcctg 1740

gccgggtaca ggccacattg atggagtagg ggctggatat gatggagtag ccatgctatg 1800

aactatgggt gttcccatga gtgttgccat gttccatgca cacagatatg accttcagta 1860

aagagctccc gtagg 1875

<210> 281

<211> 373

<212> DNA

<213> Rattus sp.

<220>

<221> misc_feature

<222> (100)..(100)

<223> n

<220>

<221> misc_feature

<222> (115)..(115)

<223> n

<220>

<221> misc_feature

<222> (150)..(150)

<223> n

<220>

<221>  misc_feature

<222>  (357)..(357)

<223>  n


<400>  281
cagttgataa tgttttacta tgacttagaa ctttttcccc caagaacaaa attatttgca      60

taaaaattca ggtatctgaa agactcaaaa ggctattttn cactttgttc aagtnctgct     120

tccaggcatt aagagtgtcg tacagttgtn gccactgctg ttttccaaat gtccgatgtg     180

tgctgtgact gacaactact tttctttgtg tctgatcgat tttgcagtag accattttag     240

ttcttacagc atcaataaca aatgcttcaa catcatcagc tccaatctgt agctcttgct     300

gcattgtatc aaaagaaatc tctttgtttt ccactgccat tcccataaag gtgaggngtc     360

tcattttcgc cat                                                        373

<210>  282

<211>  458

<212>  DNA

<213>  Rattus sp.


<400>  282
ttttgtgacg gcaaggtctc actgtgtgac gctggctggc atggaacttg ctgtgtgacc      60

aggctggcct ctgcctccta agtgagtggt atatatttga atcccagctt taaggagact     120

gaggccaaag aatcagaaag tgtctgacga tttcagacag aatgaacttt ggtcaatgtg     180

ggctacaaga gactctgaag acaaacaaac gcttctcatg ttttgtaatg tgttagccaa     240

tgctacctga gcactctggc aacaagaggg ccatacccag atgccctttg accttggacc     300

agaaccagga gtcaaaataa gccttttcct tgtctctcac ccaggccaga gagttgtggg     360

tttgccacag aaaagggact aatttctttg tatgtgtgtg tgtggcacgc atgtgtgtta     420

ggtatattca catgtgtgtt ccttattatg tacaccag                             458

<210>  283

<211>  681

<212>  DNA

<213>  Rattus sp.


<400>  283
   gggatcagag tttctttatg ggctataggg gccacaaggc tggccaggtg gcaaggtcac      60
   cggtggcccg gtcaccgata caggtagtca gcctggatgt tggccgcgat ctcggcctcc     120
   cacttgtcac cattgttgag tagcttctcc ttgttgtaca gcaactcctc atgcgtctcg     180
   gtggaaaact caaagttggg gccctccacg atagcgtcaa cagggcaggc ttcctggcag 240
   aaaccacagt acatacactt ggtcatgtca atgtcatagc gtgtagtccg gcggctgcca 300
   tctgctcttg gctcagcctc aatggtgatg gcctgtgcag gacagatggc ctcacagagc 360
   ttgcaggcga tgcaacgctc ctccccagta cggtatcggc gcagtgcatg ctccccacgg 420
   aagcgcggac tcagtgggcc cttctcaaag gggtacttga tggtggccgg ctcccgaaat 480
   gagtacctac ggtcatgccc agtctcggaa gagtccgtca catcagaacc gaactgcatg 540
   tcatcccaga ttctagtcaa aaaattcggt gccttcaatc aatactgttc gtacggccat 600
   gaagctagag gtttgcaata aaatccgtcc aggccggcaa ggcgggaaaa tcaaccaggg 660
   gaatctaata acccccgcaa t 681

<210> 284

<211> 673

<212> DNA

<213> Rattus sp.

<400> 284
ccatcacaat aaaagtgaca cagctttaca aaaataaatt tttattaaaa gcagtaaagt 60

ctgagcagga agacagtaca aagaatgtaa acaatgtaaa caccactaca ttcagctcag 120

cctgatcgga tgctgaaaga caagtctaaa tgctctagtc cctttgctag caagatacat 180

taattctatt ttacacagac aacatattct aatttttact agggaaaaat agtttatcga 240

agtcgatccc aacgccaaat gctggcatca tcgcagacag ctatgaggat gctgctatcc 300

ctgctaaaac tggtttgtcg aatagcagcg ccacatttat gatgggtcag tgttgtgcat 360

ttggctttat gaggatcttc tactttcaaa tcccaaacat acagtttgac aacctgactg 420

cccaatgcaa gcattaggtt tactttaacc atatcatcct caatttggca ggtttcaaca 480

aaaaaggcat tttcacaaaa ctgggaaata caaaacgcct aacatccttc aaatcaacat 540

aatccaagta gtccgggtaa aaaatcggga atggatttcg ggaataaagg ccggaagttt 600

gttgggtaaa accaaaatcc taaccatcaa aatccttggt ggtctcaaat tagagggtca 660

acccgggaat att 673

<210> 285

<211> 709

<212> DNA

<213> Rattus sp.

<400> 285
gacttagtgc atcagagtac aggtgtccta caatggctgc ctgagcagca tcttagagca 60

gcattacagg gatccttccc gaccgcacca tagttataaa ccagggacat gacgacgtac 120

acaacccaca cacggcagca gtggggattt acacagtgta acataacatt gggggttagc 180

agggacagct gcatgcaggg agggtgggtg ctgtggggag ccaggtcgag aggtggctgt 240

gttggcttcc gtccgtgaca gaggcagctc tcactgagat tctcagacac gagtgagtga 300

gtgaccgggg atggtctgag tgtcacagtg ggctccatgc aggtagcagg aagcagaggc 360

ttgctttagt tggaagtggc tgtatgtctg tcacaagcat gggtctcttc ctggtgctgt 420

tgccctcaga gctgacatgt atggtactgg catgaaatgg ggaaacccaa cagctgtcag 480

aacaccctta caaagacgtg gtgtgcactg acaagtagtg tgaacacgta aaggcgatgc 540

actggcctta cctccacgga aactgacgga aggcacatgc aactgagcag ctggacaaaa 600

agtcaacagg gaaaaaactc atttctagaa ctggcaacac ccgacaggtt gatgcccgac 660

agggactgga tgtcaaaaaa gggactccaa cgcaggaagg cgggaagca 709

<210> 286

<211> 570

<212> DNA

<213> Rattus sp.

<220>

<221> misc_feature

<222> (499)..(499)

<223> n

<220>

<221> misc_feature

<222> (536)..(536)

<223> n

<400> 286
aaggcggcag ctgtttattt tgaggtaact gtcacacagt actgttatat ggtagaatag 60

tcattatgta atcttgagag aggttgtcta aggtaggatt tggagccttc cacacttatc 120

agatgccttc tcattagttt cttctagttt tgcaattcta gatccaaatt gtatggcccg 180

tttgggcaga agggcagagg atgagagacc aagttccaca gctgcaaggc gtaaaatgag 240

cttctcacca actccacggg gcaaagccag gtctaccttt tcccaaactg gcagagaatt 300

caggaaagat acaacatttt catccagaaa aggaaatctt gcttcctttc catgatcagc 360

aataactcta tcatcacgac caaggtttct agaagaaatg cgacccaatt ccattgctat 420

ttcctcattc aatccttcta ggccaagaga ctgaaagcgg gcacgatgac gggaataacc 480

tgccaactgc tcatctgcna caatcccagt gagaatcacc tttgcactgc tcttgntaga 540

ctgcacagca tcctcggttc acaacaaaac 570

<210> 287

<211> 418

<212> DNA

<213> Rattus norvegicus (rat)

<400> 287
tggcaggtta ttcccgtcat cgtgcccgct ttcagtctct tggcctagaa ggattgaatg 60

aggaaatagc aatggaattg ggtcgcattt cttctagaaa ccttggtcgt gatgatagag 120

ttattgctga tcatggaaag gaagcaagat ttccttttct ggatgaaaat gttgtatctt 180

tcctgaattc tctgccagtt tgggaaaagg tagacctggc tttgccccgt ggagttggtg 240

agaagctcat tttacgcctt gcagctgtgg aacttggtct ctcatcctct gcccttctgc 300

ccaaacgggc catacaattt ggatctagaa ttgcaaaact agaagaaact aatgagaagg 360

catctgataa gtgtggaagg ctccaaatcc taccttagac aacctctctc aagattac 418

<210> 288

<211> 604

<212> DNA

<213> Rattus sp.

<400> 288
   cacagcagaa gttgtgtgag acaggaggtc acaccctaca cacaagagta tggtcagagt 60
   ctgaggtagc ccttcccacc ctgatgccaa accccaagca gtcggaccta agttctttcc 120
   cccagtccca ctttaggtgc acactgacag ctattaaagt tagtgcggcc aaaggacccg 180
   ggcccctccc taatgcccct gcttcaatgt gtttaccatt gttcttcact ggccaccatc 240
   tcccgttctg actttctttt tacatgctgg atatgtctat cacgttaagg atcagtaaca 300
   caccagcaaa tattcccctg agagacatcc atttaggagc attgccttca gaggccttaa 360
   acgtcaaggc actgtgtcag ctttggggga atggagctcc tcatatccca ccaccaaccc 420
   tacacataca cacactctcc tacccttgca aatatgggct aaagaggggg agtgatggca 480
   tccccgtgac agctaaaaca acttattgtt cctcacctat agaaacaagt cagagaggga 540
   acataaaagc cttcccagga caaaacggga gaggagatac ttagggggct ggatcctaag 600
   aata 604

<210> 289

<211> 616

<212> DNA

<213> Rattus sp.

<400> 289
aaccttatga agcagtttaa ttaggttgtt aacaattaga acaccagttt gtgagggtac 60

atgccgttcg tcagggagaa ctgagaccgc aggtagccct ggagctgggg gacagctttg 120

atctttggca aaatctgcga gtccacagct ttctgatcag cctttcgctg ctctgtaatt 180

tcgtatttct ccttctctgt gtcgaagatc tcaccctcct gatgcctggg cttgcgaagt 240

ggcttcttct tgaagtaagc atcagtcagg tgtttgggaa ttttaacctt gctgatatca 300

acttttgtag aggtggcgat gacaaacttc tggtgtgtcc tacgcagagg aactctgttg 360

agggcaagag gtccagtcac aagtagcaag gaccttttct ttcttcttct tcttctcaac 420

ctttgtcttg gcagcagagt atttcctttt gtacaaggcc tttctggaat acatagcaga 480

tcgtgaatac ctgccgattc ctctcaccag gacagggttc cggctgcaat ggggcttact 540

cttcctcagc tttttagcct tagaactact ctttttgacc gcaccagcgg gccgggcccg 600

gggggcagta gcatca 616

<210> 290

<211> 581

<212> DNA

<213> Rattus sp.

<400> 290
aacataaata aaggctttat tgttcagttc ggatgaatga cagactggca taagctcgat 60

taattgttcc acacatttaa gaccccagga tgacagcaaa agctatgctt attggcaatg 120

ggcaacaggg agggaggggg tgggaaggac ctgaaggcct ctgaagttca agaaagctat 180

cgggaccgaa accaacgtcc tagaagcagg tccaggatgg aaaagggatc atccaaagct 240

gaagatcttg gaggatctgg atcacttctg gaactgtcat gggcttcttg atgggtcact 300

gtggtctctc tccggccctc actgtccacc acagtgcggt gttcctccac tgtcccatct 360

ggcttagtga tcttggtcac agagatgctt ttgaaatagg atttaggctg gggttgaaga 420

agtggaccca gaccttcctg ggaaacctgg gagtcaagat ccttgtcctc tctggctcta 480

gaatggggac tcacaggcca tgtatcatcc aacctatgaa aaggtccctg ggacccccaa 540

tctggagctg gtttggagga ttcaggtttt gcatgactct c 581

<210> 291

<211>  586

<212>  DNA

<213>  Rattus sp.


<400>  291
cagcttctca gaaagctatt ttatttggat ttcacacaca ccaaaagcag agaggagact     60

gtggggctgg ccctctttgg gctggagtct ctggctcccc tgggcagtcg gttcccagcc    120

tcccaggctt gtcatcctct gaaggctgag tggggtgtct gccctgcacc acagctcttc    180

tccaaagccg aggaaaaccc atggggaata cagggtaaga ggacctaagg atcatgggat    240

gggagcccac attgaacctc ggtgaggtag tctgtcgcct gaggcccaca cgggtcctgc    300

tgaggtaaaa tttgtaagtt tatttcaggg acgtgggtca ggactcctcc ttggtcccca    360

ggacctgctt ccggatgatg tccgtgtact cgcggtcctt ccccttgctc tccttccatt    420

ttctgtacat caccgaggcg tccacgttgg caggcgaaaa ggtgttgggc tcattcagca    480

gggagatcac actcaggagg atggtcctga cattctgcgt ggggttccac cgctctgagg    540

gtagctcccc actctgtggg tcatcaactg ggggatggag gatgga                   586

<210>  292

<211>  475

<212>  DNA

<213>  Rattus sp.


<400>  292
agtaaagaca attttgttca tacaaaacaa cagtttacaa cagtctgagt gttgaacaca     60

ccatcaagga catgggagga gctgcagaca tctgggccct gccccaggcc cagagatgcc    120

caggagcccc tcctccacca acccctgagg gagacacccc tcctccagca cgaggggcat    180

gggggaccat ggcacaaaga ctcgtcccca actgctccgc ctcaatcctg ggagcccccc     240

acccaggttg agtagttaga gggtaaggtg gctggtgggc agggacagag tcaagacatc    300

tttggtgcga agactgtccc acccaccaga ccccgtgctg gaaatcgaga agaccacacg    360

tggtcctgcc cttgccaaac ggaggttggc aagtacccca tagttcaggg gagatgcatg    420

aaatgcaact gcctttctgg gtagataacc ttcccagggt cacccatggg actgg         475

<210>  293

<211>  523

<212>  DNA

<213>  Rattus sp.

<400> 293
aaaaagttta ttgtaaaaaa agtaagccat tcacaaaatt gaatgatttt tagcagtaca 60

gctaaaaccc aactgcatgg cagtgagaag tagtagagtg gcccagaata aaggctcttc 120

tcttaaggat gttcctttta gccaataact tctgtagcat gttttttaaa caaattttat 180

gacagcagtt aagcctcata gcagattcct tcctaaaaaa gcaaacaaca tttagaacta 240

gccttttgtg gggccaattc ccatcattaa actagtccca ccccctccca ttaacaatcc 300

acccaccctc ccaccatttt ggggaaatgt atctaatatt actttgagcc attgctttaa 360

gaaaatgggt caaaggaagg ttaaataaaa ttaaagggcc aaaacatatt gtcacaatag 420

aaatgtattc attcccttca agtaggactt aataggcatt aattaaataa aaacaattca 480

tctccagaaa aaccaagtgc agcttaactg gactagtttt ggc 523

<210> 294

<211> 774

<212> DNA

<213> Rattus sp.

<220>

<221> misc_feature

<222> (497)..(497)

<223> n

<220>

<221> misc_feature

<222> (499)..(499)

<223> n

<220>

<221> misc_feature

<222> (546)..(546)

<223> n

<220>

<221> misc_feature

<222> (550)..(550)

<223> n

<220>

<221>  misc_feature

<222>  (643)..(643)

<223>  n


<220>

<221>  misc_feature

<222>  (681)..(681)

<223>  n


<400>  294
gctcaataaa cattttatta gtaatgtgaa taaaagcagg agaagaaatt tgtaaggatt      60

tattgtgtgt gtgtgtgtgt gtgtgtgtgt gtgtgtgtgt gtgtgatttg ctcataccac     120

atgtctgtgc ttgttgcctg aggaggtcag aaaagggcaa tgactaccct ggaactggag     180

ttatggacag tcataagcca ccatgtgggt ggtgggaatt gaatctgggt cctctgtaag     240

aacaagtgct tgtaactact gaatcatcca gcccccaggg gaggaaaatt taaaagatgt     300

gggagaacga gtttaaatat ttcacccctg atgctcccaa tgtatagttt cagagtttga     360

gaagcacaca cattacgaag acaattgttt aggaactttt actgagggct gcttagctat     420

gttacgctgt gctgcatgca ctagctttcc ttctactaca gtccatgcgt gaagactttt     480

gttgataagg tcaatanana agatattaaa aagacagctt tccttttggt gtttctaatc     540

attacnacan accaaagtga aaccattctg agaggagcaa catcctcagt gtcctgtctc     600

ctgtggggct tcctcagttc tccttcggaa tcccaggtgg tanccactga agagtcatgc     660

agggcgctgg ctcctcctct nctcctcttc gcctctgtcc ccccaacact caattcttcg     720

ttagatgcct tccgttgaag ggcatttgga tttctgttaa aaaagcttgg gttt           774

<210>  295

<211>  669

<212>  DNA

<213>  Rattus sp.


<220>

<221>  misc_feature

<222>  (367)..(367)

<223>  n

<220>

<221>  misc_feature

<222>  (493)..(493)

<223>  n


<220>

<221>  misc_feature

<222>  (584)..(584)

<223>  n


<220>

<221>  misc_feature

<222>  (587)..(587)

<223>  n


<220>

<221>  misc_feature

<222>  (588)..(588)

<223>  n


<400>  295
gactgcttca tctcccgctt ttattgacta gtcacagtcc acctgatatg ggcagttttg      60

cttccctaat agtttagctt gaaaggtaat taccagaact ttgctaggtc caacatatat     120

gccccaccct gttcctctcc aaccgtttcc agggtccctg cagtatggca tgtgactaca     180

tgtatgttct gggattggaa catattccag ctttccacaa tgcttgcaat cactgccaca     240

cacacataca gtagaatttg tttgcctctg tgtctatctc ctgcattgac agtaatatac     300

aacgttacta attaagacaa gctaggtcaa aggtcctcaa ggactacata aggagattat     360

caaacanaac ataaactcca agaggactgg gggtacagtg cttgcctact atgtacaagc     420

ccctggattt actgcctagc acaagtaccc aacagtaaca gaccacgtca cagggacatt     480

tacttcaggt ggnaaatgca ggcaataaga cccatcgttc agtcatcctg tgctacacag     540

tgagtttgag gctggtctgg gctgttgaga caacttctca agancanngc aaacagaccc     600

aagaattcaa ttaaagaagc agacaatcct cgggagttta agggaagatg gtcttcatac     660

catttctgg                                                             669

<210> 296

<211> 599

<212> DNA

<213> Rattus sp.

<400> 296
aaggtgtcat gaacttcctg gtagtacctt agttaggttt ccatctctga ccaccatgga 60

caaggcaact cttagacaac acttaaatgg ggctggttta caggttcaga gtttcagtcc 120

attatcatca agatgggaaa catgggcagt actggcactg ctgagagttc tacatcttgt 180

tccaaaggaa accagaagac tgtcttccag gcagctagga gaaggtctca aagctcactt 240

ccacagtgtc gcacttcttc caacaagtcc acactactaa tagtgccatt ctctgggcca 300

agcatattca aacacatgag tcgatggggg ccaaacctct tcaaaccact acaagtagaa 360

ttctcatgaa atatgacttc atgattgcta gactctaata caggattttt catcttgtct 420

tttactattc tcagtataat caaacactga aatatttact tatgtgacta tataagtcac 480

acacaaaaat gtaaactaac attaattagg aaaattttca agataaatta cttagaaata 540

atttttataa tcccaacact taggaggcaa aaagcaagta agtgtaactt ttttccccc 599

<210> 297

<211> 562

<212> DNA

<213> Rattus sp.

<400> 297
caggattata aaaggctaca actgtttaat gggaataacc tgggacaagc agggggagct 60

ttgatgggca gggttgggca gcacaggact aagaacccaa agcccagata gcccacagct 120

ctctctccac agccccgccc tgaaggatgc cggctgcacc cgttccaagg cacacattac 180

agacttgcag cttcccatgg gctaacccac tatgcctcta catttaaaaa tacgctttca 240

tcttctctgt agtggagtct ccagtcgtgg tccttgtcta gcacaggtgt agaactgctt 300

tgttgatctc tgggtttgtc cagtcgctcc gaatgtcatc caggtgatta tcaaaatcta 360

caagtgtctc ataggagcgg ctgtccagga gcgaagcgga gatcctctga gcctccggcc 420

aatcttcaca gtagtcgtga tgtgggtctc ggcaccgcca cctgttctcg tgttgctcat 480

acacgtggat cgtgggcgct gcacagtcca tcgtgaactt ggcgttgtcc accatgatga 540

gtgcagcatc actgaagccc tc 562

<210> 298

<211> 596

<212>  DNA

<213>  Rattus sp.


<400>  298
aaagcctctt ccttccacac tctgccctgg tgaagccagc cgcccgcacg agcccgtcct      60

ccagagtggg ccaggacaag gtcatgggga agggcataag gccaaggcct caaagggcca     120

gtgggaaggt gaggacttag gcatagtggg tgcccggtgt tagagaatgg ccccctctgt     180

ggtccagctg gtcctgcaag cgggcaaact cggccagctc gttgagctcc tggaactgtc     240

ggtccgtctt atggctgacc agtgagcggt agaagtgaac ggcaaagacg ataaacacca     300

ggccacaggg gaccatgatg gcagtggagg cgatggcagc cgcctcaccc ggggtgatgc     360

caccgttgtt gctgctgttg gcgacgacga cggccggttc ggtgggtggc ttggtggggc     420

ttggctgtcc cgcctgcctc ttgagcggta agaacttcac ccagcagagc agcacgactt     480

cggccaggaa aagcagcgtc ccgatgacgg tggagaagcc caagccagct cgatgtggcg     540

gtgcatgcgc tcgtgcggcg actccttgac ggagttgagg ttgtggacgt tgctca         596

<210>  299

<211>  259

<212>  DNA

<213>  Rattus sp.


<400>  299
gaaaaaaaaa tacccaaacc aattgcaagc ctggtaatga gtggggccaa tcttggactt      60

gatggaaact gttgggaaaa catgcccgtc attgggagag agctgaatgg aggtcaatta     120

tagaatggat aaatagccac ggaagccaat ttagacatta ttccaatgga tgttctgggt     180

acagtattac ctttggtgtc ttctcacctg aatgtgggct aagataagtg ttctctgaaa     240

catacagccc tgcctcgtg                                                  259

<210>  300

<211>  581

<212>  DNA

<213>  Rattus sp.


<400>  300
cctataaaaa caatttattt ctgggggcag ggagggccag aactttgcaa ccagcgccca      60

ccccaccctc cagctcctcc tttacaaaag aaatttttac agtcaccagt cctgtacaga     120

tgctccccac ccgccttagt gtacacagct gcctgggtcc ccctggtccc tgtgttcaga     180

gcggacctga gccaacaagc cagctatcct tggagtcggc ctagacctga gctcgctttg     240

gtactggcca ctagttggcc cctcctaggc ctcttcttct tttgggtctg tcctttggcc    300

tgtctccctt tgcatgactc ttcgaagctc gaatgggata cgacaggatg acagccgtgt    360

tcagctggct ttcgcggtgt tgcagtgaga gtgtgtgtgt caccactttc ctctcttgct    420

ccagtctttg ggagtgaagt gcagacactt ggagtcaatt cgaaggagcc gcttgagcat    480

agcccgctca tgccatccac gatgtcttct gacagagtga ggatatactg gcccttgtag    540

taattgatga gattgagata ctgcaatgtg gaaatgacat c                        581

<210>  301

<211>  610

<212>  DNA

<213>  Rattus sp.

<400>  301
tattagagga aatatctaat ggctgtctta tacaaatatc agtttcccag gggcagaaca     60

agatttatct gtgttcgaag ttccaggata gatagcaaga ggcactgtgc tcaaaagtat    120

ttgtagtatg aaagggccat cataaataca aaactgttat cttcggtttc tactcacagt    180

tgacttaaca attctccgtc ccgatgaaag gaaaacagtg tatgaagaat ccccaagtag    240

attccaaccg aagccacctg gtatttttgg agctggtgct caatgcctca gcttatgcag    300

cacactcagg gtatggcaga ggcagttaag aaaatgagtc aaatttagca tctcagtact    360

acagtgcgct ttgcagacct tcggactatt tttcctagcc aaagtacagg ggaattcaga    420

caagagccac cgctgcagac cactatccca ttagtgcaaa ctctggttca gatactgaag    480

aaacatgttg gccaattgag gcaggttctc attgttggga tgcattttag tgtaggaaat    540

aaactggcga cggaggcgac tcaattctgc caaggtcaag ggacgggtaa atcggaggtg    600

ctccgtggtg                                                           610

<210>  302

<211>  547

<212>  DNA

<213>  Rattus sp.

<400>  302
tcgggataca caggcacagg cacctgcggg cgcttcagtc actcggggga ctggcgcgcc     60

ccacagaacc aaggatgggc ttttatagga ttttggggag cagaagcaag catacagaag    120

cagatgcatg gttacaggga tataattggt ggattctaat atggcaaggg tttagcccgg    180

gggcaagttt cctagctacc tttctgaaac ataacggctg actcggcccc ccaagggttc    240

agcccggggg caagtttctt agctaccttc ttggaacata acgactgact cggcccccca    300

ccagggtgtg ggacctctcc cggggctttt cttcattgtc aggtgctcaa ccgaagtcgt 360

cctgttgcca ggccttcaac caaacatatc ttgcttggca gccgctgtgt actcagtgtt 420

ctaacctttc cctgaaccag tcatcttaag tacagccttg caaaatggcg ttactgctgc 480

taagctgggg tctttcattc cccatttttt tttgttaact ttgagtgaaa tctttcactc 540

gacttgg 547

<210> 303

<211> 475

<212> DNA

<213> Rattus sp.

<400> 303
agacaacagt atgtagtagt tttgaatcca ctgagatgtt gctttcaatt tgaaatattg 60

tgtatacatg tatataaaaa ataatccaat gtatgactca tctgacagat gtttaagatc 120

aataaagttt ttgaacatgc atttaggaag agaggggaga agcccacccc gctaccctgt 180

tgttgctggc ttatttctgc agtggtctca atagtggttt ttagagggaa ccatgtgcct 240

tcagtcttca gagttacgat aagataccat aatcaagacg ggaggggaaa tggggaatgg 300

gcaggtatta atcttaaata cattttgcac tctatctggt acaagataaa aacaggttaa 360

tggggatagg tattaattag gaataaattg cagtcagatc aagacataat tgccgaattg 420

agtttttaat agactgcata tatagatgtt tagccatact ctagatcaac tcttt 475

<210> 304

<211> 359

<212> DNA

<213> Rattus sp.

<400> 304
acacccagaa cataattatc atatattaat agcaatataa cagaataaag gcttgtgggg 60

acagccagtc tttcagacat ggatggaagg ttggcgttca ttgttggtga ggttggttga 120

aggctgtgcc ttcagcttct ggttaaactg cagtgagtaa gcccagggtt agttgctgag 180

aatcatgttg caagcagaac catcgcacat gctgaaactg gcccacgagg ttgtgtggag 240

gctcctcctt aatacgatct gtggaaatga gcccggtggc ttcggaaaga acgctgccag 300

taacgaaggc tccaggaagg cttcggtctc aggagctctg ccatgctgac cctcgtgcc 359

<210> 305

<211> 423

<212>  DNA

<213>  Rattus sp.

<400>  305
aaaaaggcct tggcggcagg aatatagtgt aaaaatcatt ggaaaaacta aaaggcatcg     60

atacatatcc gaatatactt tgtacataaa ttacattttc ctttagtctt ttgagttgag    120

gtcctgattc ggctctgagg tctaataatc aagggggcct ggaagctgca cgtttagtcc    180

ggaatagtcc accatgtaca ctggccactg ctagagagag ggggaggggg gatagaggga    240

ccacgttagg actccaggtg acacggagga actcccagaa taatcagcgc gcttgctttg    300

agggtgagct caaacgttaa cttagtacgc agacactgag gcctggagag ggaaagagat    360

ctgtccgagg tcatgcaggt agaacgtggt gattgtcccc acctgtgttc caggggctca    420

atc                                                                  423

<210>  306

<211>  555

<212>  DNA

<213>  Rattus norvegicus (Norway rat)

<400>  306
tttttttttt tttttttaaa attctgttct ttttactcat cagacttact taaggatcaa     60

atgggataca tattttggat ggatgttctg gcttgaaaat gattttggat tctattctaa    120

taacgtcttt aatataaagg tatttcccac aaatctttat ataaaatcaa ctctagatag    180

acagatagaa gcacaatatt ggccttgagg gttagggcca cccaccagtg ccctgtattt    240

gtgaaggccc aaaccattca ccacattgaa cacgtaaggt taaaataagt cttcctaaga    300

cagtggacaa cccacaatgg ttaatcaaaa gataactgta tgaactccct cccatcaagc    360

tccctggcag aggctgcagg gacctcttca gctcttcagt gtatgtagat cttgtcacag    420

atggctccag gaaaatgggt catgacctgc atccgcagcc accagtagta gtccatggga    480

tggtatcggg tataaggggt ggcagcagtc atggcatggg tgacagcgtt gatgacggag    540

gacgtatcgg tggaa                                                     555

<210>  307

<211>  456

<212>  DNA

<213>  Rattus norvegicus (Norway rat)

<400>  307
   tttttttttt tttttttcct tatgcagaga acctttttta cagagtatca acggtcctat     60
   gagtcctgat gcaatatctt ttggagcatc cacatgagag tggtaaatcc tggtcacaca 120
   attgctgtct ccctcgccag gacttggctc attggcacgc agcacgtaca aatactgctg 180
   tccaggaaac agtttgtcat cggctctttg aaaatcagtg gtgttgtcag ggtagatggc 240
   cccctcgttc gccttggtgt aagttacccc atgagcatga aaagtgtagg gcctagaggc 300
   aaagttcttt acgtgaacag aaactttgtc tccaacttca gctttgatga cagggcctaa 360
   aagccctatc caggctggtt tgtctatagt cttcgtaaag gtgccatctg tgtactcaga 420
   ataaagggcc ttcttatact ttcttcactc gtgccg 456

<210> 308

<211> 390

<212> DNA

<213> Rattus norvegicus (Norway rat)

<400> 308
tttttttttt tttttttcac aataccagat caggatttat tcgatgacca cattcagtta 60

ttcagacata gaacaacaga ttgcataaat atcaatgttc ataaaatatt tatcactttt 120

gaaattttaa tcccatggga acatccctgg agggagaccg gacattttct cctgaaacac 180

tttatcaaag gcttcagctt gggatacagt gaagtgattc ttccagtccc caacagtgcc 240

tttcctcatg aggccaatac cagtagaaat tgattttttc atcaggaggc tataattgga 300

catgtcattt tctttcatga cttggcaggc actatacttg aggaccacat ccagctcatc 360

gggctctagt tttttcccca ggaagtcaca 390

<210> 309

<211> 425

<212> DNA

<213> Rattus norvegicus (Norway rat)

<400> 309
tttttttttt tttttttaca catgcgcggg ctttattata aagcacgttt cttgacctgg 60

ggcctccagc taggatgata ggacagcacc cagtctcggt ttaccaggac tcgcaactta 120

tcacaggcgc ctgagggctg tccagtaacg gagagggaac atagccactt ccggaggcgt 180

atggggtaag gtgtgtggaa aactttgaaa actccaaagc aagggccacc cctccatggc 240

tattgcacgc accggaagga actcaacgct gtacgtcagc tcttctccct gttaaaggga 300

cacagcgatc cttttttcct aaggcctcga aagacttccg cccacaccca atatggcggg 360

cgatgggagc gggtagaatt ccgggaatgt ggagcagatc actgggacgg agtggagcct 420

cgtgc 425

<210> 310

<211> 613

<212> DNA

<213> Rattus norvegicus (Norway rat)

<400> 310
tttttttttt tttttttcac aataccagaa caggatttat tcgatgacca cattcagtta 60

ttcagacata gaacaacaga aacataaata tcaatgttca taaaatattt aaaacttttg 120

aaattttatt cccatgggaa catccctgga gggaaaccgg ccattttctc ctgaaacact 180

ttatcaaagg cttcagcttg ggatacagtg aagtgattct tccagtcccc aatagtgcct 240

ttcctcatga ggccaattcc agtaaaaatt gattttttca tcaggaggct ataattggac 300

atgtcatttt ctttcatgac ttggaaggaa ctatacttga ggaccaaatc cagctcatct 360

ggctctaatt ttttccccag gaagtcacat atcttcttta tggttcccat tgtatccttt 420

ttcatgtctt catagtacag taacaagaag ttgtcccatt ctcgcatgga cagccaggca 480

cggatgtgct caaaccatga tccatataga acatttcctt tgaggaacca ttcaacataa 540

gttcccagtg agtctggctt cttcacaaga gttgaattac cccagaaata ataaccagaa 600

acaagaacat ctc 613

<210> 311

<211> 491

<212> DNA

<213> Rattus norvegicus (Norway rat)

<400> 311
tttttttttt tttgcttccg ctgctgttta ttgacattca ggtgggcact atagcaacag 60

gcctggagac gctgcagagt acgaggtgga gagtggaaca tctgcaggga cagcagtgga 120

gtgcacgagg agagaggcca aagctgttgg gaaagcaagt cagggacagg gccaaaagtc 180

atctacatgg gaaccctggg cccccagcct ctgttcttgc ggtctcctga ttccaggcca 240

gggctgggaa ttctctggaa aactttctac aggagcaaag aacacagaga taatgctgcc 300

cttctgtgat aaagtcagag ggtttccaat cctgcattcc tccttcaacc ctggctcaag 360

tagggccatg aaaaatagct gggctctatt gcatgtttca gaggcattaa tttttcctgg 420

tgtcccagcc caccagcgcc acactatggc ccagagtgag cactacaagc gttgctggcc 480

taatggatag g 491

<210> 312

<211>  145

<212>  DNA

<213>  Rattus norvegicus (Norway rat)


<400>  312
tttttttttt tttttttctc ttccttttct tcagcctgat caacattgac ttccttgtca     60

cgttccttct ccacacatag agtcatgggg tagccaataa actgggaatg tttcttcaca    120

atttctttta ttctcggttc ctcca                                          145

<210>  313

<211>  528

<212>  DNA

<213>  Rattus sp.


<400>  313
acatatactt gtatgtgggc atgtgcacgt gcctgttgag gccagaagag gacactgtat     60

cccctggaac tcggttacag gtgagtgctg ggaatcaaac ttagatcctc cacaagagcc    120

gtactttctt aattgatgag ttaactctcc aacccccaag ggagaaattc ttcacaatct    180

gtcatgctta gagaagacac ggtgtaagaa aacgagtcct ttacatttct ttttaataaa    240

acaaacgtgc cacagctcca aaccctggcc ccacatcagt ctttccgaag gggaacagtc    300

tcctccctga tgccctcttt ggtcacgatg cagatcctga gggcgtctcc agtgtacaca    360

tccctctcgg ctgcagaaat gaagacgtct ttcaccagcc tcatggctct gtccagagtc    420

agggggacat gctccacatt ctgcatattt ttgaagccaa cctggttgtc gagcagcggc    480

tgcagcatgg cacttgctga gcctccggct ttgaaagagt ctctctgc                528

<210>  314

<211>  368

<212>  DNA

<213>  Rattus sp.


<400>  314
aacatttaaa tataaaaata ctactctgct ttgagttata aatggaggac caagaaactt     60

ttttcttcct ttacagtagg gccatttgtc aggtgaactg tgtttcatga agggagggtg    120

aactcccctt ccctaagcta aggaagccaa gagaccaacc tccaactcct caccagactt    180

caatagaaag ctggtttcac cggaagaaat aacagctctc tactgacaga gaaaatgtta    240

atctaagctg ttgggcctgc cctttcaatt atcctgaagg aaaaaaaaaa ccagtcctcc    300

ctcaccacaa gtacacccga tttgtgacag ctatgcctac aggacatgca ttcccgctga    360

gatgaagc 368

<210> 315

<211> 537

<212> DNA

<213> Rattus norvegicus (Norway rat)

<400> 315
tttttttttt tttttttgaa caatgcaaag gcatttatta tagaagcact ctcagaccac 60

actggaaagg tccactcagg tggatttggg tggagactac tctgtcccca ttcccattca 120

tgcccttcct catcacctag gcctggagcc cccctcccac ggcttgcggg ctacaaaaga 180

ccccacacag gagccttctc caagtagtac acccccatca gtgacagatc tctgcattta 240

tagcaagaac agttcccact cctagcagga tcccaaactc catcccaaat gaatacagat 300

ctctgctgat ggtttcgggg gtcaccttgc agggtgaggc agggtgaggc gggtctgcag 360

gaccctgggg cgttaggtga ggatgtcttg agcctggtgt tctaccagca gctccatgct 420

cttcacatca gtgcaccaga gctccagacg gtccttcatc cccttgatct gctgcaaatc 480

cagcactcga ggctgcaccc acgtcatgtg aacccgcttg tccacctcgt ctatgct 537

<210> 316

<211> 283

<212> DNA

<213> Rattus norvegicus (Norway rat)

<400> 316
tttttttttt tttttttgcg actttgaaag attgtaatat attctgtgga aaacattcgg 60

cagagcaaaa gccctccctg ggccctcccg cagtatgcaa ccagtggaac ggtctggaaa 120

tctgcagctc tggaaaggtc cctggctcag tccttgagga atgcaggcag ctatatggga 180

agaacctgct ccaggatggt tctggatgag atggggatcc tatcagggaa gatgacttca 240

aattcgataa caaggtctcc acgtttctca ggtgttttgg gga 283

<210> 317

<211> 546

<212> DNA

<213> Rattus norvegicus (Norway rat)

<220>

<221>  misc_feature

<222>  (516)..(516)

<223>  n


<400>  317
tttttttttt tttttttaaa taaaaagcca caaatttatt gtaaatctag aagtaccaga      60

acgtcactca tacagatcag cgatcctgtg gtggttttcg tttttgtttt ccccaccctt     120

cccaacattg gcacccctga ttgttgacag actgaaaagg acacacgttt tcctgtctca     180

tgcagtagac gagagcagac ccctcagaaa ccagttataa atattaaacc atctcgaaca     240

ttcgtagtac aaaatcgctt gtaaagtgct aaagtcagat aacacctgca aaccgaccct     300

cgagcctaga gcatttattt ctcgggggca gccccacaga gcttgccaca tccatctcat     360

ttcactcgag tctgctcttt cggacagaca cgttgtcttc cagtcactcg ccctccccca     420

agttttacag attgtcctac tagttctatg gctgataaga tgctgtatta aacgtggcgg     480

gtggtgacgc acctcaccac ttccgtctca gtgcanagaa gagattcaag cttgtgtgta     540

cctgta                                                                546

<210>  318

<211>  371

<212>  DNA

<213>  Rattus norvegicus (Norway rat)


<400>  318
tttttttttt tttttttaag agcttagaaa atttattttt ttcttgaaat gacacacatg      60

ggcagggagg taggacttgg tgtaggttgg tggaaaccgt gtcccccagc atcccagttt     120

tcctctcagt tctttgatga gaggtctcct aaagactggg aggcggagcg gagagctccc     180

agttctaagc ttggaatcta acttgaagtg cggagggtat ggggatgtgg tgggagccat     240

gggactttgt tctctggtaa aaccaaaaaa tcaaaacaac aaaactccga gtcagctcgg     300

ggatacttaa tttgcatatt ggatgctggg tttcaggaag gatgttgtgt gttggtcctt     360

gcctcgtgcc g                                                          371

<210>  319

<211>  454

<212>  DNA

<213>  Rattus norvegicus (Norway rat)


<400>  319
tttttttttt tttttttaag ttttatttaa aaaaccttcg gtgcaatatt aaaaagcaat      60

acagccaggt ggaacagtcg acagacagaa agggaaggag ctagaggacc caaggtcctt 120

tacctcacct gttccccaga agctgggggt caagggggag ggtctggggg tggtgggacg 180

aggaaaaacc accaccagga tgaataggta gaacaaaaca catataagat acatctggct 240

gaactgaaga ggggtttggg gccacaggaa gctcccctgg tttttgttgt tcaaaaatgt 300

cctggtttgg ggagaaaggg tgacccctgg caaatgagcc agccaagtcg tccgacccca 360

cactccactg ctgtggagag gggtgaacta aggtcacggg tgtgagaaaa gggggaaaat 420

ggagaaggga atcccctcac tggagtctgt tagg 454

<210> 320

<211> 97

<212> DNA

<213> Rattus norvegicus (rat)

<400> 320
cagcttctgg ggaacaggtg aggtaaagga ccttgggtcc tctagctcct tccctttctg 60

tctgtcgact gttccacctg gctgtattgc tttttaa 97

<210> 321

<211> 477

<212> DNA

<213> Rattus norvegicus (Norway rat)

<400> 321
tttttttttt tttttttgac aggtacacaa ttttatttaa cagcatttaa agtccacctc 60

agaagaggga caccagagaa ttcttttttt ttttcattta ataaatacaa atgaataaaa 120

atactttgtt ttgtacagag accgcctctc ctctcttcct cccccgcttg cttgccagga 180

agggctgagg atggcaacat gccctgtggc cgctctgcat gggcatctcc ccacacagac 240

cgcttctcac agagagggct tcatctcagt ggcctacaat actatttcgg tacaatcccc 300

tcctcctgca cctaccaaca ccagactctt gcctttcaaa cagaccgact cccctgggag 360

aaggaaagca caggccccca caggtgcccg cctggagccc catagctggg gactcgtgac 420

accatgggac atgcacgctg gccactgaca tgtgggcacg ggacagaagc agacagt 477

<210> 322

<211> 499

<212> DNA

<213> Rattus norvegicus (Norway rat)

<400> 322
tttttttttt tttttttgca agggagggaa gagtttattt ggctttcaat tccagttaca 60

gttcatcatt ttggggaagt caaggtagac gtttgaagca agtaccatcc tgtctggtca 120

agcagagaaa taaatgcact gaaggcgctt gctgctcact ttctaacaac ccagaggcac 180

acttgttgga acggccaatc ttctgactag aatagctaga atacctaccc caccacctca 240

gcttagaaga ggtcactgaa tccaatttcc attacaggat tggtcttgat ttgatcaatg 300

ggaaaccaca agacaacaag caagcagggg tgtttgagcg aagagcctga agttcaaacc 360

agaagccaca tccccattcc ttgaatggat catattgggg gccgtgcata acggtgcatg 420

tctttaattc aagtactcag gaggcacaga aaggtggatc tctgggaatt gaagccaagc 480

tcatctacaa aacgatttt 499

<210> 323

<211> 194

<212> DNA

<213> Rattus norvegicus (rat)

<400> 323
atccattcaa ggaatgggga tgtggcttct ggtttgaact tcaggctctt cgctcaaaca 60

cccctgcttg cttgttgtct tgtggtttcc cattgatcaa atcaagacca atcctgtaat 120

ggaaattgga ttcagtgacc tcttctaagc tgaggtggtg gggtaggtat tctagctatt 180

ctagtcagaa gatt 194

<210> 324

<211> 442

<212> DNA

<213> Rattus norvegicus (Norway rat)

<400> 324
tttttttttt tttttttgac attccacaaa gaaatattta ttgagcgcct gccacgtgcc 60

aggcaccgtt taccccccaa tgagaaaaca aaacggaaca aaaacaggac agaggcagca 120

gaaagaaaca aagggcagtt ggaaaaaagc taagggaact aagcgcttga agagctcctg 180

gatggccgtg ctgtttccga tgaaggtgac tgccatcttg aggcctcgcg gtgggatgtc 240

acagacagcc gtcttgacgt tgttggggat ccattccacg aagtagctgc tattcttatt 300

ctgcacgttg agcatttgct catctacctc cttcatggac atccgtccac ggaagacagc 360

cgcaaccgtg aggtaccggc cgtggcgtgg gtcacaggcg gccatcatgt tcttggcgtc 420

gaagacctgc tgggtgagtt ca 442

<210> 325

<211> 430

<212> DNA

<213> Rattus norvegicus (Norway rat)

<400> 325
tttttttttt tttttttaga ggaaaaaatg ttatttttat ttcaataagt ggttcaaaac 60

ttaatggcat ttctgtaggt tagtaaatgt ggctctaatt gcctgtcatt tgttagatga 120

agtatggatc aaccagaagg gccgtgtcgc attagcatca cctccagcag tagcagcaag 180

gtcattgtcc tcatttagga gcttcaggta gacgccgcga caaagactct catactaata 240

gtcatcagaa tatggataat tagggtgttc agcagctatg ttcacaggaa aatacctgaa 300

gtttctgttc aggccatcta ggacttccat atcctcggaa ggcaactgaa attcaaatac 360

ctgaatgttc tctttcatct ccttctgttc ataagtttgg gccagggcaa caaccccacg 420

ctgtacctgg 430

<210> 326

<211> 519

<212> DNA

<213> Rattus norvegicus (Norway rat)

<400> 326
ttattttttt tttttttgga agtagaaata tttattcaga atataagaac gtttgtaaaa 60

tattataaat gtctctgtat aaataaatgg cgtttttttt tttaaacaat tctatatcaa 120

ataacacaaa ttagctattt tacagcagct aaaaactaaa ggcatctgga aacatttaaa 180

gctacaagtg aatctaaaac tgacaaggta tagtacagtg tgtagtagcc actttaaaat 240

gacactttcc atacaagcag aacagtactg acagatgcag cagacagatg tgctttaaga 300

acagtgcatt caagcaggat tttctaattc aagtggtata aaaaacattt tcaattaata 360

aaaaagttaa atttcatgca aagtaagtta atatgtctaa aagcaaatta gaaatagaag 420

tgaacatttg tagttgttgc atcaggaagg taagtgcccc aacaggagca ctgcagaaga 480

acgctgcgga ctctacagaa tcccttccac atctcaacc 519

<210> 327

<211> 453

<212> DNA

<213> Rattus norvegicus (Norway rat)

<400> 327
tttttttttt tttttttggg gtgtttccag aagagaaatt taatctaatt tacatatttc 60

taggtacatt gataaccaaa aggtggccac tgaaaaggtt aaaagttcaa ttagctcctg 120

gtatctagct ggcccaggat tgcaaaataa aaagatccac gttccttatt ctctacacaa 180

aacgcatttt ttaaaaaagt gaaagatcta gggagctata catagaaagc aacagtgaaa 240

agggggaggg tggcaggggt gggggaggag agtcccaccc tcactctacc ctcccagccc 300

tggagcccct gaggctctga catggcagga ggcaactgtc agctttgagc tcttccaagc 360

taggaaggcc cctttggggt ggggaagggg cacccatcaa gaatttccgt ggcattgtgg 420

gctcactggg agctcccaag ccctatggga tac 453

<210> 328

<211> 553

<212> DNA

<213> Rattus norvegicus (Norway rat)

<400> 328
   tttttttttt tttttttcca ttccagtttt attttaaagt tttaaaatta caaccacagt 60
   aattttttcc cggcactagt tacacttgtt taaaaagtgg ggtagggtac ccgagtttcc 120
   tactttatta aaataagagg acaactgtac agagtttttc tcccagctcc cgaggtgaga 180
   ctaagtacta gtcttagata tggatatata catatacaca catgcacaca atcccgcttc 240
   tctaaagcta cctagcaaat tcgaatccac gggagtatat cccacagtcc actccctgcc 300
   tctccccctc ctcggctact tagaaaagac tcaaaagctt ggaggggttt taagtggcag 360
   aagagaaaaa gaaatcaggt cccccaactt accaccattg gtagggcagc ccagggagtt 420
   tatcctcttc tcccccacaa ccccccttgg ccaggtagat ggggtatcac ctgctcatca 480
   aaggttctga aacgctcact agggacaagc aacctagttc ctgggtcctc acctacccag 540
   aactttgcca gtt 553

<210> 329

<211> 504

<212> DNA

<213> Rattus norvegicus (Norway rat)

<400> 329
tttttttttt tttttttata gactaggaaa tataatttat ttcataaaaa ttaattttgt 60

tacaagagga atgctaaagg ttatttacaa gttgtttaca gaatgaacgg gtggggctgg 120

gactatcccc agtggatcag aacccacaga cacacagcca tgttcacagc ctgacatcca 180

agctcccaca cacccgacct ctactagagt cccagaggag tgtgggaacc taaggggcct 240

cgtggagcat cccaggataa aaggacactt aagcccagag aaagcggtta tgtgcctgaa 300

gtcacacagc atagctacaa cttggttccc gggcttccca tttctatgtg cgggctaaca 360

gtgaccagca agagtatgcc cacggggatg agcatctttg gcaggaggag ctgaggacac 420

tctatgaggc accattcacc tagatgccag gagcacctcg gtctcagtct tagagtccca 480

cttcaggagc cactgcggaa accc 504

<210> 330

<211> 502

<212> DNA

<213> Rattus norvegicus (Norway rat)

<400> 330
tttttttttt tttttttgtc acacaggcat tgtttattcc taagctttac agtgaaagtc 60

aagtttgaaa ggatagtttg ctaccataat aacactgggg ggtagggtgg ggtgagatac 120

aacaggtatg agcttgtgtt cagaggactg acttaagact taaagctctt catctctaca 180

ggtgaacatc gatggaggag ccattgcctt gggacatcct ctgggagcat ctggctgtag 240

gattctagtg accttgttac acaccctgga gagagtgggt gggactcgtg gtgttgcagc 300

cctgtgcatt gggggtggga tggggatcgc aatgtgtgtt cagagagggt gagccaccac 360

ccttacagtt ctcattcaaa cactaaacag aaagaacgtg agaccagagg accaaagtga 420

ggacaggaac ccaggtcgac agctcgttgt actttaatgt gagacaccca aggctaagac 480

attacaccca acactgtcat aa 502

<210> 331

<211> 569

<212> DNA

<213> Rattus norvegicus (Norway rat)

<400> 331
tttttttttt tttttttata tataaaatca catttatttg agactgggac tttcgaagcc 60

cagtctggcc tgatctagtg tccagaagca ctgattagca gatgtgtttt cctctagctg 120

gctacaatgg ctgcggttca ttctattcag atgtcagaca ataggcacag ctgggttcct 180

tattcaaaat ctgaaggagt ctgggaggag gacaaacaca tagatagaat caagcttagg 240

ccaggaacca gaaactacgg attgctttgt taaaggccaa ggaggcttca aaagcgaaca 300

cagctggagt ctcatttcag tctccatttt cgcaccactt cagtggaagt tccatgaaac 360

agcccgatgg ttctgaagtg ccatcaagtc acttcgagct ccagcaactt aggtttccag 420

gacatcttct agaagaactc gatcatccct tccacgttat ggggccactc acctggcctc 480

gatgcccacc tggactcgaa gtagccctgc accgcgcctg taccctgatg acgacggatg 540

cagaaaaggt gagggccgcc ctcgtgccg 569

<210> 332

<211> 559

<212> DNA

<213> Rattus norvegicus (Norway rat)

<400> 332
tttttttttt tttttttcac acagggttgc ttttatttcc acatccaact tgagcagagg 60

ccctgccaca acctgaacag ctgtgaggtg ctgggtgcct ccagagtttc tggcacagta 120

agtgttgggt gtgcagactt cctgatggcc acatgacact ggcccacaca ggaacagcaa 180

gtccatgaat ggaaatccca ctgagctgga agtggaggct ctggaaaccc catgggcagc 240

agcaggagtt aaaggagcca ccaggaacac tgcagtgagg ctccaatgca gacagggctg 300

ataaaaaccc aaacagggca ttgtgagagc agaggctcga gtgtccccgc tgaggacccg 360

gggctgaagg cacagagctg tgtcgggatg gaagaaccct gggtgcactc gcagtccaga 420

gcacgaaagc acaggtgaga acccagcccg aggctctctg tgaagagtgt ggccttggat 480

cttgggcacg gcacagtgac acacagtgct gaggtcaatc ctgacttccc agaggaatga 540

cctcttcagt gacaaaaaa 559

<210> 333

<211> 176

<212> DNA

<213> Rattus norvegicus (rat)

<400> 333
ctccacttcc agctcagtgg gatttccatt catggacttg ctgttcctgt gtgggccagt 60

gtcatgtggc catcaggaag tctgcacacc caacacttac tgtgccagaa actctggagg 120

cacccagcac ctcacagctg ttcaggttgt ggcagggcct ctgctcaagt tggatg 176

<210> 334

<211> 393

<212> DNA

<213> Rattus norvegicus (Norway rat)

<400> 334
tttttttttt tttttttgca attcacactt gattttattc catgacactg atttgctgaa 60

accccctgtt tcctgggcag ggaagggcag tgggtcgcct ggtagaagcg gggctgcctg 120

cccaggagga ctgggattct ctcttctcag ggaagcactg gccaagcctc agcattccct 180

gccctgaccc ctaaccttcc agttcattta aaaattgagc aaaagtaaaa taaaatgaaa 240

aaaaaataaa aacaaacccc tgagttgtta aaaaccaaat taaaaatccc ctttggtgtc 300

agaaggtcct attcctaaaa gccagcaggg tagggcaggc acaagcctgg cttgggctag 360

cattcagtcc tcaggctcct catcagactc ctt 393

<210> 335

<211> 450

<212> DNA

<213> Rattus norvegicus (Norway rat)


<400> 335
tctttttttt ttttttcttt tttggaaaac caaacatgct ttattttatt cttcacaatt 60

tatttaaaca tctcacagga cacaataggt acaattcaat tttttttctg cttgtccaag 120

aaacaggact tcttcggaac cacggggagg aacgaaaatg aggctggcaa agaaacgaat 180

gctgaatcta gagaggagag aatctggggc aagtgttctt cattccttta gttggggata 240

aggtgaacga gagggccgct aagtcaaatt aagaatccca ctcactgcac atcactatgg 300

aggatcgagt cttctgtaat tcttctagct ccatccacat tctcctagta ggtctgggaa 360

gaatagtact agggttatta ggaataatag taatataaat acacctagga ggtctttaat 420

tgtataatat ggatggaatg ggattttgtc 450

<210> 336

<211> 547

<212> DNA

<213> Rattus norvegicus (Norway rat)


<400> 336
tttttttttt ttttaacacc aagaaaacac tttaatcaaa ctacagaaac aatggttata 60

gtacagaata ttcataagca aaagatacac catgttttaa gtacttacaa agttacaaac 120

catttgcttc cttaacattt tctgtttttt ttttaagttc acaacacaag tatcagattt 180

accattttgc gctttttttt tttgagggaa ggggggtgta tttatcatca gctagatgtg 240

ctcactgtat gctccattat ttatatgcaa ggcccgggtg actggaagtg cagttgtcag 300

gcattttaat aaactggaca gccatttgtt tctgcacgac aaggcatctt tacacaggag 360

caatcaggag aaaacaggaa acagccaagc actctgcact gcaacacgcc accttaacag 420

ctaaccagca ttactcaact gctacacaac tgcgcctagt gcacaaaaat acataagaga 480

agagattaga attgtgtcgg gtaaacaatc ctttaaaaaa aaaataagtc ttttcacctg 540

aaaagtc 547

<210> 337

<211> 471

<212> DNA

<213> Rattus norvegicus (Norway rat)

<400> 337
cggccgcacg aggacaacca gagcttccag tacgatcatg aggccttcct aggcaaggag 60

gattccaaga ccttcgatca gctaagcccg gacgagagca aggagaggct ggggaaaatt 120

gttgatcgaa tcgacagtga tggagacggc cttgttacta ctgaggagct gaaagtttgg 180

atcaaacggg tacagaaaag atacatctac gataatgtgg ctaaagtctg gaaggattat 240

gatagggaca aagacgaaaa gatctcctgg gaagaataca agcaggccac ctatggctac 300

tacctgggta agacaccaca gcagagatgg cacagtgggg agcaaggtca caggaggctc 360

atcatggctg cctccttcac ccaaatgtca caaccttcaa gtgaaacctg ttgtgtaaca 420

tcctcctcca cctactaaga atgtaccaag ttgcttcctt tgactgacat a 471

<210> 338

<211> 404

<212> DNA

<213> Rattus norvegicus (Norway rat)

<400> 338
tttttttttt tttttttgct tttaaaagat tttattacaa gcaggaaacc atgcacttcc 60

attgcaagcc attgtaagca gaaagacaga tacacttcag gcaaggtagg cttttattac 120

attggctaat gctcatgttc aagtgaggct ctggttcagt ctgggctgcc acctgccatg 180

cctgtgatgt gggacagcca gcacccacgg ctttgcggcc tttcacgctc ggatagctgg 240

caacaaggca gtagtaaaaa ggagtccaac ttgtcagttt tgagtagcag ctaaggcctt 300

cccagcacag aggacaaagg gcttgttata caatgagatg atcatgacat tctagtcact 360

tgtaggaact ccaccttagt ctgggtccta agttagccca catc 404

<210> 339

<211> 607

<212> DNA

<213>  Rattus sp.

<400>  339
ggaacgggta ctattattta ttataaccag ggcactccac cttccctcag gggatagatg     60

aagtatactt gtcatgtaag tcagctacat tgtcactgga gacacggatc cagccatcct    120

cccgcacatg gtagaggttg actgctcctc cggagtaggc atctctgtag gtggcttggt    180

agatggctcg gcgggccagg tcataggcct cctccacttg taggtcatag gagtagcctc    240

gatccatgac tccaaacgca tacacggagc cagatcccac tgagaaggcg gttccagaga    300

tcctgttccc ctcactgtct acatagtaga ggcccgggcc tctcttatcc cagccacaga    360

tcatggtgcc catggacagc cccatgcctt tgtactgata caccatgttg gcaagcagtt    420

tggaagccgc cgcgaccgag atgcgttcct tgttgcgaag ctcatagatt cgacactgtc    480

gagccaacaa ccgctcccag aagctgcaat ccgctgcacc cccagccatg gtgcccagaa    540

cgtaagggtt gatctctatc actttcttca ctgtctggga agcaatgtag gcacctgccg    600

tggcccg                                                              607

<210>  340

<211>  614

<212>  DNA

<213>  Rattus sp.

<400>  340
aattttattt cttctgctag agaacaggag ctggacacac gctgcccagg cacagctagg     60

tgctaacaca cgcaggcacc aggccaactc acttaagttt cttcctcttc ttcttcttcc    120

tcctcttcct cctcatcctc ttcgtcctcc tcagagctga aggtgccatc aggcaagctg    180

tagactcgga tgacctgctt gttggggtcc ttgaggatga ggtatttgcc ctcctccagc    240

ttcatgcaga tatcaatgac acagcgcagg atgccccagg cattctccac actcaggttg    300

atttggctgg caaactcatt gggcttaaac tgctgggtgc ccaggatgac gtggcgcgag    360

gagtccttta catggtaccg ggacacatac ccgagcttca agtactcaga gccagccagc    420

aaagcacagc acgtccatcg cgccaacttg tagctgttgt tcttcaactc agtggcgatg    480

acagccccac gctgagagtc cagcttctga cgccagtcaa cgccattaca atgcctggag    540

tcccattcat tgagtgtctt gatgttgatg aaggacactt ccccgttggc cccagtcatg    600

acgccatcat gttc                                                      614

<210>  341

<211>  421

<212>  DNA

<213>  Rattus sp.

<400>  341
caggaaaaaa gcattttatt cacatataac cgaagcccct gggtctgctg gggggggggg      60

ggggctgcag ggagtctcag ttggcttccg atggtctctc ctgagatgtg agcttccagc     120

tgtgccctct caccctctgc gatgaatact cagtagattc cataggtggg ctcatgactg     180

tccctgtatc ggtccagata gcgtaggggc tgctggtggg ggaagcgctt ctgcttgggg     240

tggtaagggg gtggccgtac aaattcaaac acaggttccc gcatgtctag aagctggtgg     300

aaggtgtggg tgaccgagtc atcccagcgg cactggaaga aagccagacc agctggagtc     360

atggcttcct ggtgtttctt gtacaaatca acggtgtgga aggtcctctg ggccagctgg     420

t                                                                     421

<210>  342

<211>  243

<212>  DNA

<213>  Rattus norvegicus (rat)

<400>  342
accttccacc agcttctaga catgcgggaa cctgtgtttg aatttgtacg gccaccccct      60

taccacccca agcagaagcg cttcccccac cagcagcccc tacgctatct ggaccgatac     120

agggacagtc atgagcccac ctatggaatc tactgagtat tcatcgcaga gggtgagagg     180

gcacagctgg aagctcacat ctcaggagag accatcggaa gccaactgag actccctgca     240

gcc                                                                   243

<210>  343

<211>  599

<212>  DNA

<213>  Rattus sp.

<400>  343
gagcgtcccg gaagttttat tgggttctgg ttgggtcagg gtcccccctt catcatctag      60

cgagccgcgc tcagcgcccg ttactgggcg cgctcagctg ccccatcatg tcggccagca     120

tgcgattgca cagcgcggcg tacggattga gcagcaccaa ctggcgccgt gcgaacgagc     180

tgcccagtct cgccgcctgc tcgaagtctc tgcgggcatc gtcgtcccga ccctgaaatc     240

gtgccagcag cccgcgctgc acgaagctct ggcgggcggc gcgaccccgg ccgccgctca     300

acgtcaccgc gcgctccaag tcctctaggg cgcctgctac atccccctgg agccgcctcg     360

cttgggcacg gttgttgtac gcagaggctc tctcaggtag caggctaatg gctttgccaa     420

acctctccag ggctgtgtgg aggtccccag cttctgctgc cctcactccc tgcaactcca 480

gggccttgga ttgctccaac tgtgcttgag ggaaaactcc atcttcatct ccctcctctg 540

tttcttccag gtccaatcca acaacatctc caaagggggt attagggttg aggatggcc 599

<210> 344

<211> 518

<212> DNA

<213> Rattus sp.

<400> 344
gaagagcttt ttagtagcta aatatggcac cacgtgactc agggcaatat aaattacagt 60

atgcaaaaca cactgactgg ctgaggtaaa gcgcaccgtt cctgcctcgt gtccactgtg 120

agggaaattg ctcacatgct ttaaaaaaca tctccatcat atatatatat atgtaaaaaa 180

ataatcccct agaaaggcca ccagagaggg gggctacaac gcccaccctc taccatgtac 240

ggagcaccca ctggagctgg gtagtgtagt gtccacccct actgcttgcc cagagctctg 300

tgtccaggca aagatgctgg caatagggtt ggtactggtg ggccggccct tctggtgttc 360

tcggtagtgg cgggtgactg tcccgtgagc agcctcagct tcaatggtct tcccgtcagg 420

gcagaccagc acagatgtca tcaggccaag ggagccaaat ccttgagcca ggatgtcaga 480

ctgtacatct ccgtcatagt tcttgcaggc ccacacaa 518

<210> 345

<211> 609

<212> DNA

<213> Rattus sp.

<400> 345
ggagctcttg caaggattta tttgctacag aattgctttc ataccccagc gagctggact 60

aaggacttct gggtctaaaa ggatttaggt cacttcatgt tttcaagtgc tgtgacattc 120

aaaaagcaat tttggtaggg cagagatggg gtagagtagc accatttgcc atgtggtaag 180

aggcgagaaa aataatcagt aatattaaac gtctaagaat agagaaggaa agaatacttt 240

aaagttccca tctggacagt ccctgagctg aaatcacatt tatgtgtgaa gagaaatgtt 300

tgggctgtga ccgtgaagtg acagatgctg accttgggct tggctggtga aagcttccag 360

acactctgaa tgacaggata tacgccactc atctggctga ttctggcacg tgtcctaaat 420

gtctcctaaa tcccaactct cctggttctc tgaaaggcct gtggtcatct gatccccaat 480

acttcttttt atttttgaga cagggtctct ctacatagcc ctggctgtcc tggagctcca 540

tgtagatcag gctagcctcg aactcacaga gatcctcctg ctcctgcttc ccaagtgctg 600

cccaatact                                                          609

<210>  346

<211>  686

<212>  DNA

<213>  Rattus sp.


<220>

<221>  misc_feature

<222>  (661)..(661)

<223>  n


<400>  346
atattttata aatatattta cccccaaaat gtaacacctg ccctaaatca agcaaaactt      60

taatatttaa tattaaatat taaatattta atattctgct gttccgtggc tctgtccaat     120

ggctttctca catcaaggga aacagtgaat ggtcctgagc taaatgaaag tcaaggatgc     180

ttcaggtctt ctcagtgact aggaaaagtc ttaggacatt ttctgtggga atcaagatcc     240

ccaagtcaag ctgcagctca gagacctgca ggctgatgat actcccagaa ggctacctcc     300

ccatcatcac tacaagaggc caggagccct gcctccttgg ggttccaagc cacacagttg     360

acatcctggg aatgggcctg acgaagatgg gctgtcaaag agaaggtggg ctgctgtggg     420

tctgagcctg gatcttcctc aaacactcgg atggcatcat ctccacaagc tgtagccagg     480

gcccctgtca gctgacacca agcaacgtcg tagatggtcc tggtatggaa gcccgacaaa     540

gtgcaaacac atttccagct ggggtcggag ccactgcatg ccaccccttg ttcattgcca     600

ggtagatact ggcgccagat gcgcacagtt cggtcatcac tgcaagatgc cagacgctgg     660

ncgctgggat caaaggctat gctcca                                          686

<210>  347

<211>  537

<212>  DNA

<213>  Rattus sp.


<400>  347
aaagagtctc tgctgtctac agggagcacc ctgcagacaa aattttattc tctatagaaa      60

caaagtcact gcccagcacc tcccttgtct ttatccatag cagaaaaacc agccgctccc     120

tctagggggc gacagcagtc ctgggggcga ccagtggaga tgaagtactt gctggaaggc     180

cttggttctg gttcccagag acagctgtcg tcctcctcct cctcagactc cccctcagac     240

tcaggcacgc tctcgggaag ggcaacggcc atctgctgga atctccctga ctgggcatcc 300

cacatgtatt tgatgggcac tttgaattcg gccatctcat acccaaacag cttcaggaca 360

aggacctgct ctggggtcag cacgtcaccc tccttgcaca cttcatagtc agacagcagg 420

ggcaccagac ctttcttgag ggcagtgggc agtcccagct gcctcagctg tggctccatg 480

gaatgaggga actgtttcag cggccctgga tccaggctca cagttaaagt tgctttg 537

<210> 348

<211> 102

<212> DNA

<213> Rattus sp.

<400> 348
agcacaacaa aggagccctt atttattatg tgcaaggccc taggttcaac ctccataccc 60

ccaaagacca aaaaaattaa acggatgtgt aatgtacaac tg 102

<210> 349

<211> 443

<212> DNA

<213> Rattus sp.

<400> 349
caccagttaa atttaagaac ctctctcgtc tctattaaca gataggaagc ttcagggacc 60

aggtactttg aggagctttc tgagccagtg aaagaaaaca aaatatttcc tggaactggt 120

aagcttgctg ttttcagagt gtcaacaagc aaacacacac gccacacacg tacctcgtga 180

aaacaatgcc gctctggccc ccggccacac tgcctgcagg cactgctgcg tgtccttgtc 240

agtgaatgtt aatgcagacc actgggagct gaaggcgagg gagctccctg agggagtgta 300

ggcagcagcc ctgccaccag cttctagaga tctagatgtg agctgcaatc tgcgactcca 360

tcttggcctg agcagggctg gaaggagcac aacagcacgt accagtgtat ggggaaaaga 420

actgacatgg ctggctccgg ctg 443

<210> 350

<211> 476

<212> DNA

<213> Rattus sp.

<400> 350
gctgggaaat gaatataaat taagggttta ttacaaaata aaaagtgtta agtttctcct 60

tgtcagtgat tcaagaaaac aaagccagaa gccttttgtg gagccgaagc caggacctct 120

gtgtgcaggc tggtggtcaa aagccaggct catgagcggg cagacagaca ctgattaacg 180

acctctagca ggtgggtatt ctccagggca gctgccactc gctccttatc tgcaagttgt 240

ttgttcacta agtgggaaaa gaagggtgga aaaacagggt taaaacaact cggccaggct 300

gctagcccca gtatagatta tatttctacc gatgaggttc tcttctgacg tctcaggact 360

ccaagcctgg atcaggatat atttactgcc tgtgccataa ctagcttctt cactaggaat 420

acctgtcacg gccagggact gctcattccg tctctgtccc tgtgtacttg agaact 476

<210> 351

<211> 421

<212> DNA

<213> Rattus sp.

<220>

<221> misc_feature

<222> (278)..(278)

<223> n

<400> 351
ctctgataaa tcaggactaa gtttacgagt ggtggtgacg cagagaggca tggagagggt 60

gggctgaagt gtcaggggtg gggcagtagt gtttcctccc tgaccccttc gtcctcttct 120

acaacagact gccacagtcc agaataggtg attcctctac cgtgtctgtt caactgaaga 180

aagaaacagt cagttacaac aagacacaca atgggaacac acacacacac acacacacac 240

acacacacac acacacacca agccggcact cactgtanga ggaaatgtcg atctcatcgg 300

gcagctcact gatgttgacc tcgaaacggt cctgcacgtc attcaagatc ttggcatcgt 360

tctcgtctga cacaaatgtg atggccaagc ccttggtgcc aaaccggccc gctctggcca 420

c 421

<210> 352

<211> 487

<212> DNA

<213> Rattus sp.

<220>

<221> misc_feature

<222>  (451)..(451)

<223>  n


<400>  352
   ggaaatccaa actatttttt aaaacaaaat attatttaaa tattatgaat ctctgaagtc      60
   atgagactta tctctccaaa agggaaggac ccatggtttc tattttttat gcagcatttt     120
   caaatacaca tgtcaatata tatttcataa actactaaaa aataaaaccc tttatcctct     180
   gaggttattg atgtgtccta ggtctccaac acatctcatt aaacagtaag ttctattcat     240
   cttcatgaat gaggtgggaa ctagactaaa aaataggatt ttaatccctg aggtgtcagt     300
   taaaatgcag aggttgccaa gatttttttt ttcatttaaa aattagcttt aaataattag     360
   catggatcat gctatctcaa tcaaaaccac ttcctctaca cggagtcctt tagaaaatta     420
   cattttctgg gttatggtca acctgatgtc ncagctctcc agctatgaga cttttttttt     480
   ccttttg                                                              487

<210>  353

<211>  482

<212>  DNA

<213>  Rattus sp.


<220>

<221>  misc_feature

<222>  (395)..(395)

<223>  n


<400>  353
gaaaggcctg aacacaaagg caagctggga cagcagaaag aaggcaggac attcctcaga      60

ctgctctgat tcctagagta ccaggggagg aggaaaagga aatccagagt gattgccctg     120

gcttgcccca gactcggggt tccatcctag gccaagcaag gcctcagcgg gctgcttgct     180

ccgcacgctt gggcctcagg cctccaggag cttccctagg aagcggaggt ttagaatctt     240

gatctgctgg tcttccaggt ccattcggat gatgccatcc tcaccatcaa tactcagaag     300

gaccccggta gcctctcggt cctcacccag aatcactttc accttgttgt tcttggtggg     360

ggtgatgggc tccagatgct cactggagat actgnacaac ttctcactat ctttcaggta     420

cacggagcac atgcctcccg tgacactgcg gatgacgcct gtctgcccca ctatttgtgt     480

gt                                                                    482

<210>  354

<211>  476

<212>  DNA

<213>  Rattus sp.

<400>  354
ccaagttcag cagtcgttta ttgtgctcca agagcttcca atcccggttc ttgcagcctc      60

ccttccaaag gcaagcccga gacagagcca ggttcacttg gcctgggtcc tgtaattctt     120

ggcaggtcta tagtccttat gggagaacca gttggggtga agttcaggca gcaggtctgg     180

gcatacatct gcccatagtc caagtttgca gtgctctctt cacaagatga agcgtctaca     240

gacagctcta aggcactaga gatctacaag agttctgggg tcgagttact aaccatcgga     300

accatgatgg ggtcctgggt gacccctcag gcccctgctc agaaatgcag tgagctcttg     360

cacctgggct agaaacagca agtagtgctt gagtgtatgc tgcagcgtta gcaagccagg     420

aactgtagcc actgcaaaga gtgacaagga ggttctgggg acaatttttt tttgag         476

<210>  355

<211>  386

<212>  DNA

<213>  Rattus sp.

<400>  355
gaggtataaa aatctgtatt tatattacaa tgacataatg acacagcaca gccccatctg      60

ttagacaggt gggatggggt cccattccag tctgcagctc aggtctcata actcacccac     120

tgcactggca ccaggcccct tgtgtagccc cagactctgg cacggaacct gccctagaac     180

agcaagctca accacctgca ggttagaaat acagaggcag acctagggcc catatggaga     240

caaaacctcc gtgtcctctc ctgctagaca agctgggtct gcagcaggat ggtgggtctg     300

gtacatctgg accacctcct acgaaagagg cccaagcctg agcctggagt tgcagactct     360

gttatggagc acacgtggct cacatg                                          386

<210>  356

<211>  587

<212>  DNA

<213>  Rattus sp.

<400>  356
agggtttgct gaaaatggcg gtatataggc tgaattatcg agaaggggta aggtagaacg      60

gggtttatcg attatagaac aggctcctct aaatggatat aaagtaccgc caagtccttt     120

gagttttaag ctgtagctag tagttctctg gcaaataatt ttgtaggttt aattattaag     180

gtttagggct aagcatagtg gggtatctaa tcccagtttg ggtcttagct atcgtgcatt 240

atataaatga ttagaattac tttcgttatt gggcttaagt cctaacaatg aattttcaca 300

tataacttgg attttaattc tattatgaca tttatagttg gcacgtttta cgccgaaaat 360

aattagtttg ggttaatcgt atgaccgcgg tggctggcac gaaatttacc aaccctgaca 420

ggtatagctt agtcaaactt tcgttcagtg cctaatattt atcactgctg agtcccgtgg 480

gggtgtggct aagcaaggcg tcttgagcta tattatgtgc ttgatgccct ctccttaagt 540

tttacgcaaa tgtttaaggg attttacacc ggtttatgga tgtttgc 587

<210> 357

<211> 637

<212> DNA

<213> Rattus sp.

<400> 357
cggccgccat actttttttt tttttttttt tttttttttt taaatcatga acgagttcac 60

tttgtttaga aacagtgctt accacgtcaa agcctcactt atgtgggaca taaaaataca 120

ataaaacaca cacaaaaaat tcagccacaa aatataaagt cagtatgctt gcgaaccggg 180

cctacacatt tctggtgtag cacattttca ttagtattct atgtaaaagg attcaggttt 240

tggtcacagc aatgggaaaa acacagctag aaacagtgta tacactgagt tgatttatgt 300

ctgcctatcc cacataaaca catctgctct tacgatctct agctggacac aaaagtccct 360

cccaagagtc gggctgcgtg aacgtggggc tcaagtggag acaggaatga atctgatgga 420

tttggaagat ttgggcgagt ccttccacat cccagtgctg ttcgttgggc tccggttgtt 480

agaataagaa gtctgtcttc ggctcatgct atcggagtca tccttggcga atttctgcgc 540

catgctgtgg cagcatggga aactttggac gcagtcttgc aggagatggc cactgaaaaa 600

catgtatatc cacgggttgc agcagctgtt caaggaa 637

<210> 358

<211> 499

<212> DNA

<213> Rattus sp.

<400> 358
cccacttaaa acggaaacac ctttattgac agtataggac agttaaaacg cacgcaagga 60

caaagatgag accacagagg tgtaaagttg cagcacagga gttaatattc ttcaccctca 120

tcgtctcctt cagcactatc tgccccaacc tcctcataat ccttctctag ggcagccata 180

tcctcacggg cctcagagaa ctccccctcc tccatgccct cacccacata ccagtgcaca 240

aaggcacgct tggcatacat cagatcaaac ttgtgatcca ggcgagccca ggcctcagca 300

atggctgtgg tgttgctcag catacacaca gctctctgca ccctggccag gtcgccacca 360

ggtaccacag tgggaggctg gtaattaatg ccaaccttga agccagtggg gcaccagtcc 420

acaaactgga tggtacgctt ggtcttgatg gtggcaatgg cagcattgac atccttgggg 480

accacatcac cacggtaca 499

<210> 359

<211> 662

<212> DNA

<213> Rattus sp.

<400> 359
gatgagaccc caccagctgc acaaagtact cctggcatca ggcaccttcc tccttggcaa 60

tgcgatcttt cttgagcggt cccatgaatg ctttcttttc ctccatggtc tggaagcgac 120

catgtccgaa tttggaggtg gtgtcaatga acttcaggtc aatcttctca agagcccgac 180

gcttggtctg gaccagcaag gacttccgga gagtaagcac tcgcttcttg gttcccacca 240

cacagccttt gagcatgatg aagtcattgg tcacctcacc ataatggaca aagccaccca 300

gtgggttgat gctcttgtca gacaggtcgt agtcagtaga tgcattgttc ttgatcagct 360

taccatcctt gatgaggtag ccttgaccaa tcttgtaaat cttcttgttg atctctgttc 420

ggtgatggta gcctttctgc ccagctcgag ccacagagaa ggctacacgg gcaggatgcc 480

aagctccaat acaagcaact ttgcgcagac ctctgtgggt ctttcggggc agcttctttg 540

tatgccaacg actggtcacc cctttgtagc ctttgccctt tgtcacgccg atgacgtcaa 600

tcatctcatc ttggccaaac acctggttca cagggacctg ctgctccagc ctctctcggg 660

cc 662

<210> 360

<211> 589

<212> DNA

<213> Rattus sp.

<400> 360
gcgaagccac ggcccaactt tatttacact cattgcaaag catgactgag ggtaaaggac 60

aggtaccagg ggatgggggg ctgggggtca tggagactgt gcgggtgcac ggtagggttg 120

caggagaaaa ggaaggcggt ggtgaggatg gacagcccct ctttcagtgt cccccaggct 180

cccaggcgtc cgggggcaca gagcaggtga ggctgccttc aggatttgga cttggacaca 240

gccagtccaa tgagtccagc caggcccgcc acacccaggg ccatgccacc aacaatggcc 300

atgcctacca gtccatcttt cttcatggcc ttgtcaatca ggcgttccag ctccttggcc 360

tggctgttct ggggctcagt ctgtaacagt ccccgcacat acttcagagc cttttcatat 420

tccttgagcc ggtagttgcc cacggccagg tacaagacat aatcccgctg ctcctctttg 480

ctacctttgg gcagcagctc ctccagcatc acgatgcctc tacggatgtc gtcattgtat 540

ttgcttcgaa ccaggcacca ggcgtattca aactgcgtgc tcttggaca 589

<210> 361

<211> 590

<212> DNA

<213> Rattus sp.

<400> 361
accctaaaaa cactgtcagc tttatttaac cttgatatac atttgtacaa gcaatttaaa 60

aagactattc cacaacgact cagtgggttt tacctggttt gaagataatg cagttacaaa 120

tgagaggtag aaacaccgta gtaaagacct aaaaatacaa acatcctata cataaacgtt 180

caaattaact gctagaattt catacagata aataatgtaa aaattacatt actttgttca 240

ccaagtaaat gaaaaccatt ttctctgtta actttaatca gcacactaaa gtaagtgtgg 300

tgccaaagag cttctgtgca gctatcagag cgcaggtcat tatgtcaaag ttttgaagta 360

aaaagattaa aaccggtgaa tggtttattc aagtattaaa gcaggtaagt aaaagaaacc 420

aaataatata caacatagaa accattcttg ggagatgttt gatctgccat gaccagctat 480

ttgtgaactc agggttaaca ctgagaagta cacattagtc tggagagagc tgcccatttt 540

tctaagggaa aacaaacctc atttcttact attttatgta gaaactccac 590

<210> 362

<211> 539

<212> DNA

<213> Rattus sp.

<400> 362
aacaaacttt tcttaaaaaa atggagggca ggccagggcc gtctgcggtg tcgctcaaca 60

tggcctccgg cgaatcgccg cgggcggtcg tcgggggctc aagtgagtgg aggggtcagt 120

ggtggagacc ccgttcctac gcctcgcgtc cttaaaacag gacgggatgg tcgccgcgtg 180

ggggtcgggg gtagtcgcta accactgagc tcctctgccg cccttgcctt cttcttcttt 240

ttcttccttg tggcgtcgcc gtccccgtcc tccgcagcct cctccccgcc gtccgccttc 300

tcctttttct tcttcttctc cttcttcacc cggggcgtgg tagtgggcgt cgcctcgtcg 360

gcgtcgctgt cgctatcgcg tttccttttc acgatgctgg cggcgtccgc ggtgacccca 420

gggcccggcg tggcttcggc ggccgtcgct ttcttgctgg agtcactgta gtccacgtag    480

tcgcgcgtcc aggaggcggg ggtgccgtcc gtgggtcggc catgtttgtc caggaggcc     539

<210>  363

<211>  193

<212>  DNA

<213>  Rattus norvegicus (rat)

<400>  363
gcctcctgga caaacatggc cgacccacgg acggcacccc cgcctcctgg acgcgcgact     60

acgtggacta cagtgactcc agcaagaaag cgacggccgc cgaagccacg ccgggccctg    120

gggtcaccgc ggacgccgcc agcatcgtga aaaggaaacg cgatagcgac agcgacgccg    180

acgaggcgac gcc                                                       193

<210>  364

<211>  512

<212>  DNA

<213>  Rattus sp.

<220>

<221>  misc_feature

<222>  (448)..(448)

<223>  n

<400>  364
attagccaaa gcacatactt tattagctcc tgaactctag ttctccttct cctgtacagt     60

tttggttcca cgtagccgtc cagtccggcg agcagcaatg agacccactt tgcgcccagc    120

tggggcatct cttcggatag tggaaggctt gccaatgtgc tggtggttac caccgccaaa    180

gggatgctcc acaggattca tggcaacacc ccgcacacgt ggccagcagt tcctctttgc    240

cttgtactta tggtaggcac ggccagcctt taagataggc ttgtcaattc tgcccccgcc    300

agccacgaca ccaacaacag ctcggttagc agaggaaatg accttcttgg accctgaagg    360

cagcttcact cgggtcttct tggtctctgg gttgtgggag atgactgtag catagttccc    420

ggaggctcgt gccagcttgc ccctgtcncc aggcttctcc tccagacaac acacgatagt    480

accctcaggc atggtgccca cgggcaaaac at                                 512

<210>  365

<211>  658

<212>  DNA

<213>  Rattus sp.

<400>  365
acaaacactt tttattttgt ttttaattta gaacatgata catattcaca agatttacac      60

tttatatcat accaaagcaa tctagaaaca ctgtacagag cacacttgaa catttagaag     120

gctatatata atctgtggta aagtcatagg catcgtcttc ttcactcatt ttatccaaga     180

taaaggatct gtcagatggt ttacttgctg ttgattgccc aggtgacatc tccctggtct     240

cttctacagg agtcacatct gagatctctg catttttttc accagtaaca tgttcttgat     300

catcaccatc ctgttggtct tctgtctgtt ttggtgactc ttcggggatg tccttttctt     360

ctagtattcc atttgtcagg cccgaagacc ggaaaaggat tttattagtt aaatgagggc     420

ccttgaggac ttgtatgctg tgtgcattat tcttttctag ttcttctaga ttaaagcccc     480

tcttcatgat tgctgtaata ttctcattaa aatgaggaga atgattccag gatgcagggg     540

gatggcagta gtaacctaat gaggcacctg tccactcaga ccatagcagc ttagcagcac     600

tttcgacatt tgggcttcca cctttttggt gcagacctct tctctgagca agtttagt      658

<210>  366

<211>  322

<212>  DNA

<213>  Rattus norvegicus (rat)

<400>  366
tttccggtct tcgggcctga caaatggaat actagaagaa aaggacatcc ccgaagagtc      60

accaaaacag acagaagacc aacaggatgg tgatgatcaa gaacatgtta ctggtgaaaa     120

aaatgcagag atctcagatg tgactcctgt agaagagacc agggagatgt cacctgggca     180

atcaacagca agtaaaccat ctgacagatc ctttatcttg gataaaatga gtgaagaaga     240

cgatgcctat gactttacca cagattatat atagccttct aaatgttcaa gtgtgctctg     300

tacagtgttt ctagattgct tt                                              322

<210>  367

<211>  476

<212>  DNA

<213>  Rattus sp.

<400>  367
aaagacattt tatgtgaaaa caaaaggtgc gaggtcctgt cggccctgtc agctccgcaa      60

gtcagtttgt gtgcaaatgg ggctggccac agtggcaggg agggccggca gagtgggtga 120

gtctatggag ttgtgcaaca aggaggtggc tcaatctccc tcacaaggga gactggctgt 180

acggggtagg gcaaggttca gtacaaggtc aagttcccac tacacaaatg ctttcatggg 240

tggcctccag ccccataagg attcccagca gagagaccac tccagcactg cctgactgaa 300

agctacccag ggatggaggc atctttgata ctgggaagat tctcaatgcc aaggacacac 360

atctgtgctc ctggaaacat ggtcttacag cccagaagga tcttagacca gtgcctctgg 420

actgcagtct gttcctctat ccacagtttg cctcttccct ggggtctgaa ttgagc 476

<210> 368

<211> 451

<212> DNA

<213> Rattus sp.

<400> 368
aaaaacaagg cactgctctc tatttaaagt aaacctattg agcgacagcc gtggcacaga 60

atatgttcaa gatggggcgt gaaccccagg gctcccgcat ggagcccagt actgtagatg 120

ccagcagaag gaccagtctc tgatggactc agaactggat aaaccacact gggcgtttcc 180

cagttaagtg tttgccagtg tgtggccagg cctattacag ctactgagca aacgggttgg 240

catgaatagt tatgtcctta atgtgcacat cattaagagg tctgtaagtc ttctcattta 300

ctggtaactt ctccaattca tccaaagtct ccagaccatc tattaccttt ccgaacactg 360

tatatttcat gtccaagtgt ggctgcttgc catacgtgat gaagaactga gatccgttgg 420

tgtttgggcc attattagcc atagatacaa c 451

<210> 369

<211> 409

<212> DNA

<213> Rattus sp.

<400> 369
ccgtgtcaga aactgtttaa tgttagaatt acaggctttt ggcaagtata tggcaagccc 60

atctgtccgt tcccatgtcc actgagaaca cagcagaggg ggcatgcccc aagctctggc 120

ctcagtccag acttcagctt gggtttgtcc agcgagatgt ctccagtcca tgaaagccca 180

tctggctaca gcttgagttc actgggcatt ggctcccctc ttaggccagc tgagcaatag 240

gcacaaactc tgcctgaaac tctgctgctt cctgaggcct gggctggcgc cccgtgtaga 300

gaaatctctg gacaccaaat ggtttcagtc gtcgagcaat ggcctgggcg agcgcagccc 360

tgccctgggc cggcaaaggg ccagtgacga acaccttcat cctcgtgcc 409

<210> 370

<211> 409

<212> DNA

<213> Rattus sp.

<400> 370
ccgtgtcaga aactgtttaa tgttagaatt acaggctttt ggcaagtata tggcaagccc 60

atctgtccgt tcccatgtcc actgagaaca cagcagaggg ggcatgcccc aagctctggc 120

ctcagtccag acttcagctt gggtttgtcc agcgagatgt ctccagtcca tgaaagccca 180

tctggctaca gcttgagttc actgggcatt ggctcccctc ttaggccagc tgagcaatag 240

gcacaaactc tgcctgaaac tctgctgctt cctgaggcct gggctggcgc cccgtgtaga 300

gaaatctctg gacaccaaat ggtttcagtc gtcgagcaat ggcctgggcg agcgcagccc 360

tgccctgggc cggcaaaggg ccagtgacga acaccttcat cctcgtgcc 409

<210> 371

<211> 626

<212> DNA

<213> Rattus sp.

<400> 371
actcataggt aggaatcccc tttattcctt atcaaaacag tttaacagca aatgtagata 60

atatatgttt tatttagaca tataagttat cttccatggg ggcattctat cttgcccctt 120

ctaatgaaag agggcaaatt tttatattaa tcttattcaa agactagacc aacttaacag 180

taacagacaa agagagcaga gcatctaggg tagggagtag aaaaattcta ctcataagca 240

catttaaact tcttaccagt acagagacac ctgctgccac atcagcatta ctcctgacta 300

gtaagtctcc cagtacacgt caggggaagt cccaacacgt attttaaata aatttcactg 360

acttgacatt caacgtctag attagatatt caaagtcttc tttattgcaa atatgatgtc 420

tttgacctga ggtatggagt tgtcttctag aatctttgcg taaggcatag ggacatcagc 480

accagtgact cgaacagcag gcgcatcaag gaagttgaat gcagggcctt ccataattct 540

cgcacaaatc tcagctccta ctccaaattg tggccagcct ccttccacag ttacgagatg 600

atttgtcttc atgacactgg cttcta 626

<210> 372

<211> 118

<212> DNA

<213>  Rattus norvegicus (rat)

<400>  372
gtccctatgc cttacgcaaa gattctagaa gacaactcca tacctcaggt caaagacatc      60

atatttgcaa taaagaagac tttgaatatc taatctagac gttgaatgtc aagtcagt       118

<210>  373

<211>  635

<212>  DNA

<213>  Rattus sp.

<400>  373
ccgggtaatc aaaaaggttt taatgattaa tgtttagaca ttatttgaca gtgtgggtat      60

atccagatga gctaagcaca ctttgacagc acactactga atgttatagt tttgtactaa     120

gatatgtaaa gaacctgcga gcggtaccct cggatggaga cacacatgga cgctctagat     180

tagagaagat caatgtaaac agacgctgca actccgtcca aacagaggct aatgaggacc     240

aactggctcc ttagaatatg aatgtgcaaa attaaagcat ggtaaactgg tatttacata     300

aatatcaaac ccacaattag tttgtacatt gtcaattgtc ttctaggatg tgtcacgagt     360

ggttcaatca attttcttca atgcagaagg tgttcaaagg ctaaactttc aacactttaa     420

agatgacaca tgtaacaata gtccccactt gtatgaccaa cgtctccagc gcactatctg     480

ttacttttaa acaagcgctt taaatcaatc tttagatgct aagcagaaaa aaattacaga     540

tgccaccgag atttacactg aataaagcga ttgctcccca aacacccagt tcctgcgtgc     600

tccagctctt taattttgcc tcaaaatgat tacgg                                635

<210>  374

<211>  532

<212>  DNA

<213>  Rattus sp.

<400>  374
ccacaaactt tattgtagag tctgaatact agaaagagtc ttcagtccgt tggctgtcac      60

ttccctctca ccctcatctc ttctcagcct tggtggtttt gaccgcaatg gctgggcttc     120

aggacactgg agcttgtcac agtccagctc tgattgctct aactcctgta atcccaggaa     180

ccactctttc cctgagcctg agtaaaggat tctgagttct ccatatttgt ccatctttcc     240

ccttcccacc ctgacgaccc cttcaggttt ctaggcctca gctacaactt ccttgggagg     300

ttcctcatct ggtggggcct cctcaggtgc tggcttgtgg gcaagccctc cctgtagcca     360

tgcagtttgc agtatgtctt tcagcccact acacaggatg gtgaggccac cggctattgc     420

cagtagccag gagaggagcc aggtctcctg tgccaaacgg ctacggggtt ggaggggcag 480

caccacgggt agcaccggcg ccatctccca tctctgccca agggattgag ga 532

<210> 375

<211> 515

<212> DNA

<213> Rattus sp.

<400> 375
caacattaaa atagatttat taattactgg tgaaaaacat gatatattat aaccaagtca 60

tatactttat tgaaaagaaa aaaatattcg gtaagaagtt tggcacggtc ttctgctggg 120

acctgtgtga aatcccagga cttgtaggtc ggggctgcct tcgtgagggg tgtcaatgca 180

gcccaagaag tgggtaaagt aggaagtggg ggtcaaagaa aggcaatcaa gaggtctgct 240

cacaggggcc ttttcccacg ttcatgcact gtcaggctgt atcctgggac agcggggagc 300

ctcggagagt taatgagaaa cagaattgtc actttggcga ccaatgtcag aaaacaggtt 360

cctggtcaag cgttaggtac tagcgaattc tgaccctgag acttgaggtg tcactgtctt 420

taaactgcca ggcatggagg gaagtgtcca aagatgggac ttatagagag aagtggtccc 480

ggcttcctgt agtctccatc tcaccagccc caggc 515

<210> 376

<211> 441

<212> DNA

<213> Rattus sp.

<400> 376
cgaagaaaga accaaaagtg tctactgtat aaatacaaaa ggccaaaacc gtattaacat 60

aaggtaatga actaacagag actgtccatt agagtgcgga ggcctatgcc ttcccagctg 120

acgccaggta cttgaccagg aagtctctca gcctggatat gtgcatcatc tccttcatcg 180

tggtgtctcg gcttcgaagc tggatcagcc cactttccaa ggtggtttca gtgattagaa 240

ccgtgaagag gatactcatc tcatcgtact ttgaatagag ttgctctaat gaggactgtg 300

cagtttccaa ataaccaggc cacacagcaa tcccattttc tagtaactca ttgagtagcc 360

cttggcaaac ctgtcggagt tccacggttg ggcctttccc cacatctaga gccaccttaa 420

taggggctaa acaagggtga a 441

<210> 377

<211> 472

<212>  DNA

<213>  Rattus sp.

<400>  377
caaagttgtg aacaacgtca agaacacatt tggctactta attactattt taaggatggc      60

gtgcagattc atagagtcag cattgtggtt ttcgggggga gtataaagat tctgcctcta     120

ctttccttta cagtaaaact gtgacaaatt aatgaaacaa ttatacccgc tctaattgta     180

tacggtggtt cctggcctct tttgttctac accccaccgg ctattgtata catagtgagt     240

tttaagctta taaaaaatgt agtagaaatt gctgttgtct aaataggtaa aactattcct     300

ttagtagctt gaaatattac tacgtggttg aatacaaacc tttctttagt actactttag     360

taacacttaa acatacacaa agtttaagga acaagcttgt aaccagtaag tctatgtaaa     420

attctgcatt gcagactaaa tacagcggat ggcgtataga catcagagaa aa             472

<210>  378

<211>  367

<212>  DNA

<213>  Rattus sp.

<400>  378
acagtgacaa gaaggcttac aaaggagaag gaagaagaac acattggaca cctgagattg      60

acaagggtca tttttggtcc aagtgctgtt aacatttttg agggagtttt aaggcatttg     120

ggtctcaggg tttgttagct tgccctattg ccttcttagc cagcagttct gagcaactct     180

caagctttgt taccatctga ggtgcatctt ctttctgtgt acttctattt ctaactcatt     240

gttatgctgt tactctctgg tctcccatgt agagtactca ggaggatttt cttgatcttt     300

ccgctccatt gagaacatct ttaattgtat gaaacatcgc aggttgtctt tatctacaga     360

gtaaagg                                                               367

<210>  379

<211>  597

<212>  DNA

<213>  Rattus sp.

<400>  379
catagtcaac aatgtgtttt actatacatc tcagatttat acatttcata tatgctttta      60

aagcttcaat taacagatca atacaaaata aaatatatat atatttatat atgtgcagca     120

gtggttctca ggcacaggat ttaggaggag gatggaggga gtctttcctt ggcatgtcaa     180

ccatgatagt ggaacgtgtc cttctgactt agctgccttg attgaacttt acatggcaaa     240

cagttgaaca tgtatgccat tgctcattgg aaggataaga tttgaagtaa gttcatagcc 300

tgcatgtttt gaagtcttta agacttaaat gcttgctttc ctcctacgag ctccaagatc 360

atcctatgaa aaaaatatgt ccacaaactg tctggaacac ctgcctaaga accactgcct 420

atggttattc aatgtcctca ggcctcacag gatgaggtaa tgggatgatg gatttcttct 480

cagtgtccct ggaaagtgct ttcctcatat cgtaggcatc ctcatcaggc tccccattgc 540

tggctgagta gtactctatg actgtcctgt agacactata acccaactgc ttgtaca 597

<210> 380

<211> 645

<212> DNA

<213> Rattus sp.

<400> 380
gagacagaat ataaaatgtt tttatttcaa aatgcaaaat gatattcgtt gtaataataa 60

taataataat aacaacaaca ataaaaataa cagtataaaa agcatgtctc cttccctaat 120

gcaaagtgac agctccttga tcgctcccac agccttggac aaatcacagt gcatgctgca 180

cagcagcaag tcagtcccag atcccttggg tgatctgtgc agggtcagtt ctggattcgg 240

tggcaccttg aaacagcatg tgactctctc tcgcccacct cagtcatgat ctctctgtgt 300

ctcgcttctc tgccagtcac cagccttaag aggcatgctg ccggagggac agggcccggc 360

tgcacaaagt cgcctcctgt gtcttgcctt ctgctatcag ggcctgcttg tggagggact 420

gctgctcaca agcatcccgc agctctgcca ctgccgcctc ctggtcgcgc agacggctgg 480

cagctgtgaa agggctgcac acagtctcca ctagaccgag caccacaccg aagaacgcta 540

gcacactgcc cagcacgtac agcttcacta gcttccctcg ggacttctgc gtcatcatct 600

ccttagccag aaggatcagc tcctgcacac tttccatctg agctc 645

<210> 381

<211> 630

<212> DNA

<213> Rattus sp.

<400> 381
aacaaataat gacagcttta atcaatttct ttactataaa agacaattta aagtgtattt 60

ttaaatacaa tgttagcatt gtggtaattt tgctatgaaa aggtaattct accttgtagg 120

aaagagaaaa tgcctcaaaa aacgccaagt agtttattca tatgttggaa cgggtgagcc 180

agtgaaaatc ctgggccttc cgagtacttt gtggttcaat catgatgttg ataagacacg 240

gtttacttgt gtctttcaga gcctgcctca gcgagtcttg gagctcttct ggcgtttgta 300

caaaataccc tttgcctcca aatgcagtca tgacctgctc gtagtgtgag tttggaagca 360

gacacattgg agggattgtt gtagcagttc cttgaaaatt taatattttc ccccaggtgt 420

cagcatcaaa accttggtaa attccattat tgttcactac caagattatg attggcaagt 480

tgtacctgca gatggtttcc acttccatgc cggaaaatcc aaaggcactg tctccttcca 540

cacagatgac ccgctgccca gggcttctct ccttagccac cacagcagct gcaatggcaa 600

atcccaaacc aactcccatt gttccaaagg 630

<210> 382

<211> 643

<212> DNA

<213> Rattus sp.

<400> 382
aatgccgttt atccagtctc agaggtagcc cttgaaggga agaaactttt gatttgaccc 60

ttttttctaa gatttttgtt tcaggacctg aattaagaaa aatcaaaaca aaacaaaaca 120

gaaaaattaa gagatgattt tttctttctc ttgaaagttt aagagagatt ctcagttcat 180

cttttcatga ctggaacttt ctaagacaaa ccacaaagcc atataccaag cagaaatcag 240

agaacgaagc ccagtcccca tcagctgcag gtggaaacca cggagaagcc agcacagcaa 300

gtggctcatg gttaatacct caggtctgtt ctgagaaaag atgccgatga actgctctga 360

gcaaggtttg aaacccttct ggatcagcgc cgagcctatg cactcagcca tttctgcaac 420

ctgtttgtaa gaaatccact catatggctg gtttggcttt ctagaaccta aacaagggcc 480

atcatttgac acctgaatcc ccctctggaa gccatcgtac atcgttctga catcgtcgta 540

gtagtacagc aagagcttgt cgtcctcaag gactgctgat cttcggacac cctcagtagt 600

acccgttact ttcacagact gcatggacag atcacatggg ggc 643

<210> 383

<211> 480

<212> DNA

<213> Rattus sp.

<400> 383
gggtccaaag gtgatatttc ttcacatgga aaaaaactaa attcaagcca cacaagaaca 60

tgccttgagg gaaacagtga tgaagcctgc tcggaccaca caggagcaga tgcccattca 120

gagcagaggc cgcatgaaca aatctttatt cggaacagac agacgtttac agccaataag 180

aacacaaagg ccatggacaa gactgtaagc gcacctgagc tttaaggttc tggtgaggtc 240

ctcacaaaac taacacactc cacataccag ccgggtaaga agaactcatg tgtcagggcc 300

agcgggggca cgaccataag tgagctgtga atgaccataa tgaggacctt tgttcacact 360

agctgctttt ctaatctaat caggttaatt tcaatacagc aaataaccga taaaaaataa 420

aaggtgccac ctggtcagca acatcataca ctggtgacaa gagaacgtta ctgagttgtg 480

<210> 384

<211> 464

<212> DNA

<213> Rattus sp.

<400> 384
   cactgtacag atatgaactg gtcatagctt tatttagaat aaaaagcctg aaacacaccc 60
   agtgatctca ataggggatg aaatgccagt cataggggtg aaataataca ctgattatta 120
   ccaaaagata aatacaaaat actcttttgg ggtataaaga aaaatgaagc tttattcaaa 180
   gaaatactga aaataaatgt tttacaaatt gcaatcaagc acttaaagta taatttggta 240
   atgaattctc agaaactata taatacattc tgctgttggc caatgcaaag tgtactttaa 300
   actagtattt tacatatgct caaccctcaa tcatcttacc attcattgat tataatgatt 360
   caacagtttc ttatgtagta tctcagaaaa aaaaattcca aatacaaaac attcatgcca 420
   gcaaggaaga tgtaaagaca catgtaaatt acggcaggac tctg 464

<210> 385

<211> 431

<212> DNA

<213> Rattus sp.

<400> 385
acaaataaaa taacatttat tctcattaag tgtgtttggt taccagcatt tcaacttgtc 60

ctggattggt atggattaaa catactcaaa taattaatta ggaggccatt agggctgagg 120

tgactccggg ccttgggttc ctgtataaac aaatcaaaac cctgcaaact gcaaaacaaa 180

atgtaaccca gaatattaca atgcgatcca tcttataacc tttttcagtt tgtaaaagca 240

ccagatctca agctttatgt cacagcatcc cctaagccct agagctttct ctgaagacca 300

actctagctt ctgaccccaa acctccatct ttcctggggc tacttttcct cagccttctc 360

actggtggtt cctcactcgc acacccactc ctgtctgagg cacatcaggg gactaatctt 420

tcctgtctct c 431

<210> 386

<211> 502

<212>  DNA

<213>  Rattus sp.

<220>

<221>  misc_feature

<222>  (413)..(413)

<223>  n

<400>  386
gatggtggca taaattgctt tattggagca gctgagctgg gctcaggttt ctccagtggc      60

ctggaagtcc atgtcttcca ccaagtcctg gaggcaggcc ttgtactggt cagaggtaag     120

ggagatcggc tggctgttgg agatgtgcaa gtccacggtt ttcagtgatg aggctccccc     180

ttcccgggcc atctctaaca gctccttaag acatgtagga acaaccttga ccatcacaag     240

cctcttgacc cacggctggt cctggggcca cgattccccc acacagaacc acagagtgta     300

tcgtggggaa tgtctgcttc cttccatgaa ggcaatgaga tctgccacaa agggcctgag     360

gtcgaacacg actccgttct tgtccttggg gacctctcca tcaggccctc tcncactgtc     420

tggaagcagc tcctcaccca gggcccacaa ggaatgcgag tggcctaagc gctgggccca     480

agagcactgc cctgcccgcc ac                                              502

<210>  387

<211>  354

<212>  DNA

<213>  Rattus sp.

<400>  387
aagcatgggc tttcagcagg gttcagccta aagtgcagtc ccagtaaagt cgggggctgc      60

acgaccatta cactttgcgg gggttggctc aacttcatca gaagggatga ctatgaccca     120

cagggaaaca acccatgaag agaagaacac agtgaggaaa acatccacca tcttacaagt     180

cactttgtta ccacgggcac gaaatggcag ttactgttag taggtatctg aattaacttt     240

ctgcataata caagtaggtc aaagtccagc agcaaaatat tgcaacccag caagtagata     300

cagtttcagg gtgttatact gtggaggaga gtacacaaag gacaaacaat gata           354

<210>  388

<211>  656

<212>  DNA

<213>  Rattus sp.

<400> 388
gcaaaaacat cctttaataa ctgttacctc tttctcagtg ctcccttctc aagtgacttt 60

gtcttgagag tggcaacaag ggctactcat tgctagttat ctaaaagtag attgggttgg 120

ggagaccctg agaagactgg ggaaaggctt ccatccctca aagtcagatg gcaccaaggc 180

ttctcaggac acgttcttag gctggattga ccacttggct catcatcagg ctgctccatg 240

tgaacttgtc aaagagcagg aggatgaagg gcttgttgaa cttgatgtca agtggttcag 300

agcgcaggtg taggggagcc ccgttggtag aattaggcaa cacattccct tcatccagtt 360

gtagcatggc cttgtggacc atcgttaatg tcaagggaac atctttggtg ttgcctgaga 420

aatctgattg gttggtgagc aagtccttaa tgttcaggtc ttccagcacg tctttaaggt 480

cataggtatc agacatggag aatttcggga tgtataggtt cacctgcctt ggggtcataa 540

gcttgcccca cctatcaatt gtgtcccgac taagtgcagc gatgacagtg tccatctggc 600

cctggtccgg aagaatgaag aaggcagttc catttcccac atagtccatc tgtatc 656

<210> 389

<211> 537

<212> DNA

<213> Rattus sp.

<400> 389
ccgacagtag aatttaataa aaaatactca ctggtacaaa cccctctagc actaggccca 60

agctaataag ccatgtcctg acacactgcc ttgggcgggc tagcatgtct tccagatgga 120

cacgatgttg gagtccgtca gagcagtgag gtaggtgaag gctgcattgg ccacgactgt 180

gtttaccatg gtcttggtca ctggttggcc caatgctact ggctgaggcc acttcaggat 240

ctgtgttggg ggctgcagag ttgtttcaag tggaggtggc tgcttcagta ggtgttcaac 300

atcgtagatc cacacactgc cttcctcgtc tccacacagc acgaggtttt tgtcaggaca 360

ggtgctcagt gagaagtagg ccaggctggt gggtgaccac tgcagctggg ccaggatgac 420

tacaggcaac accgactggc tgccccggct tgcccatgtc tggctccagc tccacaggta 480

gatggtgccc tgtccactcc ctttggaggc caccacatct tcgttcacaa atgccag 537

<210> 390

<211> 598

<212> DNA

<213> Rattus sp.

<400> 390
gaattagaag aattttaata aaatcatcaa gtttacattt gtacatcttg tgggtttggg 60

acccattaag aggcaaagga agctatgatg acagattcct gtgggagagc aagcaggaag 120

caagggttgc cctagttgtg caacaggcgc cccatgacag tgtgacccat ttctctgtgc 180

tccagggctg tcgaccacgg ctcacagagc ctctctaaac tctgtgctca gacctggtgc 240

ccacagcgta ggtgacaggc ctggtaggca gaagcagata aacagtcaaa ctgaacattc 300

ctgctcccct ccagatgcca atgtgacctg agctcagcct gtgcagttcc agctagcatg 360

gctacagagc ccgaggaagg agctgagcgc cagctgttgt gctacccagg gacagtcacg 420

agagagcagg gctgtgacaa aactatgctg tgttcatttt aaaaatggga tttgtgaaat 480

ggcccctggg agagtttgag gaaggtaagg aaataagagt ttgtaacatg tgaggtggct 540

ctccaccttg tagaactgtc tgctttaaag gatttggtgg gggccggggc tgggctgc 598

<210> 391

<211> 323

<212> DNA

<213> Rattus sp.

<400> 391
atttaaaatt tattgtaatg gggtctgcgc aaaaggaggg aggggagaaa aaaacaacaa 60

cagagaaaga aagcaccaac tccccaggaa ggggcagcag acagtagggg aggggacagg 120

aggagagcac aggaccttct aaggaaaggc tcacaaatcc ctgggaggaa aggcaagggc 180

ttaaaggttg agggcagtgg ctgctgctgt cctctgccac ccctgctcac tgtccagggg 240

acttcaacac aactcagggg acaggtgttt gtaaggtcag ctctgatatg gacaagaaag 300

gagcatgaga aacatcgtta aaa 323

<210> 392

<211> 506

<212> DNA

<213> Rattus sp.

<400> 392
aataatacat tatatcatca gtagaaaaca aaacgaacaa aacagccatt ttggaaaata 60

acgtgctaat tgcccaatac cagggaaagc aggtcccctc cctggactct actggcaagc 120

ccaggtatgc cctgaactct agagtctcca tcttgtatgg atgatgaagc attcaagtaa 180

acggtgaggc attctgggtg tgagtttctg agaggtacca cctcagagga gactccagaa 240

cagccgcagc cctccagttc atccgtggta tcgcgtggtc ttgggattaa ccagcagttt 300

tcttctgagg aactgtgata attgtcagcc tcaggattta gtggttataa aacaacttcc 360

atattaaaaa acacttgaga cacacgaaca tgattctcat cgcttctcca acttcttgta 420

cttggcttct agttgaggaa gaacaggaat tgtgtctttg aacactagtg ctcacaaaga 480

tattttttag ttagattttc aagcaa 506

<210> 393

<211> 492

<212> DNA

<213> Rattus sp.

<400> 393
aataaggtgt tctgaggaac caggaaatta acagagaggg gcgaataagt aatgcttctt 60

aagccctgct cagtatttcg tcagtcatct tctggtaact ggatcttgct gggatcaaaa 120

tagttggatt ccatgatggg gaggccgttg agttctcggt atttcacaag tctctcagct 180

tcccggcggg cccactcttg catcctagat tttgacttta ttttcaaatg aataattgtc 240

cttacctact tgtttgggag tactcgccct gtctgttaag ctgcaccaga aagtgctatt 300

tataaaggtt aaaaaatgtt tggatgttgg caatttccta tgggttcact taatctatag 360

caccctatac atttaagatc ctcaactaga ggttaggtac ttatatatta ggtcttttca 420

aatacacaaa cctcctaata tgcccagtag agaggaaatg gcaataataa acaccttata 480

attctgagta tt 492

<210> 394

<211> 443

<212> DNA

<213> Rattus sp.

<400> 394
aaatgaactc agaagtgcct agagaccaga gggtggtaag agttcttccc aagtccccat 60

ccagggatga agggtgggaa agatggtgtg tgaatcgatc cctggggctg cggtgtctga 120

gctccctcct ccctaactgc ctgcaggtga gctgtgatca gccagcattg ctttgctcag 180

tgaatgcagg gtccacagaa ctgaagggtg tgcagggcag caggcatagc gaggcctgct 240

gggagtcagc cccgggctat ggcgataggc aatccggtag gcagtccggt agatggttcg 300

gtaggtgcta caggctctgt gtccatcaca agtggtgagg taaggctggt atacacgctg 360

cacaaaggtc tctgagatgg agcctccgga aacccccacg gtacacactc tacggctggg 420

tctgtagaca tgctcagtag tac 443

<210> 395

<211> 407

<212>  DNA

<213>  Rattus sp.

<400>  395
caattcaaag caaggtttaa tctgtgcaca gaacagccaa tctggcggcc atctccccgt      60

aaagtttgag ttctggaact gaagggggtg gggttttgca agcaggaaga acaaggcagt     120

taaggaaatc ttctcagaac atctggtaca gaacattctt tggttatggg atggggtaca     180

gctaaattct gagaagcaga cattggaacg taagttttac agtaaacaga gcctcgaaac     240

gactcctggc cttcaaatgg acttgaactg gtttctgact catctgtctt atgtgttcct     300

gtcacataag cctcttggaa gctaacattt ctggctattg taatggctac tagtggcaag     360

cattttagca ttctttattt gtccgtagac aagccttgtg aagcgct                   407

<210>  396

<211>  555

<212>  DNA

<213>  Rattus sp.

<400>  396
aaatcagaac aaatgaagta gcagcaaaag aaccattcgg caatctggct tcactggtcc      60

tcaaacaagg agggaatgca gccagagata catctctcag cctcagcctc tcagaatctc     120

acttgggccc taaagtgcat ctgcttggtg gcattgttct tcatcccggt cttcagcatc     180

catttcgact tcatcctctg tacatactgc atgtcccgtt cacgcatgag agctgctcct     240

aaaatcatag aagtctgcaa actccagcgc agcatcacca tctgtaaaga gcttacagtg     300

gcttttgtca ttcatgtgtg cctgcacagc ttctgtggaa tagaaggact tccctttctc     360

attgcaccac aagcagattt tgccaacacc aactttctct cccaaatatt taattaatcc     420

tttcaaatct gaaagatact ctatatcagg aataaaaaag ctgtggactt tagtcatatg     480

agccacattc ttcacaaggg agcttgaatg atgggaacaa cataagcagt ctgttattgg     540

gatggcacct aaggg                                                      555

<210>  397

<211>  672

<212>  DNA

<213>  Rattus sp.

<400>  397
ataatcttta ttgagcttct gttacagaag gggtgtgaca tttgccctta tggtgagtta      60

gtctggatgc gtgttctcag cccgaatgag aagtaaaatc ttaacagagc acagaataga     120

aaggaccttg gggatcactt agcccagccc attattttca tggaggaaat gaaaccaaag 180

aaaggagaac gatctagcga aggtctcaca gcaagggagg ctggtccctg tcaatctcca 240

ttcctaacca cagtactctg tgctaagctg tttccccaag cctttgggtt tgctggcttg 300

ctttatgtgg tcccactggt taaacaacca aactatcagg tacctaacat gacggtaggg 360

acttttactt cagctttaaa atcagcatat cgaaatgatt cctgagcctt tagctacttc 420

attattgaga atgcagtcca cagtaaatca tggctatcta cggtagtcat cttcaaatgt 480

ttgcacagac accaaaagca ctaatgacac ttgtacgaaa gcatccatgc gattccagga 540

cgcttctcat ggaatacagc ggcaccatgg tggtgaccag aagaggcttc agtctctcaa 600

agtgcggtca tgagtttgtc ccggtatgtg acactgtacg cccctcctaa gttcctctgg 660

atctggtcgt gg 672

<210> 398

<211> 531

<212> DNA

<213> Rattus sp.

<400> 398
atctaatttc atttttgtta gctcgtgctc atctctgtag aatttccccc aagaagctga 60

agaaacttcc ggggcccacg ttctctgagg gaagacttcc tgtttctgtg gctttggcga 120

gtcctgccat ggatgctcca cttcctgtga agatcaccaa acccgacatt tcctttctgg 180

attcatgtat gagttcttgc ggcaatgaaa ggcatccgca aactcagcag agttctgcaa 240

agtcccaatg atcctgaaat tgccaggact gtgtacatct gttttaatgg aattgactgc 300

atactctggc cggtaggttc cacaccacac ctgggcaaag ttcaagaaga atagttgttt 360

gtgattgagg tcaagtccag ggagtaattt ttcttcacca ttctttttaa cataattctg 420

ataggctctg tatgcttggc caatccctcc attatcagca atattttctc ctagtgtgtt 480

aattccattg agatgctgtc cacctgctaa gtcccatgta aagtttccat c 531

<210> 399

<211> 480

<212> DNA

<213> Rattus norvegicus (Norway rat)

<400> 399
   tttttttttt tttttttgtt tgtttttgat tttttttaat tcacaccgaa gaagttgggg 60
   gtgggggcag ggagggtgat ttctttcagc cgcgaggtta accgagtcaa cagctgactc 120
   tgctgggctg ctgtttgcac acgaagtccg tcataaaatc aaactcattt tggcccagcc 180
   agagttctgg cagctccttg atgcggtcca aacccatctc tatcactaag gacataagca 240
   cttcctcgtc gatgaaatca gtgtctatga cattgggcgg cagcattgcc gcggggacgt 300
   gagccaccga ggcgggcatg gtgctgccac cgccactgcc gcccgcgctg ctgccacccg 360
   cgccgcccga ggtgccgccg gagccgccgg gggtgccgct gccacccgcg ccgccagggg 420
   tgctgctgcc tccactgtgc ttggggttgc aatctcggaa gtgctggttt gtcccgttca 480

<210> 400

<211> 309

<212> DNA

<213> Rattus norvegicus (Norway rat)

<400> 400
tttttttttt tttttttgga gtttttcagt ggtcttgatg atgtcataag aacatttgta 60

aaagggaagg cttttagaag agggggtatg ggaagggtgc tggctttaaa gcagaggaat 120

attccagtgc tgagaagggt gatctgtact gaagagagga ggagaggact ctctgaagca 180

ctcttctgaa gaaggctagt cacaccacca actcagcttt ctgccagtga aactcatttc 240

agatttcgga gctccacaac ggaattagtg gagcttcttg agatacaggt agatcccatt 300

tttggactt 309

<210> 401

<211> 464

<212> DNA

<213> Rattus norvegicus (Norway rat)

<400> 401
cggccgcaag ctagcgatcg cattgtgtgc caggcgcagg acgtgcaggc ccagcaggcc 60

tgggaaggtg tcctccatga ggccagccac gcggttgtgc gacaggtcca gccaacgcag 120

ggccttcatg cccagaaagg cacgaggggc cacggctgta atgaggttcc ggtccaggta 180

cagcttctgc agcctgggca aatgtacaaa gacgttagct ttgacgcttc ggagtgcgtt 240

cctgctcaga tccagctccc gcagctcgcc caagccacag aagagcgcag gctgcaggta 300

agtcagtttg ttgccagcca gcaccagctc gtggaggttg cccagtccct ggaacactgt 360

gtcaggcagg accactagac tgttccaacc caagttgagg tcccaaaggt gactgaggcc 420

ctggaacagc ccttcctcca gccggcccaa gaggttgctg ctca 464

<210> 402

<211> 378

<212>  DNA

<213>  Rattus norvegicus (Norway rat)

<400>  402
tttttttttt tttttttccc ccttaaatag ctgtttattg gcagtgggct tgataacttg     60

ggggaaggga cggccatcat ctgccttctg gtatagtgtc tcgtggaaaa ggatcacccc    120

tccaatgcag ggattcacac ggtcatcggc agtcagcagc agttggcggt agaagcgcct    180

gttctcctcg gtgttctcgg tgccaatgga ctgcaggcgc ttggcaatgc ttccagtgga    240

ctcgtctgca gccaggatgc ccttgcccgg agctacaatt cggtgagcga tgtcagccag    300

ctccttcttc tgctccgggg tcagtgctgg gtatgggtgg ggcatggtgc cggtggcagc    360

gctttcctgg cacaggac                                                  378

<210>  403

<211>  406

<212>  DNA

<213>  Rattus norvegicus (Norway rat)

<400>  403
tttttttttt tttttttact attcaagttt gtttatttat tagcttatta agccatctta     60

ctgatttgta ctgaatagtg gagaaagtat acactggaca taatatgatt ttgtgaattg    120

taaagtgatg tcagtaataa cagagtcggt gtcacagtcc ttggcattta cagtttgctt    180

tctgatcttc ctttggctgg attgaggctt gcagacagac tcctgtcctt gatgtctatc    240

ttctcatctt gatcagagtt ccatgcagaa gtttagagag gttccgtcca tcttttgctc    300

atagatttca tcaaacctct cattctgggc cacagtaaag tggtttttcc aatcacccac    360

aattcctttt ctcatgaaag gggaaatgga ctggtccatg atagtc                   406

<210>  404

<211>  539

<212>  DNA

<213>  Rattus sp.

<400>  404
aaaaaaaaaa aataacatgt ataaatagct cttcacttta aaaatacagt tccccaggtt     60

gaggtatacg gtggcctgtt gtcagggcag catgagaaca gtagagtgcc agggcggggc    120

ggctcctctc tagctggaga cagtcatcac gtagttcttc gatggactgc tcctgcccag    180

catcatctgg ttcttgcagg tgaggagccc gtaggaggtg gccaccggcg cctcctcata    240

cagcacgcag atggatccat cttccccgat gcggtaggac acttcgtagg gatcgaccca    300

cagggtcagc tcgctgggca ggagctggtg cagctggggc tggctgagtc cgatctggct 360

ggccaccttg ctgatgatgg ggtccatctt gtggttgatg cggatacagc gatagccgga 420

gcccttggat ggcttttctg gaaaccagtg gtgtttgtaa tgatcggtca gtgcgtcctg 480

gagcgcccta ctgaaaacct tgagtctctg ctcgctcagg cagccccgag tcctcagga 539

<210> 405

<211> 542

<212> DNA

<213> Rattus sp.

<400> 405
cagcctcatc atcactgact tccttgtcac gttccttctc cacaaagaga gtaatggggt 60

agccaataaa ctgagaatgt ttcttcacaa tttcttttat tctcctttcc tccaaatact 120

cagtttggtc ttcttttaga tgcaagataa cctttgttcc acgacccatt ggttcacctg 180

tgtctgtcct cacagtgaag gatcctccag ctgaggactc ccaggcgtac tgctcgtcat 240

cattatgctt ggtgatgaca gtcactttct cagcaaccaa atacgcagag taaaaaccaa 300

caccaaactg gccaatcata gagatatctg caccagcctg caaagcctcc atgaaggctt 360

tggtgcctga cttggcaata gtgccaaggt tattgatcaa gtcagccttg gtcattccaa 420

tgccagtatc cacaatagtg agggttcggt cttgcttgtt gggaatgaga ttaatgtgca 480

gctccttccc cgagtccagt ttactagggt cggtcaagct ctcgtatctg atcttatcca 540

ga 542

<210> 406

<211> 561

<212> DNA

<213> Rattus sp.

<400> 406
acagaaatcc acagactctg gggctgggta aatgattatc cctgccacat tcatcattca 60

tgcagctccc aggaagaatg actgctctat attggacata aatgtactga tggagtaatt 120

gaataagaaa accattcaga agttgaagga aaacagttct tcacagtttg agtgcacaca 180

cgtctgcgtg tatttcacta gaaaggacac tctccataag gaaagattac ttttgcgtaa 240

gagagtttga cacgagggtt tctacgtgac gctgtttgac aaggatatga cagctatttc 300

atgacaatgt ttggatactc cagcgtctaa gaagcttgtt ttgttttatt gaacattaag 360

aaaatatccg aggaagtgat cgagattgag gttttaactc aactagatgg catcccagca 420

attcctcatg agtcatgtct atatgaatta acacagaagc cttagaaaac atgttaccct 480

cccaataagt acggtcaaca tttagtcaaa atacttcatt gttgcagagc tttctgaagc 540

tggctttgtg ggtacagtgt a 561

<210> 407

<211> 477

<212> DNA

<213> Rattus sp.

<400> 407
gatacgaact attataaata tgcacaagca aaggtcatct ttttctagga gtcgtcagaa 60

tccaagttgg aaggagacac cttttgtaaa accttccatg ggtccctgct ttgagataaa 120

catctaattg ctgaagtctt tattattatt ataaacgatc acagcgcatg ctacttgata 180

tggctctcaa ctcaagcgtc tgctttccca gtgggctgtc cctctcccct tgcaacaccc 240

tcttcatcca gccttaagaa acactgcacc atcttctggt caacagtctg gacttgtggg 300

ttgctgggga gagagtatga gccaatgata gcatgcaagg tgtctattcc tacaaccaga 360

cgggagggac tacaggctct caccccagca tacagacact cagctgtcat ttcacctagc 420

tctcattatc tatcgataat gcagcagaga ggttgggggc tacttctcaa tggttta 477

<210> 408

<211> 535

<212> DNA

<213> Rattus sp.

<400> 408
cttgcggccg catgtgtttt tttttttttt tttttttttt ttgtgcccca tggtagtggg 60

aaccaaacca atcacaatgt tttattggaa catattccat catcacagga tagcatttat 120

taaacagtgg cggatttctc tagctgctac atttattctc attcctcata cataccttga 180

ggtgcatttg attccaggag agccatttgg gttttcttta gctaaataat aaatgtaccc 240

gtctcagtct tttggactga gtcgttctga aggctctcgt gtggacagca gtgtgtgcag 300

tcgtgcttac agcccgtgcc tgctccacat ggtaccagtc ttaccagtgc ttgagagctc 360

agacacaccc tgctgcatga agttggaggt ctcgggaggg ttttagattt tgtgacggga 420

accggaaagg ctcgtcagag tgtggctgtg tcatggtgag caccacgtgg ctgtagaggc 480

ctgacatgag gtaatgcact gagcacacaa cgccactgct gctgtctgtg gctgt 535

<210> 409

<211> 425

<212>  DNA

<213>  Rattus norvegicus (Norway rat)

<400>  409
ttttttttta ttgttttttt ttaacgtcat taacttgtgt tatttgcttt cctttaaaac     60

acaaataaat caaagccatt aacctgaact tgaggtatat acacaataca tattcaactt    120

taagtaaaat actttccata atattattac atttttaaaa actggaaaca gtatccgtgc    180

tgatccgtat ttgattcaat gtaatgaaaa gggaacattc acttggggca aggtgggctc    240

gtcgaggaaa tagagctcca agttcaaatg tcttcctctc ccttgtattc attagaactc    300

tcccatgtcc ggagaggatt catcatgaga aaataccagt cacaggtttg gaaggaatga    360

gagagcacac gaggtggtga catttcatcc gttttcaaat aaatggtctt aattttcttc    420

atgta                                                                425

<210>  410

<211>  474

<212>  DNA

<213>  Rattus norvegicus (Norway rat)

<400>  410
tttttttttt tttttttctc atagagaatt ttaatctgaa acctttaaca gcagagattt     60

gggccttcat ttgaagagtc ttcaaaagcc tcttacagct gtaacagtta gggtttctat    120

tgccacaaaa caccatgagc aaaaagcaag ttgggagggt gggggtgggt tatttggctt    180

acacttccat actactgttc attatcaaag aaagtcagga cgggaactca actgaggttc    240

ccttttctct gatgacccta gcgtgtgtca ggttgtcacc taagaccagc cagtacacat    300

actttagaga aaaaaggttt tattttgggg agggtgttaa gtcttgctat gtagcctgct    360

ttcttaggac ccaggatgac cagcctaggg gtggaaccag ccacagtggg ctgggccctc    420

tctcattgac cactaagcat ccttacagca tttcctcaac tgaggttccc cctc          474

<210>  411

<211>  408

<212>  DNA

<213>  Rattus norvegicus (Norway rat)

<400>  411
tttttttttt tttttttcag gatttgcaaa gttttatttt aactcatttt gcccataaat     60

gactaaaaaa agccagtgtg caggaggctg tctgctgtct gctgaaggca gcgccaggca    120

ccgggtctgt ctccgagaaa agaggctctg ctcagaaccc cccaggctgg gaaccagatg    180

ggagggaggt atgggatgga gaaacaagcc aggtacccca aggtctacca aactctgttg 240

ggctgacaac ttaaggtgct caaggcatca tggaggggaa gggcagtgtg tggggttttg 300

gaagggtcca cgtgctgacc caaggaactc ctggccagca agtgggggct ggcctgtcac 360

tgccagacac cctcctaaga gaacctctgg gtgtcactgt ctaatgta 408

<210> 412

<211> 414

<212> DNA

<213> Rattus norvegicus (Norway rat)

<400> 412
tttttttttt tttttttaat gtgctgtgta aagttagtct actctgaagc catcttggta 60

aacttcccca acagtgtgaa gttagaattc cttcagggtg gtgccaaatt ccatttggaa 120

tttatttatg gttgcttggg tggagaagcc attgtcttca aaaaccttgg tgttgctaaa 180

ctgccagtta ctgttgtaac ttaatgagtt tcaccattta aagggtcatc caagcaagat 240

cacaatttgg ttataaaatg gttgttggca caccctatgc aatatcaaaa ttgaataacg 300

gtctcagata aaataacaga tgggaatgaa gcttgtgtat catccattat catgtgtact 360

caataaacga tttaattctc ttgaaaaaaa aaaaataaaa aaaccctcgt gccg 414

<210> 413

<211> 365

<212> DNA

<213> Rattus norvegicus (Norway rat)

<400> 413
tttttttttt tttttttcca ggaaaactgc atttggcacg tcgtatgcaa ctcccttccc 60

aaacacgggc gttgtcagcc gaccgtagac ctcttccgca ttcaggtctt cgttcttact 120

gttgaagagc agggcggctg cgtcactccc caaaaggtaa gtgaaagtct tgcccaccat 180

ggtaaagcta aacacaggtc cgtacttctc atatgcattt tccaggaact caattggact 240

tttcccaaat gctatcgcgt ggccaaggaa cggaattgga gagtaaatat atggcggact 300

tttcgctcca gcgggcagct ggaccatgtg gcccactgcg aggcggaaca ggtagacaag 360

gctaa 365

<210> 414

<211> 493

<212> DNA

<213>  Rattus sp.

<400>  414
gccgctgtca tcctcctcat cagatcccac atcctcaatc ttaggcttct cctcatcctc      60

cttatcttcc tcctccttct cccctttctc ttcctctgcc tcatcatcac tgatctcctt     120

ctcgcgttcc ttctccagat agagggtgat ggggtagcct atgaactgtg agtgtttctt     180

caccacctcc ttgaccctcc tctcctctaa gtactctgtc tggtcttctt tgaggtgaag     240

gatcactttg gtaccccggc caatgggctc accgtggtct gcacggacag tgaaggatcc     300

accagcagaa gactcccagg catactgctc atcatcgttg tgcttcgtga tcacaaccac     360

tttctctgcc accaggtagg cagagtagaa gccgacacca aactgcccaa tcatggagat     420

gtctgcacca gcctggagag cctccatgaa cgcctttgta ccagacttag caatggttcc     480

caagttattt atg                                                        493

<210>  415

<211>  496

<212>  DNA

<213>  Rattus sp.

<400>  415
agcaaaactg gtaaaaacaa aattgtaatc gttgaacata gcgctctggc aatcaagacg      60

tttgaaaccg tcaatcttct ggggcgaaga aagcactgtg cgacacttag aactctgatt     120

aacagacaag gtggtcacaa attttcctgg cttgaagact tccacaactt tcctgatcag     180

gtcatcatag gaggtctgac ttaggtttgt ttcaaagcta acataagaaa attctggttc     240

tggagtgatg tgaatattcc aatatgttcc atccgatttc attccattca ttgagtagcc     300

acaaggattg aacagtgtgg catcaatgac agaacctggt atcaggtcac gaattccact     360

ctcacgagtg acatcctttg cagtaacacc atctttcatg tagaactggt ccataactgc     420

tgggtcaagc tcactcatca gaatttccac ggtttgatct ggctgattga ttactcggct     480

ctctgggagc ctcgtg                                                     496

<210>  416

<211>  408

<212>  DNA

<213>  Rattus sp.

<400>  416
gggcggagtc tccctgacac ctggccttgg agggacgcgg ctagtgcctg ctccaggcct      60

ctcggccgcg cagtcagcct tagtgtgcgg aatcaggttc gagcttcgcc ttgtcctctt     120

ctgcatgcgt tactgaacag gaccagttgc cagagccctt gacagagaag gctttgagag 180

aagccagctc tgccatcgac accttaggcg aagccttggt ggctggggcc tattctaaga 240

tgtggtcctg ccgagaagat gcactgctgg cattgtacaa gaggctgatg gagatgcctg 300

ttggaaccca gaaggaagat ttgaaaaaca tgctcagagc atctgtcttt ctcatcagaa 360

gagccataaa ggacattgta acctcagtct ttcaggcttc actgaaac 408

<210> 417

<211> 601

<212> DNA

<213> Rattus sp.

<400> 417
acatctccaa cagatttatt ttaaaggaat gacctaaggg agaaaaaaat gggggtacag 60

aagtatggac tagaaaataa atacaaatgt aagctgtttt actaatttta taaccacaaa 120

cgtgtacaga gaatggtgcc ctgtacaaaa cacaacagat tcaaacgagg tgttccctta 180

gcaaggctga agattcagtc tctggtattt ggaatttagg atgcagtcct tgtttttgga 240

tggatcactg ggtgtttggc acagtccatg ctattaacca gatttgaaca ggagaatggc 300

cacctgaccc aggtagaagt agatgaagtg tttggtctcg tgtgtcacgt agctaccgaa 360

gttccggccc acgatgcagt gccaggtggg gttgtacttc ttgtcaaact ccttcttgat 420

atgggccgcg atatccttct ctatgttgta cttctccaac gcctgagtag cgcactccac 480

cgagtcctgt tgcatctctt ccgacatgtc tgcatttttg atcaccgcct tccggtcgca 540

catggttacc gtggagcagg ggccagccgg ctgctgcgag ctcctaggga aggtgctggc 600

g 601

<210> 418

<211> 495

<212> DNA

<213> Rattus sp.

<220>

<221> misc_feature

<222> (308)..(308)

<223> n

<400> 418
cactcacaca caatattgta tgagacatta ctagttttca tatgcatcca taggattaat 60

actcatatgg agtataacat ggaaaagtgc agaactaaag aaataactct aacaacaaaa 120

gaagcacacg tttcttaaaa tttaaaaata ttgcacatag taaggttgca cagaaattac 180

tggctgcttt tacaaaaaga atgaggtatt agtcaatctc tagataaaga taagtgcaac 240

agagtaacta cacacacaca caccaaaaac aaaaaacaaa ttcatacgaa gacctagaaa 300

gtcaacanac aaaaatattc aaaaagaagt ttacaaagct atgattttga tcatacaaaa 360

tcattagccc attactaaaa cacaatttct tggcaatagt ttataaattt taaactttct 420

tcagcttaac tacgtgcaat acaatcatcc ccatcacacc aattctcctg tagaagagga 480

atctttctgt gatag 495

<210> 419

<211> 613

<212> DNA

<213> Rattus sp.

<400> 419
gaggttacac agtggccttg atctttattt ttctttacct ggaattctac aaattctgaa 60

attgatcaat agatttatct tgtttaataa acagcaccag tcgtgcccac tgatacaatt 120

caaacctgac tttctcagtt ctcaagttta ttatgaaaac actgcggtga cttgtgcagt 180

acatcatctt acaggaggga gacagttctg ttcaaaacaa ctcaccaggt tttctgacag 240

taacaaagaa caacgcgggg ccgagatttg agaaggagaa aactgagtgc agagaaatca 300

tacaattaaa ttaatagtat ccctgaaaga acacaacaag aggggaggcc ttggtactca 360

tttaaaaatg ttcattaagc tccaatgatt gtttgctatc ctcatctaca gtcatgctga 420

gtaagctaca gctaagtgga agtcaactgg actcacaggt gttaatgtta catcctagat 480

ctgcagtctc tcaagaaagc aaaagtaact acaaatagcg ctatgccaga aactggattc 540

ttgaccaaca atgtcgcgtc agcatgcaat gaactggttc attctaaagt ggtcacggct 600

gttgatgaca aga 613

<210> 420

<211> 614

<212> DNA

<213> Rattus sp.

<400> 420
cttgcggcca cgagattttt cttttatttt tttttttttt tttttttttt tttttttttt 60

tttaatttta taacattgtt ttaatcaagt aatgtagctg tttgaaacag gaaacttttt 120

aagacacctc aatagagcca tgaaggatta ctcttaccag gttaacctcc atgaataaaa 180

acccagattg tgtgaaattt gaatcccact ggtttggcac cagaatgaac agaagcccaa 240

gaccttccta agtgtctaag acctgaccat ttccctttaa agcaacagct ctaagacaaa 300

atcttgccaa cctgaggccg aaatgtgctg atctacttga gaatctcaaa aagtaatgac 360

atatggatgg gctgcccatg caacaaagag tctgaaaccc aggagacaag tgactttcca 420

agtcccactt ctttggtgtg acacagcaca ttcccctgtt accattccag gtatatggct 480

cctgttccac gcccagcctc ctatcaaggt tttaatttta ccaaatgttt ggtatgattt 540

taacgcatac tttttacatt aatcaaagtc ttataatctg tgagatggat gacacaaaga 600

ataagatcca aata 614

<210> 421

<211> 417

<212> DNA

<213> Rattus sp.

<400> 421
ctttacacag atttaagcca gtaattttta gcaaaatgat acaaaactgt cttaaccaag 60

tagaagactg gtagctgcgg tgggatcatc agggagcgca ggtccgccac cactggtggg 120

agctgagagg ctcttcacag ggagtcgggt tctctctgca atggtttccc agcctctgct 180

cttcgtccca gccccatgac aactgagctc acatggggcc atggtggcag cccctggcga 240

tggcagctct tggagcctgt ccatttagtt tgtgttttga ttccccatgt gcatcgctgg 300

gtgagtggcc tggagagctc ccggcgttaa catttcgggt ttagaccggg gggcgtgtca 360

ctagtaaagc cattggtaac agagtagatc agccatgcat tgtctgccct tcacagc 417

<210> 422

<211> 448

<212> DNA

<213> Rattus sp.

<400> 422
aaaccaattt gtacaagttt atagtttact ttgtttccag gttctcaaac ctttcaggat 60

ctgaaaagtg agatactgcg tctaagggaa tgtttttgtt tttttgtttt ttttttgttt 120

gtttttgatt ttttttaatt cacaccgaag aagttggggg tgggggcagg gagggtgatt 180

tctttcagcc gcgaggttaa ccgagtcaac agctgactct gctgggctgc tgtttgcaca 240

cgaagtccgt cataaaatca aactcatttt ggcccagcca gagttctggc agctccttga 300

tgcggtccaa acccatctct atcactaagg acataagcac ttcctcgtcg atgaaatcag 360

tgtctatgac attgggcggc agcattgccg cggggacgtg agccaccgag gcgggcatgg 420

tgctgccacc gccactgccg cccgcgct                                          448

<210>  423

<211>  474

<212>  DNA

<213>  Rattus sp.


<400>  423
atgaccaagc atcacaaaca cttttatttg tggttatcag ttatttttac agaataaaga      60

actcattaat ttgtaacact gtacattaaa ttaaaatata acccatccct acatcaaaaa     120

ttatctaagt tgaccaagat aaaaaaagtc tctaaaagct tatatacatt agaagtagca     180

aaaataataa taaaggaaga aattagaaaa cagacatcaa agtcagacat ctagaagaat     240

tctccaacat ctgctctctt atctcggcat ttgcttcggg cttttgttcg agctttgaaa     300

gctgcagagt tatataaatg cctttcaaaa cgaggaatct tcatggtttt aagtgttgca     360

gcatcgagca tcacaggggg tcccagctca aatacattgc gaaggaattc atttgtctgc     420

aagtggtact gcatccctga tccaagagcc tctttaaacg tgtcataagt gtgc           474

<210>  424

<211>  510

<212>  DNA

<213>  Rattus norvegicus (Norway rat)


<400>  424
tttttttttt tttttttcag gttttaaaca tgtttagagt aaactcgcat cttttacaga      60

caacattctg tgacagtgcc atgtgaatgg ctcctgcatt ggtgtttttt ctatctggct     120

ggatcctgct gagggcatgg agccagatgc cgtcagctac agtgtctgct gtcttgtgag     180

gcactgaagg tgtgtactca gccgatgtca ccgcatctct cagtgatgca gcacggctgg     240

ggggtccctg ctcctgtgcc atctctcctc agctttgcag tctgtaatta acttgttctt     300

ccggagactg agtctctaaa aaccacaagg ttgcttcacc ttgcttctag aactgggacc     360

aggcttcaga tgtgacagca agagtaaaag gattcttcaa ggcctctgaa atgagccctt     420

ggcatacagg tctgaaagtc ccattgttgg cccctggctg gctggagtgg aaaggctgct     480

caccctgcac aagatgtgct ccctgcatgt                                      510

<210>  425

<211>  388

<212>  DNA

<213>  Rattus norvegicus (Norway rat)

<400>  425
tttttttttt tttttttgag actgggtacc cgcattgttc tgaggaggat ctgttcttta      60

gtgtactgga aacacgaggt taccagcagg cacaacaggg accctttgga acccttacaa     120

accagaaggg tcacataaat gtactgcatg tgaggtggtg agggaaaggg acaaggggaa     180

ggggttaaga agagaaatct ctggtccact gtgactttct tcagcctgga cagttgctct     240

taaaggggta gctttcttcc agtgtactgt actcttcaga gcagcagacg gctgcagggt     300

gtgcagccag cagcagacgt atcagaaaga gtaagtccta accccttggt tagaaaaaca     360

ggagacagaa gttttaacac ccacctta                                        388

<210>  426

<211>  103

<212>  DNA

<213>  Rattus norvegicus (rat)

<400>  426
agagaaggac ttcctctcaa gcgacctcat ctttctgggc tcggacactc acccacctct      60

gttacctgat cgccctcgag cccacgtgaa gaaaaccatt ctg                       103

<210>  427

<211>  341

<212>  DNA

<213>  Rattus norvegicus (Norway rat)

<400>  427
cggccgcaaa ggtttttttt tacctactaa gaatggtttg attttgtttt aaattgggct      60

cccttgtatg acacatagat attttacaac cagaatttgt tttgagaaaa gaaaaaaaaa     120

aaaaaaagaa aaattcaact atcttacagt tacatacata tatatatata catatatata     180

tatatgtatg tatatatatg atgcttataa aagacattta ggagaggtta ttatgaaatt     240

atgccataag gaaaacttaa tttagcctgt acaaatacct ggctatgaca agattaaagg     300

aataagtaga actcctcatg ttttacattc tgacactttc a                         341

<210>  428

<211>  545

<212>  DNA

<213>  Rattus norvegicus (Norway rat)

<400> 428
tttttttttt tttttttgtt attccatacc agtttatttt agataatgtg cttcaaaaac 60

gttttatcat aaataacctt tcacatcata aaataactta gtacgattta taataaaact 120

tctgagaatt taacatctca tcaaaataca ttaaactgtg gcttgaaaat atgattaacc 180

cttttcactt ttcttttatt tgagggcatg aagaccaaaa aaaataccca aaatattgct 240

acacatttaa agtttcaaca aagcaatatt tggtttactt tactgtaacc cggccatcct 300

aaattaatag ttttctttct tgcacagttc tttgtagttt ttttgtccct tttgacttgg 360

atttctgttt ccacacagct tcctccttca gtctcacctc tctctccttc tgcaaagtca 420

tctcctagcc ctcctgtagt tgaactcagc cccttgacat cagtttctgt aatccttgca 480

tcagcgtggg gagtgactgc ttgagtaact acaaaggtat ttcaaaactt cagtgcaatt 540

tggtt 545

<210> 429

<211> 130

<212> DNA

<213> Rattus norvegicus (rat)


<400> 429
accaaattgc actgaagttt tgaaatacct ttgtagttac tcaagcagtc actccccacg 60

ctgatgcaag gattacagaa actgatgtca aggggctgag ttcaactaca ggagggctag 120

gagatgactt 130

<210> 430

<211> 602

<212> DNA

<213> Rattus sp.


<400> 430
gatggctaac taaggacttt atttcaaaca aaaattaaaa attaaaaaaa aaaaaaaatg 60

agagctattt ccctgggtgt gaggaaagga tcagggtgag aaatttcccc atggctaagc 120

caataccctc aatatattag agggatcgtg tcagttccct tccttacccc catgatgggg 180

aacctaagca agtgcaggac ccacctgtcg ggaatgtggg ctaggggtca gcccccaaat 240

ggggacagag ttgggcagca gcatggccaa acataggatt aagtcctaag taccaagaac 300

ccactcctcc catcttgctg ggacccaggg aaagtaaaag atggttttgt agaaaacctc 360

tgagatcccc gaggggaaga aggttccagt cctcctcatg ttcatcatct ctgttctctg 420

gagggcagtg agtcctggga tctgtcaggt ggcaatactg acacagcaag aggtggggaa 480

gccccagcaa gtctggaccc catggttggc tctcctaccc tttggccagc tctcatggtt 540

accacatgat cctttgtgtt ttttgccttc catttccctt gggtaggtgg ttaggtaggt 600

gg 602

<210> 431

<211> 424

<212> DNA

<213> Rattus sp.

<400> 431
ggtttttttt tggtaattag cctttttatt gggttttttt tttttttaaa ttgaagggcc 60

cttactggtc ctgcttccat gaatggccaa aatcaaggga aaggggaggg caaccagctg 120

gtgcagggag ggtcatttcc acagttcatt tacccccaga actaaacaga caagcccaga 180

gtcactattg gggttaaaag ttggcaccat gggatgggac aaatgaggag taggggtgtc 240

gttaaaaaaa aaaattcagg gtccccccaa actgggtgcc taggacggga cttggtctgg 300

ttcaacccaa gaggaatcag aagatcaaaa gcaatttggg aaggccaaaa ccgtcaggga 360

tggagggaaa aggacggtcc agggggtggg aaggattttt ggcaactggg gtgaaaggaa 420

aacc 424

<210> 432

<211> 424

<212> DNA

<213> Rattus sp.

<400> 432
ggtttttttt tggtaattag cctttttatt gggttttttt tttttttaaa ttgaagggcc 60

cttactggtc ctgcttccat gaatggccaa aatcaaggga aaggggaggg caaccagctg 120

gtgcagggag ggtcatttcc acagttcatt tacccccaga actaaacaga caagcccaga 180

gtcactattg gggttaaaag ttggcaccat gggatgggac aaatgaggag taggggtgtc 240

gttaaaaaaa aaaattcagg gtccccccaa actgggtgcc taggacggga cttggtctgg 300

ttcaacccaa gaggaatcag aagatcaaaa gcaatttggg aaggccaaaa ccgtcaggga 360

tggagggaaa aggacggtcc agggggtggg aaggattttt ggcaactggg gtgaaaggaa 420

aacc 424

<210> 433

<211> 448

<212>  DNA

<213>  Rattus norvegicus (Norway rat)

<220>

<221>  misc_feature

<222>  (401)..(401)

<223>  n

<400>  433
tttttttttt tttttttctg tgaaaagaca aaaggaccaa actttatttc tctacgcagc      60

cttggctggc ctggaactca ctatgtagaa caggttggcc ttgagctcac agagatcctc     120

ctgttgctgc ctttagagtg gctacctatt ggcaacaagc gccctcagca gagcactgat     180

gagtcctcag agctcgtcgg acgtgatgtt caccttgggt aggttacatt ctttactagt     240

ttgacagctc tgaagaatgt cctggtagtg gttcttcaga tcctcataca aggcaacagt     300

tttctgcgag tgagctaagg gtaacacctt ttcattcagc agcatttgta tctggaattt     360

ttcttgaggg gtctgtgcgt cttcacaatg gtaaagcaca natattaggt ttgaagcata     420

tggtacgatg tgaccacttc ggaactcc                                        448

<210>  434

<211>  590

<212>  DNA

<213>  Rattus norvegicus (Norway rat)

<220>

<221>  misc_feature

<222>  (135)..(135)

<223>  n

<220>

<221>  misc_feature

<222>  (163)..(163)

<223>  n

<400>  434
tttttttttt tttttttctc tgtgcacaca gctttattgg atatcgctgg agcgtcccca      60

agtggctctg attactggtg tgacaggagg aggtggtgcc tcactggaga tacagacaat     120

gatggctgac attanatgac tggggtgggg gcccagcacc tanggagaga gtattaccat 180

ctagtcacga acactctctg cactgccctg atggagagac aacagccaga ccccgtcaca 240

gcagcagagg gagcggagcg ctcttcagca ctggggagct agcctggagc tttgccattg 300

cctgctgttc caaggtccaa caggactgga gcaggatctt ccctcaagtc cagccagcct 360

gtggggtcac ctgcaaagtc ccagtgcctt caggatggtg tcttgccagg ggtggcccct 420

gtcttctttg atgctccctt cagcagcgca agcttcttag gcaggctgga gctggccttc 480

atcaccacat catgctcaat cttcttccgg attcctactt ccaggctctt cttcaacttt 540

tgctgctgca caacgcgcgc cttcttggga gcgatgactc gaccgccttt 590

<210> 435

<211> 652

<212> DNA

<213> Rattus sp.

<400> 435
ccacaacagg atttattcaa tgataacatt cagttattca gacatcatag aacaacataa 60

acataaacat cagtgttcat aaaatattta aaacttttga aaatttattc ccatgggaac 120

atccctggag ggaaaccggc cattttctcc tggaacactt tatcaaaggc ttcagcttgg 180

gctactgtga agtgattctt ccagtcatta gttgtgcctt ttctcatgaa agtaaaacca 240

gtaagaatca gttccttctc catgaggcta taattggaca tgttgttttc tttcacgact 300

tggaaggaac tatacttgag gaccaaattc agctcatctg gctctaattt tttccccagg 360

aagtcacata tcttctttat ggatcccatt gtatcctttt tcatgtcttc atagtacagt 420

accaagaagt tgtcccattc tctcatagac agccagccac ggatgtgctc aaaccatgat 480

ccatatgcaa catttccttt gaggaaccat tcaacgtaag ttcccagcga gtctggtttc 540

ttctccaggg cgatcttact ccagaaaaaa taagcagaaa caagaacatc tctgggaatt 600

tctgatgaga tatatcacct tggccttgga actgaagaga gacttggaga aa 652

<210> 436

<211> 629

<212> DNA

<213> Rattus sp.

<400> 436
agaaataccc aatttaataa agatggagaa aaaaataaag ccagatccca aggtccattc 60

cctcattcat cctcagagtc attcctggct ggagaatgag cttatgaggg aaaaggtcct 120

atacaacagg tcgagcaagc gcaggaactg gcggtctctg tagtcaaagc gctctccaaa 180

gacaatggag cagattatgt tgcctgtgac gcactggaag aggaacgttg ggtccaggga 240

gactccctga tgattcttca gttcctccac taaacatttg gcctcctcct ttatctgatc 300

ctccacactc tgctttccta tcccaaagtc tctcatggtg gctacaaata atcgccgaag 360

gatcttccac ctttcttcat taacaaagga cacaccgtat tcctgcataa ttggctgaag 420

cacagcaatt attccccggc cagagaaagc ctcagcatgg tccaccagag cctcccttat 480

ggtctgtgtc ccacacagca tgaccacagg cctaggtcca aagtatactg tgaacacatc 540

tccgtgtttt tcttgaagct gaatgaaaga cttacgaaag cctcctctgt ccatctgcaa 600

gaggtttccc aagaggggta agggacggg 629

<210> 437

<211> 722

<212> DNA

<213> Rattus sp.

<400> 437
gttgactcac agattttatt tacatttgtc tacagtatca tttccttatg aaatgaacta 60

gtacagctca attaaaccaa atcaaatcat aatcatctga actgtttgta aactcctatt 120

agctaaattt ataatcttgc atttgcctag tacagctcaa gttttaaata cagaaagcac 180

aaaaacaaac caaggctaac acgtcggatc tagaagatct gggcaactca gaaagtcgac 240

tttcgagaag tctgaggcac aattacaaga gagtaagagt tcgtgtgcaa cctacagtaa 300

atgcagcaag gaaaataaga caaaaaacac aggaaagctt gtaaaaatat taccaaaata 360

tttctacgta cgatattttg ttttcatctt tagatcagtt gagaagaaaa acactcactt 420

taaaataaaa acatttcgtt tgatctgtct actaaacatc tcagaaggct ttacttatat 480

ttaaactgca gcagacaaat actaaaggag aaacaggaag taattccctc acaaggagcc 540

ccagcataac tgttgtccct tatatactta ccttgctttt gctgctgaaa agactgttaa 600

gagaaacctt ataactttta aaccagagaa caaaatggat ctcaagtgac cagattttaa 660

tttgagaaat aaaactctga aagcatttta agccatatgt ttttttttcc aggtgagaaa 720

aa 722

<210> 438

<211> 663

<212> DNA

<213> Rattus sp.

<400> 438
gtaattccac accagtttat ttcttaggtg tgatcgtgac acagtgcctt ctgtgacaag 60

gtggggcttt caggagggca ctgaaccccc tcctcagagc agctcacctc agaaaacacc 120

cgaggcagat ttgattacga atacccccca ggccaccaat atttacatgg gaaagtggaa 180

ctgagtagag gaattatgac acaagaaagt ggggtgtcct gaagatgctg gggggcatga 240

aggcaccatc aaaagtctgg atctccttct cagcgcgggg ctctgccctc tagggccgtc 300

cttccgggta ggtggatcca gggagaggag tgtagctgga ggagtgagac ttgcgccatc 360

tgaagaagcc aagacagaag atgatgaagc ccaggcccag ggtggctgca gacacagaga 420

ccttcactgt ctggatgggg gacagcccgg gtgtccagtt cgcgcggatg ggctcagggg 480

tcccgctgtg ctgtaccacg caggtgtaga tgtcccagta ggaaggggtt aaggctaggt 540

aggagacggt ctggtatgtc cagtccccgt taggctgagc cgtcttctcc ttgttgctgt 600

gggaagttac aagctgcccg ttcttcatcc acacgacggt cacatccgct ggatagaaac 660

ccc 663

<210> 439

<211> 601

<212> DNA

<213> Rattus sp.

<400> 439
ataccaaaga aaagtcttta ttgtaaacaa ggtataaagt acaacaaaaa atattgtgca 60

aattgtaact cgtgattttt tttataattt ttaagaaaac tctttttttc aagggttcaa 120

gtttgcatgt cagaaaccta tgagatacag aaacggttta aatggaaagg tatggtgagt 180

ttttcactgt ggactttgaa taatttacct ttactaccaa gtgtgtcttc tatccacttt 240

acaacagaac agaaatactg ttagcaaaag tcactgcttt caggtgggga tacaactcgg 300

tgacagagct tacagaacac aagcaaggct ctggatcccc agaactgcag gtgtgaccac 360

attatgacac tttaaagtct cggagtttcc cggacaccca gacactgcag cggcaggaag 420

aacgtatctg caccatgaat atccttgtca agtcggaagt gaatgttcta gtaacaattt 480

cctacctatc tactcagaca tcctgacaaa ctcctataaa taaaaggaca cctgtccatc 540

tgtgatggtc actaacttcc tgatttgata gaaacagtgt acgtctgacg taagtgtcct 600

t 601

<210> 440

<211> 644

<212> DNA

<213> Rattus sp.

<400> 440
attatgaatg acattttatt cagtcatttt ctttacaact gaaactctgg gaattcaaag 60

ttaacatcct tgcctgtgag cttcttgtac acgccagaaa aagtttcgac cttatgctcc 120

acgttgttct gctgtgcttt gtctaaatga acttttatga gccggctgcc atccagtttc 180

acacggatcc tcttgcccac aatttcactt gggaagacca aatcctcaag gatggcgtcg 240

tgcactgctg tcagggtgcg gcttctgggg cgcttttgct tatttttcgt acggcttttt 300

cgggttggct tgggcagaat cctcctctga gcaatgaaga ctacgtgttt cccactgaac 360

tttttctcca attcacgaac tagccggact tggattttct ggaaagattt cagctgagga 420

actggtacaa aaattatgat ggcttttcga ccaccaccga cttcgatttc ctttgccgcg 480

gtgatgttga gttcccgcag ctgcgccttc agatccgagt tcatctccag ctcgagcagc 540

gcctgagaga tgccagactc gaactcgtcc ggcttctcgc cattgggctt cacaatcttg 600

gcgctcgagc tgaacatggc ttcgtcctta cggagcctcg tgcc 644

<210> 441

<211> 421

<212> DNA

<213> Rattus sp.


<400> 441
atctcttatt tacaaacact gggtaggaca cccaaacaaa caaacatgga ataacttaca 60

aaggcaggaa gctgtttatt atagacagta atcagctttc atcaaattaa aaaaaaatat 120

atgtacatac acagttgaga gaggcaggcc aggggagttc atccgcaatc tagcctggta 180

ctcacaagcc tccctcccct tcccagccct ccctgctttg tgttcttacg gagcactaca 240

gaagcaatct acagtctcta ttgcagtttg taagccccca cccacccccc ttaatactga 300

atgagatcga atgttaggtc catgcagttc ttggtcaatg ttaacgaaaa gtccatcgtt 360

ctggtcgcgc gggcacagcc cgttcgcaaa gcgtggcggt caacaggccg ctgctctctg 420

g 421

<210> 442

<211> 650

<212> DNA

<213> Rattus sp.


<400> 442
ttcaagcaca tgatttgtct tgatttaatg cctttttaag tgccctcaaa aactgagggg 60

aaaataaatt cactcaaaat tattttaaat tctttttaat cccctcaatt tagagtccaa 120

gggctgaagg acttataatc atgattcccc tttagatata aatttataaa ttgcacttgc 180

ctctgttaag tgtttctttt gtggggaaaa tattatatta atttttactg ttgcatgcag 240

aggtaaccct ttacccacat agaggcattt cttccataga gcctttgagt tcaaaccatt 300

ttttccctag ttttctttta ttttcctttt taagattttc aaactgggtt acacactgga 360

aaaggctggg ttaagggcca aaatttaata aatctgtacg gataactaaa ggctacagag 420

atttttatat atatatatat ataatttttt ttaactttta gaaatcagag tgcttataaa 480

atggctggct catggctctg tccccagccc ctctgacgcc gcctcctagc cttcgttgtt 540

gagataacca ggaatagtga ctccatgcgt aaacaacaag aatactaacc aagaaaacta 600

gcttatcatg caaatattaa ggcatctaga aagtcagtta aaatttattg 650

<210> 443

<211> 632

<212> DNA

<213> Rattus sp.

<400> 443
tccagaatca gaattaagca ttaagtgtcc aatattataa agctgtttat aacaggagtt 60

ttaagtagtg gtaacattta acccatgtaa aaatgtcaac agaatttaaa tcgtgacttg 120

gatcctgaac gattcaagta ccatggtctc actcagagac gggtttggtc agttttaaga 180

tgaactcctt cggatgtagt gtccgtaagt ccttattatc gctgtccatc aattcacgat 240

aggatagctg gctgcggtgg caagtccgca gtggttgttc cggtctttgg ctattttgat 300

gtagccatcc ataccccatt ctttacccca gctgtttttg acaagccagt atttatcctt 360

atttgaatct gttccttcat aaccatagcc aaccaccaga accccatggt cgaggtcctt 420

gctgctacag ttgggttcat agtagatacc tgaactatag aactggagag acggatggct 480

tgcatccatg gcaacagaaa taggccccac cgtcgctaca gccttcatga gggctttctc 540

ttgctgaggg atatccacaa accctgtgtc gttagccagt ccacagtctc cttgtgccga 600

attcttggcc tcgagggcca aattccctat ag 632

<210> 444

<211> 616

<212> DNA

<213> Rattus sp.

<400> 444
aagtcatgat attcctttat tagtgctagc tccttttaat ttttatcaga gctaaacaat 60

ttaatataaa aatgtcattt cttgttcata cagtatataa aaaagtatag tggtttggtt 120

agttttcaat agtttgcttt tagccagatg tcatataagt ctatgactgt aacaaatgag 180

aacagtataa ataagttctg tagtatttac acttacacag aaactagccc aaatggtgcc 240

caagaaatta acttgagagt taaaatgaaa ctgattcaac attgagactt taatgctttg 300

taaagtttca tattatttct acactagctt tggctataat tctgcatagt tacttataaa 360

gtgtttctgc atttcacatc acagtaggaa gttttagccg tacaaaacaa acactagctc 420

agaaaaggct ccatcctccc gaacctagtt tttctttgta tctggcttct tgctcttggg 480

aacaaggaac acgttgccat ctctggtctg ctgcagagag tactcactgg gagagtaagg 540

tttcccatcc tcatcacgta acatgctgaa gacttccaga tacaaagtgc tgagttttct 600

tttcagaaga tggagg 616

<210> 445

<211> 332

<212> DNA

<213> Rattus norvegicus (rat)

<400> 445
gaggatggag ccttttctga gctagtgttt gttttgtacg gctaaaactt cctactgtga 60

tgtgaaatgc agaaacactt tataagtaac tatgcagaat tatagccaaa gctagtgtag 120

aaataatatg aaactttaca aagcattaaa gtctcaatgt tgaatcagtt tcattttaac 180

tctcaagtta atttcttggg caccatttgg gctagtttct gtgtaagtgt aaatactaca 240

gaacttattt atactgttct catttgttac agtcatagac ttatatgaca tctggctaaa 300

agcaaactat tgaaaactaa ccaaaccact at 332

<210> 446

<211> 536

<212> DNA

<213> Rattus sp.

<400> 446
atgagtttaa ctttctcttt aaataagaca atcagggaag gggatcaagc ttgaaaaaca 60

gtaacactta ttttggcaag atagcaacca aaatctaaag tacaagctat tatatccaaa 120

ttaaaaggca ttaaagaata aaccataata ttagggaggg ggaacacatc aaacacaaga 180

tacagtatgt ctaaacatct tgaaattata atttaagggt aaaagttgaa aacttatgct 240

gttttagatt aagtgcacct ttttcctgtt tctcccaaat agaatcagag tttacgattt 300

aatcacacct caaagctttt ctctcagtac aaagcaacca ctaaatacac acaaaatact 360

tatttgtcat ttccctggct tatccaacgt gcaatgcaca tgactcaata gactacaatt 420

tggggtgagt gtacacaaac tgaaaaacca caactccaag tttcaaaatt tgctcttaaa 480

ccataggaca cacaggtcat ttttacttct catgaggatg tcacactgtc attaaa 536

<210> 447

<211> 578

<212> DNA

<213> Rattus sp.

<400> 447
agatgaagtc agatttattg ttcatcatca tgtcatacaa aaatctacaa ataatagatg 60

tgtacaggaa aagagcccaa aggaatagta aatggaggtg taacggcgat aatttaaact 120

tgctttcccc catatattag gatgacagcc atttcaaagc atagttgtga tgtcaaggca 180

gcttttgctc gcccaccaga gctggccgtt tagattgagg ccttcagtct cacagttcta 240

ctgactcttg acaaagcttt tgaggtcctc aagaaaggtg atatattcct ggtgctccag 300

ggctgtgctg agctccacca actcatctgc aaaagtgtta tccacccctc ggtccgcaag 360

gaaatccatt aggtggtcat acaaggccca gtccagggag tctgtgttga gtgtgtagtt 420

tgtatccctc cactcagagt caccagtggt ctgaaagctc acttccttaa tagagaaaat 480

gtcactctcg gcctcatctt cgtgtccgat ctcgtcctca ggatagtggc agtccagtac 540

aagggcctcg tgccgaattc ttggcctcga gggccaaa 578

<210> 448

<211> 603

<212> DNA

<213> Rattus sp.

<400> 448
cagagagtaa acggtgtcat catatcaact tggaaacagt tcagacaggg cccggctgtg 60

ggcctagggt aaatgtggct tttatttcct ctcagggaaa gaagtaaagg gtggctttcc 120

caggtacccc aacctaaggt aaggtgggtg tgctccagag gttggggcta gaattgccag 180

atcattccga cagactcctc tgtgtccact cgctggcgct tgatgcaggg agggtgtagg 240

tgagagtcat tcccctggag tagcagctca gtatcaacag aggcacaagg aggtatgtgc 300

tggtattcac aaaatggaag gcagagcagg tgccctgagt gaggagcagg actgggtggc 360

cgatccacac ccagtgtctg ccgggtacaa ggcctgactg ctgtggctct cctcccaagg 420

gccccagggg cccagaagca tcactgcgtc ctatggctgg tcccttaaat gtccatctca 480

aactgtgact cttcaccacc tgcccgctta tcttccgggc tgctgtgcag atggctctgg 540

ctggcctgca tgggaggctc atcgctggta gggctagtga cccctggaat ggttggcaag 600

tcc 603

<210> 449

<211> 613

<212> DNA

<213> Rattus sp.

<400> 449
attagataat gccatttatt atttcacaca gaagttagag accaaggtta caattattaa 60

ataccaccca cccctcaaaa gacagcccta cttggttaga ataaaaaaac aatcgataca 120

acaaaaaata ccattacact ggtagaactg gggaaataac aaaaacaaga cagaaacaca 180

agacagaaaa tctctgcaca ctgatatcaa agtggccatg acgctggggg aaagcagtca 240

tggtcagtca acatggacgc cgactaccaa gggcactggg ctcagaacag ccgcctctac 300

cgaccacagt tctggggctc tgttgcagga tttggggctg ctggtttcca agttcaggcc 360

cctggctgtg cttttggtga gggaaatgtg ccaggcatct ccttccattc cagagagaca 420

aaggaagaca caggaagggg gcgaggaacc ccaaaagctt tcttagaggc ccaagaaaag 480

agagccaggc aagattctcc cctgcagaga gaaggctaca tgagacagag ttcacagcct 540

ctgggggcca acactgcatt tacatggcat aaattcccac tgccacggtc gccaacagga 600

aactgagtgt tga 613

<210> 450

<211> 646

<212> DNA

<213> Rattus sp.

<400> 450
gttgaacaat aatttattga gacccctccc tcgcagcctc tacaattcga ggttactttc 60

tccgcttgta gatcttgttt gctagttcca ggaagatgga tgggggcagg ggcgcggagc 120

actgctctat gagactcttg aggcggttgt aactgtcttc ctcgtacttg aagaacacac 180

tccgcagatc cagctcctcg tacagtgctt tcacccgcgc cactttttct gggtccttct 240

gcccataatt ctcctctaag atctggcgct gctgaggagt ggctcgtagc agacactgaa 300

ccaccagcca gctgcatttg ttgtcctgga tgtcagtgcc gacctttccg gtcacactgg 360

ggtctccaaa gagatcaagg tagtcgtcct ggatctggaa gaactcgccc atctccagca 420

ggatcttcag ggcattagcg tgttccttct ccccatcaat tccagccatg tacatggcag 480

ccgcgatagg caggtagaaa gagtagaaag ctgtcttgta cttgacgata gatttgtacc 540

tcttttcagt gtatctacca agatccactt ggccctgggg tgctgtgatg aggtcgagag 600

tctgcccgat ctcagtctga taggaactct gtagaaagag ctccag 646

<210> 451

<211> 495

<212> DNA

<213> Rattus sp.

<400> 451
cccaaaacaa agtaacttga ggtttatggc atacagttta ggtcaacaca catacagaag 60

ggggaagagg acatggaggt gtccgaagca aagctgagtg acggaacaca ttcagttagt 120

aggtgtctat acaaagagtg gtgggacatc aggaaaagct cccatggatt catgtgcacc 180

catctggagg taccagattc ctccatggca gatccatgtg cagagctaag ggaggaggcc 240

acttctgtca ttccagtttt agaagctcca catcaaaaac aagagtagca tgtggtggga 300

tgatgcctgg gtgcccggtg gctccatagg catagtctgg ggagattatc agtttggctc 360

tctggcccac actcatctgg gctacccctt cttcccagcc tcggatcacc tcctgcttgc 420

ctagtgtaaa cttaaaaggc ttgtttctgt cccgagagga gtcaaatttt tcccatcttt 480

aagcatccac gtgta 495

<210> 452

<211> 568

<212> DNA

<213> Rattus sp.

<400> 452
cctagatcat ggcttgttta ttaaaatatt ggtgaaaaaa taggagtctc tagggctgct 60

gacaggaggg gacatcatag cactcaaggt gtctggactc tcctcctggc cactctcctt 120

ttcctgatcc cactctgtaa caggctgggg ctcctgctca ccagagacag ccttgctctt 180

ttctttggca gggcagactg ggaggacctt cttttgacct cctttgtgct gagagccttc 240

tgatgctttt tgctttcagg ctctacgggg gacacaggag tggatccatt cacctgtgac 300

agcttctctt ttttcttctg cacttttggg gtcttggcag ggggggtgct gggactcagg 360

gtggggttat tgggagccgg actcttggcc acagttactc cattcacctg ggatggggtg 420

ttagccttca ctctcttcct tcttttcttg cctgtgctgg ggggctggtc tttaccagtg 480

gcagcaggca tggcaccctc tgtcactgca tcctgctgtg atgcagcctt cttttctgat 540

gtggtaccct cagaattaag tttctttc 568

<210> 453

<211> 430

<212>  DNA

<213>  Rattus sp.


<400>  453
catagccttt tcttccttta tttcatatcc ccaccacagt aacaattcct ttaatttaaa      60

ctaaaaacca tagacttcct gaaagggtgg cagcggaggg acggtggtgt cacaggcaga     120

ggaggtggga gggcactcag cgaatcgtct tgactgggct cttgaagttg ctggcggctt     180

ggagctgcag ctggtaggcc attggatgga tcttgaaacc gtagagcctg ggcacaaact     240

ggttggccgg ccgcttgggc cggtactcgg gatgcaccat gaagagcatg tgagggaaac     300

cagtgccgaa gtatgcgcca tccgtgtggt ggtgcctata ggacttgggt gtgtacacgt     360

ccatgcactt ggggcagtag agcttcacca tggcctctcc tgggatgtcc gaaaggccga     420

tgggaaacat                                                            430

<210>  454

<211>  411

<212>  DNA

<213>  Rattus sp.


<400>  454
aactcttaaa tacttacttc ccatttattt aaagttccat cagtctgtat acactattta      60

tattaaaaac acaggaagct ggggctccta gcaaaaatat acatttcagt ttggagactt     120

cggaccctga gaggagctgc gtcctcagca ccagacctgg gggagggggc agggcagggc     180

acgcagctcc agcacagcct gggcctcaca gggaagaccc caaggcctga tcagcagagg     240

tgagggacag ggagtgtgct agcatggagt aatctggcag gagcctacac tgggcttgtc     300

ccctctccaa gccacagagg accagacgga gacccacgat gtcaggcctg gaagccaagg     360

cgtacaatgc ttttcgggtg ggcctcggca ggatctgctg ggctgggccc a              411

<210>  455

<211>  385

<212>  DNA

<213>  Rattus sp.


<400>  455
acaggcaaaa tgggcacgtc ccaaggctca atgattattt ttttcttttg ccatttacag      60

cagaataaat attttgttgc tattgctaca ctttaaattt acattctaac ctattaaatg     120

caaaagctac tgtaaagcat atagattaag tgtaggtccc atacgtatga cagtttgttc     180

aagactagta ggtttgtgta tctttttctt taacttatta aatggctatt gtgaaagatt     240

tgtgcttgtg atcagctctt aacttaaatt tttacatcac atcttccctg aaaacagtct 300

ttcttactgt ccccaatgtt ctcaccatac gccttacact caatgcggat ttcagtgtcc 360

aaggtgaggt tggtgaactg cactg 385

<210> 456

<211> 589

<212> DNA

<213> Rattus sp.

<400> 456
tgggatgtta actcttttat taactcaggg ttaacattag atttgcttca ctgatttctt 60

atttgaagtt gtggattcaa cagacaacag ctgagtcctt gccacttaca ggtgacaaga 120

caaaacacat tatattcaga gaggagagca ggaccatatg gagagagacg aggttggaaa 180

aatttaaggc aaatgttatt cccacgttaa agggaactgg accaagcaga tctgcgcatt 240

tcaatgaaaa tatatgaaat atttgtcaaa ataattaaca tcggtaaaaa ttagaacacg 300

tagcttataa aatgcccaca caggcttggc agaaaaatca atccagctct atcaacgtag 360

gcttgtacta tactgggcca gagcagtgtc cacccgagaa cagagtcctg agtgtggagg 420

ccaaatgcac ttgagaagca gttgtgtgcc atggagggct ggcgattagt gatttggggc 480

tggctttgca taaggaggaa ctgcttggcc attagaggtc tttcggctag ccttaagtag 540

ctcctttgga cccttctcca caggctctgt atctgtagat cctcgtgcc 589

<210> 457

<211> 527

<212> DNA

<213> Rattus sp.

<400> 457
aatttctgta tttttttctg tattgtatcc tcatgggaca ttaggggttt tatatggtaa 60

gacacccaag gttttggtaa aacattatca aatatatatc cagacgattc ttccctagaa 120

gaaaaaacaa tctttatgcc tgattttaaa aagttgaaaa gaggtggatt tttcctttat 180

ggtgctgaaa ggaaggatgg agaatgagga gaaaataaaa ctgtgaggat caagactggc 240

atcttgtctg tacttatttt caggacaact ggggagaacc tgctgatttc cagagctgat 300

cccagcctgg gacttcggga aatcactgag cacacagccc atgtctgcca tattggttct 360

actactcagt ccctccaaga ctgtttcata actgagaggt cattagcaag tgcatgggtg 420

ggcagaggtg ggacaaggct gaatggccaa ctgaggaatc tctgcacttt ctgattcaac 480

aaggttaggc catcacagcg agggtcttca gacataagac aggagaa 527

<210> 458

<211> 607

<212> DNA

<213> Rattus sp.

<400> 458
ggccgcacaa cttttttttt tttttttttt ttttttttgc tggttcaaaa agctttattt 60

ttacttggtc caagacttgg gagaggctcc agggcggtta caaagctgcc tagtggcttt 120

agaggttcat tcgggtggag gtgctgaggc ggtcggtgca gagcagagga gggagcccct 180

tggaaggcac agaggcctag tcgtgcttca gagtgaggcg ctcaaagaga tactcgccca 240

gagatgcctg ggccacgcca gtctgcgctg gttgtggccc tgccacccta cggaggttgg 300

tcaggtggtt gcccatcttc ttgatgagct tcacctcctt atccaggaag tggctttcca 360

agaagtcaca gaggtgaggg tctgtgcggg cagagcccag ggcgtgcaga tccaagaggg 420

cctggttcag gttcttctcc agggccaagg cagcttccat ggcctccagg gttttacccc 480

actcatcttg agatggcttc tgcacatcct ggaagagtgc acggcccccg cgttcgttct 540

gcaacttgag gagacgctcg gcgccctcgc gcttctcctc ggccaattcg cggaagaagt 600

ggcctac 607

<210> 459

<211> 478

<212> DNA

<213> Rattus sp.

<400> 459
gaataaaaag accttggtat tccatccttt tgagttacag tggggaccag tgggagaggg 60

aacagttggg agtggggtga actaggaagg gctcaagatc actggaagtc gagtccgctt 120

agttcttgta ttggaagggc tcgtcgccag tttcgttgag gagacacgtg cggacgatgg 180

cttccgtcac cgcatagggg tcgcaattgg cagaaggccg acggtcttca aagtaaccct 240

tcttctcctg gccgacaatc cggggaatgc ggatactggc gctgcggttg gcaacgccag 300

cggaaaagtc gttgatgttg gaggtttcgt ggaatccagt cagacggcgg gcgttgtcca 360

ggcccccctt ggggtcgtag gcacggatgt ggtactggtg cctcttgctc agtttatcaa 420

tggcctcctc aatgcacctc aaaccattct ccttccgcat ggccttggtc ctcgtgcc 478

<210> 460

<211> 454

<212>  DNA

<213>  Rattus sp.


<400>  460
gggtccctgt ggaacaatgc tataccctct actttgactt aatcgacctc ttcaatggtg      60

gggcctgaag aagcaccacc agatggagga gctcctccac cagggaagcc accaggcatc     120

aatgtcaaaa gtaacctcaa tctgaggaac cccacggggt gctggaggta tgcctgtgag     180

ctcaaacttc ccaagcaggt tgttgtcctt ggtcatggcc ctttcacctt catacacctg     240

gatgagtaca cctggctggt tgtcagagta ggtggtgaaa gtctgggtct gcttggtggg     300

aatggtggta ttgcgcttga tgaggacagt catgactcca ccagcagttt caatcccaag     360

ggaaagagga gtgacatcca agagcagcaa atcctgaaca ttctcagact tgtctccaaa     420

tagaatggct gcctggacag ctgcaccata agca                                 454

<210>  461

<211>  512

<212>  DNA

<213>  Rattus sp.


<400>  461
ccacgcctct cttcctttat tgaggcctag agcccaaatc ctgcaagctg ggctccaggc      60

ccaggccttc ctcagggccc acagagccca cgaagcccag ggggcacaga agggaacccc     120

ctacacacaa ggggatcccc aacctgccgc cccacctggc acacaggtca gaagcccctt     180

tggggctggt atcagatcta gcttaatcct tcttgctccc gtgttgctgg ctgtggaact     240

tctgatgcac cactcggagg gtggtgaaaa aattgccaag gaagaggagg aggaagggga     300

ggcctcacat gagcacctgc cactccttgc actcagggtg ccgggccaag ttaaacaatg     360

tcagcgcatt aaaaagctgc cagaaatgtc caaagaagag gaacggaagc aggaaggtga     420

ggcctctcca catccaagac tgaaagccct ccacagggag gtccatggtg tgtcgttcgc     480

ccagggctcg cataactagt tagcatgcca aa                                   512

<210>  462

<211>  387

<212>  DNA

<213>  Rattus sp.


<400>  462
catatatagc tgtgttcaga cacatcagaa gagagcatca gatctcatta catatggttg      60

tgagccacca tgtggttgct gggatttgaa ccaggacctc tgtaagagaa gtcagtgctc     120

ttaacactga gccatctctc cagcatttgt tttgttttaa aacaatgacc ataagaggaa 180

tggtggactt ctagcttgtt ctgcctacag taacaaaagc acatcacacc cccagctcac 240

aaacctgtgt gcaatgtcac taccaccaca caacggtact gcgttacact gttggaaacc 300

gcatttccac tcagtgcaga taaaagcaat aggaaacacc cagcccagag tcagattatt 360

ttctgttgtg aagtcaggcc tcgtgcc 387

<210> 463

<211> 646

<212> DNA

<213> Rattus sp.

<400> 463
aataaagaca agtgttcaga tttatttgga aattcacagt ttctaatggt actacagctc 60

tgtagttaca tatttaaaac cctcttttca caacacatag tgacttccca ctctggctct 120

gctggcatct ggacttcaag ggcctgctgt accatcaggc cctattgctc tgaatggcta 180

attgtgcaga ctggaagggg taaaattcat ttcctaaaac gtactgttca cctaaagcag 240

caacttaaag aaaaaggcct taaataaatc acattacaaa caacaccaaa aaaggtatta 300

gacaaattta aaaacaatta ttaacaaaag tgctcaataa gttataactt aaattttaca 360

acacaaaatg gtcaattctc tcaaaaacac atttcacttt cactcatccc tgtacaatcc 420

acacatcaag aactcaattt actcagacag acactgccaa ttacttaagg gggggggtgg 480

ggggacaaaa agtaaatgaa gacaaacgct gacaaccaca tacacacata tctgcacatt 540

gtagccaaca aaagcaaaca caaaggaatc ataatcctac tagacttcac caattagact 600

gaatcaggcc tgacgtgctc actggctggc tgtgactatg tacaaa 646

<210> 464

<211> 625

<212> DNA

<213> Rattus sp.

<400> 464
agaaatgaca ccacagggtg gactttattt taaagctcac aaggtgttca caatgatcac 60

atgatccaca cgtctcccgt gtcacacctc cacgggacag tgcatgtatg gtgatagtta 120

cagccctgct tcgcatgctg ctacggttca ctagctgttg tattcttggt aataataaag 180

caaatcactc tactggacag acttaatttg gaaagccctt atgcagatca gactcagtct 240

catatgaaca accccggcca cacatgcgga aatgaagagc aaatgcagaa gaacacagaa 300

aaccccttgg caagaacagc tgctgcagac tgagcccagc gctgtcagtg cagttcacgt 360

cctcagaaga caaacgacct ccctcctcag catatgagca gcaatactgt acagagctca 420

gtggggtccc aactccacag gagcctgtca ccaaagtcac tctcatttag ggtcagagac 480

tacagactca agctttttct tttttccctc ataatacaca aaatgtctag acagtcttta 540

aaaaaaaaaa aaaaggaaga aagaaaatat aaatagactc agtctgtcat acagaatcac 600

atacaatggc aaacacattt catga 625

<210> 465

<211> 472

<212> DNA

<213> Rattus sp.

<400> 465
aaggcaaggt ctctgtcatt agggctggga gagatggtac agatcaagcc aaacagtgaa 60

aaataaggca aactgtagct tcgggttttg agcctggcac caggaaggcc tcttcttgag 120

gaagaggggg caggcagtgg caaatcagca gacgagatgc aaatggagta acaatcacag 180

ttagtggggt aacgagatca ggcggtaaac ccttaaagca acacccaagg tccacgtctt 240

tgcatgtgca gggcacaact ccagcagcag tttctgggtt tgggggtttg ttacttttcc 300

gactttcccc accccctaaa agacacgaag acgagctcat gaagagatca aggaagggac 360

gtcttctggc tgcgctgggc aagggttggg tgacttccgg gagcacttgt ctaccttccc 420

tgtggctggc cgagtcctac aaacgtttgt aggtgcccag gagcgctttg ca 472

<210> 466

<211> 430

<212> DNA

<213> Rattus norvegicus (Norway rat)

<400> 466
ttaagaaaga aaaaataaaa atataaataa aaaattaaaa tttttaaaat aaataaaaaa 60

aaaaaaaggg ggaacccgta cacccacaaa gagtgaaaaa gggctatcac taaccaaaaa 120

tctcccacaa gaaaaaacaa aaaaaggggg aggccccccc caaccaaaat gcgcggtggt 180

aacccaaact taagccaaag tcaggagaga ggctttaaaa aaaaaaaaaa ttcccatttg 240

caaaccccac ccttggtgga ttttttccca aaaacccccc cgaaaaaaaa aaaaaaacct 300

tttttcttat tcccaaaaaa aaaacatatc tggggcgaaa aagcagaacc ccccccttga 360

aaacaacccc cccaaaaaaa tggaaaaaaa aaagctccct ttcccaaaaa aaaaaaaagg 420

gccatggaac 430

<210>  467

<211>  450

<212>  DNA

<213>  Rattus norvegicus (Norway rat)

<400>  467
ttcattatta tttttatttt tttatatttt tctatttttc attttcattt atttttattt      60

tacttttctg tatactttat taatattttt atttatattt ggacagagag aaaacccccg     120

ggcaactaaa aaaaaaaaaa aaaaaaattt aaaacaaaaa aaagccgccg gggataaaaa     180

aaaaaacaaa aacaccggtg gaaaaagcgg ggcgccccaa aaaactacaa aaaaaacaat     240

ggcccagtca ggaggaaggg gcacccagca aaaaaacctg ggcggagaaa aaaagggacc     300

cccccaacaa gaaatggggt gtggcaccac cacggggcga cgaaaaacag aagggcgaag     360

ggtgaccccc acaacaaaaa aaacttgtac tccaacgggg ggaagaaaaa aaacaccacc     420

atgggcccgg ggaccgcgga tcttattctt                                      450

<210>  468

<211>  402

<212>  DNA

<213>  Rattus norvegicus (Norway rat)

<400>  468
ttatattgct gaccttcatt tttatttaaa tatattcatc ttgagaggaa actacacagg      60

aaatatttta tataaatgta aacatgcatg tctacatcag aattaagcaa aataaatttt     120

gagtcacaag attttaaata tcattaaagg acaccaggaa ttgaagacgg aagtgacagg     180

aaacaagaac acagatccta acccttgagt tggtcttcca agctactggg taaggcctga     240

gtttcaaaaa gtttatgttc cttgccaagt ggaaatatcc agtttggctg ttcttaatac     300

atatctacaa aacaaacaat tctcttttaa aataattaat aaatatgaca cacacacata     360

cacacgcaca cacacataca catacacaca tacacacaaa ct                        402

<210>  469

<211>  448

<212>  DNA

<213>  Rattus norvegicus (Norway rat)

<220>

<221>  misc_feature

<222>  (426)..(426)

<223>  n

<400>  469
ttacaaaaac aaactttatt ttgctatctc acaagtcagc caggagattg ccatggtata      60

tgtccctgct tctggtaact tttaccagac acaaacagga tcccttcacg tcctcacggg     120

agctcaggct gcctctgcca tgctgggggc ttcccaaagc agccagagag atttctctgc     180

accacctcag cctctacaga agttctggct ggggaaagac tcgctgagcc tccgtggcta     240

accaggcttt ctgacccaag atcaggcacg gtggccctcg gctgggcttg ctgaccgaac     300

atccagacag aggtttctcc tttggcaggg cctgcctcag agccaggtcc catttgctgc     360

acagtccaag aagccatcat ctcaggagcc ttccccagac ttcactgaag gctgtacagc     420

cacctnctcg atctgccagc gacacatg                                        448

<210>  470

<211>  231

<212>  DNA

<213>  Rattus norvegicus (Norway rat)

<400>  470
ttgacttaca acagggccct ccctttgtta cagacaaggg agggtttgct tgaattaaac      60

caccctcaat acccaccctg gccctaacta aggatgggct ggccagccag ccagccacac     120

tcacttttaa caaacaaact ttctataccg atcgagggac tacctgctac atgcatggct     180

gacagaccat ttttactatc gaaagaacca acctgacgga ggcttactta a               231

<210>  471

<211>  393

<212>  DNA

<213>  Rattus norvegicus (Norway rat)

<400>  471
ttgatgctgg gaattgaaca cagggttgta acgctctatg acagctacag caagcacgtc      60

tcatcctcag ctgttcaact taactgcaag gccagtatgt tcctgtcgtc tcaaagctgc     120

acctggggaa gcatgagcga tggcctcagc ctgcagcaag tggtggtcat gcctgtgcac     180

aacaagctgg agcggagatt ggtggggctt gcacacccct ttcatccgca ttgctttaaa     240

tactggacac agcctttgca cagtggcccc tgtggccacc tatgaacact gcaagtgtag     300

taaccggatg tgtgtgggca aacaccttct aaaccacacc agtgtacccg atagccagag     360

cctaggatca cagtatagag aggtgactca ggg                                  393

<210> 472

<211> 287

<212> DNA

<213> Rattus norvegicus (Norway rat)

<400> 472
ttggtctcca tggacagcat ttattaggcg ccattctcag tgtgaagaca gatgggcagg 60

ggctgctgag gggggccagg gcagccacag cctggggact taggggctgc ccgggtgggt 120

gtggggatgt cacccacaca tattgcatat tctgtggcag tgtgcccagg cataaggcat 180

tggggaagca gagaggctgc ctgcagtggg agggggtgag acaggaagtg acagtggcct 240

tctagttccc taggcaagac cctacaagag accagcagtc ccccatg 287

<210> 473

<211> 541

<212> DNA

<213> Rattus norvegicus (Norway rat)

<400> 473
ttacctcaga acaaataaat aaagaaaatg tatttacatt agttcatcat tggaaaatct 60

ggcataactg aacgatggga aaccaggact gtgtcctagt ctcttgggtc tagacctagc 120

tcacagggca ggcatgggca ccttacaacc ttctgtagaa acggagagag gtggcatggg 180

tcttgagcag gttgagcagc agcgtcgacg cccctcagtg aagagaatcc cagcgtccca 240

atcaaggagc tcctgctgag ggcatgggct tcgtctaacc atgttaagtt cacgtagaca 300

tctgtccaag ttaaacaaag tccttccatt cagcagtaca taggacaagg agccatggtc 360

aggaatggca acctcttgcc acaaactatg tgacaagagg gatgtggaat tcatgtgtgt 420

caaatatcag gggcctcctg ggtggttttg ggtccctgtt ggctttgtgt agcagtttga 480

agagaccagt tccaatgttc attgggattc ccatgatgat gcactcagac acaccacaca 540

c 541

<210> 474

<211> 401

<212> DNA

<213> Rattus norvegicus (Norway rat)

<400> 474
ttagactaag acaatgctcc ggctttaatg tatgaaaata atacccatgt tgtctaattt 60

gggggtcata cattagaagt gtaaaggtct gcgtctgccc gccgtctagt tgaagtacgt 120

gagcacaatc atttggatcg gctgtctgca cacggggcag ggcttattcc tcttctttag 180

cttctttgca cacgtgaaac atgacatcag gtgtccggtt ttgccgtgaa caatgcaacc 240

atttttaggc cgcccctggc aaatcacaca tggctcgatg gcgttcagag agaagctgga 300

ttccatactt tcctctttgt cttgtgtgtc ctccttcaac tctttgccac tttcttggct 360

gctgtaaaca atgctactgg aagtcgacgg ctgggaatag t 401

<210> 475

<211> 437

<212> DNA

<213> Rattus norvegicus (Norway rat)

<400> 475
ttgggagaag agtctgaaaa agtaaacttt aattaaaaaa aaataataat aatacaactt 60

caggttcgtg ctaggctctc ccacttggcc ttttacagac tggtcggttt tccactgaat 120

tcggagaagc tgaagatgta ccacagagga cacagagcac catcacttct acaggcaaac 180

agctcgtgca caggcttgac caatgtccgt ggctcgagtg actaagcggc aaggctggtt 240

tcccggaagt tcaaaacgcc agcttcttca tcagcaggta gactcgggag tccacctcaa 300

atccatgcag gttgttggcg tccccctgaa gcctcatgag gaggccgcca taggacacat 360

acgcagagag acgtgtggct gcttccgttg aggtctcgtc cccttcgatc ctgtacactt 420

tcccatacat tacatac 437

<210> 476

<211> 403

<212> DNA

<213> Rattus sp.

<220>

<221> misc_feature

<222> (220)..(220)

<223> n

<220>

<221> misc_feature

<222> (232)..(232)

<223>  n

<220>

<221>  misc_feature

<222>  (235)..(235)

<223>  n

<220>

<221>  misc_feature

<222>  (320)..(320)

<223>  n

<220>

<221>  misc_feature

<222>  (357)..(357)

<223>  n

<400>  476
ctttgctgtt cacagaccta gaacagggct tgtaatccag acagcatcac cccactgtgc      60

acaggaatgc atgaagcaca atggctgttt cttcctccag aaaggcactt acagtttagc     120

ttggcccaaa aaggcaggcg aaactgagac accagtactc aactcacacc ttggagctga     180

agggccagtt aaggtggctc tagccataca gccccacctn cccttcctct gnctnctcca     240

gctgtggccc atctggggac aacctgggtc catctccctt cggtcagacc gtgggaggag     300

agacttgggc tgcaatcctn cctcaaccag gggatgtagc aaggattccc caggggncac     360

aaagtcgctc tgaaaggctt cccctggcgg aggaggacag cgg                       403

<210>  477

<211>  352

<212>  DNA

<213>  Rattus sp.

<220>

<221>  misc_feature

<222>  (189)..(189)

<223>  n

<400>  477
acctcaggat gtgactttat ttggaaatgg ccactccaga tgaaggtggg gctctgctac      60

agtagacagg gtgagcccca acccaaagac tgggatcctt gtacagaagg taagtttgga     120

gaccgacgca gtggagatga aggcaaagat ggagagatgt ttgtacaagc cctggggtac     180

acacaagtnt cagtgaccat cagacccaag aggattcact cttctagctt cggagagaac     240

cagcccactg acaccagacc ttggccttgc aaattccata actgtaacaa tgccacgtgc     300

tgttagaaga gcctacgttt agtactagtt acttatgtca gtgctaggac ta             352

<210>  478

<211>  341

<212>  DNA

<213>  Rattus sp.

<400>  478
tttggtgtca tttcactcag aagtttaatg agataggttg ttgaagacga caaagacgac      60

tcccgggaga caaaccccat tgccacaaac atggaattcg ggagtaactg tggctcctca     120

ggcctccctc ccacctcagc acccactgac aaagagggat cagtgtcggg aacaggacaa     180

ggaggtgcca ggaagatgaa agaatggaaa acgtgaacct gcaggctctg gctaggacgg     240

aaacagaaag gcagtgcggc tgggtgagca ccgacactgt gggccgacga atgagtatgt     300

caggggttac acagggaagg aaggacggac gtgggagcta t                         341

<210>  479

<211>  349

<212>  DNA

<213>  Rattus sp.

<220>

<221>  misc_feature

<222>  (9)..(9)

<223>  n

<220>

<221>  misc_feature

<222>  (109)..(109)

<223>  n

<400> 479
gtgttgggnt gtttattaac aaagtgagca aggggcttca aggactggtg gatccaggac 60

gcttcttttt cttccttctt tggagagcac cttgtttctg gtccaaatna gggagctttc 120

gcttggcagc tgcctccaaa gacgcccaaa gttcatctgt ctctgtgtct tctgggggca 180

ctaggttggg ctcttgaggt tcttggggct catcctccca gttatcccgg cctgacagga 240

gaaggcagtt cagttgagac ttgagataaa ccttatgctc tagccctcgt ggattgcgga 300

tcctgtgtat cctcaggact ctgtctaagc cacaagatgc taacagggg 349

<210> 480

<211> 363

<212> DNA

<213> Rattus sp.

<220>

<221> misc_feature

<222> (7)..(7)

<223> n

<400> 480
gtttgtnctt tttttcagag ctggggaccg aacccagggc cttgcgcttc ctaggcaagc 60

gctctaccat tgagttaaat ccccaacccc aagactttct tttaaaaaga cagtactcta 120

cggattgaag gattacaatc cttcaatcct gatgaactct ccatattact ggaaccacac 180

actggagcac tactgggagc cagtgaagtt ggtgcctcag gatatggggc tgttcccctc 240

agccatgggt tatctgtgaa tatgacccat cacagtctga atttatctgg gaagaaaaag 300

agctcataag tctgtgtaca cacttaacac accacagcac ttagactgca ttaggtatta 360

ggg 363

<210> 481

<211> 422

<212> DNA

<213> Rattus sp.

<220>

<221> misc_feature

<222> (34)..(34)

<223>  n

<220>

<221>  misc_feature

<222>  (81)..(81)

<223>  n

<400>  481
aaagatttat tcatgcagtt tatgtatatg agtnctgtct tcatacacat cagaaggaat      60

cagacctcat agatggttgt nagccactat ggggttgttg ggaattgaac ttaggacctc     120

tggaagaact actgggtgct atcactcaga cccaggtttt tgggagagac agtgtcctgt     180

gtagcctata actgattagg aatttgaatc tcttctgcct ccacctacca catgctggga     240

tgactgctaa gagttgtagc ttccagaaag gatgaacatt aagacctttg tgcttctgta     300

acagaagtta aagaaccatg ggaacattac tttggtttca acaggatggt gtttgttcaa     360

ggctgagagc ctcaagtgag caatttagca gagtctgtat acaaacagat ttaccactgg     420

gg                                                                    422

<210>  482

<211>  151

<212>  DNA

<213>  Rattus norvegicus (rat)

<400>  482
gtaaatctgt ttgtatacag actctgctaa attgctcact tgaggctctc agccttgaac      60

aaacaccatc ctgttgaaac caaagtaatg ttcccatggt tctttaactt ctgttacaga     120

agcacaaagg tcttaatgtt catcctttct g                                    151

<210>  483

<211>  569

<212>  DNA

<213>  Rattus sp.

<220>

<221>  misc_feature

<222>  (77)..(77)

<223>  n

<220>

<221>  misc_feature

<222>  (91)..(91)

<223>  n


<220>

<221>  misc_feature

<222>  (301)..(301)

<223>  n


<220>

<221>  misc_feature

<222>  (540)..(540)

<223>  n


<400>  483
ttcctggttt tggacaggga cttcccatct tctcttccac cctttctcta tggtccctgg      60

cagtagctcc gcatgtnccc taccttttct nactctggcc ctttgagtgt ggcaaatccc     120

atagctctga ccctccaaaa ctgttcgagg agaaggagga agaggaggag gaggattcga     180

gtcttctggt aagcggggag agcgcctcct cagacaggtc tcagctcact ctccgtctct     240

ttagttatgc ttgctcttaa ttttcatgac tttgttgtcc agcatgctct gagcgtttgt     300

nagatgcttg atggcatcaa acacaaggat gcccggtatc accaaccata tggcattcat     360

gataacgaag taggaaccag aaataaaggg ggtgacctag ctctccatgc tggaatccat     420

cgcggagttc ggtcaggaag tacagcacat ccccatatat ctggcccaca gacaccacaa     480

gctgtaggac aaagcggaag ggtttgatga cggagaaagg cgatcaccac ccataggctn     540

agtgggtccc cagagacaag ctgtgacaa                                       569

<210>  484

<211>  2190

<212>  DNA

<213>  Rattus sp.


<400>  484
gctgccagct acaggcagtg ggaaatccca cagggcagca gctgtattgt agacggccct      60

tgctcactgc ctgcctgcag ccagctctgt acaaggaaca atggccctga ggacaggagg     120

cccagccctg gtggtgcttc tggctttctg ggtggcactg ggcccctgtc acctgcaggg 180
gacagatccc ggagcgtcgg cagatgccga gggcccccag tgccccgtcg cctgtacctg 240
cagccatgat gactacacag atgagctcag cgtcttttgc agttcaaaga acctcacaca 300
tctgcctgat gacatcccag tcagcaccag agccctgtgg cttgatggca acaacctctc 360
ttctatcccc tcagcggcct tccagaacct gtccagcctg gactttctca acctgcaggg 420
cagctggctg aggagcctgg agccacaggc actgctgggg ctgcagaacc tctactatct 480
gcacctggaa cggaaccggc tccggaacct cgccgtgggc ttgttcacac acacaccgag 540
tctggcttca ctcagcctga gcagcaacct cttgggccgg ctggaggaag ggctgttcca 600
gggcctcagt cacctttggg acctcaacct gggttggaac agtctagtgg tcctgcctga 660
cacagtgttc cagggactgg gcaacctcca cgagctggtg ctggctggca acaaactgac 720
ttacctgcag cctgcgctct tctgtggctt gggcgagctg cgggagctgg atctgagcag 780
gaacgcactc cgaagcgtca aagctaacgt ctttgtacat ttgcccaggc tgcagaagct 840
gtacctggac cggaacctca ttacagccgt ggcccctggt gcctttctgg gcatgaaggc 900
cctgcgttgg ctggacctgt cgcacaaccg cgtggctggc ctcatggagg acaccttccc 960
aggcctgctg ggcctgcacg tcctgcgcct ggcacacaat gcgatcgcta gcttgcggcc 1020
gcgcactttc aaagacctgc acttcctgga ggaactgcag ctgggccaca atcgaatcag 1080
gcagctcggg gagaggacat tcgagggcct ggggcagctg gaggtgctga cgctcaatga 1140
caaccagatc actgaggtca gggtgggcgc cttctctggc cttttcaatg tggcggttat 1200
gaatctctcc ggcaactgtc tgaggagcct cccggagcgg gtgtttcagg gtctggacaa 1260
actgcacagc ctgcacctag agcacagctg cctgggtcac gtccgcctgc acacttttgc 1320
tggcctctca gggctgcgca ggctcttcct cagggacaac agcatctcca gcatcgaaga 1380
acagagcctg gcagggcttt cggagctcct ggaactggat cttactacca accgcctcac 1440
acatctgccc cgccagctct tccagggcct cggccacctg gagtacctgc ttctctccta 1500
caaccaactg acgactttat ccgcggaggt cctgggccct ctgcagcggg ccttctggtt 1560
ggatatctca cacaaccacc tggagacgct ggccgaaggc cttttctcat ctctggggcg 1620
cgttcgctac ctcagcctca ggaataactc cttgcagacc ttttcaccac agcccggcct 1680
ggagcgcctg tggcttgatg ccaacccctg ggactgcagc tgtcccctca aggcgcttcg 1740
agactttgcc ctgcagaacc ctggtgttgt cccccgcttt gttcagactg tctgtgaggg 1800
ggacgactgc cagccggtgt acacctacaa caatatcact tgcgctggcc ccgccaacgt 1860
ctcgggcctc gacctaagag acgttagtga aacacatttt gtgcactgct gacactggct 1920
acttactggc ccggtctggc cgaacactgt ctcatggcca ggacggtgtc tcattgttaa 1980
cagaataagc tggctctcaa attcctaccc atctctaggg gacaggtcct ggctgctcac 2040
ttcctggaag caggctgtac tggaagctat gtggcctaga aagggtgggc tcaggccaag 2100
tgtccaaggg cccaaaggag ggaggtgctc gctgaattta agcatattag tcagcggagg 2160

aaaagaaact aaccaggatt ccctcagtaa                                    2190

<210>  485

<211>  397

<212>  DNA

<213>  Rattus norvegicus (rat)

<400>  485
tgcagctgtc ccctcaaggc gcttcgagac tttgccctgc agaaccctgg tgttgtcccc      60

cgctttgttc agactgtctg tgagggggac gactgccagc cggtgtacac ctacaacaat     120

atcacttgcg ctggccccgc caacgtctcg ggcctcgacc taagagacgt tagtgaaaca     180

cattttgtgc actgctgaca ctggctactt actggcccgg tctggccgaa cactgtctca     240

tggccaggac ggtgtctcat tgttaacaga ataagctggc tctcaaattc ctacccatct     300

ctaggggaca ggtcctggct gctcacttcc tggaagcagg ctgtactgga agctatgtgg     360

cctagaaagg gtgggctcag gccaagtgtc caagggc                              397

<210>  486

<211>  832

<212>  DNA

<213>  Rattus sp.

<400>  486
ccggggctca gaacagactc tgccactcag atcaccatgt acagcttcat gggtggcggc      60

ctcttctgtg cctgggtggg gaccatcctg ttggtggtag ccacagcgac ggaccattgg     120

atgcaatacc ggctgtcagg gtcctttgca caccagggcc tgtggcgtta ctgcctgggc     180

aacaagtgct tcctgcagac agagagcatc gcatactgga atgccacgcg ggctttcatg     240

atcctgtctg ccctgtgtgc cacctcgggc atcatcatgg gtgtcctggc ctttgcgcag     300

caatccacct tcacccgcct ctccaggccc ttctctgctg gcatcatgtt ttttgcctcc     360

acccttttcg tcctgttggc cttggccatt tacaccggag tcaccgttag tttcctcggc     420

cgccgctttg gggactggcg cttttcatgg tcttacatcc tgggctgggt ggccctgctc     480

atgaccttct ttgcaggaat tttctacatg tgtgcctacc ggatgcatga gtgccggcgc     540

ctatccaccc cacgctgagc cctggggcga aacttgcagc cggaagttac aacgaggaca     600

ttgaagagga aggggaggac ctagaggccg gaagtcctgg ttcctgaggg aatgaggggg     660

ctcagtcctg tattctagcc cctgacccct ctgtcctgtg tggggaggaa aagtagggtc     720

aagtgggagg gaactgagac gaagacataa gcctccacct gcactgaggg gtggtagaaa     780

acagaaactc ctttagagag tgatttggga atctaataaa actgatgagg aa             832

<210> 487

<211> 351

<212> DNA

<213> Rattus norvegicus (rat)

<400> 487
tggccacaca atttaagtgc ctagtcacca tccaccgagc acagagattg aggctggttt 60

agcaggttac ggttcagttt tgcttgtctc cccgggcaag agaaggggac ttaggaggaa 120

ggtgatgagg tcccagggac tcctgtcacc caggactctc cctcttacag aggaaagacc 180

tagtagctta aaaggtctgg gctgtgtggg ggtggggcgg tcataccact ccctcaacca 240

tgccctatgc ctgtaagccc ccatcaccac ctccgtgcag ggtcctagct ggctgggtcc 300

tcttctagcc ttgtgcctgc tccttttctg tatcccttac tctgttgtct g 351

<210> 488

<211> 436

<212> DNA

<213> Rattus sp.

<400> 488
actttatttt ggactgtggt acggccaaca agaccactct gtatgcaaaa gcccaacatg 60

gctgtaactg gagatgaaac tgatgaggag actgaccttg ccccaagtca catggctgct 120

gccacaggtg acatgccaac ttaccagatc cgagctccta ctactgcttt gccacaaggt 180

gtggtgatgg ctgcctcacc aggaagcctg cacagtcccc agcaactagc agaagaagca 240

actcgcaagc gggagctgag gctgatgaaa aacagggaag ctgctaaaga atgtcgacgt 300

cgaaagaaag agtatgtcaa gtgtcttgag agtcgagtcg cagtgctgga agttcagaac 360

aagaagctta tagaggagct tgaaactttg aaagacattt gctctcccaa aacagattag 420

tagaaatatt taacta 436

<210> 489

<211> 704

<212> DNA

<213> Rattus sp.

<400> 489
cgccaccgcg tctccttgct gcttttgtgc tctcccgctc gccatggcca gccttaagga 60

cctggaaggg aagtggcgtc tggtggaaag ccacgggttt gaggactaca tgaaggaact 120

aggagtaggg ctggctctta ggaagatggg tgccatggcc aaaccagact gcatcattac 180

cctcgacggc aacaacctca ccgtcaaaac tgagagcacg gtgaagacga ccgtgttttc 240

ttgcaccttg ggagagaagt ttgatgaaac cacagctgat ggcaggaaaa ctgagacggt 300

ctgcaccttc acagacggtg ccctggtcca gcaccagaag tgggaaggga aagaaagcac 360

gataacgaga aaactgaagg acgggaagat ggtcgtcgag tgcgtcatga acaatgccat 420

ctgtactcgg gtctatgaga aggtacaatg aggactggct aactcgtcat cctggacagc 480

agtcagctgg ctgagggaat aagctcaatt caatgagcag gtcgtacaga accacactgc 540

ttcacttctt tggttttatt tttcatgact tttcatcata gacactttac ccgaaaccca 600

tgtcagaccg ttggtttacc caggatcatt cctttggtta gtaaataaat gcgtttgtgc 660

taaaaaaaaa aaaaaaaaaa aaaaaaaaaa aaaaaaaaaa aaaa 704

<210> 490

<211> 746

<212> DNA

<213> Rattus sp.

<400> 490
ccatgtattc cagaaaggcc ttgtacaaaa ggaaatactc tgctgccaag acaaaggttg 60

agaagaagaa gaagaaagaa aaggtccttg ctaccgtcac aaaaacagtt ggtggggaca 120

agaacggtgg cacccgggtg gtgaagcttc gaaaaatgcc taggtattac cctactgaag 180

acgtgcctcg gaagctgctg agccacggca agaagccctt cagccagcac gtgaggaggc 240

tgcgctccag catcactccc gggactgtcc tgatcatcct cactgggcgc cacaggggca 300

agagagtggt tttcctcaag cagctgggca gtggcttgct acctgtgact ggacctcttg 360

cctcaacaga gttcctctgc gtaggacaca ccagaagttt gtcatcgcca cctctacaaa 420

agttgatatc agcaaggtta aaattccaac acctgactga tgcttacttc aagaagaagc 480

cacttcgcaa gcccaggcat caggagggtg agatcttcga cacagagaag gagaaatacg 540

aaattacaga gcagcgaaag gctgatcaga aagctgtgac tcgcagattt tgccaaagat 600

caaagctgtc ccccagctcg agggcctacc tgcggtctca gttctccctg acgaacggca 660

tgtaccctca caaactggtg ttctaattgt taacaaccta ataaaactgc ttcataaaga 720

aaaaaaaaaa aaaaaaaaaa aaaaaa 746

<210> 491

<211> 1129

<212> DNA

<213> Rattus sp.

<400> 491
cagaccccct cgtaggacag actgttagaa caggctgtgc ttcatctctg tttagagaac 60

tcaagcaatt gctgccatgc cggggaagcc agtccttcac tacttcgatg gcagggggag 120

aatggagccc atccggtggc tcctggctgc agctggagta gagtttgaag aaaattttct 180

gaaaactcgg gatgacctgg ccaggttaag aagtgatggg agtttgatgt ttgaacaagt 240

gcccatggtg gagattgacg ggatgaagct ggtgcagacc aaagccattc tcaactacat 300

tgccaccaaa tacaacctct atgggaagga catgaaggag agagccctca tcgacatgta 360

tgcagaaggt gtggccgatc tggagttgat ggttctctat tacccctaca tgccccctgg 420

ggagaaagag gcgagtcttg ccaagatcaa ggacaaagca aggaaccgtt acttccctgc 480

ctatgagaag gtgttgaaga gccacggaca agattatctc gttggcaaca agctgagcag 540

ggctgatgtt tccctggttg aacttctcta ccatgtggaa gagatggacc caggcattgt 600

ggacaacttc cctctgctaa aggccctgag aaccagagtc agcaacctcc ccacagtgaa 660

gaagtttctt cagcctggca gccagaggaa gccttttgat gatgagaaat gtgtagaatc 720

agcgaagaag atcttcagtt aattcagtca gctatggata cactgtaccc acaaagccag 780

cctcagaaag ctctgcaaca atgaagtatt ttgactaaat gttgaccgta cttattggga 840

gggtaacatg ttttctaagg cttctgtgtt aattcatata gacatgactc atgaggaatt 900

gctgggatgc catctagttg agttaaaacc tcaatctcga tcacttcctc ggatattttc 960

ttaatgttca ataaaacaaa acaagcttct tagacgctgg agtatccaaa cattgtcatg 1020

aaatagctgt catatccttg tcaaacagcg tcacgtagaa accctcgtgt caaactctct 1080

tacgcaaaag taatctttcc ttatggagag tgtcctttct ctcgtgccg 1129

<210> 492

<211> 1747

<212> DNA

<213> Rattus sp.

<400> 492
gcagctgctc cgctcgctct cgaacctccg tcttcagctc actgccttca ctccagactt 60

caccatgtcc gtcagggtga ctcagaaatc ctacaagatg tccacctccg gtccccgggc 120

cttcagcagc cgctcgttca cgagtggacc cggtgcccgc atcagctctt ccagcttttc 180

ccgggtgggc agcagcagca gcagcttccg cggaagcctg ggcggctttg gcggggctgg 240

tgtcgggggc atcacggcgg tcacggtgaa ccagagcctg ctgaacccct tgaagctgga 300

ggtggacccc aacatccagg ctgtgcgcac tcaggagaaa gagcagatca agaccctgaa 360

caacaagttc gcctctttca ttgacaaggt acgcttcctg gagcagcaga acaagatgct 420

ggagaccaaa tggagcttgc tgcaacagca gaagacatcc aggagcaaca tggacaacat 480

gtttgagagt tacatcaaca acctccgtcg gcagctggaa gccctgggcc aggagaagct 540

gaagctggag gtggagcttg gcaacatgca gggcctggtg gaggacttca agaataagta 600

tgaggatgag atcaacaagc gtacagagat ggagaatgag tttgtcctca tcaagaagga 660

tgtggatgaa gcctacatga acaaggtgga gcttgagtcc cgcctggaag gactgaccga 720

cgagatcaac ttcctccggc agatccatga agaggagatc cgtgagctgc agtctcagat 780

ctcagacacg tctgtggtgc tgtccatgga caacagccgc tccctggaca tggacagcat 840

cattgctgaa gttcgtgccc agtatgagga gatcgccaac cgcagccgag ctgaggccga 900

aaccatgtac cagattaagt atgaggaatt gcagaccctg gctgggaagc acggggatga 960

tctacgtcgc tcgaagacgg agatctctga gatgaaccgt aacatcagcc gcctgcaggc 1020

ggagattgac gccctcaaag gccagagggc aaccctggag gcggccattg ctgatgcaga 1080

gcagcgtggg gaactggccg tgaaggatgc caatgccaag ctggaggatc tgaagaatgc 1140

cctgcagaag gccaagcagg acatggcccg gcagctgcgc gagtaccagg agctgatgaa 1200

cgtgaagctg gcgcttgaca tcgagatcgc cacctaccgc aagctgctgg agggcgagga 1260

gagcaggctg gagtctggga tgcagaacat gagcatccac acgaagacca ccagtggcta 1320

cgcaggagga ctgagttcat cctacggggg actcactagc cccggcttca gctatggaat 1380

gagctctttc cagcccggct tcggttctgt tgggggatcc agcacttata gccgcaccaa 1440

ggctgtggtc gtgaagaaga ttgaaacccg agatgggaaa ctggtgtctg agtcttgtga 1500

catcatgtcc aagtgaatgg ccactgaagt cattgccagc ctgaggtcct gcagctgctc 1560

aggggtcaag gggagacagc tgtatggcag agtgcaggga actagggacc agccagagta 1620

ccagccctaa acctctggcc aaccttggga ggaatttcta tctgggatat gccaatgccc 1680

aactcaattg tattttccaa aataaagcct cagtggctgt aaaaaaaaaa aaaaaaaaaa 1740

aaaaaaa 1747

<210> 493

<211> 1000

<212> DNA

<213> Rattus sp.

<400> 493
caggagcatc tggacagtac accacttgtg atggagactt ctatgcctga atactatgac 60

gtttttggtg atttccatgg atttttaatg gataaacggt tcaccaaata ttgggaagac 120

gttgaaacat tcttggcaag gccagatgac cttctcattg ttacttatcc taaatctggc 180

agcacatgga ttagtgaaat tgtggatatg atctataaag aaggtgatgt ggaaaaatgc 240

aaggaggatg cactttttaa cagaatacct gacctggagt gcagaaatga agatctaata 300

aacggaataa aacaactaaa agaaaaggaa tcgcctagaa tagtgaaaac tcacctgcca 360

gctaagctcc ttccagcatc attttgggaa aagaattgca agataatcta tctttgccga 420

aatgccaaag atgtcgtcgt ttcttattac tactttttcc tgatcatgaa aagttatcaa 480

aatcctaaat ctttttctga atttgtggag aaatttatgg aagggcaagt tccgtatggt 540

tcctggtatg atcatgtaaa atcttggtgg gagaagagta agaattcacg tgttttgttt 600

atgttctatg aggacatgaa agaggatatc cgaagagaag ttgtgaagct gatagagttc 660

ctggagagag acccatcagc agagctagta gacagaatca ttcaacatac atcattccag 720

gagatgaaga acaatccatg catcaattat tcaatgctgc cagagaccat gatagatcta 780

aaagtatcgc ctttcatgag aaagggaatt gtaggagact ggaagaacca cttccctgaa 840

gccctgaggg agagatttga ggagcactac cagcagcaaa tgaaggactg ccctgtgaaa 900

tttagagcag agccctgaga caattccttg tgtctgaaat tggagtagtc tccaatttat 960

ccttcagttt ttcttgtttt gaattcagta gaagtagaag 1000

<210> 494

<211> 867

<212> DNA

<213> Rattus sp.

<400> 494
tggtagtggt ttccgcgaca ggcaggtgcg actgctttag cccggcctgg gctccacggc 60

tgcctggagg gtctctgcag tgcccacccc ttctcctccg ggaccccacg ccagcagcga 120

ccctgagcca gcagccggag cgaccggcaa tggcggcctc gacggcctcg caccggccca 180

tcaagggaat cctgaagaac aagacctcta cgacttcctc ggtggtggcc tcggccgaac 240

agccccgccg gaccgtcgaa gaggagctga gtaaaaagtc tcagaagtgg gatgaaatga 300

acatcctggc aacatatcat ccagctgaca aagactacgg cttgatgaag atagacgagc 360

cagacactcc ataccacaat atgataggtg atgatgaaga tgtctgtagt gattcagaag 420

gcaatgaagt catgactcca gagatcttag cgaaaaagtt agctgctgct gagggctccg 480

agccaaagtt tcggactcgg gaacaagaaa gcagtggaga ggaagacaat gacctctccc 540

ctgaagagcg agaaaagaag cggcagtttg aaatgaaaag gaagcttcat tacaacgaag 600

gactgaatat taaattagcc agacagttaa tttcaaaaga cctacatgat gatgacgaag 660

atgaagaaat gtcagagact gcagatgcag acagcatgaa tattgaagag tcaaatcaag 720

gatctactgc aggcgaccat ctgcagcaca aatcacaaag ttcataaacg tgttcatcag 780

tgcagtcgtt gtcaaataca aaccttgtta cttataccac actgcttctt gttctctacg 840

actcgtgact atgtaccaaa atacaca 867

<210> 495

<211>  887

<212>  DNA

<213>  Rattus sp.

<400>  495
atggagaaaa tgtcccgaca gctccccctg aaccctacct ttatcccgcc tccctacggc      60

gtgctcaggt ccctgctgga gaacccgctg aagctccccc ttcatcctga agacgcattt     120

agcaaagaca gagacaaagg taagaagctg gatgatggta gcaacagccc gaccgtcccc     180

cagtcggcct tcttgggacc caccttatgg gacaaaaccc ttccctatga cggagatact     240

ttccagttgg aatacatgga cctggaggag tttctgtcag aaaatggcat cccccctagt     300

ccatcgcagc acgaccacag ccctcaccct cctggtttgc aacctgcttc ctccactgct     360

ccttcagtca tggatctcag cagccgggcc acggcacccc tccaccctgg catcccgtct     420

ccgaactgta tgcagaaccc catcagacca ggccagctgt tgccagcaaa ccgaaacaca     480

ccgagtccca ttgaccccga caccatccag gtcccagtcg ggtatgaacc agacccggca     540

gaccttgccc tctccagcat cccggggcca gaaatgtttg accctcgcaa acggaagttc     600

tccgaggaag aactgaagcc acagcccatg attaagaaag ctcgcaaagt cttcattcct     660

gatgatttga aggatgacaa gtactgggcg aggcgcagaa agaacaatat ggcggccaag     720

cgctcccgtg atgcccggcg gctgaaggag aaccagatcg caatccgggc ctcattcctg     780

gagaaggaga actcggccct ccgccaggag gtggctgatt taaggaagga gctgggcaaa     840

tgcaagaaca tacttgccaa gtacgaggcc aggcacgggc ccctgta                   887

<210>  496

<211>  970

<212>  DNA

<213>  Rattus sp.

<400>  496
gcttcgccac ctgcccgtgg ctcccaagct ggggatgtgt acagctttgg tatcatcctg      60

caggagattg ccctaagaag tggggtcttc tatgtggaag gtttggacct cagcccaaaa     120

gagatcattg agcgtgtgac tcggggtgag cagcccccat tccgaccctc catggatctg     180

cagagccacc tggaggaact ggggcagctg atgcagcggt gctgggcaga ggacccacag     240

gagcggccac cctttcagca gatccgcctg gcgctgcgca agttcaacaa ggagaacagc     300

agcaacatcc tggacaacct gctgtcacgc atggagcagt atgctaacaa cctggaggaa     360

ctggtagagg agagaacaca agcttatctg gaggagaagc gcaaagctga ggccttgctt     420

taccagattc tgcctcactc cgtggctgag cagctgaaga gaggcgagac agtccaggct     480

gaggcctttg atagtgttac catctacttc agtgatattg tgggctttac agctctttca     540

gcagaaagca cacccatgca ggtggtgact ctgctcaatg atctgtacac ctgttttgat 600

gctgtcatag acaactttga tgtgtacaag gtggagacca ttggtgatgc ttacatggtg 660

gtgtcagggc tcccagtgcg gaatggacaa ctccacgccc gagaggtggc ccgaatggca 720

cttgcactac tggatgctgt gcgctccttc cgcatccgcc ataggcccca ggaacagctg 780

cgcttgcgca ttggcatcca cacaggtcct gtgtgtgctg gtgtggtagg gctaaagatg 840

ccccgatact gcctctttgg agacacagtc aacacagctt caagaatgga gtctaatgga 900

gaagccctca agatccactt gtcttcagag accaaggctg tgctggaaga gttcgatggt 960

ttcgagctgg 970

<210> 497

<211> 1274

<212> DNA

<213> Rattus sp.

<400> 497
aagtcacata ttaaccgatg gatacactaa actggtttcc tgcaacctga gggtggctcc 60

tgataggtac caatttggac catggaacag agtccaggaa tgtttccgac cctgccctaa 120

agaaggcaga cacttcttta gcagccgttg tccagacccc ctcgtaggac agactgttag 180

aacaggctgt gcttcatctc tgtttagaga actcaagcaa ttgctgccat gccggggaag 240

ccagtccttc actacttcga tggcaggggg agaatggagc ccatccggtg gctcctggct 300

gcagctggag tagagtttga agaaaatttt ctgaaaactc gggatgacct ggccaggtta 360

agaagtgatg ggagtttgat gtttgaacaa gtgcccatgg tggagattga cgggatgaag 420

ctggtgcaga ccaaagccat tctcaactac attgccacca aatacaacct ctatgggaag 480

gacatgaagg agagagccct catcgacatg tatgcagaag gtgtggccga tctggagttg 540

atggttctct attaccccta catgccccct ggggagaaag aggcgagtct tgccaagatc 600

aaggacaaag caaggaaccg ttacttccct gcctatgaga aggtgttgaa gagccacgga 660

caagattatc tcgttggcaa caagctgagc agggctgatg tttccctggt tgaacttctc 720

taccatgtgg aagagatgga cccaggcatt gtggacaact tccctctgct aaaggccctg 780

agaaccagag tcagcaacct ccccacagtg aagaagtttc ttcagcctgg cagccagagg 840

aagccttttg atgatgagaa atgtgtagaa tcagcgaaga agatcttcag ttaattcagt 900

cagctatgga tacactgtac ccacaaagcc agcctcagaa agctctgcaa caatgaagta 960

ttttgactaa atgttgaccg tacttattgg gagggtaaca tgttttctaa ggcttctgtg 1020

ttaattcata tagacatgac tcatgaggaa ttgctgggat gccatctagt tgagttaaaa 1080

cctcaatctc gatcacttcc tcggatattt tcttaatgtt caataaaaca aaacaagctt 1140

cttagacgct ggagtatcca aacattgtca tgaaatagct gtcatatcct tgtcaaacag 1200

cgtcacgtag aaaccctcgt gtcaaactct cttacgcaaa agtaatcttt ccttatggag 1260

agtgtccttt ctct 1274

<210> 498

<211> 1790

<212> DNA

<213> Rattus sp.

<400> 498
aattcaggca gatagtgaat ggctatcgcc accagaagca caagaaggga aggttatttc 60

aggaacctct gatgctgcag atccccaaga ctgtggactg gagagaaaag ggttgtgtga 120

ctcctgtgaa gaatcagggc cagtgtggtt cttgctgggc ttttagcgca tcgggttgcc 180

tagaaggaca gatgttcctt aagactggca aactgatctc actgagtgaa cagaaccttg 240

tggactgttc tcacgatcaa ggcaatcagg gctgtaatgg aggcctgatg gattttgctt 300

tccagtacat taaggaaaat ggaggtctgg actcagagga gtcttatccc tatgaagcaa 360

aggatggatc ttgtaaatac agagctgagt atgctgtggc taacgacaca gggtttgtgg 420

atatccctca gcaagagaaa gccctcatga aggctgtagc gacggtgggg cctatttctg 480

ttgccatgga tgcaagccat ccgtctctcc agttctatag ttcaggtatc tactatgaac 540

ccaactgtag cagcaaggac ctcgaccatg gggttctggt ggttggctat ggttatgaag 600

gaacagattc aaataaggat aaatactggc ttgtcaaaaa cagctggggt aaagaatggg 660

gtatggatgg ctacatcaaa atagccaaag accggaacaa ccactgcgga cttgccaccg 720

cagccagcta tcctatcgtg aattgatgga cagcgataat aaggacttac ggacactaca 780

tccgaaggag ttcatcttaa aactgaccaa acccgtctct gagtgagacc atggtacttg 840

aatcgttcag gatccaagtc acgatttaaa ttctgttgac atttttacat gggttaaatg 900

ttaccactac ttaaaactcc tgttataaac agctttataa tattggacac ttaatgctta 960

attctgattc tggaatattt gttttataaa agttgtataa aactttcttt accttttaaa 1020

aataaatttt agctcagtgc atgtgtgtgt gtatgggtta ggggaacttc ctgtgtgaaa 1080

tgtgttcaca aatgtttgag actaaagact gactgattcc agatgtccgg actgattcgg 1140

gtgtcagtgg tagacctggg gaaaggtgac aggtgctctg gatggagcct tctgatttta 1200

cctcagcgtc ctgtcaggtt aggtatgtgt aagtaaatct agcttatggg gtaattgttt 1260

tttctttatt tgtgtgagta tgtgtgtgtg gaggtcagag aacaactcat ttctacagtg 1320

ttgatcctag cgatcaaaat caggttgtca ggctggacca caggtgcctt ttactactga 1380

ggtatcttgc cagccccact ggttttaagt gacgtataat tacatatgtt tatgtagtac 1440

aatataatgt gttgtgatac gtgtatacta tgaaatgatc tgatagttca cctcaaatat 1500

tttattactt tgttgaactt ttctagctgt ttctaaaata cacagtatat tatcattgga 1560

cctgtcttgt taatgtagcc caggctggcc ttaagccata atcttccttc tcagccttct 1620

gtgagctaag ataaaaaaaa aaaatcatgt aatgttatac ccagtctcaa gtcttatatc 1680

tggcaaacct tgacagtcca gaagaactag agtaaattgt ttgacagtcc tctcaacttt 1740

cctaattctg tgacctttca atatagttcc tcctgttgtg accccaaaaa 1790

<210> 499

<211> 468

<212> DNA

<213> Rattus norvegicus (rat)

<400> 499
acctggggaa aggtgacagg tgctctggat ggagccttct gattttacct cagcgtcctg 60

tcaggttagg tatgtgtaag taaatctagc ttatggggta attgtttttt ctttatttgt 120

gtgagtatgt gtgtgtggag gtcagagaac aactcatttc tacagtgttg atcctagcga 180

tcaaaatcag gttgtcaggc tggaccacag gtgcctttta ctactgaggt atcttgccag 240

ccccactggt tttaagtgac gtataattac atatgtttat gtagtacaat ataatgtgtt 300

gtgatacgtg tatactatga aatgatctga tagttcacct caaatatttt attactttgt 360

tgaacttttc tagctgtttc taaaatacac agtatattat cattggacct gtcttgttaa 420

tgtagcccag gctggcctta agccataatc ttccttctca gccttctg 468

<210> 500

<211> 2002

<212> DNA

<213> Rattus norvegicus (Norway rat)

<400> 500
ccctttaatt tcctcgaaaa ctccaatcac tcggctgaag ccatgccttg tgttcaggcg 60

cagtatgggt cctcgcctca aggagccagc cccgcttctc agagctacag ttaccactct 120

tcgggagaat acagctccga tttcttaact ccagagtttg tcaagtttag catggacctc 180

accaacactg aaattactgc caccacttct ctccccagct tcagtacctt tatggacaac 240

tacagcacag gctacgacgt caagccacct tgcttgtacc aaatgcccct gtccggacag 300

cagtcctcca ttaaggtaga agacattcag atgcacaact accagcaaca cagccacctg 360

ccccctcagt ccgaggagat gatgccacac agcgggtcgg tttactacaa gccctcttcg 420

cccccgacac ccagcacccc gggcttccag gtgcagcata gcccgatgtg ggacgatccg 480

ggctcccttc acaacttcca ccagaactac gtggccacta cgcatatgat cgagcagagg 540

aagacacctg tctcccgcct ttcactcttc tcctttaagc agtcccgccc gggcactcct 600

gtgtctagct gccagatgcg ctttgacggg cctctgcacg tccccatgaa cccggagccc 660

gcgggcagcc accacgtagt ggatgggcag accttcgccg tgcccaatcc cattcgcaag 720

ccggcatcca tgggcttccc gggcctgcag atcggccacg cgtcgcagtt gcttgacacg 780

caggtgccct cgccggcgtc ccggggctct ccctccaatg agggtctgtg cgctgtttgc 840

ggtgacaacg cggcctgtca gcattacggt gttcgcactt gtgagggctg caaaggtttc 900

tttaagcgca cggtgcaaaa aaacgcgaaa tatgtgtgtt tagcaaataa aaattgccca 960

gtggacaagc gccgccgaaa tcgttgtcag tactgtcggt ttcagaagtg cctggctgtt 1020

gggatggtta aagaagtggt tcgcacggac agtttaaaag gccggagagg tcgtctaccc 1080

tcaaaaccga agagcccaca ggatccctct cccccctcac ctccggtgag tctgatcagt 1140

gccctcgtca gagcccacgt cgactccaat ccggcaatga ccagcctgga ctattccagg 1200

ttccaggcaa accctgacta tcagatgagt ggagatgata ctcaacatat ccagcagttc 1260

tacgatctcc tgactggctc tatggagatc atcagagggt gggcagagaa gattcctggc 1320

tttgctgacc tgcccaaagc cgatcaggac ctgctttttg aatcagcttt cttagaatta 1380

tttgttctac gcttagcata caggtccaac ccagtggagg gtaaactcat cttttgcaat 1440

ggggtggtct tgcacaggtt gcaatgcgtg cgtggctttg gggaatggat tgattccatt 1500

gttgaattct cctccaactt gcagaatatg aacatcgaca tttctgcctt ctcctgcatt 1560

gctgccctgg ctatggtcac agagagacac gggctcaagg aacccaagag agtggaagag 1620

ctacaaaaca aaattgtaaa ttgtcttaaa gaccatgtga ctttcaataa tgggggattg 1680

aaccgaccca actacctgtc caaactgttg gggaagctcc cagaacttcg caccctttgc 1740

acacaggggc tccagcgcat tttctacctg aaattggaag acttggtacc accaccagca 1800

ataattgaca aacttttcct ggacacctta cctttctaag actttctccc atgcacgtca 1860

aagaactgga aagaaaaaaa aaatccagag ggggctggtc aagatgggta gagagctggc 1920

tgaagtgtcc ggttcatgtc tcccttctgt agacccctag ccctcacccc taaagtaaac 1980

aaacaaacaa gcaaacaaac gg 2002

<210> 501

<211> 2533

<212> DNA

<213> Rattus norvegicus (Norway rat)

<400> 501
ggagaggaga ggagagggaa agccagccaa gaacacatgg agagagggga ggggatgtgg 60

aaagagggag aaggcgcaga gagtaagaga gcaagggcgt caaacagccc ctttaagagt 120

aagccaggca tgcctggcta atgccaggta actgtggggg cagagcctag aggggatgct 180

aacagagctc atctcagtcc actcttctca cccagtctgc cccagtcacc ctgtctttgt 240

atccacccct tctgctagga aacaccatcc tcacacatcc gtggagctca ctgtctgtca 300

tccttcggat gcaagtgtgg ctttcttcat ccatattagt tcccatcaaa actctccttc 360

tacctctcta ttttagttat cctgtggaca gctactacac ttctcttttt tgtttttaaa 420

ttacttatgt attattttaa tacccactga aatgcaagat tttggagggc aagaaagcat 480

agcaagtatc taaaggatgt tcagtgaagg gatgacttgg tcaatcagtg tagctcattc 540

agtaaggagc ctcatagagc acccagtgca gtgatgaatg ggtcactcgc catggcattc 600

tctagtgtgt aagtctccca tttttgtgaa taactgtttg aaagattatt ttggcaatat 660

ctacctttca tcaccaaggt aaccacatct aatctctctt ttgactggtc atttagcctt 720

attttctacc tcaaaaactt gaaagataca aggaataaca aaactttcct ctaaggctct 780

ctgagagtat ttaatgaaca gcaggtaaaa gcaagccagg ctgtagaggt gacatgattg 840

cctaggagct atgtagaggc atctttcatg tatacacgtt aacaacacat tcgaactaca 900

gttagctgac tctgggacac ccagaagaat tgatgtcatg tttgtctgct ttcatcctgt 960

ttgccttagg gagccatgga catcgaagca tacttcgaaa ggattggtta caagaactca 1020

gtgaataagt tggacttggc cacattaact gaagttcttc aacaccagat gcgagcagtt 1080

ccttttgaga atcttagtat gcactgtgga gaagccatgt gtctgggctt agaggccact 1140

tttgaccaca tagtaaggaa aaagcggggt gggtggtgtc tccaggttaa tcatctgctg 1200

tactgggctc tgaccaaaat gggttttgaa accacaatgt tgggaggata tgtttacata 1260

actccagtca acaaatatag cagtgaaatg gtccaccttc tagtacaagt gaccatcagt 1320

gacaggaact acattgtgga ttctgcctat ggaagctcct accagatgtg ggagcctctg 1380

gaattaacct cagggaagga tcagcctcag gtgcctgcca tctttcgttt gacagaagag 1440

aatggaacct ggtacttgga ccaaatcaga agagagcagg atgttccaaa ccaagagttt 1500

gttaactcgg acctccttga aaagagcaaa tatcgaaaaa tctattcctt tactcttgag 1560

ccccgcacta ttgaggattt tgaatatgta aatacctacc ttcagacatc gccagcctct 1620

gtgtttgtaa gcacatcgtt ctgttccttg cagacctcag aaggggtttg ctgtttaatt 1680

ggttccaccc ttacaagtag gagattcagt tataaggaca atgtagatct ggttgagttt 1740

aagagtctga ctgaggaaga aatagaagat gtactgaaaa ccacatttgg catttctttg 1800

gagaaaaagt ttgtgcccaa acatggcgaa ctcgttttta ctatttaggg taaattgttc 1860

tccattatta tctcagtctt aaacattcta aaatatgcaa aatacatatc cataacagaa 1920

atcgcacagc tcaatattga tcaactaatg acctgtatct tctatttcct acattttata 1980

caaaacgaaa cccagttgtc ctgtcatttc accaataaaa ataccgccag ttataatgaa 2040

ataaacctga tcatggatgt aacgacaatc ctctcaacat taatcaacaa aaattactta 2100

tcgaagaggt ggcgatcttg ggagccatat tcatttacaa acctcccaac atcattttat 2160

ggttgaactc agatgaaaaa tgaatgaata tgaatgatca gagaacagca ggaagtaaag 2220

tcaggcagac taaatctgag gtccaaggtt tacaagaaac cacctgtaca acttaggatt 2280

agaataaagc aaagaagaat gaaccatcat tacaggtcca ggtaacttcc cagtcctcaa 2340

aacagaactc acgccagtgg acctgggctc tgggattagg tgccaagaca atgacacggc 2400

ttagaaggct tagaatttct tccagagata attttgcaga cacagttctt tttgtatctg 2460

atttttttta actatgagaa tactggtatt aagtgattta taccttatat ataataatct 2520

ttgtagccta taa 2533

<210> 502

<211> 1089

<212> DNA

<213> Rattus norvegicus (Norway rat)

<400> 502
ggcacgaggg gtcgcggtgg cagccgtgcg gtgcttggct ccctaagcta tccggtgcca 60

tccttgtcgc tgcggcgact cgcaacatct gacgccatga ccgagcaaat gacccttcgt 120

gggaccctca agggccataa tggatgggtt acacagatcg ccaccactcc gcagttcccg 180

gacatgatcc tgtcggcgtc tcgagacaag accatcatca tgtggaagct gaccagggat 240

gagaccaact acggcatacc acaacgtgct cttcgaggtc actcccactt tgttagcgat 300

gttgtcatct cctctgatgg ccagtttgcc ctctcaggct cctgggatgg aaccctacgc 360

ctctgggatc tcacaacggg cactaccacg agacgatttg tcggccacac caaggatgtg 420

ctgagcgtgg ctttctcctc tgacaaccgg cagattgtct ctgggtcccg agacaagacc 480

attaagttat ggaatactct gggtgtctgc aagtacactg tccaggatga gagtcattca 540

gaatgggtgt cttgtgtccg cttctccccg aacagcagca accctatcat cgtctcctgc 600

ggatgggaca agctggtcaa ggtgtggaat ctggctaact gcaagctaaa gaccaaccac 660

attggccaca ctggctatct gaacacagtg actgtctctc cagatggatc cctctgtgct 720

tctggaggca aggatggcca ggctatgctg tgggatctca atgaaggcaa gcacctttac 780

acattagatg gtggagacat catcaatgcc ttgtgcttca gccccaaccg ctactggctc 840

tgtgctgcca ctggccccag tatcaagatc tgggacttgg agggcaagat catggtagat 900

gaactgaagc aagaagttat cagcaccagc agcaaggcag agccacccca gtgtacctct 960

ttggcttggt ctgctgatgg ccagactctg tttgctggct ataccgacaa cttggtgcgt 1020

gtatggcagg tgactattgg tacccgctaa aagtttatga cagactctta gaaataaact 1080

ggctttctg 1089

<210> 503

<211> 843

<212> DNA

<213>  Rattus norvegicus (Norway rat)

<400>  503
gggccgaggt gccgcggggt tgctggaggg tcgtgggcgg cgtgcaggag acatgtcggc     60

gggcagcagt tgcagccaga ctcccagccg ggctatcccc actcgccgcg tagccctcgg    120

cgacggcgtg cagctcccgc ccggggacta cagcaccacc cccggcggca cgctcttcag    180

caccaccccg ggaggaacca gaatcatcta tgaccggaaa ttcctgatgg agtgtcggaa    240

ctcgcctgtg gccaaaacac ccccaaagga cctgccaacc attccagggg tcactagccc    300

taccagcgat gagcctccca tgcaggccag ccagagccat ctgcacagca gcccggaaga    360

taagcgggca ggtggtgaag agtcacagtt tgagatggac atttaaggga ccagccatag    420

gacgcagtga tgcttctggg cccctggggc ccttgggagg agagccacag cagtcaggcc    480

ttgtacccgg cagacactgg gtgtggatcg gccacccagt cctgctcctc actcagggca    540

cctgctctgc cttccatttt gtgaatacca gcacatacct ccttgtgcct ctgttgatac    600

tgagctgcta ctccagggta atgactctca cctacaccct ccctgcatca agcgccagcg    660

agtggacaca gaggagtctg tcggaatgat ctggcaattc tagccccaac ctctggagca    720

cacccacctt accttaggtt ggggtacctg ggaaagccac cctttacttc tttccctgag    780

aggaaataaa agccacattt accctaggcc cacagccggg ccctgtctga aaaaaaaaaa    840

aaa                                                                  843

<210>  504

<211>  861

<212>  DNA

<213>  Rattus norvegicus (Norway rat)

<400>  504
ggcacgagcc aggacccccg cgcgccatgc cgtccgagaa gaccttcaaa cagcgccgga     60

gcttcgaaca aagagtggaa gatgtccggc tcatccggga gcagcacccc accaagatcc    120

cagtgattat agagcgatac aagggtgaga agcagctgcc cgtcctggac aagaccaagt    180

tccttgtacc tgatcacgtg aatatgagcg aactcatcaa gataattaga aggcgcctgc    240

agctcaatgc taaccaagcc ttcttcctcc tggtgaatgg gcacagcatg gtgagtgtgt    300

ccacacccat ctctgaagtg tacgagagcg agagagatga agacggcttc ctgtacatgg    360

tctatgcctc ccaggagacg ttcgggacag cactggctgt tacatacatg tcagctctga    420

aggcaacagc aacaggaaga gagccatgct tgtgacagac atacagccac ttccaactaa    480

agcaagcctc tgcttcctgc tacctgcatg gagcccactg tgacactcag accatccccg    540

gtcactcact cgtgtctgag aatctcagtg agagctgcct ctgtcacgga ccggaagcca    600

acacagccac ctctcgacct gctccccaca gcacccaccc tccctgcatg caagctgtcc    660

ctgctaaccc ccaatgttat gttacactgt gtaaatcccc actgctgccg tgtgtgggtt 720

gtgtacgtcg tcatgtccct ggtttataac tatggtgcgg tcgggaagga ttcctgtaat 780

gctgctctaa ggatctggct caggcagcca ttgtaggaca cctgtactct gatgcactaa 840

gtccaataaa ggcacaactg g 861

<210> 505

<211> 2226

<212> DNA

<213> Rattus norvegicus (Norway rat)

<400> 505
aagaagcttg gcgactgtaa ggcgagagga agcctccagc gggtcttgtc gctgagctac 60

ctcagctcca cctcctctgg ccctggcccc tagtgcgcag actgcctgca gccctcctgt 120

agcatgtgtg gcatctgggc cctcttcggc agcgatgact gcctttccgt gcagtgtctg 180

agtgcgatga agattgcgca caggggccca gatgcattcc gttttgagaa cgtcaatgga 240

tacaccaact gctgttttgg cttccaccgg ctggcggtgg ttgaccccct gtttggaatg 300

cagccaataa gagtgaggaa atatccttat ctgtggctgt gttacaacgg tgaaatctac 360

aaccacaagg cgctacaaca acgtttcgaa tttgagtatc agaccaatgt ggacggtgag 420

ataattctcc atctctatga caaaggcggc atcgagaaaa ccatctgtat gttggatggg 480

gtgtttgcat ttatcttact ggacactgcc aataagaaag tattcctggg cagagatacc 540

tatggtgtca ggcctttgtt taaagccttg acagaagatg gatttctggc tgtgtgttca 600

gaagccaaag gccttgtctc cttgaaacac tccaccaccc ccttcctaaa agtggagccc 660

tttcttcctg gacactatga agttttggat ttaaaaccaa atggcaaagt cgcgtctgtg 720

gaaatggtca aataccatca ctgtacggat gaaccactgc atgccatcta tgacagtgtg 780

gagaaactct tcccaggctt tgagatagag accgtgaaaa acaatctgcg tatccttttt 840

aacaacgcta tcaagaaacg cttgatgact gaccggagga ttggctgcct tttatcagga 900

ggcctggact ccagcttggt tgctgcctcc ctgctgaagc aactcaagga ggcccaagtg 960

ccctatgctc tccagacatt tgctatcggc atggaagaca gccctgatct actggctgcc 1020

agaaaggtgg caaattatat tggaagtgag catcatgaag tcctttttaa ctctgaagaa 1080

ggcattcagt ccctggacga agtcatattt cccttggaaa cttatgatat tacgacagtt 1140

cgagcatctg taggtatgta tttaatttcc aagtatattc ggaagaacac agacagcgtg 1200

gtgatcttct ccggagaggg gtcagatgag cttacacagg gctatatata tttccacaag 1260

gcgccttctc ctgagaaggc ggaggaggag agtgagaggc tcctgaagga actctacctg 1320

tttgatgtcc tccgtgccga ccgcactact gctgctcacg gtctcgaact gagagtcccg 1380

tttctggatc atcggttttc ttcctattac ctgtctctgc caccagaaat gagaattcca 1440

aaagatggca tagaaaaaca tctcctgaga gagacttttg aggactccaa cctgctaccc 1500

aaagagattc tctggcgacc caaggaagcc ttcagtgatg ggatcacctc agtcaagaac 1560

tcctggttca agattttaca ggacttcgtt gaatatcagg ttgatgatgc gatgatgtct 1620

gaggcctccc agaaatttcc cttcaatact ccccaaacta aagaaggcta ttactaccgt 1680

cagatctttg aacaccatta ccccggccgg gctgattggc tgacccatta ttggatgccc 1740

aagtggatca atgccaccga cccttctgcc cgcactctga cccattacaa gtcaactgcc 1800

aaagcttaga cgctctctac actcttgtgt aaaagtcaat gtttcttcct cctgctctga 1860

aggtagagag acattgaaac aatcagagag aatgaaagtc aaccatcagc tgctcaggct 1920

tatttaggca tggaaagaaa taaaagtatc acatctaaaa tgcctcctgg ttgtaggtac 1980

cagtgcggcc ttgtagctag agactgagtg gctcttgctg tattgccact gtcgggatga 2040

cagtgagcta tgctaagggg catcttagtt ctgccttcat tcctaacagc tggctagtca 2100

gattgctatg tgagtccttt gtgggaactg gtgacaattc tgctttgtag gccaaggatt 2160

cagtttcttt ctctttcttt ctttctttct ttctttcttt ctttctttct ttctttcttg 2220

gaattc 2226

<210> 506

<211> 3004

<212> DNA

<213> Rattus norvegicus (Norway rat)

<400> 506
atggctccct tggctttggt aggggttgcc ctccttctgg gggcacccca ctgcttgggg 60

gaagctactc cgaccccatc cctgccacct cccccagcta atgacagcga tgccagccca 120

ggcggctgcc aaggttccta ccgctgccaa ccaggggtgc tgctgcctgt gtgggaaccc 180

gacgacccat cactggggga caaggctgca cgggccgtgg tgtactttgt ggccatggtc 240

tacatgttct tgggtctgtc tatcattgct gatcgtttta tggcatccat tgaggtcatc 300

acatccaagg agaaagagat caccatcacc aaggcaaatg gggagaccag cgtgggcact 360

gtacgcatct ggaatgaaac ggtgtccaac cttacactca tggccctggg ctcctcagca 420

cctgagattc tgctgtctgt catcgaggtc tgtggccaca acttccaggc gggtgagcta 480

ggcccaggca ccatcgtggg cagtgccgcc ttcaacatgt ttgtggtcat tgctgtgtgt 540

gtgtatgtca tcccggctgg tgagagccgt aagatcaagc acttaagggt cttcttcgtc 600

acagcctctt ggagcatctt tgcctatgtc tggctttatc tcattctagc agttttctct 660

ccaggcgtgg tccaggtgtg ggaggcactg ctcacgctgg tcttcttccc ggtgtgtgtg 720

gtgttcgcct ggatggcgga caagcgactg ctcttctaca agtacgtgta caagcgctat 780

cgcaccgacc ctcgcagcgg aatcatcatc ggggcagagg gagacccgcc caagagcatc 840

gagctggatg gcacattcgt gggcactgag gtcccaggcg agctgggtgc attgggcaca 900
ggtcccgctg aggcgcgtga gctggacgcc agccggcgcg aggtcatcca gattcttaag 960
gacttaaagc agaagcaccc ggataaggac ctggagcagc tggtgggcat cgccaagtac 1020
tatgcactgc tgcaccagca gaagagccgt gccttctacc gcatccaggc cacgcggctg 1080
atgacaggtg cgggcaacgt gctgcgccga cacgctgcgg atgctgcccg caggcctggg 1140
gccaacgatg gtgcccccga tgatgaggac gatggtgcca gccgcatctt ctttgagccc 1200
agcctctacc actgcctgga gaactgcggg tcagtgctgc tgtcggtggc ttgccagggt 1260
ggtgagggca acagcacctt ctacgtggat taccgcacgg aggatggctc tgcaaaggca 1320
ggctccgatt atgagtacag cgagggcacg ctggtgttca agcccgggga gacgcagaag 1380
gagctgcgca tcggcatcat cgacgacgac atcttcgagg aggacgagca cttcttcgtg 1440
aggctgctga acctgcgtgt gggcgatgct cagggcatgt tcgagcccga cggcggtggg 1500
cggcccaagg ggcggctggt ggcgccactg ctggccaccg tcaccattct ggacgacgac 1560
cacgcgggca tcttctcctt ccaggaccgc ctgctgcatg tgagcgagtg catgggcacc 1620
gtggatgtgc gtgtggtgcg cagctctggc gcacgtggca ccgtacgcct tccctaccgc 1680
acagtggacg gcacggctcg tggtggtggt gtgcactacg aggatgcttg tggagagctg 1740
gagttcggcg atgacgagac catgaagact cttcaggtca agatagtgga cgacgaagag 1800
tatgagaaga aggacaactt cttcatcgag ctgggccagc cccagtggct taagcggggc 1860
atctcagctc tgctactcaa ccaaggggat ggagacagga agctgactgc agaggaggag 1920
gaggcccaga ggatagcaga gatgggcaag ccagttcttg ggagaactgt tcgcctcgag 1980
gtcatcatcg aggagtctta tgactttaag aatacggtgg ataaactcat aaagaaaaca 2040
aacctggcct tggtgattgg gacccactca tggagggagc agtttttaga ggcagttaca 2100
gtgagcgcag gggacgagga ggaggatgag gatgggtctc gtgaggagcg gctgccatcc 2160
tgctttgact acgtgatgca cttcctgacg gtgttctgga aagttctgtt cgcctgcctt 2220
ccacccacgg agtactgcca tggctgggcc tgctttggtg tctgcatcct ggtcattggt 2280
ctgctcactg ccctcatcgg agacctggcc tcacactttg ggtgcaccgt gggcctcaag 2340
gactcagtca acgccgtggt cttcgtggct ctgggcacct ccatccctga cacgtttgcc 2400
agcaaggtgg ccgcgctgca ggaccagtgc gccgacgcgt ccatcggtaa cgtgaccggc 2460
tccaatgcgg tgaacgtgtt cctgggcctg ggtgtggcct ggtcggtggc cgcagtgtac 2520
tgggcggtgc agggtcgccc cttcgaggtg cgtacaggca cgctggcctt ctcggtcaca 2580
ctgttcaccg tcttcgcctt cgtgggcatc gcagtgctct tgtaccggcg ccggccacac 2640
atcggcggcg agctgggcgg cccgcgggga cccaagctag ccaccaccgc tctcttcctg 2700
ggcctctggt tcctctacat tctcttcgcc agcctggagg cttattgcca cattcggggc 2760
ttctagggcc ccagccgcta ccctgagggt ggggacttgt tggcacttgc tcttggaccc 2820
acccctgtct ccctacccag actcaacctg ggtacttcct aggctcctcc tcatcagcct 2880

tcttcctggg atcagcctcc ctttccccct ggaatcccat cccttgccct cccttctcag 2940

aggcaccccc ttcagcccat ctttgtgagt tacacctgag ccctctggcc agacacccag 3000

cccc 3004

<210> 507

<211> 1201

<212> DNA

<213> Rattus norvegicus (Norway rat)

<400> 507
cgccaccacc ccgccatgct cagagccaag atgctcggga gaggccccta caagccctta 60

gccatcctca ggcacatggg acctctctgt gccacaaggc cacagcactg gcgcttccag 120

cattcctacg cagagaaaca cagcaactgt gcccggcacc ctctctggac tggcccagtg 180

tcctcaccgg gaggcaccca gcagtctccc attaatatcc agtggacgga tagtgtctat 240

gacccgaagc tggcaccgct cagggtctcc tatgatgctg cgtcctgcag atacctctgg 300

aacactggtt acttcttcca ggtggagttt gacgattcct gtgaggagtc agggatcagt 360

ggtgggcctc tgggaaacca ctacaggctg aagcagtttc acttccactg gggagcaaca 420

gatgaatggg gctctgagca catggtggac ggccatgcct acccggctga gctccatttg 480

gttcactgga attccatgaa atatgaaaat tacaagaaag ccaccacggg ggagaatgga 540

ctggcggtga ttggagtgtt tctgaagctc ggggcccatc acgaggccct gcagaggctg 600

gtggacatct tgccggaagt aagacacaag gacacacagg tgaccatggg gccctttgac 660

ccttcttgcc tgctgcctgc ctgccgggat tactggacct accctggctc cctcaccacc 720

ccaccactgg ctgagtcagt cacctggatt gtgcacaaga tgcccattga ggtgtccccg 780

agccagctgt ccacattccg tacactcttg ttctccgggc gaggtgagga cgaggaggtg 840

atggtgaaca acttccgccc gctccaacca ctcaggggcc gcaacgttcg ctcctccttc 900

caggtcccca gggtgggaac aaagtcttga tctcaggatg aggtctgtaa ggataggcag 960

agcggatgga aaggggggtg cgcatttcca gggtgcgacg cctggattaa aaaaaaaatg 1020

gctgcagaga tggctcaggg gttaagagca ctgactgctc ttccagaggt cctgagttca 1080

gttcccagta accacatggt ggctcacaac catctgtaat gggatccgat gccctcttct 1140

ggtgtgtctg aagagagcga cactgcactc atatgcatta aattaataaa tctttaaaaa 1200

a 1201

<210> 508

<211> 2807

<212> DNA

<213>  Rattus norvegicus (Norway rat)

<400>  508
ccgcgttaaa gccggacgga gtacttctat tgctggcaga cctgccccgg agcgcgtaaa      60

cccgcacgct cgcgccgccc acgtccagct cgcgaatccg gaggcgtccg gttccaataa     120

tgccccggaa ggcagctagt ccagaggagg cagaaaggat gccgggctct gaagagatag     180

aggctgggcg gccgcggccg gttttacgct ctgtgaactc gcgcgaaccc tctcaggtca     240

tcttctgcaa ccgcagcccg cgcgtcgtgc tgcctttgtg gctcaacttt gatggtgagc     300

ctcagcccta cccgacctta ccaccgggca ccggccgccg catccacagc taccgaggtc     360

acctttggct cttcagggat gcggggaccc atgatggact tctggttaac caaacggaac     420

tgtttgtgcc atccctcaat gttgatggac agcctatttt tgccaacatc acattgccag     480

tgtataccct gaaagagcgg tgccttcagg ttgtacggag cctggtcaag cctgagaact     540

acaggaggct ggacatcgtc aggtcgctct atgaagactt ggaagaccac ccaaatgtgc     600

ggaaagacat acagcggctg acccaagagc acctcgagaa tcaggccctg ggagaggagc     660

ctgaaggagt ccactgagat tacttggtcc tgaatttcgg ccttgatggt ctgaaatcga     720

tctacacata ggacaggtca ctttctttcg gttttaaaat ggttcattct tggagtaaaa     780

tatccatcat gtaaaagaaa gttaactaac atcactgagc tttgtagtgt ttaagactaa     840

acctgcaaag tgccaccgag tctgcccttt ctagagcatt catcagaaga agctgttttc     900

agttttgctt cctagtaagg ctggatgttc agcataagga tgttagtgtg agcgattcgc     960

gaggagttgc tctgtgtcct ctaatctgaa gaatacagcc tcgtatgaac agagccccac    1020

cgctcacttg gggaaggctg agaaggccct aatgctggga tggacatcct gtagaggccc    1080

agtgcctaat gtgtgtggag tcttcagtca gtgttggagg aacaaaccat tgggtactta    1140

gtgggagagt tggagggcct ggctctctgt ctgaggaaat cttaagtgta cacgtaaagg    1200

aacaacttag cagcttctgt ttagaaagaa ctttctcttg agtttccatg tctcagcact    1260

ttctttctac tttgttagtt tattttattt tatttttgag acttgtgtag cccaggtcag    1320

cctcagtcac tccggagggg atgctggtct tgaattccgg atcctctggg agtgtgtcag    1380

cattgggagc acatgggtcg accacagtct gctctgttct tgcttttccc attttgagac    1440

gggctcatac tgtagccctg cctggcctca agctagagtc agccctgtct ccccacccag    1500

tctggctgct tagactgggt gggcttccca tattcactgc cccagacagc agagacgagc    1560

tgtccttgtg tttaggtctg gttttgagag ttccttctct gaggaagctg ctggcaggag    1620

cagggtttct gttccttccc gtcagcccct agcattccag ctgggcgccg cactctgctt    1680

tggcaggcgc tagttatgct tcgatgggag aggggagtga agctccctca gccacacact    1740

ccaaaccatg ggtcccagag cagtctgcaa tgagtagtag tttgtgggca gtcacagctt    1800

ttcttacata ttcaaatatt gatgttcact acttctatca aacattttgt agaacataat    1860

tttttatttg tttttaattc ctcagcagag aaaaatgaga gaagcttggg cattgtgtgt    1920

ggcacacacc agaaaggaca gtcagctgag agggtgcaga aaggctcttg tgcgctggct 1980

ctcagctgca tgtagtcctg taacccagtg ttctctctag tacacatttt aattggttaa 2040

agcactttgt cctggccgag atcataaggt atatacagtt ttgtggcctg atttttcatt 2100

gaacttcatt gttttgtttt cccctctttt tcgtttttgg ttttgttttt ggtttgtttt 2160

ttgctttttg ttttatcaag atagagtttc tctgtgtagc ccaggctgtc ctggaactcc 2220

ctttgtagac caaacaactg attactggcc tcaaacggag atctgtctgc ctctgcctct 2280

ccagtgctgg gattgaagta tgcctaatct tcatctaact tagtaacttg tcttttccat 2340

gttactgcac acactgtcta aattccggtg tgctagcctc tccctttgag ggtgactgtt 2400

gggagtattt gtctcagtcc tgggctcctt gagtgcagtc tgatgctcta agatcagcaa 2460

aatcactgaa cttaatactg acactcctgc cgccctctca gcgtctctcc ttagtctgca 2520

gcattccgtg agtgaagcgg cacaggccag tgtggagcct gtgtgtggga agtcagcagg 2580

tgccgagcta ttgcaacatt ggttttattt tttaagccta tctctgtctc taaaagatgt 2640

aatgtttgtc ttatttaact ggaagtttga atactgttct ctgtgcctta ggacatgagt 2700

cacttgtcct tgcacatcct gtaagtgtgt tcctcagact ttgtactctc ctttgagttc 2760

tctgaaaaag ctgaccttta gtacaataaa cggtgctaag tgaagga 2807

<210> 509

<211> 1133

<212> DNA

<213> Rattus sp.

<400> 509
cctcggctga gctgcattcc aatcccagct acatccggag cccagctaca ttcgagccca 60

gccacatccc gagccaacct tccagaagca ccatgaaccc aagtgctgct gtcgttctct 120

gcctcgtgct gctgagtctg agtgggactc aagggatccc tctcgcaaga acggtgcgct 180

gcacctgcat cgacttccat gaacagacgc tgagacccag ggccatagga aaacttgaaa 240

tcattcctgc aagtctatcc tgtccgcatg ttgagatcat tgccacaatg aagaagaaca 300

atgagaagag gtgtctgaat ccggaatctg aggccatcaa gagcttattg aaagcggtga 360

gccaaagaag gtcaaaaaga gctccgtaac tagagagaag ccactcgcca cagtgctgag 420

accgatggac agcagagaga cggtctctcc acctcccttt acccagtgtg cggctagtcc 480

taactgtccc tgtttctcct gaccatggtc ccatcagctg gtactcccac tacagcgtga 540

tggacaaggc ctggtcctga gacaaaagta actccagcag caaggcttcc caattctcta 600

agagctggtc cgaatcttcc ctcaggcagc tatgacggct ctcctagctc tgttccgtaa 660

gctatgtgca ggtactaatc tcttcagcat gtgccatgcc ccagcctgct ccacacaccc 720

tccttctccc tagctctaag ctcatcagtt ctgagttcac ctgagctcct ttatttcaaa 780

tgcagtccag gtgagatggc aaatcaagtt tgtcagaaca aacttaccac caccttccca 840

agggaatttc ataactcaga atactcacag gaacctagac atgcatgttt aaatattatt 900

taatgaccga ctgtacaaag tggaactcct agatgtattt tttgtacgat tttcattgta 960

tatgtaagaa cttgtgtggt taagtatgta tcaatgggta gttaaagttt acataggcaa 1020

atgctttgaa tgctacatat tacaagatgt gttggatggt tttcaaaata aaatgtactg 1080

tattgaatgt agtatgagac aaaaaagtaa taaagtaata ataactgaca tga 1133

<210> 510

<211> 2383

<212> DNA

<213> Rattus norvegicus (Norway rat)

<400> 510
gctaataagt gtgtcctttg ctgacggtac cgagaaaccc cgccccgtct gtacgtcctg 60

gtggcgcgcg ggccggctgt gggtgtggga agggcccggg tcagtcgaca cccggaaggc 120

cggggccgcc tcccgacgcc cacctactag cttctccagc tccatctttc ccgagaagcg 180

gcagggacct ccggcctcca gcttaccggg cggatcgaag cagcggtcgg gatgtactgc 240

tgggcttgct gcagtcaggc ggctcggtgc tcgggcaggc gatggagcag gtgacaggag 300

gcaacctgct ttccacgctg ctcatcgcct gcgccttcac gcttagcctt gtctacctgt 360

tccgcctcgc agtgggccac atggtccagc tgcccgctgg agcgaaaagt ccgccatata 420

tttactctcc aattccgttc cttggccacg cgatagcatt tgggaaaagt ccaattgagt 480

tcctggaaaa tgcatatgag aagtacggac ctgtgtttag ctttaccatg gtgggcaaga 540

ctttcactta ccttttgggg agtgacgcag ccgccctgct cttcaacagt aagaacgaag 600

acctgaatgc ggaagaggtc tacggtcggc tgacaacgcc cgtgtttggg aagggagttg 660

catacgacgt gccaaatgca gttttcctgg aacagaagaa aatactaaaa agcggcctta 720

acatagccca cttcaagcag tatgtttcta tcattgaaaa agaagcgaag aagtacttta 780

aaagttgggg agaaagcgga gaaagaaatg tgtttgaagc gctgtctgag ctcataattt 840

taacagctag ccattgctta catggaaagg aaattagaag tcagctcaac gagaaggtgg 900

ctcagctgta cgcagacctg gatggaggct ttagccatgc cgcctggctg ttgccaggct 960

ggctgcccct gccgagtttc aggcgcaggg acagagccca tcgagagatc aagaatattt 1020

tctacaaggc catccagaaa cgtaggctgt caaaagaacc agccgaggac attcttcaaa 1080

ctttactgga ttctacatac aaggatgggc gtcctctgac ggacgacgag atcgcaggga 1140

tgctcatcgg actgctgctg gccggacagc acacatcctc caccaccagt gcctggatgg 1200

gcttctttct ggccagagat aaaccacttc aagacaaatg ctacttagaa cagaaaacgg 1260

tgtgcgggga ggatctgcct cccttaactt atgagcagtt gaaggatctg aatttacttg 1320

atagatgcat aaaagaaaca ctaagactga ggcctcctat aatgaccatg atgagaatgg 1380

ccaagacccc tcagactgtg gctgggtaca ccattcctcc aggacatcag gtgtgtgttt 1440

ctccaactgt caatcaaaga ctgaaagact cctgggtaga acgcttggac ttcaatcctg 1500

accgctacct acaggacaac ccagcgtcgg gagagaagtt tgcctatgtg ccgtttggag 1560

ccgggcgcca tcgttgtatt ggagaaaatt ttgcctatgt tcaaattaag acaatttggt 1620

ccactatgct tcgtttatat gaatttgacc tcatcaatgg atattttccc agtgtgaatt 1680

atacaacaat gattcatacc ccagaaaacc cagtaatccg ttacaaacga agatcaaaat 1740

gaagaaagga acaaggagcc agtgtggaga cgggactgca agctgcagct tggcagagaa 1800

tgaagctttg acacagcttt catactgtac tgttttttaa gtgtgtggtt ctgaaagcca 1860

gtttgatttt aatgttttat taactcggtg atttttgtca gacctaatgg catttgaaac 1920

agttataata gttctgatag gatttcaggg aagccaagtt tatgttagaa atcgtttagg 1980

ggagcctcgg tattcagaga tgatacagaa tatagcatcc aggtaactaa cttcagaagc 2040

acacgttgcc gtagggagat tccggcttgg aactagtttg ggaagtttta agcctggtca 2100

gatgctacag aggcaatggg tcattggtgt ggttgggcca cttctgtgcg taaagatgtg 2160

agagggtgaa ggataagttt tctgcgaacg tctagatggt gtgagtgccc tttgtagtgt 2220

taactgagag caccactcca gcgagatggc agcaatcttg gaccttatct tgataacctt 2280

atttcctaaa aataataaat actaaagagt acttatgtta ttggttccag aaaaatccaa 2340

aatcaaatcc ttgtggaatt tttaatttta attaaactcg tgc 2383

<210> 511

<211> 709

<212> DNA

<213> Rattus norvegicus (Norway rat)

<400> 511
ccacgcgtcc gggaccgagc ggctgcgtgt gctgggtcct caccatgggg cagatcgagt 60

gggccatgtg ggccaacgaa caggcgctgg catctggcct gatcctcatc acagggggca 120

tcgtggctac tgcgggacgc ttcacgcagt ggtactttgg tgcttactct attgttgcag 180

gagtgctcat ctgtctgctg gagtaccccc ggggaaagag gaaaaagggc tccaccatgg 240

agcggtgtgg acagaagtac ctgaccgctg tggtgaagct gttcgggccc ctcaccagaa 300

attactacgt ccgggctgtc ctccacttac tgctgtccgt gcctgcaggc ttcctgctgg 360

ccaccatcct ggggaccgtc tgcttggcca ttgccagtgt gatctacctg ctggcagcca 420

tccggggtga gcagtggact cccattgagc ctaaacccaa ggagcggccg caggttggag 480

gcaccatcaa gcagccacct accaaccccc caccccggcc accagcggag gtccgcaaga 540

agccaagtga ggccgaagag gaggcagcct cggcaggagg accccaggtt aacccaattc 600

cagtgacaga tgaggtcgtg tgaccttcag tggctcctgc ttgagtttcc aaggtgttgc 660

tcccagaggg tggtgatgcc cactgtaata aacatggtta atagctggt 709

<210> 512

<211> 3980

<212> DNA

<213> Rattus norvegicus (Norway rat)

<400> 512
cgggccgggc tggaggggcg ggcgggcggc tacgagcatg gtcctggtgt tgcaccacat 60

cctcatcgct gttgtccaat tcctcaggcg gggccagcag gtcttcctca agccggacga 120

accgccgccg cagccatgcg ccgacagcct gcaggatgct ttgctgagcc tgggcgccgt 180

tatcgacatc gcgggccttc gacaggctgc caaggatgcc ctctcggccg tgctccccaa 240

agtggagact gtctacacct acctggtaga tggggagtcc aggctggtgt gtgaggaccc 300

ccctcatgag ctgccacagg aaggaaaaat tcgagaggct gtgatttctc ggaagcggct 360

gagctgcgat gggctgggac cttcagacct actaggaaag cccttggcca ggctggtggc 420

tccactggcc cctgacacgc aagtgctggt catacccctg ctggacaagg agactggaac 480

tgtggcagcc gtcatcttgg tgcactgtgg ccagctgagt gacagtgagg agcagagcct 540

gcaggtggtg gagaagcatg ctctggtggc cctgcagagg gtgcaggccc tgcagcagcg 600

caggcctgaa gctgttcaga acacctcagc ggatccctct gaagatcaaa aggatgagaa 660

gggatacacc gcccatgacc gaaagatcct gcaactgtgt ggagaactct atgacttgga 720

tgccacttct ctgcagctca aagtccttcg atatcttcag caggagacac aggccactca 780

ctgctgcctc ctgctggtgt cagaggacaa cctgcagctt tcctgcaagg tcattggaga 840

gaaagtgctg ggagaagagg tcagctttcc attgaccatg ggacgcctgg gccaggtggt 900

ggaggacaaa cagtgtatcc agttgaagga cctaacctct gacgatgtgc aacagctaca 960

aaacatgttg ggctgtgagc ttcgggctat gctatgtgtc cctgtcatca gtcgagccac 1020

tgaccaggtg gtggccctgg cttgcgcctt caacaagctt ggaggagact tcttcacaga 1080

tgaggatgaa cgtgcgatcc aacactgctt ccactacaca ggcacggtgc tcaccagtac 1140

cttggccttc cagaaggagc agaagctcaa gtgtgagtgc caggctcttc tccaagtggc 1200

aaagaacctc ttcacccacc tggatgacgt ctctgtcctg ctacaggaga tcatcacaga 1260

ggccagaaac ctcagcaacg cagagatctg ctccgtgttc ctgctggatc agaatgagct 1320

ggttgccaag gtgttcgatg gcggtgtagt ggacgatgag agttatgaga tccgcatccc 1380

tgcggaccaa ggcatcgcgg gccacgtggc gaccacgggc cagatcctga acatcccaga 1440

tgcatacgcc catccgcttt tctatcgcgg cgtagacgac agcactggct tccgcacgcg 1500

caacattctc tgcttcccta tcaagaacga gaaccaagag gtcatcggtg tggctgagct 1560

agtgaacaag atcaatgggc catggttcag caaatttgat gaggacctgg ccacagcctt 1620

ctccatctac tgtggcatta gcatcgctca ctctctccta tacaaaaagg tgaatgaagc 1680

ccagtaccgc agccacctgg ccaacgagat gatgatgtac catatgaagg tctctgacga 1740

tgaatacacc aaacttctcc atgatggcat ccaacctgtg gccgccattg actccaactt 1800

tgccaacttc acctatactc ctcggtctct gcctgaggac gatacttcta tggccatcct 1860

gagcatgctg caagacatga acttcatcaa taactacaaa attgactgcc caactctggc 1920

ccggttctgc ctgatggtga agaaaggcta ccgggatccg ccctaccaca actggatgca 1980

cgccttctct gtctctcact tttgctactt gctctacaag aatctggagc tctccaacta 2040

cctcgaggac atcgagatct ttgcgttgtt tatttcctgc atgtgtcacg acctggacca 2100

cagaggcaca aacaactcct tccaggtggc ctcgaaatct gtgctggctg cactctacag 2160

ctcagagggc tctgtcatgg agaggcacca ctttgctcag gccattgcta tcctcaacac 2220

ccatggctgt aatatctttg accacttctc tcggaaggac tatcagcgaa tgctggacct 2280

gatgagggac atcatcttgg ccacggacct ggcacaccac ctccgcatct tcaaggacct 2340

gcagaagatg gctgaagtgg gttatgaccg aaacaacaag caacaccaca ggcttcttct 2400

atgcctcctt atgacctcct gtgacctctc tgaccagaca aagggctgga agaccaccag 2460

aaagattgcg gagctgatct acaaagagtt cttctcccaa ggagacttgg agaaggccat 2520

gggcaaccga ccaatggaga tgatggatcg ggagaaggcc tacatccctg aacttcagat 2580

cagctttatg gagcacatcg ccatgcccat ctacaagctg ttacaagacc tgttccccaa 2640

ggcagcagag ctgtatgaac gcgtggcctc caatcgtgag cactggacca aggtgtccca 2700

caagttcacc atccgaggcc tccccagtaa caactcgctg gatttcctgg atgaggaata 2760

tgaggtccct gatttggacg tcaccagggc tcctgtcaat ggctgctgca gcctcgaggg 2820

ctgagtgatc ccctctaggg accctccctg cccggccctc ctttcacaac tctctaccag 2880

tctgtctgat gcattgggaa ccagagccat gggtcctggg ttctagacca ggatgtcccc 2940

tgtgacctgg cacatgccac ccttcctggg cttcgtcttt ctcatctgca gaaggaattc 3000

gagattcgca gtctcacaca gacttggccg tttgttcttc aagagcacag tagtgaccag 3060

tggcagtggg cttccttctg cacctctgac tgtaccgtgc ccagttctgc ccaagccact 3120

ccatctttga gccacagaac cttctgtgaa cccattagag ccccattacg tcagtatcct 3180

tgttttcctg gaggaatccc aggtctctgg agacttcgag atcctcatgg aagaaggaga 3240

ggccaccgag agagcagagg gtataaatca gtcaccagct agaagtcgcc atagggtgaa 3300

gggaccctga gggtgcctct gtgcttctgg caggactggg accctggagg aagctgacag 3360

ccaggttgct cagtgctctg caggaggcag tgctccagca gagaaggcag gaataaggga 3420

cagaaggatc ctcaagctgg gagacagtcc ctacacaccc ctgcccttca ggtgttgtga 3480

cctgaagtga gttcctttcc ctagctagcc aagacaggat cattagacag cctattctct 3540

ctttagatca aagtctccgt cattctgtgg agtccgtgag tggttgtggc atgctctgcc 3600

accctgaggc acagagagcc taacctcctt tcctctgagt gtatccccct ggtctgagcc 3660

cctctcccat tcctgccatc agagaggggc ctggcctctc tccacagggc ttctctcctc 3720

ttcaaggcca tatccacacc tgccccccgg agggctcagg agactctccc agggctgggc 3780

ctcttgggtg agtggccact agcttctcct tctgttttgt tctgtatgta ttgtgggtgg 3840

gggagggggc cacctgcctt acctactctg agttgccttt agagagatgc atttttctag 3900

gactctgtgc acctgttgtt gtatgtagtc ctgtgggctg agtgctttgt acatgagaat 3960

aaatctattt ctttctacca 3980

<210> 513

<211> 428

<212> DNA

<213> Rattus norvegicus (Norway rat)

<400> 513
caccctaacc taaacctttt gaattttgaa ttaatgggta ccctatagaa aaaatgacct 60

gcggaaaacc aaacagtgaa gggaaagaag gaaagaattc caattgacac aatttaaata 120

aaatatggct tttcaaataa aggaagagaa gtttcttttg ggcacacaat ctaaacaata 180

tttatgtata attcagctct tatttggctt aaatgggctt agtgggaaat gttatcattt 240

ttactatttt ctacagagaa aatattttcc agtttatcaa gtaactctgg actttggaat 300

tacctgtttt tatgttccag ctgtctatat agttgtttct ctgaaattgt acataaaatg 360

tttttacatt aaaatgaatt actgtataaa gtactgcatt atgcataata aagatactaa 420

tgctttat 428

<210> 514

<211> 976

<212> DNA

<213> Rattus norvegicus (Norway rat)

<400> 514
gtctggactg cagacaggcg gcacggagag accggcgagc tccgatcggt cggagctaac 60

cgctgccagg cggctgccgc ggccccgcac acgccccagt cgagcgaaga tggtgggccg 120

gaacagcgcc atcgccgcgg ggctgtgcgg tgccctcttc atagggtact gcatctactt 180

tgaccgcaaa aggcgaggtg accccaactt caagaacagg cttcgagaac gaagaaagaa 240

acagaagctt gctaaggaga gagctgggct ttccaagtta cctgatttaa aagatgctga 300

agctgttcag aaattcttcc ttgaagagat acagcttggt gaagagttat tagcacaagg 360

tgactatgag aagggtgtgg accacctgac aaatgcaatc gctgtgtgtg gacagcctca 420

gcagttgctg caagtgttac aacagactct tccaccacca gtgttccaga tgcttctgac 480

caagcttcca accattagtc agagaattgt cagtgctcag agcttggctg aggatgatgt 540

ggaatgagcc agacaccaac atgataaatc tcagtaaaat gataacagtt agctgcaggc 600

tgctctgctc ggggggataa gggcaaactg tgcttgtcat gaactgtctc acactgacat 660

ctccaaagtg aacctgaact ttggtagagc cattgtctgt tctatttatt tttccagtga 720

gaagtatttt gatagctttt cattttataa atacactgcg ttaaccaaaa gatcatggat 780

ttcgtttgtt cttgacatgc agttcaatgt aaatacagta gtattaggta gagactcctg 840

gtgattttaa aggattgaaa agctgaggaa tagttgaata atgcacattt taaagactag 900

aacattttat tgtcgttgta aaattgagta gaaacttgtg tttgtgaaaa ctgagcatta 960

aaaccttaca gagaca 976

<210> 515

<211> 759

<212> DNA

<213> Rattus norvegicus (Norway rat)

<400> 515
ggcacgagca gtgcccgagt taaggggaat tggaggcagg cgccgatcca cagcgatatc 60

gagacactca gagccacagg caccatgtcc gatcctggtg atgtccgacc tgttccacac 120

aggagcaaag tatgccgtcg tctgttcggt cccgtggaca gtgagcagtt gagccgcgat 180

tgcgatgcgc tcatggcgag ctgtctccag gaggcccgag aacggtggaa ctttgacttc 240

gccactgaga cgccactgga gggcaactac gtctgggagc gtgttcggag cccagggctg 300

cccaagatct acctgagccc tgggtcccgc cgccgtgatg acctgggagg ggacaagagg 360

cccagtacct cctcggccct gctgcagggg ccagggccag ctccggagga ccacgtggcc 420

ttgtcgctgt cttgcactct ggtgtctcac gcccctgaga ggcctgaaga ctccccgggc 480

gggaccggga catctcaggg ccgaaaacgg aggcagacca gcctaacaga tttctatcac 540

tccaagcgcc gattggtctt ctgcaagaga aagccctgaa gtgcccacgg gaggctcgcc 600

ttcttctgct gtgggtcagg aggcctcttc cccatcttct gccttagcct tcattcagtg 660

tgtcttaatt attatttgtg ttttaattta aacttctcct gtatatactc tgcctgcccc 720

ctcccctcgt gcaattcgat atacaagctt atcgatacc 759

<210> 516

<211> 1700

<212> DNA

<213> Rattus norvegicus (Norway rat)

<400> 516
agtggatgga gaccaagtcc atcaattaga agataagtct caaatggaga accggtgcct 60
ccccaagaaa gttccaggtt tctgttcctt tcggtatgga ctggctatcc ttttgcactt 120
ctgtaacatt gtcatcatgg ctcagcgagt gtgcctgaac ctcacaatgg tagccatggt 180
gaataaaaca gagccacccc atttatctaa caagtctgta gcagagatgc tggataatgt 240
aaagaaccct gtgcattcct ggagtcttga tattcaaggg cttgttctca gttctgtctt 300
cttgggtatg gttgtgattc aagttcctgt tgggtaccta tctggagcat acccaatgga 360
gaaaataatt gggtcttcat tgttcctcag ctctgtgctg tccctgctca tcccaccagc 420
tgcacaagtc ggagcagctt tggtcattgt atgccgagta ctccaaggaa tagcccaggg 480
agcagtgtca acaggccagc atggaatatg ggtcaaatgg gcacctccct tggaacgagg 540
cagacttacc tctatgactc tatcagggtt tgtaatggga ccatttattg ccctgcttgt 600
gagcggcttc atctgtgatc ttctgggttg gcccatggtt ttctacatct ttggtattgt 660
tgggtgtgtt ctgagtcttt tctggttcat ccttctcttt gatgacccca acaaccaccc 720
atatatgagc agcagtgaga aggattacat cacatcctct ctcatgcaac aggtgcactc 780
aggcagacaa tctctgccaa tcaaagctat gcttaagtct cttccactct gggccattat 840
cctcaatagc tttgctttca tatggtcgaa caatcttctg gttacataca cgccaacatt 900
tattagcacc acgcttcatg ttaatgttag agagaatggg ctgctgtcca gcctccccta 960
tctgcttgcc tacatctgtg gtatcgtagc aggtcagatg tcagacttcc tcctgtcaag 1020
gaagattttc agcgtagttg ctgtccggaa actcttcacc acactaggga ttttctgtcc 1080
tgtgattttt gtcgtgtgcc tgctctacct gagctacaac ttctacagca ccgtcatttt 1140
ccttacactt gccaactcga cacttagctt ttccttctgt ggacaactta tcaatgcctt 1200
ggatattgct cccagatatt atggatttct aaaggcagtc acagctttaa ttggaatatt 1260
tggggggcta atttcttcaa ctcttgctgg gttgatcctt aatcaggatc cggaatatgc 1320
ttggcataaa aacttcttct tgatggcagg cattaacgtg acatgcctgg ctttctacct 1380
tctgtttgca aaaggagata ttcaggactg ggctaaagaa acaaaaacca cacggctgtg 1440
aagttagtag aagagtgagc acctgagaaa catgaggtca gcatccatat tgaaaggaag 1500
aggattctgt gtgggactgc agtcatggga gtcatgaaca cctgaagacc ggtagcctga 1560
agcttttaag ggagtcattg cctcccactt tagtctgagt gattcttttg cactgtgtat 1620
tgtgtgtaac aagttatact tctgacttta aagaggaatc tgtctgtctt cctatgatcc 1680
aataaacaaa cgtattcggg 1700

<210> 517
<211> 4326
<212> DNA

<213>  Rattus norvegicus (Norway rat)

<400>  517
   tcctctgcca cctcagtaaa taagtgggtg tcgcctgaac tgaacagcaa gagaaaagaa      60
   gccagccaag tgagagcaca gctcctctgt cactgccgcc cccttgcaag tctggcggct     120
   tctcgcgggg ccagacgctg cgccccaggc gccctgcaga tggggtggac agggacccgg     180
   agctcaagcc accggtgacc cgcgcgtggc tggccgaccg gccttccccg cttgcagagg     240
   atgttaagca gaggagccct ggaaccgcat ccgacactgg ccgacgttcg ctaggctgca     300
   acttggaatg cccagggagc ggtcgggctc ctaagaccgc gtcgccgcgt ctagggaagc     360
   tgcgctccgg atttggaggc ggagacctca ggcggcggag acctccggcg gcggctgcag     420
   agggacgagc cgggcgcagg agggggcgcg ctttctccca gcgggtctca gtaatgagga     480
   gactgagttt gtggtggctg ctgagcaggg tctgtctgct gctgccgccg ccctgcgcac     540
   tggtgctggc cggggtgccc agctcctcct cgcacccgca accctgccag atcctcaagc     600
   gcatcggaca cgcggtgagg gtgggcgcgg tgcacttgca accctggacc acggccccac     660
   gcgcagccag tcgcgctcag gaaggcggca gggcgggtgc ccagagggat gatccagagt     720
   ccgggacgtg gcggccaccg gcgccctcgc aaggcgcacg ctggttgggg agcgccctgc     780
   atggccgggg tccacccggc tcccgaaagc tcggggaggg cgcgggggcc gagaccctgt     840
   ggccgcggga tgccctactg ttcgctgtgg aaaacttgaa ccgtgtggaa gggctcctac     900
   cctacaacct gtctttggaa gtagtgatgg ccattgaggc gggcctgggc gatctgccgc     960
   ttatgccctt ctcttcccca agctcaccgt ggagcagtga ccctttctcc tttctgcaga    1020
   gcgtgtgcca caccgtagtg gtacaagggg tgtcggcgct gctggccttc ccccagagcc    1080
   agggcgaaat gatggagctg gacttggtca gctctgtcct gcacatccca gtgctcagca    1140
   tagtgcgcca cgagtttccg cgggagagtc agaatcccct gcacctacag ctgagtttag    1200
   aaaactcact aagttctgat gctgatgtca ctgtctcaat cctgaccatg aacaactggt    1260
   acaattttag cttgttgctc tgccaggaag actggaatat cacggacttc ctcctcctta    1320
   cggagaataa ctccaagttc cacctcgagt ctgttatcaa catcactgct aatctgtcct    1380
   ccacaaagga ccttctaagt ttcctgcagg tccagatgga caacattagg aacagcacac    1440
   ccacaatggt gatgtttggc tgtgacatgg acagtatccg gcagatattt gaaatgtcca    1500
   cacagtttgg gctatcacct cctgaacttc actgggtttt aggagactcc cagaatgtgg    1560
   aggaactgag gacagaaggc ctgcccttag ggctcattgc tcatggaaaa accacacaat    1620
   ctgtctttga gtactacgtt caggatgcca tggagttggt tgcaagagct gtagccacag    1680
   ccaccatgat ccagccagag cttgctctcc ttcccagcac aatgaactgc atggatgtga    1740
   aaaccacaaa tctcacttct ggacagtatt tatcaaggtt tttagccaac accactttca    1800
   gaggtctcag tggttccatc aaagtaaagg gctccaccat catcagctca gagaacaact    1860
   tttcatctgg gaacttgcag cacgacccta tgggaaagcc aatgtggact cgcctgggca    1920
   gctggcaagg aggaaggata gtcatggact ctggaatatg gccagagcag gcccagaggc 1980
   acaaaaccca cttccagcac ccaaacaagt tacacttgag agtggtgaca ttgattgaac 2040
   acccatttgt tttcacgaga gaagtagacg atgaaggctt atgccctgct ggacagctct 2100
   gtctagaccc tatgactaac gactcttcca tgctggatcg tctgtttagc agcctgcata 2160
   gcagtaatga cacagtgcca atcaagttca agaagtgctg ttatgggtac tgcatcgatc 2220
   tcctggagca gctagcagaa gacatgaact ttgactttga cctctacatt gtaggggatg 2280
   gaaagtacgg agcttggaaa aatggtcact ggactgggct ggttggtgat ctcctgagtg 2340
   ggacagccaa catggcggtc acttctttca gcatcaatac cgcacgaagc caggtgatag 2400
   atttcaccag ccctttcttc tcaaccagtt tggggatctt agtgaggact cgagacacag 2460
   cagctccaat tggagccttc atgtggccac tccactggac catgtggctg ggaattttcg 2520
   tggctctcca tatcactgcc atttttctca ctctgtatga atggaagagc ccctttggta 2580
   tgacccctaa ggggaggaac agaaacaaag tcttctcctt ctcctcagct ttgaatgtct 2640
   gctatgccct tctgtttggc agaacagcag ccatcaaacc cccaaaatgc tggactggaa 2700
   ggtttctgat gaatctttgg gccattttct gtatgttttg cctttctaca tacacagcga 2760
   acttggctgc tgtcatggta ggtgagaaga tctatgaaga gctttctgga attcatgacc 2820
   ctaagcttca tcatccttct caaggcttcc gctttggaac tgtccgggaa agcagtgctg 2880
   aagactatgt gcgccagagc ttcccagaga tgcatgagta catgagaagg tacaacgtgc 2940
   cagccacccc tgatggagtg cagtatctga agaatgatcc agagaaacta gacgccttca 3000
   tcatggacaa agcccttctg gattatgaag tgtcaataga tgctgactgc aagcttctga 3060
   ccgtaggaaa gccatttgcc atcgaaggat atggcattgg tctccctcca aactctccat 3120
   tgacctctaa tatatctgag ctcatcagtc agtacaagtc tcacgggttt atggatgtgc 3180
   tccatgacaa gtggtacaag gtggttccct gcggaaagag aagctttgcc gtcactgaga 3240
   ctttgcaaat gggcatcaag cacttctctg gactcttcgt gctgttgtgc ataggatttg 3300
   gtctctccat cctgaccacc attggtgaac acatagtgca cagactgctg ttaccacgca 3360
   tcaaaaacaa atccaagctg cagtactggc tgcacacgag tcagaggttt cacagagcat 3420
   taaacacgtc attcgtagaa gaaaagcagc cacgttccaa gacaaaacgt gtggagaaga 3480
   ggtccaacct gggaccccag cagctcatgg tatggaatac ttccaatctg agtcatgaca 3540
   accaacgaaa atacatcttt aatgacgagg aaggacaaaa ccagctgggt acccaggccc 3600
   accaggacat ccctctccct cagaggagaa gagagctccc tgcctcactg accaccaatg 3660
   ggaaagcaga ctccctcaat gtaactcgga gctccgtgat tcaggaactc tctgagttgg 3720
   agaagcagat ccaagtgatc cgccaggagc tgcagttggc tgtaagcagg aagacagagc 3780
   tggaggagta tcaaaagaca aatcggactt gtgaatccta ggctgtgtct ccccccactt 3840
   tctcagcccc tggtactctg aagcccttga gacactttgt aaagcccttt tatattcctt 3900
   gacaaaggtg tggggtctgg caatgaagtg tgctgttctc tgcctaaggt gcagcagcag 3960
   cagcagcagc agttcttccc acaaaggccc ccaggtcttc agggtagtaa tcgttccgat 4020
   agtaggaagc tagggttagt cgggagtaaa gtctgtctga ggggatcggc gagctgccag 4080
   acaactctgg ctcctctcaa ccttttcacc tggtgccaca aaaaaaattc tggtttttag 4140
   ccctttctct gcacctcaac aagagataaa aagccggcgc tcatacctgt gtcttactgg 4200
   gttggttttt aatattacta caaaacaaaa ttatccaatg ttgcagattt gatacaactc 4260
   cccaatctag atctgatcta acattctgaa tgagcagggt aggagccagg aaaacgggac 4320
   agaaca 4326

<210> 518

<211> 806

<212> DNA

<213> Rattus norvegicus (Norway rat)

<400> 518
gcttgaatct aatacgtcga tcataccatg ttgaagatga gcgggtggca gcgacagagc 60

caaaataaca gccggaacct gaggagagag aaaccggtcc aattacagtc atggcagctg 120

agtctctgcc tttcgccttt gagacagtgt ccagctggga gctggaagcc tggtatgagg 180

atctgcagga ggtcctgtcc tcagatgaaa ttgggggcac ctatatctca tccccaggaa 240

acgaagagga agaatcaaaa accttcacta ctcttgaccc tgcatcccta gcttggctga 300

ctgaggagcc agggccagca gaggtcacaa gcacctccca aagccctcgc tctccagatt 360

ccagtcagag ttctatggct caggaagaag aagaggaaga tcaaggaaga actaggaaac 420

ggaaacagag tggtcagtgc gcagcccggg ctgggaaaca gcgactgaag gagaaggagc 480

aggagaatga gaggaaagtg gcacagcttg ctgaagagaa cgagcggctc aacgaggaaa 540

tcgagcgcct gaccagggag gtagagacca cacggcgggc tctgatcgac cgcatggtca 600

gtctgcacca agcatgaact gttggcatca cctcctgtct gtctctcccg gagtgtaccc 660

agcaccatca cgccagtgcc aagcatgtaa tctccagtgc acatgctgag gaggggactg 720

agggtagacc aaaggagagg ggcttgtaca ctgtacattc tttattcatt ccatacccag 780

taaagtgact ttgtgtgaaa aaaaaa 806

<210> 519

<211> 4387

<212> DNA

<213> Rattus norvegicus (Norway rat)

<400> 519
ggcacgaggg ggagccgggc atgggggccg ccctccgggg gggccggggc cgccgccgcc 60

gccgccgcgg cagcgactga agccgcgcaa caaggagggg cggggagtgg tcgccgccgt 120
ccccgggagg cgccggagct cgactgcact cgctgccagc cagccgtccc gagccgctcg 180
cagattccct gagaacaccc ggcaagtacc acgtgcctac aactgcttcc ccggtcacag 240
ctgtggggag cttggggtgc ggcagacaaa tttctgtatt cagctgccca ggcagaggag 300
aatgggggct ccacagcttg aagaatgaag acacgacaga ataaagactc gatgtcaatg 360
aggagtggac ggaagaaaga ggcccccggg ccccgggaag agctgagatc gaggggccgg 420
gcctcccctg gaggggtcag cacgtccagc agtgatggca aagccgagaa atccaggcag 480
acagccaaga aggcccgagt agaggagacc tctaccccaa aagccaacaa gcagggccgg 540
agcgaggaga tctcagagag tgagagcgaa gagaccagtg cgcccaaaaa gaccaagacc 600
gaggagcttc cgcgcccaca gtctccctcg gatctggaca gcttagatgg ccgcagcatc 660
aatgatgacg gcagcagtga ccctagagat atagaccagg acaaccgaag cacatccccc 720
agcatctaca gccctggcag tgtggaaaat gactcggatt catcctctgg cctgtcccag 780
ggcccggctc gcccctacca cccacctcca ctctttcctc cttcccctcc accaccagac 840
agcattcccc gacagccaga gtctggcttt gaaccgcacc cttctgtgcc gcctactgga 900
tatcatgctc caatggagcc ccccacatct cggttattcc agggcccacc tcctggagct 960
cctcccccac acccacagct ctaccctggg agtgctggtg gaggtgtttt atctggaccc 1020
cccatgggtc ccaaaggggg agcagccgcc tcctcagtgg gtccccctag tgggggcaag 1080
cagcaccccc cacccactac cccaattcca atatcaagct ctggggccag tggcgctcct 1140
ccagcaaagc cccccaatac tccagtgggt gctgggaact taccttctgc tccaccacca 1200
gctactttcc cccacgtgac accaaacctg cctcctccac ctgccctgag acccctcaac 1260
aatgcctcag cctctcctcc tggcatgggg gctcagccaa tccctgggca tctgccctct 1320
ccccatgcca tggggcaggg tatgagtgga cttcctcctg gcccagagaa gggcccaacc 1380
ctggccccct ctccccaccc tttacccccg gcttcttcct ccgctcctgg gcccccaatg 1440
cggtacccgt attcatcctg ctccagtagc tctgttgcag cgtcttctag ttcctccgcc 1500
gccacctccc agtacccagc ttcccagacc ctgcccagtt accctcactc cttcccccca 1560
ccaacaagta tgtctgtctc taatcagcca cccaagtaca cccagccttc tctcccatcc 1620
caggctgtgt ggagccaggg cccacctcct cctcctcctc cttatggccg cctcttgccc 1680
aacaacaaca ctcatccggg ccctttccct cctaccgggg gtcaatccac agcccacccg 1740
ccagcccctg cacatcacca tcaccagcag cagcagcagc cacagccaca gccacagccg 1800
caacaacatc atcatggaaa ctctgggccc cctccacctg gggcatatcc tcacccccta 1860
gagagcagta actcccacca tgcacaccct tacaacatgt caccctccct ggggtctttg 1920
aggccctacc caccagggcc agcacacctg cctccgtctc atggccaggt gtcttatagc 1980
caagcaggtc ccaatggtcc cccggtttct tcttcttcca actcttccgg gtcttcgtct 2040
caagccgcct attcatgctc acacccttct tcatcccagg gcccccaagg ggcatcctac 2100

cccttcccac cagttcctcc aatcaccacc tcctcagcta ctctttccac tgtcatcgcc 2160
accgtggctt cctcgccagc aggttataag acagcctcac cacctggccc ccctcagtac 2220
agcaagagag ccccatcccc agggtcctac aagacagcca ccccgcctgg atacaagcca 2280
gggtcaccac cgtccttcag aacagggacc ccacctggct atcgaggcac ctctccgcca 2340
gcaggcccag ggaccttcaa gccaggttca cccaccgtgg ggccggggcc cctaccaccc 2400
gcggggcctt caagtttgtc atctctgcct ccaccacccg cggccccgac tacagggccg 2460
cccctgactg ccacgcagat caaacaggaa ccagctgaag agtatgaaac ccccgagagt 2520
ccggtgcctc cggctcgcag cccctcgccc cctcctaagg tggtggacgt gcctagccat 2580
gccagccagt cagccaggtt caataagcac ctggaccgtg gcttcaactc gtgcgcgcgc 2640
agcgacctgt actttgtgcc gctggaggga tccaagctgg ccaagaagcg cgcggacctg 2700
gtggagaaag tgcggcgcga ggccgagcag cgcgcgcgcg aagaaaaaga gcgcgagcgc 2760
gagcgggaac gtgagaagga gcgcgagcgc gagaaagagc gcgagctgga gcgcagtgtg 2820
aaattggccc aggagggccg tgctccagtg gagtgcccgt ccctgggtcc agtgccccat 2880
cggcctccct ttgaacctgg cagcgctgtg gctaccgtgc ccccttacct gggtcctgat 2940
actccagccc tgcgcactct cagtgaatac gcccgacctc atgtcatgtc tcctggcaat 3000
cgcaaccacc cattctatgt gcccttgggg gcagtggacc cggggcttct gggttacaat 3060
gtcccagccc tgtacagcag cgacccagct gcccgagaac gggagcggga agcccgtgaa 3120
cgtgacctcc gtgaccggct caagcctggc tttgaggtga aacctagtga gctggaaccc 3180
ctacatgggg tccccgggcc aggcctggat cccttccccc gacacggggg cctggctcta 3240
caacctgggc cacctggcct gcatcctttc ccttttcatc cgagcctggg gcccctggaa 3300
cgagaacggc tagcgctggc agctgggcca gccctgcgtc ctgacatgtc ttatgctgag 3360
cggttggcag ctgaaaggca gcatgcagaa agggtggcag ccctgggcaa tgacccacta 3420
gcccggctgc agatgctcaa cgtgactccc catcatcacc agcactccca catccactct 3480
cacctacacc tgcaccagca ggatgccatc catgcggcct ccgcctcggt gcaccctctc 3540
attgaccccc tggcctcagg gtctcacctt acccggatcc cctacccagc tgggaccctc 3600
cctaatcccc ttcttcctca ccctctgcac gagaacgaag ttcttcgtca ccagctcttt 3660
gctgcccctt accgggacct accggcctct ctatctgcgc caatgtcagc cgcccatcag 3720
ctgcaggcca tgcacgcaca gtcagccgag ctgcagcgct tggcactgga acagcaacag 3780
tggctgcacg ctcatcaccc attgcacagc gtgccgctgc ctgcccagga ggactactac 3840
agtcacctga agaaggagag tgacaagccg ctgtagaacc tgccatccag acagcaccca 3900
ctgctccttc atccagacct tggaggacca cccccgacct tttgacccca tcccaccccc 3960
aagccgagga cagggtgctg cccacttgca gagcttctgc agccgggcag agggagggag 4020
ggaagaaggg acagacaagg tcagggcccg gggaagtggc aagggtgggg acagaaagtg 4080
gacagtatct tggaccaggt ccctcctccc atcccctgct tttcttctgt atgcccaatc 4140

cttggtggcc actgctcctc ccctaacccg ttggtgtgat ttttttcatc tgttagatgt 4200

ggctgttttg cgtagcattg tgtgctgccc ctgccccatc cctgtgtgtg caccccctcg 4260

gcgatatgtg ccccttgccc gtcccacatt aataatttat atatataaat atctatatga 4320

tgctctttaa aaacatcctg accaaaacat cccaacaaaa acatcctcac ggtgccccaa 4380

aaaaaaa 4387

<210> 520

<211> 2657

<212> DNA

<213> Rattus norvegicus (Norway rat)

<400> 520
   gttttttttt tttttttttt tttttttttt tttttttatg aagaataagc gatccgtttg 60
   ggatttattg catttaaaga cggcagatta ttaaaacagt aggtatgata aatacagtaa 120
   gaaaataact ttaaatctca aacaaaacag tgaacagaat tcaagactaa gcaatgtaaa 180
   ggcaataagg taacaaaatt agtgctttac tgggtacagg gaaagtaact gaagtctatc 240
   tcaaactctt cccggctaca tttgagggct taagttactt tagcaagtat gtctttataa 300
   caattagaaa tataactaag gatataaacc cagtttattt aaaaattaaa gttatttgct 360
   tagtggtaca taaaaggctt cagaaaattc aagtttaaca aaaaaaaaaa aaaaaggaag 420
   tgtaatcaaa gaaagtgcac ttaaagccgt ttgccagcaa ttaaagggct cttccaggct 480
   ggaggtgtgg ctcagcccta aagcgcttgc ccagcatgtg caaggccctg ggttcagttc 540
   ccagcaccgc aaaagaaaga acaagtggca attactcctt ctagtatagg atgatctctc 600
   agtcacaaaa gaacatagta ataaaatcag tcttcctgta ggcactgtga acctttaatt 660
   aaaaaaaaat aacctaaacc agacccagcc ctctgaattt tcatctactt cttcctttta 720
   gtggcttttg taaacttcgt aactaggagt gaatgctctt aagagaaacg attgcctttt 780
   taaattcaca ttaattccgt agtggagagc ctcgatgtgc ctgtgtcttt ttatcttagt 840
   agagaacacc accgagtgta aatttttcca acagtcatct gttgttctct cagtagactc 900
   agtagactgt caactcagta actgaatgta aattgatgac tagttttgct tagaaataaa 960
   agaaaaagaa tgacagagtt cactgcgcat tctacatgta ttaaacagat aatacctaac 1020
   tccccacagg tagtgacatt aatacatgtg cgccttctga cagtgcttta gtgaagtgcc 1080
   caaatctgga taacccaagg gaggcacacc caagactggc tccaccttcg tattttccgt 1140
   cagaagagga gagtagtcag tccctgttaa acagggtagg actgatcgca aggtgcgcag 1200
   atccgagagc cacgtgactt cataaatgcc cagggtacac agctcagggg aggggcactc 1260
   agtttaaatc tggtcggttc cattctcacc caacttggag tagtcctttc tccaactaca 1320
   taccgagaac gtctgtgcct tagtagataa ctttcttaag tgccctgaaa gatcctctgg 1380
   taatactagc gttttgctga cgtgatcaaa gtaaagcaag tagtccactt tttaagaaaa 1440
   gattccaagt taatgagcac agttaaagct tctaggtgac acctttggcc tcagactttc 1500
   gagctgggtt cctaacgtga cttcccttcg atgttcaagt ctaacaggtt tagttagaac 1560
   tgctttgcac actgggaagc acaaggcaca cccaaatgac attctttaaa ccagaactgg 1620
   gccgtcccat aacagagcca gcctttcagt gagtgctggg tgctgggtgc tgggtgctgg 1680
   gtgctaagtg ctctcttgag caagagaaat gtaacgcaaa ggagccgccc cttggacctc 1740
   cagtgaaaca gtctgtccaa tgagatgatc tcgagaggcg cgtcaccgct gggctgttgg 1800
   aggcaatgta aacattttag gctgtggcac agctgtttcc taaggatttg tccagcatta 1860
   gccggagggg ctgcgagtga tcacttctaa gtattcagtg cagtgaaagg cgagtggaac 1920
   tctgacttca tagtcacaac tcattacttt aggaggaaag catttctgtt cggtagcgta 1980
   gagggcctac tgccatttgc attagtgaga ataatcaacc tgagaaccag acagaaaggt 2040
   gcaaacacag ctctcctttg gcacaaggac attaagtggt gaaggcatca gactccagca 2100
   tcaactggaa caacctgaaa ggcaagaagt cctgccatac tggagtagac agaaccgccg 2160
   gctggaacat ccctatgggc ctgctgttca gcaggatcaa ccactgcaag ttcggtgagc 2220
   agagccggga agggagattg ggaggggcca tgcagaaaaa aaggggccct aagaagggcc 2280
   ataggtttgg ggtccataaa agtgaagccc ctcaaaaatg aacctcaagg aaatagggaa 2340
   gaggactagg aggaaccagg ggctaaggaa tggagtcagg acggttgtct ttattgcaaa 2400
   taatggcatt agtggctata cattcgcttg tctctctcat gatggagatc agaggtgagc 2460
   attctgtggc tgataaggtc ttgagggctc cttccttctt tctggaccat catttccatc 2520
   atagtacttc cacattcaaa aagcaacctt gctgaggcag tcacaattca ggcaagaacg 2580
   agagcaagtg gaaatgtaca ccaaccgtgg tcagtgtttg aaaagagcct tctgctgggt 2640
   tcggtcccca gctctga 2657

<210> 521

<211> 1998

<212> DNA

<213> Rattus norvegicus (Norway rat)

<400> 521
ggtgctgctg ctgcatgtct gactgagaag ggagccgagc atctgaggag caagacatca 60

gaaggctgga gtcccttccc tgactaggcc agagctaaaa ggacaaggct cagggctagc 120

ctctgcctat ctagttcttt tctccctgaa gcttacacca tcctgtggga gccgatgccc 180

gtctcatttt gactcagagt ctgtccctct gcttgtcttc tacaaaccat gtggctgtac 240

ctgctggcac tggtgggcct gtggaacctc ctgcgcttgt tcagggagag gaaggtggta 300

agccatctcc aagacaaata tgtcttcatc acgggctgtg actcaggctt tgggaacctg 360

ctggccagac agctggacag gagaggcatg agggtgctgg ctgcatgtct gactgagaag 420

ggagccgagc agctgaggag caagacatcg gacaggctgg agacagtgat ccttgatgtc 480

accaagacag agagtattgt ggcagccact cagtgggtga aggagcgagt tgggaacaca 540

ggactctggg gcctggtcaa caatgctggc atctccggcc acctgggtcc caatgagtgg 600

atgaacaaac agaacattgc aagtgtgctg gatgtgaacc tgttgggcat gatcgaggtg 660

actctgagca cggtgccctt agtgaggaag gcgaggggcc gtgtggtcaa cgttgccagc 720

atcgcaggcc gactgtcttt ttgtggtggt ggttactgca tctccaagta tggtgtagag 780

gccttctcag actccctcag gagagagctc tcctattttg gggtcaaggt ggctattgta 840

gagcctggct tcttcaggac tgatgtgacc aatggtgtca cattatcatc aaacttccag 900

atgctgtggg accagaccag ctcagaagtc agggaagtct atggcgagaa ctacctggca 960

tcttatctga aaatgctaaa tggattggac cagaggtgca acaaagatct gtctctggtg 1020

actgactgca tggagcacgc cctgacttcc tgtcaccctc gcacccggta ctcagctggt 1080

tgggatgcca agttcttcta cctccccatg agctatctgc ccacctttct tgtggatgcc 1140

cttttctact ggacttctcc aaagcctgag aaagctctct gaagtcttta cctatgtaca 1200

taactgagga gatgttggta cagtgttagg gaggggatcc tgaggaggag gtcggagtgc 1260

tgaggaggga gggaggtcat tcctctgggg tttagtccac actcttcatt ttgctaattt 1320

tcctgtgcta ctactcaagg ctgactcgac tgacccaaga aatagccaaa gaattctgcg 1380

aagaagttca aatacaaaag tgctggcagt tgcctagcct ctgggcacca tgacctacca 1440

tggcgatcca cacaaatggg gagtttgaga ccagcttgta ccaaacactg ccctgggtcc 1500

ccctgtgcag gaagtgcaca tgccctggct agtttgtacc ctatgcagca tagaagtgtc 1560

cctgctctga agtcacctgt gaccctggtg tcaggtttga gaagaatcac taataattct 1620

aacactcatg cttgaaggcc aggtcgcagc ctgggtggga gagtcggtta cttgtggtga 1680

gaatatagta gaggatagag acattaattt tttttaaatt tactttgaag tcatggctct 1740

tcttttcttt tttggagtct tttccttttt agagcatagc atgaggtgac ctcttgttcc 1800

ctgtgattta aacaatggca ttgctggttg gtgaggtttg gattgcaggg gtaaggggaa 1860

tgtcctgaga agggtgtgca taggccagca ggaatttcct tgcccgtctc agcccttcta 1920

gccactgagg tgggaataac tgctgtccat tcaaaactta acaacaaccc ctaaggcata 1980

agagcatgca tgcaggcc 1998

<210> 522

<211> 807

<212> DNA

<213> Rattus norvegicus (Norway rat)

<400>  522
gtatataaga aattctcaag ttaataaaaa aaaaagaaat gtttttcaca aaggcagtat      60

acttgtagca ggccatgcct cggtaaataa acagataaaa ttgagctgag atgttcccaa     120

tgcccatttg tcctgtgtat ctttcattgt gttcttgcca acatctaggg tggggtaatg     180

gaatagtcaa tgagaaggtc accagcagcc caagcacgaa ctctggtgtt caatctagac     240

aatgacagac aggaacaact agagttgcat aaccttgagg agaggtgggt ttagtctcaa     300

aagtcctgct ggagtgttca gtgaagcaga cactgattag accaggacca tggaacctag     360

tgtcctactt ctccttgctg tcctcctcag cttcttgcta ctcctggtca ggggccatgc     420

aaagatccat ggtcgtcttc caccaggacc ctgccctgta ccccttttgg gaaatctctt     480

gcagatggac agaagaggcc tcctcaagtc ttttattcag ttgagaccta tatggggtct     540

tagtgtgcgc ttctctgtgc ttttatcttt ggaggtcact cagtggaaaa gcaagggact     600

ccttccaggc tgtaggttca tgttggaagg ttgctggggg gagagtgaga tgctgactgg     660

tgctatatgc tgactcttgt tcaaatcaat gtgtgagtct tgttttattg gaattttttg     720

gggcactttg cttggaggtg tagagctatt gaggtgtaga tgtgtaagca cgtataattt     780

cacttgttgg agtttgtcca ttctaga                                         807

<210>  523

<211>  1139

<212>  DNA

<213>  Rattus norvegicus (Norway rat)


<400>  523
cctgctgtag cttgcccatc tcccgctccc acaatccggc gtgatggctg ccgccgttcc      60

tggcgccgtt tcgctctccc cactgctgtc cgttctcctt ggcctgctgc tcctctctgc     120

tcctcatggc gccagcggcc tgcacacgaa gggcgccctt cccttggaca cagtcacttt     180

ctacaaggtc attcccaaaa gcaagttcgt cttggtgaag ttcgacaccc agtaccccta     240

tggagagaag caagatgagt ttaagcgtct ggctgagaac tcagcctcca gcgatgatct     300

cttggtggca gaggtgggga tctcagacta tggtgacaag ctgaacatgg agctgagtga     360

gaagtacaag ctggacaaag agagctaccc agtcttctac ctcttccggg atggggactt     420

tgagaatcct gtcccataca gcggggcagt taaagttgga gccatccagc gctggctcaa     480

ggggcaggga gtctatctgg gcatgcctgg atgtctgcct gcgtacgatg ccctggcggg     540

ccagttcatc gaggcctcca gcagagaggc ccgccaggcc atcctgaaac aggggcagga     600

tggcctctca ggtgtgaagg agacagacaa gaagtgggcc agtcagtacc tgaagatcat     660

ggggaagatc ttggaccaag gtgaagactt cccggcctcc gagctggccc ggatcagtaa     720

gctcattgag aacaagatga gtgagggtaa gaaggaagag ctgcagagga gcctcaacat     780

cctcaccgcc ttccgcaaga aaggcgccga gaaggaggag ctgtgagggc accaggctgc     840

tagggtttgc tgagggcggg gagggagagc cgcctgctga ctgtggggcc ctgtgggtgg 900

agggggcagt ggagcaaagc agtcctgagc cagagacctg tgccccgagt gcctggacac 960

tgaggctgct gagaccatac cctggacatc cttggtgcag tttgtgggga gcctgtgtgc 1020

caagacaaga tggctgtgtc cccacaggag ctggtactat agaggaaggt ccttgacagg 1080

tatagttctg atcaaataaa agtctgtttt gggttaaaaa aaaaaaaaaa aaaaaaaaa 1139

<210> 524

<211> 1470

<212> DNA

<213> Rattus norvegicus (Norway rat)

<400> 524
gcggctacgg cgtgcgggga gctgaggtaa agccggggct agtacggtac caggcggtgc 60

acgagctggc taagccagat ttgtaaaaat ggagtttcaa gccgtagtta tggccgtcgg 120

tgggggatct cggatgacag acttgacttc cagcattccc aagcctctgc ttccagttgg 180

aaacaaacct ttaatttggt acccgctgaa cctgcttgag cgagttggat ttgaagaagt 240

catcgtggtt acaaccaaag atgtccaaaa ggctctgtgt gcagagttca agatgaaatt 300

gaagccagat attgtgtgca ttcctgatga ggcagacatg gggactgcag attctctacg 360

ccacatatat ccaaaactta agactgatgt gctggtcctg ggctgcgatc tgataacaga 420

tgttgcctta cacgaggttg tggacctgtt cagagcttac gatgcatcac ttgccatgtt 480

aatgaggaaa ggacaagaga gcacagaacc agttccaggt cagaagggga aaaaaaaaac 540

agtggagcag cgtgacttca ttggagtgga cagcacagga aaaaggctgc tctttatggc 600

taatgaagca gacttggatg aagagctggt cattaaggga tccatcctgc agaagcaccc 660

taggatacat ttccagacag gccttgtgga tgctcatctc tactgcttga agaaatatgt 720

tgtggatttc ttaatggaaa ataagtcaat cacttctatc cgaagcgaac tgattccgta 780

cctagtaaga aaacagtttt cctcagcctc ctcacagcaa aggcaggaag acaaagaaga 840

ggatctaaag aaaaaggagc ccaagtcttt agatatttac agttttataa aaaaagacaa 900

cactctgact ttggctccct atgatgcctg ctggaatgct tttcgaagag acaagtggga 960

agatttgtct agatcacagg ttcgctgcta tgtccacatc atgaaggaag gactttgctc 1020

tcgagtaagc acactgggac tctacatgga agccaacaga caggtaccca aattgctgtc 1080

tgttctttgt cctgaggaat caatgatcca tccatcagcc cagattgcca acaaacacct 1140

gattggagcc gacagcctta tcgggtcaga tacacaagtt ggagagaagt catctattaa 1200

gcgatctgtc attggctcat cctgtgtcat cagagacaga gtaactgtta ccaactgcct 1260

tctcatgaac tctgtcaccg tgggagaagg gagcagcatc catggcagcg tcatcttcaa 1320

caacgccgtg gtcgaggcgg gcgcggagat cagggactgt ttaattggaa gtggtcagag 1380

gattgaagcc aaagctaaac gcatgaacga ggtgatcgtg ggaaatgacc agctcatgga 1440

gatctgaatc ctgaacaagt ctgaccccac 1470

<210> 525

<211> 238

<212> DNA

<213> Rattus norvegicus (rat)

<400> 525
ttggctcatc ctgtgtcatc agagacagag taactgttac caactgcctt ctcatgaact 60

ctgtcaccgt gggagaaggg agcagcatcc atggcagcgt catcttcaac aacgccgtgg 120

tcgaggcggg cgcggagatc agggactgtt taattggaag tggtcagagg attgaagcca 180

aagctaaacg catgaacgag gtgatcgtgg gaaatgacca gctcatggag atctgaat 238

<210> 526

<211> 1806

<212> DNA

<213> Rattus norvegicus (Norway rat)

<400> 526
ggaatatcag ccatgcttgt cacagcgggt tccctactag gggccatttg gaccgtgctc 60

catctccgga ttttgctgct ggctgctgtc acctttctgt tcctggctga tttcctcaaa 120

caccggcgcc ccaagaacta cccgccaggg ccttggcgcc tgcccttagt gggttgcttg 180

ttccatttgg atcctaaaca gccacaccta tcccttcagc agtttgtgaa gaaatatggg 240

aatgttttaa gcctggattt tgctaacatt ccttcagtgg ttgtaactgg catgccctta 300

atcaaagaaa tctttactca aatggagcac aattttttga atcgccctgt cactcttctc 360

cgaaaacatc tctttaacaa aaatggattg atcttctcca gtggccagac atggaaggag 420

caaaggaggt ttgccctgat gactctgagg aactttggac taggaaagaa gagcttagag 480

cagcgaatac aagaggaagc ctaccacctt gtggaggcca taaaagatga gggaggactg 540

ccttttgacc ctcacttcaa tatcaacaaa gcagtctcta atatcatttg ctctgtcacc 600

tttggggagc gctttgagta ccatgacagt cagtttcagg agatgctgag gctactggat 660

gaggccatgt gtttggagtc atcaatgatg tgccagctct acaatatctt tccaaggata 720

cttcagtatc ttcctggatc acaccaaaca cttttcagca actggagaaa actgaagttg 780

tttatttctg atattattaa gaatcaccgg agagactggg atccagatga gccaagggac 840

ttcattgatg ctttcctcaa ggaaatggca aagtacccag acaagactac tacaagtttc 900

aatgaagaaa acctcatctg cagtactttg gatcttttct ttgctggtac agagacaaca 960

tccactacac tgcgctgggc tctgctctgc atggccctgt acccagaagt ccaagaaaaa 1020

atgcaggctg agattgacag agtgattggc caaggaagac agccaaacct ggcagaccga 1080

gactccatgc cctacaccaa cgccgtcatt cacgaggtgc agagaatagg caacatcatc 1140

ccctttaatg ttcccaggga agtggcagtt gacacctatt tggctggatt taacttgcca 1200

aagggaccct aaagagtggg ccaccccgga caccttcaac ccagagcact tttggagaa 1260

tggacagttt aagaagagag aatcctttct gcctttctca atgggaaagc gagcatgtct 1320

tggagaacaa ctggccaggt ccgagctgtt catttttatc acttctctta tccaaaaatt 1380

taccttcaag cccccggtca acgagaaggt tagtctgcaa ttcagaatgt ccgtcaccat 1440

ttccccagtc agtcaccgcc tttgcgcaat tcctaggctg tgatgtcgaa gaggaaagta 1500

tgaagaaatg gcatctgcat ggaggacact ggtgtctgct cacattgagg ggatagaatt 1560

aaagatgact gagggagtgt gtgcaatgag actaaggaaa ttgaatcaag ttatgggttc 1620

attaagtaga aagccacttg tgagctcaat ggtacagctt cacgactgac atcttgcaga 1680

gaaaaatgat tcttccagaa aagaaaatga ttacgggatc atggagaatg gattaagtga 1740

ctatgtaaat cataaaaacg atcataaaat ttatacettt actttgaaaa aaaaaaaaaa 1800

aaaaaa 1806

<210> 527

<211> 847

<212> DNA

<213> Rattus norvegicus (Norway rat)

<400> 527
acgagctaga cctccctagc tccgcccgcc gcgtgctccc gcctcccact cggaatgcct 60

gtctggggcg gtggaaataa gtgcggggcc tgcgggagaa ccgtgtacca cgctgaagag 120

gtgcagtgtg atgggcggac gttccaccgc tgctgctttc tgtgcatggt ttgcaggaaa 180

aatttagaca gcacaacagt ggcaattcat gatgaagaga tctactgcaa atcatgctac 240

ggaaagaagt atggaccaaa aggctatggt tatggccagg gcgctggcac gctcaacatg 300

gaccgtggtg agaggctggg catcaagcca gagagtgctc aacctcacag gcctacaaca 360

aatccaaaca cttctaaatt tgcccagaaa tatggaggtg ctgagaagtg ctccagatgt 420

ggggattctg tgtatgctgc tgagaagatc attggagctg gaaagccctg gcacaaaaac 480

tgtttccgat gtgccaagtg tgggaagagt ctggagtcta caactctgac tgagaaggaa 540

ggtgaaatct actgtaaagg gtgctacgca aagaactttg ggcccaaggg attcggctat 600

ggtcaaggag caggggccct tgttcatgct cagtagtggt gtaaacccag taagcatggc 660

aaagaacctc cattaatgtg gatggcctta ccgcactcag gctgtgcatc ggccagcact 720

cagcactgtg tagcacacac gctatgtgca caatcgggct ggacaggaag cactacactc 780

tcctgcccat ccgctaacgt ttaagaacgt tcttttacat ttggaataaa attttggttt 840

gatttga 847

<210> 528

<211> 1563

<212> DNA

<213> Rattus norvegicus (Norway rat)

<400> 528
cgcctcgggg gctggagcca cttggtagaa gaacagcttc tttggatttt ttttccccgg 60

tgggatactg gcagttcaaa gcgaaaccgt tgaaatgcat aacctgcaaa cactgccccc 120

taaaccaccc aagcccactt ctatagccaa caacagcatg aacaacaata tgtccttaca 180

ggatgctgaa tggtactggg gagatatctc aagggaagaa gtaaatgaaa aactccgaga 240

cactgctgat gggacctttc tggtacgaga tgcatccact aaaatgcacg gtgactacac 300

tctcacacta aggaaaggag gaaataacaa atcaatcaaa atctttcacc gagatgggaa 360

atacggtttc tctgatccat taaccttcaa ctctgtggtt gaattaataa cccactaccg 420

gaatgagtcc ttagctcagt acaatcccaa gctggatgtg aagttactct acccagtgtc 480

taaataccag caggatcaag ttgtcaagga agataatatt gaagctgtag ggaaaaaatt 540

acatgaatat aatactcaat ttcaagaaaa aagtcgggaa tatgatagat tatatgagga 600

atacatccgt acttcccagg aaatacagat gaaaagaaca gctatcgaag catttaatga 660

aaccataaaa atattcgaag agcagtgcca aacccaggag cggtacagca aagaatacat 720

agagaagttt aaacgcgaag gcaacgagaa ggaaattcaa aggataatgc ataatcacga 780

taagctgaag tctcggatca gcgagatcat cgacagcagg aggagactgg aggaagactt 840

gaagaagcag gcagccgagt atcgcgagat cgacaagcgc atgaatagca ttaagccgga 900

cctcattcag ctgagaaaga caagagatca atacttgatg tggctgacgc agaaaggggt 960

ccggcagaag aagctgaacg aatggttggg aaacgaaaac acagaagacc aatactcact 1020

gctagaagat gacgaggatt tgccccacca tgatgagaag acgtggaatg tggggagcag 1080

caaccgaaac aaagccgaga acctattgcg agggaaacga gatggcactt tcctggtccg 1140

ggagagcagt aagcagggct gctatgcctg ctctgtagtg gtagatggcg aagtcaaaca 1200

ttgcgtcatc aacaagactg ccaccggcta tggcttcgcc gagccctaca acctgtacag 1260

ctccctgaaa gagctggtgc tacattacca acacacctcc ctggtgcagc acaatgactc 1320

cctcaatgtc acactagcat acccagtata tgcacaacag aggcgatgaa gcgctgccct 1380

ccctcggata cagttcctca ccttcaagcc acccaaggcc tctgggaagc aaagggctcc 1440

tctccagccc gacctgtgaa ctgagctgcg gaatcgaagc cggatgtctg cacatgggac 1500

tagagctttc ttggacaaaa agaagtcggg gaagacatgc agcctagggc tgttggatga 1560

ccg                                                                1563

<210>  529

<211>  530

<212>  DNA

<213>  Rattus norvegicus (Norway rat)

<400>  529
tctagagaag ggggagcggt tggcacagcc ccagatctgc accattgacg tctacatggt      60

catggtcaag tgctggatga ttgatgagaa tattcgccca acctttaaag aactagccaa     120

tgagtttacc aggatggccc gtgacccacc aaggtatctg gtcatcaaga gagcgagtgg     180

gcctggaaca cctcctgcgg cagaaccctc tgttctgacc accaaagagt tgcaggaggc     240

agagctggag ccagaactgg acctagacct agacttggag gcagaggagg agggcctggc     300

cacctccctg ggttctgctc ccagcttgcc tacgggaacg ctcacccggc cacgtgggag     360

ccagagcctt ttaagtccct catctgggta catgcccatg aaccagagca gtctcggaga     420

ggcttgtctg gattctgcgg ttttgggggg tcgtgaacag ttctcccgtc ccatctctct     480

gcacccaatt ccacgggggc gtccagcatc agagtcatca gaggggcata                530

<210>  530

<211>  1728

<212>  DNA

<213>  Rattus norvegicus (Norway rat)

<400>  530
aatctagcaa aatcaatcca taatctctat ttatttgttg aaaatgttga tcttaaccaa      60

atcagtagta gtggtgcgtc ttggatccaa aatgtggata ccaaatatca aatcagaatc     120

cagatacaag aaaaactcca gcatctcaga acacagattc ataatataga cattcaacag     180

cttgctgcag agttaaaaca acagattgaa gctcttgatg tcccaatgca tttagatcaa     240

ctgagaactg caattctatt ccaaagaata agtgtcatta ttgagcgtgt caaatacttt     300

gttatgaatc ttattgaaga ttttaaagta actgagaaaa tcaatacttt tagagttata     360

gtccgtgagt taattgagaa atatgaagta gaccgacaaa tccaggtttt aatggataaa     420

tcaatagagt tggcccacag atacagcctg agtgagcctc ttcagaaact aagtaatgtg     480

ctacagcaaa ttgagataaa agactactat gataaattgg ttggttttat tgatgacact     540

gtcgagtgga ttaaagcagt gtctttcaaa aatatcattg aagaactgaa tagattgatt     600

gacatgtcgg taaaaaagtt gaaagccttt gattatcacc agtttgtaga caaaaccaac     660

agcaaaatcc gtgagatgac tcagagaatc aatgctgaaa tccaagctct cgaactccca     720

cagaaaactg aagcattaaa actgtgggta gaagacttca aaaccacggt ctccaactcc 780

ctggaaaaac tcaaggacac caaagtaact gtggtcgttg attggctgca ggatggtttg 840

gctcaaataa aagcccaatt ccaagatgct ttagaagatg tacgagaccg aatttatcaa 900

atggacattc agggggaact ggagcgctgc ttgtctctgg taagccaagt ttacagcaca 960

gtggtcacct acatttctga ctggtggact ctgactgcta aaaacataac agactttgca 1020

gagcaatatt ccacccaaaa gtgggctgag agtgtgaaag cactggtgga acaaggattc 1080

attgttcctg aaatccaaac atttctgggg accatgcctg catttgaggt cagtctccac 1140

gctctccaag aagctaactt tcagactcct gactttatag tccccttgac agatttgagg 1200

attccatcaa tttggataaa cttcaaaatg ttaaagaatg taaaaatccc attgagattt 1260

tccactccag aattcactct cctcaacacc ttccgtgtcc gttcctttac aattacgttg 1320

ctggaaataa aagcaaagat cattcgaact atcgaccaaa tgctgagcag tgagctacag 1380

tggcctcttc cagaagtgta tttgagagcc ctggagatgg tgaacatttc tcttgcaaga 1440

ctctctctgc cagacttcca tgtaccagaa atcacaattc cagaattcac aatcccaaat 1500

gtcaatctca aagatttaca ggttcctgat cttcacatac cagaattcca gcttcctcac 1560

ctctcatgca caactgaaat acctgctttc ggcaaactac atagcgtcct gaaaatccaa 1620

tctcccctct ttatattaga tgctagtgcc aacatacaga acataactac ttcagagaac 1680

aaagcagaga ttgtggcttc tgtcactgcc agaggagagt ccaaattt 1728

<210> 531

<211> 3726

<212> DNA

<213> Rattus norvegicus (Norway rat)

<400> 531
atgcctcact tcaccgtggt gccggtggac gggccgcgac gcggcgacta tgacaacctc 60

gaggggctca gttgggtgga ctacggggag cgcgccgagc gggaagactc ggatggacag 120

ggtaaccaca gagagaatag tcccttcctt agccctttgg acgcctccag aggaaatgac 180

tactatgacc ggaacctggc actgtttgag gaggagctgg acatccgccc aaaggtatca 240

tctctcctgg gcaagcttgt cagctatacc aacctcaccc aaggagccaa ggagcacgag 300

gaagctgaga gtggagaagg tggccgtcgg agagccgcca aggcacccag catgggcacc 360

cttatgggag tgtacctgcc ctgcctgcag aatatcttcg gggtcatcct cttcctgcgg 420

ctgacctgga tggtgggcac agctggcgtg ctgcaggctc tcctcattgt cctcatctgt 480

tgctgctgta ccctgctgac agccatctcc atgagtgcca tcgccaccaa tggtgtggtt 540

ccagctggtg gctcttactt catgatttcc cgctctttgg gaccagaatt cggaggtgct 600

gtgggcctat gcttctacct ggggaccaca tttgcagcag ccatgtatat cctaggagcc 660

attgagatct tgctgaccta cattgctcca ccagctgcca tcttttaccc atcgggcaca 720
cacgacatgt caagcgccac cttgaataac atgcgggtgt acggaaccat tttcctgact 780
ttcatgaccc tagtggtgtt tgtcggtgtc aagtatgtga acaagttcgc ctcactcttc 840
ctggcctgtg tgatcatctc catcctctcc atttacgtgg gaggcatcaa gtccgctttt 900
gaccctcctg tttttccggt gtgcatgctg ggcaatagga ctctgtctcg ggaccagttt 960
gacatctgtg ccaagacagt tgtggtggac aatgagacag tggccacccg gctgtggact 1020
ttcttctgcc acagccccaa ccttactgct gactcctgtg acccctactt cctgctcaac 1080
aatgtgacag agattcctgg catacctggg gcagctgctg gtgtgctcca ggaaaacctg 1140
tggagtgctt acctggagaa gggtgaggtt gtggagaagc atgggctgcc ctccacagat 1200
acccttggcc tgaaggagag cctgtccctg tatgtggtgg ccgacatcgc cacatccttc 1260
accgtgctgg ttggcatctt tttcccttct gtaacaggca tcatggctgg ctcaaaccgt 1320
tccggggacc tccgtgatgc tcagaagtct atccctgtgg ggaccattct ggctattgtc 1380
accacttcac tcgtgtactt cagcagtgtg attctcttcg gtgcctgcat cgagggtgtg 1440
gtgctccggg acaagtacgg tgatggcgtc agcaggaacc tggtggtagg caccttggcc 1500
tggccttcac cttgggtcat cgtggtcggc tccttcttct caacatgtgg tgccggcctc 1560
caaagtctca ctggggcgcc acgtttactg caagccattg ccaaggataa catcatcccc 1620
ttcctccggg tgtttggcca cgggaaagcc aatggtgagc caacgtgggc cctcctcctg 1680
acagcgctca tcgctgagct gggcatcctc atcgcctccc ttgacatggt ggcccccatt 1740
ctttccatgt tctttctgat gtgttacctc tttgtaaact tggcctgtgc tgtgcagaca 1800
cttctgagga cccccaactg gcggccccgg ttcaagtact atcactgggc gttgtctttc 1860
ctgggcatga gtctgtgcct ggctctcatg tttgtctcct cctggtacta cgccctagtg 1920
gccatggtca tcgcaggcat gatctacaag tacatcgagt accaaggggc tgagaaggag 1980
tggggtgatg ggatccgagg cctgtccctg agtgccgcac gatatgcact gctgagacta 2040
gaggaagggc ctcctcacac gaagaactgg cggcctcagc tcctggtgct gctgaagtta 2100
gacgaagatc ttcatgtgaa gtaccctcgg ctcctcacct ttgcctccca acttaaggct 2160
gggaaaggcc tgacaatcgt tggctctgtc atccagggca gctttctgga gagctatggg 2220
gaagcccagg ctgctgagca gacaatcaag aacatgatgg agattgagaa agtaaaaggc 2280
ttctgccagg tagtggtggc cagcaaggtt cgagaggggc tggcccacct catccagtct 2340
tgcggcctgg gtggcatgag acataactcc gtggtgctgg gctggcccta tggctggcga 2400
cagagtgagg acccacgtgc ctggaagacc tttatcgaca ctgtgcgctg caccacagct 2460
gcccacctgg ccctgctggt gccaaagaac atagctttct accccagcaa ccacgagcgc 2520
tacctggagg gccacattga tgtgtggtgg atcgtgcatg acggaggcat gctgatgctg 2580
ctgcccttcc tgctgcgcca gcataaggtt tggaagaagt gccggatgcg cattttcacc 2640
gtggcccaga tggacgacaa cagcatccag atgaagaagg atctggccat cttcctgtat 2700

cacctccgcc tggaagctga agtggaggtg gtagagatgc acaacagtga catctcggcc 2760

tacacctacg agcggacact gatgatggag cagcggtctc aaatgctgcg acagatgagg 2820

ctgaccaaaa cagagcggga tcgagaggcc cagctggtga aggacaggca ctcggctctg 2880

aggctagaga gcctctactc cgacgaggag gatgagtctg tgacaggcgc tgacaagatc 2940

cagatgacat ggaccagaga caagtacatg gctgaaccct gggaccccag ccatgcccct 3000

gacaacttcc gggagctggt gcacattaag ccggaccagt ccaatgtgcg gcgtatgcac 3060

actgctgtga agctcaatga agtcattgtc acacgctccc atgatgcccg cctggtccta 3120

ctgaacatgc ccggcccccc taagaacagt gagggtgatg agaactacat ggaattcctt 3180

gaagtcctaa ccgagggcct tgaacgggtg ttgttggtgc gtggtggtgg ccgggaagtc 3240

atcaccatct attcttgagc ccgatggagt cttgtggcct ggagttgggt tgtctaagac 3300

aacagtgccc agccttgcac ctacttgcca gttctgcctt gcccagcctt gctttggact 3360

agctttgcta ggtctccagg gaaaccaagc ttgggccttg caatgggaat ggatccgagg 3420

gcccacggga cctggaggat ttagggactt tcccctccca tactccaagg gaggcctctc 3480

ctgactcgag atgactggtg agggctgatg tgggatttga agtcccagac tggctcacaa 3540

gtgctattta ttgtatattt attgtgtgga tgtcatcatt tcagaaaggg gggagacaat 3600

aaaaggggga gccgagctgg gcctgtctgc aggaagatct ggctcaggct gctgtgggca 3660

gcatcaagcc aagtggaatg gagctggcca agctgagcct gacttttttc aataaaacct 3720

cgtgcc 3726

<210> 532

<211> 6075

<212> DNA

<213> Rattus norvegicus (Norway rat)

<220>

<221> misc_feature

<222> (28)..(28)

<223> n

<400> 532
agcgggtcgg tccacccgag aaaggggngc ggaggaactg ggaccgaccc gggaagactg 60

cgccagaggt gcacagagat cgcgcgggga gcgaggagcc gggcctcggc gctcagcccg 120

gatccacgtc ccgcgaagca ccggctccgg ccggccgcgg ggtcatgacg tacagcgagc 180

tctacagccg gtacacgagg gtctggatcc ccgacccaga tgaggtgtgg cgctcggctg 240

aactaaccaa ggactacaaa gatggggatg agagcctaca actcagactg gaagatgaca 300

caattctgga ttacccaatc gatgtccaaa ataaccaggt gccattcttg cggaatccag 360
atatcttagt aggagaaaac gacctcactg cactcagcca tctccacgag cccgcagttc 420
tgcataactt aaaggtccgt ttcctggagt ctaaccacat ctacacttac tgtggaattg 480
tgcttgttgc cattaatccc tatgagcagc tgccaatcta cgggcaggat gtcatctatg 540
cctacagtgg ccaaaatatg ggcgacatgg atccccacat ctttgctgta gcagaagaag 600
cctacaagca gatggccaga gatgaaaaga accaatccat catagtcagc ggagagtctg 660
gagcagggaa gaccgtgtca gccaagtatg ccatgcgcta ttttgccaca gttgggggct 720
cagccagtga taccaacatc gaagagaagg tcctggcttc cagtcccatc atggaggcca 780
tcgggaatgc caagacaact cgcaacgaca atagcagccg atttgggaag tacattgaga 840
tcggctttga taaaaagtac cacatcatcg gggccaacat gaggacctat ttgctggaga 900
agtccagggt ggtctttcag gcggatgatg agaggaacta ccacatcttt taccagctct 960
gtgctgcggc cagccttcct gagtttaagg agctcgcact aacatgtgca gaggactttt 1020
tctacaccgc ccatggagga aacacgacca tcgagggtgt agatgatgca gaggactttg 1080
agaagacaag acaagccctc acactccttg gagttcggga gtcccatcaa atcagcatct 1140
ttaagataat tgcttctatc ttgcaccttg gaagtgtgga gatccaggcc gagcgggatg 1200
gggactcctg cagtatatcg ccccaggacg aacacctgag caacttctgc cgcctgctag 1260
gaatagagca cagtcagatg gagcactggc tgtgtcatcg gaagctggtc accacctccg 1320
agacctacgt caagaccatg tccctgcagc aagtggtcaa cgcacgcaat gccttggcca 1380
agcacatcta tgcccagctg ttctcctgga ttgtggagca catcaacaag gccctgcaaa 1440
cctcccttaa acaacactcc ttcatcgggg tcctggatat ttatgggttt gagaccttcg 1500
agattaatag cttcgagcag ttttgtatca actatgccaa cgaaaagctc cagcaacagt 1560
tcaactcgca tgtgttcaag ctggagcaag aagagtatat gaaggagcag atcccgtgga 1620
ccttgattga cttctatgat aaccaaccgt gcatagacct catagaagcc aagctgggta 1680
tcctggacct gttggatgag gagtgtaagg tccccaaagg aactgatcag aactgggctc 1740
agaaactcta tgaacggcac tccaacagtc aacacttcca gaaaccacgc atgtccaaca 1800
cggccttcat tgtcatccac tttgcagaca aggtggaata cctttcagat ggttttctgg 1860
agaaaaacag ggacacggta tatgaagaac agatcaacat cctgaaagcg agcaagtttc 1920
cgctagtggc tgatttgttc cgtgatgacg aggactctgt tcctgccacc aacacagcta 1980
agagtcggtc atcttcaaag atcaatgttc gttcttccag acccctcatg aaggccccca 2040
acaaggagca caagaaatcc gtgggctacc agttccgcac ttccctaaac ctgcttatgg 2100
agactctgaa tgccacaacg ccccactacg ttcgatgcat caagcccaac gacgaaaagc 2160
tccccttcca cttcgaccca aagagagctg tgcagcagct cagagcctgc ggcgtgttgg 2220
agaccattcg gatcagcgcg gcaggctacc cgtccaggtg gacctaccat gacttcttca 2280
accggtatcg ggtgttgatg aagaagagag agcttgccaa caccaccgac aagaagaata 2340

tctgcaagtc tgtcctggag agtctcatca aggatccaga caagttccag tttggccgca 2400

ccaagatctt cttccgggca ggtcaggtgg cctacctgga gaagcttcgg gcagacaagt 2460

tccgggaggc caccatcatg atccaaaaga cggtcagggg ctggctgcag agagtgaagt 2520

accggaggct gagagcagct acgctaaccc tgcagagatt ctgccgagga tacttagccc 2580

gcagactgac tgagcacttg aggagaaccc gggcggccat agtgttccag aagcagtacc 2640

gcatgctgaa ggcccgccga gcctactgca gggtccgcag ggctgcggtc atcatccagt 2700

cctacacgag ggggcatgtg tgtacgcaga agctaccgcc agtcctcacg gagcacaaag 2760

ccaccatcat ccagaagtac gcccggggct ggatggcacg gagacatttt cagcggcagc 2820

gggatgcagc cattgtcatc cagtgtgcct tccggaggct caaggccagg caggcactga 2880

aggccctcaa gatcgaggcc cgttctgcag agcatctgaa acgcctcaac gtgggcatgg 2940

agaacaaagt tgtccagctg cagcggaaga ttgatgacca gaacaaagag ttcaagactc 3000

tgtcagagca gttgtctgca gttacctcca cccatgccat ggaggtggag aagctgaaga 3060

aggagctggc gcgttaccag cagaaccagg aggctgaccc cagccttcag ctgcaggagg 3120

aggtacagag cctgcgcacc gaactacaga aggctcattc agagcgcagg gtcctagagg 3180

atgctcacaa cagggagaat ggtgaactga gaaagcgagt cgcagacctg gaacatgaaa 3240

atgcactctt gaaggatgag aaagaacacc ttaaccacca aatcctgcgc cagtcaaaag 3300

ctgaatcttc acagagctct gtggaggaaa acctgctgat taagaaggaa ctggaggagg 3360

aacggtcccg gtaccagaac ctcgtgaagg agtactccca gctggagcag agatatgaga 3420

accttcggga cgagcagcaa actccaggcc acaggaagaa cccatcaaat caaagtagct 3480

tagaatctga ctccaattac ccctccattt ccacctcaga aatcggagac accgaggatg 3540

ccctacagca ggtggaggag attggtatag agaaggcagc catggacatg actgtcttct 3600

tgaagctgca gaagagagtg cgggaacttg agcaggagag gaagaagctg caggtgcagc 3660

tagaaaagga acaacaggac agcaagaaag tgcaggtaga acaacaaaac aatggcctag 3720

atgtggacca ggatgcagat atagcctaca atagtctgaa gagacaagag ctcgagtcag 3780

agaacaagaa gctgaagaat gatctgaatg agcgctggaa ggctgtagcc gaccaagcca 3840

tgcaggataa ctccactcat agctcccccg acagctacag cctcctactg aaccagctca 3900

agctggccaa cgaggagctg gaggtccgca aagaggaggt gctgatcctc aggacccaga 3960

tcatgaatgc tgaccagcgc agactgtccg gcaagaacat ggagccgaac atcaatgcca 4020

gaacaagttg gcccaacagc gagaagcacg tggaccagga agatgccatt gaggcctatc 4080

acggggtctg ccagacaaac agccagactg aggattgggg atatttgaat gaagatggag 4140

aactcggctt ggcttaccaa ggcctaaagc aagtcgccag gttgctggag gcccagctgc 4200

aggcccagaa cctgaagcat gaggaggagg tggagcatct caaggcccag gtggaggcca 4260

tgaaggagga gatggacaaa cagcagcaga ccttctgcca gactctgctg ctctccccag 4320

aggcccaggt agaatttggt gtccagcagg agatatcccg gctgaccaat gagaacctgg 4380

actttaagga gttggtggaa aagctggaga agaatgagaa gaagctgaaa aagcagctga 4440

agatttacat gaagaaggtc caggacttag aagctgccca ggcattggca cagagtgaca 4500

ggaggcacca tgaactcaca agacaggtca cggtccaacg aaaagagaag gacttccaag 4560

gcatgctgga gtaccacaaa gaggacgagg cactccttat ccggaacctg gtgacagacc 4620

tgaagcccca gatgctgtcg ggcaccgtgc cctgtctgcc tgcatacata ctctacatgt 4680

gcatcaggca cgcggactac accaacgatg acctcaaggt gcactcgttg ctgagctcca 4740

ccatcaatgg cattaagaaa gtcctcaaga agcacaatga ggactttgag atgacgtcat 4800

tctggttatc caacacctgc cgcctccttc actgtttgaa gcagtacagt ggggatgagg 4860

gtttcatgac acagaacacg gcgaagcaga atgagcactg tctcaagaac tttgacctca 4920

ctgaataccg tcaggtgcta agtgaccttt ccattcagat ctatcagcaa ctcattaaaa 4980

ttgctgaggg cctgctacag cctatgatag tttctgccat gctggaaaat gagagtatcc 5040

aggggctgtc aggggtgagg ccaactggct accggaagcg ctcgtccagc atggtggacg 5100

gagaaaattc gtattgcctg gaggccatca tccgccagat gaatttcttt catacagtcc 5160

tgtgtgacca gggcctggac cccgagatta tcctgcaggt gttcaaacag cttttctaca 5220

tgatcaatgc ggtgactctt aacaacctac tcctgcggaa agatgcctgc tcctggagca 5280

ccggcatgca actcaggtac aacataagtc aactcgaaga gtggcttcgg ggaaaaaacc 5340

ttcagcagag tggagcggtt cagaccatgg agcccttgat ccaggcagcc caactccttc 5400

agctgaagaa gaagacccag gaggatgctg aggccatctg ctctctgtgt acctccctca 5460

gcacccagca gattgtcaaa attttaaacc tctacactcc cttgaatgga tttgaagaac 5520

gggtgacagt gtcctttata cgaacaatcc aggctcagct acaagagagg agtgaccccc 5580

agcagctact gctggactcc aaacacatgt ttcctgttct gtttccattt aatccatctg 5640

ctctgaccat ggactcaatc cacatcccgg cctgtctcaa tctggagttc ctcaatgaag 5700

tctgaggatg cgttgtttcc aaggcaagcg agaaggaagt gcgtgctgtc ggctgaagga 5760

gtgctaggtc tgttaaatat gcccagcgta gatcaaacca tgttagagac ctgtggggag 5820

cactgaacta aacagcgggg tgcactctgg tcgttagctt ttgtacagac tgctcagaaa 5880

acacctgaag tgaggacgat tgttgcagtg ggactttcag gttaaaccaa gacacgtcag 5940

aacggacggc cgctgtgtag ctccagtcac catacaaaga tgccagttct acagagtgga 6000

agtgcctagc tttgagctgt gtatataact taagaatgtt caaactaaga ttatattaaa 6060

aacacatgac ataaa 6075

<210> 533

<211> 1201

<212> DNA

<213> Rattus norvegicus (Norway rat)

<400> 533
agatggcggc agccgaggcc gcgaactgca tcatggaggt ttcctgtggc caagcagaaa 60

gtagtgagaa gcccaatgct gaggacatga catccaaaga ctactacttt gactcctatg 120

cccactttgg catccacgag gagatgctaa aggatgaggt gcgaaccctc acgtaccgca 180

actccatgtt tcacaatcgg catctcttca aagacaaggt ggtgctggat gtgggctcgg 240

gcactggcat cctctgcatg ttcgctgcca aggcaggggc ccgcaaggtc attgggatcg 300

agtgctccag tatctctgat tatgctgtga agattgtcaa agccaataag ttagaccacg 360

tggtgaccat catcaagggc aaggtggagg aggtggagct gcctgtggag aaggtggaca 420

tcatcatcag cgagtggatg ggttattgcc tcttctatga gtccatgctc aacactgtgc 480

tgcacgctcg tgacaagtgg ctggcacctg atggcctcat cttcccagac cgagccaccc 540

tgtatgtgac agccattgag gaccgacagt ataaagacta caagatccac tggtgggaga 600

atgtatatgg ctttgatatg tcctgcatta aagacgtggc catcaaggag cccctggtgg 660

acgtggtgga cccaaagcag ctggtcacca acgcctgcct cataaaggag gtggacatct 720

acacagtcaa ggtggaggac ctgaccttca cctccccgtt ttgtctgcaa gtgaagagga 780

atgactatgt gcatgcacta gtggcctact tcaacatcga gttcacccga tgccacaaga 840

ggaccggctt ctctaccagt cctgagtctc catacacaca ttggaagcag actgtgttct 900

acatggagga ctacctaaca gtgaagaccg gcgaggagat ttttggcact attggaatga 960

ggcccaacgc caaaaacaat cgtgacttgg actttaccat cgacctggac ttcaagggtc 1020

agctgtgtga gctctcttgt tccaccgact accggatgcg ctgaggaggt gccaggctgg 1080

ccctcctgca aaggggggct caggggctgg gcttggggga tgggagggta catcgtggca 1140

gtgtttttca taacttatgt tttatatggg ttgcgtttat gccaataaat cctcagctga 1200

c 1201

<210> 534

<211> 2196

<212> DNA

<213> Rattus norvegicus (Norway rat)

<400> 534
aattcggcac gagcaaacgg agaggccgcg ggaggcgcga agccggcaga aggcgaggga 60

gagcggaggg cggccatggt ccagccctga agccaaacaa aaaaggccta cttcgaaagc 120

tgtcaacaat gaatgactct aaagatgccc ctaagtccta tgacttcgac ctgatcatca 180

ttggaggagg ctcgggaggc ctggcggcag ctaaggaggc agccaaattt gacaagaagg 240

tgatggtctt ggacttcgtc acaccaactc ctctcggaac gaatgggggt ctcgggggaa 300

cgtgtgtgaa cgtgggctgc atacctaaaa aactgatgca ccaggcggct ctgttaggac 360

aagctctgaa agactcacgc aactatggct ggaaactcga ggacacagtt aagcatgact 420

gggagaaaat gacagaatct gtgcagaatc atatcggctc gctgaactgg ggctaccgag 480

tagctctccg ggagaagaag gtcgtctatg agaatgctta cgggaaattc attggtcctc 540

acaaaattat ggcaacaaat aacaaaggta aagaaaaagt ttactcagca gagcggttcc 600

tcattgccac cggtgaaagg ccacgctacc tgggcatccc tggagacaaa gagtactgca 660

tcagcagtga cgatcttttc tccttgcctt actgcccggg gaagacccta gtggttggcg 720

cgtcctatgt cgccttggaa tgtgcaggat tcctggctgg tatcggcctc gacgtcactg 780

taatggtgcg gtccattctc cttagaggat ttgaccagga catggccaac aaaattggtg 840

aacacatgga agagcatggt atcaagttta tcaggcagtt cgtgccgacg aaaattgaac 900

agattgaagc agggacacca ggccgactca aggtgaccgc taaatccaca aacagtgagg 960

agaccataga agacgaattt aacacagtgt tgcttgcagt aggaagagat tcttgtacaa 1020

gaactattgg cttagagacc gtgggcgtga agatcaatga aaagaccggg aagatacctg 1080

tcacggatga ggagcagacc aatgtgcctt acatctacgc cattggtgac attctggagg 1140

ggaagctgga gctgaccccc gtggccatcc aggcggggag attgctggct cagaggctgt 1200

atggcggctc cactgtcaaa tgtgactatg acaatgtccc aacgactgtg tttactcctt 1260

tggagtatgg ctgctgtggc ctctctgaag aaaaagctgt agagaaattt ggggaagaaa 1320

atattgaagt ttatcacagt ttcttctggc cattggaatg gacagttcca tcccgggata 1380

acaacaaatg ttatgcaaaa gtcatctgta accttaaaga caacgaacgt gtcgtgggct 1440

tccacgtact gggtccaaat gctggagagg tgacgcaggg ctttgcagcc gcactcaagt 1500

gcgggctgac caagcagcag ctggacagca ccattggcat ccacccggtc tgtgcagaga 1560

tatttacaac gctgtcggtg actaagcgtt ctgggggaga catcctccag tctggctgct 1620

gaggttaagc cccagtgtgg atgctgttgc caagactaca gaccattgct tgcttccttg 1680

tccacaccca ggtgaagttc aggaaggctc ttgggttctt ggcaccaatt caaggtgcta 1740

tcctaaggcc accaggtccc tgggatcttg tgggtaggag gtggcaggta gaagaaggct 1800

gcagcatcgc actggggtca ccatgacgga ctcagactga cattcggcag agcatcacgg 1860

tgcgtccatg aagtcactag cctcaagccc aagtggtggg cagtgacaga aagctgtcga 1920

tctgttgggt tcaacctttc cctgtagact gttttagtct cgccttcaag ctatgtaatg 1980

tcaattctgt tttttctttt ctccatgggg ttaatgatac tagaggtagg gaatgttagc 2040

aatcagtttt tgtcatggct ggactatcca cagcacggtc gttactgtgt ggaagggggt 2100

cagatggctt atgagagcca aaccagttta tcctgagaaa gacgaattac cctgtggcta 2160

aaatacactg tttttactaa aaaaaaaaaa aaaaaa 2196

<210> 535

<211> 339

<212>  DNA

<213>  Rattus norvegicus (Norway rat)

<400>  535
cggagaaaat gcatgccagg gacttcacag tttctgctct ggtaagagtt gttggattta      60

gtaatgctaa ttatagccat taagcaggat tttactacaa tatggctgct cagtgctgtg     120

ttgtcgttcc ccctgctcag aacaattgtt tcttaactat acctgtctgc tgtctacctg     180

tagcagccag ggacgcttgg tctcatacat gatagaaaga aattaaatga atgcctgacc     240

tgaataggga ttgctgaatt gagttgttgt atttgcagca tggtgacatg gaccagaagg     300

aaagagatgt catcatgagg gaattccgat cagggtcaa                            339

<210>  536

<211>  990

<212>  DNA

<213>  Rattus norvegicus (Norway rat)

<400>  536
atggtacaag ctaagacctg gaccctaaag aagcactttg aaggcttccc tacggacagt      60

aactttgagt tgaggacgac tgagctccca cccttaaata atggagaggt cctgctggaa     120

gccctgttcc tctccgtgga cccttacatg agagttgcag caaaaaaact gaaggagggc     180

gacagcatga tgggtgaaca agtggccaga gttgtggaaa gtaaaaactc agccttccca     240

acgggaacga ttgttgtggc tttattaggt tggacatcac attccatttc tgatgggaat     300

ggactgagaa agctacctgc agagtggcct gacaagctac cactgtcttt ggctttgggg     360

acagttggca tgccaggcct cactgcctac tttggcctgc ttgacatctg tggcttgaag     420

ggtggagaaa cagtgctggt caatgcggca gccggggctg tgggctctgt tgtggggcag     480

atagctaagc tcaagggctg caaagttgtt ggtacagccg ggtctgatga aaaggttgcc     540

tatcttaaga agcttggatt cgatgtggcc tttaactaca agacagtaaa gtcattggaa     600

gaagctttga ggacagcctc tccagatggt tatgattgct actttgataa tgtcggtgga     660

gagttttcaa acactgttat actgcagatg aagacgtttg gaagaattgc catctgtggc     720

gccatctctc aatacaaccg cactggccca tgtcccccag gcccatctcc agaggtcatt     780

atctatcagc aactccgcat ggaggggttc atcgttactc ggtggcaagg agaagtccgc     840

cagaaggctc tgacagactt gatgaattgg gtttcagagg gtaagatccg gtatcatgaa     900

tacatcactg aaggatttga gaagatgccc gcagcgttca tgggaatgtt gaaaggagac     960

aatctgggga agactatagt gaaagcgtga                                      990

<210>  537

<211>  1018

<212>  DNA

<213>  Rattus norvegicus (Norway rat)

<400>  537
tccaggctgc accactggca acaaaaaatg caggccctac tattcctgat ggctcttctc     60

ttgccttctg gagctggagc tgaggagatt atcggtggtg tggagtctag accacactcc    120

cgtccttaca tggcccatct ggagatcacc actgagagag gttacaaggc cacctgtggt    180

gggtttcttg tgacccgcca atttgtgatg actgccgcac actgtaaggg aagagaaacc    240

actgttaccc ttggagttca tgatgtgagc aagacagaat ccacacagca gaagataaaa    300

gtcgaaaaac aaattgttca cccaaattat aacttctatt ccaatctcca tgatatcatg    360

ttactgaagc ttcaaaagaa agccaaggtg actcctgctg tggatgtaat tcccctgcct    420

cagccctctg actttctcaa gcctgggaag atgtgccggg cagctggctg ggggcaaact     480

ggagtgacaa aaccaacctc aaatacactg agggaggtga aacagagaat catggataaa    540

gaggcctgta aaaactattt tcattataac tataacttcc aggtctgcgt gggcagtcca    600

agaaagataa gatcagcata taagggagac tccggaggac cactagtgtg tgctggggtg    660

gcccatggta ttgtatctta tggacgtgga gatgcaaagc cccctgcagt cttcacccga    720

atctccccat atgtgccctg gattaataaa gtcataaagg gcaaggatct gaccagcctg    780

agtctgcacg agtcagagtc tccaagctag agaactcttt tgatatccct tgggatcaac    840

aaagcctgtc cccagcctgt cacctgcttg tcccatacct gttcccagcc tgcccccaac    900

ttgcccccag cccatcccca gcctgtccca agcctgtccc taggatgatc tcaacaatac    960

acaagtctgt ggtgatggat tgttccctgt aaagcacctc aataaagaca taacctca     1018

<210>  538

<211>  1765

<212>  DNA

<213>  Rattus norvegicus (Norway rat)

<400>  538
ttgaaaaggt actggaaact gaggacccta tctggatcaa agcagtttct gatggagccc     60

tcgcctcttg agctaccagt tgatgcagtg cggcgcatcg cggctgaact caattgtgac    120

ccaaccgatg agagggtggc tctccgcttg gatgaggaag ataaactgaa gcgttttaag    180

gactgttttt atatccccaa aatgcgggac ctgccttcaa ttgatctatc tttagtgaat    240

gaggatgata atgccatcta tttcctggga aattcccttg gtcttcaacc gaagatggtt    300

aaaacatacc tggaggaaga gctagataag tgggccaaaa taggagccta tggccatgag    360

gtagggaaac gtccttggat tataggagat gagagcattg taacccttat gaaggacatt    420

gtaggagccc atgagaaaga aatagctcta atgaatgctt tgactgttaa tttacatctc 480

ctgctgttat cattctttaa gcctacacca aagcggcaca aaattcttct agaagccaaa 540

gccttccctt ctgatcatta tgcgatcgag tcacagattc aacttcatgg acttgatgtt 600

gagaaaagta tgcggatgat aaagccacga gagggggaag agaccttaag aatggaggac 660

atactggaag taattgagaa ggaaggagac tcaattgctg tggtcctgtt cagtggcctg 720

cacttttata ctggacagct gttcaacatt cctgccatta cacaagccgg acatgcaaag 780

ggctgttttg ttggctttga cctagcgcat gcggttggaa atgttgaact ccacttacat 840

gactgggatg ttgactttgc ctgctggtgc tcctacaagt atttaaattc aggagctgga 900

ggtctggctg gtgccttcat ccatgagaaa cacgctcaca cgatcaagcc agcgttagtg 960

ggatggttcg gccatgaact cagtacaaga tttaacatgg ataacaaact acaattaatc 1020

cccggggtca atggattccg aatttccaac cctcccattc tgttggtctg ctccttgcat 1080

gccagtttag agatctttca gcaagcaact atgactgcgc tgaggagaaa atccattctg 1140

ctgacaggtt atctggaata cttgctcaaa cattaccatg gcggaaatga cacagaaaac 1200

aagaggccag ttgtgaacat aatcacccca tccagagcag aggaacgagg ctgccagctg 1260

acactgacct tttccatttc caagaaaggc gtttttaagg aactagaaaa aagaggagtc 1320

gtctgtgaca agcgagaacc agaaggcatc cgggtggccc cggttcctct ctataattct 1380

ttccatgatg tttataagtt catcagactg cttactgcca tactcgactc tacagaaaga 1440

aactagccat gctttctaaa taactcaagt aaatctcaca cactgggggt tccacttcta 1500

ctgcatttta gtcattcaaa agtctccaga aattgatggc atagaaatga tgatgatttt 1560

ataaacttac ataaaacctg gtacatgttt taatatctgt gtcgctgatg tgctgtggac 1620

taagaagtca cattttacat gactccaacc tacagatgac tgtcttgatc agctgtcacc 1680

ttccatggtc actgaaaggt tgtgtgttta atttgtgact gaaatgacaa cattaaaatg 1740

tatctggact tcttgtataa aaaaa 1765

<210> 539

<211> 1748

<212> DNA

<213> Rattus norvegicus (Norway rat)

<400> 539
cttagaacct ggactcagac cttcataaga gccacaaaga aaccaattct gaaccctatc 60

ttggctttgg atcctggaag agaatcgcta aacacagagc agacccagtg agtgagcagg 120

tgttttggac aatggactgg ttgagcccat ctctattata aaaatgtctc agagcaaccg 180

ggagctggtg gttgactttc tctcctacaa gctctcccag aaaggataca gctggagtca 240

gtttagcgat gtcgaagaga acaggactga agccccagaa gaaactgaac cagaaaggga 300

gacccccagt gccatcaatg gcaacccatc ctggcacctg gcggatagcc ccgcggtgaa 360

tggagccact ggccacagca gcagtttgga tgcgcgggag gtaatcccca tggcagcagt 420

gaagcaagcg ctgagagagg ctggcgatga gtttgaactg cggtaccgga gagcattcag 480

tgatctaaca tcccagcttc atataacccc agggacagca tatcagagct ttgaacaggt 540

agtgaatgaa ctctttcggg atggggtaaa ctggggtcgc attgtggcct tcttctcctt 600

tggcggggca ctgtgcgtgg aaagcgtaga caaggagatg caggtattgg tgagtcggat 660

tgcaagttgg atggccacct acctgaatga ccacctagag ccttggatcc aggagaacgg 720

cggctgggta agaaccacgc cccttgtgtg tccgcccctt gtgtgtctct cctctgtgga 780

gatccctaac tgcccttttt ggtctcctgg catggttgtt gaagatatcg attattcagg 840

agacattcct ggcttacttt aataccaggg gttaactttg ggaatattga tgaccctgtt 900

tttaaggaac ctgtattttt cattctggct acccttgtgg ccccgcagtt tcatagtttt 960

gttccaattt ctcggcaaag aaaaacagcc tgtgtgttta cttggcttaa aacctaggag 1020

agtagcaagt tctccctgtg ttctgctcac tctcacgtaa tgggtcacct tggctggggg 1080

gggaaggtaa tataatacct ggaggaactg cagagctgat aaccccaggc tcctgtgtgc 1140

tcgcatgcat gctattcaga ccatttggtc aggcaggagc cccactggct tgactggctg 1200

aatattgtct gattttagag aaggtggtct gagccaattg tgtttgtgct gtgcccctaa 1260

gttcattttc accatggttg gcttgtttgg ggagcccttg ctgtgcccca ggactttgta 1320

cctcactgcc ctggatcagt tggggatctc ccagctgctc tggcccagtg tttttctgct 1380

cagggccctg ggcctctttt actaaagcac tgtttgctgc tggcagacac gaaccaggtc 1440

tgtgaggctc cataggaaat taagatttgg tgacagactg gacgtgggtg tgagtcctga 1500

ccctgtcact taactggcca tgtgaccttg gtcaggctga cttaccttct gggcctcagc 1560

ttctcacaga ctgttgtgac tcttaaagat aaagcgagca gaagctgaca ccagtgagtg 1620

ccttttcttc tgctcaccct tgtgctcacc agggtgagat agaggtgaga atgcccttac 1680

tggtgaccac tgtggtaggc atttaccgtt cggagggaac tccattctgg aataggggag 1740

aatgcgac 1748

<210> 540

<211> 1428

<212> DNA

<213> Rattus norvegicus (Norway rat)

<400> 540
ctgtttctgc tacttgctct ttgtttcaaa ctgccttgga gagtttctca cagtaccgtg 60

tgcttcttgc taacttccgg tttaagcttt agtcgttctc tagtctcttc agttttcacc 120

ctgagcctcg acactggact actgaaatcg tgtagtgaac gttggatgtg tcccaaaaag 180

gtaatgtgga acacagccgt gcactcggaa ttcatacatg atcacgtgga ttacggcttt 240

caaaactgca agagtaaatt caattggcat gtcatcaagg agaagcggga tgcttacgtg 300

agccgcctga acaacatcta ccaaaacaat ttaaccaagt cccacatcga agtcatccac 360

ggctacgcaa catttcgaga tggtccccag cccacagcgg aagtcaacgg gaagaagttc 420

actgctccgc acatcctgat cgccacgggt ggtgtgccca cggttcctca tgagaaccag 480

atcccaggtg ccagcctggg gataaccagt gatgggttct ttcagctgga agacttgccc 540

agccgcagcg ttattgtggg tgccggttac attgccgtgg agattgcggg catcctctcc 600

gccctgggct ccaagacgtc tcttatgatc aggcatgata aggtgcttag aagctttgat 660

tcactcatca gttccaactg caccgaggag ctggagaacg ctggcggtgt tgaggttctc 720

acagttaaga agttctcaca ggttaaggaa gtaaagaaga cctcatcggg cttggaactc 780

catgtggtta ctgcacttcc cggtaggaaa cccaccgtga ccacgattcc agatgtcgat 840

tgcctgctct gggccattgg acgggaccca aactctaagg gcctgaatct aaataaactg 900

gggatacaga ctgatgacaa aggccatatc ctagtggacg agttccagaa taccaatgtc 960

aaaggcgtct atgccgtggg cgatgtctgt gggaaagcac ttctcacccc agttgcgatc 1020

gctgctggcc ggaaactcgc ccatagactt tttgagggca aagaagattc caggttggac 1080

tatgacaaca tccctaccgt ggtcttcagc cacccgccta tcgggacagt ggggctcact 1140

gaagatgaag ccgtccataa gtatggcaaa gacaatgtga aaatctactc gaccgccttc 1200

accccgatgt atcacgctgt gaccacgagg aagacgaaat gcgtgatgaa gatggtttgt 1260

gccaacaaag aggagaaggt ggttggcatc catatgcagg ggattggctg cgatgagatg 1320

cttcagggct tcgctgtagc agtgaaaatg ggggccacca aggccgactt cgacaatagg 1380

gtcgccattc atcctacctc ttcagaggag ctggtcacac ttcgttga 1428

<210> 541

<211> 482

<212> DNA

<213> Rattus norvegicus (Norway rat)

<400> 541
aaagcggcct gggactggct cattctgctg ctggtcatct acacagccgt cttcacaccg 60

tactcggctg ccttcctgct gaaggagact gaagatgggt cccaagcccc tgactgcggc 120

tatgcctgcc agcctctggc tgtagtggac ctcctcgtgg acatcatgtt cattgtggac 180

atccttatca atttccgcac cacctacgtt aacgccaatg aagaggtggt cagccatcct 240

ggccgaatcg ctgtgcacta ctttaagggc tggttcctca tcgacatggt ggctgccatc 300

cccttcgacc tgctcatctt tggctctggc tctgaggagc tcatcggact gctgaaaact 360

gccaggctgc tgcggctggt gcgagtggct cggaagctgg accgctactc ggagtatggg 420

gcagcagtgc tcttcctgct catgtgcacc ttcgcgctca ttgcacattg gctcgcatgt     480

at                                                                    482

<210>  542

<211>  187

<212>  DNA

<213>  Rattus norvegicus (Norway rat)

<220>

<221>  misc_feature

<222>  (29)..(29)

<223>  n

<220>

<221>  misc_feature

<222>  (81)..(81)

<223>  n

<220>

<221>  misc_feature

<222>  (82)..(82)

<223>  n

<220>

<221>  misc_feature

<222>  (180)..(180)

<223>  n

<400>  542
aatctgttcc ctcccaccca gcccacttnc ccccaaccct ggaaacagac caacaaccca      60

aactcaattt ccccaaaagc nnaaaattgg gagacaattt tacatggact ttggaaaaca     120

tttttttcct ttgcattcat ctctcaaact tagtttttat ctttgaccaa ctgaacgtgn     180

ccaaaaa                                                               187

<210>  543

<211> 797

<212> DNA

<213> Rattus norvegicus (Norway rat)

<220>

<221> misc_feature

<222> (74)..(74)

<223> n

<220>

<221> misc_feature

<222> (541)..(541)

<223> n

<220>

<221> misc_feature

<222> (544)..(544)

<223> n

<220>

<221> misc_feature

<222> (558)..(558)

<223> n

<220>

<221> misc_feature

<222> (605)..(605)

<223> n

<220>

<221> misc_feature

<222> (614)..(614)

<223> n

<220>

<221>  misc_feature

<222>  (794)..(794)

<223>  n


<400>  543
agccctgggc cccacaactg caagcagagc cggagccgcc cagttacagc gggacccggg      60

cctctggggt ccanacaggg gtcgccatga tccgattcat tctcatccag aaccgggcag     120

gcaagacacg cctggccaag tggtatatgc agttcgatga tgacgagaag cagaagttga     180

tcgaggaagt gcacgccgtg gtcaccgtca gggatgccaa gcacaccaac tttgtggagt     240

tccggaactt caagatcatc taccggcgct acgctggcct ctacttctgc atctgcgttg     300

acgtcaacga caacaatctg gcctatcttg aaggcatcca caacttcgta gaagtgttaa     360

atgaatactt ccacaatgtc tgtgaactgg acctggtgtt caacttctac aggttttaca     420

cggtggtaga tgagattttc ttggcaggag agatccgaga gaggagccag acgaaggtgc     480

tgaagcagct gctgatgctg cagtcgctgg agtgaggggg ggcttccgcc ccagcccggc     540

ntgntgggcc aacatgcntg tttgtcccct gccaggtttg aggcccaccc cagcagcccc     600

ttccnttctt tagntggccc agaggagtga cccagcaggg tttaggccac aggggaggag     660

gccagaaccc actgcctttg ggccctaagt ggatggcaga ggccaccgtg atgtcccgag     720

taactgtgcc attattgtgt gatgctgtga gtgtgcgtgt gtgcagcccc caataaacct     780

gtggtcctgc ctgnaaa                                                    797

<210>  544

<211>  2129

<212>  DNA

<213>  Rattus norvegicus (Norway rat)


<400>  544
gaggccgctc gagagcaggc ttagggtcta ctgcggccgg tggaccctcc aacccgcttc      60

cataaggctt tagctttcca acttcagcta cagtgttagc taagtttgga aagaagacat     120

aaagaagacc ccggtgggca gctttgctgt tctttgcctt agttgtcctt tggggtcttt     180

cggcacaagg ctgctgtgct tatagccgtg ctatctccgg ttcctcacac tcctgtgaag     240

aagccctggg caagagcagc taaagctact agcatccgaa caaacaaggg gagagcgtct     300

ggtgccatga ccaagtcaag agatcagacc catcaggaag gatgctgtgg atctttagca     360

aactacctga cctcagcaaa attcctcctc taccttggcc actctctctc cacttggggg     420

gatcggatgt ggcactttgc agtgtctgtg tttctggtgg aactctacgg aaacagcctc     480

ctcttgacag ctgtctacgg gttggtggtg gcaggctctg ttctggtcct gggagccatc     540

attggtgact gggtggataa gaatgccaga cttaaagtgg cccagacgtc cctggtggtt 600

cagaatgtat cagtcattct ctgcgggatc atcctgatga tggttttctt acacaagaat 660

gagcttctga acatgtatca tggatgggtc cttactgtct gctacatcct gatcatcacc 720

attgcaaaca ttgcgaattt ggccagtact gccactgcaa ttacaatcca aagggactgg 780

attgttgtcg tagcaggaga aaacaggagc agattagcag acatgaatgc taccattaga 840

aggattgacc agctaaccaa catcctggcc cccatggctg ttggccagat tatgacattc 900

ggttccccag tcattggctg tggtttcatt tctggttgga atttggtgtc catgtgtgtg 960

gagtacttct tgctctggaa ggtttaccag aagacccctg ctctggctgt aaaagctgct 1020

ctcaaggtag aggagtcaga actgaagcag ctgacctcac ctaaagatac ggagccaaaa 1080

cctttggagg gaactcacct aatgggtgag aaagactcta acatccgtga acttgaatgt 1140

gaacaagaac ccacctgtgc ctcccagatc gcagaaccct tccgcacttt tcgagatgga 1200

tgggtctcct actataacca gcccgtattt ttgggctggc atgggcctgg ctttcctcta 1260

tatgacagtc ctgggcttcg actgtatcgc cacagtatat gcttacactc agggactgag 1320

tggttccatc ctcagtgttt tgatgggagc atcagcaata actggaataa tgggaactgt 1380

ggccttcact tggctacgtc gaaaatgtgg ccttgttcgg actggtctgt tctcaggact 1440

ggctcagctt tcttgtttga tcttgtgtgt gatctccgtg ttcatgcctg gaagcccctt 1500

ggacctgtct gtttctccat ttgaagatat ccgttctagg tttatacatg aggaggcagt 1560

gtcctcaact accaaaatac ctgaaacgga aatgcttatg tctaatgtgt ctaatgttgt 1620

caataccgtc catgagatga gtactaaatc cgtccccata atctccgtca gcctgctgtt 1680

tgcaggagtc attgctgcta gaatcggtct ttggtccttt gatttgactg tgacacagtt 1740

gctgcaagaa aatgtaattg aatcagaaag aggcattatc aatggtgtgc agaactccat 1800

gaactacctt ctcgaccttc tgcatttcat catggtcatc ttggccccaa atcctgaagc 1860

ttttggcttg ctagtattga tttcagtcyc ctttgtggca atgggacatc ttatgtattt 1920

ccgttttgcc csgaagactc tgggcaacca gatttttgtt tgtgctcctg atgaaaagga 1980

agttacagat gaaagtcagc ctaatacatc tgttgtgtaa aatagtttag ctgtggcccc 2040

tgttactaga tttgtggaga gcatgtgtgc ttattttgta ctacagaatt ccaataaatg 2100

cctgcacttt taaaaaaaaa aaaaaaaaa 2129

<210> 545

<211> 1627

<212> DNA

<213> Rattus norvegicus (Norway rat)

<400> 545
catttctccc tcacaaactg gagcaaccgg cgagcccggg gcgggccctg tggcctcggt 60

ttggttcgtg gagtcccagc ggcagattcg ctcggcaacg cggacagggc caattctgtc 120

gcgtcaggaa tcgcgggtga agttcgccac cggtggagag aagacgttga gaaaagccgc 180

gctgggctcc ctcaggggct cgtgcgcact cccagtttcg gccttttcat gcaggaaatg 240

aatctgctgc cgacgcccga gagtccagtg actcggcagg agaagatggc gaccgtgtgg 300

gatgaagctg agcaagatgg cattggggag gaggtgctca agatgtccac agaagagatc 360

gtccagcgca cacggctgct agacagcgag atcaagatca tgaagagtga agtgctgcga 420

gtcacccacg aactccaagc catgaaagac aaaatcaaag agaacagtga gaaaatcaaa 480

gtgaacaaca ccctcccata cctagtctcc aacgttattg agttgctgga tgttgacccc 540

aatgaccagg aggaggatgg tgccaatatt gacctggact cgcagaggaa gggcaagtgt 600

gcagtgatca aaacttctac ccgacaaacg tacttcctgc cagtgattgg gttggtagat 660

gccgaaaagc tgaagccggg agacctggtg ggtgtgaaca aagactccta tctgatcctg 720

gagaccctgc ccaccgaata tgactctcgg gtgaaggcca tggaggtgga cgagcggccc 780

acagagcaat acagtgacat tggcggcctg gacaagcaga tccaggagct ggtggaagcc 840

attgtcctgc ctatgaacca caaagagaag tttgagaact tgggtatcca gcccccaaaa 900

ggagtgctga tgtatgggcc gcctggtaca gggaagactc tcctcgcccg agcctgtgct 960

gctcagacca aggccacctt cttgaagctg gcaggccctc agctggtgca gatgtttatt 1020

ggagatggcg ccaagctggt ccgtgatgct tttgccctgg ccaaggagaa ggcaccatct 1080

attatcttta tagatgagtt ggatgccatt ggcaccaagc gcttcgacag tgaaaaggca 1140

ggggaccgag aggtgcagag gaccatgcta gagctcctga accagatgga tggcttccag 1200

cccaacaccc aagtgaaggt aattgcagcc actaacaggg tggacatcct ggaccctgcc 1260

ctgctccgct caggacgcct agaccgcaag attgagtttc caatgcccaa tgaggaggcc 1320

cgagccagaa ttatgcagat ccactcacgg aagatgaacg tcagtcctga tgtgaactat 1380

gaagagctgg ctcgctgcac tgatgacttc aatggggccc agtgcaaggc cgtgtgtgtg 1440

gaggcgggta tgatcgcact gcgcaggggt gccacggagc tcactcacga ggactacatg 1500

gagggcatcc tggaggtgca ggccaagaag aaagccaacc ttcagtacta tgcctaggga 1560

cacctctagt ctgtctgctg gggcgggggc tcggttgata ataaaggggg tttttgttct 1620

tttccca 1627

<210> 546

<211> 1561

<212> DNA

<213> Rattus norvegicus (Norway rat)

<400> 546
gcgggcccca ccggccgggc ggttgggccg tgaagggaga gtcttttcgg cgctgaggac 60

gggcgctgag gaggcggcgg tggctcctgg ggcgtttgag cgggctcact cgagcccgcg 120

ggccgacgcg gacgcaggcc cagccggcgg aaacgccccg gactccccgc gggccttcct 180

agccgccatg gaggacggtg tctacgagcc cccagacttg actccagagg agcgcatgga 240

gctggagaac atccgtcggc gaaagcagga gctgctggtg gagatccagc gcctccggga 300

agagctaagt gaagctatga gcgaagtgga gggtctggag gccaatgagg gcagtaagac 360

cttgcagcgg aaccggaaga tggcaatggg ccggaagaaa ttcaacatgg accccaaaaa 420

ggggatccaa ttcttggtgg agcatgaact tctacagaac acacctgagg aaattgcccg 480

cttcctgtac aagggcgagg ggctgaacaa gacagccatt ggggactacc taggggaaag 540

ggaagagctg aacctgtctg tgctccatgc ttttgtggat ctacatgagt tcaccgacct 600

caatctggtt caggccctcc ggcaattcct gtggagcttt cgcctccctg gagaggctca 660

gaaaattgac cgaatgatgg aggcctttgc ccagagatat tgcttatgta atcccggggt 720

cttccagtcc acagacacct gctacgtgtt gtccttcgct gtgatcatgc tgaacaccag 780

ccttcacaat cccaatgtca gggacaagcc gggcctggag cgctttgtgg ccatgaaccg 840

aggcatcaat gagggtgggg acctgcctga ggaccttctc aggaacctct atgacagcat 900

ccgaaacgag cccttcaaaa tccctgagga tgatgggaat gatctcaccc acaccttctt 960

caacccagac cgagagggct ggctcctaaa gctgggaggg ggccgggtga agacttggaa 1020

gcgtcgctgg ttcatcctca cagacaactg cctctactac tttgagtaca ctacggacaa 1080

ggagcccaga gggatcatcc ccctagagaa cctgagcatt cgtgaggtgg atgatcctcg 1140

gaagccgaac tgctttgagc tttacattcc caacaataag ggacagctca tcaaagcctg 1200

caaaacggag gctgatggtc gggtagtaga ggggaaccac atggtgtacc gaatctcagc 1260

acccacacag gaggagaaag acgagtggat caagtccatc caggctgctg taagcgtgga 1320

ccccttctac gagatgttgg cagcaagaaa gaagcggatt tctgtgaaga agaagcaaga 1380

acaaccctga ccctgtcccc aactccatta tttattttgg agctgccccg cctgggtggc 1440

aggacccctg ggccctgggg ctgtggatcc tggttccctg tttggaaaat caccacctct 1500

agctcctgac tccttatttg taattaacat gttggtaatc ttattaatta tttaacccct 1560

c 1561

<210> 547

<211> 1081

<212> DNA

<213> Rattus norvegicus (Norway rat)

<400> 547
gcttggaacg gtacgcgaag aaaagtgacc atggacaaca acgaaacctc cgtggattca 60

aaatccatta ataattttga aacaaagact atccatggaa gcaagtcgat ggactctgga 120

atatatctgg acagcagtta caaaatggat taccctgaaa tgggcttgtg tataataatt 180

aataataaga acttccataa aagcactgga atgtcagctc gcaatggtac cgatgtcgat 240

gcagctaacc tcagagagac attcatggcc ctgaaatacg aagtcaggaa taaaaatgac 300

cttactcgtg aagaaattat ggaattgatg gatagtgttt ctaaggaaga tcacagcaaa 360

aggagcagtt ttgtgtgtgt gattctaagt catggagatg aaggagtaat ttttggaacg 420

aacggacctg tggacctgaa aaaactaact agtttcttca gaggcgacta ctgccggagt 480

ctgactggaa agccgaaact cttcatcatt caggcctgcc gaggtacaga gctggactgc 540

ggtattgaga cagacagtgg aactgacgat gatatggcat gccagaagat accagtgggg 600

gccgacttcc tgtatgctta ctctaccgca cccggttact attcctggag aaattcaagg 660

gacgggtcat ggttcatcca gtcactttgc gccatgctga aactgtacgc gcacaagctg 720

gaattcatgc acatcctcac tcgtgttaac cggaaggtgg ccatggaatt tgagtccttc 780

tccctggacg ccactttcca cgcaaagaaa cagatcccgt gtattgtgtc aatgctcaca 840

aaagaactgt acttttatca ctaaaggaat gactgggagt ggggtagggg catgtttctg 900

ttttggtttt tttttggttt ttggtttgtt tttttttttt ttatttgaat gccaaatgag 960

aaaactgtca gggagacttt ttttttcccc tctcatttaa atcaaatccg atgttccagg 1020

tcgtcattga acaataccac tgcctgcaat gcagccacaa tacaatacct cagctttgat 1080

a 1081

<210> 548

<211> 3622

<212> DNA

<213> Rattus norvegicus (Norway rat)

<400> 548
   gctgctctga ctttctttta gtctcagcat ggagaggacc gtcttctaaa gcttcttcat 60
   aaaaacagca gtaagattat ttaaagaata gatatctgga aacaatcaga agaacaacat 120
   gggaaaatct gagaaaaggg ttgcaaccca tggggtcaga tgttttgcca agatcaagat 180
   gtttctgttg gcattaacat gtgcatatgt atccaaatca ttatcaggaa cttatatgaa 240
   ttccatgctc acacaaatag agagacaatt cggtatcccc acatctatag ttgggcttat 300
   caatgggagc tttgaaatag gaaacctttt gttgattata tttgtgagtt attttggaac 360
   aaaacttcac agacctatca tgattggtgt tggatgtgca gttatgggcc tggggtgttt 420
   cttaatctcg ctaccccatt tcctcatggg ccaatatgaa tatgaaacga ttttacctac 480
   aagcaacgtg tcctcaaaca gcttcttctg tgtggaaaac agatcccaga ccttaaatcc 540
   aacacaagac ccctcagagt gtgtgaaaga aatgaaatca ttaatgtgga tatatgtact 600
   ggtaggaaac ataatacgtg gaattggtga aactcccatc atgcccttgg gtatttccta 660
   cattgaagac tttgccaaat ctgaaaactc tcctttatac attgggattt tagaaacagg 720
   aatgaccatt ggccctttga ttggacttct gttggcttct tcctgtgcaa acatttatgt 780
   agacattgag tctgtgaata cagatgacct gaccataact cccacagata cacgctgggt 840
   cggagcttgg tggatcggct ttttggtctg tgcaggagtg aatatcctga ccagctttcc 900
   ctttttcttt tttcccaaaa cacttccaaa ggaaggatta caggagaatg tggatggaac 960
   tgaaaatgcc aaagagaaga aacacagaaa aaaggccaag gaagaaaaac gaggaatcac 1020
   taaagatttc tttgtgttca tgaagagcct ctcctgcaat ccaatttaca tgcttttcat 1080
   ccttataagt gttctccagt tcaatgcatt tatcaattca tttaccttca tgcctaagta 1140
   tctggaacag caatatggaa aatccactgc tgaggtagtc ttccttatgg gtctttatat 1200
   gttacctcca atatgcctcg gatatttaat tggtggtttg attatgaaga agttcaaggt 1260
   tactgtcaag aaagctgcac acttagcatt ctggctctgc ctgtctgagt accttctgtc 1320
   tttccttagc tatgtgatga cctgtgataa ttttccagtg gcaggcttaa caacctctta 1380
   tgaaggggtt cagcaccaac tatatgtgga gaacaaggtc cttgctgact gtaacacaag 1440
   gtgtaactgc tcaacgaaca catgggatcc agtgtgtgga gacaatggcc tggcatacat 1500
   gtcagcctgc cttgcaggct gtgagaagtc tgttggaaca ggaaccaaca tggtgtttca 1560
   gaattgcagc tgcattcagt catcgggaaa ctcatctgca gtcctgggcc tgtgtaacaa 1620
   aggccctgac tgtgccaaca agctgcagta cttcttaatc atagcaatat ttggctgttt 1680
   catatactcg ctggcaggca ttccagggta tatggttctt ctgaggtgta tcaagtctga 1740
   agagaagtca cttggagttg ggttacatgc attttgcata agaatattag ctggcattcc 1800
   tgcacccatt tactttggag ctttgataga cagaacctgt ttacattggg gaaccctgaa 1860
   atgtggtgag cccggggcat gcaggatgta tgacataaac agcttcagac gtctttacct 1920
   tggattgccg gctgcactaa gaggagcaag ctttgtcccc gccttcttca ttctaagact 1980
   tacgaggaca ttccagttcc ctggggacat tgagtcttca aaaactgatc atgcggagat 2040
   gaagctcacc ttgaaggaaa gtgagtgcac agaagtccta aggtcgaaag tgacggagga 2100
   ctgaaaacga agctgtaatg agttttctac tgccctatgc aaggccatga agagaatgta 2160
   cacttcacta gttttgaatc atgagagata caattggaac tcttaggtta tccataaggc 2220
   cgtcaaagtt acttcattca tgataaaatt atttactgat agcattttca gaaggctgac 2280
   atagtactca agattttccc agggaaaact tctatagtgg ccttcaccct taaccttaaa 2340
   gctgccttca ttttcaacca gcatgttctc ttttaactca atcaagggaa gtggatgttt 2400
   cccacacatt ctcaaatatc tttgaaactt tcctattgca gaaatatcat ttagatgttt 2460
   ttaatttata tactgatgct ggagatcaaa atatacatct tggttaagcc agattgcgtt 2520
   agtttgtttt gatttatcct ctgcatgtgc aaaacttctg catctgtctt gtgtacttag 2580
   gagtggtaac tctcttttac ttctaagatt agactcttca gagtgtgcca tctcctgttt 2640
   tcagtccctt ctatcattac ttctgtcaca cagttgatca tttcacatac atcactgaaa 2700
   actttaatca ggttgttaac cagtcatgta gcaaagatga ttgggactct ttttctctaa 2760
   caattcaaag ctggtcatga aactcttttt taaaaatcaa gagtagggga aaactagtcc 2820
   tttcaaaggc tccttgtaga gatgggctgt atctcagtgg aatagttatt acctaatgta 2880
   tgtgaggccc caggttcaac cacaacgtag ggtaaaccaa taaagtaata aaaataacgt 2940
   aagtccagat gcatcatcag atattctaaa aggctattct catattcagg gggcttcaat 3000
   ggcttagtgt tcattctatt caagggccat ggagcacata gttattaaca ttcataataa 3060
   acttagagta aaacctttaa agagggacca gatagaaagt tcgatagaaa gaactgtttg 3120
   ccaccgaacc tgaaaaggtt gttgtgatcc ttgggaccaa cgtgaaggag agaacaaact 3180
   ctcacaagtt gtgctatatc tctttttaat tgtgcatgcc ccattgcaaa tcaattaata 3240
   aaaaaagcat taaaaggttt aagaccgaca tttgctgtaa aattatagct cataaacgtg 3300
   aaagtacaca tcaaaaataa aatcaagttg tggttgtttt aatgagaaat atccctccta 3360
   ggcataggca tttggatatt tggtttctaa tgaatgactc tgcttaggga agattggatg 3420
   tgcatcccta agacaaaagg tgaatcactg agatgggttg aaagttaaaa gcctcaccta 3480
   cttccagtac actctctgct ttgtgctttg gttgatgata tgaaatcatg gtttcctgct 3540
   ccagccacca tgcctggtgc ttgccttcat gaacttccat ccctggagtc atgcgttaaa 3600
   ataaactcct ttttttaatg tg 3622

<210> 549

<211> 1686

<212> DNA

<213> Rattus norvegicus (Norway rat)

<400> 549
cctaagttgc ttgtctgggc cactggcaaa ttcttccttc ccaggctaga cacacaggct 60

gagcaattgg ttccgaaccc agaggaaccc aggcgacgct aggtcacgtg gccgactgct 120

tacatgacct gcgggctgct gagcgtcact gtgacgttca ggagacctgc gaagtggccc 180

ggctacttcc gtcacctgtg ctgtcggggt gctgtcatgg acctgggacc gatgcgcaag 240

agttaccgcg gggaccgaga ggcatttgag gaggctcatc tgacctctct ggatcccatg 300

aagcagtttg cttcctggtt cgaggaggct gttcaatgtc cagatatagg agaagccaat 360

gctatgtgtc tggctacctg caccagagac ggaaaaccct ctgcccgcat gctgctgctg 420

aagggctttg gcaaggatgg cttccgcttc ttcactaact acgagagtcg gaaggggaaa 480

gagctggact ctaacccctt tgcctctctt gtcttctatt gggagcccct gaaccgtcag 540

gtgcgtgtgg aaggcccagt gaagaaactg ccggagaagg aggctgagaa ctacttccat 600

tcccggccca agagcagcca gattggggct gtggtcagtc gccagagttc tgtgatcccc 660

gatcgggagt acctgagaaa gaaaaatgag gaactggggc agctctaccg ggaacaagag 720

gtgcccaagc cagagtactg gggtggctac atcctatatc cccaagtgat ggagttctgg 780

caaggccaaa ccaaccgcct gcatgaccgt attgtcttcc gtcgaggcct ggcaacagga 840

gactcccctc tgggacccat gacccaccac ggggaggaag actgggtgta tgagagactt 900

gcaccttgac actggactcc agaatgttgg gggatcatca tagggaagag tgggggaagg 960

gaaggaaaaa aatggaagcc aggtatacct tctgtgtgtg tgtgtgtatg tgagacattg 1020

ggtttaaacc tcgctcacac taggtaacac tttaccactg agataaagtc ccagcatttt 1080

ttcttactac cttttatttt aagactaagt tgcttagtct ggccctgaac tcactgtgta 1140

tacatcccag acaagtcttg gctttgagtt cctcccctca gtcccttgag tagcagtatt 1200

ataggcttgt gccaccaggc ccagttctag tcctgttttt aagaagccca ttgcctttgt 1260

tttttttttt tttttttttc ttcggggctg gggactgaac ccaggacctt cggcttccta 1320

ggcaagcgct ctaccactga gccaaatccc caacccgccc attgcctttg cctttgttaa 1380

cccttctacc tgcatttctc caggactcac cctcaagttc tctgaactcg ggatcggttt 1440

agtggctgga acagagcgca gtagtgttgt agagagtcac aagtagcaaa gagtctcata 1500

attattccct tgattttgct cctggtttgg ggggacgccc gtcttgggag ctgcagctgg 1560

tctaactctg tttccagtaa cagactaatc aggccgcaga tatacaaggg ctcgctggct 1620

atggaaagga gccagaccat gtttgctgtg actcagacaa accattatat gttctggttg 1680

gccttg 1686

<210> 550

<211> 1047

<212> DNA

<213> Rattus norvegicus (Norway rat)

<400> 550
tttgggcagg acaaggcaag agaagcggct gtttcagtag tgtttgtacc cagaactcag 60

cgaggtctac ccttgggtag agcgaacgtc cggccatggc ttaccactcg ggatatggag 120

tccacggttc caagcatagg acccgggcag ctccaaaccc tcctccactc ttcgatgata 180

caagtggtgg ttattccagc caactaggcg gatacccagc cccaggagct gatgtagcct 240

tcaatgtcaa caacctgctt ggagacccag tggccaatat ggctatggcc catggcagct 300

ccatagcatc ccaagggaag gacatagtgc acaaagagct acaccgtttt gtgtctataa 360

acaaactcaa gtattttttt gctgtggaca cagcctatgt agccaagaag ctagggctat 420

tggtcttccc ttacacacat cagaactgga aagtgcagta cagtcatgat gtgcctcttg 480

cccccacgga aagacctcaa tgcgcctgac ctctatatcc ccaccatggc cttcataaca 540

tacgtgctgc tggctgggat ggcactaggc attcagaaaa ggttctcccc acagcatgtg 600

gggctttgtg agcgagacag gtactggtgt gggtgttaat ggagtgttgg ctctgcttct 660

ggcctctacc tggccaccgt acgcagtgaa ctgggcacct tccacctctt ggcctagact 720

ggctacaagt atgtagggat gatcctcagt gtgctcacag ggctagcttt tcggcagtga 780

tggctactac gtggccctgg cctggacatc gtctgcactc atgtacttca ctgtagatct 840

ttgcggactg cagcttgggt cctgacagca tgggggcccc acccctcggc agcatctcca 900

gctgtacctg accctgggag ccgctgcctc caacccctta tgatatactg gctgaccttc 960

cacctggtcc ggtgacccct ggtcctttgg tggcactgag ttttccatac attgaagatt 1020

tggtttcctt aaaaaaaaaa aaaaaaa 1047

<210> 551

<211> 1574

<212> DNA

<213> Rattus norvegicus (Norway rat)

<400> 551
acaacttcaa agcaaatgaa gttcgttctg ctgctttccc tcattgggtt ctgctgggct 60

caatatgacc cacacactgc ggatgggagg actgctattg tccacctgtt cgagtggcgc 120

tgggctgata ttgccaagga atgtgagcgg tacttagcac ctaagggatt tggaggggtg 180

caggtctctc cacccaatga aaatattata attaataatc catcaaggcc ttggtgggaa 240

agatatcaac caatcagcta caaaatttgc tcaaggtctg gaaatgaaaa tgaattcaaa 300

gacatggtga cgaggtgcaa caatgttggt gtccggattt atgtggatgc tgtcattaat 360

cacatgtgtg gctcgggcaa tagtgcagga acacacagta cctgtggaag ttacttcaat 420

cctaataaca gggaattctc agcagttcca tactctgctt ggtattttaa cgataataaa 480

tgtaatggag aaattaataa ctacaatgat gccaatcagg tcagaaattg tcgtctgtct 540

ggccttctgg atcttgcact cgataaagat tatgttcgaa ccaaggtggc tgactatatg 600

aacaatctca ttgacattgg tgtagcaggg ttcagacttg atgctgctaa gcacatgtgg 660

cctggagaca taaaggcagt tttggacaaa ctacataatc taaatacaaa atggttctcc 720

caaggaagca gacctttcat tttccaagag gtcattgatc ttggtggtga agcaattaaa 780

ggtagtgagt actttggaaa tggccgcgtg acagaattca agtatggtgc aaaacttggc 840

acagttattc gcaaatggaa tggagagaag atgtcttact taaagaactg gggagaaggt 900

tggggttttg tgcctactga cagagccctt gtgtttgtgg acaaccatga caatcagcga 960

ggacatggtg ctggaggagc atccatcctg acattctggg atgctagaat gtataaaatg 1020

gcagttggat ttatgttggc tcatccttat ggattcacca gagtaatgtc aagttaccga 1080

aggacaagaa atttccagaa tggaaaagat gtgaatgact ggattggacc acctaataac 1140

aatggagtaa caaaagaagt gaccattaat ccagacacta cttgtggcaa tgactgggtc 1200

tgtgaacatc gatggcgtca aatcaggaac atggttgcct tcaggaatgt agtcaacggt 1260

cagccttttg caaactggtg ggataatggc agcaaccaag tggcttttag cagaggaaac 1320

agaggattca ttgtctttaa caatgatgac tgggctttgt caagcactct acagactggt 1380

cttcctgctg gcacatactg tgatgtcatt tcaggagata aagtcaatgg caattgcact 1440

ggacttaaag taaatgttgg cagtgatggc aaagctcact tctctattag taactctgct 1500

gaagacccat tcattgcaat ccatgccgac tcaaagttgt aagagtcaaa ttaaagagat 1560

ttagattcag cacc 1574

<210> 552

<211> 773

<212> DNA

<213> Rattus norvegicus (Norway rat)

<400> 552
tcttgtggga gctgctgttg cttttcccgt ggatgatgat gacaagatcg ttggaggata 60

cacctgccaa gagaattctg ttccctacca agtgtccctg aactctggct accacttctg 120

tggaggttcc ctcatcaatg accagtgggt ggtgtctgca gctcactgct ataagtcccg 180

catccaagtg agactgggag agcacaacat caatgtcctt gagggcaatg agcagtttgt 240

caatgctgcc aagatcatca agcatcccaa cttcgatagg aagaccctga acaatgacat 300

catgctgatc aagctctctt cccctgtgaa actcaatgct cgagtggcca ctgtggctct 360

tcccagctcc tgtgcacctg caggcactca gtgcctcatc tctggctggg gcaacacgct 420

cagcagtggc gtcaatgaac cagacctgct ccagtgcctg gatgccccac tgctgcccca 480

agctgactgt gaagcctcct accctggaaa gatcactgac aacatggtct gtgttggctt 540

cctagaggga ggcaaggatt cctgccaggg tgactctggt ggccctgtgg tctgtaatgg 600

agagctgcag ggcattgtct cctggggcta tggctgtgcc ctgccagata accctggtgt 660

gtacaccaag gtctgcaact atgtggactg gattcaggac acaattgctg ccaactagag 720

ctctccaatc cctctacagt cattatgtca ataaagtgaa atattcttta ctg 773

<210> 553

<211> 1725

<212> DNA

<213> Rattus norvegicus (Norway rat)

<400> 553
agtgctgctc cggtaccgag tcgcgctcac gtttgtcctt aagactccct tcggtgtctc 60

caggaccctc tttccttcct ccgcagcgat cctactgcca gaacttcacc atgtccattc 120

tcaagatcca tgccagagag atctttgact cccgcgggaa tcccaccgtt gaggtggatc 180

tctacaccgc aaaaggtctc ttccgtgctg cggtgcccag cggtgcgtcc actggcatct 240

acgaggccct agaactccga gacaatgata agacccgctt catggggaag ggtgtctcaa 300

aggctgttga gcacatcaat aaaactattg cacctgctct ggttagcaag aaactgaatg 360

ttgtggagca ggagaagatt gaccagctga tgatcgagat ggacggcaca gagaataaat 420

ctaagtttgg tgcaaatgcc atcctgggag tgtccctggc tgtctgcaag gctggtgccg 480

tggggaaggg ggtgcccctt taccgtcaca ttgccgactt ggccggcaac cctgaagtca 540

ctctgccggt cccagctttc aatgtgatca acggcggttc tcatgctggc aacaagttgg 600

ccatgcaaga gttcatgatc ctgcctgtgg gggcatcctc tttccgggaa gccatgcgca 660

ttggagcaga ggtttaccac aacctgaaga acgtcatcaa agagaagtac gggaaagacg 720

ccaccaatgt gggtgatgag ggtggattcg cacctaacat cctggagaac aaagaagcac 780

tggagctgct caagtctgcc attgcaaagg ccggctacac tgaccaggtt gtcatcggca 840

tggatgtggc tgcctccgag ttctacaggg ctggcaagta tgacctggac ttcaagtctc 900

cagatgatgc cagccggtac atcacacccg accagctggc cgacctgtac aagtccttca 960

tcaaggacta cccagtggtg tccattgaag atccctttga ccaggacgac tgggatgctt 1020

ggcagaagtt cacagctact gcaggcatcc aggtggtggg ggatgacctc acagtgacca 1080

accctaagcg gatcgccaag gctgcaggcg aaaagtcctg caactgcctc ctgctcaaag 1140

tgaaccagat tggctctgtg accgagtctc tgcaggcgtg taagctggcc cagtccaatg 1200

gctggggtgt catggtgtcc catcgatctg aggagactga ggacactttc attgccgacc 1260

tggtggtggg gctctgcact gggcagatca agactggtgc cccctgccga tctgagcgcc 1320

tggccaagta caatcagatc cttagaatcg aggaggaact gggcagcaaa gccaagtttg 1380

ccggcaggtc cttcaggaac cccctggcca agtaaggcat ggaccggaga tccctggagc 1440

taccagatcc tctgtctccg tcatccaggc ggctcaaggc tggcccagtg cttgcccctc 1500

ccatgtcact gcttccttag atgtccaccc cgaccacctg gagccctgct ggagccccca 1560

gctttgtaat catgtgatca gtctgaatca ttgtttctgt cacctgactt tccagctagt 1620

gtctggagcc ctctgaactc cagcgtaatc tctagagtgc ccacaccatc aagactcccc 1680

ccagtggttt acttgcaaaa ataaaagctg cagaagctca aaaaa 1725

<210> 554

<211> 2362

<212> DNA

<213> Rattus norvegicus (Norway rat)

<400> 554
atcatcagag gacttggtgg aggagagctt ataggtttgg ctgctgggag gctgctttgc 60

ctggacttgg aagattttcc tgtaggcttc gagaggcatg gactcctacg tgattcagac 120

ggatgtcgac gacagcttgt cctcagttct ggatgtgcat gtcaatattg gtgggagaaa 180

ctcggtacaa ggaagaaaga aaggcaggaa ggccagatgg gacgtgagac cctctgacat 240

gtccaataag accttcaatc ccatccgagc catcgtggac aacatgaagg tgcagcccaa 300

tccgaacaag accgtgattt ctctgtcaat tggggaccct actgtgtttg ggaacctgcc 360

tacagaccct gaagttaccc aagccatgaa agatgccctg gactcgggga agtacaatgg 420

ctatgccccg tccatcggct acctatccag tcgggaggag gtcgcttctt actaccactg 480

tcatgaggct cctctggaag ctaaggatgt cattctgaca agcggctgca gtcaggccat 540

tgagctatgt ctagctgtgt tggccaatcc tggacaaaac atcctcattc caaggcccgg 600

gttttccctc tataggactt tggctgagtc tatgggaatt gaggtcaagc tctacaatct 660

cctgcctgag aagtcttggg aaattgacct aaaacaactg gaatctctga tcgatgaaaa 720

aacagcgtgt cttgttgtca acaacccatc caatccctgt ggctccgtgt tcagtaagcg 780

acaccttcag aagattttgg cagtggctga aaggcagtgt gtccccatct tagctgacga 840

gatctatggt gacatggtgt tttcagattg caaatacgaa ccactggcca acctcagcac 900

caatgttccc atcctgtcct gtggtgggct ggccaagcgc tggctggttc ctggctggag 960

gttgggctgg atcctcattc atgatcgaag agacattttt ggcaatgaga ttcgagacgg 1020

gctggtgaaa ctgagtcagc ggatcctggg accatgcacc atagtccagg gtgctctgaa 1080

gagcatcctt cagcgaaccc ctcaggagtt ctatcacgac acgttaagct tcctcaagtc 1140

caatgcggac ctctgctatg gggcactggc tgccatccct ggactccagc cggtccgccc 1200

ttctggagcc atgtacctta tggtgggaat tgagatggag catttcccgg aattcgagaa 1260

cgacgtggag ttcacagagc ggttgattgc ggagcaggct gtccactgtc tcccagcaac 1320

gtgcttcgag tacccaaatt tcttccgagt ggtcatcaca gtccccgagg tgatgatgct 1380

ggaggcttgt agccggatcc aggagttctg tgaacagcac taccactgtg ctgaaggcag 1440

ccaggaggag tgtgacaaat aagccctcac ccgtcctcct gaagacatgt cccacgcaag 1500

gacggttgga ctggaccgct cctcctcagg gagccaaatg tctctgatgg gagaggaacc 1560

tctaaagcac cgcatgaaga cttgaagatc atttctcctt cccccagttt cacccaacgc 1620

acgcctggaa acccagaccc ctccctactc agttctgctg gagccatgag ctcactgccc 1680

cccacccccc acttccttat cctgagggta tcagaccaat ttaccagaaa gaagctgggc 1740

caagaaaaga gagagctcag gatgagagaa gcagaagacg gaggatgcct ctcccaccca 1800

tttcctcgtg ctgacatcct cttcagtcag gtcggaagtc acctcagttg ctgcctcact 1860

tcaggcaaac ctcactcctc gtggtacagc tacaccagag agaagtcgag gacttgtgaa 1920

ttcagttaat tgtattctaa acacatcagg aaacttgatg tttaaaggat ccctgacaga 1980

atttttattt ttattttttg taaaggatta ttttgcggtg caaataaata ccagcttcct 2040

aaactgatca acttcctaac tgattagata gatctacttg tatcaggttt tcctaaaata 2100

ggaggacacc tcactgaagg agaaagaagg gaccggaggc ctctctagag actttgctgt 2160

gtacaggaca gcatcttcct ccttgtcttc tcgtagaatc atatatagcc acttttgcta 2220

tcatgtgaat attgatttat tgatatttgt aatattaact tttcatatat tttcctaaga 2280

aacatttata ttataaggcc ttttattttg tcaagggcat aaattatttt ttctttattt 2340

ttgaaataaa aatttatcaa at 2362

<210> 555

<211> 1242

<212> DNA

<213> Rattus norvegicus (Norway rat)

<400> 555
aacatggccc acctctgcat cgtgggctgc cacgcggtga acggggtagc gaagatccac 60

tcggacatcg tgaagaccca agtattcaag gacttcagtg agctagaacc agacaagttc 120

cagaataaaa ccaacgggat caccccgagg cgctggctct tactctgcaa cccagggctg 180

gctgacttga tagcagagaa aattggagaa gactatgtga aagacctgag ccagctgacg 240

aagctccaca gcttcgtggg cgacgacatc ttcctccggg aaatagccaa agtgaagcag 300

gaaaataaac tgaaattctc ccagttcctg gaaaaggagt acaaggtgaa gatcaaccca 360

tcttccatgt ttgacgtgca cgtgaagcgg atccacgagt acaaacgaca gcttctgaac 420

tgcctgcatg tcatcaccat gtacaatcgc atcaagaaag accctaagaa gttcttcgtg 480

ccaaggacag tcataattgg tgggaaagct gccccaggat atcacatggc caaaatgatc 540

ataaagctgg tcacctccgt ggcagaagtg gtgaacaacg accctatggt tggcagcaag 600

ttgaaagtca tcttcttgga gaactacaga gtgtctcttg ctgaaaaagt cattccagcc 660

acggacctgt cagaacaagt ctccactgct ggcacggaag cctcggggac gggcaacatg 720

aagttcatgc tgaacggggc cctgaccatc gggactatgg atggggccaa tgtggagatg 780

gcggaggagg ccggggagga aaacctgttc atctttggca tgagggtaga tgatgtggcc 840

gctctggaca agaaagggta tgaggccaaa gaatattatg aggcccttcc agaactgaag 900

ctggtcattg accaaattga caatggcttc ttttctccca atcagccaga cctcttcaaa 960

gacatcatca acatgttatt ttatcatgac agatttaaag tctttgcaga ctacgaagcc 1020

tatgtcaagt gtcaagaaaa agtcagtcag ctgtatatga atcaaaaagc ctggaacaca 1080

atggttctca gaaacatagc tgcctcgggg aagttctcca gtgaccgaac aatcagggag 1140

tatgccaagg acatctggaa catggagcct tccgatctga agatctccct atctaaggag 1200

tccagcaatg gggtcaacgc caatgggaag taaatgctaa aa 1242

<210> 556

<211> 25759

<212>  DNA

<213>  Rattus norvegicus (Norway rat)


<220>

<221>  misc_feature

<222>  (7)..(7)

<223>  n


<220>

<221>  misc_feature

<222>  (8)..(8)

<223>  n


<220>

<221>  misc_feature

<222>  (9)..(9)

<223>  n


<220>

<221>  misc_feature

<222>  (10)..(10)

<223>  n


<400>  556
aagcttnnnn aacaatgtgg cactgggcaa gaaactcaga gccaggggcc atactcttct      60

caggtgatgg atgaacactt caagactcat ggtgctagga gcaagatgga catttcagtc     120

taagctagag caagaggagc aatgagaagt cttggcttca ctgtggtatg atggctagcc     180

aagcacaggc ggcggcgagc tcagcacgct ctccagatag tcatttcctt tgcctggcct     240

ctctgatatg aaattgccta aatcttggga agggatctct gaaggcccta tgtctcatca     300

ccataacatg gccttacgct ggggaaagac tgtcttccaa ttccaccata gctccttgaa     360

gtgtcctaga caggagagaa catacagagg atgtttaata acggtcccta tgaagacttc     420

agtcctgctt tccccttcta cccttccagt gtatgggaag gctgtgaggg gatggtcctt     480

attatcaagt gggatacatc ggcatgcagt tctctagtaa atgaacaacc aaggtcccac     540

acaactcaga ccctcgcccc aaagattggg ccaagaaaaa cagagggatg ggaatggaca     600

attttcaact ataccttgac tctcttatta aggcttaatt ttccagtccc aatttagaaa     660

tgacagcatc tgttaggtga tacccgcccc ccctttctct tcagtcccct accgtcccct 720

cacctcccat ctaggctggc agctgtcact gtgaacacgg gctggtggca tagctccctg 780

tcattaactt gcttgttggt cctgctagag cttaagaaag gattaagtaa ataatgatag 840

taatgagaca gagagcaatg ggagaaaggg aagagatagg gaaggagaaa aaaaaaccct 900

aaattcaaca gcccaagttt tacagtcaaa ctgccccgcc acaaccttgg ggtgtgctga 960

gtctgtctca taatcccaag ggatttcgac aggtccaatc ccattttatt catttttctc 1020

atcagtgagc tttctaagca aatagcacaa tggtgtgtcc cctgttccca agtggagcag 1080

cttcttcgta tgatttcagg gtgggatggc ctgcaggatg gagacaggca gggccctgaa 1140

gcaaagacaa gcccattgct ctaggtgaag aagcaacagc cctgagtcca gagcaaggca 1200

tctcaaattc cctgctttct acagttggaa aaatggtttc ggaccctgct catttctata 1260

tatactttcc ccttggcaac gatgaaaaat acgtgtggag ggggccagtt ctcagcctgt 1320

ggtgtcagtc attctctttc caaatttaga gaaagaatga gcccaggggg ttagtggtta 1380

gcctataaaa gcccaggggg cggctctcct ctgtgcttca ctctgccaca gtcagaggta 1440

agtaaactct cttcatatct ctttggcctt tgctgcttcc ctgcctggtt gggtcactgc 1500

cctgggctgt ggccctccct ggctttgtta tagcaggtga ggctcattta tcttttcaag 1560

gcgtcctcaa agaatacagt ggagtgagat gtgagctgtg cctgccaccc actgcctcta 1620

gcaggtcatc gaagctgggg tcctgcaccc tctttctttc ctctgtttgg gaaggatcct 1680

gttggacatg cataaggcca tgatgccatg ttctcctgtt gcctctctga tccacagagt 1740

gaacagggga ctctgcactc cctacctgga ctctataccc attgagtgtt cagttgcctc 1800

aagactgcat acttgctcac caggtttaaa ttccataatc tcagcattgt ctccactttc 1860

agataaccag gaggcctgac cccaactgct aacacaagct tttactggca ggactccgtc 1920

catttagaat ccctgccctg ttcacaatag aggatcttgg agtctgaaga aactgtctgg 1980

agtgggttag aggagccaat ctgtcaggca gctggtagct atgcctagct gtagaccacg 2040

acttgtctgc agttataggg ctggtctcag ggctaaggct ctttctgtcc agtcctttct 2100

gtgtctgaat tgagcctcac caaggtctgg ttcatcatag aagcagacaa catggtgatc 2160

actaagcttc tctgtgccca tgctcttgca ggtcccatag gtccacagct gctgctgctg 2220

ctctctgctg tcctgccggt gtgactcagg tgagaattgg ggccacatcc cagggcttca 2280

agaagtcttc aggtaaaccc agatcaccca ttgccccctc acgcctgtgc cctgaggagt 2340

tacagatctt agatacagaa gaataggctt cctccccagg gctgcagcta gaagttccca 2400

tttattcttt gtggtggttt gctggttttt tgagacagga tctcactatg tagccctgga 2460

tggcatggca tagaactcac taagtagacc aggctgcctc tgcttctgaa gtgctgagat 2520

ttaaaggaat agaccaccgt gcctgggagg aagaggccat gtcaattaaa aagcagttca 2580

aactgcctct gccattttct gctcagcatc ctctctttga ggaatgcctt tacttatttt 2640

tattatggta tttaacttgg gatgttctgt tctccatacc tgtacttctc agaaaaggaa 2700

gacaaagtgt caaaagtggc atttcaaagg ctccttcacc agactgaaag aatcaagaga 2760

cacttggatt aaacagattc taagcccatg gcatacagta gcaactcaac aaacacgagt 2820

ggattatgtt ttcccagtgc tgagccctcg tgtgaagctc cactgggcat gggacaggcc 2880

agatctcctt gaccttgtct gagaggctgc catccacttc agggtgacag gttgatacta 2940

ggatgggcag gaggctgccg gctgccggct gccggcccag ggagcagctg ggctgactgg 3000

cagtttgttt tgcagccaac actatgagta gcgacaccga gatggaagtg tttggcatag 3060

ctgccccttt cctccgaaag tcggaaaagg agaggatcga agctcagaac cagccctttg 3120

atgccaaaac ctattgcttt gtggttgact caaaggaaga atatgccaag gggaaaatta 3180

agagtagcca ggatgggaaa gtcacggtgg agacagaaga caacagggta aggcatttgc 3240

tttttctctt tctcagcact ggcccacgct cctcacatga cgttgcataa gcatgtactc 3300

ctccacccac ttctggagtc agagcctggc tctggaattc agcatccgaa acagcgctgg 3360

caacccagcc gccagaagaa agccgaatgc cctagcctat gtgggagaat gggagacggg 3420

tccccaggtg gctgattaaa gcatttactt ttgggtgtct ttttatcact gccttgttct 3480

gggccagaca ccaatctcaa tgtctcaagg gtggagtgtt tttagaacac ccaccctttg 3540

tggttcgcct agctctgtcc atggtcatcc aaccaaggaa ggcggggtga tgtataagaa 3600

atgctatagg acaacaaggt caaagaacca ggctctctgg aactggaatg ggacgggcaa 3660

ctgagttcac tgtgttctgt gggaggaagg gaggaatttg ggggctttcg aaaatcccga 3720

atctggaaat gtttcaagga agaaaacaaa agcaactgtg ggtatggctc agtgatagaa 3780

cggcatacgc aaagtcctgc tttcaatcgt cacattcagt caccacctgt gtcagggtgg 3840

gtgtaacgga ggtagaagca gagacagaac agaagatgca agagacaaaa tggcggcatt 3900

cgttggagca gggaggaaat gggcagcacc agcgtggaag ctcccagaac gaaggcatcg 3960

ctacatatct aagattaaaa atctgagaaa gggcttccac cgcaggacag aaggcagggg 4020

ctaaactcaa cgtaggatcc tcacagggtc tagaacatgc ctgagaggtg tcaagagagt 4080

ccacagccta agtggattaa gggagggaag gaaggcagct ctgtggccca cagtgctcaa 4140

ggaccggctt ttccagagtc actcctaaag agagaggagg gcagggactg ataaccccac 4200

ctgcaggtcc ttaggccctc cacagaggac agccccaggg cttggtggtg aaagtcttgc 4260

tctcaaagcg gaggccaatg gctcttcccc aagagcaggg aaattggtaa aggaagtagg 4320

atggggtttg gttttcagca atggagggct gaattacata gatgggggcg tggttctcag 4380

cgctgattgg ctgcttctac agggcagtga cgttagcagg ctctgtgggc actcccaggg 4440

ctacagtgcc ttctctaatt ccctgttgtc gttggtcaag attactgcag acattatctt 4500

ttctcttagc aggtaggaga gctatagggc aaccgaagtc atagacaaat aacttcctct 4560

gactccaaat tctgtgttac aggcccctac ttagtcctca gaaatcttct ttcatcacat 4620

tggttgccat cccccaactc caggtccaag agaaacacac acacacacac acacacacac 4680

acacacacac gcacgcacgc acgcacgcac gcacgcacgc acgcacatgc cagagaatgg 4740

gagagacaga gagacagaga gacagagagt gagacagagg gacaaagaga cacagagaca 4800
caaagggaga cagagagaaa gacacacaga aagagacaga gacagagaga cagacgtcct 4860
gagtggactt cattttagaa atcgtttctt tatgagttat caaaagaagt atgggataat 4920
agtaaaggtg aaacatatat attacatata taatacatgt atatgtgtat ctacatatat 4980
gtatgtattc tgcatgtatt ttttctccca tagacctatg tgggagcaga ccacatgctt 5040
tcccactgcc acatacctcg cttttcctca ctgtcaccag gcatttcctc tgctccctgc 5100
agtctctcct aaggtgccag acatccacgg caatccaatg gctggggaac attaatgact 5160
tttcccttcc agaccctggt ggttaaacca gaggacgtgt atgccatgaa ccctcccaag 5220
tttgacaaga tcgaggacat ggccatgctg acccacctga acgagcctgc tgtgctgtac 5280
aacctcaagg accgatacac gtcctggatg atctacgtga gtggctactc accaacaggc 5340
tatgctggga cattagctgg gtgctgggga gtggggagag gcactttgaa agattctcgt 5400
ttgcctggcc aaaggagagg aaatgaagta gagaagtgaa tgaccagaca aggagagata 5460
gatgggatgg gaaggggagg ggctggagga agggagggag ggaggaggaa ggcgaaggag 5520
gagctaagat tgggaggagg agctccaaag agcaaaaaag ctgaggttac cttgagcttg 5580
gtattagctg tgggaggact ggctactctc ccccttgagt ttcagttact tcattcatca 5640
ctagaaatca cgatttcttt atgaattatc aaaagaagcc tggagtgtta gtaaaggcaa 5700
tatatataca tatgtatata tgtatatata tgcagtacat gtgtatatgt atatatgtat 5760
atacatatgt atgtatgtat gtatgtatgt acatatgtaa gtacggatgt attttctttt 5820
cctcccatag acctattcag gcctcttctg tgtcaccgtc aacccctaca agtggttgcc 5880
ggtgtacacc cctgaggtgg tggatggcta ccgaggcaaa aagcgccagg aggccccgcc 5940
ccacatcttc tccatctctg acaacgccta ccagttcatg ctgacgggtg tgttttcgag 6000
aaggaagtat gcactagaca ggcttggggg cactctggtg tgaagcccta gagaaggagg 6060
ggcaaggagg aaaagaggtt ttaggtcctt ggggtgagga gtttgcattt aacataagta 6120
ttggtgagac cataggaaag agtatcccta ccaatcagaa gactgtgaga tcagaggatc 6180
gagaagcctc atggttctca ggacagacac tttttaaatc cccacctgtg actaagcaga 6240
caaacaaaca agtaaataaa taaagggaag aggaaaggaa cagttgaaac ccactcttgc 6300
atgacgaata agaacctctt tggacatagc catcagctct gctttcttcc cctcacacag 6360
atcgtgagaa ccagtccatc ctgatcacgt gagtatccct gcttagagag gcctctcagc 6420
tgggccatgg atgtatccct cagctctatt cattagacag tgcttggagc acagcccatg 6480
gtgaaaagac agcttggggt gattcaaaca agcctcagaa aacgagtagt acccatgctt 6540
ggatcaacta aggctgtagc ccaggtcctc tctgtggcga ctcatttgat gtgtgtgcca 6600
gacgtgttga agaccactgg atgacatgcc agggtgctgg tttatgagta ggaccgactt 6660
ctctgtctcc gtctttctaa atagcggaga atccggggcc gggaagacgg tgaacaccaa 6720
gcgtgtcatc cagtactttg caacaattgc agccactggg gaccttgcca agaagaagga 6780

ctccaaaatg aaggtgggga cgggagcaag gaaagaggct ggacgggcat aagggacagc 6840

ttggggttga ccgtggattc agacccctgt gattccttag cagatgagat ttgaagactt 6900

tataaggggg gggggatatg gagtcaaatc cgtgagtttg ttccttcagg tggcctcaca 6960

gagagcagcc caagagcaca gtgaggaaca catatcccct gactcggtgt actgcaaggg 7020

cgtgggagga acacaggcag cctcattaac tgaagggtca aacgaaggca tggccactca 7080

agagatccag ctgctgacag ggacttgaga gaggcagcag tgtttcaagg aggccatgct 7140

gctccattgg cctttgagtc atcaagtcac cggggcagcg agcaagctag gcaggcagga 7200

aaagcagagc aggtggcaat ttcttaaaaa tgaggatttt tgcagcttac aggcacattt 7260

tcccttcctc tttggatttc ttacttaggg gaccctggaa gatcaaatca tcagcgccaa 7320

ccccctgctg gaggcctttg ggaatgccaa gaccgtgagg aatgacaact cttcccgctt 7380

tgtgagtctg cccttgagta tgataggctc ttcttcccgg cctgctggac agttcaagag 7440

cttagggaac ttacaggccc ggtggaaaga gacagtgaaa gaaacaatgc acagccatgt 7500

gtgtgaggtt caaaaaccca aggctgtaac tgagaaactg gggaccaggt ccccaacaga 7560

tagggctaga tgataagggc tttctaggcg ctgggaatat gagtcgaagc tgcagggtaa 7620

ggaagggaat gagggaaaga gtggcttggt tctcccaggg tgggaccgtg tttggagcac 7680

tcctgcagag gctccatgtg acagatgggg aagcagaaga tggagagact tgtcactaac 7740

aagggatgca ctgtcccttg ttattagctg tcttcagaca cacttcagaa gaggtcatca 7800

gaccccatta cagatggttg tgagccacca tgtggttgtt gggaattgaa ctcaggacct 7860

ctggaagagc agccagtgct cttaacctct gagccatctc tccagccctt gggttttgtc 7920

ttaagtgcag taggaaggat tctgtaatca gggaagtgac atggttcagc acctgggtta 7980

tttcattaat gggatatgag aaatcccgga ggaggcagac acaccagata ggagaagagg 8040

ggacaggagg gaagacctac tggtgaaagg ctcctatggg cagtgctcag gaatccagag 8100

gaagttcctc agtcatggaa ggatctgctc tccctctctc tctatgcagg gcaagttcat 8160

ccgcatccat ttcggcacca ctgggaagct ggcctctgca gatattgaaa cttgtaagta 8220

ggctcgttga gctgggtgaa gcctccaagc cacggggagc attgtgaacc ctgaccggcc 8280

ctgtgtttct gttccagatc tgctggaaaa gtccagagtc accttccagc tgaaggctga 8340

gagaagctat cacatcttct accagattct ttccaacaag aagccggaac tgatcggtaa 8400

gctgatgggg tagggtgcag gtggagggga tgacgtcacc ctttgggcag ttggatcatt 8460

agagcccgga gagttggtcg tacctgcaga tgactcttac agtcaatgca gaattatatc 8520

agccacagga tatagctctt tgggaggcgg ctgtatcagg ttttggaagt gtatatgttg 8580

tctgctggtg tatgtatgag aatgacactc ggtgtcttcc ctgatttctc taaaccttgt 8640

tgtattaagg caagggctct cactaagcct ggagctactg tagcttgaca gaatgctgct 8700

aggatccacg tctctgcctt ctgaggatta caagcagacg cccccccccc cccccgactt 8760

ttatatgagt ttaggggatt caaactctag tccttatagc tttcacggca agctctttac 8820

ctgctgagcc atctcccagg ctcactgtac caatttgaaa atctgaccag atgtgctgta 8880

atggagcaaa atcattactg gttccatggt ggtctttgaa tagttaagca cttaatggtt 8940

cttgagcgcc ctcttgtgtt tcaacatagg aattgatctc agctcgcata aggccggtgc 9000

aaactgatta aggacccaga gggtcattct atctctatat tttttcagct gatagaggcc 9060

tgtgtgttca ctaccttcct tactttctgt ctgtgatagc taagcctcct gatctccatc 9120

ctgcaaacag gctgtcagtt ttcaaccaac tttaaaacag ctatcccaat cagcctatgg 9180

tttctccagc ttgggctccc attacccagt tttacttgtt cactatccca aacatacact 9240

ggcttgcaac attcatttgc aaagcaatac cacggatatg ttttcctagg atcctcaact 9300

tacatggcat gggagattgc cctttccctt agaccagtag tagttctcaa cctaggaagg 9360

ttgaaatgct agaacccttc aatacaattc ctcacattgg ggtgatccca accataaaat 9420

tatttctgtt actacttcat aactatcatc ttgcaatgct taaaaaatat ctgtgaaaca 9480

cacatggtat gcactcattg ataagtggat attagcccaa atgtttgaat taccctaaat 9540

gcacagaaca catgaaactc aagaaggatg accaaaatgc gaatgcttca ctccttcttt 9600

aaaaggggaa caggaatacc tttgggaggg aatagggagg caaagtttag aacagaggca 9660

gaaggaacgc ccattcagag cctgcccaca tgtggcccat acatatacag ccaccatact 9720

agataagatg gatgaagcaa agaagtgaag gccgacagga accggatgta gatctctcct 9780

gagagacaca gccagaatac agcaaataca taggtgaatg ccagcagcaa accactgaac 9840

tgagatcagg acccccgttg aaggaatcag agaaagaact gagagagctt gaaggggctt 9900

gagacctcat atgaacaaca atgccaagca accagagctt ccagggacta agccactacc 9960

taaggactat acatggactg accctgggct ccaactgcat agatagcaat gaatagccta 10020

gtaagagcac cagtggaagg ggaagccctt ggtcctgcca agacaaaacc ccagtgaata 10080

ggattgttgg agggggcggt aatgggggga ggatagggag gggaacaccc ataaagaagg 10140

ggatggggag gggttagggg gatgttaggc ctggaaactg ggaaagggaa taacaattga 10200

aatgtaaata agaaataccc aatttaataa aggtggagga aaaaaaaaag aaaatatctg 10260

tgtttttcca atggtcttag atgaccctga agaaaaagcc ttttaaccca aaaggggtca 10320

cgagccacag gttgagaacc actgctctag ggtattctac tcctgcacat tttggcccca 10380

gtggttgaag tcccaggcca cacttccacc tggtggagat ctcattacac atgtgtgaat 10440

tcctcatttc agagttgctg ctgattacga ccaaccctta cgactaccca ttcatcagcc 10500

agggtgaaat cctggtggcc agcatcgatg atcgtgagga actgttggct acagacgtaa 10560

gaaggactta cggtagagag gtgggagaac ctccacgtag ggagggcaat gaatgggcag 10620

acatgctgtt ctttccactg cagagtgcca ttgacatcct gggcttcacc ccggaggaga 10680

aatctggact ctataaactg acaggggctg tgatgcatta tgggaacatg aagttcaagc 10740

agaagcagcg agaggagcaa gctgagccag atggcaccga aggtacagag tcaccacagc 10800

aggcttcaga gtggaagcgg agaggtcaac tcccctgggg ccatccagaa cctgttttaa 10860

aaatgattaa tctgtctttc ctctggagag gtggatgaac ctttcctggg tctgcaatga 10920

acataaggaa acatcaccat ctattaaaaa acacttatta gtttagtgtg tatgtgtgtg 10980

tgtgtgtgtg tgtgtgtgtg tgtgtgtgtg tgtgtgtgtg tgtaaggatt ctacacctca 11040

tgtgttgaag tcagagtaca actttaagag tcagttctct ccacaatgca agtctttggg 11100

accaaacccc gttgccgggt atggtacccc atgcctctac ctgtttagcc atctctctgg 11160

tccctctacc ccccttctgc tgaattgcta caaaggtctt cttctggatg caatgtgaaa 11220

tggccaggga cttccaaacc caatcctgtt gctccatgaa gtctgccaca ctctctggat 11280

ggtgtagtgc catgggccca cagagatgac aaggagtaag aaaattgctt tccttctgcc 11340

tcactcaaca tgatctttcc tttcttacca gtggctgaca aaacagccta cttgatgggc 11400

ctgaactctt cagacctcct caaagccttg tgctttccca gagtgaaagt tgggaatgag 11460

tacgttacaa aaggccaaac tgtggaccag gtaaggacta cctcaagtgc aggaggggcg 11520

gaggaccctc atggccgcag ccttcttacc agttcactgg cctttcctcc gacaggttca 11580

ccatgctgtg aatgctctct ccaagtccgt ttatgagaag ctgttcttgt ggatggtcac 11640

tcgtattaac cagcagctgg acacaaagct accaagacaa cacttcattg ggtttttgga 11700

cattgcgggt tttgaaattt ttgaggtttg tattatttat gtcttatctc ttcatacggg 11760

agttttcata atcctaaaaa gagggtacca agatggtttt cttgtaggaa ggatagaagg 11820

agtgagatgg atagaagaaa gacagaacat caggacagtg cgtgttattt taagacaatt 11880

ggagaggcct gggagatgat tgactcagta aactgcctgc cttgaaagtc gggagagctc 11940

agtatgattt ctagactact caagttaaaa aacaaaacaa gctgggcgtg gtgatgtgtt 12000

ctcagaagtg gagatgcaga gacaatcaga tccttggggc tcgctggcca gtcagcctac 12060

cctacccaga aatttccagg ccaagaaatc ctgtggagtt aaaaaaaaat aaagaaaaag 12120

aaaaaaatat gggtattgga tgttgcttgg ggaatgacac ccaaagttgt cttctggcct 12180

ctgcaggggt atacacacac acacacatgc ctgtgcaccc acacgtattg tgcacatgtg 12240

aatatttcat acatgctgat acacacaggg ctgtagtcct tccttcaagt ggactttcac 12300

aagatatcac caaatgggta gaattaagaa aaatctaata gggggccaga gaagtagata 12360

ctgtcaatca tgtactagtt gacataattc tctgactggt tctgagttag gagcaaatgt 12420

gtgtatttag tgtttgccag gtggcagcca tgcaggtggg gagatgggaa gcttgtagtt 12480

tagttgagaa gaaacagaag cctgtcgggc atgcagaagc acaggagggg cccagaggcg 12540

ggagaaccat gtaggtggaa ggctcgtgat tggctctgaa agcatagcca agcaggtggg 12600

acctgaccct tgagaaggaa ttcagactgc gttttccatt ttgaaagagg tttgattttt 12660

tcgcatgcac gttttatttt ggcgccagta taacagcctg gagcagctgt gcatcaactt 12720

caccaacgag aaactgcaac agtttttcaa ccaccacatg ttcgtgctgg agcaggagga 12780

gtacaagaag gaaggcatcg agtggacctt catcgacttc gggatggacc tggcggcctg 12840

catcgagctc atcgagaagg tatccccctc cgtctgctct gtgccccctc cctccccatg 12900

tgctttctgc tcctgtttac tctgctgctt catctcccct tcagccgatg ggcatcttct 12960
ccatcctgga agaggagtgc atgttcccca aggcgacaga cacctccttc aagaacaagc 13020
tgtatgacca gcaccttgga aaatccaaca acttccagaa gcctaaggtg gtcaaaggca 13080
aggccgaggc ccacttctcc ctcatccact atgcgggcac cgtggactac agtgtctcgg 13140
gctggctgga gaagaacaag gaccccctga atgagaccgt ggtggggctg taccagaagt 13200
cttccaacag actcctggcc catctctatg ccaccttcgc tacgacagat ggtaagtaac 13260
ggacttcaga cacaggagct ttgtcttcac acaatcttcc tttcccacgg cacccaaaag 13320
tcgcaataca tccactcaca gtgcctgggc cctttggtgt ctttcagctg acggtgggaa 13380
gaagaaagtc gctaagaaga aaggctcttc cttccaaaca gtgtctgcgc tgttcagggt 13440
aggacaaatt tgagttaatc cactcacagt tcacctgagt cacagcaaaa tgagcaccgt 13500
tgagatccta ggcctggttt attcaagagt agtaatgctg gctgcctgac agctctctca 13560
gagtgagatc aattgacagt ctgacaatat acagatgagg tttaacaaat gtggggcctg 13620
ggtttgttcc atcgtgtact gtaactagca ggtctggctg gctgggggca gtgagttctc 13680
agttccaatt atctagttgc ctgcagtagt acccaggccc ctggcaggga cagacacatt 13740
ttctgcgtag atttcagaaa ccattttgta gttggtggcc atcttgcaac ctgggagaaa 13800
gatgactgag gcaggtcaca tttccctgtg tttgggacca aacataagac ttcagttttc 13860
ttcttctttt ttgattaatc cgtatataat gagatttctc aaggatggga cccaaatcta 13920
aacacgacgt tcatctgtgt gtttcatata tgccttgtgc agcaaggacg tagatcgttt 13980
tacatggtat atgatcatgt aggctgtttt gcctacataa ggtgggacgt tcgatttgtg 14040
tatcatgttt gtactcaaaa aaattcagat ttatagaaga aaaacactcc agatttttag 14100
agcactttag attttagatt ttcaaggtgg ggatgtccaa gtcacggccg ttgtctacgt 14160
ctctaggttc atgactattc aaggacagag ttaacgtcac aactcttaag ttgaagccat 14220
agagtttagt tttcaccacc acgcaactat ctgtctccgt ggaagttcac agtaggctgc 14280
caaaggaaat gtttattttt tttcttttag gaaaacttga acaagctgat gtccaattta 14340
agaaccactc acccacactt tgtgcgctgt ataataccca atgaaaccaa gaccccaggt 14400
aagacgcctg ccagccctga ggttaccgct cagtctttaa ggacctgtgg gtgaagctcg 14460
ggtatcctgc ctgtgttctt aggggccatg gaacacagcc tggtcctgca ccagctgagg 14520
tgtaacgggg tgctagaagg catccgcatc tgcaggaagg ggttccccaa caggattctg 14580
tatggagact ttaaacagag gtacaccgtc aaaatgctct gtttgtcctt tgacatggat 14640
aagggttggg atttgaggga agaaatatta catgaaaaga cccatttcaa aaaggcagtt 14700
ttggttggtc ttccatgtct acggtttggt cgaggttcat gctcctgtgc tgtccacgtt 14760
tctttggaac ttgtctgata tacaacaggg gagggaaaga aagccctggt ccaggagtcc 14820
cacggcctag atcccagcaa agtggctatg ggaaggttgc tttacttcct gggaactaga 14880
ctttctggtc tgtaggaagc gatgcctgca atcacagagc actgccaaca gcctgatcgt 14940

ttccgtgtcc accatggcag ataccgagtg ctgaacgcca gtgccatccc ggagggccag 15000

ttcattgaca gcaagaaagc ttgcgagaag ctcttggctt ccattgacat cgaccacaca 15060

cagtacaaat ttggacacac caaggtgatg tatcagttcc gcctcgtgct gtaggccttc 15120

gcatcctctt aacacaatga gccgggcagc tcattcctct cacccatcct tttgaaggtg 15180

ttctttaagg ctggcttgct gggaaccctg gaggagatgc gggatgagcg tctggccaaa 15240

ctgatcactc ggacacaagc cgtgtgcaga gggttcctca tgcgtgtgga attccagaag 15300

atgatgcaga ggaggttggg cggggtcttg gtttgagcat gagctctttg agagagggtg 15360

gaagtgggag gagagaatcc cttgtaggaa tgggcaatgc tctgtctgat gagacgtggt 15420

ttctctgggt agcccctatg ctgtcctgga actcactctg tagactaaat tggcttcaca 15480

ctcaagtgat ttacctgcct ctgtctccca agcactagaa ttaaaggcat gcaccaccac 15540

tgccaggctg ctttaaaaaa aatgcctgca ctatctgtct atccatccat ccatccatcc 15600

atccatctat cctatctaat ctatctatcg atctgtctgt ctatccatgc tttttgttta 15660

tgtgagcaag tacgtatgcc aaggcacact tgtagcagtc agaggaccac ttgcaggagt 15720

cagtccttga ctcactatgt ggggcctgag aatcgggcct ggcttcggca ccttttccct 15780

gctgagccgt cttgctttgt ttgcagggaa tccatcttct gcatccagta caacatccgc 15840

gccttcatga acgtcaagca ctggccctgg atgaaactct tcttcaagat caagcccctg 15900

ctgaagagcg ccgagacgga gaaggagatg gccaccatga aggaggagtt ccagaagacc 15960

aaggacgagc tcgccaagtc ggaggcaaag aggaaggagc tggaggagaa gctggtcact 16020

ctggtacaag agaagaacga cctgcagctc caagtacagg cggtatgtcc ttaggactct 16080

tgctttttca aatccgagat tggattacga ttttaggaaa tctggattaa cccgagggcg 16140

gggcatgggt gggcgttggg tttttcttgg agtgcttcat ggaggatttc acaaagaaag 16200

atggcagtgt ttcaaacctt cgctccttaa agggaattgt gatttctttt taaaggagag 16260

tgagaatttg ctggatgccg aggagcgatg cgaccagctg atcaaagcca agttccagct 16320

ggaggccaaa attaaagagg tgaccgaaag agctgaggat gaggaggaga tcaacgccga 16380

gctcacggcc aagaagagga agctggagga cgagtgctcg gagctgaaga aggacatcga 16440

tgaccttgag ctgacactgg ccaaggttga gaaggagaag cacgccacgg agaacaaggt 16500

acacacgttc ttcttgcggt ctctcttcag cttgatgtat tcactgctaa gcaagctcgg 16560

cttcttttag gtaaaaaacc ttaccgaaga actggcaggc ctggatgaaa ctattgcaaa 16620

gctaacacgg gagaagaagg ccctccaaga ggcccaccag cagaccctgg acgacctcca 16680

ggctgaggaa gacaaggtca attctctgag caagctcaag agcaagctgg agcagcaagt 16740

ggatgatgtg agcactgggc atcagtctct ggagtctgga ggcttcaggg cggaagagcc 16800

ctgggcaggg gcggggctgg ggttatctac cggaaatcat ctaatcctaa aggggaaatg 16860

atgcctcttg cagttggaga gctccctcga acaagagaag aagctgcgtg tggacctgga 16920

aaggaacaag aggaagctgg agggagacct gaagcttgcc caagagtcca tattagatct 16980

ggagaacgac aagcaacagc tggacgagag gctcaagaag tacgtgtccc ccaaacacag 17040

cttccgttcc gtgagtggcg gggtgcgggg gatcccgtct tcacgcatcc tctgtgtcct 17100

gccgcaggaa ggactttgaa tatagccagc tgcagagcaa ggtggaagat gagcagacgc 17160

tgagcctcca acttcagaag aaaatcaaag agttacaggt aggagggggc gatcaactct 17220

cgcttgccgg tgtgggtcac tcagaaacct gagcggtgtc tgctaaagtc agagtaggta 17280

cggtaggacc gagactgcct catgggacct cactctgaca ggctgacata gcagtggtgg 17340

ccgtgatgac cacgtccccc aaacttgcaa caggatacat ggaggtggat ggagaaatgt 17400

ccagggtagt tagtgaccat atgggggcag gaaaagattc atttggaaga aaatcagcag 17460

catttgtttg tttgggggaa catccacgag cagattcatc tggtggggac ataacctctc 17520

ctccatcgtc atcgtcgtca tcgttgtcgt cgtcaccatc atcaccacca tcatcatcat 17580

catcatcatc atcatcatca tcatcaaatt ttatatatta acgggcttcc atgtgctggt 17640

tcatcatgtg cttatactat tcactgatcc agccccctct taatcacttc ccccaccttc 17700

ctcaccttta gcaaacaccg tttcatattc agattgacct aaacaaaatt ttcaggtgaa 17760

aacatgcacc gattgttttt ccggtgtcta gcgtcctcca ttttgctgca aatggtaggg 17820

cttcttttta tagacgggtc atacattcgt ggtcgatgac ctctactcag acgccatgtt 17880

agctgttgtg actctgagag tctcttgggc atctggctgc tctctatagg cccgcattga 17940

ggagctggag gaagagatag aggcggagag ggccacccgg gccaagacag agaagcagcg 18000

cagcgactac gcccgcgagc tggaggagct gagtgaacgt ctggaggagg cgggcggtgt 18060

cacctctacc cagattgagc tgaacaagaa gcgtgaggct gagttcttga agctgcgcag 18120

ggacctggag gaggccaccc tgcagcacga ggccaccgtg gccacgctgc ggaagaagca 18180

cgcggacagc gcggcggagc tggcagagca gatcgacaac ctgcagcgcg tcaagcagaa 18240

gctggagaag gagaagagcg agttcaagct ggagatcgac gacctctcca gcagtgtgga 18300

gagtgtgtcc aaatccaagg tgaggggtcg gctgaggaga ccgaaggggc acctgcagtg 18360

ggtcaaccta aggtcacctg cagacctcct tcctctttcc aatctgcagg ccaacctgga 18420

gaagatatgc cgaactctag aggatcagtt aagcgaggcc aggggcaaga acgaggagac 18480

gcagagaagc ctaagtgagc tgaccacaca gaagtcccgg ctgcagacag aagcgggtga 18540

gccaagcccc ggatgctatg cctgggtgcc aagctggagc gccagaaggc tttccgccca 18600

ggggcagcag caccctttgc ggggagagaa tgttttgttt tgcttgcttt attttaaagg 18660

cagggtccca ctctacaacc ctggttgacc tggaattcct tgtgtaaaac aggcttgtct 18720

ggatctgcct gactctgctt ctacaaccct gtttttaaag gcctgcatca ccacacctgg 18780

tgagggaggc tttctaaaaa cacaatttca ggaaaaagtc aatggaagta actcacagaa 18840

gaggctgtcc caaacctcct ggagaacact gagtgtcctc agtgaaaaca ggaagcgagc 18900

agacggtcaa acttccatga gtgatacaag tttgcatctg acatagcctg gatgggacca 18960

ggaggagtct agcctttctg cttcctgccc gccttcccgt ctgacttctg catacacctt 19020

agctgaccca tattcaccca agagtatact tttgaatttc cagagagacg aaatcctaag 19080
tgacacagct ggaatcacca accactgggg aggtgctttg ggagtggaga ggtgccctgg 19140
tgatgttcaa actcataatg gttctctcgg ctttcgctgg cagaggtcac tcctggtttt 19200
gtagggttta cagcagtatt ttattaggct gctatttcct tttcatggac acacccctac 19260
tctcaagatg gggtgagggg tctcaccttt attacttgca ccaccacccc acccccaatt 19320
tggcacagtg gttacagttg gtttttgagt gagtgaatga atgaatgaat gaatgagctg 19380
tttccttcct gacacaggtg agctgagtcg tcaactggaa gaaaaggaaa gcatagtatc 19440
tcagctttcc aggagcaagc aagcctttac ccaacagata gaggagctca agaggcaact 19500
ggaggaagag aacaaggtac cccgctcaga aggatgtttt cttggtctca ttttcaaatg 19560
gcttctgcaa aataacaaca agattacctg acaattcagc cagtgctgag aacagggcac 19620
gtggcacgtg tgctgcacac agtcctaatc catccgggga ccttttgatt ttttttttta 19680
aggccaagaa tgcgctggcc cacgccctgc agtcctcccg ccacgactgt gacctgcttc 19740
gggaacagta cgaggaggag caggaaggca aagccgagct gcagagggcg ctgtccaagg 19800
ccaacagcga ggtggcccaa tggaggacca aatatgagac ggatgccatc cagcgcacgg 19860
aggagctgga ggaggccaag tatgtgtctc taattagaac ccttaaggat ctcatggctt 19920
agcacattgg ctctagagtg gctccgggat gaagctaatg aatgtatctg ttttgttact 19980
ggaggtttcc ccatcaagag agtctttaaa tgtaacattg gctgaaatgt gaaatgtaac 20040
agaatgaact acttcagtct aacgctctcc caactgagct atttcggcgc tgcaacagaa 20100
tgaactacta tagttcattg acatcaactt tgatcctggt tagacctgag aaaggccact 20160
gatcacagac tctgagcaga tgggggattc tcttaaggtt ttttcgcaga ggcatgtgag 20220
ccaggcaatt agtgtctccg gggtggtgag cagcgatgct caagaagatt gaaacttaat 20280
ggcgtcggct ttcttttcat tttcttgaag aaaaaagctt gctcagcgcc ttcaggattc 20340
ggaggagcag gtcgaggctg tgaatgcaaa atgcgcttcc ttggaaaaga ccaagcagag 20400
gctgcaaggg gaggtggagg acttgatggt ggatgtggag agagccaact ctctggctgc 20460
cgctctggac aagaagcaga ggaactttga caaggtgcgg gggtgctccg tgctggctga 20520
gctccacggt gtggcatgtt gtaggactgt aggagacagg gataggctgc ctgctctgcc 20580
cttctttagc atggtggcca taaaaacact cctcgaactt cactagcagt ttgtttctcc 20640
atctgtgaca tgacctggga atgggatgag gcagagaaat cccttccgat gaccgttgta 20700
gcagtcacac agtttccatc gctcatacct gtatctttag gggctgagac ttcaacttag 20760
gcccctccct gtgacagaca agcatgattt gaaccactgc tttaatgaaa tacatccgtt 20820
cagagccttt ttctttttgt tattaggaaa agggtaatta tgtctagcta attttttctt 20880
taagaattca tttgctttct ggatatgaca gtggttactg actgattatc ttgagtaagc 20940
gaatgaaatg aaatctttgc tcgttcagta atctagacca ggccctagct ctgagagtag 21000
ccacttcagg ggatgacatt ctgttagaag ctcggcagag agctccctga cagaatcttt 21060

ccacgctgct gctgcaggtg ttagccgagt ggaagacgaa gtgtgaggag agccaagcag 21120
agctggaggc ggctctcaag gaatcccggt ccttgagcac tgagctcttc aaactgaaaa 21180
acgcctatga agaagccttg gatcaactcg aaactgtgaa gcgagagaat aagaacttag 21240
aacgtaagcc attccgaacc gcctgaggtc gtgggaaacc cagagtgtgc gagggcgggt 21300
gaggctccgt tattcaggct cctccacacc gctcggctga tgcaccggta gaagccgctc 21360
accgttgaca agcagttgtt ctgggtctct atacaatatc ttatctctga gaagcagagg 21420
gttttagagt cccagagagc tattgaaagg catttagtgg gtgcttcgca ttcccggagc 21480
ctgtgtggcg gcgtccaaac gcagtgcaaa cttacttcat tgcagaagag atagcagatc 21540
tcaccgaaca aattgctgag aacggtaaaa gcatccacga gctagagaaa tccagaaagc 21600
agatggagct ggagaaggct gatatccaga tggcgctgga ggaagcagag gtgaggatgg 21660
gtcgggaggc acaagccaga acagagggca acagtgtgtg tctgaggcgt tcacacttca 21720
cgcttctgaa atccagattc taaggctcgc tccttccctc tgactgcgag gctgagtgag 21780
atgatgcccg tgagttatct tacatgtatt ggagagaatc atcacgataa aataggctta 21840
ctcttgaaag tttacaaacc cttagctgag gttagaatag ctgtaaggcg cttcttgccc 21900
ttaagtaacg agaacattgt aaatacgttt cgtgtccaag cgcacgatca taaacgaata 21960
accgtaaaat ggttcgcttc tgcactgaag gtaagttagt ttatttaaag gtggctttga 22020
ctattatggg atttgattta ggactatctc ctcagagctt tgctgaccca atgtcgtgtg 22080
accggctcta ggacagtgca gaaacggttc gtgttttttt gttttgtctt ttaaaggcag 22140
ctctggagca cgaagaagcc aagatcctca ggatccagct tgaactgacg caggtgaaat 22200
cagagatcga tagaaagatc gcagagaagg atgaagagat cgagcagctg aagaggaact 22260
accagaggac agtggagacc atgcagggcg ccctggatgc tgaggtgcgc agcaggaatg 22320
aggcgatccg gctgaagaag aagatggagg gcgacctcaa tgagattgag atccagctga 22380
gccatgccaa ccgccaggcc gcagagacca tcaagcacct ccggagtgtc cagggacagc 22440
tgaaggtgga cggagcttcc gggggtgggg ggacttttcc agtgtccaga tccgaaaaga 22500
ctaggcaagg cttgtggtcc ttggccctca cttcacacac tttctcttct ggccaggaca 22560
cccagctgca cctggatgac gctctccgag gccaggagga cctgaaggag cagctggcga 22620
tcgtggagcg cagagccaac ctgctgcagg ctgaggtgga ggagctgcgg gccactctgg 22680
agcagacgga gagggcccgg aagctggcag agcaggagct gctggactcc aacgagaggg 22740
ttcagctgct gcacacccag gtgagggcag gagcattggg gtacgagggt ccacctcaga 22800
cgctgtgtat gacactccct cctcctcctc agaacaccag cctcatccac accaagaaga 22860
agttggagac tgacctcaca cagctccaga gtgaggtgga ggatgccagc agggacgcaa 22920
ggaacgccga ggagaaggcc aaaaaggcca tcactgatgt gaggcccctg ctcccttcct 22980
actgtgaccg ctgatattgt gtctcattaa agccctgctt tcccccaacc ccacgcatgc 23040
ttggtagaag tccaccctct taggacacac atggtgtctg tgctgacccc agaccactcc 23100

caactcccct cgtattgact gttccccctt gtactgtcca aagccggcag atgggggaag 23160

ggcaggatag gagtatactt tttctcctgc ctcccgagag gtgtttcttt cacttgcatc 23220

tggaaatgag accctctgat ggtgttcaca ttccaccaca tggctccttt cctctctaac 23280

gttcctgggg ctccaggctt tctgcttcag gctgatggcg ccacctactg ccctctacgg 23340

gatatcaccc caatgctgtt ttactttgct gtagtcgttc gttacttaat gagtccctcc 23400

ttgatgggat tctgttgtct caggataatc tccaagcctt aggatctttt gaaactgtgt 23460

gttggtgatg attttttttt taaaagacct cctgggcttt ggacaggtct caaggagagc 23520

aggtttagga gactgctcct cactcagtcc ttgtagagtt tatttaataa aatttatatc 23580

ccaggtgtag cttttgtctt gtttcaacgt gggaagtgtg cttattcacg accgtacccg 23640

ctgtcctttc tgtaaagcat gctctctgtt cactgtggaa ttccggcttc cacagccgca 23700

agatctttga gccttgcctt tcaggccgcc atgatggcgg aggagctaaa gaaggagcag 23760

gacaccagcg cccatctgga gcggatgaag aagaacctgg agcagacggt gaaggacctg 23820

cagcaccgtc tggacgaggc tgagcagctg gcactgaagg gcggcaagaa gcagatccag 23880

aaactggaga cgagggtact tgccgagggg gagactgcct ggccaagcct tgccaacctc 23940

ctctcccctc cccacattca gagcccagcg ctagaataac gtcccaaaca tctctccaga 24000

tcagagagct agagtttgag ctggaagggg aacagaagag gaacacagag tctgtgaagg 24060

gcctgaggaa gtatgagcgg cgtgtcaagg agctcaccta ccaggtaggt gcaaaggtca 24120

cctaagacaa gttttctctc caacgtacaa atactactca aagcttggaa ttggacactt 24180

gtgcacccct ggactggctg ggccctggta caagtgcccc ccccccccat cccttcattc 24240

atttgcagag tgaggaggac aggaagaatg tgctgagatt gcaggatctg gtggataaac 24300

ttcaagtgaa agtcaagtcc tacaagaggc aggctgagga ggctgtaagt cagacgcatg 24360

cgtagcctgt tctgtttccc acggtggggt gggttgggga gtgggggcgg ggtggggtgg 24420

gggagtggga gggaccggag agaggatcat gcagatttca cccgctgagc tctttccttt 24480

aggatgaaca agccaatgtt catctcacca agttccggaa agcccagcat gagctagagg 24540

aggccgagga acgtgcggat attgcagaat cgcaagtcaa taaactgcgg gctaaaaccc 24600

gggacttcac ctctagccgg gtaagctgac ctgggaacgc ccaccgaaat gctactttcg 24660

ccgtcggaga ctctcaagct cagagaaaga atgttcttta cagccattta cctgggtgga 24720

gctctactag aaacaccttt gtaaaaaaaa aaaaaagcat tgtctcttta gcgccctctg 24780

ctggtattag atgggttgtt taggaacatc cgctaaaggt ggccgcagat atctgcaatt 24840

actgtaacca caaatccact gtggctccta caccaatttc tcaggctgtg gagcaatttc 24900

atgtgggcca tcatgcatct tccaaaggcc cccctaattc cttgtgtccc gctgaaatat 24960

acaaacaact ctttgatgct tgcgagaaga ggggtccaga aagtgtctct gttgcctgac 25020

ttgactgaga gcatctgcgt tctctctaga tggtggtcca tgaaagtgag gagtgagcat 25080

gtcctcctgg tgaggggcag aagatatgca gaatgtatgt tttcgtggct cctgaccatc 25140

ctgcttaatt tccacgtaac ccctttccac atgcaataaa atttgccttg tttcaagttg 25200

ggtcttttgt cttgtgcatt tctctcattt cctcagagga gtttattcct cttttggcaa 25260

tttttatgct gggtgctgaa atattagcaa gcagttgtct ttgtagatta aatctgtgtg 25320

tgtgtgtgtg tgtgtgtgtg tgtgtgtgtg tgtgtgtaag ggatggcttt ttgatttaaa 25380

tttgaacttg aaaaattcaa gatacttcca actaagcatt gcttcagtgg ggtgggggag 25440

ggagagttta tgaaagtcat ctattaatat catataagcg agacacgaag gttgggttca 25500

ggaacgccta atttcaaact ggggaggatt cctctcctgc acatttttac tagggaaatg 25560

tatctcaaag ctaataaata atggttgggc tccatacaag atatgaccga atgccacggt 25620

gtctggtact gtcaagagct cagaggacag tgatagagct cttgtctcag atacacaggg 25680

ctctgggttc aactctcagc gctgccaaca aacaaatgaa aaaacaaggc atttaaaaaa 25740

tagggcagga aaggaattc 25759

<210> 557

<211> 3259

<212> DNA

<213> Rattus norvegicus (Norway rat)

<400> 557
cagctccggc gggcagcagg cgctggagcg catcgcagtt cagctcagcg cagcaccatc 60

ggtctgcgga gcggactgag ctagaagcgg agcgctgacg ccggaggcgt gcaatgagga 120

gggcaggtgc tgcctgcagc gccatggacc ggctgcgcct gctgctgctg ctgattctag 180

gggtgtcctc tggaggtgcc aaggagacat gttccacagg cctgtacacc cacagcggag 240

agtgctgcaa agcctgcaac ttgggcgaag gcgtggccca gccctgcgga gccaaccaga 300

ccgtgtgtga accctgcctg gacaatgtta cattctccga tgtggtgagc gccactgagc 360

cgtgcaagcc gtgcaccgag tgcctgggcc tgcagagcat gtccgctccc tgtgtggagg 420

cagacgatgc agtgtgcaga tgtgcctatg gctactacca ggacgaggag actggccact 480

gtgaggcttg cagcgtgtgc gaggtgggct cgggactcgt gttctcctgc caggacaaac 540

agaacacagt gtgtgaagag tgcccagagg gcacatactc agacgaagcc aaccacgtgg 600

acccgtgcct accctgcacg gtgtgcgagg acactgagcg ccagttacgc gagtgcacgc 660

cctgggctga tgctgaatgc gaagagatcc ctggtcgatg gatcccaagg tctacgcccc 720

cggagggctc cgacagcaca gcgcccagca cccaggagcc tgaggttcct ccagagcaag 780

accttgtacc cagtacagtg gcggatatgg tgaccactgt gatgggcagc tcccagcctg 840

tagtgacccg cggcaccacc gacaacctca ttcctgtcta ttgctccatc ttggctgctg 900

tggtcgtggg ccttgtggcc tatattgctt tcaagaggtg gaacagctgc aaacaaaata 960

aacaaggcgc caacagccgc cccgtgaacc agacgccccc accggaggga gagaaactgc 1020

acagcgacag tggcatctct gtggacagcc agagcctgca cgaccagcag acccatacgc 1080
agactgcctc aggccaggcc ctcaagggtg atggcaacct ctacagtagc ctgcccctga 1140
ccaagcgtga ggaggtagag aaactgctca acggggatac ctggcgacat ctggcaggcg 1200
agctgggtta ccagcctgaa catatagact cctttaccca cgaggcctgc ccagtgcgag 1260
ccctgctggc cagctggggt gcccaggaca gtgcaacgct tgatgccctt ttagccgccc 1320
tgcgacgcat ccagagagct gacattgtgg agagtctatg cagcgagtcc actgccacat 1380
ccccagtgtg aactcacaga ctgggagccc ctgtcctgtc ccacattccg acgactgatg 1440
ttctagccag cccccacaga gctgccccct ctccctcggg gatggcccaa cggtcagaac 1500
ggagcatctc tgtgcagggc ctctgtgttc ccactcctga ctccgttgct gctcccgagg 1560
gggcccttgc ttctgaccac cctctcctca gcaagagaga gagagaggac cacccgagcc 1620
tgacttgctc catttccatc tcaggccttt ccttcctttc tacacattag ctgtgtcaga 1680
tctgggggtt tgacactagg agaagggagc gggggcaccc ctaagactca ggaggtactg 1740
aagaaccaga gccatggact ccacactgtg aaccggagaa caaggggcgg ggcattgtgg 1800
taggctagac cttccttagc ccctcccttc tcccctctgg ccaaagaaga ggattacgga 1860
cctatctgag ctgaaagcag gtttggaacc cagcccacac ttctctctca cacacaggat 1920
ggtaaaaccc agagaaaggc agggactgac ctaggccacc caaccacagg aagaacaaat 1980
gaaggctgat acactccgtt tctgaatgag ggcgtcaagt gtgcttgttg acagggatgg 2040
cgtgactttc agggaaatat ctggaagcca tgtctgcccc gccctcaacc acttccaggc 2100
ccctacccaa cccttgtgca gatgaactgt ttgttcaagg gctggtccat tggtctattc 2160
tgatggagtc aagctaaggg ctcaggctta tccataaggc atttgtggag agatgaatct 2220
gttagtgcgc tcattcttgg cataagcctg aagccaacac ggcccttaat gtcagccctc 2280
ggggtcagga accaaggact cccaccccac aatccaacac tatactacat tacacacaca 2340
cacacacaca cacacacaca cacacacaca cacacacaga tatcttgctt ttctccccat 2400
ggctcttttg gggctgagac tagatcctgc tgggagtcac tgccagtgag agatccggag 2460
gggacagagc tgagcttcat ggggctgtct tcctcgcccc cgggtctggc aggccaagaa 2520
tgactgcatc tgagctggtg tctgtcttcc aatggcctgt gcgtggagga aatgctccca 2580
ctcctcccct tcttgaagct gcccccagaa gactacagtg caaaagagca gactggtgtg 2640
agaacacaag aaaaagcaga tgctggccct gcagtctgtg gcagctttct cctcagcttc 2700
aaggcccctg caaaggacgg atttcctgag cacggccagg aaggggcaag agggttcggt 2760
tcagtggcgc tttctcccgg ctccttggcc tgttctgttt tgcttgctgt tggaatgagt 2820
gggcaccccc tctatttagc atgaaggagc cccaggcagg gtatgcacag actgaccacc 2880
atccctcccc acccagggtc cacccaaccc ggtgaagaga ccaggagcat tgtacgcata 2940
cgcgggtggt atttttatgg accccaatct gcaattccca gacacctggg aagtgggaca 3000
ttctttgtgt atttattttc ctccccagga gctggggagt ggtggggggc tgcaggtacg 3060

gtttagcatg tgtttggttc tgggggtctc tccagccttg ttttgggcca agttggaacc 3120

tctggccctc cagctggtga ctatgaactc cagacccctt cgtgctcccc gacgccttcc 3180

ccttgcatcc tgtgtaacca tttcgttggg ccctcccaaa acctacacat aaaacataca 3240

ggaggaccat taaattggc 3259

<210> 558

<211> 1121

<212> DNA

<213> Rattus norvegicus (Norway rat)

<400> 558
aagatgaact ctcaatccta aatatctatg ccccaaatac aagggcacct acatacgtaa 60

aagaaacctt actaaagctc aaagcacaca ttgcacctca cacaataata atgggagatt 120

tcaacacacc actctcatca atggacagat catggaaaca gatattaagc aatgatgtcg 180

acagactaag agaagccatg agccaaatgg gcttaacgga tatttataga acattctatc 240

ctaaagcaaa aggatatacc ttcttctcag ctcctcatgg tactttctcc aaaattgacc 300

atataattgg tcaaaaaacg ggcctcaaca ggtacagaaa gatagaaata atcccatgcg 360

tgctatcgga ccaccacggc ctaaaactgg tcttcaataa caataaggga agaatgccca 420

catagacgtg gaaattgaac aatgctatac tcaatgataa cctggtcaag gaagaaataa 480

agaaagaaat taaaaacttt ttagaattta atgaaaatga agatacaaca tacccaaact 540

tatgggacac aatgaaagct gtgctaagag gaaaactcat agcgctgagt gcctgcagaa 600

agaaacagga aagagcatat gtcagcagct tgacagcaca cctaaaagct ctagaacaaa 660

aagaagcaaa tacacccagg aggagtagaa ggcaggaaat aatcaaactc agagctgaaa 720

taaaccaagt agaaacaaaa agtaccatag aaagaatcaa cagaaccaaa agttggttct 780

ttgagaaaat caacaagata gataaaccct tagccacact aacgagagga cacagagagt 840

gcgtccaaat taacaaaatc agaaatgaaa agggagacat aactacagat tcagaggaaa 900

ttcaaaaaat catcagatct tactataaaa acctatattc aaaaaaactt gaaaattttc 960

aggaaatgga caatttccta gacagatacc aggtattgaa gttaaatcag gaacagataa 1020

accagttaaa caaccccata actcctaagg aaatagaagc agtcattaaa ggtctcccaa 1080

ccaaaaagag cccaggtcca gacgggttta gtgcagaatt c 1121

<210> 559

<211> 696

<212> DNA

<213> Rattus norvegicus (Norway rat)

<400>  559
ctctttccag ccagcgccga gcgatgggca tctctcggga caactggcac aagcgccgca      60

agaccggggg taagagaaaa ccctaccaca agaagcggaa gtatgagctg ggacggccgg     120

ccgccaacac taagattggc cctcgccgca tacatacagt ccgagttcga ggaggcaata     180

agaagtatcg tgctctgaga ttggatgtgg ggaacttttc ctggggctca gagtgttgta     240

ctcgcaaaac aaggatcatt gatgttgtct acaatgcatc caataacgag cttgtccgca     300

ccaagaccct ggtgaagaac tgcattgtgc ttattgacag cacaccgtac cgacagtggt     360

acgagtccca ctatgcactg cccctgggcc gcaagaaggg ggccaagctg actcctgagg     420

aggaagagat tttaaacaaa aaacgatcaa agaaaattca gaagaaatat gatgaaagga     480

aaaagaatgc caaaatcagc agtcttctgg aggagcagtt ccagcagggc aagcttctcg     540

cctgtattgc ctcaagacca ggccagtgtg gcagagcaga tggctatgtg ctcgaaggca     600

aggagctgga gttctatctg cggaagatca aagcccggaa aggcaaataa actgtcatag     660

ctcgtgtaat aaaggtgttt gctgttctgt atatgt                               696

<210>  560

<211>  1361

<212>  DNA

<213>  Rattus rattus (black rat)


<400>  560
ccagccagtc gccatcagga acctcgagaa gctgctccag ctatgtgtga agaggaagac      60

agcacagctc tggtgtgtga caatggctcc gggctctgta aggcgggctt tgctggtgat     120

gatgctccca gggctgtttt cccatccatc gtgggacgtc ccagacacca gggagtgatg     180

gttggaatgg gccaaaagga cagctatgtg ggggacgaag cgcagagcaa gagagggatc     240

ctgaccctga agtatccgat agaacacggc atcatcacca actgggacga catggaaaag     300

atctggcacc actccttcta taacgagctt cgcgtggccc cagaggagca tccgaccttg     360

ctaacggagg cgccgctgaa ccctaaggcc aaccgggaga aaatgaccca gattatgttt     420

gagaccttca atgtccctgc catgtatgtg gctattcagg ctgtgctgtc cctctatgct     480

tctggacgta caactggtat tgtgctggac tctggagatg gcgtgactca caacgtgcct     540

atctatgagg gctatgctct gcctcatgcc atcatgcgtc tggacttggc tggccgagat     600

ctcaccgact acctcatgaa gatcctgact gagcgtggct attccttcgt gactactgct     660

gagcgtgaga tcgtccgtga catcaaggag aagctgtgct atgtcgctct ggactttgaa     720

aatgagatgg ccactgctgc ttcctcttct tccctggaga agagctacga actgcctgat     780

gggcaggtga tcaccatcgg gaatgaacgc ttccgctgcc cggagaccct cttccagcca     840

tctttcattg gaatggagtc ggcgggcatc cacgaaacca cctataacag catcatgaag     900

tgtgatattg acatcaggaa ggacctctat gctaacaacg tcctctcggg gggcactacc 960

atgtacccag gcattgctga caggatgcag aaggagatca cagccctcgc tcccagcacc 1020

atgaagatca agattattgc tcctccagaa cgcaaatatt ctgtctggat cggcggctcc 1080

atcctggctt ctctatctac cttccagcag atgtggatca gcaaacagga gtatgacgaa 1140

gctggcccct ccattgtcca ccgcaaatgc ttctaagtca cgcccccacc ctccgccccg 1200

cccccacccc cgccccgccc ccgctctgtc tctagcacac aactgtgaat gttttgtgga 1260

tcagcgcctt cagttctttt ccaaatcatt cctacctaaa gctttgattt gttactcgtg 1320

tgtttttttt taataaatca gacatgtgct acccttctaa a 1361

<210> 561

<211> 2134

<212> DNA

<213> Rattus rattus (black rat)

<400> 561
cgctgcacca tgagcgtctc tgcactgagc tccacccgct tcacgggcag catctctggc 60

ttcctccaag tggcctccgt gcttggtctg cttctgctgc tggtcaaagc agtccagttc 120

tacctgcaaa ggcaatggct actcaaggct ttccagcagt tcccatcacc tcccttccac 180

tggttctttg ggcacaagca gtttcaaggt gacaaagaac tacagcaaat tatgacatgt 240

gtggagaatt tcccaagtgc ctttcctcga tggttctggg gaagcaaagc ctacttaatt 300

gtctatgacc ctgactacat gaaggtgatt ctcgggcgat cagatccaaa ggccaatggc 360

gtctacagat tgctagctcc ttggatcgga tatggtttgc tcttgctgaa tggacaaccg 420

tggttccagc accggcgaat gctaacccca gccttccact atgacattct gaaaccctat 480

gtaaaaaaca tggctgactc cattcgactg atgctagaca aatgggaaca gctggcaggt 540

caagactcct ctatagaaat ctttcaacat atctccttaa tgaccctaga cactgtcatg 600

aagtgtgcct tcagccacaa tggcagtgtt caggtggatg gaaattacaa gagctatatc 660

caggccattg ggaacttgaa tgacctcttt cactcccgtg tgaggaacat ctttcatcag 720

aatgatacca tctataattt ttcttccaat ggccacttgt tcaaccgtgc ttgtcaactt 780

gcccatgatc acacagatgg tgtgatcaag ctaaggaagg atcagctgca gaatgcggga 840

gagctggaaa aggtcaagaa gaaaagacgt ttggattttc tggacatcct cttacttgcc 900

agaatggaga atggggacag cttgtctgac aaggacctac gtgctgaggt ggacacattt 960

atgttcgagg gtcatgacac cacagccagt ggagtctcct ggatcttcta tgctctggcc 1020

acacacccta agcaccaaca aagatgcaga gaggaagttc agagtgtcct gggggatggg 1080

tcctccatta cctgggatca cctggaccag attccctaca ccaccatgtg tatcaaggag 1140

gccctgaggc tttacccacc tgttccaggc attgtcagag aactcagcac atctgtcacc 1200

ttccctgatg ggcgctcttt acccaagggt atccaagtca cactctccat ttatggtctc 1260

caccacaacc cgaaggtgtg gccaaaccca gaggtgtttg acccttccag gtttgcacca 1320

gactctcccc gacacagcca ctcattcctg cccttctcag gaggagcgag gaactgcatt 1380

gggaaacaat ttgctatgag tgagatgaag gtgattgtgg ccctgaccct gctccgcttt 1440

gagctactgc cagatcccac caaggtcccc atccccttac cacgacttgt gctgaagtcc 1500

aaaaatggga tctacctgta tctcaagaag ctccactaat tccggacttc ttcattatac 1560

caataatcaa cggtcacaac cttgatggtc gggaataaca ggacgttatt atcttacagt 1620

tgtggagctc cgaaatctga aatgagtttc actggcagaa agctgagttg gtggtgtgac 1680

tagccttctt cagaagagtg cttcagagag tcctctcctc ctctcttcag tacagatcac 1740

ccttctcagc actggaatat tcctctgctt taaagccagc acccttccca taccccctct 1800

tctaaaagcc ttccctttta caaatgttct tatgacatca tcaagaccac tgaaaaactc 1860

caagataatt tcccatctca atattcctta ctccatctaa cctactaagt cccttttgaa 1920

ttatgaggaa taattcaatt tgttccatgg gctccaaaac tcaaggcctg agcattattg 1980

tgaaaccttt attcagccta atatcatctt cacaagactg ttacctggta cgttcatcta 2040

aatctcccct gcatagtctc tctacctgac tattcctcac acaagtttct ttaccttccc 2100

tcctttctcc aataaagtgt ccagtgtcct gcac 2134

<210> 562

<211> 2481

<212> DNA

<213> Rattus norvegicus (Norway rat)

<400> 562
gatctattag acacaaaacc taaccagaga aggaaatatg cggacctgaa cctttgcaga 60

caaagcttcc tccctctgct cagttgtaag ctacttggtc tgagccgtct gtgtaccaaa 120

gagtggacaa ggcagtcagg atagcagaaa tcaaacacat ggcatcccac cattgtgcgg 180

cgcgtgtcac agagggaaag acaggagtgt aacccacact gagagtggta gggagacgcg 240

ggtggtggaa atgcatcaaa aacaatgatg ttcacggtac tcgtcagagc attttctacc 300

cgtttgtttt aaaacattct tttcctcttc ctgttttcca ctttcccacc atccacctaa 360

attcctcagc cgaccacgta ggctcctatg gtataactgt gtatcagtat catgaatcca 420

aaggccagta cacacatgaa tttgatggtg acgagagatt ctatgtggac ttggataaga 480

aggagaccat ctggaggatc cccgagtttg gacaactgat aagctttgac ccccaaggtg 540

cacttcgaaa tatagctata ataaaacaca atttggaaat cttgatgaag aggtcaaatt 600

caaccccagc tgtcaacggt acgtgctcac caccctgctc ccccctccct tccctggggg 660

gttaggggat gtgatcagga cattaatggt ccaataaaat cctctccttt ccaaggagac 720

cccggatctc ttcatcactg gagactcatc ccctctcttc ttgtttttaa agatttattt 780

atttattata tctaagtaca cggtagctgt cttcggacac accagagaga gggcatcaga 840

tcccattaca gatggttgtg agccaccatg tggttgctgg gaattgaact caggacctct 900

ggaagagcag tcagtgctct taaccactga gccatctctc cggcccatcc cttctcttct 960

taaaaatcac acatttcatg taatatacag ctcactattc cacaatgctc tcctcgaatc 1020

ttttggaaaa gccccgacag acatcctacc tgaactccct agacgagagg ggcacaggga 1080

tcaagtggag ggcacattag ctccaagcag aggaatcggg ctctttgact ttcatatgtg 1140

taagacttgt caagaaggct ctctcactag acagtgcagg gcttcaggga gagaagctcc 1200

catcagcctc tgacatccag accatttcct ttgcagaggt tcctgaggcg accgtgtttt 1260

ccaagtcccc tgtgctgctg ggtcagccca acaccctcat ctgctttgta gacaacatct 1320

ttcctcctgt gatcaatatc acatggttga gaaacagcaa gccactcaca gaaggcgttt 1380

atgagaccag cttccttatc aacagtgact attccttcca caagatggct tacctcacct 1440

tcatcccttc caacgacgac atttatgact gcaaggtgga gcactggagc ctggacgagc 1500

cggttctaag acactggggt gtgtatgagc tctgccactt ctggcacttt ctcgttcact 1560

gtcacttctg gaacagcatg gcttctggct tcaagtgacc aaaactcact ttccactctt 1620

aaagtttcta aggctagagt tcgtgtcatc ccattagccc agcctcagag accagtaggt 1680

taactcctcc tcacctggtc ctgtcttaca catacatgag tcccttctga ctcaaagctt 1740

ctctctctca gaacctgaga ttccagcccc catgtcagag ctgacagaga ctgtggtctg 1800

tgccctgggg ttgtctgtgg gtctcgtggg catcgtggtg ggcaccatct tcatcattca 1860

aggcctgcga tcagtggctc cctccagaca cccagggccc ttgtgagtca caccctggga 1920

aagaaggtaa gggtttgtgt ttgtgggaaa caaaggcacg catggggagg ggaaggaagg 1980

ggagacatga aagtttgttg ttggaacttt gctggagcct ctgaacccat ctttcctgtg 2040

tgttttgttg taggtgcgtg gccctctaca gggaagatgt agtgtgtggg ggtgacctgg 2100

cacagtgtgt tttctggccc aattcatcgt gttctctctc ttctcctggt gtctcccatc 2160

ttgctcttcc cttggccccc aggctgtcca cctcatggct ctcacgccct tggaattctc 2220

ccctgacctg agtttcattt ttggcatctt ccaagtcgaa tctactatag attccgagac 2280

cctgattaat gctccaccaa accaataaac ctcttataag ttgtcttctt tgtttttctg 2340

tctcccattt tggagcttac tggagccagg cccatgtaat cctgtgtcca gtgtcacagg 2400

aataaccaat gcagcccaga atggtttggt atcttagcca aggttgactt tcaaattttt 2460

ttgttcagct gacatctcac g 2481

<210> 563

<211> 1857

<212> DNA

<213>  Rattus norvegicus (Norway rat)

<400>  563
aagcttaaaa ctttaaaatc gtatgttctt aagttaccaa atattttcta ttacttcatc      60

tctcttgtct tgatcacgca gcaaacttgt tttggcacaa attaccacat gaattctaga     120

gcccagtttc atatgctatt cacagaatga tgtcatggta atgatgccag ccacagacag     180

tgggcttcca acctcaataa attgtacttt tcttatgttg tctttttttg taggaaagac     240

attaaaccct tataatccaa aagatcagaa gcagggagct catagccaat ctaagtgcta     300

cctgcaattc tatcaattag aaatgggacc aatctaaggt cactccaggg catctccagg     360

gaaagggtac atgtttaatg tcaccaagct accagcgagt agttcctggg tgcagattcc     420

tttagtcatg cacagtagat taattccaag aatgacagtc aagctcctgg gcagcacccc     480

gtgagcaaac ttgagccttg ggaccacagg taagaccaac ttttctgctg caagagacct     540

gcctggtgat ctcgggacat acagaggcag aattcctcta ggaccgggca cctcctgtgt     600

ttaccgggag tcccacaccc gcggatcccg gcccgcagca gctctttgct cccagacacc     660

gtgggagaga gaccccacca cctggtcagg tgggcactcc tgaggctgca gagcggaata     720

gaccaccaac actgcccacc cctccctaca tccctggccc aagaggaaac tgtataaggc     780

ctctgggttc ccgtggggga gggcccagga gtggcagtac ctcttcctct gagacaccgc     840

cggaacctga aggaaacaga ccggataaac agttctctgc acccaaatcc cgtgggaggg     900

agagctaaac cctcagagag gcagacatgc ctgggaaccc agaagagact gcagtctgca     960

cacacatctc ggatgccaga ggaaaacacc aaacgccatc tggaaccctg gtgcactgaa    1020

gttcctggaa acagcggcgc agatcttcct ggttgctgca gccgcagaga gctcgagggc    1080

agcaccccga gagcgaactt gagcctcggg accacaggga agaccaactt ttctgctgca    1140

agtgacctgt ctggtgaact caagacagag gcccacagga acagctgaag acctgtagag    1200

aggaaaaact acacgcccga aagcagaaca ctctgtcgcc ataacaggct gaaagaaaac    1260

aggaaaacag gtctacagca ctcctgacac acaggcttat agggcagtct agccactgtc    1320

agaaatagca gaacaaagta acactagaga taatctgatg gcaagaggca agcgcaggaa    1380

cccaagcaac agaaaccaag actacatggc accatcggag cccaattctc ccaccaaaac    1440

aaacatggaa tatccaaaca caccagaaaa gcaagatcta gtttcaaaat catatttgat    1500

catgatgctg gaggacttca agaaagacgt gaagaactcc cttagagaac aagtagaaga    1560

ctacagagag gaatcacaaa aatgcctgaa agaattccag gaaaacataa ttaaacaagt    1620

agaagcccat agagaggagt cacaaaaatc cctgaaagaa ttccaggaaa tcataaataa    1680

acaagtagaa gcccatagag aggagtcaca aaaatccctg aaagaattcc aggaaaacac    1740

aatcaaacag ttgaaggaat taaaaatgga aatagaagca atcaagaaag aacacatgga    1800

aacaaccctg gatatagaaa accaaaagaa gagacaagga gctgtagata caagctt       1857

<210>  564

<211>  7776

<212>  DNA

<213>  Rattus norvegicus (Norway rat)

<400>  564
ggatcccggc atccgggctt ggaaaggggt tttagttgag aattaacaaa ccattaacag     60

cgtgtacata gtaacaagtt ggagttactc ctaagtcttc ccaatactga atatgtaagt    120

aaccagtcag tcccacttat tctttaacaa tgtaactttg gtgggggctg gggatttagc    180

tcagtggtag agcgcttacc taggaagcgc aaggccctgg gttcggtccc cagctccaaa    240

aaaaaaaaaa accaaaaaaa aaaaaacaaa aaaacaatgt aactttggtg gatacaaatt    300

cactgcccag actcttttcc ggtccagact tctcaaagtg acttagttgt agctaccctg    360

aaagatttaa cagaataagg gacagctgtt ggcttgaaac ccctcaacct cttccctaac    420

atgcgactct tgcttagata aaaggcatta tcttagcatg caactggtgc ctagaatcta    480

gttggaagct accaacgtaa aacggagtgg ctctccagcc tctgctggct tgtctctcag    540

caagttttca ggcataggca cacagggtgg tgaagcaggg tcaatctaca ccacgaagtc    600

tagtctacgt ggcccgcaca caactcaagg cccatccatc ctcggctcgc acctgcgatc    660

ccgccaggga ataaatacta cgcgctgaat gcaaagccga ggaagagttt tgctagtctt    720

ccaggccagg gctcagccgt taggacccgg ggagagactg cgtgattgaa ccggttgcaa    780

gacggacgac caagcgcctg tgcgcatgca ccggccgaat cccgcctgct gcccatagga    840

ccctgcggca agctggtagt caggactggt ggtgtggtgc gcgtgcgcgg gcctgccgca    900

gggagtgtcc ggcacgaggc cgggcgggga gcgcgggcgg atgggcgggg ggctgggcac    960

gcgcggcgcc ccgccccact gacgcgccgg gcccgcgcct cccgcctccg gcgtcgggaa   1020

ccgggttggc cgccaaggag tccccgcccc tcccccgcgc ctcccggccg gaaccgatcg   1080

gggctggttt gagctggtgc gtctccatga cgacgtgctc cgcctataag tagcggcgcg   1140

tcgcaccgtc gggctttgtc agtccctgca gccgccgccg ccggccgcct tcagtcagca   1200

gctcggcgcc acctccggtc ggcgactgcg gcgggctcga cgaggcggct gacggggcgg   1260

cggcgggaag acggccgggt gcgccttggg gtttagtggc ggcttctcca tgggtccagc   1320

cagccgcttc cctgtgctgt gaggtaagcg gggcgcggga ggattgtgcg gccacgtgtc   1380

gcgaggcccg gactgcggtc gccggcaggg gacacgtggc cggaggctgg tgaccttgcg   1440

agcctccgcc ctgaaggcag cagctgttcc tggcccttgg ctgcaagccc tcggatcgcc   1500

ccatgggagc ccgcatcgtg tcagccgggc tcggcgctcc acttgcccgg gcggtgatgg   1560

agggcaggtg caggggagcc cggccgctag cagccgccct gcagtttcct gctttcttta   1620

gttttgctac gtgtacagag cagagctgat gactcgaagt ggaagtcgat agacctaatt   1680

tagtcaggga gtccctgcta atgaatactc accggctgtg acttggattt agaactgaac   1740

tctttgaatg acttaggggt ttacgggaaa cggattcccg gctgtcttgg aagcccgact 1800
cagaatcctc aataactggt agaactgggg agcagatcct gtcaggaatg tctgggagag 1860
gacaaaagaa tgcagtggct tgttttattc aaacctaaca tcgggcagaa tttgactgga 1920
gttcattttt gttttaattg gcttgtatct catccagatt taacttcttg tgctaattgt 1980
ttaacaggat ttagctgctt cccatgccaa agtgttagca ttttttttct tttatctttt 2040
ttttcttttt cttttttttt tttttttttt ttgtattgag gcaaagatag ggaaaggcaa 2100
aataagtggt atttcaatga aagttttcca ggtttaagcc ggaaggctgc ctggaaaata 2160
cttaacaaga cctaaaatta caaagttact gccaaagccc attcatcagt tctaagtgat 2220
atacaactga gttggctggc gctggtctgt agtcttcact taagagaggt ttatgcagct 2280
aacgaagttg accagtgcca aaagtctcct taaagcttaa agtgtgcttt atcatgcatg 2340
gatttggatt tatgacaggc tcatccccgt ggcctctcat agtgtcccgt gctagagcaa 2400
actggctcct aaccattgcc cagccttggt gcatgtaggc tgctggcact gaagtgggtc 2460
acacaatgga aaaaagtttt acctggccac ttgctgctgg tgtccatttc ataagaccca 2520
ggcaggagct tttattaagc gtggtgatct tgcttatttt tgttagtaaa aaagttggcc 2580
aaaaatttct tccatatggt tattatccat aagtggatat tagaaggcac ttgaggactg 2640
ttggattccc agaagtgcgc atttggtagg caatccaggc tctagaatac tgatgtttca 2700
taacagtaac aatatactgt gaattgaatt tttttaaatg ctagtactgc ataaaagtta 2760
gggaagtttt ggtggattgc acactaacat tgagtgctct ggatacctga tatactgtat 2820
tcactaagtc attcagcata actctgcaag tgggagaaaa agaccttttc aggactgcat 2880
gttactgtat gcccattaaa gtttttgaag agctggaatg gctcaaatga agcagatagg 2940
aacatgtaat aatgccagta attagactgt taactccgtt ccgttttgga gaggggcagg 3000
gattgagatg ttctatgtgt atagagctgt ggcattcagt tttgaacagt gtggaatcta 3060
tttgagtatt ggagatattt gggggggggg tcttcacgta gttctgacct cacagttgat 3120
cttcctgtac tgtgtactct ctggtatact gttgcagtgt gtaaccttta atggtaagat 3180
ttcaagggag atagcaacat gttgtgtttc tactcactag tgtttccacc actccaggag 3240
acagcattca gagttgacct tgtgagagct ggccataatt taattccatc tctaggtttt 3300
ctgtgtaagt atcagtttat acttgtgaat ttgtgttgaa gcagtgctaa gactttgagt 3360
gcattcactt attatctgct tattgtcagc ttattgtttc agaggcacat cgagaaccaa 3420
ccatgggcag ctttactaag gaagagtttg actgccatat cctcgatgaa ggtttcactg 3480
ctaaggacat tctggaccaa aaaatcaatg aagtttcttc ctctgtaagt acgggaagcc 3540
cacagccaca actcaaaagt tccataaaag agcatgcctt gccagagaat ctagaatttg 3600
actgactggg ctgtctagtg agtgtccatg tcagtactgc taacagggat aggattttgt 3660
tagggaaggg ggaaaaagat cttcagtgtg gccaggtgca agactgttaa tgaggtccct 3720
gaacccactc actctcattc caggatgata aggatgcttt ctatgttgcg gacctcggag 3780

acgttctaaa gaagcatctg aggtggctga aagctcttcc ccgtgttact cccttctatg 3840
ctgtcaagtg taatgacagc agagccatag tgagcaccct ggctgccatt gggacaggat 3900
ttgattgtgc aagcaaggta agactgctta tctcacccca aaagggttat cagttgatgt 3960
gcttttgttt gtcaaactcc gtctatatat actgatcttc tctttcagac tgaaatacag 4020
ttggtgcagg ggcttggggt gcctccagag aggattatct atgcaaatcc ttgtaagcaa 4080
gtgtctcaga tcaagtatgc tgccagtaat ggagtccaga tgatgacttt tgacagtgaa 4140
attgagttga tgaaagttgc cagagcacat ccaaaggcaa agtaagtctt ctgattgagc 4200
acaaaaaggc tgggcttgct aggcagacta taatcttggt tccttattcc tatacatagt 4260
aagaaccagg ctaaaccctg ggttagaatc agacccacaa gcagtagtgc ctagacaggc 4320
tgattggatg aacgtgtaca gagtgcttac ttggtgcatg cacttgtttc tgtccaccta 4380
ggttggtttt gcggattgcc actgatgatt ccaaagcagt ttgtcggctc agtgttaagt 4440
ttggtgccac actgaaaacc agcaggcttc tcttggaacg ggcaaaagag ctaaatattg 4500
atgtcattgg tgtcaggtga ggtctcagtg atagttacag actgagacat taaattttaa 4560
ggcccttttt tcctctgaga actagtcaaa gaacagcatc ttgtttttat ttcagcttcc 4620
atgtgggcag tgggtgtact gaccctgaga ccttcgtgca ggcagtgtca gatgcccgct 4680
gtgtctttga catgggagta agtacatgac tcctggagga gggctgtctt gattgaaata 4740
accaaaccac tgaatctaga atttgcctct tgtagaagct gtctgcataa cagattttaa 4800
aaccatctgt cctgtgcatt taaacttgtg aatctagcaa tttgacttcc aaattttctt 4860
gattctagac agaagttggt ttcagcatgt atctgcttga cattggtggt ggctttcctg 4920
ggtctgaaga cacgaagctt aaatttgagg aggtaattat tacaacatta acctacagac 4980
ggtgagatat gtatttacca ttgtggtgtt tttactgatt aaaggagcca acatgcctgg 5040
tgttttgttc tcactacttg ttatttgcaa ctgtagccca ggctggcctt gaactttcag 5100
ttctcctgcc tgtttcccat attttaatgt ttagctcagt gcttatcact attgtactat 5160
gaagtagaca gggtggaagg agttatatct tgttggcatg tattcagaat tcagcatttg 5220
gcccctcttg taatccattt tgtgctattt tattaaatat taatataaac tattcataat 5280
gtgttagaaa gagatggtcc tgaggggagt gggaagtggt gtatgtacag actagtttcc 5340
agagaggttt gttgcttctg ttgcctgatg acagacctgc tatgctaggt catgtcctgt 5400
tcttacacac agcagtgtga tctttctcct caactataac aaatggctgc tcccttctct 5460
cacttagatc accagtgtaa tcaacccagc tctggacaag tacttcccat cggactctgg 5520
agtgagaatc atagctgagc caggcagata ctacgtcgca tcagctttca cacttgcagt 5580
gaatatcatt gccaaaaaaa ccgtgtggaa ggagcagacc ggctcggacg gtatgtggtg 5640
gcaggatgag tgagtcatgt gggataacag ttgatatgct gggtggtaat tagggtgatc 5700
tgtttttcta gatgaagatg agtcaaacga gcaaactttg atgtattacg tgaatgatgg 5760
agtgtatggg tcatttaact gcattcttta tgaccatgca catgtgaagg ccctgctgca 5820

gaaggtgagt tctgagcgca tgctctagca gtgagtgatg cataggatag agagaacatt 5880

gggccgagag atggctcagc agttaagagc actgactgct tccagaggcc ctgagttcaa 5940

tcccagcaac aacatggtgg ctcaccacca tctgtatgta atgatgccct cttctggtgt 6000

gtctgaagaa tagctacagt gtatttataa tctttaaaaa aaaacaaaca aaaaacaaac 6060

attgtcttta tacagagacc caagccagat gagaagtatt actcatccag catctgggga 6120

ccaacatgtg atggccttga tcggatcgtc gagcgctgta gcctgcctga aatgcatgtg 6180

ggtgattgga tgctgtttga gaacatgggt gcatacactg ttgctgctgc ttctactttc 6240

aatgggttcc agaggccaaa catctactac gtaatgtcac ggtcaatgtg gtgagtgggg 6300

ctgtgcctgc tcatgctctt ttggttgctc agtgtaatgt ttgcctactg tgagccagaa 6360

gcctgggtta tgcctgatgc acacacacac acatactatg gggggaaggc agtatgtgct 6420

gaggggaagg atcacctgac ctgtctcaaa gggactgaat ttgtgcttgc tttttatgtt 6480

tgataagttt tttttttacc ttgagctaaa tacagaaatg gagtttttcc aagtaattac 6540

tgctcatctt cagatattta ctactaggag aggtcttaca agtaagccat tagattcttt 6600

tgcaactaaa atattcaagt tagcatatgt gacctgtggg ttccttaata atgaaatttt 6660

acattggatt gtttcctggt atagtgacag ggtgaggtgt caggagatct cttgggaggc 6720

tgcccaattt ggagggattt gggttttgaa tatgccatat atgcctttgt ttttcaggca 6780

actcatgaag caaatccaga gccatggctt cccgccagaa gtggaggagc aggatgttgg 6840

cactctgccc atgtcttgtg cccaggagag cgggatggac cgtcaccctg cagcctgtgc 6900

ttctgctagt atcaatgtat agatgccatt cttgtagctc ttacctgcaa gtttagcttg 6960

agttcacggc atttgggggg accatttaac ttaattactg ctagtttgga atgtctttgt 7020

aagagtaggg ttggcaccaa tgcagtatgg aaagactagg agatgggggt cacacttact 7080

gtgttcctat ggaaactttg aatattttat atggattttt attcactttt cagacctgat 7140

actaatgagt gcccctcggc tgctgagcaa gcatttgtag cttgtacatt ggcagaatgg 7200

gctaaaagct tatgttgtga cccattttga aaataaagta tcttgaaatg attggacatt 7260

ggagaatgtg tgcaagtatc ccttacagaa ggcacaaact tctgcacagg ctgtgtgtta 7320

cagcagtgag tctagcccag cagagatgtg gatgatacaa agctgtgccc cctctgtaca 7380

gcatcaatgt gcttagccca tctcaagtgt ttactgtgaa cttggtgccc aaagtctctt 7440

aagagtgtca tctgcctagt ggcctcttga cttggccact tcctaaggag agggcatctg 7500

aggctctttg aaccttgcct gcagaaaccc tgactgctcc ctcaaccctt ggcctcagct 7560

gcatcaccac ctagtaacag ttgaagtatc gaacagcact tggcagtcaa cttcctgtaa 7620

taaattcaac aacagcaact actgtgttgt aaatttttac cttgaccttt catattatag 7680

tcctgctcag tttatacacc tattgcatcc ccaaagaaat gccagactct taacctagtt 7740

agtattgaag acctgtaggt caggaggaag ggatcc 7776

<210> 565

<211> 4964

<212> DNA

<213> Rattus rattus (black rat)

<400> 565
gaattctgat ctctgctctg cacacattca ctgcccctct gcacaacacc catgtacatt 60

taaaaaaaat taagaaacaa accaaagggg ttggggattt agctcagtgg tagagcgctt 120

gcctaggaag cgcaaggccc tgggtttggt ccccagctct gaaaaaaaga accaaaaaaa 180

aaaaaaaaga gagagaaaca aaccaaaaac cccaaaacaa aagtgttaac tgtttcaatt 240

ctaaactgga ggccctggtc tccactcctg gtttttagca gttttccctg cctgtaaaat 300

gagagtacta acagttactg ctgtagcatc ttctatgggt taagagtttc actgttcttc 360

tagcagttct cagaaaaatg atcatcttct aaaggttaag agtttcactg ttcttctagc 420

agttctcaga aaaatgatcc gatcactaca cctaacctgc ccaagtgacc caggagctac 480

agcgggtgaa tgacaggtgt ggattggact agctgggaag gaccggcttc ttggacctca 540

ccaggccaac gagtgtctaa acttgaggca tccagccagg catggcggca cacgccttta 600

atcccagcag ttcagaggta gaatcagaca gaactcagag aaagggccat cttggcctac 660

acagcaagct ccaggccagc caggagctcc tgtctctaaa taaacagatt aaagagaaat 720

aaaaaaaatc aactttttga tttgaggagc atcgtggagg gcagctagga tcagcaagtt 780

tcacagcctc ttcagacagt aagtagagta cccttgtcag ccagccaagc aaatgcagaa 840

agtcaagctt cattccaaat gtctggtacc agggagcttg ccccgcacct cgcagaagcc 900

acaacagtac ttctttcggt ggcactggtc ccactggtag tctctaagcg tccctcccta 960

caccgcacgc acttcgcccc tcaaggctac aacttcctgt ctagataact aggctccgcc 1020

caggcccgta ggctccgccc cactcataat aaaagactgg attcagctcc acccacgaac 1080

aactaccccg gccctaggcc ggcctcctag gcgccaacct caccctagcg gaaagctagg 1140

ccggggctcc agttttgcct ctaccaggct aggccccacc cgtgggagaa gactaggctt 1200

cgccactagc agaaggcttg gatttcatcc tatcccataa acacctaccc catggctctg 1260

tcctcatccg tagcagaaag ctggaccatc caccgtccca gccggctggc cccgttcctt 1320

agcttcttct ctcatactta gcagaaagat gaccccaccc cattgctccg gtcccgcccc 1380

tagcagaagg ctggactctg ccctacccta caggctccat cctgtatggc tcctgcctgc 1440

atccctcata caagtctgga ttctgtccag cccacaggtc tggccccatc catagcaaaa 1500

aggttggact cctccccagc ccaccactag gtatgcgact ctagagatcg ggtaccgagc 1560

tcggtacccg gggttcctct acaccccccc tgcaggacct atcccattcc atagatccgc 1620

ccctcacaga actttactcc gggttccagc cagtagcccc taccctgagc acgctccacc 1680

ctgccctcag actccatcca cccaggccca ggcccagtac taggctggcc accctcccgg 1740

atcaaggccc aacccttttc taatgactgg accctcccta tcccagcaag gcccgcccca 1800
gctaataggc cccgccaccc ggcccagcac tggactccct cggggcacac aggtcccgcc 1860
cccgcctcag gctcccaccc accccagtgc aaggctaaac tcctctttgc agaggcgccg 1920
ccccaccccg cctcctccag cctggtggtt ccgcagccgt actctcctgg cctggtgtgc 1980
acagcctcac catgagttct tctgcagcca gcccgctttt cgcgcctggc gaggactgcg 2040
gccccgcgtg gcgcgcggcc cccgcggcct atgatacgtc tgacacgcac ctgcagatcc 2100
tgggcaagcc agtaatggag cgttgggaga ccccctacat gcattcgctg gcggctgctg 2160
ctgcctccag aggtactctg cccagaagaa acggaggcta ctgtttaagg agtgtaggag 2220
ttcccatgca tgctgcatgc ctgagtttcc ttacctgagg cactgagctg ccttggggtt 2280
accttggcag ggaagggaag agtgttggca aaatgtagac gcaggcaaga gaagccggct 2340
ccgaactaga acttcctctc ctggggaaaa agacttctac ttcccggagc tgggttggag 2400
ggtggatctt cggaccacag cctcgagtgg agcttagaga acatggaggg gcgtgggact 2460
tgtgcgggtc gggaacctcc gagtttcccc aggctgttgc taagaatagc ttgggaaaac 2520
ggagcagaga ggcagctgtc cggtgctatg gccctgaact tctggacgag gggtggtgtc 2580
tgggacaccc cagcctcaaa caggtcaata gccaaagggc ttcaaagttc acccggagca 2640
catgaggacc tggtatcaag tgcggaatga aacatggagg ggttacagtg tcgggcctca 2700
gaaccaggct tggcccttta taaccccaca tagggccata gcacaaatag cgtgggtggg 2760
catgggctca gagtccaggg atccctgggg ccaagcccaa aagcacgtgg ccagtccctc 2820
aggctcccag gctcgccgct ctgattggca gctggccagg gcccacactc tctgtgctac 2880
tttgttctgt ccacccactg ctggctggct gtccttgagg catgtgtgag accggtccac 2940
cctaaccgtc cccctccccc acccatgcca gctgtagtgt ttttgtcctc ctagggggcc 3000
gggtcctgga agtgggcttt gggatggcca ttgcagcctc cagggtgcag caggccccca 3060
taaaggaaca ctggattatt gaatgcaacg atggggtctt ccagcgtcta caaaactggg 3120
ccctgaagca gccacataag gtaccttctg tcactggagt aggaggctgg gagtaccccc 3180
ccgggactag ggagatcatc gggggtacca atgtgacccc ttatatcctt ctcttgctcc 3240
cttctcctct ccaaggttgt tcccttgaaa ggcctgtggg aggaggaggc acctacactg 3300
cctgatggtc actttgatgt gagggcactt tgggagcctg gcaagggggt tggcctctgg 3360
gtactgattg gcagcctctt gggctatcat accctcctta gctcagtgga gcttgccgtc 3420
aagaatgagg accagagttt gatccccagg atgcatgtgg tgtaaggaga gaatgggtgc 3480
tcataagttg tcccctaccc caacaaatgc acctccaatg tgcactgggg acatagcctc 3540
cgggaaaagg ctccatgaac tggctagcag aggtgctaag ggcagctggg cgggctgggc 3600
agtttggcct ccaaggccat gggataggtg ggatgaagct tgggcaaggc tgtgccagcc 3660
ctgactggca ggcctgtgtt cacgctgcag gggattctat acgacacata tccactgtct 3720
gaagagacct ggcacactca ccagttcaac tttattaagg tgggactgca gggccgggga 3780

ccgagggcct tggacggtca ggtgggcagg gtgtgatatc tacagaccag cagctctctg 3840

ctcatggtcc ctccacccct tcttgtccat gccctttctc tctctctgca gactcatgct 3900

ttccgtttgc tgaagcctgg gggtatcctc acttactgca acctcacgtc ctggggggaa 3960

ctcatgaagt ccaagtacac agacatcact gccatgtttg aggtacgctg cctcatagca 4020

agtgggggct ccgacccaaa agccaggcag tggcccggca agtgtggtgt tgagacccat 4080

gacatggggg agaggtccag tcagggcttc tctgcagccc gaacctactg ctctctgcct 4140

gtggggcggt gcctgagaga tgcccaccag ggccatcagg acagcttccc ttcagcgcat 4200

gaggcagctc ttggcacaga aaactcctct tgctcgagag gcctacagca ggcctgtgca 4260

aggatgctgg tctgagacac agctgaggtc atcaggaaaa ctcgttgcct gcatagaatt 4320

cacactcaca ctaggccctg tcccgcatca tcagtggtga cctgtgaccc ctccctacag 4380

gagactcagg tgcctgcact gctggaagct ggcttccaga gagaaaacat ctgtacagag 4440

gtgatggcgc tggtgccccc agccgactgc cgctactatg ccttccctca gatgatcaca 4500

cccctggtca ccaagcactg agcggctggc ccagggctac aaggagaata tgtcctcctc 4560

agtgcctttg tagctggagt gtgggctcca gcctctccac tatccctgca gtgtgacatc 4620

ctaacctctg cctggtacgg ccatctcccc agagctcagg agtaaaataa atgctaccaa 4680

gactagtagt gggttggctg gcggtgagcc ggaggtgagg cccgcatctg gttcagagcc 4740

tgcctctccc tgcctccata gatgtggcta ttccatcact ggtgctcagt ggcccccgac 4800

ccagacatga ccaatcaacc attgtatgtt ttcaacagtg gcccaggaag agcctggggt 4860

ctcaccaggg tcagggtagg gagagcctcc acgggtccca gcccagcaca cagcaagtca 4920

tgcaaccctc atggcacgac cctactctga acatctctgg tacc 4964

<210> 566

<211> 1539

<212> DNA

<213> Rattus norvegicus (Norway rat)

<400> 566
gaattccgcg ctacaggcat ttttgagtcc aatatcttct acagtatgtc tgctgctcgg 60

ctctccgcgg tggcacagtc caccgtgtat gccttctccg cgcgcccgct ggcgggcggg 120

gagcccgtga gcctgggctc cctgcggggc aaggtgctgc tcattgagaa tgtcgcgtcc 180

ctctgaggca ccacgacccg ggactacacc gaaatgaatg atctgcagaa gcgtctgggg 240

cctcgtggcc tggtggtgct cggtttcccg tgcaatcagt tcggacatca ggagaatggc 300

aagaatgaag agattctgaa ttccctcaag tatgtccgac ccggtggtgg gttcgagccc 360

aactttacat tgtttgagaa gtgcgaggtg aatggtgaga aggctcaccc gctctttacc 420

ttcctgcgga atgccttgcc agcacccagt gacgatccca ctgcgctcat gaccgacccc 480

aagtacatca tttggtcccc ggtgtgccgc aacgacattt cctggaactt tgagaagttc 540

ctggtaggtc cagacggtgt tccagtgcgc agatacagca ggcgctttcg caccatcgac 600

atcgaacccg atatagaagc cctgctgtcc aagcagccta gcaaccccta aggcattcct 660

ggtatctggg cttggtgatg gctggctgcc ctccgggggg aggtttttcc atgacggtgt 720

ttcctctaaa tttacatgga gaaacacctg atttccagaa aaatcccctc agatgggcgc 780

tggtctcgtc cattcccgat gcctttacgc ctaaagaaag gcggtttcac cactaagaat 840

aaagtgctgc ggaattccgt gtcttcctgg gcacgttttt gtatgaatat tcaagaaggc 900

accccgatta ctccgtgtcc ctgctgctga gacttgctaa gaaatatgaa gccacactgg 960

agaagtgctg tgctgaaggc gatcctcctg cctgctacgg cacagtgctt gcagaatttc 1020

acctcttgta gaagaaccta agaacttggt caaactaact gtgagcttta cgagaagctt 1080

ggagagtatg gattccaaaa cgcattctgg tccgatacac ccagaaagca ccctaggtgt 1140

cgaccccaac tctcgtggag gcagcaagaa acctgggaag agtgggcacc aagtgttgta 1200

cccttcctga agctcagaga ctgccctgtg tggaagacta tctgtctgcc atcctgaacc 1260

gtctgtgtgt gctgcatgag aagaccccag tgagcgagaa ggctcaccaa gtgctgtagt 1320

gggtccctgg tggaaagacg gccatgtttc tctgctctga cagttgacga gacatatgtc 1380

cccaaagagt ttaaagctga gaccttcacc ttccactctg atatctgcac actcccagac 1440

aaggagaagc agataaagaa gcaaacggct ctcgctgagc tggtgaaaca caagcccaag 1500

gccacagaag atcagctgaa gacggtgatg ggtgacttc 1539

<210> 567

<211> 1150

<212> DNA

<213> Rattus norvegicus (Norway rat)

<400> 567
gcagcagcag cagcaccagc agcacttggg ggaaaagcta gaggctagag tcatggatga 60

ccagcgcgac ctcatctcta accatgagca gctgcccatc ctgggccagc gtgctagagc 120

cccagaaagc aattgcaacc gtggagtcct gtacaccagt gtctctgtcc tggtggctct 180

gctcttggct gggcaggcca ccactgctta cttcctgtac cagcagcagg gccgcctgga 240

caagctgacc gtcacctccc agaacctgca actggagaac cttcgcatga agcttccgaa 300

atctgccaaa cctgtgagcc cgatgcgcat ggctactccc ttgctgatgc gcccactgtc 360

catggataac atgctccaag cgcccgtgaa gaatgttacc aagtatggca acatgaccca 420

ggaccacgtg atgcacctgc ttacgaagtc tggacccgtg aactacccac agctgaaggg 480

gagcttcccg gagaatctga agcaccttaa gaactctatg aatggtctgg actggaaggt 540

ctttgagagc tggatgaaac agtggctgtt gtttgaaatg agcaagaact ccctggagga 600

gaagcagccc acccagactc cacctaaaga gccactggac atggaagacc catcttctgg 660

cctgggagtg accaagcagg atatgggcca aatgttcttg tgaagacaga agccagctct 720

gcacggcggc acgtcccctg ctcctcagcc cttcttacac tccctaacat cacacccatt 780

ccgtcttcct gcacctgggc ttgagactgg tatctgcttc accgtccctg acacaacaaa 840

tgaaaccgga acagaatgag aacactggag ggagggcctt gctgcctacc cccatctaaa 900

ggacccccat ttctgaccca ttagcagtct ttaatgtggg gctctgagat ctaggcccac 960

tgacagggat aggggatgcc ctacccttaa tctaggctgg atacatttgc tgtcttctca 1020

aggaagaggc caagccctcc cagcaaccct tcctcatgtc ctgccgacgc ccctgggatc 1080

cctgctcagc caagcttgtc agcagcctgt aggaccatgg ttcacgtgac aataaaaggt 1140

agaaggtaaa 1150

<210> 568

<211> 1627

<212> DNA

<213> Rattus norvegicus (Norway rat)

<400> 568
   cccctgaaga ctggataact gtcatggagg attcacagtc ggatatgagc atcgagctcc 60
   ctctgagtca ggagacattt tcatgcttat ggaaacttct tcctccagat gatattctgc 120
   ccaccacagc gacagggtca cctaattcca tggaagatct gttcctgccc caggatgttg 180
   cagagttgtt agaaggccca gaggaagccc tccaagtgtc agctcctgca gcacaggaac 240
   ctggaactga ggcccctgca cccgtggccc ctgcttcagc tacaccgtgg cctctgtcat 300
   cttccgtccc ttctcaaaaa acttaccaag gcaactatgg cttccacctg ggcttcctgc 360
   agtcagggac agccaagtct gttatgtgca cgtactcaat ttccctcaat aagctgttct 420
   gccagctggc gaagacatgc cctgtgcagt tgtgggtcac ctccacacct ccacctggta 480
   cccgtgtccg tgccatggcc atctacaaga agtcacaaca catgactgag gtcgtgagac 540
   gctgccccca ccatgagcgt tgctctgatg gtgacggcct ggctcctccc caacatctta 600
   tccgggtgga aggaaatccg tatgctgagt atctggacga caggcagact tttcggcaca 660
   gcgtggtggt accgtatgag ccacctgagg tcggctccga ctataccact atccactaca 720
   agtacatgtg caacagctcc tgcatggggg gcatgaaccg ccggcccatc cttaccatca 780
   tcacgctgga agactccagt gggaatcttc tgggacggga cagctttgag gttcgtgttt 840
   gtgcctgtcc tgggagagac cgtcggacag aggaagaaaa tttccgcaaa aaagaagagc 900
   attgcccgga gctgccccca gggagtgcaa agagagcact gcccaccagc acaagctcct 960
   ctccccagca aaagaaaaaa ccactcgatg gagaatattt cacccttaag atccgtgggc 1020
   gtgagcgctt cgagatgttc cgagagctga atgaggcctt ggaattaaag gatgcccgtg 1080
   ctgccgagga gtcaggagac agcagggctc actccagcta cccgaagacc aagaagggcc 1140
   agtctacgtc ccgccataaa aaaccaatga tcaagaaagt ggggcctgac tcagactgac 1200
   agcctctgca tcctgtcccc atcaccagcc tccccgtccc ctcctttctt gccattttat 1260
   gactttaggg cttgttatga gagctgacaa gacaatgcta gtcccttcac tgcctttttt 1320
   taccttgtag atagtactcg gccccctcta tgcaaactgg ttcctggccc agattgggga 1380
   atgggttggt agttgctggg tctctgctgg tccagcgaaa tcctatccgg tcagttgttg 1440
   gacctggcac ctacagtgaa atttcacccc accccaccgc ctgtaagatt ctatcttggg 1500
   ccctcatacg atctgtatcc tccaggaccc atttcctcca ctctgcaaag cctgtctgca 1560
   tttatccatc ccccacccct ctccctcttt ttatctcttt ttatatatcc aatttcttat 1620
   tttacaa 1627

<210> 569

<211> 1215

<212> DNA

<213> Rattus norvegicus (Norway rat)

<400> 569
   acaagcacgt tcgggagtgg aggcgccagg gcatccatgt gctagtgtcg acaagcaatg 60
   tcagttcact ggagggggcc cgtgctctca tcgctgaagc cacaaagctt gggcccgttg 120
   gaggtgtctt caacctggcc atggttttaa gggatgccat gctggagaac cagactccag 180
   aactcttcca ggatgtcaac aagcccaagt acaatggcac cctgaacctt gacagggcga 240
   cccgggaagc ctgtcctgag ctggactact ttgtggcctt ctcctctgta agctgcgggc 300
   gtggtaatgc tggccaatcc aactatggct tcgccaactc taccatggag cgtatttgcg 360
   aacagcgccg gcacgatggc ctcccaggtc ttgccgtgca atggggtgcc attggtgacg 420
   tgggcattat cttggaagcg atgggtacca atgacacagt cgttggcggc acactgccac 480
   agcgcatctc ctcctgcatg gaggtgctgg acctcttcct gaatcagccc cacgcagtcc 540
   tgagcagttt tgtgctggtt gagaagaaag ctgtggccca tggtgatggt gaagcccaga 600
   gggatctggt gaaagcagtg gcacacatcc taggcatccg cgacctcgca gggattaacc 660
   tggacagctc gctggcagac ctcggcctgg actcgctcat gggtgtggaa gtgcgccaga 720
   tcctggaacg tgaacatgat ctggtgctac ccattcgtga agtacggcaa ctcacactgc 780
   ggaagcttca ggaaatgtcc tccaaggctg gctcagacac tgagttggca gcccccaagt 840
   ccaagaatga tacatccctg aagcaggccc agctgaatct gagtatcctg ctggtgaacc 900
   ctgagggccc taccttaaca cgactcaact cagtgcagag ctctgagcgg cctctgttcc 960
   tggtgcaccc cattgaaggt tccatcactg tgttccacag cctggctgcc aagctcagtg 1020
   tgcccaccta cggtctgcag tgcacccaag cggcccccct ggacagcatt ccaaacctgg 1080
   ctgcctacta cattgattgc atcaagcagg tgcagcctga ggggccccac cgagtggctg 1140
   ggtattcttt tggagcttgt gtagccttcg agatgtgctc ccagctgcag gcccagcagg 1200
   gcccagcccc cgccc 1215

<210> 570

<211> 591

<212> DNA

<213> Rattus rattus (black rat)

<400> 570
cttttgtgag tggcagtgaa cgagcacgca ctgctatgaa ggcgtcgggc acgcttcggg 60

agtacaaggt ggtggggcgt tgcttgccaa ccccaaaatg ccacacaccg ccactgtacc 120

gaatgcgaat ctttgcaccc aaccatgtgg tggccaagtc ccgcttctgg tactttgtgt 180

cgcagctgaa gaagatgaag aagtcatccg gggaaattgt gtactgtggg caggtgtttg 240

agaagtcacc cctgcgtgtg aagaacttcg gcatctggct gcgctatgat tcccgaagtg 300

gcactcacaa catgtaccga gagtaccggg acctgaccac tgccggcgcg gtcacacagt 360

gctaccgaga catgggtgcc cgacaccgtg cccgtgcgca ctccatccag atcatgaagg 420

tggaagagat tgcagctggc aagtgccgcc ggccagctgt caagcagttc cacgactcca 480

agatcaagtt cccattgccc caccgtgtgt tgcggcgcca gcacaaacca cgcttcacca 540

ccaagaggcc aaacaccttc ttctagacac cagagaccca ctgaataaaa g 591

<210> 571

<211> 1353

<212> DNA

<213> Rattus rattus (black rat)

<400> 571
cagcagcagc agcagcagca gcaccagcag cacttggggg aaaagctaga ggctagagtc 60

atggatgacc agcgcgacct catctctaac catgagcagc tgcccatcct gggccagcgt 120

gctagagccc cagaaagcaa ttgcaaccgt ggagtcctgt acaccagtgt ctctgtcctg 180

gtggctctgc tcttggctgg gcaggccacc actgcttact tcctgtacca gcagcagggc 240

cgcctggaca agctgaccgt cacctcccag aacctgcaac tggagaacct tcgcatgaag 300

cttccgaaat ctgccaaacc tgtgagcccg atgcgcatgg ctactccctt gctgatgcgc 360

ccactgtcca tggataacat gctccaagcg cccgtgaaga atgttaccaa gtatggcaac 420

atgacccagg accacgtgat gcacctgctt acgaagtctg gacccgtgaa ctacccacag 480

ctgaagggga gcttcccgga gaatctgaag caccttaaga actctatgaa tggtctggac 540

tggaaggtct ttgagagctg gatgaaacag tggctgttgt ttgaaatgag caagaactcc 600

ctggaggaga agcagcccac ccagactcca cctaaagtat tgaccaagtg ccaggaagaa 660

gtcagccaca tccctgatgt ccacccgggg gcgttccgtc ccaagtgtga tgagaacggt 720

aactatatgc cactccagtg ccatgggagc actggctact gctggtgtgt gttccccaac 780

ggcactgagg tccctcacac caagagccgc gggcgccata actgcagtga gccactggac 840

atggaagacc catcttctgg cctgggagtg accaagcagg atatggccca aatgttcttg 900

tgaagacaga agccagctct gcacggcggc agctcccctg ctcctcagcc cttcttacac 960

tccctaacat cacaccccat ttcccgtctt cctgcaccct ggggcttgag actggtatct 1020

gcttcaccgt ccctggacac aacaaatgaa accggaacag aatgagaaca ctggagggag 1080

ggccttgctg cctaccccca tctaagggac ccccatttct gacccattag cagtctttaa 1140

tgtggggctc tgagatctag gcccactgac agggataggg gatgccctac ccttaatcta 1200

ggctggatac atttgctgtc ttctcaagga agaggccaag ccctcccagc aacccttcct 1260

catgtcctgc cgacgcccct gggatccctg ctcagccaag cttgtcagca gcctgtagga 1320

ccatggttca cgtgacaata aaaggtagaa ggt 1353

<210> 572

<211> 2681

<212> DNA

<213> Rattus norvegicus (Norway rat)

<400> 572
agctaggaag ataggaagtg aggtccaata ccttgtgggc agtggtgtca ttagctgcaa 60

cgcctaagat gtctcgagag atgggagaac tcactcaaac caggttacag aagatctgga 120

ttccacacag cagcagtagc agcgtgctgc aacggcgaag gggctcctcc ataccacagt 180

tcactaattc tcccacgatg gtgatcatgg tgggtttacc agctcgaggc aagacctaca 240

tctctacgaa gctcacacgc tatctcaact ggataggaac accaactaaa gtgtttaatt 300

taggtcagta tcgacgagag gcagtgagtt acaggaacta tgaattcttt cgcccagaca 360

acacagaggc ccagcttatc aggaagtaag taacagtatt taagactctg tactgcccct 420

tggctgagtg gggcctgtct ttatatcata agagagtggt atagagaaac ccacttttcc 480

agggtgtcca tacttttgac attacaaccc agcatagcca tctacagtat tggttcttga 540

gaattcctca gtcaagttat gaatagggaa aaatgcagaa attctcaaaa attttaaagg 600

aagtttacag tctgtgttgg gtcacatttc atagttagcc taggccatga gttgaacgcc 660

tttgtgccca gtctttctgt cagcctactc aacatctgct ctagtcatag ccattttctc 720

tcaagaaggt tttccaggcc tgtatttctg gtaagcaagc ataggaatat cttctcctcg 780

cacttgagat gagtgtttcc caagcttcag tctctgtctc atcacttcag ctttaaagtc 840

tcttttacct atgtgtttgg aagtacttgt agatgcaacc tggctctgaa cctcttatct 900

gggtcttcac tacagaaccc aataaccaat aagaaatatt ttgctgaaac ctaagtacat 960

tatcactgtt atacctgtgc ttaaaatatt gcctatgcca agaattcgtt ttaactcaaa 1020

gagcacatcc atccctatgc cttacccatg tacattaacc aattcagctt tagctccagt 1080

agcacacagt tagatcccta aaactatggt gatttcattg ttcagttttt cttgggcatg 1140

tctcctcatc ttcagcttct aagcatacac acagagccag tttcaactct gaccccttta 1200

ttagaacaga gggcaccgtt ctagatgttc gatggatagg tgaactatgt agatgttcaa 1260

cagagagatg ctttgtaaga gaactgcagt gagaggaact gatcctgagg atagggattc 1320

tctgcttctt tctctgtttt tcaggcagtg tgctctagca gccctaaagg atgtccataa 1380

gtatctcagc cgcgaggaag gtcatgttgc ggtttttgat gccaccaaca ctaccagaga 1440

acgacgatcg ttgattctac agtttgctaa ggaacatggt tataaggtct tctttattga 1500

gtctatttgt aatgaccccg aaatcattgc agaaaacatc aagcaagtga aacttggtag 1560

tcctgattac atagactgtg accaagaaaa ggttttggaa gactttctaa agagaataga 1620

gtgctatgag atcaactacc aacctttgga tgaggaattg gacagccacc tgtcctacat 1680

caagatcttc gacgtgggca cacgctacat ggtaaatcga gtgcaggacc acgttcagag 1740

ccgtacagcc tactacctca tgaacatcca tgtcacacct cgatctatct acctatgccg 1800

ccatggtgag agtgaactca accttagagg ccgcattgga ggtgactctg gcctctcagc 1860

tcggggcaag cagtatgcct atgcactagc caacttcatc cggtctcaag gcatcagctc 1920

cctgaaagta tggactagcc acatgaagag gaccattcag accgctgaag ccctaggtgt 1980

cccctatgaa cagtggaagg ccctgaatga gattgatgcg ggtgtctgtg aagagatgac 2040

ctatgaagaa attcaggaac actaccctga ggaatttgca ctacgggacc aggataaata 2100

tcgttaccgc tatcccaagg gagagtccta tgaggatctg gttcagcgtc ttgaaccagt 2160

tataatggag ctagaacggc aagaaaatgt actggtgatc tgtcaccagg ctgtcatgcg 2220

gtgcctcctg gcatacttcc tggataaaag ttcagatgag ctgccctatc tcaagtgtcc 2280

tctgcatact gtgctcaaac tcacacctgt ggcttatggc tgcagagtgg agtccatcta 2340

cctgaatgtg gaggctgtga acacacaccg ggacaagcct gagaatgtgg acatcacccg 2400

tgaagctgag gaagccttgg acactgtacc tgcccattac tgagcccttt tcaagtgatc 2460

agattgtctc tgttctcacc cccttcctcc tgtaggagct gctgcccttg ttctccttaa 2520

gcagactctg gctatggcct gagagtgccc tacctccagt gaagaagtcc gtagcagctc 2580

ccaaacaggt ctcaattcct agccacaact aaggcaccta cctagttgtg gataaagttc 2640

cttttaaata ttctctgata aataaagatg tttatttctg c 2681

<210> 573

<211> 1087

<212>  DNA

<213>  Rattus norvegicus (Norway rat)

<220>

<221>  misc_feature

<222>  (304)..(304)

<223>  n

<220>

<221>  misc_feature

<222>  (307)..(307)

<223>  n

<400>  573
caggccagtc tgggggtgag gaaaaggggg tatttctctc acggctctga ggttcaggca     60

ggcacttaaa agatatttct gacctacagc tcacaacaac taccaggccc agtccgctgt    120

ccccctgcgg cacgagaccc acggtgggga agatgtggcg gtctttgcca agggccctat    180

ggctcacctg cttcacggcg tccatgagca gaactacatc ccccacgtca tggcgtatgc    240

ctcctgcatt ggagccaacc ttgaccactg tgcctgggcc agctctgcga gcagcccctc    300

cccnggngcc ctgctgcttc cactggctct gttcccccta cgcaccctgt tctgagggcc    360

caggtcccac aagagcccac aatggacagc cggctcccct ccctttgtgg cctgccacct    420

ggccgcccac actcaacggg gaggcccagg caacctcgag caggaacaga agtttgctac    480

ctgcctcact tccgcccgga accctccgtg ggtcggattc ctggctctgc cgttgtttct    540

ctattcactg ccttttggcc agcagggtgg gtttctctct tgggccggca ggacacagac    600

tgcgcagatt cccaaagcac cttatttttc taccaaatat actctccaga ccctgcaacc    660

atcatggaac attccagatc tgaccttctc tcccctaccc cttctctctg gaacactggg    720

tcccatagtc acagccagtc cctcaaccca accctccctg gagggaagac caggtctgct    780

cagggtgaga ctcccaggaa gccacctccg gggttggctg tctacccagg gtggccaggc    840

tgggaagaac aacccagccg gacaggacgc acacactccc cacccagctc cagagactcg    900

ccaacccttc actgaagcga ctcccctgtt tggaatagca aaaaaaaaaa agaaagaaaa    960

aaaagaaaaa aattttaatt tctctttttg gtgttggtta aaagggaaca caagacattt   1020

aaataaaatg ttccaaatat ttctgaggcc agagctgagt ctttgtggtc agtgggaaag   1080

agaccaa                                                              1087

<210>  574

<211>  1738

<212>  DNA

<213>  Rattus rattus (black rat)

<400>  574
cggagcacag cggccgccga gcactgcgag cgagcggccg gcgccacgcc agcagccgcc      60

tggggccgca ggaagcaccc cggggagcgg cggcggcgtg tgcgtgtggc gcgggtgcgg     120

gcggcggcgc gggagcaggg cagagcggca gccggttcgg gcgggcggca tcatggacga     180

gaagttgttc accaaggagc tggaccagtg gatcgagcag ctgaacgagt gcaagcagct     240

ctccgagtcc caggtcaaga gcctctgcga gaaggctaaa gaaatcctta caaaagaatc     300

taatgttcag gaggttcgat gtccagttac tgtgtgtggg gatgtgcatg ggcaatttca     360

tgacctcatg gaactcttta gaattggtgg taaatcacca gatacaaatt acttgtttat     420

gggagactat gtggacagag gatattactc agttgaaaca gttacactgc ttgtagctct     480

taaggttcgt taccgagagc gtatcaccat actccgaggg aatcacgaga gcagacagat     540

cacacaagtt tatggtttct acgatgagtg tttaaggaaa tacggaaatg caaatgtttg     600

gaaatacttc acagaccttt ttgactacct tcctctcact gccttggtgg atgggcagat     660

cttctgtcta catggtggtc tttcaccatc catagacaca ctggatcaca tccgagcact     720

tgatcgccta caagaagttc ctcatgaggg tccaatgtgt gacttgctgt ggtcagatcc     780

agatgaccgt ggtggctggg ggatatctcc tcggggagct ggttatacct ttggccaaga     840

tatttctgag acatttaatc atgccaatgg cctcacgttg gtgtccagag ctcaccagct     900

ggtgatggaa ggatataact ggtgccatga ccggaatgta gtaacaattt tcagtgctcc     960

aaactattgc tatcgttgtg gtaaccaagc tgcaatcatg gaacttgatg acactcttaa    1020

gtattctttc ttgcagttcg atccagcacc tcgtagaggc gagccacatg tcactcgtcg    1080

taccccagac tacttcctgt aatgaaagtt taaccttgta cagtattgcc atgaacaccg    1140

tctgttgacc taatggaatc ggaagagcag cagtaactcc aaagtgtcag aaatagttaa    1200

cattcaaact tgtttccaca cggaccaaaa gatgtgccat ataaaataca aagcctcttg    1260

tcatcaacag ccgtgaccac tttagaatga accagttcat tgcatgctga cgcgacattg    1320

ttggtcaaga atccagtttc tggcatagcg ctatttgtag ttacttttgc tttcttgaga    1380

gactgcagat ctaggatgta acattaacac ctgtgagtcc agttgacttc cacttagctg    1440

tagcttactc agcatgactg tagagaggat agcaaacaat cattggagct taatgaacat    1500

ttttaaatga gtaccaaggc ctcccctctt gttgtgttct ttcagggata ctattaattt    1560

aattgtatga tttctctgca ctcagtttct cccttctcaa atctcggccc gcgttgttct    1620

ttgttactgt cagaaaacct ggtgagttgt ttgaacagaa ctgtctcctc ctgtaagatg    1680

atgtactgcc aagtacgcag tgtttcataa taaacttgag actgagaagt cagggttt      1738

<210>  575

<211>  1580

<212>  DNA

<213>  Rattus rattus (black rat)

<400>  575
tgcaggtcgg cggcggggga ggcggcgggg gacggctcgc cgccatggac gacaaggcgt      60

tcaccaagga gctggaccag tgggtggagc agctgaacga gtgtaagcag ctgaacgaga     120

accaagtgcg gacgctgtgc gagaaggcta aggaaatttt aacaaaagaa tcaaatgtac     180

aagaggttcg ctgtcctgtt accgtctgtg gagatgtgca tggccaattc catgacctta     240

tggaactctt cagaattggt ggaaaatcac cagacaccaa ctatctattc atgggtgact     300

atgtagacag aggatattat tctgtggaga ccgtgactct tcttgtagca ttaaaggtgc     360

gctatccaga gcgtatcaca atattgcgag gaaatcatga aagccggcag atcacacaag     420

tatatggctt ttatgatgaa tgcctacgaa agtatgggaa cgccaacgtg tggaaatact     480

ttacagatct ctttgattat cttccactta cagctttagt agatggacag atattctgcc     540

tccacggtgg cctctctcca tccatagata cactggatca cataagagcc ctggatcgct     600

tacaggaagt tccacatgag ggcccaatgt gtgatctctt atggtcagat ccagatgacc     660

gtggtggctg gggcatttct ccacgtggtg ctggctacac atttggacaa gacatttctg     720

aaacatttaa ccatgccaac ggcctcacac tggtgtcccg tgctcaccag cttgtaatgg     780

aaggatataa ttggtgccat gatcggaatg tggtcaccat ttttagtgca cccaattact     840

gctaccgctg tgggaaccag gctgctatca tggaattaga cgacacttta aaatactctt     900

ttcttcagtt tgacccagca cctcgtcgtg gagagcctca tgtgacccgg cgcaccccag     960

actacttcct ataaattcct ccccaggacc tgtctttgta tgttgaagta tacctggctt    1020

tttaaaaaat atatatacat atatatattt aaaaacaaca gttatctgtg tgtctctgta    1080

acaaattgtg ctatgtcttg acgttaaaac acatcatgga ccaaaacgtg ccatactaat    1140

ggtgagccat cagcacggtg tgaacttgag tccactgtcc tagccacgag tcaaccaggc    1200

agccgcctgc ccgcctgcct gctgtagtag ccgtccttcg tgactggtta agggaaaggg    1260

tcactggtgg cttcatcttc cttcgcgctt acttggaaat ttagttacag tttaactggc    1320

atggattata gagttggagt tttattttta agaattgaca agctgacttc cacttaaatt    1380

cataaccctt tattttgttg aaatgtatga ctaactgaag aagagattct tgggagtatg    1440

ttgtcataac actaagattt cctttcaagt ttcctgaact gaattactgt tggatgttga    1500

cctgcacatt ctgtatattt gtcctgacag tgttgcatcc tccttgctgt actgaacaat    1560

aaacttccca atttagagag                                                1580

<210>  576

<211>  1981

<212>  DNA

<213>  Rattus norvegicus (Norway rat)

<400>  576
caacgcggtt ctgttggtgt tcaggttccc tactgacttc ccttccagtg gcagcggcgg      60

aggcggcagt ggcagcggca agcactgagc acgtttgggt agttgtccaa gatgccgaat     120

atcaaaatct tcagcgggag ctcccaccag gacttatccc agaaaattgc tgaccgcctg     180

ggcctggagc taggcaaggt ggtgactaag aaattcagca accaggagac ctgtgtggaa     240

attggggaga gtgttcgtgg agaggatgtc tacattgttc agagtggctg cggtgagata     300

aatgacaatc taatggagct tttgatcatg atcaatgctt gcaagatcgc ttctgccagc     360

cgggttactg cggtcatccc atgcttccct tatgcccggc aggataagaa ggataagagc     420

cgggctccaa tctcagctaa gcttgttgca aatatgctat ctgtagcagg tgcggatcat     480

attatcacca tggatctaca tgcatctcag attcagggct tctttgatat cccagtggac     540

aatttatacg cagagccagc tgtcctgaag tggattcggg agaacatatc tgagtggaga     600

aactgtacta ttgtctcacc tgacgctggc ggagccaaga gagtgacctc cattgcagac     660

cgattgaatg tggattttgc tttgattcac aaagaacgga agaaggccaa tgaagtggac     720

cgcatggtac tagtaggaga tgtgaaggat cgtgtggcta tacttgtgga tgacatggct     780

gacacttgtg gtacaatttg tcatgctgct gacaaacttc tctcagctgg agctaccaga     840

gtttatgcca ttttgacgca tgggatcttt tctggtccag ccatttctcg catcaacaat     900

gcatgctttg aagcagtagt agttaccaat accatacctc aggaggacaa gatgaaacac     960

tgctccaaaa tccaggtgat cgacatctct atgatccttg cagaagccat caggagaacc    1020

cacaatggag aatctgtctc ctacctgttc agccatgttc ctctataata tcataaattc    1080

tgaagcgttt tcaaaatgaa accaccccca ccccttgttt ttcttgtatt tggtgagttg    1140

ggcagaagag ccagcttgct tcagtgtagc tttttacagc ccacattagc tacattagga    1200

tttatcctaa ttggagaaag acagactgct attaactgct aggacaccct acgtgcattg    1260

ttgcagcctg gctttcttgt ttattttgtg atccttgcag ctttaaattg agttccactg    1320

ttgaaagctg catagactgt taaaagagag agtattcccc aaggtcctta ggatggaatt    1380

gtggagctca tactccattg catgtgaagg ctttggagtt tccttcattc agaataacta    1440

gcgacaaagc cagccctcgt gtgatacatt ttcagaggtg tatgctacac tgcagcaagt    1500

tccctgtgca tccttaaaca gagttcgcct gctgagcacc ctgtacggca ggggcgggta    1560

gctcaatgca gtaatcaatt tttgggagga agaagcctga tctattatca tctgtttaat    1620

tctctgtagc ttggattctc tatgcctatt cctttcctgt tggcttcacc ttcagctatc    1680

tcgacccttt cctggacaaa tatcttgaac tcagtgatca ttgtttgtga caaaattttc    1740

tgtaagacag acttgcttct tgctatgtaa ctgctaacgt tgacattagg taacttgcta    1800

tagcacaatc ttccttagcc cgctctctct gagttagtgg gttttaggtg gtgtgtgtgt 1860

cgtagtgcct tttgattaat ttaagtattt aatttttatc ttccttcgat ctgtttggct 1920

taacaatttg atcttattgt ctcatgtaga agaaacttaa taaaatgtct gtcatgtgta 1980

c 1981

<210> 577

<211> 3105

<212> DNA

<213> Rattus sp.

<400> 577
aaattaaatc taaggacttt cagatttatg gctttgatca cactgtttct agagaaatct 60

aaacctggaa ggctgagtta agccagacat tccagatggc tctctcctca tagtccttgg 120

aatcacgaag gaagcagggc agagagctac cagaagtagt aaacattgat cacaggctcc 180

tagttcatcg tgaccaaatc aaaaggaatg tttctccatg gcccattaac tgtctgttag 240

tttgaacgta acatggtgat agccagactg gagctacctg agtcctgttc cagggaatct 300

tagggcaatt acctacataa cccttctgga cctcaactgc ctgatcttag ggattaataa 360

catctattta ccagagcgac tgcattgtga agggttccaa acactcctgg cacagagtaa 420

gcactgtctg ggctttggat agaaatctct tctgcaccat gagctcattt ataagacttt 480

ccaggtctgg aattgtacaa cccaaacagc tcatatcaat gtcacaagct cttcggtttg 540

gcaaaatgtc tgggagtcac caaatgcaga gaatgccata ttcaacaaag cctgataacc 600

aaggactcag tggactaatt ggcagtccta tcccagatcc aaggttcctt gagccagggg 660

caagctagga tatgctccca ggtatcttct cccttaggac tttaggtttc ttggccactt 720

cctcttattt cagtgaaagc agatccactc cattgacact tgtggtcaca gtctagcacg 780

actgctccct tccttctttt ctccctccct gcgcagcttc atttgctccc agtagtggct 840

ggaaaaacac caaattccaa tccgcggttt ctcccttcta cttcctggaa acatccaagg 900

gctcggcact tactcagcag attcaaacct tccactttcc atcactcatc gaggatgatg 960

ctgctccttg gcaccaacca ccctgcctga ctccaccctc tggcttacaa taaaaggctg 1020

aggcagagcc gctagaaatg cagagacaca gacagaggcc agcccagaaa ccagccaact 1080

ctcactgaag ccagatctct cttcctccac cactatgcag gtctctgtca cgcttctggg 1140

cctgttgttc acagttgctg cctgtagcat ccacgtgctg tctcagccag gtgagacccc 1200

agtttccttc tccttctagc atttcacccc attttttaat tgttgtgggc catcatagtg 1260

ggccttacct agtaaaatac tttttttttt ttaccaaggt aaggagcata gagccaaccc 1320

aattacaggg gttgcttctg gaaagcaact aggattttaa tcgttagatc aaagtttaga 1380

atcgcacctt catacagttc ctgctcccct atttcctgag tatttgagaa cctggttgat 1440

caaagaaggg cttgggttgg ttcatttttc cagatagagg agaatcagga agagacccag 1500

gatcttgatc tatgtttcac cagcttccag agatagcagc tcagcagagg tagttggtat 1560

cagagatact catgattcga tatagggttt ttttttgtaa cctatagtaa tgtactcggt 1620

aatcttctca gaccctagta atttgacttc taactaccct caaatgacag tccctagctt 1680

taatggcatc cctctgtcca agattgtgaa cttactttaa gtgtgtcaga gatcaccttc 1740

cagctctgat gtattggcat ttacatccca atctgctgaa actgccttct cctcatggtc 1800

cttttcttct ctaaggtcag aagcaccttt ccagttctaa tgtgctccct gcttctcttt 1860

tattctccag atgcagttaa tgccccactc acctgctgct actcattcac tggcaagatg 1920

atcccaatga gtcggctgga gaactacaag agaatcacca gcagcaggtg tcccaaagaa 1980

gctgtagtgt gagttataca ccccagccct ccctggtcca atatttttcc tcgagaacaa 2040

gggatggtct tcatagactt agaatcagtt acatgctcag ctccaatatc aagtggttcc 2100

caatggggaa actgaggcca agaagggaaa gttaattctc agcagcactg tctctatggc 2160

tgctgttcgg ggccttccat ttgcatgagc ttattgtagt aaacttgcag aagaggaagg 2220

tcactttgag tccccctttc tacctgccct cccacctcga gccctacaca gtccctccat 2280

gtatagcagg ttaaacttca tctaaccgtg tcttctctct ttccacagat ttgtcaccaa 2340

gctcaagaga gagatctgtg ctgaccccaa taaggaatgg gtccagaagt acattagaaa 2400

actggaccag aaccaagtga gatcagaaac tacagtcttc tataaaattg catcaaccct 2460

aaggacttca gcacctttga atgtgaactt gacccataaa tctgaagcta atgcatccac 2520

tctcttttcc acaaccacct caagcacttc tgtagaagtg accagtatga cagagaacta 2580

gtgtgatttg gaatgtgatg ccttaagtaa tgttaaactt atttaactta ttgatattac 2640

actattccct tccatgaata ctagaaatcc ttaaatgcaa gatgtagatc cattttttta 2700

tttctctgtg aatcctggtt caacactttc aatgtatgag agatgaatgg gtaaactttg 2760

tgtttgagag tccaaggtat tgtttaaaat attattatgg atattcctaa ttattaaaag 2820

aaatatatta tttttgtaca caagtctgac tttcggtgtt ttctgaggga aatggcaaag 2880

ctaagagtac ataagaacac acaggaggac atcacaagat gggacacata ttgagggggg 2940

gatgggggaa tgaatgctgc actcttttgt attgagtggt ctcatgtgag tgtcataaac 3000

tctttgagac agggtccagt cagggatgct agtaccatag ttccaatccc caggactgct 3060

tctcagacac atgctcgata aaagccccag tccttcccag tcatg 3105

<210> 578

<211> 466

<212> DNA

<213> Rattus norvegicus (Norway rat)

<400> 578
ctttccgtct ccggccgccg caggagagga gtcgccgcca tgtccgcgca tctgcaatgg 60

atggtcgttc ggaactgctc cagtttcttg atcaagagga ataagcagac gtacagcacg 120

gagcccaata atctgaaggc ccgaaactcc ttccgctaca acgggctaat tcaccgcaag 180

acggtcggag tggaggcctg gcctgatggc aaaggggtcg tggtggttat gaaacgcaga 240

tccggtcagc gaaaacctgc cacttcctac gtgaggacca ccatcaacaa gaatgctcgg 300

gctaccctca gcagcatcag gcacatgatc cgaaagaaca agtaccgccc tgatctgcgt 360

atggcggcca tccgcagagc cagtgccatc cttcgaagcc agaagcctgt ggtggtgaag 420

aggaaacgga cccgccccac caagagctcc tgagccccac accccg 466

<210> 579

<211> 1880

<212> DNA

<213> Rattus rattus (black rat)

<400> 579
gggggggggg gctgccgatc gcaatggaag aagaaatcgc cgccctcgtc attgacaatg 60

gctccggcat gtgcaaagct ggctttgctg gggacgacgc ccccagggcc gtgtttcctt 120

ccatcgtcgg gcgcccccga caccagggtg tcatggtggg catgggccag aaagactcgt 180

acgtgggtga tgaggcccag agcaagaggg gtattctgac cctgaagtac cctattgagc 240

acggcattgt caccaactgg gacgacatgg agaagatctg gcaccacacc ttctacaacg 300

agctgcgtgt ggcccctgag gagcacccgg tgcttctgac cgaggccccc ctgaacccca 360

aagctaacag agagaagatg acgcagataa tgtttgaaac cttcaatacc ccagccatgt 420

acgtggccat tcaggcggtg ctctccttgt atgcatctgg gcgtaccact ggcattgtca 480

tggactctgg tgacggggtc acacacacag tgcccatcta tgagggctac gcccttcccc 540

acgccatctt gcgtctggac ctggctggcc gggacctgac agactacctc atgaagatcc 600

tgactgaaag gggctacagc tttaccacca ctgctgagag ggaaattgtt cgtgacataa 660

aggagaagct gtgctatgtt gccctggatt ttgagcaaga aatggctact gctgcatcat 720

cttcctcttt ggagaagagt tatgagctgc ctgatgggca ggtgatcacc attggcaatg 780

agcgcttccg gtgtccagag gctctcttcc agccttcctt cctgggcatg gagtcctgtg 840

gcatccacga gaccaccttc aactccatca tgaagtgtga tgtggacatc cgcaaagacc 900

tgtatgccaa cacagtgctg tctggtggta ccaccatgta tccaggcatt gctgacagga 960

tgcagaagga gatcacagcc ctggctccca gcacaatgaa gattaagatc attgctcctc 1020

ctgaacgcaa gtactcagtc tggattggcg gctccatcct ggcctcactg tccaccttcc 1080

agcagatgtg gatcagcaag caggagtatg acgagtcagg cccctccatt gtccaccgca 1140

aatgcttcta gatggactga gcaggtgcca ggcatctgct gcatgagctg attctgaagt 1200

atcgatttgc cctggcaaat gtacacacct catgctagcc tcatgaaact ggaataagcc 1260

tttgaaaaga aatttgtcct tgaagcttgt atctgatatc agcactggat cgtagaactt 1320

gttgctgatt tttgaccttg tattcaagtt aactgttccc ttggtatatg tttaataccc 1380

tgtgcatatc ttgatttagt ccttagttca tgtggctcgg tcacttggtg gctggggaga 1440

tctgtggaaa agtcagtcag tccccagcct agtggatctc tgtgagcacc atgtagtgat 1500

ctgtgcaggg tattaaccaa cagcagactt ccaggatttc ccgaggctgg caagggttcc 1560

tgaactagtt accacttctt ttcttgccag tctaacaggg tgggaaagtc cgagccttag 1620

gacccagttt ctgttctggt tttttccctc ctgacctcca tgggttgtta cttgccttga 1680

gttgggaacg tttgcatcga cacctgtaaa tgtattcatc cttttaattt atgtaaggtt 1740

tttgtactca attctttaag aaatgacaaa ttttggtttt ctactgttca gtgagaacat 1800

taggccccag caacacgtca ttgtgtaaag agaaataaaa gtgctgcagt aaaaaaaaaa 1860

aaaaaccccc cccccccccc 1880

<210> 580

<211> 6335

<212> DNA

<213> Rattus norvegicus (Norway rat)

<400> 580
ggatcccggc ccgcagcagc tctctgctcc cagaacccgt gagaaagaga cctcaccgcc 60

tggtcaggtg ggcactcctg aggctgcaga gcggaagaga ccaccaacac tgcccacccc 120

tgcccacatc cctggcccaa gaggaaactg tataaggcct ctgggttccg tgggggaggg 180

cccaggagcg tcaggacccc tgcctgagac accgccggaa cctgagggaa acagaccgga 240

taaacagttc tctgcaccca aatcccatgg gagggagagc tgaaccttca gagaggcaga 300

caagccttgg aaaccagaag agactgctct ctgtacatac atctcggacg ccagaggaaa 360

acaccaaagg ccatctggaa ccctggtgca ctgaagctcc tggaaggggc ggcacaggtc 420

ttcctggttg ctgccgccac agagagccct tgggcagcac cccgcctggt gaactcaaga 480

cacaggccca caggaacagc tgaagacctg cagagaggaa aaactacacg cccgaaagca 540

gaacactctg tccccataac ggactgaaag agaggaaaac aggtctacag cactcctgac 600

acacaggctt ataggacagt ctaaccactg tcagaaatag cagaacaaag taacactaga 660

gataatctga tggtgagagg caagcgcagg aacccaagca acagaaacca agactacatg 720

gcatcatcgg agcccaattc tcccaccaaa acaaacatgg aatatccaaa cacaccagaa 780

aagcaagatc tagtttcaaa atcatatttg atcatgatgc tggaggactt caagaaagac 840

gtgaagaact cccttagaga acaagtagaa gcctacagag aggaatcgca aaaatccctg 900

aaagaattcc aggaaaacac aatcaaacag ttgaaggaat taaaaatgga aatagaagca 960

atcaagaaag aacacatgga aacaaccctg gatatagaaa accaaaggaa gagacaagga 1020

gccgtagata caagcatcac caacagaata caagagatgg aagagagaat ctcaagagca 1080

gaagattcca tagaaatcat tgactcaact gtcaaagata atgtaaagcg gaaaaagcta 1140

ctggtccaaa acatacagga aatccaggac tcaatgagaa gatcaaacct aaggataata 1200

ggtatagaag agagtgaaga ctcccagctc aaaggaccag taaatgtctt caacaaaatc 1260

atagaagaaa acttccctaa cctaaaaaaa gagataccca taggcataca agaagcctac 1320

agaactccaa atagattgga ccagaaaaga aacacctccc gtcacataat agtcaaaaca 1380

ccaaacgcac aaaataaaga aagaatatta aaagcagtaa gggaaaaagg tcaagtaaca 1440

tataaaggca gacctatcag aatcacacca gacttctcgc cagaaactat gaaggccaga 1500

agatcctgga cagatgtcat acagacccta agagaacaca aatgccagcc caggttactg 1560

tatcctgcaa aactctcaat taacatagat ggagaaacca agatattcca tgacaaaacc 1620

aaatttacac aatatctttc tacaaatcca gcactacaaa ggataataaa tggtaaagcc 1680

caacataagg aggcaagcta taccctagaa gaagcaagaa actaatcgtc ttggcaacaa 1740

aacaaagcga atgaaagcac acaaacataa cctcacatcc aaatatgaat ataacgggaa 1800

gcaataatca ctattcctta atatctctca acataaatgg ccttaactcc ccaataaaaa 1860

gacatagatt aacaaactgg atacgcaacg aggaccctgc attctgctgc ctacaggaaa 1920

cacacctcag agacaaagac agacattacc tcagagtgaa aggctggaaa acaattttcc 1980

aagcaaatgg tcagaagaag caagctggag tagccattct aatatcaaat aaaatcaatt 2040

tttaactaaa agtcatcaaa aaagataagg aaggacactt catattcatc aaaggaaaaa 2100

tccaccaaga tgaactctca atcctaaata tctatgcccc aaatacaagg gcacctacat 2160

atgtaaaaga aaccttacta aagctcaaaa cacacattgc acctcacaca ataatagtgg 2220

gagatttcaa caccccactc tcatcaatgg acagatcatg gaaacagaaa ttaaacagag 2280

atgtagacag actaagagaa gtcatgagcc aaagggactt aacggatatt tatagaacat 2340

tctatcctaa agcaaaagga tataccttct tctcagctcc tcatggtact ttctccaaaa 2400

ttgaccatat aattggtcaa aaaacgggcc tcaacaggta cagaaagata gaaataatcc 2460

catgcatgct atcggaccac cacggcctaa aactggtctt caataacaat caaggaagaa 2520

tgcccatata tacttggaaa ctgaacaatg ctctactcaa tgataacctg gtcaaggaag 2580

aaataaagaa agaaattaaa aactttttag aatttaatga aaatgaaggt acaacatacc 2640

caaacttatg ggacacaatg aaagctgtgc taagaggaaa actcatagcg ctgagtgcct 2700

gcagaaagaa acaggaaaga gcatatgtca gcagcttgac agcacaccta aaagctctag 2760

aacaaaaaga agcaaataca cccaggagga gtagaaggca ggaaataatc aaactcagag 2820

ctgaaatcaa ccaagtagaa acaaaaggac catagaaaga atcaacagaa ccaaaagttg 2880

gttctttgag aaaatcaaca agatagataa acccttagcc agactaatga gaggacacag 2940

agagtgtgtc caaattaaca aaatcagaaa tgaaaaggga gacataacaa cagattcaga 3000

ggaaattcaa aaaatcatca gatcttacta taaaaaccta tattcaacaa aacttgaaaa 3060

tcttcaggaa atggacaatt ttctagacag ataccaggta ccgaagttaa atcaggaaca 3120

gataaaccag ttaaacaacc ccataactcc taaggaaata gaagcagtca ttaaaggtct 3180

cccaaccaaa aagagcccag gtccagacgg gtttagtgca gaattctatc aaaccttcat 3240

agaagacctc ataccaatat tatccaaact attccacaaa attgaaacag atggatcact 3300

accgaatacc ttctacgaag ccacaattac tcttatacct aaaccacaca aagacacaac 3360

aaagaaagag aacttcagac caatttccct tatgaatatc gacgcaaaaa tactcaacaa 3420

aattctggca aaccgaatcc aagagcacat caaaacaatc atccaccatg accaagtagg 3480

cttcatccca ggcatgcagg gatggtttaa tatacggaaa accatcaacg tgatccatta 3540

tataaacaaa ctgaaagaac aaaaccacat gatcatttca ttagacgctg agaaagcatt 3600

tgacaaaatt caacacccct tcatgataaa agtcctggaa agaattggaa ttcaaggccc 3660

atacctgaac atagtaaaag ccatatacag caaaccagtt gctaacatta aactaaatgg 3720

agagaaactt gaagcaatcc cactaaaatc agggactaga caaggctgcc cactctctcc 3780

ctacttattc aatatagttc ttgaagttct ggccagagca atcagacaac aaaaggaggt 3840

caaggggata cagatcggaa aagaagaagt caaaatatca ctatttgcag atgatatgat 3900

agtatattta agtgatccca aacattccac cagagaacta ctaaagctga tagacaactt 3960

cagcaaagtg gctaggtata aaattaactc aaataaatca gttgccttcc tctatacaaa 4020

agagaaacaa gccgagaaag aaattaggga aacgacaccc ttcataatag acccaaataa 4080

tataaagtac ctcggtgtga ctttaacaaa gcaagtaaaa gatctgtaca ataagaactt 4140

caagacactg aagaaggaaa ttgaagaaga cctcagaaga tggaaagatc tcccgtgctc 4200

atggattggc aggattaata tagtaaaaat ggccatttta ccaaaagcaa tctacagatt 4260

caatgcaatc cccatcaaaa taccaatcca attcttcaaa gagttagaca gaacaatttg 4320

caaattcatc tggaataaca aaaaacccag gatagctaaa gctatcctca acaataaaag 4380

gacttcaggg ggaatcacta tccctgaact caagcagtat tacagagcaa tagtgataaa 4440

aactgcatgg tattggtaca gagacagaca gatagaccaa tggaatagaa ttgaagaccc 4500

agaaatgaac ccacacacct atggtcactt gatttttgac aaaggagcca aaaccatcaa 4560

atggaaaaaa gatagcattt tcagcaaatg gtgctagttc aactggaggt caacatgtag 4620

aagaatgaag atcgatccat gcttgtcacc ctgtacaagc ttaagtccaa gtggatcaag 4680

gacctccaca tcaaaccaga cacactcaaa ctaatagaag aaaaactagg gaagcatctg 4740

gaacacatgg gcactggaaa aaatttccta aacaaaacac catggcttac gctctaagat 4800

caagaatcga caaatgggat ctcataaaac tgcaaagctt ctgtaaggca aaggacactg 4860

tggttaggac aaaacggcaa ccaacagatt gggaaaatat ctttaccaat cctacaacag 4920

atagaggcct tatatccaaa atatacaaag aactcaagaa gttagaccgc agggaaacaa 4980

ataaccctat taaaaaatgg ggttcagagc taaacaaaga attcacagct gaggaatgcc 5040

aaatggctga gaaacaccta aagaaatgtt caacatcttt agtcataagg gaaatgcaaa 5100

tcaaaacaac cgtgagattt cacctcacac cagtgagaat ggctatgatc aaaaactcag 5160

gggacaacag atgctggcga ggatgtggag aaagaggaac actcctccat tgttggtggg 5220

attgcaaact ggtacaacca ttctggaaat cagtctggag gttcctcaga aaattggaca 5280

ttgaactgcc tgaggatcca gctatacctc tcttgggcat atacccaaaa gatgccccaa 5340

catataaaaa agacacgtgc tccactatgt tcattgcagc cttatttata atagccagaa 5400

gctggaaaga acccagatgc ccttcaacag aggaatggat acagaaaatg tggtacatct 5460

acacaatgga atattactca gctatcaaaa acaacgagtt tatgaaattc gtaggcaaat 5520

ggttggaact ggaaaatatc atcctgagta agctaaccca atcacagaaa gacatacatg 5580

gtatgcactc attgataagt ggctattagc ccaaatgctt gaattaccct agatacctag 5640

aacaaatgaa actcaagacg gatgatcaaa atgtgaatgc ttcactcctt ctttaaaagg 5700

ggaacaagaa tacccttggc agggaagaga gaggcaaaga ttaaaacaga gaatgaagga 5760

acacccattc agagcctgcc ccacatgtgg cccatacata tacagccacc caattagaca 5820

agatggatga agcaaagaag tgcagaccga caggagccgg atgtagatcg ctcctgagag 5880

acacagccag aatacagcaa atacagaggc gaatgccagc agcaaaccac tgaactgaga 5940

ataggacccc cgttgaagga atcagagaaa gaactggaag atcttgaagg ggctcgagac 6000

cccatatgta caacaatgct aagcaaccag agcttccagg gactaagcca ctacctaaag 6060

actatacatg gactgaccct ggactctgac ctcataggta gcaatgaata tcctagtaag 6120

agcaccagtg gaaggagaag ccctgggtcc tgctaagact gaacccccag tgaactagac 6180

tggtgggggg agggcggcaa tgggggaggg gttgggaggg gaacaccata aggaagggga 6240

gggggagggg ggatgtttgc ccggataccg aaagggaata acatcgaaat gtatataaga 6300

atactcaagt taataaaaaa aaaaaaaaaa aaaga 6335

<210> 581

<211> 2326

<212> DNA

<213> Rattus norvegicus (Norway rat)

<400> 581
ctcagccaga cagtcctcac ctgcaacagg tggcctcagg agtcaggaac atctctactt 60

ccccaacgac ccctgggttg tcctctcaga gatggctatg gatactacaa ggtgtggagc 120

ccagttgttg actctggtcg agcagatcct ggcagagttc cagctgcagg aggaagacct 180

gaagaaggtg atgagccgga tgcagaagga gatggaccgt ggcctgaggc tggagaccca 240

cgaggaggcc agtgtaaaga tgttacccac ctacgtgcgt tccaccccag aaggctcaga 300

agtcggagac tttctctcct tagacctggg aggaaccaac ttcagagtga tgctggtcaa 360

agtgggagag ggggaggcag ggcagtggag cgtgaagaca aaacaccaga tgtactccat 420

ccccgaggac gccatgacgg gcactgccga gatgctcttt gactacatct ctgaatgcat 480

ctctgacttc cttgacaagc atcagatgaa gcacaagaaa ctgcccctgg gcttcacctt 540

ctccttccct gtgaggcacg aagacctaga caagggcatc ctcctcaatt ggaccaaggg 600

cttcaaggcc tctggagcag aagggaacaa catcgtagga cttctccgag atgctatcaa 660

gaggagaggg gactttgaga tggatgtggt ggcaatggtg aacgacacag tggccacaat 720

gatctcctgc tactatgaag accgccaatg tgaggtcggc atgattgtgg gcactggctg 780

caatgcctgc tacatggagg aaatgcagaa tgtggagctg gtggaagggg atgagggacg 840

catgtgcgtc aacacggagt ggggcgcctt cggggactcg ggcgagctgg atgagttcct 900

actggagtat gaccggatgg tggatgaaag ctcagcgaac cccggtcagc agctgtacga 960

gaagatcatc ggtgggaagt atatgggcga gctggtacga cttgtgctgc ttaagctggt 1020

ggacgagaac cttctgttcc acggagaggc ctcggagcag ctgcgcacgc gtggtgcttt 1080

tgagacccgt ttcgtgtcac aagtggagag cgactccggg gaccgaaagc agatccacaa 1140

catcctaagc actctggggc ttcgaccctc tgtcaccgac tgcgacattg tgcgccgtgc 1200

ctgtgaaagc gtgtccactc gcgccgccca tatgtgctcc gcaggactag ctggggtcat 1260

aaatcgcatg cgcgaaagcc gcagtgagga cgtgatgcgc atcactgtgg gcgtggatgg 1320

ctccgtgtac aagctgcacc cgagcttcaa ggagcggttt cacgccagtg tgcgcaggct 1380

gacacccaac tgcgaaatca ccttcatcga atcagaggag ggcagcggca ggggagccgc 1440

gctggtctct gcggtggcct gcaagaaggc ttgcatgctg gcccagtgaa atccaggtca 1500

tatggaccgg gacctgggtt ccacggggac tccacacacc acaaatgctc ccagcccacc 1560

ggggcaggag acctattctg ctgctacccc tggaaaatgg ggagaggccc ctgcaagccg 1620

agtcggccag tgggacagcc ctagggctct cagcctgggg cagggggctg ggaggaagaa 1680

gaggatcaga ggcgccaagg cctttcttgc tagaatcaac tacagaaaat ggcggaaaat 1740

actcaggact tgcactttca cgattcttgc ttcccaagcg tgggtctggc ctcccaaggg 1800

aatgcttcct ggaccttgca atggcctggc ttccctgggg gggacacacc ttcatgggga 1860

ggtaacttca gcagttcggc cagaccagac cccaggagag taagggctgc tagtcaccca 1920

gacctggctg ttttcttgtc tgtggctgaa gaggccgggg agccatgaga gactgactat 1980

ccggctacat ggagaggact ttccaggcat gaacatgcca gagactgttg ccttcatata 2040

cctccacccg agtggcttac agttctggga tgaaccctcc caggagatgc cagaggttag 2100

agccccagag tccttgctct aaggggacca gaaaggggag gcctcactct gcactattca 2160

agcaggaatc atctccaaca ctcaggtccc tgacccagga ggaagaagcc accctcagtg 2220

tccctccaag agaccaccca ggtccttctc tccctcgttc ccaaatgcca gcctctctac 2280

ctgggactgt gggggagttt ttaattaaat atttaaaact acttca 2326

<210> 582

<211> 1159

<212> DNA

<213> Rattus norvegicus (Norway rat)

<220>

<221> misc_feature

<222> (427)..(427)

<223> n

<220>

<221> misc_feature

<222> (429)..(429)

<223> n

<220>

<221> misc_feature

<222> (430)..(430)

<223> n

<220>

<221> misc_feature

<222> (431)..(431)

<223> n

<220>

<221> misc_feature

<222> (442)..(442)

<223> n

<220>

<221> misc_feature

<222> (443)..(443)

<223> n

<220>

<221>  misc_feature

<222>  (444)..(444)

<223>  n


<220>

<221>  misc_feature

<222>  (445)..(445)

<223>  n


<220>

<221>  misc_feature

<222>  (446)..(446)

<223>  n


<220>

<221>  misc_feature

<222>  (453)..(453)

<223>  n


<220>

<221>  misc_feature

<222>  (454)..(454)

<223>  n


<220>

<221>  misc_feature

<222>  (455)..(455)

<223>  n


<400>  582
ccgattaggt ccccaaaacg ggggacgtcc atggggatga acatgatttc caagggtacg      60

gagaaagcac ttctgaagct tcaagagttc tttcctgagc tgcagattct ggcggtcagt     120

ggtaactatt gcaccgacaa gaaacctgct gccataaact ggatcgaagg gagaggaaag     180

actgtggttt gtgaagctgt cattccagcc aaggtggtga gagaagtatt aaagagcact 240

acggaagcta tggttgacgt aaacattaat aagaatcttg tgggctctgc catggctggt 300

agcataggag gctacaacgc ccatgctgcc aacatcgtca ctgccatcta cattgcatgt 360

gcccaggatg cagcacagaa tgtggggagt tcaaactgta ttactttaat ggaagcaagt 420

ggtcccncnn ntgaagactt gnnnnncagc tgnnncatgc cgtctataga gatcggaacc 480

gtgggtggtg ggaccaacct tctacctcag caagcctgcc tgcagatgct aggtgttcaa 540

ggggcgtgca aagacaatcc tggagaaaat gcacggcagc ttgcccgaat tgtgtgtggc 600

actgtaatgg ctggtgagtt gtccttgatg gcagcattgg cagcaggaca tcttgtcaga 660

agtcacatgg ttcacaacag atcaaagata aatttacaag atctgcaggg aacatgcacc 720

aagaaggcag cttgagcatc ctgacatact tgaactgaaa cacgggcatt gggttctcaa 780

ggtctaacat gaaatctgtg aattaaaaat gtcagtgcag tgtcttgtgg aagatgaacg 840

tgatcagtga gcctgcttgg tttctggctc tttcagagac gtctgcggtc ctttgcacca 900

gactcctcag acgtgggaac tatggttctt tccgtgccgt attctagaaa gatctcatgt 960

ggatgtcatg gtgctctgag caccacagat gtgactgcag ctcgtttcta aaagctgcca 1020

caagctggaa gctggtgttt tgacgaaatg atggatcttg gtgatcagtg tggggctcac 1080

ctccaatggg ttaaaatgga gttttaaatg acactgtagc tgacagaact ctcgattttt 1140

atttattcag tctgggcgg 1159

<210> 583

<211> 922

<212> DNA

<213> Rattus rattus (black rat)

<400> 583
tctttttctt cgggaaaaca ccaaatggcg gatgacgccg gtgcagcggg agggcccgga 60

ggacccgggg gcccaggatt aggaggccgc ggcggcttcc gcggaggatt cggcagcggc 120

cttaggggcc gcggtcgtgg ccgaggccgt ggccgcggtc gaggccgcgg ggctcgtgga 180

ggtaaagctg aagacaagga gtggatcccc gttaccaagc tgggccgcct ggttaaggac 240

atgaagatca agtccttgga ggagatctac ctgttctccc tgcccattaa ggagtctgag 300

attattgact ttttcctggg tgcatctcta aaggatgaag ttctgaaaat catgccagtg 360

cagaagcaga ctcgggctgg ccagcggacc aggttcaagg cctttgtcgc tattggggac 420

tacaatggcc acgttggtct tggtgttaag tgctccaagg aggtagccac tgccatccga 480

ggggccatca tcctggccaa gctttcgatc gtcccagtgc ggagaggcta ctgggggaac 540

aagattggca agccccacac tgtgccatgc aaggtgacag gccgctgtgg ctctgtgctg 600

gttcgtctca tccctgcccc cagaggcact ggcattgtct ctgctcctgt gcccaagaag 660

ctactgatga tggccggtat agatgactgc tacacttcag ccaggggctg cactgccacc 720

ctgggcaact ttgccaaggc cacctttgat gccatctcca agacctacag ctacctgacc 780

cctgacctct ggaaagagac tgtgttcacc aagtctcctt atcaggaatt cactgaccat 840

cttgtgaaaa cccacaccag agtctctgtt cagaggaccc aggctccagc tgtggctacc 900

acataagggt ttttatacaa ga 922

<210> 584

<211> 2674

<212> DNA

<213> Rattus norvegicus (Norway rat)

<220>

<221> misc_feature

<222> (2397)..(2397)

<223> n

<400> 584
cgcggagagt tccaggctgg gaccggactc tggacagcgc acgctcgatg gctgcgcacg 60

cctggccgac ggccgccttg ctgctgctgc tggtggactg gctgctgctg cggcccgtgc 120

tcccgggaat cttctccctg ttggttcccg aggtgccact gctccgggtc tgggccgtgg 180

gcctgagtcg ctgggctatc ctgggactag gggtccgcgg ggtcctcggg gtcaccgcgg 240

gagcccgtgg ctggctggct gctttgcagc cgctggtggc ggcgctgggt ttggccctgc 300

ctggacttgc ctcgttccga aagctgtccg cctggggagc actccgggag ggtgacaacg 360

ctggactgct ccactggaac agtcgcttag atgccttcgt tctcagttat gtggccgcat 420

tgcccgcagc tgccctgtgg cacaagttgg ggggcttctg ggcgcccagt ggccacaagg 480

gcgctggaga catgctgtgt cggatgctag gcttcctgga ctccaagaag gggcgtctcc 540

acctggttct ggttctcttg atcctctcct gccttgggga aatggccatt cccttcttca 600

caggccgcat cactgactgg atccttcagg ataagacagc ccccagcttc gcccgcaaca 660

tgtggctcat gtgtattctt accatagcca gtacagtgct ggagtttgca ggagatggaa 720

tctacaacat caccatgggc cacatgcaca gccgcgtgca tggagaggtg tttcgggccg 780

tccttcacca ggagacagga tttttcctga agaacccaac aggttccatc acatctcggg 840

tgactgagga cacctccaac gtgtgcgagt ccattagtga caagctgaac ctgttcctgt 900

ggtacctggg gcgaggcctg tgtctcctgg cgttcatgat ttgggggtca ttctacctca 960

ctgtggtcac cctgctcagc ctgcctctgc ttttccttct gcccaggagg ctggggaaag 1020

tgtaccagtc actggcagtg aaggtgcagg agtctctagc aaagtccacg caggtggccc 1080

tcgaggccct gtcggcgatg cctaccgtac ggagctttgc caacgaggag ggagaggccc 1140

agaagtttag gcagaagttg gaagaaatga agccgctaaa caagaaagag gccttggctt 1200

acgtcactga agtctggacc atgagtgtct cgggaatgct gctgaaggtg ggaattctgt 1260

acctcggtgg gcagctggtg gtcagagggg ctgtcagcag cggcaacctc gtctcctttg 1320

ttctctacca gcttcagttc accagggccg tggaggtcct gctctccatc tatccctcca 1380

tgcagaagtc cgtgggcgct tcggagaaaa tattcgaata cctggaccgg actccctgct 1440

ctccgctcag tggctcactg gcacctttaa acatgaaagg cctcgtcaag ttccaagatg 1500

tctcctttgc ctacccaaac catcccaacg tccaggtgct tcaggggctg acttttacgc 1560

tgtatcccgg gaaggtgacc gccttggtgg gacccaatgg gtcagggaag agcaccgtgg 1620

ccgccctgct gcagaacctg taccagccca ccgggggcaa ggtgctcctg gatggcgagc 1680

ccctggtcca gtatgatcac cactacctgc acacgcaggt ggccgcagtg ggacaagagc 1740

cactgctatt tggaagaagt tttcgggaaa atattgccta tggcctgacg cggactccaa 1800

ccatggagga aatcacagct gtggccatgg agtccggagc ccacgatttc atctctggat 1860

tccctcaggg ctatgacaca gaggtaggtg aaactgggaa ccagctgtca ggaggtcagc 1920

gacaggcggt ggccttggct cgagccttga tccggaagcc acgcctgctt atcttggacg 1980

atgccaccag tgccctggat gctggcaacc agctacgggt ccagcggctc ctgtatgaga 2040

gccccgagtg ggcctctcgg acggttcttc tgatcaccca gcagctcagc ctggcagagc 2100

gggcccacca catcctcttc ctcaaagaag gctctgtctg cgagcagggc acccacctgc 2160

agctcatgga gagaggaggg tgttaccggt ccatggtgga ggctcttgcg gctccttcag 2220

actgacgggc ttctggactg caagctgcgc gagtccctcc ccctgctgtc ctctgctctg 2280

tgtggcggag aacctgggag caaagatttt accacatcca cggagatagt tgaggagcga 2340

tggtgtttgt tacatgagga aaatgtaacc tctaggagat gcccggaatt taccacnaat 2400

gttttcccgc cccgccccct gttagacggg ggatgggggt aggtacccca ggctaacact 2460

gagctgctga gtctcctgtc tcccgtggag tttgcatcac ggcatgcgcc cacaaacctg 2520

gcttatgtgg cgttgggaca gaatgagaag aaacgctcaa aatgtacaga gaaggggcaa 2580

atagcttgca attaaccaaa ggcataggct ggcctatggg tgttccgcgg gttcttgata 2640

tttataataa aactggtgtt ttgtaaaaaa aaaa 2674

<210> 585

<211> 318

<212> DNA

<213> Rattus norvegicus (rat)

<220>

<221>  misc_feature

<222>  (99)..(99)

<223>  n


<400>  585
agcaaagatt ttaccacatc cacggagata gttgaggagc gatggtgttt gttacatgag      60

gaaaatgtaa cctctaggag atgcccggaa tttaccacna atgttttccc gccccgcccc     120

ctgttagacg ggggatgggg gtaggtaccc caggctaaca ctgagctgct gagtctcctg     180

tctcccgtgg agtttgcatc acggcatgcg cccacaaacc tggcttatgt ggcgttggga     240

cagaatgaga agaaacgctc aaaatgtaca gagaaggggc aaatagcttg caattaacca     300

aaggcatagg ctggccta                                                   318

<210>  586

<211>  744

<212>  DNA

<213>  Rattus rattus (black rat)


<400>  586
ctcttcctgt ctgtgccaga actgcgcgtg gtccgcgccg atcgactgag aagcccggtt      60

tgcgctctca gaatgactga atgggaaaca gccacacccg cggtggcaga gaccccggac     120

atcaagctct ttgggaaatg gagcactgat gatgtgcaga tcaacgatat ttctctacag     180

gattacattg ctgtgaagga gaagtatgcc aagtacctgc cccacagtgc aggacggtat     240

gctgccaacg gtttccgcaa agcacagtgt cccatcgtgg agcgccttac taactccatg     300

atgatgcacg gtcgtaacaa cggcaagaag ctcatgactg tacgaattgt caagcatgcc     360

tttgagatca tccacctgct cactggtgag aaccctctgc aggtcctggt gaatgctatc     420

atcaacagtg gcccccgaga agactcaaca cgcattgggc gggctggaac agtgagacgg     480

caggctgtgg atgtatcccc acttcgccga gtgaatcagg ccatctggct gctgtgcacg     540

ggggctcgtg aggctgcttt ccggaacatc aagaccatcg ctgagtgcct tgcagatgag     600

ctcattaatg ctcgcaaggg ctcctccaac tcctatgcta tcaagaagaa agatgaactg     660

gagcgtgtgg ccaagtctaa ccgctgattt cccagctgct gcctaataaa ttgtgtgccc     720

tttgggacag ttacatcaaa aaaa                                            744

<210>  587

<211>  531

<212>  DNA

<213>  Rattus norvegicus (rat)

<400> 587
atgatgtgca gatcaacgat atttctctac aggattacat tgctgtgaag gagaagtatg 60

ccaagtacct gccccacagt gcaggacggt atgctgccaa cggtttccgc aaagcacagt 120

gtcccatcgt ggagcgcctt actaactcca tgatgatgca cggtcgtaac aacggcaaga 180

agctcatgac tgtacgaatt gtcaagcatg cctttgagat catccacctg ctcactggtg 240

agaaccctct gcaggtcctg gtgaatgcta tcatcaacag tggcccccga gaagactcaa 300

cacgcattgg gcgggctgga acagtgagac ggcaggctgt ggatgtatcc ccacttcgcc 360

gagtgaatca ggccatctgg ctgctgtgca cgggggctcg tgaggctgct ttccggaaca 420

tcaagaccat cgctgagtgc cttgcagatg agctcattaa tgctcgcaag ggctcctcca 480

actcctatgc tatcaagaag aaagatgaac tggagcgtgt ggccaagtct a 531

<210> 588

<211> 1547

<212> DNA

<213> Rattus norvegicus (Norway rat)

<400> 588
gcacaagttt cctgggctgg accatttgag cagagaggat tatttgactt catgctagaa 60

gatgaggcct ctggcataat cccttacgac cctgacaatc ccctgatatc tatgtgcccc 120

taccgatgcc aatgccatct ccgagtggtg cagtgttctg atctgggtct ggacaaagta 180

ccctgggaat ttccacctga cacaacattg ctagacctgc aaaacaacaa aataacagag 240

atcaaagagg gggcctttaa gaacctgaag gacttgcata ccttgatcct tgtcaacaac 300

aagatcagca agatcagccc agaggcattt aaacctctag tgaagttgga aaggctttat 360

ctgtctaaga accacctaaa ggagctgccc gaaaaattgc ccaaaacact ccaggagctt 420

cgactccacg acaatgagat caccaaactg aagaaatctg tgttcaatgg actgaaccgt 480

atgattgtca tagaactggg cggcaaccca ctgaaaaact ctgggattga aaatggagcc 540

ttgcagggaa tgaagggtct cggatacatc cgcatctcag acaccaacat aactgctatt 600

cctcaaggtc tgcccacttc tatcagtgaa ctgcatctgg atggcaacaa gatagccaaa 660

gttgatgcag ccagcctgaa aggaatgtct aatttgtcta agctgggttt gagcttcaat 720

agcatcaccg ttgtggaaaa tggcagtctg gctaatgttc ctcatctgag ggagctccac 780

ttggacaaca acaaactcct cagagtgcct gctgggctgg cacagcataa atatgtccag 840

gtcgtctacc ttcataacaa caacatctcc gaagttgggc agcatgactt ctgcctccct 900

tcataccaga ctaggaagac ttcctacact gccgtgagtc tttatagcaa ccctgtccgg 960

tattggcaaa ttcacccaca caccttcaga tgtgtcttcg ggcgctctac cattcaactt 1020

gggaactaca agtaactccc aaacagcctc atttttataa tcgggaacaa aaaaaccaat 1080

ctgtcaatat tattgctaaa aagaaaaaaa tattttgaaa aagaaagaat gctagattct 1140

ggaaattcaa gtatagcgcg gatgcctttc ccgtatgact tatcatgcat caagccaaat 1200

atacagattg aataattacc tacaataaaa atattttatc tgtagtattc agaatcactt 1260

ttaaaatttt ctgtagatgt gaactgaggt atcgaagtct gatgtaatca tagatgtcaa 1320

catttaataa agctaaaagg aacagaatac aaagtcttct gtaattcata gagctaaatt 1380

gactcttttg acataaagtc aaatgtagaa ctctagcaat gtattaatct cctttattat 1440

tggtaaagtc ttatttgaat atctaagttc aacatgggct atgtaaaatt ttcactgtca 1500

tactgaaaaa caataaaatt taaaaaatgc attcaaaaaa aaaaaaa 1547

<210> 589

<211> 631

<212> DNA

<213> Rattus norvegicus (Norway rat)

<400> 589
ctgttagcgg ccagaggtga cctgtgaaga tggttcgcta ctcccttgac ccagaaaacc 60

ccacgaaatc atgcaagtca agaggctcaa accttcgtgt tcactttaag aacacccggg 120

aaactgccca ggccatcaag ggtatgcata tccgcaaagc caccaagtat ctgaaggatg 180

tcactttaaa gaagcagtgt gtgccattcc ggcggtataa cggtggagtt ggtaggtgcg 240

cccaggccaa acagtggggc tggacacagg gacggtggcc aaaaaagagt gctgaatttt 300

tgctgcacat gcttaaaaat gcagagagta atgctgaact taagggtttg gatgtagact 360

ctctggtcat tgaacacatc caggtgaaca aggctcctaa gatgcgcaga cggacctaca 420

gagctcacgg ccggattaac ccatacatga gctccccctg ccacatcgaa atgatcctca 480

ctgagaagga acagattgtt ccaaagccag aagaggaggt tgcacagaag aaaaagatat 540

cccagaagaa attgaagaaa caaaagctca tggcacggga ataaattcag cataaataaa 600

tgcggataaa gtaaaaaaaa aaaaaaaaaa a 631

<210> 590

<211> 1194

<212> DNA

<213> Rattus norvegicus (Norway rat)

<400> 590
ttcgccgcgc ccgcgcccgc gcaccacgac ttcctttccg acctcttcgc cgacgactac 60

ggcgccaagc cgagcaagaa gccgtccgac tacggttacg tgagcctcgg ccgcgcgggc 120

gccaaggccg caccgcccgc ctgcttcccg ccgccgcctc ccgccgcact caaggccgag 180

ccgggcttcg aacccgcgga ctgcaagcgc gcggacgacg cgcccgccat ggcggccggc 240

ttcccgttcg ccctgcgcgc ctacctgggc taccaggcga cgccgagcgg cagcagcggc 300

agcctgtcca cgtcgtcgtc gtccagcccg cccgggacgc cgagccccgc cgacgccaag 360

gccgcgcccg ccgcctgctt cgcggggccg ccggccgcgc ccgccaaggc caaggccaag 420

aaggcggtgg acaagctgag cgacgagtac aagatgcggc gcgagcgcaa caacatcgcg 480

gtgcgcaaga gccgcgacaa ggccaagatg cgcaacctgg agacgcagca caaggtgctg 540

gagctgacgg cggagaacga gcggctgcag aagaaggtgg agcagctgtc gcgagagctc 600

agcacgctgc ggaacttgtt caagcagctg cccgagccgc tgctggcctc ggcgggtcac 660

tgctagcccg gcgggggtgg cgtgggggcg ccgcggccac cctgggcacc gtgcgccctg 720

ccccgcgcgc tccgtccccg cgcgcgcccg gggcaccgtg cgtgcaccgc gcgcacctgc 780

acctgcaccg aggggacacc gtgggcaccg cgcgcacgca cctgcaccgc gcaccgggtt 840

tcgggacttg atgcaatccg gatcaaacgt ggctgagcgc gtgtggacac gggactgacg 900

caacacacgt gtaactgtca gccgggccct gagtaatcac ttaaagatgt tcctgcgggg 960

ttgttgctgt tgatgttttt gtttttgttt tttgtttttt gttttttttt tggtcttatt 1020

atttttttgt attatataaa aaagttctat ttctatgaga aaagaggcgt atgtatattt 1080

tgagaacctt ttccgtttcg agcattaaag tgaagacatt ttaataaact tttttggaga 1140

atgtttaaaa aaaaaaaaaa aaaaaaaaaa aaaaaaaaaa aaaaaaaaaa aaaa 1194

<210> 591

<211> 1632

<212> DNA

<213> Rattus norvegicus (Norway rat)

<400> 591
gaattaacat tgatggagaa accaagatat tccatgacaa aaccaaattt acacaatatc 60

tttctacaaa tccagcacta caaaggataa taaatggtaa agcccaacat aaggaggcaa 120

gctataccct agaggaagca agaaactaat cgtcttggca acaaaacaaa gagaatgaaa 180

gcacacaaac ataacctcac atccaaatat gaatataaag ggagcaataa tcactattcc 240

ttaatatctc tcaatatcaa tggcctcaac tccccaataa aaagacatag attaacaaac 300

tggatacgca atgaggaccc tgcattctgc tgcctacagg aaacacacct cagagacaaa 360

gacagacatt acctcagagt gaaaggctgg aaaacaactt tccaagcaaa tggtcagaag 420

aagcaagctg gagtagccat tctaatatca aataaaatca atttccaact aaaagtcatc 480

aaaaaagata aggaaggaca cttcatattc atcaaaggaa aaatccacca agatgaactc 540

tcaatcctaa atatctatgc cccaaataca agggcaccta catacgtaaa agaaacctta 600

ctaaagctca aaacacacat tgcacctcac acaataatag tgggagactt caacacccca 660

ctctcatcaa tggacagatc atggaaacag aaattaaaca gtgatgtcga cagactaaga 720

gaagtcatga gccaaatgga cttaacggat atttatagaa cattctatcc taaagcaaaa 780

ggatatacct tcttctcagc tcctcatggt actttctcca aaattgacca tataattggt 840

caaaaaacgg gcctcaacag gtacagaaag atagagataa tcccatgcat gctatcggac 900

caccacggcc taaaactggt cttcaataac aataagggaa gaatgcccac atatacgtgg 960

aaattgaaca atgctctact caatgataac ctggtcaagg aagaaataaa gaaagaaaat 1020

aaaaagtttt tagaatttaa tgaaaatgaa gatacaacat acccaaactt atgggacaca 1080

atgaaagctg tgctaagagg aaaactcata gcgctgagtg cctgcagaaa gaaacaggaa 1140

agagcatatg tcagcagctt gacagcacac ctaaaagctc tagaacaaaa agaatcaaat 1200

acgcccagga ggagtagaag gcaggaaata atcaaactca gagctgaaat caaccaagta 1260

gaaacaaaaa ggaccataga aagaatcaac agaaccaaaa gttggttctt tgagaaaatc 1320

aacaagatag ataaaccctt agccagacta acgagaggac acagagagtg cgtccaaatt 1380

aacaaaatga gaaatgaaaa gggagacata actacagatt cagaggaaat tcaaaaaatc 1440

atcagatctt actataaaaa cctatattca acaaaatctg aaaatcttca ggaaatggac 1500

aatttcctag acagatacca ggtatcgagg ttaaatcagg aacagataaa ccagttaaac 1560

aaccccataa ctcctaagga aatagcagca gtcactaaag gtctcccaac caaaaagagc 1620

ccaggtccag ac 1632

<210> 592

<211> 2073

<212> DNA

<213> Rattus norvegicus (Norway rat)

<400> 592
ccaaaaaaag cccgctcatt aggcggcgtg gcctcgatgg ccatttaggt gacactatag 60

aagggacgac actcgaggga tcttgtgcac ttttctcctc tggtttacaa ccgtcatctt 120

tctctgactt tctttgtttc ttccccgttg ctttagtctt tgcagaagac gtgctcggtt 180

ggctagacat ctggcattcc acttcttccc ttggctcctc tgcacgcccc ttcttgcttc 240

cagacggctc catcttgaat ttagatgaga cctccgaaag aaagttctcc taagcttccc 300

ttccacagga gtcttagagc ttccctgatg tttgactgtg gctctctgta tgtcagttcc 360

cccacctctc tctgtgtgga atgaatttcg cccccctctt atcccacccc cgtaaacctc 420

agttcttccc tgcaaatctt cttcccacat tcacgacttg tttcatgtcc taaggacatt 480

atctagtgcc atgtgcgtga ctgtgagttt ggaagcctgc gggtcaccag tgggttcaca 540

actgagtaca attcctcctt ctctcattag gcagcaatga gtgtcaggcc ctgtctcttt 600

ctcactggca ccaaagggtg gcaggtgttg aattttggtg gatagatata gtagaggaat 660

atttctttgt gagtctgtgt cttccatatc ccaggcttgt ctcaaacact ctatgttgct 720

ttgaactttg tctttgaact tctgatgccc ctgcctctgc tctgtaagtg caggattgca 780

gaagtctatt acttatgata gtacactggg gatagagcca gagttctgtg actgctaagc 840

agaatgtctc tccaccaact gatccttatc attactccta gcgtttcttt ttcttttttc 900

acaaagaagc actcatcatg agtgctggac agtgcctcag gggttgagtg ggcttttgca 960

cttctggaga actggtgctt gattcctaga cattacagtg gagatatagg tccttgagca 1020

gctccagtat ttttcagtga aggagaaagt ccttctgtaa tggtgtgcag atgacacagt 1080

tttggcttaa tttcatgaag atttgaatga agtcagcctt ggtgagagat tattttatat 1140

tatcgaattt gcttgaattt ctgatagaaa caatctaaga aactttggtc ttacttatat 1200

ggcactgttg ttcactgacg ccacccacaa tgttaactca aaggaggtca aaataggctg 1260

tagatgaact tcatgaactg tctaggggaa gagagtacca aagtccacgt gaggcagagg 1320

tgatccatct actggctgga tcctggtgcc acccatcttt ccagcatata agtactgcag 1380

gctggcctca gtgcagcggt gtgggcagaa aggaagccct gcagagcatc agaggcccag 1440

ctagagggac aacacagagg agtaatttgc tgacagacct gcagggatgg acctgctttc 1500

agctctcaca ctggaaacct gggtcctcct ggcagtcgtc ctggtgctcc tctacgggtg 1560

agtgacttca gaagttcccc ctgcgaccag gttcttgggt ttgaagttat attcagacct 1620

gaactttttt attattgggt ggctcaaact ggtaccagaa tcaggctgga gtgatgatgg 1680

ctccgtgttt aaggccagtg gctgctcttc agaggatcac tatttgaacc ccaacatcca 1740

catgcaactc acagtcatct gtaactatgg ctaaaggtga tctgacacct tccacaggca 1800

agctaggatg ggactgaaag gcggcagttt tctaatctgg ctaactccta aataattaaa 1860

agagaggact attaagttta ttagcaagct ttaaggacta tgacggggca ggttctaagc 1920

tattcttacc cacaaagctc tctcaaccat agtccccaaa tacttgcagt gtgacttttt 1980

ccctgggttg ctctgttccg tgtgtgtcct catggtaagc tcccttggtg aatccttctc 2040

atgcctgctt ttgtggtgtc cttgttcttc ctc 2073

<210> 593

<211> 843

<212> DNA

<213> Rattus rattus (black rat)

<400> 593
ctcttttggc cttgcttgcc ggcagactcg ccgccatggg ccgtgtgatc cgaggccaga 60

ggaaaggtgc cggttctgtg tttcgtgcgc acgtgaagca ccgtaaggga gccgcgcgtc 120

tgcgtgctgt ggacttcgcg gagcgacacg gctacattaa aggcatcgta aaggacatca 180

ttcatgaccc tggccgcggc gctcccctcg cgaaagtagt ctttcgtgat ccctatcgat 240

tcaagaagcg gacagagctg ttcattgccg cagagggaat ccacactgga cagtttgtgt 300

actgcggcaa gaaggcccag ctgaatattg gcaatgtttt gcccgtgggc accatgcctg 360

agggtactat cgtgtgttgt ctggaggaga agcctgggga caggggcaag ctggcacgag 420

cctccgggaa ctatgctaca gtcatctccc acaacccaga gaccaagaag acccgagtga 480

agctgccttc agggtccaag aaggtcattt cctctgctaa ccgagctgtt gttggtgtcg 540

tggctggcgg gggcagaatt gacaagccta tcttaaaggc tggccgtgcc taccataagt 600

acaaggcaaa gaggaactgc tggccacgtg tgcggggtgt tgccatgaat cctgtggagc 660

atccctttgg cggtggtaac caccagcaca ttggcaagcc ttccactatc cgaagagatg 720

ccccagctgg gcgcaaagtg ggtctcattg ctgctcgccg gactggacgg ctacgtggaa 780

ccaaaactgt acaggagaag gagaactaga gttcaggagc taataaagta tgtgctttgg 840

cta 843

<210> 594

<211> 3219

<212> DNA

<213> Rattus rattus (black rat)

<400> 594
agggactgga tgatgctgtc ccgctggaga ggattcgtgg cttgagtcct gttccacccg 60

atcaccgatc accggggtgg ccgaggccgg taccagcgct gcggctctct cagctctgcc 120

acattccctg agttaacata tgagagattg gtataaaatg gctggcattt tgtctggata 180

aatcacacaa ccctcccaga aagtgatcac caggtaaagg tcaagtggat ttcgaatagt 240

gtctgagacc aactcaaccc agaaaaacaa aatggccctt aactcttctg ctgaagatgg 300

tatcaaaaga atccaagatg actgccccaa ggctggcagg cacagttaca tatttgtcat 360

gatccctacc ctctacagca tcatctttgt ggtgggaata tttggaaaca gcttggtggt 420

gattgtcatt tacttttaca tgaagctgaa gactgtggcc agcgtctttc ttctcaatct 480

cgccttggct gacttatgct ttttgctgac tttgcccctg tgggcagtct ataccgctat 540

ggagtaccgc tggcccttcg gcaatcacct atgtaagatc gcttcggcca gcgtgagctt 600

caacctctac gccagtgtgt tccttctcac gtgtctcagc atcgaccgct acctggccat 660

cgtccaccca atgaagtctc gccttcgccg cacgatgctg gtggccaaag tcacctgcat 720

catcatctgg ctgatggctg gcttggccag tttgccagct gtcatccacc gaaatgtata 780

cttcatcgag aacaccaata tcacagtgtg cgcgtttcat tatgagtctc ggaattcgac 840

gctccccata gggctgggcc ttaccaagaa tattctgggc ttcttgttcc ctttccttat 900

cattctcacc agctataccc ttatttggaa agctctaaag aaggcttatg aaattcaaaa 960

gaacaaacca agaaacgatg acatctttag gataattatg gcgattgtgc ttttcttctt 1020

cttttcctgg gtcccccacc aaatattcac tttcctggat gtgctgattc agctgggcgt 1080
catccatgac tgtaaaattt ctgacatcgt ggacactgcc atgcccatca ccatctgcat 1140
agcgtatttt aacaactgcc tgaaccctct gttctacggc tttctgggga agaaatttaa 1200
aaagtatttc ctccagctcc tgaaatatat tcccccaaag gccaagtccc actcaagcct 1260
gtctacgaaa atgagcacgc tttcttaccg gccttcggat aacatgagct catcggccaa 1320
aaagcctgcg tcttgttttg aggtggagtg acaggttcaa agcacactgg caatgtaatg 1380
ccctgacaga agccagggca gcctctgact aaatggctta cgaccaaagg accattcacc 1440
ctgcctcagg atctaagcag aaaaatgcgc tcatcagact gtagataatg actaaaactc 1500
tgagagaaga ctttgaagga gtaaccaagc aaagccgtct tgcattaata gatgatggct 1560
agccaaagga agagtcagga gctggatgga ttggtgggtc tggaaaacag ttcgctggca 1620
gaaatgcaat ctcatcagtc tccctttgct atgttccttt tgatttccac acgtaggtat 1680
gtggcccatg ctaaatattt aggcaagaaa caagagatgg aaatcaaggt cacttgttct 1740
gttcaactcc caaagggcaa ggaacctttg ttgtccctgt agtcattagc agtggagacc 1800
cacttgtcac tgttactaca cctatcgtgc aaagatttgc taatctgtag ttatcaagtt 1860
tcaggactcc tttgtaaaat tcagccagtg ttttagaaac atgtactgtc cccagacaat 1920
gctagccagt ggtgtactta tgtagcagtg ttacccaagt ccacacatca aagtcacact 1980
gctttaataa cgtgacatac taggtacatc tgcgttattt cgcatctgag aaatgtacac 2040
agtcagaggt gaatacacta tattccatac agtctgcctt gctctctaaa aatatatatt 2100
ctccacatac gtgaacatct ttatctctaa actgtcattt gatgaaattt tggcaatgta 2160
tgtttacctc taaataaagt aattttattg taatgtaaaa aaaaaaaaaa aaagaggcat 2220
ctaggtctca gccacctaac ttcctgcctg cactcttctc aacagtctag ggactctggg 2280
cagtgctggc cgcctgggtt ctcaccagca ccacggacgt gctgtcgaag atggtctgta 2340
ggtacacgag gatgggagtg atgcctgtca gctgctgcag aaaacgcatc agcactgtaa 2400
tgagaatagg ccggtacaca cggggctccc aggcctccgc ccacgacact cggctactct 2460
ggaacacaaa gcgtctgttg agggctatgc aggctctcca gccctggcca ccctgtcttg 2520
gcacctgcgg gtcgaggaga gacctccttc cctcatccaa gagaggccca accaccagag 2580
gctgcatggg ctctgcatgc tagcaggaac ctctcctttg aggaaaggca gccaaagcac 2640
agagaggcct ggtggatgtc caaggctacc cagagcttcc tgggttgggg cccgaccagg 2700
cgggtaccac acatgcttca ccgtctccgc acgttgtcct ggatctgctc aaactcccag 2760
tggacctcag agtccgctcg cagccagatc agcgcctgca gtgcttcctc atcccggctc 2820
ttagacagta ggaagcgagg cgagttgggc atgaagctaa gcagcaggat catgaccaga 2880
acaggcccct ccccggccac gcaagccatg cccaaggcag cagaagacct ggtgagagag 2940
gcttcggtga gcagatgggc cctctgttgc tctcccagcc tagcttcccg actgcccaag 3000
tccctgtagg aggggcaatg tgggacttta agaattgcct ggggctggag agatggctca 3060

gcgattaaga gcacccgact actcttccag aggtcctgag ttcaattccc agcaaccaca 3120

tggtggctca caaccatctg taaagagatc cgataccctc ttctggtgtg tctgaagaca 3180

gctacagtgt acttatatgt aataaatgaa taaatcttt 3219

<210> 595

<211> 785

<212> DNA

<213> Rattus norvegicus (Norway rat)

<400> 595
gccccgatgg ctgacctggc ggcagcagtg tggtcagtgc aggggaagct taggtcccag 60

catgtatgtc ccccatcaag cgtcaccggg tgggtttctc agtccctcct gccccaccct 120

actgagaatc ctgcaccagg ttggctgagc cccttggctg gacaacagga agtaggatgg 180

tgttggctcc acccactttg ctgctcttcc ctgggacaag acagatgggc gctaccaccc 240

atctcgagtg aggtcctgag cacttccacc taaggcagac ggggctccaa caggcgcctc 300

ccccactaca ctcttcctct agcagggcac caaggagctc gccttgcccc cctctccttt 360

tgctcctccc tgcccccaag gttctggttc cactttttcc tctgttcaca aactacctct 420

ggacagttgt gttgtgtttt ttgttcaaat gttcattctt agacacccac cattgctgct 480

accagcgcca gatgtccatc ctcccgattt tgcctgtacc ccccacacaa tacactctag 540

gggaaggggg ggctggtgtg cagcagcagg ctggttaccg agtaccccac gctagggaag 600

atggccccgc cccgtggatg ctgcagcaga gtgaggaggg ggggcccact tggatgggaa 660

aagggtatag gaccacctcc ccttgttgtg ggggaggggt ggccagtatt tgtcccagcc 720

tggggctccc cctctggttt cctatttgca gttacttgaa taaaaaaaaa tagccttttc 780

tggac 785

<210> 596

<211> 1339

<212> DNA

<213> Rattus rattus (black rat)

<400> 596
ggtgacagag accagaccag acacacacac ctttccagcg ccagcgctgt ggatggggag 60

ctgccaccag gcttgaaaga agcccagagc tggcgactgc acacggtgca ttttaaatag 120

tgtcagagag caattcacgt cgccaaggga gacatgaccc ttaactcctc tactgaagat 180

ggaattaaaa gaatccaaga tgactgcccc aaggctggca ggcacaatta catatttgtc 240

atgatcccta ctctctacag catcatcttt gtggtgggaa tatttggaaa cagcttggtg 300

gtgattgtca tttactttta catgaagcta aagactgtgg ccagtgtttt tcttctgaat 360

cttgccctgg ctgatttatg ctttttgttg actttgccac tgtgggcagt ctataccgct 420

atggaatacc gatggccctt cggcaaccac ctatgtaaga tcgcttctgc cagcgtcagt 480

ttcaatctct atgccagtgt gtttctgctc acgtgtctca gcatcgatcg ctacctggcc 540

attgtccacc caatgaagtc tcgcctccgc cgcacgatgc tggtagccaa agtcacctgc 600

atcatcatct ggctaatggc tggcttggcc agtttgccag ccgtcatcta ccgaaacgta 660

tatttcatcg agaacaccaa tatcacagtt tgcgcttttc attatgaatc tcagaactca 720

acactcccca ttggactggg tctaacaaag aacattctgg gcttcgtgtt ccctttcctt 780

atcattctca ccagctatac tcttatttgg aaagccctaa agaaggctta taaaattcag 840

aagaacacgc caagaaatga tgacatcttt aggataatta tggcgattgt gcttttcttc 900

ttcttttcct gggttcccca ccaaatattc acttttctgg atgtgctcat tcagctgggc 960

attatccgtg actgtgaaat tgctgacatt gtggacaccg ctatgcccat caccatctgc 1020

atagcttatt ttaacaattg cctgaatcct ctgttttatg gctttctggg gaaaaaattt 1080

aaaaaatatt tcctccagct tctgaaatac attcccccaa cggccaagtc acacgcaggc 1140

ttgtcaacaa aaatgagcac tctttcctac cgcccttcag ataacatgag ttcatctgcc 1200

aaaaagtctg catctttttt tgaagtggag tgagagggtt caaagcctgc aagtgaagtg 1260

atttcctgac agcagaagcc agaggaccat ttgggctaag cagctcactc actaccgaag 1320

agtgttcaac ctccagcaa 1339

<210> 597

<211> 3091

<212> DNA

<213> Rattus norvegicus (Norway rat)

<400> 597
gccaacccag atcccgaggt gcgccacgcc cagcccagat ctccacgcct gccaggagcg 60

agcttcgccg gctcgctgtc ccccgagcag cctctgtcct tctgtccgag tcccgcgccc 120

ttctcgggac ccctgcccag cggcagcact gccaccctgc cggccatgga gaccccgtct 180

cagcggcggc ccacccgcag tggggcgcag gccagctcta cccctggtcg cccactcgga 240

tcacacggct gcaggagaag gaggacctgc aggagctcaa tgaccgtctg gccgtgtaca 300

tcgatcgcgt gcgctccctg gagacggaga acgcggggct gcgccttcgc atcactgagt 360

ctgaagaggt ggtcagccga gaggtgtccg gcatcaaggc ggcctacgag cgagctgggg 420

gatgcccgca agaccctcga ctctgtggcc aaggagcgcg cccgcctcca gttggagctg 480

agcaaagtgc gtgaggagtt caaggagctg aaggcgcgca ataccaagaa ggagggggat 540

ttgttggctg cgcaggcccg gctcaaggac ctggaggctc ttctcaactc caaggaggct 600

gccctgagca ctgctctcag tgagaaacgc acgttggaag gcgagctcca tgatctgcgg 660
gggcaggtag ccaagcttga ggcagccctg ggtgaggcta agaagcaact ccaggatgag 720
atgctgaggc gagtggatgc tgagaacagg ctacagaccc tgaaggagga gctggatttc 780
cagaagaaca tttacagtga ggaactgcgt gagaccaagc gccggcacga gacccggctg 840
gtggagattg ataatgggaa gcagcgagag tttgagagcc ggctggcaga tgctctgcag 900
gagctgcggg cccagcatga ggaccaggtg gagcagtaca agaaggagct agaaaagaca 960
tactctgcca agctggataa tgcccggcag tctgccgaga ggaacagcaa cctcgtggga 1020
gctgcccatg aggagctcca gcagtctcga atccgcattg acagcctctc agcccagctc 1080
agccagctcc agaagcagtt ggcagccaag gaggccaagc tgcgtgacct ggaggactcc 1140
ctggcccgtg agcgggatac cagccggcgc ctgctggctg aaaaagagcg ggagatggct 1200
gagatgcggg caaggatgca gcagcagctg gatgagtacc aggaattgct ggacatcaag 1260
ctggccctgg acatggagat ccatgcctac cgaaagctgc tggagggcga ggaggagagg 1320
ctacgcctgt cccccagccc tacctcgcag cgcagccgtg gccgcgcctc ctcccactcc 1380
tcccagtctc agggtggagg cagcgtcacc aaaaagcgca agctggagtc ttctgagagc 1440
cggagcagct tctcgcagca tgctcgcact agcggccgtg tggccgtaga ggaagtagat 1500
gaagagggca agttcgtgcg gctgcgcaac aagtccaacg aggaccagtc catgggcaac 1560
tggcagatca ggcgtcagaa tggtgatgat cctttgatga cttatcgctt cccaccgaag 1620
ttcaccctaa aggccgggca ggtggtgacg atctgggctt caggagctgg ggccacccat 1680
agccccccta ctgacttggt gtggaaggca cagaacactt ggggctgtgg gagcagcctt 1740
cgcacggctc tcatcaacgc cactggagaa gaagtggcca tgcgcaagct ggtgcgctca 1800
ctgaccatgg ttgaggacaa tgacgatgag gaagaggatg gagatgagct gctccatcac 1860
caccgtggct cccactgcag cagctcgggg gaccccgctg agtacaacct gccgtcagca 1920
ccgtgctgtg cgggacatgt gggcagcctg ctgacaaggc tgccagtggc tcaggaccgc 1980
aggtgggcgg atccatctcc tctggctctt ctgcctccag tgtcacagtc actcgaagct 2040
tccgcagtgt ggggggcagt gggggtggca gcttcgggga caacctagtc acccgctcct 2100
acctcctggg caactccagt ccccggaccc agagctccca gaactgcagc atcatgtaat 2160
ctgggacctg ccaggcgagg gtgggggcag aggccacctg ctccctcttc accacatgcc 2220
acctcctgcc ctgttcctta tgagagcagg tctgaagcca aagaaaacta tcccctgcct 2280
ttgggtgttt ttcttttttc cttctatttt ttttctaaga gaagttattt tctacagtgg 2340
ttttatactg aaggaaaaac acaagcaaaa aaaaaaaagt gtctttatct caatccttag 2400
tccttccctt ttccctgtat ctgccttaaa ccaaagggct tctcatagga gcccagggaa 2460
aggactgctt ttgtagtcta gatttttgtc ctgctgcctt gcgtttgccc tcatcccagg 2520
accctgtgac acggtgcctg agaggcaggc atggagttct ctttaccggc ctcctccaca 2580
gctggcccac tgccatgcca gctgcaaaga aaaggggtgc agagagaatg actgaaaggt 2640

caagcccctc ctggctcact acacttggcc gagactcctc tgcctgcctt accctcctgc 2700

cttcccgagc ctgggacgag tggattccca gaggcaaatc tgccatgctt gcttttttct 2760

atattgtatt cagacaagag atgggaaaga tggggaagga gaagggacgg tcagtttgaa 2820

cctacctttc ccccgtgcgt acagcctgct ggtgggctct gtctgtcggt ggagggcagt 2880

attaagtggg attggggaga gagtgaggga aaggctcctg gagattggga gaacctgctg 2940

gggccagtga gaagactaga gggtgtggtg attggtggct acagcaaaca ctaaggaacc 3000

cttcattccc acttcccatc tgcacctctg ctctcccccc aaatcaatac actagttgtt 3060

tccatcccga aaaaaaaaaa aaaaaaaaaa a 3091

<210> 598

<211> 1489

<212> DNA

<213> Rattus norvegicus (Norway rat)

<400> 598
caagattgcc ctgctggagg aggcacggag gcgcaaggag gacgaggtgg aggagtggca 60

gcaccgggct aaagaggccc aggacgacct ggtgaagacc aaagaggagc tgcacctggt 120

gatgacggct cccccgcccc cacccccgcc cgtgtacgag cccgtgaatt accacgtgca 180

ggaggggctg caggatgagg gagcagagcc catgggctac agtgccgagc tctccagcga 240

gggcatcctg gacgaccgta acgaggagaa gcggatcacc gaggcagaga agaacgagcg 300

cgtgcagcgg cagccgttga ctctgagcaa tgagttgtcc caggcccggg atgagaataa 360

gaggacccac aatgacatca tccacaatga gaacatgcgg caaggccggg acaagtataa 420

gacgctgcgg cagatcaggc agggcaacac caagcagcgc atcgacgagt ttgaggccat 480

gtagaggcca ggccgggacc aagggcggag ggcacctcac agcaggcagg tgtcactctt 540

ggctctttag tactctataa gtctagaaac ccccttgccg tgttccagtc ccttaaagag 600

caggtaccgg gcgatgttct gccccaagac ccagtggaat cctccctggt tgcttccaat 660

agtatcacat agtgccaaac aggccagatt tttatgacag ttgccgaatc acttcccgtt 720

cggagcaggg attcggaggg ctggcccgaa tgacgcgatt cagtgtcact ggggataata 780

atagcactgt ggctcacaca cgggtgccat actttctcta gttttacaat gagctcaaat 840

cgattttgtt cttgatttct atgaaggatc catctctgtg tgttgagggg tgaaaatgat 900

tttgaaattt gagtcttaag acgccccaca cagtctcctt cctccagacc gctggcagag 960

tctcctgcgg tccctcagag tgtacggtac aggactctcc gatacaaaat tctcatgctt 1020

accctgttag cattcattgt tggactgata cacctaatga tcttctatag agaatagtat 1080

ttatatataa gcgataatat gggtttgtaa cattagtttt aaaaagggaa agttttgttc 1140

tgtatatttt gttacctttt acagaataaa agaattcaac attaagaacc agtaatcacg 1200

acacttgatc tgacacgggg gcaggtggga gaccgatgac cggaatcgcc gctgtacaaa 1260

aatgttagtg ggttttgtgc atgtaaaacg cacacttcaa cttcctgtca gttcttcact 1320

gagagcagtc agactctagc ccgaagggac tcaggcctgt ttattgtgaa caacttgttc 1380

ctgttttcag tttgtcttcc ttccttgagc cggactgtcc tttttggttg tgatgtcctg 1440

tgcaggtcac tccgcaccag atttacacac aataaaagtt ctccaaggc 1489

<210> 599

<211> 2450

<212> DNA

<213> Rattus norvegicus (Norway rat)

<400> 599
gtcgacccaa tggggcggct ttgcaccaag ttcctgtcct ccgtgagctg tctgattttg 60

ctatgggtga ctggctctgg gggcatcaag gtcctgggtg accccacctg cttctctgac 120

tacatccgca cttctacgtg tgagtggcag ctggatagca ctgtggactg cagttctcag 180

ctcctcctgg actacaggct gctcttcgag ttctctgaaa acctcacatg cacccccaag 240

aacagtgccg acaccgtgtg tgtgtgccaa atggccatag aggagccgat ccaggcagac 300

acatactggc tggaactgtg gtctgagcgg ggacagctgt ggcaaggctc cttcaagcct 360

agtgacaatg tgaagccccc agctccagac aacctcacac tccacaccaa tgtctccaat 420

ggcctgctgc tgatgtggag caacccatac ccatcgaaca acttcctgca caaaggcctc 480

atctgcatgg tcaacatctc cagagaggac aacccggcgg aattcaaagt ctataatgtg 540

acctacacgg aacccaagct gagcttccca gtcaacaccc taacatcagg ggtgcgctat 600

agggcacgtg tgagggtctt gtcccagagc ttccctggca tctggagtga gtggagtccc 660

agcatcacgt ggtacaacca cttccagctg cccctgctgc agcgcctccc gctgggtgtc 720

agcatctcct gcatctgcat cctattgttt tgcctgacct gttacttcag cattatcaag 780

attaagaaga tatggtggga ccagattccc actccagcac gcagtccctt agcagccatc 840

atcattcagg acacgaaggt gctcctctgg gagaagcaga cccgaagcca ggagtcaacc 900

aagagccggc actggaagac ttgtctaacc aagctgctgc cctgcttgct ggagcataga 960

gtgaagaaag agagagaatc cccgaaggct gccaaaacca agcctctcca gagtcctgaa 1020

aaagcaggct ggtatcctgc ggaggtcagc aggaccgtcc tctggccaga gaacgttcat 1080

gtcagtgtgg tgcgctgtat ggagctgttt gaggccccag tacagaatgt ggaggaggaa 1140

gaggatgaga tggtcaaagg ggacctgagc atgtcacctg agaacagcgg agggggcttc 1200

caggagagtc aggcagacat catggcccgg ctcactgaga acctgttttc agacttgctg 1260

ggggctgaga atggaggcgt tggccagtcg agcatggcag agtcatcctc ccttctgcct 1320

tcagaaagtg ggcaagcttc tacgtcctgg gcctgcttcc ccacggggcc cagtgagacc 1380

acatgccagg tcacagggca gcagcctcca cacccagacc ctgagagagc aactggcaca 1440

gcctgcacac aggtccccct tgtcatttca gacaatcctg cctaccggag ttttagtgac 1500

ttctccagcc ccgccccaaa tcctggagag ctggcttcag aacagaagca ggctggacat 1560

ctggaagaag gggatcttct gagcccggtt gacccccatt cttcagggcc accaatgcag 1620

caagcggaga gctgggagca gatccttcac atgagtgtcc tgcagcacgg gacagctggc 1680

tccaccccag cccctaccag tggctaccaa gagtttgtgc aggcagtgaa gcagggtgcg 1740

tcccaggcca ggtgggtgcc tggtgtcggg ccttctggag acactggtta caaggctttc 1800

tcgagcctgc tcagcagcag tggcgtctgc acggacacag cagcagcagg gactgacagt 1860

ggatgtggag ggtacaagcc cttccagaac cctgttccta accagtcccc caactccatg 1920

cccttattca ccttcggact ggacatggag ctgccaccca gtcccctgaa ctcagcccca 1980

cccaatagca ccccagaatg ccttggtcta gagctggggc tcaaaggagg cacctggctg 2040

aaggcccctc ctccttcaga gcaggtgccc aagccctttg gggatgacct gggccttggc 2100

attgtgtact cgtccctcac ctgccatctg tgtggccacc tgaagcaaca ccacagccag 2160

gaggaagatg gtcagatcca tgtcgttgca tgccctggct gcggctgttg ctatgatgaa 2220

aaatcaccat ccttggggaa tctctcgggg accttggaga gctgtcctgg ggaaatgtca 2280

caggaagcca acctcacctt agcacccagg acaccctcca atttgtcagg ggtgcaaggc 2340

cctggccact ctcctgttcc cagccagact actgaggtac ctgtgggcac tctgggcgtc 2400

actgtgtctt aggtgagtgt gctgttgttg ctgaggtctg tgctgtcgac 2450

<210> 600

<211> 2217

<212> DNA

<213> Rattus norvegicus (Norway rat)

<400> 600
tgcccccgga gagaaaggtg aaggaggccc tcctggtgcc gcaggacccc ctggtggttc 60

tggacctgct ggtcctcctg ggcctcaagg tgtaaagggt gaacggggca gtcctggcgg 120

tcctggtgct gctggctttc ccggtggccg tggtcttcct ggtcctcccg gcaacaatgg 180

taatccaggg cccccaggac ccagtggtgc tcctggcaag gatggccctc caggtcctgc 240

aggtaacagt ggttctcctg gaaaccctgg agtagctgga ccaaaaggtg atgctggcca 300

accaggagag aaggggccac ctggtgctca gggtcctcca ggatctccag gtccacttgg 360

aattgcaggg ctaactggag cacgaggtct tgctggacca ccaggcatgc caggtccacg 420

gggtagccct ggacctcagg gtatcaaggg tgaaagtgga aagccaggag ccagtggcca 480

taatggggaa cgtggtcctc ctggacccca aggtcttcct ggtcagcctg gtacagctgg 540

tgagcccgga agggatggaa accctggatc agatggtcag ccaggtcgag acggatctcc 600

tggtggcaag ggtgatcgtg gtgaaaatgg ttctcctggt gccccaggcg ctcctggtca 660

tccaggacca cctggtcctg ttggtccatc tggaaaaaat ggtgacagag gagaaacggg 720

tcctgctggt ccttctggtg ctccaggtcc tgctggagct cggggtgctc ctggtcccca 780

aggtccacga ggtgacaaag gtgaaactgg tgaacgtggc tctaatggca tcaaaggaca 840

tcgaggattc cctggcaatc caggtccccc aggttctccc ggtgctgctg gacaccaggg 900

tgcagttggt agtccaggcc ctgcaggtcc cagaggccca gttggtccac atggacctcc 960

tggaaaagat ggatcaagtg gacatccagg tcccattgga ccaccagggc ctcgaggtaa 1020

cagaggtgaa agaggatctg agggctcgcc cggtcaccct ggacagccag gaccccctgg 1080

accccctggt gcccctggtc cctgctgtgg tggcggtgct gccattgctg gagttggagg 1140

tgaaaagtct ggcggctttt caccatatta cggggatgat ccaatggatt tcaagatcaa 1200

cactgaggag atcatgtctt cactcaagtc tgttaacgga caaatagaga gtcttatcag 1260

ccctgatggt tctagaaaaa accctgctcg gaattgcaga gacctgaaat tctgccaccc 1320

tgaactcaag agtggagaat actgggttga tcctaaccaa ggctgcaaga tggatgctat 1380

aaaagtgttc tgtaatatgg aaaccggaga aacatgcata aatgccagtc ccatgactgt 1440

cccgcggaag cactggtgga cagatgctgg tgctgagaag aaacatgttt ggtttggaga 1500

atctatgaat ggtggctttc agttcagcta tggcaatcct gatcttcctg aagatgtcct 1560

tgatgtacag ctggccttcc tcagacttct ttccagccgg gcctcccaga acattacata 1620

ccactgcaaa aacagcattg cctacatgga tcaggccaat ggcaatgtaa agaagtctct 1680

gaagctgatg ggatccaatg agggagaatt caaggctgaa ggaaatagca aattcactta 1740

cacagttcta gaggatggct gcactaaaca cactggtgaa tggagcaaga cagtctttga 1800

atatcaaaca cgcaaggcca tgagactccc catcatagat atcgcaccct atgacattgg 1860

tggtcctgat caagaatttg gtgtggacat tggccctgtt tgctttttat aagccaaact 1920

ctctgaaacc ccagcaaaac aaaaaccaca tccatgtgtt cctcttgctt taatctcgtc 1980

aaccagtgaa agtgaccaac taaattccag ttatttattt ccaaactttt ggaaaaagca 2040

taatttgaca aaaatagaat acaatttttt tgctgtttca cccaccaata cagttcaaat 2100

gcttttgttt tattttttta ccaattccaa cttcataatg tctcaatggt gctataataa 2160

ataaacatca acacttttat gataaaaaaa aaaaaaaaaa aaaaaaaaaa aaaaaaa 2217

<210> 601

<211> 3169

<212> DNA

<213> Rattus norvegicus (Norway rat)

<400> 601
ccgcacgctg aaccggagga actgcgccta gtcggggcgc tgagggaccc tccaccggga 60

cgccggcccc tccccgggcc tctgctcact tgcccccctg cgagcccgtc cccctagtcg 120
gcctctcgga tcggggacgt ggggcgagct gagagcaggc ccggggtggg tggtcactgt 180
ggagaagacg tggctgtcaa gatgatagaa gtactgacaa ctgactctca gaaactgcta 240
caccagctga acaccctgtt ggaacaggag tccagatgtc agccaaaggt ctgtggcctg 300
aaactgattg agtctgcaca tgataatggc ctcaggatga ctgcaagact ccgggacttt 360
gaagtcaaag atctactgag tctaactcag ttctttggct tcgacacaga aacattttcc 420
cttgctgtga atttactgga cagattcttg tctaaaatga aggtacaggc gaagcatctc 480
ggctgtgtcg gactgagctg cttttatttg gctgtgaaat cgattgaaga ggaaaggaac 540
gtcccgctgg caactgattt gatccggata agtcagtata ggttcacagt ttcagacctg 600
atgagaatgg aaaagattgt gttggagaaa gtgtgctgga aagtcaaagc tactactgcc 660
ttccaatttc tgcagctcta ttactccctc attcgggaga ccttgccatt tgaaaggaga 720
aacgatctga attttgaaag actagaagcc caactgaagg cgtgccactg caggatcata 780
ttttctaagg caaagccttc tgtgctggcg ctggcaatca tcgctttgga gatccaagca 840
ctgaagtatg tggagttaac agaaggagta gaatgtattc agaaacattc caagataagt 900
ggccgagatc tgaccttctg gcaagagctt gtttccaagt gtttaactga atattcatca 960
aacaagtgtt ccaagccgaa cggtcagaag ttaaaatgga tcgtgtctgg gcgcactgca 1020
cgacaactga agcacagtta ttacaggata acccacctcc caacaattcc cgaaaccatg 1080
ggttagttgg caaatctggt tgttatcctc tgtgtacaga acatttccca gtgagatcgt 1140
ttttgtgcta taacttaagg attgaaatac taccttcaat ataaagaata caggatgaaa 1200
acagtaaagg aaacgtgagt ttgttggtct agacagagaa tactgggagg cattcactgt 1260
gtaccgcagt ctgaagagaa atgagtatca aacctctaga cacatgctca tactgctgtc 1320
aaaggactag cgtagaaaag agagtcctcc aaaccggaag tttaaatgta gttactaaaa 1380
tagcacttct tataacttac atatcccccc actgtggctt atttaaagtt acagaagtcc 1440
aagcagaacg acaaaagatg tgacccatat atgaacacat tttaatctgt tcattgatta 1500
ggagagtgaa tatgaacttg catgatgccc atgttaggtt tctggaaact gccggggtat 1560
cttaattctc tagtattctc cctctgtggc agttgggcta atacaaagta actatacgca 1620
tgagaatata aaatcagtct ctgatacata cacattttta ccatcaaaat ttcttaatca 1680
tagcaaagac ttaccttttt atgattagga attttttttt taatgtatgg cagcacatgc 1740
ctttaatccc aacactaggg aggcagaggc aggtggatct ctttgagttc gaagccaggc 1800
tggtctttac agtgagttcc aggacagctg gagagctaca gaatggagag acgctgtctc 1860
aaaaacactc aaaacaaaca aacaaaccat accagtttgt aggcagactt ctgttgggtt 1920
gggtttgtac tgtttgccta tgcagtggga ttacagcagc agcaacaaaa actgtccctg 1980
aagtctttct ctgccactgt gacctgagtt tcctatggta cgcgatttac tctaggaaac 2040
ctcagcccct caccacgtta gctgttggca aatggcctca cagttgcgga aagtcccaat 2100

tctaggcttg ggaaagcaat gcttagattt gaattggccc atgaagcatt caaatcaagg 2160

ctaaagacat aaatgtgaaa taaaactgtg aaccttcatt ttaacattga tctcacttcc 2220

cagatttaat caatatatac ttaggtggta ttaaaaatgg taaactgcct aatttaaatc 2280

tcaaaattta aactatgagg tttacatcaa agccaacatt tcacaaatgt actttttaag 2340

gtattaaaag aggtatttaa gcagtaaatg gtttcttggc acccataacc aagtaatagt 2400

taagttagag gtgggacttt tttattgcta tgagaattac atttaaactt ttgggtgttt 2460

tataaaaagc agatttcaca agttttgaaa attgtgacct ttactgaaat ttgttacctt 2520

taatatttct tctagaggat aggtatttat aaaagaaaaa ttcgtcagaa ttgctgcctc 2580

aatctagtcc catttgagaa aatttgtttc tactgtctca ataactggat gaaatatcac 2640

tctgaaaact tgcctattgc actaaagcta gtttaggctt gataaaacac tccaggaggt 2700

ttttaccaca gactgtttct attaaaactg ctgcttctca tgtacaattt tgttttaaaa 2760

ggaaccgagt acatctgcaa aacctaagtc ttaagggacg tcaggaggta ccttcagaat 2820

tataggatca ccatggtagt ggggattctc catgctggcc ttgaatgttt gatcttcact 2880

gctgaaatgt gggtagctcc tcagcgccct gtagagcctg agtctaccta gaatagctgt 2940

aaccattttg acaagtaatg gataagaaaa ttatccattg agaagctaaa aacaaaacaa 3000

aacaaaacca aagaacgggt gtattttatt cttaaccttt gtaaaccatc actgagaaca 3060

cttcagttct tcctaacagc tgttatgctt cgatttgaaa aaaatactga gtggataacc 3120

aactaccatc atgctttggg tacacctttc aataaaatta ctgaaatgc 3169

<210> 602

<211> 1713

<212> DNA

<213> Rattus norvegicus (Norway rat)

<400> 602
attcctgcgc ctccgctccg tcccctgcgg tctcctctgt gtgccatgga cggcatcgtc 60

ccagacatag cagtcggtac aaagcgggga tccgacgagc tcttctccac gtgtgtcagc 120

aacggcccct tcatcatgag cagctcggcc tcagcagcca acggaaatga tagcaagaag 180

ttcaaaggtg ataacaggag cacaggagtt cctaccagag tcatccatgt ccgaaagcta 240

cctagtgatg tcactgaggg cgaggtcatc tccctggggc tgccctttgg gaaagttacc 300

aacctcctta tgctgaaggg gaagaaccag gccttcattg aaatgagcac agaggaggct 360

gccaacacta tggttaacta ctacacatcg gtggcgccag tgcttcgtgg acagcccatc 420

tacatccagt tctccaacca caaggagctc aaaaccgaca gctcgcccaa ccaggcacgt 480

gcccaggcag ccctgcaggc tgtgaactcc gtacagtcag gaaacctggc cttgcgagcc 540

tctgctgctg ccgtggatgc aggaatggca atggcaggcc agagcccagt gctcaggatc 600

attgtggaga accttttcta cccagtgaca ctggacgtgc tgcaccagat cttctcgaag 660

tttggcacag tcctgaagat catcacgttc accaagaaca accagttcca ggcgctgctg 720

cagtatgctg accctgtgag cgcccagcat gccaagctgt ccctggatgg ccagaacatc 780

tacaatgcct gctgcacgct gcgcatcgac ttctccaagc tcaccagtct caacgtcaaa 840

tacaacaacg acaagagccg agactacaca cgccctgacc tgccctctgg agacagccag 900

ccatcactgg atcagaccat ggcagcagcc tttggtgcgc ccggcataat gtcagcctct 960

ccgtatgcag gagccgggtt ccctcccacc tttgccatcc ctcaggccgc aggcctctct 1020

gttcctaatg tccatggagc cctggcaccc ctagccatac cctctgctgc tgctgctgct 1080

gctgcagccg gccgcattgc catcccaggg ctggcaggcg ccgggaattc cgtccttttg 1140

gtcagcaact tgaaccctga gagagtcaca ccccaaagcc tctttattct cttcggcgtc 1200

tacggtgatg tgcagcgggt gaagatcctg ttcaacaaga aggagaacgc acttgtcgag 1260

atggcagacg gcagccaggc ccagctggcc atgagccacc tgaacgggca caagctgcat 1320

gggaagtcag tgcgcatcac cctgtcaaag caccagagtg tgcagctgcc tcgtgaggga 1380

caggaggacc agggcctgac caaggactat ggcagctcac cgctgcaccg cttcaagaaa 1440

cctggctcca agaactttca gaacatcttt cctccctcag ctaccctgca cctctccaac 1500

atcccgccct ctgtgtcaga ggatgacctc aagagcctct tctccagcaa tggcggtgtg 1560

gtcaaaggct tcaagttctt ccagaaggac cgcaagatgg cactgatcca gatgggctct 1620

gtggaggagg cagtgcaggc actaattgag ctgcacaacc atgacctggg cgagaaccac 1680

cacctgcgag tgtccttttc caagtccacc atc 1713

<210> 603

<211> 966

<212> DNA

<213> Rattus norvegicus (Norway rat)

<220>

<221> misc_feature

<222> (50)..(50)

<223> n

<220>

<221> misc_feature

<222> (58)..(58)

<223> n

<220>

<221> misc_feature

<222> (63)..(63)

<223> n

<220>

<221> misc_feature

<222> (625)..(625)

<223> n

<400> 603
ggcgtgagga ggttggagag ttttttctgg gacctaaaca aaggcacccn cggccctnct 60

aanctgaagt tgagcctcac atatcctgga aaggaaaatg cccataccag aacccaagcc 120

tggagatctg attgagattt tccgccctat gtacagtcac tgggccatct atgttggtga 180

tggatatgtg atccacctgg ctcccccaag tgaaatccca ggagctgggg cagccagcat 240

catgtctgct ttgacggaca aggccatagt gaagaaagag ctgctgcgtg atgtggctgg 300

gaaggacaag taccaggtca acaacaagca cgacaaggag tacactccgc tgcctctgaa 360

caagatcatc cagcgagctg aggagctggt ggggcaggag gtgctgtaca ggctgaccag 420

tgagaactgt gagcacttcg tgaacgaact gcgttatgga gtccctcgga gtgaccaggt 480

cagagatacc gtcaaggtgg cgaccgtcac tggagtgggc ttggcggcct tgggcctcat 540

tggagtcatg ctctcaagaa acaagaaaca gaagcagtga gctgaatgac tatccagctt 600

tagggctctt cttttgctag agggntggag tttgatttat agattctact gctttataat 660

taggtatatt ttcacaatat acaataaacc acaagaaggg aattttcatg gagtacactg 720

tagctatctt cagacacacc agaagagggc accagatccc attacagatg gttgtgagcc 780

atcatgtggt tgctgggatt tgaactcagg acctccggaa gagcaatcag tgctcttaac 840

cgctgagcca cctctccagc cctgaagggc tctttcaaag gtttattctt tctcctttca 900

caagtcggca tcgaaacttc caagtgtcct caaagtccag ggctccttgg actccataac 960

gtttct 966

<210> 604

<211> 1067

<212> DNA

<213> Rattus norvegicus (Norway rat)

<400> 604
acggaggacc gtcagccagc aatcgcgtag ctcgatcgat cacccaggaa gagaaattgt 60

aagcagataa gaagaacgcg ttcggtttgg gacaccattt gcagctggaa atggggaatg 120

gactgtcaga ccagacttcc atcctatcca gcctgccgtc ctttcagtcc tttcacattg 180

ttattctggg tttggactgt gctggaaaga caacagtttt atacaggctg cagttcaacg 240

aatttgtaaa tactgtacct accaaaggat ttaacactga gaaaattaag gtaaccttgg 300

gcaattccaa aacagtcact tttcacttct gggatgtagg tggtcaagag aaattaagac 360

cactgtggaa gtcatatacc cgatgcacag atggcattgt gtttgtggtg gactctgttg 420

atgttgagag aatggaagaa gccaaaactg aacttcataa aataactagg atatcagaaa 480

atcagggagt ccctgtgctt attgttgcta acaaacaaga cctgaggaac tcactctctc 540

tctcagagat cgagaagttg ttagcaatgg gtgaactgag ctcatcgact ccttggcatt 600

tacagcccac ctgtgcaatc ataggagatg ggctaaagga aggacttgag aaactacatg 660

atatgataat taaaagaaga aaaatgttgc ggcaacagaa aaagaagaga tgaatggcag 720

tacttttata tcggtgtgga ataggtttta cttggtctga tttctgacaa gctgaagagt 780

gtctacagcc tggtttgcct gtctgccctc acggatgcta ttaaagcttt gttttgttga 840

acagtcagat acccaactct gttgccttgt ggaagatgag taaatgcaat gcttcttaaa 900

ggggtctctt ctccgtgacc cacaaatctt ttggtactac cattttggga agccaaaaaa 960

ggctagtaat tgaccagaaa acaattttgt ggaaatttga cctgaagtta gtgaaataaa 1020

actttgaaga gtgtaaaaaa aaaaaaaaaa aaaaaaaaaa aaaaaaa 1067

<210> 605

<211> 2462

<212> DNA

<213> Rattus norvegicus (Norway rat)

<400> 605
gtcgacccac gcgtccgacc accttagctg ccatcactgc acattggagc agctttctaa 60

acaggtgcca ccatgccagt gaagaagtac ctgctgaagt gcctgcacag gctgcagaag 120

ggcccaggct acacctacaa ggagctgcta gtgtggtact gcaacaacac caacacacac 180

ggccccaaac gcatcatctg cgagggggcc aagaagaagg ccatgtggtt cctgctcacg 240

ctgctcttcg cctgcctggt gtgctggcag tggggcgtct tcatccagac ctacctgagc 300

tgggaggtca gcgtctcgct ctccatgggc ttcaagacca tgaacttccc agcagtcacc 360

gtctgcaatt ccagcccctt ccagtactcc aaggtcaagc acttgctgaa ggacttgtac 420

aagctgatgg aggctgtcct ggacaagatt ctggctccga agtccagcca caccaacacc 480

accagtaccc tgaactttac catctggaac cacacgcccc tggtccttat tgatgagcgg 540

aaccctgacc atccagtggt cctcaacttg tttggggaca gccacaacag cagcaaccca 600

gccccaggaa gcacctgtaa tgcccaaggg tgcaaagtgg ccatgaggct gtgcagtgcc 660

aatgggaccg tgtgtacctt ccgaaacttc accagtgcca cccaggccgt gactgagtgg 720

tacatcctgc aggccaccaa catcttctca caagtgctcc cccaggacct ggtggggatg 780

ggctatgctc ctgatcgcat aatcctagcc tgtctgtttg gaacggagcc ctgcagtcat 840

cggaacttca cacctatctt ctaccctgat tatggcaact gctacatctt caactggggc 900

atgacagaga aggcacttcc ttctgccaac cctggactgg aatttggtct caagttgatc 960

ctggacattg gtcaggagga ctatgtcccc ttccttgcgt ccacagcagg ggctaggctg 1020

atgctccacg agcagaggac ataccccttc attagagaag agggcatcta tgccatggca 1080

ggaactgaga cttctattgg ggtgctgctg gacaagctgc agggcaaggg ggagccatac 1140

agtccctgca ccatgaacgg ctccgacgtt gccattcaga acctctacag tgactacaac 1200

acgacctatt ccatccaggc ctgccttcat tcctgtttcc aagaccacat gatccataac 1260

tgcagctgtg gtcactactt gtaccctttg cctgctgggg agaaatactg caacaacaga 1320

gacttcccag actgggccta ctgctaccta agcctacaga tgagtgtggt ccagagagag 1380

acctgcctca gcatgtgcaa ggagtcctgc aacgacaccc agtataagat gaccatctcc 1440

atggctgact ggccatccga ggcctctgag gattggatcc tacatgtcct gtctcaggag 1500

cgggaccaga gctcaaatat caccctgagc aggaagggta ttgtcaagct caatatctac 1560

ttccaagagt tcaactaccg taccatcgag gaatcgccgg ccaacaatat cgtgtggctg 1620

ctctctaacc tgggtggcca gtttggcttc tggatggggg gctcggtgct gtgcctcatt 1680

gagtttgggg agatcattat cgacttcatt tggatcactg tcatcaagct agtggcctcc 1740

tgtaaaggcc tgcgcaggag gcggccacag cgaccctaca ctggcccgcc gcccactgtg 1800

gccgagctgg tggaggccca caccaactgt gtcttccagc ctgacacaac cagctgcagg 1860

cccaatgccg aggtctaccc tgaccaacag actctgccca ttccgggcac tccacctccc 1920

aactatgact ccctgaggct gcagccgctg gacaccatgg agtctgacag cgaggtggag 1980

gccatctaga tccgcatccc cacccgggaa ctagtgaact caaaactgag gagtcacaac 2040

cattgtcagt gcctcatttc attagccctt gtccaaagag ccagggcaca gagcccatgt 2100

ccctctggtc agccccaggc tgaggggttc atagggtcaa gatgctggta ccagaatatt 2160

gaacttgtat ccttttctag ctcttgccca ccctagccca gtctttgctc tctgttgacc 2220

tagcagacag gctccagaga cccatagagt ccctctcctg gtgataggcc acttctctgt 2280

cttgttacaa cctcagtctc ccagaatcag tgaccttgcc ctagttggca ttggctgaac 2340

cctgttaata gacttggggg tgtgcagacc atagggaggg agcatcaggt aagaaggctt 2400

gacaggggag cacatgcttt gttagaaaat aaagagagaa aacaccgaaa aaaaaaaaaa 2460

aa 2462

<210> 606

<211>  1969

<212>  DNA

<213>  Rattus norvegicus (Norway rat)

<400>  606
agacgcttct gggtcgcgtt agggagacgc tctggtccgg ttgtgcgtga gaggaggaga      60

gcccagcgcc acagcgaccc ggagagggat ggcggccacc gggaccgcgg ccgctgcggc     120

caccggcaag cttcttgtcc tgctgctgct cgggctcacg gcacccgcag cggctctggc     180

tggctatatc gaggctctcg cagccaatgc tggaacaggc tttgcggttg ctgaacctca     240

gattgcgatg ttctgtggga agctgaacat gcatgtgaac attcagactg ggaagtggga     300

gcctgaccca accggcacca agagctgcct gggaacaaag gaggaagttc ttcagtactg     360

ccaggagata tatccagagc tgcagatcac aaatgtgatg gaagcaaacc agccagtcaa     420

tattgatagt tggtgccgaa gggacaaaaa acagtgcagg agtcacattg ttataccatt     480

caagtgtctt gtgggtgaat ttgtaagtga tgttctgcta gttccagaaa actgccagtt     540

tttccaccaa gagcggatgg aggtgtgtga gaagcatcag cggtggcaca cagtcgtcaa     600

ggaggcatgt ctgactgagg ggatgacctt atatagctat ggcatgctgc tgccctgtgg     660

ggtagaccag ttccatggca ctgagtatgt gtgctgccct cagacaaagg ttgttgactc     720

tgactccact atgtccaaag aagaggagga agaagaggaa gaggatgaag aagaagacta     780

tgctcttgat aaaagtgaat ttcctaccga agcagatctg gaagacttca cagaagcagc     840

tgcagatgag gacgaggatg aggaggagga ggaggaggag gaaggggagg aagtggtaga     900

agaccgtgat tactactatg actcctttaa aggagatgat tacaatgagg agaacccaac     960

tgaacccagc agcgatggca ccatctcaga caaggagatt gctcacgatg ttaaagctgt    1020

ctgctcccag gaggccatga cggggccctg ccgggccgtg atgcctcgtt ggtacttcga    1080

cctctccaag ggaaagtgcg tacgctttat atatggcggc tgcggcggca acaggaacaa    1140

ttttgagtct gaggattatt gtatggctgt gtgtaaaacg atgattcccc cgactcccct    1200

gccaaccaat gatgtggacg tgtattttga gacgtcggct gatgacaatg agcacgcccg    1260

ctttcagaag gctaaggagc agttggaaat tcggcatcgc agccgaatgg acagggtaaa    1320

aaaggagtgg gaagaagcag aacttcaagc caagaacctc cccaaggcag agcgacagac    1380

cctcattcag catttccaag ctatggttaa agccttagag aaggaagcag ccagtgagaa    1440

gcagcagctt gtggagaccc atttggcccg agtagaagca atgctgaatg accgtcggcg    1500

catagctctg gaaaactacc tggctgccct gcagtctgac cctcctcggc cacatcgcat    1560

tcttcaagct cttcgtcgtt acgtccgtgc tgagaacaaa gatcgcctgc acaccattcg    1620

tcattaccaa catgtgttgg ctgttgaccc agaaaaggct gcgcagatga aatcccaggt    1680

gatgacacac ctccatgtca ttgaagaaag aaggaaccaa agtctctctc tgctgtacaa    1740

agtgccttat gtggctcaag agattcagga ggaaattgat gagctccttc aggaacagcg    1800

agcagatatg gaccagttta cctcctccat ctcagagaac cctgtggatg tccgggtgag 1860

ctctgaggaa agtgaggaga tcccgccgtt ccaccctttc catcccttcc catccttgtc 1920

tgagaacgaa gacactcagc cggagttgta ccacccaatg aaaaaagga 1969

<210> 607

<211> 722

<212> DNA

<213> Rattus norvegicus (Norway rat)

<400> 607
tcctttccgc tcggccgttc tcctgtacag gaggcagcca tggcgcccag ccggaatggc 60

atgatcctga agccccactt ccacaaggac tggcagcagc gagtggacac gtggttcaac 120

cagccggccc gcaagatccg cagacgcaag gcccggcagg cgaaagcgcg ccgcatcgcc 180

cctcgccccg cgtccggtcc catcagcccc atcgtgaggt gccctacagt tagataccac 240

accaaggtcc gggctggcag gggcttcagc ctggaggagc tcagggtggc tggtatccac 300

aagaaaatgg cacgcaccat cggcatctcc gtggacccaa ggaggcgaaa caaatccacg 360

gagtcactgc aggccaacgt gcagcgcctg aaggagtacc gctccaagct catacttttc 420

cccaggaagc cttctgctcc gaagaaggga gacagttctg ctgaagaact taaattggcc 480

acgcagctaa caggacctgt gatgcccatc cggaatgtgt acaaaaagga gaaggccaga 540

gccatcacgg aagaggagaa gaactttaag gctttcgcca gccttcgcat ggcccgagcc 600

aatgcccggc tcttcggcat ccgagcaaag agggcgaaag aagccgcaga gcaagacgtt 660

gagaagaaga aataatgcgc ggctggagag ttgtaataaa ttttccataa agcaaaaaaa 720

aa 722

<210> 608

<211> 3628

<212> DNA

<213> Rattus norvegicus (Norway rat)

<400> 608
gaattccatt gggcatcgtg ggcaaaactt ccgaacgtcc tctgcagagt ctggtgtgga 60

atgacactgg gcgtgtagca ccctgaactg gccatcttgt ttcaggaagt ggaagtcaca 120

gtctccctcc accgcatgct cgcctgttgc ctcacagaac aatttgctaa gggggttgaa 180

gggagaggtt agaggagaga gcaggttgag gaagtgtctc ttggggttct tcagccacag 240

caagtatctc cacggctgaa tggacgacaa cgacttttaa gcagtatttt cttacttaca 300

gaaaggagtc caagtgaaga gagcaacatg gtctctgccc agcttcactt cctgtgtctt 360

ctcacgcttt atttgactgg cgcatatggc caagaaggga agttcagcgg ccccctgaaa 420
cccatgacat tctctatttt tgaaggccaa gaaccgagtc aaatcatatt ccagtttaag 480
gccaaccctc cagctgtgac ttttgagcta acaggagaga cggatggtat atttaagata 540
gaaaaggatg gacttcttta tcacacaaga gtcctggaca gagaaacaag ggcggttcat 600
catctgcagc ttgcagcctt ggattctcag ggagctatag tggatggccc agtccccatc 660
atcatagaag tcaaggacat caatgacaac cgacctacat ttctccagac aaaatatgaa 720
ggctcagtga ggcagaactc tcgcccagga aagcctttca tgtatgtcaa tgctacagac 780
ctggatgacc cggctactcc caatggccag cttttttatc aaattgtcat ccagcttccc 840
aaaatcaaca atgttatgta cttccagatc gacaacaaaa caggggcaat ctcgcttacc 900
ccagaaggtt ctcaggtatt ggatccaata aagaatccgt actacaacct ggtggtctct 960
gtgaaggaca tgggggccca gaatgagaat tccttcagcg ataccacatc tgtggatatt 1020
actgtcagag agaacatctg gaaagcacca gagcctgtgg agattcgaga aaacttaact 1080
gatcctcacc ccatcaaaat cactcaggtg cagtggaatg acccaggggc ccactattcc 1140
ttaatcaaca aggagaagct gccacagttt ccattctcga tcgaccaaga aggaaatatt 1200
tacgtgactc agcctttgga ccgtgaggaa aaggactctc atgttttctt tgccactgcc 1260
aaggatgaga atggaaaacc ccttgcatat ccactggaaa ttcgtgtgaa agttattgac 1320
attaacgaca acccaccgac atgtctctct caagtgactg tatttgaagt ccaggagaat 1380
gaagtcttgg gtagtagcat tgggatcttt gctgcccacg atatggatga agctaacaat 1440
atcaacagta ttttgaagta cagacttgta gaccaaacac ccaaagttcc ctcagatgaa 1500
ctctttctca ttgacgaata tggaggaaag gtccagttgg gtaaacggtc cttgaagaag 1560
caagacagtc ctcagtacaa cttaacagta gaggtgtctg acatagattt caagactctc 1620
tgttctcttc aagtcaacgt tattgatatc aacgatcaga ttcccatctt tgaaagatca 1680
gactatggaa gcaagactct ttctgaagac acagccatcg gatccaccat cttaatcatc 1740
caagctactg atgatgatga gccctttacc gggagctcta aaattctgta caagattgta 1800
cagggagaca ctgagggaag actggaagtt gtcacagatc ccatgaccaa tacaggatac 1860
gtcaagatca gaaagcctct tgattttgaa accgaaccag ttactagcat tgtgttcaaa 1920
gcagaaaacc ctgagccgct ggtgaatggc atagagtaca atgctagttc ttttgcctcc 1980
ttcgagctga ccgtgacgga tgtgaatgaa gtgcctgtat ttccccagca aatattccaa 2040
gccaacgtta gcgaggatac tgctataggc actaaagtgg gcactgtgac tgccagggat 2100
ccggaaggcc tgactgtgag ttattcactg aaagacaata agagaggttg gcttaaaatc 2160
gactccgtca ccggcgacat atttagcact gctccgctgg acagggaaac agaaagtgtg 2220
tatcgagtac aagtggtggc cactgaagta ggtgggtcct ctctgagttc tacagcatat 2280
ttccacctgg tcctcatgga tgtgaatgac aacccccctc gcctagctaa ggactacaca 2340
ggcttgttct tctgccatcc cctaagtgcc cctgggagcc tcatctttga agccactgat 2400

gatgaccagc agtcagttcg gaggcccaag tttacgtttg cccttggcag agaaagctta 2460

caaagtgact gggaagtgtc caaaatcaat ggcacacatg ctagactctc caccaagcac 2520

acacgctttg aggaacaagt ttatgacatc ccaatcctca tcaatgatgg ggggcagcca 2580

cccatggaag gcattgtctc cttatcagtt actttctgcc agtgtgtgga cggaagttgt 2640

ttccggccag caggcaacca ggttgggata cccaccgtgg gcatggcagt tggtatactt 2700

ctgaccacat ttctggtcat tggtataatt ttagcggttg tattcatccg tatgaggaaa 2760

gataaagttg aagatccaca gtcccctgag aataagcctc tgaggagctg aatttgaaca 2820

gaaatgttca aggttgtaca ccaagcactg tatcaatgcc tcagctgttt aatttcatcc 2880

aaaatgtgaa ctataattgg tagagtttat gcatcatgtg ctatatctgg tccccagctg 2940

tttaatctca taatttcatc caaaatgtga actataattt ctttgatggc atgccttctt 3000

gtcctgcaac acttaacgct acttagatat tttatctctg tagacattag agttactctc 3060

cattccccca taacattttg ctcctttaca aaagcctaca taacatgaac tttcccttct 3120

ggaaagaaga tgaactaggt ataaaccctg tgtgctgttg tttcagagac aagtctgttt 3180

tctcccaact gacttttcta ggctccactt atccctttct gtttccacct gcgccccctg 3240

atctcagcat tacattaaat ttaaaatgtg tcacacaaaa gaacaaagtg aagtccttgg 3300

ggggggtgtt gctaagtgga cagccttatt ctttagcaca aacaacagct tctctgtgtt 3360

gtcatcttta atagaggtcc tccagcttgg ctatggtgta gaccctgggg aggtatcaaa 3420

tacaactgcc gtgtttcaag aaagacctac ttcatgaggc acaggaactg acgagctgtc 3480

tgggtttact cactactccg tgcttacata catgctgtac atgttttatt tgtatattga 3540

agttttgtta tatatttatc atgtggagga aagacgaaag catgtacgtg gtgaagaaat 3600

ccaaaataaa tgttggtttc aggaattc 3628

<210> 609

<211> 2578

<212> DNA

<213> Rattus norvegicus (Norway rat)

<400> 609
gatctggtgg gatggggaca tctttgtgga gacacagggg ggcaggggtg ggaggaggta 60

tgggatgtgg aactgtctgg agggtggacc gggaggggag taaaatctgg agtataaaaa 120

taaataaatt aattaagaat actgttaatt gaacaagtcc tgaaaaaaaa aaaaagaaaa 180

agaaacggga tggagatgtc tctgtggtta agagtgcata ctgctcttgg agaggcctgg 240

agtttacttc ccactatgtg tcaggtggct tagcgcgcgc gcgcgcgcgc gcgcgctcgc 300

ggtgtgtgtg tgtgtgtgtg tgtgtgtgtg tgtgtgtgtg tgtgtgtgtg tgtgtgtgtg 360

cgtgtgtgtg ttgtatatga gtagaatgaa cctaagacct tgtgcatact ggacaagcta 420

tcactgcatt gtatccatat tccacagagt ttagcaccca ggtatggcat aataataata 480

aatcttattg taaattggag aaaggacaga tttaggttcc cttttacaga agcccaggag 540

gcacagcctt atttgaaaga aaaagcaact ggcataaagt ggtggattat gtcactttgt 600

gtgatggtag taatcactta attatgtaaa ccagagtatc ctattgcaca ccttaaatgt 660

aggcaataaa agtaaaactt taagcagtaa aaaatactgt agaggccacc gccatgccat 720

ccagactgag gaagacccgg aaattccggg gccacgtgag ccacagccac ggtcgcattg 780

gtaagcaccg caagcaccca ggaggccgcg ggaatgctgg aggcacgcaa ttaccacagg 840

atcaactttg acaaatatca tcctggttac tttggcaaag tcggcatgag tcattaccac 900

ttgaagagga gccagagctt ctgcccaact gtcaacctgg ataaattatg gacgttggtc 960

agccagcaga cacgtgtcaa tgcagcaaaa aacaagagtg gagctgctcc catcattgat 1020

gtttttccaa tcaggctact acaaagttct gaggaagggg aagcttccta agcaacctgt 1080

catcgtgaag gccaaattct tcagcagaag agctgaagag aagataaagg gtgttggagg 1140

tgcctgttct ggtggcttaa agtcacttca gaggttaatt aaatgcaaac attttccatg 1200

aaaaaaaaat attgtagaaa tgaattagca gttaagagca ctggctgctc ttccagagaa 1260

tctcacctgt ttgtaactct agttccacag cttctgagac cctcacacag acatacatgg 1320

aggcaaaaca gggttgaact ataagataaa aaaccagtgt ggtgtatatc ccaagtgatg 1380

tctgtgagtg gcattatgac cattctgtta tcccgcattc tctgctaaaa gggctttgta 1440

tgcaaaaggt acctttatta tatgagctaa ttacatgact taattggtga gtaatcatgg 1500

ctaatattac tatagcatgc taaaggaagt atcctaacaa ctgggaagat ttactgtgta 1560

aacattactt tatttacaag tatatggttt accttcactg ctaaaactca gtctaagaat 1620

gcaatgatct ttggataaca tgatttacta gattagccct gagtttagat tgtatgttga 1680

aactctctgg ggtatttgaa aaaaagaaaa aaaaagacag taaatgagtg atatgggagg 1740

gggtgcctta gttggctcat gctgaggcac acctgcccac atctccccct tctcccctcc 1800

cccgaggtac cagccatata tgggtctagt ataaaagaga gcttatttgg aggcatggga 1860

aagggggttg agaaggaagt tgaggcagag aaagagaagt acagagagag agagaggaaa 1920

gaaaggagaa gaggcaggcc aggaatatgt ggagaggatg agagaggact tgagaggaaa 1980

gagagagagg gtaagaggga ctgagggaga gggagagaag gagagaagga gagagggaga 2040

gagagggaga gagagagagg aaggttagag agcaaagaga ggctagtcat ttttatggca 2100

agccaggctc ttgtctggat gttgctatgt aacagttggg tagagccaag aaggaatgct 2160

aagaagctaa ctttgggtcc cacccctggt tagttacaca gatttgtggt agtagaaaga 2220

ggaagaacag ttttcacttg tgtattttaa caggtcaggg tccacaaaga agaaatatta 2280

aagcatatct agttgattgg tcacttaggt atcagaagct catgttgaat tggcagctag 2340

cattataaaa gtcctggaca gcaagctcac aggagtctcc ctgagaaggc tgccatggat 2400

ccagtcctag tcctggtgct cactctctcc tctctgcttc tcctctcact ctggagacag 2460

agctttggga gagggaagct ccctcctggt ccaacacctc tcccaatcat tggaaacacc 2520

cttcagatat atatgaagga catcggccaa tcaataaaaa aggtaagtgt tttttaag 2578

<210> 610

<211> 443

<212> DNA

<213> Rattus norvegicus (Norway rat)

<400> 610
caagatcatc gaagacctga gggctcagat ctttgcgaat tctgtggaca atgcccgcat 60

cgtcttgcag atcgacaatg cccgtcttgc cgctgatgac tttagagtca agtatgagac 120

ggaactggcc atgcgccagt ctgtggagag tgacattcat ggactccgca aggtggtgga 180

tgacaccaac atcacgaggt tgcagctgga gacagaaatc gaagcgctca aggaggagct 240

gctgttcatg aagaagaatc atgaggagga agtccaaggc ctggaagctc agattgccag 300

ttctgggttg actgtggaag tggatgctcc caaatctcag gacctcagca agatcatggc 360

ggacatccgt gcccagtatg aacagctggc tcagaagaac cgtgaggaac tggacaagta 420

ctggtctcag cagattgagg aga 443

<210> 611

<211> 3276

<212> DNA

<213> Rattus norvegicus (Norway rat)

<400> 611
cgaccgacct gcagttgagc cgagttggaa atgtgaacgc aagaagcagg cttgattttt 60

ttttcctccc cccctctcct agctctctcc tcctctctct ccctctcgca cccacactca 120

cgcacacctc cagcccgcac acagacgtgc acgcaccccc cgccgcccgg cagttatgta 180

ttctccgctc tgtctcaccc aggatgagtt tcatcctttc attgaagcac ttctgcccca 240

tgtccgcgcc ttcgcctaca catggttcaa cctgcaggcc cgaaagcgga aatacttcaa 300

aaagcatgag aagcgcatgt cgaaagaaga ggagagggct gtgaaggatg aactgctaag 360

tgagaagcct gaggtcaagc aaaagtgggc ttcccgactt ctggccaagt tacggaaaga 420

tatccgaccc gagtaccgag aggattttgt tcttacagtt acagggaaaa aacctccatg 480

ctgtgttctt tccaacccag accagaaagg caagatgcgg agaattgact gcctccgcca 540

ggcagataaa gtatggaggt tggacctcgt catggtgatc ttgttcaaag gtattccgct 600

ggaaagtact gatggtgaac gccttgtgaa gtccccacag tgctcgaatc cagggctctg 660

tgtccagccc catcacatag gggtttctgt aaaggaactc gatttatatt tggcatactt 720

tgtacatgca gcagattcaa gtcaatctga aagtcccagc caaccaagtg atgctgacat 780

taaggaccag ccagaaaatg gacatttggg cttccaggac agcttcgtca catcaggtgt 840

tttcagtgtg actgagctag taagagtgtc acaaacacca atagctgcag gaaccggccc 900

caatttttct ctctctgatt tggaaagttc ctcatactac agcatgagtc caggagcaat 960

gaggaggtct ctacccagca catcctctac cagctctacc aagcgcctca agtctgtgga 1020

ggacgaaatg gacagtcctg gtgaagaacc attttacaca ggccaagggc gctccccagg 1080

gagtggcagc cagtccagcg gatggcatga agtagagcca ggcatgccat ctccaaccac 1140

tctgaagaag tctgagaagt ctggtttcag cagcccctcc ccttcgcaga cctcctccct 1200

cggaacagca ttcacacagc atcaccgacc tgtcataaca ggaccgagag caagcccaca 1260

tgcgacgcca tcgactctcc actttccaac gtcacctatc atccagcagc ctgggcctta 1320

tttctcacac ccagccatcc gttaccaccc tcaggagacg ctgaaggagt ttgtccaact 1380

tgtctgcccc gatgctggtc agcaagctgg acaggtgggg ttcctcaatc ccaatggaag 1440

cagtcaaggc aaggtgcaca acccattcct ccccacccca atgttgccgc cgccaccgcc 1500

accaccgatg gccaggcctg tgcctctgcc catgccagac accaagcctc caaccacatc 1560

aacagaaggc ggtgcagcct cccccacctc accgatcctg gtacctggga taaaagttgc 1620

agcgtcccac catcctccag acagaccacc tgaccccttc tcaactctgt aacatggacg 1680

caacctcaac ccggcgcagt tacaacttca ctgtcagtgg aaggggagag acaccaactc 1740

gaatcaactt gtacatggaa acagcgaacg tcgtggtcaa acagcaaagg cccataactt 1800

ttcgggattc tttttttttt ttaatacttt agggactgtc gaattctcta aatatggtgc 1860

tggaaatgcg ttgggctgtg tgacatttga agtgttccag tggtggtgtg agcagtaggt 1920

gaagcggctg gtaggggatg agccttgctc acgaacttcc tgttgtaaca cttttgagcc 1980

tggccttttc acagtatttc aagaatttgc ccgcgcaggt gtgtgaccct cccggagcac 2040

ttggagacac ctggagaatg aaatctttcg tagtagctaa gatctgtgat ctgtaactta 2100

caggacactc aagggcaatg tttttggggt tttctttttt ttctgtaaca tattagaaaa 2160

agcaatgcag ttaagttatc tttcttttag tttggtttgg ttcagcagtc agcagttaaa 2220

tacataacac aaccctcaag gacagtgaac ccactcatgt tgcagagcaa tccaaaaaga 2280

cagcaaagac tactactact actctcagtt cggttgtttg tttttttttt tttttttttg 2340

tttttttatt aagttttgaa atgctgcact tacatttaaa aaaaacattt tcaacaattt 2400

caacaatgac acaaaagttc acatggaaat ggggaagatg gtctgttttg acagaaactg 2460

acaggaatca aaacaatcga attttgaatt gagtgaagtg caatttcatt ggatagctaa 2520

gtatctttgt aagatagaga ttgttgaaaa ttctattttt gtttttcaag tcctcccacc 2580

ccaggactct aaattattgg ggttaaaaaa aaacagcctt gcaagaaaaa ggggagctat 2640

ttttgctttt tatgtttttt attgttaaac ttgtatccct ttaaaaaaaa aaaccgaagg 2700

aaaatttaaa aaaaaagcaa aaaaaaaaaa tacaaatcta atggtgcttt tacctccaat 2760

atgttaacta cattaaatgc taattaatta ttttctgtta tcaaagcaca taactcaaat 2820

gaaatcatgg tatctgttaa ttttataagc tagaaatcac tataatgaat tatgccaatt 2880

ctggaaaatt ttacacatgt atggcaacac cagatctatc ggttttcaaa cgcctcgtcg 2940

taaccagaca ttgtccagaa gtttcaaaga cccttcgcaa accctgaatc ggctacggga 3000

aatatatcag atcgataagg acataactgt gtgagccatt cagatcaaag tacttgttag 3060

gaaaataaac aaacaaacaa aaacaaaatg ttcacacaca cacacacccc aaatgtcttg 3120

tatcttatgt ttaaggaaaa agaaaagaca aaacggcacg agggtctgac caccatgaca 3180

acctacagca acaaggacca aagctgagag aggcgtctca ttcactcgtg accttctggt 3240

ggcaccgagc aggctgctct cttgcacaga tggttg 3276

<210> 612

<211> 1907

<212> DNA

<213> Rattus leucopus (mottle-tailed rat)

<400> 612
ccacgcgtcc gcgaacctac tgaacactcc accggcggcg gctcggttcc ccgccagccg 60

cccagcgcgc agacggggct tgatgtccag gttgtcagcg cagctggcga ctcaggtacc 120

atgagccagg acaccgaagt ggacatgaaa gatgtggagc tgaacgagct ggaaccggag 180

aagcagccta tgaatgcagc ggacggggcg gcagccgggg agaagaacgg tctggtgaag 240

attaaggtgg ccgaagacga ggcggaagcc ggggtcaagt tcacaggctt atccaaggag 300

gagctattga aggtagctgg cagcccgggc tgggtgcgca cccgctgggc gctgctgctg 360

ctcttctggc tcggttggct gggtatgctg gcgggcgccg tggttatcat cgttcgggcg 420

ccacgctgcc gtgagctgcc ggtacagaga tggtggcaca agggcgccct ctaccgcatc 480

ggcgaccttc aggccttcgt aggcccggaa gcgagaggca tagctggtct gaagaaccat 540

ctggagtact tgagcaccct gaaggtgaag ggcctagttt tgggcccaat tcacaagaac 600

cagaaggatg aagtcaatga aaccgacttg aaacagattg atcccgattt aggctcccag 660

gaagatttta aagaccttct acaaagtgcc aagaaaaaga gcattcacat cattttggac 720

ctcactccca actataaggg ccagaatgca tggttcctcc ctcctcaggc tgacattgta 780

gccaccaaaa tgaaggaggc tctgagttct tggttgcagg acggtgtgga tgggttccaa 840

gttcgggatg tgggaaagct ggcgaatgca tccttgtact tggctgagtg gcagaatatc 900

accaagaact tcagtgagga caggcttttg attgcaggga ccgcgtcctc tgacctgcaa 960

caaattgtca acatacttga atccaccagc gatctgctgc tgaccagctc atacctgtca 1020

cagcccgttt tcactgggga gcatgcagaa ctcctagtga ttaagtattt gaatgccact 1080

ggcagccgct ggtgcagctg gagtgtgtcg caggcaggac tcctgacatc ctttataccg 1140

gctcagtttc tccgactcta ccagctgctg ctcttcactc tgccaggaac tcctgttttc 1200

agctatgggg atgagcttgg ccttcaggca gttgcccttc ctggacagcc tatggaggct 1260

ccattcatgc tgtggaatga gtctagcaac tcccaaacct caagtcctgt aagcctcaac 1320

atgacagtga agggccaaaa tgaagacccc ggctccctcc tcacccagtt ccggcgactg 1380

agtgacctcc gtggtaagga gcgctctctg ttacacggtg actttgatgc actgtcttcc 1440

tcatctggcc tcttctccta cgtccgccac tgggaccaga atgagcgtta cctggtggtg 1500

ctcaacttcc aggatgtggg cctgtcagcc agggtaggag cctccaacct ccctgctggc 1560

ataagcctgc cagccagtgc taaccttttg cttagtactg acagcacccg gctaagccgt 1620

gaggagggca cctccctgag cctggaaaac ctgagcctga atccttatga gggcttgttg 1680

ttacagttcc cttttgtggc ctgatccctc tacacagaac ctgccaccct tctttcctct 1740

ctcaggcctt tggaattctg gtctttctct ccttattttg tttttgtttt taaacttttg 1800

cagattacat atgaattctt acactgggtg tttttgtctt caaaataaaa aaaatccccc 1860

ctgcaaaaaa aaaaaaaaaa aaaaaaaaaa aaaaaaaaaa aaaaaaa 1907

<210> 613

<211> 2509

<212> DNA

<213> Rattus norvegicus (Norway rat)

<400> 613
ccatagcgaa gacttcatga agactgtccc aggcatgctg tgacacaaac tacagaaggt 60

gggaaaagat ctttgtggtc aaaccatccg gaccttggct accgagacag aacaatactg 120

accgcattca ctcatacaca gttctcggca cctcccagtg ctcagagcag accctcaagg 180

agatgagcag atccaggatg gggagcccaa tgcaccgagt gtccctgggg gaccactgga 240

gctggcaagt gcacccggac atagacagcg aaaggcactc accgtccttc agtgtggagc 300

gacttaccaa catccttgat ggaggcctcc caaacaccgt gctgcgaaga aaagtcgaaa 360

gcatcataca aagtgaccca gtgtttaatt tgaagaagct ttacttcatg acccgagagg 420

agctatatga ggatgcgatt caaaagagat tccatctcga gaagctagcc tggagcctgg 480

gctggtcaga agatggtcct gaacgcattt atgctaacag agtccttgat ggaaacgtca 540

acttaagctt acatggtgtt gccatgaatg ctatccgaag cctgggctca gatgaacaga 600

ttgctaaatg gggccaactc tgcaaaaact tccaaatcat cacaacatac gcccagacag 660

agctgggaca cgggacatac ctacagggcc tggagactga agccacctat gatgaagcca 720

ggcaggagct tgtgatacac agccctacga tgacttccac caagtggtgg cctggggact 780

tgggatggtc ggtcacccat gctgtggtcc tagcccagtt gacctgctta ggagtccggc 840

acggcatgca cgccttcatt gtgcccattc ggagcctaga ggatcacacc ccactgccag 900

gaatcacagt tggggacata ggccccaaga tgggtttgga acacatagac aatggcttcc 960
tgcaactgaa ccacgtgcgg gttcccagag aaaacatgct cagtcgcttt gcagaggtct 1020
tgccagatgg tacctaccag aggcttggga cgccacagag caattatctt ggcatgttgg 1080
tgacccgggt gcagctgctg tgtaaaggaa tcctaccctc cctccagaag gcttgcatca 1140
ttgccacgcg ctactcagta atccgccatc agtctcgact tcggcccagt gacccagagg 1200
caaaaatcct ggaataccag acgcagcagc agaaactcct tcctcagctt gctgtgagct 1260
atgccttcca cttcacggcc accagcctct cagaattctt ccacagctcc tacagtgcta 1320
ttctgaagag agacttcagc ctcctgcctg agctccatgc attgagcact ggtatgaagg 1380
ccacgtttgc agacttctgt gcccagggcg ccgagatctg tcgcagagct tgcgggggcc 1440
atggctactc aaagctgagc ggcctgccga cactggttgc tcgagcaaca gcctcttgca 1500
catatgaggg tgagaatacg gtgctctacc tgcaagtggc caggtttctg atgaagagct 1560
atctgcaggc tcaagcgtcc ccaggcgcca caccacagaa gcctctccct cagtccgtca 1620
tgtatattgc cacacaaagg ccagccaggt gctcagccca gactgcagct gacttccgct 1680
gcccagatgt ctataccaca gcctgggcat atgtgtctac caggctcata agagatgcag 1740
cacaccgtac acagaccctc atgaagtccg gggttgacca gcatgatgcc tggaatcaaa 1800
ctactgtcat ccaccttcag gctgctaagg ctcactgcta cttcatcact gtgaagaatt 1860
tcaaggaagc tgtggagaaa ctagacaagg aaccagagat tcagcgtgtg ctccaacgcc 1920
tctgtgacct ctatgcctta cacggtgttc tgactaactc aggggacttt ctgcatgatg 1980
gcttcctgtc tggggcccag gtggacatgg ccagagaagc cttcctagac ctgcttccct 2040
tgatccggaa ggatgccatc ttgttaaccg atgcttttga cttctcggac cattgtttaa 2100
actcggcact tggctgttat gatggacacg tctacgaacg cctgtttgag tgggctcaga 2160
agtacccagc caatactcag gagaaccctg cctataagaa gtatatccga ccactgatgc 2220
tcggctggag acacaagatg tgaaaagtca aaggatttgg gaccgagaag caccacggcc 2280
ttactatggc acatatacat agagaattta aagcacgggg gggggggggg ggggggtgct 2340
gctcggttaa atcaggtagt aaattggtac atgaatggat ggtcatccta ttagtctact 2400
attgagcatg tttgaaactt tcccttgtcc atctatagca tgtatttggc taaatgctaa 2460
aatttttgtt ttacatacag gaaaagctaa taaacttgtc agttacaaa 2509

<210> 614

<211> 3548

<212> DNA

<213> Rattus norvegicus (Norway rat)

<400> 614
gttacaaggt tgtcgaggtg cttgccctca cctgctgaac catcccaccc tgggcagcac 60

ttcctgatta ttaaggcttg tgaaacacct gcctaggtgt tcaaatggcg ctttacaagc 120

aatctatatg aggcgataga atcttttgaa atagaaatga cctgtttcct ttattttact 180

gtattcctaa tatacgtctt attttaatct aaatagccac ttgtgtctaa tagcaatagg 240

aaatgaaact tgacctcaag aagcctattt ccagaatgtc aggaaccagt gacaaaactt 300

gtttcccaaa tgtaattttc tgagcctgcg gcatcatgtt atctttaaaa aggactaaag 360

ggtgcatcat actgctattt tgagcacaaa agagcagcct tctctgaggc ccacacctct 420

gtacacttcg atactaacat tccggtaagg caaagctctt agaagtatct aaagggatac 480

cccaggctag taaggaatgt ttcctgccca aagattaccc aatagcttcg ttttggtgtc 540

ctaagaggga aattcaatac aaattccgat tcatcagcaa tttcttccag tttctcctac 600

aattcaagtt cctacgactt aagtgaaagc tttatttttt taaacaccag aaagagttcc 660

aatttgcaag ttggggaaaa tggtgcaaaa tttatacaaa cactgaagaa gaccgtgtcg 720

catgggcaag ctaaatgcat gattaccggt ccgacagctg agcaccctcc caccagcctt 780

ctcctgcagt cttcctggct gctggcttca aaactcccta ggaaaaagcc tgagatgtca 840

atcttatgac cttgatattt tcctggctgg tttttaacca ccatcaccgg cctacatact 900

cagaaataac agacaatctg acaactttct tttcatgcta aactttgata aaaacagcca 960

cagtcctggg aaggtgagcc acttccaaga catacatcaa tttgaccaga gacaacacaa 1020

ccagtccccg gcaagccacg cccatggggg gaggggcttg gactgcgcgt gcgtgctggt 1080

tccattccaa atcctggccc cctggaaaac cggactagaa aagctgtccc gctgcctccc 1140

agctgcagag tttaccccag gttctttggt ctccgacggc ctttctacgc acacgcagcc 1200

atgtcttccg acagacccgt ggcactggtg actggtgcta acaaaggaat cggattcgcg 1260

atcgtacgtg atctctgtcg taaattcttg ggggacgtgg tcctcacggc gcgggacgag 1320

tcacggggcc acgaggcggt gaagcagctg cagaccgagg gcctgagccc acgcttccac 1380

cagctggaca tcgacaaccc gcagagcatc cgcgcgctgc gtgactttct gcttcaggag 1440

tacggaggac tgaacgtgct ggtcaacaat gcgggcatcg ccttcaaagg taagccacag 1500

ggtgtggtga cttgcccaaa gcctgggacc agagtggact gtgcctctct ggggagcatt 1560

actaggatca gaagtactat gatgtaggac tctgaaacag atcccctagg agaaacgctg 1620

cagggtagcg gctgcaggcc tccaggaaag cagagcctta atggtcgcca tcatttccag 1680

gttcccctca agcggttgtt gagtaattgg gcttagcaat acttaagttg aattatatct 1740

agtaaataaa caagagattg aggggagaga gacaggggga aaagtgttac attacgatcc 1800

tgatacacaa ccttcactca gcctccccca gtggtggtgt gtttcaaaaa caatagaaca 1860

ttgtcagata cacaatgggg cagtgctgca actaaacaca gaagtacttg gaagtggatt 1920

tctaatgctc tggaactgtc cgcccagcta actctgttct ctttccctct gaaagttgtt 1980

gaccccaccc ccttccacat tcaagcagag gtgacaatga aaaccaactt ttttggtacc 2040

caagatgtct gcaaggagct actccctata ataaaacccc aaggtgagtc tggcccagct 2100

cctctccctc tctgcacctg cctctctgcc acttggaggt gtggctctgc tccttggggt 2160

ctgatctttt gtagccattg tcgctatctt tgcataccca gctcttcttt aacctttcag 2220

tatgattcca aggtcccttg gttccacctc ctaacccctg agggggtttt aggattccaa 2280

ttatctcaat acacttcaca tacatactga catcacactg ctcacaatag cctgtcccca 2340

caagtcccca ggttggaccc aagtgcctcc ctcagctcag tctcacagca ggtctcagct 2400

gtcaagctgc actcctgcaa accctgaggg ggcctccttt ctgcagcagc cttttttcct 2460

gggcttctac acctcttaca gaacccttcc cctcctaaaa atcttttagg aagtagatca 2520

ggtctttagc ttggttcaca ggactctgta atctgacctt ccttcctctg ggctcatgtt 2580

agcgccccat caacaagctt cacctttctg caaaacaagc tatcacatga ctttcgtcgt 2640

gaagtgctgt aacaccccca aatctctgga tagcatatga accactgctc attaaataca 2700

caccagtaaa gacaagtgag ctgctttgga aatgtgagtc tctggtacag agttgactaa 2760

gtgtagctct ctcaagactc ctctctccag aatgccctct ggaccagacc tcatggtcat 2820

ctggcttcgc tacccaccca aaccttgacg gcgagacagc agattctgtg ctatttgagt 2880

ccttttctgt gcatccctgt ctcaactgaa atcttgggga tctaatgtct caccgtttgt 2940

aaagtgttgc tcccgtgtcc gcatctctac atgatgatgt gttttcaggc agagtggtga 3000

atgtatcaag cagcgtgagt ctcagggccc tgaaaagctg cagcccggag ctgcagcaga 3060

agtttcgaag tgagaccatc actgaggaag agctggtggg gctcatgaac aagtttatag 3120

aggatgcaaa gaaaggagtc catgcgaaag aaggctggcc caatagtgca tatggggtca 3180

ccaagatagg ggtgacagtc ctgtccagaa tctatgccag gaaactcaat gaggagagga 3240

gagaggacaa gatcctcctg aatgcctgct gccctgggtg ggtcagaacc gacatggcag 3300

gaccaaaagc caccaaaagc ccagaagaag gagcagagac ccccgtgtac ttggcccttt 3360

tgcctccagg tgcagagggg cctcacgggc agtttgttca agataaaaaa gttgaaccat 3420

ggtgaatcca actctcaccc ccaccccttc tatcctgact tggtgaaagc caagggacat 3480

ttataatata ccatcacttc tggaaaaata aacataacta agtctttaag cacacaacag 3540

gtgtttgc 3548

<210> 615

<211> 1780

<212> DNA

<213> Rattus norvegicus (Norway rat)

<400> 615
gccttcagtt tcctgtacta agtgcttctg cccaccagag caaccgattc taaggcctgg 60

ctctagcaat ggccttcgca aatctgcgca aaatactcat cagtgatagc ctcgacccct 120

gctgccggaa gatcctgcaa gatggagggc tgcaggtggt ggagaagcag aacttgagca 180

aggaggagct gatagccgaa ctccaggact gtgaaggcct tatcgtccgg tcagctacta 240

aggtcactgc tgatgtcatc aatgcagcag agaagctcca ggtggtgggc agggctggta 300

caggcgtgga caatgtggat ctggaggctg ccacaaggaa gggcgtcctc gtcatgaaca 360

cccccaatgg aaatagcctc agtgctgcgg aactcacctg tgggatgctc atgtgcctgg 420

ccaggcagat cccccaggcg acggcttcga tgaaagatgg caaatgggac cggaagaagt 480

tcatggggac agagctgaac gggaagacac tgggaattct tggcctgggc agaattggaa 540

gagaggtggc cgcccgaatg caggcctttg gaatgaagac tgtaggctat gaccccatca 600

tttctccaga agtcgctgcc tcctttggtg ttcagcagct gccgctagag gagatctggc 660

ctctctgtga tttcatcact gtccataccc cgctcctgcc ctccactaca ggcttgctca 720

atgacagcac ctttgcccag tgcaagaaag gcgtgcgggt ggtgaactgt gctcgaggag 780

gcattgtgga tgaaggtgcc ctgctccgtg ccctgcagtc tggtcagtgt gctggtgctg 840

cactggatgt gtttacagaa gagccaccac gggaccgggc cttagtggac cacgagaacg 900

tcatcagctg tccccacctg ggcgccagca ccaaggaggc ccagagccgc tgtggggagg 960

aaatcgcagt ccagtttgtg gacatggtga aggggaaatc tctaacaggg gttgtaaacg 1020

cccaggctct taccagtgcc ttctctccac acaccaagcc ttggattggt ctggcagaag 1080

cattgggcac gctgatgcac gcctgggctg gctcccctaa agggaccatc caggtggtga 1140

cacaaggaac atctctgaag aatgctggga cctgcctgag ccctgcggtc attgtcggcc 1200

ttctgagaga agcatcaaaa caggcagatg tgaacttggt gaacgctaag ctactggtga 1260

aagaggctgg cctcaatgtc accacctccc acagtcctgg tgtcccagga gagcagggca 1320

tcggggaatg cctcctgact gtggccttgg caggtgcccc ctaccaagct gtgggcttgg 1380

tccagggcac cacaccaatg ttgcagatgc tcaacggagc tgtcttcagg ccagaggtgc 1440

ctctacgcag ggccagcccc ctgctcctgt tccgggctca gccctccgac cctgtcatgc 1500

tgcccactat gatcggccta ctggcagagg cgggggtaca gctgctgtcc taccagacct 1560

ccaaggtgtc tgacggagac acttggcacg tcatgggcct ctcctcccta ctgcccagcc 1620

tggacgcatg gaagcagcat gtttctgagg ctttccagtt ctgcttctga cccaggggct 1680

cagcggtccc agcccctcag gctcttctga ggaaacccgc tcactgtgac ctgaactaat 1740

atctagtaaa gaatctaact ccaaaaaaaa aaaaaaaaaa 1780

<210> 616

<211> 140

<212> DNA

<213> Rattus norvegicus (rat)

<400> 616
ctatgatcgg cctactggca gaggcggggg tacagctgct gtcctaccag acctccaagg 60

tgtctgacgg agacacttgg cacgtcatgg gcctctcctc cctactgccc agcctggacg 120

catggaagca gcatgtttct 140

<210> 617

<211> 7225

<212> DNA

<213> Rattus norvegicus (Norway rat)

<400> 617
aagcttcttc ccagaacctg gaagcccagc aagcaaatgc cctgccccca cccgacctcc 60

gccctcgttg gcttcgcaaa gctgtgatct gtggccagta gagggcacac tttactttcg 120

caaatctgag agccacaacc cgggggaggg ggcggggaaa gagtctctgc agcaaaatgc 180

agcctaggga ttggtggctc ttggcgtttg aggcaaaatc ctagaggctg tagtcatttt 240

gcaatcctta aagctgaatt gtgcaatgag ctcgatgaag gaagatacta tcatccaaca 300

gctgaatcct aaattgcaaa ctcaatggct aatgactact ttgaacaatg cgcaccttac 360

acacgctaca cgctactgta gtctcttctt tttttagggg gtagcagtga gaggtttctt 420

aaaatgcctt ggggcgagga gtcctgaatg aggagactgc tttgggtttt gatgcgtagg 480

ataagcaaat cccgaggaaa tatgcactat ataataaatc tagaaccaaa acacagagca 540

aaagattcat gtttcacgtt ggttaataaa ctaggttatc gtgtatatat taagtgtgta 600

tacctttggg gactctacaa aatgcacggc atggtattga ggaaaaagta aattgggaaa 660

ttagtgtaaa aattagagtc ggcttttaat tagtttaaca cacacatacg aaggcaaaga 720

cacaacgtta ctttgatctg accggggccg accttttttt aagtgcataa ttacgattcc 780

agtaataaaa ggggaaagtt tggggtttgt cctgagagga aggggttaac ggtttccttt 840

attctagggt ctccgcaggc tccccagatc tgggttggca attctactcc tccccctttc 900

tgggaagtcc gggtttcccc caacccccaa tccatggcat attctcgcgt ctagcgcctt 960

gattcccgcc cccccacacc cccaacctct aagccggagt ctggtgcaaa ctggctccac 1020

aggggcaaag aggatttgcc tttagtggaa actggctgtg gaactggaac tctgagtgga 1080

ggtgcatggg gtgtagactg gcagagactc gaccgggagg atccgggtag agcgcgccca 1140

ccgccgcagt cctactctac tccagctctg gaacgcgcag aaagacctac ggggcaagga 1200

gccgcctcca ccccaccccg cctggtggaa ggtccgaacg ggaggtgttg gagtggaggg 1260

cctttctggc agcaaattgg gggattcatt ctgtaaagaa tgtgcccagt caacatagct 1320

gtacgcccaa acgcaaaata cacactgccc tccccgagag atgcagtggc tgtttattcc 1380

taagtggctc tccaagtata cgtggcattg agttgctgag caattttaat gattccaggc 1440

atcgtttttc tgctcagacc tcatctgtcg taggtcttca actatcactc cacactgagc 1500

aagggctcct acataactct tttttttttt cgtccttccc tttttaaatt ctgtttcccc 1560

gaccttagag agacgactgg ccgcccgaga cgtgcgtgac gcggctcagg gtacacggcg 1620
tattgtgtgg agcgaggcag ctgttccacc tgcggtgact aatacacgca ggacaagaac 1680
acagttcagc cgagcgctgc gcccgaacaa ctgtacagaa agggaaagga ccgacgcgcg 1740
agcaggagaa aatggtcggg cgcgcagtta attcatgctg cgctattact gtttacaccc 1800
cgagtcggag tactgggctg cgggactgag gctcctcctc tttccccggc tccccactag 1860
tgcccctccc gagttcccaa agcagagggc gggggagcga gaggaggaaa aaaatagagg 1920
tcgggagggg agggagaaag agattctctg gcctaatccc cgcccacccg ccctttataa 1980
tccgggggtc tgcgcggcta aggacccccg ggctgcgctg ctctccgctg ccgcctccgc 2040
cgcgcccact ccgctcgcct cctgcctcca aaagggcagg cgttcgccga ggcttggcgg 2100
gaaaaagaag cgaggggagg gatccggagt cgcagtataa aagaagcttt tcgggcgttt 2160
tttttctgac tcgctgtagt aattccagcg agagacagag ggagtgagcg ggcgggttgg 2220
aagagcccag tgtgcagagc cccactccgg gcttcctagg aaggcagctc tggagtgaga 2280
agggctttgc ctccaggctt gctgcctcct cgacccaatc ctcccgctga cccaacatca 2340
gcggtcgcaa ccctcgccgc ctctgggaaa ctttgcccat tgcaacgggc agacacttct 2400
cactggaact tacaatctgc gagccaggac aggactcccc aggcgcaggg gagggaattt 2460
ttgtctattt ggggacagtg ttctctgcct ctgcccgcga tcggctcccc tgaaaagagc 2520
tcctcgcgtt atttgaagcc tgaatttcct ttgggaggtg gaaaacccgg taagcacaga 2580
tctgttggtc ttctactgtt ttctctacgt cctgagtgta gcgaccggtt agaacagtct 2640
ttattcaatt tctgtgcttt tgacacttta ctcagagtag ttggggtagg ccggggcgga 2700
gttggggtgg atctgagtcg gggtagaacg acttgtcaag atgacaggaa aggggaagga 2760
aaaaaccggg gtgcattttg aaactgtctt ccagaggtta ccgtgggctg acgctgaccc 2820
gggccggttg gacattcttg ctgtgctaca ttaattgata tttatgtcct ttgggggggg 2880
ggtgtcaaag ccggaggtct cttcgcggtg gccaaaggaa gcccttcgta tcctgaggtc 2940
tttgggaaag ggattacctt ttgcgtgttc tccgcaaaaa gcgagaaggc tccgtagcct 3000
ctgacttccc agtctttggg agggcattta aatttcggct tggtgcattt ctgacagcct 3060
gagaccaact cgggggtgcg tcccgcccgc caatccccgg cggcgattgc aaccggtccc 3120
tgatcctttt aagaagttgc cattgggctt taaaaatagt gatcatagta aaatttaagc 3180
ctaaccctcc gcggtattag gacttggtgt tggactagcg cactgaggag aggcaaaact 3240
ggggcaggga tgtgacagat tcgttgactt ggggaaaacc agagggaatc ctcacattcc 3300
tggttgggat ccgcgggtat tccccgcgcc cctgaattgc tagggagact gcggtgagtc 3360
ttgatctgag ctgttcggta acagctgcta ccctcggcgg ggagagggaa gacgccctgc 3420
accgggtgct gaatcgctgt ggggtctctg gcgcagtggc gtcgcggttt agagtgtagc 3480
agggaggtgt ctcttattat ttgacacccc ctcccctttg gagaggcttg ttagagcctg 3540
agactgagct ctcccctcca agtcagcaat aggaaagaaa agccggcaaa ggaaggaagg 3600

ggggcgcgct gggggtggag aaagaggagg gcggagaggg gcggcggcgc cggctgggta 3660

agagccgggc gacggcgcga gtagggaccc ggacccggtc ggcggcgcaa agagccggca 3720

ccggaggggg ccgagcaccg cggcgcctct cgcctttctc cttcaggtgg cgcaaaactt 3780

tgcgcctagg cttttggcag actgtattcc ctacagtcgc ctccctcagc ctctgaagga 3840

ggccaaggcc gatggcgatt cctgggcgtc tgcagtgcta agtccctgct ctaaggaggc 3900

ggggactccg agcagctgct agtccgacca gcgtcactga tagtagggag taaaagagtg 3960

cattcccccc aaccacacac acacacacac acacacacac acacacacac acacacacac 4020

acacacttgg aagtacagca ggctgaaagg ggagtggttc aggattgggg tacgcgctgc 4080

gccaggtttc cgcatcaacc agagctgggt aactctagac ttgcttccct tgctgtgccc 4140

cctccaacag acagtcacga cgatgcccct caacgtgagc ttcgctaaca ggaactatga 4200

cctcgactac gactcggtgc agccctattt catctgcgac gaggaagaga atttctatca 4260

ccagcaacag cagagcgagc tgcagccgcc cgcacccagt gaggatatct ggaagaaatt 4320

cgagctgctg cccaccccgc ccctgtcccc cagccgccgc tccgggctct gctctccgtc 4380

ctatgtcgcg gtcgctacgt ccttctcccc aagggaggac gatgacggtg gcggtggcaa 4440

cttctccacc gccgatcagc tggagatgat gaccgagcta cttggaggag acatggtgaa 4500

tcagagcttc atctgcgatc ctgacgatga gaccttcatc aagaacatca tcatccagga 4560

ctgtatgtgg agcggcttct cggccgctgc caaactggtc tccgagaagc tggcctctta 4620

ccaggctgcg cgcaaagaca gcaccagcct gagccccgcc cgcgggcaca gcgtctgctc 4680

cacctccagc ctgtacctgc aggacctcac cgccgcagcg tccgagtgca tcgacccctc 4740

agtggtcttc ccctacccgc tcaacgacag cagctcgccc aaatcctgta cctcgtccga 4800

ttccacggcc ttctcttctt cctcggactc gctgctgtcc tccgagtcct ccccacgggc 4860

cacccctgag cccctagtgc tgcatgaaga gacaccgccc accaccagca gcgactctgg 4920

taagccgccc caaccccagc agggtaggaa gccagaggtt ggatggacct ccttctcatt 4980

ggcattaatt caattggccc ccgggctcac cctttctttc ccttcggtct aagctcttca 5040

tccctggact cctgtgcttc agatatccct ccccttcccc cagaccgccc tggttgtcag 5100

ccccaccccc acccttccca cccctcagga atttcatttg ggtttttaaa tcttctggct 5160

tatctttcaa ctcaatccac tccctttacc cctcctaggc attttaatta cccagagaag 5220

ggtgtgaagg aggataggga actgatcctg agaagagtga atgaattacc tgcttttctt 5280

tgacttcaga agaatatttg aatttaatgt ctacaagacc caagagaagc aatgacagaa 5340

gcaggacagc ctttagcctc agagccaggc actagtgaaa gatcctgaag agttgaagag 5400

ctgttttccc tccatcccgt tttgaacact tcaaagcaaa tcctcttcag tttggatttt 5460

cgtaccccct ccctccccaa atgctcgccc caccactgcc accaccagga caggacgttt 5520

ggcaaaacca caagacttta ctattgcact tccagtaata tagggagttg gtaaagtcat 5580

agcaagatat ttgcagctat cgcctacagg acctgaaggg ttcttggtga agtcccttaa 5640

aaatgggagg tgcttgggaa tgtgctttgc tttgggtgtg tctgaagcct cattaagtct 5700

taggtaagaa ttggcaacga tgccatatcc tggtacatgg taattttctc acctgtgccc 5760

taaccctgtt ctacctttct gggaaacggg aggatggtgt ctagatctgg ttcttacttc 5820

tcaatccttt ccaacttggc acttggatag catcggtcaa acccccatct gggattcagg 5880

aggcattgct aactaatctt ccacttcctt ccttacagaa gaagaacaag atgatgagga 5940

agaaattgat gtggtgtctg tggaaaagag gcaaccccct gccaagaggt ccgagtcagg 6000

gtcatcccca tcaagaggcc acagcaaacc tccacacagc ccactggtcc tcaagaggtg 6060

ccatgtctct actcaccagc acaattatgc agcacccccc tccacaagga aggactatcc 6120

agctgccaag agggccaagt tggacagtgg cagggtcctg aaacagatca gcaacaaccg 6180

caaatgctcc agccccaggt cctcagacac cgaggaaaac gacaagaggc ggacacacaa 6240

cgtcttggaa cgtcagagga gaaacgagct gaagcgtagc ttttttgccc tgcgcgacca 6300

gatccctgag ttggaaaaca acgaaaaggc ccccaaggta gttatcctca aaaaagccac 6360

cgcctacatc ctgtccgttc aagcagatga gcacaaactc atctcagaaa aggacttact 6420

gaggaaacgg cgagaacagt tgaaacacaa actcgaacag cttcgaaact ctggtgcata 6480

aactgaccgg aagtgaggag gagctggaat ctcgagtgta aggagaacgg ttccttctga 6540

cagaacttgg acttcaaaaa atgcatgctc aaagcctaac ctcacaacct tggctggggc 6600

tttgggactt cagccataat gttaactgcc tcaaagttaa ggcataaaag aacttttttt 6660

tatgcttccc atcttctttc tttttccttt aacagatttg tatttaattg ttttttttaa 6720

aaaaatcttc cggtgtacat agggccttta aatgtaaata actttaataa aacgtttata 6780

acagttatac aagattttaa gacatgtatg ataaaccata atttttttta tttaaagacc 6840

ttttcatttt taaagttgat ttttttctat tgtttttaga aaaaataaaa taattggaaa 6900

aaatataatt gagccaactc ttaaatggtg ttttcttttt tttttttcct ccaatttctc 6960

ttcctcaact atagatgaac agaactgacc gtcttcccag gacgggttta caagggtggg 7020

aaggggttat cttaaaatgg ggcgggggag gggggtgcct caagacctct aagttcccag 7080

tgttatgaga tcctttctgt ggatgctgat aggatgagag ttgagaaagg gcaaaaggca 7140

agtgaggtta gatacaagga acagggaagc aagaggttaa agatagcaga cagggaagag 7200

atggcagaat gaatctcctg cagtt 7225

<210> 618

<211> 292

<212> DNA

<213> Rattus norvegicus (rat)

<400> 618
gaaactctgg tgcataaact gaccggaagt gaggaggagc tggaatctcg agtgtaagga 60

gaacggttcc ttctgacaga acttggactt caaaaaatgc atgctcaaag cctaacctca 120

caaccttggc tggggctttg ggacttcagc cataatgtta actgcctcaa agttaaggca 180

taaaagaact ttttttatg cttcccatct tctttctttt tcctttaaca gatttgtatt 240

taattgtttt ttttaaaaaa atcttccggt gtacataggg cctttaaatg ta 292

<210> 619

<211> 1662

<212> DNA

<213> Rattus norvegicus (Norway rat)

<400> 619
cattcagttt agtcagaatc catgggtggg ctcagctgtc tcctccgtac ctagtctgcg 60

gttatggctt cgctcacggt gaaggcctat ctactgggca aggaggaggc ggcccgcgag 120

atccgccgct tcagcttctg cttcagcccg gagccggagg cggaagccgc ggctggcccg 180

gggccctgcg agaggctgct gagccgggtg gctgtgctgt tccccgcgct gcggcctgga 240

ggctttcagg cgcactaccg cgatgaggat ggggacttgg tcgccttctc cagtgatgag 300

gaactgacaa tggccatgtc ctatgtgaaa gatgacatct tccgcatcta cattaaagag 360

aagaaggagt gccggcggga acatcgcccc ccatgtgctc aggaggcacg aagcatggtg 420

caccccaacg tgatttgtga tggttgcaat gggcctgtgg tgggaactcg ctataagtgc 480

agtgtgtgcc ccgactacga cctgtgcagc gtctgcgagg ggaagggcct gcacagggag 540

cacagcaagc tcatctttcc caaccccttt ggccacctct ctgatagctt ctctcatagc 600

cgctggcttc ggaagctgaa acatgggcac tttggctggc ctggctggga gatgggccca 660

ccagggaact ggagcccacg tcctcctcgc gcaggggatg gtcgcccttg ccccacagct 720

gagtcggctt ctgctccatc agaggatccc aatgtcaatt tcctgaagaa tgtgggggag 780

agcgtggcag ctgccctcag ccctctaggc atcgaggttg acattgatgt ggaacatgga 840

gggaagagaa gccgcctgac acccacctct gcagaaagtt ccagcacagg cacagaagat 900

aagagtggta ctcagccaag cagctgctct tcggaagtca gcaaacctga cggggccggg 960

gagggccctg ctcagtctct gacagagcag atgaagaaga tagccttgga gtcggtggga 1020

cagccagagg aactgatgga gtcggataac tgctcaggag gggatgacga ctggacgcat 1080

ttgtcttcta aagaagtgga cccatccaca ggtgaactcc agtctctaca gatgccagaa 1140

tcggaagggc caagctctct agacccctca caggaaggcc ccacagggct gaaggaagct 1200

gccctgtacc cacatctccc accagaggct gatccccggc tgattgagtc actctctcag 1260

atgctgtcca tgggtttctc ggatgaaggc ggctggctca ccaggctcct acagaccaag 1320

aattatgaca tcggggctgc tctggacacg atccagtatt caaagcaccc tccaccattg 1380

tgacagtgct gtggccaagt cccacaaccc acctcccttg tcttctagtt gcatcatgta 1440

gagtagcagg gcttctaagg cccagtgtct tggcattctt ctagaacctt caggtgggac 1500

tgtgaggcct tcttaggcag taggaaagtg catgagaaga gagtctgagt gtgcacatgc 1560

tgacccctga gcacagatcc aagcagctgt ggctgggctt mcgctgcttt ccctcggcct 1620

ggcctttgcc agggagctgt ggagtcatgc tgcactccac tt 1662

<210> 620

<211> 1711

<212> DNA

<213> Rattus norvegicus (Norway rat)

<400> 620
cgggcctacg gctcagtcta aggactgcaa ataggcagct ggccactaga ggatctctaa 60

cttttcctac gaaactgagg gctgaagtca aagatacaaa atggtggcct cgtctttcgc 120

tgtcctgaga gcaagcaggt tgtgccaatg gggttggaag agctggacgc agctgtcagg 180

tcctccgccg ctcagcaccg gtggccggac cacttttgcg cggacaaatg ctacgctgag 240

cctggagccc gggagccgca gctgctggga cgagccgttg agcatcaccg tgcgcggcct 300

ggcccccgag cagcccgtca cgctgcgcgc ggccctgcgt gacgagaagg gcgcgctctt 360

ccgagcccac gcgcgctacc gcgccgacgc cggtggtgag ctggacctgg cgcgcgctcc 420

cgcgctgggc ggcagcttca cggggctcga gcccatgggg ctgatctggg ccatggagcc 480

cgaacggcct ctctggcgcc tggtcaagcg cgacgtgcag aagccttatg tggtggagct 540

ggaggtgctg gacggacacg agcccgacgg cggtcagcgg ctggcacagg cagtgcacga 600

gcgtcacttc atggctccag gggtgcggcg cgtgcccgtg cgcgacgggc gggtgcgcgc 660

cacgctcttc ctgcccccag aacctgggcc ctttcctgaa atcatagacc tttttggagt 720

tggaggcggc cttctggagt accgggcgag tctgctggct gggaagggtt ttgccgtcat 780

ggctctggct tattacaact acgacgacct ccccaagacc atggaaacca tgcgcattga 840

gtactttgaa gaagccgtga actacctgcg tggccaccct gaggtaaaag gaccaggaat 900

tgggctgctt gggatttcca aagggggtga acttggcctt gctatggcct ccttcctgaa 960

gggcatcacg gctgctgttg tcatcaatgg ctccgtggct gctgttggga acaccgtatg 1020

ctacaaggat gagactatac cccctgtgtc ccttctgaga gacaaagtca aaatgaccaa 1080

agatggtctc ttggatgtcg tggaagctct gcaaagccct ttggtagaca agaagagctt 1140

catccctgtg gaaaggtctg acacgacctt cctgttcctc gttggtcagg atgaccacaa 1200

ctggaagagc gagttctatg ccagagaggc ctccaaacgc ttgcaggccc acgggaaaga 1260

gaagccccag atcatctgct acccagaagc agggcactat atcgagcctc cttacttccc 1320

actgtgcagc gctggcatgc acctcttggt gggtgctaac atcacctttg gaggggagcc 1380

taagcctcac tctgtggccc agttggatgc atggcagcaa ctccagactt tcttccacaa 1440

acagttgagt ggtaagagtt aggaggtgcc ccctaaaata taacctgtta tgtggtggtt 1500

tggggaaaaa cccaaatatc agaatgccac ttcagtttag ttcatttgaa cacatactaa 1560

tttttttaag tttctttctt ccttcctttc tttctttctg tttttttttt ttgttgttgt 1620

tgttgttgtt tgtttgtttg tttgagacag ggtttgtctg tttacccctg gctggcctgg 1680

aacttgcttt gtagaccaga ggctaggcct g 1711

<210> 621

<211> 2817

<212> DNA

<213> Rattus rattus (black rat)

<400> 621
gggcgcggcc tggcgggccg ggcggcgctg cagcccatgg agctcgagaa catcgtagcg 60

aacacggtgc tactcaaggc ccgggaaggt ggtggcggga atcgtaaagg caagagcaag 120

aaatggcgcc agatgctgca gttcccccac atcagccagt gtgaagagct ccggctcagc 180

cttgaacgtg actaccacag cctgtgtgag cgtcagccca ttgggcgcct gttatttcgt 240

gagttctgcg ctacgaggcc tgagctgacc cgctgtactg ccttcctgga tggggtggct 300

gagtatgagg tgacccctga tgagaaacgg aaggcatgtg ggtgtcggct aatgcagaat 360

tttctgagcc acacgggtcc tgacctcatc cctgaagttc cccggcagct ggtgagtaac 420

tgtgcccagc ggctagagca gggaccctgc aaagacctct tccaggagct gacccggctg 480

acccatgagt acctaagcat ggcccctttt gccgactacc tcgacagcat ctacttcaac 540

cgtttcctgc agtggaagtg gctggaaagg cagccagtga ccaaaaacac ctttaggcag 600

taccgagtcc tgggcaaagg tggctttggg gaggtgtgtg cctgccaggt gcgggcaaca 660

ggcaagatgt atgcgtgcaa aaaactggaa aagaaacgaa taaagaagcg gaagggggag 720

gccatggctc tcaacgagaa gcagatcctg gagaaagtga acagtaggtt tgtagtgagc 780

ttagcctacg catatgagac caaggatgca ctgtgcctgg tgctgacatt gatgaatgga 840

ggcgacctca agttccacat ctaccacatg ggccaggctg gctttcccga agcacgtgct 900

gtcttctatg ctgccgagat ctgctgtggt ctggaggact tacaccggga acgcatcgtg 960

tacagggacc taaagccaga gaatatcctt ctggatgacc atggccacat tcgaatctcc 1020

gacctgggcc tgactgtgca tgttcctgag ggccagacca tcaaaggccg tgtgggcact 1080

gtgggctaca tggctccaga ggtggtgaag aatgagcgct acacattcag tcctgactgg 1140

tgggcgctag gctgcctcct gtacgagatg attgcgggcc agtcgccctt ccagcagagg 1200

aagaagaaga tcaagcggga ggaggtggag cggctggtca aggaggtggc tgaggagtac 1260

accgaccgct tctccccaca ggcacgctca ctctgttctc agcttcccaa caaggaccct 1320

gctgagcgcc tggggtgtcg tggaggtggt gcccgtgagg taaaggagca cccccttttc 1380

aagaaactga atttcaagcg gctgggagct ggaatgctag aaccaccttt taaacctgac 1440

ccccaggcca tttactgcaa ggacgtgctg gacattgaac agttctccac agttaaaggt 1500

gtggatctgg agcccacaga ccaagacttc taccagaagt ttgccacggg tagtgtgtcc 1560

atcccctggc agaacgagat ggtggagact gagtgcttcc aggaactaaa tgtctttggg 1620

ctggatgggt ctgttccccc agacctggac tggaaggggc agcccactgc accccccaag 1680

aagggattgc tacagagact cttcagtaga caagattgct gtgggaactg tagcgacagt 1740

gaagaagagc tccccacccg cctctagccc ccaggccgag gcccccaccg gcggctggcg 1800

gtagcagcta ctcagtgact gacgttgaca gttttgcaca gtgctgtttc cagttgtcca 1860

cgccagtcgt ggtctgtgga acacagacgg aactgtcccc agtgtcctcc gttcctcagc 1920

cactggccca gctgagtttg acgaggcctg ggccatcttg ggacaaaggt gcgtcccttc 1980

agctcttctc tgtggagctc ggggctttat gtgtttatgt atttgtacga atgtatatag 2040

ctaccagagc gttattaact ccaccctggg tctggcaccc cagggcaact agccctggcc 2100

aggagagcca ggagctggca agaggaacca gtgccaacct ccaggctaat ggggcccagt 2160

ccagatgagc aagcagtgtg tgggccaggt cagacctctg accacagcat tgctccggcc 2220

accatcagac tcaagtgaga ctcgtgcctg tctgttgtgg actgaggcca ccatgctcca 2280

gttgcccaat gctttcattc cgtctcagcg tttgtcagag ctggagacgg gggctttgtg 2340

ttctccgggc ctgggtcaca gtgtgactgc aaactgagct gacggggacc tatcacacca 2400

ctgccccaga tgggttgctt tttgccttcc agctcccaga gcacattggt ccttattctg 2460

ggtcatatcc aggccataag aggggttaga attagccctc caacattcca gaatccctca 2520

taaaagtaga catgtgccca gcaataatcc aaccttcctg tgtgtgcctg cgggaggcgg 2580

gtgtgagtgt atatcattgt gtgaagagag tagggtaagg ctgtgcctaa gccccagacc 2640

ctagccctag cctttcccag actgtgatgg ctgtcctgat gctagtaagg acagtatggg 2700

accatacggc cctggcgttt ccatgtttaa agccaatgtg agctgccatg tgtctatgac 2760

caatgtaagc tgccatgtgt ctgtctgtgt gaatacagtc tgagcacaag gctggcc 2817

<210> 622

<211> 1614

<212> DNA

<213> Rattus norvegicus (Norway rat)

<400> 622
cgggccagca caagatggcg ttgcgagcga tgcggggaat cgtgaacggg gccgcgcccg 60

agctgcccgt gcccaccggt gggccgatgg ccggagctcg ggagcaggcg ctggcggtga 120

gccggaacta cctctcccag cctcgtctca cctacaagac tgtctctgga gtgaatggtc 180

cactagtgat cttagatcat gtaaagtttc ccagatatgc tgagattgtc cacttgacat 240

taccagatgg cacaaaaaga agtgggcaag ttctagaagt tagtggctcc aaagctgtgg 300

ttcaggtatt tgaaggaaca tccggcatag atgccaagaa aacatcctgt gagtttactg 360

gagatattct ccgcacacca gtgtctgagg atatgcttgg tcgagtattc aatggatcag 420

gaaaacccat tgaccgaggt cctgtggtgt tggccgaaga cttccttgac atcatgggtc 480

agccaatcaa ccctcagtgt cgcatctacc cagaagagat gattcagacg ggcatttctg 540

ccatcgacgg catgaacagt attgcgaggg gacagaaaat ccccatcttt tctgctgccg 600

ggttaccaca caacgagatt gcagctcaga tctgtcgcca ggctggtttg gtaaagaaat 660

ccaaagacgt ggtagactac agtgaagaaa actttgccat tgtgtttgct gctatgggag 720

taaacatgga aacagcccgg ttcttcaaat ctgactttga agaaaatggc tcaatggaca 780

atgtctgcct tttcttgaat ctggctaatg acccaactat cgagaggatc atcactcctc 840

gcctggctct gaccaccgct gagtttctgg cttaccagtg tgagaagcat gtcctggtca 900

tcctgacaga tatgagttct tacgctgaag cacttcgaga ggtttcagct gccagggaag 960

aggttcctgg tcggcgaggc ttccccggct acatgtatac ggatttagcc accatctatg 1020

aacgcgctgg gcgagtggaa ggtagaaatg gctctattac ccaaatccct attctcacca 1080

tgcccaatga tgatatcact catcctatcc ctgacttgac tgggtatatt actgagggcc 1140

agatctatgt ggacagacag ctgcacaaca gacagattta ccctcctatt aatgtgctgc 1200

cctcactctc tcggttaatg aagtcagcta ttggagaagg aatgaccagg aaggatcatg 1260

ctgatgtgtc taaccagttg tacgcatgct atgctatcgg taaggatgtg caagccatga 1320

aagctgtggt gggagaagaa gccctgacct cagatgacct cctttacttg gaatttctgc 1380

agaagtttga gaaaaacttc attactcagg gtccctatga aaatcgaact gtctatgaga 1440

ctttggacat tggctggcag ttgcttcgaa tcttccccaa agaaatgctg aagaggatcc 1500

ctcagagtac cctgagcgaa ttttaccctc gagactctgc aaagcactag ctgctgctgc 1560

ttgtgcggct cgaccctctt gtgaagtgct ggttctgttt cctgattcct tttg 1614

<210> 623

<211> 2228

<212> DNA

<213> Rattus norvegicus (Norway rat)

<400> 623
atgttgcagc ccgctcaccc gcatcacgat gaacgcacag ctgaccatgg aggcgatcgg 60

cgagctgcac ggggtgagcc atgagccggt gcccgcccct gctgacctgc tgggcgggag 120

ccctcacgcg cgcagctccg tggggcaccg cggcagccac ctgcctcccg ctcacccgcg 180

ttccatgggc atggcgtcgc tgctggacgg cggcagcgga ggcagcgatt accaccacca 240

ccaccgcgcc cctgagcaca gcttggctgg ccccctgcac cccaccatga ccatggcctg 300

tgaaactccc ccaggtatga gcatgcccac cacctacacc accttaaccc ctctgcagcc 360

gctgccgccc atctccaccg tgtccgacaa gttccctcac caccatcacc accaccatca 420

ccaccaccac ccacatcacc accagcgcct ggcgggcaac gtgagcggta gtttcacact 480

tatgcgggat gagcgcgggc tggcctccat gaataacctc tataccccct accacaagga 540

cgtggctggc atgggccaga gcctctcgcc cctctctggc tcgggtctgg gcagcattca 600

caactcccag caaggacttc cccactatgc tcatcccggc gcggctatgc ccaccgacaa 660

gatgctcacc cccaatgact ttgaagccca ccaccctgcc atgctcggtc gccacgggga 720

gcagcacctc acgcccacct cggccggcat ggtacccatc aacggccttc ctccacacca 780

tccccatgcc cacctgaatg cccagggcca cggacagctc ctgggcacag cccgggagcc 840

caacccttcg gtgaccggcg cgcaggtcag caatggaagt aattcagggc agatggaaga 900

gatcaatacc aaagaggtgg ctcagcgtat caccaccgag ctcaaacgtt acagcatccc 960

acaggccatc ttcgcgcaga gggtgctctg ccgttcccag gggacccttt cggacctgct 1020

gcgaaacccc aagccctgga gcaaactcaa gtccggtcgg gagactttcc ggaggatgtg 1080

gaagtggctg caggagccgg agttccagcg catgtcggcg ctccgcttag cagaaagcgc 1140

catgggtggc agtgtgcctt ctctgaggat cacatctgga ggcccccaac tgtctgttcc 1200

tcccttacct gcctgcaaaa ggaaagagca agaacacggg aaggacagag gcaacacccc 1260

caaaaagccc aggctggtct tcacagacgt ccaacgtcgg actctacatg caatattcaa 1320

ggaaaataag cgtccgtcca aagaattaca aatcaccatt tcccagcagc tggggttgga 1380

gctgagcact gtcagcaact tcttcatgaa tgcgagaagg aggagtctgg acaaatggca 1440

ggacgagggc agctccaatt caggcaactc ttcgtcttca tcaagcactt gtaccaaagc 1500

atgaaggaag aaccacggac tagaacctcg gtggaaaagc tttaaattaa ataaaaaaag 1560

tttttaaaga ccaggacctc aagatagcag gtttatactt agaaatattt gaaggaaaac 1620

aagtggtatt tatagtccaa agaaaccaaa gactaaaaag ctcacctgca ttctgacttt 1680

gttcagagac acatacttca gcggggcaag actcggcaag aaagatgatg gagaaaacgt 1740

cggatctcgc gccttcaacc cctgcacctc actgtgctcg gctgctcagt ggctctaagc 1800

acagtaatgt ttgagccatc gagtggacat cttttaagat cagacgttct tatctgttcc 1860

accacactgc aagggtgtgt agtgtctcaa tatcacgtgt gagtatgtgt gtgcatgcgt 1920

gcacacgtaa acacacagca acaccagtgt ttagggactc aggcacggct ggtcttcagg 1980

aagggttgca ccatatgaca tgaccaagct ggtgtgggat ttctaagttc attggatctg 2040

agatgtgagt tttctcatct aagcctagcg tttgaacctg ccaggcccac aacctaaatg 2100

cagattcaaa cccagcaaag aaaatttgaa tggcacctaa accagtacgc tctctttgtt 2160

tgttaagaaa tgttcagata tttttaaaaa aaaatgaaac aagagtccat ccattaaaaa 2220

aaaaaaaa 2228

<210>  624

<211>  1414

<212>  DNA

<213>  Rattus norvegicus (Norway rat)

<400>  624
gcgcctcctt gttcccagcg tcaccactgc cgccatgccc ggagggctgc ttctcgggga      60

cgaagccccc aactttgagg ccaataccac catcggccac atccgcttcc acgatttcct     120

aggagattca tggggcattc tcttttccca cccacgggac tttaccccag tgtgcaccac     180

agaacttggc agagctgcaa agctggcgcc agagtttgcc aagaggaatg ttaagttgat     240

tgctctttca atagactctg ttgaggacca ttttgcctgg agcaaggaca tcaatgctta     300

caatggtgca gcacccacag aaaagctacc atttcccatc atcgacgata aggacaggga     360

ccttgccatc ctgttgggca tgttggatcc agcagagaag gatgaaaaag gcatgcccgt     420

gacagcccgt gtggtattca tttttggccc tgacaagaaa ctaaaactgt ccatcctcta     480

cccagccacc acgggcagaa actttgatga gattctcaga gtggttgact ccctccagct     540

gacagcatca aatccagttg ccactccagt tgactggaag aagggagaga gtgtgatggt     600

ccttcccacc ctccctgaag aggaagccaa acaacttttc cctaaaggag tcttcaccaa     660

agagctccca tctggcaaga aatacctccg ttatacgccc cagccttaag tctgcgggaa     720

ctggggctgc aactgctcga ccagcactgg gacctgagga tgacagctga cagctgcagt     780

tccgtagaaa gattgtggcg tggtcacagc cgaagtcgcg taggtcgctc gctatactac     840

tggctcatta aatggaaatg gcaccaaaac attctcggga ttctttactc tgtgccttcg     900

ccagcattct gcccctccat ccatcacagc gccctgctga ctggctttct ttgaaacgaa     960

ttatgtgttg gtgactgcag ggtctctgca gggtctgtga tcagcaaggt agtgtcagtg    1020

tgggctccat gctagaatga taaacatctt ttgtaggttt ccaaactgaa tcttctgtta    1080

cccattgtgg agaaccataa agtggggaga aactttcaat tctgtacttt cagtaaataa    1140

agcaggggag gtgggggggg ggggagagtt ctctatagga aatccatggt gagtttctat    1200

caaagtgggc ttccaagaat gtgtggaaga cagcacatgt acttcctgga gtttccaggt    1260

ctgctctcta gtgatctgtg aatgtgaact cgccttatga aagacaggca gtgacattgt    1320

ttgccactaa atgtcctaaa ggagtgggtt gccatttcta aattctgctg tgtgaggttc    1380

aacaataaaa tcctgattag aaaaaaaaaa aaaa                                1414

<210>  625

<211>  2244

<212>  DNA

<213>  Rattus norvegicus (Norway rat)

<400> 625
atggacttcc ttgatgagcc cttccctgat gtggggacct atgaagactt ccacaccata 60

gactggctga gggaaaagtc ccgggacact gacagacata gaaagataac cagcaaaagt 120

aaagagtcta tttgggagtt catcaagagt ctgctggacg catggtcagg atgggtggtg 180

atgctactta ttggtctgct agcaggtacc ctggctggag ttatcgatct cgctgttgac 240

tggatgacag acctcaagga gggcgtctgt ctgtctgcat tctggtacag ccatgagcaa 300

tgctgctgga cctccaatga gaccactttt gaggacaggg acaagtgtcc cctgtggcag 360

aagtggtcag agcttcttct gagccagtca gagggtgcta gcgcttacat tctgaattac 420

ttaatgtaca ttctatgggc attgctgttt gcatttctgg ctgtctccct ggtacgtgtg 480

tttgcaccat atgcctgtgg ctctggcata cctgagataa agactatttt gagtggcttt 540

atcatcaggg gctacttggg gaaatggact cttctaatca agactgtcac tctcgtgctg 600

gttgtatcct ctggcctgag tcttgggaaa gaggggccac tggtgcatgt ggcatgttgc 660

tgtggcaact tctttagcag ccttttctcc aagtatagca agaatgaagg caagaggcgt 720

gaggtgcttt ccgctgcagc tgctgctggt gtctctgtgg cctttggtgc tcccatagga 780

ggtgtactct tcagtctaga ggaggtcagt tactactttc ccttgaaaac cttgtggagg 840

tcattttttg cagccctggt ggctgccttc acactgcgct ccatcaaccc ctttggaaat 900

agccgcctgg ttctctttta tgtggagtat catacacctt ggtacatggc tgaactcttc 960

cctttcatcc tgcttggagt ctttgggggt ttgtggggaa ccgtcttcac acgctgcaac 1020

attgcctggt gcaggaggcg taaaaccacc aggctgggga agtatccagt gttggaggtc 1080

attgtggtga cagccatcac tgccatcatt gcttatccca atccctacac tcgccagagc 1140

accagtgagc tcatatctga gctattcaat gattgtgggg ctcttgagtc ttctcagctc 1200

tgtgactaca tcaatgaccc caacatgact cggcctgtgg atgacattcc ggaccggcca 1260

gctggggttg gagtttacac agccatgtgg cagctggcct tggcactgat cttcaaaata 1320

gtcattacta tatttacctt tggcatgaag attccctcag gtctcttcat ccccagtatg 1380

gctgtaggag ccatggcagg ccggatggtg ggaattggtg tggagcagct ggcctaccat 1440

catcatgact ggatcatctt caggaactgg tgcaggcctg gagcggactg tgtcacacca 1500

gggctttatg cgatggtggg agctgcagcc tgtctaggtg gagtgactag gatgacagtg 1560

tctctggtag tcatcatgtt tgaactgact ggaggtctgg aatatattgt acccctgatg 1620

gcagcggctg ttaccagcaa gtgggtggct gatgcctttg ggaaagaagg gatttatgaa 1680

gcccacatcc atctgaatgg gtacccattt cttgatgtga aggatgagtt cactcaccgt 1740

accctagcca ctgatgtgat gcggccccgc aggggagaac cacctttgtc agtactaacc 1800

caggacagca tgactgtgga ggatgtggag actctcataa aagagacaga ctacaatggg 1860

tttcctgtgg tggtctcgag agattcggag cgcctcatag gctttgccca gaggcgggag 1920

ctaatcttgg ctataaaaaa tgccagacag aggcaagagg gcatcgtgag caattccatc 1980

atgtacttca cagaggagcc ccctgagctg cctgccaaca gcccacatcc actgaagctg 2040

aggcgcattc tgaacctgag cccttttact gtcacagacc acacccccat ggagacagtg 2100

gtggacattt tccggaaact ggggctccga caatgcctgg tgacacggag tgggaggctt 2160

cttgggatca tcacaaaaaa agacgttctg agacacatgg cgcagatggc aaaccaggac 2220

cctgaatcca tcatttttaa ttag 2244

<210> 626

<211> 1121

<212> DNA

<213> Rattus norvegicus (Norway rat)

<400> 626
ggactccata ccctttccta tctttaatgg caaaagaaat gactgggcac gaacgcctaa 60

accaaaacta tgttttaaat ctgtgttggg agagcgtgag agtgaatgtg gattcaaaaa 120

taccttttct ggaaacgtcg aaacattatc ataacgtgga aaatcaagaa tatgtaatca 180

gatagatact cccattgtga attttaaata ttttaaagaa tcgtctttaa gactgtttag 240

tggcagtgat tgtctgtgct gtgggtctta acttgtgata ctaagtatta aatagctacg 300

tttttcatgt atgtagatca tgcttttttg aaaaagcaaa acaatcaggt ggcttttgca 360

gttcaggaaa ttgaatgcag attttagcac agactgattt ctttttcctt tttaaatagc 420

taggagctaa aactctaggg gagaaggtaa aactagtttg gatatgcaaa acatttattt 480

tgacataaaa ttgataaaga tatttttaat aatttacact gtaagcatga gtattttata 540

atacactaca gatatattgt tagttcataa caatccatct acggatgtaa caactacaat 600

gtgtagaggg gttgatgctt tggtcaatga acctaaagaa aaatgaacaa gttagatttt 660

tacaaagccc ttttttataa ctcccaaaat ttcttaaatc taaaaatgtc tgcattattg 720

gaacaaaccc attttaaatt tgtaaatgaa tatttttttt aattttgaaa actttaattt 780

tttatacaaa ttaacgtatc atacaccaaa cagtaacaga agtgattata ataatggaag 840

aaacagtttc ttcaccaaat ttccattcaa gccacaaggc tgtgtgtaag agaagtagaa 900

gtgatgtggt gtgactggct aggataaagg agatcttcag gagcacatgg gtttagagag 960

cccagggatc aggaaaagga ggctctcaca tctgagcagt gctgtccttt caaacacagc 1020

ttggctttta tagtaaccta agaattaatg catacaggag gagtttcaca tgtgtgaaat 1080

ccaatagcag atgggttaaa gttaaacaag ttatagcaca g 1121

<210> 627

<211> 315

<212> DNA

<213>  Rattus norvegicus (rat)

<400>  627
tttacactgt aagcatgagt attttataat acactacaga tatattgtta gttcataaca     60

atccatctac ggatgtaaca actacaatgt gtagaggggt tgatgctttg gtcaatgaac    120

ctaaagaaaa atgaacaagt tagattttta caaagccctt ttttataact cccaaaattt    180

cttaaatcta aaaatgtctg cattattgga acaaacccat tttaaatttg taaatgaata    240

ttttttttaa ttttgaaaac tttaattttt tatacaaatt aacgtatcat acaccaaaca    300

gtaacagaag tgatt                                                     315

<210>  628

<211>  1444

<212>  DNA

<213>  Rattus norvegicus (Norway rat)

<400>  628
ccctctagat caggacgtcg ccggggtggc tgtgacttgg ccaagtgctc gcatgagtca     60

aatgacaagg aagagacttc tgccgtggaa cccatgccgc accggccacc tttgccaaga    120

ccgcctgtgc ctttttctct cgcaggtgcg gcggggcata cctgtgatcc cagcaattgg    180

gagactgaga caggaggatc caaccttcaa agctacatgc catggctgcc caggagtccc    240

tgcacgtgaa gaccccacta cgtgacacga tggcattgtc caaagtggcc ggcactagtg    300

tgttccttaa gatggacagc tctcagccct ctggctcctt caagatccga ggcattgggc    360

atctctgcaa gatgaaggca aaacaaggct gtaaacattt cgtctgctct tcagtcgtcc    420

agatttgggg ttccagaatg aggggcagaa gtcactctgg agatgagcag ccccacgtga    480

ggtcccaggc cctccttcct gatacaccct ctccactgac agcgggcaac gcgggcatgg    540

cgactgccta tgctgccagg aggctgggcc tcccagccac tattgttgtg ccaagcacca    600

cacctgccct caccattgag cggctgaaga acgaaggggc cacagttgaa gtggtgggag    660

agatgctgga tgaggccatc caactggcca aggctctgga aaagaacaac caaggttggg    720

tgtacatctc ccccttcgat gaccctctca tctgggaagg ccacacttcc cttgtgaagg    780

agctgaagga gacactgagc gccaagcccg gggccattgt gctgtctgtg ggcggtggag    840

gcctgctgtg cggagtggtc caggggctgc gggaggtggg ctgggaggat gtgcccatca    900

tcgccatgga gaccttcggc gcccacagct tccacgctgc cgtcaaggaa ggaaagctgg    960

tcaccctgcc caagatcacc agtgttgcca aggccttggg tgtgaacact gtgggggcac   1020

agaccctgaa gctgttttac gaacacccca ttttctctga ggtcatctca gaccaggagg   1080

ctgtgactgc tatcgagaag ttcgtagacg atgagaagat cctggtggag cccgcgtgtg   1140

gcgctgccct ggctgcagtg tacagcggtg tggtgtgcag gctgcaggct gagggccgac   1200

tgcaaacccc actggcctcg ctggttgtca ttgtgtgtgg tggcagcaac atcagcctgg 1260

cacagctgca ggcactcaag gcacagctgg gcctgaatga gctactcaag tgatatctgc 1320

tgctgccctg gccaccctga ggggtcacca gcacccctga gtaggctggg tgggcgtccg 1380

cctgacagtg gcccaccctc ctttatccat gtttataata tgcacttttt cattgtaaat 1440

aaaa 1444

<210> 629

<211> 3545

<212> DNA

<213> Rattus norvegicus (Norway rat)

<400> 629
ggtctcccct ttggaaattt tcctgctttt gcaaaatgat gactatttct ttgatttggg 60

gaattgccgt gttggtgagc tgttgcatat ggtttattgt tggaataagg agaaggaaag 120

ctggtgaacc tcctttggag aacgggttga ttccgtacct gggctgtgct ctgaaatttg 180

gatctaatcc tcttgagttc ctaagagcta atcaaaggaa gcatggtcac gtttttacct 240

gcaaactgat ggggaaatat gtccatttca tcacaaactc cctgtcatac cacaaagtct 300

tatgtcatgg aaaatatttt gactggaaaa aatttcatta cactacttct gcgaaggcat 360

ttggacacag aagcattgac ccaaatgatg gaaataccac ggaaaatata aacaacactt 420

ttaccaaaac cctccaggga gatgctctgt gttcactttc tgaagccatg atgcaaaacc 480

tccaatctgt catgagacct cctggccttc ctaaatcaaa gagcaatgcc tgggtcacgg 540

aagggatgta tgccttctgt taccgagtga tgtttgaagc tggctatcta acactgtttg 600

gcagagatat ttcaaagaca gacacacaaa aagcacttat tctaaacaac cttgacaact 660

tcaaacaatt tgaccaagtc tttccggcac tggtggcagg ccttcctatt cacttgttca 720

agaccgcaca taaagctcgg gaaaagctgg ctgagggatt gaagcacaag aacctgtgtg 780

tgagggacca ggtctctgaa ctgatccgtc tacgtatgtt tctcaatgac acgctctcca 840

cctttgacga catggagaag gccaagacgc acctcgctat tctctgggca tctcaagcaa 900

acaccattcc tgcaaccttt tggagcttat ttcaaatgat caggagtcct gaagcaatga 960

aagcagcctc tgaagaagtg agtggagctt tacagagtgc tggccaagag ctcagctctg 1020

gagggagtgc catttacttg gatcaagtgc aactgaatga cctgccggta ctagacagca 1080

tcatcaagga ggctctgagg ctttccagtg catccttgaa tatccgcaca gctaaggagg 1140

acttcactct ccatcttgag gacggttcct ataacatccg aaaagatgac atgatagctc 1200

tttatccaca gttaatgcac ttggatcctg aaatctaccc agaccctttg actttcaaat 1260

atgaccggta ccttgatgaa agcgggaaag caaagaccac cttctacagt aatggaaaca 1320

agctgaagtg tttctacatg cccttcggat caggcgcgac aatatgtcct ggaagactct 1380

ttgccgtcca agaaatcaag cagtttttga tcctgatgct ctcctgcttt gaactggagt 1440
ttgtggagag ccaagtcaag tgtccccctc tagaccagtc ccgggcaggc ttgggaattt 1500
tgccaccact acatgatatt gagtttaaat ataaactgaa acactgatac gtggttggaa 1560
gaagcgaaca ctggatgatg tcacttggcg gctgagagtc atcactaaac aggccttcgg 1620
gaccaatgct cactgatgcg ccctagcgac tggattagtg ggaagaactt tgttctcgct 1680
gcccacattc ctgggtgttc acatagctgg ggccagagct tcatcacttt cagaaagcaa 1740
tgtcttttgt atttattttc aaaatgaaga tattccaatt ggcaggatat ttttcctaag 1800
gaaattgctt tatattttta tgaaaactac caattaatta tgaaagggct tgaaattcac 1860
gttttagtga aattactgat ttttcactag taaggttctt caggtgtgaa actgtattat 1920
aaaaatgttg taatgggtca cactgtgctt tgcataaagg taaaggaaac tatgtttcag 1980
ccttttctgt gtctatgagc ttcgaaaata atcttactgt tctagaaaca ctggggaggt 2040
ttcgacatgc tctcgctata ttttatttta ctgttgctag aaattttcat tccagttttc 2100
aactacctta tctttccccc attttgacat gcatgccaat gagaagagta ttttttagga 2160
attaacaagg cacctcccag aaccctaccc tgagactttt aagcctttaa tcccagcact 2220
cgagaagtag agccaggcag atctctgagt ctgaggttat tctggtctac atcagctcca 2280
gacaagccag gactacagaa tgggatcttg tctaaaaaat acagctaatc tttatgtcat 2340
aactgattat gaatcaacct aaaagataaa ttttcaatca ggactcagag aaaatgagca 2400
attaaaaaac ttagctctga ggtatgtgga attcattaag tacaagttga cattacatgt 2460
tctttaaaaa tagtttatgt tttatctcta aatgccctgc agatgaagaa taataatgaa 2520
aagttgaata atactgttta aacactaagt gcaataatgc tttggtaatg tactttaaga 2580
gaatcattag ccgtgccagt tttactaaaa tatatttata tgtaaattat atttatcttt 2640
ttcttatacc ataaatataa aaatattgca acatttagta attttaaaat tatatacctt 2700
tcagaaaatg atgtatgaat gtttgtatgt tttttaactt tgaacagaac atttaaatta 2760
ttcatctacg gtgattttta tcttatttat ttctttttgt ctcattcata tcttgaagaa 2820
atccaaaaat atctgaagga atcgctcact caaatgtctc cctatggtta cagaaaaatt 2880
caataccatg tttttgtcct cggggactga agcagggtgt cgtgggtgcc gagcagaggc 2940
tcctgctgca gcgagcttta tccacgggac tccttaaact tttaaaatct tatcactatt 3000
atcatgcatt tattacctaa gtaggatatt tccctttcct ttttcatttc agccgagtcc 3060
cttagcaacc caggctgact gggaccctcc atgtagctta agctgtgaac tcactgtact 3120
tcctgttttc acttatttta ggaagtaatt ttccctatca gaaattttaa ttgtttagat 3180
gatgtataag agtaacacaa ttctgttata tactaatctg tagtaaacta aatttgttct 3240
tagaacaagt ttgatgactc tcaaattgaa tgtatccata catctttcca tggcttcttg 3300
aatgcccatt tctcatacac agaatgatgg gtttcacggt gatgtcttcc tttcatgtct 3360
ttattcttgt gcggtgatgg ttggcaaatg atacccatgg agcaaggtta ctcttcctat 3420

ttctgtgcag cctaagtgtt aagaataatt tttaaatact tggagggaag gcacattttg 3480

tgtcatatgt gaagtgacat gtgacacaca gactagcaaa tccttgagta aaattttatt 3540

gggat 3545

<210> 630

<211> 9578

<212> DNA

<213> Rattus rattus (black rat)

<400> 630
gaattccgga gctgctgtgg gacagccgcc ctggaattgc gtgtcactga gcaagctgtt 60

cttcaccctg aggacatcca aggaacatct ccagagattg cccagtgtcc cctgggtaca 120

aaaatcacac caccttgagg acagctgagt tgaacagagg tgtgggaagg aggcgtgtga 180

gaatgatcgg caaagctggt gggtgtgggg gctgggaatc cggcgcagat gactgagggc 240

tttcctggca ttctgaggga cctgagtctg atcctcagtg ccatgtgagg tgggcacggt 300

tggcacgcgc ctgtaatggc agcattctga aggcggaggc aggaggatcg ggagttgaag 360

ggcatcttca gctactcggt gagtccgagg ccagcctggg ttatgtgaga ccctgactca 420

attttaaatg ataggtgtgt ccaaactacc cagtcctgtt gtcatctgtc cccagcccaa 480

cccaatcctc cacatcagct ctgcactctc ttaaaactat gagtccctgg gttcaattcc 540

cagtgtcctc agctaccctc tgccctggtg actctcagca aacaccatcc tccaagcctg 600

ccctcatcta caacaaggac taagggagcc cacccactgc tgtgtcttaa cacacgtgaa 660

accccccaca caggaggcaa atgatgtgga accagggtcc cccattgctt tggcaatgag 720

atgctggtcc agagagtaca aggtagctgt gtccacacat gggctttgtg tcatcctggc 780

ttgcctactt ccctctatcc ctgacccaaa cctgtggtca tccctaatgc ggatggtcag 840

aaagaacaat gcacaccaca ggccagaaaa agcagcagac gtggggctgg agagatggct 900

cagtggttaa gagcactggc tgctcctcca gaggtcctga gttcaaatcc cagcaaccac 960

atggtggctc acaaccatct gtcatggact ctgatgccct cttctggtgt gtctgaagtc 1020

agcgatggtg tgctcacata tattaaataa acaaataaat cttttttaaa aaaaagcaca 1080

tggactctgt gtgtgtgtgt gtgtgtgtgt gtgtgtgtgt gtgtgtttat gctgtggaca 1140

cacaggtctc tgcacacagc ctggaaacgg gtcagagggg atggtcacga tggtcatagt 1200

ggtggtggtg gtgatggtta ctggtactga tactgatggt agtgctgata gtgttgacag 1260

tggtggccaa tagtgaatag tgatggtgat gataaaggtt accaacactg gctacctgtt 1320

caatttcaag tctctcacct aacgcagtcc cacctcttca aagcctggaa cagcggtacc 1380

catgtcatcc ctgtttacac acgaacagag gctctcgagg tcaaggttct tgcccagagc 1440

catgtatcta gtaacagcag agcaggactt gaacccagat cccctttgtc taacccatac 1500

gcccttttat atgaagagga tggcagggga tggtggtaag caaggggtgt caatcatagc 1560
ttgggcgtca acccccaagt cccgcctttc actatctata cgagctgtgc ctcatttcca 1620
ctctgtacag acctgttgga tggttttaca ttgatttggg ttcatgaagc tgagccccat 1680
actccccgcc ctttttcttt ctattttgag acaggctctc actccttagt ccaggctgtc 1740
cttctgcctc agcttctatt gttctggctt gaaggtgtga gttgccatat ggctggcctg 1800
tgtgtttctt ggccataaac agaccagcaa ctgtccggct tttctgttat ttctgggatt 1860
gacccctggg actctggcag gagctcctca gtgctgcgct gcccgcccac gagtcctata 1920
ggcctttcgg agatgaatgt cttcatttgc aacggaaaaa aaaaaaaaaa aaaaaaagaa 1980
cggggagaga aggtgaggcg gaccttggag ggcagaacaa gtggtgcagc atggaccagg 2040
gggcggggat ttctgggggc gtggcctgcc tgggccctca ttggctgccc ggaggctcag 2100
cccggagaat aaagccctgg ggtgtcctgc caactttcca ctctcccagg acacagacaa 2160
aatctaccct tctcgctcgc tctgtccagc tcctcctgtg ccgggtatgt ggccctcagc 2220
ctcagtctca ccccaagatt gacagccaga gcagggtacc gggaagggaa gagggactgg 2280
gactcatcca gttcaagtct ccatgtactg atctgtaaaa tgggacgatg atataggccc 2340
tctagatcag gacgtcgccg gggtggctgt gacttggcca agtgctcgca tgagtcaaat 2400
gacaaggaag agacttctgc cgtggaaccc atgccgcacc ggccaccttt gccaagaccg 2460
cctgtgcctt tttctctcgc aggtcatatc acctatagag gcggatgcga ctggcctctg 2520
gggtgggaat cagtatggag tacactgccc attttcccaa aggtttccaa tgctacagcc 2580
tgggacagga accaggcagg gttcgccaaa gccacctgga aggggtctgc gtggcggaaa 2640
ctatccacta gccaaaggct gcactggctg ggccttttgg gtccagagaa gaccctgtat 2700
gtcaggatgg ggctagctag gacaaagcaa agaaatttgg agttactctt gtgtgccatt 2760
gtttccgact gcctttcaag aggtcttcta gaaagaggag tctggtggct tgcctataat 2820
ctcagcactt gagagactga ggcaggagga tcacaagtct gtctctgtct gtctgtctct 2880
tgactgaggc aggagaatca caaggagttt aatctctcct acactcacac acagtcactc 2940
gaattcgtgt agatgctggg aagagacacc acctgcctag gtttggtccc acttgactct 3000
gtatctctct tggggcttct cacagggctc caaggtgaaa gtggctgagc cggtgttaac 3060
ccaaaggctt cctcactgtc ctggctcccg aggggacctc agggtgccat tagccaaact 3120
tgccgtgcag cctctatctg catcgaggtt ccctctcatt gtggacgctg tatgacgagg 3180
gcaagccaga ggtctctgtt gcctcttccc catgccagaa aaagcaattc cataattcta 3240
tctttttaaa agaatgagag cctttcagag ctcgatgctg gatttccagg tcttttgggg 3300
gttttgtttt gttttgaaac agactcctat acagcccagg gtggccctca aactccctac 3360
atagccaagg ttgaccttga atggctagtc ctcttgccct tgcccccaag caatgggatt 3420
acaggcctcc tgcaccaagc ccattttttt cttcaaaaat atgtatttgt cgaacacagg 3480
tgcggcgggg catacctgtg atcccagcaa ttgggagact gagacaggag gatccaacct 3540

tcaaagctac atggtaagaa catactgagt ttgttttaaa gcatgtgtgg ggttgggata 3600
tggctcagtc tatgaagggc ttcctgggaa agcatgaggg cctgtgttca tattcccaga 3660
atgcacctca aaaatgctga gctcaacaat aaatggctat catccctgca ctagggagca 3720
agagacagaa ggacctcaag ggacatccag gggcctctgg aacctcaggg gacttctggg 3780
aacctctggg gacctcagag cctccggggt tctctggcca gccagttgga ctgaatcagg 3840
gtgctcctgg ttcagtgcaa gaccctgcct caaaaatagt gggcagctga ggaggctact 3900
gactgatgtt gacctctggt ttctccacac acacacacac acacacacac acacacacgc 3960
acgcacgcac gcacgtgcgc gcgcacgcac acacacacac atgtttaagt aatatataat 4020
agggcaaaat gctgagcacg gaggggtgct gtggcccgga gggtcactct tcacaggtac 4080
cccatttggc ccattttgtg tgtgtgtgtg tgtgtgtgtg tgtgtgtgtg tgtgtgtgtg 4140
tgtgtgtgtg ctgacatact ctgagctcat gggaacaggt cagcccgggt tggttcctga 4200
ggactttgca gaaacagacc ctggctgacc ctggctgtgc tttgttccca tcttgtggcc 4260
tcattctgag ccagtttgaa caagcccctc cctctgaact ttagcccaac gctgcagcct 4320
aaagatgttt ctgagcttgt gaccttctaa ctttgggcca ctgggtagtt ggagctgagc 4380
tttgatttgg tgtcctttcc ttatcattat gggtatgaaa gagtggtcct agagcccagg 4440
agggcagcct ctcttgaccc tctgtatctc ctctctgccc ccagccatgg ctgcccagga 4500
gtccctgcac gtgaagaccc cactacgtga cagcatggca ttgtccaaag tggccggcac 4560
tagtgtgttc cttaagatgg acagctctca gccctctggc tccttcaaga tccgaggcat 4620
tgggcatctc tgcaagatgg tacaaggcag ggtggcatac tggcaggaga ggcagtccct 4680
cctacctgac agctgtagag gcaggtgtgc agatacacac ccaggattcc aaacggaggc 4740
aggaaggtgg aaagtgaatg gtatcccaga gtgcatagca agaacctgtc tctaaggaat 4800
aataataatt ttagggactg cagttgagac agctcggcgg ctgagagcac tggctgcttt 4860
tgcaggggac ttgagctcag ttcccgcaac cccatggtgg ctcacgatca actataactc 4920
cagctccagg agatccaaca ctctcttctg atttcccgga gcaactgcat cacatgcata 4980
taattcaaaa taagtaaata ttgggctgga gcgataattc agtggttagg aatacttggg 5040
gctctttcag aacagccatg ctcaccctca gcatccaagt gagcgagggg ctgtaactcc 5100
agctgcaggg catcaaatgc tctcctctgc cctctacagg caatacgtac acacagacac 5160
aagtgcacat aaagaaaaca catctttttt ttttcttttt aagaattttt atttatttta 5220
cttatatgag cacactgttg ctgtcttcag aaacaccaga agagggcacc agatcccatc 5280
gcagatggtt gtgagccacc atgtggttgc tggggattga actcaggacc tctggaagag 5340
cagtcagtgc tcttaacccc taagccatct ctccagccca agaaagcaca tcctaaataa 5400
ataaataaaa tatgttttaa agaaaataat aattttagaa taggtttcac acatcagggc 5460
tctctctggc taaggggtgc ccaccctaca gactgacacc tgcctcctta ccccagttaa 5520
tcaccttggg ggagatgagc ctgagagagt ggttcagata ccaagtgggc aagctcagat 5580

gttccagtgg tgaggacgtg tcctaggcag ggacttgggg gtggcctgat ccttcctctt 5640

gtccccacag aaggcaaaac aaggctgtaa acatttcgtc tgctcttcag gtgagtgccc 5700

agttcgtttt ctttcccttc ctaccctgtt ctgtgtgtcc tgtggcccag caccaggcat 5760

tctccctgga ctctgggctc tgcaacaaca catccattcg tagtcgtcca gatttggggt 5820

tccagaatga ggggcagaag tcactctgga gatgagcagc cccacgtgag gtcccaggcc 5880

ctccttcctg atacaccctc tccactgaca gcgggcaacg cgggcatggc gactgcctat 5940

gctgccagga ggctgggcct cccagccact attgttgtgc caagcaccac acctgccctc 6000

accattgagc ggctgaagaa cgaaggggcc acagttgaag tggtgggaga ggtgagtgca 6060

gacctattgt gggcagcaga gctggcgggt ggaggtgggg ggctgataac tggatagagc 6120

cctcgttcat ctcaatctct tcctgccccc acccctgcag atgctggatg aggccatcca 6180

actggccaag gctctggaaa agaacaaccc aggttgggtg tacatctccc ccttcgatga 6240

ccctctcatc tggtaagttg gagccacccc actccctact gcatgtgtgg tctctgctca 6300

tgttctcgcc ggcacaccag ccatgagggg tccccagatg cagagattcg gagagatcga 6360

ctcatcttgg ggtggctagg agcaaactaa ctagggaacg catcggtgga ggtggctttc 6420

atgtgccagc atctgccctg aacaactgaa gcttggcccc tgggaaccct gggagtcggt 6480

ggggaatgag catctaagga tgtgtccact ggtgggcagg aaatgggggt ttatctacca 6540

cacccacagc catctcctct ctgggagtgc gtggggtgag gggtgggggg ggtggggtct 6600

tgtctcctac cctgtgccct gagcagcagg gaggtcagag gtcaggttca tcatggcctg 6660

gcatagaggg aaaaacagac cttagctggt atctttgacc agcccttgtg tcttcaggaa 6720

gccctgacct gacctgccac ccctctcttg cccagggaag gccacacttc ccttgtgaag 6780

gagctgaagg agacactgag cgccaagccc ggggccattg tgctgtctgt gggcggtgga 6840

ggcctgctgt gcggagtggt ccaggggctg cgggaggtgg gctgggagga tgtgcccatc 6900

atcgccatgg agaccttcgg cgcccacagc ttccacgctg ccgtcaagga aggaaagctg 6960

gtcaccctgc ccaagatcac caggtgagcc cggggcacct ctccagcaca caggcggtgg 7020

agctgagcct ttggtccttt gcccgaagcc acactctcgc gcgccgtctg ggccttgaac 7080

gtgagtccgt tctttatgtg acagtgctta agtgacgtcc ttcgcttctt gaggtcctca 7140

cgcctcattt cttttgctgc tgtttgatct ggcctccgaa atgtgccaga ccctggctgg 7200

tcctggactc agcctgggag aaaacaatct ctggcctgca aagaatttat aaccaaaccc 7260

caccgcggcg gctcaaacca aactgagctc tagttctatt taagactatt aggggcaact 7320

cttcgaacca ggggctcagt tcctttagtc tcttgtcccg ccactgagag tgttgaccta 7380

gggtccaaag tggctgcttt ggtgccagcc accgcacaca cactccctct gaaaagaggc 7440

taagcaagag cagaagtgtt tctaaacttt aaagaccaag tagagctgct ccccagctga 7500

caatggggtc acacccaatc tttaaccatt cgttaaagat tgctatgaat gagttgaacc 7560

tggtctgctg gacgccttac ctaagctttt tggcacacgg tacagaactg gctgcttctt 7620

gctgataggc tgatgggaga ggtctccctc aaggctgcca ctgggcaacc caagagcgga 7680

ttgtactgca aacccatagt ctgagaaaag gtctgagttc aactctcaaa gtttggtttg 7740

taccaaatgt ggattttttt ttttttgaca ccacaacgtt gaactctcat agaccaaccc 7800

tcagaaatca gagctgtctg tacttccctc ttatcatgca ctgggcagtg cttggtcaca 7860

tgaccatacc tagctgcaag ggaggcaggg caaggttcac actaggcatc cacaagaaca 7920

gctagaaggg atttctagag agaggatggc ttctgtcata ggtagggaac accgaatcag 7980

ttaaaagact cttccatatt tttgggcacc atggtgagac tgatgccatc ctgctaccac 8040

tattctttcc tgccgatagg aatacagatg cagtagctgg cactcaagca gccatttttg 8100

gaccctaagt taatatgacc ctggggcaga tattgcctca tctgtttccc actctataca 8160

acttggagct tggaaagtgt gtgacagaca cctgatactt taggaaggac tagttctccc 8220

tgtttgttag agtgtgggag ggctgtctga gagcctcaac taatccatga gaccaaggag 8280

tccctctggg agtctccctg gaagaggtag caagtgagca aagcaggcgg aggttacaag 8340

ctggcaggct gggggtgggg gtggggcagc agagtccttc tggtatctga atcaggccag 8400

tttgatatcc tagaccactg tacaggttcc tggggctggg cagggttgcc cactcccact 8460

gttcacctgt agtccccgca tctcacgtgc acccttctac cttgtctctc cccatgtcca 8520

cccccctctc attcccacct cccagtgttg ccaaggcctt gggtgtgaac actgtggggg 8580

cacagaccct gaagctgttt tacgaacacc ccattttctc tgaggtcatc tcagaccagg 8640

aggctgtgac tgctatcgag aagttcgtag gtatgttctg tgggatcttt ttgtccttga 8700

ttttctccct cctcagccta gaccatctca tgaaaggaaa ctaaagccag tcagtggtaa 8760

acgcaaaagc tgctcgagaa aacatgaacc tggttaacca tgatagcagg gaggacaaac 8820

tagttttata agtgccagtt ttgagctttc tggtagccag agtcaaaagg gaaagggcca 8880

agatgaggtg atctctctgg gcttagcctg ggagatggtc aaaggaacga gagtagtgca 8940

cactgccatt aataacttca gcttgctaca ccactagaga gcaaccttct caggggccca 9000

ggaaccccaa gcacagactg tagaaaccga gactgggttc agaacaaaca gtcctggcca 9060

cagtgactga cgagagctga cgagccccct cccctacaga cgatgagaag atcctggtgg 9120

agcccgcgtg tggcgctgcc ctggctgcag tgtacagcgg tgtggtgtgc aggctgcagg 9180

ctgagggccg actgcaaacc ccactggcct cgctggttgt cattgtgtgt ggtggcagca 9240

acatcagcct ggcacagctg caggcactca aggcacagct gggcctgaat gagctactca 9300

agtgatatct gctgctgccc tggccaccct gaggggtcac cagcacccct gagtaggctg 9360

ggtgggcgtc cgcctgacag tggcccaccc tcctttatcc atgtttataa tatgcacttt 9420

ttcattgtaa ataaaaatta atatatatat gtttataaag caaagatctt ttgagctttg 9480

ttattgacat ggggtctatg tgcagcccag gctggcttca aactggagat ccaagcttgg 9540

gtgaggtcga ctctagagga tcgatccccg ggcgagct 9578

<210>  631

<211>  266

<212>  DNA

<213>  Rattus norvegicus (rat)


<400>  631
tagaaaggtg cacttctctg agaaagttac agtccatttc cttgctgtct gggcaggacc      60

agcccaggct gctcgtcgag gcccctggga gcagtttgca cgagatcgaa gccgctttgc     120

tcgacgcatt gccaggcaga ggagcagctg ggtccttacc ttacccctgc tttcagggcc     180

agagcatgga cacgccttag aaacctaccc cttcctctgt cgtcctcgtc tcttccactg     240

cctgagcctt gctcttccac tgaggc                                          266

<210>  632

<211>  1733

<212>  DNA

<213>  Rattus norvegicus (Norway rat)


<400>  632
agcagacaga aactgcccgc acccgctagc tttagcagcc ctcagcgtgg accctctcgc      60

aacttggcac cctcaagatc cctgcggttc caaccagacc cgctccacgg cgctggctgt     120

gcagcccgag cctcggccgc ctggcagtca ccctgggaag cggtgggacg gggagacagc     180

cgttctctct ggtagccgat aacggggaat ggagaccaat tgtcgcaaac tagtgtcggc     240

ctgtgtgcaa ttagagaaag ataagggcca gcagggaaag aatgaggatg tgggcgcaga     300

ggacccgtcc aagaagaagc ggcaacgccg gcagaggact cactttacta gtcagcagct     360

ccaggagctg gaagccactt tccagagaaa ccgctaccca gacatgtcca ctcgcgaaga     420

aatcgccgtg tggaccaacc ttacggaagc ccgagtccgg gtttggttca agaatcgccg     480

ggccaaatgg agaaagcggg agcgcaacca gcaggccgag ctgtgtaaga atggcttcgg     540

gccgcagttc aatgggctca tgcagccgta cgatgacatg taccccggct actcgtacaa     600

caattgggct gccaagggcc tcacgtcagc gtctctgtcc accaagagct tccccttctt     660

caactctatg aacgtcaatc ccctatcctc ccagagcatg ttttccccgc ccaactccat     720

ctcatccatg agtatgtcgt ccagcatggt gccgtccgcg gtgaccggcg tcccgggctc     780

cagcctcaat agcctgaata acttgaacaa cctgagcagc ccgtcgctga attccgcggt     840

gcccacgccc gcctgtcctt acgcgccgcc gactcctccg tacgtttata gggacacatg     900

taactcgagc ctggccagtc tgagactaaa agcaaagcaa cactccagct tcggctacgc     960

cagcgtgcag aacccggcct ccaacctgag cgcttgccag tatgcggtcg accggccggt    1020

gtgaaccgcg cccagggcgc gggattccga ggactgtcgg agtgggcaac tctgcccgag    1080

aaagactgag aattgtgcta gaaggtcgtg cgcactacgg ggagaaagag ggggggacaa 1140

aagagatcag aggaaaagaa accactgaat tcaaagagag agagcgcctt tgatttcaaa 1200

ggaatgcccc caagtgtctg ccatcttccg ctgaaagtat tcccaacagt tggaggacgc 1260

gtgcaccaac aaatgtttga ctggatatga cattttaaca ttactataag cttgttattt 1320

tttaagttta gcattgttaa cattaaaatg actgaaagga tgtatatata tcgaaatgtc 1380

aaattaattt tataaaagca gttgttagta ctctcacgac agtgttttta aaggctaggc 1440

tttaaaataa agcatgttat ccagaatcag ttaggatttt tcggcttgcg agcaaaggaa 1500

tgtatatact aaatgccaca ctgtatgttt ctaacatatt attattataa aaaatgtgtg 1560

aatataagtt ttagagtagt ttctctggtg gatgccttgt ttctgaaact gctacgtaca 1620

acctatcctg tgtataacat ttcgtacgat attgttttac ttttcagcaa atatgaaaaa 1680

atgtgtttta tttcttggga gtaaaatata ccgcataaaa aaaaaaaaaa aaa 1733

<210> 633

<211> 2335

<212> DNA

<213> Rattus norvegicus (Norway rat)

<400> 633
gtccccaagc tccaccagcc cttggtgaac cgacagggag cgtccggctt tcccagcacc 60

actttgccag actcaaaaca gccacaccgc aaagtgagcc tcgggcggaa ggaggcggag 120

ctacaggcgg ccccgcctcc gcggagggat acgtgtctcc gcggtccaaa accccggggc 180

gaggcggccg gggcgtgtga gccgctcggc cagctgccgt ctacgggctt ccgcgcggcc 240

accgggcaac tgcgccgcgc ggctgcccca ctgagcgctc ggcctcgggg ccgtgggatc 300

cgccgcgctg tctgcggtca ggaagaccgc cctcccgcgt ccgtgccgga cgggtcagag 360

gcggcgccgc acgcgaggcc acccgcgatg ctgctgtcca agttcggctc cctggcgcac 420

ctctgcgggc ctggcggcgt ggaccacctc ccagtgaaga tcctacagcc agccaaggcg 480

gacaaggaga gcttcgagaa ggtgtaccag gtgggcgccg tgctcggcag cggcggcttc 540

ggcacggtct acgcgggcag ccgcatcgcc gacggactcc cggtggctgt gaagcacgtg 600

gtgaaggagc gggtgaccga gtggggcagt ctcggcggaa tggccgtgcc cctggaggtg 660

gtgctgctgc gcaaggtggg cgcggcgggc ggcgcgcgcg gcgtcatccg cctgctggac 720

tggttcgagc ggcccgacgg cttcctgctg gtgctggagc gacccgagcc ggcacaggac 780

ctcttcgact tcatcactga acgcggcgcc ctggacgagc ctctggctcg tcgcttcttc 840

gcgcaggtgc tcgccgctgt gcggcactgc cacaattgtg gggtcgtgca ccgcgacatc 900

aaggacgaga acctgctggt ggacttgcgc tcgggcgagc tgaagctcat cgacttcggc 960

tcgggcgcgg tgctcaagga cacggtctac actgactttg atggcacccg tgtgtacagc 1020

cccccagagt ggatccggta tcatcgatat cacgggcggt ctgccactgt gtggtctctg 1080

ggtgtactgc tctacgacat ggtgtgtggg gacattccct ttgagcagga tgaggagatc 1140

ttgcgcggca ggctcttttt ccggaggagg gtctccccag agtgccagca gcttattgag 1200

tggtgtctct ccctgcggcc ctcagagagg ccctcgctgg accaaattgc tgcccatccc 1260

tggatgctgg ggacagaggg cagcgttcca gagaactgtg accttcggct ctgtgccctg 1320

gatactgatg acggagccag taccacttcc agcagtgaga gcttgtgagg aggaggaggg 1380

gcctggactc cacactgggg gcctgggctc agcctagcca gccctctccc agaatgaaca 1440

ttttctgcct gggatgtctc ctgcaaaagc agtgacctct gacccctggt gacctttgct 1500

ctcggcaccg ggcctgtttc ctttgctttg agtgcctttt tgaacgctgc tccacagggc 1560

ctgggttttc ttgagctctt ctgtccaaag atggctgcgg gctaagcaag gtcccgcctg 1620

ccctgggtgg atacttgaac ccgagaccct accctgctgc tccatcttgc ggcagccttc 1680

ctgaccaagt gtgtttgaca tggagcgccc tgtggtgccc acctccaacc ctccagtctc 1740

ctggtcttcg tctgggcatg tctgcacaag caatgcaacg ctgggccact gctgcccgcc 1800

tgcctccctg gcaccgcacg caacgagcgt gccacggtct cttatttatg gtgtgatcac 1860

cctggagggc gcccctgccc tgctggggct atttattgtt taatttattt gctgaggtta 1920

cttcctccaa gcaaccacct tctccaggcc cctggggtgt tcaggaaagc caagggtggc 1980

cgttcagtcc acagacggca tcctggttcc tgcacctgca gtaggtccct aaccccatgt 2040

ttgtgggagg aggaatttgt acagtggcta atttaagggg agtgggagac cctgtcaccc 2100

tgggcactct gcgctgggga gggggtttaa attattgacc ttgtacagtc tgcttgctgg 2160

ctctgaaagc tggggtgggg gacagagtct caagccctta atttatttta gcaactgtgt 2220

tctgtgaccc tggtgtgagt aggcatcagg ggtggggttg tataagttca aaagtgtgaa 2280

atgtctggag atcatatttt ttatacaggt atttcaatta aatgttttgg tatat 2335

<210> 634

<211> 2600

<212> DNA

<213> Rattus norvegicus (Norway rat)

<220>

<221> misc_feature

<222> (1714)..(1714)

<223> n

<400> 634
   gcgatctact ctcagtcttc ctgagtcaca ggctaccaag gacaagtagc tctgtctgcc 60
   ctttgttgag gggagctgtg acactggttt gctgctgctg ctgctgctgc tgctgcatct 120
   tctttgggaa atcagactca ataaactgcc atccatctga ggttctcaac tcagagccat 180
   ctacctggag catgggactt tcaaatgtcc agctctggac aatactgctg tgggcattgg 240
   catgggtgca gagtacaaga tctgcgtgcc cgtcctgtgg ggccccaact ctgacacccc 300
   aaggagaacg cgctctggtc ctagagctag ccaagcagca aatcctggag ggactgcacc 360
   taaccagccg tcccagaata actcgtcctc tgccccaggc agcactgacc agagccctcc 420
   ggagactgca gcccaggagc atggtccctg gcaaccgaga gaaagtcatc agctttgcta 480
   ccagcataga caaatccact tcaacctacc gctccgtgct caccttccaa ctgtcccctc 540
   tttggtccca ccacctgtac catgcccgcc tctggctgca cgtgcctccc tcttttccgg 600
   ccactctgta tctgaggatc ttcggttgcg gtaccacgag gtgcagagga tcccgcacgt 660
   tcctagctga gcaccaaacc acttcctccg gctggcacgc cctgactctg ccctctagcg 720
   gcttgcggag tgaggaatct ggagtcacaa aactccaact ggaattcaga cctctggacc 780
   ttaacagcac tactgccaga ctgccacggc tgctgttgga cacagcggga cagcagcgtc 840
   ccttcttgga acttaagatc cgagctaatg aacccggagc aggccgggcc aggaggagga 900
   ctcccacctg tgagtctgag acccccttat gttgtagacg agaccactat gtcgatttcc 960
   aggagctggg gtggagagac tggatcctgc agccggaggg ataccagctg aattactgca 1020
   gtgggcagtg cccgccccac ctggctggca gccctggcat tgctgcttcc ttccattctg 1080
   ctgtctttag cctcctcaaa gccaacaacc cttggcctgc gggttcttcc tgctgtgtcc 1140
   ccaccgcgcg aaggcctctc tccctcctct accttgacca taatggcaat gtggtcaaga 1200
   ccgatgtgcc agacatggtt gtagaggcct gtggctgcag ctagcaagag gacctgaagg 1260
   ttttgagtga agttcaaggt tcaagttggg ggttcccaag caaaagggtc ctgtgtctgg 1320
   aagcacaagt tccggatcca tactgacacc caacaagctg tgtagcagta tgcctgagtt 1380
   tgacccctgt ggaactcaaa tgggcatttt tcttgtccca gattctggcc tatttcaggc 1440
   tgtttcaaat gtggacagat gggtaaaacg tttcctttcg agaaactgcc tggccagcac 1500
   catttcctac atcaagccct gttccaggac agcaggggat gccgtgggag ggaaggaaga 1560
   atgcagggaa aactacttag tctctcccga aaaagaattc ctcaagtaat aaaggaggaa 1620
   ttagaaggtt aggcagattg gggaaaggta tacaggctaa gaacagggac tgttgcctgc 1680
   tttgagcaag gtcaagagga atatgagcag gcantgaggt gggagagcag ttaggaggcc 1740
   ctggaattga cagtcagtaa aaaggggtgc tgaacccata agttctctag cttccccttg 1800
   gggagaggac ccatgtaaat gacatacgtt tatattttct taataaaaag gagaaagaaa 1860
   agcaacagtg tgtaaggggt tgttaaaatg agccagaaac aaagtgtggt ctggggacct 1920
   ctgtgcttcg gggccttttc aaggggtttt caaactattt gcataatcac atagagaagt 1980
   tatttgtcat ttactatcat tattttttca ttgttctgtg ggaacagggt ttacacttat 2040
   aactacagcc ctcaggaggc tgagacagga ggatcgccgt gagttcaaga ccagccttca 2100
   tagtgaatgc tgggctagcc tgagctatgg agtgagatcc tgtctcaaag caaaagaaac 2160
   aaacaaaaca acaaagacta agtagtgagt ctttccagag tgtattatat gatacagccg 2220
   atgacattgt ggcttatata ttctcctttc tctcctttaa acaattttat ttatttttat 2280
   tgtatgtgta tgatgagtct taggcctgca ccggcttgca gtgcccatag aggtcagaag 2340
   aggacgctgg atccttggaa ctaaagttag agcagtgtga gctgcctgtg tctgttaaca 2400
   gaactcaggt ccacagtaag agaaacaaac aattaaaggc tgagctacct ctccacctga 2460
   gacatgtgca cacacagtca cacattgttt tgttttgttt ggaaacagcc cacgctatct 2520
   tggaactctc aatcctcctg cctcagaccc tcctgccctg gaattaaagg cataaaccac 2580
   caaaaaaaaa aaaaaaaaaa 2600

<210> 635

<211> 92

<212> DNA

<213> Rattus norvegicus (rat)

<400> 635
agccgacatt ctgtttggca cacgttatta tctgcttatt ttccaggata cacatttccc 60

tgagaaccca cggcagagat gtgagggcag aa 92

<210> 636

<211> 2199

<212> DNA

<213> Rattus norvegicus (Norway rat)

<400> 636
ccggaaggag ttcggcaggt ctcccggacc ccctttgtga actacatttt gggtagaaca 60

ttttaatcac cgaaaccctt ctgaggaaaa aaagactccc actgccccct gcaaaaaaac 120

aaaagcaaaa acaaaaaaac caaaccccaa acccccaaat ctcagtctta gctggtgcca 180

ccaaagaagc ttttgagcca tgtggacttt tcttggaatt gctactttca cctattttta 240

taagaaatgc ggggatgtca ccctggccaa caaggagctc ctgctgtgcg tactggtgtt 300

cctgtcgctg ggcctggtgc tctcctaccg ctgtcgccat cgaaacgggg gcctccttgg 360

ccgccatcag agcggctccc agttcgctgc cttctcagat attctctctg ctttgcctct 420

tattggcttc ttctgggcca aatcaccccc tgaatcagaa aagaaagaac agctggagtc 480

caagaggcgc agaaaagaag tcaatctgtc agagacgacg ttaacgggag cagctacctc 540

agtatcgaca tcatctgtga cggacccaga agtgatcatc atagggtctg gtgtacttgg 600

ctctgctttg gcaacagtac tctctagaga tggaagaacg gtgacagtaa tcgagagaga 660

tttaaaagag cctgacagaa tacttggaga gtgtctgcag cccggtggct atcgagttct 720

tcgagagctg ggccttggag atacagtaga aagtcttaat gcccatcata tacacggcta 780

cgtaattcat gactgtgaaa gcaggtctga agttcaaatt ccatacccgg tgtcagaaaa 840

caaccaagta cagagtgggg ttgctttcca ccatggcaag ttcatcatga gtctccggaa 900

agcagctatg gcagagccca atgtaaagtt tatagaaggt gttgtgcttc ggttactaga 960

ggaagatgat gctgtgattg gtgtgcaata caaggacaag gagactgggg acaccaagga 1020

gctccatgcc ccgctcactg ttgttgccga cgggctcttc tccaagttca ggaagaacct 1080

catctccaat aaagtctctg tttcctccca cttcgttggc ttcattatga aggatgcacc 1140

acagtttaaa gccaatttcg cggagcttgt tctggtcgat cccagtccag ttctcatcta 1200

ccagatttca cccagcgaaa ctagggtgct tgtcgatatt cgaggagaat tgccaagaaa 1260

cttaagagaa tacatgacag aacaaattta cccacagata cctgatcact tgaaggaatc 1320

atttctggag gcctgtcaga atgctcgtct gcggaccatg ccagcaagct tccttcctcc 1380

ttcctcagtg aacaaacgag gcgtcctact tctgggagat gcgtataacc tgaggcatcc 1440

tcttactggt ggaggaatga cagtcgcttt aaaagatata aaaatatgga gacaactgtt 1500

aaaagatatt cctgaccttt atgatgatgc cgctattttc caggccaaaa agtcattctt 1560

ttggtcaaga aaaaggagcc attcctttgt tgtgaacgtg ctggctcagg cgttgtatga 1620

actattttct gctacagatg attccttgcg tcagctccga aaagcttgct ttctttattt 1680

taaacttggt ggagaatgtt tgactggtcc tgttgggttg ctttctatat tgtctcctga 1740

ccctctccta ttgattcgac acttcttctc cgttgcagtc tatgccacgt atttctgctt 1800

caagtcagag ccatgggcta caaaacctcg ggctcttttc agtagtggtg ctatactgta 1860

caaagcgtgc tctataatat ttcctctcat ctattcagaa atgaagtatc tggttcactg 1920

aaagggaaac cacatggaag tcattgggac ttgaggagga tgtacatagc ctaggactgt 1980

gtgtgtgact tctgcagtgg tgactcaagg accaagcttg tgtataagca tgtaaatata 2040

tgccttaatt cacaattgag aatgaagggt caataagcta gatgtttaaa agaatgattg 2100

tttccacaaa ttaatgttaa cactcaagtg tttggatttc agtattgggg tgattcaatt 2160

ggggacaatg ccaaataaaa tgaaggagtg gaactcctt 2199

<210> 637

<211> 1111

<212> DNA

<213> Rattus norvegicus (Norway rat)

<400> 637
agagccagga agaaacagga acaagacaca gaccatgagc tccagccaat caggggtctg 60

cccatgccag ggctctgcaa gccaccccac cattctctac acacttctaa gccccggtcc 120

caggaccagg cccatggcac ccgcatctcg cagccactgc ctgtgccagc aacaccggcc 180

tgtgcgcctg tgtgctccac accgcacctg cagggaggcc ttggatgtcc taggcaagac 240

tgtagccttc ctcaggaacc tgccgtcctt ctgcctcctg ccccatgagg atcagcgacg 300

gctgctagag ggctgctggg gccctctctt cctgcttggg ttggcccagg atactgtgac 360

ctttgaggtg gccgaggctc cggtccccag catacttaaa aagatcttgc tagaggaacc 420

caacagtggt gcccagggtg cccagccacc agacccacca caaccctcac tggctgccgt 480

gcagtggctg cagcactgcc tggagtcttt ctggagcctt gagctgggtc ccaaggagta 540

cgcatacctg aaaggcacta tcctcttcaa cccagatgtg ccaggcctcc atgcctcctg 600

ccacattgca cacctgcaac aggaggctca ctgggcattg tgtgaagtcc tggagccctg 660

gtacccagct agccaaggcc gcctggcccg aatcctcctc atggcttcca ccctcaagaa 720

catctcctgt acccttctgg tagatctctt cttccgccct gtcatcggag atgttgacat 780

cactgaactg cttgaagaca tgcttttgtt gaggtgacct gtagaatggg gtccggtgcc 840

tccaaagggg agcctgaaag gccgcactca gctcccctgg agcagccctc gtctcagccc 900

cacccccagc ttggatttcc tcggtttgca tacagtgttt gactaactgt ccagcaggcc 960

cttgatggct cccaaaacct ccaggccaat atgccttccc ttacctgggt agggatggga 1020

gcatgaggct gtgcttcagg gcagaattcc tccagacttt atatagaact aagttaagtt 1080

attggttttt gtaataaaag gtgtgaactg c 1111

<210> 638

<211> 8377

<212> DNA

<213> Rattus norvegicus (Norway rat)

<400> 638
aagcttgctt ctaatttcca aaatgttaga gttggttttt ctggagaatc tcaggattaa 60

caaaacaaag acacaaaaag tgaagtcctt gttgagatca aaagtcactt tagagtagga 120

gagatggccg ggtggttcac agtacttgct gcccttacag aggacctgaa ttcagttccc 180

tacatgggca gctcactacc ccctgtgact cccagtcatg ggggacccaa tgccctcttc 240

tttcttctca ggcatctata tgcacgtggc atgaatacat acatacattt acttgggtct 300

acagacacac ataaaataaa taatatgttt tcgttgttgt tgtcttttcc agacaggttt 360

tctctgtgta gccttggctg tcctggaact ccctctgtag accaagctgg cctcaaactc 420

acagagatgt gccagcctct gcctcctggg tgctaggctc aaaggcctgc ctgtaccatc 480

actacctgga aaacagtaaa tactttttaa agaggtattt taagaagtaa atacttttaa 540

caataacaaa aaaggacaac tagaactacg gaggctttga agaaccaccc agcatgtttg 600

tgtattctct gtcctgtacc aaaggtccag ctcgccagga attcggaggg taattgtcca 660

agtcctcccc tctaagcttc ggtgggttat ctgccgttat agatgtgtca gagccatgtg 720
tcctgacttc agtctccggt actcaggcac caggaagtca cagtgtggcc caggttctag 780
gagagcacca aggaggcgcc agaggtgggt gctgcactcc atgagaccct gggcagaaag 840
cttcatcctc cagctcacat tccccgcctg taagactgga gccatctatc ccccactgtg 900
acacatgggg atgaaccttg gaaaagtcag tctaggcagg tgttagtaaa acaaacagga 960
ctatgtacat aaaggattga gaaaaccagt catccttaga gttccgggaa ggtagactca 1020
agtatgtaga cttggggccc ggatgggtca atccctgggt ttgcccacca gcatctcaca 1080
gctagaattg tctcctagtt ctggaacctt ccagattcct gagaggctgg ctactcctgc 1140
cttcctatca ctgaatagag tagagggttc tggaaaggac ccaaaaaatt ctgctaagag 1200
gcaattcact cagatttcct tttttttttt ttaagagctt gccagagtca tatgatttat 1260
ccccatacag gccaggccga ccagcctcgg ggcggggtgt tgcaacaaca gccagaacgc 1320
cggctcagct gggcggccac cacgtgactc gagtacttaa agggctggcg cgggcagctg 1380
ctcgctcaca gtcgccagtc gcctccagag tttccgcctc caaccagcga gtggagcctg 1440
ccggagcaga gccatctcga gcggagcccg gagcctgaag cgagcggagc cagcctgaac 1500
tagcccagtc ccgcgatgga gcgcccacag ctcgacaggc aagcgactac ttggggacgg 1560
gaactgtgat gtcccgcgtc ccttgtccag cctagaaatg caggctgcgg gaccaaggaa 1620
cgagttgccg cccagtctac ctgtagccat gggcagctgg agggagaaaa tgtgattcac 1680
ccctctgaca cgctggaggt gaccggatgg gtgtgtcttg tagtatccag gtttgaggga 1740
tgaacagtgc tgagtcctgg atcctggact caccgttcta gggggcgggg gttggggagc 1800
ttggccctct tggtggcgcg gagtggtcac atcaattccc aacttcagac gacttgtaag 1860
acgcagggag gcagccgtag cagaactaga gggtgttgag gctgggtgtt ttttgtttgg 1920
ggatgggggg gacaggaagt tttttgtttg cttggtgttt tgttctgttt tgctttgggg 1980
tccctgtgta gatcaagttg gccttgaact ccagttcgct tccctttgct tcctgagtac 2040
tgagattgaa ggtgccccgc caccacactt ggcttctatt cctcctcctc ctcctcctcc 2100
tccttctcct cttcctcctc ttcctcttcc tcttcttcct ccttctcctc ttcctccttc 2160
tcctctttga ttttttttga gacggagcgc ctctttgtag ccctggatgt cttcactctg 2220
tagatcaggc tggcctagaa ctcggagatc tgcctgcctc tgcctcccta gtgctgagat 2280
tacaggcctg caccactctg tccagctctt ttttcttttt ttccttttat aaagccagtt 2340
ttatttttat atttttattt aaagcgtctc gagttacatt taatttatcg tgcatgtaga 2400
gtggccacat atatgacaca ctaaatatgt ggaggtcaga gttcgacttg aagaaagtca 2460
cttctcagtc taccatgtgg cttccaagaa agattggctt atcaggtggc aagtgcgttt 2520
cctggctgag cttacactgt ctagtcctca cctccaactt ttagcagtaa ggattgaact 2580
taggacttca aacattctag gcaagcgctt tacccataag gtacagtatc agcacctttt 2640
ttatttaaag acagtgccta actaagttga ccaggctggt ctggaactct tgaatcttct 2700

gctgcagaac ctctctaaat aaagctgggt tacatagact atttaatgca gaaactttga 2760
gtatagtgta tagttggttt tggatgggtt ttttgtttgt tttttgtttt gttttgctga 2820
gactcttcct acatagccct gtgctgaaat tcattcacta tatagataga cccaggttgg 2880
ctttaaacta agagatctgc ctacctccac ctcctgcatg ctggaattaa aggtgtgcac 2940
catcacgtcc tgctgcttgt aaaatctgta actgaaattt tttaagaaat atcttcaggt 3000
tggatctaaa agctcaccaa agtatctacc cttagccggg ctgtggtggc ccatgtcttt 3060
aatcccagca ctcaggaggc agagggaagc acatctctag agttctgggc cagcctgctc 3120
tacagagtga gatccaggac agccagggct acacagagaa acgctgtctt ggaaagagaa 3180
aatctgccct tgagtatggt ccttggactg gcgtggctct tccctgaggt caggacatac 3240
cttgcctcct cttcactggg aataactggc gtaggaatcc tccctggggt tttggggact 3300
acgtcacata gtgtttaatg actgacatct gtttgcatgg ttaagatcat ggcttggcct 3360
acattggagg ctggagctat tctgcaggtc taatcattca taggcatccc cggctgactc 3420
tcctctcttc ctccacagca tgtcccagga tttgtccgag gccttgaagg aggccaccaa 3480
ggaggtgcac atccgtgcag agaattctga gttcatgagg aactttcaga agggtcaggt 3540
gtccagggaa ggctttaagg tacgtgacgt gtaggttgac cagaccactg ggtatagcca 3600
ggtataccca gggcttaagc caatagttta atggggaggt tggtgccagg agtgatgagg 3660
gcctgtccaa ggacgccatc ttggtaatgc tgaccctgta gctcagagag gaaagggtct 3720
gctggtggag gagctggtgc agtggggtgg ttaagtgaac acgtattcta tgttggtcat 3780
tttatgttct tgaggagtgc ctaaggagaa ggaacagaga ggttaaagag gccatcctgt 3840
cagctttcag tggaaaagac caaaaccaaa gatggccaaa tttcagagct tgcgctcttg 3900
acctggttac cgcagggcct catacatgcc ggctaggttc tctaccactg acccacaatc 3960
caagctctta ggttactaga tcgggtctca ctacacagtt caggaactct caatactcct 4020
gcgtcagcct cccgggtatt tggaccatgg gagacccaaa tctcctgacc caatttcaac 4080
ttgcctctga aggaaaggag aagtttgcct tagccctgca gtgtcctgtt cccacagtga 4140
gattggctac ctcatttaac ctcccaagcc ctcttgcctg aggaatgggg ttttgacagc 4200
tgggctgaaa tgcacagtag acacaataga ccctgtttcc tgccagctgt ggaactgatg 4260
tctgcagcaa acatcagaca cagggaagag agcactgact gggtggggag tggagggtgg 4320
tggagacagg agaccgtcct tccagaaggg ttcttctagc gacaagttga ttctgttttt 4380
ttcagctggt gatggcctcc ttgtaccata tctatacggc cctggaagag gagatagagc 4440
gaaacaagca gaacccagtc tatgccccgc tctacttccc tgaggagctg caccgaaggg 4500
ctgccctaga gcaggacatg gccttctggt atgggcccca ctggcaggag gccatccctt 4560
acacaccagc cacacagcac tacgtaaagc gtctccacga ggtgggaggt actcatcctg 4620
agctgctggt ggcccacgca tatacccgct acctgggtga cctctcaggg ggtcaggtcc 4680
tgaagaagat tgcgcagaag gccatggcct tgccaagctc tggggaaggc ctggcttttt 4740

tcaccttccc gagcatcgac aaccccacca agttcaaaca gctctatcgt gctcgcatga 4800

acactctgga gatgaccccc gaggtcaagc acagggtgac agaagaggct aagaccgcct 4860

tcctgctcaa cattgaggtg agccccactg ttccttcaag ccccattcat tccagttatt 4920

ggaggaaggg tccccagagt cccagttagt acaaggactt ggggccagcc agggtcctgt 4980

tttgctgctt aactagctgg gtgattgtgg gcaaatctct tcatcctcag gttctaaatc 5040

tccaaaatag ggataataat agcatccatc ttcaaggcga gaggggcaag caagttgtgg 5100

ttcagtccct gtgggaggag tgatacaggg gaagttccct gatgtcattg ctctcttgtg 5160

tggtgggctg tgatcctctg acaatggtgt agctggacct ggggtgcctg agtactggtg 5220

catggccagg ccagcacagt ccaggcttcg gatgctgaca tgtcccaggt ggcatttgct 5280

tccttttctt tgatttgcat tgatgtgttg cccacgggta tatctgtttg gtgtcagatt 5340

cgttgggggt ggagctacag acagctggga gctgccacag gggaaccggg aattgaaccc 5400

gcatcctctg gagagcaact aacgctctta atctttgaac tatctttagc tcggtgtttt 5460

gagcaaggcc ccgaaaggca gagctggggg agtagactgt cctatttata ctatgggtgc 5520

tatggtggtc agggttccaa tttgggacct cctgtctctc cccactttca gcctgagagc 5580

ctttaaaagt tccaccaggc aagcacctgt tagagaagta accttcagat gtcctaaatt 5640

catctaggcc cgttcacctg aggccttccg aggacatgat ataaagaatg agtttagaag 5700

tggggaagtg atggcccatt cctttaatct cagcactcag aaggcagaga caggaggatc 5760

tctgtgagtt ccaggtcagc ttggtctaca cagctagttc taggacagcc gaggctacac 5820

aaagaaaccc tgtcttgaaa aacaaaacaa aacaccaaca agaaaagagg attagacaca 5880

ggcccagctg agaactgggt ctccatcctg acccctagtc gatctcaaga aagccctgct 5940

ccaggagctg tggttccctg acctgcaaaa gcagcgacca tactccatta cacgacacac 6000

cgcctagcaa ggaggagctc aagtacagtc atgaagagag cacgggatgt aggctgcacg 6060

gctcccacac attcgtgtgt tatttccgaa gaggcaaact ggttctcctc atagggatac 6120

agaaaagctc ctgaagtctt tattcagggc agccgttact ggtggggtcc aagcgagggt 6180

acagacagta tcggggacgc acaggcaggg agccaactaa acctgtcaca tctcatgaga 6240

taagtgaaag acgcggtatg cgatagtttc ctcaagtcgt gtcatttgta caatatttcc 6300

caacaggctt caagctgtgc cttcccctgg gtccccccac attgtgagta tcactaacag 6360

gcagtgtgtc ttgtcttgtc ttcagctgtt tgaggagctg caggcactgc tgacagagga 6420

acacaaagac cagagtccct cacagacaga gtttcttcgc cagaggcctg ctagcctggt 6480

tcaaggtaaa gatggcctct tggcttctgc tctccttagc ccagctggaa tttctttact 6540

cttctccatt ctgaacgttt taaattagag atagggtgca gacaaaagag cccagggtaa 6600

gtctgtagta tgactgtggg taagctatca gacttttcta ccttagtttc tccacattag 6660

aaaatcagga ggttgggggc tgtgtagctc gttggctgag gggcatataa agccctcaga 6720

agtcactggc acagagatcc tctcatttag atcccgcctc ccacttcttt tcccccaagg 6780

cagagccatg ctgcatgacc caggctggtc tcaaaccctg ggagtcaaac aatcctcctg 6840

cttcaatctt tcataaaaat ctagggtgtg tttacctttt gaggcccact caaggctccc 6900

atgcaggccc catatgaggc aaggatttgg gaagctattt ctagcgctga ccctccagga 6960

gacacgacgg agttcatgta attactggag tgtcagccct tttcctgtgc cttgatacac 7020

tacccttgga gcaggagtaa gggttcttaa aaagaccaaa gtggcagaaa atgtgccttc 7080

cctgagccta tcacccctgg gacagtatgg cccagggagt gggtgactga aactgagcag 7140

ggcaggttac gtgaagcttg attgcttgaa tgggcccacc ttttgcccaa ggctctccca 7200

agggaggcag gacccgccac cttgtcatta gtggtactgt ttctggtggg ctgaaatatg 7260

gtcagtgctg atagaattag ttacctgttt gggtctggag tctttgacct gctgcggcct 7320

ggcggagcac aggaggaaca gcgtggggga gttggtgagc tcctatcttt gaaggtgttt 7380

aagcagtggc tagtggttca agctgtagca gaatcctggc cttggactgg aactgtagca 7440

gacacggcac cgagtgtcct ctgctctgtg cagtgtcatc caccccaggc tgtgtgtttg 7500

accttgtctg tgtttctcct acagatacta cctctgcaga gacgccccga ggaaaatccc 7560

agatcagcac tagttcatcc cagacaccgc tcctgcgatg ggtcctcaca ctcagtttcc 7620

tgttggcgac cgtggcagtg ggaatttatg ccatgtaaat gcagtgttgg cccccagagg 7680

ctgtgaactc tgtctcatgt agccttctct ctgcagggga gaatcttgcc tggctctctt 7740

ttcttgggcc tctaagaaag cttttggggt tcctcgcccc cttcctgtgt cttcctttgt 7800

ctctctggaa tggaaggaga tgcctggcac atttccctca ccaaaagcac agccaggggc 7860

ctgaacttgg aaaccagcag ccccaaatcc tgcaacagag ccccagaact gtggtcggta 7920

gaggcggctg ttctgagccc agtgcccttg gtagtcggcg tccatgttga ctgaccatga 7980

ctgctttccc ccagcgtcat ggccactttg atatcagtgt gcagagattt tctgtcttgt 8040

gtttctgtct tgtttttgtt atttccccag ttctaccagt gtaatggtat tttttgttgt 8100

atcgattgtt tttttattct aaccaagtag ggctgtcttt tgaggggtgg gtggtattta 8160

ataattgtaa ccttggtctc taacttctgt gtgaaataat aaatggcatt atctaataat 8220

caccggaagt tcgttcatgt ctctgccgtc cacgaacagg gccctgctcc tcctgtctct 8280

tcagtgtctg gcaggaggag ggcaggtggt ggtggcatca gtagctttga cggtccactg 8340

tgtctgagag ttggaagtga gcttcaagca tttgggc 8377

<210> 639

<211> 1937

<212> DNA

<213> Rattus norvegicus (Norway rat)

<400> 639
ccgacgtccc ctcagattcc atcgcgatgg cccctccatc attctttgcc caggttccac 60

aggccccgcc ggttctggtc tttaagctca ttgcggactt ccgggatgat cccgatcccc 120

gcaaggttaa cctcggcgtg ggagcgtacc gcacagatga ctctcagccc tgggttttgc 180

cagtagtgac gaaggtcgaa cagaagattg ctaacgacca cagtctcaac cacgagtact 240

tgcccatcct gggcctggcg gagttccgga gctgtgcttc tcagctagta cttggggaca 300

acagcccagc tctcagggag aatggggttg ggggtgtgca gtctttggga gcgaccggtg 360

cacttcgaat tggagctgac ttcttagcgc gatggtacaa tggcacagac aacaagaaca 420

cgcccgtcta cgtatcatcg ccgacctggg agaaccataa tggcgtgttt tctgccgctg 480

gttttaaaga cattcggtcc tatcgctact gggatgcaga gaagagagga cttgatctcc 540

agggtttcct gaatgatctg gagaatgctc ctgagttctc catctttgtc ctccacgcct 600

gtgcacacaa cccaacgggg accgacccaa ctgaagagga gtggaagcag atcgccgccg 660

tcatgaagcg ccgttttctg ttccccttct ttgactcagc ctatcagggc tttgcatctg 720

gagacctaga gaaagatgcc tgggctattc gctattttgt gtctgaaggc ttcgagctct 780

tctgtcccca gtccttctcc aagaacttcg ggctctacaa tgagagagtg gggaatctga 840

ccgtggtcgg aaaagagcat gacagcgtcc tgcgggtcct ttcccagatg gagaagattg 900

tacgaatcac ctggtccaat ccccctgccc agggagctcg gatcgtggcc accaccctct 960

ccaaccctga gctctttaag gagtggaaag gaaacgtgaa gacaatggct gaccggattc 1020

tgaccatgag atccgaactc agggcgcgac tagaagctct caagactccc gggacttggt 1080

ctcacatcac tgagcagatt ggaatgttca gctttactgg gttgaacccc aagcaggtcg 1140

agtatttggt caacgagaag cacatctatc tgatgccgag cggtcggatc aacatgtgcg 1200

gcttgaccac caagaaccta gattatgtgg ctacctccat caatgaagct gtcaccaaat 1260

tccagtgaag aaacaccgag tagttcatac cccaaagcag ttcctgtcac agctttcctg 1320

cctgcgcaaa cctagccgta catgttgttt attagagatg accaccatgg ggaggcagcc 1380

gctgtttagc tggccccaca agagaagaca tttcttgaaa tgaacctggg tcgggtgggg 1440

ggatgactgg ggttagggcc ttttggaaac cagagcagat taaagttatt taagaataaa 1500

aaaacccttt gatatgagat gtaatcatct tgccttcctc tgtagtattc tgcaggagtg 1560

ttgcccacga agccgtgggc ttctgcacgt tgcttgagtc tgtacagagt cctgtcccca 1620

aaatcaagtt gtctgaggag ccggctgtga ctgtggatgt tggcattaaa actcaccatt 1680

tccatcgtct ctgtctctcg gccccctgat ctttccgcat ggttgtgacc ctggtcttgg 1740

aacattagtt ttttaaggcc actgtggcca gtatttatat catgacacac aagtggattt 1800

acatatttaa ctgagatgaa agttccgcta aacggtattt gctcttgtga tacgtggcac 1860

attgtgacat tttcttagtc tcttctgtcg tgttctgttt catttaaaaa aataaaaatg 1920

ctgatcaaga caaacgg 1937

<210> 640

<211>  409

<212>  DNA

<213>  Rattus norvegicus (Norway rat)

<400>  640
tgcaactgaa atgcattctc tagcttgcca gtggccccca ctgcctgcct gcctgcagga      60

actactctag acatagattc ctcctccact ctagcaagag aagatgctgt ctaatttgag     120

gatcctgctc aacaaggcag ctcttagaaa ggctcacact tccatggttc gaaattttcg     180

gtatgggaag ccagtccaga gtcaagtaca gctgaaaggc cgtgacctcc tcaacctgaa     240

gaacttcaca ggagaggaga ttcagtacat gctatggctc tctgcagatc tgaaattcag     300

gatcaaacag aaaggagaat acttgccttt attgcaaggg aaatccttag ggatgatttt     360

tgagaaaaga agtactcgaa caagactgtc cacagaaaca cgcttcgct                 409

<210>  641

<211>  2237

<212>  DNA

<213>  Rattus norvegicus (Norway rat)

<400>  641
gccagcttcc tgaggtacca aggaggaagg atggaggaaa gaatgaacgt gctccacgac      60

tttgggatcc agtcgactcg ctacctccaa gtgaattacg aagactccca ggactggttt     120

gtcttggtgt ctgtgatcgc tgacctcagg aacgccttct atgtcctctt tcccatctgg     180

ttccacattc aagagactgt gggcatcaat ctcctctggg tggcagtggt tggagactgg     240

ttcaacctcg tctttaagtg gattcttttt ggacagcgcc cgtattggtg ggtcctggac     300

actgactatt acagcaacag ctccgtgcct ctgataaaac agttccccgt cacctgtgag     360

actggaccag ggagtccctc gggccatgcc atgggcacag caggtgtata ctacgttatg     420

gttacttcta ctctcgctat ctttcgtgga aagaaaaagt caacgtatgg attccggtgc     480

ttgaatgtcg tcttgtggtt gggatactgg gctgtgcagc tgaacgtctg tctgtcccgg     540

atctaccttg cggctcactt tccccatcag gtggtggctg gagtcttgtc aggcattgct     600

gtggctgaaa ctttcagcca catccggggc atctacaatg ccagcctcca gaggtactgt     660

ctcatcacct tcttcctgtt tggtttcgca cttggatttt acctgctgct aaaggggcta     720

ggggtggacc tcctgtggac tttggagaag gccaagagat ggtgtgagcg ccccgagtgg     780

gtccaccttg acactacacc ctttgccagc ctcttcaaaa acctggggac cctcttgggg     840

ttggggctgg ccctcaactc cagcatgtac cgcaagagct gcaaaggaga actgcgcaag     900

tcgctcccat tccgtttggc ctgcatcgtg gcctccttgg gcctgctgca tctctttgac     960

tcgctgaagc ccccgtccca gattgagtcg atcttctaca tcttgtcttt ctgcaagagt    1020

gcgaccgtcc cctttgcatc tgtcagtctt atcccctact gcctagcccg gctcctggga 1080
cagacacaca agaagtcttt gtaaagcgtt aagtctctga cacaagtcag cggccgtgca 1140
aaggactagg agcaacccaa gccttgtgca acccagtgtg gggccagagg tgttcacagc 1200
caccctggta gccctgtctt tctttgccat cgtaaccaaa aaggtgaatt tttaaaagct 1260
aacagggctg tttgaggaaa gcgtgaatgc tggaagatgt ttccctgatg aatcacttcc 1320
ggtctcctgt ccgacacaga agagaaagcc cagactagag atcctgacag aatgctctca 1380
ctgtgcagaa tcctccaggc cgagggtaaa agaaaagagc gttgtgcatt tgctaggaag 1440
agaaggaagg atcgggagga gggggagtgt ttggtggatt gagcaaacca tatgcaagcc 1500
atgtctaccc ggcttcagtt atgcctgggt ctttagatat gtaggacact ctcaataatg 1560
atagaccagc ccgtgtaatg agtagccagt tagaggcgat cattctgctc ccagattttt 1620
ctctactgta catgaaagtc acaccgtgtg cttgtattcc tgaatggtaa tggtgactgt 1680
cactcaggaa ccaccacctt ctctgtgaca ccacagcttt gcctcctcgg cccaggtctg 1740
cgtgccatag gactcatcac tgcttccctg accacgctgc tggaccctgc attgctgaac 1800
cccatgctgc tggaccctgt ggtgtacggt gggcaattgt ttgctggtgc ttttgtaggg 1860
ttaagttaaa ctctgagatc ttgggcaaaa cggcaagggg agagtcagga ttcctctctc 1920
caaaggtcac tccgaagtta cttttgattc ctggagcaga aatgactcct tcctctagcc 1980
caagccagcc aagaactcat tcttagaggc aaagcagccc aagcccgact gtgcctgtcc 2040
tccagcctgg gctggttttg cagagtgtgt cttcaagaag aaaaaatggt aaagctattt 2100
attcaaaatg cttgtttttt gctatgaatg atgcctgtgt ttccacccac gccaagcact 2160
tgttcctatt gtcttgataa gaaactacat gtgtgcagta ttttattaaa acgacatttt 2220
atttaaaaaa aaaaaaa 2237

<210> 642

<211> 2335

<212> DNA

<213> Rattus norvegicus (Norway rat)

<400> 642
ctccagtggc tgagtgatca actatactcg atgtatcttc cagggcttct gcagaagtgc 60
aagatgtctt catgacctac accgtgtatg atgacatcat caccattaag ctcatccaag 120
aagcctgcaa ggttctggat gtgtccatgg aagccattct gaagctcttt ggcgaatact 180
tctttaagtt ctgtaagatg tctggctatg acaggatgct gcggacactt ggaggaaatc 240
tcaccgagtt tattgaaaac ctagatgcac tccacagtta cctggcactg tcctatcagg 300
aaatgaacgc accatccttt cgagtggagg aaggagctga cggggcgatg cttctccact 360
actactcaga cagacatggt ctgtgtcaca ttgtaccagg tatcattgaa gctgtggcca 420

aggacttctt tgacactgat gtggccatga gtatcctgga tatgaacgaa gaggtggaaa 480

ggacagggaa gaaagaacat gttgtgtttc tggtcgtgca gaaggctcac agacagataa 540

gaggagcaaa ggcaagccgg ccacaaggca gtgaggacag ccaggcagac caggaggctc 600

tccagggaac actccttcgg atgaaggaga gatatttaaa catccctgtt tgccctgggg 660

agaaatctca ctcaactgct gtgagggcat cggtcctttt tggaaaaggg cccctcaggg 720

acaccttcca gcccgtctat cctgagagac tatgggtcga agaggaggtg ttctgtgatg 780

cttttccttt ccacattgtc tttgatgaag cactaagggt caagcaagct ggagtgaata 840

ttcagaagta tgtccctgga atcttaaccc agaagtttgc actagatgag tatttttcca 900

tcatccaccc tcaagttact ttcaacatct ccagcatctg caagttcatt aacagtcagt 960

ttgtcttgaa gacaagaaaa gaaatgatgc ccaaagcaag gaagagccag ccgatgctca 1020

aactccgggg tcagatgatc tggatggagt ctctgaggtg catgatcttc atgtgttccc 1080

caaacgtccg cagcctgcaa gagctggaag agagcaagat gcatctttct gatatcgctc 1140

cgcacgacac gaccagggat ctcatcctcc tcaaccagca gaggctggca gagatggagc 1200

tgtcctgcca actggaaaag aagaaggagg agttgcgtgt cctttccaat cacctggcca 1260

tcgagaagaa gaagacagag accttgctgt atgccatgct gcctgaacat gtggccaacc 1320

aactcaagga gggcagaaag gtggctgcag gagaatttga aacatgtaca atccttttca 1380

gcgatgttgt gacatttacc aacatctgtg cagcctgtga acctatccaa atcgtgaaca 1440

tgctgaattc aatgtactcc aagtttgaca ggttaaccag tgtccatgat gtctacaaag 1500

tagaaacaat aggggatgct tacatggtgg tgggtggagt accagtaccc gttgaaagcc 1560

atgctcaaag agtcgccaat tttgctctgg ggatgagaat ttctgcaaaa gaagtgatga 1620

atcctgtcac tggggaacct atccagatca gagtgggaat ccacactgga ccagtcttag 1680

caggtgttgt gggagacaag atgcctcggt actgcttgtt tggtgacact gtaaacacag 1740

cctctaggat ggaaagtcac gggcttccca gcaaagtgca tctgagcccc acagcccaca 1800

gagccctgaa aaacaaaggg tttgaaattg tcaggagagg cgagatcgaa gtgaagggga 1860

aaggaaagat gaccacatac tttctgatcc agaacctgaa tgccaccgag gatgagataa 1920

tggggcgacc ttcagccccc gctgatggga aggaagtatg tactcccgga aaccaagtca 1980

ggaagtcccc tgctgtcccg aggaacacag accatcagca acaagtctac aaaggagacc 2040

cagcagacgc ttctaatgaa gtcacacttg ctgggagccc agtggcaggg cgaaactcca 2100

cagatgcagt caataaccag ccatcaccag atgagaccaa gacaagtgtc gttgctagtg 2160

gccctgtgct gtctgctttc tgtgttgtgc tgtgatcacg agaaaaagtg atcctatggg 2220

atccatttcc tgtattccat ggcagcaaag ggaattaatt tataaaaatg cttaagttca 2280

aaatgttttt gtttccatat ctcccttggg gcccctttga gaataaaaaa attag 2335

<210> 643

<211>  2519

<212>  DNA

<213>  Rattus norvegicus (Norway rat)

<400>  643
agagcccgtg aggcggccgg accatcatcg tcctgctaat acagctactt cctcaaccac      60

cggtatgagc cacgggaaga gaaccgacat gctcccggag atcgccgccg ccgtaggttt     120

cctcaccagt ctcctgagga ctcggggctg cgtgagcgag cagagactca aggttttcag     180

tagggcgctc caggacgcac tgaccgatca ttacaaacac cactggtttc cagaaaagcc     240

atccaagggc tccggctatc gctgtatccg catcaaccac aagatggacc ccatcatcag     300

caaggtggcc agccagatcg gactcagcca gccccagctg caccagctcc tgcccagcga     360

gctgaccctg tgggtcgatc cctacgaagt gtcctaccgc atcggggaag atggatccat     420

ctgcgtgctg tatgaggagg cgccggtggc cacctcctac gggctcctca cctgcaagaa     480

ccagatgatg ctgggcagga gcagtccatc gaagaactac gtgatgactg tctccagcta     540

gagaggagcc gccccgccct ggcactctac tgttctcatg ctgccctgac aacaggccac     600

cgtatacctc aacctgggga actgtatttt taaagtgaag agctatttat acatgttatt     660

ttttttttta agaaaagagg aggaaaaaaa ccaaaagttt ttttttaaaa aaacaaaaaa     720

gaaaaaacaa ttcgttaacg ggagctgctt ggaagtggtc tccccaggtg cctttggaga     780

gaactgttct tgattgagtc tatgagccag tgtttgccta ggggagtggg ttggggattg     840

gcctagccaa ggtaaaaggg gattcttggc tgatccccca ggaggtggtg gaagggagca     900

aggttagcaa ctgtgaacga gaggggtcag ggtctgctct gggttaccgt tcccgctggg     960

atgcctgtat tcctggtccc tctcttactc aggggcattc aagcctggtc tcaaataata    1020

ctacattgcc taatcttctc ttttgttttt ctgctgagat cctgggcaca cggaaaggcc    1080

tctcctgtcc cttccgtctg agcagagttt cttgaaactg tgtctcgttt ctgatcctac    1140

cctcggggtc ctgaagaggt ggtttcccgg cctagaatct atctaaacgt ttttggaggg     1200

tgggctataa ggcagatata atggagggga accgcacaaa ccctttgctt tgctctgtgc    1260

tgctttgtat ggatggatgg ttaataactt agggatgatt tgcaatggaa ttttgggacc    1320

caaagagtat ccaatggggg tgggtgtttt ggacctaagc cctccttttg ggaaccacgt     1380

gacagtctga atgctgctac cattattcct ttgagaggtg gctcaaagct ccagggaact    1440

ccaggtcctt tcttactgcc ttctcttcaa gagcaacctc ccccatttct tttccctctt    1500

tcctgcggtt gggtcctgga gggccccatt tcctaggaca agagttctca atcactgtgc    1560

aatagtccca ggaagctctg gaactgggcc tcccagcccc tcctgattcc tggtgggttt    1620

taggaccccg ccttccccgt tcttctgact ggctggtggg ccttgaggag atctccctcg    1680

gccgcaggga gggcacctgt gcactgcagg actacctggt actcctgtgg ggctgccacg    1740

gagagccaaa ccttaggcat agctttgtct cctcggtgct cagagcacct gcagggggag    1800

gttgcccccc tcagtaaaaa tccaaattta tttgtagatg tgtgcaatat ttactgttct 1860

gggttggaga aaatcgggaa acactgggaa gaagtggcct tccttcaggt tcagtgacac 1920

tgatgagggc ttctcagaag gcctcgagtc tctcaaacca aaggacagag ctagagccag 1980

ccagtcaccc ttagtgagga tccccttccc catgtctctc cactgccgtg gcatcccatg 2040

tcctggattt ctcaattcct cagtttctac tcaaaggtgc tacttaccaa acactctgcc 2100

cgtcccgctc tccccagctt cgcacagccg tcccaggtgg cttcgtctct cctgctttaa 2160

agttaacttt gggcccacag acccgagagc tgtgggttga agcaaagctg tgaatcgctc 2220

cagatggtcc ctgtgttctg tccacacaca ggtccccgcc tttttagaag cagcctcctg 2280

gtctcatgct taaatctgtt cctcactgcc cgtgttcact ttagaaatgg cagaaccaca 2340

gagctggact gttgagcagg cctgtctctc tcattaaata gaaataagta agtttgtaag 2400

ctattccgac agaagagaca aaggttactg attgtacaat agcgctttta tatggaagac 2460

tgtacagctt tatggacaaa tgtaaaactt ttttgttttt aataaaaatg tagcagacc 2519

<210> 644

<211> 2563

<212> DNA

<213> Rattus norvegicus (Norway rat)

<400> 644
cggaacagtc taagggtgtt cactttgggg actcagtgag tccccctctg ttatggagta 60

cagtgtctct tgaatcttac ttctggaaat ccacggacaa gaagactggt gacctatcga 120

ttttgtgatg cccacccctg gctcctggtt ccctctctag aggcagaaga gccaggcgca 180

gctaatggga cgctttcgga gatgctgcgt ctgacaggtg cgaagcaaca tgtgcaactc 240

ctttcttctt cctcccctca gatcctcctc catcttcacc catctttaga ggaagagcct 300

acagaaagaa gatggctcct gagcaaagag aaggggcctc tcaggtgtca gtgacatttg 360

aagatgtggc tgtgctcttt actcgggacg agtggaagaa gctggatctg tctcagagaa 420

gcctgtaccg tgaggtgatg ctggagaatt acagcaacct ggcctccatg gcaggattcc 480

tgtttaccaa accaaaggtg atctccctgt tgcagcaagg agaggatccc tggcaggtgg 540

agaaagaggg ccccagatac ttctctctcg gattgaagtg cagtcataga accactaagt 600

caactcaaac acaagactct tcatttcagg aactgattgt aagaaaatct aaaagaacct 660

ttgccttcga acctttgaac atgaagtcag aaaatctttt catacatgaa ggcaaattag 720

aggaaaagtg ggataagaat actttgactg tagaaagaag ccataaaaac aatgaattta 780

gcccaaagtc ccatagagaa aaacggtcct cagaatgtaa aaagcagata tcttatttat 840

ctaacccacc aggaatcaca ccggataaac gctataaatg tagcatgtgt gagaaaacct 900

tcattaacac ctcatctctt cgcaaacatg agaaaaacca tagtggagag aaattattta 960

aatgtaaaga atgttcaaaa gccttcagcc aaagttcagc ccttattcaa catcaaataa 1020

ctcacactgg agagaagcct tacgtatgta aagaatgtgg gaaggccttc actctcagta 1080

cgtccctgta taaacatctc agaacccaca ctgtggagaa atcctacaga tgtaaggaat 1140

gtggtaaatc ctttggccaa aggtcaggtc tttttataca ccagaagatc catgcccgag 1200

aaaaccctca tagatataac ccaggaagga aggcatccgc ttccctctct ggatgccaga 1260

gagctcattc caggaagaag acctacttgt gtaatgaatg tggcaacacc ttcaagtcta 1320

gctcctccct ccgttaccat cagagaatcc acaccggaga gaaacctttc agatgtagcg 1380

aatgcggcag agccttcagt cagagcgcat cacttattca gcacgaaaga atccacacgg 1440

gagaaaagcc ctacaggtgt ggcgagtgtg ggaaaggctt cacttctatc tcaagactca 1500

atagacaccg gataattcat acaggagaga aattgtataa ttgcaatgag tgtggcaaag 1560

ccttaagttc ccactcaact cttattattc acgaacgaat ccacactgga gagaaaccgt 1620

gtaaatgtaa agtttgtggg aaagccttca gacagagttc agctctgatc caacatcaga 1680

gaatgcacac tggggaaaga ccctataaat gtaatgagtg cgggaaaaca ttcaggtgta 1740

actcatccct aagtaaccac cagcgaatcc acactggaga gaaaccttat caatgtatag 1800

aatgtgggat gtcgtttgga caaagttctg ctcttattca gcaccgaagg attcacacgg 1860

gagagaaacc gtttaaatgt aacacatgcg gaaagacctt taggcagagc tcctcacgta 1920

tagcacatca gcgaattcat actggggaga aaccctacga atgtaacacg tgtgggaaac 1980

ttttcaacta taggtcatcc cttaccaatc attataaaat ccatgtggat gaggaccctt 2040

aaaaagtaaa tttgcatgta aaaaagcctt aaaccaaagc tcttcagaga atgggcttga 2100

gagctgtaat aaacatacta tggcgtccct gtagtcattt agttactgtc aactaaattc 2160

catggataaa tcctgtgtaa caggtaggag tgaaatattc atgcctgtcc aacactttaa 2220

aaaaattgct tgaaatccgg tggttatgtg tagtcctggc taccggggac cctgaggatt 2280

gcttgactcc acaagtttga agagcaaatg ttatggagct ttccatctca gaagattagt 2340

gacctgaaat gaagaattca cagctgaagg ccttgacttc aggtatttgg aaaatggtgc 2400

ttttctctct gcaacagtgt gtgctagata caattgtaat cataggcatc taagtgaaca 2460

gaggagtggg atggacttag caaaacacaa aactctactg ttgtgatact tttatactat 2520

tttaataata tccaaaaggg atcaacacaa atcaaaaaaa aaa 2563

<210> 645

<211> 499

<212> DNA

<213> Rattus sp.

<400> 645
ggtaacccaa ccaattcttt attgtaaaga tgagtgctac aaaggaggcc tagtgtgtag 60

ggagtggggg gtcaccttct ttcaagggac tccacaaggt ccactagaga tgacatatag 120

ccaaggaata agcctgagcc agggcagcag tggttctgaa ctctattgga agttggggtt 180

ctgaatcaag cccactgacc caggtccatg aggaagtgtt caatgtcatc gtagagacta 240

ttggtgctag acaagcacag gccaagactg ctgctgtcca gagaggacac accctcacgc 300

tgcacgcctt ccagatgtgt cttgcacagc catggctcca gcttcaccag gatcaggctc 360

ttctccttgg gcctccctgc tgacaagtct tgcccaaaac ccaggtagat ggtgtaatgt 420

ggtgatcctt gtcgcctccg ggcccggaac tcctccagtt ctcggaagaa atgctgaagt 480

caaagatggg agtatggca 499

<210> 646

<211> 452

<212> DNA

<213> Rattus sp.

<400> 646
aaaacatgat tacaagaaaa gaatacttca tttacgtgta acactgtctt catggacgta 60

ccgtggtcac ggagtagaaa tacaacatga gtatacagga aaggagagga gagaccggtg 120

tttggccctg gtggccccct cacagagaca cagaaagcag cagacacctc tgtgcattca 180

atgggacagt ggacacttgt gtttggagca gccagagagt cagggaggat gggcaaagcc 240

acctctggga ccctaatttc gtgtggtaac cccaaagaga tactggtctg cactggtgga 300

ggctggctcc tgtcaggttc ttcgcagcct tcagggacaa gctgaggctc ctgagtgccc 360

agtggtttgt cgcatagctt gctgggccca tgggctccat ggtgtgaaga gaaaaaaggc 420

caaagtgggc gactggctga tctctaggtg ac 452

<210> 647

<211> 605

<212> DNA

<213> Rattus sp.

<400> 647
cctataataa ctgtgatgat ttattccatc atagagatta agatcacatg tatgttacat 60

acaatacaga ataatgtgta tgatgactat ataatacccc tgtacatata tttctgtatc 120

tgtacataca accagcaaag agaaaatcta catctgtgac ctaagacaca gaattcacac 180

cctgcttctc cagccaggct aacagtgaga tcacagtcag tttcctgagt gctggggcca 240

ggttagagtc cctgtaacca acacatacaa ccttagaaga gctttaagaa aacacgcttg 300

ctttctcaca gtcaacctac tggagcggga tctgtgctat aaacgtgacc tcaagaatta 360

tttctgaata cccatgtaca tcataaggat gggaaacaaa gcctctgatt tcattgcaga 420

cctttcctgt gagtccatgg aaccacgtta acaaaagaac gagcaggcag aaggggagtc 480

ttagcagaac ttggttcacc cccaatccca ctgccgtgag acttctcagt tcaacctatc 540

cttacccaca ccatataaag taaacccacc ttttacattt aagtgatgct ttttcataaa 600

gtacc 605

<210> 648

<211> 612

<212> DNA

<213> Rattus norvegicus (Norway rat)

<400> 648
tttttttttt tttttttaaa tgtcatcatc atagtctttt aatattttac ataaaaaact 60

gtatacacag cttatagaac ttttatgtaa acatcataag ctcaccactt tgtcatctgt 120

cagtttattt aaaaaaaaat aaaacaaaaa caaaacaaga caatttagta gaagtaccac 180

tgggctggga aaggagggaa taaggaagag gaagctttgc tctgcacaga gaatgtacag 240

aaaatttcac atagatgaag agacttgtct tgtggggaga gggagggagg tttaaataga 300

ccccaatacg tgttgccttc ctttaccaaa actgtgtgta tgctacacaa atttttaaaa 360

agtcacatgg ttgacatttt tttcaagaaa acaaggcaaa aattagaatc ttttttgtaa 420

tctccttaaa gtcatcatag tttgtttaaa aaaaaaaaca tcagaagtag atgaggttac 480

agcatacaag tgtggtcaaa atgctgcctt atgcaaatga caagtgcact gggtgtcaaa 540

caatgggaca attgtgcaaa gaattctttc atcaacaaca aaaaaagctt cagcctttaa 600

ataaattcag ca 612

<210> 649

<211> 549

<212> DNA

<213> Rattus sp.

<400> 649
agaaaagaat attttattga aacagtttct caattaacaa tggaacaaag tacaatttga 60

ttcaaacctg tccaaccagc ctgaactgct aatgaaagaa ctcaaacaca caggggggaa 120

ctgtgtagga cctttaagtc tctctgccaa tgtggcaaaa aaaaaaaaaa aaaaggtgga 180

gaggggtggg ggtggggtag aaaagacaaa acaactgaca tcaggtttgc tttgcccctg 240

cactggggtg gccctacctc ctgctacagg tgcaatactg gaggacaggc actctaggca 300

tggttatagt gggcaagggg ctctccttcc tggcaaagga cactgtcaag ttgaaccctg 360

acccctcttt ctttacccac aaagcttgcc tgagagtgag gtggcatttt tacattcaca 420

ccatgatctc tgtcccacag ggtcttggga aaggggctca cagtagatag cacatttttg 480

gtcagcccag ggggaagaag gaaagagcct gaagggtaga ttttacagga actggggatg 540

accaggatg 549

<210> 650

<211> 478

<212> DNA

<213> Rattus norvegicus (Norway rat)

<400> 650
tttttttttt tttttttgaa tgtgttcatt cttttattgt ccattgtgtt aacctgactg 60

acacaagacc aacaagagca ctgtactcct ggcaatcatt acatatgcta gaacatggat 120

ttgcactgta gacaacattt aacaccagtc tatggggtac tgcattgctt tttataaagt 180

tcaaaataaa gattttattt tcaaacaagt gactttggtt tatttcatac attacacttt 240

ttcttgcaga aaaaaataaa attgtacaac tgcataaata taaaattctt ccaccatgaa 300

aatggttaaa acattcataa agacacagca ccagcacatg atgcctccag agaaaggaaa 360

gaaggaaaag gaaatgtact aagcaaatta actagcccca tcactagcca aagtgacggg 420

cagatccaga tcagacacag cttcggagca cactggagga caaggggaga gatggcga 478

<210> 651

<211> 440

<212> DNA

<213> Rattus sp.

<400> 651
acagtcatga cattatttat ttacaagcac agatgaatcc ctgacctctc ccaagacagt 60

gaccctacct cagcccagtt caatacttca actggaagta aagccggaag gaggaattca 120

gggatgcaca gggttagggg aacatatccc atattaaata gttatatata tacacaacag 180

ttcctgtagt tctgtacaga gcagtggctg accccacccc acgggacccg aaatgtgggg 240

agaagagact gagggtgttg aggccggagc cagcccctgg tgaagtgcaa tagcagcagc 300

aaggtcctaa cggtacacaa gagggagggg aacaccccct cccactttcc acccccaccc 360

tgccctggga atgtactgag ggacaggaat ggttctcaaa gagcacatac aggaaggaca 420

tggctagaac catctttagg 440

<210> 652

<211>  2462

<212>  DNA

<213>  Rattus sp.

<400>  652
tgtgctgttg gaaccatggc gcctgtctgc ctgtttgcgc cgctgctgct gttgctcctc      60

ggaggtttcc ccgtcgcccc aggcgagtcg attcgagaga ctgaggtcat agacccccag     120

gacctcctgg aaggcagata cttctctgga gccctcccgg acgatgaaga cgctgggggc     180

cttgagcagg actctgactt tgagctgtcg ggttccggag atctagatga cacggaggag     240

cccaggacct tccctgaggt gatttcaccc ttggtgccac tagataacca catccccgag     300

aatgcccagc ctggcatccg tgtcccctca gagcccaagg aactggaaga gaatgaggtc     360

attcccaaaa gggtcccctc cgacgtgggg gatgacgatg tgtccaacaa agtgtccatg     420

tccagcactt cccagggcag caacattttt gaaagaactg aggtcttggc agctctgatt     480

gtgggcggcg tagtgggcat cctcttcgcc gttttcctga tcctgctgct ggtgtaccgc     540

atgaagaaga aggatgaagg cagttacgac ttgggcaaga aacccatcta caaaaaagcc     600

cccaccaacg agttctacgc atgaagcttc ttcccatgag tgctgcttgg acttcatggg     660

gagaggagtt gaggattgtg gacagtggac attggcagag agagggcacc ttaatactga     720

cttgtatctc catctctggt cacctttctg gtgtcagaag agatatgatc ttctactgtg     780

ctgcctcaga gagagagaga gagagagaga gagagatggg atggggtgcg gagggagtgt     840

ccgtgtgtgt gtgtgtgtgt gtgtgtgtgt gtgtgtgtgt gtgtctgtct gtctgtctga     900

gttgccctgg cagaaaaatg gggttaaact tgttctttct tgaaggcaag cctggaattg     960

ggtctttttg ttgttgtttc aaatttctag aatagaatgt aggaccagtt tagttcctgc    1020

cgttaacatg tctcatttat gactgccttt attctagagg caaggagttg ggggcaagga    1080

gctggaaccc gctgcacctt gagatgtgtt cacccgagta cttcctcaca ctacagggtc    1140

tctgtggtgt atctcggggc attctaggct cagtgacttt tgaaattcaa cctttttttt    1200

ttttttttaa atccagggag ggtgggactg aagtgctgac agctcatgct gaagtacact    1260

tgtagaagat ttgtaaaatg taaggttttt tttttttttt tttaatggtc cattccttca    1320

tgggagcgtg tgccctgggc tgagagcgtg gggatgcaca gatgttcttt ctagaacata    1380

ttcgttgcaa cagctaactt tgtgttttca tggtttttta tgttttgttt tgtttttttg    1440

aaaatgagag aagagctgga gagatgattt ttatgatttt tttttgtttt gttttttact    1500

atttatagct tcagacgggg ctgcttttct ctacctttct gtctttactg tttcccacta    1560

tttttttttt ttaatgttct gtgctcttgt ttttgaccct ggccctttct gaagttgctt    1620

tatcttaaaa agtagctaca gtgttctagc agattccaga atataatgta gggggtagcg    1680

ggatatttgt gttcttgtaa tatatattat ccttcctcgg ttctaggaga atagataaat    1740

atattttttt aggatataga atgatactac aggtctcatg ttggctgggt ggctgcgtga    1800

gtgagttttc gtgcggctga gtaagctgtt gccctcttct cttgccctgc tcctggtgcc 1860

ttctcgagat cgagctggag tgactgaggg tacctgactc taacctcact gtgccttctg 1920

ccgggggctc tgcccaggag cctctgggtt tgctttctcc aggctctcta gatgcacgat 1980

ccaatacagt gacctcctgt ggctgtatca atcagttcac ttgactatgt gattggaaat 2040

cattcctctg ttgcactggc cacacaattt aagtgcctag tcaccatcca ccgagcacag 2100

agattgaggc tggtttagca ggttacggtt cagttttgct tgtctccccg ggcaagagaa 2160

ggggacttag gaggaaggtg atgaggtccc agggactcct gtcacccagg actctccctc 2220

ttacagagga aagacctagt agcttaaaag gtctgggctg tgtgggggtg gggcggtcat 2280

accactccct caaccatgcc ctatgcctgt aagcccccat caccacctcc gtgcagggtc 2340

ctagctggct gggtcctctt ctagccttgt gcctgctcct tttctgtatc ccttactctg 2400

ttgtctgtta ctgatttttt tgataaaaag ataataaaac ctggtacttt ctaaaaaaaa 2460

aa 2462

<210> 653

<211> 4056

<212> DNA

<213> Rattus norvegicus (Norway rat)

<220>

<221> misc_feature

<222> (913)..(913)

<223> n

<220>

<221> misc_feature

<222> (1102)..(1102)

<223> n

<400> 653
ggatcccccc tgcaacaatc agagatgctc agtgtgtcgt ctttgagcaa ggagtacctt 60

cccagaaaca tttattttct taattcacgc tgagcctttt gctttaaaga cgctgttctc 120

aaacgttcta aaactcagta ggaacaagca gatccgaaca aaggcaaata aatcgaatcc 180

tgcaaagtaa caaagcagac gccttttccc aacagccagc acaaagctaa atgaaaaata 240

tcagtcatcc actagataca gtatttcccc cactagacag gagctggggc ctgtagatcc 300

cccctccccc aaacaggagg ccgagaaggt acaaaacaaa gtattcattc ctaaacgagt 360

catcacttcg agctgcacct tctacctctc cgcactcgcg gtttgaaaga cctcggctgt 420

tacttctctg tacctgcctc caaacacacg tggaggtggt ggcgagaggg agggagccca 480

ccgccacgta tgcataaggc gagggaggag gggattgggg ctgagggcag ttccctgagg 540

acagttagtg ctgcctaaag tgcaaggctc ccccattcct ctgtaccccg aaactggagg 600

aaacagtgtg ggggcctggg gacgcctgtc cacggtccag gcagagccct ggaaccctga 660

gctttgatgc tgcagcagcg atcgcctgtg ggcacctggg tttctggggg gcggggcctc 720

ccccaggtgt gcggagagcg aaggccacca gagccagccc agaggacccc aggagcctcc 780

tccgggggac acggtacaaa agagcggctc gtcggtgccc ccagcagcag gcaccgggcc 840

ccacgggcct ggctccggga aggggcgccc gcgcggcggc accgaggggg ttaaccgggc 900

ggcccgcggc cgnagcgcgc ttaaccctcg gtcagccgcg ccagcccggg ggtggcggcg 960

tccgagtcgc gcgccgtgga gcccgggggg cgcgtgggcc gcaggccggc agctggggcg 1020

cggcgcggcc ggtgggaggc ccgggcgcgc gggggagggc gggggacggc ggcggcgggc 1080

gcgcagcgtc gccaggacga anccggcgtg cagcgcggcg cggcgggcgc ggagcgggcg 1140

ggcgggcggg cgagcgcctc cgtccccgga tgtgagctcc ggctgcccgc ggtcccgagc 1200

cagcggcggc gcgggcggcg acggcggcgg cggggcaccg ggcaccgggg cgggcgggcg 1260

gacgggcggc atggggggcg cgtcgagcgg ccccggcggc cgggcgggca ccaccgcggt 1320

gtccctccgc tagaggaggg gaccaagcca attctccttt gcagcaaaaa aagagaaaaa 1380

agaaaaaaaa gaaaaattta cttgtatata ttactaagat aatatataca tctgatttta 1440

ttttttacaa aaagtttatt ttgctccatt tttcaaaaaa agaaggagcg tgggtggcag 1500

gcggttttta taaattattc ttattttgat tatttgtctc tgtccttccc caccccctgc 1560

tgaagcgaaa atagggcagg gaccgcggct cctacctatc agtaacctcc catacccgtt 1620

cctccccctc cctaacgctg agcatagtgc cctgcacaca gtaggcgctc aataaatgtt 1680

cgtggatgat gatgatgatg atgatgaaaa aaatgcagca tcaacggcag cagcaagagg 1740

accacgcgaa cggtgagcag ccagagcccg ggtacccgct gccaaacctc atcctatcag 1800

gatcctactc ttcccactcc ccaaccccca tctccgatgc gttgggtggg ttgagggggt 1860

tggagatgat gcttttggct tggtgtcatc tacatagaaa ggggtgatgc gcggcagcta 1920

cttcgttctc ctcttcaggc cctgaatgtc agagttaatt ctgcatttgc tggggtgggg 1980

cggccattgg gtgtgtggag tactgtgcag ccaaggggcg agagtgaggt gattcgggtt 2040

caagtgccct cgggctcaac ttttctcttt cattgcccct gagcgatgga agaggtgtta 2100

gtcttcagcc tgaacccctt ttccagcctc cccccccacc cccacgcctc tggttgacta 2160

agaggaaggt ctttagtagc atcaggaaaa ttctaggatt gctccgcttt tactcgttgg 2220

gtcacttggt aaagtgcaca tccatcatct catgggagaa gccccccggt cttgggatct 2280

gggggaacag ggtcaggtgg agcgagtatt cccgctcgct ccctgtgcct cctgtgcaca 2340

cgtcgtcggt aacctagcaa tgcccagaga gttgctgccg ctgtggggct ctgcaactca 2400

gccctggcca tggcaccacg tgcagtttcc acaccagcgg ggctgggtct gagaaggttg 2460

ggttgctttt ggctaggttc cctccgtgga ggtcgttggc atctcccaac cctgttttct 2520

tgaagccttt tccggtgagc acttctcaaa gggaaagctt ggcaaaatta gttcctgatc 2580

tcagattgag tgccacctgc ctgctttgct gctgtcccag gctcatagga cagtggggca 2640

ggagacctgt gcttgctttg actctttgct tagtgataat tagctttgca aacgcctgga 2700

tctccaacct ctctccaccc ttctcacttt gacatctgct tcagcagagg tgatgatggt 2760

ggcccaacgt gggcacctgt gagaccgtct gagggatggc cagacccata gtgaggcggg 2820

ctccgcacat gaagagaccc gacccccccc cccccccccg cgccccacct ttgagtattt 2880

tgccttgtgt gtacctggat gctactctac gccgagataa ttcttgaata ggccttgagg 2940

ttccctggca taacgcaccc tgtctcttag gtgtaggggt gcaggctggc ttggctgcta 3000

ctgctgcaat taaaaaaaaa aaaaaagccc taaagatgtt tttctgtttt cggaagcttg 3060

tggaggaaat gagtcccaag caccatgaat tttcatttgt aggaagtgtt agtgcataat 3120

ctagatattt gttatggggc ttttgctatt gcctggtgcc agtgaggagt ttgttttttt 3180

tttcttctac gtagaaaaaa tgcatttatg gctgtggaga gctgttggaa ggacacccta 3240

aaaatggtta agactgttga atttattggc tctgtgtgtg tgtgtgtgtg tgtgtgtgtg 3300

tgtgtgttgg taataaattt tagagaaagg cagctgtata gatgtatctg ttaagccatg 3360

agagatacgt gtgttttcaa agtcaggaaa agacacagga taaattcgag aatagcccca 3420

gtttgtcaga agagaggcac tcatggcttc cctttgaaga cacaggtatg ggagagtacc 3480

agtgtggcta ggtgtggtgc agcaacgcat gtctagtgct cagggcagag ctctgctgac 3540

ttctgcattg cctggccaaa ccccactact tgtgacacaa ggttcaaaat gggaaacaca 3600

agatgggaga cacttttgct tttttaaaaa gatagtatag atcaaggtct ttcttttttt 3660

tttttttctt ccccaaagaa aaataatctc ttttctcctt ctcctcagag gcaaactatg 3720

caagaggcac cagactccct atttctggtg aaggaccaac ttctcagccg aacagctcca 3780

agcaaaccgt cctgtcttgg caagctgcaa ttgatgctgc tagacaggcc aaggccgccc 3840

agaccatgag cacgtctgca cccccacctg taggatctct ctcccaaaga aaacgtcagc 3900

aatacgccaa gagcaaaaaa cagggtaact cgtctaacag ccgacctgcc cgtgccctct 3960

tctgtttatc cctcaataac cccatccgaa gagcctgcat tagtatagtg gattggaagt 4020

atcctttttc tgtgggaaag tctttcaaaa ggatcc 4056

<210> 654

<211> 2104

<212> DNA

<213> Rattus norvegicus (Norway rat)

<400> 654
gcacgagacg gggttggatc tgaggtgcag cctcagaggg attaggagct gctagacttt 60
tttctttttt cctttttctc ctctcagtgg cacggagtcc gaattaattg gatttcattc 120
actgggtagg aacaaaactg ggcactttca ttcagagaga ttcattgact ctgagagtga 180
tctggtgcag agggaccacc ggcttggctt ctgtcgcctt ccctaaccgc tagcttcggc 240
ttgggaaagg ccgaatcaaa cccggctccg agagccggag cttctcacgg cttgcttggc 300
ctatgcctgc ctcgaggggc gtctgctagg caccccgcct tctcctgcag ctcgaccccc 360
atgatagata cgctcagacc cgtgcccttc gcgtcggaaa tggcgatcag caagacggtg 420
gcgtggctca acgagcagct ggagctgggc aacgaacagc tgctgctgat ggactgccga 480
ccgcaggagc tgtacgagtc gtcgcacatc gaatctgcca tcaacgtggc catcccgggc 540
atcatgctgc ggcgtctgca gaagggcaac ctgccggtgc gcgcgctatt cacgcgctgc 600
gaggaccggg accgctttac caggcgctgc ggcaccgaca ccgtggtgct ctacgacgag 660
aacagcagcg actggaatga gaacacaggt ggagagtcgg tcctcgggct gctgctcaag 720
aaactcaaag acgagggctg ccgggcgttc taccttgaag gtggcttcag taagttccag 780
gccgagttcg ccctgcactg cgagaccaat ctagacggct cgtgcagcag cagctccccg 840
cccttgccag tgctgggact cgggggcctg aggatcagct ccgactcttc ctcggacatt 900
gagtctgacc ttgaccgaga ccccaatagt gcaacggact ccgatggcag cccgctgtcc 960
aacagccagc cttccttccc ggtggagatt ttgcccttcc tttacctggg ctgtgccaag 1020
gactctacta acttggacgt gttggaagag tttggcatca agtacatctt gaacgtcacc 1080
cccaatctgc ccaatctgtt tgagaatgca ggggagttca agtacaagca aattcctatc 1140
tctgatcact ggagccaaaa cctgtcccag tttttccctg aggccatttc tttcatagat 1200
gaagcccgag gcaaaaactg tggtgtcctg gtgcattgct tggcgggcat cagccgctcc 1260
gtcacggtga cagtggctta ccttatgcag aagctcaact tgtccatgaa cgatgcttat 1320
gacattgtca aaatgaagaa gtccaacatc tctcccaact tcaacttcat gggccagctg 1380
cttgactttg aaaggaccct gggactcagc agcccctgtg acaatcgtgt ccccgcacag 1440
cagctctact tcaccgcgcc ctccaaccag aatgtctacc aagtggactc cctgcaatct 1500
acgtgaaagg cacccacctt tcctagccgg gagtgtctca ttccttcagt ttctcttggg 1560
cagcatcgac caggctgctt tctttgtgtg tggccccagg tgtcaaaaat gtcaccagct 1620
gtctgtatta gacaaggttg ccaagtgcaa aattggttat tacggaggga gagatttgct 1680
ccattcattg ttttttggaa ggacagggta tgctgtctag atccaggcaa taggtttgct 1740
tttgtacccc agcctaccca agcagggact ggacctccat ccagatagag ggtaggacaa 1800
aggagccggg gataggagca tgtgttcctt agggccacat atggctgttt cctgttgcat 1860
ctggaaccaa ctatattgtc ttcagtgaag actgattcaa ctttgcgtat agtggagcca 1920
aagagatttt agctctgtat ttgtggtatc ggttttgaaa acaaaagaac tgatccttta 1980
atttgattat tgtaaatatt tgatcttcac ttgagagtgt ttgtttggct tgtgtttggt 2040

ttttaatctt tgggtttaaa cgagatccaa agggagaaag agcagtatgc ctcgtgccga 2100

attc 2104

<210> 655

<211> 2971

<212> DNA

<213> Rattus rattus (black rat)

<400> 655
cggcggccga gtgcgaggca gagagcgaga tctgtacggt gtgcgagcga gggcgatcac 60

agcctgcgaa caagcaaaga aagagagcga gtgagcggcg aggcgcctgc gcgatgctcg 120

ggcccctgag cccgcggccc tgagctagcc gggacggaca tgcgcgggag ggcgccgcgg 180

ggccccgctc ccctggggga atgaaagcta ctggttgact taagagcacc tggccttcac 240

agattcgtgg ccatcccagg gtgggcacaa tgtcaacagc aggagttgct gctcaggata 300

ttcgagtccc attaaaaact ggatttctcc ataatggcca ggctttgggg aatatgaaga 360

cctgctgggg cagtcgcaat gagtttgaaa agaatttttt aaatatcgat ccaataacca 420

tggcctacaa tctgaactcc cctgctccgg agcatctaac aaccctcgga tgtgcttctc 480

cgtccgctcc agggagcggt cacttctttg cagagcgtgg tccatctcca aagtcaagct 540

tgccccctct tgttatccca ccaagtgaaa gctcgggaca gcgtgaagag gatcaagttc 600

tgtgtggttt taagaaactc tcagtgaatg gggtgtgcgc ttccacacct ccacttacac 660

ccattcaaag ctgctcttcc ccgtttccct gtgcagctcc ctgtgatcgc agttcccgac 720

cgctcccacc attgcccatc tctgaggacc catctctgga tgaggccgac tgtgaggtgg 780

aatttctaac cagtgcagat acagacttcc ttttagaaga ctgcgtgcct tccgatttca 840

aatacgatgt tcctggcagg cgaagcttcc gtgggtgcgg acagatcaat tatgcgtatt 900

ttgacagccc aactgtttct gtggcagatc ttagctgtgc atctgaccag aacagagttg 960

ttccagatcc aaaccctccc ccgcctcaaa gccatcgcag attaaggagg tctcactcag 1020

gaccagccgg gtcgtttaac aagccagcca tccggatatc tagctgcaca cacagagcct 1080

ctcctagctc ggatgaagac aagcctgaga tcccgccccg ggttcctata cctcccaggc 1140

cagccaagcc agactacaga aggtggtcag cagaagtgac ctctaacaca tacagtgatg 1200

aagataggcc tcccaaagtc cccccgagag aacctttgtc tcggagtaac tcccgtaccc 1260

caagtcctaa aagccttccg tcttacctca atggggtcat gcctccaaca cagagctttg 1320

cccccgaccc caagtatgtc agcagcaaag ccctgcagag acagagcagc gaagggtctg 1380

ccaaggcccc ctgcatcctg cccatcattg agaatggaaa gaaggtcagc tccacgcatt 1440

attacctact acctgagaga ccgccatacc ttgacaaata tgagaagtat tttagggaag 1500

cagaagaagc aaacccaagc acccagattc agccattacc tgctgcctgt ggtatggtct 1560

ctgccacaga caagctggcc tccagaatga aaatggatgt gggcggccac gggaagcgca 1620

aacacttatc ctacgtggtt tctccgtaga tatggggtca tggttcaaca gaagttacat 1680

ggaacggatg gctgccaagt ttccaatttg aggttcatag aacagtgtca agtggcaaca 1740

tgaagtggtg gactctgcct tggtgaggaa ggcatagagc cgttatgagg tgctgttgtg 1800

ctgggagtcc ctgacctatc agcataggaa gaaaaaagta tgatttaaag atgtgctaga 1860

gggacacttt tacatttgac tacattatat acctatgtat aaacgtgcgg tgtaaccata 1920

gaccatagct gcaggataac caattagtca ctctagagta atctatattc agaacaattc 1980

aaacgagctg gaggcacagc tccagacagt gtgaaaattg agcaaacggg agagagcagt 2040

actgttgatc agttataaat gtagagattc ctggcattca ggcctgctat ctagtttgtt 2100

ttcttcccct tccctgccag cagtcttctc catacacgac agggcgtgct cttcgccagg 2160

cctgtaacgt cttgttgaaa tcgttctatg gcctaatact tgccgctctg ggcgtttgtc 2220

ttgagaggag aggacagccg tttctggacc atgttatcta tcacctgtgt atgtctctct 2280

tggaaatgga cagaattggt gacttttcca tgctattcct gcttttcccg tccactgaag 2340

aggctttcaa gagtgcagtt gagtggtgct ggccgtcagt gttgggtata agttttatag 2400

accagcccag tgattagcca tgattgagag ttatcgtggg gtgtatggga tgttgttttg 2460

tttttgagac tttaaagtac aacacaagct ggtcttgtgt tgttggttcc tattcagtat 2520

ttcctgggga ttgtttgctt tttaagtgaa acacttctga ccaatagcac agaacgtctt 2580

aatgccagag gtcacttcag catcttcctg cttagaaaac tcacagctgg ctgcctcact 2640

gccctgagag tcagtgagac gcatagcttg tgttcaattt ttacatcctc tgattgttta 2700

tcttgtatag ataagcacaa agagaaggtg cttgctaaca gagggacacc gctgccatgt 2760

cccaacaagc tgttcagttt aaactgctga atgacattat ttgagctatt taaagcttat 2820

ttttagtatg aactaaatga aggttaaaac atgctttaga aaatgcactg atctccgcac 2880

tgtgtgtaca gtattggaca aaggatttgt tcattttgtt gcattatttt gaatattgtc 2940

ttttcatttt aataaagtta tattacttat t 2971

<210> 656

<211> 1508

<212> DNA

<213> Rattus rattus (black rat)

<400> 656
cccaactgcc agcagcctgc accctgcacc cgctcagctc cgcacagcca tgtttcagcc 60

agctgggcac ggccaggact gggccatgga gggcccgcgg gacggcctca agaaggagcg 120

gttggtggac gaccgccacg acagcggctt ggactccatg aaggacgagg attacgagca 180

gatggtgaag gagctgcggg agatccgcct gcagccgcag gaggcgcctc tggctgccga 240

gccctggaag caacagctca cggaggacgg agactcgttc ctgcacttgg caatcatcca 300

tgaagagaag acactgacca tggaagtgat tggtcaggtg aagggagacc tggccttcct 360

caacttccag aacaacctgc agcagactcc actccacttg gctgtgatca ccaaccagcc 420

aggaattgct gaggcacttc tgaaagctgg ctgtgatcct gagctccgag actttcgagg 480

aaatacccct ctccatcttg cctgtgagca gggctgcctg gccagtgtag cagtcttgac 540

tcagacctgt acgccccagc atctccactc cgtcctgcag gccaccaact acaacggcca 600

cacgtgtcta cacttagcct ctatccatgg ctacctgggc atcgtggagc acttggtgac 660

tttgggtgct gatgtcaacg ctcaggagcc ctgtaatggc cggacagccc tccaccttgc 720

agtggacctg cagaatcctg acctggtctc gctcctgttg aagtgtgggg ctgatgtcaa 780

cagagtaacc taccagggct actcccccta ccagcttacg tggggccgcc caagtacccg 840

gatacagcag cagctgggcc agctgaccct ggaaaatctt cagacgctgc ccgagagtga 900

ggatgaggag agctatgaca cggagtcaga attcacggaa gatgagttgc cctacgatga 960

ctgtgtgttt ggaggccagc gtctgacgtt ataagaggaa agtggccaaa ggaacgtgga 1020

cttgtatatt tgtacaaata gagttttatt tttctaaaaa aaaaaaaaaa aaagaaaaag 1080

aaaaaaaata agaaaggata cttagcacca cagcacacag cgcctagccc cgagcattct 1140

attgtggtga ttcagctgtt gttcttctca aatttgggga aacagatcat tgaaattcaa 1200

aggaaatgtc tcctaaggtt catgtttgtg ggattttttt tttttttttg aggtgatttt 1260

ccagaatctg ataaaaggac cacatttaat ttatgtgctt ttggttgaac cgccatagac 1320

gtagctgacc ccagttgccc tctacgtaag aaccaggtcg atagtggtat gtgcttaagc 1380

accccagctg agatttgtac cactttcttg taaatgttgt atatggatgt attttgttgg 1440

taattatttt tggtacttct aagatgtata tttattaaac agatttttac agtatcaaaa 1500

aaaaaaaa 1508

<210> 657

<211> 1758

<212> DNA

<213> Rattus norvegicus (Norway rat)

<400> 657
gaatagcgtc tcgggagtgt ccctgggtaa tcgctgcatc tcgttttgtg tgtgtgctct 60

gtcgaaacgt ctgtgatgtg tgtatggttt tcacgtgaac ggaggcaggc agagttgggt 120

ctgtgggttc tagtgtgttc ttaatctttt tggaagtttg tgcagtgact tcccctctgg 180

gattgtttcc tatgcatacc ttcaagtatg tgtgaatgtg ttttctgtgg tcccttgatc 240

ttactgggct tctctaagga gggtggctgg accatctgct acgtgtctgg gagcccctct 300

ctagctacca catgtcccga catgtgctta ctcccaatat ttgcacccac tcgcattcac 360

aactcccagg caaaaagaaa aaatcaaaaa aacaaaaaaa acgtgcactg gggcacctag 420

aaaatgggca gccaaggctc cgcctacatt ttgcttcctc cttggaattt ccagccctgt 480

gcagccaact gctccctagc tgcgaagggc ggagtttccc ggctctataa gtagtggtgc 540

cttaccgggg atccgcactc ctccgtaccc tctgtacagc gcgcgaatta tgtgccattc 600

gcgtaaccac ctccacacca tgactggcct gagggctccg gcgccagttc cctccaccgg 660

cccggaactc cggcggggct ccggtcccgg aattttcacc ttcgaccccc tcccggagcg 720

ggcggtagcg tccgccgtgc gtccgaacac ttctcgcggg cacggaaaac gcagccgacg 780

ggtgctctac cctcgagtgg tgagtatcgc ccgagtgggc agcaagaggt cgagtcgccc 840

cggaaccttt gggaacaact agatgcgggg actttcgtct gacgtttccc tcttttctct 900

gctcaggtcc ggcgccagct accaaccgag gaacccaaca ttgccaagag gatcctcttt 960

ctcctgctcg ccatcatctt ctgccagatt ttgatggctg aagaaggtgt gtcgcaggcc 1020

ctggctccgg aggatgctac cagcgccata tcacctgagc ccatctctgc gcccattact 1080

gcgcctcctg tcctcgagcc tttgaacctg acctcggagt cctcggacta tgctctggat 1140

ctcaaagctt ttctccagca acacccggcg gcattctaaa cgcgatgggt cacagtccgg 1200

aaaaaaaaac ccaaagagca ccaggggtac ctggtcggca agaacgtatc ccaaactggg 1260

acttataagg caacgctaac tcagaacact accgccaaga tgccggttct tggggcgagc 1320

ccgaggcacc gtgggtcctg gctaggctca ctggtgacgg acggaggtct tctccgtgtc 1380

taattaatat ttatgtattt atgtatatcc tcctaggtga aggaggggtg tatgtaatat 1440

ttattctaac ttatgcaggg gtgcgagata tgcccccttg ctgtgacaca gatatttatt 1500

acgatttata gggtccgtga gacagagttg tgggaggatt agtttagtct tggtcggggg 1560

gcataggttt aggggtaaca ctccattttc cagcacttcg actctgttgt aagactttgg 1620

cagacccttg ggaatacggt ctgtgtgcct ttgatgtctt cctgactgga agtcttgggt 1680

ccatggattg tcagagggcg gctgtctggg gtcgcctagt atgttctgtg aacacgaata 1740

aacttgattt gcctgtcg 1758

<210> 658

<211> 528

<212> DNA

<213> Rattus norvegicus (Norway rat)

<400> 658
gttcttaaac tttgctctcc aacctccaac atgaaggcgc tgagcccggt gcgcggctgc 60

tacgaggcgg tgtgctgcct gtcggaacgt agcctagcca ttgcgcgagg ccggggtaag 120

agcccgtcgg cggaggagcc gcttagcctc ttggacgaca tgaaccactg ctactcgcgc 180

ctgcgggaac tggtgccggg agtcccgcgg ggcactcagc ttagccaggt ggaaatcctg 240

cagcgtgtca tagactacat cctcgacctt caagtggttc tggcagagcc cgcgcctgga 300

cccccggatg gtccgcatct cccgatccag gtacgagagg gagctagacc aggctcatct 360

gaacgtgcgg gctgggatgc tgcgggtctt ccccaccgcg tcctcgagta ccttggctaa 420

ctcgtctctt atccttcttt cacagacagc tgagctcact ccggaacttg tgatctccaa 480

ggacaagagg agtttttgcc actgacccgg tcgtcctggc acctcccg 528

<210> 659

<211> 918

<212> DNA

<213> Rattus norvegicus (Norway rat)

<400> 659
gcgatctgtg ggtgacagtg tctgcgagag actttgccat accattttgc gggaatttgg 60

agagaaacaa ccagctgctt ccagtcccct ccccctccgc caccatttcg gacaccccgc 120

acagactcgt tttggggtac cctgtgactt ccaggaagca cgcgaggtcc tttggcccca 180

gctcgggcga ccacctgtct tggacgtgtt gactcatctc ccatgaccct gcggtgcctg 240

gagccctctg ggaatggcgc ggacaggaca cggagccagt gggggacagc ggggtcgccg 300

gaggatcagt ccccggaggc ggcgcgtctg gcgaaggccc tgcgtgagct cagtcaaaca 360

ggatggtact ggggaagtat gactgttaat gaagccaaag agaaattaaa agaggcgcca 420

gaaggaactt tcttgattag agatagctcc cactcagact acctattaac catatctgtt 480

aagacatcag ccgggccgac taacctgcgg attgagtacc aagacgggaa attcagattg 540

gattctatca tatgtgtcaa gtccaagctt aagcagtttg acagcgtggt tcatctgatt 600

gactactacg ttcagatgtg caaggacaaa cggacaggcc ctgaagcccc ccggaatggg 660

actgttcacc tttacctgac caaacctctg tatacatcag caccgacgct gcagcacttc 720

tgccgactca gcattaacaa gtgtaccggt accatccggg gactaccttt accaacaaga 780

ctgaaagatt acttggaaga gtataaattc caggtataag tatttctctc tctctctctc 840

tttttttttt tttttaaatg aatgcctcat atagagtatc accgaatgca gctatgtgaa 900

agagaaccag aggccctc 918

<210> 660

<211> 1992

<212> DNA

<213> Rattus norvegicus (Norway rat)

<400> 660
aaagtctcca gggcacagag ggagtcaact cattggcgct tgagtcggca aagaaatcaa 60

gatggccaaa gttcctgact tgtttgaaga cctaaagaac tgctatagtg aaaatgaaga 120

atacagttct gccattgacc atctgtctct gaatcagaaa tccttctatg atgcaagcta 180

tggctcactt catgagaact gcacagataa atttgtatct ctgagaacct ctgaaacatc 240

aaagatgtcc accttcacct tcaaggagag ccgggtggtg gtgtcagcaa catcaaacaa 300

agggaagatt ctgaagaaga gacggctaag tttcaatcag ccctttactg aagatgacct 360

ggaggccata gcccatgatt tagaagagac catccaaccc agatcagcac ctcacagctt 420

ccagaataat ttgagataca aattgataag gatcgtcaag caggagttca tcatgaatga 480

ttccctcaac caaaatatat atgtggatat ggacagaata catctcaaag ctgcttcgtt 540

aaatgacctg cagcttgaag taaaatttga catgtatgcc tactcatcgg gaggagacga 600

ctctaaatat cctgtgactc tcaaagtctc aaatactcag ctctttgtga gtgctcaggg 660

agaagacaag cctgtgttgc tgaaggagat tccggaaaca ccaaaactca tcacaggtag 720

tgagaccgac ctcattttct tctgggaaaa aatcaactct aagaactact tcacatccgc 780

agctttccca gagctgttaa ttgccacaaa agaacaaagt caggtgcacc tggcacgggg 840

actgccctcc atgatagatt tccagatatc ataaaaacag ccttatttag gagtctactt 900

tcttgagaag tgctgacaat ctgtatgtac catgtacagg aaatgttcct cgtcctaagt 960

cactcgcatg gcatgtgctg agtctttgta attctaaatg aatgtttacc ctctttgtaa 1020

gagaagagca aagcctagtg gaaccagccc gacatatgat actgtttgtt atttaaagag 1080

taccctatag tttgctcact actaatcatt ttaattatta ttcttcatgg tattcttagg 1140

agggtcaaaa agactctgct catattacag atgggctaac taagggataa gacaactgaa 1200

aagtacactc aattgcatct ggaatgggaa ttcacagacc catgaaactt atctcactgt 1260

gcaccttctg cttctaaagt gccggatggg taggataaag ttgtaagaac gtaatgctat 1320

cattttcaaa aggaagggga caatagctac atctttccta cctcagtggg ttttattcca 1380

gtaagagcat ttggataaaa tccttctgta acagacctca agaaggagac agactgttga 1440

atgttatttt taagttattt tatatatgta tttataaata tatttatgat aattatatta 1500

tttatggcac atccttaaat cctctgagct tgccaggcat cctcagcagc agaattttct 1560

aggtggtcag ttagatgcag tgtcctctga aacaccatgc tatagacaga cgtacactgt 1620

ttccacatcc gtggagctct ctttacattc ttgtccttag gagccctgca gtcatgatca 1680

ccaatctgta ctgttcactt cgttcattta aaatgataac tgggtcagtc ttttgccctc 1740

ctgtccttaa agctgtctgg gtcttcttac atcattcaac tcacctgtaa ttaaagctaa 1800

ccatctaaag acgggaagaa cctaactgtg acaaccacat ccctgttacc tgaagtttct 1860

tttctaggat gtaatcagtg tttcctctgg gttccaaatt tgttctcaaa ccagggtgtc 1920

acaaaaatat caacaataat aatcaactca tcattactat tgatcataat taaataaagc 1980

aagtttgagc tg 1992

<210>  661

<211>  237

<212>  DNA

<213>  Rattus norvegicus (Norway rat)

<400>  661
atctgctgct gccctggcca ccctgagggg tcaccagcac ccctgagtag gctgggtggg      60

cgtccgcctg acagtggccc accctccttt atccatgttt ataatatgca ctttttcatt     120

gtaaataaaa attaatatat atatgtttat aaagcaaaga tcttttgagc tttgttattg     180

acatggggtc tatgtgcagc ccaggctggc ttcaaactgg agatccaagc ttgggtg        237

<210>  662

<211>  2639

<212>  DNA

<213>  Rattus norvegicus (Norway rat)

<400>  662
gaatgctggc agtgtgggtg ctatgtaaaa gtatttacat agctttgtag agtgagccaa      60

gcccagtctg tttgggatga ctcttcacag tgcctcgaat ctgtcacacg tcttagtaag     120

cagagtcaca gagtttctgt cacatcatcc tcctgcctac agggaagtag gccatgtccc     180

tgccccctac tctgagccca gctgtgggag ccagccctgc ccaatgggct ctctctgatt     240

gacttctcac tcacttctaa actccagtga gcaacttctc tcggctcgtt caattggcgt     300

gaaggtctgt gtcttgcaga gaaggttctt cacaactggg ataaaggtct cgctgctcaa     360

gtgtagccca gtagaactgc caagcccctt cccctcctct ccctagactc ttggatgcaa     420

gaagaatcca ggcagctcca agggtgattg tgtccaacct agaatgtctt gaaaaagaca     480

ttaaggggac tagagaagac aggggatcca acggttctct gcagcccagc ctgactgaca     540

tgtaactctt ctggttctca ccagccagct ggacctgctt agtattcttt ctgcctcagt     600

ttcccagcct gtacccaggg ctgtcatagt tccatttcag gcagtagtaa tgaatgagct     660

gacataaaac atttagagca ggggtcagta tgtatataga gtgattattc tatatcaggc     720

attgcctcct cggaatgaag cttacaatca cccctccctc tgcagttcat cttggggtgg     780

ccagaggatc cagcagacac ctagtggggt aacacacccc agccaactcg gctgttgcag     840

actttgtcta gaagtttcac gtctcagagc tgaattccct tctcatgacc tttggccgtg     900

ggagtgacac ctcacagctg tggtgttttg acaaccagca gccactggca cacaaaatgt     960

gcagccagca gcatatgaag tccaagaggc gtcccggcca gccctgtcct tgacccccac    1020

ctgacaatta aggcaagagc ctatagtttg catcagcaac agtcacggtc aaagtttagt    1080

caatcaaacg ttgtgtaagg actcaactat ggctgacacg ggggcctgag gcctcccaac    1140

attcattaac aacagcaagt tcaatcatta tctccccaaa gtttattgtg ttaggtcagt 1200

tccaaaccgt gctgaccatg gctatgatcc aaaggccggc cccttacgtc agaggcgagc 1260

ctccaggtcc agctgagggg cagggctgtc ctcccttctg tatactattt aaagcgagga 1320

gggctagcta ccaagcacgg ttggccttcc ctctgggaac acacccttgg ccaacagggg 1380

aaatccggcg agacgctctg agatctctga tccagacctt ccaaaaggag gaagaaaggt 1440

aggcagggcc attccactct cctctccaaa agtaccactg aggggtccac tcacagtctt 1500

tgccctgaca accaaggccc tctagccatg tgcaactcat gcaaagtcta cagaaggaac 1560

gggaagggcc catgggtgcc ggtgttcaag agcctctctt tttgtttcag gtggcaccag 1620

agtccctgcc tctctccaca ccattgcaag aatgcctcct cagctgcata atggtctgga 1680

cttctctgcc aaggtcatcc agggcagcct cgacagcctg ccccaggaag tgaggaagtt 1740

tgtggaaggc aatgcccagc tgtgccagcc agagtatatt cacatctgcg acggctccga 1800

ggaggagtac gggcggctgc tggcccacat gcaggaggag ggtgtcatcc gcaagctgaa 1860

gaaatatgac aactggtgag tcccttcagc ccaaagtctc tgcctacagg ctccccagct 1920

ctaccttctc ggagtctgga gaataaagac ccttgcagta gcaaacgcct gagaagctta 1980

ggagatcagt cagaatccag acaccctgtg aggttagcat agccagcttc tagattccag 2040

atagccatgc aaggcggccc ctttccctag ggccagctca tggcttagca aatgtctacc 2100

tggtcaagtt cccgttggct tggcttccat acctaagtaa cacggtcaac gtccttgtgt 2160

ctgcaaggcc gggaagagcc tgaggtaggc aagagaggta catccccaag ggtaccttgc 2220

tgaaggcatc cctgtgactt tttcagttgg ctggctctca ctgaccccag ggatgtggcc 2280

aggatcgaaa gcaagacggt catcattacc caagagcaga gagacaccgt gcccatcccc 2340

aaaagtgggc agagccagct gggccgctgg atgtcagaag aggacttcga gaaagcattc 2400

aacgccaggt tcccggggtg catgaaaggt gggtgaggcc agaccccagg cctgacagtg 2460

tgctgtgtgc acgttcacgc tcccggtagt agcttaagct gcaaagggtg gcccgcgtgc 2520

gaagctccgc gtctggttct gtctcacgga atatcaaatt gttctctgag agtacgggtg 2580

gctccgacct gtcatctcag acatggaagc tgaggcaggg gcattcaaag tttaaggcc 2639

<210> 663

<211> 2410

<212> DNA

<213> Rattus norvegicus (Norway rat)

<400> 663
tacgctgctt tggtggcgtg cacctctcac ggtgtgccta gccgccgatg cccaggctgc 60

acgatcacgt ctggagctac ccaagcgcgg gcgctgcgag gccgtacagc ctcccgcgag 120

gcatgattgc ggcggccctc tgtccgcagg gccccggagc ccccgagccc gagcccgcgc 180

cccggggcca gcgagaggga accgcaggct tcagcgcacg acccggcagc tggcaccacg 240
acctggtgca gcggagcctc gtgctcttct catttggcgt ggtcctggct ctggtgctca 300
acctgctgca gatccagcgg aatgtcacgc tcttcccgga cgaggtgata gccaccatct 360
tctcctccgc ctggtgggtg cccccgtgct gtggcacggc agccgctgtt gtcggcttat 420
tgtatccctg tattgacagt cacctgggag aaccacacaa gttcaagaga gagtgggcca 480
gtgtcatgcg atgtatcgcg gtgtttgttg gcatcaacca tgccagtgcc aaattagatt 540
tcgccaataa tgtgcagctg tccctgactc tggcagccct atccttgggc ttgtggtgga 600
cgtttgatcg atcccgaagt ggcctggggc tcgggatcac catcgccttc ctagccacgc 660
tgatcactca gtttcttgtt tataatggcg tctaccagta cacgtcccca gatttcctct 720
atatccgttc ttggctccct tgtatatttt tctcaggagg tgtcacagtg ggaaacatag 780
gacgacagtt agctatgggt gttccagaaa agcctcacag tgactgagtt tgagcacatg 840
attcagggcg gaagcagaat gtggagacac tggtcctggg tgtggtgaag aggatctttt 900
tctcaatgtt ccatttagac tgggctgatg ataaatgact cctaaagatg cgttcacgta 960
gtctaaatag caagtggagg caaggactac ttacctaaag tcttaccttg ctcacccacc 1020
ctcacacctg tctgcactgg aacattctat cccaggctgt atgtgagagt tgggtaaggg 1080
ggccggtttc ccgagtatta gatttcactc atcattcaaa gcaaaatgcc atatttcaaa 1140
gccttgaatc aaaatgaatt accaactagc agttttatat cagtgcccaa aggagagagg 1200
ttgatggtgc ttaacagaga tgaagtatgt gcagtaagaa tatttatcca gaattaaaat 1260
atagggttgt gtaaagaggg gctaagggca gcagtaagtt ggaggaagat catgctcccc 1320
ggaggaccca gtgcagccac atctccaggt ctcgctcagg ctggcgctca cacgtgggtc 1380
tcatcagtgt gggaactatg ctgtttactg acaggaggct tgtagacaat cttactgaca 1440
gcccaggaca acacaaagtc aggattctgc attgcgatgc tggacttttc atctcaattt 1500
aagtgaagtt ttatccaaga tctggagcat ctaagagtga atagctgtct gctgtttcag 1560
tcgtaatgag ccgaaattgt gtctctgtca ctccagagtg gagaggactt ttccacagcc 1620
ctatggagct tgcaatctgt gattgccttg taaaaggttg agtgtgcacg tcactgcgtt 1680
cggtgcgcag tgtcctgtgt gtgttggaca gcgtagaaca catgggacct tgcaagtatt 1740
gggtcttcaa cttcaagtgc aatgtgtatg aaaccaatct gagccttgta ttctcttaaa 1800
tatttattat ttttttttaa ccgcgcgagc tgttctggag aagggttctc gggtcatttc 1860
agagctgtgt gaggcacact cagcaatact gtgtcagccg tgacgctccc cagtcacacc 1920
ctccactaca ccctagtcct ttgacatact ccaggtttgt aagtttagtg atttttactt 1980
acaaatttac ccttttttgc attctaaaat tgtgtttaaa ttatatggaa gtacttggtg 2040
taggcagtca ttggtccccg ggcagcagaa gctctgcctg tggaatcggg tttgggttca 2100
ctctgcaggg ctcctcatag aggctttgct tatttgtttt gaggaaaatg tctggagtaa 2160
acctttgttt tctgaaacta ctttagctaa aagaaaatgg gtgttctaga ctttggaatg 2220

gttcttaaag tttcctggaa ataaaaataa tgattggcac ttcaaagaca ttctttagcc 2280

aagacttcag tgtctagcag aaaccacaag tgactagaag agcaagtgat cttggtgatg 2340

cacttgattg tatacaatga gtattttttc tcttaaactg gaaataaatc tgttagaaat 2400

aatatagcca 2410

<210> 664

<211> 418

<212> DNA

<213> Rattus norvegicus (rat)

<400> 664
tgacatactc caggtttgta agtttagtga tttttactta caaatttacc cttttttgca 60

ttctaaaatt gtgtttaaat tatatggaag tacttggtgt aggcagtcat tggtccccgg 120

gcagcagaag ctctgcctgt ggaatcgggt ttgggttcac tctgcagggc tcctcataga 180

ggctttgctt atttgttttg aggaaaatgt ctggagtaaa cctttgtttt ctgaaactac 240

tttagctaaa agaaaatggg tgttctagac tttggaatgg ttcttaaagt ttcctggaaa 300

taaaaataat gattggcact tcaaagacat tctttagcca agacttcagt gtctagcaga 360

aaccacaagt gactagaaga gcaagtgatc ttggtgatgc acttgattgt atacaatg 418

<210> 665

<211> 8351

<212> DNA

<213> Rattus norvegicus (Norway rat)

<400> 665
gaattccatc agaatttgcc tttctggtgg cttctctttc cctgcccttg tggtgttttt 60

ccttcagtga gtgacaaatt tcacaaccct ggctgggact cccgaggtgc taagattaca 120

ggcctgggtc acaccaccca aacctgactt tctttttcat tgttgcttgt atttttctgt 180

ttgtaaccaa agctggacga ctggaactca ctgtgtagac taggctggcc ttgaactcat 240

agaactctac ttgcctctgc ctcctgagtg ctctgattaa cggcactgac caatacatcc 300

aacctaccta ctttcatttt ctaaatctaa gtcctaacag gaagtgggaa ctgggcagga 360

ataacagtac ggtgggttaa ctccatgagt ttaccggact ttgcgagcct cgactgccaa 420

cgacatcctg gcttgaggct ggaagtcaca gcccaacagg ggcaaagatt gctctgtgac 480

cagtctggaa agggagcact ggagcacaga aatcaatccg gttcaagttc atacccaggg 540

caaccacgga aagtgccagg aaaggaaaga acaggatgtt ttccacacat tccatgggca 600

gccatgggga tccaggagaa agtgatgctt ggctgagcca agaagcagtg ccccagttta 660

cagtaagggc tgagaggaca gcctgtcctg agcttccggt aacacatttc ctgccttctc 720

aaatgacaga cattccatct acgactttga gtctgatttc agcagtctta tgcaagaggg 780

gaaacaccat atgcctccag ggaaagaaaa tttggctgcc gtctccaccc tttccctcag 840

catccaccgt gggtgggggt ggggagggtt agtggggctt tccatccctg tctttcagaa 900

cactacgatc tggccctttc tgcttggcca acacctgcgc agagtcctag ttcatatcct 960

cccagaatgg cctgctctcc acctccagca gagaccccca tcattttttc ctgttcactc 1020

tctgcccccc accccccacc aagaataagt atccttagca caaggcttgt gtctttatgg 1080

tctctagtct ctgacaactg gctggagtct cagtggattc gaaccctcca ttcatcttgg 1140

gctaatgact atgtgattgc gcctccgttt ccacttttct actgtgaaaa taatgaacac 1200

cccaagctat gttgtaagga aaaatgagag ccctaacagt gcccccagca cgtgacacgc 1260

agggggtacg tgacacgcag ggggtacgta accaaggccg gtaaagtctg ggctagggct 1320

ggtttttgtt acctgttcac actgtcagct aggttttcct gtatgcgggg tctccaagcc 1380

ccgctttcac ctaagttagc actcaagacg tgctgtgggg actgtgtccc cgtggacgct 1440

gcagggggtt cgatgtcccg caactcctct gcacccggcc acttgggccc agggcacgtg 1500

agcaggtttc ctggaaccgg tccccaccgg atcgcagacc ctttgcgctc agccctttgc 1560

tctcagtccc tgcgccagga gaaagggggt gtgactcagc gcgggggcgt gtgcaggctc 1620

tgtacccacg tgcaaaagga gggatgcttg cagacttcgg gtcgtgcgca ggctcccggg 1680

cgtgtgcggg ccatttccct tgagccagaa gaagggcgtg tgcaggcagt ggggaggagg 1740

gcaggtggcg ccccgccacc cgggcggagc ttttgcgcgc gacccaatac tctgggctat 1800

aaaggtcgcg ctccgcgtgc ttctctccat cacgctccta gaactctaca gcgatctctc 1860

gttgatctcc aactgccgcc tccattcgcc atggacccca actgctcctg tgccacaggt 1920

aaggggggct gctgacgggc ctctgtaacc ggagcttctg ggagagcagg acggactttt 1980

gggcccctac tctggtaact acttttaggg tactactggc tgctgccttc cgaacgaatt 2040

ctggaacact cccgcccctt ttaaactagt ccttgagata atggctcgcc caagctggct 2100

ggcttgcacc cgagttcttt ggagaactgt gttcagttat gcccgggtcc gctcaccgcg 2160

ctccctgcct tcttcctcta gatggatcct gctcctgcgc tggctcctgc aaatgcaaac 2220

aatgcaaatg cacctcctgc aagaaaagtg agttggattt attttctcta ccctttccct 2280

tcgcgcccct gcggtcccta gcccgccgca ccttcccaga gcgtccaggc tgcctctaac 2340

tcggtttctc gctcacgctc aacttttttc ccccaggctg ctgttcctgc tgccccgtgg 2400

gctgtgcgaa gtgctcccag ggctgcatct gcaaagaggc ttcggacaag tgcagctgct 2460

gcgcctgaag tgggggcgtc ctcacaatgg tgtaaataaa acaacgtaag gaacctagcc 2520

tttttttgta caaccctgac cggttctcca cacttttttc tataaagcat gtaactgaca 2580

ataaaataaa aaaacttgac ttgattaacc cagctttgtc tgtgttcatt ggaaataagg 2640

ggctggcaga ggcgttgaaa tgggattggt gcaccttgat ttgggataag tggattgatg 2700

acccctctgg actttgatag tctcgaacat ggtgggcaga aacatgtact ggtcacaaat 2760

gtgggcatgt gtatattggg gattaaaccc aaagcttcct gcttataaac cagggtgctc 2820

taatgagcca cactcctacc cctagatgca taatgattct ggtttaattt tggattatta 2880

ggcttaaagc agtatgaagt acctgttcat aagctttggt aaataaaata aaagttggag 2940

tgagtctcat acgactcctc tttgtagtcc caatatttgg gagcctgagg cagaaggatc 3000

ggtgcaactc cgaagccaac ttggtctcaa attctgttaa cctttgattt tgagaccatc 3060

ttactgtgta acctaaaatg gtccttgaac ttgcagtcct gcctcagact tctaggtact 3120

gggattacag gctcagctta aaatcagggc tggagagatg gctcagcggt taagagcacc 3180

cgactgctct tccagaggtc atgagttcaa ttcccagcaa ccacatggtg ctcacaccat 3240

ctgtaatgag atcttacgcg ttctggcgta atgcaagcag aaaagacatc agtaacgtga 3300

acaaaaccat gaaaagtact gtaaacacta taaatatcca agggtgtgcc ttgcagtttg 3360

gagactaaat ggcacatgtc caacctagag ctcccatgag gaactgccca tctctggtat 3420

acagggacac ggacaggatt ttttttttcc tcttccagag agccctgtga taggacttgg 3480

ctgtcagtct ggaagttctt ctcaaggtca ggcagaaatc tacctacccc tcactccata 3540

ccaacccctg gcaatttaag caaagtaact agaaatttgg aaggaattga ctagcatctt 3600

cccaggagct aggcatccag gttgagtctg caatttggag ggcggggtgg agtttcctac 3660

tctataggaa ggaggtgaat acatgcaatt aaaaccagcc gttaatgccc cctggctatt 3720

tgttgaggta atgcgatttg gtcttcaatc aaagggaaag tttcttggct agaagtaagg 3780

accaagcttg ccgtaggctt tctctgtgaa gagtaaattt acaagacagc ctctgtttct 3840

tgctgtcagg aagtcctagt tcacagccca ctttctctct tattggtcat gtagcctggg 3900

caagtcactg aacccctcaa atgctgatat cctgccctcc tagatgctga taaccacctg 3960

tccccaaaga acacacgggc aaccaagcac agatctgatt tttaaggaat ttgttttgta 4020

agtgcagttt gggaatctgg cctcatttgt ctcttgtgtg cccttgctga caccattcat 4080

tcagccctgg ccttgattta ggtgacaccg aactcgggct gtaccctcag agatttccct 4140

ctttgtctac aaacaaacaa acaaagcaaa tatcctaatt aagactcttg tgtgtcaagt 4200

agggcatcta ggaatgagtg ctgggaccac tcttagtccc agaatgcctt gaaaccaagt 4260

gaatgacaat tatacattta gcttctcaat taaaatggaa gacattgggc cgggaattgg 4320

ctcacagtgg agagcctacc aggcttttgt gaagctctga ggttcatccc tagaaccatt 4380

aaaaaaaggt ccgtgggcct gggaatgtag ctccctggta cagtgcttac ctaacatgca 4440

cggacccctg ggtttgctcc acagcatgga gtaagcagtc tgatggcaca cacctgtaat 4500

tctaacacgc aggaggcaga ggcaaggagg atcaaacgtt caaggaccac ggcaagtttg 4560

aggtgtgggc cacatgtaaa gccgtctcca aaaagacatc acacacaaaa cacaacagta 4620

ttgtgataca cacgtatacc tgtatcctag caacctggga aactgaagca ggagactgtc 4680

ttgagttcaa ggccagactg ggctgttcgg tgatcgacag gccattctga gttacagagt 4740

gaggcccttg gaaaaggaga ggaaggagag gaggagagac tgggcctggc aacatgcatc 4800

tatcatctta gctactcagg agactgaggc agggggagga tttccagctc aaagtctagc 4860

tacagagcac gtctaaagcc agcctggaca gcttagtgag accctgtttc aaaataaaaa 4920

gaatctaaaa gactggaggt aaagctccag tgtagaatgc ttgcctggta accaggaagc 4980

cttgggttca atccttactg taaaaaaagg aaaaaaaatc atattatgca agaggtctaa 5040

aggcccaaga atctgttaca gatctcagtt ttggtaatag acaataaaat ataacaagtt 5100

ggtaaaaaca agcaagagta ccaactacaa acatacttca tgtggttcag cagaagcatc 5160

tcagtatgca tccagaaaac agcagacaga cagaattggg catccttggg ctagggcaca 5220

cctcagcctg acttctaccc gagaagccag cagtcttagc cagtgcagaa ccactggtgt 5280

ctctgacttg ggatctctgc ttaggatgcg cccttgagtg cttagaattt gtctctagtc 5340

aggctgaatc ctctctcttt ccaaacccag tccttagcta tttaaaacca gtaaactcat 5400

gagatttgga gtcatccaac gttatccagg caaggattct gtttttttct taatttttat 5460

atttaattgc ttattaattt ttgaaatagg atctcatttg tgtggccctg gctggccttg 5520

aactcaagaa gaccatctgc ctctgacttc taatagctga gattaaagag gtagcctcag 5580

gcaagaactt aactatagac caagactcag ttccacgtga agttttttgg atcttcccac 5640

acagagggta taactgtgtc atctccaaga tgaggtatcc cgaggaagga gaaatggcct 5700

gggtcattgt caccaaacca gtgggtaata ggttaatgga aagacacatg tgtctaaacc 5760

accaaggagg aggaggaaga gggcaaagag gggaaagaag gaggaggggg aggagtgtca 5820

tagcccagga ctaggtgcct tctttgccta cacacggacc tacgtacaga aggacagcat 5880

cagagaactt gggacccgta caggaacatt ggtgtcaagc tgtactgctt cacagcccgt 5940

tttactactg actggttgta tggcccaccc ggcaggtcat tgaatcctct gtccttgtgt 6000

gtaaatagaa tttgcatctc tatataggta ttaggtgaga gatcggtttg actcctggtt 6060

ctggcataat catcatatcg cacagtggct ggtggaggtc ctataacagt taagcaaaac 6120

ctgcccaagg tctcatagct ctgagtacgc gtgaaccaat ggcatagctg atctcttgcc 6180

ctagtctcaa gggctgacag aatctaacgt tactctaaag tcagaaacat tgaaaatata 6240

aacagctgcc cgtattggtt ttggtttttg tttttgtttt tttttttttg tttatttgtt 6300

ttgttttgtt ttatctaatg cagtccctgg atatcaccta aaatgatccc tctgcctcgg 6360

tttttttttt tttttttttt tttttttttt tttttggttc tttttttcgg agctggggac 6420

cgaacccagg gccttgcgct tcctaggtaa gcgctctacc actgagctaa atcctctgcc 6480

tcggttttta aaaccggcct ggagtagagc cgatggctaa aggtttgtga cccccagccc 6540

ggaacgtgcc tacatatgcc cgctcatgag tggggaatat gttgcgatga gtgtccgttg 6600

gctctgttgc tgtgtccaga aggaaggggc tcaaccaaag accatgatgg gacagagaca 6660

gacaataagg acccggaaag ttcgtaatca aggctagtct ttataaaact gtctccttcg 6720

cctctgctag cttcgattca gagagacgtg ggcggagccg gtcgctgccc aggaactcca 6780

ggaaaggaga agctgaggat agcgcgctac gattgtgttt acagagacag ttgggcttcc 6840

tgaggtgtgt tctcgtaatg cactggatca gtgatggcct gtaatatccc ggaaagcact 6900

acagaaacat gatgttccac acgtcacacg ggtcctccta cccgggccct cctactcggg 6960

cctgtggcac caaagggggc ggtcccgttg tgcacaccgg cgcccgaggg agctctgcac 7020

tccgcccgaa gagtgcgctc ggctctgcca aggacgctgc gctcgtgact gagcgcgggc 7080

tggagcaacc gccaactgag tgcaaaccct ttgcgcccgg acccgtccaa cgactataaa 7140

gagagcagac tgtccgctaa gcctcatccc gacttcagca gcctgactgc cttcttgtcg 7200

cttacaccgt tgctccagat tcaccagatc tcggaatgga ccccaactgc tcctgctcca 7260

ccggtaagac gcccggtcct tggtctttag aatacccagt tgtaggggtt tggcgggaat 7320

aggcaccttt agttgacaat tcgtcctagt tctttctaga acccgctctt ggaatcgcct 7380

tcacctgttc ttggagtatt attattgtcc gaacggctcc ttgtcggggt ttggggtagg 7440

atttagacgc gcaaataaat gtcccgatca cccacgtagt gggacatctg agttgagacc 7500

cagttgttac taaccttatt gtgaattgcc tgatctacaa gagaggtgag agaccgttgt 7560

gtcttgagat caaagaccca agccttaccc taccctgtga ggagagaaga ggggctaggc 7620

tccctggagt tctgaatagc actttgaatt gagcagggca catggtgttg gccactgctg 7680

taatcctgcc tcttactgac cgctgtcttc cttctcctcc acaggcggct cctgcacctg 7740

ctccagctcc tgcggctgca agaactgcaa atgcacctcc tgcaagaaga gtgagttggg 7800

accctcgggt ggtggtgggg gaactcctac agagctggct ctgagaaacg tctgaggcca 7860

ttcggtttgg ggcaagaagc aggtcttctg ccagacctgt gcgaccggag gactaggaag 7920

cctactctga catcttcctc tatctttctt tccaggctgc tgctcctgct gccccgtggg 7980

ctgctccaaa tgtgcccagg gctgtgtctg caaaggtgcc tcggacaagt gcacgtgctg 8040

tgcctgaagt gacgaacagt gctgctgccc tcaggtgtaa ataatttccg gaccaactca 8100

gagtcttgcc gtacacctcc acccagttta ctaaaccccg ttttctaccg agcatgtgaa 8160

taataaaagc ctgtttattc taactctggt tttcttggtg tcgtttagaa ataagaaact 8220

ggggcgacac gggttaactt gatagtctgg ggatctggtt ttggactcgc ccgtgccttt 8280

taactcccgc ctctggctcc caaagagggg taataatgtc tttgggtaaa gccaagttat 8340

cccataagct t 8351

<210> 666

<211> 91

<212> DNA

<213> Rattus norvegicus (rat)

<400> 666
ggtgtctcag ggtcttctgt gacctcaccg aactgtccga ctgcaccgtt tccaacttgt 60

gtctcactaa tgggtctgca ttagttgcaa c                                    91

<210>  667

<211>  2130

<212>  DNA

<213>  Rattus norvegicus (Norway rat)

<400>  667
ttttctccga gttttctgaa ctctggctca tgatcgggct tactggatac gagaatcctg      60

gaggaccgta ccctgatttc catctacctc tgactttgag cctttctaac ccggggctca     120

cgctgccaac acccgggcca cctggtccga tcgtcttact tcattcacca gcgttgccaa     180

ttgctgccct gtccccagcc ccaatggggg agtgagagag gccactgccg gccggacatg     240

ggtctcccca tcgtgcctgg cctgctgctg tcactggtgc tcctggctct gctgatgggg     300

atacacccat caggggtcac cggactggtt ccttctcttg gtgaccggga gaagagggat     360

aatttgtgtc cccagggaaa gtatgcccat ccaaagaata attccatctg ctgcaccaag     420

tgccacaaag gaacctactt ggtgagtgac tgtccaagcc cagggcagga aacagtctgc     480

gaggtgtgtg ataaaggcac ctttacagct tcgcagaacc acgtcagaca gtgtctcagt     540

tgcaagacat gtcggaaaga aatgttccag gtggagattt ctccttgcaa agctgacatg     600

gacaccgtgt gtggctgcaa gaagaaccaa ttccagcgct acctgagtga gacgcatttc     660

cagtgtgtgg actgcagccc ctgcttcaat ggcaccgtga caatcccctg taaggagaaa     720

cagaacaccg tgtgtaactg ccacgcagga ttctttctaa gcggaaatga gtgcacccct     780

tgcagccact gcaagaaaaa tcaggaatgt atgaagctgt gcctacctcc agttgcaaat     840

gtcacaaacc cccaggactc aggtactgcc gtgctgttgc ctctggttat cttcctaggt     900

ctttgccttt tattctttat ctgcatcagt ctactgtgcc gatatcccca gtggaggccc     960

agggtctact ccatcatttg tagggattca gctcctgtca aagaggtgga gggtgaagga    1020

attgttacta agcccctaac tccagcctct atcccagcct tcagccccaa ccccggcttc    1080

aaccccactc tgggcttcag caccacccca cgcttcagtc atcctgtctc cagtaccccc    1140

atcagccccg tcttcggtcc tagtaactgg cacaacttcg tgccacctgt aagagaggtg    1200

gtcccaaccc agggtgctga ccctctcctc tacggatccc tcaaccctgt gccaatcccc    1260

gcccctgttc ggaaatggga agacgtcgtc gcggcccagc cacaacggct tgacactgca    1320

gaccctgcga tgctgtatgc tgtggtggat ggcgtgcctc cgacacgctg gaaggagttc    1380

atgcggctcc tggggctgag cgagcacgag atcgagcggc tggagctgca gaacgggcgt    1440

tgcctccgcg aggctcatta cagcatgctg gaagcctggc ggcgccgcac accgcgacac    1500

gaggccacgc tggacgtagt gggccgcgtg ctttgcgaca tgaacctgcg tggctgcctg    1560

gagaacatcc gcgagactct agaaagccct gcccactcgt ccacgaccca cctcccgcga    1620

taaggccaca cccccacctc aggaacggga ctcgaaggac catcctgcta gatgccctgc 1680

ttccctgtga acctcctctt tggtcctcta gggggcaggc tcgatctggc aggctcgatc 1740

tggcagccac ttccttggtg ctaccgactt ggtgtacata gcttttccca gctgccgagg 1800

acagcctgtg ccagccactt gtgcatggca gggaagtgtg ccatctgctc ccagacagct 1860

gagggtgcca aaagccagga gaggtgattg tggagaaaaa gcacaatcta tctgataccc 1920

acttgggatg caaggaccca aacaaagctt ctcagggcct cctcagttga tttctggccc 1980

cttttcacag tagataaaac agtctttgta ttgattatat cacactaatg gatgaacggt 2040

tgaactccct aaggtagggg caagcacaga acagtggggt ctccagctgg agcccccgac 2100

tcttgtaaat acactaaaaa tctaaaagtg 2130

<210> 668

<211> 325

<212> DNA

<213> Rattus sp.

<400> 668
   aaataaatca tttttttaat tttggaaaaa aagaatcaaa aggcagggag aggggggact 60
   aggaagagct gagtaggtaa acaccttgct tctatctcaa ggacagactt tggtctccag 120
   aaccctgggt tcccttgtag tcctagcact gggtaggcag agacacttgg gtccctgggg 180
   ctctctagcc agtctagccg tatgtatgta tgtatgtatg tatgtatgta tgtatgtatg 240
   tatgtgtgta tgtggtttct tagtatagga tctgtgggga ttggaggaca aggggccatg 300
   gaaaatgcca tatggtgaag caagg 325

<210> 669

<211> 506

<212> DNA

<213> Rattus sp.

<400> 669
cacactcaag attttattta aaaattccaa aaaaacccac aaaaatagac ttgtacggta 60

acaatatatt acttaatata ttatagtata tacttaatac agtatcagtt ttaacattac 120

aaaaagaaaa acggagtaga cgacccaata tatataaata ctataaaatc ttattacaat 180

aaataaagtc gtcataaata aagggtccat tggttggggc aggggcgtgg gggaaaaggg 240

atttggcaat ggctttggct atttgctttg ggagggaaat ttgagcacca ggattctcag 300

gagaggctac aaccaccgga cactgaactt cggggtctag taaaaattag gatactattg 360

tgggagccgg gtcttcacag cacttaacat tccaggagac cagagtttcc gaggggctgg 420

tgcatacctg taaccccaga acttggaaaa ggctaaggca ggattatatt gagttcaaag 480

ccagcctggg gtaccctagt aatccc 506

<210> 670

<211> 462

<212> DNA

<213> Rattus norvegicus (Norway rat)

<400> 670
tttttttttt tttttttaaa aatagtttat ttatttttaa aaccattaag gcaaaactgt 60

accctgaaat taactcttta cattcctcag cttacagtag gatgttccaa acatacaggt 120

tcccataaat tcaggatata aatcaatgca tacagtcagt tcggtctcct acggagaaaa 180

cgaagactcg attttttttt tttaaattaa gattaagtta tagccatttt ctaattgccc 240

ggaaccaatc agctcatgtg actagaaaat tctatttgcc cttctgtgtt caccctgatt 300

cctgatgtcc tggcgagccc tgtatccaca acaagctggg ggttggggtg tgtgtgtgag 360

gatgctggta ccggctcgac aagtcttatt caggcctgtt agtatcacag gggtgaaggg 420

ctgcaagctc ccaggtctga taggatggca ccaaggacat ta 462

<210> 671

<211> 626

<212> DNA

<213> Rattus sp.

<400> 671
caaagtagaa atttaattca attaaagatc aaacaaagca ggtgtttgta ttggtctaaa 60

tatttgatcc actctaatga atattattct tctcaccgct aggcatttat ttttagatat 120

ccacaaactt cattgttaat ttccctgtga ataaaaagat aacagagaac caatgtttag 180

ctcccgccct caacccaaag tggctcactg tatgggtcag agggtgcgtc tcctctttgt 240

caggggtcac gagcatcaag gcagaagaca tggcttgtgg tatacgttgg tctgctgccc 300

cgtggtctcc caaatgaagg acaattttcg cagagtcatc gagaaggtaa atgcattggg 360

tttctgaaga aacacaaaac tttcttcagg ttaaacactc cattgttttg atgactcttc 420

tgaatctgtt tccaccagga agctgtctag ataacatcca gtacgtttac tacttaactc 480

ttctaccagt atattaacat gaggctatgt gattcaaagt aatatctaag ggattctgga 540

aaatgttagg tttcaaacca gtcaactaca gaactactta ctaaatacta tgttacaata 600

agatttattg acctaaacat tattac 626

<210> 672

<211> 567

<212> DNA

<213> Rattus sp.

<400> 672
cacatcgaat tccattttaa aaacaaaaat aattttaatt aaaacaattt cttggcattg 60

ttcaaaaaca taagactctt tttacaaaag aaaacttccc atcttggttt taaaaaaggc 120

aaacgaggaa gtaagctgca ttagacataa gaggatggac agcctctgca gccacaggag 180

aaatgtgttt gctttcttat tacagttagc tccttctgtg ggagcctgga gtcttgcatt 240

tgttttatgc atattgggcc ttgttgtatt acttggcagg agcctgtact cccttgagtc 300

agccgtgctc attggtcaga acacattcca gcatctgaca tcaatcccat taatcacggg 360

cgggcgtgag tgagatgact caacagtgag cagtgccggc tatgcatggt attcagtgat 420

gagaggtcgg agatcctctg aattcagcat acgtgagatg ccaaggacaa agtcctctca 480

catcaaatag gtagtgcatg atcactaata ctcttaatgc ttcaaccttg aagggattta 540

aatgaatttc cacataaagc catcttc 567

<210> 673

<211> 447

<212> DNA

<213> Rattus sp.

<400> 673
ttgtattcat aaagtgctct aaacaagatg ttctttttag cagttgggga aaaaggttct 60

ctaaaaggca tttaattctt agtggaaaaa taatattaac aaaagccttg tgcgaatgtt 120

tgaatgacaa tttgctcatt ttcttcatga attggggttt gatagaaaat gcatatgtgt 180

cactgaaaga cagagtgatg ggtctgtgtg gttggaactc aaaatgacat tgctctgtca 240

gtgtgtgctg tgccggcttg atggctttga tgggggaggg gtacacttgg ctggtggtac 300

ttccaaaggt gaatcttgct atgtagggtt agtggtcagg gcagccattc aggctgacag 360

aaccttggac ttctgtggct tctgtgatgg ggacagggac atggttgact tgaatattct 420

tcagacagcc aaaaaatgag ttccaca 447

<210> 674

<211> 405

<212> DNA

<213> Rattus sp.

<400> 674
ggcttttatt attcacatgc tcggtagaaa acggggttta gtaaactggg tggaggtgta 60

cggcaagact ctgagttggt ccggaaatta tttacacctg agggcagcag cactgttcgt 120

cacttcaggc acagcacgtg cacttgtccg aggcaccttt gcaaacacag ccctgggcac 180

atttggagca gcccacgggg cagcaggagc agcagctctt cttgcaggag gtgcatttgc 240

agtttttgca gccgcaggag ctggaccagg tgcaggagcc gccggtggag caggaccagt 300

tggggtccat tccgagatct ggtgaatctg gagcaacggg gtaagctaca agaaggcagt 360

ccctcgtgcc gaattcttgg cctctagggc caaattccct atagg 405

<210> 675

<211> 370

<212> DNA

<213> Rattus sp.

<400> 675
caagtcaagt ttttttattt tattgtcagt tacatgcttt atagaaaaaa gtgtggagaa 60

ccggtcaggg ttgtacaaaa aaaggctagg ttcctacgtt gttttattta caccattgtg 120

aggacgcccc cacttcaggc gcagcagctg cacttgtccg aagcctcttt gcagatgcag 180

ccctgggagc acttcgcaca gcccacgggg cagcaggaac agcagctttt cttgcaggag 240

gtgcatttgc attgtttgca tttgcaggag ccagcgcagg agcaggatcc atctgtggca 300

caggagcagt tggggtccat ggcgaatgga ggcggcagtt ggagatcaac gagagatcgc 360

tcctcgtgcc 370

<210> 676

<211> 608

<212> DNA

<213> Rattus sp.

<220>

<221> misc_feature

<222> (596)..(596)

<223> n

<400> 676
   cggccaactt gatgttctag tgctgaaggg agcaagggcc aggcatgtgg tggagatgat 60
   gctgaaatgg tttatccaat accatgcaaa tcaagtcctt tggatagagg tgaagaactt 120
   ggacatggct gtttcaggca gctgaagtca aaaggaccgg gactggggag ggcagggacg 180
   ctaagtgaaa gggctggtgg ccaacgggcc ttctactgac tagaaggact tggctctgag 240
   atcttccatc tgacctaaga ggcagcccct cttgcgggcc atctcttccc tcctgtcctt 300
   tattgtctct cgcactcccc tccctttgct ctctttcatt ccctttagca aatttcaatt 360
   gtcctaggag aaaaggttgc tgtcatgtct gaaagacccg ttgaggtgct gaaggagttg 420
   ctgacccctg cagactggaa ggtgctgacc tgggcaggga aagcaggtgg gacggaggca 480
   taggtggtgg ccaccgaggg cgatgggaag ccactgtgcg caggagacgg gtaggtagag 540
   gagcccggag aggagtaaga ggtgggcact ggggatggaa atgaggtggt ggcggnggat 600
   gggttaga 608

<210> 677

<211> 463

<212> DNA

<213> Rattus norvegicus (Norway rat)

<400> 677
ttgagaaaga acttaaagtt gggagggtag agcagtagag gatctggata tacttggggg 60

gagggaggga agtgcatgag caaagcataa atttaaaagt tgcttttaaa taataagaaa 120

ttttatatag aaagaatact tacttatcaa tgcacacttt aattttaagc tgataattca 180

actcaggaaa tttgaccagc aacctattaa aaacatatag acaaagaaag ttagtaggct 240

tcgtaccatg atctcttgtt taaaaaaaat gtaaaacccc taatacttca ggtcctaaga 300

agtaaataca ttgctgagca tggcggtgca cacctttaat cccagaactg gggaggcaga 360

ggcagatggc tctctgagtt caagcaagcc tggtctacag agtcccaggc tagccagggc 420

tacaaagagt cctgtctaga tgaaaagaaa aaaaaatatt tac 463

<210> 678

<211> 294

<212> DNA

<213> Rattus sp.

<400> 678
aaatggcaag tgtagaaatt tatttgaacc aacattccca ccacaatgaa atgactcaac 60

actgatccag gcgcccacgg ctgagggtta gggagtattg cactttgcat ttctttagga 120

ctccatacac ggcaaaaagt ttcaaaaacg ttttgcttgt tttgttttac acactctccc 180

aactcatgca cacactcatt ccaaaccaac gtttggttat cccaaaaatg gggaagaact 240

ccaaaaaaaa aaaaaaaaaa acccatagcc ttaaattgca ctaaagttct gtaa 294

<210> 679

<211> 2130

<212> DNA

<213> Rattus norvegicus (Norway rat)

<400> 679
gagacctctt cctccaacct gcacaacttc ccgcttttca cctgggaccg gcggagtgac 60

ctgcggactt aggcgcgggc aattctgggc tgggcgcccc gctgaggggc cgcccatcgg 120

ggcgaaggga cttgtccctt tgcccctcta gccgacttgc acggatggcg ccgcggctga 180

gtgacgagag ccccggtgca ggacttcccc ctcctggatc ttcggccttc gcggggcaag 240

atgtacgaaa gtgtagaagt cggggggctt acccccgccc ctaacccctt cctagtggtg 300

gacttttata accagaaccg ggcctgtttg ctccaggaga aggggctccc tgccccgggt 360

ccctactcca ccccactccg gactccgctt tggaatggct caaaccactc catcgagacc 420

cagagcagca gttcggaaga gatagtgccc agccctccct caccaccacc cctgcctcga 480

atctacaagc cttgctttgt ttgtcaagac aagtcatccg gctaccacta tggggttagc 540

gcctgtgagg gctgtaaggg cttcttccga cgcagtatcc aaaagaacat ggtgtatacg 600

tgtcaccggg acaagaactg catcatcaac aaggtgaccc ggaaccgctg ccagtactgc 660

cggctgcaga aatgcttcga agtgggcatg tccaaggagt cggtgcgaaa cgaccgaaac 720

aaaaagaaga aagaaacacc caagcccgag tgctcagaga gctacacgct gacaccggag 780

gtgggggaac tcatcgagaa ggtgcgcaaa gccaaccagg agaccttccc ggccctctgc 840

cagctgggca agtacactac gaacaacagc tcagaacaac gtgtctctct ggacattgac 900

ctctgggaca agttcagtga actctccacc aagtgtatca ttaagactgt ggagttcgcc 960

aagcagcttc ccggcttcac caccctcacc attgccgacc agattaccct tctcaaggct 1020

gcctgcctgg acatcctgat tctgcgaatc tgcacgcggt acacacctga gcaagacaca 1080

atgaccttct cagatggact gaccctgaac cggactcaga tgcacaacgc tggctttggc 1140

cccctcaccg acttggtctt tgccttcgcc aaccagctgc tgcccctgga gatggacgat 1200

gctgagaccg gactgctcag tgccatctgc ctcatctgtg gagaccgaca ggacctggag 1260

cagccagaca aggtggacat gctgcaggag ccgctgttgg aagcactgaa agtctatgtc 1320

cggaaacgga ggcccagcca gccccacatg ttccccaaga tgctgatgaa gatcacggac 1380

cttcggagta tcagcgccaa gggagctgaa cgggtgatca cattgaagat ggagatccct 1440

ggttccatgc caccacttat ccaggaaatg ttggagaact ctgagggctt ggacactcta 1500

agcggacagt cggggggcgg aacacgagat gggggtggcc tggcccctcc tccgggtagc 1560

tgtagcccca gcctcagtcc cagctcccac agaagcagcc cagccactca atccccatga 1620

ccacctcgac acatggacag ccctcacccc tgccctggct ttctctgcct tcctactggc 1680

catgtgacct atcagcccag ccctgcttgt ccttccggac agaactgggg accttccagg 1740

ggggacagga gggagaaggc agtgactctg tggacagagg cctggaccct gagatggact 1800

gctctctcct ggcagcctgg gctggcatca ggggccaggc attgagccac gtgaggacct 1860

gggcctgagc cctcacccag cttgccatgt cttcatcacc agcaaacacc agggcctggc 1920

tccctgtcct ccgaactcaa gccatcaccc cccagttggg gaacttcccc ctgcttcgga 1980

tggtgacaga agatgcccag ggtgggggag accccctgta catatcctgc gtaccaaccc 2040

ccagatatta attctcgatg gttttgtttt tattttaatt tttttgtttt tgtttttttt 2100

taataagaat tttcatttta agcacattta 2130

<210> 680

<211> 170

<212> DNA

<213> Rattus norvegicus (rat)

<400> 680
gatgtcaaca gagtaaccta ccagggctac tccccctacc agcttacgtg gggccgccca 60

agtacccgga tacagcagca gctgggccag ctgaccctgg aaaatcttca gacgctgccc 120

gagagtgagg atgaggagag ctatgacacg gagtcagaat tcacggaaga 170

<210> 681

<211> 924

<212> DNA

<213> Rattus norvegicus (Norway rat)

<400> 681
cctggtggtt ccgcagccgt actctcctgg cctggtgtgc acagcctcac catgagttct 60

tctgcagcca gcccgctttt cgcgcctggc gaggactgcg gccccgcgtg gcgcgcggcc 120

cccgcggcct atgatacgtc tgacacgcac ctgcagatcc tgggcaagcc agtaatggag 180

cgttgggaga ccccctacat gcattcgctg gcggctgctg ctgcctccag agggggccgg 240

gtcctggaag tgggctttgg gatggccatt gcagcctcca gggtgcagca ggcccccata 300

aaggaacact ggattattga atgcaacgat ggggtcttcc agcgtctaca aaactgggcc 360

ctgaagcagc cacataaggt tgttcccttg aaaggcctgt gggaggagga ggcacctaca 420

ctgcctgatg gtcactttga tgggattcta tacgacacat atccactgtc tgaagagacc 480

tggcacactc accagttcaa ctttattaag actcatgctt tccgtttgct gaagcctggg 540

ggtatcctca cttactgcaa cctcacgtcc tggggggaac tcatgaagtc caagtacaca 600

gacatcactg ccatgtttga ggagactcag gtgcctgcac tgctggaagc tggcttccag 660

agagaaaaca tctgtacaga ggtgatggcg ctggtgcccc cagccgactg ccgctactat 720

gccttccctc agatgatcac acccctggtc accaagcact gagcggctgg cccagggcta 780

caaggagaat atgtcctcct cagtgccttt gtagctggag tgtggctcca gcctctccac 840

tatccctgca gtgtgacatc ctaacctctg cctggtacgg ccatctcccc agagctcagg 900

agtaaaataa atgctaccaa gact 924

<210> 682

<211> 8048

<212> DNA

<213> Rattus norvegicus (Norway rat)

<220>

<221> misc_feature

<222> (2005)..(2005)

<223> n

<220>

<221> misc_feature

<222> (3503)..(3503)

<223> n

<400> 682
gaattcggcc caggataaac agatatatag gtaaatgact ggatagatac atggatttga 60

tctctaactc aacttaggca ccatagaaaa caatccttga attattttaa taattttaaa 120

tcctagttta ctgagaaaca cgttgacgag gcagggcttc agcctctctg aagcagcact 180

gcccctctgt ggccacacag gaagtcttca ctcagcagtt tcctgactta aggcctaggc 240

ctgaggaaat gcattgaata tttctctggg ggacaatagt tctgagaagg aaagagagtt 300

tgtctatgtt gaggtcagtt aaggatggtt ttctttcctt ccctcccagc cttttctcca 360

tgcaacatca tgagtcccca tctttctatc ctgcctaaaa ccacttcctt ctgggaaatg 420

agaaaatcca ctcatacaaa atggaaacat cccgaggatc ccggcccgca gcagctctct 480

gcccccagac cctgtgagag agagacccaa ccgcctggtc aggtgggcac tcctgaggct 540

gcagagggaa gagaccacca acactgctca cccctgccca catccctggc ccaagaggaa 600

actgtataag gcctctgggt cctgtggggg agggcccagg agcggcagga cccctgtgcc 660

tgagagacca ccaccagaac cagaaggaaa cagaccggat aaacagttct ctgcaccaaa 720

tcccgtggag ggagagctga accttcagag agaaagacaa gcctgggaaa ccagaagaga 780

ctgctctctg cacacacatc tcggacgcca gaggaaaaag ccaaagacca tctggaaccc 840
tggtgcactg aagctcccgg aaatggtgca caggtcttcc tggttgctgc cgctgcagag 900
agcccgtggt agcaccccac gagcgaactt gagcctcagg accacaggta agaccaactt 960
ttctgctgca agaaagctgc ctggtgaact caagacacag gcccacagga acagctgaag 1020
acctgtagag aggaaaaacc acacgccgga aagcagaaca ctctgtcccc ataactgact 1080
gaaagagagg aaaacaggtc tacagcactc ctgacacaca ggcttatagg acagtctagc 1140
cactgtcaga aatagcagaa caaagtaaca ctagagataa tctgatggcg aaaggcaagc 1200
gcaggaaccc aagcaacaga aaccaagact acatggcacc atcggagccc aattctccca 1260
tcaaaacaaa catggaatat ccaaacacac cagaaaagca agatctagtt ccaaaatcat 1320
ttttgatcat gatgctggag gacttcaaga aagacgtgaa gaactcctta gagaacaagt 1380
agaagcctac agagaggaat cgcaaaaatg cctgaaagaa tcgcaaaaat ccctgaaaga 1440
attccaagaa aacataaata aacaagtaga agcccataga gaggagacac aaaaatccct 1500
gaaagaattc caggaaaaca taaataaaca agtagaagcc catagagagg agacacaaaa 1560
atccctgaaa gaattccagg aaaacataaa taaacaagta gaagcccata gagaggagac 1620
acaaaaatcc ctgaaaggaa ttccaggaaa acacaatcaa acagttgaag gaattaaaaa 1680
tggaaataga agcaatcaaa aaagaacaca tggaaacaac cctggatata gaaaaccaaa 1740
agaagagaca aggagctgta gataaaagct tcaccaacag aatacaagag atggaagaga 1800
gaatctcagg agcagaagat tccatagaaa tcattgactc aactgtcaaa gataatgtaa 1860
agcggaaaaa gctactggtc caaaacatac aggaaatcca ggactcaatg agaagatcaa 1920
acctaaggat aataggtata gaagagagtg aagactccca gctcaaagga ccagtaaata 1980
tcttcaacaa aaccatagaa gaaancttcc ctaacctaaa aaaagagata cccatagaca 2040
cacaagaagc ctacagaact ccaaatagat tggaccagaa aagaaacacc tcccgtcaca 2100
taattgtcaa aacaccaaac gcacaaaata aagaaagaat attaaaaaca gtaagggaaa 2160
aaggtcaagt aacatataaa gggagaccta tcagaatcac accagacttc tcgccagaaa 2220
ctatgaaggc cagaagatcc tggactgatg ttatacagac cctaagagaa cacaaatgcc 2280
agcccaggtt actgtatcca gcaaaactct caattaacat tgatggagaa accaagacat 2340
tccatgacaa aaccaaattt acacaatatc tttccacaaa tccagcacta caaaggataa 2400
taaatggtaa agcccaacat aaggaggcaa gctataccct agaagaagca agaaactaat 2460
cgtcttggca acaaaacaaa gagaatgaaa gcacacaaac ataacctcac atccaaatat 2520
gaatataacg ggaagcaata atcactattc cttaatatct ctcaacatca atggcctcaa 2580
ctccccaata aaaagtcata gattaacaaa ctggatacac aacgaggacc ctgcattctg 2640
ctgcctacag gaaacacacc tcagagacaa agacagacac tacctcagag tgaaaggctg 2700
gaaaacaaat ttccaagcaa atggtcagaa gaagcaagct ggagtagcca ttctaatatc 2760
aaataaaatc aatttccaac taaaagtcat caaaaaagat aaggaaggac acttcatatt 2820

catcaaagga aaaatccacc aagatgaact ctcaatccta aatatctatg ccccaaatac 2880

aagggcacct acatacgtaa aagaaacctt actaaagctc aaagcacaca ttgcacctca 2940

cacaataata gtgggagatt tcaacacacc actctcatca atggacagat catggaaaca 3000

gaaattaaac agtgatgtcg acagcataag agaagtcatg agccaaatgg acttaacgga 3060

tatttttaga acattctatc ctaaagcaaa aggatatacc ttcttctcag ctcctcatgg 3120

cactttctcc aaaattgacc atataattgg tcaaaaaacg ggcctcaaca ggtacagaaa 3180

gatagaaata atcccatgcg tgctatcgga ccaccacggc ctaaaactgg tcttcaataa 3240

caataaggga agaatgccca catatacctg gaaattgaac aatgctctac tcaatgataa 3300

cctggtcaag gaagaaataa agaaagaaat taaaaacttt ttagaattta atgaaaatga 3360

aggtacaaca tacccaaact tatgggacac aatgaaagct gtgctaagag gaaaactcat 3420

agcgctgagt gcctgcaaaa agaaacagga aagagcatat gtcagcagct tgacagcaca 3480

cctaaaagct ctagaacaaa aangaagcaa atacactgag gaggagtaga aggcaggaaa 3540

taatcaaact cagagctgaa atcaaccaag tagaaacaaa aaggaccata gaaagaatca 3600

acagaaccaa aagctggttc tttgagaaaa tcaacaagat agataaaccc ttagccagac 3660

taacgagagg acacagagag tgtgtccaaa ttaacaaaat cagaaatgaa aagggagaca 3720

taactacaga ttcagaggaa attcaaaaaa tcatcagatc ttactataaa aacctatatt 3780

caataaaact tgaaaatctt caggaaatgg acaatttcct agacagatac cacgtatcga 3840

agttaaatca ggaacagata aaccagttaa acaaccccat aactcctaag gaaatagaag 3900

cagtcattaa aggtctccca accaaaaaga gtccaggtcc agacgggttt agtgcagaat 3960

tctatcaaac cttcatagaa gacctcatac caatattatc caaactattc cacaaaattg 4020

aaacagatgg agcactaccg aattccttct acgaagccac aattactctt atacctaaac 4080

cacacaaaga cacaacaaag aaagagaact tcagaccaat ttcccttatg aatatcgatg 4140

caaaaatact caataaaatt ctggcaaacg aattcaagag cacatcaaaa caatcatcca 4200

ccatgatcaa gtaggcttca tcccaggcat gcagggatgg tttaatatac ggaaaaccat 4260

caacgtgatc cattatataa acaaactgaa agaacagaac cacatgatca tttcattaga 4320

tgctgagaaa gcatttgaca aaattcaaca cccttcatga taagagtccc ggaaagaata 4380

ggaattcaag gcccatacct aaacatagta aaagccatat acagcaaacc agttgctaac 4440

attaaactaa atggagagaa acttgaagca atcccactaa aatcagggac tagacaaggc 4500

tgcccactct ctccctactt attcaatata gttcttgaag ttctagccag accaatcaga 4560

caacaaaagg agatcaaggg gatacagatc ggaaaagaag aagtcaaaat atcactattt 4620

gcagatgaca tgatagtata tttaagtgat cccaaaagtt ccaccagaga acgactaaag 4680

ctgataaaca acttcagcaa agtggctggg tataaaatta actcaaataa atcagttgcc 4740

ttcctctata caaaagagaa acaagccgag aaagaaatta gggaaacgac acccttcata 4800

atagacccaa ataatataaa gtacctcggt gtgactttaa ccaagcaagt aaaagatctg 4860

tacaataaga acttcaagac actgaggaaa gaaattgaag aagacctcag aagatggaaa 4920

gatctcccat gctcatggat tggcaggatt aatatagtaa aaatggccat tttacccaaa 4980

gcaatctaca gattcaatgc aatccccatc aaaataccaa tccaattctt caaagagtta 5040

gacagaacaa tttgcaaatt catctggaat aacaaaaaac ccaggatagc taaagctatc 5100

ctcaacaata aaaggacttc agggggaatc actatccctg aactcaagca gtattacaga 5160

gcaatagtga taaaaactgc atggtgattg gtacagagac agacagatag accaatggaa 5220

tagaactgaa gacccagaaa tgaacccaca cacctatggt cacttgattt ttgacaaagg 5280

agccaaaacc atccaatgga aaaaagatag cattttcagc aaatggtgct ggttcaactg 5340

gagggcaaca tgtagaagaa tgcagatcga tccatcctta tcaccctgta caaagcttaa 5400

gtccaagtgg atcaaggacc tccacatcaa accagacaca ctcaaactaa tagaagaaaa 5460

actagggaag catctggaac acatgagcac tggaaaaaat ttcctgaaca aaacaccaat 5520

ggcttatgct ctaagatcaa gaatcgacaa atgggatctc ataaaactgc aaagcttctg 5580

taaggcaaag gacactgtgg ttaggacaaa acggcaacca acagattggg aaaagatctt 5640

taccaatcct acaacagata gaggccttat atccaaaata tacaaagaac tcaagaagtt 5700

agaccgcagg aaacaaataa ccctattaaa aatggggttc agagctaaac aaaaattcac 5760

agctgaggaa tgccgaatgg ctgagaaaca cctaaagaaa tgttcaacat ctttagtcat 5820

aagggaaatg caaatcaaaa caaccctgag atttcacctc acagtagtga gaatggctaa 5880

gatcaaaaac tcaggtgaca gcagatgctg gcgaggatgt ggagaaagag gaacactcct 5940

ccattgttgg tgggattgca gactggtaaa accattctgg aaatcagtct ggaggttcct 6000

cagaaaattg gacattgaac tgcctgagga tccagctata cctctcttgg gcatataccc 6060

aaaagatgcc tcaacatata aaagagacac gtgctccact atgttcattg ccgccatatt 6120

tataatagcc agaaactgga aagaacccag atgcccttca acagaggaat ggatacagaa 6180

aatgtggtac atctacacaa tggaatatta ctcagctatc aaaaacaacg agtttatgaa 6240

attcgtaggc aaatggttgg aactggaaaa tatcatcctg agtgagctaa cccactcaca 6300

gaaagacata catggtatgc actcattgat aagtggctat tagcccaaat gcttgaatta 6360

ccctagatcc ctagaacaaa cgaaactcaa gacggatgat caaaatgtga atgcttcact 6420

ccttctttaa atgaggaaaa agaataccct tggctgggaa gggagaggca aagattaaaa 6480

cagagactga aggaacaccc attcagagcc tgccccacag gtggcccata catatacagc 6540

cacccaattg gacaagatgg atgaagcaaa gaagtgcaga ctgacaggag ccggatgtag 6600

atcgctcctg agagacacag ccagaataca gcaaatacag aggcgaatgc cagcagcaaa 6660

ccactgaact gagaataggt cccccgttga aggaatcaga gaaagaactg gaagagcttg 6720

aaggggctca agaccccaaa agtacaacaa tgtcaagcaa ccagagcttc cagggactaa 6780

gccactacct aaatactata catggactga ccctggactc tgaccccata ggtagcaatg 6840

aatatcctag taagagcacc agtggaaggg gaagccctgg gtcctgctaa gactggaccc 6900

ccagtgaact agactatggg gggagggcgg caatgggggg agggttggga gggggacacc 6960

cataaggaag gggagggggg agggggatgt ttacccggaa accgaaaggg aataacactt 7020

aaattatata agaaatactc aagttaatta aaaataataa taataataat aaaaagaatt 7080

actcataaaa taaagacaag atgatggata aaattaaaaa aacaaaaaaa acaaaaaaaa 7140

accaaaatgg aaacatgaaa atgctcacta cttacaatag tgctttatgt aataagcaaa 7200

tgactgcatt tgacacgagt tataatttat taacccctta gttagactac ttgtacacat 7260

ggtgattatt aatccacaat tattcttttt cactaaaatc cttatatttt attctttgag 7320

attataatat aatcacacaa attccctctt ccctgtcatc tcttcaaaac cttccatata 7380

gtcctccatg ctgtcttttg aacgttatgg cttctttttt atattcttgt tttatattat 7440

atatatataa tatatattat aatatatatc atactatata tattcctcag gactgaccaa 7500

tccatattgg ataaccaatt ggtgtgctcg tctcagggaa agaccatttc cccaccatca 7560

gcattctcgg tttccagtag ttcattgtct agaatggaga ctgtgtgtgc tttccccgtc 7620

cactttagct gctgtgtttc agctcctctt gaggcagtta tgttggtgag acctcatctg 7680

tgcggcttcc aacattcctc agagacacag tgagttacac agtgtcacct gatcctctaa 7740

ctcccaccat ctttcctgct gtcttctcca gggtttcctg aacctcagca ggagcagggg 7800

ttgtactgta gacacgccca ccttcactag gatccacagc tcctcacttt gatttgctgt 7860

ggttttctgc aatggcttcc atgtcctagt aaagtcatgg agaccccata accacgctgt 7920

actaaaccag acaatcccta gctgcattct aagttcttgt ccttatccca tacatgtgca 7980

tttagagagt ctgcttattt tgctcattat agaacgagtc aatcattctc tttgagttgt 8040

aaaagctt 8048

<210> 683

<211> 602

<212> DNA

<213> Rattus sp.

<220>

<221> misc_feature

<222> (564)..(564)

<223> n

<400> 683
gacaaggaca aaggcttgtt ttattttaat gactgatcca cgtgaggcca cagcctggcc 60

actatgtaca gacacgaggg acgcttcatt tcttggtctc ttcctccttg gacagagtct 120

tgatgatctc ctccttcttg gcttggaggc gctcctcccg acgctttcgt gcttccttgg 180

tcttagacct gcgagcctca gcctggtcag ccagtagctt cttgcgggcc ttgtctgcct 240

tcagtttgtg gatgtgctcc atgagaatcc gcttgttttt gaacacattc cctttgacct 300

tcaggtacag gctgtgatac atatgacggt caatcttctt agattcccgg tatctcctga 360

gaagccggcg caggatcctc atccttcgca tccaggtcac cttctcgggc atccgagcgt 420

tggcagtacc cttcctcttc cctatgccca tatgcctgcc cttccgtcgg gccaaggtgt 480

tcttccggca tcgagcccgg gaatggacag tcacaggctt ccggatgatc aggccatctt 540

gatcagcttc ctgatctgtt gacnaagttg gcattggcaa gttcgttggt ttcattgggg 600

tc 602

<210> 684

<211> 618

<212> DNA

<213> Rattus sp.

<400> 684
caaccacaga agagtttatt actccaatga ttcgcacagc tgggttgcag gtctaagggt 60

tgcctgcctt cctggaggca ctcccccact tccccctctg ggggaattac acttggccat 120

ggaccccata aacctctgaa tgcatttatt tagaattttg gacatctgcg ttatttccct 180

ctgaaaatgc taaccaccgt gtgctgccca ccccacggcc tgccagtcca gcctgcagag 240

ggccattcac gctgccttgt aattccgaaa taaatagaat tggtagcaag tgagtgctga 300

gtgatgtcag gatggcccca gcagcctcca gaggggcact gtggggacag tctcaaggtg 360

tcttctgatg ggcgggcact gtgcagcgtg tggcagtacc aggctgccta cagtttgtcc 420

aggaagttgt ccagcgctgg gatgccctcc ttcaggcctt tgcgcttgcg ggtctcagct 480

acaacttggc tggggcggct gctgttgtca aaggggtccc caggcaggat ctgccagtgg 540

tcaaacacac actgggggaa ggcctggcca ccagtgttgg atcgaaggtc ggcagtgaag 600

ccaaaggatt cattgaca 618

<210> 685

<211> 6335

<212> DNA

<213> Rattus norvegicus (Norway rat)

<400> 685
ggatcccggc ccgcagcagc tctctgctcc cagaacccgt gagaaagaga cctcaccgcc 60

tggtcaggtg ggcactcctg aggctgcaga gcggaagaga ccaccaacac tgcccacccc 120

tgcccacatc cctggcccaa gaggaaactg tataaggcct ctgggttccg tgggggaggg 180

cccaggagcg tcaggacccc tgcctgagac accgccggaa cctgagggaa acagaccgga 240
taaacagttc tctgcaccca aatcccatgg gagggagagc tgaaccttca gagaggcaga 300
caagccttgg aaaccagaag agactgctct ctgtacatac atctcggacg ccagaggaaa 360
acaccaaagg ccatctggaa ccctggtgca ctgaagctcc tggaaggggc ggcacaggtc 420
ttcctggttg ctgccgccac agagagccct tgggcagcac cccgcctggt gaactcaaga 480
cacaggccca caggaacagc tgaagacctg cagagaggaa aaactacacg cccgaaagca 540
gaacactctg tccccataac ggactgaaag agaggaaaac aggtctacag cactcctgac 600
acacaggctt ataggacagt ctaaccactg tcagaaatag cagaacaaag taacactaga 660
gataatctga tggtgagagg caagcgcagg aacccaagca acagaaacca agactacatg 720
gcatcatcgg agcccaattc tcccaccaaa acaaacatgg aatatccaaa cacaccagaa 780
aagcaagatc tagtttcaaa atcatatttg atcatgatgc tggaggactt caagaaagac 840
gtgaagaact cccttagaga acaagtagaa gcctacagag aggaatcgca aaaatccctg 900
aaagaattcc aggaaaacac aatcaaacag ttgaaggaat taaaaatgga aatagaagca 960
atcaagaaag aacacatgga aacaaccctg gatatagaaa accaaaggaa gagacaagga 1020
gccgtagata caagcatcac caacagaata caagagatgg aagagagaat ctcaagagca 1080
gaagattcca tagaaatcat tgactcaact gtcaaagata atgtaaagcg gaaaaagcta 1140
ctggtccaaa acatacagga aatccaggac tcaatgagaa gatcaaacct aaggataata 1200
ggtatagaag agagtgaaga ctcccagctc aaaggaccag taaatgtctt caacaaaatc 1260
atagaagaaa acttccctaa cctaaaaaaa gagataccca taggcataca agaagcctac 1320
agaactccaa atagattgga ccagaaaaga aacacctccc gtcacataat agtcaaaaca 1380
ccaaacgcac aaaataaaga aagaatatta aaagcagtaa gggaaaaagg tcaagtaaca 1440
tataaaggca gacctatcag aatcacacca gacttctcgc cagaaactat gaaggccaga 1500
agatcctgga cagatgtcat acagacccta agagaacaca aatgccagcc caggttactg 1560
tatcctgcaa aactctcaat taacatagat ggagaaacca agatattcca tgacaaaacc 1620
aaatttacac aatatctttc tacaaatcca gcactacaaa ggataataaa tggtaaagcc 1680
caacataagg aggcaagcta taccctagaa gaagcaagaa actaatcgtc ttggcaacaa 1740
aacaaagcga atgaaagcac acaaacataa cctcacatcc aaatatgaat ataacgggaa 1800
gcaataatca ctattcctta atatctctca acataaatgg ccttaactcc ccaataaaaa 1860
gacatagatt aacaaactgg atacgcaacg aggaccctgc attctgctgc ctacaggaaa 1920
cacacctcag agacaaagac agacattacc tcagagtgaa aggctggaaa acaattttcc 1980
aagcaaatgg tcagaagaag caagctggag tagccattct aatatcaaat aaaatcaatt 2040
tttaactaaa agtcatcaaa aaagataagg aaggacactt catattcatc aaaggaaaaa 2100
tccaccaaga tgaactctca atcctaaata tctatgcccc aaatacaagg gcacctacat 2160
atgtaaaaga aaccttacta aagctcaaaa cacacattgc acctcacaca ataatagtgg 2220

gagatttcaa caccccactc tcatcaatgg acagatcatg gaaacagaaa ttaaacagag 2280
atgtagacag actaagagaa gtcatgagcc aaagggactt aacggatatt tatagaacat 2340
tctatcctaa agcaaaagga tataccttct tctcagctcc tcatggtact ttctccaaaa 2400
ttgaccatat aattggtcaa aaaacgggcc tcaacaggta cagaaagata gaaataatcc 2460
catgcatgct atcggaccac cacggcctaa aactggtctt caataacaat caaggaagaa 2520
tgcccatata tacttggaaa ctgaacaatg ctctactcaa tgataacctg gtcaaggaag 2580
aaataaagaa agaaattaaa aactttttag aatttaatga aaatgaaggt acaacatacc 2640
caaacttatg ggacacaatg aaagctgtgc taagaggaaa actcatagcg ctgagtgcct 2700
gcagaaagaa acaggaaaga gcatatgtca gcagcttgac agcacaccta aaagctctag 2760
aacaaaaaga agcaaataca cccaggagga gtagaaggca ggaaataatc aaactcagag 2820
ctgaaatcaa ccaagtagaa acaaaaggac catagaaaga atcaacagaa ccaaaagttg 2880
gttctttgag aaaatcaaca agatagataa acccttagcc agactaatga gaggacacag 2940
agagtgtgtc caaattaaca aaatcagaaa tgaaaaggga gacataacaa cagattcaga 3000
ggaaattcaa aaaatcatca gatcttacta taaaaaccta tattcaacaa aacttgaaaa 3060
tcttcaggaa atggacaatt ttctagacag ataccaggta ccgaagttaa atcaggaaca 3120
gataaaccag ttaaacaacc ccataactcc taaggaaata gaagcagtca ttaaaggtct 3180
cccaaccaaa aagagcccag gtccagacgg gtttagtgca gaattctatc aaaccttcat 3240
agaagacctc ataccaatat tatccaaact attccacaaa attgaaacag atggatcact 3300
accgaatacc ttctacgaag ccacaattac tcttatacct aaaccacaca aagacacaac 3360
aaagaaagag aacttcagac caatttccct tatgaatatc gacgcaaaaa tactcaacaa 3420
aattctggca aaccgaatcc aagagcacat caaaacaatc atccaccatg accaagtagg 3480
cttcatccca ggcatgcagg gatggtttaa tatacggaaa accatcaacg tgatccatta 3540
tataaacaaa ctgaaagaac aaaaccacat gatcatttca ttagacgctg agaaagcatt 3600
tgacaaaatt caacacccct tcatgataaa agtcctggaa agaattggaa ttcaaggccc 3660
atacctgaac atagtaaaag ccatatacag caaaccagtt gctaacatta aactaaatgg 3720
agagaaactt gaagcaatcc cactaaaatc agggactaga caaggctgcc cactctctcc 3780
ctacttattc aatatagttc ttgaagttct ggccagagca atcagacaac aaaaggaggt 3840
caaggggata cagatcggaa aagaagaagt caaaatatca ctatttgcag atgatatgat 3900
agtatattta agtgatccca aacattccac cagagaacta ctaaagctga tagacaactt 3960
cagcaaagtg gctaggtata aaattaactc aaataaatca gttgccttcc tctatacaaa 4020
agagaaacaa gccgagaaag aaattaggga aacgacaccc ttcataatag acccaaataa 4080
tataaagtac ctcggtgtga ctttaacaaa gcaagtaaaa gatctgtaca ataagaactt 4140
caagacactg aagaaggaaa ttgaagaaga cctcagaaga tggaaagatc tcccgtgctc 4200
atggattggc aggattaata tagtaaaaat ggccatttta ccaaaagcaa tctacagatt 4260

caatgcaatc cccatcaaaa taccaatcca attcttcaaa gagttagaca gaacaatttg 4320

caaattcatc tggaataaca aaaaacccag gatagctaaa gctatcctca acaataaaag 4380

gacttcaggg ggaatcacta tccctgaact caagcagtat tacagagcaa tagtgataaa 4440

aactgcatgg tattggtaca gagacagaca gatagaccaa tggaatagaa ttgaagaccc 4500

agaaatgaac ccacacacct atggtcactt gatttttgac aaaggagcca aaaccatcaa 4560

atggaaaaaa gatagcattt tcagcaaatg gtgctagttc aactggaggt caacatgtag 4620

aagaatgaag atcgatccat gcttgtcacc ctgtacaagc ttaagtccaa gtggatcaag 4680

gacctccaca tcaaaccaga cacactcaaa ctaatagaag aaaaactagg gaagcatctg 4740

gaacacatgg gcactggaaa aaatttccta aacaaaacac catggcttac gctctaagat 4800

caagaatcga caaatgggat ctcataaaac tgcaaagctt ctgtaaggca aaggacactg 4860

tggttaggac aaaacggcaa ccaacagatt gggaaaatat ctttaccaat cctacaacag 4920

atagaggcct tatatccaaa atatacaaag aactcaagaa gttagaccgc agggaaacaa 4980

ataaccctat taaaaaatgg ggttcagagc taaacaaaga attcacagct gaggaatgcc 5040

aaatggctga gaaacaccta aagaaatgtt caacatcttt agtcataagg gaaatgcaaa 5100

tcaaaacaac cgtgagattt cacctcacac cagtgagaat ggctatgatc aaaaactcag 5160

gggacaacag atgctggcga ggatgtggag aaagaggaac actcctccat tgttggtggg 5220

attgcaaact ggtacaacca ttctggaaat cagtctggag gttcctcaga aaattggaca 5280

ttgaactgcc tgaggatcca gctatacctc tcttgggcat atacccaaaa gatgccccaa 5340

catataaaaa agacacgtgc tccactatgt tcattgcagc cttatttata atagccagaa 5400

gctggaaaga acccagatgc ccttcaacag aggaatggat acagaaaatg tggtacatct 5460

acacaatgga atattactca gctatcaaaa acaacgagtt tatgaaattc gtaggcaaat 5520

ggttggaact ggaaaatatc atcctgagta agctaaccca atcacagaaa gacatacatg 5580

gtatgcactc attgataagt ggctattagc ccaaatgctt gaattaccct agatacctag 5640

aacaaatgaa actcaagacg gatgatcaaa atgtgaatgc ttcactcctt ctttaaaagg 5700

ggaacaagaa tacccttggc agggaagaga gaggcaaaga ttaaaacaga gaatgaagga 5760

acacccattc agagcctgcc ccacatgtgg cccatacata tacagccacc caattagaca 5820

agatggatga agcaaagaag tgcagaccga caggagccgg atgtagatcg ctcctgagag 5880

acacagccag aatacagcaa atacagaggc gaatgccagc agcaaaccac tgaactgaga 5940

ataggacccc cgttgaagga atcagagaaa gaactggaag atcttgaagg ggctcgagac 6000

cccatatgta caacaatgct aagcaaccag agcttccagg gactaagcca ctacctaaag 6060

actatacatg gactgaccct ggactctgac ctcataggta gcaatgaata tcctagtaag 6120

agcaccagtg gaaggagaag ccctgggtcc tgctaagact gaacccccag tgaactagac 6180

tggtgggggg agggcggcaa tgggggaggg gttgggaggg gaacaccata aggaagggga 6240

gggggagggg ggatgtttgc ccggataccg aaagggaata acatcgaaat gtatataaga 6300

atactcaagt taataaaaaa aaaaaaaaaa aaaga 6335

<210> 686

<211> 744

<212> DNA

<213> Rattus rattus (black rat)

<400> 686
ctcttcctgt ctgtgccaga actgcgcgtg gtccgcgccg atcgactgag aagcccggtt 60

tgcgctctca gaatgactga atgggaaaca gccacacccg cggtggcaga gaccccggac 120

atcaagctct ttgggaaatg gagcactgat gatgtgcaga tcaacgatat ttctctacag 180

gattacattg ctgtgaagga gaagtatgcc aagtacctgc cccacagtgc aggacggtat 240

gctgccaacg gtttccgcaa agcacagtgt cccatcgtgg agcgccttac taactccatg 300

atgatgcacg gtcgtaacaa cggcaagaag ctcatgactg tacgaattgt caagcatgcc 360

tttgagatca tccacctgct cactggtgag aaccctctgc aggtcctggt gaatgctatc 420

atcaacagtg gcccccgaga agactcaaca cgcattgggc gggctggaac agtgagacgg 480

caggctgtgg atgtatcccc acttcgccga gtgaatcagg ccatctggct gctgtgcacg 540

ggggctcgtg aggctgcttt ccggaacatc aagaccatcg ctgagtgcct tgcagatgag 600

ctcattaatg ctcgcaaggg ctcctccaac tcctatgcta tcaagaagaa agatgaactg 660

gagcgtgtgg ccaagtctaa ccgctgattt cccagctgct gcctaataaa ttgtgtgccc 720

tttgggacag ttacatcaaa aaaa 744

<210> 687

<211> 444

<212> DNA

<213> Rattus norvegicus (rat)

<400> 687
aagccaagga tgagctgtag aggccacacc acctgcctcc agggctggac tgaggcctga 60

acaccccgcc acagagctgg ctgctcccaa taatgtctct atgagactca agaacttttc 120

attttttttt caggcaggtt caggtctggg gtggattctg atttttgttc ccctgcctcc 180

ccatcctccc cacccccctt tttttttact ggtgtttgtc tttaattctt cagccctcac 240

ctcctggcct ctcatttttg aatcaacatt ttttctttct gtccctttct ccatctcttg 300

gtcactatcc tccaactcta ggaacaggta tggaggaaaa gcctaggctt gagatttcat 360

ctgctctcct ttctgaatct cagaggaggg taggagaagg gggtggtatc ttccctcccc 420

ccagcactga ggaagaatgg ggct 444

<210> 688

<211> 1021

<212> DNA

<213> Rattus sp.

<400> 688
acagagttcc atcatgagaa cctcatgatc tccctgctct ctctcttctt tgctggcact 60

gagaccggca gcaccacact ccgctatggt ttcctgctga tgctcaagta cccccatgtc 120

gcagagaaag tccaaaagga gattgatcag gtgattggct ctcacaggcc accatccctt 180

gatgatcgta ccaaaatgcc atacactgat gcagtcatcc acgagattca gagatttgca 240

gatcttgccc caattggttt accacacaga gtcaccaaag acaccatgtt ccgagggtac 300

ctgctcccca agaacactga ggtgtatccc atcctgagtt cagctctcca tgacccacag 360

tactttgacc atccagacac cttcaatcct gagcacttcc tggatgccga tgggacactg 420

aaaaagagtg aagcttttat gcccttctcc acaggaaagc gcatttgtct tgacgaaggc 480

attgcccgaa atgaattgtt cctcttcttc accaccatcc tccagaactt ctctgtgtca 540

agccatttgg ctcccaagga cattgacctc acgcccaagg agagtgacat tgcaaaaata 600

cctccaacat accagatctg cttctcagct cggtgatcgg gctgaggcag ccaggtgccc 660

cagttctgtt gggaatggcc tcatgtttct gcctctgggg gacctgctga aaaccaggct 720

caaggccact gctcacatct tcctattgca gttctccaaa gtcccaaggc ttgttcttat 780

tcctgtgaat ggcactgaag aagtcaatcg actgtcttat tttgacatgt gaacagagat 840

ttcatgagta cacatctcat gctgagtcac ttccctcttc ctcctaatag cccacgtccc 900

cacttatcag ccctccatgg tctgtgatct gtgctaatgg actctgtata tggtctcagt 960

gctatgtcta cagacttaca tagtatgtat ggttcaggta aacagaatca cagagtgtgt 1020

g 1021

<210> 689

<211> 1867

<212> DNA

<213> Rattus norvegicus (Norway rat)

<400> 689
agctttgctt ctcagtggaa aagtaatttt aacaagcaat ttagtagttg attgagcaaa 60

tgcattgcaa aaaagaatgc ttttgggaca gcgttttctt cagagggagt acccagagct 120

ggcactcctg agccagtggc ccagcatcca gggagagata tgcttgtcat caccgaagcc 180

tgatgctgta gagccacacc ctggtattgg ccaatctgct cacacaggac agcgagagca 240

ggagccaggc agagcataaa aggtggggcg ggatcagtcg ctcctcacat ttgcttctga 300

catagttgtg ttgactcaca aactcagaaa cagacaccat ggtgcacctg actgatgctg 360

agaaggctgc tgttaatggc ctgtggggaa aggtgaaccc tgatgatgtt ggtggcgagg 420

ccctgggcag gttggtatcc aggttacaag gcaagtagaa gtttggtgct tggagacaga 480

ggtctgcttt ccagcaggca ctaactttta ttgtatcctg gctatgtttc cctttgtagg 540

ctgctggttg tctacccttg gacccagagg tactttgata gctttgggga cctgtcctct 600

gcctctgcta tcatgggtaa ccctaaggtg aaggcccatg gcaagaaggt gataaacgcc 660

ttcaatgatg gcctgaaaca cttggacaac ctcaagggca cctttgctca tctgagtgaa 720

ctccactgtg acaagctgca tgtggatcct gagaacttca gggtgagtct gatgggctcc 780

tcgctgggtg tccttcctgt ggctttcctg ctcaaattcc tatcagaagg aaagaggaag 840

ccactctagg gagcagtttt gatgatgatg tgtggatgtg ccctgtggag tgttgacagg 900

agtccagtta ttttatcctc tattcacaat cacttctccc tctcactcta ttattctatg 960

ttgtcatttc ctctttcttt ggtaaacttt taattttcag ttgcagtttt ttctttttaa 1020

aaaaattaat taactgactt atttactttc catcccgatc tcagcttccc ctcctcctct 1080

cttcccagtc cttctctctg ctttctatta ccctacctct ttcttttctc ctcagtccat 1140

tttctctttt ttcaatcaac tttttgtttt cttttaaata tttcttagta actttctctg 1200

aggacaagga agatatgtga gtccctgttt cttcccacag ctctaaagag tagtagcagt 1260

aattggcttt catgccagag tggaagagaa gaatatattt tacaataaat tctgtctgac 1320

atagaagaat tctcattata atttttcagt actttaagtt ggaaacgaaa acaccatttg 1380

aaatgagcct gaagtgtctg gtatttttgc tctgcaatta tgttgatggt tcttccctct 1440

tcccacagct cctgggcaat atgattgtga ttgtgttggg ccaccacctg ggcaaggaat 1500

tcaccccctg tgcacaggct gccttccaga aggtggtggc tggagtggcc agtgccctgg 1560

ctcacaagta ccactaaacc tcttttcctg ctcttgtctt tgtgcaatgg tcaattgttc 1620

ccaagagagc atctgtcagt tgttgtcaaa atgacaaaga cctttgaaaa tctgtcctac 1680

taataaaagg catttacttt cactgcaatg gtgtgttaaa ttaattgtat ctcatagaag 1740

ggttcatgct taggtttcaa ggtacaaagc agtgagggtt cagttctgac cttggggaaa 1800

taaatgattt gtgcttcatg atatatgctg ggacagtaag ctataaggca cagctcctaa 1860

tgctccc 1867

<210> 690

<211> 153

<212> DNA

<213> Rattus norvegicus (rat)

<400> 690
gccatgcccc gtgtctcaca gcattgtaac gttctccctg gtgccccctt ggcatgtatc 60

ccttgcttgg ccacctcagc ctccctcccc accatacact ctatcctgaa ctacagagga 120

acagagagca gacaaacgtc agaatgagag cag 153

<210> 691

<211> 654

<212> DNA

<213> Rattus sp.

<400> 691
aactggggac atgtgtttat tcagcagaaa gggggagccc gttggtgggg cagcgttaac 60

acaggtggat ggggagagct gatcaaataa gagggatcag atgcttggtg gaatcatggc 120

tggtggggca gccaggtgat tctctggcaa caggaaagat ggagcggcag acagacaggt 180

gggacctgaa ccatcaggcc actgcacatc ccagtcagcc acgctcaccg tctgttcagt 240

tgtcaatgca ttggtcggtg ggaacagaga aaacgatgtt gttatccttg aggcccagag 300

acttggcaaa gctgacgaat cgctccttca gttcatcgga cagccccttg gttcttccgt 360

acagggtgac tttgaagtac tgtttgtttt cagaggtctt ctggaaaaat accatggcaa 420

actggtcgta gtcagtgtcg gccacttgca catcgtagct ctgtatctga gggtagctgt 480

gaatattccc cagggtgaac tggccaggcc tggagcttgg aacgaatgtt ctgatccagt 540

agcgacagcc ctggcccctg acgaggatgg aagtgacgtt gtagctattg tcttcctgta 600

actcatagat ggtgctgtac atggtaaagc ggctttgtct ttctttctgg accg 654

<210> 692

<211> 3622

<212> DNA

<213> Rattus norvegicus (Norway rat)

<400> 692
   gctgctctga ctttctttta gtctcagcat ggagaggacc gtcttctaaa gcttcttcat 60
   aaaaacagca gtaagattat ttaaagaata gatatctgga aacaatcaga agaacaacat 120
   gggaaaatct gagaaaaggg ttgcaaccca tggggtcaga tgttttgcca agatcaagat 180
   gtttctgttg gcattaacat gtgcatatgt atccaaatca ttatcaggaa cttatatgaa 240
   ttccatgctc acacaaatag agagacaatt cggtatcccc acatctatag ttgggcttat 300
   caatgggagc tttgaaatag gaaacctttt gttgattata tttgtgagtt attttggaac 360
   aaaacttcac agacctatca tgattggtgt tggatgtgca gttatgggcc tggggtgttt 420
   cttaatctcg ctaccccatt tcctcatggg ccaatatgaa tatgaaacga ttttacctac 480
   aagcaacgtg tcctcaaaca gcttcttctg tgtggaaaac agatcccaga ccttaaatcc 540
   aacacaagac ccctcagagt gtgtgaaaga aatgaaatca ttaatgtgga tatatgtact 600
   ggtaggaaac ataatacgtg gaattggtga aactcccatc atgcccttgg gtatttccta 660
   cattgaagac tttgccaaat ctgaaaactc tcctttatac attgggattt tagaaacagg 720
   aatgaccatt ggccctttga ttggacttct gttggcttct tcctgtgcaa acatttatgt 780
   agacattgag tctgtgaata cagatgacct gaccataact cccacagata cacgctgggt 840
   cggagcttgg tggatcggct ttttggtctg tgcaggagtg aatatcctga ccagctttcc 900
   ctttttcttt tttcccaaaa cacttccaaa ggaaggatta caggagaatg tggatggaac 960
   tgaaaatgcc aaagagaaga aacacagaaa aaaggccaag gaagaaaaac gaggaatcac 1020
   taaagatttc tttgtgttca tgaagagcct ctcctgcaat ccaatttaca tgcttttcat 1080
   ccttataagt gttctccagt tcaatgcatt tatcaattca tttaccttca tgcctaagta 1140
   tctggaacag caatatggaa aatccactgc tgaggtagtc ttccttatgg gtctttatat 1200
   gttacctcca atatgcctcg gatatttaat tggtggtttg attatgaaga agttcaaggt 1260
   tactgtcaag aaagctgcac acttagcatt ctggctctgc ctgtctgagt accttctgtc 1320
   tttccttagc tatgtgatga cctgtgataa ttttccagtg gcaggcttaa caacctctta 1380
   tgaaggggtt cagcaccaac tatatgtgga gaacaaggtc cttgctgact gtaacacaag 1440
   gtgtaactgc tcaacgaaca catgggatcc agtgtgtgga gacaatggcc tggcatacat 1500
   gtcagcctgc cttgcaggct gtgagaagtc tgttggaaca ggaaccaaca tggtgtttca 1560
   gaattgcagc tgcattcagt catcgggaaa ctcatctgca gtcctgggcc tgtgtaacaa 1620
   aggccctgac tgtgccaaca agctgcagta cttcttaatc atagcaatat ttggctgttt 1680
   catatactcg ctggcaggca ttccagggta tatggttctt ctgaggtgta tcaagtctga 1740
   agagaagtca cttggagttg ggttacatgc attttgcata agaatattag ctggcattcc 1800
   tgcacccatt tactttggag ctttgataga cagaacctgt ttacattggg gaaccctgaa 1860
   atgtggtgag cccggggcat gcaggatgta tgacataaac agcttcagac gtctttacct 1920
   tggattgccg gctgcactaa gaggagcaag ctttgtcccc gccttcttca ttctaagact 1980
   tacgaggaca ttccagttcc ctggggacat tgagtcttca aaaactgatc atgcggagat 2040
   gaagctcacc ttgaaggaaa gtgagtgcac agaagtccta aggtcgaaag tgacggagga 2100
   ctgaaaacga agctgtaatg agttttctac tgccctatgc aaggccatga agagaatgta 2160
   cacttcacta gttttgaatc atgagagata caattggaac tcttaggtta tccataaggc 2220
   cgtcaaagtt acttcattca tgataaaatt atttactgat agcattttca gaaggctgac 2280
   atagtactca agattttccc agggaaaact tctatagtgg ccttcaccct taaccttaaa 2340
   gctgccttca ttttcaacca gcatgttctc ttttaactca atcaagggaa gtggatgttt 2400
   cccacacatt ctcaaatatc tttgaaactt tcctattgca gaaatatcat ttagatgttt 2460
   ttaatttata tactgatgct ggagatcaaa atatacatct tggttaagcc agattgcgtt 2520
   agtttgtttt gatttatcct ctgcatgtgc aaaacttctg catctgtctt gtgtacttag 2580
   gagtggtaac tctcttttac ttctaagatt agactcttca gagtgtgcca tctcctgttt 2640
   tcagtccctt ctatcattac ttctgtcaca cagttgatca tttcacatac atcactgaaa 2700
   actttaatca ggttgttaac cagtcatgta gcaaagatga ttgggactct ttttctctaa 2760
   caattcaaag ctggtcatga aactcttttt taaaaatcaa gagtagggga aaactagtcc 2820
   tttcaaaggc tccttgtaga gatgggctgt atctcagtgg aatagttatt acctaatgta 2880
   tgtgaggccc caggttcaac cacaacgtag ggtaaaccaa taaagtaata aaaataacgt 2940
   aagtccagat gcatcatcag atattctaaa aggctattct catattcagg gggcttcaat 3000
   ggcttagtgt tcattctatt caagggccat ggagcacata gttattaaca ttcataataa 3060
   acttagagta aaacctttaa agagggacca gatagaaagt tcgatagaaa gaactgtttg 3120
   ccaccgaacc tgaaaaggtt gttgtgatcc ttgggaccaa cgtgaaggag agaacaaact 3180
   ctcacaagtt gtgctatatc tctttttaat tgtgcatgcc ccattgcaaa tcaattaata 3240
   aaaaaagcat taaaaggttt aagaccgaca tttgctgtaa aattatagct cataaacgtg 3300
   aaagtacaca tcaaaaataa aatcaagttg tggttgtttt aatgagaaat atccctccta 3360
   ggcataggca tttggatatt tggtttctaa tgaatgactc tgcttaggga agattggatg 3420
   tgcatcccta agacaaaagg tgaatcactg agatgggttg aaagttaaaa gcctcaccta 3480
   cttccagtac actctctgct ttgtgctttg gttgatgata tgaaatcatg gtttcctgct 3540
   ccagccacca tgcctggtgc ttgccttcat gaacttccat ccctggagtc atgcgttaaa 3600
   ataaactcct ttttttaatg tg 3622

<210> 693

<211> 1884

<212> DNA

<213> Rattus norvegicus (Norway rat)

<400> 693
   caagcctttg ctggagaccg ctcctgtcca gtccgcagct ggcttcagcg ccactcagga 60
   caccggaaag atggcaccga ttgccggcaa gaaggccaag aggggaatct tagaacgctt 120
   aaatgctggc gaagtcgtga tcggagatgg gggatttgtc tttgcactgg aaaagagggg 180
   ctacgtaaag gctggaccct ggaccccaga ggctgcggtg gagcaccccg aggcagttcg 240
   gcagcttcat cgggagttcc tcagagctgg atcgaacgtc atgcagacct tcactttcta 300
   tgcaagtgag gacaagctgg aaaaccgagg gaactacgtg gcagagaaga tatctgggca 360
   gaaggtcaat gaagctgctt gtgacattgc acggcaagtt gctgacgaag gggatgcatt 420
   ggttgcagga ggtgtgagtc agacaccttc ctacctcagc tgcaagagtg agacggaagt 480
   taaaaagata tttcaccaac agcttgaggt cttcatgaag aagaatgtgg acttcctcat 540
   tgcagagtat tttgaacatg ttgaagaagc cgtgtgggca gtcgaggcct taaaaacatc 600
   cgggaagcct atagcggcta ccatgtgcat cggacctgaa ggagatctac atggcgtgtc 660
   tcctggagag tgcgcagtgc gtttggtaaa agcaggtgcc gccattgtcg gtgtgaactg 720
   ccacttcgac cccagcacca gcttgcagac aataaagctc atgaaggagg gtctggaagc 780
   agctcggctg aaggcttact tgatgagcca cgccctggcc taccacaccc ctgactgtgg 840
   caaacaggga tttattgatc tcccagaatt cccctttgga ttggaaccca gagttgccac 900
   cagatgggat attcaaaaat acgccagaga ggcctacaac ctgggggtca ggtacattgg 960
   cggctgctgc ggatttgagc cctaccacat cagggccatt gcagaggagc tcgccccaga 1020
   aaggggattt ttaccaccag cttcagaaaa acatggcagc tggggaagtg gtttggacat 1080
   gcacaccaaa ccctggatca gggcaagggc caggaaagaa tactggcaga atcttcgaat 1140
   agcttcgggc agaccgtaca atccttcgat gtccaagccg gatgcttggg gagtgacgaa 1200
   aggggcagca gagctgatgc agcagaagga agccaccact gagcagcagc tgagagcgct 1260
   cttcgaaaaa caaaaattca aatccgcaca gtagccacag gccagcggtt cggggcgaat 1320
   tcctccaggt ccgggccaca gtgtgcaccc ggaaggagaa ggcatctcta aaccagcgtt 1380
   tgtgttgatg ccggcttaca cctgtgattg gtgctagtta gacaaaatgg agtcacagat 1440
   agcatttcac agttacaaaa ctacgcttta gaattttacc tagaaggaag aaaggagaag 1500
   tccacagtaa atcctgaaca catttcctac gtgcctgtcg cattacaggc gcacaggagt 1560
   cactgcagcg aagagaaagt cacccgacgt caatctcatt tcagataggg ggataggaca 1620
   ccacctccac gagtgacata gaaccattca gggaccgtat cataagtgac acagcaacca 1680
   tctatatcta agatgcttcc caagtggatt ccaagatctt ttgagcagga cccttaggca 1740
   gaaacaacac acaccagccc tgtaaaactt aacagataac tgatccattc tgtaattctg 1800
   taatctctgt tctgactgct tccattccat ttcattaata aaaacatgcc ggttgaaaac 1860
   cttcaaaaaa aaaaaaaaaa aaaa 1884

<210> 694

<211> 4198

<212> DNA

<213> Rattus norvegicus (Norway rat)

<400> 694
gacgcgctga gccgctgccg ccgcgggggg gaagcctgcg agtagctcgc gtgcccgcag 60

tccgcgtaag ccccgcgccc cttccacccc tggccaggcc atggcggaac gtggaggggc 120

gggcggtggt cccggaggcg ccgggggcgg cagcagtcag cggggctctg ggttgcccca 180

gtccccgcag cagcagccgc cgcagcagcc atctcagccg caacagccaa cgccccccaa 240

actggcccag gccacctcgt cctcctcgtc cacctcggct gcggccgcct cctcctcgtc 300

ctcctccacc tccacctcca tggccgtcgc ggtggcctcg ggctctgcgc ctcccggcgg 360

tccgggacca ggccgcaccc ccgccccagt gcagatgaat ttgtatgcta cctgggaggt 420

ggaccggagc tcatccagct gcgtgcccag actcttcagt ttgactctga agaaacttgt 480

tatgcttaaa gaaatggaca aagatcttaa ctcagtagtc attgctgtga agctgcaggg 540

ttcaaaaagg attcttcgct ctaatgagat catccttcca gccagtggcc tggtggaaac 600

agagctccag ttaaccttct ccctccagta ccctcatttc cttaagcgag atgccaacaa 660

actgcagatt atgttgcaaa ggaggaagcg gtacaagaac cggactatct tgggctataa 720

aaccttggct gtgggactca tcaacatggc agaggtgatg caacacccta acgagggtgc 780

tttagtactt ggcctgcata gcaacgtaaa ggatgtctct gtgcctgtgg cagaaataaa 840

aatctactct ttatccagcc agcccattga ccatgaagga atcaagtcta agctgtctga 900

tcgctcccca gatattgaca actactctga agaagaggag gaaagcttct cctcagagca 960

ggaaggcagt gatgacccat tacatggaca ggacctattc tatgaagatg aagatcttcg 1020

gaaagtaaag aagactcgaa ggaaactgac ctcaacctca gccatcacta ggcaaccgaa 1080

catcaaacag aagtttgtgg ccctcctgaa acgattcaaa gtttctgatg aggtgggctt 1140

tgggctagaa catgtgtcaa gggagcagat tcgggaagtg gaagaggacc tggatgaact 1200

atacgacagt ctagaaatgt acaaccccag cgacagtggc cctgagatgg aagaaacaga 1260

aagcatcctc agcaccccaa agcccaagct caagcccttc tttgagggga tgtctcagtc 1320

cagctcacag acggagatcg gcagtctcaa cagcaagggc agccttggaa aagacaccac 1380

cagccctatg gaattggcag ctttagaaaa agttaagtct acttggatta aaaaccaaga 1440

tgacagcttg actgaaacag acactttaga aatcactgac caggacatgt ttggagatgc 1500

gagcacaagt ctggttgtac cagaaaaagt caaaactcct atgaagtcca gcaaagctga 1560

tctgcagggc tccgcctcgc cgagcaaggt ggaagggaca cacacacctc gccaaaagag 1620

gagcactccc ctgaaggagc ggcagctctc caagcccctg agtgagagga ccaacagctc 1680

tgacagtgag cgctctcctg acctagggca cagcacacag attccacgaa aggtggtata 1740

cgatcaactg aatcagatcc tggtttccga tgcagcgctc cctgagaatg tcatcctggt 1800

gaacaccacg gactggcagg gccagtatgt ggccgaattg cttcaagacc agaggaagcc 1860

tgttgtgtgt acctgctcaa cagtggaggt ccaggccgtg ttgtctgccc tgctcactag 1920

gattcagcgc tactgcaact gcaactcatc tatgccgagg ccagtcaagg tggcagctgt 1980

gggcagccag agttacctga gctccatcct tcggtttttt gtcaagtccc ttgccagcaa 2040

gaccccggac tggctgggcc acatgcgctt cctcatcgtt cccctaggtt ctcatccagt 2100

ggccaaatac ttgggctcag tagatagtag atatagcagc acctttcttg attctgcttg 2160

gagagacctg ttcagtcgct cagagcctcc agtgtcagag ccactggatg tggtgggtcg 2220

tgtgatgcag tatgtcaatg gagctaccac aacgcaccag ctgcctgtgg ctgaagccat 2280

gttgacctgc aggcataagt tccctgatga agactcctac cagaagttta tccccttcat 2340

tggtgttgtg aaagtgggcc ttgttgaaga ctcgccctcc actgcaggtg atggggacga 2400

ttcacctgtg gtcagcctta ctgtgccctc cacatcacca ccctcaagct cgggcctgag 2460

ccgagatgcc acagctaccc ctccctcatc cccatccatg agcagtgccc tcgccatcgt 2520

ggggagccca aatagccctt atggggatgt gattggcctc caggtggatt actggctggg 2580

ccaccctggg gagcgaagga gagaaggtga taagagggac gccagttcaa agaataccct 2640

caagagtgtc ttccgctcag tgcaggtttc tcgcctgccc catgcagggg aagcccagct 2700

ttctggcacc atggcaatga ctgtggtcac caaagagaag aacaagaaag tgcccactat 2760

cttcctgagc aagaagcccc gggaaaagga ggtggattct aagagccaag tcatcgaggg 2820

catcagccgc ctcatctgtt ctgcaaagca gcagcaaacc atgctgagag tgtccattga 2880

tggagttgaa tggagtgaca tcaagttctt ccagctggca gctcagtggc ccactcatgt 2940

caagcacttc ccagtgggac tcttcagtgg cagcaaaccc acctgagacc tgcctcccaa 3000

ccactccctc ctggcactgc cacccagccc agcctccttt cctagcactg gggcttgcct 3060

ccacttgccc agtcctgaag gacactgcca ctggggatgc agtcctccct gctcactgcc 3120

cagccatctc tgcccagctc ccccctgttc caggcccaga gctgggtgat cctgccttct 3180

ggtgcccata attcctgtaa ttgaataatc tgggccttcc ttcacctcac ccctgcagcg 3240

ctggtaccct ttctagaaat gaaacacctg gagttctggt acacagggag acagcccagc 3300

atcctacata agctggccac ccagctctgc atttctaagg ctctgttgcc tgttgtactc 3360

accctgcctt cctccagcag ggctggggcc tcctgccatt cctcacctgc ctcacagttt 3420

cccagtaggg ggaaggagga tggcacctgg gcatcctgga gtttctggga ttgaagggac 3480

cttcccagtc tcccatgcat gatttcctga cacttcaagt cgcttggatg tcctgtgttt 3540

tgtgtgtttt tagttgtttg ggtaacttgt cctgtgcaca ggtccttaag gctccttctc 3600

cctctgcctc ccagcctcct acctaacatt gctctgtggc ttcatgaggt aggagacagc 3660

aaacccaccc acccacccac cccttcaccc ctcaacacat acatcatctg tggctctcat 3720

ccagtcatat tgtccccctc tcctgtgtca gcttgcctcc tggctctgct ctgtcatccc 3780

acttccttca tggcagggca gcagaatgag gcccttctcc accaggtctc cctccagcag 3840

cctccttccc tctctcagcc atgaatgggt cccagaccta cctcagcctc cttatcagtg 3900

tcttcatgag aggctgagga caagggacag atctggccca gcctccatgc cctctgtgtc 3960

ctatggcacc tcagagcttc tccgttgccc ctcagtgttt cctagcagtg tggcacggaa 4020

ctgcacttgg aacctagttt tggtgttctt cacatccaaa actcttaagc ccctcccgct 4080

gcccagcctg tccagagtat ctcctgggcc tgagaataag tggggtggga agactggggc 4140

catgttgggt tttttcattc aataaactgg tgatttctta ccaaaaaaaa aaaaaaaa 4198

<210> 695

<211> 2051

<212>  DNA

<213>  Rattus norvegicus (Norway rat)

<400>  695
tggcaaccct gaacatcagg agtcagcaat cacagaggca ggcagctggc tggtatcgct      60

ctgcagcctg aagactggag aagatgaccc gccttgtgac tctggagctc ttgatggctg     120

ggatcggctc tgctctcctc tgcttcccag attgcatact gggagaggac actctattcc     180

atgaagacca agacaagggg acacaactgg acagtctcac attggcctcc atcaatactg     240

actttgcctt cagcctctac aagaagctgg ctttgaggaa tccagataaa aatgttgtct     300

tctccccact tagcatctca gccgccttgg ccgtcgtgtc cctgggagca aagggcaaca     360

gcatggaaga gattctagaa ggtctcaagt tcaatctcac agagacccct gagacagaaa     420

tccaccgggg ctttggacac ctcctccaga ggctcagcca gccaagggac gagatacaga     480

tcagtacagg caatgccctg tttattgaaa aacgccttca ggtcctggca gagttccagg     540

agaaggcaaa ggctctgtac caagctgagg ccttcacagc tgatttccag cagtctcgtg     600

aggccaaaaa gctcatcaat gactatgtga gtaaacagac ccaggggaag atccagggac     660

tgatcacaaa cctagctaag aagacatcca tggtactggt gaattacatc tactttaaag     720

gcaaatggaa ggtgcctttt gaccctcggg acacattcca gtctgagttc tactctggca     780

aaaggaggcc tgtgaaagtg cccatgatga agcttgagga cctgaccaca ccctacgtcc     840

gggatgagga gctgaactgc actgttgtgg agctgaagta cacaggaaat gccagcgccc     900

tgtttatcct ccctgaccag ggcaagatgc agcaggtgga agccagcttg caaccagaga     960

ccctgaggag atggaaggac tctctcaggc ccagcatgat agatgagctc tacctgccca    1020

agttctccat ctctgctgac tacaacctgg aggacgtcct tccagagctg ggcatcaaag    1080

aagtcttctc cacacaggct gacctgtctg ggatcacagg ggataaggac ctgatggtct    1140

ctcaggtggt ccacaaggct gttctggatg tggctgagac aggcacagaa gcagccgctg    1200

ccacaggggt caaatttgtt ccaatgtctg caaaactgga ccctctgatt atagctttcg    1260

accggccttt cctgatgatt atctctgaca cagaaactgc aatagctccc tttttggcca    1320

agatatttaa ccccaaatga gattcgaact tcccaagagt tgatcgttct cctgaggcat    1380

tgagcctgtc tgtgggtctc tgtgtgcatt tttggcttct atgctctgat tggccatggc    1440

ggcatgcctg gatgagacag taactaactg tgtaacagcc tcatgtacag acgcctgtgc    1500

agagtcgctg ccatgctccc aaacttcttg gtaccactag ctcatatttc tgagcctaaa    1560

atttgtcttt cccctgccct tgctctctct cccctgtatc tgcctcaacc cagaagccag    1620

ggccccatca ggttgtctca gtcccttctt aggccttagt tatatcttcc ttcagcgttg    1680

ctgtcttgat gggactgtgc acgattaccg gccaacccac atggaccaag aagaacactt    1740

gctggtccgt atctttctgc agtatgtggg atcacttggt gcccagtgct gcctcactat    1800

ttccttcctc tgggcactgc tccttgcagc atggcctgac cttgtccaca tctggcacag    1860

agctggagcc ctcccttctg cagatgcatg gcacctgtgg gtcagaccag atccccctcc 1920

ccagcactcc tacttagagc aatgcagcct ttcttttagt tcccagctga ccaacctcac 1980

acaaaagatg accaacaaca accaaaatga agaggtagga gcaaaggatc aataaacaca 2040

tcactgcatt g 2051

<210> 696

<211> 1003

<212> DNA

<213> Rattus norvegicus (Norway rat)

<400> 696
tgggcaccat cagcagagag attgtcccga cagagaggca attctattcc ctaccaacat 60

gaagctgttg ctgctgctgc tgtgtctggg cctgaccctg gtctgtggcc atgcagaaga 120

agctagtttc gagagaggga acctcgatgt ggacaagctc aatggggatt ggttttctat 180

tgtcgtggcc tctgataaaa gagaaaagat agaagagaac ggcagcatga gagtttttgt 240

gcagcacatc gatgtcttgg agaattcctt aggcttcacg ttccgtatta aggaaaatgg 300

agtgtgcaca gaattttctt tggttgccga caaaacagca aaggatggcg aatattttgt 360

tgagtatgac ggagaaaata catttactat actgaagaca gactatgaca attatgtcat 420

gtttcatctc gttaatgtca acaacgggga aacattccag ctgatggagc tctacggcag 480

aacaaaggat ctgagttcag acatcaagga aaagtttgca aaactatgtg tggcacatgg 540

aatcactagg gacaatatca ttgacctaac caagactgat cgctgtctcc aggcccgagg 600

ttgaagaaag gcctgagcct ccagattgca gggcaagatc tatttcttca tcctttgttc 660

tatacaatag agtgcctctc tgtccagaag tcaatccaag aagtgcttaa tgggttcctt 720

tattctttct tcctggatta ctccgtgctg agtggagact tctcaccagg actccagcat 780

taccatttcc tgtccatgga gcatcctgag acaaattctg cgatctgatt tccatcctgt 840

ctcacagaaa agtgcaatcc tggtctctcc agcatcttcc ctagttaccc aggacaacac 900

atcgagaatt aaaagctttc ttaaatttct ctttgcccca ctcatgatca ttccgcacaa 960

atttcttgct cttgcagtgc aataaatgat tacccttgca ctt 1003

<210> 697

<211> 924

<212> DNA

<213> Rattus norvegicus (Norway rat)

<400> 697
cctggtggtt ccgcagccgt actctcctgg cctggtgtgc acagcctcac catgagttct 60

tctgcagcca gcccgctttt cgcgcctggc gaggactgcg gccccgcgtg gcgcgcggcc 120

cccgcggcct atgatacgtc tgacacgcac ctgcagatcc tgggcaagcc agtaatggag 180

cgttgggaga ccccctacat gcattcgctg gcggctgctg ctgcctccag agggggccgg 240

gtcctggaag tgggctttgg gatggccatt gcagcctcca gggtgcagca ggcccccata 300

aaggaacact ggattattga atgcaacgat ggggtcttcc agcgtctaca aaactgggcc 360

ctgaagcagc cacataaggt tgttcccttg aaaggcctgt gggaggagga ggcacctaca 420

ctgcctgatg gtcactttga tgggattcta tacgacacat atccactgtc tgaagagacc 480

tggcacactc accagttcaa ctttattaag actcatgctt tccgtttgct gaagcctggg 540

ggtatcctca cttactgcaa cctcacgtcc tggggggaac tcatgaagtc caagtacaca 600

gacatcactg ccatgtttga ggagactcag gtgcctgcac tgctggaagc tggcttccag 660

agagaaaaca tctgtacaga ggtgatggcg ctggtgcccc cagccgactg ccgctactat 720

gccttccctc agatgatcac acccctggtc accaagcact gagcggctgg cccagggcta 780

caaggagaat atgtcctcct cagtgccttt gtagctggag tgtggctcca gcctctccac 840

tatccctgca gtgtgacatc ctaacctctg cctggtacgg ccatctcccc agagctcagg 900

agtaaaataa atgctaccaa gact 924

<210> 698

<211> 959

<212> DNA

<213> Rattus norvegicus (Norway rat)

<400> 698
agagggagca gctttttaac aagagaactc aagcaattgc tgccatgccg gggaagccag 60

tccttcacta cttcgatggc agggggagaa tggagcccat ccggtggctc ctggctgcag 120

ctggagtaga gtttgaagaa caatttctga aaactcggga tgacctggcc aggctaagga 180

atgatgggag tttgatgttc cagcaagtgc ccatggtgga gattgatggg atgaagctgg 240

tgcagaccag agccattctc aactacattg ccaccaaata caacctctat gggaaggaca 300

tgaaggagag agccctcatc gacatgtatg cagaaggagt ggcggatctg gatgaaatag 360

ttctccatta cccttacatt ccccctgggg agaaagaggc aagtcttgcc aaaatcaagg 420

acaaagcaag gaaccgttac tttcctgcct ttgaaaaggt gttgaagagc catggacaag 480

attatctcgt tggcaatagg ctgagcaggg ctgatgttta cctagttcaa gttctctacc 540

atgtggaaga gctggacccc agcgctttgg ccaacttccc tctgctgaag gccctgagaa 600

ccagagtcag caacctcccc acagtgaaga agtttcttca gcctggcagc cagaggaagc 660

cattagagga tgagaaatgt gtagaatctg cagttaagat cttcagttaa ttcaggcatc 720

tatggataca ctgtacccac aaagccagcc ttcgaaagct ttgcaacaat cgcatatttt 780

gactaaatgt tgaccctact tattgggagg ccaacacgtt ttctaatgct tctgtgttaa 840

ttcatataga catgactgat gaggaattgc tgggatgcta tttggttgta gttaaaattt 900

gaaatcatga tcacttcctc agatattact ttgaatctca ataaaaactt cgcaagctt 959

<210> 699

<211> 2638

<212> DNA

<213> Rattus norvegicus (Norway rat)

<400> 699
cacaatcttc aatgagtaga catattcctc agttctgtgg tgttctcggt cacacattta 60

tggagtttct gaagggcagt ggagactact gccaggcaca gcacgacctc tatgcagaca 120

agtgaactgt agaaattcat tactactcca ccaagaagcc cccataagag tggatagcct 180

ggacacagtc gtgttgaatt gaaatctgca gagcattttc caagagctca gacctggatg 240

gggtaaacct cagtgcactt cctctgtatc gcctcagtat tcctggattg aagagtcact 300

gcttcttgtg aggaggttca tttcattgcc cgtttctccc gactcatact caaagcactg 360

agaatttcaa gtggagtata ttgaatattg aagtagactt caggttgttt tttggttttg 420

ttttggtttt ttgttttgtt ttgttttgtt ttgttttggt tttcagtttt tgtttggaat 480

catttctgta ttcaattttt taattctttc ataaccctat tgggtgtttt tttaaactaa 540

attaacatgg ctcgaatgaa ccgccctgct cctgtggaag tcacatacaa gaacatgaga 600

tttcttatta cacacaatcc aaccaatgcg accttaaaca aatttataga ggaacttaag 660

aagtatggag ttaccacaat agtaagagta tgcgaagcaa cttacgacac tactcttgtg 720

gagaaagaag gcattcatgt tcttgactgg ccttttgatg atggtgcacc accatccaac 780

cagattgttg atgactggtt aagtcttgtg aagattaagt ttcgtgaaga acctggttgc 840

tgtattgctg tccattgtgt cgcaggcctt ggcagagctc cggtgcttgt tgccctagca 900

ttaattgaag gtggaatgaa atatgaagat gcagtacaat tcataagaca aaagcggcgc 960

ggagctttta acagcaagca acttctgtac ctggagaagt accgtcctaa aatgcggctc 1020

cgcttcaagg attccaacgg tcatagaaac aactgttgta ttcaataaaa ctggggtgcc 1080

tgatgccatt gccttggaag aggaacttca gatggacctt gatttgttat ttacccaatg 1140

tgtccactta cctgtggaag ctccagggga atattgaaaa agttttacca ggccacaagc 1200

ttgacagaat tgcaacctct ataattgggc tatgatcaac acgtttggac acttagcaaa 1260

agatttttgc tggtcagcat ttaaaatgtg cttattattt gtaccaattg acctttccta 1320

aaataaggta ttgagtaatg tcattaaatg tactcctgtg ccagaatatt attagtctat 1380

aaggaattta gaaggattag gtgccaaaat acccagcaca atacttgtat atttttagca 1440

tcatacagaa ccaaaattcc aagaactaag aactctccag accttccatg gtgtattcct 1500

tcagtcattt caaacaccgc agggcttctc ttgttatctg cctgctcact ctatgtttac 1560

atctcccaca cttacaccag aacacatcag gtttgcttag ctatctttta agtcttgcaa 1620

tgattattta atgtctctgt cttattttgt gctgttttgg gaaacctcca tttgaaaatc 1680

aactttgtta cagaagcaca tatcttcaat aatgtctcca gacaaaaagc cttatagtta 1740

atttaatgtt tgcactcggg tgcaacctga cagggagggc ctgaacaaga aaggagagga 1800

ggctattaaa tatttttagt aatatgttgc ctttgtcttg tgcagaacat gtagagtatg 1860

ctctttaatt tagtaaatat ttttaagacg tagagataca ttgttgtagc taaccactta 1920

atcaaaattt ctgaaattct tgtgttttcc atacctatct gaggttttcc aacttgtttg 1980

aattatggtt ttccccttct cttcccaatc tcttgcaaaa aagtaaaagt gggatctgct 2040

agtgaactga gcagaaatat tttatacgcc ttttgagcta tgtaacttaa taattggata 2100

cttgatcatt tgttttatta tgtaatcgat aaaatggtga tgtgtattaa tgttagttca 2160

accatatatt tatactgtct gggaatgtgt ggttatagtt ctgtgggaga aatagtttgt 2220

cagtgttcac cagcttgtaa aaacttagtg cgagagcttc aacatctaaa taaatgatga 2280

aacgcattcg tcactgaggt cactttgctt aaaattaact taatttgtag aaaacagtgg 2340

attcaattat tatcatttca gtttatggac aaatttgtta gggttaccaa gtgcgtttaa 2400

aaattgctct ttaaaggtct agataattgt gaatcaattg aatgttgggt accaagggaa 2460

aacggtttgt aatagttgat gaccttgatt tttaattcaa ttccaccagt cacttgtagc 2520

tttatgcagt ttccaatcca cttttctcat ttttaagttt attacttacc tgtatatatt 2580

ttgaaattaa tttgaacctg cgtatttggc acatgatggc ttataaattt taactttc 2638

<210> 700

<211> 2495

<212> DNA

<213> Rattus norvegicus (Norway rat)

<400> 700
attgcctacc ccgggtggag accgtgctcg tccggccctc ttgcctcacg ttctgcagct 60

ctgcagctcc gcaatcctac accatggcgg acagccggga cccagccagc gaccagatga 120

agcagtggaa ggagcagcgg gcccctcaga aacccgatgt cctgaccacc ggaggcggga 180

acccaatagg agataaactt aatatcatga ctgcggggcc ccgagggccc ctcctcgttc 240

aagatgtggt tttcaccgac gagatggcac actttgacag agagcggatt cctgagagag 300

tggtacatgc aaagggagca ggtgcttttg gatactttga ggtcacccac gatattacca 360

gatactccaa ggcaaaggtg tttgagcata ttgggaagag gactcctatt gccgtccgat 420

tctccacagt cgctggagag tcaggctcag ctgacacagt tcgtgaccct cgtgggtttg 480

cagtgaaatt ctacactgaa gatggtaact gggacctcgt gggaaacaac acccctattt 540

tcttcatcag ggatgccatg ttgtttccat cctttatcca tagccagaag agaaacccac 600

aaactcacct gaaggaccct gacatggtct gggacttctg gagtctttgt ccagagtctc 660

tccatcaggt tactttcttg ttcagcgacc gagggattcc agatggacat cggcacatga 720

atggctatgg ctcacacacc ttcaagctgg ttaatgcgaa tggagaggca gtgtactgca 780

agttccatta caagactgac cagggcatca aaaacttgcc tgttgaagag gcaggaagac 840

ttgcacagga agacccggat tatggcctcc gagatctttt caatgccatc gccagtggca 900

attacccatc ctggactttt tacatccagg tcatgacttt caaggaggca gaaaccttcc 960

catttaatcc atttgacctg accaaggttt ggcctcacaa ggactaccct cttataccag 1020

ttggcaaact ggtcttaaac agaaatcctg ctaattattt tgctgaagtt gaacagatgg 1080

cttttgaccc aagcaacatg ccccctggca ttgagcccag cccggacaag atgctccagg 1140

gccgcctttt tgcttaccca gacactcacc gccaccgcct gggaccaaac tatctgcaga 1200

tacctgtgaa ctgtccctac cgtgctcgcg tggccaacta ccagcgcgat ggccccatgt 1260

gcatgcatga caaccagggt ggtgctccca actactaccc caacagcttc agcgcaccag 1320

agcagcaggg ctcggccctg gagcaccata gccagtgctc tgcagatgtg aagcgcttca 1380

acagtgctaa tgaagacaac gtcactcagg tgcggacatt ctatacgaag gtgttgaatg 1440

aggaggagag gaaacgcctg tgtgagaaca ttgccaacca cctgaaagat gctcagcttt 1500

tcattcagag gaaagcggtc aagaatttca ctgacgtcca ccctgactac ggggcccgag 1560

tccaggctct tctggaccag tacaactccc agaagcctaa gaatgcaatt cacacctacg 1620

tacaggccgg ctctcacata gctgccaagg gaaaagctaa cctgtaaagc acgggtgctc 1680

agcctcctca gcctgcactg aggagatccc tcatgaagca gggcacaagc ctcaccagta 1740

atcatcgctg gatggagtct cccctgctga agcgcagact cacgctgacg tctttaaaac 1800

gataatccaa gcttctagag tgaatgatag ccatgctttt gatgacattt cccgaggggg 1860

aaattaaaga ttagggctta gcaatcactt aacagaaaca tggatctgct taggacttct 1920

gtttggatta ttcatttaaa atgattacaa gaaaggtttt ctagccagaa acatgatttg 1980

attagatatg atatatgata aaatcttggt gattttacta tagtcttatg ttacctcaca 2040

gcctggtata tatacaacac acacacacac acacacacac acacaccaaa acacacatac 2100

actatacaca cacacacaca cacacactaa aacacacata cacaacacac acatacacta 2160

cacacacaga acacacaaca caaacataca cacataggca cacacacaca cacacacaca 2220

cacacacaca cacacacaca cacacatgaa tgaagggatt ataaagatgg cccacccaga 2280

attttttttt atttttctaa ggtccttata agaaaaacca tacttggatc atgtcttcca 2340

aaaataactt tagcactgtt gaaacttaat gtttattcct gtgtagttga ttggattcct 2400

tttccccttg aaattatgtt tatgctgata cacagtgatt tcacataggg tgatttgtat 2460

ttgcttacat ttttacaata aaatgatctt catgg 2495

<210>  701

<211>  1292

<212>  DNA

<213>  Rattus norvegicus (Norway rat)

<400>  701
cgtagacttc ttccctcctg cagcgaagga cagcatgagc acagctggaa aagtaatcaa      60

atgcaaagcg gccgtgctat gggagcctca caagcccttc accatcgagg acatagaagt     120

cgcacccccc aaggcccatg aagttcgcat taagatggtg gccaccggag tctgccgctc     180

agatgatcac gcggttagtg gatccctgtt cacgcctctt cctgcagttc taggccacga     240

gggagctggc attgttgaga gcattggaga aggggtgact tgtgtgaaac caggtgataa     300

agtcatcccg ctgttctctc cccagtgtgg aaaatgcagg atctgcaagc acccggaaag     360

caacctctgt tgccaaacta agaatctgac acagcctaag ggagctttgc tcgacggcac     420

cagcagattc tcctgcaggg gaaagcccat tcaccacttt ctcagcacca gcaccttctc     480

ccagtacact gtggtagatg acatagcggt ggccaaaatc gatgcggctg caccactgga     540

caaagtctgc ctcatcggct gtggcttctc gactggctat ggctctgccg tccaagtcgc     600

caaggtgacc ccaggctcca cctgtgccgt gtttggcctg ggaggtgttg gtctgtctgt     660

cgtcattggc tgtaaaacag caggagcagc caagatcatt gccgtggaca tcaacaaaga     720

caagtttgcg aaggccaaag agttaggtgc cactgactgt atcaaccctc aagactacac     780

caaacccatc caggaagttc tccaggagat gactgatgga ggggtggact tttcatttga     840

agtcattggc cgtcttgaca ccatgacttc tgccctgtta agctgccatt cagcatgcgg     900

tgtaagcgtc attgtcgggg tgcctcccag tgcccaaagc ctctccgtta accccatgtc     960

gctgctgctg ggacgcacct ggaaaggagc aatattcggc gggtttaaga gtaaagatgc    1020

cgtccccaaa cttgtcgctg acttcatggc taagaagttt ccgttggagc cgctgattac    1080

tcatgtttta ccttttgaaa agataaatga agcatttgac ctgctccgtg ctggaaagag    1140

tatccgtacc gtcctgacgt tctgagaaca catgaatgcc ttcccggttt ctgactcctc    1200

cgcctaccga atcaagcgcc agccacatca caaccttaac cttgctcttc agagacacac    1260

agccaataaa gcacctgtgc aagctttccg tg                                  1292

<210>  702

<211>  505

<212>  DNA

<213>  Rattus sp.

<400>  702
gacagtctga agcttttatt cagtgggtct ctgtgtctag aagaaggtgt ttggcctctt      60

ggtggtgaag cgtggtttgt gctggcgccg caacacacgg tggggcaatg ggaacttgat 120

cttggagtcg tggaactgct tgacagctgg ccggcggcac ttgccagctg caatctcttc 180

caccttcatg atctggatgg agtgcgcacg ggcacggtgt cgggcaccca tgtctcggta 240

gcactgtgtg accgcgccgg cagtggtcag gtcccggtac tctcggtaca tgttgtgagt 300

gccacttcgg gaatcatagc gcagccagat gccgaagttc ttcacacgca ggggtgactt 360

ctcaaacacc tgcccacagt acacaatttc cccggatgac ttcttcatct tcttcagctg 420

cgacacaaag taccagaagc gggacttggc caccacatgg ttgggtgcag attcgcattc 480

ggtacagtgg cggtgtgtgg cattt 505

<210> 703

<211> 400

<212> DNA

<213> Rattus norvegicus (Norway rat)

<400> 703
tttttttttt tttttatgtt cagatgcatt tattcagcaa accttcttgc taaacattat 60

ggtagcccta aaagatttcg aaatcaggac attcagcaaa gccctctcct ctgaatgcgg 120

atagatgttc caaatttcct gttttcaaag catagaatgc attcattggc gtaagttttt 180

ccgtcagtac cacacacagg atcataatcc ctggggcatc caacaagtgt attagggcaa 240

ttagcctttt tcccaatcac cttagctgta gtgttacctg ctaaattgag cagggccaaa 300

gcactgagaa gaaagataat tgctaccttc atgggtgtag acctaggtcc aaaactcaga 360

agaactgtgc agggtgcttc tcttcaacct cgtgccgaaa 400

<210> 704

<211> 635

<212> DNA

<213> Rattus sp.

<400> 704
aacatttcac taacagaact aaactttatt tccatgtttg ttacatgttt gtcttggatg 60

ctgagtacat attagaattg acataacata caacctggga gaaagtctga tattctccac 120

tgaatgagtt aaaaaggaat gtaaaactac aaaatactac tttacgtgtg agttctaagg 180

tatgattaat acaacaacaa acatcttgct atttttctct aattttccga ggtggatttc 240

actataaaag cttttttttc tcatttcaag aaaggaaggg ctactcataa aaaatcttta 300

agtatagggt acaaattagt ctcaacacaa aattctactg cctacctaaa agttatgaat 360

tgaggggact aatgtcaact gcaaatctag tgtctgaaag gttagtctaa tctaaggatg 420

acacgcaaat acaactccat ttgtcattta tgttaaccac ttggtactca gaacggggtg 480

gccaaacact gcttagtgac agagatccaa catgaatctg ttccaagggg accagagcca 540

agagggatga ctcattctat ctccggaccc acactgtaca caaacggaaa ctactagttt 600

acactacatg tttcaagaca aaattggcat gtgta 635

<210> 705

<211> 499

<212> DNA

<213> Rattus sp.

<400> 705
cccacttaaa acggaaacac ctttattgac agtataggac agttaaaacg cacgcaagga 60

caaagatgag accacagagg tgtaaagttg cagcacagga gttaatattc ttcaccctca 120

tcgtctcctt cagcactatc tgccccaacc tcctcataat ccttctctag ggcagccata 180

tcctcacggg cctcagagaa ctccccctcc tccatgccct cacccacata ccagtgcaca 240

aaggcacgct tggcatacat cagatcaaac ttgtgatcca ggcgagccca ggcctcagca 300

atggctgtgg tgttgctcag catacacaca gctctctgca ccctggccag gtcgccacca 360

ggtaccacag tgggaggctg gtaattaatg ccaaccttga agccagtggg gcaccagtcc 420

acaaactgga tggtacgctt ggtcttgatg gtggcaatgg cagcattgac atccttgggg 480

accacatcac cacggtaca 499

<210> 706

<211> 711

<212> DNA

<213> Rattus sp.

<400> 706
caaacttttt ttttattttt tttttttttt ttgcgttgaa aatcatttaa tgagaattat 60

actttttctc tatcagatta gatctgttct agtgattatt gtctggtgtt acaagctcat 120

ctgtctcaga gttggttagt gtgcatgaag ctacttaaga aagatatgga caagcacagc 180

tcactggtcc ccctgagcac ctcacaagac tgggaacatc ctctcaaggg ttaaaacaga 240

cgggcatacg ggctgagaat ttattttgaa ggcaggataa acaggccagc tctaagaaga 300

tactgttttg aaagctcaca cgtttcataa gtttctgagt tcaggcagtc tcagttctca 360

tccactgagt cctgggccct caaaagccct ccaggtccac cacagaggct gttctaactg 420

ggtgctgagc ctatacaaag ccctggcact cacacaactg ccattcccca agatcaactc 480

gtctcctgta aatatgtgct gtttgtatcc ggcacagggc tgatgaagat ggatcccgat 540

ggagcctact gtgcattact tcctgatgtg cagagatgtg tgtggtgaga ctgtgcggga 600

tgctgggagc agtgtgcagt gcgttgggag actgagaagt gaaagccctg ttggtttgag 660

gggaaagcaa ggaaatgtga cctttcccat agctactccc acagtgagaa a 711

<210> 707

<211> 664

<212> DNA

<213> Rattus sp.

<400> 707
gcaagcaatg cgcagacaaa aactaaattt attaattaaa gattcatttg aataaatttt 60

gtacagaaga atagtattta ttgcatctat agtataaaag aactgctcac caagctttat 120

aaagtatgaa gcaataagga actaattact taaagtgttt ctatggaggt aagtgtcagg 180

gtaaattgag ttataaccag aatacatcat atgtatgatg atgctaacat tttaccaagt 240

tatttttatc ataatgctta atctgcttaa tcatacatca tcatgatatg cgaaatcata 300

attttttcca ggattggacc acaacatcac gtctctgaac tacaaatata aatatgttac 360

accaatcaca tatttgtaaa aatttataaa ataaaagttg actgggaatc ctgtagcctg 420

gactgatgtt cagaagaact caatctgatt catcgctgct gaagtaggag ctgtcgctgt 480

actcagctgt gtaaaggaac ttgtgaatga tcggattcat ctttggtatc cagtgtcggg 540

gaaccaggta cgatgagagg ttaaataaat ccacgaacac cttgtacctg tcgctcacag 600

ttgagcgcag ataataatag cctgaggatc ccccagtgcc agccttgctg cctagcatcc 660

tgtg 664

<210> 708

<211> 536

<212> DNA

<213> Rattus sp.

<400> 708
atattctttc tattttttat taattgcggc acagagaaaa gaagtcaaat gttcaaagaa 60

atttgtgttc cttagggtgt tggttttaca ccaacagaag agatgagtcc tgagtcttca 120

tgtctttttt tctcagggta gcctgagatg gttgtgatgt gtttaggcta aggcttcttt 180

gcttctagca acaaggtttg gcccctcagt ggcgaagcag ttatccttgt cggcagcctt 240

gcaacacttg tccacgaatt gtgcgaagtc acccatcacc gtcttcagct gatcttctgt 300

ggccttgggc ttgtgtttca ccagctcagc gagagccgtt tgcttcctta tctgcttctc 360

cttgtctggg agtgtgcaga tatcatagtg gaaggtgaag gtctcagctt taaactcttt 420

ggggacatat gtctcgtcaa ctgtcatagc agtgaaacat ggccgtcttt ccaccaggga 480

cccactacag cacttggtga ccttcacgct cactggggtc ttctcatgca gcacac 536

<210> 709

<211> 577

<212> DNA

<213> Rattus sp.

<400> 709
   accctcacta taaggactaa gcttgcggac gctaaagttt tttttttttt tttttttttt 60
   ttttattttg catactttta atttcagaac aaaataaaac aaacaaaaaa acacaataca 120
   caacacccaa ccctgctatt ctgtgggagg ggatcttcct gccttaaggc acaaggacag 180
   gatagaagag attcttgtag acatgagcag agagccaggt gggtcagggc cagtcaaggc 240
   tgttggggga gccacaggga tactatacag agggcggggg cttcccagga gcctttaatc 300
   cacttcttcc atgcgagagg catcctcatc accctccagt ggggggatct catcgggaac 360
   agcagcactg ggctcttctg cagtgacctc atcttcatca atgcccaggc ctagtttaat 420
   catgcggtag atgcggttgg agtgggtttg ggggtcctca agtgagaagc agaggagagc 480
   agagcattct caaacagcag caccaccagg tctttgacag ctttgtcctt tttgtctgcc 540
   tcagccttct gccgcaaagt ctccacaatg gggtggg 577

<210> 710

<211> 613

<212> DNA

<213> Rattus sp.

<400> 710
aatttttatt tcaaaagctt ggatagcttc aatatccagg tcgtggcaaa atcaggacac 60

gtgtaaaata ccttacaata cattagattc ccaaaaggta ccaaaaagta cagtaaaatt 120

aacacttcca ttactggaaa tgtatgacac aaataatata aaattaaaag gtgaaaaagg 180

tgacactggt ttcctaagat acagtttact ctttacaacc agggtccaca ggtccaggct 240

gcagagcggc agcaggaagc agaccctcca atctagaaca gggtaacctg gtaataaaat 300

cagcccccta atgccgctct gacctctgag acgccacacg ctgcctacaa caactagagc 360

tctggaatag taaacaggag agtgatttcc gggggaaatt ttaaataaga tgcacatggg 420

acaggccata aaagtttgcc aagttaaatt tggtacataa ttgtgttcac tccgcgtcca 480

aacaaagcgt gtgcggtcgt cactcggccg ctcctgctgc ctctcagtat ggcttgtagt 540

tattctgatg accaccacgt cgctggctct tcccgtaatt tgtactaccc tgactgtagt 600

cgtagccggg gcc                                                    613

<210>  711

<211>  465

<212>  DNA

<213>  Rattus sp.

<400>  711
ctcatttagt ttttattcat aatcataaac ttaactttgc aatcctgcta gacttgaagg    60

ggaatgagga aaatatggaa cccaaagaac ttcagtgaga gcagagatta cagggtattg   120

cgagcagatg gggtgggggt gctctcctga gctacagaag gaatggtttg atgggtaaaa   180

tgcccgcaag tcaaagaaat tagagttgtc cacagtcgga gatggtgatc ttcttgctgg   240

tcttggcagt gcagataaaa aactgggaac catttgtgtt tggtccagca tttgccatgg   300

acaagatgcc aggacctgta tgcttcagga tgaagttctc atcctagaaa tttctctccg   360

tagatggact tgccaccaat gccattatgg cgtgtgaagt caccaccctg acgcatgaat   420

cctggactag ttatgtgaaa ggagtaaccc ctatagccaa atcct                   465

<210>  712

<211>  582

<212>  DNA

<213>  Rattus sp.

<400>  712
gggtagcata aattttctca aactttaatc ttcacaatta ttttcactct atacacttta    60

ttgaaaaggt ctagatttat ttgacaaaat gattatgacc agaataaaga tatcttcttt   120

ttcatatatc agtaagtggc tggaatagtt aatttagtca tgtatcctgg aaaatgagtt   180

tcaaaatctt cctctttttt tttgaggtca gctactgtca ataatggaca ttaggcaata   240

gatcataaca cttcagtaac atgctgtgtc agaaccttgc ataattcaca cattcatttg   300

ctctctgcta cattatgact tcatggatta aagtttatta aattccaaat atttcttgca   360

ggagggaatg agtaaaacat caggataatg ctgtcttcat ttttaaatat atattgttgt   420

tttaattgat acatagtaat tgcacataat tatggcacag tatgacgttt caataatgta   480

tagtgtacat aataatcaaa tgaaggtaat tggcatgtca caccagatgt aactatttcc   540

tttctttctg ggacatggct attggacata gtcaattaat tg                      582

<210>  713

<211>  1840

<212>  DNA

<213>  Rattus norvegicus (Norway rat)

<400>  713
cgagaaacta caggcaaact ttcagctttg aagttgtcac cttggagaag tgaagtcgag      60

aagtgtgaca ccatgaatgg acctgtggat ggcttgtgtg accattctct aagtgaagag     120

ggagccttca tgttcacatc tgaatcggta ggagaagggc atccagataa gatctgtgac     180

cagattagtg atgcagtgct ggatgcccat ctcaagcaag accccaatgc caaggtggcc     240

tgtgagacag tgtgcaagac agggatggtg ctcctgtgtg gagagatcac ctcaatggcc     300

atgattgact accagcgggt ggtgagagac accattaagc acattggcta cgatgactct     360

gccaagggct tcgacttcaa gacctgcaat gtgctcgtgg ctctggagca acagtcccca     420

gacattgccc aatgtgtcca tctagacaga aatgaggagg acgttggtgc aggagatcag     480

ggtctgatgt tcggctatgc cactgacgag acagaggagt gcatgccgct caccattgtt     540

cttgctcaca aactcaacac ccggatggca gatctgaggc gctctggtgt ccttccctgg     600

ctgagacctg attctaagac tcaggtaaca gttcagtacg tgcaggataa tggtgctgtc     660

atccctgttc gcgtccacac catcgtcatc tctgtgcaac acaacgaaga cataacactg     720

gaggccatgc gagaggccct gaaggagcag gtgatcaaag ctgtggtgcc agccaagtac     780

ctggatgaag acaccatcta ccacctgcag ccaagtgggc ggtttgtcat cggaggtccc     840

cagggggatg caggtgtcac aggccgcaag attattgtgg acacatacgg aggctggggt     900

gcccatggtg gtggtgcctt ctctggaaag gactacacca aggtggaccg ctcagcagct     960

tatgccgccc gctgggtggc caagtctctg gtgaaggctg ggctctgccg gagagtcctt    1020

gttcaggtgt cctatgccat tggtgtggca gaacctctgt ccatttccat tttcacctac    1080

ggaacttcca agaagaccga gcgagacgag ctactagagg ttgtgaacaa gaactttgac    1140

ctccggccgg gtgttattgt cagggacttg gatctgaaga agcccatcta ccagaagact    1200

gcatgctatg gtcatttcgg aagaagcgag tttccctggg aggtccccaa gaagcttgtg    1260

ttttagagct gccgggaagc ttagctctgt cctatactca acctcctcaa gatctcaact    1320

ccctggtctc agagagccct ccctgcttcc caccctatgc aaagccagct tcctccgatt    1380

ttccccctac ccggcaatct gcaaacatct taatagtggc aaggcaggtg gtttctggtg    1440

ctaggtcatc aggtctcctt gcaaggatag tcagtgttcc ctccctgtct ctcagatgac    1500

aacaatgtta attttgtgta aaagttgctc ctgtcatccc ctcccgtagt ctgaagctaa    1560

ccaggccatc tcttcctccg tcctggacat gtgcccagat gaatgttttg tcccatatct    1620

ctggcttccc ctttacattg gcagccagag gtcctgagcc ggggcttcct cagggtccct    1680

ctgagactgg gcatctgctc cctaggatat ggtcagtgtc ccctgaacat ctgcattctg    1740

tgagggactg agctgtgtgt tcttcatccc aacacaataa ggtggtatcg tcattatcca    1800

cagtgacaga tggggaaact gaggctcaga gagcgagctc                          1840

<210> 714

<211> 1628

<212> DNA

<213> Rattus norvegicus (Norway rat)

<400> 714
ctgtattgag tgttggccag tggtctttgg tagcctgggc agcagcgggg cagacatgga 60

gctgctggct gggactggcc tgtggcccat ggccatattc acagtcatct tcatattact 120

ggtggacctg atgcaccggc gccagcgctg gacttctcgc taccctccag gccctgtgcc 180

gtggcctgtg ctgggcaacc tgctgcaggt ggacctctgt aacatgccat acagcatgta 240

caagcttcaa aaccgctatg gtgacgtgtt cagcctacag atgggttgga agccagtggt 300

ggtgatcaat ggactgaagg cagtgcagga actgctggtg acctgtggag aggacactgc 360

tgaccgccct gaaatgccca tctttcaaca tataggctat gggcacaaag ctaaaggtgt 420

ggtgcttgca ccttacgggc ctgagtggcg agagcagagg cgattctctg tgtccaccct 480

gcgcaacttc ggtgtgggca agaaatccct ggagcagtgg gtgacagacg aggccagcca 540

cctctgtgat gccctcaccg ctgaggcagg gcggcccctc gatccttaca ccctcctgaa 600

caaggctgtg tgcaatgtaa ttgcatctct catttatgcc cgccgcttcg actatggaga 660

ccctgatttc atcaaggtgc taaaaatatt gaaagaaagc atgggagagc aaactggctt 720

gtttcctgag gttttgaaca tgttcccagt tctcctgcgc atcccagggt tagctgacaa 780

agtcttccct gggcaaaaga catttctcac catggtggat aacctggtga ctgagcacaa 840

gaagacttgg gaccctgacc agccacctcg agacctgact gatgcctttc tggctgagat 900

agagaaggcc aaggggaacc ctgagagtag tttcaatgat gcaaatctac gtctggtggt 960

caatgacctg tttggtgctg ggatggtgac tacttctatc actctaacct gggccctgct 1020

gctcatgatc ctgcatccgg atgtgcagtg ccgagtacaa caggaaatcg atgaggtcat 1080

agggcaggtg cggcatccag agatggcaga ccaggcccac atgccgttca ccaatgctgt 1140

catccatgag gtgcagcgct ttgcagacat tgtcccaatg aatttgccac acaagacttc 1200

tcgtgacatt gaagtgcagg gcttccttat ccctaagggg acaaccctca tccccaacct 1260

gtcctcagtg ctgaaggatg agactgtctg ggagaagccc ctccgattcc accctgaaca 1320

cttcctggat gcccagggca actttgtgaa gcatgaggcc ttcatgccat tctcagcagg 1380

ccgcagagca tgcctggggg agcccctggc ccgcatggag ctcttcctct tcttcacctg 1440

cctcctgcaa cgcttcagct tctccgtgcc cactggacag ccccggccca gcgactatgg 1500

tgtctttgcc tttctcctta gcccttcccc ctaccagctc tgtgcattca aacgttagaa 1560

ggaaagaaat tctagtccag cctctacctt agtgtcatac acagattaaa acagttctgt 1620

ggctgcca 1628

<210>  715

<211>  1272

<212>  DNA

<213>  Rattus norvegicus (Norway rat)

<400>  715
gaattcgact gctggaacca acgtcctctc ttaccctcca ccttcttctg ccacctctac      60

cacggtcacc atgtcgcaag cccggcctgc cactgtgctg ggtgccatgg agatgggtcg     120

ccgcatggat gtgacctcca gctccgcgtc ggtgcgcgcc ttcctgcagc gcggccacac     180

ggagatagac accgccttcg tgtatgcgaa cggtcagtct gagaccatcc taggagacct     240

ggggctcgga ctgggccgca gcggctgcaa agtaaaaatt gccaccaagg ctgccccaat     300

gtttgggaag acactgaagc cagccgatgt tcggttccag ctggagacgt cactgaagag     360

gctgcagtgt ccccgggtgg acctcttcta tttacacttt ccagaccacg gcactcctat     420

agaggagacc ctgcaggcct gccaccagct gcatcaggag ggcaagtttg tggagcttgg     480

tctgtccaac tatgtctcct gggaagtggc tgagatttgt accctctgca agaaaaatgg     540

ctggatcatg ccaactgtgt accagggcat gtacaacgcc atcaccaggc aggtggagac     600

tgagctcttc ccctgcctca gacacttcgg actaaggttc tacgccttca accctttggc     660

tgggggcctg ctgactggca gatataaata ccaggataag gatgggaaga atcctgagag     720

ccgcttcttt gggaatccat tttctcaact gtacatggac cgctactgga aggaggaaca     780

cttcaatggc atcgccttgg tggagaaggc tctgaagact acctatggcc ccactgcccc     840

cagtatgatc tcagctgccg tacggtggat gtaccatcac tcacagctca agggcaccca     900

aggggatgca gtcattctgg gcatgtccag tctggaacaa ctggagcaga acttggcctt     960

ggtcgaggaa gggcctctgg agccagctgt tgtggatgcc tttgaccaag cctggaacct    1020

agttgcccac gagtgtccca actatttccg ctaagataca tctgccttgg ggatggcgca    1080

gcttactgcc tgccccgcct tgtcctgggc tcgatctgat ctggttcttt cctttttaga    1140

caggtcactg tctttttctt ccctgctttc tatacagcca gttgctttca aagtgagagc    1200

tggctgagcc ccaatacctc ctgctgaata aaactgttcc ctgtcacagc ctgggctaca    1260

actggcggcc ga                                                        1272

<210>  716

<211>  1816

<212>  DNA

<213>  Rattus norvegicus (Norway rat)

<400> 716
ccccctcggc ccgccatgct cctttcggtg ccgctcctgc ttggcctcct cggcctggct 60

gccgcagacc ctgccatcta tttcaaagag cagttcttgg acggagatgc ctggaccaac 120

cgctgggtcg aatccaaaca taagtctgat tttggcaaat tcgtcctcag ttctggcaaa 180

ttctacgggg accaggagaa ggataaaggg ttgcagacaa gccaagatgc ccgattttac 240

gcgctgtccg ccagattcga acccttcagc aacaagggcc agacactggt ggtacagttc 300

accgtgaagc atgagcagaa tatcgactgt gggggcggct acgtgaagct gtttccgggt 360

ggcttggacc agaaggacat gcatggagac tcagaatata acatcatgtt tggtccggac 420

atctgcggtc ctggcaccaa gaaggttcat gtcatcttta actacaaggg caagaacgtg 480

ctgatcaaca aggatatccg gtgtaaggat gatgaattca cacatctata cacgctgatt 540

gtgcggccag acaacaccta cgaggtgaaa attgacaaca gccaggtgga gtcgggctcc 600

ttggaggatg attgggactt tctgccgccc aagaagatta aggatcctga cgctgccaag 660

ccagaagact gggatgaacg agccaagatt gatgacccca cagattccaa gcctgaggac 720

tgggacaagc cagagcacat ccctgaccct gatgctaaga agcctgagga ctgggacgaa 780

gagatggatg gagagtggga accaccagtg attcaaaatc ctgaatacaa gggcgaatgg 840

aagccacgtc aaattgacaa cccagattac aagggtacct ggatacaccc agagattgac 900

aatcctgaat actcccccga tgcgaatatc tatgcctatg atagttttgc tgtactgggc 960

ttagacctct ggcaggtcaa gtctggcaca atttttgaca acttcctcat caccaatgat 1020

gaggcctatg cagaggagtt tggcaatgag acctggggtg tcaccaaggc tgcagagaag 1080

cagatgaagg acaagcagga tgaggagcag aggcttaagg aagaagaaga agacaagaag 1140

cgtaaagagg aagaggaggc cgaggataaa gaggatgagg atgacagaga tgaagatgaa 1200

gatgaagagg atgagaagga agaagatgag gaggatgcca ctggccaagc caaggatgag 1260

ctgtagaggc cacaccacct gcctccaggg ctggactgag gcctgaacac cccgccacag 1320

agctggctgc tcccaataat gtctctatga gactcaagaa cttttcattt ttttttcagg 1380

caggttcagg tctggggtgg attctgattt ttgttcccct gcctccccat cctccccacc 1440

cccctttttt tttactggtg tttgtcttta attcttcagc cctcacctcc tggcctctca 1500

tttttgaatc aacatttttt ctttctgtcc ctttctccat ctcttggtca ctatcctcca 1560

actctaggaa caggtatgga ggaaaagcct aggcttgaga tttcatctgc tctcctttct 1620

gaatctcaga ggagggtagg agaagggggt ggtatcttcc ctccccccag cactgaggaa 1680

gaatggggct cttccccttt ctcccttgcc cccaggactg ggccatttgt ggggcagcca 1740

gttctagcac agctcacact gagagtgtaa gaactacaaa caaaatttct attaaattaa 1800

gttttgtgtc ttccca 1816

<210> 717

<211> 6849

<212>  DNA

<213>  Rattus norvegicus (Norway rat)

<220>

<221>  misc_feature

<222>  (2293)..(2293)

<223>  n

<400>  717
ccctcacagt attaagtacc cttaccctac cgctttcctc ccaactaatc acatagaagt      60

ttaggatata cagttcaaga gccttcaaag cccttagaaa acaaacaagt ttaacttctg     120

ataaggactg taagactata tcttacatct gttaaatgca aatcaactgc tttaattaag     180

ctaaatcctc aactagattg gaaggattca aacctacgaa aatttagtta acaactaaat     240

accctactta ctggcttcaa tctacttctc ccgcctatca gaaaagaggc gggagaagcc     300

ttagtagagg agattctcta caccttccaa tttgcaattc gacatgataa tcaccttaag     360

gctttttggt aaaaaggggg ctcaacccct gtctttagat ttacagtcta atgcttactc     420

agccatttta cctatgttcg taaacggttg actcttttca actaacccta aagatatcgg     480

aaccctctac ctattatttg gagcctgagc aggaatagta gggacagctt taagtattct     540

aattcgagct gaactaggac agctaggcgc actcctagga gatgaccaaa tctataatgt     600

cctcgtcaca gcccatgcat tcgtaataat tttctttata gtaataccta taataattgg     660

aggcttcgga aactgacttg taccactaat aattggagcc cctgatatag cattcccacg     720

aataaataac ataagctttt gactgcttcc tccatcattt ctactccttt tagcatcctc     780

catagtagaa gctggagctg gaacaggatg aacagtatat ccccccttag ccggaaacct     840

agcccatgct ggggtatccg tagatttaac tattttttcc ctccacctag ccggggtgtc     900

ttctatctta ggagctatca actttatcac cactatcatt aatataaaac cccctgctat     960

aacccaatat cagacacctc tctttgtatg atccgtacta attacagccg tcctactact    1020

tctctcactg ccagtattag cagcaggtat cactatactc cttacagacc gaaatctaaa    1080

tactactttc ttcgaccccg ctggaggcgg aggcccaatt ctttatcaac acctattctg    1140

attcttcggc cacccagaag tgtacatctt aattcttcta gggtttgaaa ttatttcaca    1200

tgtagttacc tattactctg gaaaaaaaga acccttcgga tatataggta tggtatgaac    1260

cataatatct attggcttcc taggatttat tgtatgagca catcacatat tcactgtagg    1320

cctggatgta gacacccgag cctactttac atctgccact ataattatcg caattcctac    1380

aggcgtaaaa gtattcagct gactcgctac actacatgga ggaaatatca aatgatcccc    1440

cgccatatta tgagccttag ggtttatctt cttattcaca gtagggggcc taacagggat    1500

cgtactatct aactcatccc ttgacattgt acttcatgat acatactacg tagtagctca    1560

cttccactat gtcttatcta taggagcagt attcgccatc atagcttgct tcgtccactg 1620
attcccacta ttctcaggct ataccctaaa tgacacatga gcaaaagccc tctttgccat 1680
tatatttgta ggtgtaaaca taacattttt tcctcaacac ttcctaggat tagcagggat 1740
acctcgtcgt tactctgatt atccagatgc ttataccaca tgaaatacag tctcctctat 1800
aggctcattc atctcactta cggccgtcct tgtaatgatc ttcatgattt gagaagcctt 1860
cgcatcaaaa cgagaagtgc tctcaatttc ctactcttca actaacctag aatgactgca 1920
tggatgcccc ccaccttacc acacattcga agaaccttcc tatgtaaaag ttaaataaga 1980
aaggaaggat tcgaaccccc tacaactggt ttcaagccaa tttcataacc attatgtctt 2040
tctcaatgag atattagtaa aataattaca taaccttgtc aaggttaagt tatagactta 2100
aatctatata tcttacatgg cttacccatt tcaacttggc ttacaagacg ccacatcacc 2160
aatcatagaa gaacttacaa actttcatga ccacacccta ataattgtat tcctcatcag 2220
ctccctagta ctttatatta tttcactaat actaacaaca aaactaacac acacaagcac 2280
aatagacgcc cangaagtag aaacaatttg aacaattctc ccagctgtca ttcttattct 2340
aatcgccctt ccctccctac gaattctata cataatagac gagattaata acccagttct 2400
aacagtaaaa actataggac accaatgata ctgaagctat gaatatactg actatgaaga 2460
cctatgcttt gactcctaca taatcccaac caatgaccta aaactaggtg aacttcgctt 2520
attagaagtt gataatcggg tagtcttacc aatagaactt ccaattcgta tactaatctc 2580
atccgaagac gtcctgcact catgacccat cccttcacta gggttaaaaa ccgacgcaat 2640
ccccggccgc ctaaaccaag ctacagtcac atcaaaccga ctaggtctat tctatggcca 2700
atgctctgaa atttgcggct caaatcacag cttcatactc attgtactag aaatagtgcc 2760
tctaaaatat ttcgaaaact gatcagcttc tataatttaa actcattgcg aagcttagag 2820
cgttaacctt ttaagttaaa gttagagaca acaaatctcc acattgacat gccacaacta 2880
gacacatcca catgatttat tacaatcatc tcctcaatag ccacactatt taatttattt 2940
caattaaaaa tttcttccca aacctttcct gcacctccct cacccaaaac tatagccaca 3000
gaaaaaacga ataacccttg agaatcaaaa tgaacgaaaa tctatttccc tctttcatta 3060
cccccacaat aataggtcta ccaattgttg tgaccattat tatgttccca tcaattctat 3120
tcccatcatc agaacgccta atcagcaacc gactacactc atttcaacac tgactaatca 3180
aacttatcat caaacaaata atgttaattc acacaccaaa aggacgaacc tgagccctaa 3240
taattgtatc cctaattata tttatcggct caaccaacct tctagggctt cttccccata 3300
catttacccc taccactcag ctatctataa acctaagcat agccatcccc ctatgagcag 3360
gagccgtaat tctaggcttc cgacacaaac taaaaaattc tttagcccac ttcttaccgc 3420
aaggaacccc catctcacta attcccatac taatcatcat cgaaactatc agcctattta 3480
ttcaaccgat agcactagca gtacgactaa cagcaaacat tacagcaggc catctattaa 3540
tgcatctaat cggaggagct accctagtac ttatagacat cagcccacca accgctacaa 3600

ttacatttat tattctactt ctacttacag tacttgaatt tgtcgtagcc ttaattcaag 3660
cctatgtatt cacccttcta gtaagcctgt acctacatga taacacataa tgacccacca 3720
aacccatgca taccatatag taaacccaag cccatgacca ctaacaggag ccctatcagc 3780
tcttctactc acatccggct tagtaatatg attccattac aactccacaa ttctcctatc 3840
attaggcctc ctgacaaaca tcctaactat atatcaatga tgacgagata tcatccgtga 3900
aggaacatac caaggccacc acacccctat tgtacaaaaa ggcctccgat acggaataat 3960
cctgtttatt gtctccgaag tattcttctt tgccggattt ttctgagcat tttatcattc 4020
cagcctagtt cctacccacg acctaggcgg ttgctgaccc ccaacaggaa tcaccccttt 4080
aaatccccta gaagtacccc ttctaaatac atcagtcctc ttagcatcag gagtctcaat 4140
tacatgagcc catcacagcc taatagaagg caaccgaaac catataaacc aagccctact 4200
aatcaccatt ctcttaggat tatatttcac tatcttacaa gcctcagagt atttcgaaac 4260
atcattttct atctcagacg gaatttacgg ctcaacattc ttcatagcaa cgggatttca 4320
tggccttcac gtaattattg gctcaacttt cctaattgtc tgtctactac gacaactaaa 4380
attccacttc acatcaaaac atcatttcgg atttgaagcc gcagcatgat actgacactt 4440
cgtagatgta gtttgactat tcctatacgt ttctatctat tgatgaggat cctactccct 4500
tagtataaac aatacaactg acttccaatc agttaattct gaaaaaactc agaagagagt 4560
aattaaccta cccattatca tcacaattaa catcacctta tcttttatcc tcatttcaat 4620
tgcattctga ttgcctcaaa taaacttata ctccgaaaaa gcaaacccat atgaatgtgg 4680
cttcgaccca acaagttctg cacgccttcc tttttcaata aaatttttct tagtagccat 4740
tacatttcta ctattcgacc tagaaatcgc cttactactt cccctcccat gagcgattca 4800
aacaaccaat accgctacaa taatagcaac tgcctttatt ttagtcacta ttttagctct 4860
tggcctaagc tacgaatgaa cacaaaaagg actagaatga acagaataat tggtaattag 4920
tttaaataaa attaatgatt tcgactcatt agattatgat aataatcata attaccaaca 4980
atgacatctg ctttcctaaa tttaactata gcctttacat tatctctact aggtactttt 5040
atatttcgct cccacttaat atctactctc ctctgcctag aaggaataat actatcacta 5100
tttgtcataa cttcaacatc cacattaaac tccaactcca taatctccat aaccatccca 5160
attaccattc tagtttttgc agcctgcgaa gtagcagtag gtttagcctt actagtaaaa 5220
atttcaaata cccacggaac agactacgta caaaacctca accttctaca atgttaaaaa 5280
tcattttccc atctatcata ctcctcccac taacatgact ctcagccaac aaaaaaatct 5340
gaaccaatgt cacctcctac agctttctag tgagcctatt aagcttatca ctcctatgac 5400
aaaatgacga aaattaccta aatttctcag ttatattctc ctccgatcca ttatccaccc 5460
cactaatcat tctaacaact tgactcctcc cactaataat gctcgctagc caaaatcaca 5520
taaaaaaaga aaatataatg catcaaaaac tttacatctc aatacttatt agcctccaaa 5580
ttttactcat cataacattc tccgcaacag aactaatttt attttatatc ctgttcgaag 5640

ccactctaat cccaacacta attatcatta cacgatgagg caaccaaaca gaacgcttaa 5700

atgcaggaat ttatttcctg ttttatacac taattggctc catcccactc ttaattgccc 5760

tcatttcaat ccaaaactca ataggaacac tcaacttcct aatcctctcc ctcacaacac 5820

accccctacc ctcaacatga tccaacacca ttctatgact agcatgtata atagcattta 5880

taatcaaaat accattatac ggagtccatc tatgattacc aaaagcccac gtagaagctc 5940

caattgcagg ctctataatt ttagcagcaa ttctcctaaa gctagggggt tatgggataa 6000

tacgagtttc catcattcta gaccccctaa caaaatcttt agcctaccca ttcatcatcc 6060

tctcattatg aggcataatt ataactagct cggtctgcct acgccaaaca gatctaaaat 6120

cattaattgc ttactcatca gtaagccata tagccctagt catcacagcc attataatcc 6180

agacaccatg aagcttcatg ggagctacaa tactaataat cgcccacggc ttaacctcat 6240

cactcttatt ctgcctagca aacaccaact acgaacgaat tcacagccga actataatta 6300

tagctcgagg attacaaata atcttttcat tgatagcaac atgatgatta ttagcaagct 6360

tagccaacct agcactacca cccctaatta acctcatagg cgagttattc attgttatag 6420

caacattttc ctgatcgaac ccctctatca tccttatagc aactaacatt gtcactacag 6480

gaatatactc aatatatatg attatcacaa cccaacgagg aaaactaacc agccacataa 6540

acaacctcca accttcccac acacgagaat taacactcat agctctacac attattcccc 6600

tcatactatt aacaatcaac cctaaactca tcacaggcct aacaatatgt agatatagtt 6660

tacaaaaaac attagactgt gaatctaaca acaggaaatc aaattcctta tttaccaaga 6720

aagtatgcaa gaactgctaa ttcatgcacc catacctaaa acatatggct ttcttacttt 6780

tataggatag aagtaatcca ttggtcttag gaaccaaaaa ccttggtgca actccaaata 6840

aaagtaata 6849

<210> 718

<211> 959

<212> DNA

<213> Rattus norvegicus (Norway rat)

<400> 718
agagggagca gctttttaac aagagaactc aagcaattgc tgccatgccg gggaagccag 60

tccttcacta cttcgatggc agggggagaa tggagcccat ccggtggctc ctggctgcag 120

ctggagtaga gtttgaagaa caatttctga aaactcggga tgacctggcc aggctaagga 180

atgatgggag tttgatgttc cagcaagtgc ccatggtgga gattgatggg atgaagctgg 240

tgcagaccag agccattctc aactacattg ccaccaaata caacctctat gggaaggaca 300

tgaaggagag agccctcatc gacatgtatg cagaaggagt ggcggatctg gatgaaatag 360

ttctccatta cccttacatt ccccctgggg agaaagaggc aagtcttgcc aaaatcaagg 420

acaaagcaag gaaccgttac tttcctgcct ttgaaaaggt gttgaagagc catggacaag 480

attatctcgt tggcaatagg ctgagcaggg ctgatgttta cctagttcaa gttctctacc 540

atgtggaaga gctggacccc agcgctttgg ccaacttccc tctgctgaag gccctgagaa 600

ccagagtcag caacctcccc acagtgaaga agtttcttca gcctggcagc cagaggaagc 660

cattagagga tgagaaatgt gtagaatctg cagttaagat cttcagttaa ttcaggcatc 720

tatggataca ctgtacccac aaagccagcc ttcgaaagct ttgcaacaat cgcatatttt 780

gactaaatgt tgaccctact tattgggagg ccaacacgtt ttctaatgct tctgtgttaa 840

ttcatataga catgactgat gaggaattgc tgggatgcta tttggttgta gttaaaattt 900

gaaatcatga tcacttcctc agatattact ttgaatctca ataaaaactt cgcaagctt 959

<210> 719

<211> 8351

<212> DNA

<213> Rattus norvegicus (Norway rat)

<400> 719
gaattccatc agaatttgcc tttctggtgg cttctctttc cctgcccttg tggtgttttt 60

ccttcagtga gtgacaaatt tcacaaccct ggctgggact cccgaggtgc taagattaca 120

ggcctgggtc acaccaccca aacctgactt tctttttcat tgttgcttgt atttttctgt 180

ttgtaaccaa agctggacga ctggaactca ctgtgtagac taggctggcc ttgaactcat 240

agaactctac ttgcctctgc ctcctgagtg ctctgattaa cggcactgac caatacatcc 300

aacctaccta ctttcatttt ctaaatctaa gtcctaacag gaagtgggaa ctgggcagga 360

ataacagtac ggtgggttaa ctccatgagt ttaccggact ttgcgagcct cgactgccaa 420

cgacatcctg gcttgaggct ggaagtcaca gcccaacagg ggcaaagatt gctctgtgac 480

cagtctggaa agggagcact ggagcacaga aatcaatccg gttcaagttc atacccaggg 540

caaccacgga aagtgccagg aaaggaaaga acaggatgtt ttccacacat tccatgggca 600

gccatgggga tccaggagaa agtgatgctt ggctgagcca agaagcagtg ccccagttta 660

cagtaagggc tgagaggaca gcctgtcctg agcttccggt aacacatttc ctgccttctc 720

aaatgacaga cattccatct acgactttga gtctgatttc agcagtctta tgcaagaggg 780

gaaacaccat atgcctccag ggaaagaaaa tttggctgcc gtctccaccc tttccctcag 840

catccaccgt gggtgggggt ggggagggtt agtggggctt tccatccctg tctttcagaa 900

cactacgatc tggccctttc tgcttggcca acacctgcgc agagtcctag ttcatatcct 960

cccagaatgg cctgctctcc acctccagca gagaccccca tcatttttcc ctgttcactc 1020

tctgcccccc accccccacc aagaataagt atccttagca caaggcttgt gtctttatgg 1080

tctctagtct ctgacaactg gctggagtct cagtggattc gaaccctcca ttcatcttgg 1140

gctaatgact atgtgattgc gcctccgttt ccacttttct actgtgaaaa taatgaacac 1200

cccaagctat gttgtaagga aaaatgagag ccctaacagt gcccccagca cgtgacacgc 1260

agggggtacg tgacacgcag ggggtacgta accaaggccg gtaaagtctg ggctagggct 1320

ggtttttgtt acctgttcac actgtcagct aggttttcct gtatgcgggg tctccaagcc 1380

ccgctttcac ctaagttagc actcaagacg tgctgtgggg actgtgtccc cgtggacgct 1440

gcagggggtt cgatgtcccg caactcctct gcacccggcc acttgggccc agggcacgtg 1500

agcaggtttc ctggaaccgg tccccaccgg atcgcagacc ctttgcgctc agccctttgc 1560

tctcagtccc tgcgccagga gaaagggggt gtgactcagc gcgggggcgt gtgcaggctc 1620

tgtacccacg tgcaaaagga gggatgcttg cagacttcgg gtcgtgcgca ggctcccggg 1680

cgtgtgcggg ccatttccct tgagccagaa gaagggcgtg tgcaggcagt ggggaggagg 1740

gcaggtggcg ccccgccacc cgggcggagc ttttgcgcgc gacccaatac tctgggctat 1800

aaaggtcgcg ctccgcgtgc ttctctccat cacgctccta gaactctaca gcgatctctc 1860

gttgatctcc aactgccgcc tccattcgcc atggacccca actgctcctg tgccacaggt 1920

aaggggggct gctgacgggc ctctgtaacc ggagcttctg ggagagcagg acggactttt 1980

gggcccctac tctggtaact acttttaggg tactactggc tgctgccttc cgaacgaatt 2040

ctggaacact cccgcccctt ttaaactagt ccttgagata atggctcgcc caagctggct 2100

ggcttgcacc cgagttcttt ggagaactgt gttcagttat gcccgggtcc gctcaccgcg 2160

ctccctgcct tcttcctcta gatggatcct gctcctgcgc tggctcctgc aaatgcaaac 2220

aatgcaaatg cacctcctgc aagaaaagtg agttggattt attttctcta ccctttccct 2280

tcgcgcccct gcggtcccta gcccgccgca ccttcccaga gcgtccaggc tgcctctaac 2340

tcggtttctc gctcacgctc aacttttttc ccccaggctg ctgttcctgc tgccccgtgg 2400

gctgtgcgaa gtgctcccag ggctgcatct gcaaagaggc ttcggacaag tgcagctgct 2460

gcgcctgaag tgggggcgtc ctcacaatgg tgtaaataaa acaacgtaag gaacctagcc 2520

tttttttgta caaccctgac cggttctcca cacttttttc tataaagcat gtaactgaca 2580

ataaaataaa aaaacttgac ttgattaacc cagctttgtc tgtgttcatt ggaaataagg 2640

ggctggcaga ggcgttgaaa tgggattggt gcaccttgat ttgggataag tggattgatg 2700

acccctctgg actttgatag tctcgaacat ggtgggcaga aacatgtact ggtcacaaat 2760

gtgggcatgt gtatattggg gattaaaccc aaagcttcct gcttataaac cagggtgctc 2820

taatgagcca cactcctacc cctagatgca taatgattct ggtttaattt tggattatta 2880

ggcttaaagc agtatgaagt acctgttcat aagctttggt aaataaaata aaagttggag 2940

tgagtctcat acgactcctc tttgtagtcc caatatttgg gagcctgagg cagaaggatc 3000

ggtgcaactc cgaagccaac ttggtctcaa attctgttaa cctttgattt tgagaccatc 3060

ttactgtgta acctaaaatg gtccttgaac ttgcagtcct gcctcagact tctaggtact 3120

gggattacag gctcagctta aaatcagggc tggagagatg gctcagcggt taagagcacc 3180

cgactgctct tccagaggtc atgagttcaa ttcccagcaa ccacatggtg ctcacaccat 3240

ctgtaatgag atcttacgcg ttctggcgta atgcaagcag aaaagacatc agtaacgtga 3300

acaaaaccat gaaaagtact gtaaacacta taaatatcca agggtgtgcc ttgcagtttg 3360

gagactaaat ggcacatgtc caacctagag ctcccatgag gaactgccca tctctggtat 3420

acagggacac ggacaggatt ttttttttcc tcttccagag agccctgtga taggacttgg 3480

ctgtcagtct ggaagttctt ctcaaggtca ggcagaaatc tacctacccc tcactccata 3540

ccaacccctg gcaatttaag caaagtaact agaaatttgg aaggaattga ctagcatctt 3600

cccaggagct aggcatccag gttgagtctg caatttggag ggcggggtgg agtttcctac 3660

tctataggaa ggaggtgaat acatgcaatt aaaaccagcc gttaatgccc cctggctatt 3720

tgttgaggta atgcgatttg gtcttcaatc aaagggaaag tttcttggct agaagtaagg 3780

accaagcttg ccgtaggctt tctctgtgaa gagtaaattt acaagacagc ctctgtttct 3840

tgctgtcagg aagtcctagt tcacagccca ctttctctct tattggtcat gtagcctggg 3900

caagtcactg aacccctcaa atgctgatat cctgccctcc tagatgctga taaccacctg 3960

tccccaaaga acacacgggc aaccaagcac agatctgatt tttaaggaat ttgttttgta 4020

agtgcagttt gggaatctgg cctcatttgt ctcttgtgtg cccttgctga caccattcat 4080

tcagccctgg ccttgattta ggtgacaccg aactcgggct gtaccctcag agatttccct 4140

ctttgtctac aaacaaacaa acaaagcaaa tatcctaatt aagactcttg tgtgtcaagt 4200

agggcatcta ggaatgagtg ctgggaccac tcttagtccc agaatgcctt gaaaccaagt 4260

gaatgacaat tatacattta gcttctcaat taaaatggaa gacattgggc cgggaattgg 4320

ctcacagtgg agagcctacc aggcttttgt gaagctctga ggttcatccc tagaaccatt 4380

aaaaaaaggt ccgtgggcct gggaatgtag ctccctggta cagtgcttac ctaacatgca 4440

cggacccctg ggtttgctcc acagcatgga gtaagcagtc tgatggcaca cacctgtaat 4500

tctaacacgc aggaggcaga ggcaaggagg atcaaacgtt caaggaccac ggcaagtttg 4560

aggtgtgggc cacatgtaaa gccgtctcca aaaagacatc acacacaaaa cacaacagta 4620

ttgtgataca cacgtatacc tgtatcctag caacctggga aactgaagca ggagactgtc 4680

ttgagttcaa ggccagactg ggctgttcgg tgatcgacag gccattctga gttacagagt 4740

gaggcccttg gaaaaggaga ggaaggagag gaggagagac tgggcctggc aacatgcatc 4800

tatcatctta gctactcagg agactgaggc agggggagga tttccagctc aaagtctagc 4860

tacagagcac gtctaaagcc agcctggaca gcttagtgag accctgtttc aaaataaaaa 4920

gaatctaaaa gactggaggt aaagctccag tgtagaatgc ttgcctggta accaggaagc 4980

cttgggttca atccttactg taaaaaaagg aaaaaaaatc atattatgca agaggtctaa 5040

aggcccaaga atctgttaca gatctcagtt ttggtaatag acaataaaat ataacaagtt 5100

ggtaaaaaca agcaagagta ccaactacaa acatacttca tgtggttcag cagaagcatc 5160

tcagtatgca tccagaaaac agcagacaga cagaattggg catccttggg ctagggcaca 5220

cctcagcctg acttctaccc gagaagccag cagtcttagc cagtgcagaa ccactggtgt 5280
ctctgacttg ggatctctgc ttaggatgcg cccttgagtg cttagaattt gtctctagtc 5340
aggctgaatc ctctctcttt ccaaacccag tccttagcta tttaaaacca gtaaactcat 5400
gagatttgga gtcatccaac gttatccagg caaggattct gtttttttct taatttttat 5460
atttaattgc ttattaattt ttgaaatagg atctcatttg tgtggccctg gctggccttg 5520
aactcaagaa gaccatctgc ctctgacttc taatagctga gattaaagag gtagcctcag 5580
gcaagaactt aactatagac caagactcag ttccacgtga agttttttgg atcttcccac 5640
acagagggta taactgtgtc atctccaaga tgaggtatcc cgaggaagga gaaatggcct 5700
gggtcattgt caccaaacca gtgggtaata ggttaatgga aagacacatg tgtctaaacc 5760
accaaggagg aggaggaaga gggcaaagag gggaaagaag gaggaggggg aggagtgtca 5820
tagcccagga ctaggtgcct tctttgccta cacacggacc tacgtacaga aggacagcat 5880
cagagaactt gggacccgta caggaacatt ggtgtcaagc tgtactgctt cacagcccgt 5940
tttactactg actggttgta tggcccaccc ggcaggtcat tgaatcctct gtccttgtgt 6000
gtaaatagaa tttgcatctc tatataggta ttaggtgaga gatcggtttg actcctggtt 6060
ctggcataat catcatatcg cacagtggct ggtggaggtc ctataacagt taagcaaaac 6120
ctgcccaagg tctcatagct ctgagtacgc gtgaaccaat ggcatagctg atctcttgcc 6180
ctagtctcaa gggctgacag aatctaacgt tactctaaag tcagaaacat tgaaaatata 6240
aacagctgcc cgtattggtt ttggtttttg tttttgtttt tttttttttg tttatttgtt 6300
ttgttttgtt ttatctaatg cagtccctgg atatcaccta aaatgatccc tctgcctcgg 6360
tttttttttt tttttttttt tttttttttt tttttggttc tttttttcgg agctggggac 6420
cgaacccagg gccttgcgct tcctaggtaa gcgctctacc actgagctaa atcctctgcc 6480
tcggttttta aaaccggcct ggagtagagc cgatggctaa aggtttgtga cccccagccc 6540
ggaacgtgcc tacatatgcc cgctcatgag tggggaatat gttgcgatga gtgtccgttg 6600
gctctgttgc tgtgtccaga aggaaggggc tcaaccaaag accatgatgg gacagagaca 6660
gacaataagg acccggaaag ttcgtaatca aggctagtct ttataaaact gtctccttcg 6720
cctctgctag cttcgattca gagagacgtg ggcggagccg gtcgctgccc aggaactcca 6780
ggaaaggaga agctgaggat agcgcgctac gattgtgttt acagagacag ttgggcttcc 6840
tgaggtgtgt tctcgtaatg cactggatca gtgatggcct gtaatatccc ggaaagcact 6900
acagaaacat gatgttccac acgtcacacg ggtcctccta cccgggccct cctactcggg 6960
cctgtggcac caaagggggc ggtcccgttg tgcacaccgg cgcccgaggg agctctgcac 7020
tccgcccgaa gagtgcgctc ggctctgcca aggacgctgc gctcgtgact gagcgcgggc 7080
tggagcaacc gccaactgag tgcaaaccct ttgcgcccgg acccgtccaa cgactataaa 7140
gagagcagac tgtccgctaa gcctcatccc gacttcagca gcctgactgc cttcttgtcg 7200
cttacaccgt tgctccagat tcaccagatc tcggaatgga ccccaactgc tcctgctcca 7260

ccggtaagac gcccggtcct tggtctttag aatacccagt tgtaggggtt tggcgggaat 7320

aggcaccttt agttgacaat tcgtcctagt tctttctaga acccgctctt ggaatcgcct 7380

tcacctgttc ttggagtatt attattgtcc gaacggctcc ttgtcggggt ttggggtagg 7440

atttagacgc gcaaataaat gtcccgatca cccacgtagt gggacatctg agttgagacc 7500

cagttgttac taaccttatt gtgaattgcc tgatctacaa gagaggtgag agaccgttgt 7560

gtcttgagat caaagaccca agccttaccc taccctgtga ggagagaaga ggggctaggc 7620

tccctggagt tctgaatagc actttgaatt gagcagggca catggtgttg gccactgctg 7680

taatcctgcc tcttactgac cgctgtcttc cttctcctcc acaggcggct cctgcacctg 7740

ctccagctcc tgcggctgca agaactgcaa atgcacctcc tgcaagaaga gtgagttggg 7800

accctcgggt ggtggtgggg gaactcctac agagctggct ctgagaaacg tctgaggcca 7860

ttcggtttgg ggcaagaagc aggtcttctg ccagacctgt gcgaccggag gactaggaag 7920

cctactctga catcttcctc tatctttctt tccaggctgc tgctcctgct gccccgtggg 7980

ctgctccaaa tgtgcccagg gctgtgtctg caaaggtgcc tcggacaagt gcacgtgctg 8040

tgcctgaagt gacgaacagt gctgctgccc tcaggtgtaa ataatttccg gaccaactca 8100

gagtcttgcc gtacacctcc acccagttta ctaaaccccg ttttctaccg agcatgtgaa 8160

taataaaagc ctgtttattc taactctggt tttcttggtg tcgtttagaa ataagaaact 8220

ggggcgacac gggttaactt gatagtctgg ggatctggtt ttggactcgc ccgtgccttt 8280

taactcccgc ctctggctcc caaagagggg taataatgtc tttgggtaaa gccaagttat 8340

cccataagct t 8351

<210> 720

<211> 8048

<212> DNA

<213> Rattus norvegicus (Norway rat)

<220>

<221> misc_feature

<222> (2005)..(2005)

<223> n

<220>

<221> misc_feature

<222> (3503)..(3503)

<223> n

<400> 720
   gaattcggcc caggataaac agatatatag gtaaatgact ggatagatac atggatttga 60
   tctctaactc aacttaggca ccatagaaaa caatccttga attattttaa taattttaaa 120
   tcctagttta ctgagaaaca cgttgacgag gcagggcttc agcctctctg aagcagcact 180
   gcccctctgt ggccacacag gaagtcttca ctcagcagtt tcctgactta aggcctaggc 240
   ctgaggaaat gcattgaata tttctctggg ggacaatagt tctgagaagg aaagagagtt 300
   tgtctatgtt gaggtcagtt aaggatggtt ttctttcctt ccctcccagc cttttctcca 360
   tgcaacatca tgagtcccca tctttctatc ctgcctaaaa ccacttcctt ctgggaaatg 420
   agaaaatcca ctcatacaaa atggaaacat cccgaggatc ccggcccgca gcagctctct 480
   gcccccagac cctgtgagag agagacccaa ccgcctggtc aggtgggcac tcctgaggct 540
   gcagagggaa gagaccacca acactgctca cccctgccca catccctggc ccaagaggaa 600
   actgtataag gcctctgggt cctgtggggg agggcccagg agcggcagga cccctgtgcc 660
   tgagagacca ccaccagaac cagaaggaaa cagaccggat aaacagttct ctgcaccaaa 720
   tcccgtggag ggagagctga accttcagag agaaagacaa gcctgggaaa ccagaagaga 780
   ctgctctctg cacacacatc tcggacgcca gaggaaaaag ccaaagacca tctggaaccc 840
   tggtgcactg aagctcccgg aaatggtgca caggtcttcc tggttgctgc cgctgcagag 900
   agcccgtggt agcaccccac gagcgaactt gagcctcagg accacaggta agaccaactt 960
   ttctgctgca agaaagctgc ctggtgaact caagacacag gcccacagga acagctgaag 1020
   acctgtagag aggaaaaacc acacgccgga aagcagaaca ctctgtcccc ataactgact 1080
   gaaagagagg aaaacaggtc tacagcactc ctgacacaca ggcttatagg acagtctagc 1140
   cactgtcaga aatagcagaa caaagtaaca ctagagataa tctgatggcg aaaggcaagc 1200
   gcaggaaccc aagcaacaga aaccaagact acatggcacc atcggagccc aattctccca 1260
   tcaaaacaaa catggaatat ccaaacacac cagaaaagca agatctagtt ccaaaatcat 1320
   ttttgatcat gatgctggag gacttcaaga aagacgtgaa gaactcctta gagaacaagt 1380
   agaagcctac agagaggaat cgcaaaaatg cctgaaagaa tcgcaaaaat ccctgaaaga 1440
   attccaagaa aacataaata aacaagtaga agcccataga gaggagacac aaaaatccct 1500
   gaaagaattc caggaaaaca taaataaaca agtagaagcc catagagagg agacacaaaa 1560
   atccctgaaa gaattccagg aaaacataaa taaacaagta gaagcccata gagaggagac 1620
   acaaaaatcc ctgaaaggaa ttccaggaaa acacaatcaa acagttgaag gaattaaaaa 1680
   tggaaataga agcaatcaaa aaagaacaca tggaaacaac cctggatata gaaaaccaaa 1740
   agaagagaca aggagctgta gataaaagct tcaccaacag aatacaagag atggaagaga 1800
   gaatctcagg agcagaagat tccatagaaa tcattgactc aactgtcaaa gataatgtaa 1860
   agcggaaaaa gctactggtc caaaacatac aggaaatcca ggactcaatg agaagatcaa 1920
   acctaaggat aataggtata gaagagagtg aagactccca gctcaaagga ccagtaaata 1980
   tcttcaacaa aaccatagaa gaaancttcc ctaacctaaa aaaagagata cccatagaca 2040
   cacaagaagc ctacagaact ccaaatagat tggaccagaa aagaaacacc tcccgtcaca 2100
   taattgtcaa aacaccaaac gcacaaaata aagaaagaat attaaaaaca gtaagggaaa 2160
   aaggtcaagt aacatataaa gggagaccta tcagaatcac accagacttc tcgccagaaa 2220
   ctatgaaggc cagaagatcc tggactgatg ttatacagac cctaagagaa cacaaatgcc 2280
   agcccaggtt actgtatcca gcaaaactct caattaacat tgatggagaa accaagacat 2340
   tccatgacaa aaccaaattt acacaatatc tttccacaaa tccagcacta caaaggataa 2400
   taaatggtaa agcccaacat aaggaggcaa gctataccct agaagaagca agaaactaat 2460
   cgtcttggca acaaaacaaa gagaatgaaa gcacacaaac ataacctcac atccaaatat 2520
   gaatataacg ggaagcaata atcactattc cttaatatct ctcaacatca atggcctcaa 2580
   ctccccaata aaaagtcata gattaacaaa ctggatacac aacgaggacc ctgcattctg 2640
   ctgcctacag gaaacacacc tcagagacaa agacagacac tacctcagag tgaaaggctg 2700
   gaaaacaaat ttccaagcaa atggtcagaa gaagcaagct ggagtagcca ttctaatatc 2760
   aaataaaatc aatttccaac taaaagtcat caaaaaagat aaggaaggac acttcatatt 2820
   catcaaagga aaaatccacc aagatgaact ctcaatccta aatatctatg ccccaaatac 2880
   aagggcacct acatacgtaa aagaaacctt actaaagctc aaagcacaca ttgcacctca 2940
   cacaataata gtgggagatt tcaacacacc actctcatca atggacagat catggaaaca 3000
   gaaattaaac agtgatgtcg acagcataag agaagtcatg agccaaatgg acttaacgga 3060
   tatttttaga acattctatc ctaaagcaaa aggatatacc ttcttctcag ctcctcatgg 3120
   cactttctcc aaaattgacc atataattgg tcaaaaaacg ggcctcaaca ggtacagaaa 3180
   gatagaaata atcccatgcg tgctatcgga ccaccacggc ctaaaactgg tcttcaataa 3240
   caataaggga agaatgccca catatacctg gaaattgaac aatgctctac tcaatgataa 3300
   cctggtcaag gaagaaataa agaaagaaat taaaaacttt ttagaattta atgaaaatga 3360
   aggtacaaca tacccaaact tatgggacac aatgaaagct gtgctaagag gaaaactcat 3420
   agcgctgagt gcctgcaaaa agaaacagga aagagcatat gtcagcagct tgacagcaca 3480
   cctaaaagct ctagaacaaa aangaagcaa atacactgag gaggagtaga aggcaggaaa 3540
   taatcaaact cagagctgaa atcaaccaag tagaaacaaa aaggaccata gaaagaatca 3600
   acagaaccaa aagctggttc tttgagaaaa tcaacaagat agataaaccc ttagccagac 3660
   taacgagagg acacagagag tgtgtccaaa ttaacaaaat cagaaatgaa aagggagaca 3720
   taactacaga ttcagaggaa attcaaaaaa tcatcagatc ttactataaa aacctatatt 3780
   caataaaact tgaaaatctt caggaaatgg acaatttcct agacagatac cacgtatcga 3840
   agttaaatca ggaacagata aaccagttaa acaaccccat aactcctaag gaaatagaag 3900
   cagtcattaa aggtctccca accaaaaaga gtccaggtcc agacgggttt agtgcagaat 3960
   tctatcaaac cttcatagaa gacctcatac caatattatc caaactattc cacaaaattg 4020
   aaacagatgg agcactaccg aattccttct acgaagccac aattactctt atacctaaac 4080
   cacacaaaga cacaacaaag aaagagaact tcagaccaat ttcccttatg aatatcgatg 4140
   caaaaatact caataaaatt ctggcaaacg aattcaagag cacatcaaaa caatcatcca 4200
   ccatgatcaa gtaggcttca tcccaggcat gcagggatgg tttaatatac ggaaaaccat 4260
   caacgtgatc cattatataa acaaactgaa agaacagaac cacatgatca tttcattaga 4320
   tgctgagaaa gcatttgaca aaattcaaca cccttcatga taagagtccc ggaaagaata 4380
   ggaattcaag gcccatacct aaacatagta aaagccatat acagcaaacc agttgctaac 4440
   attaaactaa atggagagaa acttgaagca atcccactaa aatcagggac tagacaaggc 4500
   tgcccactct ctccctactt attcaatata gttcttgaag ttctagccag accaatcaga 4560
   caacaaaagg agatcaaggg gatacagatc ggaaaagaag aagtcaaaat atcactattt 4620
   gcagatgaca tgatagtata tttaagtgat cccaaaagtt ccaccagaga acgactaaag 4680
   ctgataaaca acttcagcaa agtggctggg tataaaatta actcaaataa atcagttgcc 4740
   ttcctctata caaaagagaa acaagccgag aaagaaatta gggaaacgac acccttcata 4800
   atagacccaa ataatataaa gtacctcggt gtgactttaa ccaagcaagt aaaagatctg 4860
   tacaataaga acttcaagac actgaggaaa gaaattgaag aagacctcag aagatggaaa 4920
   gatctcccat gctcatggat tggcaggatt aatatagtaa aaatggccat tttacccaaa 4980
   gcaatctaca gattcaatgc aatccccatc aaaataccaa tccaattctt caaagagtta 5040
   gacagaacaa tttgcaaatt catctggaat aacaaaaaac ccaggatagc taaagctatc 5100
   ctcaacaata aaaggacttc agggggaatc actatccctg aactcaagca gtattacaga 5160
   gcaatagtga taaaaactgc atggtgattg gtacagagac agacagatag accaatggaa 5220
   tagaactgaa gacccagaaa tgaacccaca cacctatggt cacttgattt ttgacaaagg 5280
   agccaaaacc atccaatgga aaaaagatag cattttcagc aaatggtgct ggttcaactg 5340
   gagggcaaca tgtagaagaa tgcagatcga tccatcctta tcaccctgta caaagcttaa 5400
   gtccaagtgg atcaaggacc tccacatcaa accagacaca ctcaaactaa tagaagaaaa 5460
   actagggaag catctggaac acatgagcac tggaaaaaat ttcctgaaca aaacaccaat 5520
   ggcttatgct ctaagatcaa gaatcgacaa atgggatctc ataaaactgc aaagcttctg 5580
   taaggcaaag gacactgtgg ttaggacaaa acggcaacca acagattggg aaaagatctt 5640
   taccaatcct acaacagata gaggccttat atccaaaata tacaaagaac tcaagaagtt 5700
   agaccgcagg aaacaaataa ccctattaaa aatggggttc agagctaaac aaaaattcac 5760
   agctgaggaa tgccgaatgg ctgagaaaca cctaaagaaa tgttcaacat ctttagtcat 5820
   aagggaaatg caaatcaaaa caaccctgag atttcacctc acagtagtga gaatggctaa 5880
   gatcaaaaac tcaggtgaca gcagatgctg gcgaggatgt ggagaaagag gaacactcct 5940
   ccattgttgg tgggattgca gactggtaaa accattctgg aaatcagtct ggaggttcct 6000
   cagaaaattg gacattgaac tgcctgagga tccagctata cctctcttgg gcatataccc 6060
   aaaagatgcc tcaacatata aaagagacac gtgctccact atgttcattg ccgccatatt 6120
   tataatagcc agaaactgga aagaacccag atgcccttca acagaggaat ggatacagaa 6180
   aatgtggtac atctacacaa tggaatatta ctcagctatc aaaaacaacg agtttatgaa 6240
   attcgtaggc aaatggttgg aactggaaaa tatcatcctg agtgagctaa cccactcaca 6300
   gaaagacata catggtatgc actcattgat aagtggctat tagcccaaat gcttgaatta 6360
   ccctagatcc ctagaacaaa cgaaactcaa gacggatgat caaaatgtga atgcttcact 6420
   ccttctttaa atgaggaaaa agaataccct tggctgggaa gggagaggca aagattaaaa 6480
   cagagactga aggaacaccc attcagagcc tgccccacag gtggcccata catatacagc 6540
   cacccaattg gacaagatgg atgaagcaaa gaagtgcaga ctgacaggag ccggatgtag 6600
   atcgctcctg agagacacag ccagaataca gcaaatacag aggcgaatgc cagcagcaaa 6660
   ccactgaact gagaataggt cccccgttga aggaatcaga gaaagaactg gaagagcttg 6720
   aaggggctca agaccccaaa agtacaacaa tgtcaagcaa ccagagcttc cagggactaa 6780
   gccactacct aaatactata catggactga ccctggactc tgaccccata ggtagcaatg 6840
   aatatcctag taagagcacc agtggaaggg gaagccctgg gtcctgctaa gactggaccc 6900
   ccagtgaact agactatggg gggagggcgg caatgggggg agggttggga gggggacacc 6960
   cataaggaag ggggaggggg agggggatgt ttacccggaa accgaaaggg aataacactt 7020
   aaattatata agaaatactc aagttaatta aaaataataa taataataat aaaaagaatt 7080
   actcataaaa taaagacaag atgatggata aaattaaaaa aacaaaaaaa acaaaaaaaa 7140
   accaaaatgg aaacatgaaa atgctcacta cttacaatag tgctttatgt aataagcaaa 7200
   tgactgcatt tgacacgagt tataatttat taacccctta gttagactac ttgtacacat 7260
   ggtgattatt aatccacaat tattcttttt cactaaaatc cttatatttt attctttgag 7320
   attataatat aatcacacaa attccctctt ccctgtcatc tcttcaaaac cttccatata 7380
   gtcctccatg ctgtcttttg aacgttatgg cttctttttt atattcttgt tttatattat 7440
   atatatataa tatatattat aatatatatc atactatata tattcctcag gactgaccaa 7500
   tccatattgg ataaccaatt ggtgtgctcg tctcagggaa agaccatttc cccaccatca 7560
   gcattctcgg tttccagtag ttcattgtct agaatggaga ctgtgtgtgc tttccccgtc 7620
   cactttagct gctgtgtttc agctcctctt gaggcagtta tgttggtgag acctcatctg 7680
   tgcggcttcc aacattcctc agagacacag tgagttacac agtgtcacct gatcctctaa 7740
   ctcccaccat ctttcctgct gtcttctcca gggtttcctg aacctcagca ggagcagggg 7800
   ttgtactgta gacacgccca ccttcactag gatccacagc tcctcacttt gatttgctgt 7860
   ggttttctgc aatggcttcc atgtcctagt aaagtcatgg agaccccata accacgctgt 7920
   actaaaccag acaatcccta gctgcattct aagttcttgt ccttatccca tacatgtgca 7980
   tttagagagt ctgcttattt tgctcattat agaacgagtc aatcattctc tttgagttgt 8040
   aaaagctt 8048

<210> 721

<211> 12728

<212> DNA

<213> Rattus norvegicus (Norway rat)

<400> 721
tgttgggaac ttctacagtg gaagtggagc tgtctctgac tctttttcca gatcttggga 60

cccttttcct cctcttgggt catctggcct ttatatgagg ttttgtaccc agtcttaata 120

tatatcccca ggaggattgc tctttgccaa aggaaaacag agaagtgatc tggggaagta 180

gggaatggag gactgggaaa ctttggtcat gatgtgttgt atgagaaaaa aataaattta 240

aaataaaatg acaactgtcc ctggtagtct aggagactta attaatgagc agattacaaa 300

accacaactg aggtttgaag gacaaagtag tctgcactaa tagttcattc agagaacatt 360

caggttagaa tactgtgtat ggtgtacagc atcattttat atagatgaaa gaatcatgag 420

ccataaataa ctcaaaaatc tgttacttca tgatccactg ccatgttttc tagtgcatcc 480

aaagatgcat agttacataa aaattataag gcttaaacta acatcttttc tgtattatca 540

gttagaggac aatgtgacac aaaaagattt cttcatcgtt taaaactacc aatggtccac 600

aatatctcta gaaccataac agaaaagtga atatgaggct gtaaaaaaga aatactcagt 660

attcttacgt gcatcgacat cttgtgatgc agagagggtg gaccagtgcc tgcactgcct 720

gaatgaacaa atactcgaaa caaatccaat aaaaatcctc atcttttttc ttcagaattt 780

ggcaaaatgt tttcgaaaag cattctccac ttactttttc acaaatgagt ggggctgttg 840

gaggactagt gatacctctt caggtgcact tgcctagcat gtgcaagacc ctgggcttgg 900

tccttggcac tgaaaatatt aacatacaca tacagtcaga atatctcaaa gttggttaac 960

tttatctgct gctacatttt tgcctccatg gaattttctg gctgatttga catgaaatgg 1020

gatccagatg ctgttctgct ctcattttcc aaacaggcca ttagcgctgt ggtgccagat 1080

cagaagttaa gtatttgcag cctagtgccg tctttggtct ggaatgagcg ataaatagtt 1140

ggtagtgctt tgcaaaaacc tttcccctac aaacaaacac gtgcaaacat gatctgaatg 1200

catgcccact atgctgacca gtgccagggg gaggacagct tcccaccccc aggactgacc 1260

tatgagttgg tgaggtattc ctgcatgtgc tcaccagcac tgactggtgc cagcaaccag 1320

agtattgaga aagcccagcc tcattcatgt caggtcagta gatgcaattt ctaagaggta 1380

aaggctccaa ggttcagcct ttgaaatgat agccaactgc agctaataat aaaccagtac 1440

cttaggccaa gggagaagag tgattattgg ctgatgagcc accctccttc tcaaggatac 1500

attataaaat ctttgtttct cttcagcaaa ttcctacaag tttacagtgg agccccagtg 1560

gcaccatggc ggttcttggc atcaccattg ccttgctggt gtgggtggcc accctcctcg 1620

tcatatccat ctggaagcag atctataaca gttggaacct gcccccagga cctttccctc 1680

ttcccatcct tgggaacatt tttcagctgg atttgaagga tatccccaag tctttcacca 1740

aggtaagggg aaacattgtt tcattaagaa agcaagttaa agattagata taatggatat 1800

atattaaact cattgtcatt tgttatccaa tgacagacta ccatgttact gaactaacag 1860

ttcagttcat cctgggcatg gaacactcac agtgaatagt gccatgcagg cccggagccc 1920

tctgcagtta aagtgactat gagatactga tgttgctggt atcattctgt gccaactgat 1980

acaacaacag gtgtaccctg gctggtatca acctgtgcca aagaacagac tgaaaaggaa 2040

aatactgagc tgaccgtgat tcaccagaat aacagcatct gccttttgct aatgctgtga 2100

acactgtgtg ggctaagcag ccccaaatgc tatcaggcat gggattaatg cttggtgtct 2160

ttgcacacag cagggtagct acaggtttca ggagcactga gtccaagtta gcttgtactg 2220

tcagataagg cagagcttaa ctttagaagc aaacaaaagg gtaatgagtg ctggcattgg 2280

ctgaataggc acagagaaga gaatcgtgtc tagatagaag gcaggtcccc cgcaacccag 2340

caacttaagg tggaaaaagt atccccctat cagacgagac aaaatgccct ttcccccaag 2400

tccgtctaac tcctgtgaag tacagtacag gagcaatagc aggagacacg gtctatctaa 2460

agcaggccga gccctgtcta tcttctgtgc attggtccct gcagttggca aagcgcttcg 2520

ggccagtgtt cacactgcac cttggctcaa ggcgcatcgt ggtcctgcat ggctacaagg 2580

ctgtcaagga ggtgctactg aaccacaaga atgagttttc tggacggggg gacattcctg 2640

tgttccagga gtacaagaac aagggtaagt tggcttctta ggacattggg aggtggggca 2700

aactaacaga aagtaatgta ggtaccattt gatcctctga atcgtagtga cagcaacaga 2760

gtaaagacag aggcaaaggc tcagatgatg ggtgagcatg aatgctgttt tgaggcttgt 2820

aatgggcaag cattgcgaac ccagtgacac ctccagaccc ccatggtcac catatccctg 2880

attgacttac agagaatgac agatgggttg tttctgaaca ggaagtctcc cccggagaat 2940

tcctcctcag gccctcgtgc tttcctacag ttcttgggac tttccactca gcctcagcta 3000

tctctgagct gtagctggtt ccatgcaccc aagtcctttc acttacctat ccttgcctgg 3060

cactctactt gtggaacgca ccaggcttta ggtgagctag agagagacag agagacagaa 3120

tgagagagag agagagagac agagacagag acagagacag agacagagag acagacagag 3180

agacagagac agagagacag agtgacagag agacagagtg acagagagac agagtgacag 3240

agacagagac agagagacaa ctgttctgcc ctgaatcttg ttcacctgtt ctcccctgca 3300

gatgggaatg tgggctaatt ttcccatctt cacagctcca tcttctactg agtgtgggca 3360

cccagaacca gagctactcc cagctcgctg gtgccacctg tctgcccagc ctccgtctta 3420

cctgcacctt cttacttcct ctttttcact ccaggtaccc aaagcagatg agaaataagg 3480

tgccctgtga ttctgcctga actagcactg gtgatacatt tctacaaagc tttttccttt 3540

tgtatgagat ttcagatcaa agcatggctg gctctagatc ttattctcag tgtctggctt 3600

ggcttcccag tttgtgtttc caaaactatt ttacctcaag catttgatgt cccctgatca 3660

atagtgtggg tttctgtact atctcggggt ggggggaaca aataactagc tcagtctttg 3720

tcctcaatat aggagttact gattgaagag ggaccatgca aggtgacaag gactgctggg 3780

aagggcacaa acctttatgt gccctctagt ccagctcagg caatgggcca gtgcaatcag 3840

ggtctgcctc ttcacaggct agattcaaaa ggcctttgct gctcctgcct ggggctccaa 3900

agtcatattt tcatgctatc tacacagaag agggtgcttg taaaatcagc tgggccttag 3960

gtaaagatgc tttgagtctc tagtgtccat tgtgtgaatg attgttctaa taacgttaag 4020

catgggtgtg cggcaaatgt accaaaataa gtgcacgcat catttgagca aatgctgaga 4080

aagtgtggtt ctgacctaga aagtcagcct actgaaaggc cccagctctg ccactctctc 4140

agccactggc ctttaacttt caggataaaa ctgacccaca atgcttagct catgtcacat 4200

atatcccttt cccctctcag gctggccata tcttgttcta gaaagttctt tagaccataa 4260

taagcatatg tctcctagaa cttccacatt ttctgttgtg tgttccacta ggcatctcac 4320

atgtgattag actgttaaaa tggaagagat tatcaaatta caagcaagct ctacacccca 4380

tgtagttata actcattata gtttgtctcc gaatactcag agcctagtaa gtgccttaga 4440

cacagtagat accaaaacat tatttattga agaaaagata ggaaacaagg agtagggagg 4500

tgttaaagaa agagaaagac ccctggagtg tagagaggcc agagggtttt actaggcaag 4560

agttgagaga aactgggcag ggctgtccct ggataccttg aactggttcc aaggggatcc 4620

aaaagctcca agagacaagt tttgaggaat gaaaaaaatg cccacaaaat tagctgagct 4680

agagataaaa agaaaaattc tttttagagt tgttgattcc cagtgtcctc atgggggtca 4740

caaggctatg ttctaggagg aaaaacaata cattatgttt ggaaggaaag tacaccgtaa 4800

catttcattg agagaaaata atacttggtc tttctatctt aatgtctcca aactgggaaa 4860

tcttctgacc ttttgatttt agtaaaagat gtagctttga aaccataaag ccttgagttg 4920

aagggcttat gttgagaagg ttaaaatcca tgagcttgga tatgctgctt aagatttcta 4980

tcagtagaca aaaagaggag agtccatttt tagagtggct gcctacagtt atttagcatg 5040

gcaagattca gagcaaagga tgaaaccaaa aaaaaaaaaa aaaagttcag aagaaacaag 5100

tatgaaacac agcctttcac tcccttttcc tgtgcttctc tgcacctgta catcccccgc 5160

ccccgccccc gcccccagta cccaagtgca gcttttaatg ctacttcaag ggttctttgt 5220

taatgtttct tttaatgcct ccttttccta caaacagtaa ccagttctct taacagccag 5280

ccctcctccc agcttccagc cctcctccca gcttccagcc ctcctcccag cttccagccc 5340

tcctcccagc ttccagccct cctcccagct tccagccctc ctcccagctt ccagccctcc 5400

tcccagcttc cagccctcct cccagcttcc agccatttca aaggtttttc ccaaccattc 5460

ctccctcagg tccccagcag tccacataaa cagtccttct cccaattttc ctaattctag 5520

cagttccaaa ctgtaacaac tattctagcc tatctgctcg gctctgctca gcttctcttg 5580

gctcacctgc cgacaactgt ctgtcttctc actctgctag cccaattccc tctcaactgt 5640

cccgtaacag tatcttctct ggctcagctc agcttcttct gcaactgtct tctgtttgaa 5700

ctctgatcaa tcttttctgc acagacttct cttctttagc ccctattgga tcttctttct 5760

gcaccaactg tcttctctaa ccctcatctc tgaccatact cttctaccgc ctggccccta 5820

atcccttctc tcagcctctg ctggcatttc atatgctctc ctcttcccag aagtccaaaa 5880

ggcaacagga cattgaaggt catagggcca agaagttctc ttaaaggacc aattgactag 5940

caactagaga ttctttaaag ggccaggcac acaacataaa tcaccctaca cccaagacca 6000

tttcctcctc cttccttact tctccaagaa ttgggtattt atagtatccc atcggtctct 6060

ttttcttagg gattattttc aataatgggc ccacatggaa ggatgtgcgg aggttttccc 6120

taagcattct ccgagactgg ggaatgggga aacagggtaa tgaggcccgc atccaaaggg 6180

aggcgcaatt cctggtggag gagctcaaaa agaccaaagg ttagtatctc agcagtccag 6240

aaggattgtg ggcaggctga acactgaaca acacggaggc agttggcagc tggggaaggc 6300

agaggtcaag ttggtcccct ggcactgcct tctcaatttg agtcccagct cattctagga 6360

gagcaaaagt ggctgccagg catgactgct gttcccttag tagccttaaa aagtaaagac 6420

acttggaggc tcccacattc actgtgtcct cagtgcaaac tcaagaagac tagaatggct 6480

agagaaggcc atggtgagat actcacactc tacctatttt ccaggccagc cttttgaccc 6540

cacatttctg attggctgcg caccctgcaa tgtcattgcg gatatcctct tcaacaaacg 6600

tttcgactac aatgacaaga agtgtctgag gctcatgagt ttgttcaatg aaaacttcta 6660

cctgctgagc accccctgga tccaggtgac catgcttctc ctttgccaca cgccttccgt 6720

cacgctgttc ctgttagagc cagagggaag gtcaggttgc ctaagagaga caggcctaga 6780

aaagcaaact acaggaacac acagtagggc atttgaccag gttggctcag catcttctca 6840

cttcaggact gtgtacactt gactgtaccc attcctacac acacgacttg gaaagatggc 6900

attatcccca aaagatgcca ttagacccaa tattctaagt agtgtggtaa agacaaccat 6960

aagttcattg tttagaggtt cctatcagtt acacagtttt gttctcctta gctttacaat 7020

aactttgcgg attatctacg atacctacct ggaagccata gaaaaatcat gaaaaatgtg 7080

tctgaaataa aacagtacac acttgaaaaa gccaaggaac accttcagtc actggacatc 7140

aactgcgccc gggatgtgac tgactgtctc ctcatagaga tggagaaggt acagaacagg 7200

acgggctcgc tgggatgggc agagtgcttc caggtcttac attatgaccc actctctggt 7260

gtccacgctg agcaccgtgg ttgcatcttg atgaccatgg tttttcataa acacacttgg 7320

cttcagcatg acaccattct cttctctggt cacttttgga tggcagagac cctctgttca 7380

acaccttcca ggccaagatg aactgcaact ctttgggaaa tctacccttg tctgagaaaa 7440

cttgagcata cactttgtta tttcttaatc aatcaatgtt gcttaaactt ccaaagttct 7500

aggcacccct gccttcattt tcctgcagcc tctaagactc ttgaatgcct accccttact 7560

ttcttgctgt aatggcttga aaccatgtaa aattctacac tgatttcctc tagataatct 7620

ccacttgagg attgctatca gctcagtctc tttctgtccc tggtccaaaa agcatggcct 7680

taagcctgaa aagaggctga gcctagcgcc tccagcttag aaggtcatta tcattatcag 7740

ataactctgg cacttaccga ggcaagaccc tgtggcctgg ttaagaaacc ctttcctgtg 7800

ctttgcctat ttcctggaaa acatcagttt ccctcgtcat gaggcagaaa gagctgtttc 7860
ctgatagcac aaggagtctg agaaaatctg gttccctccc tgatgaagag caataggcat 7920
cactttctta cacctaaaga gatcttacca ggtccctgtc tgtctccaac ttcacaggaa 7980
aaacacagcc aagaacccat gtacacaatg gaaaatgttt ctgtgacttt ggccgacctg 8040
ttctttgcag gaactgagac caccagcaca actctgagat atgggctcct gatcctcatg 8100
aaatacccag aaattgaagg tacatgaaac tcccatgcgt cacagctgag acttgtcagg 8160
gccactctgt gcaattcagg cctctgtagc tctcactaac cttcagggac ccagtcactc 8220
aatgaaattt tttaaaaagc acatgatgtt gtatgtgcca ggacctagca ccctgaagac 8280
tggctagtcc tgaacacact ccactgggat gtttatagcc cagtgctgaa atggaaaggg 8340
gactgagtct atgatctgct ctgtaaataa tttctgatta gaaaataatg ttccagtttt 8400
ccctgagaaa catctcctgg gtttataggc tgagacagga tgcgagccag gccagagtca 8460
tcccagagca tcttaacccc agggagctct gaagtgggaa ttgaaagaga agcaattgag 8520
gctgtcctaa atacatttca cttcctcatc aagactttga aaccaaaatc tgggcaggaa 8580
gaattctgtg cctgaagcag gcatgcaaaa gatctaaaca catttaccag atagaggaag 8640
tgtgatcacc cccacatgta agtcactggg gctcacctta gtagagaaac tgagagggct 8700
gacagcctaa ggtgaaggtg ctcaaaggcc tgaaacccta ccatcctctg agctgagctt 8760
agatctggca gctactgcat acaaagtaat gtttcccaag aacatggttg tgtgtctatc 8820
tagtccagga ccaagatctg agctctggcc ttccggtcag caatgccttt gactttccac 8880
accacataaa aaaagaaaaa aaaaaccaaa aggtgactaa agttcaagga ggaaaatgta 8940
caaattacag tagttagaac ttttactact atgctaggga aacataaaat ttaaaatgtt 9000
ttaaattatt agtataatga atttctaata tagtcaagga gcaagtaaat gtgacaggca 9060
cccatatatc aattaccaaa ctgaactcct attgacattt tgtgatgttt gctttggagc 9120
tgtttaaaaa ataaaccatt atacagcgag ctggagcccc actcactccc cagatacagt 9180
gatgcttcag ggccactatg tagcatcccc catgtgactt agacatttaa tgttaactaa 9240
atatcaacat ataacattat tttattttat tcatgcttta gtattatacc ataccatacc 9300
caacttattg caacttgctt ttttcactcc acgtattata agatccatag caaatcccac 9360
agcaacgacc aaggaacttg acaatggttg gttgctgtac ctctgacata gtcatggtga 9420
cttttagatt tcagaaacta tagtctagtc tgccaccctc tgcttatttt taacaatagt 9480
aaactgtcct agagtaataa tgcaaagatt ccccttagaa atttccaaga ctcttttatc 9540
tctactccag ctataaagag aattggaaaa acgatcacca actatgacaa cagatgccca 9600
aactgtcaac tgtgaataag ctcatgggtc tcagctttca ctgacattta taaggaaatg 9660
atttaacaat tgaaagtact tggggattac aaacgcagtt aagtgctcca taaacataca 9720
atgggtggta gatacatgaa gaaaaactag gtatgtaggt gaatggagct actggaggat 9780
agttggatgg atgagtggat gagtgggtga atgaggatgt aaggatggag aggtactatt 9840

ctggcgggta aatgaatagg taagtagatg gaaaagtgtg tgggttacag gggtagataa 9900

gtgagcaggt agatggaaat gtatgggtag atggtacgta gacatcaatt ctggaattaa 9960

cccttgagat tgaccttatt caaatcttgg cagagaaact tcatgaagaa attgacaggg 10020

ttattgggcc aagccgcgtc cctgctgtca gagacagact ggatatgccc tacatggatg 10080

ctgtggtgca tgagatccag agattcatca atcttgtccc ttccaaccta ccccatgaag 10140

caaccagaga tactgtgttc caaggatatg tcatccccaa ggtcaggcaa gagttggaaa 10200

gtactccatt ggcactgttc tattggttta tcaccttggt tgcccttcag gatagagact 10260

caagggcact gttgaccagc atatattgtt agatgtggga atcaagccag cattcattct 10320

tgccctgcag aatgtttcac ccaaagtcac cccaataacc tcatgaccaa agaaatgaaa 10380

gaaatggaaa gtgtcacctc aactggtcca gaggacttgt agaactctct aaaactgaga 10440

tcccatgaaa gcagcaaccc agcctgggac ccgtcctatc aaagatacag ggccttcctt 10500

ttcatttaga cctgatctga tccctgtctc tttttctaag tcatcctgaa ataaacaaca 10560

ctacaaatta aaaatatgct agcctacata tgtagaattc caacagccct gtccgctgag 10620

atcccccaga taaaagcctg tgtttaacct gactgataga gccctctact tctcctaggg 10680

tacagttgtg attccaactc tggactccct cttatatgac agccatgagt ttccagatcc 10740

agagaagttt aaacctgagc atttcctgaa tgaaaatggg aagttcaagt acagtgacta 10800

tttcaaggca ttttctgcag gtaagcagag tctgggagtc acaccttccc tgggaaagtg 10860

tcttcagtcc taaacttact tcccacgtgc tcttccccat cctagatgag gtctaccatc 10920

ccaactccat cactccttgt aatgtataac ccaagtacat tcctccctgt ttagaaagtc 10980

aaatggcaca catctcacaa ggaatctagg aaatgagagt tctgggtaga ttatcacaga 11040

atggatgtgg tttcctaatt aatttttcag ctatttctac tctaaccaga tgcagtaaaa 11100

agctctcaaa actgtattac ctaattcttc ataagtttca acaaaaattg ctaaactcta 11160

ttctttatct acctagattc ttcttaggaa gcatccagac ctcctcagat ggtcccatcg 11220

cagggcctca cctagggttc ctgtaaaggg agataccata gccagtttga taactttaat 11280

ggcctgttta cataggcttc acctgtacca tctggatgtt ttgggtctat ttccctaaac 11340

taccacttat ttatgcagtg gagttctcaa tgagtggtgt cagtagctgg tccctaagtc 11400

agtgccactg ccattcttgt tctctgacat atgatcactg gtgttcaggg accaatgggc 11460

agggatgatg aatcctactt taaggtggtg tgcctgaagc ctcctcccac actccaacac 11520

ttgatgttat cagtgctagc tttgcaaggg tccctcagta cctcatcagc actgttgtcc 11580

cttctaggaa agcgtgtgtg tgttggagaa ggcctggccc gcatggaatt gtttctgctc 11640

ctgtctgcta ttctgcagca ttttaacctg aagtctctgg ttgaccctaa ggatatcgac 11700

ctcagtcctg tcacagttgg ctttggcagt atcccacccc aatttaaact ctgtgtcatt 11760

ccccgttcat gagacctgaa aacttcctga tatcccttcc attgttatcc cttaaactat 11820

gactgttttt aaaaaaccca agcatataga gttcctatca taaaatgaaa ccgaaaaaat 11880

aaataaaacc ttgatgaatt gtattggttg tggatttaat gagaatctta tccccttgcc 11940

atgtgaaagt tccccaggga gtaagatgca tttcacagcc agtagtataa tcttccaatc 12000

tatctgtccc tgacttctca taatttgttt gcatatgcac agatgaccct ggctatctgc 12060

ccatcacaag acactgaatt aacaatatat tcaaagttct tttaccatac agtatcatat 12120

tcataggctc ttgggattat aatgtgagca gttttatgct gaccctactt tccagctcat 12180

ctaccttggg gtactactga aacccaaagc taacagacac cacaacagtg gcctcactga 12240

aggcgtttgg atcatcatag ctccacaatg gcaagattgc ttccagggga aaaaaatgta 12300

attttaaaat ttgctttggg gcagagatgt ttcaacttca tagattacca ttagcaaaat 12360

tctcaagtga caagggaaag ataggatgta tgtagctcta ggagccatta actaattgtg 12420

gtattatctg gctactgtct aaggttgaca cctggtcagc agagtcacca attatcttct 12480

ctcagtggta aagcttgaga tggttcaagg aagggtcatt aacgtcacaa atacagttcc 12540

aaaaaggcat gcttgcacat atctttaaac ccagcactca ggagacatag gcagacagat 12600

agatctctga gagtctaagg ccagcctggt ctacttagaa aattccaaac cagacagaac 12660

taaaaaaatg agactctgtc tcaaaacaaa gagacaaata acaggagtag agttccttag 12720

aagaattc 12728

<210> 722

<211> 19080

<212> DNA

<213> Rattus norvegicus (Norway rat)

<220>

<221> misc_feature

<222> (6102)..(6102)

<223> n

<220>

<221> misc_feature

<222> (6103)..(6103)

<223> n

<220>

<221> misc_feature

<222> (6320)..(6320)

<223>  n

<220>
<221>  misc_feature
<222>  (6321)..(6321)
<223>  n

<220>
<221>  misc_feature
<222>  (6322)..(6322)
<223>  n

<220>
<221>  misc_feature
<222>  (6323)..(6323)
<223>  n

<220>
<221>  misc_feature
<222>  (6834)..(6834)
<223>  n

<220>
<221>  misc_feature
<222>  (6840)..(6840)
<223>  n

<220>
<221>  misc_feature
<222>  (6843)..(6843)
<223>  n

<220>
<221>  misc_feature

<222>  (6844)..(6844)

<223>  n


<220>

<221>  misc_feature

<222>  (6846)..(6846)

<223>  n


<400>  722
tcaacggatc tcactcttga ctgtccagtg ccagcacctt agcaaactcc cccagcttgg      60

aggcagtggc ccaccacatc atggaggtgt cgtctgagca caggttccgg aagactgtca     120

aagtctgcct taataggcag gggtactcag gggtagcaaa ctgagaagag accacaggcc     180

aaggtgtggg aggtgaacca gtctctaccc accagccact tcatcagagg caccaagtga     240

gcctctaaaa cggaaggtgg gtgctcatgt gagatgcccc acacggactt tactactgtc     300

tcttccactg tggccagtga ctcaagaggt ggcagcagac agtgtcacat actcaggacc     360

tcccaccaaa gtgtgaggct cccagctatt cagccaaggc caagaggacc ttggtgtcac     420

agatggcatc tgtaaggaag ggcaggagct aacttctggt tcattcaatg acaaaggcaa     480

aggccatggt ggagagcatc ttgatacagt tgagacagag ctgagtgtcc tcatcacaca     540

gttcatctat gagcaccatg gtggggtaga gcgaatcgtt gccgttgaca gctgctgtct     600

tggctccttg gcatttcttt ttaaaaagaa gagaaaaagg tcagcagtct gttttactta     660

tgtttgtttg taaatacaca cactgtacta gaagcatgaa attcagaaag aagctgacag     720

taagatggat agggaggttg gttactgact gtgtattcac cttaaatctc tgagggaatg     780

tggagccagc aatgactgtg tgtttgtgtc tccaagccta gcccacaggt tagcttacat     840

tggtgtgagg aacacagtat tgtataacac catacaaaag tgagcagaga cagggcccat     900

gaaatcacaa gtagaaagtg tttgttactg ttccacctgg tcacccaacc aggtgtcctg     960

tgtgtctaaa agccacacag aaagacaaaa tggagtatgt ttggggtggg gcgaagagag    1020

tgaggtattg gcagcccacg tgcgaagggg attgaaggaa acatgggtaa gattggatac    1080

aagttgggtt ttcagctgtt tgaagaattg gcttgtagag gaaaggacac tatattgtgc    1140

actgtcttag gaataagaca caggaggccg ttgtatggag gaaaatgcag gggcttcacg    1200

agagtgagca aagacctgct agaggtcttc tgagtcccca tgctctctgt gtgtttatat    1260

atgcatatac atagcatatt aatgtattaa taaattacat atataataag acaagatata    1320

ttatgtatat gtggtgtttg aatatgcttg gaccagggag tggcactatc aggaagtgtg    1380

accttgttgg aagaaatgtg tcactggttg tgaactttga gaccctcctc ctagctgtct    1440

ggaagacagt cttctcctgg ctaccttgga tcaagatgta gagctctcag ctccttctcc    1500

agcaccaggt cttcctggat gctgccatgc tcctcccttg atggtaatgg actgaacctt    1560

tgaacctgta agccagcccc aattaaatgg tgtcctttgt aagagttgcc ttggtcatgg 1620

tgtctcttcc cagcaataag accctaagta agacagaata tattaactat ttatctattc 1680

agatgctgtg acaggttaaa ccattctctc tgatagggag aactcagcta ttggtaattt 1740

tagtataatt taattgaatt ttttagacag gatcttacat atctcaagcc ggcttcaaac 1800

tcttatatag ccaaagatga tcttgacatc tggattctct tgcctttgcc ccctgagacc 1860

tgggtttcag catgcgccat cacaactgga gtatgtggtt ctgggaattg taccaagatc 1920

ctcatgcagc cttaggtaag cctaccaact aagctttgtc cctgggcctc attggtaata 1980

tttagaatta ccaagtagtt cttttggtta gccattgact ggttctacag aaatcacagc 2040

tgtgaagtaa taattggggt caggtgattt gtagaagaaa tcttgatttg aagccgattc 2100

ttttggccaa ttgtcttggg gctcattagc ttacattcat agataaaggc tgtgataaag 2160

gctgtgactg ggaagtttcg aaatgaaatt cagaaagcgt ggcccgtctg cagcatgagg 2220

aaagctaaag atggcttaag gaacatcatc tttctccagg cacgtgctaa gcagtctagg 2280

actgagtagc cactgtcctt tctcctggga aatcccagat gggaaggatc aacccacaaa 2340

gcatttctgg cctcacggat gcccaagaaa aggtgctaag gaaactttct caacccttgg 2400

cagattcaac cacttatgat tttattagcc atggcttcgt catgtggttg acttgatgta 2460

cccttaaaga agtgctgcct agagactggt tcttagtgtg ggtcacggga ctctgctcat 2520

gcccacttct actttctttt cttggtaact gaagctgcct ggtcgcatgc tgagctcact 2580

gagccggcta atggcagggc tgtaggatgt gcaggaaggg caccaggtag acagtcagat 2640

gcaaagacat ttgggcagcc agagatgctg agcaaagctt aaagagaaaa aatagatcac 2700

cacaaagctc tttgtctcac tccagccctc ttccccacga ccacagaggg aagaagccaa 2760

gcctgagtcc tccgtcacag ctgcacatgt cccgtgactc agcacctcgc tcgaggctaa 2820

tgtggagtct ttagggtaag gaagtgggtg gtcctagtct gctctgcatg gtcaggtcac 2880

tcactcggca ttgataaact cagagctggg tttggggatt tcaagagcgg caaggtcaga 2940

aggtcaaagc ctggaagagt ccgcagatga agataagcaa ggagcttccg gttccaggtc 3000

tccaggggaa gccgactgag gcttcaacca taagatggac ttcgggagat ataactagta 3060

tttctttgtg tggttctgag gacagaattg agctcagggt cagcccttac agagcaggga 3120

gctccatcaa aaatggcttt cctaaagaat gaattgcttg caagggaggt agccagttct 3180

caggttggga gcatccaaaa ataaaagcag gaaaacctgg cggggaccct gagagacaag 3240

taggaagagg atcttgggtg aagggcagag tggtggcctt ctgcagtttc cctagaatac 3300

tgaaatccta gggcttgaga gatgaggtag ttggtaagac agaagcatga ggatctgaat 3360

tggatccctg gtacctgtgt acaacatggc ccatactcat aaaccagagc tgggagggta 3420

gagttgggat cctacagatt gttgggcagc tggtgagttc cataccagta agcgagcctg 3480

cctaaggtac aaacagttgg atggtgtaac atctgaggtc atcctgtggt ctcacacatg 3540

cacgtgtatg catttgcgcg cgcgcgcgcg cacgcacgca cacacacaca cacaaatcaa 3600

gaagtgcatt caagatttgt gtcagtttgc ttaaagctga attcccaagc ttacaaggtt 3660

tgggaacagg agaaaagtga ccacttgtaa ttgtgtcggg gacatcctta tctccctctc 3720

cccagatctc cctctgctaa ggcatatctc cctgcctgat taccaaagga cgacttttat 3780

agacactgct attgtaccct gtccctacta cacacaacgg gagggagctg ctgatcaaaa 3840

aaagttacaa ggacatcagt gtccatggaa aatgtagctg ttactgataa ttctttcttt 3900

ttatttttct ttataaagaa aacaaagaga agtttcgggg ggaggggcat tcctccaagt 3960

ttctgttgac tctgcttttc tggttagtta tcttcccaaa cttggcactg aaccagatta 4020

ggctaaagaa agtcttagag agggctcatt tcagattcat ctgccccaga ctgaagaaac 4080

taaggccaga gggcagagtg gtttgtctaa aggcactgga tgcctcgggg cacagctggg 4140

actgaagcca gccttagccc acttctgacc aagcctagac atttccagta ggctgccttt 4200

tgggaaagga ccaaatcacc atagcctctg ggccttggac aaatggaaat ctggcctcat 4260

ggctgttcct ttaaagatgt gagagggaag ctcggccctc gaccacacgt ttctccttca 4320

agccagtttt cttttattta catcgttttc aaggtctgcc gcccaaactg cgcgctgcct 4380

acatgggttc ctcgctattg cctgactctt gatacacaaa tgttccaggg tccagagtgg 4440

gaagcttggc ttctccccca agaagtagca tggtgggggg cttctaggtt gtttctcctt 4500

tccagagatg cctgtaacag atgtttctgc tcttagccaa aagcagctag gaatcctctt 4560

agcacctctg gacacagcta cttaatcagc agggctccat ggagaacttc cactatagct 4620

tcttgttcag gaaccaatgt gaaggccaag tgtgagacaa gtagtttttg tctaaaaatt 4680

tgtattatta tatattaatt atacacaatg agtatatata tatatatgtg tgtgtgtgtg 4740

tatatatatt cagcctcccc ccccatttcc ctttctcctc ccataatctc ttctctctgt 4800

tcctctcatg aatttcttta gggttgctca caggagcatg gagaggaggg ttattcacag 4860

gaacatggac acttaccagg gactatacta ctgaagacag ttgggtagtg tacttgaaaa 4920

taggtgtgag gttcctaggt ccagtgccag tgaagcccac cctgcctgac tgggttccaa 4980

gtgggtaaga accatgttta ggttttatgc atctcagaga caggggaggc cttctacgtt 5040

atctcattct tttttttttt tttttttttt tttggttctt tttttcggag ctggggaccg 5100

aacccagggc cttgtgcttc ctaggtaagc gctctaccac tgaactaaat ccccagcccc 5160

ggttatctca ttcttaaccc gtttctgatt ccttcatctg tctgccaagg gaccggcagc 5220

ccagtgcagc atttagggag ccaggtactt catgtatgct cttccctagc aagatgcctg 5280

taccatcttc agtttctttt actcttccat taaaatagga acaaagaaaa ggagtgcagt 5340

aatgggttaa tgtgaaagat gaacgtgggc gtcgggcggt gaaatcctga acctgtttgg 5400

ggctaggagc ggtccagagt agagtgtgga ttcctcttcc cttaaggctc ttctgttggg 5460

gcctgctgag cccttaggta cctagcagag aatagaacag ccatcaatct agctaggggc 5520

cctcaggcaa ccagcgcggt catttgtgat gcccctgcgc cccctggtgc ccccgctggg 5580

ctgtgcgcct ctcgtcccct cccgaccccc ccaccaggct tccagaaagc tcctagtgca 5640

ttccccggca ttctctggcg tgagtcacgc aggtttgcag ccagccaaag ggggtgtact 5700
tgagcagagc gctataaata gggcgcttcc ccggtgctca ccacccgcgt caccaggagg 5760
agcgcactgg agccaagccg cagacgggtg agacagctgc accttttcta ccccaacacc 5820
gaaggcacac cggcagggat gggagggcaa gggtaggagc caggtagaga gctgccgtgg 5880
gtaagtggga ggtgctagag ccccatcacc aggcagtcga gcttggggta gggggtgcac 5940
gctggctcag attcaggagc cacccccccg cccctgttct ttaacccgca tcacaattcc 6000
aaggctgttc aataggccac atgcaaagat cttccttttg caaaaatggg gaaactgaga 6060
cgtgaagcct gcagtgagat gcggatcgcg agggcgctag tnntgaacgc cgcgagtagg 6120
tgcggatctc tgtagtggct gttgtcttac cggcatgagt cattgcattt ggaccttccc 6180
tccagctcct gggacagcgc ataataaata agatgttgtg tatttaaaag gacgtgctgc 6240
agccatcgag acctgcttta ctgaacagaa tgcatgcatt cagttcttta gagaaatgct 6300
tccccaggcc tgagatgtcn nnnctaccgg atcattttat agatggtaat ctgaggactg 6360
gagacgataa gaggttcagc ctccaaacgt tctatgaatg agaaactgaa gccctttggc 6420
tttccagaat ttctagaccc agttttaaaa aacggtgctg gtgctgtttt taaaggacaa 6480
agttaggaat tcaagagttc atacaagttc tctaacagga aggtcttggg acaataccat 6540
ttagtgcctg accttgtgag aataagaaaa caaaccacaa atggtagttg gtggaatatt 6600
aggcagcagc aaatgtgtga ctttaaaaat atttgaaaac aggacctatg atggcattgt 6660
ttataaaaat gcattgaaag aaaaggagaa aatatgccac aatggaagag actgtgtttt 6720
cttactagat aaggtggtcc ttgcgctggt ggggctgttg taccagggta gttggcagcc 6780
aggcagtagg ccagcctcca aatgatgaca ggaagtgagt cagtgtagag gaanaggcan 6840
gcnngntgag ctgtgttgtc ctcggaccaa cagcaccaca gcacctagaa gcttgggaga 6900
caaacagatt ctgaccagag acagtattac tggcagacct caggggagtc tattcttaga 6960
tttacagaat agaggatcgc ttctttgaga tatcaggccc attttacaga taaagaaaca 7020
gaagctgtga gacttttgtt gtcaaggaaa taacctggga ggcgaatgac ttttgttgtc 7080
aaagaaagaa tattaaaatg aggttttgga gggctgcatt ctttcctggg gtgaccactg 7140
gcttgcagtg tgtcttgaga agcccaacag gtgtcttaga agtgctgagg ctgtctctct 7200
agaccccagg ctccttacag ctcatcttgc tactgcttcc cctcccattc agtgaccttc 7260
cttgctgcct gcttcctctt ctgggcattg tactgtcctc acatcagaag ttatttccaa 7320
ggcagttaaa tggtttttcc aaaaatggtc acacttaagg cctctcccac ctacagatat 7380
ctctgtggct ttgtctctga tgtggcaagg ggtctgtccc tgcctggggc atttcaggcc 7440
tcagtgagca cctgggaggt ctatgagatc agggccatcc tggtggatca tgaaccaggg 7500
cgccagccac ccaccatctc tgggaaagtg cttgtctctt ctcccacacc actcctctgc 7560
tctgtgtgca taattatgcc tcaggaaagc cttgcctatt aatgatctgc ccagtgtcct 7620
gatgggcctg cctagctggg agcaggtttt aagagcaagg catttcagga agacagatcg 7680

ctcccctttc cctgttcttg tggctgtagg aagctggtga gcccagataa gccattctgg 7740

gcagggttag agatgcaaga tgggtgtgtg tgtgtgbtgt gtgtgtgtgt gtgtgtctct 7800

ttatgcaacc aatccagctt ctgggaaagg atccattgtg taatactggg cttcggcctg 7860

tgtccctggc tcaggcagcc ggcccagtgg tggctggggc accttggcag agcatgctag 7920

ttcaggctta gagggagcag gcatgtgaaa gggatttgct ggagccaatg ggcttgtcct 7980

cctgaagggg ctttcctggg ggctcactgc taatctctgg ctttgtcctt cccttttcta 8040

gtgctgcctt ctctacagtc ctaaacagtc cctcccttcc ctttttgatt ttacccaact 8100

cccctcccca gtccatactc agcctggcac aagcttcacc cagagatggt gcattgggtt 8160

gaatttcctg tgctgatgga agggcgtgca tattaccacg gactcctgaa aagcacactt 8220

agacagcctc gggaggctgt accgtaaggg gagcccagga ctgtgttgtg agtgaggttc 8280

agtcccatgg aagggattgg cgccagccac ccaccatctc tgggaaagtg cttgtctctt 8340

ctcccacacc actcctctgc tctgtgtgca taattatgcc tcaggaaagc cttgcctatt 8400

ctgcctcggg gatttatggg aagaaaggac tctgcttgga gcttgtccct tccaactcca 8460

gatagcctgg gtcctctgtg cggctgaacc atggttcttc ccagggcccc actgcttcca 8520

ttgtcttttg aaggcatgaa tttaatcttg tttttgtccc cacgttcttc agaagtaaac 8580

tgcagtgatg ccagatgggg aaacagatga gggccatgag ttccaatccc atcccacctc 8640

ccctctcagg agcagaaaac ccctaagctg cccagaggtt accatgacat tctgctgttc 8700

tttgcaggac tccagactcc aaagaggcca caccatgaag attctcctgc tgtgtgtggc 8760

actgctgctg acctgggaca atggcatggt cctgggagag caggagttct ctgacaatga 8820

gctccaaggt agactaaact gctgtccctc tggtgggtct ggcagagcca cccctcaaag 8880

ttcagtccct aagtgctgag ggctgtttgc atactgcacc tgggactgtc catgtcctct 8940

gcactctcag agtctctaag ctctagctct tgccttggtg tttttgtggt cagatgcctt 9000

ctagacattg gcctctgtct caagcttctc tgttggctgc atgccatacc tttgtttcct 9060

ctaggtcccc ttacctggct ctcctattct tctctcaaag ctgtgtccgg accctttgaa 9120

agtttgtcag tctccccttt gtgtgacaac cagagttctt accaatgtgt ctccaagctg 9180

atttccccct ggaaaccttg ctctaccagt ttccttctct ggaaaagggc agcacagttc 9240

aacatgaagt agtcaactga gagcgttctc tcccttcctc cccatcttct aatgcttttc 9300

tggaaacccg cctttccttc cctccttctc tcctaatctc cgctcatagc tgaccagttc 9360

tgtgagaatt tcattaaaaa taatatgatc tgactctctg atatggatat gcttggtgaa 9420

atgactgctg tggtcaagcg cttagctgcc catcacctca acttattgtt gggttcagac 9480

acgtgagatg cattattcac ttagtgcacg tttttttttt tttttttttg gagctgggac 9540

cgaacccagg gccttgctgt gctaggcaag gttctaccac tgagctaaat ccccaacccg 9600

acttagcgca cttcaagggt agaacacatt gatcaaccac gagctctgct tggaccgtgg 9660

accttcaggc ttagtcatgc attgagactc ttgcccttgc tcggcagccc cccccgcccc 9720

catgcccttc tgtttggaag tgtttggcag tggcatcagg tagaacacag cagaggtgtg 9780

ggaaatggca cttgacacag gataagtaag tggtaccccg tgacataatg tgtcactgcc 9840

ttgtcaccag ggctgagtct tggttacggt ctctatggtg aatgcagctc ttcatttatc 9900

tccccgtctc catggtgttt ggtgccaagt cactcatctt ccggtcacca tcacctgaca 9960

ctgtgcattg cagcaaagaa gtgccaagac tgggttaacc tgaatgtcac aaagatcgtg 10020

tccaaaatgg cacttttatt atattttgat gcttaggtta actatctttt ttttctgtat 10080

ttccttgaaa tatcctctgt ggctgttggc tccccagggt ggcatggtgg ggtcagtcat 10140

acatagtatg gctgccctct ttgcaccact cctgtgcttc tgagctgtga cgcagggtct 10200

accatggacc ctttgatgag accaagggac aggactgagg ccacaggact gccacaggct 10260

gggggtagta gtgacagcat ctcacgttga cagcctccag cactcccatc agagcatggc 10320

tttctcaccg tcccgccggc ttccaggcta tttctccagc catttcttcc cttctcaact 10380

cactgtacct tcctgtctct cacagaactg tccactcaag gaagtaggta tgttaataag 10440

gagattcaga acgccgtcca gggggtgaag cacataaaga ccctcataga aaaaaccaac 10500

gcagagcgca agtccctgct caacagttta gaggaagcca agaagaagaa agaggtaggc 10560

ccagccttga ctgagccctg gctctccctg ctggaacacg gtggcaggtg ccttgctggg 10620

ttgccatgtg tcctggtgca gtctttgcag ggctttgcta atactgggcc agactaagga 10680

gccaggaaaa gcacattcgc ttgtggagcc tcagggctga ggtcttggat ctggtttcct 10740

tgcaagtctg cccaggcctg aagccaagat tcatttcaat aggtttttct aacccttgct 10800

gtgtgcaggg catggtgcag gactcgggag aggggtcatg gcagcacctg gccacttccc 10860

accactcttt tcttctgtat tcaaagtggc tcttaacatg aatgtgctta tttttaacat 10920

gaatggcaat attttaaagt taacgtaaaa tcctcatgat ctatccttag acatccattc 10980

atccccactc agcttcgggt gtggggttgg ggaccgtttt caaagccctt agcagaggct 11040

gaggttatgg gtagaaccag gccctctata gattgtccct acctgtgata atgcttactt 11100

tataaattac agacagtagg gtaggagcag caacgatgac gtagaatagt tacaatgtta 11160

caccaaacaa aaggttatgc agatgcaatt tctcccactc tcagaatatc tcagttcctc 11220

agcctcagca acactatttt tagactgtgg ttgaccatgg gtagctgaga actgataaca 11280

ggggactgcc ctgttaccat ttgtttaact tgccttttct acatgtggac gtctatgttg 11340

accctggtac acacacacac acacacacac acacacacac acacacacac acacacgaac 11400

acacatacta gaacttatac acagatccat ccactcattt attttccata tctttatgta 11460

gacgtacaca tttgctgtaa actaatatct cttcaggtgg agaatttaaa ttttttattt 11520

tggtgatccc tggagccaat aattgggtca gggggcatgg taatgaggtt gtggtcgatt 11580

tgacttcagg ttggatagct ttgatttgca catcaggggc cacggttcta atagaaagag 11640

aagactggcc tctttgggga gatgagaaga gacagtgagg tggcataagg gagagatggg 11700

tcctgaagaa gagacagtca gagcagcaaa tcagagcgga cagggaaaac ttagagaaca 11760

gctgggcctt gaaggatgag aggcccctgt gagcggtgtg ggacgccatt gacggatggg 11820

ctagagcaaa gctgtgggct gagtagcacc ttgtacacag atgggagaga tcggctgggt 11880

ctctgtgtgg gtcacctcac cttgtcatct ccatgaaggg tgctctagat gacaccaggg 11940

attctgaaat gaagctgaag gctttcccgg aagtgtgtaa cgagaccatg atggccctct 12000

gggaagagtg taagccctgc ctgaagcaca cctgcatgaa gttctacgca cgcgtctgca 12060

ggagcggctc ggggctggtt ggtcgccagg tgagaaggag acatgtccat ggcctgggtt 12120

ctggatgggg agtgtggaga atccacgaaa taggcctcac tccatatact gggtacagtt 12180

gattagcgtt gcttgactgc atgcctttct agtgtcgtgg tcgattggtg atgtctgaca 12240

cgagtacaca tgtagccaat catcatgggt atgcatgaca gtcttacaag acctttaacc 12300

agctgggtaa aatggcctca tggtatggta gaaccccatg ctcatatagg aaaggggggc 12360

taagaatatt tagtatattt gcatcaattc tatccctacc tgtgagcttc actggttggg 12420

actcagtgaa actcactgtt tcctgaaggc tgagcagcca agtgggagcc gctgtccttc 12480

atgaccaaag atgagccctc tgagtttcac cgtgagaggt ggctgcaggg cctgacaggg 12540

tattcaggag gcagaacagc gggaacttgg gtgagctgct gtgtggtcac agggtgaggc 12600

aggctggggt gcacaaggcc ttactgtact tctctgggtc ccagctagag gagtttctga 12660

accagagctc acccttctac ttctggatga acggggaccg catcgactcc ctgctggaga 12720

gtgaccggca gcagagccaa gtcctagatg ctatgcagga cagcttcact cgggcgtctg 12780

gcatcataga tacgcttttc caggaccggt tcttcaccca tgagccccag gacatccacc 12840

atttctcccc catgggcttc ccacacaagc ggcctcattt cttgtacccc aagtcccgct 12900

tggtccgcag cctcatgcct ctctcccact acgggcctct gagcttccac aacatgttcc 12960

agcctttctt tgatatgata caccaggctc aacaggccat ggacgtccag ctccatagcc 13020

cagctttaca gttcccggat gtggatttct taaaaggtca gaaagcattc gttgtctttc 13080

caagacggaa tgggagtctc caagatgttt aggctctgct agggcttgac atgtgtttta 13140

tgttcccagc agcccctgag gctggtattt tcccccattt gcagggtaaa ggagcatgga 13200

tgggggtaaa gcaatgcaga aggcaaactg ggacccagtt caatctcaca cttgtagagc 13260

ttggagcctg tgttctttac agccagctca gggtgtgatc cagctgtacc tcctctagga 13320

ccctggcttt tctttcttgg gaacttttga ggatgtggtg ttggaggcct gcctttccag 13380

gcaacaggtc tgcgtcatct taaagaagat ggtcatgact gcggcctaag catgtaacac 13440

ctgtaagatg aaatgtgatg tggacacgag aaacccgtgt ctgaggagtg attttcttca 13500

tcagtgtgtg tcctaatgcc gtgtccttca cgtatggttt gcacgagaaa cataggaagg 13560

cctggcaccc aattcacgtc tcttgtagct ctgttatcta aggatggtgt atgttgtcag 13620

gacacacttg tgctttctgg ggcttgatgg ccagttaggg agtaccgcca gccaaggaga 13680

atgaagaaca gccacgatgc cagaaggcac acagctgagg gaatgtgtga gatctctcag 13740

agggagatgg tgagaaggca ggcagtacct cgtaagagcc atgggaatat ggctagggag 13800

ccccagggaa atcgcatgtc tgcaagcgaa cgactaagct tcgtcttgcc tgaccacagc 13860

gctgagcaga ggtgtgggga ctcgggggtg ggtgtctttc caatgtgaca cgtgcctcat 13920

tctgcgcccc tttctcctca tgtgtccaga aggtgaagat gacccgacag tgtgcaagga 13980

gatccgccat aactccacag gatgcctgaa gatgaagggc cagtgtgaga agtgccaaga 14040

gatcttgtct gtgggtgagt cgggaaccag tctataatct gggcatctga ctaagggtca 14100

ccagtgacta actggtactg tccttcagag actagttctc tgcctgtgat ctgtagtccc 14160

tgggagactc ctccctggtg catagcttac tatgctcata cctacagctt cgttatttgg 14220

gagctggggt agaacccgtg cctcatgcat gctgggtgag tgctctaccg ttgagccacg 14280

ccaattttac ttagaatcaa atccaggggc agatttttct caagtagatg aggtctgtct 14340

aaagcaccca cacaccctgg tggctcatgg caaacctgca acccgcggtg gatatctcca 14400

tagaatttaa ccgtagccac cagcaatctg cagctcattc ccctggttgc tttgttccac 14460

agtgccaagt acagtagacc ctgcttttta actttagatt atgaaatctt tgacagcatg 14520

cattttgttt aatcagtgtt ccacatgcct ggcactgttg cgaacatgtc acaggtgttt 14580

gttctcagaa tgatcttgtg agcctggttc agctttcttt acattttgca gaatggctac 14640

atgacttatc taagattaca tgacatgaga catctcactt aggctgtgct caggaacagc 14700

ctgctggggt ttctccctgg gaaccctgag ctatgtcctt gcgatggtgg tccttgatat 14760

cctggccaca ttcctttggg ttgattagtc cctcagaaag gaactgtttt ccttcatcgg 14820

cttcccaggc agtctgggta tggcggcggc acatccctat agtaccaaca tctgggaggc 14880

tgaggaagga gggtggtgaa tctgaggcca gtcttggctg catagcaagg ttctgtctca 14940

gtgaaaacaa agtacagcaa acccagaacc gttccccaca gatttgtcag gtctggcggt 15000

caatcaaatg tttctgcttt cagtccggtc ttcacagtgg ctcacggtat acagcgcttc 15060

ctgctcagat cacaaggagt taatcgtcct ctttgttgtt ttgtcatatg tgtagttggc 15120

agggacagac gttcagagtc tggttaaata ttcccagcct cactcctgca atgttaggag 15180

cccaggaaag atacattact gctttcaaag gttatagtgt gaaattataa acaaatatgt 15240

acccacacca gcaggccgac ccccaggaaa ccgtcaggga tctcttccat caggcttagt 15300

gaccctctca acagtcaccg tttctgtcac ctgctagctc ctgtgtacgg tgcttacaat 15360

gacagggagg ctccacaggg acctctcatc tgggacaact tgactcagcc tgcgggtgga 15420

cccgaacatg gctgagcatt taacgttttt ggcttctacc tgccatgtgc caagtgacaa 15480

tcagaaatat cttcagaacg tgtggaatgt ccctggtggg tagtgtggtc ccaggttgag 15540

aaccacagtt ctacaccact ggtcccctcc aggactcttc cttgtttggg ctaagagtaa 15600

gggtactcag gctattcctc aaagatttct tctcatctgt gctgcttgtc tttaatcctc 15660

tttcttgccc catgcaacat tttatcttat ttagtatgaa tgattgcagc tgtcacgtgg 15720

ggcacagagg ctcccttgtc ttgcttagtt ttgatacaca gactctccta gatgttttaa 15780

aaatagtaga ggagggggtt ttcagaaaaa atggttttca gaaaacatgg gcattttgta 15840

ggacccagat gcctgatacc tgcaatgtac tcatggacca gagggaagac accagttttc 15900

ctgttttctg taggagcctt cctggccagc tgtgggtgga caagggcagg caaggaccga 15960

ccctcagcac cggatgcctg tgttttgtag cctaggaacc tggctgtctc ttctgcagct 16020

tgggttctgg gaaaatggta ttgtggaaat ttctcaaata tgttccaaaa taagcttgga 16080

gaatgttcta gaaccatggt ctcaggggag gaagagctca ggttgaggag ctgaagatgc 16140

gggttcagtc ctggttccag agattgcttc ccctctagat ctgggcaagt tccttacttc 16200

caaaaccctg tttcatgact ggtaaatcag gagtgttgat gcttgtgtca agcccagcat 16260

actttgaggc acacagtagg tccacctaag ggaataatgc tcctcttctc tgtggagtgt 16320

ccagtgcaac agtagcttcc ttctacttgg aaatgagcta agtggggagc taagggaatg 16380

agaataactc caggtctgag aacatggagt agttggtgtc agtactctcc agaacatggt 16440

cagtggcatt gtgacaggtc atcgagattg gtcatgccta ccacccagag aggattcaac 16500

cacactgctc caacacagcc tagcatgggc actattgcct cccaaagcgt gaagggagca 16560

gccccttgat ttccatgtca gggggactgt tctgtgggaa ctgtaagaac tggtttgtag 16620

acttccattg aagagctcct ctgcttcaga tcccagagct ggagccatcc cccttaacat 16680

ctctctcatg cttctgcctt ctgcttcttt cttccctggc cagactgttc gaccaacaat 16740

cctgcccagg ctaacctgcg ccaggagcta aacgactcgc tccaggtggc tgagaggctg 16800

acccagcagt acaacgagct gcttcattcc ctccagtcca agatgctcaa cacctcatcc 16860

ctgctggaac agctgaacga ccagttcacg tgggtgtccc agctggctaa cctcacacag 16920

ggcgatgacc agtaccttcg ggtctccaca gtgagtagta tcccagttat ttgatgagct 16980

aaggcccacg tgtgatccag aggtgatgga tgcctcttag aggtgccgtg ggacaaaggt 17040

ttacaccatc aggaactggg aaaaccatac tcgcgttgta gcattgaaat gccttcaccc 17100

ctcgaggtca gatatccaag tcttatttcc aagatgagga aagagagagg ttctgtggct 17160

tgctaagtag acggaacttt gggcagcttc cctgagtcag cctgtcagct gctgctttct 17220

gcccagggcc atgctgagtg ccgcatgaat tagtgttagc cagtgagatc ggcttccttt 17280

agtgctcaca gcccccctcg tcctctttcc accatagact ggctagcatt tgtacaaagc 17340

aaatgttggg gtcaggtgtt gtggtgagtg tctgtaagcc ggcaccaaag aaactgaggc 17400

aggaaagtgg agatcttgag cccgggctgg actatacagt gaaggcctct ctcaaacaag 17460

gcaaaacaag aagaaaacat ttgacaatca tttcaagttt ctggggtgcc tacttggcta 17520

gtcagttttc aaataacatc tgtcccttca aatcgagata gttttgctca gaattctctg 17580

aacatcttga gtagcaacag gtaacctgga gctccatact cggttgcagg ggcaaagaga 17640

agggcacgct ctgtagacat tgtgcagaga cacttctcct gccttcttct aggtgacaac 17700

ccattcttct gactcagaag tcccctctcg tgtcactgag gtggtggtga agctgtttga 17760

ctctgacccc atcacagtgg tgttaccaga agaagtctcc aaggataacc ctaagtttat 17820

ggacacagtg gcagagaaag cgctacagga ataccgcagg aaaagccggt aagcgggcag 17880

cctttctgtg accctgaggg ctgataaaca agtctttcta aacaagaccc tggaggactg 17940

gaaagtccga aggagcccag agaaggagca aaatatgcat tctggatgca gggcctatcc 18000

ctgaggccag accccacggg tgagctagga aaacccctgg ggattttatt ttctttgctc 18060

tgggaaatat ttttcctcag tggcaaggat gtgggtgttg gtggctgctt cttgggggga 18120

tattgattct cttctctact ctcctttttt ttccagcatg gaatgagaca gaagcatcag 18180

ttttctatat gtaggagtct caaggaggga atctcccagc tttccgaggt tgctgcagac 18240

ccctagagaa ctccacatgt ctccagcgcc taggcctcca ccccagcagc ctctccttcc 18300

tctgggttct gtactctaat gcctgcactt gatgctctcg ggaagaactg cttcccccac 18360

gcaactaatc caataaagcc accttgcgta tagtgtgcct tgggcccgct ttcatatttt 18420

catacttgat tctcctgcag gccgcacatc ttcatttgat atggagagaa aacaaggaag 18480

tagctgggtc agttcgtaaa gcacttgcca catagacatg agtttggtaa agtcaggcat 18540

ggtggcaccc tcttgtaact taatctcggc acctcagcgc tctcagagca gagggtccct 18600

ggagctggtg ggtggtcggt gagcgtcagg ccccagtgag agatcttgtc tcagaacaca 18660

ggtatgagga gaccaaagaa tgacacaagg ccacacatgt agaagaaatc tcgaagttaa 18720

cagctgagac tgtgtaagag tctccatata ctggagagta aagagtactc atgacaactg 18780

gggggacctc tggctttggg ggagaggttt gttccaataa gctgctgagg caagttcctg 18840

aagccagaaa cttttccctc ccaccacaac actgtccacg agttctgaca aagtggggca 18900

gtctcccaga gaatgcactg aaattaaggc aggaagagct gctagtggga cacatgggtt 18960

aatgtgtaac tgacaatttc ctgtggaaac tgctcagcag aagccaaatg aagaggcagt 19020

tttcttgttg tttgtaagaa catgggaacc tgggaccact tccggaccat ggcgtctccc 19080

<210> 723

<211> 602

<212> DNA

<213> Rattus sp.

<220>

<221> misc_feature

<222> (564)..(564)

<223> n

<400> 723
gacaaggaca aaggcttgtt ttattttaat gactgatcca cgtgaggcca cagcctggcc 60

actatgtaca gacacgaggg acgcttcatt tcttggtctc ttcctccttg gacagagtct 120

tgatgatctc ctccttcttg gcttggaggc gctcctcccg acgctttcgt gcttccttgg 180

tcttagacct gcgagcctca gcctggtcag ccagtagctt cttgcgggcc ttgtctgcct 240

tcagtttgtg gatgtgctcc atgagaatcc gcttgttttt gaacacattc cctttgacct 300

tcaggtacag gctgtgatac atatgacggt caatcttctt agattcccgg tatctcctga 360

gaagccggcg caggatcctc atccttcgca tccaggtcac cttctcgggc atccgagcgt 420

tggcagtacc cttcctcttc cctatgccca tatgcctgcc cttccgtcgg gccaaggtgt 480

tcttccggca tcgagcccgg gaatggacag tcacaggctt ccggatgatc aggccatctt 540

gatcagcttc ctgatctgtt gacnaagttg gcattggcaa gttcgttggt ttcattgggg 600

tc 602

<210> 724

<211> 386

<212> DNA

<213> Rattus norvegicus (Norway rat)

<400> 724
tttttttttt ttttggtgaa agattttttt ttttttttta ttggtaatgg ggtctttaag 60

tagtctgaaa ctaacactca agaaatcttt gctagttagg atctattgcc aggaaacatt 120

ctcagacctt cttatgtatg agattaaggt caacattcgt tggtgagcgg agatcactgt 180

gataggcacc ttacaatatg gcaaagggct gaaatgatgg cctcactttc catgatgtgg 240

gtgtagccag cttgtcacag gtcacaacaa agagctctgt ggaacaccgt tccaatcagc 300

agttttcctt tgtacacagc ggcgactgtc gtaccttgca acaccgtgcc attctctgaa 360

taaactacag ttacttcctc gtgccg 386

<210> 725

<211> 445

<212> DNA

<213> Rattus sp.

<400> 725
actttacaag ccagatatat ttttaaaaga tcatgtttac aataggtaaa ttacgtagtg 60

aagaaatgtc aaaatgaagt tgagtaacaa aggcacattt gaaagtttaa gagtacaaag 120

agtcccatgt ttgttctccg agcataggag gaaagggaga cagatatatt acaattacat 180

tctcaggggg agggtttctg tcagtggaag tgattaacac tggcttcttt tctcccctct 240

ctggggcagt cttttccttc cttggcttcg gacagacagg ttaaccttct gccatgtaga 300

ggcgatacat cagagctacc accatggctg agatggctgg gatcacccag ttggtccacc 360

aactggaatt agactcgaca gtagtgataa gggtttctgt actttgccaa gacagcttgg 420

gaatttggct tctgacagac tccca                                          445

<210>  726

<211>  552

<212>  DNA

<213>  Rattus sp.

<400>  726
gaatgaaata aaggtggttt aataagaacg tttcacggca tcttcccgag ttgctaacac     60

ggtttataga gcaagaacac tgtttcagta caaatgggaa gctactgctt tggcaagatc    120

ctggtcctcc tgggctgggt ccctcagttc tgggggttac tgacgacagc agtggtgctg    180

taggagtacg ggctgagcag ggctgcgatg gtgtagtggc gatgaccaga gtcattggct    240

gtgaaaacca cctctgcgta ttcatggaat ggggaaatgc caagagcctt ccagtacgat    300

ttggtgtcca gttctaccct gtacacccct tccgtgaact tctcatctgt ggtgagcccg    360

tgcagctctc cagactcggc ggtcttccca gaggcaaacg gctcccagct tccgtctgca    420

gtctttttga acactttcac ggccacatca acagcagggc tgcctcggac agcatccagg    480

actttgacca tcagaggaca cttggattct ccagcacccc cagggccagc ttcagacgca    540

aatatcagtc ca                                                        552

<210>  727

<211>  479

<212>  DNA

<213>  Rattus sp.

<400>  727
gaagagcaca agtattttta tttctttctt ttttttttct tttttttcct ttttttcgga     60

gctggggacc aaacccaggg ccttgcgctt gctaggcaag cgctctacca ctgagctaaa    120

tccccaaccc cttttttatt tctttaacaa tgcaaaatat acacgttgac ttgactagga    180

catattcaca tgtaaatttt ttctcatatc cacaactctg gatagatcag aggaatgaca    240

aagatcatca caggaagcag actgtagtat ctccaaagtt tattccagtc catttcttct    300

tttatactcc aaaacatctt cgttgtgttg gtggatgaga taaatccaca tgaaaatcga    360

ggtgcccaag gtcagcccaa ctttcaccca gttaggtttg gcattgactg gctcccggac    420

atagaacttt gaccgtgttg aagagcatct cggcagcctg gcgggggcca gcagccgga     479

<210>  728

<211>  377

<212>  DNA

<213>  Rattus sp.

<400>  728
gcgcaagtta ggttttgtca aagaaagggt gtaaaacgca gctcagtaac agtccgccta      60

gaagcatttg cggtgcacga tggaggggcc ggactcatcg tactcctgct tgctgatcca     120

catctgctgg aaggtggaca gtgaggccag gatagagcca ccaatccaca cagagtactt     180

gcgctcagga ggagcaatga tcttgatctt catggtgcta ggagccaggg cagtaatctc     240

cttctgcatc ctgtcagcaa tgcctgggta catggtggtg ccaccagaca gcactgtgtt     300

ggcatagagg tctttacgga tgtcaacgtc acacttcatg atggaattga atgtagtttc     360

atggatgcca caggatt                                                    377

<210>  729

<211>  428

<212>  DNA

<213>  Rattus sp.

<400>  729
attttgcata cttttaattt cagaacaaaa taaaacaaac aaaaaaacac aatacacaac      60

acccaaccct gctattctgt gggaggggat cttcctgcct taaggcacaa ggacaggata     120

gaagagattc ttgtagacat gagcagagag ccaggtgggt cagggccagt caaggctgtt     180

gggggagcca cagggatact atacagaggg cgggggcttc ccaggagcct ttaatccact     240

tcttccatgc gagaggcatc ctctcctcta agtactctgt ctggtcttct ttgaggtgaa     300

ggatcacttt ggtaccccgg ccaatgggct caccgtggtc tgcacggaca gtgaaggatc     360

caccagcaga agactcccag gcatactgct catcatcgat gtgcttcgtg agtgtttctt     420

caccacct                                                              428

<210>  730

<211>  1052

<212>  DNA

<213>  Rattus sp.

<400>  730
gcagcgggga ccttattgga ctatctcccc ttaagtggga agggcttagt caaatgcagt      60

aaagagctat aaaacaccga gaactcttga tgtgttgtga aacttagagg gagcagcttt     120

ttaacaagag aactcaagca attgctgcca tgccggggaa gccagtcctt cactatttcg     180

atggcagggg gagaatggag cccatccggt ggctcctggc tgcagctgga gtagagtttg     240

aagaacaatt tctgaaaact cgggatgacc tggccaggct aaggaatgat gggagtttga 300

tgttccagca agtgcccatg gtggagattg atgggatgaa gctggtgcag accagagcca 360

ttctcaacta cattgccacc aaatacaacc tctatgggaa ggacatgaag gagagagccc 420

tcatcgacat gtatgcagaa ggagtggcgg atctggatga aatagttctc cattaccctt 480

acattccccc tggggagaaa gaggcaagtc ttgccaaaat caaggacaaa gcaaggaacc 540

gttactttcc tgcctttgaa aaggtgttga agagccatgg acaagattat ctcgttggca 600

ataggctgag cagggctgat gtttacctag ttcaagttct ctaccatgtg gaagagctgg 660

accccagcgc tttggccaac ttccctctgc tgaaggccct gagaaccaga gtcagcaacc 720

tccccacagt gaagaagttt cttcagcctg gcagccagag gaagccatta gaggatgaga 780

aatgtgtaga atctgcagtt aagatcttca gttaattcag gcatctatgg atacactgta 840

cccacaaagc cagccttcga aagctttgca acaatcgcat attttgacta aatgttgacc 900

ctacttattg ggaggccaac acgttttcta atgcttctgt gttaattcat atagacatga 960

ctgatgagga attgctggga tgctatttgg ttgtagttaa aatttgaaat catgatcact 1020

tcctcagata ttactttgaa tctcaataaa aa 1052

<210> 731

<211> 413

<212> DNA

<213> Rattus norvegicus (Norway rat)

<400> 731
atgaagctgc ttgcaatggt tgcactgctg gtcaccatct gtagcctaga aggagctttg 60

gttcggagac aggcagcgga gacggatgtg cagaccctgt tcagccagta tcttcagagt 120

ttaactgact atggcaagga tttgatggag aaggcccagc cctcagagat tcagaaccaa 180

gccaaggctt actttcagaa tgcacaggag agactgacac cctttgtcca gagaactggg 240

acgaatctga tggacttctt aagccgttta atgagccccg aggagaaacc agctcctgcc 300

gctaagtgag gtgtgtcagg actcgtcttc ctaccccagc tgacccactg gccatcgctg 360

gagtccctct cccgactttc tgcttgtctt ttccaataaa ttcggaagga gtt 413

<210> 732

<211> 2324

<212> DNA

<213> Rattus norvegicus (Norway rat)

<400> 732
gtgaacgtgt ttggcagcgg caactaaatt cagaaaacat catggcagag caggtggctt 60

tgagccggac ccaggtgtgt gggatcctga gggaagagtt gtaccagggt gatgccttcc 120
accaagctga tacacacata tttatcatca tgggtgcatc gggtgacctg gccaagaaga 180
agatttatcc taccatctgg tggctgttcc gggatggcct tctacccgaa gacaccttca 240
ttgtaggcta tgcccgctca cgactcacag tggatgacat ccgcaaacag agtgagccct 300
tctttaaagt cactccagaa gaaagaccca agctagagga gttctttgcc cgtaactcct 360
atgtagctgg ccagtatgat gatccagcct cctacaagca cctcaacagc cacatgaatg 420
ccctgcacca gggaatgcag gccaaccgtc tgttctacct ggccttgccc cccactgtct 480
atgaagcagt caccaagaac attcaagaga tctgcatgag tcagacaggc tggaaccgca 540
tcatagtgga gaagcccttc gggagagacc tgcagagctc caatcaactg tcgaaccaca 600
tctcctctct gtttcgtgag gaccagatct accgcattga ccactacctg ggcaaagaga 660
tggtccagaa cctcatggtg ctgagatttg ccaacaggat ctttggaccc atctggaatc 720
gagacaacat tgcttgtgtg atccttacat ttaaagagcc ctttggtact gagggtcgtg 780
ggggctattt tgatgaattt gggatcatca gggatgtcat gcagaaccac ctcctgcaga 840
tgttgtgtct agtggccatg gaaaagcctg cctctacaga ttcagatgat gtccgtgatg 900
agaaggtcaa agtgttaaaa tgtatctcag aggtggaaac tgacaacgtg gtccttggcc 960
agtatgtggg gaaccccagt ggagaaggag aagctaccaa tgggtactta gatgacccca 1020
cagtacccca tgggtctacc actgctacct ttgcagcagc tgtcctctat gtggagaatg 1080
aacggtggga tggagtaccc ttcatcctgc gctgtggcaa agctctgaat gagcgcaaag 1140
ctgaagtgag acttcagttc cgcgatgtgg caggtgacat cttccaccag cagtgcaagc 1200
gtaacgagct ggtcatccgt gtgcagccca atgaggcggt atacaccaag atgatgacca 1260
agaagcctgg catgttcttc aaccctgagg agtctgagct ggacctaacc tatggcaaca 1320
gatacaagaa tgtgaagctc cctgatgcct atgaacgcct catcctggat gtcttctgtg 1380
ggagccaaat gcactttgtc cgtagtgatg aactcaggga agcctggcgt atcttcacac 1440
cattgctgca caagattgat cgagagaagc cccagcccat cccgtatgtc tatggcagcc 1500
gaggtcccac agaggcagat gagctgatga agagagtggg cttccagtat gagggtacct 1560
acaagtgggt gaaccctcac aagctctgag ccctggaaac ttacaccatc tgcactctgc 1620
ctcttctggc caccctttct gcatctgccc ttctcaccat ctaaccctct attaggacta 1680
ttgacctcat attggaaaga ctttgggacc ataggcctta gctacacatt ctagtccctg 1740
ggcttaggcc accattctgt cctatgctgc tgccactgcc actaccacta agcccagcta 1800
cattcctcag ataccaggca ttcaaaacgc attgcaatgc tttcaggacc accactgtcc 1860
ctatctgagc cacccatctt tccacaagac ctgaatcacc tcctcccctc aatcccctgc 1920
agaaagaacg cctatcagtc tgtccctgga ctccttaaga taggagttag gaacaattgg 1980
gaggagcctt gggccttgga gggacaatga ccaaaccaca cttccctgag actgtggcca 2040
agctcctcaa aacttaaagt gatcaaggac acccatctga gaggacctgc ccatagccac 2100

actagcctta gtgctacttg acattcctcc tcaccagctg gaagaactct catgctgcct 2160

agcaatattt tgggggccat agatatctcc taaacaattc catagtccat agtcagcctc 2220

atccaaccca tgggcagcct ccttaccaaa ggaaggtaag agcagcagct agaattttcc 2280

taccccaacc ctgccattaa atcctcaaaa aaaaaaaaaa aaaa 2324

<210> 733

<211> 859

<212> DNA

<213> Rattus rattus (black rat)

<400> 733
tgcttcctga gtcacccgca aacgagagca ggaccctctt ggctctccgt ctgctcggcg 60

cagcgatgtc cggggcgtcc gccaggagcc tggggaaggg aagcgcgccc cctggcccgg 120

tcccggaggc cagatccgag tctacagcat gaggttctgt ccctttgctc agaggacgct 180

gatggtcctg aaggccaagg gaatccggca tgaaatcatc aacatcaacc tgaagaataa 240

gcccgagtgg ttctttgaga agaatccctt tgggctggtg ccggttctgg agaacactca 300

gggtcacttg atcactgaat ctgtcatcac ttgcgagtac ctggatgaag catacccgga 360

gaagaagtta ttcccagatg acccgtacga gaaagcttgc cagaagatga cctttgagtt 420

attctcaaag gtgccgtctc tggttacgag ttttattagg gcgaagagaa aggaagacca 480

tccgggcata aaggaagaac tgaagaaaga gttcagcaag ctagaagagg ctatggctaa 540

taagaggaca gccttcttcg gtgggaattc gctctcaatg atcgattatc ttatttggcc 600

gtggtttcag cgactggaag cactggagct caatgagtgt atagaccaca ccccaaaact 660

caagctctgg atggcaacca tgcaggaaga ccctgtggca tcatcccact tcattgatgc 720

caagacctac cgtgattact taagtctcta cctacaggac agccccgagg cctgtgatta 780

tgggctctga ggggcaagag ccctcagcga atgatgtttt ccttcatcga ttgaatagca 840

tgcttttatt ttacccatt 859

<210> 734

<211> 1442

<212> DNA

<213> Rattus norvegicus (Norway rat)

<400> 734
aattccctgg ggcgccctct tttaaaaatg gagtcccaaa tacagagaag atttcatcac 60

catggtctcc ctgtgtcaaa gagctcttgt tcttccgcca tgtggctctg tgctctggtc 120

tgggcttctc ttgctgtttg cccaatttgg ggacacccat cctcaccacc agtggtggac 180

accacaaaag gcaaagtcct ggggaagtat gtcagcttag aaggatttac acagcctgtg 240

gccgtcttcc tgggagtccc ttttgccaag cctcctcttg gatctctgag gtttgctcca 300

ccagagcctg cagagccctg gagcttcgtg aagaacacca ccacctaccc gcctatgtgc 360

tcccaagatg gagttgtggg aaagttactc gcagatatgt tgagcaccgg aaaagagagt 420

atacctctcg agttttccga agactgtctc tacctgaata tttacagtcc tgctgacttg 480

acaaaaaaca gccgattgcc cgtgatggtg tggatccatg gaggtggact aataataggc 540

ggagcatcac cctatagtgg actagctctc tctgcccacg aaaacgtggt ggtggtaacc 600

attcaatacc gcctgggtat ttggggattg tttagcaccg gtgatgaaca cagccggggg 660

aactgggctc acttggacca gctggctgca ctacgctggg tccaggataa cattgcaaac 720

tttggaggga acccggattc agtgaccatc tttggagagt cagcaggagg tgtcagtgtc 780

tctgctcttg tcttatctcc tctggccaag aacctcttcc acagagccat ttctgagagt 840

ggtgtgctcc tcactacaaa cctggacaag aagaatactc aggctgtggc tcaaatgatt 900

gctactcttt ctgggtgtaa taacacctca tcagccgcca tggttcagtg cttgcgccag 960

aagacagagg ctgagctctt ggagcttaca gtgaaactgg acaatacctc catgtccact 1020

gtgattgatg gagtggtact gccaaagaca ccggaagaga tcctgactga gaagagtttc 1080

aacacggtcc cctacatagt gggcttcaac aagcaagagt ttggctggat cattccaacg 1140

atgatgggaa atctactctc tgaaggcaga atgaatgaga aaatggccag ttctttcttg 1200

aagaggttca gccctaacct taacatctct gagagtgtga ttccagcaat cattgagaag 1260

tacttaagag gaacagatga ccctgccaaa aagaacgaac ttctcctgga catgttttca 1320

gatgtctttt tcggtatccc agctgtactc atgtcccgta gcctcagaga tgccggagcg 1380

cccacctaca tgtatgagtt tcagtatcgc ccaagcttcg tgtctgacca gagaccccag 1440

ac 1442

<210> 735

<211> 2602

<212> DNA

<213> Rattus sp.

<400> 735
ctgctgcctg cactttgccc tggtcctcca atggcttcaa cccgtgccag gcccatgctg 60

cctctgctcc tggtcctggt cgccgttgtg atccccgggc ctgtcggtgc tcaggtatcc 120

atccatccca cagaagcctt cctgcctcgg ggtggatccg tgcaggtgaa ctgctcttcc 180

tcttgcgaag acgagaacct cggcctgggg ttggagacta actggatgaa agacgaacta 240

tcgagtggac acaactggaa gctcttcaag ctgagcgaca ttggggaaga cagcagacca 300

ctgtgctttg agaactgtgg caccacgcag tcctcggctt ctgccaccat cactgtgtat 360

tcgttcccag agcgagtgga gctggatcct ctgcccgcct ggcagcaggt gggcaagaac 420
ctcatcctgc gctgcctggt ggaaggcgga gcaccgcgga cacagctctc agtagtgctg 480
ctccgtggga atgagacact gagccgccag gcagtggatg gggaccccaa ggagatcaca 540
ttcacggtgc tggccagcag aggcgaccac ggagccaatt tctcatgctt cacagaactg 600
gacctcaggc cacaagggct gtcactgttc aagaatgtct ccgaggtcag gcagctccgg 660
actttcgatc ttccgactag ggtcctgaag ctcgacaccc ctgacctcct ggaggtgggc 720
acccagcaga agttcttgtg ttccctggaa ggcctgtttc ctgcctctga agctcagata 780
tacctggaga tgggaggcca gatgctgacc ctggagagca caaacagcag agattttgtg 840
tcagccactg cctcagtgga ggtgactgag aagttggaca gaaccctgca gctgcgctgt 900
gttttggagc tggcggacca gaccctggag atggagaaga ccttgagaat ctacaacttt 960
tcagctccca tcctgaccct gagccagccg gaggtctcag aaggggacca agtaactgtg 1020
aagtgtgaag cccacggtgg ggcacaggtg gtgcttctga acagtacttc ccccaggcca 1080
cccacctcac agggtacttc ccccaggcca cccacctcac agatccaatt cacactgaat 1140
gccagcccgg aggatcacaa acgacgcttc ttttgctctg cggccttgga ggtggatggg 1200
aagtccctgt ttaaaaacca gaccttggaa ctccatgtgc tatatggtcc tcacctggac 1260
aagaaggact gcttggggaa ctggacctgg caagaggggt ctcagcagac tcttacatgc 1320
cagccccagg ggaatccagc ccctaatctg acctgcagcc ggaaagcaga tggtgtcccg 1380
ctgcctatcg ggatggtgaa gtctgtcaaa cgggagatga atggtaccta caagtgccgt 1440
gcctttagct cccgtgggag tatcaccagg gacgtgcacc tgacagtgct gtaccatgat 1500
cagaatacct gggtcataat tgttggtgtg ttggtactga tcattgcggg cttcgtgatc 1560
gtggcgtcca tttacaccta ttaccgccag aggaagatca ggatatacaa gttacagaag 1620
gctcaggagg aggccctaaa actcaaggta caagccccgc ctccctgagc ccactggaca 1680
ggacacctgc ctgggccccg ctgctcttga acagatcaat ggacagcatt tacccctcac 1740
ccacctcctc tggctgtcac aggacaggac agtggcctgg ggatgcatac ttgtagcctc 1800
aggcctaaga ggactcggag gggcaagact gtgaactcgt gacctggaca cacctacagc 1860
ctggtgggcc tgcagccaag aaaggctgac ttccttctct attacccctg ctgaggggcc 1920
ccctacctta ggaaggtgtg atatccggta gacacaagca agagaagaaa aggaacacca 1980
tgcttcctct gacatgggaa agctgggaca ctgtccccaa ctcttgttga tgtatttatt 2040
aattcagagt tctgacagtt atttattgag taccctgtac agacactaga ggagtgagca 2100
ggttaacatg taagttattg cctagaccct ggtgaagggg cacaacagag tctggggaaa 2160
gatcatacgg gtttgggctt ctccacaggt cagggtgctt tcctcaaaag agctgatttc 2220
tttcacgagt catataaata ctatgtggac gagcagtggc cctctgctcg tagacctctc 2280
tgggacccct gcctcctccc acagcctgga gtctcccagc accagcatgg gtgaccacct 2340
ccccacctac atacattcct acctttgttc ccaatgtcaa ccaccatgcc taaatatgga 2400

cgctcacctt tagcagctca acaatggagt ctcatgcccg tgaaattatg gtcaatccct 2460

gcatgcctcc acccggctcc acctcaaaga gaatgcctgg gagaaaatgt tccaaccact 2520

tagaagggtc ctgcaagctg ttgtgggagg gtaggcaccc ctcccagcgc agaagccttt 2580

cctttgaatc aataaagttt ta 2602

<210> 736

<211> 1018

<212> DNA

<213> Rattus norvegicus (Norway rat)

<400> 736
gctggaacgg gtccgtacgg cgttgttctt ggttcccatc gtaacttaaa agggaaactt 60

acacaatgtc cggaggcctt gacgtcctgc agatgaagga ggaggatgtc ctcaaattcc 120

ttgctgcagg aacccactta ggtggcacca accttgactt tcagatggag cagtacatct 180

acaaaaggaa aagtgacggt atctacatca tcaacctgaa gaggacttgg gagaagctgt 240

tgttagccgc tcgagctatt gttgccattg agaaccctgc tgatgtcagc gtcatctcct 300

ccaggaacac tggccagcga gctgtgctga agtttgccgc tgccacagga gccactccaa 360

ttgctggccg cttcacacct gggaccttca ctaaccagat ccaagcagcc ttcagggagc 420

cccggcttct agtggtgact gatccccggg ctgaccacca gcccctcaca gaggcctctt 480

acgtcaacct gcccaccatt gctctttgta acacagattc tcccctgcgc tatgtggaca 540

ttgccatccc atgcaacaac aagggagctc actcagtggg tctcatgtgg tggatgctgg 600

ccagggaagt actccgcatg cgaggaacca tctcccggga gcacccatgg gaggttatgc 660

ctgatcttta cttctacagg gacccagagg agattgagaa ggaggagcag gctgccgctg 720

agaaggctgt gaccaaggag gaattccagg gtgaatggac ggcaccagcg cctgagttca 780

ctgctgctca gcctgaggtg gccgactggt ctgagggtgt gcaggtgccc tctgtgccca 840

ttcagcagtt ccccacagaa gactggagtg cacagccggc cactgaggac tggtcagcag 900

ctcccacagc acaggctact gagtgggttg gagccaccac tgagtggtcc tgagctcctc 960

tgcaggtgcc tgagcgaagg gaaaaaaggt ggaaggaaaa taaagttcct gaaagctg 1018

<210> 737

<211> 514

<212> DNA

<213> Rattus norvegicus (rat)

<400> 737
agaggcctct tacgtcaacc tgcccaccat tgctctttgt aacacagatt ctcccctgcg 60

ctatgtggac attgccatcc catgcaacaa caagggagct cactcagtgg gtctcatgtg 120

gtggatgctg gccagggaag tactccgcat gcgaggaacc atctcccggg agcacccatg 180

ggaggttatg cctgatcttt acttctacag ggacccagag gagattgaga aggaggagca 240

ggctgccgct gagaaggctg tgaccaagga ggaattccag ggtgaatgga cggcaccagc 300

gcctgagttc actgctgctc agcctgaggt ggccgactgg tctgagggtg tgcaggtgcc 360

ctctgtgccc attcagcagt tccccacaga agactggagt gcacagccgg ccactgagga 420

ctggtcagca gctcccacag cacaggctac tgagtgggtt ggagccacca ctgagtggtc 480

ctgagctcct ctgcaggtgc ctgagcgaag ggaa 514

<210> 738

<211> 1879

<212> DNA

<213> Rattus norvegicus (Norway rat)

<400> 738
aagacttttt ccccagcctt aactggatag tctgaagttt tcaaaactct tatccacaaa 60

gttgtcagaa ccttgattgg gaagtcctgt gcatctgtgc taacctacag ggcctcctta 120

tccagagcac tctgcatttc agagggtcgc tgtcgaacta cggttttggc gaagacattc 180

ctgaagaatt gtctgaggtt tcctctgcaa aaatggccaa gctgacagct gttcctctca 240

gtgcacttgt tgatgagcct gtgcatatcc gggtcacagg cctgaccccc ttccaggtgg 300

tgtgccttca ggcatcactg aaagatgata agggaaacct gtttaattct caggccttct 360

acagggccag tgaagtgggt gaggtagatc tggaacgtga ttcctctctt ggaggagact 420

acatgggggt ccaccccatg ggtcttttct ggtccatgaa acctgaaaag ctattgacta 480

gattggtaaa aagagatgtg atgaataggc cccacaaagt ccacataaaa ctttgccatc 540

catactttcc agtagaaggc aaagttatca gttcctcctt ggatagtctg attctggaaa 600

ggtggtatat ggcacctggt gtcactagga tccatgtaaa ggaaggccga atccggggag 660

ccctgtttct gcctccagga gaaggtcctt tcccaggggt cattgacttg tttggaggag 720

ctggtggact gtttgagttc cgggccagcc tcctggccag tcatggcttt gccactttag 780

ctctggctta ctggggctat gatgacctgc cctctcgact ggagaaggta gatctagagt 840

attttgaaga aggtgtagag tttctcctga gacatcctaa ggtcctgggc ccaggggttg 900

gcatcctttc tgtgtgcatt ggagcagaga ttggactttc tatggctatt aacctaaaac 960

agataacagc cactgtactt ataaatgggc ctaattttgt ttctagcaat ccacatgtat 1020

atcgtggtaa ggtcttccag cctacaccct gcagtgaaga atttgtaacc accaatgctt 1080

tgggacttgt agagttctat cgaacctttg aggaaactgc agataaggat agcaaatact 1140

gttttcccat tgaaaaagct cacggacatt ttcttttcgt ggtgggagaa gatgataaga 1200

acctcaacag caaagtgcat gctaagcaag ccatagccca gctgatgaaa agtggaaaga 1260

agaactggac tctgctgtct taccctgggg caggtcacct gattgagcct ccctactccc 1320

cactgtgctc agcctcaagg atgccctttg taatcccaag catcaactgg ggaggagagg 1380

ttatcccaca cgcagctgcg caggaacatt cttggaagga gatacagaaa tttctcaagc 1440

agcatcttaa tccaggtttc aacagtcagc tgtgagtgga cttgattata ttactggaaa 1500

gaggagctgg gcatctcctg gccagctcca ctcctcactt ccatagagga atgtctttaa 1560

tctcttatca catgaggaag aagagtacca ccagaaaatg ccgaaggaca gagagtgata 1620

acctcatgac tttggaaggg gagacatgtt ttccatggaa taaaatgtcc ctcagtgaga 1680

gtcctatatc tgtataaata aaatcttagg gttttcctaa aatgttcaac accacagcaa 1740

ctttctgtga tgataattat caaggaaatt atcactgata atccacagga tactttagtt 1800

tataaaagag acatgaaaag aattatatat tgttacttat taatttctta aaactcacat 1860

taaatatgct tagatcatc 1879

<210> 739

<211> 1560

<212> DNA

<213> Rattus norvegicus (Norway rat)

<400> 739
atgccttctg tgtatggatt cccagccttc acatcagcca cagagctgct cctggccgtc 60

accacattct gccttggatt ctgggtggtt agagtcacaa gaacctgggt tcccaaaggt 120

ctgaagagtc cacccggacc ctggggcttg cccttcatag ggcacgtgct gaccctgggg 180

aagaacccac acctgtcact gacaaaactg agtcagcagt atggggacgt gctgcagatc 240

cgtattggct ccacacccgt ggtggtgctg agcggcctga acaccatcaa gcaggccctg 300

gtgaaacagg gggatgactt caaaggccgg ccagacctct acagcttcac acttatcgct 360

aatggccaga gcatgacttt caacccagac tctggaccgc tgtgggctgc ccgccggcgc 420

ctggcccaga atgcgctgaa gagtttctcc atagcctcag acccaacact ggcatcctct 480

tgctacttgg aagagcacgt gagcaaagag gctgaatact taatcagcaa gttccagaag 540

ctgatggcag aggttggcca cttcgaccct ttcaagtatt tggtggtgtc agtggccaat 600

gtcatctgtg ccatatgctt tggcagacgt tatgaccacg atgaccaaga gctgctcagc 660

atagtcaatc taagcaatga gtttggggag gttactggtt ctggataccc agctgacttc 720

attcctatcc tccgttacct ccctaactct tccctggatg ccttcaagga cttgaataag 780

aagttctaca gtttcatgaa gaagctaatc aaagagcact acaggacatt tgagaagggc 840

cacatccggg acatcacaga cagcctcatt gagcattgtc aggacaggag gctggacgag 900

aatgccaatg tccagctctc agatgataag gtcattacga ttgtttttga cctctttgga 960

gctgggtttg acacaatcac aactgctatc tcttggagcc tcatgtacct ggtaaccaac 1020

cctaggatac agagaaagat ccaggaggag ttagacacag tgattggcag ggatcggcag 1080

ccccggcttt ctgacagacc tcagctgccc tatctggagg ccttcatcct ggagatctac 1140

cgatacacat cctttgtccc cttcaccatc ccccacagca caacgaggga cacctcactg 1200

aatgggttcc acattcccaa ggagtgctgc atcttcataa accagtggca ggtcaaccat 1260

gatgagaagc agtggaaaga cccctttgtg ttccgcccag agcggtttct taccaatgac 1320

aacacggcca tcgacaagac cctgagtgag aaggtgatgc tcttcggctt gggaaagcgc 1380

cggtgcattg gggagatccc ggccaagtgg gaagtcttcc tcttcttagc catcctcctg 1440

catcagctgg agttcactgt gccaccgggc gtgaaggtgg acctgacacc cagctatggg 1500

ctgaccatga agcccagaac ctgtgaacac gtccaggcct ggccacgctt ctccaagtga 1560

<210> 740

<211> 341

<212> DNA

<213> Rattus sp.

<220>

<221> misc_feature

<222> (16)..(16)

<223> n

<220>

<221> misc_feature

<222> (149)..(149)

<223> n

<400> 740
cagaaccatg cctatnatac tgggatactg gaacgtccgc gggctgacac acccgatccg 60

cctgctcctg gaatacacag actcaagcta tgaggagaag agatacgcca tggggacgct 120

cccgactatg acagaagcca gtggctgant gagaagttca aactgggcct ggacttcccc 180

aatgtggaga gacagaggag gagcggattc gtgcagacat tgtggagaac caggtcatgg 240

acaaccgcat gcagctcatc atgctttgtt acaaccccga ctttgagaag cagaagccag 300

agttcttgaa gaccatccct gagaagatga agctctactc t 341

<210> 741

<211>  684

<212>  DNA

<213>  Rattus norvegicus (Norway rat)

<400>  741
acgagtgctg acatgatcac tctctgtgtt cacaggaaag cgcatttgtc ttggcgaagg      60

cattgcccga aatgaattgt tcctcttctt caccaccatc ctccagaact tctctgtgtc     120

aagccatttg gctcccaagg acattgacct cacgcccatg gagagtggca ttgcaaaaat     180

acctccaacg taccagatct gcttctcagc tcggtgatcg ggctgaggca gccaggtgcc     240

ccagttctgt tgggaatggc ctcatgtttc tgcctctggg ggacctgctg aaaaccaggc     300

tccaaggcca ctgctccaca tcttcctatt gcagttctcc aaagtcccaa ggcttgttct     360

tattcctgtg aatggcactg aagaagtcaa tcgactgtct tattttgaca tgtgacagag     420

atttcatgag tacacatctc atgctgagtc acttccctct tcctcctaat agcccacgtc     480

cccacttatc agccctccat ggtctgtgat ctgtgctaat ggactctgta tatggtctca     540

gtgctatgtc tacagactta catagtatgt atggttcagg taaacagaat cacagagtgt     600

gtgagcttcg gtgtgttgtg cctttacttc acataatatt atctaggttc ctgtgttcta     660

caggccacag tcacacacat tcat                                            684

<210>  742

<211>  2509

<212>  DNA

<213>  Rattus norvegicus (Norway rat)

<400>  742
taaagagacg gatgacctac agccccaggc ccacccattc aacaggccta gctcattccc      60

aagcccagac atcaaggcat gggacaccca cgcatggcag cttcgtgtcc agctttatta     120

gggacagcat gtttaggtga ggtctgggga gggataaagg catgagaata tactttcccc     180

ttagagcaac cttcggaggc agcaggatag atggccagac acatctggaa catggaggtc     240

tcacggctca agagttggtg ttgttagttg gtcctcaggg ccagactccc agaggccagt     300

gaacttatca gtgaacttgc tccagtagcc tttcagggat ttgaagcgat tgtccatcca     360

gcccctgggg gttaaaacag taataggtgt gtgtgtgtgt gtgtgtgtgt gtgtgtgtgt     420

gtgtgtgtgt gtgtgtgtgt gaaaagatct cctgtgggca gctagaccca ggggtgcacc     480

taggcctcca ctggctccct aagccaccag caccacctta cctagcacca ccctcagaat     540

cacctgcagc taccactcaa ggtggaggag atggtaaagg ctaagaaaac ccaccttcat     600

cagagcccca ttacctcccg tccaatctct cttcaggcct gaggcaccaa ccactgggga     660

ggtggatact aaggtcagct tgcctttgta gtccatagaa acagtgtcct tgaggtgtcc     720

catccctggc tctaagtgga tggatcaagg gaggggtgaa ctttctgttt ggaaacatta 780

ccagagggct tctaagctct gtgatctagg ccaggttatc taactctttc ccagctgcgg 840

aggtagatag tacctctcca caacctgttc tgggcacaga gactgtcaac atcttttgct 900

acataggcaa tgatcaaatg tcacgtaaac gattggcagg gtaatgtttc atcacgggca 960

agatgcctca cttaggttga gcccagggat ggaaacaggg cagaaccccc aacccgtaat 1020

gctcaacctt ccaacttccc tgtaatcaga gcaggaaggc ctcccagagc acaccctaga 1080

ccctgtgctc aaagaagaaa acctgcaggg aggctgaacg ctcctcaggc tgctctgagg 1140

agaagcagag gagatagaag aagtctgctt gcactgcctg tcatcttagt cacagtcccc 1200

agcaaaggcc ccgtgagaga ctggatgctc agacgggccc aagaccctgg taaacttggt 1260

gggccacagt ctccgtgtcc agggcctcag caccagggca ggaggggcga ggaccaggaa 1320

aggaggtccg tgtgcatacc tggccaccac agctatatca gactcctgca tgctgcttag 1380

tgcatcctgg accgtcttgg aggcttgttc catgtagccc tgcatagagc ccagcagcaa 1440

ggatccctct ccctcatcag ctcctgcaag agagcagagt tgagccaggc cagccctcag 1500

ctcttgccca gccatcgctt ttcagggtag ggtccccaga ccagctcccg cagaaatccc 1560

agccccactt ccaccagctt acgggcagag gccaggagag ccacgagggc cacgatgagg 1620

agcattcggg gctgcatggc acctgtgcac ctgcgggaga ccatcttgtg agagggtatt 1680

gtggatctcc acatctaagc ccttccctgg agaacaccac ggcccctctg tcatgaatcc 1740

ccaagccttt ctcctactga tatcagctct cggagagaga actaagaaga cccagaccca 1800

ccccaagggg ctggaaggtg gaatgtggga atcctctgca aagcagaaca tctacccagc 1860

ctctgcccca atatatggag aaacaacagg tttctttttc tctctaggct tcaggctttt 1920

cagtctgggg taggcacgga tatcaaaggc ttctaatagc tcagagcaag acgaacaagg 1980

ggcagcatga cccagttccc aatcagctct gccactaccc agtgcaaggc ttttttgccc 2040

agtggcctcc ctttcctcag cttctagcct cccccaccca ccaggatacc caagggctgg 2100

aggccgtgaa ttccaagcat tctgtaggct agctggctga gtggccagag cgtcttctct 2160

ctgtctcctc cctcccttcc tctcctcccc aggggcatta cctggagtag ctagctgctt 2220

ctagggataa aactgggcag gcaagccggg acgctctgat ctgttttata ttggctccag 2280

gatgggacag cgggcacaga aggcccagtg agctggtcaa aggtcacctg ctgaacagtc 2340

cagaccagag cccgaggcag ggaggccatg cagccagctg ccagaggagt tgagaaatcc 2400

ctcagagatt gcccacaccg ttcacttcca cctccgcagc caagagatca gctactgacc 2460

tgcctcgatg agactggtga gacaggaaaa gactcagggg acaagcctt 2509

<210> 743

<211> 2237

<212> DNA

<213>  Rattus norvegicus (Norway rat)

<400>  743
gccagcttcc tgaggtacca aggaggaagg atggaggaaa gaatgaacgt gctccacgac      60

tttgggatcc agtcgactcg ctacctccaa gtgaattacg aagactccca ggactggttt     120

gtcttggtgt ctgtgatcgc tgacctcagg aacgccttct atgtcctctt tcccatctgg     180

ttccacattc aagagactgt gggcatcaat ctcctctggg tggcagtggt tggagactgg     240

ttcaacctcg tctttaagtg gattcttttt ggacagcgcc cgtattggtg ggtcctggac     300

actgactatt acagcaacag ctccgtgcct ctgataaaac agttccccgt cacctgtgag     360

actggaccag ggagtccctc gggccatgcc atgggcacag caggtgtata ctacgttatg     420

gttacttcta ctctcgctat ctttcgtgga aagaaaaagt caacgtatgg attccggtgc     480

ttgaatgtcg tcttgtggtt gggatactgg gctgtgcagc tgaacgtctg tctgtcccgg     540

atctaccttg cggctcactt tccccatcag gtggtggctg gagtcttgtc aggcattgct     600

gtggctgaaa ctttcagcca catccggggc atctacaatg ccagcctcca gaggtactgt     660

ctcatcacct tcttcctgtt tggtttcgca cttggatttt acctgctgct aaaggggcta     720

ggggtggacc tcctgtggac tttggagaag gccaagagat ggtgtgagcg ccccgagtgg     780

gtccaccttg acactacacc ctttgccagc ctcttcaaaa acctggggac cctcttgggg     840

ttggggctgg ccctcaactc cagcatgtac cgcaagagct gcaaaggaga actgcgcaag     900

tcgctcccat tccgtttggc ctgcatcgtg gcctccttgg gcctgctgca tctctttgac     960

tcgctgaagc ccccgtccca gattgagtcg atcttctaca tcttgtcttt ctgcaagagt    1020

gcgaccgtcc cctttgcatc tgtcagtctt atcccctact gcctagcccg gctcctggga    1080

cagacacaca agaagtcttt gtaaagcgtt aagtctctga cacaagtcag cggccgtgca    1140

aaggactagg agcaacccaa gccttgtgca acccagtgtg ggccagaggt gttcacagcc    1200

caccctggta gccctgtctt tctttgccat cgtaaccaaa aaggtgaatt tttaaaagct    1260

aacagggctg tttgaggaaa gcgtgaatgc tggaagatgt ttccctgatg aatcacttcc    1320

ggtctcctgt ccgacacaga agagaaagcc cagactagag atcctgacag aatgctctca    1380

ctgtgcagaa tcctccaggc cgagggtaaa agaaaagagc gttgtgcatt tgctaggaag    1440

agaaggaagg atcgggagga gggggagtgt ttggtggatt gagcaaacca tatgcaagcc    1500

atgtctaccc ggcttcagtt atgcctgggt ctttagatat gtaggacact ctcaataatg    1560

atagaccagc ccgtgtaatg agtagccagt tagaggcgat cattctgctc ccagattttt    1620

ctctactgta catgaaagtc acaccgtgtg cttgtattcc tgaatggtaa tggtgactgt    1680

cactcaggaa ccaccacctt ctctgtgaca ccacagcttt gcctcctcgg cccaggtctg    1740

cgtgccatag gactcatcac tgcttccctg accacgctgc tggaccctgc attgctgaac    1800

cccatgctgc tggaccctgt ggtgtacggt gggcaattgt ttgctggtgc ttttgtaggg    1860

ttaagttaaa ctctgagatc ttgggcaaaa cggcaagggg agagtcagga ttcctctctc    1920

caaaggtcac tccgaagtta cttttgattc ctggagcaga aatgactcct tcctctagcc 1980

caagccagcc aagaactcat tcttagaggc aaagcagccc aagcccgact gtgcctgtcc 2040

tccagcctgg gctggttttg cagagtgtgt cttcaagaag aaaaaatggt aaagctattt 2100

attcaaaatg cttgtttttt gctatgaatg atgcctgtgt ttccacccac gccaagcact 2160

tgttcctatt gtcttgataa gaaactacat gtgtgcagta ttttattaaa acgacatttt 2220

atttaaaaaa aaaaaaa 2237

<210> 744

<211> 2495

<212> DNA

<213> Rattus norvegicus (Norway rat)

<400> 744
attgcctacc ccgggtggag accgtgctcg tccggccctc ttgcctcacg ttctgcagct 60

ctgcagctcc gcaatcctac accatggcgg acagccggga cccagccagc gaccagatga 120

agcagtggaa ggagcagcgg gcccctcaga aacccgatgt cctgaccacc ggaggcggga 180

acccaatagg agataaactt aatatcatga ctgcggggcc ccgagggccc ctcctcgttc 240

aagatgtggt tttcaccgac gagatggcac actttgacag agagcggatt cctgagagag 300

tggtacatgc aaagggagca ggtgcttttg gatactttga ggtcacccac gatattacca 360

gatactccaa ggcaaaggtg tttgagcata ttgggaagag gactcctatt gccgtccgat 420

tctccacagt cgctggagag tcaggctcag ctgacacagt tcgtgaccct cgtgggtttg 480

cagtgaaatt ctacactgaa gatggtaact gggacctcgt gggaaacaac acccctattt 540

tcttcatcag ggatgccatg ttgtttccat cctttatcca tagccagaag agaaacccac 600

aaactcacct gaaggaccct gacatggtct gggacttctg gagtctttgt ccagagtctc 660

tccatcaggt tactttcttg ttcagcgacc gagggattcc agatggacat cggcacatga 720

atggctatgg ctcacacacc ttcaagctgg ttaatgcgaa tggagaggca gtgtactgca 780

agttccatta caagactgac cagggcatca aaaacttgcc tgttgaagag gcaggaagac 840

ttgcacagga agacccggat tatggcctcc gagatctttt caatgccatc gccagtggca 900

attacccatc ctggactttt tacatccagg tcatgacttt caaggaggca gaaaccttcc 960

catttaatcc atttgacctg accaaggttt ggcctcacaa ggactaccct cttataccag 1020

ttggcaaact ggtcttaaac agaaatcctg ctaattattt tgctgaagtt gaacagatgg 1080

cttttgaccc aagcaacatg ccccctggca ttgagcccag cccggacaag atgctccagg 1140

gccgcctttt tgcttaccca gacactcacc gccaccgcct gggaccaaac tatctgcaga 1200

tacctgtgaa ctgtccctac cgtgctcgcg tggccaacta ccagcgcgat ggccccatgt 1260

gcatgcatga caaccagggt ggtgctccca actactaccc caacagcttc agcgcaccag 1320

agcagcaggg ctcggccctg gagcaccata gccagtgctc tgcagatgtg aagcgcttca 1380

acagtgctaa tgaagacaac gtcactcagg tgcggacatt ctatacgaag gtgttgaatg 1440

aggaggagag gaaacgcctg tgtgagaaca ttgccaacca cctgaaagat gctcagcttt 1500

tcattcagag gaaagcggtc aagaatttca ctgacgtcca ccctgactac ggggcccgag 1560

tccaggctct tctggaccag tacaactccc agaagcctaa gaatgcaatt cacacctacg 1620

tacaggccgg ctctcacata gctgccaagg gaaaagctaa cctgtaaagc acgggtgctc 1680

agcctcctca gcctgcactg aggagatccc tcatgaagca gggcacaagc ctcaccagta 1740

atcatcgctg gatggagtct cccctgctga agcgcagact cacgctgacg tctttaaaac 1800

gataatccaa gcttctagag tgaatgatag ccatgctttt gatgacattt cccgaggggg 1860

aaattaaaga ttagggctta gcaatcactt aacagaaaca tggatctgct taggacttct 1920

gtttggatta ttcatttaaa atgattacaa gaaaggtttt ctagccagaa acatgatttg 1980

attagatatg atatatgata aaatcttggt gattttacta tagtcttatg ttacctcaca 2040

gcctggtata tatacaacac acacacacac acacacacac acacaccaaa acacacatac 2100

actatacaca cacacacaca cacacactaa aacacacata cacaacacac acatacacta 2160

cacacacaga acacacaaca caaacataca cacataggca cacacacaca cacacacaca 2220

cacacacaca cacacacaca cacacatgaa tgaagggatt ataaagatgg cccacccaga 2280

attttttttt atttttctaa ggtccttata agaaaaacca tacttggatc atgtcttcca 2340

aaaataactt tagcactgtt gaaacttaat gtttattcct gtgtagttga ttggattcct 2400

tttccccttg aaattatgtt tatgctgata cacagtgatt tcacataggg tgatttgtat 2460

ttgcttacat ttttacaata aaatgatctt catgg 2495

<210> 745

<211> 1346

<212> DNA

<213> Rattus norvegicus (Norway rat)

<400> 745
gaattccggg agacaacctt gtcaagcacc taattgtgcc atccgtgcga tgccagccag 60

ctccatccac cttacaggag tgaagtcagc gttcatatac gctgtgtggt ctggcagctg 120

agatagtgtt tcccaaaggg actgtggaat gttacctcag caggcattca tttcgcgttt 180

ggaaaatcgt ctccaaatga actttctttc cgtgctgggt ctgcagaaca tttcagaagc 240

ggagctagca atctgcttca actttttttc ccccggtgct tgcagcagga gatgcctgaa 300

gggagctgct gcgtggtggt ctgaaacata gttcaaggaa tagaggtcaa ctgcctagag 360

cggtgcaggc agggcacagg ctggctttgt actctgagtc tcaaggtatt tcccagtgcc 420

tgagccaggg ggaaggtggg gggaggaagg gaaggagctg ccacttggat cgctattctg 480

ggtagccaag acagatagcc atacaatgga aattagtggc ttcaacttgg gggaaaggat 540

ggactctaac ttcgagctgt gcagttcgcc cattgtttga atggacaaaa ggcagtttac 600

ccaggctcct agcatacctg cctgggtgtc caaatgtaac tagatgcttt cacaaacccc 660

acccacaaaa caacacatgt tcttaagtcc tgggctttgt tttcacttcg gcctcataat 720

acccactctg acctgctgtg taaacgaccc gggacgtacc aaaatgagcg cacctccaat 780

aaagatacac atcatgtcgt cttcacatct cttctacctg gcactctgct tgctcacctt 840

taccagctcg gccacagccg gaccagagac cctttgcggg gctgagctgg tggacgctct 900

tcagttcgtg tgtggaccaa ggggctttta cttcaacaag cccacaggct atggctccag 960

cattcggagg gcaccacaga cgggcattgt ggatgagtgt tgcttccgga gctgtgatct 1020

gaggaggctg gagatgtact gtgtccgctg caagcctaca aagtcagctc gttccatccg 1080

ggcccagcgc cacactgaca tgcccaagac tcagaaggaa gtacacttga agaacacaag 1140

tagaggaagt gcaggaaaca agacctacag aatgtaggag gagcctcccg aggaacagaa 1200

aatgccacgt caccgcaaga tcctttgctg cttgagcaac ctgcaaaaca tcggaacacc 1260

tgccaaatat caataatgag ttcaatacca tttcagagat gggcatttcc ctcaatgaaa 1320

tacacaagta aacattcccg gaattc 1346

<210> 746

<211> 1780

<212> DNA

<213> Rattus norvegicus (Norway rat)

<400> 746
tctcttgctg tttgcccaat ttggggacac ccatcctcac caccagtggt ggacaccaca 60

aaaggcaaag tcctggggaa gtatgtcagc ttagaaggat ttacacagcc tgtggccgtc 120

ttcctgggag tcccttttgc caagcctcct cttggatctc tgaggtttgc tccaccagag 180

cctgcagagc cctggagctt cgtgaagaac accaccacct acccgcctat gtgctcccaa 240

gatggagttg tgggaaagtt actcgcagat atgttgagca ccggaaaaga gagtatacct 300

ctcgagtttt ccgaagactg tctctacctg aatatttaca gtcctgctga cttgacaaaa 360

aacagccgat tgcccgtgat ggtgtggatc catggaggtg gactaataat aggcggagca 420

tcaccctata gtggactagc tctctctgcc cacgaaaacg tggtggtggt aaccattcaa 480

taccgcctgg gatttggggg attgtttagc accggtgatg aacacagccg ggggaactgg 540

gctcacttgg accagctggc tgcactacgc tgggtccagg ataacattgc aaactttgga 600

gggaacccgg attcagtgac catctttgga gagtcagcag gaggtgtcag tgtctctgct 660

cttgtcttat ctcctctggc caagaacctc ttccacagag ccatttctga gagtggtgtg 720

gtcctcacta caaacctgga caagaagaat actcaggctg tggctcaaat gattgctact 780

ctttctgggt gtaataacac ctcatcagcc gccatggttc agtgcttgcg ccagaagaca 840

gaggctgagc tcttggagct tacagtgaaa ctggacaata cctccatgtc cactgtgatt 900

gatggagtgg tactgccaaa gacaccggaa gagatcctga ctgagaagag tttcaacacg 960

gtcccctaca tagtgggctt caacaagcaa gagtttggct ggatcattcc aacgatgatg 1020

ggaaatctac tctctgaagg cagaatgaat gagaaaatgg ccagttcttt cttgaagagg 1080

ttcagcccta accttaacat ctctgagagt gtgattccag caatcattga gaagtactta 1140

agaggaacag atgaccctgc caaaaagaag gaacttctcc tggacatgtt ttcagatgtc 1200

tttttcggta tcccagctgt actcatgtcc cgtagcctca gagatgccgg agcgcccacc 1260

tacatgtatg agtttcagta tcgcccaagc ttcgtgtctg accagagacc ccagacagtt 1320

cagggagacc atggtgatga aatcttctct gtatttggga ctccattttt aaaagagggc 1380

gcctcagaag aggagaccaa tcttagcaag ttggtgatga agttctgggc caactttgct 1440

cgaaatggga acccaaatgg agaggggctg cctcactggc caaaatatga tcagaaggaa 1500

gggtatttgc agattggtgc caccacccag caagcccaga aactgaaagg agaagaagtg 1560

gctttctgga ctgagctctt ggctaagaat ccacctcaga cagaacacac tgaacacaca 1620

taaatgggtc tctgccagcc tcagtaccag aggagcaaag atggggctat tccacaaagg 1680

atggctattt gccaaagatg aatcttacag aggaaactgc acaattgtac agttggggta 1740

tttgtatctc aactttgaaa taaatttcct tttagaaagc 1780

<210> 747

<211> 334

<212> DNA

<213> Rattus norvegicus (Norway rat)

<400> 747
ggcggggtcc tggatatcac cattgggctc acttcctgct tcgtgtgttg tcttctacct 60

gcgggctggg tcctgtcaca cctggaaagc tacaagaagc gggagtgaag ggagcgtctt 120

ccctcatcct ttgactagac cactcttgcc agcccacctt gatcatgttt cctgcattcc 180

tggctggctt acccygggat catgttattc aattccagtc acctcttctg caatcatgac 240

ctctcgatgt ctccatggtg acaactggga ccacatatat tggttctgct tgatggggtc 300

cccctttgta acaataaagt ctatttaaac cttg 334

<210> 748

<211> 1000

<212> DNA

<213> Rattus norvegicus (Norway rat)

<400> 748
ctggaatcct aacagcacct acacaaagct atttataatg ccagactata cttggtttga 60

aggaatacct tttcatgcct ttgggatttc aaaagaaact ttgcaaaatg tttgtaataa 120

gtttgtggtg aaagacgaag atttgatttt attggcttat cccaagtcag gaacgaactg 180

gctgattgaa attgtctgct tgattcagac caagggagat cccaagtgga tccaatctgt 240

gaccatctgg gatcgctcac cctggataga gactgatgta ggatatgata tattaatcaa 300

aaagaaagga ccacgactca tgacctccca tcttcccatg catcttttct ccaagtctct 360

cttcagttcc aaggccaagg tgatctatct cgtcagaaat cccagagatg ttcttgtttc 420

tggttattat ttctggggta attcaactct tgcgaagaag ccagactcac tgggaactta 480

tgttgaatgg ttcctcaaag gaaatgttct atatggatca tggtttgagc acatccgtgc 540

ctggctgtcc atgcaagaat gggacaactt cttgttactg tactatgaag acatgaaaaa 600

ggatacaatg ggaaccataa agaagatatg tgacttcctg ggaaaaaaat tagagccaga 660

tgagctggat ttggtcctca agtatagttc cttccaagtc atgaaagaaa atgacatgtc 720

caattatagc ctcctgatga aaaaatcaat ttttactggt actggcctca tgaggaaagg 780

cactgttggg gactggaaga atcacttcac tgtatcccaa gctgaagcct ttgataaagt 840

gtttcaggag aaaatggccg gtttccctcc agggatgttc ccatgggaat aaaatttcaa 900

aagttttaaa tattttatga acattgatat ttatgtttct gttgttctat gtctgaataa 960

ctgaatgtgg tcatcgaata aatcctgttc tggtattgtg 1000

<210> 749

<211> 1715

<212> DNA

<213> Rattus norvegicus (Norway rat)

<400> 749
gaaaaagaaa aaccaacaag aaagccaatg gctctcttca tttttctggg gatttggctt 60

tcttgtttgg tttttctttt tctatggaat cagcaccatg tcagaaggaa gctcccacct 120

ggtcccactc ctctaccaat ttttggcaat attttgcaag tgggtgttaa aaatatgagc 180

aaatctatgt gcatgctagc aaaagagtac gggcctgtgt tcaccatgta tctgggcatg 240

aagcccactg tggtgctgta tggatatgaa gtattgaaag aagctctgat tgaccgggga 300

gaagagtttt ctgataaaat gcattcgtca atgctcagta aagtcagcca agggttaggc 360

attgtcttca gcaatggaga aatatggaaa caaacacggc gtttctccct catggtctta 420

aggtctatgg ggatgggaaa gagaactatt gaaaaccgaa ttcaggagga agttgtttat 480

ctgctggaag cactgagaaa aaccaatggg tccccgtgtg accccagctt ccttctggcc 540

tgtgttccct gcaatctgat ctccagtgtc attttccagc accgttttga ctacagtgat 600

gagaaattcc aaaaattcat agagaatttc catacaaaaa ttgaaatttt agcatctcct 660

tgggcccagt tgtgcagtgc ctatcctgtt ctatactatc tcccgggaat ccataacaaa 720

ttcttaaaag atgtgactga acagaaaaag ttcatcttga tggaaataaa caggcatcga 780

gcatctctga acctcagtaa ccctcaggac tttattgact atttcctgat taaaatggaa 840

aaggaaaaac acaatgaaaa gtctgaattt accatggaca acctgatagt caccataggt 900

gatctttttg gagcaggaac agagacaacg agttccacca taaaatatgg gcttttgctc 960

ttgctgaagt acccagaggt cacagctaaa attcaggaag aaattactcg cgtgattggt 1020

agacaccgga gaccctgcat gcaggacagg aaccacatgc cctatacgga tgctgtgctg 1080

catgagatcc agagatacat tgattttgtt cccattccct tgcctcgtaa gacaacgcag 1140

gatgtggagt tcagaggata ccacattccc aagggcacaa gcgtaatggc atgtctgact 1200

tctgccctgc acgatgacaa agaatttccc aacccagaga agttcgaccc tggccacttt 1260

ctcgatgaga aaggcaactt caagaagagt gactacttca tggctttctc agcaggaaga 1320

agagcctgta ttggagaagg tttagcccgc atggagatgt tcttaatcct gaccagcatt 1380

ttacagcatt ttattttaaa acctcttgtc aatccagagg acatcgacac aaccccagtt 1440

caacctggtt tattgtctct gcccccaccc tttcagctct gtttcattcc agtctgaagc 1500

ggcatagtct ttcacacaga tgactgagac cctgcttcta cttgtgagca aacaggtcct 1560

cctgtcctcg tgtacctgta actggcagtc cttcctgaat ggcagggatg ctacctctga 1620

actgtcctac atttttttca attctttgtg aaccagttcc aatttttcat atgtgaaaaa 1680

acttcactgt tttagcacta ataaagtttt ttttc 1715

<210> 750

<211> 19080

<212> DNA

<213> Rattus norvegicus (Norway rat)

<220>

<221> misc_feature

<222> (6102)..(6102)

<223> n

<220>

<221> misc_feature

<222> (6103)..(6103)

<223> n

<220>

<221> misc_feature

<222> (6320)..(6320)

<223> n

<220>

<221> misc_feature

<222> (6321)..(6321)

<223> n

<220>

<221> misc_feature

<222> (6322)..(6322)

<223> n

<220>

<221> misc_feature

<222> (6323)..(6323)

<223> n

<220>

<221> misc_feature

<222> (6834)..(6834)

<223> n

<220>

<221> misc_feature

<222> (6840)..(6840)

<223> n

<220>

<221> misc_feature

<222> (6843)..(6843)

<223>  n

<220>
<221>  misc_feature
<222>  (6844)..(6844)
<223>  n

<220>
<221>  misc_feature
<222>  (6846)..(6846)
<223>  n

<400>  750
tcaacggatc tcactcttga ctgtccagtg ccagcacctt agcaaactcc cccagcttgg      60
aggcagtggc ccaccacatc atggaggtgt cgtctgagca caggttccgg aagactgtca     120
aagtctgcct taataggcag gggtactcag gggtagcaaa ctgagaagag accacaggcc     180
aaggtgtggg aggtgaacca gtctctaccc accagccact tcatcagagg caccaagtga     240
gcctctaaaa cggaaggtgg gtgctcatgt gagatgcccc acacggactt tactactgtc     300
tcttccactg tggccagtga ctcaagaggt ggcagcagac agtgtcacat actcaggacc     360
tcccaccaaa gtgtgaggct cccagctatt cagccaaggc caagaggacc ttggtgtcac     420
agatggcatc tgtaaggaag ggcaggagct aacttctggt tcattcaatg acaaaggcaa     480
aggccatggt ggagagcatc ttgatacagt tgagacagag ctgagtgtcc tcatcacaca     540
gttcatctat gagcaccatg gtggggtaga gcgaatcgtt gccgttgaca gctgctgtct     600
tggctccttg gcatttcttt ttaaaaagaa gagaaaaagg tcagcagtct gttttactta     660
tgtttgtttg taaatacaca cactgtacta gaagcatgaa attcagaaag aagctgacag     720
taagatggat agggaggttg gttactgact gtgtattcac cttaaatctc tgagggaatg     780
tggagccagc aatgactgtg tgtttgtgtc tccaagccta gcccacaggt tagcttacat     840
tggtgtgagg aacacagtat tgtataacac catacaaaag tgagcagaga cagggcccat     900
gaaatcacaa gtagaaagtg tttgttactg ttccacctgg tcacccaacc aggtgtcctg     960
tgtgtctaaa agccacacag aaagacaaaa tggagtatgt ttggggtggg gcgaagagag    1020
tgaggtattg gcagcccacg tgcgaagggg attgaaggaa acatgggtaa gattggatac    1080
aagttgggtt ttcagctgtt tgaagaattg gcttgtagag gaaaggacac tatattgtgc    1140
actgtcttag gaataagaca caggaggccg ttgtatggag gaaaatgcag gggcttcacg    1200
agagtgagca aagacctgct agaggtcttc tgagtcccca tgctctctgt gtgtttatat    1260
atgcatatac atagcatatt aatgtattaa taaattacat atataataag acaagatata    1320

ttatgtatat gtggtgtttg aatatgcttg gaccagggag tggcactatc aggaagtgtg 1380

accttgttgg aagaaatgtg tcactggttg tgaactttga gaccctcctc ctagctgtct 1440

ggaagacagt cttctcctgg ctaccttgga tcaagatgta gagctctcag ctccttctcc 1500

agcaccaggt cttcctggat gctgccatgc tcctcccttg atggtaatgg actgaacctt 1560

tgaacctgta agccagcccc aattaaatgg tgtcctttgt aagagttgcc ttggtcatgg 1620

tgtctcttcc cagcaataag accctaagta agacagaata tattaactat ttatctattc 1680

agatgctgtg acaggttaaa ccattctctc tgatagggag aactcagcta ttggtaattt 1740

tagtataatt taattgaatt ttttagacag gatcttacat atctcaagcc ggcttcaaac 1800

tcttatatag ccaaagatga tcttgacatc tggattctct tgcctttgcc ccctgagacc 1860

tgggtttcag catgcgccat cacaactgga gtatgtggtt ctgggaattg taccaagatc 1920

ctcatgcagc cttaggtaag cctaccaact aagctttgtc cctgggcctc attggtaata 1980

tttagaatta ccaagtagtt cttttggtta gccattgact ggttctacag aaatcacagc 2040

tgtgaagtaa taattggggt cagggtgatt gtagaagaaa tcttgatttg aagccgattc 2100

ttttggccaa ttgtcttggg gctcattagc ttacattcat agataaaggc tgtgataaag 2160

gctgtgactg ggaagtttcg aaatgaaatt cagaaagcgt ggcccgtctg cagcatgagg 2220

aaagctaaag atggcttaag gaacatcatc tttctccagg cacgtgctaa gcagtctagg 2280

actgagtagc cactgtcctt tctcctggga aatcccagat gggaaggatc aacccacaaa 2340

gcatttctgg cctcacggat gcccaagaaa aggtgctaag gaaactttct caacccttgg 2400

cagattcaac cacttatgat tttattagcc atggcttcgt catgtggttg acttgatgta 2460

cccttaaaga agtgctgcct agagactggt tcttagtgtg ggtcacggga ctctgctcat 2520

gcccacttct actttctttt cttggtaact gaagctgcct ggtcgcatgc tgagctcact 2580

gagccggcta atggcagggc tgtaggatgt gcaggaaggg caccaggtag acagtcagat 2640

gcaaagacat ttgggcagcc agagatgctg agcaaagctt aaagagaaaa aatagatcac 2700

cacaaagctc tttgtctcac tccagccctc ttccccacga ccacagaggg aagaagccaa 2760

gcctgagtcc tccgtcacag ctgcacatgt cccgtgactc agcacctcgc tcgaggctaa 2820

tgtggagtct ttagggtaag gaagtgggtg gtcctagtct gctctgcatg gtcaggtcac 2880

tcactcggca ttgataaact cagagctggg tttggggatt tcaagagcgg caaggtcaga 2940

aggtcaaagc ctggaagagt ccgcagatga agataagcaa ggagcttccg gttccaggtc 3000

tccaggggaa gccgactgag gcttcaacca taagatggac ttcgggagat ataactagta 3060

tttctttgtg tggttctgag gacagaattg agctcagggt cagcccttac agagcaggga 3120

gctccatcaa aaatggcttt cctaaagaat gaattgcttg caagggaggt agccagttct 3180

caggttggga gcatccaaaa ataaaagcag gaaaacctgg cggggaccct gagagacaag 3240

taggaagagg atcttgggtg aagggcagag tggtggcctt ctgcagtttc cctagaatac 3300

tgaaatccta gggcttgaga gatgaggtag ttggtaagac agaagcatga ggatctgaat 3360

tggatccctg gtacctgtgt acaacatggc ccatactcat aaaccagagc tgggagggta 3420

gagttgggat cctacagatt gttgggcagc tggtgagttc cataccagta agcgagcctg 3480

cctaaggtac aaacagttgg atggtgtaac atctgaggtc atcctgtggt ctcacacatg 3540

cacgtgtatg catttgcgcg cgcgcgcgcg cacgcacgca cacacacaca cacaaatcaa 3600

gaagtgcatt caagatttgt gtcagtttgc ttaaagctga attcccaagc ttacaaggtt 3660

tgggaacagg agaaaagtga ccacttgtaa ttgtgtcggg gacatcctta tctccctctc 3720

cccagatctc cctctgctaa ggcatatctc cctgcctgat taccaaagga cgacttttat 3780

agacactgct attgtaccct gtccctacta cacacaacgg gagggagctg ctgatcaaaa 3840

aaagttacaa ggacatcagt gtccatggaa aatgtagctg ttactgataa ttctttcttt 3900

ttatttttct ttataaagaa aacaaagaga agtttcgggg ggaggggcat tcctccaagt 3960

ttctgttgac tctgcttttc tggttagtta tcttcccaaa cttggcactg aaccagatta 4020

ggctaaagaa agtcttagag agggctcatt tcagattcat ctgccccaga ctgaagaaac 4080

taaggccaga gggcagagtg gtttgtctaa aggcactgga tgcctcgggg cacagctggg 4140

actgaagcca gccttagccc acttctgacc aagcctagac atttccagta ggctgccttt 4200

tgggaaagga ccaaatcacc atagcctctg ggccttggac aaatggaaat ctggcctcat 4260

ggctgttcct ttaaagatgt gagagggaag ctcggccctc gaccacacgt ttctccttca 4320

agccagtttt cttttattta catcgttttc aaggtctgcc gcccaaactg cgcgctgcct 4380

acatgggttc ctcgctattg cctgactctt gatacacaaa tgttccaggg tccagagtgg 4440

gaagcttggc ttctccccca agaagtagca tggtgggggg cttctaggtt gtttctcctt 4500

tccagagatg cctgtaacag atgtttctgc tcttagccaa aagcagctag gaatcctctt 4560

agcacctctg gacacagcta cttaatcagc agggctccat ggagaacttc cactatagct 4620

tcttgttcag gaaccaatgt gaaggccaag tgtgagacaa gtagtttttg tctaaaaatt 4680

tgtattatta tatattaatt atacacaatg agtatatata tatatatgtg tgtgtgtgtg 4740

tatatatatt cagcctcccc ccccatttcc ctttctcctc ccataatctc ttctctctgt 4800

tcctctcatg aatttcttta gggttgctca caggagcatg gagaggaggg ttattcacag 4860

gaacatggac acttaccagg gactatacta ctgaagacag ttgggtagtg tacttgaaaa 4920

taggtgtgag gttcctaggt ccagtgccag tgaagcccac cctgcctgac tgggttccaa 4980

gtgggtaaga accatgttta ggttttatgc atctcagaga caggggaggc cttctacgtt 5040

atctcattct tttttttttt tttttttttt tttggttctt tttttcggag ctggggaccg 5100

aacccagggc cttgtgcttc ctaggtaagc gctctaccac tgaactaaat ccccagcccc 5160

ggttatctca ttcttaaccc gtttctgatt ccttcatctg tctgccaagg gaccggcagc 5220

ccagtgcagc atttagggag ccaggtactt catgtatgct cttccctagc aagatgcctg 5280

taccatcttc agtttctttt actcttccat taaaatagga acaaagaaaa ggagtgcagt 5340

aatgggttaa tgtgaaagat gaacgtgggc gtcgggcggt gaaatcctga acctgtttgg 5400

ggctaggagc ggtccagagt agagtgtgga ttcctcttcc cttaaggctc ttctgttggg 5460
gcctgctgag cccttaggta cctagcagag aatagaacag ccatcaatct agctaggggc 5520
cctcaggcaa ccagcgcggt catttgtgat gcccctgcgc cccctggtgc ccccgctggg 5580
ctgtgcgcct ctcgtcccct cccgaccccc ccaccaggct tccagaaagc tcctagtgca 5640
ttccccggca ttctctggcg tgagtcacgc aggtttgcag ccagccaaag gggtgtactt 5700
tgagcagagc gctataaata gggcgcttcc ccggtgctca ccacccgcgt caccaggagg 5760
agcgcactgg agccaagccg cagacgggtg agacagctgc accttttcta ccccaacacc 5820
gaaggcacac cggcagggat gggagggcaa gggtaggagc caggtagaga gctgccgtgg 5880
gtaagtggga ggtgctagag ccccatcacc aggcagtcga gcttggggta gggggtgcac 5940
gctggctcag attcaggagc cacccccccg cccctgttct ttaacccgca tcacaattcc 6000
aaggctgttc aataggccac atgcaaagat cttccttttg caaaaatggg gaaactgaga 6060
cgtgaagcct gcagtgagat gcggatcgcg agggcgctag tnntgaacgc cgcgagtagg 6120
tgcggatctc tgtagtggct gttgtcttac cggcatgagt cattgcattt ggaccttccc 6180
tccagctcct gggacagcgc ataataaata agatgttgtg tatttaaaag gacgtgctgc 6240
agccatcgag acctgcttta ctgaacagaa tgcatgcatt cagttcttta gagaaatgct 6300
tccccaggcc tgagatgtcn nnnctaccgg atcattttat agatggtaat ctgaggactg 6360
gagacgataa gaggttcagc ctccaaacgt tctatgaatg agaaactgaa gccctttggc 6420
tttccagaat ttctagaccc agttttaaaa aacggtgctg gtgctgtttt taaaggacaa 6480
agttaggaat tcaagagttc atacaagttc tctaacagga aggtcttggg acaataccat 6540
ttagtgcctg accttgtgag aataagaaaa caaaccacaa atggtagttg gtggaatatt 6600
aggcagcagc aaatgtgtga ctttaaaaat atttgaaaac aggacctatg atggcattgt 6660
ttataaaaat gcattgaaag aaaaggagaa aatatgccac aatggaagag actgtgtttt 6720
cttactagat aaggtggtcc ttgcgctggt ggggctgttg taccagggta gttggcagcc 6780
aggcagtagg ccagcctcca aatgatgaca ggaagtgagt cagtgtagag gaanaggcan 6840
gcnngntgag ctgtgttgtc ctcggaccaa cagcaccaca gcacctagaa gcttgggaga 6900
caaacagatt ctgaccagag acagtattac tggcagacct caggggagtc tattcttaga 6960
tttacagaat agaggatcgc ttctttgaga tatcaggccc attttacaga taaagaaaca 7020
gaagctgtga gacttttgtt gtcaaggaaa taacctggga ggcgaatgac ttttgttgtc 7080
aaagaaagaa tattaaaatg aggttttgga gggctgcatt ctttcctggg gtgaccactg 7140
gcttgcagtg tgtcttgaga agcccaacag gtgtcttaga agtgctgagg ctgtctctct 7200
agaccccagg ctccttacag ctcatcttgc tactgcttcc cctcccattc agtgaccttc 7260
cttgctgcct gcttcctctt ctgggcattg tactgtcctc acatcagaag ttatttccaa 7320
ggcagttaaa tggtttttcc aaaaatggtc acacttaagg cctctcccac ctacagatat 7380
ctctgtggct ttgtctctga tgtggcaagg ggtctgtccc tgcctggggc atttcaggcc 7440

tcagtgagca cctgggaggt ctatgagatc agggccatcc tggtggatca tgaaccaggg 7500

cgccagccac ccaccatctc tgggaaagtg cttgtctctt ctcccacacc actcctctgc 7560

tctgtgtgca taattatgcc tcaggaaagc cttgcctatt aatgatctgc ccagtgtcct 7620

gatgggcctg cctagctggg agcaggtttt aagagcaagg catttcagga agacagatcg 7680

ctcccctttc cctgttcttg tggctgtagg aagctggtga gcccagataa gccattctgg 7740

gcagggttag agatgcaaga tgggtgtgtg tgtgtgbtgt gtgtgtgtgt gtgtgtctct 7800

ttatgcaacc aatccagctt ctgggaaagg atccattgtg taatactggg cttcggcctg 7860

tgtccctggc tcaggcagcc ggcccagtgg tggctggggc accttggcag agcatgctag 7920

ttcaggctta gagggagcag gcatgtgaaa gggatttgct ggagccaatg ggcttgtcct 7980

cctgaagggg ctttcctggg ggctcactgc taatctctgg ctttgtcctt ccctttctca 8040

gtgctgcctt ctctacagtc ctaaacagtc cctcccttcc ctttttgatt ttacccaact 8100

cccctcccca gtccatactc agcctggcac aagcttcacc cagagatggt gcattgggtt 8160

gaatttcctg tgctgatgga agggcgtgca tattaccacg gactcctgaa aagcacactt 8220

agacagcctc gggaggctgt accgtaaggg gagcccagga ctgtgttgtg agtgaggttc 8280

agtcccatgg aagggattgg cgccagccac ccaccatctc tgggaaagtg cttgtctctt 8340

ctcccacacc actcctctgc tctgtgtgca taattatgcc tcaggaaagc cttgcctatt 8400

ctgcctcggg gatttatggg aagaaaggac tctgcttgga gcttgtccct tccaactcca 8460

gatagcctgg gtcctctgtg cggctgaacc atggttcttc ccagggcccc actgcttcca 8520

ttgtcttttg aaggcatgaa tttaatcttg tttttgtccc cacgttcttc agaagtaaac 8580

tgcagtgatg ccagatgggg aaacagatga gggccatgag ttccaatccc atcccacctc 8640

ccctctcagg agcagaaaac ccctaagctg cccagaggtt accatgacat tctgctgttc 8700

tttgcaggac tccagactcc aaagaggcca caccatgaag attctcctgc tgtgtgtggc 8760

actgctgctg acctgggaca atggcatggt cctgggagag caggagttct ctgacaatga 8820

gctccaaggt agactaaact gctgtccctc tggtgggtct ggcagagcca cccctcaaag 8880

ttcagtccct aagtgctgag ggctgtttgc atactgcacc tgggactgtc catgtcctct 8940

gcactctcag agtctctaag ctctagctct tgccttggtg tttttgtggt cagatgcctt 9000

ctagacattg gcctctgtct caagcttctc tgttggctgc atgccatacc tttgtttcct 9060

ctaggtcccc ttacctggct ctcctattct tctctcaaag ctgtgtccgg accctttgaa 9120

agtttgtcag tctccccttt gtgtgacaac cagagttctt accaatgtgt ctccaagctg 9180

atttccccct ggaaaccttg ctctaccagt ttccttctct ggaaaagggc agcacagttc 9240

aacatgaagt agtcaactga gagcgttctc tcccttcctc cccatcttct aatgcttttc 9300

tggaaacccg cctttccttc cctccttctc tcctaatctc cgctcatagc tgaccagttc 9360

tgtgagaatt tcattaaaaa taatatgatc tgactctctg atatggatat gcttggtgaa 9420

atgactgctg tggtcaagcg cttagctgcc catcacctca acttattgtt gggttcagac 9480

acgtgagatg cattattcac ttagtgcacg tttttttttt tttttttttg gagctgggac 9540

cgaacccagg gccttgctgt gctaggcaag gttctaccac tgagctaaat ccccaacccg 9600

acttagcgca cttcaagggt agaacacatt gatcaaccac gagctctgct tggaccgtgg 9660

accttcaggc ttagtcatgc attgagactc ttgcccttgc tcggcagccc cccccgcccc 9720

catgcccttc tgtttggaag tgtttggcag tggcatcagg tagaacacag cagaggtgtg 9780

ggaaatggca cttgacacag gataagtaag tggtaccccg tgacataatg tgtcactgcc 9840

ttgtcaccag ggctgagtct tggttacggt ctctatggtg aatgcagctc ttcatttatc 9900

tccccgtctc catggtgttt ggtgccaagt cactcatctt ccggtcacca tcacctgaca 9960

ctgtgcattg cagcaaagaa gtgccaagac tgggttaacc tgaatgtcac aaagatcgtg 10020

tccaaaatgg cacttttatt atattttgat gcttaggtta actatctttt ttttctgtat 10080

ttccttgaaa tatcctctgt ggctgttggc tccccagggt ggcatggtgg ggtcagtcat 10140

acatagtatg gctgccctct ttgcaccact cctgtgcttc tgagctgtga cgcagggtct 10200

accatggacc ctttgatgag accaagggac aggactgagg ccacaggact gccacaggct 10260

gggggtagta gtgacagcat ctcacgttga cagcctccag cactcccatc agagcatggc 10320

tttctcaccg tcccgccggc ttccaggcta tttctccagc catttcttcc cttctcaact 10380

cactgtacct tcctgtctct cacagaactg tccactcaag gaagtaggta tgttaataag 10440

gagattcaga acgccgtcca gggggtgaag cacataaaga ccctcataga aaaaaccaac 10500

gcagagcgca agtccctgct caacagttta gaggaagcca agaagaagaa agaggtaggc 10560

ccagccttga ctgagccctg gctctccctg ctggaacacg gtggcaggtg ccttgctggg 10620

ttgccatgtg tcctggtgca gtctttgcag ggctttgcta atactgggcc agactaagga 10680

gccaggaaaa gcacattcgc ttgtggagcc tcagggctga ggtcttggat ctggtttcct 10740

tgcaagtctg cccaggcctg aagccaagat tcatttcaat aggtttttct aacccttgct 10800

gtgtgcaggg catggtgcag gactcgggag aggggtcatg gcagcacctg gccacttccc 10860

accactcttt tcttctgtat tcaaagtggc tcttaacatg aatgtgctta tttttaacat 10920

gaatggcaat attttaaagt taacgtaaaa tcctcatgat ctatccttag acatccattc 10980

atccccactc agcttcgggt gtggggttgg ggaccgtttt caaagccctt agcagaggct 11040

gaggttatgg gtagaaccag gccctctata gattgtccct acctgtgata atgcttactt 11100

tataaattac agacagtagg gtaggagcag caacgatgac gtagaatagt tacaatgtta 11160

caccaaacaa aaggttatgc agatgcaatt tctcccactc tcagaatatc tcagttcctc 11220

agcctcagca acactatttt tagactgtgg ttgaccatgg gtagctgaga actgataaca 11280

ggggactgcc ctgttaccat ttgtttaact tgccttttct acatgtggac gtctatgttg 11340

accctggtac acacacacac acacacacac acacacacac acacacacac acacacgaac 11400

acacatacta gaacttatac acagatccat ccactcattt attttccata tctttatgta 11460

gacgtacaca tttgctgtaa actaatatct cttcaggtgg agaatttaaa ttttttattt 11520

tggtgatccc tggagccaat aattgggtca gggggcatgg taatgaggtt gtggtcgatt 11580

tgacttcagg ttggatagct ttgatttgca catcaggggc cacggttcta atagaaagag 11640

aagactggcc tctttgggga gatgagaaga gacagtgagg tggcataagg gagagatggg 11700

tcctgaagaa gagacagtca gagcagcaaa tcagagcgga cagggaaaac ttagagaaca 11760

gctgggcctt gaaggatgag aggcccctgt gagcggtgtg ggacgccatg gacggatggg 11820

ctagagcaaa gctgtgggct gagtagcacc ttgtacacag atgggagaga tcggctgggt 11880

ctctgtgtgg gtcacctcac cttgtcatct ccatgaaggg tgctctagat gacaccaggg 11940

attctgaaat gaagctgaag gctttcccgg aagtgtgtaa cgagaccatg atggccctct 12000

gggaagagtg taagccctgc ctgaagcaca cctgcatgaa gttctacgca cgcgtctgca 12060

ggagcggctc ggggctggtt ggtcgccagg tgagaaggag acatgtccat ggcctgggtt 12120

ctggatgggg agtgtggaga atccacgaaa taggcctcac tccatatact gggtacagtt 12180

gattagcgtt gcttgactgc atgcctttct agtgtcgtgg tcgattggtg atgtctgaca 12240

cgagtacaca tgtagccaat catcatgggt atgcatgaca gtcttacaag acctttaacc 12300

agctgggtaa aatggcctca tggtatggta gaaccccatg ctcatatagg aaaggggggc 12360

taagaatatt tagtatattt gcatcaattc tatccctacc tgtgagcttc actggttggg 12420

actcagtgaa actcactgtt tcctgaaggc tgagcagcca agtgggagcc gctgtccttc 12480

atgaccaaag atgagccctc tgagtttcac cgtgagaggt ggctgcaggg cctgacaggg 12540

tattcaggag gcagaacagc gggaacttgg gtgagctgct gtgtggtcac agggtgaggc 12600

aggctggggt gcacaaggcc ttactgtact tctctgggtc ccagctagag gagtttctga 12660

accagagctc acccttctac ttctggatga acggggaccg catcgactcc ctgctggaga 12720

gtgaccggca gcagagccaa gtcctagatg ctatgcagga cagcttcact cgggcgtctg 12780

gcatcataga tacgcttttc caggaccggt tcttcaccca tgagccccag gacatccacc 12840

atttctcccc catgggcttc ccacacaagc ggcctcattt cttgtacccc aagtcccgct 12900

tggtccgcag cctcatgcct ctctcccact acgggcctct gagcttccac aacatgttcc 12960

agcctttctt tgatatgata caccaggctc aacaggccat ggacgtccag ctccatagcc 13020

cagctttaca gttcccggat gtggatttct taaaaggtca gaaagcattc gttgtctttc 13080

caagacggaa tgggagtctc caagatgttt aggctctgct agggcttgac atgtgtttta 13140

tgttcccagc agcccctgag gctggtattt tcccccattt gcagggtaaa ggagcatgga 13200

tgggggtaaa gcaatgcaga aggcaaactg ggacccagtt caatctcaca cttgtagagc 13260

ttggagcctg tgttctttac agccagctca gggtgtgatc cagctgtacc tcctctagga 13320

ccctggcttt tctttcttgg gaacttttga ggatgtggtg ttggaggcct gcctttccag 13380

gcaacaggtc tgcgtcatct taaagaagat ggtcatgact gcggcctaag catgtaacac 13440

ctgtaagatg aaatgtgatg tggacacgag aaacccgtgt ctgaggagtg attttcttca 13500

tcagtgtgtg tcctaatgcc gtgtccttca cgtatggttt gcacgagaaa cataggaagg 13560

cctggcaccc aattcacgtc tcttgtagct ctgttatcta aggatggtgt atgttgtcag 13620

gacacacttg tgctttctgg ggcttgatgg ccagttaggg agtaccgcca gccaaggaga 13680

atgaagaaca gccacgatgc cagaaggcac acagctgagg gaatgtgtga gatctctcag 13740

agggagatgg tgagaaggca ggcagtacct cgtaagagcc atgggaatat ggctagggag 13800

ccccagggaa atcgcatgtc tgcaagcgaa cgactaagct tcgtcttgcc tgaccacagc 13860

gctgagcaga ggtgtgggga ctcgggggtg ggtgtctttc caatgtgaca cgtgcctcat 13920

tctgcgcccc tttctcctca tgtgtccaga aggtgaagat gacccgacag tgtgcaagga 13980

gatccgccat aactccacag gatgcctgaa gatgaagggc cagtgtgaga agtgccaaga 14040

gatcttgtct gtgggtgagt cgggaaccag tctataatct gggcatctga ctaagggtca 14100

ccagtgacta actggtactg tccttcagag actagttctc tgcctgtgat ctgtagtccc 14160

tgggagactc ctccctggtg catagcttac tatgctcata cctacagctt cgttatttgg 14220

gagctggggt agaacccgtg cctcatgcat gctgggtgag tgctctaccg ttgagccacg 14280

ccaattttac ttagaatcaa atccaggggc agatttttct caagtagatg aggtctgtct 14340

aaagcaccca cacaccctgg tggctcatgg caaacctgca acccgcggtg gatatctcca 14400

tagaatttaa ccgtagccac cagcaatctg cagctcattc ccctggttgc tttgttccac 14460

agtgccaagt acagtagacc ctgcttttta actttagatt atgaaatctt tgacagcatg 14520

cattttgttt aatcagtgtt ccacatgcct ggcactgttg cgaacatgtc acaggtgttt 14580

gttctcagaa tgatcttgtg agcctggttc agctttcttt acattttgca gaatggctac 14640

atgacttatc taagattaca tgacatgaga catctcactt aggctgtgct caggaacagc 14700

ctgctggggt ttctccctgg gaaccctgag ctatgtcctt gcgatggtgg tccttgatat 14760

cctggccaca ttccttttgg ttgattagtc cctcagaaag gaactgtttt ccttcatcgg 14820

cttcccaggc agtctgggta tggcggcggc acatccctat agtaccaaca tctgggaggc 14880

tgaggaagga gggtggtgaa tctgaggcca gtcttggctg catagcaagg ttctgtctca 14940

gtgaaaacaa agtacagcaa acccagaacc gttccccaca gatttgtcag gtctggcggt 15000

caatcaaatg tttctgcttt cagtccggtc ttcacagtgg ctcacggtat acagcgcttc 15060

ctgctcagat cacaaggagt taatcgtcct ctttgttgtt ttgtcatatg tgtagttggc 15120

agggacagac gttcagagtc tggttaaata ttcccagcct cactcctgca atgttaggag 15180

cccaggaaag atacattact gctttcaaag gttatagtgt gaaattataa acaaatatgt 15240

acccacacca gcaggccgac ccccaggaaa ccgtcaggga tctcttccat caggcttagt 15300

gaccctctca acagtcaccg tttctgtcac ctgctagctc ctgtgtacgg tgcttacaat 15360

gacagggagg ctccacaggg acctctcatc tgggacaact tgactcagcc tgcgggtgga 15420

cccgaacatg gctgagcatt taacgttttt ggcttctacc tgccatgtgc caagtgacaa 15480

tcagaaatat cttcagaacg tgtggaatgt ccctggtggg tagtgtggtc ccaggttgag 15540

aaccacagtt ctacaccact ggtcccctcc aggactcttc cttgtttggg ctaagagtaa 15600

gggtactcag gctattcctc aaagatttct tctcatctgt gctgcttgtc tttaatcctc 15660
tttcttgccc catgcaacat tttatcttat ttagtatgaa tgattgcagc tgtcacgtgg 15720
ggcacagagg ctcccttgtc ttgcttagtt ttgatacaca gactctccta gatgttttaa 15780
aaatagtaga ggagggggtt ttcagaaaaa atggttttca gaaaacatgg gcattttgta 15840
ggacccagat gcctgatacc tgcaatgtac tcatggacca gagggaagac accagttttc 15900
ctgttttctg taggagcctt cctggccagc tgtgggtgga caagggcagg caaggaccga 15960
ccctcagcac cggatgcctg tgttttgtag cctaggaacc tggctgtctc ttctgcagct 16020
tgggttctgg gaaaatggta ttgtggaaat ttctcaaata tgttccaaaa taagcttgga 16080
gaatgttcta gaaccatggt ctcaggggag gaagagctca ggttgaggag ctgaagatgc 16140
gggttcagtc ctggttccag agattgcttc ccctctagat ctgggcaagt tccttacttc 16200
caaaaccctg tttcatgact ggtaaatcag gagtgttgat gcttgtgtca agcccagcat 16260
actttgaggc acacagtagg tccacctaag ggaataatgc tcctcttctc tgtggagtgt 16320
ccagtgcaac agtagcttcc ttctacttgg aaatgagcta agtggggagc taagggaatg 16380
agaataactc caggtctgag aacatggagt agttggtgtc agtactctcc agaacatggt 16440
cagtggcatt gtgacaggtc atcgagattg gtcatgccta ccacccagag aggattcaac 16500
cacactgctc caacacagcc tagcatgggc actattgcct cccaaagcgt gaagggagca 16560
gccccttgat ttccatgtca gggggactgt tctgtgggaa ctgtaagaac tggtttgtag 16620
acttccattg aagagctcct ctgcttcaga tcccagagct ggagccatcc cccttaacat 16680
ctctctcatg cttctgcctt ctgcttcttt cttccctggc cagactgttc gaccaacaat 16740
cctgcccagg ctaacctgcg ccaggagcta aacgactcgc tccaggtggc tgagaggctg 16800
acccagcagt acaacgagct gcttcattcc ctccagtcca agatgctcaa cacctcatcc 16860
ctgctggaac agctgaacga ccagttcacg tgggtgtccc agctggctaa cctcacacag 16920
ggcgatgacc agtaccttcg ggtctccaca gtgagtagta tcccagttat ttgatgagct 16980
aaggcccacg tgtgatccag aggtgatgga tgcctcttag aggtgccgtg ggacaaaggt 17040
ttacaccatc aggaactggg aaaaccatac tcgcgttgta gcattgaaat gccttcaccc 17100
ctcgaggtca gatatccaag tcttatttcc aagatgagga aagagagagg ttctgtggct 17160
tgctaagtag acggaacttt gggcagcttc cctgagtcag cctgtcagct gctgctttct 17220
gcccagggcc atgctgagtg ccgcatgaat tagtgttagc cagtgagatc ggcttccttt 17280
agtgctcaca gcccccctcg tcctctttcc accatagact ggctagcatt tgtacaaagc 17340
aaatgttggg gtcaggtgtt gtggtgagtg tctgtaagcc ggcaccaaag aaactgaggc 17400
aggaaagtgg agatcttgag cccgggctgg actatacagt gaaggcctct ctcaaacaag 17460
gcaaaacaag aagaaaacat ttgacaatca tttcaagttt ctggggtgcc tacttggcta 17520
gtcagttttc aaataacatc tgtcccttca aatcgagata gttttgctca gaattctctg 17580
aacatcttga gtagcaacag gtaacctgga gctccatact cggttgcagg ggcaaagaga 17640

agggcacgct ctgtagacat tgtgcagaga cacttctcct gccttcttct aggtgacaac 17700

ccattcttct gactcagaag tcccctctcg tgtcactgag gtggtggtga agctgtttga 17760

ctctgacccc atcacagtgg tgttaccaga agaagtctcc aaggataacc ctaagtttat 17820

ggacacagtg gcagagaaag cgctacagga ataccgcagg aaaagccggt aagcgggcag 17880

cctttctgtg accctgaggg ctgataaaca agtctttcta aacaagaccc tggaggactg 17940

gaaagtccga aggagcccag agaaggagca aaatatgcat tctggatgca gggcctatcc 18000

ctgaggccag accccacggg tgagctagga aaacccctgg ggattttatt ttctttgctc 18060

tgggaaatat ttttcctcag tggcaaggat gtgggtgttg gtggctgctt cttgggggga 18120

tattgattct cttctctact ctcctttttt ttccagcatg gaatgagaca gaagcatcag 18180

ttttctatat gtaggagtct caaggaggga atctcccagc tttccgaggt tgctgcagac 18240

ccctagagaa ctccacatgt ctccagcgcc taggcctcca ccccagcagc ctctccttcc 18300

tctgggttct gtactctaat gcctgcactt gatgctctcg ggaagaactg cttcccccac 18360

gcaactaatc caataaagcc accttgcgta tagtgtgcct tgggcccgct ttcatatttt 18420

catacttgat tctcctgcag gccgcacatc ttcatttgat atggagagaa aacaaggaag 18480

tagctgggtc agttcgtaaa gcacttgcca catagacatg agtttggtaa agtcaggcat 18540

ggtggcaccc tcttgtaact taatctcggc acctcagcgc tctcagagca gagggtccct 18600

ggagctggtg ggtggtcggt gagcgtcagg ccccagtgag agatcttgtc tcagaacaca 18660

ggtatgagga gaccaaagaa tgacacaagg ccacacatgt agaagaaatc tcgaagttaa 18720

cagctgagac tgtgtaagag tctccatata ctggagagta aagagtactc atgacaactg 18780

gggggacctc tggctttggg ggagaggttt gttccaataa gctgctgagg caagttcctg 18840

aagccagaaa cttttccctc ccaccacaac actgtccacg agttctgaca aagtggggca 18900

gtctcccaga gaatgcactg aaattaaggc aggaagagct gctagtggga cacatgggtt 18960

aatgtgtaac tgacaatttc ctgtggaaac tgctcagcag aagccaaatg aagaggcagt 19020

tttcttgttg tttgtaagaa catgggaacc tgggaccact tccggaccat ggcgtctccc 19080

<210> 751

<211> 9136

<212> DNA

<213> Rattus norvegicus (Norway rat)

<400> 751
   agagacggca gcagcgtccc gtccagttcg cctgccgcgc tcgcttgtcg tctgcctcca 60
   gatcccagac agagaagagc catggaggag gtggtgatag ccggtatgtc cgggaaattg 120
   cccgagtcag agaacctgca ggagttctgg gccaacctca ttggcggtgt ggacatggtc 180
   acagacgatg acaggaggtg gaaggctggg ctctatgggt tgcctaagcg gtctggaaag 240
   ctgaaggatc tgtccaagtt cgacgcctcc tttttggggg tccaccccaa gcaggcacac 300
   acaatggacc cgcagctccg gctgctgctg gaagtcagct atgaagctat tgtggacgga 360
   ggtatcaacc cggcctcact ccgaggaaca aacactggtg tctgggtggg tgtgagtggt 420
   tccgaggcgt cggaggccct gagcagagat cctgagactc ttctgggcta cagcatggtg 480
   ggctgccaga gagcaatgat ggccaaccgg ctctctttct tcttcgactt caaaggaccc 540
   agcattgccc tggacacagc ctgctcctct agcctactgg cactacagaa tgcctatcag 600
   gctatccgca gtggggagtg ccctgctgcc attgtgggcg ggatcaacct gctgctaaag 660
   cctaacacct ctgtgcagtt catgaagcta ggcatgctca gccccgatgg cacctgcaga 720
   tcctttgatg attcagggaa cgggtattgc cgtgctgagg ctgtcgtggc agttctgctg 780
   actaagaagt ccttggctcg gcgagtctat gccactattc tgaatgccgg gacgaacaca 840
   gatggctgca aggagcaagg cgtgacattc ccctctggag aagcccagga acaactcatc 900
   cgttctctgt atcagccggg cggtgtggcc cccgagtctc ttgaatatat tgaagcccat 960
   ggcacgggca ccaaggtggg ggacccccag gaactgaacg gcattactcg gtccctgtgt 1020
   gctttccgcc agagcccttt gttaattggc tccaccaaat ccaacatggg acaccctgag 1080
   cctgcctcgg ggcttgcagc cctgaccaag gtgctgttat ccctagaaaa tggggtttgg 1140
   gcccccaacc tgcatttcca caaccccaac cctgaaatcc cagcacttct tgatgggcgg 1200
   ctgcaggtgg tcgataggcc cctgcctgtt cgtggtggca tcgtgggcat caactcgttt 1260
   ggcttcggag gtgccaatgt tcacgtcatc ctccagccca acacacagca ggccccagca 1320
   cctgccccac atgctgccct accgcatttg ctgcatgcca gtggacggac catggaggca 1380
   gtgcagggcc tgctggaaca gggccgccag cacagtcagg acttggcctt tgtgagcatg 1440
   ctcaatgaca ttgcagcaac ccctacagca gccatgccct tcagaggtta cactgtgtta 1500
   ggtgttgagg gccatgtcca ggaagtgcag caagtgcctg ccagccagcg cccactctgg 1560
   ttcatctgct cagggatggg cacacagtgg cgtggaatgg ggctgagcct tatgcgcctg 1620
   gacagtttcc gtgagtccat cctgcgctct gatgaggctc tgaagccctt gggagtcaaa 1680
   gtgtcagacc tgctgctgag cactgatgag cacacctttg atgacatcgt gcattccttt 1740
   gtgagcctca ccgccatcca gattgccctc atcgacctgc tgacgtctat ggggctgaaa 1800
   cctgatggca tcattgggca ctccttggga gaggttgcct gtggctatgc agatggctgt 1860
   ctctcccaga gagaggctgt gcttgcagcc tactggagag gccagtgcat taaggatgcc 1920
   aaccttccgg ctggatccat ggcagctgtt ggtttgtcct gggaagaatg taaacaacgc 1980
   tgccctcctg gtgtggtgcc tgcctgccac aactctgagg acactgtgac catctctgga 2040
   cctcaggctg cagtgaatga atttgtggag cagctaaagc aagagggcgt gtttgccaag 2100
   gaggtgcgaa caggtggcct ggccttccac tcctacttca tggaaggaat tgcccccacg 2160
   ctgctgcagg ctctcaagaa ggtgatccgg gagccacggc cacgctcagc acgctggctc 2220
   agcacctcta tccctgaggc ccagtggcag agcagcctgg cccgcacatc ttctgctgag 2280
   tacaacgtca acaacctggt gagccctgtg ctcttccagg aagcactgtg gcacgtcccc 2340
   gagcacgccg tggtgctgga gattgcaccc catgcactgt tgcaggctgt cctgaagcga 2400
   ggcgtgaagc ctagctgcac catcatcccc ttgatgaaga gggaccataa agataacttg 2460
   gagttcttcc tcaccaacct cggcaaggtg cacctcacag gcatcgacat caaccctaat 2520
   gccttgttcc cacctgtgga attcccggtt ccccgaggga ctcctctcat ctcccctcac 2580
   atcaagtggg accacagtca gacttgggat atcccagttg ctgaagactt ccccaacggt 2640
   tccagctcct cctcagctac agtctacaac attgacgcca gttccgagtc atctgaccac 2700
   tacctggtcg accactgcat tgacggccgt gtcctcttcc ctggcactgg ctacctgtac 2760
   ctggtgtgga agacactggc tcgaagcctg agcttgtccc tagaagagac ccctgtggtg 2820
   tttgagaacg tgacatttca tcaggccacc atcctgccca ggacaggaac cgtgcctctg 2880
   gaggtgcggc tgctagaggc ctcacatgca tttgaggtgt ctgacagtgg caacctgata 2940
   gtgagcggga aagtgtacca gtgggaagac cctgactcca agttattcga ccacccagaa 3000
   gtcccgatcc ccgccgagtc cgagtctgtc tcccgcttga cgcagggaga agtatacaag 3060
   gagctgcggc tacgtggcta tgactatggc cctcatttcc agggcgtcta tgaggccacc 3120
   ctcgaaggtg agcaaggcaa gctgctctgg aaagacaact gggtgacctt catggacaca 3180
   atgctgcaga tatccatcct gggcttcagc aagcagagtc tgcagctacc cacccgtgtg 3240
   actgccatct atattgaccc tgcaacccac ctgcagaagg tgtacatgct ggagggagac 3300
   actcaagtgg ctgacgtgac cacgagccgc tgtctgggcg tgaccgtctc tggtggtgtc 3360
   tacatttcga gactacagac aacagcaacc tcacggcggc agcaggaaca gctggtcccc 3420
   accctggaga agtttgtctt cacaccccat gtggagcctg agtgcctgtc tgagagtgct 3480
   atcctgcaga aagagctgca gctgtgcaag ggtctggcaa aggctctgca gaccaaggcc 3540
   acccagcaag ggctgaagat gacagtgcct gggctagagg accttcccca gcatggactg 3600
   cctcgactct tggctgctgc ctgccagctg cagctcaacg ggaacctgca actggagtta 3660
   ggtgaggtac tggctcgaga gaggctcctg ctgccagaag accctctgat cagtggcctc 3720
   cttaactccc aggccctcaa ggcctgcata gacacagccc tggagaacct gtctactctc 3780
   aagatgaagg tggtggaggt gctggctgga gaaggccact tgtattccca catctcagca 3840
   ctgctcaaca cccagcctat gctgcaactg gagtatacag ccaccgaccg gcacccccag 3900
   gccctgaagg atgttcagac caagctgcag cagcatgatg tagcacaggg ccagtgggac 3960
   ccttctggtc ctgctcctac caacctgggt gctcttgacc ttgtggtgtg caactgtgcg 4020
   ttagccaccc tgggggatcc agccctggcc ctggacaaca tggtagctgc cctcaaggat 4080
   ggtggtttcc tgctaatgca cacagtgctc aaaggacatg cccttgggga aaccctggcc 4140
   tgcctccctt ctgaggtgca gcctgggccc agcttcttaa gccaggaaga gtgggagagc 4200
   ctgttctcaa ggaaggcact gcacctggtg ggccttaaaa agtcattcta cggtactgcg 4260
   ctgttcctgt gccgccgtct cagcccacag gacaagccca tcttcctgcc tgtggaggat 4320
   actagtttcc agtgggtgga ctctctgaag agcattctgg ccacatcctc ctcccagcct 4380
   gtgtggctaa cagccatgaa ctgccccacc tcaggtgtgg taggcttggt gaactgtctc 4440
   cgaaaagagc cgggtggaca ccggattcgg tgtatcctgc tgtccaacct cagcagcaca 4500
   tctcacgtcc ccaagctgga ccctggctct tcagagctac agaaggtgct agagagtgat 4560
   ctggtgatga acgtgtacag ggacggtgcc tggggtgcct tccgtcactt ccagttagag 4620
   caggacaagc ccgaggagca gacagcacat gcctttgtaa acgtccttac ccgaggggac 4680
   cttgcctcca tccgctgggt ctcttctccc ctgaaacaca tgcagccgcc ctcgagctca 4740
   ggagcacagc tctgcactgt ctactatgcc tcactgaact tccgagatat catgctggcc 4800
   acgggcaagc tgtcccctga tgccattcca ggtaaatggg ccagccggga ctgcatgctt 4860
   ggcatggagt tctcaggccg tgataagtgc ggccggcgtg tgatggggct ggtacccgca 4920
   gaaggcctgg ccacctcagt cctgttatca cccgacttcc tctgggatgt accctctagc 4980
   tggaccctgg aggaggcggc ttctgtgcct gttgtctaca ccaccgccta ctactcctta 5040
   gtagtgcgtg gtcgtattca gcacggggaa actgtgctca ttcactcggg ctccggtggt 5100
   gtgggccaag cggccatttc cattgccctt agcctgggct gccgagtctt caccactgtg 5160
   ggctccgctg agaagcgagc ttacctccag gccagattcc ctcagctgga tgacaccagc 5220
   tttgctaact ctcgagacac atcgtttgag cagcatgtgt tactgcacac aggtggcaaa 5280
   ggggtggacc tggtcctcaa ctccctggca gaagagaagc tgcaggccag tgtgcggtgc 5340
   ttggctcagc atggccgctt cctagagatc ggcaaatttg atctttctaa caaccaccct 5400
   ctgggcatgg ccatcttctt gaagaacgtc actttccatg ggatcctgct ggatgcactt 5460
   tttgaggggg ccaacgacag ctggcgggag gtggcagagc tgctgaaggc cggcatccgt 5520
   gatggggttg tgaagcctct caagtgtaca gtgtttccca aggcccaggt ggaggacgcc 5580
   ttccgataca tggctcaagg aaaacatatt ggcaaagtcc ttgtccaggt acgggaggag 5640
   gagcccgagg ctatgctgcc aggggctcag cccaccctga tttccgccat ctccaagacc 5700
   ttctgcccag agcataagag ttacatcatc actggtggcc taggtggctt tggcctggaa 5760
   ctggcccggt ggcttgtgct tcgtggggcc caaaggcttg tactaacttc ccgatctgga 5820
   atccgcacag gctaccaagc caagcacgtt cgggagtgga ggcgccaggg catccatgtg 5880
   ctagtgtcga caagcaatgt cagttcactg gagggggccc gtgctctcat cgctgaagcc 5940
   acaaagcttg ggcccgttgg aggtgtcttc aacctggcca tggttttaag ggatgccatg 6000
   ctggagaacc agactccaga actcttccag gatgtcaaca agcccaagta caatggcacc 6060
   ctgaaccttg acagggcgac ccgggaagcc tgtcctgagc tggactactt tgtggccttc 6120
   tcctctgtaa gctgcgggcg tggtaatgct ggccaatcca actatggctt cgccaactct 6180
   accatggagc gtatttgcga acagcgccgg cacgatggcc tcccaggtct tgccgtgcaa 6240
   tggggtgcca ttggtgacgt gggcattatc ttggaagcga tgggtaccaa tgacacagtc 6300
   gttggcggca cactgccaca gcgcatctcc tcctgcatgg aggtgctgga cctcttcctg 6360
   aatcagcccc acgcagtcct gagcagtttt gtgctggttg agaagaaagc tgtggcccat 6420
   ggtgatggtg aagcccagag ggatctggtg aaagcagtgg cacacatcct aggcatccgc 6480
   gacctcgcag ggattaacct ggacagctcg ctggcagacc tcggcctgga ctcgctcatg 6540
   ggtgtggaag tgcgccagat cctggaacgt gaacatgatc tggtgctacc cattcgtgaa 6600
   gtacggcaac tcacactgcg gaagcttcag gaaatgtcct ccaaggctgg ctcagacact 6660
   gagttggcag cccccaagtc caagaatgat acatccctga agcaggccca gctgaatctg 6720
   agtatcctgc tggtgaaccc tgagggccct accttaacac gactcaactc agtgcagagc 6780
   tctgagcggc ctctgttcct ggtgcacccc attgaaggtt ccatcactgt gttccacagc 6840
   ctggctgcca agctcagtgt gcccacctac ggtctgcagt gcacccaagc ggcccccctg 6900
   gacagcattc caaacctggc tgcctactac attgattgca tcaagcaggt gcagcctgag 6960
   gggccccacc gagtggctgg gtattctttt ggagcttgtg tagccttcga gatgtgctcc 7020
   cagctgcagg cccagcaggg cccagccccc gcccacaaca acctcttctt gtttgatggc 7080
   tcacacacct acgtattggc gtacacccag agctaccggg caaagctgac cccaggctgt 7140
   gaggctgagg ctgaagctga agccatatgc ttcttcatta agcagtttgt tgatgcagag 7200
   catagcaagg tgctagaggc cctgctacca ctgaagagcc tggaggaccg ggttgctgct 7260
   gctgtggacc tcatcactag aagccaccag agcctggacc gccgtgacct gagctttgct 7320
   gccgtgtcct tctactacaa gcttcgagcc gccgaccagt ataaacccaa ggccaagtac 7380
   cacggcaatg tgatcctgct gcgggccaag acaggtggca cctacggcga ggacttgggt 7440
   gccgattaca acctgtccca ggtgtgtgat gggaaggtgt ctgtgcacat cattgagggt 7500
   gaccatcgta cgctgctgga gggcaggggc ctggagtcta tcatcaacat catccacagc 7560
   tccctggctg agcctcgagt gagtgtacgg gagggctaga cctgcctacc atgaagccac 7620
   gacccacacc ggccaccaga gatgctccga tccccatcac accctgagtg cagggactgg 7680
   ggagggtcct gctggtggga ccccctcacc ccagtggccc agcaccaccc cctcccctgg 7740
   tggctgctac aaacaggacc atcacatgtg tcccagccac ttagtggggt tcccagagcc 7800
   actgacttgg aggcaccctg gtctgtgaag agtcagtgga ggccagcaag agccaaactg 7860
   agccttttct gccaagtgac atttgtcaca ctggttgttt ctccattaaa ttctcatatt 7920
   tattgcattg ctgggaaaga ccgcccaccc cagggttaac tcattccaga acccctaaag 7980
   tgggaaaagc catgtgggga aggctgctgg ctggagcccc tttttgtctt agccctgtac 8040
   ccgctcactg cagggcaggg tatggagagg gctggttccc ggggaacgag gaccccagca 8100
   gacactgtag cccatggccc ttggtcccca gcactcccgg ctgcacccat gatgcagggc 8160
   ctaccagact ctgcggaccg caccgggcac tcactgtatt tgttttccaa gattcaaatt 8220
   gctgcttggg ttttgaattt actgcagctg tcagtgtaaa gaaacatgtc tgaactgtgt 8280
   cctttttaca ccaacctggt aaaaatgctc ttgatgctgt cccgttgcca caattaaact 8340
   gcacgtgagc tctggcttcc gttcagtctc tttccagtcc cagacctgag tccccagagc 8400
   ctccacagct cttacagtga gaatcaaatt ggcccactcc ttggaaggcg tggcattctg 8460
   tcagagtaaa aggaaagtag agtgtgctga ttcacgttca gcgtgtgggg ctggctagag 8520
   accttggcac tgtagtgaac agaatgtgtc cacctttaag tcaccctgaa ggcatcacca 8580
   tagctacagc ctcacccagg ggtagagaat agtactgtct acttgttgac tacctggcag 8640
   ttggtgccag cccctataga ggaaaacagc agtgtgtggc cactgtgaga agcatatccc 8700
   tggaaacagg tgaccagagc agagggctaa tgcctacctg agtcacacaa aactgaccag 8760
   gcttgagtgt ccagaagagt ctatcagaag gccacagcat tcagtcctat ccacagagag 8820
   cagcagacta agttgtctcc ttgccagctt agaaaactgc agtgctgggg tacaggtagg 8880
   gtgttcagga ggtccgggcc ccagtgatta gtctaagact gaagcatctg gttggctgtg 8940
   gtcccaccta gaaaattctt aaagctcttg tcatgtactt cctgggaagg acctaccctg 9000
   tctcaataat gtctctagct cgttggagtc tactgactca aacatttata aagtgtccta 9060
   gaaaggcctg actcccctac aaggctgtgt gatccttcaa actcacatat gtgagccaat 9120
   aaaaccttga gactct 9136

<210> 752

<211> 560

<212> DNA

<213> Rattus sp.

<400> 752
gaaagtgtct gcaagtttta tttgtagact ctgttaagct tgaaccataa aagctgcaaa 60

acagtggttt agagagcatg tcaataccat gggggttggt gggtggaaac tgttccttct 120

gccagttcct aaggctggaa gtggctaggg caggcagtgg cgaggaaata gctggatgag 180

ctgaagcttg ggtggcagtg cttactcaag cctgactcct gcctgtctca ggccctgggg 240

tcatatacac ggcccatgaa gactgggaac ttgtgtcgct ggtcccagag caggaagagg 300

aaaggctgct gcacctcaaa gatgagtaag tttcgggcca cggagatggt ggaggctgcg 360

gctgcttcca cacctgtctc tgtcagttcc aacaccgtct cgtgtttcat ggaagacacc 420

tgaagatctg ggtcctcagt cagcccacac aggttgagat cgtaagtgaa gtcaaagaat 480

tccagtttct ccatgattga cagcatgtct tggatgctct ttactttaat gcgaggcatc 540

atcacgtaag tgggctgaaa 560

<210> 753

<211> 400

<212> DNA

<213> Rattus norvegicus (Norway rat)

<400>  753
tttttttttt tttttatgtt cagatgcatt tattcagcaa accttcttgc taaacattat      60

ggtagcccta aaagatttcg aaatcaggac attcagcaaa gccctctcct ctgaatgcgg     120

atagatgttc caaatttcct gttttcaaag catagaatgc attcattggc gtaagttttt     180

ccgtcagtac cacacacagg atcataatcc ctggggcatc caacaagtgt attagggcaa     240

ttagcctttt tcccaatcac cttagctgta gtgttacctg ctaaattgag cagggccaaa     300

gcactgagaa gaaagataat tgctaccttc atgggtgtag acctaggtcc aaaactcaga     360

agaactgtgc agggtgcttc tcttcaacct cgtgccgaaa                           400

<210>  754

<211>  533

<212>  DNA

<213>  Rattus norvegicus (Norway rat)


<220>

<221>  misc_feature

<222>  (525)..(525)

<223>  n


<400>  754
tttttttttt tttttctgag agccacagaa gtcctgctaa cacctggaca aggagcctgg      60

tgtagcctcc tacaccacac agcttcgcca aagccacgaa gagaaaggca tctactcacg     120

tgcccaggaa gacatccttg gcctcagcat agttcttaca cacttcctta tcctcaacaa     180

agtcagcagc tattgagggc agatcggcag gaatgttgtc atgttctgtc tcagcgagac     240

actgggattt ctgcagcact ggcttatcac agcaagcctg cagtttgctg gagatagtgg     300

cctggttctc acacatgtac ttggcaagtt ctgcctgtga gaatggaagg caaaggtttc     360

agagaaattc tcttggctac attttatccc cgcaccactg ttgggctggg atagcctatc     420

catcctgctc tgagccattc gcaaccaaag tggcttctag aaacacactc tcagccacga     480

agtgcttcag tactatgacc agaacattcc aatgggattc gaganagact gtt            533

<210>  755

<211>  559

<212>  DNA

<213>  Rattus norvegicus (Norway rat)

<400> 755
tttttttttt tttttttgta ggattattag ttttcacatt tctattggag ggcaacaggg 60

aagggacgga ttccatcaac agaagcattc acaggttctt gagccattcc aagcttggac 120

ccagaggttc ctgtgggtgg ctgggcacat caggcatgtt cccattatct ctcacaggca 180

ctgggtagtt gtagggtgtg gccttgttga tcatgctagc atactgggtg taggggctaa 240

tcatgggcat aattatagcg aggccccaga cagtgaagga caccaccagc accggctcct 300

ttgcccaggc attcttaagg aaggcggaga ttcctagagg tgagaaaaaa gtcgcccttg 360

gggactgctg cggtgacctc taacactctc gtatcaccct gctctgcacc ctattggctg 420

ctgcgcccct cttctgggac ggatcctacc actccccaag gctctactga gcctcagcca 480

cgggtcaagc cacggtttcc tggaattaca tggtccccgc gcgcccagaa cgcgtactta 540

ctagccctcg tgccgaatt 559

<210> 756

<211> 555

<212> DNA

<213> Rattus sp.

<400> 756
ccagggacct ccgtagtcgg tctccctatc ccactcccaa acctcagagc aggaaatggg 60

cttggctgag aagattcatg cttgatgacc aggggaggcg tgcagccccc caagaagaag 120

gggaaaagaa aaacggggag gttgaaaagc agagaggtgc accttccctt ctgaggaagc 180

aattctggtc tgggaccagt tgcaaggggt tagtaagaga aacctaaggg gtgcttacat 240

ttttattctg gcaaatgaat ctcttaaaag gctccctcct aggggtgctt acatttttat 300

tctggcaaat gaatctctta aaaggctccc tccttcgttc gggggaacag cacatgtacc 360

tgtgtcagcg tgagatgcaa tgctacacaa gaacgtggca ttgggccaat catgtggacc 420

cctgtgctgc tcccaaggga gaggttctgt ttgggtgtgg gataaatcta aacaagcata 480

cactcgtgtt atatgtggcc ttaagggtag gggagcaaaa ggaatggact tctctgtaga 540

gcagctcaag aggga 555

<210> 757

<211> 711

<212> DNA

<213> Rattus sp.

<400> 757
caaacttttt ttttattttt tttttttttt ttgcgttgaa aatcatttaa tgagaattat 60

actttttctc tatcagatta gatctgttct agtgattatt gtctggtgtt acaagctcat 120

ctgtctcaga gttggttagt gtgcatgaag ctacttaaga aagatatgga caagcacagc 180

tcactggtcc ccctgagcac ctcacaagac tgggaacatc ctctcaaggg ttaaaacaga 240

cgggcatacg ggctgagaat ttattttgaa ggcaggataa acaggccagc tctaagaaga 300

tactgttttg aaagctcaca cgtttcataa gtttctgagt tcaggcagtc tcagttctca 360

tccactgagt cctgggccct caaaagccct ccaggtccac cacagaggct gttctaactg 420

ggtgctgagc ctatacaaag ccctggcact cacacaactg ccattcccca agatcaactc 480

gtctcctgta aatatgtgct gtttgtatcc ggcacagggc tgatgaagat ggatcccgat 540

ggagcctact gtgcattact tcctgatgtg cagagatgtg tgtggtgaga ctgtgcggga 600

tgctgggagc agtgtgcagt gcgttgggag actgagaagt gaaagccctg ttggtttgag 660

gggaaagcaa ggaaatgtga cctttcccat agctactccc acagtgagaa a 711

<210> 758

<211> 153

<212> DNA

<213> Rattus norvegicus (rat)

<400> 758
gccatgcccc gtgtctcaca gcattgtaac gttctccctg gtgccccctt ggcatgtatc 60

ccttgcttgg ccacctcagc ctccctcccc accatacact ctatcctgaa ctacagagga 120

acagagagca gacaaacgtc agaatgagag cag 153

<210> 759

<211> 664

<212> DNA

<213> Rattus sp.

<400> 759
gcaagcaatg cgcagacaaa aactaaattt attaattaaa gattcatttg aataaatttt 60

gtacagaaga atagtattta ttgcatctat agtataaaag aactgctcac caagctttat 120

aaagtatgaa gcaataagga actaattact taaagtgttt ctatggaggt aagtgtcagg 180

gtaaattgag ttataaccag aatacatcat atgtatgatg atgctaacat tttaccaagt 240

tatttttatc ataatgctta atctgcttaa tcatacatca tcatgatatg cgaaatcata 300

attttttcca ggattggacc acaacatcac gtctctgaac tacaaatata aatatgttac 360

accaatcaca tatttgtaaa aatttataaa ataaaagttg actgggaatc ctgtagcctg 420

gactgatgtt cagaagaact caatctgatt catcgctgct gaagtaggag ctgtcgctgt 480

actcagctgt gtaaaggaac ttgtgaatga tcggattcat ctttggtatc cagtgtcggg 540

gaaccaggta cgatgagagg ttaaataaat ccacgaacac cttgtacctg tcgctcacag 600

ttgagcgcag ataataatag cctgaggatc ccccagtgcc agccttgctg cctagcatcc 660

tgtg 664

<210> 760

<211> 654

<212> DNA

<213> Rattus sp.

<400> 760
aactggggac atgtgtttat tcagcagaaa gggggagccc gttggtgggg cagcgttaac 60

acaggtggat ggggagagct gatcaaataa gagggatcag atgcttggtg gaatcatggc 120

tggtggggca gccaggtgat tctctggcaa caggaaagat ggagcggcag acagacaggt 180

gggacctgaa ccatcaggcc actgcacatc ccagtcagcc acgctcaccg tctgttcagt 240

tgtcaatgca ttggtcggtg ggaacagaga aaacgatgtt gttatccttg aggcccagag 300

acttggcaaa gctgacgaat cgctccttca gttcatcgga cagccccttg gttcttccgt 360

acagggtgac tttgaagtac tgtttgtttt cagaggtctt ctggaaaaat accatggcaa 420

actggtcgta gtcagtgtcg gccacttgca catcgtagct ctgtatctga gggtagctgt 480

gaatattccc cagggtgaac tggccaggcc tggagcttgg aacgaatgtt ctgatccagt 540

agcgacagcc ctggcccctg acgaggatgg aagtgacgtt gtagctattg tcttcctgta 600

actcatagat ggtgctgtac atggtaaagc ggctttgtct ttctttctgg accg 654

<210> 761

<211> 423

<212> DNA

<213> Rattus sp.

<400> 761
   gttcctaaat tttatttatg aattaataca aaacatggca gaccaatgtg tcttaatcct 60
   catcctcctc ctcctcctcg tcctggttga tctggaagta gcgcagctcg tagctctcct 120
   tgctgttggc aacgacgcgc agccagtctc ggaggttgtt cttcttcaaa tatttttggg 180
   tgagatattt caaatacctt ttggagaaag gcacctcgga agtgacagtg atcttgctct 240
   tgctccgctc gatggtcaca acccctccgc caaggttacc agctttcccg ttcaccttga 300
   tcctctcctg gaggaactgc tcaaaattgg cagcatccat gattccatct tctacagggt 360
   gagtgcagtc aagggtgaac ttcaaaacct gcttcttttt ttttgccccc cttcgccaca 420
   agc 423

<210> 762

<211> 495

<212> DNA

<213> Rattus sp.

<220>

<221> misc_feature

<222> (308)..(308)

<223> n

<400> 762
cactcacaca caatattgta tgagacatta ctagttttca tatgcatcca taggattaat 60

actcatatgg agtataacat ggaaaagtgc agaactaaag aaataactct aacaacaaaa 120

gaagcacacg tttcttaaaa tttaaaaata ttgcacatag taaggttgca cagaaattac 180

tggctgcttt tacaaaaaga atgaggtatt agtcaatctc tagataaaga taagtgcaac 240

agagtaacta cacacacaca caccaaaaac aaaaaacaaa ttcatacgaa gacctagaaa 300

gtcaacanac aaaaatattc aaaaagaagt ttacaaagct atgattttga tcatacaaaa 360

tcattagccc attactaaaa cacaatttct tggcaatagt ttataaattt taaactttct 420

tcagcttaac tacgtgcaat acaatcatcc ccatcacacc aattctcctg tagaagagga 480

atctttctgt gatag 495

<210> 763

<211> 613

<212> DNA

<213> Rattus sp.

<400> 763
aatttttatt tcaaaagctt ggatagcttc aatatccagg tcgtggcaaa atcaggacac 60

gtgtaaaata ccttacaata cattagattc ccaaaaggta ccaaaaagta cagtaaaatt 120

aacacttcca ttactggaaa tgtatgacac aaataatata aaattaaaag gtgaaaaagg 180

tgacactggt ttcctaagat acagtttact ctttacaacc agggtccaca ggtccaggct 240

gcagagcggc agcaggaagc agaccctcca atctagaaca gggtaacctg gtaataaaat 300

cagcccccta atgccgctct gacctctgag acgccacacg ctgcctacaa caactagagc 360

tctggaatag taaacaggag agtgatttcc gggggaaatt ttaaataaga tgcacatggg 420

acaggccata aaagtttgcc aagttaaatt tggtacataa ttgtgttcac tccgcgtcca 480

aacaaagcgt gtgcggtcgt cactcggccg ctcctgctgc ctctcagtat ggcttgtagt 540

tattctgatg accaccacgt cgctggctct tcccgtaatt tgtactaccc tgactgtagt 600

cgtagccggg gcc 613

<210> 764

<211> 449

<212> DNA

<213> Rattus norvegicus (Norway rat)

<400> 764
tttttttttt ttttcttgaa aaaacggggt ttattgattt ttaaactgca aatagtcgtt 60

acaaaaagtt tttttttctt ttaaataaat tcacacaaag aaagagaaat agaaagcgac 120

ggtagtgacc agcaagagga ataataatta cattcatctt tatgtgtgtg ccagttgttt 180

acatcaacat cggaaaactc caacctggag tccagaacct caaatctgca agcggaatgt 240

cttgagatgg gcacatggaa ttcaaagggt ttttctctct ctctcttttt tttttttttg 300

ttttcccttt tagaagctat acataaatgg tggttttcct tctgtactgt cacagaactt 360

atacattctc agtcctacct gtgaaaggcc taaggagaaa aaaaaacctt tgcggccgct 420

ccaacatcag aacctcgtgc cgaattctt 449

<210> 765

<211> 651

<212> DNA

<213> Rattus sp.

<400> 765
ctaggcaagc gctctaccac tgagctaaat ccccaacccc tggatatgtt ttaaaggagg 60

aaaggaccag tctttgtggc agcacaaatg cttagtgata atactatgtc agtctgtctg 120

cgatggatca gcccagggtg gaggctgctt ttgctttgga ggacagggct tgcctgaagc 180

gcctcttcag gtcctgcatg ttgggggatc ggggccgggg gatgatggat gctctcctcc 240

gctccccgct aaggctgtgc agcttgctca cttctttgca gagatgttga aacacatcgc 300

agacgtcttc gtagttttca ctagtagaaa tttcaaggaa gagactaccc agctcattgg 360

cgagctgaag accttcgtgt gtctgcacct gccgggcatg caaaaggtcc cctttgtttc 420

ccacgatgac gacgggggct tttccatcag ggtgagaatt tattttgtga atgtggcaca 480

aatcatgtaa atgtttttaa gtgtgaacag gaaaatgaac gtaggccatt acattaattt 540

ctctattgaa acaatgacag aaaacaattc tatttccaaa gctacagaag agcacacagt 600

gctacttgta agtggcgtct gatgaaggtc ttgcatatct ggtgtcagtt g 651

<210> 766

<211> 550

<212> DNA

<213> Rattus sp.

<400> 766
agggctcaga gaactgaccc cgaggcttta tctgctgatt tttattgctc atttgtttaa 60

gcactgaggg tggaacccag catccagcac agaaggaccc agtgagcgtg tgtgcgtgcg 120

cgtggtagtc tgcacagagc tcctcccttc cccagtttac tggggacgct ccccacacac 180

cggccaccaa ccctgccctg tttatcaaat cctgactttc ttggcccaag acgctttggt 240

gaaggggttt tctcttggca ggcgcagtgt gcttggtggt ggcttttctc aggatcttgt 300

tgggtttttg ggcgctggct gaggaagcct gcacgccact ttacgagttc ttctgtggca 360

ctttgacggt gaccgtcttc acttccgtgt aggcctgcag gccatactcg cccagctccc 420

tgccgctccc cgacatctta tagccaccaa atggggactg ggccccaaac acatcgtagc 480

agttgatcca cacagtccca gcctgcagag cttgggacag gtaattggcc ttgtccaggt 540

cctttgtgaa 550

<210> 767

<211> 465

<212> DNA

<213> Rattus sp.

<400> 767
ctcatttagt ttttattcat aatcataaac ttaactttgc aatcctgcta gacttgaagg 60

ggaatgagga aaatatggaa cccaaagaac ttcagtgaga gcagagatta cagggtattg 120

cgagcagatg gggtgggggt gctctcctga gctacagaag gaatggtttg atgggtaaaa 180

tgcccgcaag tcaaagaaat tagagttgtc cacagtcgga gatggtgatc ttcttgctgg 240

tcttggcagt gcagataaaa aactgggaac catttgtgtt tggtccagca tttgccatgg 300

acaagatgcc aggacctgta tgcttcagga tgaagttctc atcctagaaa tttctctccg 360

tagatggact tgccaccaat gccattatgg cgtgtgaagt caccaccctg acgcatgaat 420

cctggactag ttatgtgaaa ggagtaaccc ctatagccaa atcct 465

<210> 768

<211> 582

<212> DNA

<213>  Rattus sp.

<400>  768
gggtagcata aattttctca aactttaatc ttcacaatta ttttcactct atacacttta      60

ttgaaaaggt ctagatttat ttgacaaaat gattatgacc agaataaaga tatcttcttt     120

ttcatatatc agtaagtggc tggaatagtt aatttagtca tgtatcctgg aaaatgagtt     180

tcaaaatctt cctctttttt tttgaggtca gctactgtca ataatggaca ttaggcaata     240

gatcataaca cttcagtaac atgctgtgtc agaaccttgc ataattcaca cattcatttg     300

ctctctgcta cattatgact tcatggatta aagtttatta aattccaaat atttcttgca     360

ggagggaatg agtaaaacat caggataatg ctgtcttcat ttttaaatat atattgttgt     420

tttaattgat acatagtaat tgcacataat tatggcacag tatgacgttt caataatgta     480

tagtgtacat aataatcaaa tgaaggtaat tggcatgtca caccagatgt aactatttcc     540

tttctttctg ggacatggct attggacata gtcaattaat tg                        582

<210>  769

<211>  2462

<212>  DNA

<213>  Rattus sp.

<400>  769
tgtgctgttg gaaccatggc gcctgtctgc ctgtttgcgc cgctgctgct gttgctcctc      60

ggaggtttcc ccgtcgcccc aggcgagtcg attcgagaga ctgaggtcat agacccccag     120

gacctcctgg aaggcagata cttctctgga gccctcccgg acgatgaaga cgctgggggc     180

cttgagcagg actctgactt tgagctgtcg ggttccggag atctagatga cacggaggag     240

cccaggacct tccctgaggt gatttcaccc ttggtgccac tagataacca catccccgag     300

aatgcccagc ctggcatccg tgtcccctca gagcccaagg aactggaaga gaatgaggtc     360

attcccaaaa gggtcccctc cgacgtgggg gatgacgatg tgtccaacaa agtgtccatg     420

tccagcactt cccagggcag caacattttt gaaagaactg aggtcttggc agctctgatt     480

gtgggcggcg tagtgggcat cctcttcgcc gttttcctga tcctgctgct ggtgtaccgc     540

atgaagaaga aggatgaagg cagttacgac ttgggcaaga aacccatcta caaaaaagcc     600

cccaccaacg agttctacgc atgaagcttc ttcccatgag tgctgcttgg acttcatggg     660

gagaggagtt gaggattgtg gacagtggac attggcagag agagggcacc ttaatactga     720

cttgtatctc catctctggt cacctttctg gtgtcagaag agatatgatc ttctactgtg     780

ctgcctcaga gagagagaga gagagagaga gagagatggg atggggtgcg gagggagtgt     840

ccgtgtgtgt gtgtgtgtgt gtgtgtgtgt gtgtgtgtgt gtgtctgtct gtctgtctga     900

gttgccctgg cagaaaaatg gggttaaact tgttctttct tgaaggcaag cctggaattg     960

ggtctttttg ttgttgtttc aaatttctag aatagaatgt aggaccagtt tagttcctgc 1020

cgttaacatg tctcatttat gactgccttt attctagagg caaggagttg ggggcaagga 1080

gctggaaccc gctgcacctt gagatgtgtt cacccgagta cttcctcaca ctacagggtc 1140

tctgtggtgt atctcggggc attctaggct cagtgacttt tgaaattcaa cctttttttt 1200

ttttttttaa atccagggag ggtgggactg aagtgctgac agctcatgct gaagtacact 1260

tgtagaagat ttgtaaaatg taaggttttt tttttttttt tttaatggtc cattccttca 1320

tgggagcgtg tgccctgggc tgagagcgtg gggatgcaca gatgttcttt ctagaacata 1380

ttcgttgcaa cagctaactt tgtgttttca tggtttttta tgttttgttt tgtttttttg 1440

aaaatgagag aagagctgga gagatgattt ttatgatttt tttttgtttt gttttttact 1500

atttatagct tcagacgggg ctgcttttct ctacctttct gtctttactg tttcccacta 1560

tttttttttt ttaatgttct gtgctcttgt ttttgaccct ggccctttct gaagttgctt 1620

tatcttaaaa agtagctaca gtgttctagc agattccaga atataatgta gggggtagcg 1680

ggatatttgt gttcttgtaa tatatattat ccttcctcgg ttctaggaga atagataaat 1740

atattttttt aggatataga atgatactac aggtctcatg ttggctgggt ggctgcgtga 1800

gtgagttttc gtgcggctga gtaagctgtt gccctcttct cttgccctgc tcctggtgcc 1860

ttctcgagat cgagctggag tgactgaggg tacctgactc taacctcact gtgccttctg 1920

ccgggggctc tgcccaggag cctctgggtt tgctttctcc aggctctcta gatgcacgat 1980

ccaatacagt gacctcctgt ggctgtatca atcagttcac ttgactatgt gattggaaat 2040

cattcctctg ttgcactggc cacacaattt aagtgcctag tcaccatcca ccgagcacag 2100

agattgaggc tggtttagca ggttacggtt cagttttgct tgtctccccg ggcaagagaa 2160

ggggacttag gaggaaggtg atgaggtccc agggactcct gtcacccagg actctccctc 2220

ttacagagga aagacctagt agcttaaaag gtctgggctg tgtgggggtg gggcggtcat 2280

accactccct caaccatgcc ctatgcctgt aagcccccat caccacctcc gtgcagggtc 2340

ctagctggct gggtcctctt ctagccttgt gcctgctcct tttctgtatc ccttactctg 2400

ttgtctgtta ctgatttttt tgataaaaag ataataaaac ctggtacttt ctaaaaaaaa 2460

aa 2462

<210> 770

<211> 1052

<212> DNA

<213> Rattus sp.

<400> 770
gcagcgggga ccttattgga ctatctcccc ttaagtggga agggcttagt caaatgcagt 60

aaagagctat aaaacaccga gaactcttga tgtgttgtga aacttagagg gagcagcttt 120

ttaacaagag aactcaagca attgctgcca tgccggggaa gccagtcctt cactatttcg 180

atggcagggg gagaatggag cccatccggt ggctcctggc tgcagctgga gtagagtttg 240

aagaacaatt tctgaaaact cgggatgacc tggccaggct aaggaatgat gggagtttga 300

tgttccagca agtgcccatg gtggagattg atgggatgaa gctggtgcag accagagcca 360

ttctcaacta cattgccacc aaatacaacc tctatgggaa ggacatgaag gagagagccc 420

tcatcgacat gtatgcagaa ggagtggcgg atctggatga aatagttctc cattaccctt 480

acattccccc tggggagaaa gaggcaagtc ttgccaaaat caaggacaaa gcaaggaacc 540

gttactttcc tgcctttgaa aaggtgttga agagccatgg acaagattat ctcgttggca 600

ataggctgag cagggctgat gtttacctag ttcaagttct ctaccatgtg gaagagctgg 660

accccagcgc tttggccaac ttccctctgc tgaaggccct gagaaccaga gtcagcaacc 720

tccccacagt gaagaagttt cttcagcctg gcagccagag gaagccatta gaggatgaga 780

aatgtgtaga atctgcagtt aagatcttca gttaattcag gcatctatgg atacactgta 840

cccacaaagc cagccttcga aagctttgca acaatcgcat attttgacta aatgttgacc 900

ctacttattg ggaggccaac acgttttcta atgcttctgt gttaattcat atagacatga 960

ctgatgagga attgctggga tgctatttgg ttgtagttaa aatttgaaat catgatcact 1020

tcctcagata ttactttgaa tctcaataaa aa 1052

<210> 771

<211> 951

<212> DNA

<213> Rattus sp.

<400> 771
aagcttatga aggctctgtg ggccctgctg ttggtcccat tgctgacagg atgcctggcc 60

gagggagagc tggaggtgac agatcagctc ccagggcaaa gcgaccaacc ctgggagcag 120

gccctgaacc gcttctggga ttacctgcgc tgggtgcaga cgctttctga ccaggtccag 180

gaagagctgc agagctccca agtcacacag gaactgacgg tactgatgga ggacactatg 240

acggaagtaa aggcatacaa aaaggagctg gaggaacagc tgggcccagt ggcggaggag 300

acacgggcca ggctggctaa agaggtgcag gcgacacagg cccgtctggg agctgacatg 360

gaggatctac gcaaccgact cgggcagtac cgcaacgagg taaacaccat gctgggccag 420

agcacagatg agctgcggtc gcgcctctcc acacacctgc gcaagatgcg caagcgcctg 480

atgcgggatg cggatgatct gcagaagcgc ctggcggtgt acaaggccgg ggcacaggag 540

ggcgccgagc gcggtgtgag tgctatccgt gagcgcctgg ggccactggt ggagcagggt 600

cgtcagcgca cagccaacct aggcgctggc gccgcccagc ccctgcgcga tcgcgcccag 660

gctttgagtg accgcatccg agggcggctg gaggaagtgg gcaaccaggc ccgagaccgc 720

ctagaggagg tgcgtgagca gatggaggag gtgcgctcca agatggagga gcagacccag 780

cagatacgcc tgcaggccga gatcttccag gcccgcatca agggctggtt cgagccgcta 840

gtggaagaca tgcagcgcca gtgggcaaac ctaatggaga agatacaggc ctctgtggct 900

accaactcca ttgcctccac cacagtgccc tggagaaatc aatgaggatc c 951

<210> 772

<211> 1058

<212> DNA

<213> Rattus rattus (black rat)

<400> 772
ctctcgccag gcgtcctcat tagagtgaca tcgtctttaa accccgcgtg gcaatccctg 60

acgcaccgcc gtgatgccca gggaagacag ggcgacctgg aagtccaact acttccttaa 120

gatcatccaa cttttggatg actacccaaa atgcttcatt gtgggagcag acaatgtggg 180

ctccaagcag atgcagcaga tccgcatgtc cctccgcggg aaggctgtgg tgctgatggg 240

caagaacacc atgatgcgca aggccatccg gggccacctg gagaacaacc ccgctctgga 300

gaagctgctg cctcacatcc gggggaacgt gggctttgtg ttcaccaagg aggacctcac 360

cgagattagg gacatgctgc tggccaataa ggtgccagct gctgcccgag ccggtgccat 420

cgccccgtgt gaggtcacag tacctgctca gaacaccggt ctggggcccg agaagacctc 480

tttcttccaa gctttgggca tcaccactaa aatctccaga ggtaccattg aaatcctgag 540

cgatgtgcag ctgataaaga ctggagacaa ggtgggagcc agcgaagcca cactgctgaa 600

catgttgaac atctccccct tctccttcgg gctgatcatc cagcaggtgt ttgacaatgg 660

cagcatctac agcccagagg tgctggacat cacagagcag gccctgcaca ctcgcttcct 720

agagggtgtc cgcaatgtgg ccagcgtctg tctgcagatt ggctacccga ctgttgcctc 780

agtgcctcac tccatcatca atggatacaa aagggtcctg gctttgtctg tggagactga 840

ctacaccttc ccactggctg aaaaggtcaa ggccttcctg gccgatccat ctgcatttgc 900

ggctgcggcc cctttggctg ctgccaccac tctggctcct gcagctgctg ccccagccaa 960

ggtcgaagca aaggaagagt cggaggaatc cgatgaggac atgggattcg gtctcttcga 1020

ctaagcccct cacaccaagt cagcctgctt tatttgag 1058

<210> 773

<211> 435

<212> DNA

<213> Rattus norvegicus (Norway rat)

<400> 773
ggatcatagt ggtgggaggt ggtgggtact agtgatgccc cctatccgcg cgcgtctgcc 60

tccagggtgc ccctccagcc aggatgaggc tcttcatcgc acttcctgtc ctgatcgtgg 120

tcgtagccat ggctttggaa ggcccagccc ctgcccaggc ggcccctgac ttttccagcg 180

caatggagag cttaccggat aaactaaaag agtttgggaa cactttggaa gataaggccc 240

gggcagccat tgagcatatc aaacagaagg aaattatgat caagactcgg aactggtttt 300

cagagacgct caacaaaatg aaggagaaat taaaaaccac atttgcctga gtgcctggcg 360

agccacctcg cagcagcaaa gagggccacg tgacgggcca ggtccctctt caaataccaa 420

taaaaacttg tagag 435

<210> 774

<211> 1140

<212> DNA

<213> Rattus norvegicus (Norway rat)

<400> 774
ggatcctaac ttcttcccaa gcaccagtca ctggagacat aggagaggca agaagcctct 60

gggcaactga taaggattcc ccgcacccag gggaggcgat ccccaagccc agcctctcag 120

ggtcctgaca agccatgctg cggagagcaa tcacaggcca gccaatgaca gctgctccaa 180

gggcgtgtcc ttacctcctg cacatataaa tgaaacttgc tgcggggcca atacattctc 240

attctgatag actcaggaag caatcatggt gctctctgca gctgacaaaa ccaacatcaa 300

gaactgctgg gggaagattg gtggccatgg tggtgaatat ggcgaggagg ccctacagag 360

gtaacatcag gaccctgttc tttaaggaca gcaggatcca aaccggacca gggactcagt 420

gggtagctcc taagtgtgct ttcccgtggc ctcaacttat ctctccttct cacaggatgt 480

tcgctgcctt ccccaccacc aagacctact tctctcacat tgatgtaagc cccggctctg 540

cccaggtcaa ggctcacggc aagaaggttg ctgatgccct ggccaaagct gcagaccacg 600

tcgaagacct gcctggtgcc ctgtccactc tgagcgacct gcatgcccac aaactgcgtg 660

tggatcctgt caacttcaag gtgtgagctc agacctggca gggggcacct gggaccttca 720

aggatccctt ggggcagtgg tgaggggcac agggggaggg ggaaggggtt cctcatgccc 780

agggcaggga acacagtgtt ccaggaaggg gagcttaccc agcagggtgt ctactactag 840

ggactgaccc ttcctgtctc tgcagttcct gagccactgc ctgctggtga ccttggcttg 900

ccaccaccct ggggatttca cacctgccat gcacgcctct ctggacaaat tccttgcctc 960

tgtgagcacc gtgctgacct ccaagtaccg ttaagccacc tcctgtcggg cttgccttct 1020

gaccaggccc ttcttccgtc ccttgcacct gtctttgaat aaagcaaaag taggaagaat 1080

cccgtgtgcc tgtttctcac atgtgcaaag gtgacaatgt ttggtatggg aaaatcctcc 1140

<210> 775

<211> 922

<212> DNA

<213> Rattus rattus (black rat)

<400> 775
tctttttctt cgggaaaaca ccaaatggcg gatgacgccg gtgcagcggg agggcccgga 60

ggacccgggg gcccaggatt aggaggccgc ggcggcttcc gcggaggatt cggcagcggc 120

cttaggggcc gcggtcgtgg ccgaggccgt ggccgcggtc gaggccgcgg ggctcgtgga 180

ggtaaagctg aagacaagga gtggatcccc gttaccaagc tgggccgcct ggttaaggac 240

atgaagatca agtccttgga ggagatctac ctgttctccc tgcccattaa ggagtctgag 300

attattgact ttttcctggg tgcatctcta aaggatgaag ttctgaaaat catgccagtg 360

cagaagcaga ctcgggctgg ccagcggacc aggttcaagg cctttgtcgc tattggggac 420

tacaatggcc acgttggtct tggtgttaag tgctccaagg aggtagccac tgccatccga 480

ggggccatca tcctggccaa gctttcgatc gtcccagtgc ggagaggcta ctgggggaac 540

aagattggca agccccacac tgtgccatgc aaggtgacag gccgctgtgg ctctgtgctg 600

gttcgtctca tccctgcccc cagaggcact ggcattgtct ctgctcctgt gcccaagaag 660

ctactgatga tggccggtat agatgactgc tacacttcag ccaggggctg cactgccacc 720

ctgggcaact ttgccaaggc cacctttgat gccatctcca agacctacag ctacctgacc 780

cctgacctct ggaaagagac tgtgttcacc aagtctcctt atcaggaatt cactgaccat 840

cttgtgaaaa cccacaccag agtctctgtt cagaggaccc aggctccagc tgtggctacc 900

acataagggt ttttatacaa ga 922

<210> 776

<211> 2093

<212> DNA

<213> Rattus norvegicus (Norway rat)

<400> 776
ctctggtctc atctctgcca cgccactgct cagaggcaca gagtgagcaa cagtttggta 60

gcgctgagtc catccactgg aaattctcct cctgtctcag cggctttagc tctgcagaaa 120

tgaagcatcc agcctacact ctccttctct ctctcatcat gtctatgtgt gctgggagca 180

aaggccttgc tgagcagcta actaaggaaa acctgaccgt gtccctcctg cctcctaact 240

tccacaaaga gaacacagtc accaatgact ggattccaga aggagaggaa gatgacgact 300

acctggacct ggagaagctg ctcagtgaag atgacgacta catctatgtt gtcgatgcag 360

tttccccaac agactcagaa tcaagtgctg gcaacatcct gcagcttttc caaggcaaaa 420

gccggatcca gcgccttaac atcctcaatg caaagtttgc cttcaacctt taccgagtcc 480
tgaaggacca ggctacctct tctgataaca ttttcatagc acccgttggc atttccactg 540
ccatggggat gatctcctta ggtttgaggg gagagaccca tgaggaagta cactcagttc 600
tacacttcaa agactttgtc aatgctagca gcaagtacga ggtgacaacc attcacaacc 660
tctttcgaaa gctgacccat cgcctattca gaaggaattt tgggtacaca cttcagtcag 720
ttaatgacct ttatattcag aagcagtttc ccatacggga ggacttcaaa gctgccatga 780
gagagtttta ctttgctgag gcccaggagg ctgacttctc agatcctgcc ttcatatcaa 840
aggcaaacag ccacattctg aagctcacca agggcctcat aaaagaagct ttggagaata 900
cagactctgc tactcagatg atgattctga actgcatcta cttcaaagga gcttggatga 960
ataaattccc agtagaaatg acgcataacc acaacttccg gctgaatgag agagaagtag 1020
taaaagtctc catgatgcaa actaaaggga acttccttgc ggcaaatgac caggagctag 1080
actgtgacat tctgcagctg gagtatgtag ggggcatcag catgctaatt gtaattccac 1140
ggaagctgtc gggaatgaag actcttgaag cacagcttac accccaggta gtggagagat 1200
ggcaaaaaag catgacaaac agaacccgag aggtacttct gcccaagttt aagcttgaga 1260
agaattacaa cctggtggag gtcctcaagt ccatgggaat cacaaagctc ttcaacaaga 1320
atggcaatat gtcaggcatc tcagaccaga ggatcatcat tgatctgttc aagcaccaga 1380
gtaccatcac agtgaatgaa gagggtaccc aagctgcagc tgtaactaca gtggggttta 1440
tgccgctgtc tacccaagtc agattcactg ttgaccgtcc cttcctgttc ctagtgtatg 1500
agcaccgaac cagctgtctg ctgttcatgg gaagagtggc caaccctgcc aagtcttaat 1560
ggtgctggtc tgggcacctg atgtagtttg gggacactct gtaatcctgt tttcattcta 1620
agaggacaga gatattttga tattgataat ctatgtagtt tgagataata atctaacagt 1680
aaacagtgtc ataagtgcac agatgttaat gcaactaatg gtcgtgccag ctattcccta 1740
gaggtcctag acacagactg cctcagcgcc aacgcctctg tcactgagaa tgggcagagt 1800
ggagctgaca ctttctcctc agttagccgt accagtcaag gtgtctcctc agccaggccc 1860
ctccatatgg atggtactga ggaactctca cccttccagg gaggatgtcc aaggagcacc 1920
attcttgcct agtggaaaag aaacctcaac atgtctgaga catttggact taattcaagg 1980
ctgaaccttt taaattcatg taaatatcat ctgttctgct ttgtgtcaat agggctacca 2040
tagcctattc tagagacaaa taaacaacca tatttactaa gcaaaaaaaa aaa 2093

<210> 777

<211> 3551

<212> DNA

<213> Rattus norvegicus (Norway rat)

<400> 777
gtgtcctctg tctctgtctc tgtctctgtc tctgtctctg tctctgtctc tgtctctgtc 60
tctgtctctg tctctctctc tctctctctc tgtgtgtgtg tgtgtgtgtg tgtgtgtgtg 120
ccttagagtc tcttagactg gctggcctga gagtcacagg gaactaccta gctacacttc 180
tcagtactgg gatgagaggt gtgcattaga atgcctgcgt tttacacgag ctctggagac 240
taaactcaaa tcttcctact tgccaggcaa gcagttaatg tcattatact gcatttccat 300
tttgatgtgt tagctagtca tttctctttt aacatttgga gtattttata actttggagg 360
tagggctatt cttaccctgt gaactgcttt tcctctctct ctctctctct ctctctctct 420
ctctctctct ctctctctct ctctctctct caagtcaaga gattcccccc tccgcatcat 480
tttcccctag ttaataatta ccaagaaatt tgcaccaaga ccaaaaaaag cctatgtatt 540
tcagaaatag attaactagg ttacttttta acaaagctta ggtcaaagaa tactataaaa 600
cgttggtttt gccccagtcg cattactctc ttgaatgtgg cctctttttt aaatttaaac 660
atgagacact taaaacttac aggttttatt tttttcctgt tgtctttgac tgaatcccta 720
gccctaccca caaagcctca ggatgtaggt aagaaattta cttgtgtatt accagggaca 780
actaagtgaa ggaaattact ttcctttagc cttttaaaac tttgattatt ccggtggttt 840
tatttttctc tctcttctct tagtatatgt atactggtca gtagtttcaa aggacccact 900
acagattttc atattatcta ttctgtagga tgcttgcctt tagaaacggc caacaatctg 960
gatcatgttg acttgagatg taatttggac ttcaaaaata ttagaatttt gtgacagata 1020
tagatatata aatatatgta tagtccaaca gaagagcata gatacagtat atacatattc 1080
atacatatac atacatacac acatatgtac acactcacac acaaacacat gtataaacat 1140
ttatatacaa tctttaaaaa atatttttta aatatttttg gttgtccaat atgtcttcca 1200
gctagatgat aaataccgtc aggtatatga gatcctgctt tctccttcag ctggcgttac 1260
acacctgcaa catactacgc caagagcaag gcgtgtcaac cgggtggaaa aggcaatttt 1320
tacattttac caaatgagga aactttgaaa ttttctttga aagaaaagaa aattttcttt 1380
gaaaggtctt tttaaatacc tctggccaga aaggcacatt ttttttttac aggaatgtag 1440
tttccttaat atcatcccag agaacatata ctcaagtgat attatacaga ctgataaggt 1500
tgtatctatg tatcagagaa tgtaaaaatt tataatatat ataacatcaa tcaatcaatt 1560
aatgaaaaag aggccatgca tttgaaagag agcaaggagg ggttaacagg agaacaggca 1620
ggggatcatt tcaatgctac tcagaaattc ataaatgaga acgttgcata cctgaccatc 1680
attgcatctg cccagtatgt tcaggaagca tcctttgagg aggtggaaat gctggtgaag 1740
gtcatgctgg attacaagga taggtgcttg gctgacagca cacttccgga gtgttcaaag 1800
atagctaatg atgctatcca ggacatgctc tgtgatatga aggggctgcc acagaagcat 1860
aacttctccc attgctgtag gcaagccggc tttcaaagaa gactctgctt cttctacaac 1920
aagaaagcta atgtaggatt tctgccccct ttccctactc tggatccaga agagaaatgc 1980
caagcttata aaaataacag tgaatctttt ctcaacctct atatgtatga ggtggccaga 2040

aggaacccct ttgccttcgc cccagttctt ctgaacgttg ccgctaggtt tgaggaggcg 2100

gcgaccacgt gctgtgaaca gcaacagaaa gccacctact ttcaagacaa ggcagccccc 2160

atcacacagt atttaaaagc gttatcttct tatcaaagaa atgtttgtgg agccctttta 2220

aaatttggac ccaagacctt aaactctata aacatcgccg tgtttagtaa gaagttcccc 2280

aagattggat ttgaagacct tacgtccctc ttagaagatg tttcttccat gtatgacggg 2340

tgctgtgaag gggacgtcgt gcagtgcatc cggagccaga gccaggttat gcaccatatt 2400

tgttcaaaac aagactccat ctcaagtaag atcaaagcgt gctgtgagaa gaaactgcca 2460

gagcgcgcag actgcataat caacgcaaac aaggatgaca gaccagaaga cttatctctg 2520

agaacaccca aatttaccga cagtgaaaat gtgtgccaag agcgagattc cgagcaggac 2580

aagttctttg ctgagtttct ttatgattac tccagaagac atactgaact gtctacacca 2640

gagctcctaa gaattactaa agtgtataag gatctcctgg aagattgctg caacagaaaa 2700

aacccgttaa gctgttaccg ccatgcggag gataagttca acgagacaac agagcgaagc 2760

cttgcgatgg tccagcaaga gtgcaaacag ttccaggaat tggggaagga cgctctacaa 2820

cgccatttcc ttgtcaagtt caccaaggca gcccctcaac tccccatgga agaactggtc 2880

tcccttagta aggaaatggt ggcggctttg gcaacctgct gcactctcag tgacgagttc 2940

gcctgtgtgg acaacttggc agatttagtt cttggagaat tatgtggaat gaataagaat 3000

cgaactatca atcccactgt ggatcactgc tgcagagctg actttgcctt caggagaccc 3060

tgctttgagc acctgaaagc cgacaccacc tatgcactcc catcagtctc tgcacttgta 3120

tctgctttgc gtgcagactg gtgtcagcct ctcaaggagg accttcagaa taagagacac 3180

aggtttctgg tcaacttagt gaagtggatg ccagagatca cagatgagga acgtctgtgt 3240

ttgttcacaa agttcacggc tgctggggag gagtgtggca acatacagaa gcctgaggcc 3300

tgtttcagtc cagagagttc aaaaaccggt gatgtgagtc aagatgcaga gaaacaaagg 3360

tgaacggaat gaccagagcc agtaccagct cacaggatcg tcctgtggag aagttcagct 3420

cttaagaaga ctggaataga aggtccttct gtaacccttt cttgaatcat gttcctacaa 3480

aaggcaataa aaagggagga taaagtttct cgcggcgcga aaaaaaaaaa aaaaaaaaaa 3540

aaaaaaaaaa a 3551

<210> 778

<211> 2578

<212> DNA

<213> Rattus norvegicus (Norway rat)

<400> 778
gatctggtgg gatggggaca tctttgtgga gacacagggg ggcaggggtg ggaggaggta 60

tgggatgtgg aactgtctgg agggtggacc gggaggggag taaaatctgg agtataaaaa 120

taaataaatt aattaagaat actgttaatt gaacaagtcc tgaaaaaaaa aaaaagaaaa 180
agaaacggga tggagatgtc tctgtggtta agagtgcata ctgctcttgg agaggcctgg 240
agtttacttc ccactatgtg tcaggtggct tagcgcgcgc gcgcgcgcgc gcgcgctcgc 300
ggtgtgtgtg tgtgtgtgtg tgtgtgtgtg tgtgtgtgtg tgtgtgtgtg tgtgtgtgtg 360
cgtgtgtgtg ttgtatatga gtagaatgaa cctaagacct tgtgcatact ggacaagcta 420
tcactgcatt gtatccatat tccacagagt ttagcaccca ggtatggcat aataataata 480
aatcttattg taaattggag aaaggacaga tttaggttcc cttttacaga agcccaggag 540
gcacagcctt atttgaaaga aaaagcaact ggcataaagt ggtggattat gtcactttgt 600
gtgatggtag taatcactta attatgtaaa ccagagtatc ctattgcaca ccttaaatgt 660
aggcaataaa agtaaaactt taagcagtaa aaaatactgt agaggccacc gccatgccat 720
ccagactgag gaagacccgg aaattccggg gccacgtgag ccacagccac ggtcgcattg 780
gtaagcaccg caagcaccca ggaggccgcg ggaatgctgg aggcacgcaa ttaccacagg 840
atcaactttg acaaatatca tcctggttac tttggcaaag tcggcatgag tcattaccac 900
ttgaagagga gccagagctt ctgcccaact gtcaacctgg ataaattatg gacgttggtc 960
agccagcaga cacgtgtcaa tgcagcaaaa aacaagagtg gagctgctcc catcattgat 1020
gtttttccaa tcaggctact acaaagttct gaggaagggg aagcttccta agcaacctgt 1080
catcgtgaag gccaaattct tcagcagaag agctgaagag aagataaagg gtgttggagg 1140
tgcctgttct ggtggcttaa agtcacttca gaggttaatt aaatgcaaac attttccatg 1200
aaaaaaaaat attgtagaaa tgaattagca gttaagagca ctggctgctc ttccagagaa 1260
tctcacctgt ttgtaactct agttccacag cttctgagac cctcacacag acatacatgg 1320
aggcaaaaca gggttgaact ataagataaa aaaccagtgt ggtgtatatc ccaagtgatg 1380
tctgtgagtg gcattatgac cattctgtta tcccgcattc tctgctaaaa gggctttgta 1440
tgcaaaaggt acctttatta tatgagctaa ttacatgact taattggtga gtaatcatgg 1500
ctaatattac tatagcatgc taaaggaagt atcctaacaa ctgggaagat ttactgtgta 1560
aacattactt tatttacaag tatatggttt accttcactg ctaaaactca gtctaagaat 1620
gcaatgatct ttggataaca tgatttacta gattagccct gagtttagat tgtatgttga 1680
aactctctgg ggtatttgaa aaaaagaaaa aaaaagacag taaatgagtg atatgggagg 1740
gggtgcctta gttggctcat gctgaggcac acctgcccac atctccccct tctcccctcc 1800
cccgaggtac cagccatata tgggtctagt ataaaagaga gcttatttgg aggcatggga 1860
aagggggttg agaaggaagt tgaggcagag aaagagaagt acagagagag agagaggaaa 1920
gaaaggagaa gaggcaggcc aggaatatgt ggagaggatg agagaggact tgagaggaaa 1980
gagagagagg gtaagaggga ctgagggaga gggagagaag gagagaagga gagagggaga 2040
gagagggaga gagagagagg aaggttagag agcaaagaga ggctagtcat ttttatggca 2100
agccaggctc ttgtctggat gttgctatgt aacagttggg tagagccaag aaggaatgct 2160

aagaagctaa ctttgggtcc cacccctggt tagttacaca gatttgtggt agtagaaaga 2220

ggaagaacag ttttcacttg tgtattttaa caggtcaggg tccacaaaga agaaatatta 2280

aagcatatct agttgattgg tcacttaggt atcagaagct catgttgaat tggcagctag 2340

cattataaaa gtcctggaca gcaagctcac aggagtctcc ctgagaaggc tgccatggat 2400

ccagtcctag tcctggtgct cactctctcc tctctgcttc tcctctcact ctggagacag 2460

agctttggga gagggaagct ccctcctggt ccaacacctc tcccaatcat tggaaacacc 2520

cttcagatat atatgaagga catcggccaa tcaataaaaa aggtaagtgt tttttaag 2578

<210> 779

<211> 1018

<212> DNA

<213> Rattus norvegicus (Norway rat)

<400> 779
gctggaacgg gtccgtacgg cgttgttctt ggttcccatc gtaacttaaa agggaaactt 60

acacaatgtc cggaggcctt gacgtcctgc agatgaagga ggaggatgtc ctcaaattcc 120

ttgctgcagg aacccactta ggtggcacca accttgactt tcagatggag cagtacatct 180

acaaaaggaa aagtgacggt atctacatca tcaacctgaa gaggacttgg gagaagctgt 240

tgttagccgc tcgagctatt gttgccattg agaaccctgc tgatgtcagc gtcatctcct 300

ccaggaacac tggccagcga gctgtgctga agtttgccgc tgccacagga gccactccaa 360

ttgctggccg cttcacacct gggaccttca ctaaccagat ccaagcagcc ttcagggagc 420

cccggcttct agtggtgact gatccccggg ctgaccacca gcccctcaca gaggcctctt 480

acgtcaacct gcccaccatt gctctttgta acacagattc tcccctgcgc tatgtggaca 540

ttgccatccc atgcaacaac aagggagctc actcagtggg tctcatgtgg tggatgctgg 600

ccagggaagt actccgcatg cgaggaacca tctcccggga gcacccatgg gaggttatgc 660

ctgatcttta cttctacagg gacccagagg agattgagaa ggaggagcag gctgccgctg 720

agaaggctgt gaccaaggag gaattccagg gtgaatggac ggcaccagcg cctgagttca 780

ctgctgctca gcctgaggtg gccgactggt ctgagggtgt gcaggtgccc tctgtgccca 840

ttcagcagtt ccccacagaa gactggagtg cacagccggc cactgaggac tggtcagcag 900

ctcccacagc acaggctact gagtgggttg gagccaccac tgagtggtcc tgagctcctc 960

tgcaggtgcc tgagcgaagg gaaaaaaggt ggaaggaaaa taaagttcct gaaagctg 1018

<210> 780

<211> 1560

<212> DNA

<213>  Rattus norvegicus (Norway rat)

<400>  780
atgccttctg tgtatggatt cccagccttc acatcagcca cagagctgct cctggccgtc      60

accacattct gccttggatt ctgggtggtt agagtcacaa gaacctgggt tcccaaaggt     120

ctgaagagtc cacccggacc ctggggcttg cccttcatag ggcacgtgct gaccctgggg     180

aagaacccac acctgtcact gacaaaactg agtcagcagt atggggacgt gctgcagatc     240

cgtattggct ccacacccgt ggtggtgctg agcggcctga acaccatcaa gcaggccctg     300

gtgaaacagg gggatgactt caaaggccgg ccagacctct acagcttcac acttatcgct     360

aatggccaga gcatgacttt caacccagac tctggaccgc tgtgggctgc ccgccggcgc     420

ctggcccaga atgcgctgaa gagtttctcc atagcctcag acccaacact ggcatcctct     480

tgctacttgg aagagcacgt gagcaaagag gctgaatact taatcagcaa gttccagaag     540

ctgatggcag aggttggcca cttcgaccct ttcaagtatt tggtggtgtc agtggccaat     600

gtcatctgtg ccatatgctt tggcagacgt tatgaccacg atgaccaaga gctgctcagc     660

atagtcaatc taagcaatga gtttggggag gttactggtt ctggataccc agctgacttc     720

attcctatcc tccgttacct ccctaactct tccctggatg ccttcaagga cttgaataag     780

aagttctaca gtttcatgaa gaagctaatc aaagagcact acaggacatt tgagaagggc     840

cacatccggg acatcacaga cagcctcatt gagcattgtc aggacaggag gctggacgag     900

aatgccaatg tccagctctc agatgataag gtcattacga ttgtttttga cctctttgga     960

gctgggtttg acacaatcac aactgctatc tcttggagcc tcatgtacct ggtaaccaac    1020

cctaggatac agagaaagat ccaggaggag ttagacacag tgattggcag ggatcggcag    1080

ccccggcttt ctgacagacc tcagctgccc tatctggagg ccttcatcct ggagatctac    1140

cgatacacat cctttgtccc cttcaccatc ccccacagca caacgaggga cacctcactg    1200

aatgggttcc acattcccaa ggagtgctgc atcttcataa accagtggca ggtcaaccat    1260

gatgagaagc agtggaaaga cccctttgtg ttccgcccag agcggtttct taccaatgac    1320

aacacggcca tcgacaagac cctgagtgag aaggtgatgc tcttcggctt gggaaagcgc    1380

cggtgcattg gggagatccc ggccaagtgg gaagtcttcc tcttcttagc catcctcctg    1440

catcagctgg agttcactgt gccaccgggc gtgaaggtgg acctgacacc cagctatggg    1500

ctgaccatga agcccagaac ctgtgaacac gtccaggcct ggccacgctt ctccaagtga    1560

<210>  781

<211>  4689

<212>  DNA

<213>  Rattus norvegicus (Norway rat)

<400> 781
ctgaacaccc atccggtgag acaggagggc aggtttccaa gcgccgctcc gccacacttg 60
atctcagcgc tgggaaagtg aagcgagcaa ccgacagcca cgatgccggc ccacatgctc 120
caagagatct ccagttccta cacgaccacc actaccatca cagagcctcc ttccggaaac 180
ctgcagaatg gacgagagaa gatgaagaag gtgcccctct atctggaaga agacatccgt 240
cctgaaatga gagaagatat ccacgacccc agctaccagg atgaggaggg gcccccgccc 300
aagctggagt acgtctggag gaacatcatc ctcatggccc tgctgcacgt gggagccctg 360
tacgggatca cactgatccc ctcctccaag gtctacacct tgctctgggg gatattttac 420
taccttatca gtgccctggg catcacagcc ggggctcatc gcttgtggag ccacaggact 480
tacaaggctc ggctgcccct gaggatcttc ctcatcattg ccaacaccat ggcgttccag 540
aacgatgtat atgaatgggc ccgggatcac cgcgcccacc acaagttctc tgagacacac 600
gccgaccctc acaactcccg ccgtggcttt ttcttctctc acgtgggttg gctgcttgtg 660
cgcaaacacc cggctgtcaa agagaagggc ggaaagctgg acatgtctga cctgaaagct 720
gagaagctgg tgatgttcca gaggaggtac tacaagcctg gcctcctgct catgtgcttc 780
atcctgccca cactggtgcc ctggtactgc tggggcgaaa ctttcctgca cagcctgttc 840
gtcagcacct tcttgagata cactctggtg ctcaacgcca cctggctggt gaacagcgct 900
gcccacctct atgggtatcg cccctacgac aagaacattc aatctcggga gaacatcctg 960
gtttccctgg ggtccgtggg cgagggcttc cacaactacc atcacgcctt cccctacgac 1020
tactctgcca gtgagtaccg ctggcacatc aacttcacca cgttcttcat cgactgcatg 1080
gctgccctgg gcctggctta cgaccggaaa aaagtttcta aggccgctgt cttagccagg 1140
attaagagaa ctggagatgg gagccacaag agtagctgag ttctgggctt ctgagttcct 1200
gttccaaaag ttttctgggc agagatttaa tattctgttg attaactact ggataatgct 1260
atcggaatgt taatgatgca tttaaactat tccggtacag tattatgaga aagctttgat 1320
catgttttga ggtaatgcat attttaatta gccaggatta accatgccac aatacatata 1380
tatttctaag catacatgat aaatgcatat acaattgtgc acatcagctt taaataataa 1440
cgatgaattt gaacattcta tacagagagt attaaagcca aggaacatgc tgttttgatg 1500
ctagggcaag catggtgctc tttccctgtt tgcactgcat ccagcttcgt tccttcctct 1560
gtcatcacca tcttcaggca aatagctgac cagccactgg cctgtgtcca ccttccaaag 1620
cccaggcaac ctttctgctt tctgaaatac tgatccttcc tcctggatat gtctttcctt 1680
gttctgaact ttgagattgc tggctcagac ttaagataga acaaaaacct tcctggaagt 1740
tcccactggt gaacttcact ataggttttg ccagagggaa tagggaaatt atcttgattg 1800
tagcaagtag atcaccttga gagaagaatt agcacgcacg gtgtggtttg taagtaagga 1860
tgggagagag ctaggaaaag gtggccatat ctaatggcct acttaccaag cccccaggcc 1920
tctctgcata ggcatggagt ccttccagtc catattctga acaacagttt ttagtgagat 1980
ttgaataatt aaattatttt cagagtgaag ggaatgggaa atgcatggtc tgtgccaggg 2040

gcggagagtg gggaagggtt agcttttggt aactgaagat ttcttcaatg gaaagactct 2100
taatgttaaa tgtgcctaga actggtaact aaacagctga catttggagg gaatgtggtg 2160
aaaactatgt aaatataagc aaaatccgca catgaaaata tttcacttag aaaaagtgtt 2220
gctaaagaaa aaaattcgaa ggacaagagg tgttgcctgg gttgccagtt tctttcgtgg 2280
cagggcagga aatagtgagg ttgagaggca gcatctgtaa gtagatgcta agaggtacat 2340
ttctaaatga agcccttggg gaacatctgc cagggatcta gagagtgctg gtttcatcca 2400
ttgttttaat ttcctccttg gattgagtag aaacgtggct tcttgtggtg ttgaaccttc 2460
cccgacttct ttgctttgtt gccatctagc ctgcccagca ctgcctaggg agcgcctacc 2520
caccctgatt tcttctgcca gctcctaccc accctgattt cttctgccac ttctttcttt 2580
ggcattcatt tttctttccc tggacaggaa gctagttacc cgagtgtatc aggagcacct 2640
ggcttgcctt tgtccaggcc cattcctcct ggtatccttg ggtgtggaac tgctggacag 2700
cttcaggaag cagagccagg tgccactttt ctgtggctcc agatcctccc tacctccacc 2760
cctatgtgcc tctgccacag taatttcaga aaagcttggt acaccctcta gcaacatctt 2820
ggttcagaga gtctctctgg cttgtgagtt aacagctcag ccaaatgctg tgttgtctca 2880
gtgaggcaac cactgaacga gagggttggt tgttgggctg tctctgttgg agtcagagta 2940
gacaacatgt tcacttcttc ctgtctgctt tgcaaggtgt ctgtgactga tcaaggcagg 3000
cagggagtgg tatagtactt ctgaaaggaa gaagttccct atttatctgt taaaagacca 3060
gaggcatggg caagtgctca ggacctcact tcgggaagag ggtctgcttc ctgaaagcca 3120
gtgtataagg aaaagcttcg actgaacctg gtatctaagg taccatgaac taacactcta 3180
tcacacagcc cagggcaaac agtggtatag aaaagtctag aagaaaacat cacatcagta 3240
ttttagaacc atccaccttt actgtccctg tagcccaatc cagagcctgg cttttagatc 3300
tggctgcgta cagtgccaaa tgctcagttt acttgtaaga tcagagcctt tctttctgtg 3360
ttaattgaac acatgctctg agctgttttg ttgaagtaga aaatctagta gaaaatcact 3420
gtagatctga tgacctggaa acctctggaa atgccttcgg gtggttttat ttttccaggt 3480
cacagatgcc tgatttataa gatgaacaat gaaatcagct ttctttctct tctgatctta 3540
ttccctggat ttgatgcagc ccatgttcca aagcaaagct acatagagac ttcaggctga 3600
ggtcctggtg tctttaagca aggacactct ttcagatcct ctcagaggcg tatttgtaac 3660
agtttccaca tgaatgtact aatcatgttg ggtgccttat cgctttccta atctacagtg 3720
cctgttagcc cagcctcact ccttccctta accctgagat cccgtagatg gagcgggtgt 3780
agacaagagg ctggctggag gatcatcagt acagtgattt gcagaaatct tttatgggct 3840
tcattttgga aaaatttgtt tttagtaaat gctcaaattt ggtgggaagt aatttgaatg 3900
tatttgattg tattgtcctc attacaattg ttaattagac acgttctaca tgacagtagt 3960
actcagggga gttttctccc tggctgctaa atatctgggt gtaatctctg aaactgtagg 4020
tccagtgagg cggcgtgaaa agttagcaaa gccaccatct gctggtggtt taagccaagg 4080

cctctcttct tcgccactga cttgctatgt tatcctttct cttgtaacaa acccatccca 4140

gagatgaggc ctttaatcat cccaagaacc tcctgggcta agtatctgac agtctcacat 4200

ctcaacatcg tgtattaagt gtccagaaca tcagctcagg aggacacgct gaaactctca 4260

ctctgggcga gtcctgataa aacaggctcc taatactgac ctacctcaag ggcagttctg 4320

aggtgattag agccttttct ttttttaagc aagtgtttgg agatgcatgg cagaggtttt 4380

cctgcagact tgtaaagagc tagatgtgtt ctaagaccat ctttagtcta cattgctctt 4440

cccagagatc tacaactatg acccagccaa agtgcaagcc tacctactca ctgccataaa 4500

aactattcag gaaacctttt cctggcctga atgagatttt gttttttttt tttttatgtg 4560

gggcggttat ttgtgaccca agtgtaattt ggatgatttt taattaatat caactcttga 4620

agcctaatat gtactggatt tgagattgca tttgttccta ataaaagtgg atctagctgt 4680

gctgcctgg 4689

<210> 782

<211> 2424

<212> DNA

<213> Rattus norvegicus (Norway rat)

<400> 782
tccccaaact cgggagacca gggtgaagcg aaagggggaa cctcttggaa tgcaacgaaa 60

ctttgaggcg gggatgtgag ttcaggagcc caaaattcct cactcttcct accctgctcc 120

aggttcaggg cactagcaac acagaggagc ccaggagtgg actacatgag actaactaaa 180

agaacacata taccgcagga tggggcaact gccttacccc ctggggaccc ttggagtctg 240

cagctactcc ccgctgcccc cacctgaacc cttgatccca gctctgcagc ccccgcagct 300

tcctgtttgc ccactctgtt tgcctagcct cgggaacaga gctgatcctt gaactctaag 360

ttccacatcg ccagcaaaag taagcagtga cagggccagc ctgagcttat cagtctccca 420

gcccaggccc tgcccacaca catatatagg tcagggaaga agacctggac acccagactg 480

ttggagagct ccgggggagg taagtgctgc tacctaccct aggctacttc ttagttccct 540

catgcctccc agtggatgcc ccactgtcca aagcaatacc atggatatct gggatctgtc 600

cccactccag atagttcttc tactgggttg aatgtgtgtg gcacctctcc atggcagcac 660

ctcccttcag tgcagccatt cttcttcttc ttctccaggt cgcccacatc cttcaggatg 720

aaagctgcag tgttggctgt ggccctggtc ttcctgacag gtgggtgcct cttggtctcc 780

atggactaac tccctccctg ggcacagaga gaacagaatt cccactgctc tcctccaagg 840

ctccaagtct gacatgtcag ctcaggcccc agcccagagt tgacctaaca tgggtctcct 900

ctccatcctc aggttgccaa gcttgggagt tctggcagca agatgagccc cagtcccaat 960

gggacagggt gaaggatttc gccactgtgt atgtggatgc agtcaaggac agcggcagag 1020

actatgtgtc ccagtttgaa tcctccactt tgggcaaaca gctgaagtaa ggaaggacct 1080

agtgtgggtc ctggagcagg gcaagggctg cccatccagg gtgggcagag gaaccctgta 1140

agaatgatcc tgtaactgag ctggctagac cttcacctgc tttcctacca gctgggcacc 1200

atggaggatt ccaatgggag gactagagat gggattcatc tggttgtagg gtaaccagcc 1260

tgtggattca aaatcccttt tccttggtaa cccccagtac tggtcagaca gcacaaaaaa 1320

aaaaaaaaaa aaaagacaaa aaaccaaaac caacaacaac aacaaaaatg ggactggcct 1380

tgcaactggc accaaccaca tctgtgatct tggtgcctca ggccatgtgt acagagggat 1440

gggaagggag gaagggagtt atagggagtt atagagttca gggaaactag gagcatagca 1500

actactattg gaacaggtgg cacctaggcc tgaccccagg tatcatgtgc atggatctgc 1560

agaccagggg agtgcaggat gcctggggtc atctctcagc tgctctcttc ccctctagcc 1620

tgaatctcct ggacaactgg gacactctgg gttcaactgt tggtcgccta caggaacagc 1680

taggcccagt gactcaggag ttctgggcta acctggagaa agaaacagat tggctgagaa 1740

acgagatgaa caaggacctg gagaatgtga aacagaagat gcagccccac ctggatgaat 1800

tccaggagaa gtggaacgag gaggtcgagg cctaccgcca gaagctggag cctctgggca 1860

ccgagcttca caaaaacgcg aaggagatgc aaaggcatct aaaggttgtg gccgaggagt 1920

ttcgagaccg catgcgtgtg aatgcagacg cgctgcgcgc aaagtttggg ctctacagcg 1980

atcagatgcg cgagaacctg gcccagcgcc tgaccgagat caagaaccac cctaccctga 2040

tcgagtatca taccaaggcc agcgaccacc tgaagacact tggtgagaaa gccaaacccg 2100

cgctggatga cctgggccag ggcctgatgc cggtgctgga agcctggaag gccaaaatca 2160

tgagtatgat cgatgaggcc aaaaagaagc tgaacgctta gtgaggcgcc cgtcaccact 2220

ccccacccct gaattggctt tcttacaata aacgtttcca aagtggaatg acttctttct 2280

ttgggtgaca tagggaggac gctaagggga catcaaggga tatggaaaga tggtgctgcg 2340

gtgagggttc ctttgtacgg ggctactcag ctcttcaagc tcactcaagc tgggcacctg 2400

gctggtttag agtatgagac acaa 2424

<210> 783

<211> 924

<212> DNA

<213> Rattus norvegicus (Norway rat)

<400> 783
cctggtggtt ccgcagccgt actctcctgg cctggtgtgc acagcctcac catgagttct 60

tctgcagcca gcccgctttt cgcgcctggc gaggactgcg gccccgcgtg gcgcgcggcc 120

cccgcggcct atgatacgtc tgacacgcac ctgcagatcc tgggcaagcc agtaatggag 180

cgttgggaga ccccctacat gcattcgctg gcggctgctg ctgcctccag agggggccgg 240

gtcctggaag tgggctttgg gatggccatt gcagcctcca gggtgcagca ggcccccata 300

aaggaacact ggattattga atgcaacgat ggggtcttcc agcgtctaca aaactgggcc 360

ctgaagcagc cacataaggt tgttcccttg aaaggcctgt gggaggagga ggcacctaca 420

ctgcctgatg gtcactttga tgggattcta tacgacacat atccactgtc tgaagagacc 480

tggcacactc accagttcaa ctttattaag actcatgctt tccgtttgct gaagcctggg 540

ggtatcctca cttactgcaa cctcacgtcc tggggggaac tcatgaagtc caagtacaca 600

gacatcactg ccatgtttga ggagactcag gtgcctgcac tgctggaagc tggcttccag 660

agagaaaaca tctgtacaga ggtgatggcg ctggtgcccc cagccgactg ccgctactat 720

gccttccctc agatgatcac acccctggtc accaagcact gagcggctgg cccagggcta 780

caaggagaat atgtcctcct cagtgccttt gtagctggag tgtggctcca gcctctccac 840

tatccctgca gtgtgacatc ctaacctctg cctggtacgg ccatctcccc agagctcagg 900

agtaaaataa atgctaccaa gact 924

<210> 784

<211> 1227

<212> DNA

<213> Rattus norvegicus (Norway rat)

<400> 784
ggaattcgca acgcctacac aaagatccct atcactgagc acccggaggc aaggctcaga 60

accccaggat cagcaacatg gagttctccc gtccaccgct agtgcatgtg aagggtatcc 120

cactcatcaa atactttgca gagacaattg ggccattgca gaacttcaca gcctggcctg 180

atgacttgct gatcagcaca tacccaaagt ctggtactac ctggatgagt gagatcctgg 240

atatgatcta tcagggtggc aagctagaga agtgtggccg cgcccccatc tatgcccggg 300

tacccttcct tgagttcaaa tgtccagggg ttccctcagg tcttgaaact ttggaagaga 360

caccagcccc acggctcctt aagacacatc tgcccctgtc cttgctccct cagagtctgc 420

tggatcagaa ggtcaaggtg atctacattg cccgaaatgc aaaggatgtg gttgtctcct 480

attataactt ctacaacatg gccaagctgc accctgatcc aggcacctgg gacagcttct 540

tggagaactt catggatggg gaagtgtcct atgggtcgtg gtaccagcac gtgaaggagt 600

ggtgggagct gagacacact caccctgttc tctatctctt ctatgaagac ataaaggaga 660

accccaaaag ggagatcaag aagattctag agtttttggg gcgctctcta cccgaggaga 720

ctgtggattc cattgttcac cacacatctt tcaagaaaat gaaagagaac tgcatgacta 780

actacacaac catccccact gagattatgg accacaatgt ttctcccttc atgaggaaag 840

gtactactgg ggactggaaa aataccttca ctgtagccca gaatgagcgc tttgatgccc 900

actatgctaa gacaatgaca gattgtgact tcaagtttcg ttgtgaacta tgagtggatt 960

atggctatac tgggaaccaa ggcaaactga cacagcccat catgatctca agtaaaatgt 1020

gatgtgttca atctacttgt tgtatgccta gaggaaatct gagctaagag aataggattg 1080

gggatgtggc tgaggcagag ggttttatca acgcatgtca ggaaagcaat cagtcccaac 1140

acctaaaaag aacctaaagt acaaacatgc aaaaaatagt aagataaact atattttacc 1200

tgaaagaata aatgccactg ggaaatg 1227

<210> 785

<211> 8048

<212> DNA

<213> Rattus norvegicus (Norway rat)

<220>

<221> misc_feature

<222> (2005)..(2005)

<223> n

<220>

<221> misc_feature

<222> (3503)..(3503)

<223> n

<400> 785
gaattcggcc caggataaac agatatatag gtaaatgact ggatagatac atggatttga 60

tctctaactc aacttaggca ccatagaaaa caatccttga attattttaa taattttaaa 120

tcctagttta ctgagaaaca cgttgacgag gcagggcttc agcctctctg aagcagcact 180

gcccctctgt ggccacacag gaagtcttca ctcagcagtt tcctgactta aggcctaggc 240

ctgaggaaat gcattgaata tttctctggg ggacaatagt tctgagaagg aaagagagtt 300

tgtctatgtt gaggtcagtt aaggatggtt ttctttcctt ccctcccagc cttttctcca 360

tgcaacatca tgagtcccca tctttctatc ctgcctaaaa ccacttcctt ctgggaaatg 420

agaaaatcca ctcatacaaa atggaaacat cccgaggatc ccggcccgca gcagctctct 480

gcccccagac cctgtgagag agagacccaa ccgcctggtc aggtgggcac tcctgaggct 540

gcagagggaa gagaccacca acactgctca cccctgccca catccctggc ccaagaggaa 600

actgtataag gcctctgggt cctgtggggg agggcccagg agcggcagga cccctgtgcc 660

tgagagacca ccaccagaac cagaaggaaa cagaccggat aaacagttct ctgcaccaaa 720

tcccgtggag ggagagctga accttcagag agaaagacaa gcctgggaaa ccagaagaga 780

ctgctctctg cacacacatc tcggacgcca gaggaaaaag ccaaagacca tctggaaccc 840

tggtgcactg aagctcccgg aaatggtgca caggtcttcc tggttgctgc cgctgcagag 900

agcccgtggt agcaccccac gagcgaactt gagcctcagg accacaggta agaccaactt 960

ttctgctgca agaaagctgc ctggtgaact caagacacag gcccacagga acagctgaag 1020

acctgtagag aggaaaaacc acacgccgga aagcagaaca ctctgtcccc ataactgact 1080

gaaagagagg aaaacaggtc tacagcactc ctgacacaca ggcttatagg acagtctagc 1140

cactgtcaga aatagcagaa caaagtaaca ctagagataa tctgatggcg aaaggcaagc 1200

gcaggaaccc aagcaacaga aaccaagact acatggcacc atcggagccc aattctccca 1260

tcaaaacaaa catggaatat ccaaacacac cagaaaagca agatctagtt ccaaaatcat 1320

ttttgatcat gatgctggag gacttcaaga aagacgtgaa gaactcctta gagaacaagt 1380

agaagcctac agagaggaat cgcaaaaatg cctgaaagaa tcgcaaaaat ccctgaaaga 1440

attccaagaa aacataaata aacaagtaga agcccataga gaggagacac aaaaatccct 1500

gaaagaattc caggaaaaca taaataaaca agtagaagcc catagagagg agacacaaaa 1560

atccctgaaa gaattccagg aaaacataaa taaacaagta gaagcccata gagaggagac 1620

acaaaaatcc ctgaaaggaa ttccaggaaa acacaatcaa acagttgaag gaattaaaaa 1680

tggaaataga agcaatcaaa aaagaacaca tggaaacaac cctggatata gaaaaccaaa 1740

agaagagaca aggagctgta gataaaagct tcaccaacag aatacaagag atggaagaga 1800

gaatctcagg agcagaagat tccatagaaa tcattgactc aactgtcaaa gataatgtaa 1860

agcggaaaaa gctactggtc caaaacatac aggaaatcca ggactcaatg agaagatcaa 1920

acctaaggat aataggtata gaagagagtg aagactccca gctcaaagga ccagtaaata 1980

tcttcaacaa aaccatagaa gaaancttcc ctaacctaaa aaaagagata cccatagaca 2040

cacaagaagc ctacagaact ccaaatagat tggaccagaa aagaaacacc tcccgtcaca 2100

taattgtcaa aacaccaaac gcacaaaata aagaaagaat attaaaaaca gtaagggaaa 2160

aaggtcaagt aacatataaa gggagaccta tcagaatcac accagacttc tcgccagaaa 2220

ctatgaaggc cagaagatcc tggactgatg ttatacagac cctaagagaa cacaaatgcc 2280

agcccaggtt actgtatcca gcaaaactct caattaacat tgatggagaa accaagacat 2340

tccatgacaa aaccaaattt acacaatatc tttccacaaa tccagcacta caaaggataa 2400

taaatggtaa agcccaacat aaggaggcaa gctataccct agaagaagca agaaactaat 2460

cgtcttggca acaaaacaaa gagaatgaaa gcacacaaac ataacctcac atccaaatat 2520

gaatataacg ggaagcaata atcactattc cttaatatct ctcaacatca atggcctcaa 2580

ctccccaata aaaagtcata gattaacaaa ctggatacac aacgaggacc ctgcattctg 2640

ctgcctacag gaaacacacc tcagagacaa agacagacac tacctcagag tgaaaggctg 2700

gaaaacaaat ttccaagcaa atggtcagaa gaagcaagct ggagtagcca ttctaatatc 2760

aaataaaatc aatttccaac taaaagtcat caaaaaagat aaggaaggac acttcatatt 2820

catcaaagga aaaatccacc aagatgaact ctcaatccta aatatctatg ccccaaatac 2880

aagggcacct acatacgtaa aagaaacctt actaaagctc aaagcacaca ttgcacctca 2940

cacaataata gtgggagatt tcaacacacc actctcatca atggacagat catggaaaca 3000

gaaattaaac agtgatgtcg acagcataag agaagtcatg agccaaatgg acttaacgga 3060

tatttttaga acattctatc ctaaagcaaa aggatatacc ttcttctcag ctcctcatgg 3120

cactttctcc aaaattgacc atataattgg tcaaaaaacg ggcctcaaca ggtacagaaa 3180

gatagaaata atcccatgcg tgctatcgga ccaccacggc ctaaaactgg tcttcaataa 3240

caataaggga agaatgccca catatacctg gaaattgaac aatgctctac tcaatgataa 3300

cctggtcaag gaagaaataa agaaagaaat taaaaacttt ttagaattta atgaaaatga 3360

aggtacaaca tacccaaact tatgggacac aatgaaagct gtgctaagag gaaaactcat 3420

agcgctgagt gcctgcaaaa agaaacagga aagagcatat gtcagcagct tgacagcaca 3480

cctaaaagct ctagaacaaa aangaagcaa atacactgag gaggagtaga aggcaggaaa 3540

taatcaaact cagagctgaa atcaaccaag tagaaacaaa aaggaccata gaaagaatca 3600

acagaaccaa aagctggttc tttgagaaaa tcaacaagat agataaaccc ttagccagac 3660

taacgagagg acacagagag tgtgtccaaa ttaacaaaat cagaaatgaa aagggagaca 3720

taactacaga ttcagaggaa attcaaaaaa tcatcagatc ttactataaa aacctatatt 3780

caataaaact tgaaaatctt caggaaatgg acaatttcct agacagatac cacgtatcga 3840

agttaaatca ggaacagata aaccagttaa acaaccccat aactcctaag gaaatagaag 3900

cagtcattaa aggtctccca accaaaaaga gtccaggtcc agacgggttt agtgcagaat 3960

tctatcaaac cttcatagaa gacctcatac caatattatc caaactattc cacaaaattg 4020

aaacagatgg agcactaccg aattccttct acgaagccac aattactctt atacctaaac 4080

cacacaaaga cacaacaaag aaagagaact tcagaccaat ttcccttatg aatatcgatg 4140

caaaaatact caataaaatt ctggcaaacg aattcaagag cacatcaaaa caatcatcca 4200

ccatgatcaa gtaggcttca tcccaggcat gcagggatgg tttaatatac ggaaaaccat 4260

caacgtgatc cattatataa acaaactgaa agaacagaac cacatgatca tttcattaga 4320

tgctgagaaa gcatttgaca aaattcaaca cccttcatga taagagtccc ggaaagaata 4380

ggaattcaag gcccatacct aaacatagta aaagccatat acagcaaacc agttgctaac 4440

attaaactaa atggagagaa acttgaagca atcccactaa aatcagggac tagacaaggc 4500

tgcccactct ctccctactt attcaatata gttcttgaag ttctagccag accaatcaga 4560

caacaaaagg agatcaaggg gatacagatc ggaaaagaag aagtcaaaat atcactattt 4620

gcagatgaca tgatagtata tttaagtgat cccaaaagtt ccaccagaga acgactaaag 4680

ctgataaaca acttcagcaa agtggctggg tataaaatta actcaaataa atcagttgcc 4740

ttcctctata caaaagagaa acaagccgag aaagaaatta gggaaacgac acccttcata 4800

atagacccaa ataatataaa gtacctcggt gtgactttaa ccaagcaagt aaaagatctg 4860

tacaataaga acttcaagac actgaggaaa gaaattgaag aagacctcag aagatggaaa 4920

gatctcccat gctcatggat tggcaggatt aatatagtaa aaatggccat tttacccaaa 4980

gcaatctaca gattcaatgc aatccccatc aaaataccaa tccaattctt caaagagtta 5040

gacagaacaa tttgcaaatt catctggaat aacaaaaaac ccaggatagc taaagctatc 5100

ctcaacaata aaaggacttc agggggaatc actatccctg aactcaagca gtattacaga 5160

gcaatagtga taaaaactgc atggtgattg gtacagagac agacagatag accaatggaa 5220

tagaactgaa gacccagaaa tgaacccaca cacctatggt cacttgattt ttgacaaagg 5280

agccaaaacc atccaatgga aaaaagatag cattttcagc aaatggtgct ggttcaactg 5340

gagggcaaca tgtagaagaa tgcagatcga tccatcctta tcaccctgta caaagcttaa 5400

gtccaagtgg atcaaggacc tccacatcaa accagacaca ctcaaactaa tagaagaaaa 5460

actagggaag catctggaac acatgagcac tggaaaaaat ttcctgaaca aaacaccaat 5520

ggcttatgct ctaagatcaa gaatcgacaa atgggatctc ataaaactgc aaagcttctg 5580

taaggcaaag gacactgtgg ttaggacaaa acggcaacca acagattggg aaaagatctt 5640

taccaatcct acaacagata gaggccttat atccaaaata tacaaagaac tcaagaagtt 5700

agaccgcagg aaacaaataa ccctattaaa aatggggttc agagctaaac aaaaattcac 5760

agctgaggaa tgccgaatgg ctgagaaaca cctaaagaaa tgttcaacat ctttagtcat 5820

aagggaaatg caaatcaaaa caaccctgag atttcacctc acagtagtga gaatggctaa 5880

gatcaaaaac tcaggtgaca gcagatgctg gcgaggatgt ggagaaagag gaacactcct 5940

ccattgttgg tgggattgca gactggtaaa accattctgg aaatcagtct ggaggttcct 6000

cagaaaattg gacattgaac tgcctgagga tccagctata cctctcttgg gcatataccc 6060

aaaagatgcc tcaacatata aaagagacac gtgctccact atgttcattg ccgccatatt 6120

tataatagcc agaaactgga aagaacccag atgcccttca acagaggaat ggatacagaa 6180

aatgtggtac atctacacaa tggaatatta ctcagctatc aaaaacaacg agtttatgaa 6240

attcgtaggc aaatggttgg aactggaaaa tatcatcctg agtgagctaa cccactcaca 6300

gaaagacata catggtatgc actcattgat aagtggctat tagcccaaat gcttgaatta 6360

ccctagatcc ctagaacaaa cgaaactcaa gacggatgat caaaatgtga atgcttcact 6420

ccttctttaa atgaggaaaa agaataccct tggctgggaa gggagaggca aagattaaaa 6480

cagagactga aggaacaccc attcagagcc tgccccacag gtggcccata catatacagc 6540

cacccaattg gacaagatgg atgaagcaaa gaagtgcaga ctgacaggag ccggatgtag 6600

atcgctcctg agagacacag ccagaataca gcaaatacag aggcgaatgc cagcagcaaa 6660

ccactgaact gagaataggt cccccgttga aggaatcaga gaaagaactg gaagagcttg 6720

aaggggctca agaccccaaa agtacaacaa tgtcaagcaa ccagagcttc cagggactaa 6780

gccactacct aaatactata catggactga ccctggactc tgaccccata ggtagcaatg 6840

aatatcctag taagagcacc agtggaaggg gaagccctgg gtcctgctaa gactggaccc 6900

ccagtgaact agactatggg gggagggcgg caatgggggg agggttggga gggggacacc 6960

cataaggaag gggagggggg agggggatgt ttacccggaa accgaaaggg aataacactt 7020

aaattatata agaaatactc aagttaatta aaaataataa taataataat aaaaagaatt 7080

actcataaaa taaagacaag atgatggata aaattaaaaa aacaaaaaaa acaaaaaaaa 7140

accaaaatgg aaacatgaaa atgctcacta cttacaatag tgctttatgt aataagcaaa 7200

tgactgcatt tgacacgagt tataatttat taacccctta gttagactac ttgtacacat 7260

ggtgattatt aatccacaat tattcttttt cactaaaatc cttatatttt attctttgag 7320

attataatat aatcacacaa attccctctt ccctgtcatc tcttcaaaac cttccatata 7380

gtcctccatg ctgtcttttg aacgttatgg cttctttttt atattcttgt tttatattat 7440

atatatataa tatatattat aatatatatc atactatata tattcctcag gactgaccaa 7500

tccatattgg ataaccaatt ggtgtgctcg tctcagggaa agaccatttc cccaccatca 7560

gcattctcgg tttccagtag ttcattgtct agaatggaga ctgtgtgtgc tttccccgtc 7620

cactttagct gctgtgtttc agctcctctt gaggcagtta tgttggtgag acctcatctg 7680

tgcggcttcc aacattcctc agagacacag tgagttacac agtgtcacct gatcctctaa 7740

ctcccaccat ctttcctgct gtcttctcca gggtttcctg aacctcagca ggagcagggg 7800

ttgtactgta gacacgccca ccttcactag gatccacagc tcctcacttt gatttgctgt 7860

ggttttctgc aatggcttcc atgtcctagt aaagtcatgg agaccccata accacgctgt 7920

actaaaccag acaatcccta gctgcattct aagttcttgt ccttatccca tacatgtgca 7980

tttagagagt ctgcttattt tgctcattat agaacgagtc aatcattctc tttgagttgt 8040

aaaagctt 8048

<210> 786

<211> 1977

<212> DNA

<213> Rattus norvegicus (Norway rat)

<400> 786
gtcctcctct gggacaggga aggatgtcac agctgagcct ctcctggctg ggactggggc 60

ctgaggtggc cttcccatgg cagaccctgc tactgtttgg agcctcctgg atcctggccc 120

aaattctgac ccagatctac gctgcctata ggaactttcg tcgcctccgt ggcttccctc 180

agccccccaa acggaactgg cttatgggtc atgtgggcat ggtcactcct acagagcagg 240

gcttgaagga attgactcgg ttggtgggta cctaccccca aggctttttg atgtggattg 300

ggccaatggt ccccgttatt actctgtgcc actctgacat cgtccgatct atcctcaatg 360

cctcagctgc cgttgcgctc aaggatgtga tattctacac catcctcaag ccttggctgg 420

gggatgggct cctggtgagt gctggtgaca agtggagccg ccaccgccgc atgctgacac 480

ctgccttcca cttcaacatc ctgaaaccct atgtgaagat tttcaacgac agcaccaaca 540

tcatgcacgc caagtggaaa cggttgattt cagaaggcag ttcccgtctg gacatgtttg 600

agcacgtcag cctcatgact ctggacagtc tgcagaaatg tgtcttcagc tttgacagca 660

actgccagga gaagtccagt gaatatattg ctgccatcct ggagctcagt gccctagtgg 720

ccaaaaggca ccagcagcct ttgctgttca tggacctgct gtacaatctc acccctgatg 780

ggatgcgctt ccataaggcc tgcaacctgg tgcatgaatt cactgatgcc gtcatccggg 840

agcggcgccg caccctccca gatcagggtt tggatgagtt tctcaagtct aaggccaagt 900

ccaagacttt ggatttcatt gatgtgctgc tcctgaccaa ggatgaagat ggaaaggagc 960

tgtcggatga ggacatcaga gcagaggccg acaccttcat gtttgagggc catgacacca 1020

cagccagtgg gctctcctgg attctataca acctggcgaa cgacccagag taccaggagc 1080

gctgccggca ggaagttcag gagcttctga gggaccgaga tcctgaggag attgaatggg 1140

acgacctggc ccagctgccc ttcctgacca tgtgcatcaa ggagagtctg cggctgcacc 1200

ccccagtcac ggtcatctcc cgatgctgca cccaggacat tctgctccca gatggcagga 1260

ccattcccaa aggtatcatc tgccttatca gtatctttgg aattcatcac aacccgtctg 1320

tgtggccgga ccctgaggtc tacaacccct tccgctttga ccctgaaaac atcaaagaca 1380

gctcacctct ggcatttatt cccttctcgg cggggcccag gaactgcata ggtcagactt 1440

tcgccatgag cgagatgaag gtggcgctgg cactaacact gctgcgcttc cgactcctac 1500

cggacgacaa ggaaccgcgc aggcagccag agctgatcct gcgtgccgag ggcggactgt 1560

ggctgcgggt ggagccgctg acggcgggcg cgcagtgacc tcacagttgg tctggagcac 1620

ccccaccatc ctacctctga ggatcccaga atgaacagaa ctgtcaccca taccagaatc 1680

ttacggaggg ccagcagggg gcgctgtgaa tccagtgagt gggtgagaga ggccagttag 1740

tgtctgagcc aaagaccttt gacttcgtct aactgatcgc cagcctttat cataaggtgc 1800

attcttctcc aaccaacctg gactattctc tggacaatag gaagccaaag aaacgtctga 1860

gcaagagacc agaatgaaac gcaaatttga gctcttattc tcgcagcaaa tcccttattc 1920

agctccattt ccattatttt gacctacatt caaatcatct aataaataca ttctctc 1977

<210> 787

<211> 602

<212> DNA

<213> Rattus sp.

<220>

<221> misc_feature

<222> (564)..(564)

<223>  n

<400>  787
gacaaggaca aaggcttgtt ttattttaat gactgatcca cgtgaggcca cagcctggcc      60
actatgtaca gacacgaggg acgcttcatt tcttggtctc ttcctccttg gacagagtct     120
tgatgatctc ctccttcttg gcttggaggc gctcctcccg acgctttcgt gcttccttgg     180
tcttagacct gcgagcctca gcctggtcag ccagtagctt cttgcgggcc ttgtctgcct     240
tcagtttgtg gatgtgctcc atgagaatcc gcttgttttt gaacacattc cctttgacct     300
tcaggtacag gctgtgatac atatgacggt caatcttctt agattcccgg tatctcctga     360
gaagccggcg caggatcctc atccttcgca tccaggtcac cttctcgggc atccgagcgt     420
tggcagtacc cttcctcttc cctatgccca tatgcctgcc cttccgtcgg gccaaggtgt     480
tcttccggca tcgagcccgg gaatggacag tcacaggctt ccggatgatc aggccatctt     540
gatcagcttc ctgatctgtt gacnaagttg gcattggcaa gttcgttggt ttcattgggg     600
tc                                                                    602

<210>  788
<211>  607
<212>  DNA
<213>  Rattus sp.

<400>  788
ggccgcacaa cttttttttt tttttttttt ttttttttgc tggttcaaaa agctttattt      60
ttacttggtc caagacttgg gagaggctcc agggcggtta caaagctgcc tagtggcttt     120
agaggttcat tcgggtggag gtgctgaggc ggtcggtgca gagcagagga gggagcccct     180
tggaaggcac agaggcctag tcgtgcttca gagtgaggcg ctcaaagaga tactcgccca     240
gagatgcctg ggccacgcca gtctgcgctg gttgtggccc tgccacccta cggaggttgg     300
tcaggtggtt gcccatcttc ttgatgagct tcacctcctt atccaggaag tggctttcca     360
agaagtcaca gaggtgaggg tctgtgcggg cagagcccag ggcgtgcaga tccaagaggg     420
cctggttcag gttcttctcc agggccaagg cagcttccat ggcctccagg gttttacccc     480
actcatcttg agatggcttc tgcacatcct ggaagagtgc acggcccccg cgttcgttct     540
gcaacttgag gagacgctcg gcgccctcgc gcttctcctc ggccaattcg cggaagaagt     600
ggcctac                                                               607

<210>  789
<211>  1000
<212>  DNA

<213>  Rattus sp.

<400>  789
caggagcatc tggacagtac accacttgtg atggagactt ctatgcctga atactatgac      60
gtttttggtg atttccatgg atttttaatg gataaacggt tcaccaaata ttgggaagac     120
gttgaaacat tcttggcaag gccagatgac cttctcattg ttacttatcc taaatctggc     180
agcacatgga ttagtgaaat tgtggatatg atctataaag aaggtgatgt ggaaaaatgc     240
aaggaggatg cactttttaa cagaatacct gacctggagt gcagaaatga agatctaata     300
aacggaataa aacaactaaa agaaaaggaa tcgcctagaa tagtgaaaac tcacctgcca     360
gctaagctcc ttccagcatc attttgggaa aagaattgca agataatcta tctttgccga     420
aatgccaaag atgtcgtcgt ttcttattac tactttttcc tgatcatgaa aagttatcaa     480
aatcctaaat ctttttctga atttgtggag aaatttatgg aagggcaagt tccgtatggt     540
tcctggtatg atcatgtaaa atcttggtgg gagaagagta agaattcacg tgttttgttt     600
atgttctatg aggacatgaa agaggatatc cgaagagaag ttgtgaagct gatagagttc     660
ctggagagag acccatcagc agagctagta gacagaatca ttcaacatac atcattccag     720
gagatgaaga acaatccatg catcaattat tcaatgctgc cagagaccat gatagatcta     780
aaagtatcgc ctttcatgag aaagggaatt gtaggagact ggaagaacca cttccctgaa     840
gccctgaggg agagatttga ggagcactac cagcagcaaa tgaaggactg ccctgtgaaa     900
tttagagcag agccctgaga caattccttg tgtctgaaat tggagtagtc tccaatttat     960
ccttcagttt ttcttgtttt gaattcagta gaagtagaag                          1000

<210>  790

<211>  6335

<212>  DNA

<213>  Rattus norvegicus (Norway rat)

<400>  790
ggatcccggc ccgcagcagc tctctgctcc cagaacccgt gagaaagaga cctcaccgcc      60
tggtcaggtg ggcactcctg aggctgcaga gcggaagaga ccaccaacac tgcccacccc     120
tgcccacatc cctggcccaa gaggaaactg tataaggcct ctgggttccg tgggggaggg     180
cccaggagcg tcaggacccc tgcctgagac accgccggaa cctgagggaa acagaccgga     240
taaacagttc tctgcaccca aatcccatgg gagggagagc tgaaccttca gagaggcaga     300
caagccttgg aaaccagaag agactgctct ctgtacatac atctcggacg ccagaggaaa     360
acaccaaagg ccatctggaa ccctggtgca ctgaagctcc tggaaggggc ggcacaggtc     420
ttcctggttg ctgccgccac agagagccct tgggcagcac cccgcctggt gaactcaaga     480
cacaggccca caggaacagc tgaagacctg cagagaggaa aaactacacg cccgaaagca     540

gaacactctg tccccataac ggactgaaag agaggaaaac aggtctacag cactcctgac 600

acacaggctt ataggacagt ctaaccactg tcagaaatag cagaacaaag taacactaga 660

gataatctga tggtgagagg caagcgcagg aacccaagca acagaaacca agactacatg 720

gcatcatcgg agcccaattc tcccaccaaa acaaacatgg aatatccaaa cacaccagaa 780

aagcaagatc tagtttcaaa atcatatttg atcatgatgc tggaggactt caagaaagac 840

gtgaagaact cccttagaga acaagtagaa gcctacagag aggaatcgca aaaatccctg 900

aaagaattcc aggaaaacac aatcaaacag ttgaaggaat taaaaatgga aatagaagca 960

atcaagaaag aacacatgga aacaaccctg gatatagaaa accaaaggaa gagacaagga 1020

gccgtagata caagcatcac caacagaata caagagatgg aagagagaat ctcaagagca 1080

gaagattcca tagaaatcat tgactcaact gtcaaagata atgtaaagcg gaaaaagcta 1140

ctggtccaaa acatacagga aatccaggac tcaatgagaa gatcaaacct aaggataata 1200

ggtatagaag agagtgaaga ctcccagctc aaaggaccag taaatgtctt caacaaaatc 1260

atagaagaaa acttccctaa cctaaaaaaa gagataccca taggcataca agaagcctac 1320

agaactccaa atagattgga ccagaaaaga aacacctccc gtcacataat agtcaaaaca 1380

ccaaacgcac aaaataaaga aagaatatta aaagcagtaa gggaaaaagg tcaagtaaca 1440

tataaaggca gacctatcag aatcacacca gacttctcgc cagaaactat gaaggccaga 1500

agatcctgga cagatgtcat acagacccta agagaacaca aatgccagcc caggttactg 1560

tatcctgcaa aactctcaat taacatagat ggagaaacca agatattcca tgacaaaacc 1620

aaatttacac aatatctttc tacaaatcca gcactacaaa ggataataaa tggtaaagcc 1680

caacataagg aggcaagcta taccctagaa gaagcaagaa actaatcgtc ttggcaacaa 1740

aacaaagcga atgaaagcac acaaacataa cctcacatcc aaatatgaat ataacgggaa 1800

gcaataatca ctattcctta atatctctca acataaatgg ccttaactcc ccaataaaaa 1860

gacatagatt aacaaactgg atacgcaacg aggaccctgc attctgctgc ctacaggaaa 1920

cacacctcag agacaaagac agacattacc tcagagtgaa aggctggaaa acaattttcc 1980

aagcaaatgg tcagaagaag caagctggag tagccattct aatatcaaat aaaatcaatt 2040

tttaactaaa agtcatcaaa aaagataagg aaggacactt catattcatc aaaggaaaaa 2100

tccaccaaga tgaactctca atcctaaata tctatgcccc aaatacaagg gcacctacat 2160

atgtaaaaga aaccttacta aagctcaaaa cacacattgc acctcacaca ataatagtgg 2220

gagatttcaa caccccactc tcatcaatgg acagatcatg gaaacagaaa ttaaacagag 2280

atgtagacag actaagagaa gtcatgagcc aaagggactt aacggatatt tatagaacat 2340

tctatcctaa agcaaaagga tataccttct tctcagctcc tcatggtact ttctccaaaa 2400

ttgaccatat aattggtcaa aaaacgggcc tcaacaggta cagaaagata gaaataatcc 2460

catgcatgct atcggaccac cacggcctaa aactggtctt caataacaat caaggaagaa 2520

tgcccatata tacttggaaa ctgaacaatg ctctactcaa tgataacctg gtcaaggaag 2580

aaataaagaa agaaattaaa aactttttag aatttaatga aaatgaaggt acaacatacc 2640

caaacttatg ggacacaatg aaagctgtgc taagaggaaa actcatagcg ctgagtgcct 2700

gcagaaagaa acaggaaaga gcatatgtca gcagcttgac agcacaccta aaagctctag 2760

aacaaaaaga agcaaataca cccaggagga gtagaaggca ggaaataatc aaactcagag 2820

ctgaaatcaa ccaagtagaa acaaaaggac catagaaaga atcaacagaa ccaaaagttg 2880

gttctttgag aaaatcaaca agatagataa acccttagcc agactaatga gaggacacag 2940

agagtgtgtc caaattaaca aaatcagaaa tgaaaaggga gacataacaa cagattcaga 3000

ggaaattcaa aaaatcatca gatcttacta taaaaaccta tattcaacaa aacttgaaaa 3060

tcttcaggaa atggacaatt ttctagacag ataccaggta ccgaagttaa atcaggaaca 3120

gataaaccag ttaaacaacc ccataactcc taaggaaata gaagcagtca ttaaaggtct 3180

cccaaccaaa aagagcccag gtccagacgg gtttagtgca gaattctatc aaaccttcat 3240

agaagacctc ataccaatat tatccaaact attccacaaa attgaaacag atggatcact 3300

accgaatacc ttctacgaag ccacaattac tcttatacct aaaccacaca aagacacaac 3360

aaagaaagag aacttcagac caatttccct tatgaatatc gacgcaaaaa tactcaacaa 3420

aattctggca aaccgaatcc aagagcacat caaaacaatc atccaccatg accaagtagg 3480

cttcatccca ggcatgcagg gatggtttaa tatacggaaa accatcaacg tgatccatta 3540

tataaacaaa ctgaaagaac aaaaccacat gatcatttca ttagacgctg agaaagcatt 3600

tgacaaaatt caacacccct tcatgataaa agtcctggaa agaattggaa ttcaaggccc 3660

atacctgaac atagtaaaag ccatatacag caaaccagtt gctaacatta aactaaatgg 3720

agagaaactt gaagcaatcc cactaaaatc agggactaga caaggctgcc cactctctcc 3780

ctacttattc aatatagttc ttgaagttct ggccagagca atcagacaac aaaaggaggt 3840

caaggggata cagatcggaa aagaagaagt caaaatatca ctatttgcag atgatatgat 3900

agtatattta agtgatccca aacattccac cagagaacta ctaaagctga tagacaactt 3960

cagcaaagtg gctaggtata aaattaactc aaataaatca gttgccttcc tctatacaaa 4020

agagaaacaa gccgagaaag aaattaggga aacgacaccc ttcataatag acccaaataa 4080

tataaagtac ctcggtgtga ctttaacaaa gcaagtaaaa gatctgtaca ataagaactt 4140

caagacactg aagaaggaaa ttgaagaaga cctcagaaga tggaaagatc tcccgtgctc 4200

atggattggc aggattaata tagtaaaaat ggccatttta ccaaaagcaa tctacagatt 4260

caatgcaatc cccatcaaaa taccaatcca attcttcaaa gagttagaca gaacaatttg 4320

caaattcatc tggaataaca aaaaacccag gatagctaaa gctatcctca acaataaaag 4380

gacttcaggg ggaatcacta tccctgaact caagcagtat tacagagcaa tagtgataaa 4440

aactgcatgg tattggtaca gagacagaca gatagaccaa tggaatagaa ttgaagaccc 4500

agaaatgaac ccacacacct atggtcactt gatttttgac aaaggagcca aaaccatcaa 4560

atggaaaaaa gatagcattt tcagcaaatg gtgctagttc aactggaggt caacatgtag 4620

aagaatgaag atcgatccat gcttgtcacc ctgtacaagc ttaagtccaa gtggatcaag 4680

gacctccaca tcaaaccaga cacactcaaa ctaatagaag aaaaactagg gaagcatctg 4740

gaacacatgg gcactggaaa aaatttccta aacaaaacac catggcttac gctctaagat 4800

caagaatcga caaatgggat ctcataaaac tgcaaagctt ctgtaaggca aaggacactg 4860

tggttaggac aaaacggcaa ccaacagatt gggaaaatat ctttaccaat cctacaacag 4920

atagaggcct tatatccaaa atatacaaag aactcaagaa gttagaccgc agggaaacaa 4980

ataaccctat taaaaaatgg ggttcagagc taaacaaaga attcacagct gaggaatgcc 5040

aaatggctga gaaacaccta aagaaatgtt caacatcttt agtcataagg gaaatgcaaa 5100

tcaaaacaac cgtgagattt cacctcacac cagtgagaat ggctatgatc aaaaactcag 5160

gggacaacag atgctggcga ggatgtggag aaagaggaac actcctccat tgttggtggg 5220

attgcaaact ggtacaacca ttctggaaat cagtctggag gttcctcaga aaattggaca 5280

ttgaactgcc tgaggatcca gctatacctc tcttgggcat atacccaaaa gatgccccaa 5340

catataaaaa agacacgtgc tccactatgt tcattgcagc cttatttata atagccagaa 5400

gctggaaaga acccagatgc ccttcaacag aggaatggat acagaaaatg tggtacatct 5460

acacaatgga atattactca gctatcaaaa acaacgagtt tatgaaattc gtaggcaaat 5520

ggttggaact ggaaaatatc atcctgagta agctaaccca atcacagaaa gacatacatg 5580

gtatgcactc attgataagt ggctattagc ccaaatgctt gaattaccct agatacctag 5640

aacaaatgaa actcaagacg gatgatcaaa atgtgaatgc ttcactcctt ctttaaaagg 5700

ggaacaagaa tacccttggc agggaagaga gaggcaaaga ttaaaacaga gaatgaagga 5760

acacccattc agagcctgcc ccacatgtgg cccatacata tacagccacc caattagaca 5820

agatggatga agcaaagaag tgcagaccga caggagccgg atgtagatcg ctcctgagag 5880

acacagccag aatacagcaa atacagaggc gaatgccagc agcaaaccac tgaactgaga 5940

ataggacccc cgttgaagga atcagagaaa gaactggaag atcttgaagg ggctcgagac 6000

cccatatgta caacaatgct aagcaaccag agcttccagg gactaagcca ctacctaaag 6060

actatacatg gactgaccct ggactctgac ctcataggta gcaatgaata tcctagtaag 6120

agcaccagtg gaaggagaag ccctgggtcc tgctaagact gaacccccag tgaactagac 6180

tggtgggggg agggcggcaa tgggggaggg gttgggaggg gaacaccata aggaagggga 6240

gggggagggg ggatgtttgc ccggataccg aaagggaata acatcgaaat gtatataaga 6300

atactcaagt taataaaaaa aaaaaaaaaa aaaga 6335

<210> 791

<211> 922

<212> DNA

<213> Rattus rattus (black rat)

<400> 791
   tctttttctt cgggaaaaca ccaaatggcg gatgacgccg gtgcagcggg agggcccgga 60
   ggacccgggg gcccaggatt aggaggccgc ggcggcttcc gcggaggatt cggcagcggc 120
   cttaggggcc gcggtcgtgg ccgaggccgt ggccgcggtc gaggccgcgg ggctcgtgga 180
   ggtaaagctg aagacaagga gtggatcccc gttaccaagc tgggccgcct ggttaaggac 240
   atgaagatca agtccttgga ggagatctac ctgttctccc tgcccattaa ggagtctgag 300
   attattgact ttttcctggg tgcatctcta aaggatgaag ttctgaaaat catgccagtg 360
   cagaagcaga ctcgggctgg ccagcggacc aggttcaagg cctttgtcgc tattggggac 420
   tacaatggcc acgttggtct tggtgttaag tgctccaagg aggtagccac tgccatccga 480
   ggggccatca tcctggccaa gctttcgatc gtcccagtgc ggagaggcta ctgggggaac 540
   aagattggca agccccacac tgtgccatgc aaggtgacag gccgctgtgg ctctgtgctg 600
   gttcgtctca tccctgcccc cagaggcact ggcattgtct ctgctcctgt gcccaagaag 660
   ctactgatga tggccggtat agatgactgc tacacttcag ccaggggctg cactgccacc 720
   ctgggcaact ttgccaaggc cacctttgat gccatctcca agacctacag ctacctgacc 780
   cctgacctct ggaaagagac tgtgttcacc aagtctcctt atcaggaatt cactgaccat 840
   cttgtgaaaa cccacaccag agtctctgtt cagaggaccc aggctccagc tgtggctacc 900
   acataagggt ttttatacaa ga 922

<210> 792

<211> 744

<212> DNA

<213> Rattus rattus (black rat)

<400> 792
ctcttcctgt ctgtgccaga actgcgcgtg gtccgcgccg atcgactgag aagcccggtt 60

tgcgctctca gaatgactga atgggaaaca gccacacccg cggtggcaga gaccccggac 120

atcaagctct ttgggaaatg gagcactgat gatgtgcaga tcaacgatat ttctctacag 180

gattacattg ctgtgaagga gaagtatgcc aagtacctgc cccacagtgc aggacggtat 240

gctgccaacg gtttccgcaa agcacagtgt cccatcgtgg agcgccttac taactccatg 300

atgatgcacg gtcgtaacaa cggcaagaag ctcatgactg tacgaattgt caagcatgcc 360

tttgagatca tccacctgct cactggtgag aaccctctgc aggtcctggt gaatgctatc 420

atcaacagtg gcccccgaga agactcaaca cgcattgggc gggctggaac agtgagacgg 480

caggctgtgg atgtatcccc acttcgccga gtgaatcagg ccatctggct gctgtgcacg 540

ggggctcgtg aggctgcttt ccggaacatc aagaccatcg ctgagtgcct tgcagatgag 600

ctcattaatg ctcgcaaggg ctcctccaac tcctatgcta tcaagaagaa agatgaactg 660

gagcgtgtgg ccaagtctaa ccgctgattt cccagctgct gcctaataaa ttgtgtgccc 720

tttgggacag ttacatcaaa aaaa 744

<210> 793

<211> 685

<212> DNA

<213> Rattus norvegicus (Norway rat)

<400> 793
acgagtgctg acatgatcac tctctgtgtt cacaggaaag cacatttgtc ttggcgaagg 60

cattgcccga aatgaattgt tcctcttctt caccaccatc ctccagaact tctctgtgtc 120

aagccatttg gctcccaagg acattgacct cacgcccaag gagagtggca ttggaaaaat 180

acctccaacg taccagatct gcttctcagc tcggtgatcc ggctgaggca gccatgtgcc 240

ccagttctgt tgggaatggc ctcatgtttc tgcctctggg ggacctgctg aaaaccaggc 300

tccaaggcca ctgctccaca tcttcctatt gcagttctcc aaagtcccaa ggctttttct 360

tattcctgtg aatggcactg aagaagtcaa tcggctgtct tattttgaca tgtgacagag 420

atttcatgag tccacatctc atgctgagtt acttccctct tcctcctaac agcccatgtc 480

cccagttatc agccctccat ggtctgtgat ctgtgctaat ggactctgta tatggtctca 540

gtgctatgtc tacagactta catagtatgt atggttcagg taaacagaat cacagagtgt 600

gtgagcttcg gagtcttgtg cctttacttc acataatatt attctaggtt cctgtgttct 660

acaggccaca gtcacacaca ttcat 685

<210> 794

<211> 654

<212> DNA

<213> Rattus sp.

<400> 794
aactggggac atgtgtttat tcagcagaaa gggggagccc gttggtgggg cagcgttaac 60

acaggtggat ggggagagct gatcaaataa gagggatcag atgcttggtg gaatcatggc 120

tggtggggca gccaggtgat tctctggcaa caggaaagat ggagcggcag acagacaggt 180

gggacctgaa ccatcaggcc actgcacatc ccagtcagcc acgctcaccg tctgttcagt 240

tgtcaatgca ttggtcggtg ggaacagaga aaacgatgtt gttatccttg aggcccagag 300

acttggcaaa gctgacgaat cgctccttca gttcatcgga cagccccttg gttcttccgt 360

acagggtgac tttgaagtac tgtttgtttt cagaggtctt ctggaaaaat accatggcaa 420

actggtcgta gtcagtgtcg gccacttgca catcgtagct ctgtatctga gggtagctgt 480

gaatattccc cagggtgaac tggccaggcc tggagcttgg aacgaatgtt ctgatccagt 540

agcgacagcc ctggcccctg acgaggatgg aagtgacgtt gtagctattg tcttcctgta 600

actcatagat ggtgctgtac atggtaaagc ggctttgtct ttctttctgg accg 654

<210> 795

<211> 1140

<212> DNA

<213> Rattus norvegicus (Norway rat)

<400> 795
ggatcctaac ttcttcccaa gcaccagtca ctggagacat aggagaggca agaagcctct 60

gggcaactga taaggattcc ccgcacccag gggaggcgat ccccaagccc agcctctcag 120

ggtcctgaca agccatgctg cggagagcaa tcacaggcca gccaatgaca gctgctccaa 180

gggcgtgtcc ttacctcctg cacatataaa tgaaacttgc tgcggggcca atacattctc 240

attctgatag actcaggaag caatcatggt gctctctgca gctgacaaaa ccaacatcaa 300

gaactgctgg gggaagattg gtggccatgg tggtgaatat ggcgaggagg ccctacagag 360

gtaacatcag gaccctgttc tttaaggaca gcaggatcca aaccggacca gggactcagt 420

gggtagctcc taagtgtgct ttcccgtggc ctcaacttat ctctccttct cacaggatgt 480

tcgctgcctt ccccaccacc aagacctact tctctcacat tgatgtaagc cccggctctg 540

cccaggtcaa ggctcacggc aagaaggttg ctgatgccct ggccaaagct gcagaccacg 600

tcgaagacct gcctggtgcc ctgtccactc tgagcgacct gcatgcccac aaactgcgtg 660

tggatcctgt caacttcaag gtgtgagctc agacctggca gggggcacct gggaccttca 720

aggatccctt ggggcagtgg tgaggggcac agggggaggg ggaaggggtt cctcatgccc 780

agggcaggga acacagtgtt ccaggaaggg gagcttaccc agcagggtgt ctactactag 840

ggactgaccc ttcctgtctc tgcagttcct gagccactgc ctgctggtga ccttggcttg 900

ccaccaccct ggggatttca cacctgccat gcacgcctct ctggacaaat tccttgcctc 960

tgtgagcacc gtgctgacct ccaagtaccg ttaagccacc tcctgtcggg cttgccttct 1020

gaccaggccc ttcttccgtc ccttgcacct gtctttgaat aaagcaaaag taggaagaat 1080

cccgtgtgcc tgtttctcac atgtgcaaag gtgacaatgt ttggtatggg aaaatcctcc 1140

<210> 796

<211> 1884

<212> DNA

<213> Rattus norvegicus (Norway rat)

<400> 796
caagcctttg ctggagaccg ctcctgtcca gtccgcagct ggcttcagcg ccactcagga 60

caccggaaag atggcaccga ttgccggcaa gaaggccaag aggggaatct tagaacgctt 120

aaatgctggc gaagtcgtga tcggagatgg gggatttgtc tttgcactgg aaaagagggg 180

ctacgtaaag gctggaccct ggaccccaga ggctgcggtg gagcaccccg aggcagttcg 240

gcagcttcat cgggagttcc tcagagctgg atcgaacgtc atgcagacct tcactttcta 300

tgcaagtgag gacaagctgg aaaaccgagg gaactacgtg gcagagaaga tatctgggca 360

gaaggtcaat gaagctgctt gtgacattgc acggcaagtt gctgacgaag gggatgcatt 420

ggttgcagga ggtgtgagtc agacaccttc ctacctcagc tgcaagagtg agacggaagt 480

taaaaagata tttcaccaac agcttgaggt cttcatgaag aagaatgtgg acttcctcat 540

tgcagagtat tttgaacatg ttgaagaagc cgtgtgggca gtcgaggcct taaaaacatc 600

cgggaagcct atagcggcta ccatgtgcat cggacctgaa ggagatctac atggcgtgtc 660

tcctggagag tgcgcagtgc gtttggtaaa agcaggtgcc gccattgtcg gtgtgaactg 720

ccacttcgac cccagcacca gcttgcagac aataaagctc atgaaggagg gtctggaagc 780

agctcggctg aaggcttact tgatgagcca cgccctggcc taccacaccc ctgactgtgg 840

caaacaggga tttattgatc tcccagaatt cccctttgga ttggaaccca gagttgccac 900

cagatgggat attcaaaaat acgccagaga ggcctacaac ctgggggtca ggtacattgg 960

cggctgctgc ggatttgagc cctaccacat cagggccatt gcagaggagc tcgccccaga 1020

aggggatttt ttaccaccag cttcagaaaa acatggcagc tggggaagtg gtttggacat 1080

gcacaccaaa ccctggatca gggcaagggc caggaaagaa tactggcaga atcttcgaat 1140

agcttcgggc agaccgtaca atccttcgat gtccaagccg gatgcttggg gagtgacgaa 1200

aggggcagca gagctgatgc agcagaagga agccaccact gagcagcagc tgagagcgct 1260

cttcgaaaaa caaaaattca aatccgcaca gtagccacag gccagcggtt cggggcgaat 1320

tcctccaggt ccgggccaca gtgtgcaccc ggaaggagaa ggcatctcta aaccagcgtt 1380

tgtgttgatg ccggcttaca cctgtgattg gtgctagtta gacaaaatgg agtcacagat 1440

agcatttcac agttacaaaa ctacgcttta gaattttacc tagaaggaag aaaggagaag 1500

tccacagtaa atcctgaaca catttcctac gtgcctgtcg cattacaggc gcacaggagt 1560

cactgcagcg aagagaaagt cacccgacgt caatctcatt tcagataggg ggataggaca 1620

ccacctccac gagtgacata gaaccattca gggaccgtat cataagtgac acagcaacca 1680

tctatatcta agatgcttcc caagtggatt ccaagatctt ttgagcagga cccttaggca 1740

gaaacaacac acaccagccc tgtaaaactt aacagataac tgatccattc tgtaattctg 1800

taatctctgt tctgactgct tccattccat ttcattaata aaaacatgcc ggttgaaaac 1860

cttcaaaaaa aaaaaaaaaa aaaa 1884

<210> 797

<211>  4198

<212>  DNA

<213>  Rattus norvegicus (Norway rat)

<400>  797
gacgcgctga gccgctgccg ccgcgggggg gaagcctgcg agtagctcgc gtgcccgcag      60

tccgcgtaag ccccgcgccc cttccacccc tggccaggcc atggcggaac gtggaggggc     120

gggcggtggt cccggaggcg ccgggggcgg cagcagtcag cggggctctg gggttgccca     180

gtccccgcag cagcagccgc cgcagcagcc atctcagccg caacagccaa cgccccccaa     240

actggcccag gccacctcgt cctcctcgtc cacctcggct gcggccgcct cctcctcgtc     300

ctcctccacc tccacctcca tggccgtcgc ggtggcctcg ggctctgcgc ctcccggcgg     360

tccgggacca ggccgcaccc ccgccccagt gcagatgaat ttgtatgcta cctgggaggt     420

ggaccggagc tcatccagct gcgtgcccag actcttcagt ttgactctga agaaacttgt     480

tatgcttaaa gaaatggaca aagatcttaa ctcagtagtc attgctgtga agctgcaggg     540

ttcaaaaagg attcttcgct ctaatgagat catccttcca gccagtggcc tggtggaaac     600

agagctccag ttaaccttct ccctccagta ccctcatttc cttaagcgag atgccaacaa     660

actgcagatt atgttgcaaa ggaggaagcg gtacaagaac cggactatct tgggctataa     720

aaccttggct gtgggactca tcaacatggc agaggtgatg caacacccta acgagggtgc     780

tttagtactt ggcctgcata gcaacgtaaa ggatgtctct gtgcctgtgg cagaaataaa     840

aatctactct ttatccagcc agcccattga ccatgaagga atcaagtcta agctgtctga     900

tcgctcccca gatattgaca actactctga agaagaggag gaaagcttct cctcagagca     960

ggaaggcagt gatgacccat tacatggaca ggacctattc tatgaagatg aagatcttcg    1020

gaaagtaaag aagactcgaa ggaaactgac ctcaacctca gccatcacta ggcaaccgaa    1080

catcaaacag aagtttgtgg ccctcctgaa acgattcaaa gtttctgatg aggtgggctt    1140

tgggctagaa catgtgtcaa gggagcagat tcgggaagtg gaagaggacc tggatgaact    1200

atacgacagt ctagaaatgt acaaccccag cgacagtggc cctgagatgg aagaaacaga    1260

aagcatcctc agcaccccaa agcccaagct caagcccttc tttgagggga tgtctcagtc    1320

cagctcacag acggagatcg gcagtctcaa cagcaagggc agccttggaa aagacaccac    1380

cagccctatg gaattggcag ctttagaaaa agttaagtct acttggatta aaaaccaaga    1440

tgacagcttg actgaaacag acactttaga aatcactgac caggacatgt ttggagatgc    1500

gagcacaagt ctggttgtac cagaaaaagt caaaactcct atgaagtcca gcaaagctga    1560

tctgcagggc tccgcctcgc cgagcaaggt ggaagggaca cacacacctc gccaaaagag    1620

gagcactccc ctgaaggagc ggcagctctc caagcccctg agtgagagga ccaacagctc    1680

tgacagtgag cgctctcctg acctagggca cagcacacag attccacgaa aggtggtata    1740

cgatcaactg aatcagatcc tggtttccga tgcagcgctc cctgagaatg tcatcctggt    1800

gaacaccacg gactggcagg gccagtatgt ggccgaattg cttcaagacc agaggaagcc 1860
tgttgtgtgt acctgctcaa cagtggaggt ccaggccgtg ttgtctgccc tgctcactag 1920
gattcagcgc tactgcaact gcaactcatc tatgccgagg ccagtcaagg tggcagctgt 1980
gggcagccag agttacctga gctccatcct tcggtttttt gtcaagtccc ttgccagcaa 2040
gaccccggac tggctgggcc acatgcgctt cctcatcgtt cccctaggtt ctcatccagt 2100
ggccaaatac ttgggctcag tagatagtag atatagcagc acctttcttg attctgcttg 2160
gagagacctg ttcagtcgct cagagcctcc agtgtcagag ccactggatg tggtgggtcg 2220
tgtgatgcag tatgtcaatg gagctaccac aacgcaccag ctgcctgtgg ctgaagccat 2280
gttgacctgc aggcataagt tccctgatga agactcctac cagaagttta tccccttcat 2340
tggtgttgtg aaagtgggcc ttgttgaaga ctcgccctcc actgcaggtg atggggacga 2400
ttcacctgtg gtcagcctta ctgtgccctc cacatcacca ccctcaagct cgggcctgag 2460
ccgagatgcc acagctaccc ctccctcatc cccatccatg agcagtgccc tcgccatcgt 2520
ggggagccca aatagccctt atggggatgt gattggcctc caggtggatt actggctggg 2580
ccaccctggg gagcgaagga gagaaggtga taagagggac gccagttcaa agaataccct 2640
caagagtgtc ttccgctcag tgcaggtttc tcgcctgccc catgcagggg aagcccagct 2700
ttctggcacc atggcaatga ctgtggtcac caaagagaag aacaagaaag tgcccactat 2760
cttcctgagc aagaagcccc gggaaaagga ggtggattct aagagccaag tcatcgaggg 2820
catcagccgc ctcatctgtt ctgcaaagca gcagcaaacc atgctgagag tgtccattga 2880
tggagttgaa tggagtgaca tcaagttctt ccagctggca gctcagtggc ccactcatgt 2940
caagcacttc ccagtgggac tcttcagtgg cagcaaaccc acctgagacc tgcctcccaa 3000
ccactccctc ctggcactgc cacccagccc agcctccttt cctagcactg gggcttgcct 3060
ccacttgccc agtcctgaag gacactgcca ctggggatgc agtcctccct gctcactgcc 3120
cagccatctc tgcccagctc ccccctgttc caggcccaga gctgggtgat cctgccttct 3180
ggtgcccata attcctgtaa ttgaataatc tgggccttcc ttcacctcac ccctgcagcg 3240
ctggtaccct ttctagaaat gaaacacctg gagttctggt acacagggag acagcccagc 3300
atcctacata agctggccac ccagctctgc atttctaagg ctctgttgcc tgttgtactc 3360
accctgcctt cctccagcag ggctggggcc tcctgccatt cctcacctgc ctcacagttt 3420
cccagtaggg ggaaggagga tggcacctgg gcatcctgga gtttctggga ttgaagggac 3480
cttcccagtc tcccatgcat gatttcctga cacttcaagt cgcttggatg tcctgtgttt 3540
tgtgtgtttt tagttgtttg ggtaacttgt cctgtgcaca ggtccttaag gctccttctc 3600
cctctgcctc ccagcctcct acctaacatt gctctgtggc ttcatgaggt aggagacagc 3660
aaacccaccc acccacccac cccttcaccc ctcaacacat acatcatctg tggctctcat 3720
ccagtcatat tgtccccctc tcctgtgtca gcttgcctcc tggctctgct ctgtcatccc 3780
acttccttca tggcagggca gcagaatgag gcccttctcc accaggtctc cctccagcag 3840

cctccttccc tctctcagcc atgaatgggt cccagaccta cctcagcctc cttatcagtg 3900

tcttcatgag aggctgagga caagggacag atctggccca gcctccatgc cctctgtgtc 3960

ctatggcacc tcagagcttc tccgttgccc ctcagtgttt cctagcagtg tggcacggaa 4020

ctgcacttgg aacctagttt tggtgttctt cacatccaaa actcttaagc ccctcccgct 4080

gcccagcctg tccagagtat ctcctgggcc tgagaataag tggggtggga agactggggc 4140

catgttgggt tttttcattc aataaactgg tgatttctta ccaaaaaaaa aaaaaaaa 4198

<210> 798

<211> 1234

<212> DNA

<213> Rattus norvegicus (Norway rat)

<400> 798
ggggtagctt cttcagatcc gtacagcctg ctacaaaaat gggtactgca gaagatgttt 60

ttagaaaaga tctgaagatc attcatggct accccatggt ctatgctttt gcactgggtt 120

gggaaaaaat cgaagagttc cagagcagac catgtgacat tgtaataccc acttacccta 180

aatcaggtac tacttggctt agtgaaattg tggacatggt tctaaatgat ggaaatgttg 240

aaaaatgtaa gcgagatgtt attacctcta aagttccaat gttggaacaa aatgttcctg 300

gagcaagaag atcaggtgtt gaactcttga agaaaactcc atcacctcga attataaaga 360

cacatcttcc aattgatcta ctcccaaaat ccttctggga taacaagtgc aagatgattt 420

accttgctag aaatggcaag gatgttgctg tgtcctatta tcattttgat ctaatgaata 480

atattcagcc tcttcctggc acctgggaag aatatctgga gaaattcctc gctggaaatg 540

tggcctatgg ctcatggttc gatcatgtta agagttggtg ggaaaagagg gaagggcatc 600

ccatactttt cttatactat gaagacttga aaaagaaccc gaagaaagaa atcaagaaga 660

ttgccaactt tctagacaag accttggatg aacatacctt ggaaaggatc gtccatcaca 720

cctcctttga agtgatgaag gataaccccc tggtcaatta cacccatctg cccacagaaa 780

taatggatca cagcaagtcc ccattcatga gaaaaggtgt tgttggtgac tggaaaaatt 840

acttcacaat gacccaaagt gagaaatttg atgccatata taagaagaaa ttgtctggaa 900

caacacttga gttctgcaca gatattcaga gtgcctaaac ttcaacttga atatatgatt 960

tcttgaaata gtagtttgac agggaaatca gatggatttg tgagggagaa ataaatgtgc 1020

ttttaaaaca ctgattaaat atgccctgca catccctcag caggaattat taataattcc 1080

gaattatcta gggacaaggt cttttgtgat cttagttttc aaagggtatg tcttcagatt 1140

ccaaagtgac tactgaatta aataaataag ttttttttaa aacactgatt aaatatgccc 1200

tgcacatccc tcagcaggaa ttattaataa ttcc 1234

<210> 799

<211> 1003

<212> DNA

<213> Rattus norvegicus (Norway rat)

<400> 799
tgggcaccat cagcagagag attgtcccga cagagaggca attctattcc ctaccaacat 60

gaagctgttg ctgctgctgc tgtgtctggg cctgaccctg gtctgtggcc atgcagaaga 120

agctagtttc gagagaggga acctcgatgt ggacaagctc aatggggatt ggttttctat 180

tgtcgtggcc tctgataaaa gagaaaagat agaagagaac ggcagcatga gagtttttgt 240

gcagcacatc gatgtcttgg agaattcctt aggcttcacg ttccgtatta aggaaaatgg 300

agtgtgcaca gaattttctt tggttgccga caaaacagca aaggatggcg aatattttgt 360

tgagtatgac ggagaaaata catttactat actgaagaca gactatgaca attatgtcat 420

gtttcatctc gttaatgtca acaacgggga aacattccag ctgatggagc tctacggcag 480

aacaaaggat ctgagttcag acatcaagga aaagtttgca aaactatgtg tggcacatgg 540

aatcactagg gacaatatca ttgacctaac caagactgat cgctgtctcc aggcccgagg 600

ttgaagaaag gcctgagcct ccagattgca gggcaagatc tatttcttca tcctttgttc 660

tatacaatag agtgcctctc tgtccagaag tcaatccaag aagtgcttaa tgggttcctt 720

tattctttct tcctggatta ctccgtgctg agtggagact tctcaccagg actccagcat 780

taccatttcc tgtccatgga gcatcctgag acaaattctg cgatctgatt tccatcctgt 840

ctcacagaaa agtgcaatcc tggtctctcc agcatcttcc ctagttaccc aggacaacac 900

atcgagaatt aaaagctttc ttaaatttct ctttgcccca ctcatgatca ttccgcacaa 960

atttcttgct cttgcagtgc aataaatgat tacccttgca ctt 1003

<210> 800

<211> 6849

<212> DNA

<213> Rattus norvegicus (Norway rat)

<220>

<221> misc_feature

<222> (2293)..(2293)

<223> n

<400> 800
ccctcacagt attaagtacc cttaccctac cgctttcctc ccaactaatc acatagaagt 60

ttaggatata cagttcaaga gccttcaaag cccttagaaa acaaacaagt ttaacttctg 120
ataaggactg taagactata tcttacatct gttaaatgca aatcaactgc tttaattaag 180
ctaaatcctc aactagattg gaaggattca aacctacgaa aatttagtta acaactaaat 240
accctactta ctggcttcaa tctacttctc ccgcctatca gaaaagaggc gggagaagcc 300
ttagtagagg agattctcta caccttccaa tttgcaattc gacatgataa tcaccttaag 360
gctttttggt aaaaaggggg ctcaacccct gtctttagat ttacagtcta atgcttactc 420
agccatttta cctatgttcg taaacggttg actcttttca actaacccta aagatatcgg 480
aaccctctac ctattatttg gagcctgagc aggaatagta gggacagctt taagtattct 540
aattcgagct gaactaggac agctaggcgc actcctagga gatgaccaaa tctataatgt 600
cctcgtcaca gcccatgcat tcgtaataat tttctttata gtaataccta taataattgg 660
aggcttcgga aactgacttg taccactaat aattggagcc cctgatatag cattcccacg 720
aataaataac ataagctttt gactgcttcc tccatcattt ctactccttt tagcatcctc 780
catagtagaa gctggagctg gaacaggatg aacagtatat ccccccttag ccggaaacct 840
agcccatgct ggggtatccg tagatttaac tatttttccc ctccacctag ccggggtgtc 900
ttctatctta ggagctatca actttatcac cactatcatt aatataaaac cccctgctat 960
aacccaatat cagacacctc tctttgtatg atccgtacta attacagccg tcctactact 1020
tctctcactg ccagtattag cagcaggtat cactatactc cttacagacc gaaatctaaa 1080
tactactttc ttcgaccccg ctggaggcgg aggcccaatt ctttatcaac acctattctg 1140
attcttcggc cacccagaag tgtacatctt aattcttcta gggtttgaaa ttatttcaca 1200
tgtagttacc tattactctg gaaaaaaaga acccttcgga tatataggta tggtatgaac 1260
cataatatct attggcttcc taggatttat tgtatgagca catcacatat tcactgtagg 1320
cctggatgta gacacccgag cctactttac atctgccact ataattatcg caattcctac 1380
aggcgtaaaa gtattcagct gactcgctac actacatgga ggaaatatca aatgatcccc 1440
cgccatatta tgagccttag ggtttatctt cttattcaca gtagggggcc taacagggat 1500
cgtactatct aactcatccc ttgacattgt acttcatgat acatactacg tagtagctca 1560
cttccactat gtcttatcta taggagcagt attcgccatc atagcttgct tcgtccactg 1620
attcccacta ttctcaggct ataccctaaa tgacacatga gcaaaagccc tctttgccat 1680
tatatttgta ggtgtaaaca taacattttt tcctcaacac ttcctaggat tagcagggat 1740
acctcgtcgt tactctgatt atccagatgc ttataccaca tgaaatacag tctcctctat 1800
aggctcattc atctcactta cggccgtcct tgtaatgatc ttcatgattt gagaagcctt 1860
cgcatcaaaa cgagaagtgc tctcaatttc ctactcttca actaacctag aatgactgca 1920
tggatgcccc ccaccttacc acacattcga agaaccttcc tatgtaaaag ttaaataaga 1980
aaggaaggat tcgaaccccc tacaactggt ttcaagccaa tttcataacc attatgtctt 2040
tctcaatgag atattagtaa aataattaca taaccttgtc aaggttaagt tatagactta 2100

aatctatata tcttacatgg cttacccatt tcaacttggc ttacaagacg ccacatcacc 2160

aatcatagaa gaacttacaa actttcatga ccacacccta ataattgtat tcctcatcag 2220

ctccctagta ctttatatta tttcactaat actaacaaca aaactaacac acacaagcac 2280

aatagacgcc cangaagtag aaacaatttg aacaattctc ccagctgtca ttcttattct 2340

aatcgccctt ccctccctac gaattctata cataatagac gagattaata acccagttct 2400

aacagtaaaa actataggac accaatgata ctgaagctat gaatatactg actatgaaga 2460

cctatgcttt gactcctaca taatcccaac caatgaccta aaactaggtg aacttcgctt 2520

attagaagtt gataatcggg tagtcttacc aatagaactt ccaattcgta tactaatctc 2580

atccgaagac gtcctgcact catgacccat cccttcacta gggttaaaaa ccgacgcaat 2640

ccccggccgc ctaaaccaag ctacagtcac atcaaaccga ctaggtctat tctatggcca 2700

atgctctgaa atttgcggct caaatcacag cttcatactc attgtactag aaatagtgcc 2760

tctaaaatat ttcgaaaact gatcagcttc tataatttaa actcattgcg aagcttagag 2820

cgttaacctt ttaagttaaa gttagagaca acaaatctcc acattgacat gccacaacta 2880

gacacatcca catgatttat tacaatcatc tcctcaatag ccacactatt taatttattt 2940

caattaaaaa tttcttccca aacctttcct gcacctccct cacccaaaac tatagccaca 3000

gaaaaaacga ataacccttg agaatcaaaa tgaacgaaaa tctatttccc tctttcatta 3060

cccccacaat aataggtcta ccaattgttg tgaccattat tatgttccca tcaattctat 3120

tcccatcatc agaacgccta atcagcaacc gactacactc atttcaacac tgactaatca 3180

aacttatcat caaacaaata atgttaattc acacaccaaa aggacgaacc tgagccctaa 3240

taattgtatc cctaattata tttatcggct caaccaacct tctagggctt cttccccata 3300

catttacccc taccactcag ctatctataa acctaagcat agccatcccc ctatgagcag 3360

gagccgtaat tctaggcttc cgacacaaac taaaaaattc tttagcccac ttcttaccgc 3420

aaggaacccc catctcacta attcccatac taatcatcat cgaaactatc agcctattta 3480

ttcaaccgat agcactagca gtacgactaa cagcaaacat tacagcaggc catctattaa 3540

tgcatctaat cggaggagct accctagtac ttatagacat cagcccacca accgctacaa 3600

ttacatttat tattctactt ctacttacag tacttgaatt tgtcgtagcc ttaattcaag 3660

cctatgtatt cacccttcta gtaagcctgt acctacatga taacacataa tgacccacca 3720

aacccatgca taccatatag taaacccaag cccatgacca ctaacaggag ccctatcagc 3780

tcttctactc acatccggct tagtaatatg attccattac aactccacaa ttctcctatc 3840

attaggcctc ctgacaaaca tcctaactat atatcaatga tgacgagata tcatccgtga 3900

aggaacatac caaggccacc acacccctat tgtacaaaaa ggcctccgat acggaataat 3960

cctgtttatt gtctccgaag tattcttctt tgccggattt ttctgagcat tttatcattc 4020

cagcctagtt cctacccacg acctaggcgg ttgctgaccc ccaacaggaa tcaccccttt 4080

aaatccccta gaagtacccc ttctaaatac atcagtcctc ttagcatcag gagtctcaat 4140

tacatgagcc catcacagcc taatagaagg caaccgaaac catataaacc aagccctact 4200

aatcaccatt ctcttaggat tatatttcac tatcttacaa gcctcagagt atttcgaaac 4260

atcattttct atctcagacg gaatttacgg ctcaacattc ttcatagcaa cgggatttca 4320

tggccttcac gtaattattg gctcaacttt cctaattgtc tgtctactac gacaactaaa 4380

attccacttc acatcaaaac atcatttcgg atttgaagcc gcagcatgat actgacactt 4440

cgtagatgta gtttgactat tcctatacgt ttctatctat tgatgaggat cctactccct 4500

tagtataaac aatacaactg acttccaatc agttaattct gaaaaaactc agaagagagt 4560

aattaaccta cccattatca tcacaattaa catcacctta tcttttatcc tcatttcaat 4620

tgcattctga ttgcctcaaa taaacttata ctccgaaaaa gcaaacccat atgaatgtgg 4680

cttcgaccca acaagttctg cacgccttcc tttttcaata aaatttttct tagtagccat 4740

tacatttcta ctattcgacc tagaaatcgc cttactactt cccctcccat gagcgattca 4800

aacaaccaat accgctacaa taatagcaac tgcctttatt ttagtcacta ttttagctct 4860

tggcctaagc tacgaatgaa cacaaaaagg actagaatga acagaataat tggtaattag 4920

tttaaataaa attaatgatt tcgactcatt agattatgat aataatcata attaccaaca 4980

atgacatctg ctttcctaaa tttaactata gcctttacat tatctctact aggtactttt 5040

atatttcgct cccacttaat atctactctc ctctgcctag aaggaataat actatcacta 5100

tttgtcataa cttcaacatc cacattaaac tccaactcca taatctccat aaccatccca 5160

attaccattc tagtttttgc agcctgcgaa gtagcagtag gtttagcctt actagtaaaa 5220

atttcaaata cccacggaac agactacgta caaaacctca accttctaca atgttaaaaa 5280

tcattttccc atctatcata ctcctcccac taacatgact ctcagccaac aaaaaaatct 5340

gaaccaatgt cacctcctac agctttctag tgagcctatt aagcttatca ctcctatgac 5400

aaaatgacga aaattaccta aatttctcag ttatattctc ctccgatcca ttatccaccc 5460

cactaatcat tctaacaact tgactcctcc cactaataat gctcgctagc caaaatcaca 5520

taaaaaaaga aaatataatg catcaaaaac tttacatctc aatacttatt agcctccaaa 5580

ttttactcat cataacattc tccgcaacag aactaatttt attttatatc ctgttcgaag 5640

ccactctaat cccaacacta attatcatta cacgatgagg caaccaaaca gaacgcttaa 5700

atgcaggaat ttatttcctg ttttatacac taattggctc catcccactc ttaattgccc 5760

tcatttcaat ccaaaactca ataggaacac tcaacttcct aatcctctcc ctcacaacac 5820

accccctacc ctcaacatga tccaacacca ttctatgact agcatgtata atagcattta 5880

taatcaaaat accattatac ggagtccatc tatgattacc aaaagcccac gtagaagctc 5940

caattgcagg ctctataatt ttagcagcaa ttctcctaaa gctagggggt tatgggataa 6000

tacgagtttc catcattcta gaccccctaa caaaatcttt agcctaccca ttcatcatcc 6060

tctcattatg aggcataatt ataactagct cggtctgcct acgccaaaca gatctaaaat 6120

cattaattgc ttactcatca gtaagccata tagccctagt catcacagcc attataatcc 6180

agacaccatg aagcttcatg ggagctacaa tactaataat cgcccacggc ttaacctcat 6240

cactcttatt ctgcctagca aacaccaact acgaacgaat tcacagccga actataatta 6300

tagctcgagg attacaaata atcttttcat tgatagcaac atgatgatta ttagcaagct 6360

tagccaacct agcactacca cccctaatta acctcatagg cgagttattc attgttatag 6420

caacattttc ctgatcgaac ccctctatca tccttatagc aactaacatt gtcactacag 6480

gaatatactc aatatatatg attatcacaa cccaacgagg aaaactaacc agccacataa 6540

acaacctcca accttcccac acacgagaat taacactcat agctctacac attattcccc 6600

tcatactatt aacaatcaac cctaaactca tcacaggcct aacaatatgt agatatagtt 6660

tacaaaaaac attagactgt gaatctaaca acaggaaatc aaattcctta tttaccaaga 6720

aagtatgcaa gaactgctaa ttcatgcacc catacctaaa acatatggct ttcttacttt 6780

tataggatag aagtaatcca ttggtcttag gaaccaaaaa ccttggtgca actccaaata 6840

aaagtaata 6849

<210> 801

<211> 924

<212> DNA

<213> Rattus norvegicus (Norway rat)

<400> 801
cctggtggtt ccgcagccgt actctcctgg cctggtgtgc acagcctcac catgagttct 60

tctgcagcca gcccgctttt cgcgcctggc gaggactgcg gccccgcgtg gcgcgcggcc 120

cccgcggcct atgatacgtc tgacacgcac ctgcagatcc tgggcaagcc agtaatggag 180

cgttgggaga ccccctacat gcattcgctg gcggctgctg ctgcctccag agggggccgg 240

gtcctggaag tgggctttgg gatggccatt gcagcctcca gggtgcagca ggcccccata 300

aaggaacact ggattattga atgcaacgat ggggtcttcc agcgtctaca aaactgggcc 360

ctgaagcagc cacataaggt tgttcccttg aaaggcctgt gggaggagga ggcacctaca 420

ctgcctgatg gtcactttga tgggattcta tacgacacat atccactgtc tgaagagacc 480

tggcacactc accagttcaa ctttattaag actcatgctt tccgtttgct gaagcctggg 540

ggtatcctca cttactgcaa cctcacgtcc tggggggaac tcatgaagtc caagtacaca 600

gacatcactg ccatgtttga ggagactcag gtgcctgcac tgctggaagc tggcttccag 660

agagaaaaca tctgtacaga ggtgatggcg ctggtgcccc cagccgactg ccgctactat 720

gccttccctc agatgatcac acccctggtc accaagcact gagcggctgg cccagggcta 780

caaggagaat atgtcctcct cagtgccttt gtagctggag tgtggctcca gcctctccac 840

tatccctgca gtgtgacatc ctaacctctg cctggtacgg ccatctcccc agagctcagg 900

agtaaaataa atgctaccaa gact 924

<210> 802

<211> 1444

<212> DNA

<213> Rattus norvegicus (Norway rat)

<400> 802
ccctctagat caggacgtcg ccggggtggc tgtgacttgg ccaagtgctc gcatgagtca 60

aatgacaagg aagagacttc tgccgtggaa cccatgccgc accggccacc tttgccaaga 120

ccgcctgtgc ctttttctct cgcaggtgcg gcggggcata cctgtgatcc cagcaattgg 180

gagactgaga caggaggatc caaccttcaa agctacatgc catggctgcc caggagtccc 240

tgcacgtgaa gaccccacta cgtgacacga tggcattgtc caaagtggcc ggcactagtg 300

tgttccttaa gatggacagc tctcagccct ctggctcctt caagatccga ggcattgggc 360

atctctgcaa gatgaaggca aaacaaggct gtaaacattt cgtctgctct tcagtcgtcc 420

agatttgggg ttccagaatg aggggcagaa gtcactctgg agatgagcag ccccacgtga 480

ggtcccaggc cctccttcct gatacaccct ctccactgac agcgggcaac gcgggcatgg 540

cgactgccta tgctgccagg aggctgggcc tcccagccac tattgttgtg ccaagcacca 600

cacctgccct caccattgag cggctgaaga acgaaggggc cacagttgaa gtggtgggag 660

agatgctgga tgaggccatc caactggcca aggctctgga aaagaacaac caaggttggg 720

tgtacatctc ccccttcgat gaccctctca tctgggaagg ccacacttcc cttgtgaagg 780

agctgaagga gacactgagc gccaagcccg gggccattgt gctgtctgtg ggcggtggag 840

gcctgctgtg cggagtggtc caggggctgc gggaggtggg ctgggaggat gtgcccatca 900

tcgccatgga gaccttcggc gcccacagct tccacgctgc cgtcaaggaa ggaaagctgg 960

tcaccctgcc caagatcacc agtgttgcca aggccttggg tgtgaacact gtgggggcac 1020

agaccctgaa gctgttttac gaacacccca ttttctctga ggtcatctca gaccaggagg 1080

ctgtgactgc tatcgagaag ttcgtagacg atgagaagat cctggtggag cccgcgtgtg 1140

gcgctgccct ggctgcagtg tacagcggtg tggtgtgcag gctgcaggct gagggccgac 1200

tgcaaacccc actggcctcg ctggttgtca ttgtgtgtgg tggcagcaac atcagcctgg 1260

cacagctgca ggcactcaag gcacagctgg gcctgaatga gctactcaag tgatatctgc 1320

tgctgccctg gccaccctga ggggtcacca gcacccctga gtaggctggg tgggcgtccg 1380

cctgacagtg gcccaccctc ctttatccat gtttataata tgcacttttt cattgtaaat 1440

aaaa 1444

<210> 803

<211> 959

<212>  DNA

<213>  Rattus norvegicus (Norway rat)

<400>  803
agagggagca gctttttaac aagagaactc aagcaattgc tgccatgccg gggaagccag      60

tccttcacta cttcgatggc agggggagaa tggagcccat ccggtggctc ctggctgcag     120

ctggagtaga gtttgaagaa caatttctga aaactcggga tgacctggcc aggctaagga     180

atgatgggag tttgatgttc cagcaagtgc ccatggtgga gattgatggg atgaagctgg     240

tgcagaccag agccattctc aactacattg ccaccaaata caacctctat gggaaggaca     300

tgaaggagag agccctcatc gacatgtatg cagaaggagt ggcggatctg gatgaaatag     360

ttctccatta cccttacatt ccccctgggg agaaagaggc aagtcttgcc aaaatcaagg     420

acaaagcaag gaaccgttac tttcctgcct ttgaaaaggt gttgaagagc catggacaag     480

attatctcgt tggcaatagg ctgagcaggg ctgatgttta cctagttcaa gttctctacc     540

atgtggaaga gctggacccc agcgctttgg ccaacttccc tctgctgaag gccctgagaa     600

ccagagtcag caacctcccc acagtgaaga agtttcttca gcctggcagc cagaggaagc     660

cattagagga tgagaaatgt gtagaatctg cagttaagat cttcagttaa ttcaggcatc     720

tatggataca ctgtacccac aaagccagcc ttcgaaagct ttgcaacaat cgcatatttt     780

gactaaatgt tgaccctact tattgggagg ccaacacgtt ttctaatgct tctgtgttaa     840

ttcatataga catgactgat gaggaattgc tgggatgcta tttggttgta gttaaaattt     900

gaaatcatga tcacttcctc agatattact ttgaatctca ataaaaactt cgcaagctt      959

<210>  804

<211>  1606

<212>  DNA

<213>  Rattus norvegicus (Norway rat)

<400>  804
ctgcagtcaa gtgtactggg tgccaagggg acacagcttc cttctaactg gttgagcaag      60

ctgctaaaga aagagttggc cgcccactga ggcagtcatg cagacctgaa gtgacaagca     120

gaagcctggc aggtttgtcc ctgggtagat ggatcgcctg atacggacac tggcgaccaa     180

acgctgggat tggctcaaaa caaggccgtg ttgatccagt gactgggttt tggcgtcctg     240

tcaatctgct gtctgctcaa aagcctagaa atagtgcggg ggcagtttgc tggtctctga     300

gaaaggaagc agccatgcaa gtgctaacga aacgctaccc caagaattgc ctactgaagg     360

tcatggatcg gtactcggct gtggtgcgga acatggagca agtggtgatg atccccagcc     420

ttctgaggga tgtggagctg agtgggtccg gaggctcggt ccaggacgga gcccccgatc     480

tctataccta cttcaccatg ctcaagagca tctgtgtaga agtagaccac gggctgctgc     540

cgcgggagga atggcaggcc aaggtggcag gcaatgaagg cagtgaggct gagaatgagg 600

ctgctgaaac agaggaggct gaggaagaca ggctttcgga ggagctggac ctagaagccc 660

agttccacct gcacttctcc agcctccatc acatccttac ccacctgacc cagaaagcac 720

aggaggtgac gcagaaatac caggaaatga cggggcaggt cctgtaggtc ttcgactcta 780

tggaagatca ctttacacga gagggaagac gatgaggacc cagcggtgca tcagccccga 840

gaccaggaag gaagacctgg acccaggtgg atgcgaaggc ggctcctgtc cctggtgtcc 900

cccacctccc tctgagcgca tctgtggact tgctgcaggc tcacctttaa gatgtctgtt 960

gtgattccca acttctcttc catgtcttag cagtttgagg tttcacaggc agcaagtatc 1020

agtagaaggc agaggtaata gcaagcggcc agagttcaca tcccatccct gggatgtcct 1080

ttagtgcctg gggtcacagt tactctttca tgtataaagc agggacgctg tatttacctc 1140

acaaggttac taaacggaca cggaatcaaa ggcacctgtc acgtgcggat gtctgaacag 1200

ctggtgtttc tcttaccttc ctcagagagc atgctctgtg tagctcttgc ttttgaccct 1260

cacccaggaa aaggttgagg tagttgagtg tgtgggatga gcttctcatc ctgttggatc 1320

tgatgaggct gagcacaagt gaactatgca actattatag tagcaattaa aaaatacatg 1380

gcatgctgat cttttttttt cttcttctta ttgattcttc atgaatttca catcatgacc 1440

cccagtccca ttcatcttct gcctccatct tcttcgtccc tccatatctg ccctccaccc 1500

ttgcaacttc ctccacaaaa gaaaacaaac aaataaacaa aacttcgctg tggaagctac 1560

ggtgtgtcac acagtctacc cttttcccaa acagcttttc ctgcag 1606

<210> 805

<211> 2638

<212> DNA

<213> Rattus norvegicus (Norway rat)

<400> 805
cacaatcttc aatgagtaga catattcctc agttctgtgg tgttctcggt cacacattta 60

tggagtttct gaagggcagt ggagactact gccaggcaca gcacgacctc tatgcagaca 120

agtgaactgt agaaattcat tactactcca ccaagaagcc cccataagag tggatagcct 180

ggacacagtc gtgttgaatt gaaatctgca gagcattttc caagagctca gacctggatg 240

gggtaaacct cagtgcactt cctctgtatc gcctcagtat tcctggattg aagagtcact 300

gcttcttgtg aggaggttca tttcattgcc cgtttctccc gactcatact caaagcactg 360

agaatttcaa gtggagtata ttgaatattg aagtagactt caggttgttt tttggttttg 420

ttttggtttt ttgttttgtt ttgttttgtt ttgttttggt tttcagtttt tgtttggaat 480

catttctgta ttcaattttt taattctttc ataaccctat tgggtgtttt tttaaactaa 540

attaacatgg ctcgaatgaa ccgccctgct cctgtggaag tcacatacaa gaacatgaga 600

tttcttatta cacacaatcc aaccaatgcg accttaaaca aatttataga ggaacttaag 660

aagtatggag ttaccacaat agtaagagta tgcgaagcaa cttacgacac tactcttgtg 720

gagaaagaag gcattcatgt tcttgactgg ccttttgatg atggtgcacc accatccaac 780

cagattgttg atgactggtt aagtcttgtg aagattaagt ttcgtgaaga acctggttgc 840

tgtattgctg tccattgtgt cgcaggcctt ggcagagctc cggtgcttgt tgccctagca 900

ttaattgaag gtggaatgaa atatgaagat gcagtacaat tcataagaca aaagcggcgc 960

ggagctttta acagcaagca acttctgtac ctggagaagt accgtcctaa aatgcggctc 1020

cgcttcaagg attccaacgg tcatagaaac aactgttgta ttcaataaaa ctggggtgcc 1080

tgatgccatt gccttggaag aggaacttca gatgggacct gatttgttat ttacccaatg 1140

tgtccactta cctgtggaag ctccagggga atattgaaaa agttttacca ggccacaagc 1200

ttgacagaat tgcaacctct ataattgggc tatgatcaac acgtttggac acttagcaaa 1260

agatttttgc tggtcagcat ttaaaatgtg cttattattt gtaccaattg acctttccta 1320

aaataaggta ttgagtaatg tcattaaatg tactcctgtg ccagaatatt attagtctat 1380

aaggaattta gaaggattag gtgccaaaat acccagcaca atacttgtat atttttagca 1440

tcatacagaa ccaaaattcc aagaactaag aactctccag accttccatg gtgtattcct 1500

tcagtcattt caaacaccgc agggcttctc ttgttatctg cctgctcact ctatgtttac 1560

atctcccaca cttacaccag aacacatcag gtttgcttag ctatctttta agtcttgcaa 1620

tgattattta atgtctctgt cttattttgt gctgttttgg gaaacctcca tttgaaaatc 1680

aactttgtta cagaagcaca tatcttcaat aatgtctcca gacaaaaagc cttatagtta 1740

atttaatgtt tgcactcggg tgcaacctga cagggagggc ctgaacaaga aaggagagga 1800

ggctattaaa tatttttagt aatatgttgc ctttgtcttg tgcagaacat gtagagtatg 1860

ctctttaatt tagtaaatat ttttaagacg tagagataca ttgttgtagc taaccactta 1920

atcaaaattt ctgaaattct tgtgttttcc atacctatct gaggttttcc aacttgtttg 1980

aattatggtt ttccccttct cttcccaatc tcttgcaaaa aagtaaaagt gggatctgct 2040

agtgaactga gcagaaatat tttatacgcc ttttgagcta tgtaacttaa taattggata 2100

cttgatcatt tgttttatta tgtaatcgat aaaatggtga tgtgtattaa tgttagttca 2160

accatatatt tatactgtct gggaatgtgt ggttatagtt ctgtgggaga aatagtttgt 2220

cagtgttcac cagcttgtaa aaacttagtg cgagagcttc aacatctaaa taaatgatga 2280

aacgcattcg tcactgaggt cactttgctt aaaattaact taatttgtag aaaacagtgg 2340

attcaattat tatcatttca gtttatggac aaatttgtta gggttaccaa gtgcgtttaa 2400

aaattgctct ttaaaggtct agataattgt gaatcaattg aatgttgggt accaagggaa 2460

aacggtttgt aatagttgat gaccttgatt tttaattcaa ttccaccagt cacttgtagc 2520

tttatgcagt ttccaatcca cttttctcat ttttaagttt attacttacc tgtatatatt 2580

ttgaaattaa tttgaacctg cgtatttggc acatgatggc ttataaattt taactttc 2638

<210> 806

<211> 2401

<212> DNA

<213> Rattus norvegicus (Norway rat)

<400> 806
caacatgggg gactctcacg aagacaccag tgccaccatg cctgaggccg tggctgaaga 60

agtgtctcta ttcagcacga cggacatggt tctgttttct ctcatcgtgg gggtcctgac 120

ctactggttc atctttagaa agaagaaaga agagataccg gagttcagca agatccaaac 180

aacggcccca cccgtcaaag agagcagctt cgtggaaaag atgaagaaaa cgggaaggaa 240

cattatcgta ttctatggct cccagacggg aaccgctgag gagtttgcca accggctgtc 300

caaggatgcc caccgctacg ggatgcgggg catgtccgca gaccctgaag agtatgactt 360

ggccgacctg agcagcctgc ctgagatcga caagtccctg gtagtcttct gcatggccac 420

atacggagag ggcgacccca cggacaatgc gcaggacttc tatgactggc tgcaggagac 480

tgacgtggac ctcactgggg tcaagtttgc tgtatttggt cttgggaaca agacctatga 540

gcacttcaat gccatgggca agtatgtgga ccagaggctg gagcagcttg gcgcccagcg 600

catctttgag ttgggccttg gtgatgatga cgggaacttg gaagaggatt tcatcacgtg 660

gagggagcag ttctggccag ctgtgtgcga gttctttggg gtagaagcca ctggggagga 720

gtcgagcatt cgccagtatg agctcgtggt ccacgaagac atggacgtag ccaaggtgta 780

cacgggtgag atgggccgtc tgaagagcta cgagaaccag aaacccccct tcgatgctaa 840

gaatccattc ctggctgctg tcaccgccaa ccggaagctg aaccaaggca ctgagcggca 900

tctaatgcac ctggagttgg acatctcaga ctccaagatc aggtatgaat ctggagatca 960

cgtggctgtg tacccagcca atgactcagc cctggtcaac cagattgggg agatcctggg 1020

agctgacctg gatgtcatca tgtctctaaa caatctcgat gaggagtcaa acaagaagca 1080

tccgttcccc tgccccacca cctaccgcac ggccctcacc tactacctgg acatcactaa 1140

cccgccacgc accaatgtgc tctacgaact ggcacagtac gcctcagagc cctcggagca 1200

ggagcacctg cacaagatgg cgtcatcctc aggcgagggc aaggagctgt acctgagctg 1260

ggtggtggaa gcccggaggc acatcctagc catcctccaa gactacccat cactgcggcc 1320

acccatcgac cacctgtgtg agctgctgcc acgcctgcag gcccgatact actccattgc 1380

ctcatcctcc aaggtccacc ccaactccgt gcacatctgt gccgtggccg tggagtacga 1440

agcgaagtct ggccgagtga acaagggggt ggccactagc tggcttcggg ccaaggaacc 1500

agcaggcgag aatggcggcc gcgccctggt acccatgttc gtgcgcaaat ctcagttccg 1560

cttgcctttc aagtccacca cacctgtcat catggtgggc cccggcactg ggattgcccc 1620

tttcatgggc ttcatccagg aacgagcttg gcttcgagag caaggcaagg aggtgggaga 1680

gacgctgcta tactatggct gccggcgctc ggatgaggac tatctgtacc gtgaagagct 1740

agcccgcttc cacaaggacg gtgccctcac gcagcttaat gtggcctttt cccgggagca 1800

ggcccacaag gtctatgtcc agcaccttct gaagagagac agggaacacc tgtggaagct 1860

gatccacgag ggcggtgccc acatctatgt gtgcggggat gctcgaaata tggccaaaga 1920

tgtgcaaaac acattctatg acattgtggc tgagttcggg cccatggagc acacccaggc 1980

tgtggactat gttaagaagc tgatgaccaa gggccgctac tcactagatg tgtggagcta 2040

ggagctacca ccctcccacc cctcgctccc tgtaatcacc taacttctgc cgacctccac 2100

ctctggtggt tcctgcctgg cctggacaca gggaggccca gggactgact cctcctggcc 2160

tgagtggtgc cctcctgggc ccctaggcag agcccggtcc attgtatcag gcagcccagc 2220

cccagggcac atggcaagag ggactggacc cacctttggg tgatgggtgc cttaggtcct 2280

ctgcagctgt acagaagggg ctcttctctc cacagagctg ggtgcagccc cccacacgtg 2340

attttgaatg agtgtaaata attttaaata acctggccct tggaataaag ttgttttcag 2400

t 2401

<210> 807

<211> 2495

<212> DNA

<213> Rattus norvegicus (Norway rat)

<400> 807
attgcctacc ccgggtggag accgtgctcg tccggccctc ttgcctcacg ttctgcagct 60

ctgcagctcc gcaatcctac accatggcgg acagccggga cccagccagc gaccagatga 120

agcagtggaa ggagcagcgg gcccctcaga aacccgatgt cctgaccacc ggaggcggga 180

acccaatagg agataaactt aatatcatga ctgcggggcc ccgagggccc ctcctcgttc 240

aagatgtggt tttcaccgac gagatggcac actttgacag agagcggatt cctgagagag 300

tggtacatgc aaagggagca ggtgcttttg gatactttga ggtcacccac gatattacca 360

gatactccaa ggcaaaggtg tttgagcata ttgggaagag gactcctatt gccgtccgat 420

tctccacagt cgctggagag tcaggctcag ctgacacagt tcgtgaccct cgtgggtttg 480

cagtgaaatt ctacactgaa gatggtaact gggacctcgt gggaaacaac acccctattt 540

tcttcatcag ggatgccatg ttgtttccat cctttatcca tagccagaag agaaacccac 600

aaactcacct gaaggaccct gacatggtct gggacttctg gagtctttgt ccagagtctc 660

tccatcaggt tactttcttg ttcagcgacc gagggattcc agatggacat cggcacatga 720

atggctatgg ctcacacacc ttcaagctgg ttaatgcgaa tggagaggca gtgtactgca 780

agttccatta caagactgac cagggcatca aaaacttgcc tgttgaagag gcaggaagac 840

ttgcacagga agacccggat tatggcctcc gagatctttt caatgccatc gccagtggca 900

attacccatc ctggactttt tacatccagg tcatgacttt caaggaggca gaaaccttcc 960

catttaatcc atttgacctg accaaggttt ggcctcacaa ggactaccct cttataccag 1020

ttggcaaact ggtcttaaac agaaatcctg ctaattattt tgctgaagtt gaacagatgg 1080

cttttgaccc aagcaacatg ccccctggca ttgagcccag cccggacaag atgctccagg 1140

gccgcctttt tgcttaccca gacactcacc gccaccgcct gggaccaaac tatctgcaga 1200

tacctgtgaa ctgtccctac cgtgctcgcg tggccaacta ccagcgcgat ggccccatgt 1260

gcatgcatga caaccagggt ggtgctccca actactaccc caacagcttc agcgcaccag 1320

agcagcaggg ctcggccctg gagcaccata gccagtgctc tgcagatgtg aagcgcttca 1380

acagtgctaa tgaagacaac gtcactcagg tgcggacatt ctatacgaag gtgttgaatg 1440

aggaggagag gaaacgcctg tgtgagaaca ttgccaacca cctgaaagat gctcagcttt 1500

tcattcagag gaaagcggtc aagaatttca ctgacgtcca ccctgactac ggggcccgag 1560

tccaggctct tctggaccag tacaactccc agaagcctaa gaatgcaatt cacacctacg 1620

tacaggccgg ctctcacata gctgccaagg gaaaagctaa cctgtaaagc acgggtgctc 1680

agcctcctca gcctgcactg aggagatccc tcatgaagca gggcacaagc ctcaccagta 1740

atcatcgctg gatggagtct cccctgctga agcgcagact cacgctgacg tctttaaaac 1800

gataatccaa gcttctagag tgaatgatag ccatgctttt gatgacattt cccgaggggg 1860

aaattaaaga ttagggctta gcaatcactt aacagaaaca tggatctgct taggacttct 1920

gtttggatta ttcatttaaa atgattacaa gaaaggtttt ctagccagaa acatgatttg 1980

attagatatg atatatgata aaatcttggt gattttacta tagtcttatg ttacctcaca 2040

gcctggtata tatacaacac acacacacac acacacacac acacaccaaa acacacatac 2100

actatacaca cacacacaca cacacactaa aacacacata cacaacacac acatacacta 2160

cacacacaga acacacaaca caaacataca cacataggca cacacacaca cacacacaca 2220

cacacacaca cacacacaca cacacatgaa tgaagggatt ataaagatgg cccacccaga 2280

attttttttt atttttctaa ggtccttata agaaaaacca tacttggatc atgtcttcca 2340

aaaataactt tagcactgtt gaaacttaat gtttattcct gtgtagttga ttggattcct 2400

tttccccttg aaattatgtt tatgctgata cacagtgatt tcacataggg tgatttgtat 2460

ttgcttacat ttttacaata aaatgatctt catgg 2495

<210> 808

<211> 8048

<212> DNA

<213> Rattus norvegicus (Norway rat)

<220>

<221>  misc_feature

<222>  (2005)..(2005)

<223>  n


<220>

<221>  misc_feature

<222>  (3503)..(3503)

<223>  n


<400>  808
gaattcggcc caggataaac agatatatag gtaaatgact ggatagatac atggatttga      60

tctctaactc aacttaggca ccatagaaaa caatccttga attattttaa taattttaaa     120

tcctagttta ctgagaaaca cgttgacgag gcagggcttc agcctctctg aagcagcact     180

gcccctctgt ggccacacag gaagtcttca ctcagcagtt tcctgactta aggcctaggc     240

ctgaggaaat gcattgaata tttctctggg ggacaatagt tctgagaagg aaagagagtt     300

tgtctatgtt gaggtcagtt aaggatggtt ttctttcctt ccctcccagc cttttctcca     360

tgcaacatca tgagtcccca tctttctatc ctgcctaaaa ccacttcctt ctgggaaatg     420

agaaaatcca ctcatacaaa atggaaacat cccgaggatc ccggcccgca gcagctctct     480

gcccccagac cctgtgagag agagacccaa ccgcctggtc aggtgggcac tcctgaggct     540

gcagagggaa gagaccacca acactgctca cccctgccca catccctggc ccaagaggaa     600

actgtataag gcctctgggt cctgtggggg agggcccagg agcggcagga cccctgtgcc     660

tgagagacca ccaccagaac cagaaggaaa cagaccggat aaacagttct ctgcaccaaa     720

tcccgtggag ggagagctga accttcagag agaaagacaa gcctgggaaa ccagaagaga     780

ctgctctctg cacacacatc tcggacgcca gaggaaaaag ccaaagacca tctggaaccc     840

tggtgcactg aagctcccgg aaatggtgca caggtcttcc tggttgctgc cgctgcagag     900

agcccgtggt agcaccccac gagcgaactt gagcctcagg accacaggta agaccaactt     960

ttctgctgca agaaagctgc ctggtgaact caagacacag gcccacagga acagctgaag    1020

acctgtagag aggaaaaacc acacgccgga aagcagaaca ctctgtcccc ataactgact    1080

gaaagagagg aaaacaggtc tacagcactc ctgacacaca ggcttatagg acagtctagc    1140

cactgtcaga aatagcagaa caaagtaaca ctagagataa tctgatggcg aaaggcaagc    1200

gcaggaaccc aagcaacaga aaccaagact acatggcacc atcggagccc aattctccca    1260

tcaaaacaaa catggaatat ccaaacacac cagaaaagca agatctagtt ccaaaatcat    1320

ttttgatcat gatgctggag gacttcaaga aagacgtgaa gaactcctta gagaacaagt    1380

agaagcctac agagaggaat cgcaaaaatg cctgaaagaa tcgcaaaaat ccctgaaaga    1440

attccaagaa aacataaata aacaagtaga agcccataga gaggagacac aaaaatccct    1500

gaaagaattc caggaaaaca taaataaaca agtagaagcc catagagagg agacacaaaa 1560
atccctgaaa gaattccagg aaaacataaa taaacaagta gaagcccata gagaggagac 1620
acaaaaatcc ctgaaaggaa ttccaggaaa acacaatcaa acagttgaag gaattaaaaa 1680
tggaaataga agcaatcaaa aaagaacaca tggaaacaac cctggatata gaaaaccaaa 1740
agaagagaca aggagctgta gataaaagct tcaccaacag aatacaagag atggaagaga 1800
gaatctcagg agcagaagat tccatagaaa tcattgactc aactgtcaaa gataatgtaa 1860
agcggaaaaa gctactggtc caaaacatac aggaaatcca ggactcaatg agaagatcaa 1920
acctaaggat aataggtata gaagagagtg aagactccca gctcaaagga ccagtaaata 1980
tcttcaacaa aaccatagaa gaaancttcc ctaacctaaa aaaagagata cccatagaca 2040
cacaagaagc ctacagaact ccaaatagat tggaccagaa aagaaacacc tcccgtcaca 2100
taattgtcaa aacaccaaac gcacaaaata aagaaagaat attaaaaaca gtaagggaaa 2160
aaggtcaagt aacatataaa gggagaccta tcagaatcac accagacttc tcgccagaaa 2220
ctatgaaggc cagaagatcc tggactgatg ttatacagac cctaagagaa cacaaatgcc 2280
agcccaggtt actgtatcca gcaaaactct caattaacat tgatggagaa accaagacat 2340
tccatgacaa aaccaaattt acacaatatc tttccacaaa tccagcacta caaaggataa 2400
taaatggtaa agcccaacat aaggaggcaa gctataccct agaagaagca agaaactaat 2460
cgtcttggca acaaaacaaa gagaatgaaa gcacacaaac ataacctcac atccaaatat 2520
gaatataacg ggaagcaata atcactattc cttaatatct ctcaacatca atggcctcaa 2580
ctccccaata aaaagtcata gattaacaaa ctggatacac aacgaggacc ctgcattctg 2640
ctgcctacag gaaacacacc tcagagacaa agacagacac tacctcagag tgaaaggctg 2700
gaaaacaaat ttccaagcaa atggtcagaa gaagcaagct ggagtagcca ttctaatatc 2760
aaataaaatc aatttccaac taaaagtcat caaaaaagat aaggaaggac acttcatatt 2820
catcaaagga aaaatccacc aagatgaact ctcaatccta aatatctatg ccccaaatac 2880
aagggcacct acatacgtaa aagaaacctt actaaagctc aaagcacaca ttgcacctca 2940
cacaataata gtgggagatt tcaacacacc actctcatca atggacagat catggaaaca 3000
gaaattaaac agtgatgtcg acagcataag agaagtcatg agccaaatgg acttaacgga 3060
tatttttaga acattctatc ctaaagcaaa aggatatacc ttcttctcag ctcctcatgg 3120
cactttctcc aaaattgacc atataattgg tcaaaaaacg ggcctcaaca ggtacagaaa 3180
gatagaaata atcccatgcg tgctatcgga ccaccacggc ctaaaactgg tcttcaataa 3240
caataaggga agaatgccca catatacctg gaaattgaac aatgctctac tcaatgataa 3300
cctggtcaag gaagaaataa agaaagaaat taaaaacttt ttagaattta atgaaaatga 3360
aggtacaaca tacccaaact tatgggacac aatgaaagct gtgctaagag gaaaactcat 3420
agcgctgagt gcctgcaaaa agaaacagga aagagcatat gtcagcagct tgacagcaca 3480
cctaaaagct ctagaacaaa aangaagcaa atacactgag gaggagtaga aggcaggaaa 3540

taatcaaact cagagctgaa atcaaccaag tagaaacaaa aaggaccata gaaagaatca 3600

acagaaccaa aagctggttc tttgagaaaa tcaacaagat agataaaccc ttagccagac 3660

taacgagagg acacagagag tgtgtccaaa ttaacaaaat cagaaatgaa aagggagaca 3720

taactacaga ttcagaggaa attcaaaaaa tcatcagatc ttactataaa aacctatatt 3780

caataaaact tgaaaatctt caggaaatgg acaatttcct agacagatac cacgtatcga 3840

agttaaatca ggaacagata aaccagttaa acaaccccat aactcctaag gaaatagaag 3900

cagtcattaa aggtctccca accaaaaaga gtccaggtcc agacgggttt agtgcagaat 3960

tctatcaaac cttcatagaa gacctcatac caatattatc caaactattc cacaaaattg 4020

aaacagatgg agcactaccg aattccttct acgaagccac aattactctt atacctaaac 4080

cacacaaaga cacaacaaag aaagagaact tcagaccaat ttcccttatg aatatcgatg 4140

caaaaatact caataaaatt ctggcaaacg aattcaagag cacatcaaaa caatcatcca 4200

ccatgatcaa gtaggcttca tcccaggcat gcagggatgg tttaatatac ggaaaaccat 4260

caacgtgatc cattatataa acaaactgaa agaacagaac cacatgatca tttcattaga 4320

tgctgagaaa gcatttgaca aaattcaaca cccttcatga taagagtccc ggaaagaata 4380

ggaattcaag gcccatacct aaacatagta aaagccatat acagcaaacc agttgctaac 4440

attaaactaa atggagagaa acttgaagca atcccactaa aatcagggac tagacaaggc 4500

tgcccactct ctccctactt attcaatata gttcttgaag ttctagccag accaatcaga 4560

caacaaaagg agatcaaggg gatacagatc ggaaaagaag aagtcaaaat atcactattt 4620

gcagatgaca tgatagtata tttaagtgat cccaaaagtt ccaccagaga acgactaaag 4680

ctgataaaca acttcagcaa agtggctggg tataaaatta actcaaataa atcagttgcc 4740

ttcctctata caaaagagaa acaagccgag aaagaaatta gggaaacgac acccttcata 4800

atagacccaa ataatataaa gtacctcggt gtgactttaa ccaagcaagt aaaagatctg 4860

tacaataaga acttcaagac actgaggaaa gaaattgaag aagacctcag aagatggaaa 4920

gatctcccat gctcatggat tggcaggatt aatatagtaa aaatggccat tttacccaaa 4980

gcaatctaca gattcaatgc aatccccatc aaaataccaa tccaattctt caaagagtta 5040

gacagaacaa tttgcaaatt catctggaat aacaaaaaac ccaggatagc taaagctatc 5100

ctcaacaata aaaggacttc agggggaatc actatccctg aactcaagca gtattacaga 5160

gcaatagtga taaaaactgc atggtgattg gtacagagac agacagatag accaatggaa 5220

tagaactgaa gacccagaaa tgaacccaca cacctatggt cacttgattt ttgacaaagg 5280

agccaaaacc atccaatgga aaaaagatag cattttcagc aaatggtgct ggttcaactg 5340

gagggcaaca tgtagaagaa tgcagatcga tccatcctta tcaccctgta caaagcttaa 5400

gtccaagtgg atcaaggacc tccacatcaa accagacaca ctcaaactaa tagaagaaaa 5460

actagggaag catctggaac acatgagcac tggaaaaaat ttcctgaaca aaacaccaat 5520

ggcttatgct ctaagatcaa gaatcgacaa atgggatctc ataaaactgc aaagcttctg 5580

taaggcaaag gacactgtgg ttaggacaaa acggcaacca acagattggg aaaagatctt 5640

taccaatcct acaacagata gaggccttat atccaaaata tacaaagaac tcaagaagtt 5700

agaccgcagg aaacaaataa ccctattaaa aatggggttc agagctaaac aaaaattcac 5760

agctgaggaa tgccgaatgg ctgagaaaca cctaaagaaa tgttcaacat ctttagtcat 5820

aagggaaatg caaatcaaaa caaccctgag atttcacctc acagtagtga gaatggctaa 5880

gatcaaaaac tcaggtgaca gcagatgctg gcgaggatgt ggagaaagag gaacactcct 5940

ccattgttgg tgggattgca gactggtaaa accattctgg aaatcagtct ggaggttcct 6000

cagaaaattg gacattgaac tgcctgagga tccagctata cctctcttgg gcatataccc 6060

aaaagatgcc tcaacatata aaagagacac gtgctccact atgttcattg ccgccatatt 6120

tataatagcc agaaactgga aagaacccag atgcccttca acagaggaat ggatacagaa 6180

aatgtggtac atctacacaa tggaatatta ctcagctatc aaaaacaacg agtttatgaa 6240

attcgtaggc aaatggttgg aactggaaaa tatcatcctg agtgagctaa cccactcaca 6300

gaaagacata catggtatgc actcattgat aagtggctat tagcccaaat gcttgaatta 6360

ccctagatcc ctagaacaaa cgaaactcaa gacggatgat caaaatgtga atgcttcact 6420

ccttctttaa atgaggaaaa agaataccct tggctgggaa gggagaggca aagattaaaa 6480

cagagactga aggaacaccc attcagagcc tgccccacag gtggcccata catatacagc 6540

cacccaattg gacaagatgg atgaagcaaa gaagtgcaga ctgacaggag ccggatgtag 6600

atcgctcctg agagacacag ccagaataca gcaaatacag aggcgaatgc cagcagcaaa 6660

ccactgaact gagaataggt cccccgttga aggaatcaga gaaagaactg gaagagcttg 6720

aaggggctca agaccccaaa agtacaacaa tgtcaagcaa ccagagcttc cagggactaa 6780

gccactacct aaatactata catggactga ccctggactc tgaccccata ggtagcaatg 6840

aatatcctag taagagcacc agtggaaggg gaagccctgg gtcctgctaa gactggaccc 6900

ccagtgaact agactatggg gggagggcgg caatgggggg agggttggga gggggacacc 6960

cataaggaag gggagggggg agggggatgt ttacccggaa accgaaaggg aataacactt 7020

aaattatata agaaatactc aagttaatta aaaataataa taataataat aaaaagaatt 7080

actcataaaa taaagacaag atgatggata aaattaaaaa aacaaaaaaa acaaaaaaaa 7140

accaaaatgg aaacatgaaa atgctcacta cttacaatag tgctttatgt aataagcaaa 7200

tgactgcatt tgacacgagt tataatttat taacccctta gttagactac ttgtacacat 7260

ggtgattatt aatccacaat tattcttttt cactaaaatc cttatatttt attctttgag 7320

attataatat aatcacacaa attccctctt ccctgtcatc tcttcaaaac cttccatata 7380

gtcctccatg ctgtcttttg aacgttatgg cttctttttt atattcttgt tttatattat 7440

atatatataa tatatattat aatatatatc atactatata tattcctcag gactgaccaa 7500

tccatattgg ataaccaatt ggtgtgctcg tctcagggaa agaccatttc cccaccatca 7560

gcattctcgg tttccagtag ttcattgtct agaatggaga ctgtgtgtgc tttccccgtc 7620

cactttagct gctgtgtttc agctcctctt gaggcagtta tgttggtgag acctcatctg 7680

tgcggcttcc aacattcctc agagacacag tgagttacac agtgtcacct gatcctctaa 7740

ctcccaccat ctttcctgct gtcttctcca gggtttcctg aacctcagca ggagcagggg 7800

ttgtactgta gacacgccca ccttcactag gatccacagc tcctcacttt gatttgctgt 7860

ggttttctgc aatggcttcc atgtcctagt aaagtcatgg agaccccata accacgctgt 7920

actaaaccag acaatcccta gctgcattct aagttcttgt ccttatccca tacatgtgca 7980

tttagagagt ctgcttattt tgctcattat agaacgagtc aatcattctc tttgagttgt 8040

aaaagctt 8048

<210> 809

<211> 1228

<212> DNA

<213> Rattus sp.

<400> 809
ctgtgattga taacggggag aagttttctg gtagaggaag ctatccaatg attgaaaatg 60

ttactaaagg ttttggcatt gtttttagca atggaaacag atggaaagag atgagacgat 120

tcacaatcat gacctttcgg aatttgggaa ttggaaaaag gaacattgag gaccgtgttc 180

aagaggaagc acagtgccta gtggaagaac tgaggaaaac aaaaggctca ccctgtgacc 240

cctccttgat cctgaactgt gctccatgca atgtcatctg ctccattact ttccagaatc 300

attttgatta taaagataag gaaatgctta cgttcatgga aaaagtgaac gagaatctca 360

agattatgag ctccccatgg atgcaggtct gcaatagttt tccttcactc attgactatt 420

ttcctggaac tcatcacaaa atagcaaaaa atattaatta tatgaaaagc tatttgttga 480

agaaaataga agaacaccag gaatcattgg atgttacaaa ccctcgtgat tttgttgatt 540

attacctgat taaacaaaaa caggcaaaca acatcgaaca atcagaatat tcacatgaaa 600

atctgacatg cagtatcatg gatctcattg gtgcagggac agagacaatg agcacaacat 660

tgagatatgc tctcctgctt ctgatgaagt acccacatgt cacagctaaa gtccaggaag 720

agattgaccg tgtgattggc agacatgcga gcccctgcat gcaggataga aaacacatgc 780

cctacacaga tgccatgatt catgaggtac agagattcat taactttgtc ccgaccaacc 840

tgccccatgc agtgacctgt gacattaaat tcaggaacta cctcatcccg agaagggcaa 900

aagtgttaac atcactgaca tcagtgctgc atgacagcaa ggagttcccc aacccagaga 960

tgtttgtacc ctggccattt ctagatgaga atggaaactt taagaaaagt gactactttt 1020

tgcctttctc agcaggaaaa cgagcttgtg ttggagaggg cctggcccgc atgcagttgt 1080

ttctattctt gacaaccatt ttacagaact ttaacctgaa atctctggtt cacccaaagg 1140

acattgatac gatggcagtt ctgaatggtt ttgcctctct gccacccact taccagctct 1200

gcttcattcc ttcctgaata gatcaggc                                          1228

<210>  810

<211>  1292

<212>  DNA

<213>  Rattus norvegicus (Norway rat)

<400>  810
cgtagacttc ttccctcctg cagcgaagga cagcatgagc acagctggaa aagtaatcaa      60

atgcaaagcg gccgtgctat gggagcctca caagcccttc accatcgagg acatagaagt     120

cgcacccccc aaggcccatg aagttcgcat taagatggtg gccaccggag tctgccgctc     180

agatgatcac gcggttagtg gatccctgtt cacgcctctt cctgcagttc taggccacga     240

gggagctggc attgttgaga gcattggaga aggggtgact tgtgtgaaac caggtgataa     300

agtcatcccg ctgttctctc cccagtgtgg aaaatgcagg atctgcaagc acccggaaag     360

caacctctgt tgccaaacta agaatctgac acagcctaag ggagctttgc tcgacggcac     420

cagcagattc tcctgcaggg gaaagcccat tcaccacttt ctcagcacca gcaccttctc     480

ccagtacact gtggtagatg acatagcggt ggccaaaatc gatgcggctg caccactgga     540

caaagtctgc ctcatcggct gtggcttctc gactggctat ggctctgccg tccaagtcgc     600

caaggtgacc ccaggctcca cctgtgccgt gtttggcctg ggaggtgttg gtctgtctgt     660

cgtcattggc tgtaaaacag caggagcagc caagatcatt gccgtggaca tcaacaaaga     720

caagtttgcg aaggccaaag agttaggtgc cactgactgt atcaaccctc aagactacac     780

caaacccatc caggaagttc tccaggagat gactgatgga ggggtggact tttcatttga     840

agtcattggc cgtcttgaca ccatgacttc tgccctgtta agctgccatt cagcatgcgg     900

tgtaagcgtc attgtcgggg tgcctcccag tgcccaaagc ctctccgtta accccatgtc     960

gctgctgctg ggacgcacct ggaaaggagc aatattcggc gggtttaaga gtaaagatgc    1020

cgtccccaaa cttgtcgctg acttcatggc taagaagttt ccgttggagc cgctgattac    1080

tcatgtttta ccttttgaaa agataaatga agcatttgac ctgctccgtg ctggaaagag    1140

tatccgtacc gtcctgacgt tctgagaaca catgaatgcc ttcccggttt ctgactcctc    1200

cgcctaccga atcaagcgcc agccacatca caaccttaac cttgctcttc agagacacac    1260

agccaataaa gcacctgtgc aagctttccg tg                                  1292

<210>  811

<211>  1780

<212>  DNA

<213>  Rattus norvegicus (Norway rat)

<400> 811
tctcttgctg tttgcccaat ttggggacac ccatcctcac caccagtggt ggacaccaca 60
aaaggcaaag tcctggggaa gtatgtcagc ttagaaggat ttacacagcc tgtggccgtc 120
ttcctgggag tcccttttgc caagcctcct cttggatctc tgaggtttgc tccaccagag 180
cctgcagagc cctggagctt cgtgaagaac accaccacct acccgcctat gtgctcccaa 240
gatggagttg tgggaaagtt actcgcagat atgttgagca ccggaaaaga gagtatacct 300
ctcgagtttt ccgaagactg tctctacctg aatatttaca gtcctgctga cttgacaaaa 360
aacagccgat tgcccgtgat ggtgtggatc catggaggtg gactaataat aggcggagca 420
tcaccctata gtggactagc tctctctgcc cacgaaaacg tggtggtggt aaccattcaa 480
taccgcctgg gatttggggg attgtttagc accggtgatg aacacagccg ggggaactgg 540
gctcacttgg accagctggc tgcactacgc tgggtccagg ataacattgc aaactttgga 600
gggaacccgg attcagtgac catctttgga gagtcagcag gaggtgtcag tgtctctgct 660
cttgtcttat ctcctctggc caagaacctc ttccacagag ccatttctga gagtggtgtg 720
gtcctcacta caaacctgga caagaagaat actcaggctg tggctcaaat gattgctact 780
ctttctgggt gtaataacac ctcatcagcc gccatggttc agtgcttgcg ccagaagaca 840
gaggctgagc tcttggagct tacagtgaaa ctggacaata cctccatgtc cactgtgatt 900
gatggagtgg tactgccaaa gacaccggaa gagatcctga ctgagaagag tttcaacacg 960
gtcccctaca tagtgggctt caacaagcaa gagtttggct ggatcattcc aacgatgatg 1020
ggaaatctac tctctgaagg cagaatgaat gagaaaatgg ccagttcttt cttgaagagg 1080
ttcagcccta accttaacat ctctgagagt gtgattccag caatcattga gaagtactta 1140
agaggaacag atgaccctgc caaaaagaag gaacttctcc tggacatgtt ttcagatgtc 1200
tttttcggta tcccagctgt actcatgtcc cgtagcctca gagatgccgg agcgcccacc 1260
tacatgtatg agtttcagta tcgcccaagc ttcgtgtctg accagagacc ccagacagtt 1320
cagggagacc atggtgatga aatcttctct gtatttggga ctccattttt aaaagagggc 1380
gcctcagaag aggagaccaa tcttagcaag ttggtgatga agttctgggc caactttgct 1440
cgaaatggga acccaaatgg agaggggctg cctcactggc caaaatatga tcagaaggaa 1500
gggtatttgc agattggtgc caccacccag caagcccaga aactgaaagg agaagaagtg 1560
gctttctgga ctgagctctt ggctaagaat ccacctcaga cagaacacac tgaacacaca 1620
taaatgggtc tctgccagcc tcagtaccag aggagcaaag atggggctat tccacaaagg 1680
atggctattt gccaaagatg aatcttacag aggaaactgc acaattgtac agttggggta 1740
tttgtatctc aactttgaaa taaatttcct tttagaaagc 1780

<210> 812
<211> 1590

<212>  DNA

<213>  Rattus norvegicus (Norway rat)

<400>  812
ggatctcaga tggaggcgat ggcaccgcgc acgctgctcc tgctgctggc ggccgccctg      60

gccccgaccc agacccgcgc gggctcacac tcgctgcggt atttctacac cgccgtgtcc     120

cggcccggcc tcggggagcc ccggttcatc gctgtcggct acgtggacga cacggagttc     180

gtgcgcttcg acagcgacgc ggagaatccg aggatggagc cgcgggcgcg gtggatggag     240

cgggaggggc cggagtattg ggagcagcag acacggatcg ccaaggaatg ggagcagatt     300

taccgagtgg acctgaggac cctgcgcggc tactacaacc agagcgaggg cggctctcac     360

accatccagg agatgtatgg ctgtgacgtg gggtcggacg ggagcctcct ccgcggatat     420

aggcaggacg cctacgatgg ccgcgattac atcgccctga acgaagacct gaagacgtgg     480

acggcggcgg actttgcggc acagatcacc cggaacaagt gggagcgggc tcgttatgca     540

gagagactca gggcctacct ggagggcacg tgtgtggagt ggctcagcag atacctggag     600

ctcgggaagg agacactgct gcgctcagat cccccagagg cacatgtgac ccttcacccc     660

agacctgaag gtgatgtcac cctgaggtgc tgggccctgg gcttctaccc tgctgacatc     720

accctgacct ggcagttgaa tggggaggac ctgacccagg acatggagct tgtggagacc     780

aggcctgcag gggatggaac cttccagaag tgggcatctg tggtggtgcc tcttgggaag     840

gagcagaatt acacatgccg tgtggagcat gaggggctgc ctaagccact ttcccagaga     900

tgggaacctt ctccatcgac tgactccaac atggaaacca ctgtcattta tgtcatcctt     960

ggagctgtgg ccatgattgg agctgtggcc atcataggag ctatggtggc tgttgtgagg    1020

aggaggaaga gaaacacagg tgggaaagga ggagactacg cccctgctcc aggcagggac    1080

agctcccaga gctccgatgt gtctctccca gattgtaaag catgaagaca gctgcctgga    1140

gtggactgag tgacagacga tgtgttcagg tctctcctgt gacatccaga gccctcagtt    1200

ctctttagac accagtgtct gaagttccct gtgattctat ggcttcagtg tgaagaactg    1260

tggagcccag cctgccctgc acttcaggac cctgtccctt cactgcccgc tttcccttcc    1320

acattcagcc ttgctggtcc agcctggggg tggagggtgg ggacatctgc atcctgtccc    1380

cctgtgctac cctgagcttc agcccctcac ttccaccctg agaataagaa tctgagtgtg    1440

aacttgattg ttcacatcct tgacacaagt gtagatggct ttttaaatta ctggattgag    1500

aatacttaga ggttttttgt tttgggtttt gctttgtttg attttgtttt tgaaaaaaat    1560

aaatggcaga tggagaagct tccaaaaaaa                                      1590

<210>  813

<211>  451

<212>  DNA

<213>  Rattus norvegicus (Norway rat)

<400>  813
tcctctttct ctcgaaccgg gcggcctcat ccgcgaacag cgcagcaatg gccaagatta      60

aggctcggga cctgcgcggc aagaagaagg aggagctgtt gaaacaactg gatgatctga     120

aggtggaact gtcccagctt cgcgtggcca aagtgacagg cggcgccgcg tccaagctct     180

ccaagatacg agtcgtacgc aaatccatcg cccgtgtcct cactgtcatt aatcagactc     240

aaaaggaaaa cctcaggaaa ttctacaagg gaaagaagta caagcccctg gacctgcgac     300

ccaagaagac aagagccatg cgccgccggc tcaccaagca tgaagagaag ctgaagacca     360

agaagcagca gcggaaggag cggctgtacc cactgcgcaa gtacgcagtc aaggcctgag     420

acgacgacaa taacaataaa gtccaaaact g                                    451

<210>  814

<211>  16299

<212>  DNA

<213>  Rattus norvegicus (Norway rat)

<220>

<221>  misc_feature

<222>  (1292)..(1292)

<223>  n

<220>

<221>  misc_feature

<222>  (1589)..(1589)

<223>  n

<220>

<221>  misc_feature

<222>  (12941)..(12941)

<223>  n

<400>  814
attcaattga tatagtcccg ttgaagacac actttattca acctctcaaa agtccaagac      60

aataaagtct caaaccaaaa gaaatatgcc aagtctcata taagcatggg cttggtagaa     120

tattattaga gtctacagca gaaacttttc ataaatggac taaactttcc aagcgttagg     180

ttgactgaat ggattttttt aatgaaattc aactgtatgc tatctacaaa aaaaatctca 240

caagaaggtg ctaagggatg gaaagaaacc cactgattcc aggaaagcaa aacaagcacc 300

agagagagcc cttggcatcc caaggcatgg actgcccgtg agactggcct acctagaagg 360

agaagagcag cagtgcctag atgctttcta gtctgaatta gcaaccagcc accatccatt 420

tggcatggac aattcagaag aaactatgta ggtgctgggt ctgctgtgga aactcaagga 480

cctcacaaga gcaacaaata aagccacaga cataagctat gcagcttggg aaagagccag 540

tgtgtgagga cccccatatc acacaagact ttaacaagag atatttattt ttatatgaca 600

accttgtttg atttttaata attaaatgtt tcaagaaaag ataaaaagat taatggatga 660

atatttaagg aaattatgat tattctgctt aacttatatg tgcagtaccc aggtgttgaa 720

atatcacact gtatcctata agtgtatacg ccaaatacaa tgaaaaaggg aaataaaagc 780

caaacaagtc aatgttcttg actagcacaa agagaggaaa atgggtgcag gtctggctat 840

tagccactgt cggctggaaa aaaaaaaatc atgtctctga ttcccaaggt aaatcattcc 900

tgccaactga actttgagat caagattggt ctgcctcacc tttctccctc ccacaggtag 960

gcgggcaatc cttacaggat ttaggcaaga ggcagaaagg atctgtgaga gtgagggcag 1020

ctactgagga gtggctgcac catgagcgtc tctgcactga gctccacccg cttcacgggc 1080

agcatctctg gcttcctcca agtggcctcc gtgcttggtc tgcttctgct gctggtcaaa 1140

gcagtccagt tctacctgca aaggcaatgg ctactcaagg ctttccagca gttcccatca 1200

cctcccttcc actggttctt tgggcacaag gtatgaagga aagggaagga tgggtgggaa 1260

agcagggaag atgggtgtct cagagactgg tnatcgaaat cacaggacaa agcctgatgt 1320

ataaaaattc caagtacctc atgagatacc cagctcaggc tgaggcacac catccaacga 1380

tagggttatt caaccagtct ggctgttgct caactctgta ctgatagccc cacactttga 1440

aagctttagt tggtcttacc tagtctcctc ctcttctggc tgagtacctt taatatctct 1500

gctgttctca aacaactttt aagtagttcc ttacctagtg agttacctac cgctattctc 1560

tcccaagctc agggcttgcc agggaatgna tcagtgagtt gaaaatcttg cccctgtggg 1620

ttcttggtag agcagtagtg cacatcttag aagataccag gcacaggaag ggttcacatt 1680

gctgggatca gtgggtggct gccccagact aggagtattt tcaggattgg tgaaggtcca 1740

aattgaaatc ccaaagtcat ggcacattaa aggaaaaggc tggggagggt cctcagatga 1800

cagcactaat gttagaattc ccacagggtc atttagctct cccttctcct tcccacacac 1860

cctgcactgt aagctactta gattgaattt atttccttgt accacagagt tcagctcaag 1920

agccaggaac agattataaa attccctaga aagggatagc agcctgtagg ctaggaaggc 1980

tgtaggccac tgacccattg cactgtcacc ccagggaata atgtacagag ccaatgaggc 2040

tacatagaaa ggcgttcaac aaaggattgt gcaatagatg ctcttagaaa gacctgggta 2100

tgcagtgttt tgtgtgcaga tctgagaatc tgaccctgtg gctcctctga ggaacacagc 2160

tccaaccacc aggactctcg aatctccctc aggatcagcc ctcgtacagc aaagctatga 2220

gtaatagcac agataattca tcgaactgcc tcaccctcct gacagcttcg gaaaaatcca 2280

aaactatgtt cctgtgtttg gtctcctctt gtctccagtc caaaaaataa tcaagagtcc 2340

cactaccttc tttttccctc aacagcagtt tcaaggtgac aaagaactac agcaaattat 2400

gacatgtgtg gagaatttcc caagtgcctt tcctcgatgg ttctggggaa gcaaagccta 2460

cttaattgtc tatgaccctg actacatgaa ggtgattctc gggcgatcag gtgagcgtga 2520

cacgctgttg tgaagacata agcacattcc tccttggtac ttgccctagg ctgtcaaatt 2580

ccagaagagc gtgaccctcc acactctacc attcttccag actacaaccc agcacagggc 2640

caacaatact cagaactgaa aagtcaaatc tcttagccta atgtctttgt ctttctttat 2700

tatttattca aattaagatg gtaagtctag ttaattgttt caggtttgaa atctctaatt 2760

gttacctgcc tgactcatag aataacgaag ccaccacaaa cacaccaaca cacacacaca 2820

cacacacaca cacacacaca cacacacaca cacacaccac ttgttccatt ctttgacttt 2880

gatctcatgg actgtttctc accagctcag catctcagcc atggaggagt ccagctacta 2940

gaacacgaat cctaaccttc ctaagcctga atgttaggac agagtttgaa gggaatatga 3000

gaaattgggc agtgagttca gacagtttgc caacagacag cacttaaaac atcatagaaa 3060

ctcgtgcctt taaccacagt tgccttgggg ctgcctttta atcccttttc ttgttaagtc 3120

attgtttccc tttcagatcc aaaggccaat ggcgtctaca gattgctagc tccttggatc 3180

ggtatgtatg tctaagttgg gaatgtggtc tacttctaat tggattttcc gagtgctgtg 3240

cctctggttc acagagaata aatgtgacag actgccacta tcatggtcta cccccaaatc 3300

tctacttctg acccttcact tctccaaaca gtcctatctg aacatcagtg tgatctgcct 3360

ccttctaact ctttccgagt tttatctcac agctctcata atcacagcat gtatgccagt 3420

gccgctagct gggaaattca cgttctggac tgtagcattc ttcagaatta tctctaacac 3480

ttcatagttc cctgtcccca atttatttag tggtctctct gatttgatca cacatataca 3540

cacacctctc tcctgcccag gatatggttt gctcttgctg aatggacaac cgtggttcca 3600

gcaccggcga atgctaaccc cagccttcca ctatgacatt ctgaaaccct atgtaaaaaa 3660

catggctgac tccattcgac tgatgctagt gagttcattt aactttattc tcctctattc 3720

ccctcagctg tgctgttctt tctacatcag ctgcagcttt ggctactact actgccacag 3780

ataccatagc tgctgtggct gcggctgctt cttcttcttc ttcttcttct tcttcttctt 3840

cttcttcttc ttcttcttct tcttcttctt cttcttcttc atcgtcttca tctccttttt 3900

tcttcttcct tctctgctta tagtctcctc tctcctcaat ctctctcttc acctactcag 3960

tgtatgcatc aacaagcttt acatatgtat tctactttga atacaggttc ttcacatata 4020

gttaaaatag aaatattcca ggtactagga tatggccata cttgcttatt acccaaggca 4080

aagagcatca gagcatcttc tccttcttca aatcttcaac tttagagaga cttcccagga 4140

tgacacttag ttggggtttc ccttgggtct tagtgcttct gcatggactt gagtcagaga 4200

gatggactcc agatccacat tgtcagagcc aaaccttcca taccaccacc tttcaacagg 4260

cctctgggtt atgactgttt tagttcattg gagctagctt atagttcaga ggttggtcca 4320
ttgccatcat agtggaaatt atggtagaac acagacatat atggtgtgct agggaggtag 4380
ctaagaattc tatatcctga tgcaaaggca gcaggaagag agtgagacac tagtgtgcct 4440
tgagcatctg agttctcaaa gccctacccc aatgaccact tcctccaaca agactacccc 4500
tactctaaca aggccacacc tcctaatgga cctatgaggg ccgttttcat tcaaaccacc 4560
aaccacattc ctctccctga cccccataag cttgtagcca tatcttaatg cagaaataca 4620
atcagttcaa cttcaaaagt cctcatagtc taccacagca ctgttcaaaa gttcaaagtt 4680
caaagtctaa gagactcatg caatctctta actttaatcc tctataaaat caaaataaaa 4740
aaaacagatt acatactcct gacatgtacg ggatatacct tacaatccca aaatggagaa 4800
aaggcagcat ggtgaagaaa tactgaacca aagcaagact ggaaaaacgt tggcaaactc 4860
caaactctgc atctcaatgt cttatgtcaa agtgctcttc tgatttccaa ctcctttcag 4920
ctttgttggc tggttccact ccctgttagc agctttgctc agcaaacaac ccacggcatc 4980
tccaacactg taggcttcca aggcaattgt ggcttcatct tcaagcttca cacagtaacc 5040
tctttaagcc tctatgcaag aacacccatc acacgtgcct ggcttcagca gttttcctta 5100
gacaaggagg gcaatcccat aatccattcc ttctttcctt gactctaaac cagaaccatg 5160
tggccaaagc tgccaagttc tcatactcat tcaattacat cttcaccagc tggttttgat 5220
ggtttccttt actgcctaag cttggctgtc ctaaaactca atctgtgtaa caggccagcc 5280
ttaaacttta agatccacaa acctctgcct cctgagcact tggatcaaag gtgtgcacca 5340
ccaggcctgg ccctgggctt ttctttaatt ccttttcact tttctttaat ttcttttcca 5400
aaattggaag tttatctggg tgggatcttg gtctgaggtc accgctccct ttattccact 5460
tagcatcaag cttttctcta atctgtttat ctccttcaac acaggcctta gctacagtct 5520
acttcctggt gcctcctttc tcctcaaagt atgcattttt gtattttttc tctttctcag 5580
tttgctcctt ttcactgtag atttgcatga gcgaatacta ataaccatac aacaaaatcc 5640
atactaggca attgtaagat atcctctgcc aatgcagcta atccaactct tcaccttagc 5700
cttaggcaaa ccttttgaac aagggcaaaa gcagccacat tctttgccaa aatatcataa 5760
gatatctacg ccacatacta aaatttttct cctctgtaac ctcttgagcc aggaccccac 5820
aattcaaatt accctcagcc ccaccgtcct tcaactacta cagcccatta agccccactt 5880
aaatcattca aatgctttta taatccaaag ttccaaagtc cacattactc caaacaaagc 5940
atggctaggt ctatcgcagc aataccccat tgctggtacc aacttctgtc ttaattaggg 6000
tctcattgct gtgaagagac actataacca cacaaccaat tataaaggac aacatttcat 6060
ggaagccagc ttacagttta aaggtttagt ccattatcat gatgggaagc atggtagcac 6120
aaaggcatac atgatgctag agaggtagct gcgagttcta cgtcatatcc aaaggcagca 6180
ggaagagagt gaaacactgg gtcaagcttg agcatctgag acctcaaagc ctgccccgca 6240
gtaacgactt tctccaacaa ggtcacacct aatccaacaa gtccacacct cctaatagtg 6300

ctgcttccta gaggcctata ggggctattt tcattcatcc acaataatga gtcaattgtt 6360

ctcagcaagc ttttgatgaa tacactgggg acccccatta gaaatgcctt cgtggctact 6420

tgggtgtttt tatactctat atgtagaagt cctaccatta tctcaggttg agctgagctc 6480

tccaatcact gatgtatcat ctttttaatc tcctaaaaac agaattgctt atcaggtaaa 6540

acatgttaac taactgcaaa catgtgaaac ttgtttcctt tcttatgagt ttctatattt 6600

aattttgaag taatttacaa gaatataggt gtcctttcac aaagatttgt tttgcagtct 6660

gtaaaacatc tgttatcaaa attgagtaac ttagaacata gtatcttaca atatagggaa 6720

acaattcagt ctattgagca catattttct agggaaatta agtcaatgtt cccctcccac 6780

tacaggacaa atgggaacag ctggcaggtc aagactcctc tatagaaatc tttcaacata 6840

tctccttaat gaccctagac actgtcatga agtgtgcctt cagccacaat ggcagtgttc 6900

aggtggatgg gtgagtgaca acccttgatc ttcagggcct ttgatcttac caactaatat 6960

ggatttatct gtgatgcatg tgggacattt tggatactca ataaagagtc ttatcccagc 7020

cccaagtcaa tccctgaccc aattgaatcc tatcccaacc acagaccact gatccctagc 7080

tgaggtggaa tcctagacac tcatgccacc ttctaccacc ttctaccttg aaaattacaa 7140

gggttatgag acagagttat gtgcctcagt gttgtctctt cttatcatat agaaatctcc 7200

ccagagtccg tactatctgt gggttggaga gctctacagt ctaatctgtt ctacttcttc 7260

ttcttcttct tcttcttctt cttcttcttc ttcttcttct tcttcttctt cttcttcttc 7320

ttcttcttct tcttcttcag aaattacaag agctatatcc aggccattgg gaacttgaat 7380

gacctctttc actcccgtgt gaggaacatc tttcatcaga atgataccat ctataatttt 7440

tcttccaatg gccacttgtt caaccgtgct tgtcaacttg cccatgatca cacaggttct 7500

gtttgtgcct tctgtctccc tcatttccta agatcagctc aagggggcta ttgatgactt 7560

ctgaggcaga tccagaccct tcagtgcatc cccctatagg acctgggtca catccatgct 7620

agttactttt acactgacaa tgttagcagt tggaatgtca gaatgccagg tgttgaatgt 7680

atgtcatcat attggtaatg tgtagtgaga cctgacccag caactcaagc agagcttcta 7740

ttgcccatgg tgctaagaga gttctgacct gtgtcagatc acactggtcc tctaacccta 7800

cccaatccta tatagcgcac ccgcacatag cttcaggcaa ggagacacat ggagctacat 7860

ggggttactg tagctctcag gtctttggaa taattgatgt tctggaatag atggtgtgat 7920

caagctaagg aaggatcagc tgcagaatgc gggagagctg gaaaaggtca agaagaaaag 7980

acgtttggat tttctggaca tcctcttact tgccagagtg agtatgtgca gcagaggcct 8040

gagagtctgc ctgggaacac acaaggaaca agtatagttc tgatgatgcc tgttgtttcc 8100

ctcagatgga gaatggggac agcttgtctg acaaggacct acgtgctgag gtggacacat 8160

ttatgttcga gggtcatgac accacagcca gtggagtctc ctggatcttc tatgctctgg 8220

ccacacaccc tgagcaccaa caaagatgca gagaggaagt tcagagtgtc ctgggggatg 8280

ggtcctccat tacctggtaa gagttcaagt agaagtgccc ttattctttg ggtctccagt 8340

ctacctgacc tccacattgg actttatttc agggatcacc tggaccagat tccctacacc 8400
accatgtgta tcaaggaggc cctgaggctt tacccacctg ttccaggcat tgtcagagaa 8460
ctcagcacat ctgtcacctt ccctgatggg cgctctttac ccaagggtat gaatttccca 8520
ggcctttcat cctagagaga atggatgctc agtagtctgt gtccttcaac agttctgaat 8580
ccctctgagt gccgtttcct tttcagtaaa taaatgcttc ttactcataa agtacagtaa 8640
taagcttaaa tgacctccta taaagttaaa tgctactttg catgaatgac attgaaagca 8700
ttcttcatca gctaacattc cttcttcttt tgaatctatg atctcatcat gaagcatcca 8760
ttctaatttg ggaacaatga tgttttccag actcaattcc agatgccagc accatataac 8820
tcatagccac acttattctt cagatgtcct cactgtcaca tatgttttca tgttggggct 8880
ggaaacacag gcatatgttt gtacatagaa gtgggaggca gaggtcagca ttagttgtca 8940
ttccacaggc actgtccacc atgatttttc agttcaggag gtcttgtact ggactgtagc 9000
tcatctctta ggctaggctg gccactgagc taggaacctg cctgtcccca cctcaccagc 9060
agtgggataa caagcatgtg ccaccacaca cagtcttttt tatatggatt gaggtctttc 9120
tctcaatccc caatctttta tcatgttcta catttgatat aatgtgtaga catttttata 9180
aaaaactact atttatatat agatcagtaa ataaaatgtg acctcagatt cttgccaagg 9240
caagtaaccg gttactctcc ataacctgat agtcagaccc tattgctgaa gacacaactt 9300
atgtcatcaa acacagagaa aatgatctgg tgcccaacta gatttagacc taattattaa 9360
cccaaatagt gaatagggtc tctttttcaa gcttatttca ctaatagttt tacattatac 9420
atttatacat atatttatat tatacaggca cagaacacac acacaagggt ggggagagag 9480
agagagacag agacagaggc agagacagag agacagtgac aaagacagag agagagagac 9540
agagttttca aaaataaaaa gagcaaatca ggcaaattta aacagaagag gaagagtttt 9600
ttcttgcaga gctccttgga atcttcaatg tgtctatcta gatgtctgtg aaggaaataa 9660
ggaacagagt ttcctcacgc cctgatgatt gacatcttgt ctctagcgta cattggaagt 9720
ggagccccac agttcttttc tgctgacctt ttgctgtggc ttggtatttg tttcctgcca 9780
aactcataca gctatgtaat acccagagtc acacgttaat ggggttggga gggtgaaagc 9840
tttgtgaatt tatgttaatc atggtggtgc tctaaattgt actgaaggcc tcctgtttgc 9900
taagcatgtg ctctaggact gagccactta ccatccccaa gatgagatgg ataaacttgg 9960
cttgactacg tgctttagag cacctgggag tggatagatt acattagggt tggtctcaca 10020
ttattctata tagtggcttt agaaagagaa ggagggagac tgctaagata tacacctgtc 10080
ctcaccatga taggatgcaa ccaagggaga gagctgtttg aactttcagc tccaaatctg 10140
agctaaataa gcttcatata tacatatata tacatacata catatataca gacatacata 10200
catacataca tacatacata catacataca tacatacata catacataca ctgggatgta 10260
cagggtagat agagaatcag acaggggcta gaggaatgtg tttcttttat gagacaaagc 10320
aagtaaaaaa gagcaatgaa ttccatgccg tgagtcatat gtggtccagt ctctgagaag 10380

gcacttgggt tagtgacaga gatgaactga tcaccaaata caagacccct gcaatgtcag 10440
gaaagcaccc tttgtctcac agctcattat ggcaaaactc tagtattgcc acatcctgct 10500
ttggtgatat gtaacccctc tctgaaattt tagtaactca cctgaaaaaa aaaacagaat 10560
aaatcaactc actctgggga cttaatatct taatatctta acatctttag tggaaatcct 10620
tcatcaaatt taagttctaa ctttatttat tcccaaaacc ggttcatctt ttttgcttct 10680
ctctctctaa ttccattttt ttatacttct cacttgtgcc tcaggtttgt ttccaactca 10740
tgcacatgtg ctgaaagtga tggtctatag caccctaacc tcttgtgcaa acgtatcatt 10800
ccctgctgtc tcaggaacca aggagtcacc cctgggtgta acccatcttc tctcacattt 10860
ctatctgccc cacaggtatc caagtcacac tctccattta tggtctccac cacaacccga 10920
aggtgtggcc aaacccagag gtaggaggtt cccccaagga gggcagagga gttggtctca 10980
agatcaatac cacctattgt ccccacctct aggcacattg tccattgtga gttgcaagca 11040
agggtttgac ctctatctat cctgactgaa gtgctgtctc cctgcaggtg tttgaccctt 11100
ccaggtttgc accagactct ccccgacaca gccactcatt cctgcccttc tcaggaggag 11160
cgaggtgaga tagcctgtgt atgatgattg atatactgta aagatctttg cagcattcct 11220
tggttttatc tcatgcatat atggcatctt cgggtatatc ctgattgggg gtggggggga 11280
gaagcttctt gaggatgtgt ctgtgcacca aaggagggtg gcatttcatg acacactgtg 11340
aagacattaa cccattggcc aaacaaattt ctatgcagtg catgcacttg cttgtgttct 11400
gatgtttccc tcactttaag ggcaggactt tacattgatt tttttcacat gactttaact 11460
tcaggtgtcc agcttgtcca atttgggggc tcttaaaggg catgtctttt ccacacctct 11520
atctgaccca tcactattcc tgcgtagttc tctattctag aatcactgtg caaggaatta 11580
atttgcttgc atgtctgcca cattcaactg tccagtgaag tattacatca tttgaggcaa 11640
ggttcacacc agggtatgct aattccacct ttgctctctc tctcccaaca caattttcaa 11700
tccgtgttga tgctcacatc tctcttgcaa ttgcccaatg gccaaagctt gacagataat 11760
gtatattcct caagtgaggc tatgtagtct aacttcagac aaaaggcatt aaaaagaagg 11820
agagtaatct gctctgaagt aaacctctat tttacatata acagcatttt taaggtttct 11880
gggagtgaaa tagaagacac tcttgaaata gctactgaac tggacaaaga ctttcccatg 11940
gcttctaggg aaatccttcc acgggaccta actcactgca aactttaaaa ggtggtgcag 12000
aagaaggatt ttctgagcct atttttcatg aattcatgaa gtggcaaatc agttttcaca 12060
gaacaggcta gaagtaagtt gttcttccag acacaggcta tgtccacaca cttggcaaac 12120
ttcaggattg aatgcactca ccgcaaatca tgccactgcc actagaagac aggagagtat 12180
tgtcttaact cagtagtaac tcagcttaac tcaccgagcc atgctgctgg tggtgtttgc 12240
taagacagag tctcaggctt gtctggagct catggtccct ctacctctgc ctcccaagtg 12300
ttgggattca gacacagtcc cccatggctt gctttggtca gaagcttttt catctttatt 12360
atatttcgtt ctattttttt tcatccctta aatccacatg aattacattt taagaaccaa 12420

ggaaaggtaa ctattttaac tgcaaaataa attttaaaca actcctgtgt gtccggtgct 12480

atgctataaa aatatagaaa taatgatact atcccattcc tgcctctaag agttcaaaac 12540

tgtggcccac aaagacaaat atatctgtga ttaaaatata ttcttccccc attctttcac 12600

tcatccactc agcagtttgc agaatgttcc atgttcctgg catcatccca cgccctgagg 12660

gtggcatggc cttgaatatg gtatagatgg tgccttgagg cctagaagga gcagggtcag 12720

tcatcgaaga aataagagcc aatcactcat tgacctgagc ttaggggata tgaaggaagg 12780

ggtggtcctc aaatacctca acagcttgat cttaagtagg aagtgggatg ggcaggaaag 12840

aggtgagggc agcagccagt agagggtata actgcagcta gacctttgtt gttttgtttt 12900

gtttaatttt atatttttat ttattcactt tacattctac ngccttcctt ccagtcaccc 12960

ctcccacaat tctcccccct ttcccctccc ctcttcccct cctctgagca gatggaggcc 13020

tccagagtgc cgccaccccc caccccccca acccccccac cctgtgtact tcaagtctct 13080

atgagactcg gcacttcctc tcccactgag gccagacaag gcagcccagt tagaagaaca 13140

catctcacat acagacaaca gaaaagctaa acaagaagaa aagcctaagt aaggaagctc 13200

aaatctcact tagaagagga aataaaagtc ataagaggca gatggaggga gagaactggg 13260

aggaagagaa ggagaggagg gggtgggggt ttaggatcag gtatggggaa ggacaggaga 13320

gatggccgga tggccatgaa aattaatgga aatctgcaac tgatgggggt gggggaagca 13380

tctccaggag gagacagaga cctgggataa gggaggagcc caaaaatcaa tgggggtgac 13440

cttagctgtg actcactaca ctggagatat ggaacctgaa gaggcaacta ggcattgaac 13500

acagccctac ctggtggagg tatagaggat cagtgtggag ggagggccag ggagaccaaa 13560

gaagcccagg aaaggaaact gaagaaggaa tagtgtacat ttctgaagta gagttggaaa 13620

gctatagagg ccgatggatt aaggatgtgg ggtaccctaa gtaggaggca gatggggcag 13680

acacttgaag atcctcaaca cagtcttcct gtaggtcctc ttctactgat acccaattcc 13740

actctgtgaa agctatatgc tagatcttcc tgaatgaagt aaacgattaa cttattaaat 13800

gattcacaag tttgacttct agtccttaaa atactgtcct aacagatttc atacgtctaa 13860

aacaagaaaa actaggatca aggtggcatt gaaaaatgtt gaggctatgt caaaaagtca 13920

ggagagccaa gtaggcatga cccgtcatct cagccattaa actgaacctg tgacagtagg 13980

ccctgagtgt tgttctggga tccttcacgg gtcagccctt aaaacaggta agaagctaat 14040

gagttttatt ttccctttct gacccaggaa ctgcattggg aaacaatttg ctatgagtga 14100

gatgaaggtg attgtggccc tgaccctgct ccgctttgag ctactgccag atcccaccaa 14160

ggtccccatc cccttaccac gacttgtgct gaagtccaaa aatgggatct acctgtatct 14220

caagaagctc cactaattcc ggtaaggaca aggacagact ctatgaacat gtggcctggc 14280

accttgtccc ctgtcatccc ttcctgtcac catcttgctt tctgtctgtt catcctcaca 14340

cctatttgct ccctggcctc ttggtctcct cctctctctc tctctctctc tctctctctc 14400

tctctctctc tctctctctc tctctcctct agattcccca tttgtttttt ggtctcctgc 14460

tacccacatc tgactgtgca cctgatcctg aacatccttc atcctccatc cttctctcct 14520

tgcctcttca cttcttacca tctttttccc ctacaggact tcttcattat accaataatc 14580

aacggtcaca accttgatgg tcgggaataa caggacgtta ttatcttaca gttgtggagc 14640

tccgaaatct gaaatgagtt tcactggcag aaagctgagt tggtggtgtg actagccttc 14700

ttcagaagag tgcttcagag agtcctctcc tcctctcttc agtacagatc acccttctca 14760

gcactggaat attcctctgc tttaaagcca gcacccttcc cataccccct cttctaaaag 14820

ccttcccttt tacaaatgtt cttatgacat catcaagacc actgaaaaac tccaagataa 14880

tttcccatct caatattcct tactccatct aacctactaa gtcccttttg aattatgagg 14940

aataattcaa tttgttccat gggctccaaa actcaaggcc tgagcattat tgtgaaacct 15000

ttattcagcc taatatcatc ttcacaagac tgttacctgg tacgttcatc taaatctccc 15060

ctgcatagtc tctctacctg actattcctc acacaagttt ctttaccttc cctcctttct 15120

ccaataaagt gtccagtgtc ctgcacagca tctgtcttct catgctggct ctgaacacag 15180

gttcccattc tgtttgaaac tgctcctgga tcaggatcca tgatgagttc tctgccttcc 15240

ctgagatgcc atgctctgct ttctcacaca cacacataca ctcacacata cacacacaac 15300

acatacacac acactcacac acacacatac acacacactc acacacactc acacgtacac 15360

acacaacaca tacacacaca ctcacacaca cacatacaca cacacactca cacacactca 15420

cacgtacaca cacaacacat acacacacaa cacatacaca cacacacaca cacacatgca 15480

cacatcagag ttcttggatt tattcaaata ctcatgaaga actggtatta ataatggtgt 15540

cttgattaga agacaccaga gtcaggaagc ttcagctcaa gttccttcta gattagtgtt 15600

ccgccggttc cttcctttaa tatttaaatt cagtcacagc ctcctcctat cagagagggg 15660

gtacctccac ccagtctaca ctacccgagt gccaaggtgc tgaagaaaca aatgttacag 15720

tttagactca ggcaccaccc agtgacgcat gatggttatg actggagaat cagcgcccac 15780

gtcataactg ttgaaatctt gctacatctg ttcagagaac agtttatctg tgtgctaggg 15840

caaaagggca agttagttgc attaaggctt ctagctaaca ggcacactat aatgggacct 15900

taacatccca gcttcgagtg tacagggagg gctttacagg agttagcaag tatcttaggg 15960

cagagcttcc aagctgtgga cttcttccaa gcagaaaaga attggtagta ggaaaggtat 16020

atggcacatt tcccaagatg ccctagagtg gtggttctga accttcccaa cgctttgacc 16080

ttttaataca gttcctcatg ttggggtgac tcccccaccc ccacccccaa ccataaaatt 16140

aactcattac tactttataa gtgtaacttc gcaaagaaca taatataaaa tctgtgcttt 16200

ctggttattg ttggtggctc ctgtgaaaga gtcattcaac taccaaaggg gtcatgagcc 16260

acaggtaaag ccactgccct agtcccaggg ctagaattc 16299

<210> 815

<211> 560

<212>  DNA

<213>  Rattus sp.


<400>  815
gaaagtgtct gcaagtttta tttgtagact ctgttaagct tgaaccataa aagctgcaaa      60

acagtggttt agagagcatg tcaataccat gggggttggt gggtggaaac tgttccttct     120

gccagttcct aaggctggaa gtggctaggg caggcagtgg cgaggaaata gctggatgag     180

ctgaagcttg ggtggcagtg cttactcaag cctgactcct gcctgtctca ggccctgggg     240

tcatatacac ggcccatgaa gactgggaac ttgtgtcgct ggtcccagag caggaagagg     300

aaaggctgct gcacctcaaa gatgagtaag tttcgggcca cggagatggt ggaggctgcg     360

gctgcttcca cacctgtctc tgtcagttcc aacaccgtct cgtgtttcat ggaagacacc     420

tgaagatctg ggtcctcagt cagcccacac aggttgagat cgtaagtgaa gtcaaagaat     480

tccagtttct ccatgattga cagcatgtct tggatgctct ttactttaat gcgaggcatc     540

atcacgtaag tgggctgaaa                                                 560

<210>  816

<211>  400

<212>  DNA

<213>  Rattus norvegicus (Norway rat)


<400>  816
tttttttttt tttttatgtt cagatgcatt tattcagcaa accttcttgc taaacattat      60

ggtagcccta aaagatttcg aaatcaggac attcagcaaa gccctctcct ctgaatgcgg     120

atagatgttc caaatttcct gttttcaaag catagaatgc attcattggc gtaagttttt     180

ccgtcagtac cacacacagg atcataatcc ctggggcatc caacaagtgt attagggcaa     240

ttagcctttt tcccaatcac cttagctgta gtgttacctg ctaaattgag cagggccaaa     300

gcactgagaa gaaagataat tgctaccttc atgggtgtag acctaggtcc aaaactcaga     360

agaactgtgc agggtgcttc tcttcaacct cgtgccgaaa                           400

<210>  817

<211>  406

<212>  DNA

<213>  Rattus norvegicus (Norway rat)


<400>  817
tttttttttt tttttttgtg gttcacactt tattgcaaat caaatgggtc cttcctgggg      60

accagggcag cacagtgaga tctaatctct caggccaggc tggggattca ggggtctggt     120

acagtcctcc gaaaatcagt gagggaaggg ttggatgcag ggcagagaca agggaggggg 180

taatttgcgc gcctgctgcc ttccttggat gtggtagccg tttctcaggc tccctctccg 240

gaatcgaacc ctgattcccc gtcacccgtg gtcaccatgg taggcacggc gactaccatc 300

gaaagttgat agggcagacg ttcgaatggg tcgtcgccgc cacggagggc gtgcgatcgg 360

cccgaggtta tctagagtca ccaaagccgc cggcgcccga cccacg 406

<210> 818

<211> 664

<212> DNA

<213> Rattus sp.

<400> 818
gcaagcaatg cgcagacaaa aactaaattt attaattaaa gattcatttg aataaatttt 60

gtacagaaga atagtattta ttgcatctat agtataaaag aactgctcac caagctttat 120

aaagtatgaa gcaataagga actaattact taaagtgttt ctatggaggt aagtgtcagg 180

gtaaattgag ttataaccag aatacatcat atgtatgatg atgctaacat tttaccaagt 240

tatttttatc ataatgctta atctgcttaa tcatacatca tcatgatatg cgaaatcata 300

attttttcca ggattggacc acaacatcac gtctctgaac tacaaatata aatatgttac 360

accaatcaca tatttgtaaa aatttataaa ataaaagttg actgggaatc ctgtagcctg 420

gactgatgtt cagaagaact caatctgatt catcgctgct gaagtaggag ctgtcgctgt 480

actcagctgt gtaaaggaac ttgtgaatga tcggattcat ctttggtatc cagtgtcggg 540

gaaccaggta cgatgagagg ttaaataaat ccacgaacac cttgtacctg tcgctcacag 600

ttgagcgcag ataataatag cctgaggatc ccccagtgcc agccttgctg cctagcatcc 660

tgtg 664

<210> 819

<211> 536

<212> DNA

<213> Rattus sp.

<400> 819
atattctttc tattttttat taattgcggc acagagaaaa gaagtcaaat gttcaaagaa 60

atttgtgttc cttagggtgt tggttttaca ccaacagaag agatgagtcc tgagtcttca 120

tgtctttttt tctcagggta gcctgagatg gttgtgatgt gtttaggcta aggcttcttt 180

gcttctagca acaaggtttg gcccctcagt ggcgaagcag ttatccttgt cggcagcctt 240

gcaacacttg tccacgaatt gtgcgaagtc acccatcacc gtcttcagct gatcttctgt 300

ggccttgggc ttgtgtttca ccagctcagc gagagccgtt tgcttcctta tctgcttctc 360

cttgtctggg agtgtgcaga tatcatagtg gaaggtgaag gtctcagctt taaactcttt 420

ggggacatat gtctcgtcaa ctgtcatagc agtgaaacat ggccgtcttt ccaccaggga 480

cccactacag cacttggtga ccttcacgct cactggggtc ttctcatgca gcacac 536

<210> 820

<211> 333

<212> DNA

<213> Rattus norvegicus (Norway rat)

<400> 820
tttttttttt tttttttgga gtctaaactt ttattggtcc ctcagtcccc aaggggttcc 60

cttcttctga gccttagtgc ctcagaactc tggtatcatt ggtctcggac accactttgc 120

catccacgac cttacgggta gttgtcctct ggacagtttg catggagttg ctggagtcca 180

gggcgtcgtt gagactgaaa tcgtccccat cctccaacaa gcggcggtag gtggcaatct 240

ccgcctcaag cttgaccttg atgttcaaca gggcttcgta ttcctgggtc tggcgctgtc 300

cttctgcccg agtttgtgcc agctctgatt cca 333

<210> 821

<211> 405

<212> DNA

<213> Rattus sp.

<400> 821
ggcttttatt attcacatgc tcggtagaaa acggggttta gtaaactggg tggaggtgta 60

cggcaagact ctgagttggt ccggaaatta tttacacctg agggcagcag cactgttcgt 120

cacttcaggc acagcacgtg cacttgtccg aggcaccttt gcaaacacag ccctgggcac 180

atttggagca gcccacgggg cagcaggagc agcagctctt cttgcaggag gtgcatttgc 240

agtttttgca gccgcaggag ctggaccagg tgcaggagcc gccggtggag caggaccagt 300

tggggtccat tccgagatct ggtgaatctg gagcaacggg gtaagctaca agaaggcagt 360

ccctcgtgcc gaattcttgg cctctagggc caaattccct atagg 405

<210> 822

<211> 613

<212> DNA

<213> Rattus sp.

<400> 822
aatttttatt tcaaaagctt ggatagcttc aatatccagg tcgtggcaaa atcaggacac 60

gtgtaaaata ccttacaata cattagattc ccaaaaggta ccaaaaagta cagtaaaatt 120

aacacttcca ttactggaaa tgtatgacac aaataatata aaattaaaag gtgaaaaagg 180

tgacactggt ttcctaagat acagtttact ctttacaacc agggtccaca ggtccaggct 240

gcagagcggc agcaggaagc agaccctcca atctagaaca gggtaacctg gtaataaaat 300

cagcccccta atgccgctct gacctctgag acgccacacg ctgcctacaa caactagagc 360

tctggaatag taaacaggag agtgatttcc gggggaaatt ttaaataaga tgcacatggg 420

acaggccata aaagtttgcc aagttaaatt tggtacataa ttgtgttcac tccgcgtcca 480

aacaaagcgt gtgcggtcgt cactcggccg ctcctgctgc ctctcagtat ggcttgtagt 540

tattctgatg accaccacgt cgctggctct tcccgtaatt tgtactaccc tgactgtagt 600

cgtagccggg gcc 613

<210> 823

<211> 638

<212> DNA

<213> Rattus sp.


<400> 823
gaatttatag agttgatatt acctttattt tatttaatgt tacattaggc atcattgttt 60

cattctgtat gaggtaaaat tgatacaact gtgtgcttaa atccatagtt acaatcaagt 120

tcacaactgc aggctacaaa gtggtttgtg acatttatta caattacatt tttactgtgc 180

aagtgttgca agaatttctc atggtgaggt gtaccccaaa atttgtgtat ctctggaaag 240

gggctcatag aaatttgtgg gtagggctca cagaaatatc aggcaggact gttccaagac 300

ccttcaccag gcctcaggag ttcctagttg cttacaaata ggtccctgtt atatgaccgt 360

aatgaaagaa attccatgat cctccttcct ggattcccaa gcagaagtgg agtccaagat 420

gatttgtcat agtatacctc atctttgcgc agaactgtgc ctttgatgat ctccagggcg 480

cattcctcct tgggagcagc ttgactcaag attatcccag aggtttcttt caaagctgtt 540

tctgtgtcta tgaagccgag gacacagaga gtgatggaca cgttgacctt ggtcatcaag 600

tgttcttttc taatggtgga aaagaaccca tccagagc 638

<210> 824

<211> 465

<212> DNA

<213> Rattus sp.

<400> 824
ctcatttagt ttttattcat aatcataaac ttaactttgc aatcctgcta gacttgaagg 60

ggaatgagga aaatatggaa cccaaagaac ttcagtgaga gcagagatta cagggtattg 120

cgagcagatg gggtgggggt gctctcctga gctacagaag gaatggtttg atgggtaaaa 180

tgcccgcaag tcaaagaaat tagagttgtc cacagtcgga gatggtgatc ttcttgctgg 240

tcttggcagt gcagataaaa aactgggaac catttgtgtt tggtccagca tttgccatgg 300

acaagatgcc aggacctgta tgcttcagga tgaagttctc atcctagaaa tttctctccg 360

tagatggact tgccaccaat gccattatgg cgtgtgaagt caccaccctg acgcatgaat 420

cctggactag ttatgtgaaa ggagtaaccc ctatagccaa atcct 465

<210> 825

<211> 1000

<212> DNA

<213> Rattus sp.

<400> 825
caggagcatc tggacagtac accacttgtg atggagactt ctatgcctga atactatgac 60

gtttttggtg atttccatgg atttttaatg gataaacggt tcaccaaata ttgggaagac 120

gttgaaacat tcttggcaag gccagatgac cttctcattg ttacttatcc taaatctggc 180

agcacatgga ttagtgaaat tgtggatatg atctataaag aaggtgatgt ggaaaaatgc 240

aaggaggatg cactttttaa cagaatacct gacctggagt gcagaaatga agatctaata 300

aacggaataa aacaactaaa agaaaaggaa tcgcctagaa tagtgaaaac tcacctgcca 360

gctaagctcc ttccagcatc attttgggaa aagaattgca agataatcta tctttgccga 420

aatgccaaag atgtcgtcgt ttcttattac tactttttcc tgatcatgaa aagttatcaa 480

aatcctaaat ctttttctga atttgtggag aaatttatgg aagggcaagt tccgtatggt 540

tcctggtatg atcatgtaaa atcttggtgg gagaagagta agaattcacg tgttttgttt 600

atgttctatg aggacatgaa agaggatatc cgaagagaag ttgtgaagct gatagagttc 660

ctggagagag acccatcagc agagctagta gacagaatca ttcaacatac atcattccag 720

gagatgaaga acaatccatg catcaattat tcaatgctgc cagagaccat gatagatcta 780

aaagtatcgc ctttcatgag aaagggaatt gtaggagact ggaagaacca cttccctgaa 840

gccctgaggg agagatttga ggagcactac cagcagcaaa tgaaggactg ccctgtgaaa 900

tttagagcag agccctgaga caattccttg tgtctgaaat tggagtagtc tccaatttat 960

ccttcagttt ttcttgtttt gaattcagta gaagtagaag 1000

<210> 826

<211>  3237

<212>  DNA

<213>  Rattus norvegicus (Norway rat)

<400>  826
cggggatggg gagagttcac ccgtggcaga ggaatcaaca aagaggggcc agggctaaaa      60

ctcagttttc accccagaag ctgagcctct taacagatag agccccccac ctatttccat     120

ttaagcctcc agcccttttc cgtacaacga atgcaccaga ccccgcggga aaggggagaa     180

gcagtactca gtgccccagc accaaggcct ggattattca atgaggagtc agctcctttt     240

tggggggggg gggtcacaag gcatcaaaac tccacctctt tcctctgccc tgctgtgaag     300

ggggggggag agcaacccgc ctcgtgacag ggggttggcc cagcccgccc tagccctgag     360

gagggggcgg gcagggggga gtcctataat tggacaggtc tgggatccgg tcccctgctc     420

agaccccgga ggctaaggag ttgtttcgga aggagctggt aagacaagct tgggctggcg     480

attcacccag ggggcttggt aagacgtggg caggggacct tgaaatccgc tggagtccag     540

gaaacaggca caattattag aaaagcagga agcccgatag aagacttagg gtggcgggga     600

gacaaactaa gatcgtgagg taactagcct ttgccggggt cagaacagat ggaggagatg     660

gaggggtcat ctccaggagt actgtcggtg cacggcatac gggctcaact agaaggtagt     720

atcaatcttt agcgaaggac ccgcgatgag ccttaggttg cgtttatgta aatacctact     780

gatttccatc acagtcccca aaattaccct ggccagtttc aaagcgaaga ccctcatgcc     840

tgagcgtccg aagttctggg gtcgcgggag ggtaccactt cgcagggatg gaggacgatt     900

aaaagcttac attagtacta aaccgagagc ccggcttagc agggaaggta gaaaggaatg     960

taaattggac caccggctgt ccctctgcgt tgtggaattt gaaccctagg actgggagct    1020

ggaatttttg gcagcgggtc caccccgggg tgctgagata gagataggag ggggaggtaa    1080

atagaccttt gggcagatgc tctattgtgg agatgtttgt gatgactcac agacctgaga    1140

agggaattaa ggtgggggct ctttgggtgg cccgagtttc tccaccccta cccactggtg    1200

ttcaaagaca gcttttcctt ccgcagactg gccaatcaca actgggaaga tgaaggctct    1260

gtgggccctg ctgttggtcc cattgctgac aggtatgggg caaggggtgg ctttccctgc    1320

tggcaggttg gggttggggc agttctgaga cctctgagat tcaatagccc tgagcagctg    1380

ttttacatgc aggccttgtc tttccctatc cataatatga gaggattgat gctcagaggg    1440

taaaggtgct tgtcataaag ctcgaagact tatctcggag cccaacatgg tggaaggaga    1500

gagcggattc tctctagttg tcctctgacc tccaagggta cacacacaga cacacaatta    1560

aaaagttgaa gtgtgtaatt tcttagtcgc ttttaaaggg gaggtataca ttttctaacc    1620

ccataccagc ctagtctcag ctctgttcta cataggatac tagacagtgt atgcttgtag    1680

tatctaaaca gactccacga ctgacttcca gacgcactgt tgcttcaaag agacccaaga    1740

gcactgtagg tcctgaccca gccttaaact tactactcta cacaggatgc ctggccgagg    1800

gagagctgga ggtgacagat cagctcccag ggcaaagcga ccaaccctgg gagcaggccc 1860

tgaaccgctt ctgggattac ctgcgctggg tgcagacgct ttctgaccag gtccaggaag 1920

agctgcagag ctcccaagtc acacaggaac tgacgtgagt gctcagcgct tcaccctccg 1980

cacctgctga gtatccagat ccaggggttc ctcctatctg ggcacctacc tacttcttgt 2040

ttcctttctc catgagtgtg tgggccaggt tggcttgaac tctcaatact tctgctttct 2100

ttagccttct ggatactggg atgaacaggc attcatttat gttcttggtc gaatggcttt 2160

tggctttttg agacaggatc ccaatctaac ttaagctggc ttcgaaggct ctgcaattct 2220

ccttcctcag cttctcaact tctgggaata caagcgagta ccaccgcacc tcgctctgtg 2280

gtttcctccc accctaattc tgaccttttt gtctctgcat tcatctccct cttgtgtttc 2340

ctctgggcct gcagggtact gatggaggac actatgactg aagtaaaggc atacaaaaag 2400

gagctggagg aacagctggg cccagtggcg gaggagacac gggccaggct gactaaagag 2460

gtgcaggcgg cacaggcccg tctgggagct gacatggagg atctacgcaa ccgactcggg 2520

cagtaccgca acgaggtaaa caccatgctg ggccagagca cagaggagct gcggtcgcgc 2580

ctctccacac acctgcgcaa gatgcgcaag cgcctgatgc gggatgcgga tgatctgcag 2640

aagcgcctag cggtgtacaa ggccggggca caggagggcg ccgagcgcgg tgtgagtgct 2700

atccgtgagc gcctggggcc actggtggag cagggtcgtc agcgcacagc caacctgcgc 2760

tggcgccgcc cagccccgcg cgatcgcgcc caggctttga gtgaccgcat ccgagggcgg 2820

ctggaggaag tgggcaacca ggcccgagac cgcctagagg aggtgcgtga gcagatggag 2880

gaggtgcgct ccaagatgga ggagcagacc cagcagatac gcctgcaggc cgagatcttc 2940

caggcccgca tcaagggctg gttcgagccg ctagtggaag acatgcagcg ccagtgggca 3000

aacctaatgg agaagataca ggcctctgtg gctaccaact ccattgcctc caccacagtg 3060

cccctggaga atcaatgatc atccctcacc tacgccctgc cgcaacatcc atgaccagct 3120

aggtggccct gtcccaagca ccactctggc cctctggtgg cccttgctta ataaagattc 3180

tccaagcacg ttctgagtct ctgtgagtga ttccacacag cttcagcctc agtttat 3237

<210> 827

<211> 1840

<212> DNA

<213> Rattus norvegicus (Norway rat)

<400> 827
cgagaaacta caggcaaact ttcagctttg aagttgtcac cttggagaag tgaagtcgag 60

aagtgtgaca ccatgaatgg acctgtggat ggcttgtgtg accattctct aagtgaagag 120

ggagccttca tgttcacatc tgaatcggta ggagaagggc atccagataa gatctgtgac 180

cagattagtg atgcagtgct ggatgcccat ctcaagcaag accccaatgc caaggtggcc 240

tgtgagacag tgtgcaagac agggatggtg ctcctgtgtg gagagatcac ctcaatggcc 300

atgattgact accagcgggt ggtgagagac accattaagc acattggcta cgatgactct 360

gccaagggct tcgacttcaa gacctgcaat gtgctcgtgg ctctggagca acagtcccca 420

gacattgccc aatgtgtcca tctagacaga aatgaggagg acgttggtgc aggagatcag 480

ggtctgatgt tcggctatgc cactgacgag acagaggagt gcatgccgct caccattgtt 540

cttgctcaca aactcaacac ccggatggca gatctgaggc gctctggtgt ccttccctgg 600

ctgagacctg attctaagac tcaggtaaca gttcagtacg tgcaggataa tggtgctgtc 660

atccctgttc gcgtccacac catcgtcatc tctgtgcaac acaacgaaga cataacactg 720

gaggccatgc gagaggccct gaaggagcag gtgatcaaag ctgtggtgcc agccaagtac 780

ctggatgaag acaccatcta ccacctgcag ccaagtgggc ggtttgtcat cggaggtccc 840

cagggggatg caggtgtcac aggccgcaag attattgtgg acacatacgg aggctggggt 900

gcccatggtg gtggtgcctt ctctggaaag gactacacca aggtggaccg ctcagcagct 960

tatgccgccc gctgggtggc caagtctctg gtgaaggctg ggctctgccg gagagtcctt 1020

gttcaggtgt cctatgccat tggtgtggca gaacctctgt ccatttccat tttcacctac 1080

ggaacttcca agaagaccga gcgagacgag ctactagagg ttgtgaacaa gaactttgac 1140

ctccggccgg gtgttattgt cagggacttg gatctgaaga agcccatcta ccagaagact 1200

gcatgctatg gtcatttcgg aagaagcgag tttccctggg aggtccccaa gaagcttgtg 1260

ttttagagct gccgggaagc ttagctctgt cctatactca acctcctcaa gatctcaact 1320

ccctggtctc agagagccct ccctgcttcc caccctatgc aaagccagct tcctccgatt 1380

ttccccctac ccggcaatct gcaaacatct taatagtggc aaggcaggtg gtttctggtg 1440

ctaggtcatc aggtctcctt gcaaggatag tcagtgttcc ctccctgtct ctcagatgac 1500

aacaatgtta attttgtgta aaagttgctc ctgtcatccc ctcccgtagt ctgaagctaa 1560

ccaggccatc tcttcctccg tcctggacat gtgcccagat gaatgttttg tcccatatct 1620

ctggcttccc ctttacattg gcagccagag gtcctgagcc ggggcttcct cagggtccct 1680

ctgagactgg gcatctgctc cctaggatat ggtcagtgtc ccctgaacat ctgcattctg 1740

tgagggactg agctgtgtgt tcttcatccc aacacaataa ggtggtatcg tcattatcca 1800

cagtgacaga tggggaaact gaggctcaga gagcgagctc 1840

<210> 828

<211> 9176

<212> DNA

<213> Rattus norvegicus (Norway rat)

<400> 828
aagcttcgca tgcctgagct gctctgtttg caacagagga aggtactcat gctagttcgc 60

tcaatgagga cctgtaacat ttagagagac tataaaaaca agtaaaatat ttccatgttt 120
aagtttctga tctactggaa gagacagatc atgcctccta caatgataaa taccacaagt 180
aatagctgat gcaagaaaag atagagaaac tagggaacat ttatttcagg aatccaacca 240
ggagcatgca gaatgtccag caaatatttg ggtgctcaat aagctgttgg tgaccactgt 300
tactaggagg ggcttcacat aggaagcaga atttcagaga aagtaggact ggttgccagg 360
taacagaata tcctgttaag tctgaattcc acatgtacaa caacattcta gtacaagtat 420
attcagaaca tcacaaaaga catgcacgct ataagttgta ttggttttct gtctgcaatt 480
ccaatttaac tggtcatcct cagcttttac tactagtcat agcacactta agtcacaggc 540
ttcttttatg tcactgaatc atgactgata cgtaaatgtc ctatatgttt gatgtgaaat 600
aacccaagct cattagcaca tgtatcacct catttaatta tcttttcctt tttcttcctt 660
tttgtttccc tggtttctca tctcctagca accactactg tgctcaacca ctactgtgtc 720
ccaatctctt gagttacaca cttaaggttt cacatggaag tgaaatcaca tctttcgctt 780
agcgtgaggt cctcaaggtt cctgcatatt tccccaaatg cttgattttc ttctttccac 840
agcctgaaca atgatctctt gtatctgccc ttctgcttta tccattcatc cattgcatta 900
ggctgcttcc attgtgtggg attgtgaata atacatcaat attctcttca aagtccagct 960
gttgcttttg ggggccgggg ggtatagaag tgggtgggtg gctggcacga aagtttcact 1020
gcgttcagaa gaattgtaca aggaaaggaa gagcagaggt caggcccaga ggcacaagag 1080
gaaagaaagc acattctcca tgacacttct ccaatcatgg ccagcactta ctcccaggtg 1140
ttggtgacaa tcattcccca aaggccttga aatagctctc ccatttgttt accaacatgt 1200
gtaggatgtt gttttcgccc ctgttcctta aatgaggaga ctgattcaca aggatgagca 1260
ggtgaccttc ataagtgcac agaaccagga agctggacct aggattgttg gtgtttggcg 1320
ccatcggtta ctgtcttgac ctttgggtag aggaaaataa tctgttaaca taaatggctt 1380
ttaggtcatt ttgaaattca gatgagctct gaatcctaca cctagtctaa tgtctaatgt 1440
ctctgcttca agaagtgata gccagaatcc tctgtcagtc ctcatacttc ttcagatgtg 1500
aaagtgttca tctttgtagc ttcaaaggcc ccacttcctg gaatgtagaa tctccccgcc 1560
cacaaatgct gtctacacaa tcaaatgcta ccatttgcaa caacttatcg gaaacaaaca 1620
agctacagag aattgagcaa gaatttctgg gatgccgtgg ttattatggt cagagcaaag 1680
gacacactgt gagctttggc tatctgagta ggacaagggt gatgattaac ctagtttcct 1740
gcaggtttaa gtaggatagg agcagtgagt gaagtcagtc ctccttccct tcagcttcgg 1800
tgcttcccat gagccatccc tgcaatcaga aactatgctt tccctgaggg tcgcctgcct 1860
catcctgagc ttggccagca cagtctgggt atgtgctttc tcttctcctc actctctgtt 1920
atttcttcct cgaggagttt tgatttcaga gactaccagt cttttgttct tagcattata 1980
aatgccagac caggaggcaa attcctaggt aagcctgaca agtctagggg gatgtgactt 2040
ccagagggag gccctagggg aacaaggcat cttgacacct gtcattcagg ccgattcaga 2100

ttcagtcttt caacactgca ggtgtgtttg ttagcataat ttctcggtgc tgggacttga 2160

tcatgttgtg atgacctgca accataaaat tatttttgtt actacttcat aactttaatt 2220

ttgttacttt tatgaaccat attgccaaat attttggggg ataaagggtt gccacagggg 2280

tcatgacccc caggttgaga accactgggc atgccagtaa atccctctac aactgagcta 2340

tagtgacaga tttccagcct catgaatccc caccaccacc accacatctt tgtccctcta 2400

ccctctggag acatcattct aacagaacaa aacatttgat aagaactgat ctctagctgg 2460

taattccaga catttgtctt tgatgagcag ggtttagtat gatttacctc taggttttgc 2520

tttatctgta aacgttttag ttttgtttgt aatattgagg actgaagcag aactttctga 2580

agtgctgacc aagcattcta cacctgcagc cctaagaaga acttgttata tctttttgaa 2640

gacataaaag gaaaagggca aattaattgc ctttgaaaac atatagcaaa ttccaaagaa 2700

atttgtcatg aggcagtgag gaaggatttg tgttcctttt agataacttg taaatactga 2760

catcttttcc aaaattaagc tccaaagaca acaaaagaaa gaaacctaaa ttaatggagc 2820

ttctgaaaca ttttaatgta taaaatgtgt caactatgac caaggaccta agagatatcc 2880

taattcgtta cccaggctgt gtattattgt attatttcag ttgtttttgt tggtgagttt 2940

tttttttttg ctttccattc aaaaattttg atatcaagag taaaaaataa catatttttg 3000

agggaattaa acctaaataa ccagctgagg cgatatttct ggataatttt tccttttatt 3060

gtcttcctta tctcttctta ttatgtgcac tttctgtttg ctctattctt gtactatttc 3120

attcatacaa ttgcattttc cattatgctt cttatacaaa agggctctac ttgttctttt 3180

taaataaatt gttctctgct gctttaacta tgctaattaa gattatttga attttcacaa 3240

acaagaatga gattgtgttg ataattataa ggatgaacta tcccacacta acatagtgac 3300

aggaaacctg taagttggca gtgctgagtg aggcatgaag acctcgaacc aatcgaagcc 3360

aagcattccc atcccttaga ctaggaagtc ttatgggaca caatgtttgt atttcatttg 3420

gtttatagct gagaactttt agctttgggt ttctaattat aaggtgttta aaaaattgct 3480

ggttgctgac tactgtttca actgttcatt attttcattt caaatgaaaa tcttcagttg 3540

catgattgtc ctgcaaagca ttgccaagtt ttaactttcc acatttgtat acttgataag 3600

tgcttgtctg aatcatggac cgtctccaaa ggttaccata gaaacctgaa ggagaaagga 3660

gcatgggcac caagagggca tagattttcg aatacacaga gaggtcttag gagaaaaaac 3720

tagacttttt cagctaactt gtctatggtc atgaaagaaa agtcaacagt gaaatttaat 3780

tgatgctgtt aatcgggata atttttcttt taaaacccct aacatctagc agatgcttat 3840

ctagagtcaa atcctgtttt acaaattcag cctttacagc agcattggct gttaatgtct 3900

gtcatttctc ctctgggctt ttgagcatga caatgtctct tctgctggct aaccttgttg 3960

cctttgctcc tttttgaata tttgagaccc cttaaagact gcagacaccg gcaccacaag 4020

tgaattcata gaagcaggag gagatattcg tggcccaaga attgtggaga gacagcctag 4080

tcaatgcaag gagacagatt ggcccttctg ctctgatgaa gactgggtaa gcaggggaca 4140

tgttgatcag gggtccttcc ttatgtcact gtctgtctgt ctgtctgtct gtctgtctgt 4200
ctatctatgt atctatctat ctgtcctata atataaataa tatgttaaca tattatttgc 4260
acacacacat atacatatat ttgtttcaag gaggattgtt agttatgttg ggtctgtcat 4320
gggataaaca catgggatgc ctgagtagtg ggactacaaa attcccagag catcatgcaa 4380
gactaagtgg aatgtcattt cagaatttcc ctatggcctg ttaactacct tttgagtctg 4440
tggttacttg gaagagcctg gggaggagaa gccagccaag ggctatgata acattgccca 4500
accttcctag tagctgaaag gcagaccctt cataagatct ctcccttcat tttcagaacc 4560
acaaatgccc ttcaggctgc aggatgaaag ggttgattga tgaagccaat caggacttta 4620
caaacagaat caacaagctc aaaaactcac tatttgattt tcaaaagaac aacaaggatt 4680
ctaattcact gaccaggaat atcatggagt atttgagagg ggacttcgct aacgccaaca 4740
gtaagtggga catatttagt gcttggactt tctaacaagg atggcaacac aattctccag 4800
ttgagaatgt cttcttgcag atgctgcagt tgacttgagc actcgtgtgg aaatcattga 4860
atttaagaga gaatgtcatt tcacaaagtt agaaattagc ttatattttt aatgttccat 4920
atttttcaaa caaagagagg gggcaccttt caagtagcta ttctgctttt atcctacaga 4980
ctaagagtct cagaggtcaa gggacttgtc aatgacacaa aatagaggtc aggtacacgt 5040
tctactgagt caattacgtc tccctaccta ccccacccct ggactcacca ggtctggggc 5100
acactgtggt cactctggga ataaagagca agtccattga agtcccagtt cttgagccct 5160
tgtctgcctt attctgtctc tctgagacct caacagttta tgtcaatggt acaacagtag 5220
ttggcaggta agggattttg ttaacaccca aaagcttaga aaggatttca aagttcaggt 5280
agaaagaaaa actccttgga aaatataagc aataatacat tgaagtccca taaatgaagt 5340
tataatcaaa taatcagatg tgattaaact atttaccttc tacagttttc aagccctcaa 5400
gtaatttctg gatttatttg gattccttgt catgttagag acagcgtgac taagacccat 5460
ggatgactct tgtgtggaac aatctaattt aaccggaaac ttgcagatta gacatccaga 5520
gaacaaacca cagtagaatg aagaatacgt gtggaaatac ttacaagcaa cttccttttt 5580
cacttttatt tatttattta tttatttatt tatttattta tttattgttt attttttaat 5640
tttatgagca aatcagtctg cagctaccca aataccttgc attttctgtt tcagactttg 5700
ataacacttt cgggcaagtg tcagaggacc tgaggcgcag aattcagatc ctaaagcgca 5760
aagtcataga gaaagcgcaa cagattcagg ttctgcagaa agacgtccgg gatcagctga 5820
tagacatgaa gcgcctggag gtaagcctga ggcccgggcc ccaatttgtc tttgactaag 5880
aaaaaaggaa aaggaacact ctagccgcta cggaacgtct cctaaatcca ttatccaccc 5940
aaaatagaag tgtctccacc ctagagaaga agacagaagt ccagaaatgt gaaggaaatt 6000
cttgaagggt caattgtgta tttgaaaaga acaggggctg gggatttagc tcagtggtag 6060
agcgcttacc taggaagcgc aaggccctgg gttcgatccc cagctccgaa aaaaagaacc 6120
aaaaaaaaaa aaaagaaaag aaaaaaaaaa gaaaagaaca tagtctgatc ggtctgctca 6180

ccacatgccg agaccttggc cttagcatca cctaggctct tcaggcaggg ctaacagtaa 6240

gattagtgcc ttcctccttc ccattccaat tctaaaatgg atccaaatag ctcccattgc 6300

acagcggcct ccttggcctc cacagcttcc agtgaggatg gcatgagtgg cgaaagacaa 6360

cgggtaggat agatttttct gagagtcaaa gaaataaaac ccatgcccaa aatgcaaacc 6420

aaccaccagg aactcaatta tttcaataga tagaattcat ttccctgtct tcctctcttt 6480

aggtggacat tgatatcaag atccgctctt gcaaaggatc ctgcagcagg tctgtaagcc 6540

gtgagataaa tctaaaggac tacgaaggtc agcaaaagca acttgaacag gtcattgcta 6600

aagacttgct tccggcaaaa gacaggcagt acttgccagc aataaaaatg tctccagttc 6660

ccgacttggt tcccggaagt tttaagagcc agcttcagga ggggccccca gagtggaagg 6720

cattaacaga aatgaggcag atgagaatgg agctggagag gcccgggaag gatggggctt 6780

cgcgaggaga tttaccagga gattcgcgag gagactctgc aacacgtgga ccagggtcga 6840

agatagaaaa ccccatgacc cctggacatg gtgggtctgg gtattggcgt cctgggagct 6900

ccggatctgg aagtgatgga aattggggct ctgggacaac ggggtctgat gacactggaa 6960

cctggggtgc aggaagctcc agacctagct caggctctgg gaaccttaag cctagcaacc 7020

ctgactgggg tgagttttca gagtttggag ggagtagcag cccagcgaca agaaaagagt 7080

atcacacagg aaaactggtc acttctaaag gagataaaga gctcctcatt ggaaacgaga 7140

aagttacctc tactggcaca agcaccacac gtcgttcatg ctctaaaacc attactaaga 7200

ctgttttggg taatgatggt caccgggaag tggtcaaaga agtggtcact tcggatgatg 7260

gttctgactg cggtgatggc atggacttag gcctgaccca cagttttagt ggcagacttg 7320

acgaactttc ccgaatgcat cctgaacttg gttcctttta tgacagccgc tttggttcac 7380

tcacaagtaa cttcaaagaa tttggcagta agacctctga ttctgacatc ttcacagaca 7440

tcgagaaccc tagctcccat gtacctgagt tttcttccag tagtaaaacc tcaactgtca 7500

ggaaacaagt aaccaagagc tataaaatgg cagatgaggc agcaagtgaa gctcaccaag 7560

aaggagacac tcgaaccacc aagagggccg gagctcgcac aatgagaggt atccacgctt 7620

aactctggga agttgccctg accccctaga ctaagttaac catttctgca aagtgcttac 7680

caggcacgct ctttcttaac ctcttctagt gctttggtgg aatctcattt tttttcatgc 7740

tagactgtac gttccttggg ggcagggact ttgccatgtg tctatttctg taattcccaa 7800

atgcataaca gtgcagtcat ttctcaataa atatatttta aataaatgaa cgaattcttc 7860

tgaaactcaa ttctgagtct gtttaaccga attcattcaa atcgtgtgct actgtaatac 7920

ccaacccgct aactttaaaa gttagtttat gtctccaatt gatatttaga atcaagttta 7980

aaaatttgtt ctattagtat tgattgatga atgcttagta actgccttta actatcattt 8040

gatgttagcc actgcaagta agctttcaaa tccatttgaa ggaagtttgc taaagcatga 8100

gtgtccttac ctgctaaata ttacatctcg atgtaggttc gacctttcct gtgggaggag 8160

ggaagggagg agggaaggca gacagacagg cagtatctaa actgggcaat gcctgtcttt 8220

gtaattaatg agagtaactt cttccaacca gcttaatttt ttttttagac tgcgatgatg 8280

tccttcaaac acatccttca ggtgcccaaa atggcatttt cagtatcaag ctacctggat 8340

ccagtaagat attttctgtt tattgcgatc aagagaccag tttgggagga tggcttttga 8400

tccagcaaag aatggatgga tcactgaatt ttaaccggac ctggcaagac tacaagagag 8460

gtttcggcag cctgaatgac aagggggaag gagagttctg gctaggcaat gactacctcc 8520

acttactcac tctgagaggc tctgtcctca gggttgaatt agaggactgg gctggaaaag 8580

aggcttatgc ggagtaccac ttccgggtag gctctgaggc agagggctat gcactgcagg 8640

tctcctccta ccagggtacc gctggagatg ctctgatgga gggctctgtg gaggagggga 8700

cagaatacac ttcacacagc aacatgcagt tcagtacctt tgacagagat gcagaccaat 8760

gggaagagaa ctgtgccgag gtctacgggg gaggctggtg gtacaatagc tgtcaagccg 8820

ccaatctcaa tggcatttac taccctgggg gcacctatga ccccaggaac aacagtccct 8880

atgagataga gaacggagtg ctctgggttc ccttcagagg agcggattat tctctgtggg 8940

ccgttcggat gaaaatcaga ccgctggtgg gacagtagct gaaggaatgg aaagtggggg 9000

ctctgctttc tttgcttggt tagccgagaa gaatgatcag aagaggaagg tgtcacggat 9060

cttgtgaact ttttagaaat tccctggtgc tattccattg ttctttgtac tgtagctgaa 9120

cacagctgag atgcgttact gctttgaaaa aaaataaagt tttacatttt ttcccc 9176

<210> 829

<211> 3628

<212> DNA

<213> Rattus norvegicus (Norway rat)

<400> 829
tggggatgct agtgcctact gcgctggctg ctcggctgct gagcctgttc cagcaacagc 60

tgggttccct ctggagtggc ctggccatgc tgttctgctg gctgagaata gcattggggt 120

ggccagatcc tggaaaggga cagccacggg tccggggtga gcccaaggag actcaggaga 180

cccacgaaga tccaggcagt gctcagccca caacccccgt tagtgtcaac taccacttca 240

cgcgtcagtg caactacaaa tgtggcttct gcttccacac ggccaagaca tccttcgtgc 300

tgcccctgga ggaggccaag cgaggactgc ttctgctcaa acaggctggt atggagaaga 360

tcaacttttc aggaggagaa cccttcctac aggacagggg tgaatacttg ggcaagctcg 420

tgagattctg caaggaggag ctagccctgc cctctgtgag catagtgagc aatggcagcc 480

ttatccgaga gagatggttc aaggactatg gagactattt ggacattctt gctatctcct 540

gtgacagctt tgatgagcag gttaatgttc tcattggtcg tggccaaggg aaaaagaacc 600

atgtggaaaa ccttcaaaag ctgcggaagt ggtgcaggga ttacaaggtg gctttcaaga 660

tcaattccgt cattaatcgc ttcaacgtgg acgaagatat gaatgagcac atcaaggcgc 720

tgagccctgt ccgctggaag gttttccaat gcctcctgat tgagggtgag aactcaggag 780

aagatgccct gagggaagca gaaagatttc ttataagcaa tgaagaattt gaagcattct 840

tacagcgtca caaagatgtg tcctgcttgg tgcctgaatc taaccagaag atgaaagact 900

cctaccttat cctggatgaa tatatgcgct ttttgaactg taccggtggc cggaaggacc 960

cttccaggtc catcctggat gttggcgtgg aggaagcgat caaattcagt gggtttgatg 1020

agaagatgtt tctgaagcgc ggggggaagt atgtatggag taaggctgac ctgaagctag 1080

actggtgagg tgagattgga cggagactct aaccagctac gaggagtcca cgagcagccg 1140

ctcgccacca cgcacatctg gctgtctacg gactgttgct actgagaatg cactctatgt 1200

tctttctttc agtttgactg aactaagaaa ggagtaaaac cattctgtga acccttatac 1260

agccgataag ggtcctccac aggggaagga gttcagttca cagaggaaca aggtccagcg 1320

atctcaaaga actcacattt tatttgtatt cgaatttttg tatggatcgt tactgtgaat 1380

cccatgtttc cttttccctt tctgttcccc catgagaaac tgggttattt tctgctaact 1440

atgccagttt tgtcaacagg aaggatcaca aaacacaccc tgcgattact gctgactgtt 1500

ctaatgcttt tctgctttgg aatctcggac tttttgctca gaaatataga atcaagaagt 1560

gtgttataga gccgtacgtg agaaccttac tcttttgcaa ggagcatatg gaaggaggta 1620

cttctgagga cggtgtgttc atggcctcag cctctgctgc tgctgctgct tcaaggatgg 1680

ctgttctata tcgtaccaat tccttcaagg ttctggacaa agccaaggta cactttacct 1740

ttcatggcat ggccgtgaag atggcactga cagccgcaac caccccagca ctgaggctgc 1800

ctgagtctgt caggctaaaa ggttgtccca cttttctgaa aagctacaag ggctcttctg 1860

cacatccagc agaatcatga caggatgggc atggaggact cttcaggagt atgaaatgtg 1920

gctgctgctc cttaccatat ttctcatgaa actctgaaat aagacctagt agtctaggtc 1980

accatgagaa aataatttct tctctacatc aaataagcaa aagagagaac atgaactagc 2040

ccaaatcttc ttcctgtgga agcattgcat gagtttattc aacctagaca gcctcactgc 2100

ttggaaaata attccctgga aattaaataa ataccatgga aaagaaatta aactcctctg 2160

caatcccact gtggccacta gagggcacca ggaagtcaga cttcattgtc tcagagactt 2220

ttcttcagtc tttttttaga atttggtgaa ttttaattct gttccttatc tgttttgtct 2280

ttgttttttg ttttggtgcc tattactaaa attaatacta aacattttcc aaggaactgg 2340

aattttggcc atttacaaaa ggtcttcatt ctttagtgag gtcactataa ttcaactaag 2400

acatactctg tctcattaaa aataggtcag gttttcctgt gttttctgtc ttgtttggtt 2460

gtgtgtgtgt gtttacctat ttttgcttga atatagatat aactatgtat cccaggctag 2520

cctggaactc atgatacctc tgccttaacc tccccctctt aagtgcccag gaattaccca 2580

actcctgtac agagattttt aagagaagtt tgagtcacat tcccccctgc agattgtgat 2640

tttttttttt aagttaagtc ctgtgtacat agttcacatg ggaaaccatg acatcccaga 2700

ggacatgggc agacacccac ttggtcacac gacagaggga agctgcatgt ttctttccct 2760

aattaaagta gagcatgaag aaagtatgat tggaaagtga gccatcattt acatgtaccc 2820

ccaacccctt gtgcatgcac tggcggtggg agatctctgt gcttaaaata ttgaaaagtc 2880

ccctttcctt aaagggaaaa attacgatat tttactatcg tagtacaaat caaacttgga 2940

ttagatcata cattggtttg ctttttcagt gttaaatktg ggtgttcatt ttgtaaagca 3000

tacgctccct gctggggaaa caaggagtga tctagttgac atttgttaag tgtgaccagc 3060

agataccttg tgtcccatga ttgatttatg cagagcagta atactgggaa gggggattta 3120

cttaagaaca tcgacgggta ggacatccaa cactgatttc aaagttttgg cttctttatt 3180

ataatgtgtt cggcggtaaa aaaaagtcat gggttgtttt ctttgaagct gaaagtctca 3240

tggtgttatt tacctaaatt atctgaaaaa tttaaaaggg gcccaggttt cattctgcgt 3300

tggagtttgg gggtctaatt gttgcataaa taaacaggca accaatcatc agaggttgac 3360

tcctcccatc aataattcag ctcgtgtttt acccttttga tggactgatg ttgtttcata 3420

agctattagt agtccaagaa caatcatgca ggcctcatgt gaatgtgttc agatgaggtt 3480

tgatgttcgg gatacatgtg catgtatctc cctgtagaat gacactgtat tttgaggcat 3540

tgctctagtg tatggaaacg tgatctgtgt attaaaatgt tttatgcatg acctcaaaaa 3600

aaaaaaaaaa aaaaaaaaaa aaaaaaga 3628

<210> 830

<211> 21

<212> DNA

<213> Artificial

<400> 830
gcggctcaga tctttcaaag c 21

<210> 831

<211> 22

<212> DNA

<213> Artificial

<400> 831
agtggtcaca tcttggctga gg 22

<210> 832

<211> 22

<212> DNA

<213> Artificial

<400>  832
ataactgtcg gcgtgtacga gg                                                22

<210>  833

<211>  21

<212>  DNA

<213>  Artificial

<400>  833
atccatgtag cgactttccc g                                                 21

<210>  834

<211>  22

<212>  DNA

<213>  Artificial

<400>  834
tttcgccttt gagacagtgt cc                                                22

<210>  835

<211>  22

<212>  DNA

<213>  Artificial

<400>  835
tgaccactct gtttccgttt cc                                                22

<210>  836

<211>  22

<212>  DNA

<213>  Artificial

<400>  836
ttggcctagc caaggtaaaa gg                                                22

<210>  837

<211>  21

<212>  DNA

<213>  Artificial

<400>  837
atagcccacc ctccaaaaac g                                                21

<210>  838
<211>  21
<212>  DNA
<213>  Artificial

<400>  838
tgctcaagta cccccatgtc a                                                21

<210>  839
<211>  20
<212>  DNA
<213>  Artificial

<400>  839
caaatgcgct ttcctgtgga                                                  20

<210>  840
<211>  20
<212>  DNA
<213>  Artificial

<400>  840
ttctttccac cccaattccc                                                  20

<210>  841
<211>  22
<212>  DNA
<213>  Artificial

<400>  841
ctgcatgctt ctgatgtctt cg                                               22

<210>  842
<211>  20

<212>  DNA

<213>  Artificial

<400>  842
ttctttccac cccaattccc                                                    20

<210>  843

<211>  22

<212>  DNA

<213>  Artificial

<400>  843
ctgcatgctt ctgatgtctt cg                                                 22

<210>  844

<211>  22

<212>  DNA

<213>  Artificial

<400>  844
acacatggcc agagttgaag cc                                                 22

<210>  845

<211>  22

<212>  DNA

<213>  Artificial

<400>  845
tcttgaatct cctcagccac gg                                                 22

<210>  846

<211>  21

<212>  DNA

<213>  Artificial

<400>  846
catgatcacg tggattacgg c                                                  21

<210>  847

<211>  22

<212>  DNA

<213>  Artificial

<400>  847
gaacccatca ctggttatcc cc                                              22

<210>  848

<211>  22

<212>  DNA

<213>  Artificial

<400>  848
caacatgcac ccagctattt ca                                              22

<210>  849

<211>  22

<212>  DNA

<213>  Artificial

<400>  849
agtcttggtc cagaactgca gc                                              22

<210>  850

<211>  22

<212>  DNA

<213>  Artificial

<400>  850
ctttcctttg tcctgcattc cc                                              22

<210>  851

<211>  21

<212>  DNA

<213>  Artificial

<400>  851
tcaatgctgc ccttgttctc c                                               21

<210>  852

<211>  21

<212>  DNA

<213>  Artificial



<400>  852
caacccttga tgaccgcagt a                                                21


<210>  853

<211>  21

<212>  DNA

<213>  Artificial



<400>  853
cgccaagaca aatgtgcttt c                                                21


<210>  854

<211>  22

<212>  DNA

<213>  Artificial



<400>  854
gagccgtctt ctggatcgag ta                                               22


<210>  855

<211>  22

<212>  DNA

<213>  Artificial



<400>  855
ggtcccaacg ctgtcttctt tt                                               22


<210>  856

<211>  21

<212>  DNA

<213>  Artificial



<400>  856
caaagccaag caaaccaatg g                                                21

<210> 857

<211> 21

<212> DNA

<213> Artificial

<400> 857
tcacgattgc acatgtccag c                                                21

<210> 858

<211> 22

<212> DNA

<213> Artificial

<400> 858
cccagaacat catccctgca tc                                               22

<210> 859

<211> 22

<212> DNA

<213> Artificial

<400> 859
atgtaggcca tgaggtccac ca                                               22

<210> 860

<211> 598

<212> DNA

<213> Rattus norvegicus

<400> 860
tgctcccatc ctcaagtctt ttcgtccgca atggctccca aaggcggctc caagcagcag      60

tccgaggagg acctgctcct tcaggatttc agccgcaacc tgtccgccaa atcgtcggcg     120

ctgttcttcg ggaatgcgtt catcgtgtct gccattccca tctggttgta ctggagaata     180

tggcatatgg atcttatcca gtctgctgtt ctgtacagcg tgatgacgtt agtaagcact     240

tacttggtag cctttgcata caagaatgta aaatttgttc tcaagcacaa agtagcacag     300

aagagggaag atgctgtttc caaagaagtg acccgcaaac tttctgaagc tgataataga     360

aagatgtccc ggaaggagaa agatgaaaga atcctgtgga agaagaatga agttgccgat     420

tatgaagcta caacattctc catcttctat aacaacactc tgttcctggt tctggttatt 480

gttgcctcct tctttatact gaagaacttc aaccccagag tgaactacat tctgtccata 540

agtgcttcat ctggactcat cgccctcttg tctactggct ccaaatagac tgtgtagc 598

<210> 861

<211> 2023

<212> DNA

<213> Rattus norvegicus

<400> 861
attcggatgc agagcatcag aggcccagct agagggacaa cacagaggag taatttgctg 60

acagacctgc agggatggac ctgctttcag ctctcacact ggaaacctgg gtcctcctgg 120

cagtcgtcct ggtgctcctc tacggatttg ggacccgcac acatggactt ttcaagaaac 180

aggggattcc tgggcccaaa cctctgcctt tttttggcac tgtgctgaat tactatatgg 240

gtttatggaa attcgatgtg gagtgccata aaaagtatgg aaaaatatgg gggttgtttg 300

atggtcaaat gcctctgttt gccatcacgg acacagaaat gatcaagaat gtgctagtga 360

aggaatgctt ttctgtcttc acaaaccggc gggattttgg cccagtgggg attatgggga 420

aagccatctc tgtatctaag gatgaggagt ggaagagata tagagccttg ctgtccccca 480

cgttcaccag tggaagactc aaggagatgt tccctgtcat cgaacagtat ggagacattt 540

tggtaaaata cttgaggcaa gagaaaggca aacctgtccc tgtgaaagaa gtgtttggtg 600

cctacagcat ggatgtgatc accagcacat catttggagt gaatgttgat tccctcaaca 660

acccgaagga tccttttgtg gagaaagcca agaagctctt aagaattgat ttttttgatc 720

cgttgttctt gtcagtagta ctctttccat tcctcacgcc agtatatgag atgttaaaca 780

tctgcatgtt cccaaaagat tcaatagaat ttttcaaaaa atttgtgtac agaatgaagg 840

aaacccgcct ggattctgtg cagaagcatc gagtggattt tcttcagctg atgatgaatg 900

ctcataatga ttctaaagac aaagaatctc atacagccct atccgatatg gagatcacag 960

cccagtcaat catttttatt tttgctggat atgaacccac cagcagcaca ctttcctttg 1020

tcctgcattc cctggccact cacccagata cacagaagaa actgcaggag gagatcgaca 1080

gggctctgcc caataaggca cctcccacct atgatactgt gatggaaatg gaatacctgg 1140

atatggtgtt gaatgaaacc ctcagattgt atccaattgg taatagactt gagagagtct 1200

gtaaaaaaga tgttgaaatc aatggtgtgt ttatgcccaa agggtcagtg gtcatgattc 1260

catcttatgc tcttcaccgt gatccacagc actggccaga gcctgaggaa tttcgcccag 1320

aaaggttcag caaggagaac aagggcagca ttgatcctta tgtatatctg ccctttggaa 1380

atggacccag gaactgcatt ggcatgaggt ttgctctcat gaatatgaaa ctcgctctca 1440

ctaaagttct gcaaaacttc tccttccagc cttgtaagga aacacagata cctctgaaat 1500

taagcagaca aggacttctt caaccaacaa aacccattat tctaaaggtt gtgccacggg 1560

atgaaatcat aactggatca tgattttccc tcaaggagtt ctgctgaatt cttcagaaat 1620

gtggtgtcta agaacaccag accctttaat ttatgtcatg aataaaattc agatgaaatt 1680

agggcttaat cgactttgtt ttgattcggt acatctttgc tctttctcag tgtctacaat 1740

gtacccatct aatataaagg aaatgacaag tcagtgacag aacaggaact taacctttgg 1800

tgattctcat gggactacct ccatccacat ctggttgtct ctgttaattt cttttgatag 1860

taaccttgtc tctgtaattt gatcaagaat ttttcatgaa aatgtgaact attgtgacaa 1920

ctttaattgt agatttggta tcagatgttt tagatgcatt attctacact aaatgttaca 1980

tggaaaaaat gtgaataaac acttctttaa aaatccccat ttt 2023

<210> 862

<211> 895

<212> DNA

<213> Rattus norvegicus

<400> 862
gttcataatt accctgtgct aaacaagact gtttcactgt ctttcctgtc actcaactct 60

cctctgccac cacctgaaac aaaacacttg agtgggaagt atacatgatt tattttaagt 120

tgcttgtgag acttttccct aaaacaacaa atgttgttaa gtcatcaaat tgcctcctct 180

ttaatcttag ttgtatacat tgtcccttca gctataagga gaatgccatg agattatcca 240

gaatccacca tgatcagcca gtgaagcccc tggaccgagc cgtcttctgg atcgagtatg 300

tcatgcgtca caaaggagcc aagcacctcc gctcaactct gcatgacctt agctggttcc 360

agtaccactc tctggatgtc attgggttcc tattgctctg tgtggtaggt gtggtattca 420

tcatcacaaa attctgcctc ttttgttgcc gtaagactgc taacatggga aagaagaaga 480

aagagtagca tcataaaggc tgaagcagag ccctgagaga tgagcctctg ccagctgctt 540

ccagcaggaa cctgttgtca tgccagtgcc ttccctctaa aagaagacag cgttgggacc 600

tcattgaaca tggctccaat gaattcacta tgttctgaag acatgcaaga tttcatgcca 660

aatatatatt cagtgctaaa aaaacaaaat cctgtgttca gtttagaatg ttttgatgta 720

gctgagaagc tttgcccaac aacaataact gaagctactg tagttcataa agttcacatg 780

gctttatagc ctttgcaaaa catatctata aatcaattac tttttgaaaa tacccagcct 840

gctttgtctt catttagtag actatttttc tctccttctt tcttttttct tcttt 895